(19) Europäisches Patentamt / European Patent Office / Office européen des brevets

(11) **EP 4 480 948 A1**

(12) **EUROPEAN PATENT APPLICATION**
published in accordance with Art. 153(4) EPC

(43) Date of publication:
**25.12.2024 Bulletin 2024/52**

(21) Application number: **23752414.5**

(22) Date of filing: **10.02.2023**

(51) International Patent Classification (IPC):
***C07D 401/04*** (2006.01) ***C07D 401/14*** (2006.01)
***C07D 405/04*** (2006.01) ***C07D 405/14*** (2006.01)
***A61K 31/517*** (2006.01) ***A61P 35/00*** (2006.01)
***A61P 37/00*** (2006.01)

(52) Cooperative Patent Classification (CPC):
**A61K 31/517; A61K 31/5377; A61K 31/5386; A61K 31/551; A61P 1/08; A61P 1/16; A61P 3/10; A61P 7/04; A61P 7/06; A61P 9/00; A61P 11/00; A61P 29/00; A61P 31/00; A61P 35/00; A61P 35/02;**
(Cont.)

(86) International application number:
**PCT/CN2023/075345**

(87) International publication number:
**WO 2023/151635 (17.08.2023 Gazette 2023/33)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.02.2022 CN 202210134085**

(71) Applicant: **Gluetacs Therapeutics (Shanghai) Co., Ltd.**
**Shanghai 201306 (CN)**

(72) Inventors:
- **YANG, Xiaobao**
  **Shanghai 201306 (CN)**
- **SUN, Renhong**
  **Shanghai 201306 (CN)**
- **ZHAO, Baoyin**
  **Shanghai 201306 (CN)**
- **ZHOU, Yuedong**
  **Shanghai 201306 (CN)**
- **LI, Yan**
  **Shanghai 201306 (CN)**
- **REN, Yinbo**
  **Shanghai 201306 (CN)**

(74) Representative: **J A Kemp LLP**
**80 Turnmill Street**
**London EC1M 5QU (GB)**

(54) **COMPOUND BASED ON QUINAZOLINE-SUBSTITUTED GLUTARIMIDE SKELETON AND USE THEREOF**

(57) The present disclosure provides a compound of Formula (I) or salts, enantiomers, stereoisomers, solvates, or polymorphs thereof, and uses thereof. The present disclosure also provides pharmaceutical compositions comprising, as an active ingredient, the compound of Formula (I) or salts, enantiomers, stereoisomers, solvates, or polymorphs thereof, and uses thereof. The series of compounds designed and synthesized in the present disclosure can effectively prevent and/or treat diseases or disorders associated with the cereblon protein.

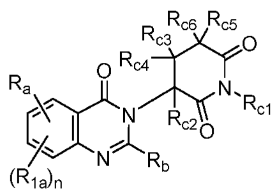

Formula (I)

EP 4 480 948 A1

(52) Cooperative Patent Classification (CPC): (Cont.)
**A61P 37/00; A61P 37/06; C07D 401/04;
C07D 401/14; C07D 405/04; C07D 405/14;
C07D 487/08; C07D 498/08**

## Description

### Technical Field

The present disclosure relates to a compound of Formula (I) or salts, enantiomers, diastereoisomer, isotopically enriched analogs, solvates, prodrugs, or polymorphs thereof, and uses thereof, especially their use in the prevention and/or treatment of diseases or disorders associated with cereblon protein (CRBN).

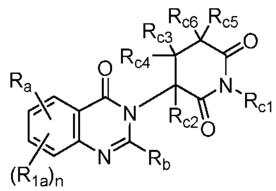

Formula (I)

### Background

Although Thalidomide, lenalidomide and other phthalimide immunomodulatory drugs (IMiDs) have shown significant efficacy in the treatment of multiple myeloma and autoimmune diseases, it was not until 2010 that the E3 ubiquitin ligase cereblon (CRBN) was identified as a direct target of IMiDs. Subsequent research confirmed that these drugs function as molecular glue, inducing the interaction between transcription factors IKZF1/3 and CRBN protein, ultimately leading to their ubiquitination and degradation. Compared with traditional small molecule inhibitors, molecular glue protein degraders have natural mechanism advantages: the former works by occupying the functional domain of the target protein for a long time to inhibit its function, while the protein degraders directly degrade and eliminate the entire target protein, often having a much greater efficacy than traditional small molecule inhibitors. The protein degraders can target "non-drugable" targets, and have low requirements for binding force, catalytic capacity, and low concentration, which can overcome the clinical drug resistance problem of traditional small molecules. Moreover, molecular glue degraders usually have small molecular weights and desirable druggability, so the research and development of such drugs have received great attention. Based on the CRBN E3 ubiquitin ligase and molecular glue degradation mechanism, a series of compounds have been developed, such as pomalidomide, which has been launched, and CC-122 of Bristol-Myers Squibb (BMS), which is undergoing clinical trials, CC-220, CC-90009, CC-99282 and CC-92480, DKY709 from Novartis, and CFT7455 from C4 Therapeutics. The degradation substrates of these molecular glue have also expanded from the transcription factors IKZF1/3 initially discovered to include casein kinase 1$\alpha$ (CK1$\alpha$), zinc finger protein 91 (ZFP91), and translation factor GSPT1. The degradation of these protein substrates will enable the molecular glues to exert immune regulatory, antiinflammatory, and anti-tumor pharmaceutical activities. Currently, the number of discovered candidate compounds for molecular glue is very limited, and there are theoretically a wide variety of pathogenic proteins that can be used as degradation substrates for the development of molecular glue. Therefore, it is necessary to design and develop more molecular glue through rationalization and diversification to degrade and eliminate more pathogenic proteins and apply them to the treatment of diseases associated with these pathogenic proteins.

The compounds listed above all contain the phthalimidyl backbone shared by thalidomide-type IMiDs. Another novel molecular glue, Avadomide (CC-122), developed by Bristol Myers Squibb (BMS), exhibits significant structural differences and remarkable pharmacological effects. Avadomide can bind to CRBN, leading to more pronounced degradation of IKZF1 and IKZF3, thereby downregulating cytokines such as TNF-$\alpha$ and exhibiting antitumor and immunomodulatory activities. Currently, Avadomide has entered Phase II clinical trials to test its therapeutic effects on various related tumors (NCT02406742, NCT02859324, NCT03834623). Based on the quinazolinyl-substituted glutarimide backbone of Avadomide, the present invention aims to develop more novel, highly effective, low-toxicity, stable, and cost-effective molecular glue degraders through rational design to meet the demands of clinical applications.

### Summary of Invention

In view of the above, the objectives of the present disclosure are to provide novel molecular glue protein degraders, their applications and usage methods.

To achieve the above objectives and other related goals, in one aspect, the present disclosure provides a compound of Formula (I):

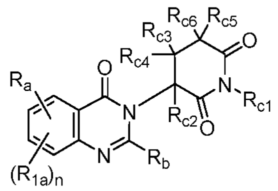

Formula (I)

or a salt, stereoisomer (including enantiomer, diastereoisomer), isotopically enriched analog, solvate, prodrug, or polymorph thereof,

wherein $R_{c1}$ represents H or $C_{1-3}$ alkyl, and $R_{c2}$, $R_{c3}$, $R_{c4}$, $R_{c5}$ and $R_{c6}$ are the same or different and each independently represent H, D or $C_{1-3}$ alkyl;
$(R_{1a})_n$ indicates that quinazoline ring of Formula (I) is optionally substituted with n $R_{1a}$, wherein $R_{1a}$ represents halogen, and n represents an integer of 0, 1, 2, or 3; and
$R_a$ represents the following formula:

$R_{a1}$-$X_2$-$L_1$-$X_1$- ,

wherein $X_1$ represents optionally substituted cycloalkylene, optionally substituted heterocyclylene, optionally substituted arylene, or optionally substituted heteroarylene;
$L_1$ represents optionally substituted linear or branched $C_{1-60}$ alkylene, or optionally substituted linear or branched $C_{2-60}$ alkylene with one or more groups $R_{d1}$ and/or one or more groups $R_{d2}$ and/or any combination of one or more groups $R_{d1}$ and $R_{d2}$ inserted into its backbone carbon chain, wherein carbon-carbon bond between one or more pairs of adjacent carbon atoms in the backbone carbon chain is interrupted by the group $R_{d1}$, $R_{d2}$, or a combination of $R_{d1}$ and $R_{d2}$; wherein each $R_{d1}$ is independently selected from the group consisting of O, S, $N(R_{d3})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, where $R_{d3}$ represents H or $C_{1-3}$ alkyl, and in case that two or more groups $R_{d1}$ are inserted into the backbone carbon chain of the linear or branched $C_{2-60}$ alkylene group, the two or more groups $R_{d1}$ are not directly connected to each other; and wherein each $R_{d2}$ is independently selected from the group consisting of optionally substituted cycloalkylene, optionally substituted heterocyclylene, optionally substituted arylene, optionally substituted heteroarylene, alkenylene, alkynylene, or any combination thereof;
$X_2$ represents a bond; and
$R_{a1}$ represents $NR_{d4}R_{d5}$, $C(O)NR_{d6}R_{d7}$, NHC(O)Ras, $NHC(O)NR_{d9}R_{d10}$, $C(O)OR_{d11}$, $OC(O)R_{d12}$, $OR_{d13}$, $SR_{d14}$, $C(O)R_{d15}$, $C(S)R_{d16}$, $S(O)R_{d17}$, $S(O)_2R_{d18}$, $S(O)_2NHR_{d19}$, $NHS(O)_2R_{d20}$, $S(O)_2OR_{d21}$, $OS(O)_2Ra_{22}$, optionally substituted linear or branched $C_{1-10}$ alkyl, optionally substituted cycloalkyl, optionally substituted heterocyclyl, optionally substituted aryl or optionally substituted heteroaryl, wherein $R_{d4}$, $R_{d5}$, $R_{d6}$, $R_{d7}$, $R_{d9}$, $R_{d10}$, $R_{d11}$, $R_{d13}$, $R_{d14}$, $R_{d19}$ and $R_{d21}$ each independently represent H, optionally substituted linear or branched $C_{1-10}$ alkyl, optionally substituted cycloalkyl, optionally substituted aryl, optionally substituted heterocyclyl or optionally substituted heteroaryl, and Ras, $R_{d12}$, $R_{d15}$, $R_{d16}$, $R_{d17}$, $R_{d18}$, $R_{d20}$ and $R_{d22}$ represents optionally substituted linear or branched $C_{1-10}$ alkyl, optionally substituted cycloalkyl, optionally substituted heterocyclyl, optionally substituted aryl or optionally substituted heteroaryl;
or
$X_1$ represents $N(R_{e1})$, O, S, alkynylene, alkenylene, optionally substituted cycloalkylene, optionally substituted heterocyclylene, optionally substituted arylene, or optionally substituted heteroarylene, wherein $R_{e1}$ represents H or $C_{1-3}$ alkyl, or $X_1$ represents a bond;
$L_1$ represents optionally substituted linear or branched $C_{1-60}$ alkylene, or optionally substituted linear or branched $C_{2-60}$ alkylene with one or more groups $R_{e2}$ and/or one or more groups $R_{e3}$ and/or any combination of one or more groups $R_{e2}$ and $R_{e3}$ inserted into its backbone carbon chain, wherein carbon-carbon bond between one or more pairs of adjacent carbon atoms in the backbone carbon chain is interrupted by the group $R_{e2}$, $R_{e3}$, or a combination of $R_{e2}$ and $R_{e3}$; wherein each $R_{e2}$ is independently selected from the group consisting of O, S, $N(R_{e4})$, C(O), C(O)O, OC(O), C(O)NH, NHC(O), NHC(O)NH, C(S), S(O), $S(O)_2$, $S(O)_2O$, $OS(O)_2$, $S(O)_2NH$, or $NHS(O)_2$, wherein $R_{e4}$ represents H or $C_{1-3}$ alkyl, and in case that two or more groups $R_{e2}$ are inserted into the backbone carbon chain of the linear or branched $C_{2-60}$ alkylene group, the two or more groups $R_{e2}$ are not directly connected to each other; and wherein each $R_{e3}$ is independently selected from the group consisting of optionally substituted cycloalkylene, optionally substituted heterocyclylene, optionally substituted arylene, optionally

substituted heteroarylene, alkenylene, alkynylene, or any combination thereof;

$X_2$ represents C(O), S(O) or $S(O)_2$, or $X_2$ represents a bond;

$R_{a1}$ represents the following formula:

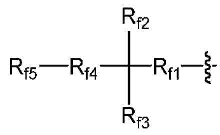

,

wherein $R_{f1}$ represents:

$-NCH_2CH_2N-$;

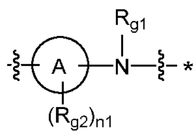

,

wherein symbol * indicates the point of attachment to $X_2$, $R_{g1}$ represents H or $C_{1-3}$ alkyl, ring A represents cycloalkylene or heterocyclylene, and $(R_{g2})_{n1}$ indicates that ring A is optionally substituted with n1 $R_{g2}$ groups, wherein n1 represents an integer of 0 to 8, and each $R_{g2}$, the same or different from each other, is independently selected from the group consisting of deuterium, optionally deuterated $C_{1-6}$ alkyl, optionally deuterated $C_{3-6}$ cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

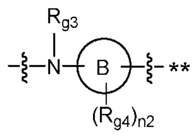

,

wherein symbol ** indicates the point of attachment to $X_2$, $R_{g3}$ represents H or $C_{1-3}$ alkyl, ring B represents cycloalkylene or heterocyclylene, $(R_{g4})_{n2}$ indicates that ring B is optionally substituted with n2 $R_{g4}$, wherein n2 represents an integer of 0 to 8, and each $R_{g4}$, the same or different from each other, is independently selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof; or

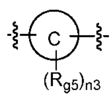

,

wherein ring C represents cycloalkylene or heterocyclylene, $(R_{g5})_{n3}$ indicates that ring C is optionally substituted with n3 $R_{g5}$ groups, wherein n3 represents an integer of 0 to 8, and each $R_{g5}$, the same or different from each other, is independently selected from the group consisting of deuterium, optionally deuterated $C_{1-6}$ alkyl, optionally deuterated $C_{3-6}$ cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

$R_{f2}$ represents H, halogen, $C_{1-3}$ alkyl or halogenated $C_{1-3}$ alkyl;

$R_{f3}$ represents H, halogen, $C_{1-3}$ alkyl, halogenated $C_{1-3}$ alkyl,

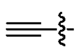, 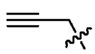, 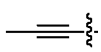

or

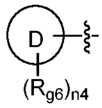,

wherein ring D represents cycloalkylene, heterocyclylene, arylene or heteroarylene, $(R_{g6})_{n4}$ indicates that ring D is optionally substituted with n4 $R_{g6}$ groups, wherein n4 represents an integer of 0 to 8, and each $R_{g6}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

$R_{f4}$ represents

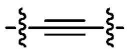, 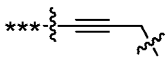, 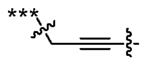 or 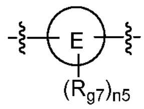,

wherein symbol *** indicates the point of attachment to $R_{f5}$, ring E represents cycloalkylene, heterocyclylene, arylene or heteroarylene, $(R_{g7})_{n5}$ indicates that ring E is optionally substituted with n5 $R_{g7}$, wherein n5 represents an integer of 0 to 8, and each $R_{g7}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof; and

$R_{f5}$ represents H, halogen, $C_{1-3}$ alkyl, halogenated $C_{1-3}$ alkyl,

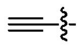, 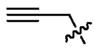, 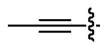

or

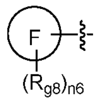,

wherein ring F represents cycloalkyl, heterocyclyl, aryl or heteroaryl, $(R_{g8})_{n6}$ indicates that ring F is optionally substituted with n6 $R_{g8}$, wherein n6 represents an integer of 0 to 8, and each $R_{g8}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;
or

$X_1$ represents $N(R_1)$, O, S, alkynylene, or alkenylene, wherein $R_1$ represents H or $C_{1-3}$ alkyl, or $X_1$ represents a bond;
$L_1$ represents optionally substituted linear or branched $C_{1-60}$ alkylene;
$X_2$ represents $N(R_2)$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted 4- to 8-membered heterocyclylene containing 1 to 2 nitrogen atoms, triazolylene, triazolylene-NH-, -NH-triazolylene, or optionally substituted phenylene, wherein $R_2$ represents H or $C_{1-3}$ alkyl, or $X_2$ represents a bond; and
$R_{a1}$ represents hydroxy, optionally substituted adamantanyl, optionally substituted noradamantanyl, optionally substituted cubanyl, optionally substituted spirocycloalkyl, optionally substituted bicyclo[2.2.2]octan-1-yl, optionally substituted bicyclo[2.2.2]octan-2-yl, optionally substituted bornyl, optionally substituted bicyclo[2.2.1]heptanyl, optionally substituted bicyclo[2.2.1]heptenyl, optionally substituted p-menthanyl or optionally substituted m-menthanyl, wherein when $R_{a1}$ represents hydroxy, $X_2$ represents a bond;
or
$X_1$ represents $N(R_3)$, O or S, wherein $R_3$ represents H or $C_{1-3}$ alkyl;
$L_1$ represents optionally substituted linear or branched $C_{4-60}$ alkylene;

$X_2$ represents $N(R_4)$, C(O)O, OC(O), C(O)NH, NHC(O), C(O), O, C(S), S, S(O), $S(O)_2$, $S(O)_2NH$, $NHS(O)_2$, $S(O)_2O$, or $OS(O)_2$, wherein $R_4$ represents H or $C_{1-3}$ alkyl, or $X_2$ represents a bond; and
$R_{a1}$ represents optionally substituted heterocyclyl;
or
$X_1$ represents alkynylene or alkenylene, or $X_1$ represents a bond;
$L_1$ represents optionally substituted linear or branched $C_{1-60}$ alkylene; and
$X_2$ represents $N(R_5)$, C(O)O, OC(O), C(O)NH, NHC(O), C(O), O, C(S), S, S(O), $S(O)_2$, $S(O)_2NH$, $NHS(O)_2$, $S(O)_2O$, or $OS(O)_2$, wherein $R_5$ represents H or $C_{1-3}$ alkyl, or $X_2$ represents a bond; and
$R_{a1}$ represents optionally substituted heterocyclyl;
or
$X_1$ represents $N(R_6)$, O, S, alkynylene, or alkenylene, wherein $R_6$ represents H or $C_{1-3}$ alkyl, or $X_1$ represents a bond;
$L_1$ represents optionally substituted linear or branched $C_{2-60}$ alkylene with one or more groups $R_7$ and/or one or more groups $R_8$ and/or any combination of one or more groups $R_7$ and $R_8$ inserted into its backbone carbon chain, wherein carbon-carbon bond between one or more pairs of adjacent carbon atoms in the backbone carbon chain is interrupted by the group $R_7$, $R_8$, or a combination of $R_7$ and $R_8$; wherein each $R_7$ is independently selected from the group consisting of O, S, $N(R_9)$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_9$ represents H or $C_{1-3}$ alkyl, and in case that two or more groups $R_7$ are inserted into the backbone carbon chain of the linear or branched $C_{2-60}$ alkylene group, the two or more groups $R_7$ are not directly connected to each other; and wherein each $R_8$ is independently selected from the group consisting of cycloalkylene, heterocyclylene, triazolylene, alkenylene, alkynylene or any combination thereof, wherein the cycloalkylene, heterocyclylene and triazolylene are each independently optionally substituted with a substituent selected from the group consisting of D, optionally deuterated $C_{1-4}$ alkyl, optionally deuterated $C_{3-6}$ cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof;
$X_2$ represents $N(R_{10})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted 4- to 8-membered heterocyclylene containing 1 to 2 nitrogen atoms, triazolylene, triazolylene-NH-, -NH-triazolylene, or optionally substituted phenylene, wherein $R_{10}$ represents H or $C_{1-3}$ alkyl, or $X_2$ represents a bond; and
$R_{a1}$ represents hydroxy, optionally substituted heterocyclyl or optionally substituted cycloalkyl, wherein when $R_{a1}$ represents hydroxy, $X_2$ represents a bond;
or
$R_a$ represents the following formula:

$$R_{a2}\text{-}X_3\text{-},$$

wherein $X_3$ represents $N(R_{11})$, C(O)O, OC(O), C(O)NH, NHC(O), O, S, optionally substituted cycloalkylene, optionally substituted arylene, optionally substituted heteroarylene or optionally substituted heterocyclylene, wherein $R_{11}$ represents H or $C_{1-3}$ alkyl, and
$R_{a2}$ represents optionally substituted cycloalkyl, optionally substituted heterocyclyl, optionally substituted heteroaryl or optionally substituted aryl;
and

$R_b$ represents $C_{1-60}$ alkyl optionally substituted with D or halogen, or
$R_b$ represents the following formula:

$$R_{b1}\text{-}X_4\text{-}L_2\text{-},$$

wherein $L_2$ represents optionally substituted linear or branched $C_{2-60}$ alkylene, or optionally substituted linear or branched $C_{2-60}$ alkylene with one or more groups $R_{12}$ and/or one or more groups $R_{13}$ and/or any combination of one or more groups $R_{12}$ and $R_{13}$ inserted into its backbone carbon chain, wherein carbon-carbon bond between one or more pairs of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group

consisting of cycloalkylene, heterocyclylene, arylene, heteroarylene, alkenylene, alkynylene or any combination thereof, wherein the cycloalkylene, heterocyclylene, arylene and heteroarylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof;
$X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted cycloalkylene, optionally substituted arylene, optionally substituted heterocyclylene, optionally substituted heteroarylene, triazolylene-NH-, or -NH-triazolylene, wherein $R_{15}$ represents H or $C_{1-3}$ alkyl, or $X_4$ represents a bond; and
$R_{b1}$ represents optionally substituted cycloalkyl, optionally substituted aryl, optionally substituted heterocyclyl or optionally substituted heteroaryl;
or

$R_a$ represents halogen or cyano, and
$R_b$ represents the following formula:

$$R_{b2}\text{-}X_5\text{-}L_3\text{-},$$

wherein $L_3$ represents optionally substituted linear or branched $C_{2-60}$ alkylene, or optionally substituted linear or branched $C_{2-60}$ alkylene with one or more groups $R_{16}$ and/or one or more groups $R_{17}$ and/or any combination of one or more groups $R_{16}$ and $R_{17}$ inserted into its backbone carbon chain, wherein carbon-carbon bond between one or more pairs of adjacent carbon atoms in the backbone carbon chain is interrupted by the group $R_{16}$, $R_{17}$, or a combination of $R_{16}$ and $R_{17}$; wherein each $R_{16}$ is independently selected from the group consisting of O, S, $N(R_{18})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{18}$ represents H or $C_{1-3}$ alkyl, and in case that two or more groups $R_{16}$ are inserted into the backbone carbon chain of the linear or branched $C_{2-60}$ alkylene group, the two or more groups $R_{16}$ are not directly connected to each other; and wherein each $R_{17}$ is independently selected from the group consisting of cycloalkylene, heterocyclylene, arylene, heteroarylene, alkenylene, alkynylene or any combination thereof, wherein the cycloalkylene, heterocyclylene, arylene and heteroarylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof;
$X_5$ represents $N(R_{19})$, C(O)O, OC(O), C(O)NH, NHC(O), C(O), O, C(S), S, S(O), $S(O)_2$, $S(O)_2NH$, $NHS(O)_2$, $S(O)_2O$, or $OS(O)_2$, wherein $R_{19}$ represents H or $C_{1-3}$ alkyl, or $X_5$ represents a bond; and
$R_{12}$ represents optionally substituted cycloalkyl, optionally substituted aryl, optionally substituted heterocyclyl or optionally substituted heteroaryl, or represents the following formula:

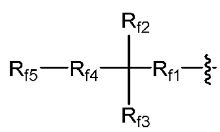
,

wherein $R_{f1}$, $R_{f2}$, $R_{f3}$, $R_{f4}$ and $R_{f5}$ are defined as above.

In another aspect, the present disclosure provides a pharmaceutical composition comprising the compound of Formula (I) or pharmaceutically acceptable salts, stereoisomers (including enantiomers, diastereoisomer), solvates, isotopically enriched analogs, prodrugs, or polymorphs thereof, and at least one pharmaceutically acceptable carrier.
In a further aspect, the present disclosure further provides a medicine kit or reagent kit comprising: the compound of Formula (I) or a pharmaceutically acceptable salt, or the pharmaceutical composition comprising the same.
In a further aspect, the present disclosure provides the compound of Formula (I) or pharmaceutically acceptable salts, enantiomers, diastereoisomer, solvates, prodrugs, or polymorphs thereof, for use as a medicament.
In a further aspect, the present disclosure provides the compound of Formula (I) or pharmaceutically acceptable salts, stereoisomers (including enantiomers, diastereoisomer), solvates, isotopically enriched analogs, prodrugs, or polymorphs thereof, or the pharmaceutical composition for use in the prevention or treatment of diseases or disorders associated with cereblon protein.
In a further aspect, the present disclosure provides use of the compound of Formula (I) or pharmaceutically acceptable salts, stereoisomers (including enantiomers, diastereoisomer), solvates, isotopically enriched analogs,

prodrugs, or polymorphs thereof, or the pharmaceutical composition of the present disclosure for the manufacture of a medicament for the prevention or treatment of diseases or disorders associated with cereblon protein.

In a further aspect, the present disclosure provides a method for treating or preventing diseases or disorders associated with cereblon protein in a subject, comprising administering to the subject a therapeutically effective amount of the compound of Formula (I) or pharmaceutically acceptable salts, stereoisomers (including enantiomers, diastereoisomer), solvates, isotopically enriched analogs, prodrugs, or polymorphs thereof, or the pharmaceutical composition.

**Detailed Description of the Invention**

The following detailed description is provided as exemplary specific embodiments to assist those skilled in the art in understanding and practicing the present disclosure. It should be appreciated, however, that such description is not intended to limit the scope of the present disclosure, and that various modifications and changes may be made to the specific embodiments described in the present disclosure without departing from the spirit and scope of the present disclosure. Such changes and modifications are to be understood as being included within the scope of the present invention as defined by the appended claims.

**I. Compounds**

**Compounds of Formula (I)**

The present disclosure provides a compound of Formula (I):

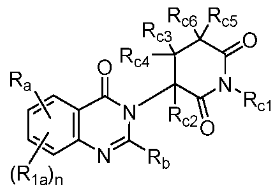

Formula (I)

or salts (including pharmaceutically acceptable salts), stereoisomers (including enantiomers, diastereoisomer), solvates, isotopically enriched analogs, prodrugs, or polymorphs thereof, wherein $R_{c1}$, $R_{c2}$, $R_{c3}$, $R_{c4}$, $R_{c5}$, $R_{c6}$, $R_b$, $(R_{1a})_n$, $R_a$, $R_{1a}$ and n are as defined in the compound of Formula (I) and its various embodiments herein.

In some embodiments of the present disclosure, $R_{c1}$ represents H or $C_{1-3}$ alkyl (e.g., methyl, ethyl, or propyl).

In some embodiments of the present disclosure, $R_{c1}$ represents H.

In some embodiments of the present disclosure, $R_{c2}$, $R_{c3}$, $R_{c4}$, $R_{c5}$ and $R_{c6}$ are the same or different and each independently represent H, D or $C_{1-3}$ alkyl (e.g., methyl, ethyl, or propyl). In some sub-embodiments of the present disclosure, $R_{c2}$, $R_{c3}$, $R_{c4}$, $R_{c5}$ and $R_{c6}$ are the same or different and each independently represent H.

In some embodiments of the present disclosure, $(R_{1a})_n$ indicates that quinazoline ring of Formula (I) is optionally substituted with n $R_{1a}$, wherein $R_{1a}$ represents halogen (e.g., fluorine, chlorine, bromine, or iodine), and n represents an integer of 0, 1, 2 or 3. In some sub-embodiments of the present disclosure, n represents an integer of 0.

In some embodiments of the present disclosure, the compound of Formula (I) is also of Formula (I-1):

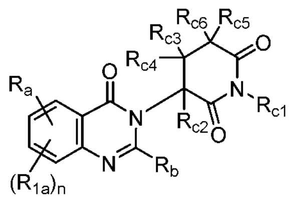

Formula (I-1)

wherein $R_{c1}$, $R_{c2}$, $R_{c3}$, $R_{c4}$, $R_{c5}$, $R_{c6}$, $R_b$, $(R_{1a})_n$, $R_a$, $R_{1a}$ and n are as defined in the compound of Formula (I) above and its various embodiments herein;

wherein $R_a$ represents the following formula:

$R_{a1}$-$X_2$-$L_1$-$X_1$- ,

wherein $X_1$ represents optionally substituted cycloalkylene (e.g., optionally substituted $C_{3-20}$cycloalkylene, or optionally substituted $C_{3-15}$cycloalkylene), optionally substituted heterocyclylene (e.g., optionally substituted 4- to 20-membered heterocyclylene, or optionally substituted 4- to 15-membered heterocyclylene), optionally substituted arylene (e.g., optionally substituted $C_{6-10}$ arylene), or optionally substituted heteroarylene (e.g., optionally substituted 5-to 20-membered heteroarylene, or optionally substituted 5- to 15-membered heteroarylene);
$L_1$ represents:

optionally substituted linear or branched $C_{1-60}$ alkylene (e.g., $C_1$-$C_{40}$ alkylene, $C_1$-$C_{30}$ alkylene, $C_1$-$C_{29}$ alkylene, $C_1$-$C_{28}$ alkylene, $C_1$-$C_{27}$ alkylene, $C_1$-$C_{26}$ alkylene, $C_1$-$C_{25}$ alkylene, $C_1$-$C_{24}$ alkylene, $C_1$-$C_{23}$ alkylene, $C_1$-$C_{22}$ alkylene, $C_1$-$C_{21}$ alkylene, $C_1$-$C_{20}$ alkylene, $C_1$-$C_{19}$ alkylene, $C_1$-$C_{18}$ alkylene, $C_1$-$C_{17}$ alkylene, $C_1$-$C_{16}$ alkylene, $C_1$-$C_{15}$ alkylene, $C_1$-$C_{14}$ alkylene, $C_1$-$C_{13}$ alkylene, $C_1$-$C_{12}$ alkylene, $C_1$-$C_{11}$ alkylene, $C_1$-$C_{10}$ alkylene, $C_1$-$C_9$ alkylene, $C_1$-$C_8$ alkylene, $C_1$-$C_7$ alkylene, $C_1$-$C_6$ alkylene, $C_1$-$C_5$ alkylene, $C_1$-$C_4$ alkylene, $C_1$-$C_3$ alkylene, or $C_1$-$C_2$ alkylene), or
optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{d1}$ and/or one or more (e.g., 1-20, or 1-15, 1-10, 1-9, 1-8, 1-7, 1-6, 1-5, 1-4, 1-3 or 1-2, or 1) groups $R_{d2}$ and/or any combination of one or more (e.g., 1-20, or 1-15, 1-10, 1-9, 1-8, 1-7, 1-6, 1-5, 1-4, 1-3 or 1-2, or 1) groups $R_{d1}$ and $R_{d2}$ inserted into its backbone carbon chain, wherein carbon-carbon bond between one or more pairs (e.g., 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_{d1}$, $R_{d2}$, or a combination of $R_{d1}$ and $R_{d2}$; wherein each $R_{d1}$ is independently selected from the group consisting of O, S, N($R_{d3}$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, where $R_{d3}$ represents H or $C_{1-3}$ alkyl (e.g., methyl, ethyl, or propyl), and in case that two or more groups $R_{d1}$ are inserted into the backbone carbon chain of the linear or branched $C_{2-60}$ alkylene group, the two or more groups $R_{d1}$ are not directly connected to each other; and wherein each $R_{d2}$ is independently selected from the group consisting of optionally substituted cycloalkylene (e.g., optionally substituted $C_{3-20}$cycloalkylene, or optionally substituted $C_{3-15}$cycloalkylene), optionally substituted heterocyclylene (e.g., optionally substituted 4- to 20-membered heterocyclylene, or optionally substituted 5- to 15-membered heterocyclylene), optionally substituted arylene (e.g., optionally substituted $C_{6-10}$ arylene), optionally substituted heteroarylene (e.g., optionally substituted 5- to 20-membered heteroarylene, or optionally substituted 5- to 15-membered heteroarylene), alkenylene (e.g., $C_{2-6}$ alkenylene), alkynylene (e.g., $C_{2-6}$ alkynylene) or any combination thereof;

$X_2$ represents a bond; and
$R_{a1}$ represents $NR_{d4}R_{d5}$, $C(O)NR_{d6}R_{d7}$, $NHC(O)R_{d8}$, $NHC(O)NR_{d9}R_{d10}$, $C(O)OR_{d11}$, $OC(O)R_{d12}$, $OR_{d13}$, $SR_{d14}$, $C(O)R_{d15}$, $C(S)R_{d16}$, $S(O)R_{d17}$, $S(O)_2R_{d18}$, $S(O)_2NHR_{d19}$, $NHS(O)_2R_{d20}$, $S(O)_2OR_{d21}$, $OS(O)_2R_{d22}$, optionally substituted linear or branched $C_{1-10}$ alkyl, optionally substituted cycloalkyl (e.g., optionally substituted $C_{3-20}$cycloalkyl, or optionally substituted $C_{3-15}$cycloalkyl), optionally substituted heterocyclyl (e.g., optionally substituted 4- to 20-membered heterocyclyl, or optionally substituted 5- to 15-membered heterocyclyl), optionally substituted aryl (e.g., optionally substituted $C_{6-20}$ aryl, or optionally substituted $C_{6-10}$ aryl) or optionally substituted heteroaryl (e.g., optionally substituted 5- to 20-membered heteroaryl, or optionally substituted 5-to 15-membered heteroaryl), wherein $R_{d4}$, $R_{d5}$, $R_{d6}$, $R_{d7}$, $R_{d9}$, $R_{d10}$, $R_{d11}$, $R_{d13}$, $R_{d14}$, $R_{d19}$ and $R_{d21}$ each independently represent H, optionally substituted linear or branched $C_{1-10}$ alkyl, optionally substituted cycloalkyl (e.g., optionally substituted $C_{3-20}$cycloalkyl, or optionally substituted $C_{3-15}$cycloalkyl), optionally substituted aryl (e.g., optionally substituted $C_{6-20}$ aryl, or optionally substituted $C_{6-10}$ aryl), optionally substituted heterocyclyl (e.g., optionally substituted 4- to 20-membered heterocyclyl, or optionally substituted 5- to 15-membered heterocyclyl) or optionally substituted heteroaryl (e.g., optionally substituted 5- to 20-membered heteroaryl, or optionally substituted 5- to 15-membered heteroaryl), and $R_{d8}$, $R_{d12}$, $R_{d15}$, $R_{d16}$, $R_{d17}$, $R_{d18}$, $R_{d20}$ and $R_{d22}$ represents optionally substituted linear or branched $C_{1-10}$ alkyl, optionally substituted cycloalkyl (e.g., optionally substituted $C_{3-20}$cycloalkyl, or optionally substituted $C_{3-15}$cycloalkyl), optionally substituted heterocyclyl (e.g., optionally substituted 4- to 20-membered heterocyclyl, or optionally substituted 5- to 15-membered heterocyclyl), optionally substituted aryl (e.g., optionally substituted $C_{6-20}$ aryl, or optionally substituted $C_{6-10}$ aryl) or optionally substituted heteroaryl (e.g., optionally substituted 5- to 20-membered heteroaryl, or optionally substituted 5-to 15-membered heteroaryl);

and

$R_1$ represents $C_{1-60}$ alkyl (e.g., $C_1$-$C_{30}$ alkyl, $C_1$-$C_{29}$ alkyl, $C_1$-$C_{28}$ alkyl, $C_1$-$C_{27}$ alkyl, $C_1$-$C_{26}$ alkyl, $C_1$-$C_{25}$ alkyl, $C_1$-$C_{24}$ alkyl, $C_1$-$C_{23}$ alkyl, $C_1$-$C_{22}$ alkyl, $C_1$-$C_{21}$ alkyl, $C_1$-$C_{20}$ alkyl, $C_1$-$C_{19}$ alkyl, $C_1$-$C_{18}$ alkyl, $C_1$-$C_{17}$ alkyl, $C_1$-$C_{16}$ alkyl, $C_1$-$C_{15}$ alkyl, $C_1$-$C_{14}$ alkyl, $C_1$-$C_{13}$ alkyl, $C_1$-$C_{12}$ alkyl, $C_1$-$C_{11}$ alkyl, $C_1$-$C_{10}$ alkyl, $C_1$-$C_9$ alkyl, $C_1$-$C_8$ alkyl, $C_1$-$C_7$ alkyl, $C_1$-$C_6$ alkyl, $C_1$-$C_5$ alkyl, $C_1$-$C_4$ alkyl, $C_1$-$C_3$ alkyl, or $C_1$-$C_2$ alkyl) optionally substituted with D or halogen, or $R_b$ represents the following formula:

$$R_{b1}\text{-}X_4\text{-}L_2\text{-},$$

wherein $L_2$ represents:

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene), or

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{12}$ and/or one or more (e.g., 1-20, or 1-15, 1-10, 1-9, 1-8, 1-7, 1-6, 1-5, 1-4, 1-3 or 1-2, or 1) groups $R_{13}$ and/or any combination of one or more (e.g., 1-20, or 1-15, 1-10, 1-9, 1-8, 1-7, 1-6, 1-5, 1-4, 1-3 or 1-2, or 1) groups $R_{12}$ and $R_{13}$ inserted into its backbone carbon chain, wherein carbon-carbon bond between one or more pairs (e.g., 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of cycloalkylene (e.g., $C_{3-20}$cycloalkylene, or $C_{3-15}$cycloalkylene), heterocyclylene (e.g., 4- to 20-membered heterocyclylene, or 4- to 15-membered heterocyclylene), arylene (e.g., $C_{6-10}$ arylene), heteroarylene (e.g., 5- to 20-membered heteroarylene, or 5- to 15-membered heteroarylene), alkenylene (e.g., $C_{2-6}$ alkenylene), alkynylene (e.g., $C_{2-6}$ alkynylene) or any combination thereof, wherein the cycloalkylene, the heterocyclylene, arylene and the heteroarylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof;

$X_4$ represents $N(R_{15})$, C(O)O, OC(O), C(O)NH, NHC(O), C(O), O, C(=S), S, S(O), $S(O)_2$, $S(O)_2NH$, $NHS(O)_2$, $S(O)_2O$, $OS(O)_2$, optionally substituted cycloalkylene (e.g., optionally substituted $C_{3-20}$cycloalkylene, or optionally substituted $C_{3-15}$cycloalkylene), optionally substituted arylene (e.g., optionally substituted $C_{6-20}$ arylene, or optionally substituted $C_{6-10}$ arylene), optionally substituted heterocyclylene (e.g., optionally substituted 4- to 20-membered heterocyclylene, or optionally substituted 4- to 15-membered heterocyclylene), optionally substituted heteroarylene (e.g., optionally substituted 5- to 20-membered heteroarylene, or optionally substituted 5- to 15-membered heteroarylene), triazolylene-NH-, or -NH-triazolylene, wherein $R_{15}$ represents H or $C_{1-3}$ alkyl, or $X_4$ represents a bond; and

$R_{b1}$ represents optionally substituted cycloalkyl (e.g., optionally substituted $C_{3-20}$cycloalkyl, or optionally substituted $C_{3-15}$cycloalkyl), optionally substituted aryl (e.g., optionally substituted $C_{6-20}$ aryl, or optionally substituted $C_{6-10}$ aryl), optionally substituted heterocyclyl (e.g., optionally substituted 4- to 20-membered heterocyclyl, or optionally substituted 4- to 15-membered heterocyclyl) or optionally substituted heteroaryl (e.g., optionally substituted 5- to 20-membered heteroaryl, or optionally substituted 5- to 15-membered heteroaryl).

In embodiments of the present disclosure, the number of substituents is not theoretically limited in any way or

automatically limited by the size of the building units.

In some embodiments of the compound of Formula (I-1), $R_a$ represents the following formula:

$R_{a1}$-$X_2$-$L_1$-$X_1$-,

wherein $X_1$ represents optionally substituted $C_{3-20}$cycloalkylene (e.g., optionally substituted $C_{3-15}$cycloalkylene), optionally substituted 4- to 20-membered nitrogen-containing heterocyclylene (e.g., optionally substituted 4- to 15-membered nitrogen-containing heterocyclylene), optionally substituted $C_{6-10}$ arylene, or optionally substituted 5- to 20-membered heteroarylene (e.g., optionally substituted 5- to 15-membered heteroarylene);

$L_1$ represents:

optionally substituted linear or branched $C_{1-60}$ alkylene (e.g., $C_1$-$C_{40}$ alkylene, $C_1$-$C_{30}$ alkylene, $C_1$-$C_{29}$ alkylene, $C_1$-$C_{28}$ alkylene, $C_1$-$C_{27}$ alkylene, $C_1$-$C_{26}$ alkylene, $C_1$-$C_{25}$ alkylene, $C_1$-$C_{24}$ alkylene, $C_1$-$C_{23}$ alkylene, $C_1$-$C_{22}$ alkylene, $C_1$-$C_{21}$ alkylene, $C_1$-$C_{20}$ alkylene, $C_1$-$C_{19}$ alkylene, $C_1$-$C_{18}$ alkylene, $C_1$-$C_{17}$ alkylene, $C_1$-$C_{16}$ alkylene, $C_1$-$C_{15}$ alkylene, $C_1$-$C_{14}$ alkylene, $C_1$-$C_{13}$ alkylene, $C_1$-$C_{12}$ alkylene, $C_1$-$C_{11}$ alkylene, $C_1$-$C_{10}$ alkylene, $C_1$-$C_9$ alkylene, $C_1$-$C_8$ alkylene, $C_1$-$C_7$ alkylene, $C_1$-$C_6$ alkylene, $C_1$-$C_5$ alkylene, $C_1$-$C_4$ alkylene, $C_1$-$C_3$ alkylene, or $C_1$-$C_2$ alkylene), or

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{d1}$ and/or one or more (e.g., 1-20, or 1-15, 1-10, 1-9, 1-8, 1-7, 1-6, 1-5, 1-4, 1-3 or 1-2, or 1) groups $R_{d2}$ and/or any combination of one or more (e.g., 1-20, or 1-15, 1-10, 1-9, 1-8, 1-7, 1-6, 1-5, 1-4, 1-3 or 1-2, or 1) groups $R_{d1}$ and $R_{d2}$ inserted into its backbone carbon chain, wherein carbon-carbon bond between one or more pairs (e.g., 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_{d1}$, $R_{d2}$, or a combination of $R_{d1}$ and $R_{d2}$; wherein each $R_{d1}$ is independently selected from the group consisting of O, S, N($R_{d3}$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, where $R_{d3}$ represents H or $C_{1-3}$ alkyl, and in case that two or more groups $R_{d1}$ are inserted into the backbone carbon chain of the linear or branched $C_{2-60}$ alkylene group, the two or more groups $R_{d1}$ are not directly connected to each other; and wherein each $R_{d2}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH- or any combination thereof, wherein the linear or branched $C_{1-60}$ alkylene and the linear or branched $C_{2-60}$ alkylene are each independently optionally substituted with a substituent selected from the group consisting of deuterium, optionally deuterated $C_{1-6}$ alkyl, optionally deuterated $C_{3-6}$cycloalkyl, hydroxy, amino, mercapto, nitro, halogen, cyano, optionally deuterated $C_{1-6}$ alkoxy, halogenated $C_{1-6}$ alkyl, or any combination thereof;

$X_2$ represents a bond; and

$R_{a1}$ represents $NR_{d4}R_{d5}$, $C(O)NR_{d6}R_{d7}$, NHC(O)Ras, $NHC(O)NR_{d9}R_{d10}$, $C(O)OR_{d11}$, $OC(O)R_{d12}$, $OR_{d13}$, $SR_{d14}$, $C(O)R_{d15}$, $C(S)R_{d16}$, $S(O)R_{d17}$, $S(O)_2R_{d18}$, $S(O)_2NHR_{d19}$, $NHS(O)_2R_{d20}$, $S(O)_2OR_{d21}$, $OS(O)_2R_{d22}$, optionally substituted linear or branched $C_{1-10}$ alkyl, optionally substituted $C_{3-20}$cycloalkyl (e.g., optionally substituted $C_{3-15}$cycloalkyl), optionally substituted 4- to 20-membered heterocyclyl (e.g., optionally substituted 5- to 15-membered heterocyclyl), optionally substituted $C_{6-20}$ aryl (e.g., optionally substituted $C_{6-10}$ aryl) or optionally substituted 5- to 20-membered heteroaryl (e.g., optionally substituted 5- to 15-membered heteroaryl), wherein $R_{d4}$, $R_{d5}$, $R_{d6}$, $R_{d7}$, $R_{d9}$, $R_{d10}$, $R_{d11}$, $R_{d13}$, $R_{d14}$, $R_{d19}$ and $R_{d21}$ are the same or different and each independently represent H, optionally substituted linear or branched $C_{1-10}$ alkyl, optionally substituted $C_{3-20}$cycloalkyl (e.g., optionally substituted $C_{3-15}$cycloalkyl), optionally substituted $C_{6-20}$ aryl (e.g., optionally substituted $C_{6-10}$ aryl), optionally substituted 4- to 20-membered heterocyclyl (e.g., optionally substituted 5- to 15-membered heterocyclyl) or optionally substituted 5- to 20-membered heteroaryl (e.g., optionally substituted 5- to 15-membered heteroaryl), and $R_{d8}$, $R_{d12}$, $R_{d15}$, $R_{d16}$, $R_{d17}$, $R_{d18}$, $R_{d20}$ and $R_{d22}$ each independently represent optionally substituted linear or branched $C_{1-10}$ alkyl, optionally

substituted $C_{3-20}$cycloalkyl (e.g., optionally substituted $C_{3-15}$cycloalkyl), optionally substituted 4- to 20-membered heterocyclyl (e.g., optionally substituted 5- to 15-membered heterocyclyl), optionally substituted $C_{6-20}$ aryl (e.g., optionally substituted $C_{6-10}$ aryl) or optionally substituted 5- to 20-membered heteroaryl (e.g., optionally substituted 5- to 15-membered heteroaryl).

In some embodiments of the compound of Formula (I-1), $X_1$ represents:
optionally substituted $C_{3-20}$cycloalkylene (e.g., optionally substituted $C_{3-15}$cycloalkylene), optionally substituted 4- to 20-membered nitrogen-containing monocyclic heterocyclylene (e.g., optionally substituted 4- to 15-membered nitrogen-containing monocyclic heterocyclylene), optionally substituted 5- to 20-membered nitrogen-containing bridged heterocyclylene (e.g., optionally substituted 5- to 15-membered nitrogen-containing bridged heterocyclylene), optionally substituted 5- to 20-membered nitrogen-containing spiro-heterocyclylene (e.g., optionally substituted 5- to 15-membered nitrogen-containing spiro-heterocyclylene), optionally substituted 5- to 20-membered nitrogen-containing fused heterocyclylene (e.g., optionally substituted 5- to 15-membered nitrogen-containing fused heterocyclylene), optionally substituted $C_{6-10}$ arylene, or optionally substituted 5- to 20-membered heteroarylene (e.g., optionally substituted 5- to 15-membered heteroarylene), wherein the $C_{3-20}$cycloalkylene, the 4- to 20-membered nitrogen-containing monocyclic heterocyclylene, the 5- to 20-membered nitrogen-containing bridged heterocyclylene, the 5- to 20-membered nitrogen-containing spiro-heterocyclylene, the 5- to 20-membered nitrogen-containing fused heterocyclylene, the $C_{6-10}$ arylene, and the 5- to 20-membered heteroarylene are each independently optionally substituted with one or more (e.g., 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and$NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof. In embodiments of the present disclosure, the number of substituents is not theoretically limited in any way or automatically limited by the size of the building units.

In some embodiments of the compound of Formula (I-1), $R_{a1}$ represents:
$NR_{d4}R_{d5}$, $C(O)NR_{d6}R_{d7}$, $NHC(O)R_{d8}$, $NHC(O)NR_{d9}R_{d10}$, $C(O)OR_{d11}$, $OC(O)R_{d12}$, $OR_{d13}$, $SR_{d14}$, $C(O)R_{d15}$, $C(S)R_{d16}$, $S(O)R_{d17}$, $S(O)_2R_{d18}$, $S(O)_2NHR_{d19}$, $NHS(O)_2R_{d20}$, $S(O)_2OR_{d21}$, $OS(O)_2Rd_{22}$, optionally substituted linear or branched $C_{1-10}$ alkyl, optionally substituted $C_{3-20}$cycloalkyl, optionally substituted 4- to 20-membered heterocyclyl, optionally substituted $C_{6-20}$ aryl or optionally substituted 5- to 20-membered heteroaryl, wherein the linear or branched $C_{1-10}$ alkyl, the $C_{3-20}$cycloalkyl, the 4- to 20-membered heterocyclyl, the $C_{6-20}$ aryl or the 5- to 20-membered heteroaryl are each independently optionally substituted with one or more (e.g., 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof;

wherein $R_{d4}$, $R_{d5}$, $R_{d6}$, $R_{d7}$, $R_{d9}$, $R_{d10}$, $R_{d11}$, $R_{d13}$, $R_{d14}$, $R_{d19}$ and $R_{d21}$ each independently represent H, optionally substituted linear or branched $C_{1-10}$ alkyl, optionally substituted $C_{3-20}$cycloalkyl, optionally substituted $C_{6-20}$ aryl, optionally substituted 4- to 20-membered heterocyclyl or optionally substituted 5- to 20-membered heteroaryl, wherein the linear or branched $C_{1-10}$ alkyl, the $C_{3-20}$cycloalkyl, the 4- to 20-membered heterocyclyl, the $C_{6-20}$ aryl or the 5- to 20-membered heteroaryl are each independently optionally substituted with one or more (e.g., 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-,

and $CH_3CH_2CH_2$-NHC(O)-), $C_{1-4}$ alkyl-C(O)NH-(e.g., $C_{1-3}$ alkyl-C(O)NH-, such as $CH_3$-C(O)NH-, $CH_3CH_2$-C(O)NH-, and $CH_3CH_2CH_2$-C(O)NH-), optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof; and

$R_{d8}$, $R_{d12}$, $R_{d15}$, $R_{d16}$, $R_{d17}$, Rais, $R_{d20}$ and $R_{d22}$ each independently represent optionally substituted linear or branched $C_{1-10}$ alkyl, optionally substituted $C_{3-20}$cycloalkyl, optionally substituted 4- to 20-membered heterocyclyl, optionally substituted $C_{6-20}$ aryl or optionally substituted 5- to 20-membered heteroaryl, wherein the linear or branched $C_{1-10}$ alkyl, the $C_{3-20}$cycloalkyl, the 4- to 20-membered heterocyclyl, the $C_{6-20}$ aryl or the 5- to 20-membered heteroaryl are each independently optionally substituted with one or more (e.g., 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH-(e.g., $C_{1-3}$ alkyl-NH-, such as $CH_3NH$-, $CH_3CH_2NH$- or $CH_3CH_2CH_2NH$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof. In embodiments of the present disclosure, the number of substituents is not theoretically limited in any way or automatically limited by the size of the building units.

In some embodiments of the compound of Formula (I-1), $X_1$ represents:

cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, bornylene, bicyclo[2.2.1]heptanylene, 2-oxobicyclo[2.2.1]heptanylene or bicyclo[2.2.1]heptenylene, with each group being optionally substituted with one or more (e.g., 1-10, 1-9, 1-8, 1-7, 1-6, 1-5, 1-4, 1-3 or 1-2, or 1) substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof;

phenylene or naphthylene, wherein the phenylene or naphthylene is optionally substituted with one or more (e.g., 1-10, 1-9, 1-8, 1-7, 1-6, 1-5, 1-4, 1-3 or 1-2, or 1) substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof;

azetidinylene, pyrrolidinylene, imidazolidylene, pyrazolidylene, oxazolidinylene, thiazolidinylene, piperidinylene, piperazinylene, morpholinylene, thiomorpholinylene, azepanylene, azacyclooctylene, diazacycloheptanylene, diazacyclooctylene, 3-azabicyclo[3.1.0]hexylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octylene, 3,8-diazabicyclo[3.2.1]octylene, 2,5-diazabicyclo[2.2.2]octylene, 3-azaspiro[5.5]undecylene or 7-azaspiro[3.5]nonylene, with each group being optionally substituted with one or more (e.g., 1-10, 1-9, 1-8, 1-7, 1-6, 1-5, 1-4, 1-3 or 1-2, or 1) substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof; or

furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene and imidazo[2,1-b]thiazolylene, with each group being optionally substituted with one or more (e.g., 1-10, 1-9, 1-8, 1-7, 1-6, 1-5, 1-4, 1-3 or 1-2, or 1) substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof. In embodiments of the present disclosure, the number of substituents is not theoretically limited in any way or automatically limited by the size of the building units.

In some embodiments of the compound of Formula (I-1), $R_{a1}$ represents $NR_{d4}R_{d5}$, $C(O)NR_{d6}R_{d7}$, $NHC(O)R_{d8}$, $NHC(O)NR_{d9}R_{d10}$, $C(O)OR_{d11}$, $OC(O)R_{d12}$, $OR_{d13}$, $SR_{d14}$, $C(O)R_{d15}$, $C(S)R_{d16}$, $S(O)R_{d17}$, $S(O)_2R_{d18}$, $S(O)_2NHR_{d19}$, $NHS(O)_2R_{d20}$, $S(O)_2OR_{d21}$, or $OS(O)_2R_{d22}$,

wherein $R_{d4}$, $R_{d5}$, $R_{d6}$, $R_{d7}$, $R_{d9}$, $R_{d10}$, $R_{d11}$, $R_{d13}$, $R_{d14}$, $R_{d19}$ and $R_{d21}$ are the same or different and each independently represent:

H, or

methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl or decyl, with each group being optionally substituted with one or more (e.g., 1-10, 1-9, 1-8, 1-7, 1-6, 1-5, 1-4, 1-3 or 1-2, or 1) substituents selected from the group consisting of D, optionally deuterated $C_{1-4}$ alkyl, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, or bicyclo[2.2.1]heptenyl, with each group being optionally substituted with one or more (e.g., 1-10, 1-9, 1-8, 1-7, 1-6, 1-5, 1-4, 1-3 or 1-2, or 1) substituents selected from the group consisting of D, optionally deuterated $C_{1-4}$ alkyl, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4-to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

phenyl or naphthyl, with each group being optionally substituted with one or more (e.g., 1-10, 1-9, 1-8, 1-7, 1-6, 1-5, 1-4, 1-3 or 1-2, or 1) substituents selected from the group consisting of optionally deuterated $C_{1-4}$ alkyl, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo [3.2.1]octyl, 3,8-diazabicyclo[3.2.1]octyl, 2,5-diazabicyclo[2.2.2]octyl, or azaspirocycloalkyl (e.g., 3-azaspiro [5.5]undecyl or 7-azaspiro[3.5]nonyl), with each group being optionally substituted with one or more (e.g., 1-10, 1-9, 1-8, 1-7, 1-6, 1-5, 1-4, 1-3 or 1-2, or 1) substituents selected from the group consisting of D, optionally deuterated $C_{1-4}$ alkyl, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano,

amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof;

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl or imidazo[2,1-b]thiazolyl, with each group being optionally substituted with one or more (e.g., 1-10, 1-9, 1-8, 1-7, 1-6, 1-5, 1-4, 1-3 or 1-2, or 1) substituents selected from the group consisting of D, optionally deuterated $C_{1-4}$ alkyl, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; and

$R_{d8}$, $R_{d12}$, $R_{d15}$, $R_{d16}$, $R_{d17}$, Rais, $R_{d20}$ and $R_{d22}$ each independently represent:

methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl or decyl, with each group being optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, or bicyclo[2.2.1]heptenyl, with each group being optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

phenyl or naphthyl, with each group being optionally substituted with one or more (e.g., 1-10, 1-9, 1-8, 1-7, 1-6, 1-5, 1-4, 1-3 or 1-2, or 1) substituents selected from the group consisting of halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, or azaspirocycloalkyl (e.g., 3-azaspiro[5.5]undecyl or 7-azaspiro[3.5]nonyl), with each group being optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof;
furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl or imidazo[2,1-b]thiazolyl, with each group being optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof;

In some embodiments of the compound of Formula (I-1), $R_{a1}$ represents:

methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl or decyl, with each group being optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4-to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or
cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, or bicyclo[2.2.1]heptenyl, with each group being optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or
phenyl or naphthyl, with each group being optionally substituted with one or more (e.g., 1-5, 1-4, 1-3 or 1-2, or 1)

substituents selected from the group consisting of halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, or azaspirocycloalkyl (e.g., 3-azaspiro[5.5]undecyl or 7-azaspiro[3.5]nonyl), with each group being optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4-to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl or imidazo[2,1-b]thiazolyl, with each group being optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more (e.g., 1-10, 1-9, 1-8, 1-7, 1-6, 1-5, 1-4, 1-3 or 1-2, or 1) substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof. In embodiments of the present disclosure, the number of substituents is not theoretically limited in any way or automatically limited by the size of the building units.

In some embodiments of the compound of Formula (I-1), $X_1$ represents:

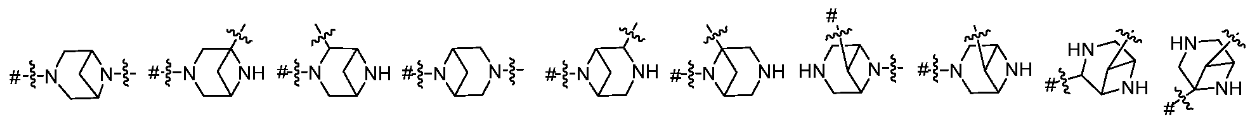

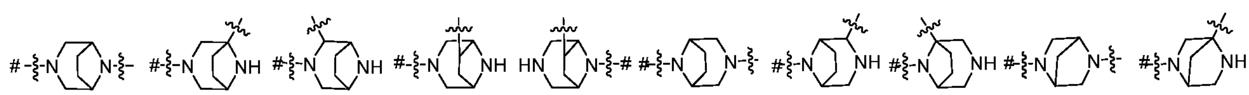

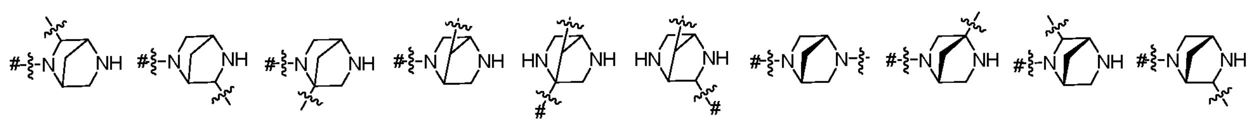

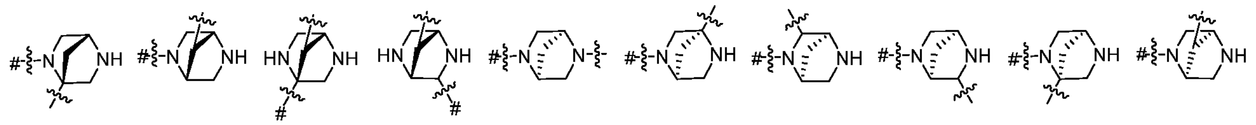

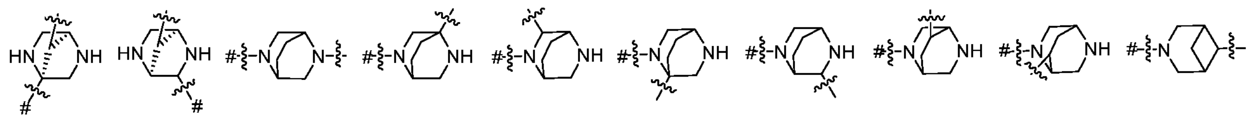

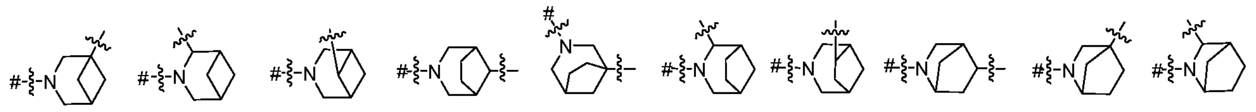

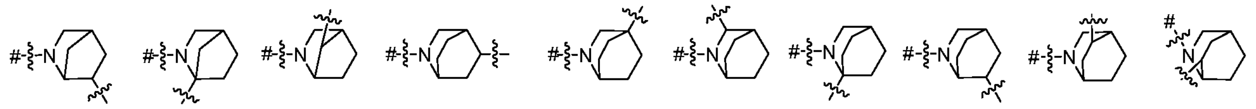

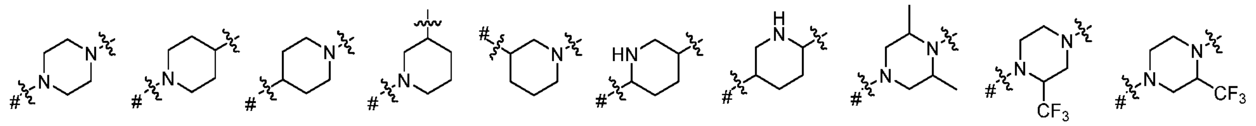

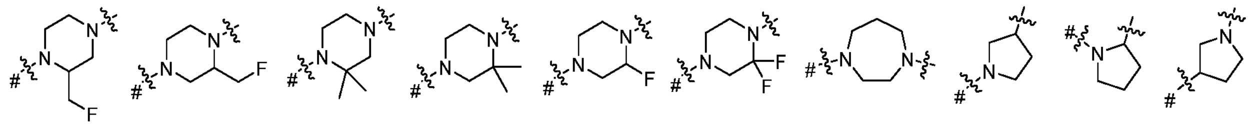

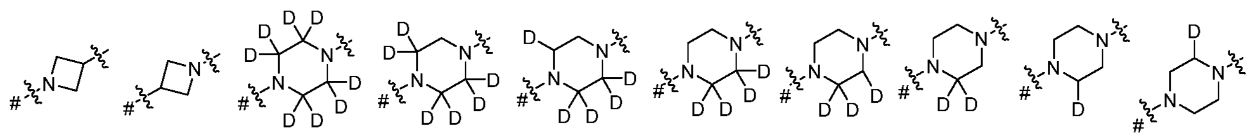

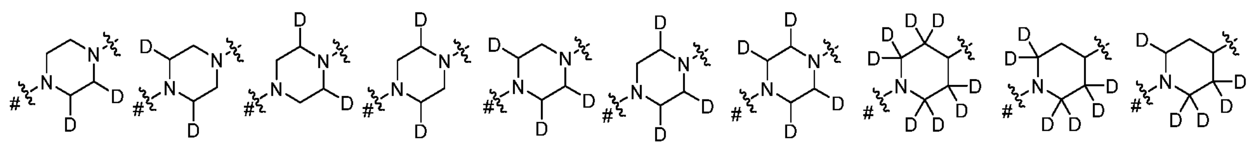

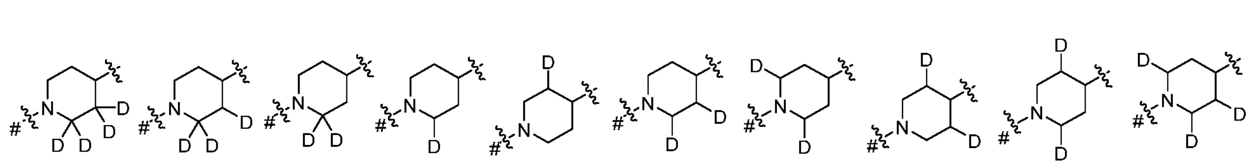

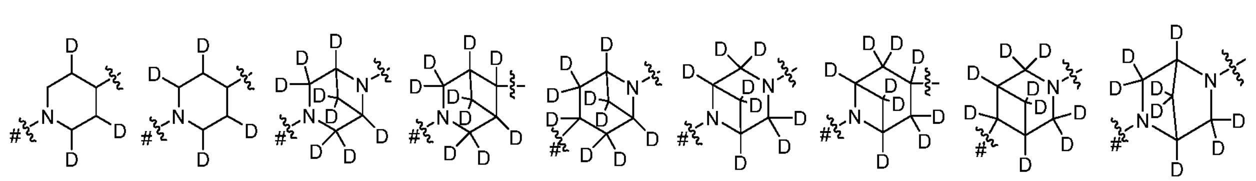

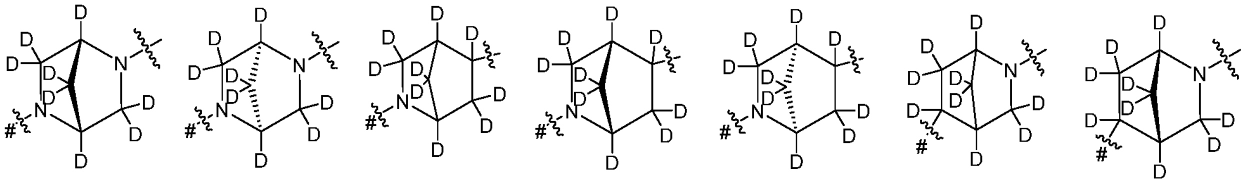

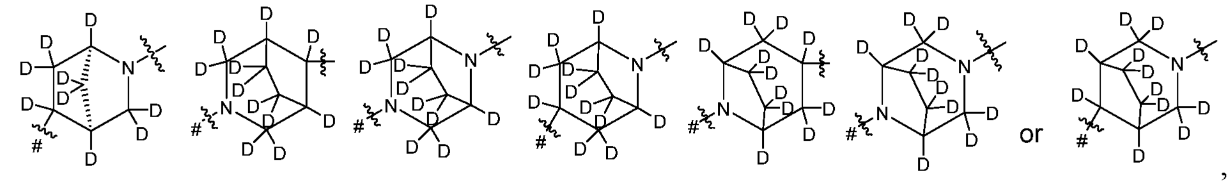

or ,

wherein symbol # indicates the point of attachment to $L_1$.

In some embodiments of the compound of Formula (I-1), $R_{a1}$ represents hydroxy.

In some embodiments of the compound of Formula (I-1), $R_{a1}$ represents:

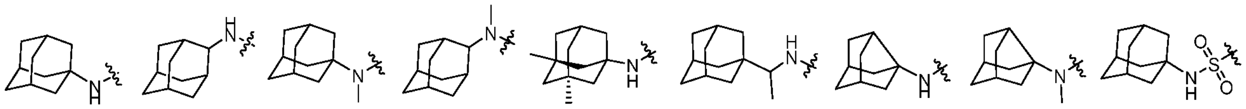

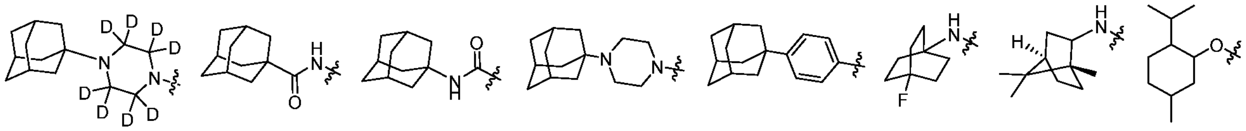

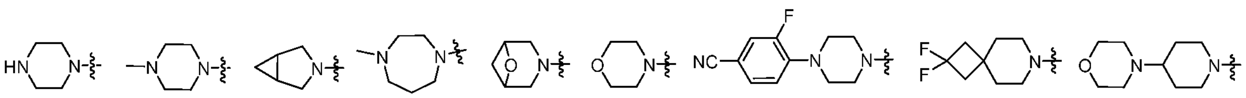

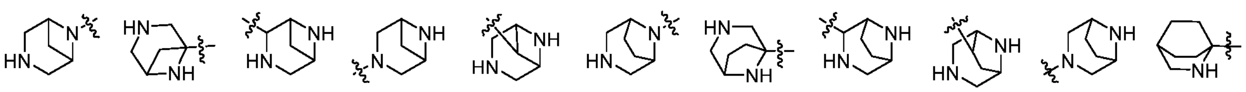

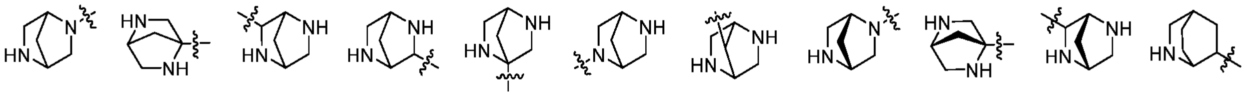

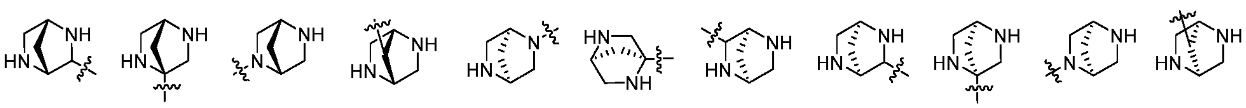

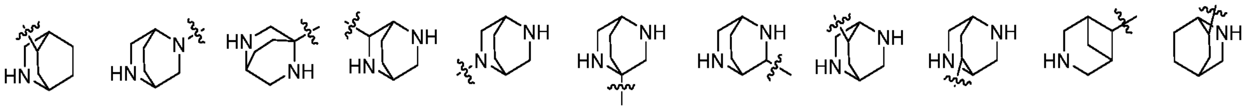

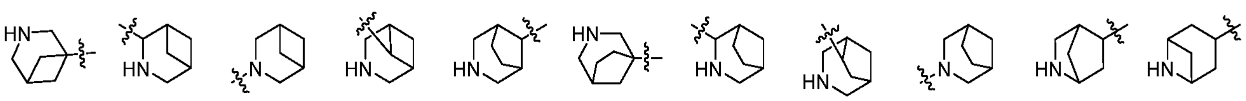

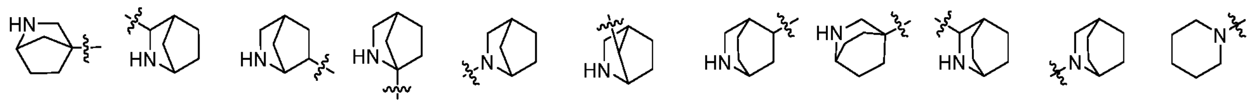

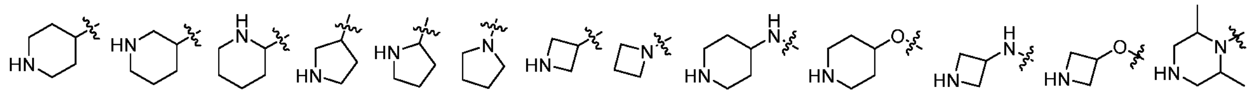

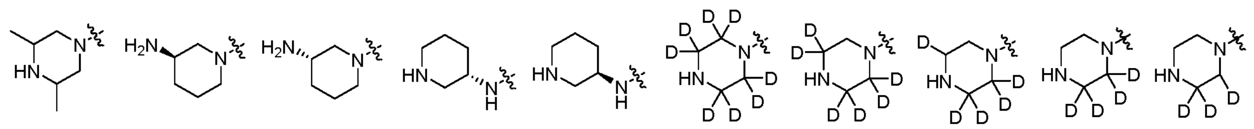

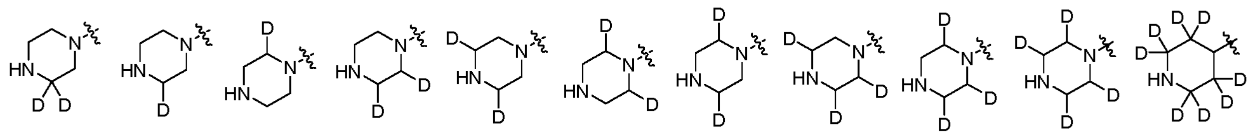

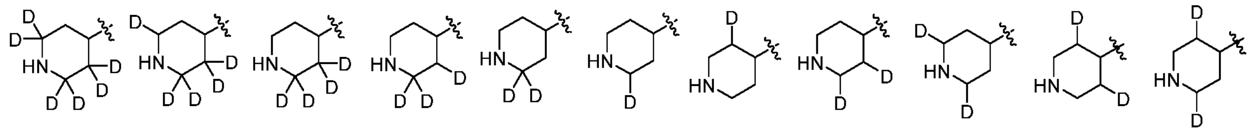

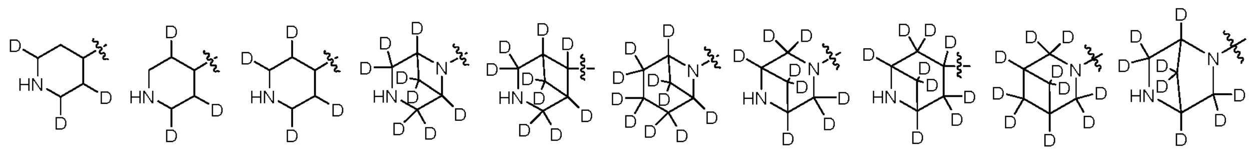

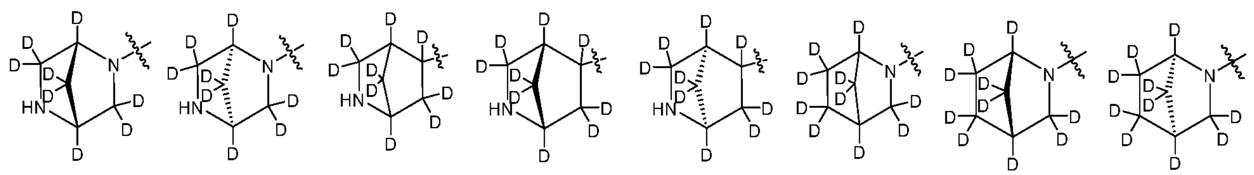

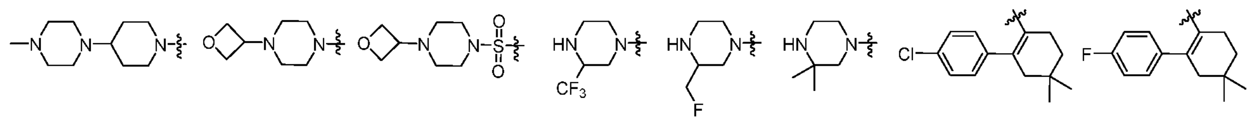

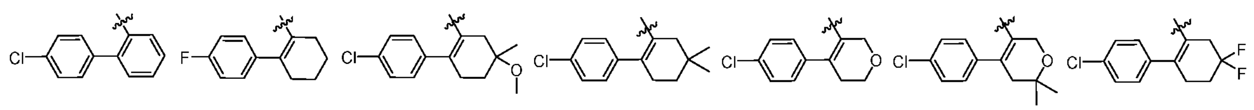

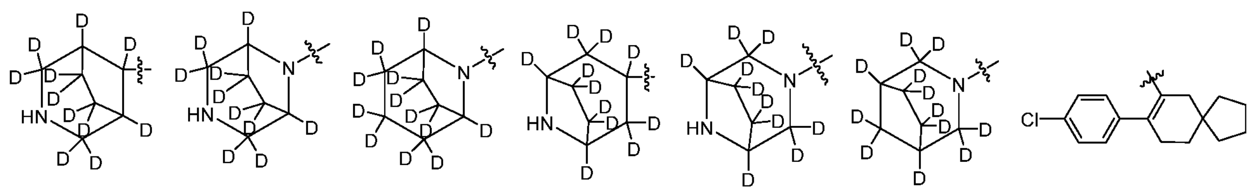

or

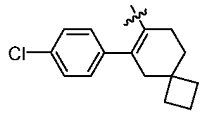

In some embodiments of the compound of Formula (I-1), $L_1$ represents the structure of the following formula:

##-$(C(R^{a1})(R^{a2}))_{m1}$-$(R_{d1}(C(R^{a3})(R^{a4}))_{m2})_{p1}$-;

##-$(C(R^{a1})(R^{a2}))_{m1}$-$(R_{d1}(C(R^{a3})(R^{a4}))_{m2})_{p1}$-$(R_{d1}(C(R^{a5})(R^{a6}))_{m3})_{p2}$-;

##-$(C(R^{a1})(R^{a2}))_{m1}$-$(R_{d1}(C(R^{a3})(R^{a4}))_{m2})_{p1}$-$(R_{d1}(C(R^{a5})(R^{a6}))_{m3})_{p2}$-$(R_{d1}(C(R^{a7})(R^{a8}))_{m4})_{p3}$-;

##-$(C(R^{a1})(R^{a2}))_{m1}$-$(R_{d2}(C(R^{a3})(R^{a4}))_{m2})_{p1}$-;

##-$(C(R^{a1})(R^{a2}))_{m1}$-$(R_{d2}(C(R^{a3})(R^{a4}))_{m2})_{p1}$-$(R_{d2}(C(R^{a5})(R^{a6}))_{m3})_{p2}$-;

##-$(C(R^{a1})(R^{a2}))_{m1}$-$(R_{d2}(C(R^{a3})(R^{a4}))_{m2})_{p1}$-$(R_{d2}(C(R^{a5})(R^{a6})_{m3})_{p2}(R_{d2}(C(R^{a7})(R^{a8})_{m4})_{p3}$-;

##-$(C(R^{a1})(R^{a2}))_{m1}$-$(R_{d1}$-$R_{d2}$-$(C(R^{a3})(R^{a4}))_{m2})_{p1}$-;

##-$(C(R^{a1})(R^{a2}))_{m1}$-$(R_{d1}(C(R^{a3})(R^{a4}))_{m2})_{p1}$-$(R_{d2}(C(R^{a5})(R^{a6}))_{m3})_{p2}$-;

##-$(C(R^{a1})(R^{a2}))_{m1}$-$(R_{d2}$-$R_{d1}$-$(C(R^{a3})(R^{a4}))_{m2})_{p1}$-; or

##-$(C(R^{a1})(R^{a2}))_{m1}$-$(R_{d2}(C(R^{a3})(R^{a4}))_{m2})_{p1}$-$(R_{d1}(C(R^{a5})(R^{a6}))_{m3})_{p2}$-;

wherein each group $R_{d1}$ is independently selected from the group consisting of O, S, $N(R_{d3})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_{d3}$ independently represents H or $C_{1-3}$ alkyl; each group $R_{d2}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, optionally deuterated $C_{1-6}$ alkoxy (e.g., $C_{1-3}$ alkoxy, such as methoxy, ethoxy, or propoxy), optionally deuterated $C_{1-6}$ alkyl-NH- (e.g., $C_{1-3}$ alkyl-NH-, such as $CH_3NH$-, $CH_3CH_2NH$- or $CH_3CH_2CH_2NH$-), halogenated $C_{1-6}$ alkyl halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH-(e.g., $C_{1-3}$ alkyl-C(O)NH-, such as $CH_3$-C(O)NH-, $CH_3CH_2$-C(O)NH-, and $CH_3CH_2CH_2$-C(O)NH-) or any combination thereof;

$R^{a1}$, $R^{a2}$, $R^{a3}$, $R^{a4}$, $R^{a5}$, $R^{a6}$, $R^{a7}$ and $R^{a8}$ each independently represent H, deuterium, hydroxy, amino, mercapto, nitro, halogen, cyano, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), optionally deuterated $C_{1-6}$ alkoxy (e.g., $C_{1-3}$ alkoxy, such as methoxy, ethoxy, or propoxy), optionally deuterated $C_{1-6}$ alkyl-NH- (e.g., $C_{1-3}$ alkyl-NH-, such as $CH_3NH$-, $CH_3CH_2NH$- or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-) or any combination thereof;

symbol ## indicates the point of attachment to $X_1$; and

m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-1), $L_1$ represents the structure of the following formula:

$##-(C(R^{a1})(R^{a2}))_{m1}-(O(C(R^{a3})(R^{a4}))_{m2})_{p1}-$;

$##-(C(R^{a1})(R^{a2}))_{m1}-(O(C(R^{a3})(R^{a4}))_{m2})_{p1}-(O(C(R^{a5})(R^{a6}))_{m3})_{p2}-$;

$##-(C(R^{a1})(R^{a2}))_{m1}-(O(C(R^{a3})(R^{a4}))_{m2})_{p1}-(O(C(R^{a5})(R^{a6}))_{m3})_{p2}-(O(C(R^{a7})(R^{a8}))_{m4})_{p3}-$; $##-(C(R^{a1})(R^{a2}))_{m1}-(N(R_{g9})(C(R^{a3})(R^{a4}))_{m2})_{p1}-$;

$##-(C(R^{a1})(R^{a2}))_{m1}-(N(R_{g9})(C(R^{a3})(R^{a4}))_{m2})_{p1}-(N(R_{g9})(C(R^{a5})(R^{a6}))_{m3})_{p2}-$;

$##-(C(R^{a1})(R^{a2}))_{m1}-(N(R_{g9})(C(R^{a3})(R^{a4}))_{m2})_{p1}-(N(R_{g9})(C(R^{a5})(R^{a6}))_{m3})_{p2}-(N(R_{g9})(C(R^{a7})(R^{a8}))_{m4})_{p3}-$;

$##-(C(R^{a1})(R^{a2}))_{m1}-(C(O)NH-(C(R^{a3})(R^{a4}))_{m2})_{p1}-$;

$##-(C(R^{a1})(R^{a2}))_{m1}-(C(O)NH-(C(R^{a3})(R^{a4}))_{m2})_{p1}-(C(O)NH-(C(R^{a5})(R^{a6}))_{m3})_{p2}-$;

$##-(C(R^{a1})(R^{a2}))_{m1}-(C(O)NH-(C(R^{a3})(R^{a4}))_{m2})_{p1}-(C(O)NH-(C(R^{a5})(R^{a6}))_{m3})_{p2}-(C(O)NH-)(C(R^{a7})(R^{a8}))_{m4})_{p3}-$;

$##-(C(R^{a1})(R^{a2}))_{m1}-(C(O)NH-(C(R^{a3})(R^{a4}))_{m2})_{p1}-(O-(C(R^{a5})(R^{a6}))_{m3})_{p2}-$;

$##-(C(R^{a1})(R^{a2}))_{m1}-(C(O)NH-(C(R^{a3})(R^{a4}))_{m2})-(O-(C(R^{a5})(R^{a6}))_{m3})_{p1}-$;

$##-(C(R^{a1})(R^{a2}))_{m1}-(NHC(O)-(C(R^{a3})(R^{a4}))_{m2})_{p1}-$;

$##-(C(R^{a1})(R^{a2}))_{m1}-(NHC(O)-(C(R^{a3})(R^{a4}))_{m2})_{p1}-(O-(C(R^{a5})(R^{a6}))_{m3})_{p2}-$;

$##-(C(R^{a1})(R^{a2}))_{m1}-(NHC(O)-(C(R^{a3})(R^{a4}))_{m2})_{p1}-(O)-(C(R^{a5})(R^{a6}))_{m3})_{p2}-(O)(C(R^{a7})(R^{a8}))_{m4})_{p3}-$;

$##-(C(R^{a1})(R^{a2}))_{m1}-NHC(O)-(C(R^{a3})(R^{a4}))_{m2})_{p1}-(O-(C(R^{a5})(R^{a6}))_{m3})_{p1}-$;

$##-(C(R^{a1})(R^{a2}))_{m1}-NHC(O)NH-(C(R^{a3})(R^{a4}))_{m2}-$;

$##-(C(R^{a1})(R^{a2}))_{m1}-C(O)-(C(R^{a3})(R^{a4}))_{m2}-$;

$##-(C(R^{a1})(R^{a2}))_{m1}-C(S)-(C(R^{a3})(R^{a4}))_{m2}-$;

$##-(C(R^{a1})(R^{a2}))_{m1}-S-(C(R^{a3})(R^{a4}))_{m2}-$;

$##-(C(R^{a1})(R^{a2}))_{m1}-C_{6-10}$ arylene-$(C(R^{a3})(R^{a4}))_{m2})_{p1}-$;

$##-(C(R^{a1})(R^{a2}))_{m1}-C_{6-10}$ arylene-$(C(R^{a3})(R^{a4}))_{m2})_{p1}-C_{6-10}$ arylene-$(C(R^{a5})(R^{a6}))_{m3}-$;

$##-(C(R^{a1})(R^{a2}))_{m1}-$(4- to 15-membered heterocyclylene-$(C(R^{a3})(R^{a4}))_{m2})_{p1}-$;

$##-(C(R^{a1})(R^{a2}))_{m1}-$(4- to 15-membered heterocyclylene-$(C(R^{a3})(R^{aa}))_{m2})_{p1}-$(4- to 15-membered heterocyclylene-$(C(R^{a5})(R^{a6}))_{m3})_{p2}-$;

$###-(C(R^{a1})(R^{a2}))_{m1}-$(5- to 15-membered heteroarylene-$(C(R^{a3})(R^{a4}))_{m2})_{p1}-$;

$##-(C(R^{a1})(R^{a2}))_{m1}-$(5- to 15-membered heteroarylene-$(C(R^{a3})(R^{a4}))_{m2})_{p1}-$(5- to 15-membered heteroarylene-$(C(R^{a5})(R^{a6}))_{m3})_{P2}-$;

##-$(C(R^{a1})(R^{a2}))_{m1}$-($C_{3-15}$cycloalkylene-$(C(R^{a3})(R^{a4}))_{m2})_{p1}$-; or

##-$(C(R^{a1})(R^{a2}))_{m1}$-($C_{3-15}$cycloalkylene-$(C(R^{a3})(R^{a4}))_{m2})_{p1}$-($C_{3-15}$cycloalkylene-$(C(R^{a5})(R^{a6}))_{m3})_{p2}$-;

wherein each $R_{g9}$ independently represents H or $C_{1-3}$ alkyl;
the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-6}$ alkyl, optionally deuterated $C_{3-6}$cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano or any combination thereof;
$R^{a1}$, $R^{a2}$, $R^{a3}$, $R^{a4}$, $R^{a5}$, $R^{a6}$, $R^{a7}$ and $R^{a8}$ each independently represent H, deuterium, hydroxy, amino, mercapto, nitro, halogen, cyano, optionally deuterated $C_{1-6}$ alkyl, halogenated $C_{1-6}$ alkyl, optionally deuterated $C_{3-6}$cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof;
symbol ## indicates the point of attachment to $X_1$; and
m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-1), $L_1$ represents the following group:
##-$CH_2$-O-$CH_2$-, ##-$CH_2$-O-$(CH_2)_2$-, ##-$(CH_2)_1$-O-$(CH_2)_3$-, ##-$(CH_2)_1$-O-$(CH_2)_4$-, ##-$(CH_2)_1$-O-$(CH_2)_5$-, ##-$(CH_2)_1$-O-$(CH_2)_6$-, ##-$(CH_2)_1$-O-$(CH_2)_7$-, ##-$(CH_2)_1$-O-$(CH_2)_8$-, ##-$(CH_2)_1$-O-$(CH_2)_9$-, ##-$(CH_2)_1$-O-$(CH_2)_{10}$-, ##-$(CH_2)_2$-O-$(CH_2)_1$-, ##-$(CH_2)_2$-O-$(CH_2)_2$-, ##-$(CH_2)_2$-O-$(CH_2)_3$-, ##-$(CH_2)_2$-O-$(CH_2)_4$-, ##-$(CH_2)_2$-O-$(CH_2)_5$-, ##-$(CH_2)_2$-O-$(CH_2)_6$-, ##-$(CH_2)_2$-O-$(CH_2)_7$-, ##-$(CH_2)_2$-O-$(CH_2)_8$-, ##-$(CH_2)_2$-O-$(CH_2)_9$-, ##-$(CH_2)_2$O-$(CH_2)_{10}$-, ##-$(CH_2)_2$-O-$(CH_2)_{11}$-, ##-$(CH_2)_2$-O-$(CH_2)_{12}$-, ##-$(CH_2)_3$-O-$(CH_2)_1$-, ##-$(CH_2)_3$-O-$(CH_2)_2$-, ##-$(CH_2)_3$-O-$(CH_2)_3$-, ##-$(CH_2)_3$-O-$(CH_2)_4$-, ##-$(CH_2)_3$-O-$(CH_2)_5$-, ##-$(CH_2)_3$-O-$(CH_2)_6$-, ##-$(CH_2)_3$-O-$(CH_2)_7$-, ##-$(CH_2)_4$-O-$(CH_2)_1$-, ##-$(CH_2)_4$-O-$(CH_2)_2$-, ##-$(CH_2)_4$-O-$(CH_2)_3$-, ##-$(CH_2)_4$-O-$(CH_2)_4$-, ##-$(CH_2)_4$-O-$(CH_2)_5$-, ##-$(CH_2)_4$-O-$(CH_2)_6$-, ##-$(CH_2)_5$-O-$(CH_2)_1$-, ##-$(CH_2)_5$-O-$(CH_2)_2$-, ##-$(CH_2)_5$-O-$(CH_2)_3$-, ##-$(CH_2)_5$-O-$(CH_2)_4$-, ##-$(CH_2)_5$-O-$(CH_2)_5$-, ##-$(CH_2)_6$-O-$(CH_2)_1$-, ##-$(CH_2)_6$-O-$(CH_2)_2$-, ##-$(CH_2)_6$-O-$(CH_2)_3$-, ##-$(CH_2)_6$-O-$(CH_2)_4$-, ##-$(CH_2)_7$-O-$(CH_2)_1$-, ##-$(CH_2)_7$-O-$(CH_2)_2$-, ##-$(CH_2)_7$-O-$(CH_2)_3$-, ##-$(CH_2)_8$-O-$(CH_2)_1$-, ##-$(CH_2)_8$-O-$(CH_2)_2$-, ##-$CH(CH_3)$-O-$(CH_2)_1$-, ##-$CH(CH_3)$-O-$(CH_2)_2$-, ##-$CH(CH_3)$-O-$(CH_2)_3$-, ##-$CH(CH_3)$-O-$(CH_2)_4$-, ##-$CH(CH_3)$-O-$(CH_2)_5$-, ##-$CH(CH_3)$-O-$(CH_2)_6$-, ##-$CH(CH_3)$-O-$(CH_2)_7$-, ##-$CH(CH_3)$-O-$(CH_2)_8$-, ##-$CH(CH_3)$-O-$(CH_2)_9$-, ##-$CH(CH_3)$-O-$(CH_2)_{10}$-, ##-$CH_2$-$(O(CH_2)_2)_2$-, ##-$CH_2$-$(O(CH_2)_2)_3$-, ##-$CH_2$-$(O(CH_2)_2)_4$-, ##-$CH_2$-$(O(CH_2)_2)_5$-, ##-$CH_2$-$(O(CH_2)_2)_6$-, ##-$CH_2$-$(O(CH_2)_2)_7$-, ##-$CH_2$-$(O(CH_2)_2)_8$-, ##-$CH_2$-$(O(CH_2)_2)_9$-, ##-$CH_2$-$(O(CH_2)_2)_{10}$-, ##-$CH_2$-$(O(CH_2)_2)_1$-$OCH_2$-, ##-$CH_2$-$(O(CH_2)_2)_2$-$OCH_2$-, ##-$CH_2$-$(O(CH_2)_2)_3$-$OCH_2$-, ##-$CH_2$-$(O(CH_2)_2)_4$-$OCH_2$-, ##-$CH_2$-$(O(CH_2)_2)_5$-$OCH_2$-, ##-$CH_2$-$(O(CH_2)_2)_6$-$OCH_2$-, ##-$CH_2$-$(O(CH_2)_2)_7$-$OCH_2$-, ##-$CH_2$-$(O(CH_2)_2)_8$-$OCH_2$-, ##-$CH_2$-$(O(CH_2)_2)_9$-$OCH_2$-, ##-$CH_2$-$(O(CH_2)_2)_{10}$-$OCH_2$-, ##-$(CH_2)_2$-$(O(CH_2)_2)_2$-, ##-$(CH_2)_2$-$(O(CH_2)_2)_3$-, ##-$(CH_2)_2$-$(O(CH_2)_2)_4$-, ##-$(CH_2)_2$-$(O(CH_2)_2)_5$-, ##-$(CH_2)_2$-$(O(CH_2)_2)_6$-, ##-$(CH_2)_2$-$(O(CH_2)_2)_7$-, ##-$(CH_2)_2$-$(O(CH_2)_2)_8$-, ##-$(CH_2)_2$-$(O(CH_2)_2)_9$-, ##-$(CH_2)_2$-$(O(CH_2)_2)_{10}$-, ##-$(CH_2)_3$-$(O(CH_2)_2)_2$-, ##-$(CH_2)_3$-$(O(CH_2)_2)_3$-, ##-$(CH_2)_3$-$(O(CH_2)_2)_4$-, ##-$(CH_2)_3$-$(O(CH_2)_2)_5$-, ##-$(CH_2)_3$-$(O(CH_2)_2)_6$-, ##-$(CH_2)_3$-$(O(CH_2)_2)_7$-, ##-$(CH_2)_3$-$(O(CH_2)_2)_8$-, ##-$(CH_2)_3$-$(O(CH_2)_2)_9$-, ##-$(CH_2)_3$-$(O(CH_2)_2)_{10}$-, ##-$(CH_2)_4$-$(O(CH_2)_2)_2$-, ##-$(CH_2)_4$-$(O(CH_2)_2)_3$-, ##-$(CH_2)_4$-$(O(CH_2)_2)_4$-, ##-$(CH_2)_4$-$(O(CH_2)_2)_5$-, ##-$(CH_2)_4$-$(O(CH_2)_2)_6$-, ##-$(CH_2)_4$-$(O(CH_2)_2)_7$-, ##-$(CH_2)_4$-$(O(CH_2)_2)_8$-, ##-$(CH_2)_4$-$(O(CH_2)_2)_9$-, ##-$(CH_2)_4$-$(O(CH_2)_2)_{10}$-, ##-$CH_2$-$(O(CH_2)_3)_2$-, ##-$CH_2$-$(O(CH_2)_3)_3$-, ##-$CH_2$-$(O(CH_2)_3)_4$-, ##-$CH_2$-$(O(CH_2)_3)_5$-, ##-$CH_2$-$(O(CH_2)_3)_6$-, ##-$CH_2$-$(O(CH_2)_3)_7$-, ##-$CH_2$-$(O(CH_2)_3)_8$-, ##-$CH_2$-$(O(CH_2)_3)_9$-, ##-$CH_2$-$(O(CH_2)_3)_{10}$-, ##-$(CH_2)_2$-$(O(CH_2)_3)_2$-, ##-$(CH_2)_2$-$(O(CH_2)_3)_3$-, ##-$(CH_2)_2$-$(O(CH_2)_3)_4$-, ##-$(CH_2)_2$-$(O(CH_2)_3)_5$-, ##-$(CH_2)_2$-$(O(CH_2)_3)_6$-, ##-$(CH_2)_2$-$(O(CH_2)_3)_7$-, ##-$(CH_2)_2$-$(O(CH_2)_3)_8$-, ##-$(CH_2)_2$-$(O(CH_2)_3)_9$-, ##-$(CH_2)_2$-$(O(CH_2)_3)_{10}$-, ##-$(CH_2)_3$-$(O(CH_2)_3)_2$-, ##-$(CH_2)_3$-$(O(CH_2)_3)_3$-, ##-$(CH_2)_3$-$(O(CH_2)_3)_4$-, ##-$(CH_2)_3$-$(O(CH_2)_3)_5$-, ##-$(CH_2)_3$-$(O(CH_2)_3)_6$-, ##-$(CH_2)_3$-$(O(CH_2)_3)_7$-, ##-$(CH_2)_3$-$(O(CH_2)_3)_8$-, ##-$(CH_2)_3$-$(O(CH_2)_3)_9$-, ##-$(CH_2)_3$-$(O(CH_2)_3)_{10}$-, ##-$CH_2$-O-$(CH_2)_2$-O-$(CH_2)_3$-, ##-$CH_2$-$(O(CH_2)_2)_2$-$(O(CH_2)_3)_2$-, ##-$CH_2$-$(O(CH_2)_2)_3$-$(O(CH_2)_3)_3$-, ##-$CH_2$-$(O(CH_2)_2)_4$-$(O(CH_2)_3)_4$-, ##-$CH_2$-$(O(CH_2)_2)_5$-$(O(CH_2)_3)_5$-, ##-$CH_2$-$(O(CH_2)_2)_6$-$(O(CH_2)_3)_6$-, ##-$(CH_2)_2$-O-$(CH_2)_2$-O-$(CH_2)_3$-, ##-$(CH_2)_2$-$(O(CH_2)_2)_2$-$(O(CH_2)_3)_2$-, ##-$(CH_2)_2$-$(O(CH_2)_2)_3$-$(O(CH_2)_3)_3$-, ##-$(CH_2)_2$-$(O(CH_2)_2)_4$-$(O(CH_2)_3)_4$-, ##-$(CH_2)_2$-$(O(CH_2)_2)_5$-$(O(CH_2)_3)_5$-, ##-$(CH_2)_2$-$(O(CH_2)_2)_6$-$(O(CH_2)_3)_6$-, ##-$(CH_2)_3$-O-$(CH_2)_2$-O-$(CH_2)_3$-, ##-$(CH_2)_3$-$(O(CH_2)_2)_2$-$(O(CH_2)_3)_2$-, ##-$(CH_2)_3$-$(O(CH_2)_2)_3$-$(O(CH_2)_3)_3$-, ##-$(CH_2)_3$-$(O(CH_2)_2)_4$-$(O(CH_2)_3)_4$-, ##-$(CH_2)_3$-$(O(CH_2)_2)_5$-$(O(CH_2)_3)_5$-, ##-$(CH_2)_3$-$(O(CH_2)_2)_6$-$(O(CH_2)_3)_6$-, ##-$CH_2$-O-$(CH_2)_3$-O-$(CH_2)_2$-, ##-$CH_2$-$(O(CH_2)_3)_2$-$(O(CH_2)_2)_2$-, ##-$CH_2$-$(O(CH_2)_3)_3$-$(O(CH_2)_2)_3$-, ##-$CH_2$-$(O(CH_2)_3)_4$-$(O(CH_2)_2)_4$-, ##-$CH_2$-$(O(CH_2)_3)_5$-$(O(CH_2)_2)_5$-, ##-$CH_2$-$(O(CH_2)_3)_6$-$(O(CH_2)_2)_6$-, ##-$(CH_2)_2$-O-$(CH_2)_3$-O-$(CH_2)_2$-, ##-$(CH_2)_2$-$(O(CH_2)_3)_2$-$(O(CH_2)_2)_2$-,

##-$(CH_2)_2-(O(CH_2)_3)_3-(O(CH_2)_2)_3$-, ##-$(CH_2)_2-(O(CH_2)_3)_4-(O(CH_2)_2)_4$-, ##-$(CH_2)_2-(O(CH_2)_3)_5-(O(CH_2)_2)_5$-, ##-$(CH_2)_2-(O(CH_2)_3)_6-(O(CH_2)_2)_6$-, ##-$(CH_2)_3-O-(CH_2)_3-O-(CH_2)_2$-, ##-$(CH_2)_3-(O(CH_2)_3)_2-(O(CH_2)_2)_2$-, ##-$(CH_2)_3-(O(CH_2)_3)_3-(O(CH_2)_2)_3$-, ##-$(CH_2)_3-(O(CH_2)_3)_4-(O(CH_2)_2)_4$-, ##-$(CH_2)_3-(O(CH_2)_3)_5-(O(CH_2)_2)_5$-, ##-$(CH_2)_3-(O(CH_2)_3)_6-(O(CH_2)_2)_6$-, ##-$CH_2-O-(CH_2)_2-O-CH_2$-, ##-$(CH_2)_2-O-(CH_2)_2-O-CH_2$-, ##-$(CH_2)_2-(O(CH_2)_2)_2-O-(CH_2)_3$-, ##-$(CH_2)_2-(O(CH_2)_2)_3-O-(CH_2)_3$-, ##-$(CH_2)_2-(O(CH_2)_2)_4-O-(CH_2)_3$-, ##-$(CH_2)_5-(O(CH_2)_2)_2-O-(CH_2)_5$-, ##-$(CH_2)_5-(O(CH_2)_2)_2-O-(CH_2)_6$-, ##-$CH_2-C(O)-CH_2$-, ##-$CH_2-C(O)-(CH_2)_2$-, ##-$(CH_2)_1-C(O)-(CH_2)_3$-, ##-$(CH_2)_1-C(O)-(CH_2)_4$-, ##-$(CH_2)_1-C(O)-(CH_2)_5$-, ##-$(CH_2)_1-C(O)-(CH_2)_6$-, ##-$(CH_2)_1-C(O)-(CH_2)_7$-, ##-$(CH_2)_1-C(O)-(CH_2)_8$-, ##-$(CH_2)_1-C(O)-(CH_2)_9$-, ##-$(CH_2)_1-C(O)-(CH_2)_{10}$-, ##-$(CH_2)_2-C(O)-(CH_2)_1$-, ##-$(CH_2)_2-C(O)-(CH_2)_2$-, ##-$(CH_2)_2-C(O)-(CH_2)_3$-, ##-$(CH_2)_2-C(O)-(CH_2)_4$-, ##-$(CH_2)_2-C(O)-(CH_2)_5$-, ##-$(CH_2)_2-C(O)-(CH_2)_6$-, ##-$(CH_2)_2-C(O)-(CH_2)_7$-, ##-$(CH_2)_2-C(O)-(CH_2)_8$-, ##-$(CH_2)_2-C(O)-(CH_2)_9$-, ##-$(CH_2)_2-C(O)-(CH_2)_{10}$-, ##-$(CH_2)_2-C(O)-(CH_2)_{11}$-, ##-$(CH_2)_2-C(O)-(CH_2)_{12}$-, ##-$(CH_2)_3-C(O)-(CH_2)_1$-, ##-$(CH_2)_3-C(O)-(CH_2)_2$-, ##-$(CH_2)_3-C(O)-(CH_2)_3$-, ##-$(CH_2)_3-C(O)-(CH_2)_4$-, ##-$(CH_2)_3-C(O)-(CH_2)_5$-, ##-$(CH_2)_3-C(O)-(CH_2)_6$-, ##-$(CH_2)_3-C(O)-(CH_2)_7$-, ##-$(CH_2)_4-C(O)-(CH_2)_1$-, ##-$(CH_2)_4-C(O)-(CH_2)_2$-, ##-$(CH_2)_4-C(O)-(CH_2)_3$-, ##-$(CH_2)_4-C(O)-(CH_2)_4$-, ##-$(CH_2)_4-C(O)-(CH_2)_5$-, ##-$(CH_2)_4-C(O)-(CH_2)_6$-, ##-$(CH_2)_5-C(O)-(CH_2)_1$-, ##-$(CH_2)_5-C(O)-(CH_2)_2$-, ##-$(CH_2)_5-C(O)-(CH_2)_3$-, ##-$(CH_2)_5-C(O)-(CH_2)_4$-, ##-$(CH_2)_5-C(O)-(CH_2)_5$-, ##-$(CH_2)_6-C(O)-(CH_2)_1$-, ##-$(CH_2)_6-C(O)-(CH_2)_2$-, ##-$(CH_2)_6-C(O)-(CH_2)_3$-, ##-$(CH_2)_6-C(O)-(CH_2)_4$-, ##-$(CH_2)_7-C(O)-(CH_2)_1$-, ##-$(CH_2)_7-C(O)-(CH_2)_2$-, ##-$(CH_2)_7-C(O)-(CH_2)_3$-, ##-$(CH_2)_8-C(O)-(CH_2)_1$-, ##-$(CH_2)_8-C(O)-(CH_2)_2$-, ##-$CH_2-S-CH_2$-, ##-$CH_2-S-(CH_2)_2$-, ##-$(CH_2)_1-S-(CH_2)_3$-, ##-$(CH_2)_1-S-(CH_2)_4$-, ##-$(CH_2)_1-S-(CH_2)_5$-, ##-$(CH_2)_1-S-(CH_2)_6$-, ##-$(CH_2)_1-S-(CH_2)_7$-, ##-$(CH_2)_1-S-(CH_2)_8$-, ##-$(CH_2)_1-S-(CH_2)_9$-, ##-$(CH_2)_1-S-(CH_2)_{10}$-, ##-$(CH_2)_2-S-(CH_2)_1$-, ##-$(CH_2)_2-S-(CH_2)_2$-, ##-$(CH_2)_2-S-(CH_2)_3$-, ##-$(CH_2)_2-S-(CH_2)_4$-, ##-$(CH_2)_2-S-(CH_2)_5$-, ##-$(CH_2)_2-S-(CH_2)_6$-, ##-$(CH_2)_2-S-(CH_2)_7$-, ##-$(CH_2)_2-S-(CH_2)_8$-, ##-$(CH_2)_2-S-(CH_2)_9$-, ##-$(CH_2)_2-S-(CH_2)_{10}$-, ##-$(CH_2)_2-S-(CH_2)_{11}$-, ##-$(CH_2)_2-S-(CH_2)_{12}$-, ##-$(CH_2)_3-S-(CH_2)_1$-, ##-$(CH_2)_3-S-(CH_2)_2$-, ##-$(CH_2)_3-S-(CH_2)_3$-, ##-$(CH_2)_3-S-(CH_2)_4$-, ##-$(CH_2)_3-S-(CH_2)_5$-, ##-$(CH_2)_3-S-(CH_2)_6$-, ##-$(CH_2)_3-S-(CH_2)_7$-, ##-$(CH_2)_4-S-(CH_2)_1$-, ##-$(CH_2)_4-S-(CH_2)_2$-, ##-$(CH_2)_4-S-(CH_2)_3$-, ##-$(CH_2)_4-S-(CH_2)_4$-, ##-$(CH_2)_4-S-(CH_2)_5$-, ##-$(CH_2)_4-S-(CH_2)_6$-, ##-$(CH_2)_5-S-(CH_2)_1$-, ##-$(CH_2)_5-S-(CH_2)_2$-, ##-$(CH_2)_5-S-(CH_2)_3$-, ##-$(CH_2)_5-S-(CH_2)_4$-, ##-$(CH_2)_5-S-(CH_2)_5$-, ##-$(CH_2)_6-S-(CH_2)_1$-, ##-$(CH_2)_6-S-(CH_2)_2$-, ##-$(CH_2)_6-S-(CH_2)_3$-, ##-$(CH_2)_6-S-(CH_2)_4$-, ##-$(CH_2)_7-S-(CH_2)_1$-, ##-$(CH_2)_7-S-(CH_2)_2$-, ##-$(CH_2)_7-S-(CH_2)_3$-, ##-$(CH_2)_8-S-(CH_2)_1$-, ##-$(CH_2)_8-S-(CH_2)_2$-, ##-$CH_2-C(S)-CH_2$-, ##-$CH_2-C(S)-(CH_2)_2$-, ##-$(CH_2)_1-C(S)-(CH_2)_3$-, ##-$(CH_2)_1-C(S)-(CH_2)_4$-, ##-$(CH_2)_1-C(S)-(CH_2)_5$-, ##-$(CH_2)_1-C(S)-(CH_2)_6$-, ##-$(CH_2)_1-C(S)-(CH_2)_7$-, ##-$(CH_2)_1-C(S)-(CH_2)_8$-, ##-$(CH_2)_1-C(S)-(CH_2)_9$-, ##-$(CH_2)_1-C(S)-(CH_2)_{10}$-, ##-$(CH_2)_2-C(S)-(CH_2)_1$-, ##-$(CH_2)_2-C(S)-(CH_2)_2$-, ##-$(CH_2)_2-C(S)-(CH_2)_3$-, ##-$(CH_2)_2-C(S)-(CH_2)_4$-, ##-$(CH_2)_2-C(S)-(CH_2)_5$-, ##-$(CH_2)_2-C(S)-(CH_2)_6$-, ##-$(CH_2)_2-C(S)-(CH_2)_7$-, ##-$(CH_2)_2-C(S)-(CH_2)_8$-, ##-$(CH_2)_2-C(S)-(CH_2)_9$-, ##-$(CH_2)_2-C(S)-(CH_2)_{10}$-, ##-$(CH_2)_2-C(S)-(CH_2)_{11}$-, ##-$(CH_2)_2-C(S)-(CH_2)_{12}$-, ##-$(CH_2)_3-C(S)-(CH_2)_1$-, ##-$(CH_2)_3-C(S)-(CH_2)_2$-, ##-$(CH_2)_3-C(S)-(CH_2)_3$-, ##-$(CH_2)_3-C(S)-(CH_2)_4$-, ##-$(CH_2)_3-C(S)-(CH_2)_5$-, ##-$(CH_2)_3-C(S)-(CH_2)_6$-, ##-$(CH_2)_3-C(S)-(CH_2)_7$-, ##-$(CH_2)_4-C(S)-(CH_2)_1$-, ##-$(CH_2)_4-C(S)-(CH_2)_2$-, ##-$(CH_2)_4-C(S)-(CH_2)_3$-, ##-$(CH_2)_4-C(S)-(CH_2)_4$-, ##-$(CH_2)_4-C(S)-(CH_2)_5$-, ##-$(CH_2)_4-C(S)-(CH_2)_6$-, ##-$(CH_2)_5-C(S)-(CH_2)_1$-, ##-$(CH_2)_5-C(S)-(CH_2)_2$-, ##-$(CH_2)_5-C(S)-(CH_2)_3$-, ##-$(CH_2)_5-C(S)-(CH_2)_4$-, ##-$(CH_2)_5-C(S)-(CH_2)_5$-, ##-$(CH_2)_6-C(S)-(CH_2)_1$-, ##-$(CH_2)_6-C(S)-(CH_2)_2$-, ##-$(CH_2)_6-C(S)-(CH_2)_3$-, ##-$(CH_2)_6-C(S)-(CH_2)_4$-, ##-$(CH_2)_7-C(S)-(CH_2)_1$-, ##-$(CH_2)_7-C(S)-(CH_2)_2$-, ##-$(CH_2)_7-C(S)-(CH_2)_3$-, ##-$(CH_2)_8-C(S)-(CH_2)_1$-, ##-$(CH_2)_8-C(S)-(CH_2)_2$-, ##-$CH_2-C(O)O-CH_2$-, ##-$CH_2-C(O)O-(CH_2)_2$-, ##-$(CH_2)_1-C(O)O-(CH_2)_3$-, ##-$(CH_2)_1-C(O)O-(CH_2)_4$-, ##-$(CH_2)_1-C(O)O-(CH_2)_5$-, ##-$(CH_2)_1-C(O)O-(CH_2)_6$-, ##-$(CH_2)_1-C(O)O-(CH_2)_7$-, ##-$(CH_2)_1-C(O)O-(CH_2)_8$-, ##-$(CH_2)_1-C(O)O-(CH_2)_9$-, ##-$(CH_2)_1-C(O)O-(CH_2)_{10}$-, ##-$(CH_2)_2-C(O)O-(CH_2)_1$-, ##-$(CH_2)_2-C(O)O-(CH_2)_2$-, ##-$(CH_2)_2-C(O)O-(CH_2)_3$-, ##-$(CH_2)_2-C(O)O-(CH_2)_4$-, ##-$(CH_2)_2-C(O)O-(CH_2)_5$-, ##-$(CH_2)_2-C(O)O-(CH_2)_6$-, ##-$(CH_2)_2-C(O)O-(CH_2)_7$-, ##-$(CH_2)_2-C(O)O-(CH_2)_8$-, ##-$(CH_2)_2-C(O)O-(CH_2)_9$-, ##-$(CH_2)_2-C(O)O-(CH_2)_{10}$-, ##-$(CH_2)_2-C(O)O-(CH_2)_{11}$-, ##-$(CH_2)_2-C(O)O-(CH_2)_{12}$-, ##-$(CH_2)_3-C(O)O-(CH_2)_1$-, ##-$(CH_2)_3-C(O)O-(CH_2)_2$-, ##-$(CH_2)_3-C(O)O-(CH_2)_3$-, ##-$(CH_2)_3-C(O)O-(CH_2)_4$-, ##-$(CH_2)_3-C(O)O-(CH_2)_5$-, ##-$(CH_2)_3-C(O)O-(CH_2)_6$-, ##-$(CH_2)_3-C(O)O-(CH_2)_7$-, ##-$(CH_2)_4-C(O)O-(CH_2)_1$-, ##-$(CH_2)_4-C(O)O-(CH_2)_2$-, ##-$(CH_2)_4-C(O)O-(CH_2)_3$-, ##-$(CH_2)_4-C(O)O-(CH_2)_4$-, ##-$(CH_2)_4-C(O)O-(CH_2)_5$-, ##-$(CH_2)_4-C(O)O-(CH_2)_6$-, ##-$(CH_2)_5-C(O)O-(CH_2)_1$-, ##-$(CH_2)_5-C(O)O-(CH_2)_2$-, ##-$(CH_2)_5-C(O)O-(CH_2)_3$-, ##-$(CH_2)_5-C(O)O-(CH_2)_4$-, ##-$(CH_2)_5-C(O)O-(CH_2)_5$-, ##-$(CH_2)_6-C(O)O-(CH_2)_1$-, ##-$(CH_2)_6-C(O)O-(CH_2)_2$-, ##-$(CH_2)_6-C(O)O-(CH_2)_3$-, ##-$(CH_2)_6-C(O)O-(CH_2)_4$-, ##-$(CH_2)_7-C(O)O-(CH_2)_1$-, ##-$(CH_2)_7-C(O)O-(CH_2)_2$-, ##-$(CH_2)_7-C(O)O-(CH_2)_3$-, ##-$(CH_2)_8-C(O)O-(CH_2)_1$-, ##-$(CH_2)_8-C(O)O-(CH_2)_2$-, ##-$CH_2-OC(O)-CH_2$-, ##-$CH_2-OC(O)-(CH_2)_2$-, ##-$(CH_2)_1-OC(O)-(CH_2)_3$-, ##-$(CH_2)_1-OC(O)-(CH_2)_4$-, ##-$(CH_2)_1-OC(O)-(CH_2)_5$-, ##-$(CH_2)_1-OC(O)-(CH_2)_6$-, ##-$(CH_2)_1-OC(O)-(CH_2)_7$-, ##-$(CH_2)_1-OC(O)-(CH_2)_8$-, ##-$(CH_2)_1-OC(O)-(CH_2)_9$-, ##-$(CH_2)_1-OC(O)-(CH_2)_{10}$-, ##-$(CH_2)_2-OC(O)-(CH_2)_1$-, ##-$(CH_2)_2-OC(O)-(CH_2)_2$-, ##-$(CH_2)_2-OC(O)-(CH_2)_3$-, ##-$(CH_2)_2-OC(O)-(CH_2)_4$-, ##-$(CH_2)_2-OC(O)-(CH_2)_5$-, ##-$(CH_2)_2-OC(O)-(CH_2)_6$-, ##-$(CH_2)_2-OC(O)-(CH_2)_7$-, ##-$(CH_2)_2-OC(O)-(CH_2)_8$-, ##-$(CH_2)_2-OC(O)-(CH_2)_9$-, ##-$(CH_2)_2-OC(O)-(CH_2)_{10}$-, ##-$(CH_2)_2-OC(O)-(CH_2)_{11}$-, ##-$(CH_2)_2-OC(O)-(CH_2)_{12}$-, ##-$(CH_2)_3-OC(O)-(CH_2)_1$-, ##-$(CH_2)_3-OC(O)-(CH_2)_2$-, ##-$(CH_2)_3-OC(O)-(CH_2)_3$-, ##-$(CH_2)_3-OC(O)-(CH_2)_4$-,

##-$(CH_2)_3$-OC(O)-$(CH_2)_5$-, ##-$(CH_2)_3$-OC(O)-$(CH_2)_6$-, ##-$(CH_2)_3$-OC(O)-$(CH_2)_7$-, ##-$(CH_2)_4$-OC(O)-$(CH_2)_1$-, ##-$(CH_2)_4$-OC(O)-$(CH_2)_2$-, ##-$(CH_2)_4$-OC(O)-$(CH_2)_3$-, ##-$(CH_2)_4$-OC(O)-$(CH_2)_4$-, ##-$(CH_2)_4$-OC(O)-$(CH_2)_5$-, ##-$(CH_2)_4$-OC(O)-$(CH_2)_6$-, ##-$(CH_2)_5$-OC(O)-$(CH_2)_1$-, ##-$(CH_2)_5$-OC(O)-$(CH_2)_2$-, ##-$(CH_2)_5$-OC(O)-$(CH_2)_3$-, ##-$(CH_2)_5$-OC(O)-$(CH_2)_4$-, ##-$(CH_2)_5$-OC(O)-$(CH_2)_5$-, ##-$(CH_2)_6$-OC(O)-$(CH_2)_1$-, ##-$(CH_2)_6$-OC(O)-$(CH_2)_2$-, ##-$(CH_2)_6$-OC(O)-$(CH_2)_3$-, ##-$(CH_2)_6$-OC(O)-$(CH_2)_4$-, ##-$(CH_2)_7$-OC(O)-$(CH_2)_1$-, ##-$(CH_2)_7$-OC(O)-$(CH_2)_2$-, ##-$(CH_2)_7$-OC(O)-$(CH_2)_3$-, ##-$(CH_2)_8$-OC(O)-$(CH_2)_1$-, ##-$(CH_2)_8$-OC(O)-$(CH_2)_2$-, ##-$(CH_2)_1$-N($R_{g10}$)-$(CH_2)_1$-, ##-$(CH_2)_1$-N($R_{g10}$)-$(CH_2)_2$-, ##-$(CH_2)_1$-N($R_{g10}$)-$(CH_2)_3$-, ##-$(CH_2)_1$-N($R_{g10}$)-$(CH_2)_4$-, ##-$(CH_2)_1$-N($R_{g10}$)-$(CH_2)_5$-, ##-$(CH_2)_1$-N($R_{g10}$)-$(CH_2)_6$-, ##-$(CH_2)_1$-N($R_{g10}$)-$(CH_2)_7$-, ##-$(CH_2)_1$-N($R_{g10}$)-$(CH_2)_8$-, ##-$(CH_2)_1$-N($R_{g10}$)-$(CH_2)_9$-, ##-$(CH_2)_1$-N($R_{g10}$)-$(CH_2)_{10}$-, ##-$(CH_2)_2$-N($R_{g10}$)-$(CH_2)_1$-, ##-$(CH_2)_2$-N($R_{g10}$)-$(CH_2)_2$-, ##-$(CH_2)_2$-N($R_{g10}$)-$(CH_2)_3$-, ##-$(CH_2)_2$-N($R_{g10}$)-$(CH_2)_4$-, ##-$(CH_2)_2$-N($R_{g10}$)-$(CH_2)_5$-, ##-$(CH_2)_2$-N($R_{g10}$)-$(CH_2)_6$-, ##-$(CH_2)_2$-N($R_{g10}$)-$(CH_2)_7$-, ##-$(CH_2)_2$-N($R_{g10}$)-$(CH_2)_8$-, ##-$(CH_2)_2$-N($R_{g10}$)-$(CH_2)_9$-, ##-$(CH_2)_2$-N($R_{g10}$)-$(CH_2)_{10}$-, ##-$(CH_2)_2$-N($R_{g10}$)-$(CH_2)_{11}$-, ##-$(CH_2)_2$-N($R_{g10}$)-$(CH_2)_{12}$-, ##-$(CH_2)_3$-N($R_{g10}$)-$(CH_2)_1$-, ##-$(CH_2)_3$-N($R_{g10}$)-$(CH_2)_2$-, ##-$(CH_2)_3$-N($R_{g10}$)-$(CH_2)_3$-, ##-$(CH_2)_4$-N($R_{g10}$)-$(CH_2)_1$-, ##-$(CH_2)_4$-N($R_{g10}$)-$(CH_2)_2$-, ##-$(CH_2)_4$-N($R_{g10}$)-$(CH_2)_3$-, ##-$(CH_2)_4$-N($R_{g10}$)-$(CH_2)_4$-, ##-$(CH_2)_5$-N($R_{g10}$)-$(CH_2)_1$-, ##-$(CH_2)_5$-N($R_{g10}$)-$(CH_2)_2$-, ##-$(CH_2)_5$-N($R_{g10}$)-$(CH_2)_3$-, ##-$(CH_2)_5$-N($R_{g10}$)-$(CH_2)_4$-, ##-$(CH_2)_5$-N($R_{g10}$)-$(CH_2)_5$-, ##-$(CH_2)_6$-N($R_{g10}$)-$(CH_2)_1$-, ##-$(CH_2)_6$-N($R_{g10}$)-$(CH_2)_2$-, ##-$(CH_2)_6$-N($R_{g10}$)-$(CH_2)_3$-, ##-$(CH_2)_7$-N($R_{g10}$)-$(CH_2)_1$-, ##-$(CH_2)_7$-N($R_{g10}$)-$(CH_2)_2$-, ##-$(CH_2)_7$-N($R_{g10}$)-$(CH_2)_3$-, ##-$(CH_2)_8$-N($R_{g10}$)-$(CH_2)_1$-, ##-$(CH_2)_8$-N $R_{g10}$)-$(CH_2)_2$-, ##-$(CH_2)_8$-N($R_{g10}$)-$(CH_2)_3$-, ##-CH($CH_3$)-N($R_{g10}$)-$(CH_2)_1$-, ##-CH($CH_3$)-N($R_{g10}$)-$(CH_2)_2$-, ##-CH($CH_3$)-N($R_{g10}$)-$(CH_2)_3$-, ##-CH($CH_3$)-N($R_{g10}$)-$(CH_2)_4$-, ##-CH($CH_3$)-N($R_{g10}$)-$(CH_2)_5$-, ##-CH($CH_3$)-N($R_{g10}$)-$(CH_2)_6$-, ##-CH($CH_3$)-N($R_{g10}$)-$(CH_2)_7$-, ##-CH($CH_3$)-N($R_{g10}$)-$(CH_2)_8$-, ##-CH($CH_3$)-N($R_{g10}$)-$(CH_2)_9$-, ##-CH($CH_3$)-N($R_{g10}$)-$(CH_2)_{10}$-, ##-$CH_2$C(O)NH$CH_2$-, ##-$(CH_2)_2$C(O)NH$(CH_2)_2$-, ##-$(CH_2)_2$C(O)NH$(CH_2)_3$-, ##-$(CH_2)_2$C(O)NH$(CH_2)_4$-, ##-$(CH_2)_2$C(O)NH$(CH_2)_5$-, ##-$(CH_2)_3$C(O)NH$(CH_2)_3$-, ##-$(CH_2)_3$C(O)NH$(CH_2)_4$-, ##-$(CH_2)_4$C(O)NH$(CH_2)_4$-, ##-$(CH_2)_5$C(O)NH$(CH_2)_5$-, ##-$(CH_2)_6$C(O)NH$(CH_2)_7$-, ##-$(CH_2)_6$C(O)NH$(CH_2)_6$-, ##-$(CH_2)_7$C(O)NH$(CH_2)_7$-, ##-$(CH_2)_8$C(O)NH$(CH_2)_8$, ##-$(CH_2)_9$C(O)NH$(CH_2)_9$-, ##-$(CH_2)_{10}$C(O)NH$(CH_2)_{10}$-, ##-$(CH_2)_2$C(O)NH$(CH_2)_2$-O-$(CH_2)_2$-, ##-$CH_2$NHC(O)$CH_2$-, ##-$(CH_2)_2$NHC(O)$(CH_2)_2$-, ##-$(CH_2)_2$NHC(O)$(CH_2)_3$-, ##-$(CH_2)_2$NHC(O)$(CH_2)_4$-, ##-$(CH_2)_2$NHC(O)$(CH_2)_5$-, ##-$(CH_2)_3$NHC(O)$(CH_2)_3$-, ##-$(CH_2)_3$NHC(O)$(CH_2)_4$-, ##-$(CH_2)_4$NHC(O)$(CH_2)_4$-, ##-$(CH_2)_5$NHC(0)$(CH_2)_5$-, ##-$(CH_2)_6$NHC(O)$(CH_2)_7$-, ##-$(CH_2)_6$NHC(O)$(CH_2)_6$-, ##-$(CH_2)_7$NHC(O)$(CH_2)_7$-, ##-$(CH_2)_8$NHC(O)$(CH_2)_8$, ##-$(CH_2)_9$NHC(O)$(CH_2)_9$-, ##-$(CH_2)_{10}$NHC(O)$(CH_2)_{10}$-, ##-$(CH_2)_4$NHC(O)$(CH_2)_8$-, ##-$(CH_2)_2$NHC(O)$(CH_2)_2$-O-$(CH_2)_2$-, ##-$(CH_2)_4$NHC(O)$CH_2$-, ##-$CH_2$-piperidinylene-$CH_2$-, ##-$CH_2$-piperidinylene-$(CH_2)_2$-, ##-$CH_2$-piperidinylene-$(CH_2)_3$-, ##-$CH_2$-piperidinylene-$(CH_2)_4$-, ##-$CH_2$-piperidinylene-$(CH_2)_5$-, ##-$CH_2$-piperidinylene-$(CH_2)_6$-, ##-$CH_2$-piperidinylene-$(CH_2)_7$-, ##-$CH_2$-piperidinylene-$(CH_2)_8$-, ##-$(CH_2)_2$-piperidinylene-$(CH_2)_1$-, ##-$(CH_2)_2$-piperidinylene-$(CH_2)_2$-, ##-$(CH_2)_2$-piperidinylene-$(CH_2)_3$-, ##-$(CH_2)_2$-piperidinylene-$(CH_2)_4$-, ##-$(CH_2)_2$-piperidinylene-$(CH_2)_5$-, ##-$(CH_2)_2$-piperidinylene-$(CH_2)_6$-, ##-$(CH_2)_2$-piperidinylene-$(CH_2)_7$-, ##-$(CH_2)_2$-piperidinylene-$(CH_2)_8$-, ##-$(CH_2)_3$-piperidinylene-$CH_2$-, ##-$(CH_2)_3$-piperidinylene-$(CH_2)_2$-, ##-$(CH_2)_3$-piperidinylene-$(CH_2)_3$-, ##-$(CH_2)_3$-piperidinylene-$(CH_2)_4$-, ##-$(CH_2)_3$-piperidinylene-$(CH_2)_5$-, ##-$(CH_2)_3$-piperidinylene-$(CH_2)_6$-, ##-$(CH_2)_3$-piperidinylene-$(CH_2)_7$-, ##-$(CH_2)_3$-piperidinylene-$(CH_2)_8$-, ##-$(CH_2)_4$-piperidinylene-$CH_2$-, ##-$(CH_2)_4$-piperidinylene-$(CH_2)_2$-, ##-$(CH_2)_4$-piperidinylene-$(CH_2)_3$-, ##-$(CH_2)_4$-piperidinylene-$(CH_2)_4$-, ##-$(CH_2)_4$-piperidinylene-$(CH_2)_5$-, ##-$(CH_2)_4$-piperidinylene-$(CH_2)_6$-, ##-$(CH_2)_4$-piperidinylene-$(CH_2)_7$-, ##-$(CH_2)_4$-piperidinylene-$(CH_2)_8$-, ##-$(CH_2)_5$-piperidinylene-$(CH_2)_1$-, ##-$(CH_2)_5$-piperidinylene-$(CH_2)_2$-, ##-$(CH_2)_5$-piperidinylene-$(CH_2)_3$-, ##-$(CH_2)_5$-piperidinylene-$(CH_2)_4$-, ##-$(CH_2)_5$-piperidinylene-$(CH_2)_5$-, ##-$(CH_2)_5$-piperidinylene-$(CH_2)_6$-, ##-$(CH_2)_5$-piperidinylene-$(CH_2)_7$-, ##-$(CH_2)_5$-piperidinylene-$(CH_2)_8$-, ##-$(CH_2)_6$-piperidinylene-$(CH_2)_1$-, ##-$(CH_2)_6$-pipendinylene-$(CH_2)_2$-, ##-$(CH_2)_6$-piperidinylene-$(CH_2)_3$-, ##-$(CH_2)_6$-piperidinylene-$(CH_2)_4$-, ##-$(CH_2)_6$-piperidinylene-$(CH_2)_5$-, ##-$(CH_2)_6$-piperidinylene-$(CH_2)_6$-, ##-$(CH_2)_6$-piperidinylene-$(CH_2)_7$-, ##-$(CH_2)_6$-piperidinylene-$(CH_2)_8$-, ##-$(CH_2)_7$-piperidinylene-$(CH_2)_1$-, ##-$(CH_2)_7$-piperidinylene-$(CH_2)_2$-, ##-$(CH_2)_7$-piperidinylene-$(CH_2)_3$-, ##-$(CH_2)_7$-piperidinylene-$(CH_2)_4$-, ##-$(CH_2)_7$-piperidinylene-$(CH_2)_8$-, ##-$(CH_2)_8$-piperidinylene-$CH_2$-, ##-$(CH_2)_8$-piperidinylene-$(CH_2)_2$-, ##-$(CH_2)_8$-piperidinylene-$(CH_2)_3$-, ##-$(CH_2)_8$-piperidinylene-$(CH_2)_4$-, ##-$(CH_2)_8$-piperidinylene-$(CH_2)_5$-, ##-$(CH_2)_8$-piperidinylene-$(CH_2)_6$-, ##-$(CH_2)_8$-piperidinylene-$(CH_2)_7$-, ##-$(CH_2)_8$-piperidinylene-$(CH_2)_8$-, ##-$CH_2$-piperazinylene-$CH_2$-, ##-$CH_2$-piperazinylene-$(CH_2)_2$-, ##-$CH_2$-piperazinylene-$(CH_2)_3$-, ##-$CH_2$-piperazinylene-$(CH_2)_4$-, ##-$CH_2$-piperazinylene-$(CH_2)_5$-, ##-$CH_2$-piperazinylene-$(CH_2)_6$-, ##-$CH_2$-piperazinylene-$(CH_2)_7$-, ##-$CH_2$-piperazinylene-$(CH_2)_8$-, ##-$(CH_2)_2$-piperazinylene-$(CH_2)_1$-, ##-$(CH_2)_2$-piperazinylene-$(CH_2)_2$-, ##-$(CH_2)_2$-piperazinylene-$(CH_2)_3$-, ##-$(CH_2)_2$-piperazinylene-$(CH_2)_4$-, ##-$(CH_2)_2$-piperazinylene-$(CH_2)_5$-, ##-$(CH_2)_2$-piperazinylene-$(CH_2)_6$-, ##-$(CH_2)_2$-piperazinylene-$(CH_2)_7$-, ##-$(CH_2)_2$-piperazinylene-$(CH_2)_8$-, ##-$(CH_2)_3$-piperazinylene-$CH_2$-, ##-$(CH_2)_3$-piperazinylene-$(CH_2)_2$-, ##-$(CH_2)_3$-piperazinylene-$(CH_2)_3$-, ##-$(CH_2)_3$-piperazinylene-$(CH_2)_4$-, ##-$(CH_2)_3$-piperazinylene-$(CH_2)_5$-, ##-$(CH_2)_3$-piperazinylene-$(CH_2)_6$-, ##-$(CH_2)_3$-piperazinylene-$(CH_2)_7$-, ##-$(CH_2)_3$-piperazinylene-$(CH_2)_8$-, ##-$(CH_2)_4$-piperazinylene-$CH_2$-, ##-$(CH_2)_4$-piperazinylene-$(CH_2)_2$-, ##-$(CH_2)_4$-piperazinylene-$(CH_2)_3$-, ##-$(CH_2)_4$-piperazinylene-$(CH_2)_4$-, ##-$(CH_2)_4$-piperazinylene-$(CH_2)_5$-, ##-$(CH_2)_4$-piperazinylene-$(CH_2)_6$-, ##-$(CH_2)_4$-piperazinylene-$(CH_2)_7$-, ##-$(CH_2)_4$-piperazinylene-$(CH_2)_8$-, ##-$(CH_2)_5$-piperazinylene-$(CH_2)_1$-, ##-$(CH_2)_5$-piperazinylene-$(CH_2)_2$-, ##-$(CH_2)_5$-piperazinylene-$(CH_2)_3$-, ##-$(CH_2)_5$-piperazinylene-$(CH_2)_4$-, ##-$(CH_2)_5$-piperazinylene-$(CH_2)_5$-, ##-$(CH_2)_5$-piperazinylene-$(CH_2)_6$-, ##-$(CH_2)_5$-piperazinylene-$(CH_2)_7$-, ##-$(CH_2)_5$-piperazinylene-$(CH_2)_8$-,

##-$(CH_2)_6$-piperazinylene-$(CH_2)_1$-, ##-$(CH_2)_6$-piperazinylene-$(CH_2)_2$-, ##-$(CH_2)_6$-piperazinylene-$(CH_2)_3$-, ##-$(CH_2)_6$-piperazinylene-$(CH_2)_4$-, ##-$(CH_2)_6$-piperazinylene-$(CH_2)_5$-, ##-$(CH_2)_6$-piperazinylene-$(CH_2)_6$-, ##-$(CH_2)_6$-piperazinylene-$(CH_2)_7$-, ##-$(CH_2)_6$-piperazinylene-$(CH_2)_8$-, ##-$(CH_2)_7$-piperazinylene-$(CH_2)_1$-, ##-$(CH_2)_7$-piperazinylene-$(CH_2)_2$-, ##-$(CH_2)_7$-piperazinylene-$(CH_2)_3$-, ##-$(CH_2)_7$-piperazinylene-$(CH_2)_4$-, ##-$(CH_2)_7$-piperazinylene-$(CH_2)_8$-, ##-$(CH_2)_8$-piperazinylene-$CH_2$-, ##-$(CH_2)_8$-piperazinylene-$(CH_2)_2$-, ##-$(CH_2)_8$-piperazinylene-$(CH_2)_3$-, ##-$(CH_2)_8$-piperazinylene-$(CH_2)_4$-, ##-$(CH_2)_8$-piperazinylene-$(CH_2)_5$-, ##-$(CH_2)_8$-piperazinylene-$(CH_2)_6$-, ##-$(CH_2)_8$-piperazinylene-$(CH_2)_7$-, ##-$(CH_2)_8$-piperazinylene-$(CH_2)_8$-, ##-$CH_2$-propylene-$CH_2$-, ##-$CH_2$-propylene-$(CH_2)_2$-, ##-$CH_2$-propylene-$(CH_2)_3$-, ##-$CH_2$-propylene-$(CH_2)_4$-, ##-$CH_2$-propylene-$(CH_2)_5$-, ##-$CH_2$-propylene-$(CH_2)_6$-, ##-$CH_2$-propylene-$(CH_2)_7$-, ##-$CH_2$-propylene-$(CH_2)_8$-, ##-$(CH_2)_2$-propylene-$(CH_2)_1$-, ##-$(CH_2)_2$-propylene-$(CH_2)_2$-, ##-$(CH_2)_2$-propylene-$(CH_2)_3$-, ##-$(CH_2)_2$-propylene-$(CH_2)_4$-, ##-$(CH_2)_2$-propylene-$(CH_2)_5$-, ##-$(CH_2)_2$-propylene-$(CH_2)_6$-, ##-$(CH_2)_2$-propylene-$(CH_2)_7$-, ##-$(CH_2)_2$-propylene-$(CH_2)_8$-, ##-$(CH_2)_3$-propylene-$CH_2$-, ##-$(CH_2)_3$-propylene-$(CH_2)_2$-, ##-$(CH_2)_3$-propylene-$(CH_2)_3$-, ##-$(CH_2)_3$-propylene-$(CH_2)_4$-, ##-$(CH_2)_3$-propylene-$(CH_2)_5$-, ##-$(CH_2)_3$-propylene-$(CH_2)_6$-, ##-$(CH_2)_3$-propylene-$(CH_2)_7$-, ##-$(CH_2)_3$-propylene-$(CH_2)_8$-, ##-$(CH_2)_4$-propylene-$CH_2$-, ##-$(CH_2)_4$-propylene-$(CH_2)_2$-, ##-$(CH_2)_4$-propylene-$(CH_2)_3$-, ##-$(CH_2)_4$-propylene-$(CH_2)_4$-, ##-$(CH_2)_4$-propylene-$(CH_2)_5$-, ##-$(CH_2)_4$-propylene-$(CH_2)_6$-, ##-$(CH_2)_4$-propylene-$(CH_2)_7$-, ##-$(CH_2)_4$-propylene-$(CH_2)_8$-, ##-$(CH_2)_5$-propylene-$(CH_2)_1$-, ##-$(CH_2)_5$-propylene-$(CH_2)_2$-, ##-$(CH_2)_5$-propylene-$(CH_2)_3$-, ##-$(CH_2)_5$-propylene-$(CH_2)_4$-, ##-$(CH_2)_5$-propylene-$(CH_2)_5$-, ##-$(CH_2)_5$-propylene-$(CH_2)_6$-, ##-$(CH_2)_5$-propylene-$(CH_2)_7$-, ##-$(CH_2)_5$-propylene-$(CH_2)_8$-, ##-$(CH_2)_6$-propylene-$(CH_2)_1$-, ##-$(CH_2)_6$-propylene-$(CH_2)_2$-, ##-$(CH_2)_6$-propylene-$(CH_2)_3$-, ##-$(CH_2)_6$-propylene-$(CH_2)_4$-, ##-$(CH_2)_6$-propylene-$(CH_2)_5$-, ##-$(CH_2)_6$-propylene-$(CH_2)_6$-, ##-$(CH_2)_6$-propylene-$(CH_2)_7$-, ##-$(CH_2)_6$-propylene-$(CH_2)_8$-, ##-$(CH_2)_7$-propylene-$(CH_2)_1$-, ##-$(CH_2)_7$-propylene-$(CH_2)_2$-, ##-$(CH_2)_7$-propylene-$(CH_2)_3$-, ##-$(CH_2)_7$-propylene-$(CH_2)_4$-, ##-$(CH_2)_7$-propylene-$(CH_2)_8$-, ##-$(CH_2)_8$-propylene-$CH_2$-, ##-$(CH_2)_8$-propylene-$(CH_2)_2$-, ##-$(CH_2)_8$-propylene-$(CH_2)_3$-, ##-$(CH_2)_8$-propylene-$(CH_2)_4$-, ##-$(CH_2)_8$-propylene-$(CH_2)_5$-, ##-$(CH_2)_8$-propylene-$(CH_2)_6$-, ##-$(CH_2)_8$-propylene-$(CH_2)_7$-, ##-$(CH_2)_8$-propylene-$(CH_2)_8$-, ##-$CH_2$-diazacycloheptanylene-$CH_2$-, ##-$CH_2$-diazacycloheptanylene-$(CH_2)_2$-, ##-$CH_2$-diazacycloheptanylene-$(CH_2)_3$-, ##-$CH_2$-diazacycloheptanylene-$(CH_2)_4$-, ##-$CH_2$-diazacycloheptanylene-$(CH_2)_5$-, ##-$CH_2$-diazacycloheptanylene-$(CH_2)_6$-, ##-$CH_2$-diazacycloheptanylene-$(CH_2)_7$-, ##-$CH_2$-diazacycloheptanylene-$(CH_2)_8$-, ##-$(CH_2)_2$-diazacycloheptanylene-$(CH_2)_1$-, ##-$(CH_2)_2$-diazacycloheptanylene-$(CH_2)_2$-, ##-$(CH_2)_2$-diazacycloheptanylene-$(CH_2)_3$-, ##-$(CH_2)_2$-diazacycloheptanylene-$(CH_2)_4$-, ##-$(CH_2)_2$-diazacycloheptanylene-$(CH_2)_5$-, ##-$(CH_2)_2$-diazacycloheptanylene-$(CH_2)_6$-, ##-$(CH_2)_2$-diazacycloheptanylene-$(CH_2)_7$-, ##-$(CH_2)_2$-diazacycloheptanylene-$(CH_2)_8$-, ##-$(CH_2)_3$-diazacycloheptanylene-$CH_2$-, ##-$(CH_2)_3$-diazacycloheptanylene-$(CH_2)_2$-, ##-$(CH_2)_3$-diazacycloheptanylene-$(CH_2)_3$-, ##-$(CH_2)_3$-diazacycloheptanylene-$(CH_2)_4$-, ##-$(CH_2)_3$-diazacycloheptanylene-$(CH_2)_5$-, ##-$(CH_2)_3$-diazacycloheptanylene-$(CH_2)_6$-, ##-$(CH_2)_3$-diazacycloheptanylene-$(CH_2)_7$-, ##-$(CH_2)_3$-diazacycloheptanylene-$(CH_2)_8$-, ##-$(CH_2)_4$-diazacycloheptanylene-$CH_2$-, ##-$(CH_2)_4$-diazacycloheptanylene-$(CH_2)_2$-, ##-$(CH_2)_4$-diazacycloheptanylene-$(CH_2)_3$-, ##-$(CH_2)_4$-diazacycloheptanylene-$(CH_2)_4$-, ##-$(CH_2)_4$-diazacycloheptanylene-$(CH_2)_5$-, ##-$(CH_2)_4$-diazacycloheptanylene-$(CH_2)_6$-, ##-$(CH_2)_4$-diazacycloheptanylene-$(CH_2)_7$-, ##-$(CH_2)_4$-diazacycloheptanylene-$(CH_2)_8$-, ##-$(CH_2)_5$-diazacycloheptanylene-$(CH_2)_1$-, ##-$(CH_2)_5$-diazacycloheptanylene-$(CH_2)_2$-, ##-$(CH_2)_5$-diazacycloheptanylene-$(CH_2)_3$-, ##-$(CH_2)_5$-diazacycloheptanylene-$(CH_2)_4$-, ##-$(CH_2)_5$-diazacycloheptanylene-$(CH_2)_5$-, ##-$(CH_2)_5$-diazacycloheptanylene-$(CH_2)_6$-, ##-$(CH_2)_5$-diazacycloheptanylene-$(CH_2)_7$-, ##-$(CH_2)_5$-diazacycloheptanylene-$(CH_2)_8$-, ##-$(CH_2)_6$-diazacycloheptanylene-$(CH_2)_1$-, ##-$(CH_2)_6$-diazacycloheptanylene-$(CH_2)_2$-, ##-$(CH_2)_6$-diazacycloheptanylene-$(CH_2)_3$-, ##-$(CH_2)_6$-diazacycloheptanylene-$(CH_2)_4$-, ##-$(CH_2)_6$-diazacycloheptanylene-$(CH_2)_5$-, ##-$(CH_2)_6$-diazacycloheptanylene-$(CH_2)_6$-, ##-$(CH_2)_6$-diazacycloheptanylene-$(CH_2)_7$-, ##-$(CH_2)_6$-diazacycloheptanylene-$(CH_2)_8$-, ##-$(CH_2)_7$-diazacycloheptanylene-$(CH_2)_1$-, ##-$(CH_2)_7$-diazacycloheptanylene-$(CH_2)_2$-, ##-$(CH_2)_7$-diazacycloheptanylene-$(CH_2)_3$-, ##-$(CH_2)_7$-diazacycloheptanylene-$(CH_2)_4$-, ##-$(CH_2)_7$-diazacycloheptanylene-$(CH_2)_8$-, ##-$(CH_2)_8$-diazacycloheptanylene-$CH_2$-, ##-$(CH_2)_8$-diazacycloheptanylene-$(CH_2)_2$-, ##-$(CH_2)_8$-diazacycloheptanylene-$(CH_2)_3$-, ##-$(CH_2)_8$-diazacycloheptanylene-$(CH_2)_4$-, ##-$(CH_2)_8$-diazacycloheptanylene-$(CH_2)_5$-, ##-$(CH_2)_8$-diazacycloheptanylene-$(CH_2)_6$-, ##-$(CH_2)_8$-diazacycloheptanylene-$(CH_2)_7$-, ##-$(CH_2)_8$-diazacycloheptanylene-$(CH_2)_8$-, ##-$CH_2$-diazabicyclo[2.2.1]heptanylene-$CH_2$-, ##-$CH_2$-diazabicyclo[2.2.1]heptanylene-$(CH_2)_2$-, ##-$CH_2$-diazabicyclo[2.2.1]heptanylene-$(CH_2)_3$-, ##-$CH_2$-diazabicyclo[2.2.1]heptanylene-$(CH_2)_4$-, ##-$CH_2$-diazabicyclo[2.2.1]heptanylene-$(CH_2)_5$-, ##-$CH_2$-diazabicyclo[2.2.1]heptanylene-$(CH_2)_6$-, ##-$CH_2$-diazabicyclo[2.2.1]heptanylene-$(CH_2)_7$-, ##-$CH_2$-diazabicyclo[2.2.1]heptanylene-$(CH_2)_8$-, ##-$(CH_2)_2$-diazabicyclo[2.2.1]heptanylene-$(CH_2)_1$-, ##-$(CH_2)_2$-diazabicyclo[2.2.1]heptanylene-$(CH_2)_2$-, ##-$(CH_2)_2$-diazabicyclo[2.2.1]heptanylene-$(CH_2)_3$-, ##-$(CH_2)_2$-diazabicyclo[2.2.1]heptanylene-$(CH_2)_4$-, ##-$(CH_2)_2$-diazabicyclo[2.2.1]heptanylene-$(CH_2)_5$-, ##-$(CH_2)_2$-diazabicyclo[2.2.1]heptanylene-$(CH_2)_6$-, ##-$(CH_2)_2$-diazabicyclo[2.2.1]heptanylene-$(CH_2)_7$-, ##-$(CH_2)_2$-diazabicyclo[2.2.1]heptanylene-$(CH_2)_8$-, ##-$(CH_2)_3$-diazabicyclo[2.2.1]heptanylene-$CH_2$-, ##-$(CH_2)_3$-diazabicyclo[2.2.1]heptanylene-$(CH_2)_2$-, ##-$(CH_2)_3$-diazabicyclo[2.2.1]heptanylene-$(CH_2)_3$-, ##-$(CH_2)_3$-diazabicyclo[2.2.1]heptanylene-$(CH_2)_4$-, ##-$(CH_2)_3$-diazabicyclo[2.2.1]heptanylene-$(CH_2)_5$-, ##-$(CH_2)_3$-diazabicyclo[2.2.1]heptanylene-$(CH_2)_6$-, ##-$(CH_2)_3$-diazabicyclo[2.2.1]heptanylene-$(CH_2)_7$-, ##-$(CH_2)_3$-diazabicyclo[2.2.1]heptanylene-$(CH_2)_8$-, ##-$(CH_2)_4$-diazabicyclo[2.2.1]heptanylene-$CH_2$-, ##-$(CH_2)_4$-diazabicyclo[2.2.1]heptanylene-$(CH_2)_2$-, ##-$(CH_2)_4$-diazabicyclo[2.2.1]heptanylene-$(CH_2)_3$-, ##-$(CH_2)_4$-diazabicyclo[2.2.1]heptanylene-$(CH_2)_4$-, ##-$(CH_2)_4$-diazabicyclo[2.2.1]heptanylene-$(CH_2)_5$-, ##-$(CH_2)_4$-diazabicyclo[2.2.1]

heptanylene-$(CH_2)_6$-, ##-$(CH_2)_4$-diazabicyclo[2.2.1]heptanylene-$(CH_2)_7$-, ##-$(CH_2)_4$-diazabicyclo[2.2.1]heptanylene-$(CH_2)_8$-, ##-$(CH_2)_5$-diazabicyclo[2.2.1]heptanylene-$(CH_2)_1$-, ##-$(CH_2)_5$-diazabicyclo[2.2.1]heptanylene-$(CH_2)_2$-, ##-$(CH_2)_5$-diazabicyclo[2.2.1]heptanylene-$(CH_2)_3$-, ##-$(CH_2)_5$-diazabicyclo[2.2.1]heptanylene-$(CH_2)_4$-, ##-$(CH_2)_5$-diazabicyclo[2.2.1]heptanylene-$(CH_2)_5$-, ##-$(CH_2)_5$-diazabicyclo[2.2.1]heptanylene-$(CH_2)_6$-, ##-$(CH_2)_5$-diazabicyclo[2.2.1]heptanylene-$(CH_2)_7$-, ##-$(CH_2)_5$-diazabicyclo[2.2.1]heptanylene-$(CH_2)_8$-, ##-$(CH_2)_6$-diazabicyclo[2.2.1]heptanylene-$(CH_2)_1$-, ##-$(CH_2)_6$-diazabicyclo[2.2.1]heptanylene-$(CH_2)_2$-, ##-$(CH_2)_6$-diazabicyclo[2.2.1]heptanylene-$(CH_2)_3$-, ##-$(CH_2)_6$-diazabicyclo[2.2.1]heptanylene-$(CH_2)_4$-, ##-$(CH_2)_6$-diazabicyclo[2.2.1]heptanylene-$(CH_2)_5$-, ##-$(CH_2)_6$-diazabicyclo[2.2.1]heptanylene-$(CH_2)_6$-, ##-$(CH_2)_6$-diazabicyclo[2.2.1]heptanylene-$(CH_2)_7$-, ##-$(CH_2)_6$-diazabicyclo[2.2.1]heptanylene-$(CH_2)_8$-, ##-$(CH_2)_7$-diazabicyclo[2.2.1]heptanylene-$(CH_2)_1$-, ##-$(CH_2)_7$-diazabicyclo[2.2.1]heptanylene-$(CH_2)_2$-, ##-$(CH_2)_7$-diazabicyclo[2.2.1]heptanylene-$(CH_2)_3$-, ##-$(CH_2)_7$-diazabicyclo[2.2.1]heptanylene-$(CH_2)_4$-, ##-$(CH_2)_7$-diazabicyclo[2.2.1]heptanylene-$(CH_2)_8$-, ##-$(CH_2)_8$-diazabicyclo[2.2.1]heptanylene-$CH_2$-, ##-$(CH_2)_8$-diazabicyclo[2.2.1]heptanylene-$(CH_2)_2$-, ##-$(CH_2)_8$-diazabicyclo[2.2.1]heptanylene-$(CH_2)_3$-, ##-$(CH_2)_8$-diazabicyclo[2.2.1]heptanylene-$(CH_2)_4$-, ##-$(CH_2)_8$-diazabicyclo[2.2.1]heptanylene-$(CH_2)_5$-, ##-$(CH_2)_8$-diazabicyclo[2.2.1]heptanylene-$(CH_2)_6$-, ##-$(CH_2)_8$-diazabicyclo[2.2.1]heptanylene-$(CH_2)_7$-, ##-$(CH_2)_8$-diazabicyclo[2.2.1]heptanylene-$(CH_2)_8$-, ##-$CH_2$-diazabicyclo[3.1.1]heptanylene-$CH_2$-, ##-$CH_2$-diazabicyclo[3.1.1]heptanylene-$(CH_2)_2$-, ##-$CH_2$-diazabicyclo[3.1.1]heptanylene-$(CH_2)_3$-, ##-$CH_2$-diazabicyclo[3.1.1]heptanylene-$(CH_2)_4$-, ##-$CH_2$-diazabicyclo[3.1.1]heptanylene-$(CH_2)_5$-, ##-$CH_2$-diazabicyclo[3.1.1]heptanylene-$(CH_2)_6$-, ##-$CH_2$-diazabicyclo[3.1.1]heptanylene-$(CH_2)_7$-, ##-$CH_2$-diazabicyclo[3.1.1]heptanylene-$(CH_2)_8$-, ##-$(CH_2)_2$-diazabicyclo[3.1.1]heptanylene-$(CH_2)_1$-, ##-$(CH_2)_2$-diazabicyclo[3.1.1]heptanylene-$(CH_2)_2$-, ##-$(CH_2)_2$-diazabicyclo[3.1.1]heptanylene-$(CH_2)_3$-, ##-$(CH_2)_2$-diazabicyclo[3.1.1]heptanylene-$(CH_2)_4$-, ##-$(CH_2)_2$-diazabicyclo[3.1.1]heptanylene-$(CH_2)_5$-, ##-$(CH_2)_2$-diazabicyclo[3.1.1]heptanylene-$(CH_2)_6$-, ##-$(CH_2)_2$-diazabicyclo[3.1.1]heptanylene-$(CH_2)_7$-, ##-$(CH_2)_2$-diazabicyclo[3.1.1]heptanylene-$(CH_2)_8$-, ##-$(CH_2)_3$-diazabicyclo[3.1.1]heptanylene-$CH_2$-, ##-$(CH_2)_3$-diazabicyclo[3.1.1]heptanylene-$(CH_2)_2$-, ##-$(CH_2)_3$-diazabicyclo[3.1.1]heptanylene-$(CH_2)_3$-, ##-$(CH_2)_3$-diazabicyclo[3.1.1]heptanylene-$(CH_2)_4$-, ##-$(CH_2)_3$-diazabicyclo[3.1.1]heptanylene-$(CH_2)_5$-, ##-$(CH_2)_3$-diazabicyclo[3.1.1]heptanylene-$(CH_2)_6$-, ##-$(CH_2)_3$-diazabicyclo[3.1.1]heptanylene-$(CH_2)_7$-, ##-$(CH_2)_3$-diazabicyclo[3.1.1]heptanylene-$(CH_2)_8$-, ##-$(CH_2)_4$-diazabicyclo[3.1.1]heptanylene-$CH_2$-, ##-$(CH_2)_4$-diazabicyclo[3.1.1]heptanylene-$(CH_2)_2$-, ##-$(CH_2)_4$-diazabicyclo[3.1.1]heptanylene-$(CH_2)_3$-, ##-$(CH_2)_4$-diazabicyclo[3.1.1]heptanylene-$(CH_2)_4$-, ##-$(CH_2)_4$-diazabicyclo[3.1.1]heptanylene-$(CH_2)_5$-, ##-$(CH_2)_4$-diazabicyclo[3.1.1]heptanylene-$(CH_2)_6$-, ##-$(CH_2)_4$-diazabicyclo[3.1.1]heptanylene-$(CH_2)_7$-, ##-$(CH_2)_4$-diazabicyclo[3.1.1]heptanylene-$(CH_2)_8$-, ##-$(CH_2)_5$-diazabicyclo[3.1.1]heptanylene-$(CH_2)_1$-, ##-$(CH_2)_5$-diazabicyclo[3.1.1]heptanylene-$(CH_2)_2$-, ##-$(CH_2)_5$-diazabicyclo[3.1.1]heptanylene-$(CH_2)_3$-, ##-$(CH_2)_5$-diazabicyclo[3.1.1]heptanylene-$(CH_2)_4$-, ##-$(CH_2)_5$-diazabicyclo[3.1.1]heptanylene-$(CH_2)_5$-, ##-$(CH_2)_5$-diazabicyclo[3.1.1]heptanylene-$(CH_2)_6$-, ##-$(CH_2)_5$-diazabicyclo[3.1.1]heptanylene-$(CH_2)_7$-, ##-$(CH_2)_5$-diazabicyclo[3.1.1]heptanylene-$(CH_2)_8$-, ##-$(CH_2)_6$-diazabicyclo[3.1.1]heptanylene-$(CH_2)_1$-, ##-$(CH_2)_6$-diazabicyclo[3.1.1]heptanylene-$(CH_2)_2$-, ##-$(CH_2)_6$-diazabicyclo[3.1.1]heptanylene-$(CH_2)_3$-, ##-$(CH_2)_6$-diazabicyclo[3.1.1]heptanylene-$(CH_2)_4$-, ##-$(CH_2)_6$-diazabicyclo[3.1.1]heptanylene-$(CH_2)_5$-, ##-$(CH_2)_6$-diazabicyclo[3.1.1]heptanylene-$(CH_2)_6$-, ##-$(CH_2)_6$-diazabicyclo[3.1.1]heptanylene-$(CH_2)_7$-, ##-$(CH_2)_6$-diazabicyclo[3.1.1]heptanylene-$(CH_2)_8$-, ##-$(CH_2)_7$-diazabicyclo[3.1.1]heptanylene-$(CH_2)_1$-, ##-$(CH_2)_7$-diazabicyclo[3.1.1]heptanylene-$(CH_2)_2$-, ##-$(CH_2)_7$-diazabicyclo[3.1.1]heptanylene-$(CH_2)_3$-, ##-$(CH_2)_7$-diazabicyclo[3.1.1]heptanylene-$(CH_2)_4$-, ##-$(CH_2)_7$-diazabicyclo[3.1.1]heptanylene-$(CH_2)_8$-, ##-$(CH_2)_8$-diazabicyclo[3.1.1]heptanylene-$CH_2$-, ##-$(CH_2)_8$-diazabicyclo[3.1.1]heptanylene-$(CH_2)_2$-, ##-$(CH_2)_8$-diazabicyclo[3.1.1]heptanylene-$(CH_2)_3$-, ##-$(CH_2)_8$-diazabicyclo[3.1.1]heptanylene-$(CH_2)_4$-, ##-$(CH_2)_8$-diazabicyclo[3.1.1]heptanylene-$(CH_2)_5$-, ##-$(CH_2)_8$-diazabicyclo[3.1.1]heptanylene-$(CH_2)_6$-, ##-$(CH_2)_8$-diazabicyclo[3.1.1]heptanylene-$(CH_2)_7$-, ##-$(CH_2)_8$-diazabicyclo[3.1.1]heptanylene-$(CH_2)_8$-, ##-$CH_2$-diazabicyclo[3.2.1]octylene-$CH_2$-, ##-$CH_2$-diazabicyclo[3.2.1]octylene-$(CH_2)_2$-, ##-$CH_2$-diazabicyclo[3.2.1]octylene-$(CH_2)_3$-, ##-$CH_2$-diazabicyclo[3.2.1]octylene-$(CH_2)_4$-, ##-$CH_2$-diazabicyclo[3.2.1]octylene-$(CH_2)_5$-, ##-$CH_2$-diazabicyclo[3.2.1]octylene-$(CH_2)_6$-, ##-$CH_2$-diazabicyclo[3.2.1]octylene-$(CH_2)_7$-, ##-$CH_2$-diazabicyclo[3.2.1]octylene-$(CH_2)_8$-, ##-$(CH_2)_2$-diazabicyclo[3.2.1]octylene-$(CH_2)_1$-, ##-$(CH_2)_2$-diazabicyclo[3.2.1]octylene-$(CH_2)_2$-, ##-$(CH_2)_2$-diazabicyclo[3.2.1]octylene-$(CH_2)_3$-, ##-$(CH_2)_2$-diazabicyclo[3.2.1]octylene-$(CH_2)_4$-, ##-$(CH_2)_2$-diazabicyclo[3.2.1]octylene-$(CH_2)_5$-, ##-$(CH_2)_2$-diazabicyclo[3.2.1]octylene-$(CH_2)_6$-, ##-$(CH_2)_2$-diazabicyclo[3.2.1]octylene-$(CH_2)_7$-, ##-$(CH_2)_2$-diazabicyclo[3.2.1]octylene-$(CH_2)_8$-, ##-$(CH_2)_3$-diazabicyclo[3.2.1]octylene-$CH_2$-, ##-$(CH_2)_3$-diazabicyclo[3.2.1]octylene-$(CH_2)_2$-, ##-$(CH_2)_3$-diazabicyclo[3.2. 1]octylene-$(CH_2)_3$-, ##-$(CH_2)_3$-diazabicyclo[3.2.1]octylene-$(CH_2)_4$-, ##-$(CH_2)_3$-diazabicyclo[3.2. 1]octylene-$(CH_2)_5$-, ##-$(CH_2)_3$-diazabicyclo[3.2.1]octylene-$(CH_2)_6$-, ##-$(CH_2)_3$-diazabicyclo[3.2.1]octylene-$(CH_2)_7$-, ##-$(CH_2)_3$-diazabicyclo[3.2.1]octylene-$(CH_2)_8$-, ##-$(CH_2)_4$-diazabicyclo[3.2.1]octylene-$CH_2$-, ##-$(CH_2)_4$-diazabicyclo[3.2.1]octylene-$(CH_2)_2$-, ##-$(CH_2)_4$-diazabicyclo[3.2.1]octylene-$(CH_2)_3$-, ##-$(CH_2)_4$-diazabicyclo[3.2.1]octylene-$(CH_2)_4$-, ##-$(CH_2)_4$-diazabicyclo[3.2.1]octylene-$(CH_2)_5$-, ##-$(CH_2)_4$-diazabicyclo[3.2.1]octylene-$(CH_2)_6$-, ##-$(CH_2)_4$-diazabicyclo[3.2.1]octylene-$(CH_2)_7$-, ##-$(CH_2)_4$-diazabicyclo[3.2.1]octylene-$(CH_2)_8$-, ##-$(CH_2)_5$-diazabicyclo[3.2. 1]octylene-$(CH_2)_1$-, ##-$(CH_2)_5$-diazabicyclo[3.2.1]octylene-$(CH_2)_2$-, ##-$(CH_2)_5$-diazabicyclo[3.2. 1]octylene-$(CH_2)_3$-, ##-$(CH_2)_5$-diazabicyclo[3.2.1]octylene-$(CH_2)_4$-, ##-$(CH_2)_5$-diazabicyclo[3.2. 1]octylene-$(CH_2)_5$-, ##-$(CH_2)_5$-diazabicyclo[3.2.1]octylene-$(CH_2)_6$-, ##-$(CH_2)_5$-diazabicyclo[3.2.1]octylene-$(CH_2)_7$-, ##-$(CH_2)_5$-diazabicyclo[3.2.1]octylene-$(CH_2)_8$-, ##-$(CH_2)_6$-diazabicyclo[3 .2.1]octylene-$(CH_2)_1$-,

##-$(CH_2)_6$-diazabicyclo[3.2.1]octylene-$(CH_2)_2$-, ##-$(CH_2)_6$-diazabicyclo[3.2.1]octylene-$(CH_2)_3$-, ##-$(CH_2)_6$-diazabicyclo[3.2.1]octylene-$(CH_2)_4$-, ##-$(CH_2)_6$-diazabicyclo[3.2.1]octylene-$(CH_2)_5$-, ##-$(CH_2)_6$-diazabicyclo[3.2.1]octylene-$(CH_2)_6$-, ##-$(CH_2)_6$-diazabicyclo[3.2.1]octylene-$(CH_2)_7$-, ##-$(CH_2)_6$-diazabicyclo[3.2.1]octylene-$(CH_2)_8$-, ##-$(CH_2)_7$-diazabicyclo[3.2. 1]octylene-$(CH_2)_1$-, ##-$(CH_2)_7$-diazabicyclo[3.2.1]octylene-$(CH_2)_2$-, ##-$(CH_2)_7$-diazabicyclo[3.2.1]octylene-$(CH_2)_3$-, ##-$(CH_2)_7$-diazabicyclo[3.2.1]octylene-$(CH_2)_4$-, ##-$(CH_2)_7$-diazabicyclo[3.2.1]octylene-$(CH_2)_8$-, ##-$(CH_2)_8$-diazabicyclo[3.2.1]octylene-$CH_2$-, ##-$(CH_2)_8$-diazabicyclo[3.2.1]octylene-$(CH_2)_2$-, ##-$(CH_2)_8$-diazabicyclo[3.2.1]octylene-$(CH_2)_3$-, ##-$(CH_2)_8$-diazabicyclo[3.2.1]octylene-$(CH_2)_4$-, ##-$(CH_2)_s$-diazabicyclo[3.2.1]octylene-$(CH_2)_5$-, ##-$(CH_2)_8$-diazabicyclo[3.2.1]octylene-$(CH_2)_6$-, ##-$(CH_2)_8$-diazabicyclo[3.2.1]octylene-$(CH_2)_7$-, ##-$(CH_2)_8$-diazabicyclo[3.2.1]octylene-$(CH_2)_8$-, ##-$CH_2$-diazabicyclo[2.2.2]octylene-$CH_2$-, ##-$CH_2$-diazabicyclo[2.2.2]octylene-$(CH_2)_2$-, ##-$CH_2$-diazabicyclo[2.2.2]octylene-$(CH_2)_3$-, ##-$CH_2$-diazabicyclo[2.2.2]octylene-$(CH_2)_4$-, ##-$CH_2$-diazabicyclo[2.2.2]octylene-$(CH_2)_5$-, ##-$CH_2$-diazabicyclo[2.2.2]octylene-$(CH_2)_6$-, ##-$CH_2$-diazabicyclo[2.2.2]octylene-$(CH_2)_7$-, ##-$CH_2$-diazabicyclo[2.2.2]octylene-$(CH_2)_8$-, ##-$(CH_2)_2$-diazabicyclo[2.2.2]octylene-$(CH_2)_1$-, ##-$(CH_2)_2$-diazabicyclo[2.2.2]octylene-$(CH_2)_2$-, ##-$(CH_2)_2$-diazabicyclo[2.2.2]octylene-$(CH_2)_3$-, ##-$(CH_2)_2$-diazabicyclo[2.2.2]octylene-$(CH_2)_4$-, ##-$(CH_2)_2$-diazabicyclo[2.2.2]octylene-$(CH_2)_5$-, ##-$(CH_2)_2$-diazabicyclo[2.2.2]octylene-$(CH_2)_6$-, ##-$(CH_2)_2$-diazabicyclo[2.2.2]octylene-$(CH_2)_7$-, ##-$(CH_2)_2$-diazabicyclo[2.2.2]octylene-$(CH_2)_8$-, ##-$(CH_2)_3$-diazabicyclo[2.2.2]octylene-$CH_2$-, ##-$(CH_2)_3$-diazabicyclo[2.2.2]octylene-$(CH_2)_2$-, ##-$(CH_2)_3$-diazabicyclo[2.2.2]octylene-$(CH_2)_3$-, ##-$(CH_2)_3$-diazabicyclo[2.2.2]octylene-$(CH_2)_4$-, ##-$(CH_2)_3$-diazabicyclo[2.2.2]octylene-$(CH_2)_5$-, ##-$(CH_2)_3$-diazabicyclo[2.2.2]octylene-$(CH_2)_6$-, ##-$(CH_2)_3$-diazabicyclo[2.2.2]octylene-$(CH_2)_7$-, ##-$(CH_2)_3$-diazabicyclo[2.2.2]octylene-$(CH_2)_8$-, ##-$(CH_2)_4$-diazabicyclo[2.2.2]octylene-$CH_2$-, ##-$(CH_2)_4$-diazabicyclo[2.2.2]octylene-$(CH_2)_2$-, ##-$(CH_2)_4$-diazabicyclo[2.2.2]octylene-$(CH_2)_3$-, ##-$(CH_2)_4$-diazabicyclo[2.2.2]octylene-$(CH_2)_4$-, ##-$(CH_2)_4$-diazabicyclo[2.2.2]octylene-$(CH_2)_5$-, ##-$(CH_2)_4$-diazabicyclo[2.2.2]octylene-$(CH_2)_6$-, ##-$(CH_2)_4$-diazabicyclo[2.2.2]octylene-$(CH_2)_7$-, ##-$(CH_2)_4$-diazabicyclo[2.2.2]octylene-$(CH_2)_8$-, ##-$(CH_2)_5$-diazabicyclo[2.2.2]octylene-$(CH_2)_1$-, ##-$(CH_2)_5$-diazabicyclo[2.2.2]octylene-$(CH_2)_2$-, ##-$(CH_2)_5$-diazabicyclo[2.2.2]octylene-$(CH_2)_3$-, ##-$(CH_2)_5$-diazabicyclo[2.2.2]octylene-$(CH_2)_4$-, ##-$(CH_2)_5$-diazabicyclo[2.2.2]octylene-$(CH_2)_5$-, ##-$(CH_2)_5$-diazabicyclo[2.2.2]octylene-$(CH_2)_6$-, ##-$(CH_2)_5$-diazabicyclo[2.2.2]octylene-$(CH_2)_7$-, ##-$(CH_2)_5$-diazabicyclo[2.2.2]octylene-$(CH_2)_8$-, ##-$(CH_2)_6$-diazabicyclo[2.2.2]octylene-$(CH_2)_1$-, ##-$(CH_2)_6$-diazabicyclo[2.2.2]octylene-$(CH_2)_2$-, ##-$(CH_2)_6$-diazabicyclo[2.2.2]octylene-$(CH_2)_3$-, ##-$(CH_2)_6$-diazabicyclo[2.2.2]octylene-$(CH_2)_4$-, ##-$(CH_2)_6$-diazabicyclo[2.2.2]octylene-$(CH_2)_5$-, ##-$(CH_2)_6$-diazabicyclo[2.2.2]octylene-$(CH_2)_6$-, ##-$(CH_2)_6$-diazabicyclo[2.2.2]octylene-$(CH_2)_7$-, ##-$(CH_2)_6$-diazabicyclo[2.2.2]octylene-$(CH_2)_8$-, ##-$(CH_2)_7$-diazabicyclo[2.2.2]octylene-$(CH_2)_i$-, ##-$(CH_2)_7$-diazabicyclo[2.2.2]octylene-$(CH_2)_2$-, ##-$(CH_2)_7$-diazabicyclo[2.2.2]octylene-$(CH_2)_3$-, ##-$(CH_2)_7$-diazabicyclo[2.2.2]octylene-$(CH_2)_4$-, ##-$(CH_2)_7$-diazabicyclo[2.2.2]octylene-$(CH_2)_5$-, ##-$(CH_2)_8$-diazabicyclo[2.2.2]octylene-$CH_2$-, ##-$(CH_2)_8$-diazabicyclo[2.2.2]octylene-$(CH_2)_2$-, ##-$(CH_2)_8$-diazabicyclo[2.2.2]octylene-$(CH_2)_3$-, ##-$(CH_2)_8$-diazabicyclo[2.2.2]octylene-$(CH_2)_4$-, ##-$(CH_2)_8$-diazabicyclo[2.2.2]octylene-$(CH_2)_5$-, ##-$(CH_2)_8$-diazabicyclo[2.2.2]octylene-$(CH_2)_6$-, ##-$(CH_2)_8$-diazabicyclo[2.2.2]octylene-$(CH_2)_7$-, or ##-$(CH_2)_8$-diazabicyclo[2.2.2]octylene-$(CH_2)_8$-,

wherein the propylene, the piperidinylene, the piperazinylene, the diazacycloheptanylene, the diazabicyclo[2.2.1]heptanylene, the diazabicyclo[3.1.1]heptanylene, the diazabicyclo[3.2.1]octylene, the diazabicyclo[2.2.2]octylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;
each $R_{g10}$ independently represents H or $C_{1-3}$ alkyl; and
symbol ## indicates the point of attachment to $X_1$.

In some embodiments of the compound of Formula (I-1), $L_1$ represents the following group:

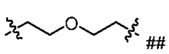 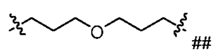 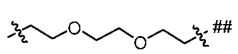 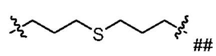 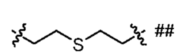 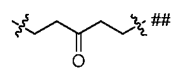

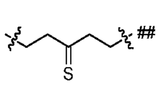 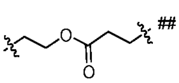 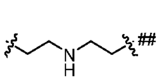 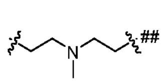 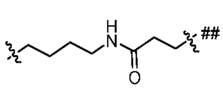 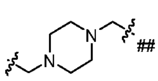 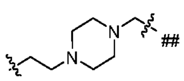

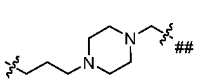 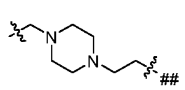 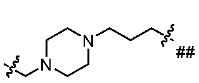 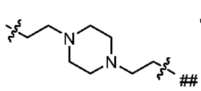 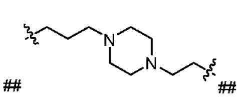 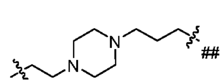

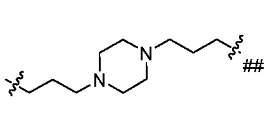 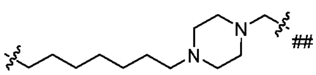 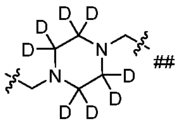 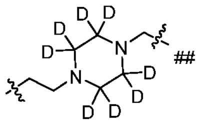 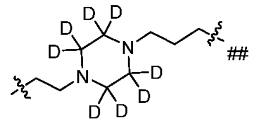

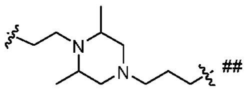 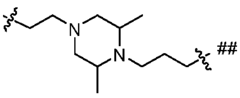 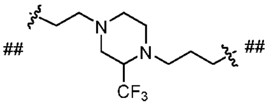 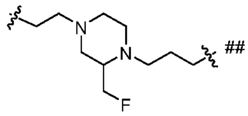 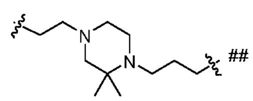

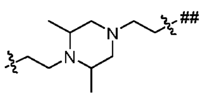 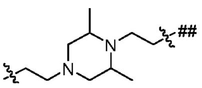 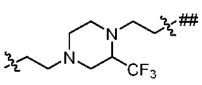 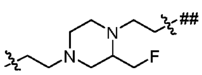 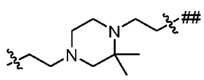 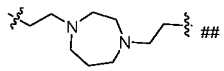

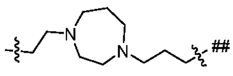 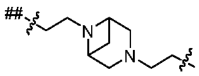 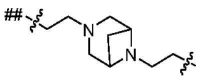 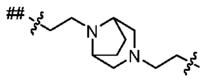 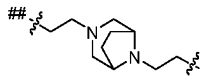 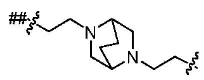

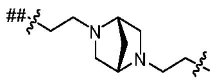 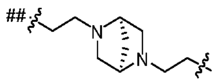 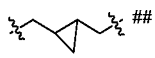 or 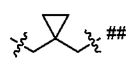 ;

wherein symbol ## indicates the point of attachment to $X_1$.

In some embodiments of the compound of Formula (I-1), $L_1$ represents the structure of the following formula: ##-$C_{1-30}$ alkylene-, wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the $C_{1-30}$ alkylene is optionally replaced with a substituent selected from the group consisting of: D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy or any combination thereof, and symbol ## indicates the point of attachment to $X_1$. In embodiments of the present disclosure, the number of substituents is not theoretically limited in any way or automatically limited by the size of the building units.

In some embodiments of the compound of Formula (I-1), $L_1$ represents the following group: ##-$CH_2$-, ##-$(CH_2)_2$-, ##-$(CH_2)_3$-, ##-$(CH_2)_4$-, ##-$(CH_2)_5$-, ##-$(CH_2)_2$-$CF_2$-$(CH_2)_2$-, ##-$(CH_2)_6$-, ##-$(CH_2)_7$-, ##-$(CH_2)_8$-, ##-$(CH_2)_9$-, ##-$(CH_2)_{10}$-, ##-$(CH_2)_{11}$-, ##-$(CH_2)_{12}$-, ##-$(CH_2)_{13}$-, ##-$(CH_2)_{14}$-, ##-$(CH_2)_{15}$-, ##-$(CH_2)_{16}$-, ##-$(CH_2)_{17}$-, ##-$(CH_2)_{18}$-, ##-$(CH_2)_{19}$-, ##-$(CH_2)_{20}$-, ##-$(CH_2)_{21}$-, ##-$(CH_2)_{22}$-, ##-$(CH_2)_{25}$-, or ##-$(CH_2)_{30}$-;

wherein a hydrogen atom of one or more (e.g., 1-20, or 1-15, 1-10, 1-9, 1-8, 1-7, 1-6, 1-5, 1-4, 1-3 or 1-2, or 1) $CH_2$ of the group is optionally replaced with a substituent selected from the group consisting of: D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy or any combination thereof, and symbol ## indicates the point of attachment to $X_1$. In embodiments of the present disclosure, the number of substituents is not theoretically limited in any way or automatically limited by the size of the building units.

In some embodiments of the compound of Formula (I-1), $R_b$ represents $C_{1-60}$ alkyl (e.g., $C_1$-$C_{30}$ alkyl, $C_1$-$C_{29}$ alkyl, $C_1$-$C_{28}$ alkyl, $C_1$-$C_{27}$ alkyl, $C_1$-$C_{26}$ alkyl, $C_1$-$C_{25}$ alkyl, $C_1$-$C_{24}$ alkyl, $C_1$-$C_{23}$ alkyl, $C_1$-$C_{22}$ alkyl, $C_1$-$C_{21}$ alkyl, $C_1$-$C_{2O}$ alkyl, $C_1$-$C_{19}$ alkyl, $C_1$-$C_{18}$ alkyl, $C_1$-$C_{17}$ alkyl, $C_1$-$C_{16}$ alkyl, $C_1$-$C_{15}$ alkyl, $C_1$-$C_{14}$ alkyl, $C_1$-$C_{13}$ alkyl, $C_1$-$C_{12}$ alkyl, $C_1$-$C_{11}$ alkyl, $C_1$-$C_{10}$ alkyl, $C_1$-$C_9$ alkyl, $C_1$-$C_8$ alkyl, $C_1$-$C_7$ alkyl, $C_1$-$C_6$ alkyl, $C_1$-$C_5$ alkyl, $C_1$-$C_4$ alkyl, $C_1$-$C_3$ alkyl, or $C_1$-$C_2$ alkyl) optionally substituted with D or halogen.

In some embodiments of the compound of Formula (I-1), $R_b$ represents the following group: $CH_3$, $CF_3$, $CD_3$, $CH_2CH_3$, -$(CH_2)_2$-$CH_3$, -$(CH_2)_3$-$CH_3$, -$(CH_2)_4$-$CH_3$, -$(CH_2)_5$-$CH_3$, -$(CH_2)_6$-$CH_3$, - $(CH_2)_7$-$CH_3$, -$(CH_2)_8$-$CH_3$, -$(CH_2)_9$-$CH_3$, -$(CH_2)_{10}$-$CH_3$, -$(CH_2)_{11}$-$CH_3$, -$(CH_2)_{12}$-$CH_3$, -$(CH_2)_{13}$-$CH_3$, - $(CH_2)_{14}$-$CH_3$, -$(CH_2)_{15}$-$CH_3$, -$(CH_2)_{16}$-$CH_3$, -$(CH_2)_{17}$-$CH_3$, -$(CH_2)_{18}$-$CH_3$, -$(CH_2)_{19}$-$CH_3$, -$(CH_2)_{20}$-$CH_3$, -$(CH_2)_{21}$-$CH_3$, -$(CH_2)_{22}$-$CH_3$, -$(CH_2)_{23}$-$CH_3$, -$(CH_2)_{24}$-$CH_3$, -$(CH_2)_{25}$-$CH_3$, -$(CH_2)_{26}$-$CH_3$, -$(CH_2)_{27}$-$CH_3$, -$(CH_2)_{28}$-$CH_3$, or -$(CH_2)_{29}$-$CH_3$.

In some embodiments of the compound of Formula (I-1), $R_b$ represents the following formula:

$$R_{b1}\text{-}X_4\text{-}L_2\text{-} ,$$

wherein $L_2$ represents:

linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene,

$C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, $C_{1-4}$ alkyl, halogen, $C_{3-6}$cycloalkyl or any combination thereof, or optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{12}$ and/or one or more (e.g., 1-20, or 1-15, 1-10, 1-9, 1-8, 1-7, 1-6, 1-5, 1-4, 1-3 or 1-2, or 1) groups $R_{13}$ and/or any combination of one or more (e.g., 1-20, or 1-15, 1-10, 1-9, 1-8, 1-7, 1-6, 1-5, 1-4, 1-3 or 1-2, or 1) groups $R_{12}$ and $R_{13}$ inserted into its backbone carbon chain, wherein carbon-carbon bond between one or more pairs (e.g., 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, N($R_{14}$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of $C_{3-15}$cycloalkylene, 4- to 15-membered heterocyclylene, $C_{6-10}$ arylene, 5- to 15-membered heteroarylene, $C_{2-6}$ alkenylene, $C_{2-6}$ alkynylene or any combination thereof, wherein the $C_{3-15}$cycloalkylene, the 4- to 15-membered heterocyclylene, $C_{6-10}$ arylene and the 5- to 15-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH-(e.g., $C_{1-3}$ alkyl-C(O)NH-, such as $CH_3$-C(O)NH-, $CH_3CH_2$-C(O)NH-, and $CH_3CH_2CH_2$-C(O)NH-), cyano or any combination thereof, and the linear or branched $C_{2-60}$ alkylene is optionally substituted with one or more substituents selected from the group consisting of D, $C_{1-4}$ alkyl (e.g., $C_{1-3}$ alkyl, such as methyl, ethyl, propyl, isopropyl, butyl, sec-butyl or tert-butyl), halogen, $C_{3-6}$cycloalkyl or any combination thereof;

$X_4$ represents a bond, or $X_4$ represents N($R_{15}$), C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted $C_{3-20}$cycloalkylene, optionally substituted $C_{6-20}$ arylene, optionally substituted 4- to 20-membered heterocyclylene, optionally substituted 5- to 20-membered heteroarylene, triazolylene-NH-, or -NH-triazolylene, wherein $R_{15}$ represents H or $C_{1-3}$ alkyl, and the $C_{3-20}$cycloalkylene, the $C_{6-20}$ arylene, the 4- to 20-membered heterocyclylene, and the 5- to 20-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, halogen, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof; and

$R_{11}$ represents optionally substituted $C_{3-20}$cycloalkyl, optionally substituted $C_{6-20}$ aryl, optionally substituted 4- to 20-membered heterocyclyl or optionally substituted 5- to 20-membered heteroaryl, wherein the $C_{3-20}$cycloalkyl, the $C_{6-20}$ aryl, the 4- to 20-membered heterocyclyl, and the 5- to 20-membered heteroaryl are each independently optionally

substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally substituted $C_{3-15}$cycloalkyl (e.g., optionally substituted cyclopropyl, optionally substituted cyclobutyl, optionally substituted cyclopentyl or optionally substituted cyclohexyl), optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof; and the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH-(e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof. In embodiments of the present disclosure, the number of substituents is not theoretically limited in any way or automatically limited by the size of the building units.

In some embodiments of the compound of Formula (I-1), $L_2$ represents the structure of the following formula: -$C_{2-30}$ alkylene-, wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the $C_{2-30}$ alkylene is optionally replaced with a substituent selected from the group consisting of: D, $C_{1-4}$ alkyl, halogen, $C_{3-6}$cycloalkyl or any combination thereof.

In some embodiments of the compound of Formula (I-1), $L_2$ represents the following group:

$-CH_2-$, $-(CH_2)_2-$, $-(CH_2)_3-$, $-(CH_2)_4-$, $-(CH_2)_5-$, $-(CH_2)_2-CF_2-(CH_2)_2-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{15}-$, $-(CH_2)_{16}-$, $-(CH_2)_{17}-$, $-(CH_2)_{18}-$, $-(CH_2)_{19}-$, $-(CH_2)_{20}-$, $-(CH_2)_{21}-$, $-(CH_2)_{22}-$, $-(CH_2)_{25}-$, or $-(CH_2)_{30}-$;
wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the group is optionally replaced with a substituent selected from the group consisting of: D, $C_{1-4}$ alkyl, halogen, $C_{3-6}$cycloalkyl or any combination thereof.

In some embodiments of the compound of Formula (I-1), $L_2$ represents the structure of the following formula:

$$-(C(R^{a9})(R^{a10}))_{m1}-(R_{12}(C(R^{a11})(R^{a12}))_{m2})_{p1}-;$$

$$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-;$$

$$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-(R_{12}(C(R^{15})(R^{a16}))_{m4})_{p3}-;$$

$$-(C(R^{a9})(R^{a10}))_{m1}-(R_{13}(C(R^{a11})(R^{a12}))_{m2})_{p1}-;$$

$$-(C(R^{a9})(R^{a10}))_{m1}-(R_{13}(C(R^{a11})(R^{a12}))_{m2})_{p1}-(R_{13}(C(R^{a13})(R^{a14}))_{m3})_{p2}-;$$

$$-(C(R^{a9})(R^{a10}))_{m1}-(R_{13}(C(R^{a11})(R^{a12}))_{m2})_{p1}-(R_{13}(C(R^{a13})(R^{a14}))_{m3})_{p2}-(R_{13}(C(R^{15})(R^{a16}))_{m4})_{p3}-;$$

$$-(C(R^{a9})(R^{a10}))_{m1}-(R_{12}-R_{13}-(C(R^{a11})(R^{a12}))_{m2})_{p1}-;$$

$$-(C(R^{a9})(R^{a10}))_{m1}-(R_{12}(C(R^{a11})(R^{a12}))_{m2})_{p1}-(R_{13}(C(R^{a13})(R^{a14}))_{m3})_{p2}-;$$

$-(C(R^{a9})(R^{a10}))_{m1}-(R_{12}-R_{12}-(C(R^{a11})(R^{a12}))_{m2})_{p1}-$; or

$-(C(R^{a9})(R^{a10}))_{m1}-(R_{13}(C(R^{a11})(R^{a12}))_{m2})_{p1}-(R_{12}(C(R^{a13})(R^{a14}))_{m3})_{p2}-;$

wherein each group $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_{14}$ independently represents H or $C_{1-3}$ alkyl; and each group $R_{13}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-) or any combination thereof;

$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and

m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-1), $L_2$ represents the structure of the following formula:

$-(C(R^{a9})(R^{a10}))_{m1}-(O(C(R^{a11})(R^{a12}))_{m2})_{p1}-;$

$-(C(R^{a9})(R^{a10}))_{m1}-(O(C(R^{a11})(R^{a12}))_{m2})_{p1}-(O(C(R^{a13})(R^{a14})_{m3})_{p2}-;$

$-(C(R^{a9})(R^{a10}))_{m1}-(O(C(R^{a11})(R^{a12}))_{m2})_{p1}-(O(C(R^{a13})(R^{a14})_{m3})_{p2}-(O(C(R^{a15})(R^{a16})_{m4})_{p3};$

$-(C(R^{a9})(R^{a10}))_{m1}-(N(R_{g11})C(R^{a11})(R^{a12}))_{m2})_{p1}-;$

$-(C(R^{a9})(R^{a10}))_{m1}-(N(R_{g11})(C(R^{a11})(R^{a12}))_{m2})_{p1}-(N(R_{g11})(C(R^{a13})(R^{a14})_{m3})_{p2}-;$

$-(C(R^{a9})(R^{a10}))_{m1}-(N(R_{g11})(C(R^{a11})(R^{a12}))_{m2})_{p1}-(N(R_{g11})(C(R^{a13})(R^{a14})_{m3})_{p2}-(N(R_{g11})(C(R^{a15})(R^{a16})_{m4})_{p3}-;$

$-(C(R^{a9})(R^{a10}))_{m1}-(C(O)NH-(C(R^{a11})(R^{a12}))_{m2})_{p1}-;$

$-(C(R^{a9})(R^{a10}))_{m1}-(C(O)NH-(C(R^{a11})(R^{a12}))_{m2})_{p1}-(C(O)NH-(C(R^{a13})(R^{a14})_{m3})_{p2}-$

$-(C(R^{a9})(R^{a10}))_{m1}-(C(O)NH-(C(R^{a11})(R^{a12}))_{m2})_{p1}-(C(O)NH-(C(R^{a13})(R^{a14})_{m3})_{p2}-(C(O)NH-(C(R^{a15})(R^{a16})_{m4})_{p3}-;$

$-(C(R^{a9})(R^{a10}))_{m1}-(C(O)NH-(C(R^{a11})(R^{a12}))_{m2})_{p1}-(O-(C(R^{a13})(R^{a14})_{m3})_{p2}-;$

$-(C(R^{a9})(R^{a10}))_{m1}-(C(O)NH-(C(R^{a11})(R^{a12}))_{m2})-(O(C(R^{a13})(R^{a14})_{m3})_{p1}-;$

$-(C(R^{a9})(R^{a10}))_{m1}-(NHC(O)-(C(R^{a11})(R^{a12}))_{m2})_{p1}-;$

$-(C(R^{a9})(R^{a10}))_{m1}-(NHC(O)-(C(R^{a11})(R^{a12}))_{m2})-(O(C(R^{a13})(R^{a14})_{m3})_{p1}-;$

$-(C(R^{a9})(R^{a10}))_{m1}-(NHC(O)-(C(R^{a11})(R^{a12}))_{m2})_{p1}-(O(C(R^{a13})(R^{a14})_{m3})_{p2}-(O(C(R^{a15})(R^{a16})_{m4})_{p3}-;$

$-(C(R^{a9})(R^{a10}))_{m1}-(NHC(O)-(C(R^{a11})(R^{a12}))_{m2})_{p1}-(O(C(R^{a13})(R^{a14})_{m3})_{p1}-;$

$-(C(R^{a9})(R^{a10}))_{m1}-(NHC(O)NH-(C(R^{a11})(R^{a12}))_{m2}-$;

$-(C(R^{a9})(R^{a10}))_{m1}-(C(O)-(C(R^{a11})(R^{a12}))_{m2}-$;

$-(C(R^{a9})(R^{a10}))_{m1}-(C(O)-(C(R^{a11})(R^{a12}))_{m2}-$;

$-(C(R^{a9})(R^{a10}))_{m1}-S-(C(R^{a11})(R^{a12}))_{m2}-$;

$-(C(R^{a9})(R^{a10}))_{m1}-(C_{6-10}$ arylene$-(C(R^{a11})(R^{a12}))_{m2})_{p1}-$;

$-(C(R^{a9})(R^{a10}))_{m1}-(C_{6-10}$ arylene$-(C(R^{a11})(R^{a12}))_{m2})_{p1}-(C_{6-10}$ arylene$-(C(R^{a13})(R^{a14}))_{m3}-$;

$-(C(R^{a9})(R^{a10}))_{m1}-$(4- to 15-membered heterocyclylene$-(C(R^{a11})(R^{a12}))_{m2})_{p1}-$;

$-(C(R^{a9})(R^{a10}))_{m1}-$(4- to 15-membered heterocyclylene$-(C(R^{a11})(R^{a12}))_{m2})_{p1}-$(4- to 15-membered heterocyclylene$-(C(R^{a13})(R^{a14}))_{m3})_{p2}-$;

$-(C(R^{a9})(R^{a10}))_{m1}-$(5- to 15-membered heterocyclylene$-(C(R^{a11})(R^{a12}))_{m2})_{p1}-$;

$-(C(R^{a9})(R^{a10}))_{m1}-$(5- to 15-membered heterocyclylene$-(C(R^{a11})(R^{a12}))_{m2})_{p1}-$(5- to 15-membered heteroarylene$-(C(R^{a13})(R^{a14}))_{m3})_{p2}-$;

$-(C(R^{a9})(R^{a10}))_{m1}-(C_{3-15}$cycloalkylene$-(C(R^{a11})(R^{a12}))_{m2})_{p1}-$; or

$-(C(R^{a9})(R^{a10}))_{m1}-(C_{3-15}$cycloalkylene$-(C(R^{a11})(R^{a12}))_{m2})_{p1}-(C_{3-15}$cycloalkylene$-(C(R^{a13})(R^{a14}))_{m3})_{p2}-$;

wherein each $R_{g11}$ independently represents H or $C_{1-3}$ alkyl;
the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2-C_{1-4}$ alkylene, $C_{1-4}$ alkyl-NHC(O)-, $C_{1-4}$ alkyl-C(O)NH- or any combination thereof;
$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and
m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-1), $L_2$ represents the structure of the following formula: $-CH_2-O-CH_2-$, $-CH_2-O-(CH_2)_2-$, $-(CH_2)_1-O-(CH_2)_3-$, $-(CH_2)_1-O-(CH_2)_4-$, $-(CH_2)_1-O-(CH_2)_5-$, $-(CH_2)_1-O-(CH_2)_6-$, $-(CH_2)_1-O-(CH_2)_7-$, $-(CH_2)_1-O-(CH_2)_8-$, $-(CH_2)_1-O-(CH_2)_9-$, $-(CH_2)_1-O-(CH_2)_{10}-$, $-(CH_2)_2-O-(CH_2)_1-$, $-(CH_2)_2-O-(CH_2)_2-$, $-(CH_2)_2-O-(CH_2)_3-$, $-(CH_2)_2-O-(CH_2)_4-$, $-(CH_2)_2-O-(CH_2)_5-$, $-(CH_2)_2-O-(CH_2)_6-$, $-(CH_2)_2-O-(CH_2)_7-$, $-(CH_2)_2-O-(CH_2)_8-$, $-(CH_2)_2-O-(CH_2)_9-$, $-(CH_2)_2-O-(CH_2)_{10}-$, $-(CH_2)_2-O-(CH_2)_{11}-$, $-(CH_2)_2-O-(CH_2)_{12}-$, $-(CH_2)_3-O-(CH_2)_1-$, $-(CH_2)_3-O-(CH_2)_2-$, $-(CH_2)_3-O-(CH_2)_3-$, $-(CH_2)_3-O-(CH_2)_4-$, $-(CH_2)_3-O-(CH_2)_5-$, $-(CH_2)_3-O-(CH_2)_6-$, $-(CH_2)_3-O-(CH_2)_7-$, $-(CH_2)_4-O-(CH_2)_1-$, $-(CH_2)_4-O-(CH_2)_2-$, $-(CH_2)_4-O-(CH_2)_3-$, $-(CH_2)_4-O-(CH_2)_4-$, $-(CH_2)_4-O-(CH_2)_5-$, $-(CH_2)_4-O-(CH_2)_6-$, $-(CH_2)_5-O-(CH_2)_1-$, $-(CH_2)_5-O-(CH_2)_2-$, $-(CH_2)_5-O-(CH_2)_3-$, $-(CH_2)_5-O-(CH_2)_4-$, $-(CH_2)_5-O-(CH_2)_5-$, $-(CH_2)_6-O-(CH_2)_1-$, $-(CH_2)_6-O-(CH_2)_2-$, $-(CH_2)_6-O-(CH_2)_3-$, $-(CH_2)_6-O-(CH_2)_4-$, $-(CH_2)_7-O-(CH_2)_1-$, $-(CH_2)_7-O-(CH_2)_2-$, $-(CH_2)_7-O-(CH_2)_3-$, $-(CH_2)_8-O-(CH_2)_1-$, $-(CH_2)_8-O-(CH_2)_2-$, $-CH(CH_3)-O-(CH_2)_1-$, $-CH(CH_3)-O-(CH_2)_2-$, $-CH(CH_3)-O-(CH_2)_3-$, $-CH(CH_3)-O-(CH_2)_4-$, $-CH(CH_3)-O-(CH_2)_5-$, $-CH(CH_3)-O-(CH_2)_6-$, $-CH(CH_3)-O-(CH_2)_7-$, $-CH(CH_3)-O-(CH_2)_8-$, $-CH(CH_3)-O-(CH_2)_9-$, $-CH(CH_3)-O-(CH_2)_{10}-$, $-CH_2-(O(CH_2)_2)_2-$, $-CH_2-(O(CH_2)_2)_3-$, $-CH_2-(O(CH_2)_2)_4-$, $-CH_2-(O(CH_2)_2)_5-$, $-CH_2-(O(CH_2)_2)_6-$, $-CH_2-(O(CH_2)_2)_7-$, $-CH_2-(O(CH_2)_2)_8-$, $-CH_2-(O(CH_2)_2)_9-$, $-CH_2-(O(CH_2)_2)_{10}-$, $-CH_2-(O(CH_2)_2)_1-OCH_2-$, $-CH_2-(O(CH_2)_2)_2-OCH_2-$, $-CH_2-(O(CH_2)_2)_3-OCH_2-$, $-CH_2-(O(CH_2)_2)_4-OCH_2-$, $-CH_2-(O(CH_2)_2)_5-OCH_2-$, $-CH_2-(O(CH_2)_2)_6-OCH_2-$, $-CH_2-(O(CH_2)_2)_7-OCH_2-$, $-CH_2-(O(CH_2)_2)_8-OCH_2-$, $-CH_2-(O(CH_2)_2)_9-OCH_2-$, $-CH_2-(O(CH_2)_2)_{10}-OCH_2-$, $-(CH_2)_2-(O(CH_2)_2)_2-$, $-(CH_2)_2-(O(CH_2)_2)_3-$, $-(CH_2)_2-(O(CH_2)_2)_4-$, $-(CH_2)_2-(O(CH_2)_2)_5-$, $-(CH_2)_2-(O(CH_2)_2)_6-$, $-(CH_2)_2-(O(CH_2)_2)_7-$, $-(CH_2)_2-(O(CH_2)_2)_8-$, $-(CH_2)_2-(O(CH_2)_2)_9-$, $-(CH_2)_2-(O(CH_2)_2)_{10}-$, $-(CH_2)_3-(O(CH_2)_2)_2-$, $-(CH_2)_3-(O(CH_2)_2)_3-$, $-(CH_2)_3-(O(CH_2)_2)_4-$, $-(CH_2)_3-(O(CH_2)_2)_5-$, $-(CH_2)_3-(O(CH_2)_2)_6-$, $-(CH_2)_3-(O(CH_2)_2)_7-$, $-(CH_2)_3-(O(CH_2)_2)_8-$, $-(CH_2)_3-(O(CH_2)_2)_9-$, $-(CH_2)_3-(O(CH_2)_2)_{10}-$, $-(CH_2)_4-(O(CH_2)_2)_2-$, $-(CH_2)_4-(O(CH_2)_2)_3-$, $-(CH_2)_4-(O(CH_2)_2)_4-$, $-(CH_2)_4-(O(CH_2)_2)_5-$, $-(CH_2)_4-(O(CH_2)_2)_6-$,

$-(CH_2)_4-(O(CH_2)_2)_7-$, $-(CH_2)_4-(O(CH_2)_2)_8-$, $-(CH_2)_4-(O(CH_2)_2)_9-$, $-(CH_2)_4-(O(CH_2)_2)_{10}-$, $-CH_2-(O(CH_2)_3)_2-$, $-CH_2-(O(CH_2)_3)_3-$, $-CH_2-(O(CH_2)_3)_4-$, $-CH_2-(O(CH_2)_3)_5-$, $-CH_2-(O(CH_2)_3)_6-$, $-CH_2-(O(CH_2)_3)_7-$, $-CH_2-(O(CH_2)_3)_8-$, $-CH_2-(O(CH_2)_3)_9-$, $-CH_2-(O(CH_2)_3)_{10}-$, $-(CH_2)_2-(O(CH_2)_3)_2-$, $-(CH_2)_2-(O(CH_2)_3)_3-$, $-(CH_2)_2-(O(CH_2)_3)_4-$, $-(CH_2)_2-(O(CH_2)_3)_5-$, - $(CH_2)_2-(O(CH_2)_3)_6-$, $-(CH_2)_2-(O(CH_2)_3)_7-$, $-(CH_2)_2-(O(CH_2)_3)_8-$, $-(CH_2)_2-(O(CH_2)_3)_9-$, $-(CH_2)_2-(O(CH_2)_3)_{10}-$, $-(CH_2)_3-(O(CH_2)_3)_2-$, $-(CH_2)_3-(O(CH_2)_3)_3-$, $-(CH_2)_3-(O(CH_2)_3)_4-$, $-(CH_2)_3-(O(CH_2)_3)_5-$, - $(CH_2)_3-(O(CH_2)_3)_6-$, $-(CH_2)_3-(O(CH_2)_3)_7-$, $-(CH_2)_3-(O(CH_2)_3)_8-$, $-(CH_2)_3-(O(CH_2)_3)_9-$, $-(CH_2)_3-(O(CH_2)_3)_{10}-$, $-CH_2-O-(CH_2)_2-O-(CH_2)_3-$, $-CH_2-(O(CH_2)_2)_2-(O(CH_2)_3)_2-$, $-CH_2-(O(CH_2)_2)_3-(O(CH_2)_3)_3-$, $-CH_2-(O(CH_2)_2)_4-(O(CH_2)_3)_4-$, $-CH_2-(O(CH_2)_2)_5-(O(CH_2)_3)_5-$, $-CH_2-(O(CH_2)_2)_6-(O(CH_2)_3)_6-$, $-(CH_2)_2-O-(CH_2)_2-O-(CH_2)_3-$, $-(CH_2)_2-(O(CH_2)_2)_2-(O(CH_2)_3)_2-$, $-(CH_2)_2-(O(CH_2)_2)_3-(O(CH_2)_3)_3-$, $-(CH_2)_2-(O(CH_2)_2)_4-(O(CH_2)_3)_4-$, $-(CH_2)_2-(O(CH_2)_2)_5-(O(CH_2)_3)_5-$, $-(CH_2)_2-(O(CH_2)_2)_6-(O(CH_2)_3)_6-$, $-(CH_2)_3-O-(CH_2)_2-O-(CH_2)_3-$, $-(CH_2)_3-(O(CH_2)_2)_2-(O(CH_2)_3)_2-$, $-(CH_2)_3-(O(CH_2)_2)_3-(O(CH_2)_3)_3-$, $-(CH_2)_3-(O(CH_2)_2)_4-(O(CH_2)_3)_4-$, $-(CH_2)_3-(O(CH_2)_2)_5-(O(CH_2)_3)_5-$, $-(CH_2)_3-(O(CH_2)_2)_6-(O(CH_2)_3)_6-$, $-CH_2-O-(CH_2)_3-O-(CH_2)_2-$, $-CH_2-(O(CH_2)_3)_2-(O(CH_2)_2)_2-$, $-CH_2-(O(CH_2)_3)_3-(O(CH_2)_2)_3-$, $-CH_2-(O(CH_2)_3)_4-(O(CH_2)_2)_4-$, $-CH_2-(O(CH_2)_3)_5-(O(CH_2)_2)_5-$, $-CH_2-(O(CH_2)_3)_6-(O(CH_2)_2)_6-$, $-(CH_2)_2-O-(CH_2)_3-O-(CH_2)_2-$, $-(CH_2)_2-(O(CH_2)_3)_2-(O(CH_2)_2)_2-$, $-(CH_2)_2-(O(CH_2)_3)_3-(O(CH_2)_2)_3-$, $-(CH_2)_2-(O(CH_2)_3)_4-(O(CH_2)_2)_4-$, $-(CH_2)_2-(O(CH_2)_3)_5-(O(CH_2)_2)_5-$, $-(CH_2)_2-(O(CH_2)_3)_6-(O(CH_2)_2)_6-$, $-(CH_2)_3-O-(CH_2)_3-O-(CH_2)_2-$, $-(CH_2)_3-(O(CH_2)_3)_2-(O(CH_2)_2)_2-$, $-(CH_2)_3-(O(CH_2)_3)_3-(O(CH_2)_2)_3-$, $-(CH_2)_3-(O(CH_2)_3)_4-(O(CH_2)_2)_4-$, $-(CH_2)_3-(O(CH_2)_3)_5-(O(CH_2)_2)_5-$, $-(CH_2)_3-(O(CH_2)_3)_6-(O(CH_2)_2)_6-$, $-CH_2-O-(CH_2)_2-O-CH_2-$, $-(CH_2)_2-O-(CH_2)_2-O-CH_2-$, $-(CH_2)_2-(O(CH_2)_2)_2-O-(CH_2)_3-$, $-(CH_2)_2-(O(CH_2)_2)_3-O-(CH_2)_3-$, - $(CH_2)_2-(O(CH_2)_2)_4-O-(CH_2)_3-$, $-(CH_2)_5-(O(CH_2)_2)_2-O-(CH_2)_5-$, $-(CH_2)_5-(O(CH_2)_2)_2-O-(CH_2)_6-$, $-CH_2-C(O)-CH_2-$, $-CH_2-C(O)-(CH_2)_2-$, $-(CH_2)_1-C(O)-(CH_2)_3-$, $-(CH_2)_1-C(O)-(CH_2)_4-$, $-(CH_2)_1-C(O)-(CH_2)_5-$, $-(CH_2)_1-C(O)-(CH_2)_6-$, $-(CH_2)_1-C(O)-(CH_2)_7-$, $-(CH_2)_1-C(O)-(CH_2)_8-$, $-(CH_2)_1-C(O)-(CH_2)_9-$, $-(CH_2)_1-C(O)-(CH_2)_{10}-$, $-(CH_2)_2-C(O)-(CH_2)_1-$, $-(CH_2)_2-C(O)-(CH_2)_2-$, $-(CH_2)_2-C(O)-(CH_2)_3-$, $-(CH_2)_2-C(O)-(CH_2)_4-$, $-(CH_2)_2-C(O)-(CH_2)_5-$, $-(CH_2)_2-C(O)-(CH_2)_6-$, $-(CH_2)_2-C(O)-(CH_2)_7-$, $-(CH_2)_2-C(O)-(CH_2)_8-$, - $(CH_2)_2-C(O)-(CH_2)_9-$, $-(CH_2)_2-C(O)-(CH_2)_{10}-$, $-(CH_2)_2-C(O)-(CH_2)_{11}-$, $-(CH_2)_2-C(O)-(CH_2)_{12}-$, - $(CH_2)_3-C(O)-(CH_2)_1-$, $-(CH_2)_3-C(O)-(CH_2)_2-$, $-(CH_2)_3-C(O)-(CH_2)_3-$, $-(CH_2)_3-C(O)-(CH_2)_4-$, $-(CH_2)_3-C(O)-(CH_2)_5-$, $-(CH_2)_3-C(O)-(CH_2)_6-$, $-(CH_2)_3-C(O)-(CH_2)_7-$, $-(CH_2)_4-C(O)-(CH_2)_1-$, $-(CH_2)_4-C(O)-(CH_2)_2-$, $-(CH_2)_4-C(O)-(CH_2)_3-$, $-(CH_2)_4-C(O)-(CH_2)_4-$, $-(CH_2)_4-C(O)-(CH_2)_5-$, $-(CH_2)_4-C(O)-(CH_2)_6-$, - $(CH_2)_5-C(O)-(CH_2)_1-$, $-(CH_2)_5-C(O)-(CH_2)_2-$, $-(CH_2)_5-C(O)-(CH_2)_3-$, $-(CH_2)_5-C(O)-(CH_2)_4-$, $-(CH_2)_5-C(O)-(CH_2)_5-$, $-(CH_2)_6-C(O)-(CH_2)_1-$, $-(CH_2)_6-C(O)-(CH_2)_2-$, $-(CH_2)_6-C(O)-(CH_2)_3-$, $-(CH_2)_6-C(O)-(CH_2)_4-$, $-(CH_2)_7-C(O)-(CH_2)_1-$, $-(CH_2)_7-C(O)-(CH_2)_2-$, $-(CH_2)_7-C(O)-(CH_2)_3-$, $-(CH_2)_8-C(O)-(CH_2)_1-$, - $(CH_2)_8-C(O)-(CH_2)_2-$, $-CH_2-S-CH_2-$, $-CH_2-S-(CH_2)_2-$, $-(CH_2)_1-S-(CH_2)_3-$, $-(CH_2)_1-S-(CH_2)_4-$, $-(CH_2)_1-S-(CH_2)_5-$, $-(CH_2)_1-S-(CH_2)_6-$, $-(CH_2)_l-S-(CH_2)_7-$, $-(CH_2)_1-S-(CH_2)_8-$, $-(CH_2)_1-S-(CH_2)_9-$, $-(CH_2)_1-S-(CH_2)_{10}-$, $-(CH_2)_2-S-(CH_2)_1-$, $-(CH_2)_2-S-(CH_2)_2-$, $-(CH_2)_2-S-(CH_2)_3-$, $-(CH_2)_2-S-(CH_2)_4-$, $-(CH_2)_2-S-(CH_2)_5-$, $-(CH_2)_2-S-(CH_2)_6-$, $-(CH_2)_2-S-(CH_2)_7-$, $-(CH_2)_2-S-(CH_2)_8-$, $-(CH_2)_2-S-(CH_2)_9-$, $-(CH_2)_2-S-(CH_2)_{lO}-$, $-(CH_2)_2-S-(CH_2)_{11}-$, $-(CH_2)_2-S-(CH_2)_{12}-$, $-(CH_2)_3-S-(CH_2)_1-$, $-(CH_2)_3-S-(CH_2)_2-$, $-(CH_2)_3-S-(CH_2)_3-$, $-(CH_2)_3-S-(CH_2)_4-$, $-(CH_2)_3-S-(CH_2)_5-$, $-(CH_2)_3-S-(CH_2)_6-$, $-(CH_2)_3-S-(CH_2)_7-$, $-(CH_2)_4-S-(CH_2)_l-$, $-(CH_2)_4-S-(CH_2)_2-$, $-(CH_2)_4-S-(CH_2)_3-$, $-(CH_2)_4-S-(CH_2)_4-$, $-(CH_2)_4-S-(CH_2)_5-$, $-(CH_2)_4-S-(CH_2)_6-$, $-(CH_2)_5-S-(CH_2)_1-$, $-(CH_2)_5-S-(CH_2)_2-$, $-(CH_2)_5-S-(CH_2)_3-$, $-(CH_2)_5-S-(CH_2)_4-$, $-(CH_2)_5-S-(CH_2)_5-$, $-(CH_2)_6-S-(CH_2)_1-$, $-(CH_2)_6-S-(CH_2)_2-$, $-(CH_2)_6-S-(CH_2)_3-$, $-(CH_2)_6-S-(CH_2)_4-$, $-(CH_2)_7-S-(CH_2)_1-$, $-(CH_2)_7-S-(CH_2)_2-$, $-(CH_2)_7-S-(CH_2)_3-$, $-(CH_2)_8-S-(CH_2)_l-$, $-(CH_2)_8-S-(CH_2)_2-$, $-CH_2-C(S)-CH_2-$, $-CH_2-C(S)-(CH_2)_2-$, $-(CH_2)_1-C(S)-(CH_2)_3-$, $-(CH_2)_1-C(S)-(CH_2)_4-$, $-(CH_2)_1-C(S)-(CH_2)_5-$, - $(CH_2)_1-C(S)-(CH_2)_6-$, $-(CH_2)_1-C(S)-(CH_2)_7-$, $-(CH_2)_1-C(S)-(CH_2)_8-$, $-(CH_2)_1-C(S)-(CH_2)_9-$, $-(CH_2)_1-C(S)-(CH_2)_{10}-$, $-(CH_2)_2-C(S)-(CH_2)_1-$, $-(CH_2)_2-C(S)-(CH_2)_2-$, $-(CH_2)_2-C(S)-(CH_2)_3-$, $-(CH_2)_2-C(S)-(CH_2)_4-$, $-(CH_2)_2-C(S)-(CH_2)_5-$, $-(CH_2)_2-C(S)-(CH_2)_6-$, $-(CH_2)_2-C(S)-(CH_2)_7-$, $-(CH_2)_2-C(S)-(CH_2)_8-$, - $(CH_2)_2-C(S)-(CH_2)_9-$, $-(CH_2)_2-C(S)-(CH_2)_{10}-$, $-(CH_2)_2-C(S)-(CH_2)_{11}-$, $-(CH_2)_2-C(S)-(CH_2)_{12}-$, $-(CH_2)_3-C(S)-(CH_2)_1-$, $-(CH_2)_3-C(S)-(CH_2)_2-$, $-(CH_2)_3-C(S)-(CH_2)_3-$, $-(CH_2)_3-C(S)-(CH_2)_4-$, $-(CH_2)_3-C(S)-(CH_2)_5-$, $-(CH_2)_3-C(S)-(CH_2)_6-$, $-(CH_2)_3-C(S)-(CH_2)_7-$, $-(CH_2)_4-C(S)-(CH_2)_1-$, $-(CH_2)_4-C(S)-(CH_2)_2-$, - $(CH_2)_4-C(S)-(CH_2)_3-$, $-(CH_2)_4-C(S)-(CH_2)_4-$, $-(CH_2)_4-C(S)-(CH_2)_5-$, $-(CH_2)_4-C(S)-(CH_2)_6-$, $-(CH_2)_5-C(S)-(CH_2)_1-$, $-(CH_2)_5-C(S)-(CH_2)_2-$, $-(CH_2)_5-C(S)-(CH_2)_3-$, $-(CH_2)_5-C(S)-(CH_2)_4-$, $-(CH_2)_5-C(S)-(CH_2)_5-$, $-(CH_2)_6-C(S)-(CH_2)_1-$, $-(CH_2)_6-C(S)-(CH_2)_2-$, $-(CH_2)_6-C(S)-(CH_2)_3-$, $-(CH_2)_6-C(S)-(CH_2)_4-$, - $(CH_2)_7-C(S)-(CH_2)_1-$, $-(CH_2)_7-C(S)-(CH_2)_2-$, $-(CH_2)_7-C(S)-(CH_2)_3-$, $-(CH_2)_8-C(S)-(CH_2)_1-$, $-(CH_2)_8-C(S)-(CH_2)_2-$, $-CH_2-C(O)O-CH_2-$, $-CH_2-C(O)O-(CH_2)_2-$, $-(CH_2)_1-C(O)O-(CH_2)_3-$, $-(CH_2)_1-C(O)O-(CH_2)_4-$, $-(CH_2)_1-C(O)O-(CH_2)_5-$, $-(CH_2)_1-C(O)O-(CH_2)_6-$, $-(CH_2)_1-C(O)O-(CH_2)_7-$, $-(CH_2)_1-C(O)O-(CH_2)_8-$, $-(CH_2)_1-C(O) O-(CH_2)_9-$, $-(CH_2)_1-C(O)O-(CH_2)_{10}-$, $-(CH_2)_2-C(O)O-(CH_2)_1-$, $-(CH_2)_2-C(O)O-(CH_2)_2-$, $-(CH_2)_2-C(O)O-(CH_2)_3-$, $-(CH_2)_2-C(O)O-(CH_2)_4-$, $-(CH_2)_2-C(O)O-(CH_2)_5-$, $-(CH_2)_2-C(O)O-(CH_2)_6-$, $-(CH_2)_2-C(O)O-(CH_2)_7-$, $-(CH_2)_2-C(O) O-(CH_2)_8-$, $-(CH_2)_2-C(O)O-(CH_2)_9-$, $-(CH_2)_2-C(O)O-(CH_2)_{10}-$, $-(CH_2)_2-C(O)O-(CH_2)_{11}-$, $-(CH_2)_2-C(O)O-(CH_2)_{12}-$, $-(CH_2)_3-C(O)O-(CH_2)_1-$, $-(CH_2)_3-C(O)O-(CH_2)_2-$, $-(CH_2)_3-C(O)O-(CH_2)_3-$, $-(CH_2)_3-C(O)O-(CH_2)_4-$, $-(CH_2)_3-C(O) O-(CH_2)_5-$, $-(CH_2)_3-C(O)O-(CH_2)_6-$, $-(CH_2)_3-C(O)O-(CH_2)_7-$, $-(CH_2)_4-C(O)O-(CH_2)_1-$, $-(CH_2)_4-C(O)O-(CH_2)_2-$, $-(CH_2)_4-C(O)O-(CH_2)_3-$, $-(CH_2)_4-C(O)O-(CH_2)_4-$, $-(CH_2)_4-C(O)O-(CH_2)_5-$, $-(CH_2)_4-C(O)O-(CH_2)_6-$, $-(CH_2)_5-C(O) O-(CH_2)_1-$, $-(CH_2)_5-C(O)O-(CH_2)_2-$, $-(CH_2)_5-C(O)O-(CH_2)_3-$, $-(CH_2)_5-C(O)O-(CH_2)_4-$, $-(CH_2)_5-C(O)O-(CH_2)_5-$, $-(CH_2)_6-C(O)O-(CH_2)_1-$, $-(CH_2)_6-C(O)O-(CH_2)_2-$, $-(CH_2)_6-C(O)O-(CH_2)_3-$, $-(CH_2)_6-C(O)O-(CH_2)_4-$, $-(CH_2)_7-C(O) O-(CH_2)_1-$, $-(CH_2)_7-C(O)O-(CH_2)_2-$, $-(CH_2)_7-C(O)O-(CH_2)_3-$, $-(CH_2)_8-C(O)O-(CH_2)_1-$, $-(CH_2)_8-C(O)O-(CH_2)_2-$, $-CH_2-O-$

$C(O)-CH_2-$, $-CH_2-OC(O)-(CH_2)_2-$, $-(CH_2)_1-OC(O)-(CH_2)_3-$, $-(CH_2)_1-OC(O)-(CH_2)_4-$, $-(CH_2)_1-OC(O)-(CH_2)_5-$, $-(CH_2)_1-OC(O)-(CH_2)_6-$, $-(CH_2)_1-OC(O)-(CH_2)_7-$, $-(CH_2)_1-OC(O)-(CH_2)_8-$, $-(CH_2)_1-OC(O)-(CH_2)_9-$, $-(CH_2)_1-OC(O)-(CH_2)_{10}-$, $-(CH_2)_2-OC(O)-(CH_2)_1-$, $-(CH_2)_2-OC(O)-(CH_2)_2-$, $-(CH_2)_2-OC(O)-(CH_2)_3-$, $-(CH_2)_2-OC(O)-(CH_2)_4-$, $-(CH_2)_2-OC(O)-(CH_2)_5-$, $-(CH_2)_2-OC(O)-(CH_2)_6-$, $-(CH_2)_2-OC(O)-(CH_2)_7-$, $-(CH_2)_2-OC(O)-(CH_2)_8-$, $-(CH_2)_2-OC(O)-(CH_2)_9-$, $-(CH_2)_2-OC(O)-(CH_2)_{10}-$, $-(CH_2)_2-OC(O)-(CH_2)_{11}-$, $-(CH_2)_2-OC(O)-(CH_2)_{12}-$, $-(CH_2)_3-OC(O)-(CH_2)_1-$, $-(CH_2)_3-OC(O)-(CH_2)_2-$, $-(CH_2)_3-OC(O)-(CH_2)_3-$, $-(CH_2)_3-OC(O)-(CH_2)_4-$, $-(CH_2)_3-OC(O)-(CH_2)_5-$, $-(CH_2)_3-OC(O)-(CH_2)_6-$, $-(CH_2)_3-OC(O)-(CH_2)_7-$, $-(CH_2)_4-OC(O)-(CH_2)_1-$, $-(CH_2)_4-OC(O)-(CH_2)_2-$, $-(CH_2)_4-OC(O)-(CH_2)_3-$, $-(CH_2)_4-OC(O)-(CH_2)_4-$, $-(CH_2)_4-OC(O)-(CH_2)_5-$, $-(CH_2)_4-OC(O)-(CH_2)_6-$, $-(CH_2)_5-OC(O)-(CH_2)_1-$, $-(CH_2)_5-OC(O)-(CH_2)_2-$, $-(CH_2)_5-OC(O)-(CH_2)_3-$, $-(CH_2)_5-OC(O)-(CH_2)_4-$, $-(CH_2)_5-OC(O)-(CH_2)_5-$, $-(CH_2)_6-OC(O)-(CH_2)_1-$, $-(CH_2)_6-OC(O)-(CH_2)_2-$, $-(CH_2)_6-OC(O)-(CH_2)_3-$, $-(CH_2)_6-OC(O)-(CH_2)_4-$, $-(CH_2)_7-OC(O)-(CH_2)_1-$, $-(CH_2)_7-OC(O)-(CH_2)_2-$, $-(CH_2)_7-OC(O)-(CH_2)_3-$, $-(CH_2)_8-OC(O)-(CH_2)_1-$, $-(CH_2)_8-OC(O)-(CH_2)_2-$, $-(CH_2)_1-N(R_{g11})-(CH_2)_1-$, $-(CH_Z)_1-N(R_{g11})-(CH_2)_2-$, $-(CH_2)_1-N(R_{g11})-(CH_2)_3-$, $-(CH_2)_1-N(R_{g11})-(CH_2)_4-$, $-(CH_2)_1-N(R_{g11})-(CH_2)_5-$, $-(CH_2)_1-N(R_{g11})-(CH_2)_6-$, $-(CH_2)_2-N(R_{g11})-(CH_2)_7-$, $-(CH_2)_1-N(R_{g11})-(CH_2)_8-$, $-(CH_2)_1-N(R_{g11})-(CH_2)_9-$, $-(CH_2)_1-N(R_{g11})-(CH_2)_{10}-$, $-(CH_2)_2-N(R_{g11})-(CH_2)_1-$, $-(CH_2)_2-N(R_{g11})-(CH_2)_2-$, $-(CH_2)_2-N(R_{g11})-(CH_2)_3-$, $-(CH_2)_2-N(R_{g11})-(CH_2)_4-$, $-(CH_2)_2-N(R_{g11})-(CH_2)_5-$, $-(CH_2)_2-N(R_{g11})-(CH_2)_6-$, $-(CH_2)_2-N(R_{g11})-(CH_2)_7-$, $-(CH_2)_2-N(R_{g11})-(CH_2)_8-$, $-(CH_2)_2-N(R_{g11})-(CH_2)_9-$, $-(CH_2)_2-N(R_{g11}-(CH_2)_{10}-$, $-(CH_2)_2-N(R_{g11})-(CH_2)_{11}-$, $-(CH_a)_a-N(R_{g11})-(CH_2)_{12}-$, $-(CH_2)_3-N(R_{g11})-(CH_2)_1-$, $-(CH_2)_3-N(R_{g11})-(CH_2)_2-$, $-(CH_2)_3-N(R_{g11})-(CH_2)_3-$, $-(CH_2)_4-N(R_{g11})-(CH_2)_1-$, $-(CH_2)_4-N(R_{g11})-(CH_2)_2-$, $-(CH_2)_4-N(R_{g11})-(CH_2)_3-$, $-(CH_2)_4-N(R_{g11})-(CH_2)_4-$, $-(CH_2)_5-N(R_{g11})-(CH_2)_1-$, $-(CH_2)_5-N(R_{g11})-(CH_2)_2-$, $-(CH_2)_5-N(R_{g11})-(CH_2)_3-$, $-(CH_2)_5-N(R_{g11})-(CH_2)_4-$, $-(CH_2)_5-N(R_{g11})-(CH_2)_5-$, $-(CH_2)_6-N(R_{g11})-(CH_2)_1-$, $-(CH_2)_6-N(R_{g11})-(CH_2)_2-$, $-(CH_2)_6-N(R_{g11})-(CH_2)_3-$, $-(CH_2)_7-N(R_{g11})-(CH_2)_1-$, $-(CH_2)_7-N(R_{g11})-(CH_2)_2-$, $-(CH_2)_7-N(R_{g11})-(CH_2)_3-$, $-(CH_2)_8-N(R_{g11})-(CH_2)_1-$, $-(CH_2)_8-NR_{g11}-(CH_2)_2-$, $-(CH_2)_8-N(R_{g11})-(CH_2)_3-$, $-CH(CH_3)-N(R_{g11})-(CH_2)_1-$, $-CH(CH_3)-N(R_{g11})-(CH_2)_2-$, $-CH(CH_3)-N(R_{g11})-(CH_2)_3-$, $-CH(CH_3)-N(R_{g11})-(CH_2)_4-$, $-CH(CH_3)-N(R_{g11})-(CH_2)_5-$, $-CH(CH_3)-N(R_{g11})-(CH_2)_6-$, $-CH(CH_3)-N(R_{g11})-(CH_2)_7-$, $-CH(CH_3)-N(R_{g11})-(CH_2)_8-$, $-CH(CH_3)-N(R_{g11})-(CH_2)_9-$, $-CH(CH_3)-N(R_{g11})-(CH_2)_{10}-$, $-CH_2C(O)NHCH_2-$, $-(CH_2)_2C(O)NH(CH_2)_2-$, $-(CH_2)_2C(O)NH(CH_2)_3-$, $-(CH_2)_2C(O)NH(CH_2)_4-$, $-(CH_2)_2C(O)NH(CH_2)_5-$, $-(CH_2)_3C(O)NH(CH_2)_3-$, $-(CH_2)_3C(O)NH(CH_2)_4-$, $-(CH_2)_4C(O)NH(CH_2)_4-$, $-(CH_2)_5C(O)NH(CH_2)_5-$, $-(CH_2)_6C(O)NH(CH_2)_7-$, $-(CH_2)_6C(O)NH(CH_2)_6-$, $-(CH_2)_7C(O)NH(CH_2)_7-$, $-(CH_2)_8C(O)NH(CH_2)_8$, $-(CH_2)_9C(O)NH(CH_2)_9-$, $-(CH_2)_{lo}-C(O)NH(CH_2)_{10}-$, $-(CH_2)_2C(O)NH(CH_2)_2-O-(CH_2)_2-$, $-CH_2NHC(O)CH_2-$, $-(CH_2)_2NHC(O)(CH_2)_2-$, $-(CH_2)_2NHC(O)(CH_2)_3-$, $-(CH_2)_2NHC(O)(CH_2)_4-$, $-(CH_2)_2NHC(O)(CH_2)_5-$, $-(CH_2)_3NHC(O)(CH_2)_3-$, $-(CH_2)_3NHC(O)(CH_2)_4-$, $-(CH_2)_4NHC(O)(CH_2)_4-$, $-(CH_2)_5NHC(O)(CH_2)_5-$, $-(CH_2)_6NHC(O)(CH_2)_7-$, $-(CH_2)_6NHC(O)(CH_2)_6-$, $-(CH_2)_7NHC(O)(CH_2)_7-$, $-(CH_2)_8NHC(O)(CH_2)_8$, $-(CH_2)_9NHC(O)(CH_2)_9-$, $-(CH_2)_{10}NHC(O)(CH_2)_{10}-$, $-(CH_2)_4NHC(O)(CH_2)_8-$, $-(CH_2)_2NHC(O)(CH_2)_2-O-(CH_2)_2-$, $-(CH_2)_4NHC(O)CH_2-$, $-CH_2-$piperidinylene$-CH_2-$, $-CH_2-$piperidinylene$-(CH_2)_2-$, $-CH_2-$piperidinylene$-(CH_2)_3-$, $-CH_2-$piperidinylene$-(CH_2)_4-$, $-CH_2-$piperidinylene$-(CH_2)_5-$, $-CH_2-$piperidinylene$-(CH_2)_6-$, $-CH_2-$piperidinylene$-(CH_2)_7-$, $-CH_2-$piperidinylene$-(CH_2)_8-$, $-(CH_2)_2-$piperidinylene$-(CH_2)_1-$, $-(CH_2)_2-$piperidinylene$-(CH_2)_2-$, $-(CH_2)_2-$piperidinylene$-(CH_2)_3-$, $-(CH_2)_2-$piperidinylene$-(CH_2)_4-$, $-(CH_2)_2-$piperidinylene$-(CH_2)_5-$, $-(CH_2)_2-$piperidinylene$-(CH_2)_6-$, $-(CH_2)_2-$piperidinylene$-(CH_2)_7-$, $-(CH_2)_2-$piperidinylene$-(CH_2)_8-$, $-(CH_2)_3-$piperidinylene$-CH_2-$, $-(CH_2)_3-$piperidinylene$-(CH_2)_2-$, $-(CH_2)_3-$piperidinylene$-(CH_2)_3-$, $-(CH_2)_3-$piperidinylene$-(CH_2)_4-$, $-(CH_2)_3-$piperidinylene$-(CH_2)_5-$, $-(CH_2)_3-$piperidinylene$-(CH_2)_6-$, $-(CH_2)_3-$piperidinylene$-(CH_2)_7-$, $-(CH_2)_3-$piperidinylene$-(CH_2)_8-$, $-(CH_2)_4-$piperidinylene$-CH_2-$, $-(CH_2)_4-$piperidinylene$-(CH_2)_2-$, $-(CH_2)_4-$piperidinylene$-(CH_2)_3-$, $-(CH_2)_4-$piperidinylene$-(CH_2)_4-$, $-(CH_2)_4-$piperidinylene$-(CH_2)_5-$, $-(CH_2)_4-$piperidinylene$-(CH_2)_6-$, $-(CH_2)_4-$piperidinylene$-(CH_2)_7-$, $-(CH_2)_4-$piperidinylene$-(CH_2)_8-$, $-(CH_2)_5-$piperidinylene$-(CH_2)_1-$, $-(CH_2)_5-$piperidinylene$-(CH_2)_2-$, $-(CH_2)_5-$piperidinylene$-(CH_2)_3-$, $-(CH_2)_5-$piperidinylene$-(CH_2)_4-$, $-(CH_2)_5-$piperidinylene$-(CH_2)_5-$, $-(CH_2)_5-$piperidinylene$-(CH_2)_6-$, $-(CH_2)_5-$piperidinylene$-(CH_2)_7-$, $-(CH_2)_5-$piperidinylene$-(CH_2)_8-$, $-(CH_2)_6-$piperidinylene$-(CH_2)_1-$, $-(CH_2)_6-$piperidinylene$-(CH_2)_2-$, $-(CH_2)_6-$piperidinylene$-(CH_2)_3-$, $-(CH_2)_6-$piperidinylene$-(CH_2)_4-$, $-(CH_2)_6-$piperidinylene$-(CH_2)_5-$, $-(CH_2)_6-$piperidinylene$-(CH_2)_6-$, $-(CH_2)_6-$piperidinylene$-(CH_2)_7-$, $-(CH_2)_6-$piperidinylene$-(CH_2)_8-$, $-(CH_2)_7-$piperidinylene$-(CH_2)_1-$, $-(CH_2)_7-$piperidinylene$-(CH_2)_2-$, $-(CH_2)_7-$piperidinylene$-(CH_2)_3-$, $-(CH_2)_7-$piperidinylene$-(CH_2)_4-$, $-(CH_2)_7-$piperidinylene$-(CH_2)_8-$, $-(CH_2)_8-$piperidinylene$-CH_2-$, $-(CH_2)_8-$piperidinylene$-(CH_2)_2-$, $-(CH_2)_8-$piperidinylene$-(CH_2)_3-$, $-(CH_2)_8-$piperidinylene$-(CH_2)_4-$, $-(CH_2)_8-$piperidinylene$-(CH_2)_5-$, $-(CH_2)_8-$piperidinylene$-(CH_2)_6-$, $-(CH_2)_8-$piperidinylene$-(CH_2)_7-$, $-(CH_2)_8-$piperidinylene$-(CH_2)_8-$, $-CH_2-$piperazinylene$-CH_2-$, $-CH_2-$piperazinylene$-(CH_2)_2-$, $-CH_2-$piperazinylene$-(CH_2)_3-$, $-CH_2-$piperazinylene$-(CH_2)_4-$, $-CH_2-$piperazinylene$-(CH_2)_5-$, $-CH_2-$piperazinylene$-(CH_2)_6-$, $-CH_2-$piperazinylene$-(CH_2)_7-$, $-CH_2-$piperazinylene$-(CH_2)_8-$, $-(CH_2)_2-$piperazinylene$-(CH_2)_1-$, $-(CH_2)_2-$piperazinylene$-(CH_2)_2-$, $-(CH_2)_2-$piperazinylene$-(CH_2)_3-$, $-(CH_2)_2-$piperazinylene$-(CH_2)_4-$, $-(CH_2)_2-$piperazinylene$-(CH_2)_5-$, $-(CH_2)_2-$piperazinylene$-(CH_2)_6-$, $-(CH_2)_2-$piperazinylene$-(CH_2)_7-$, $-(CH_2)_2-$piperazinylene$-(CH_2)_8-$, $-(CH_2)_3-$piperazinylene$-CH_2-$, $-(CH_2)_3-$piperazinylene$-(CH_2)_2-$, $-(CH_2)_3-$piperazinylene$-(CH_2)_3-$, $-(CH_2)_3-$piperazinylene$-(CH_2)_4-$, $-(CH_2)_3-$piperazinylene$-(CH_2)_5-$, $-(CH_2)_3-$piperazinylene$-(CH_2)_6-$, $-(CH_2)_3-$piperazinylene$-(CH_2)_7-$, $-(CH_2)_3-$piperazinylene$-(CH_2)_8-$, $-(CH_2)_4-$piperazinylene$-CH_2-$, $-(CH_2)_4-$piperazinylene$-(CH_2)_2-$, $-(CH_2)_4-$piperazinylene$-(CH_2)_3-$, $-(CH_2)_4-$piperazinylene$-(CH_2)_4-$, $-(CH_2)_4-$piperazinylene$-(CH_2)_5-$, $-(CH_2)_4-$piperazinylene$-(CH_2)_6-$, $-(CH_2)_4-$piperazinylene$-(CH_2)_7-$, $-(CH_2)_4-$piperazinylene$-(CH_2)_8-$, $-(CH_2)_5-$piperazinylene$-(CH_2)_1-$, $-(CH_2)_5-$piperazinylene$-(CH_2)_2-$, $-(CH_2)_5-$piperazinylene$-(CH_2)_3-$, $-(CH_2)_5-$piperazinyle-

ne-$(CH_2)_4$-, -$(CH_2)_5$-piperazinylene-$(CH_2)_5$-, -$(CH_2)_5$-piperazinylene-$(CH_2)_6$-, -$(CH_2)_5$-piperazinylene-$(CH_2)_7$-, -$(CH_2)_5$-piperazinylene-$(CH_2)_8$-, -$(CH_2)_6$-piperazinylene-$(CH_2)$i-, -$(CH_2)_6$-piperazinylene-$(CH_2)_2$-, -$(CH_2)_6$-piperazinylene-$(CH_2)_3$-, -$(CH_2)_6$-piperazinylene-$(CH_2)_4$-, -$(CH_2)_6$-piperazinylene-$(CH_2)_5$-, -$(CH_2)_6$-piperazinylene-$(CH_2)_6$-, -$(CH_2)_6$-piperazinylene-$(CH_2)_7$-, -$(CH_2)_6$-piperazinylene-$(CH_2)_8$-, -$(CH_2)_7$-piperazinylene-$(CH_2)_1$-, -$(CH_2)_7$-piperazinylene-$(CH_2)_2$-, -$(CH_2)_7$-piperazinylene-$(CH_2)_3$-, -$(CH_2)_7$-piperazinylene-$(CH_2)_4$-, -$(CH_2)_7$-piperazinylene-$(CH_2)_8$-, -$(CH_2)_8$-piperazinylene-$CH_2$-, -$(CH_2)_8$-piperazinylene-$(CH_2)_2$-, -$(CH_2)_8$-piperazinylene-$(CH_2)_3$-, -$(CH_2)_8$-piperazinylene-$(CH_2)_4$-, -$(CH_2)_8$-piperazinylene-$(CH_2)_5$-, -$(CH_2)_8$-piperazinylene-$(CH_2)_6$-, -$(CH_2)_8$-piperazinylene-$(CH_2)_7$-, -$(CH_2)_8$-piperazinylene-$(CH_2)_8$-, -$CH_2$-propylene-$CH_2$-, -$CH_2$-propylene-$(CH_2)_2$-, -$CH_2$-propylene-$(CH_2)_3$-, -$CH_2$-propylene-$(CH_2)_4$-, -$CH_2$-propylene-$(CH_2)_5$-, -$CH_2$-propylene-$(CH_2)_6$-, -$CH_2$-propylene-$(CH_2)_7$-, -$CH_2$-propylene-$(CH_2)_8$-, -$(CH_2)_2$-propylene-$(CH_2)_1$-, -$(CH_2)_2$-propylene-$(CH_2)_2$-, -$(CH_2)_2$-propylene-$(CH_2)_3$-, -$(CH_2)_2$-propylene-$(CH_2)_4$-, -$(CH_2)_2$-propylene-$(CH_2)_5$-, -$(CH_2)_2$-propylene-$(CH_2)_6$-, -$(CH_2)_2$-propylene-$(CH_2)_7$-, -$(CH_2)_2$-propylene-$(CH_2)_8$-, -$(CH_2)_3$-propylene-$CH_2$-, -$(CH_2)_3$-propylene-$(CH_2)_2$-, -$(CH_2)_3$-propylene-$(CH_2)_3$-, -$(CH_2)_3$-propylene-$(CH_2)_4$-, - $(CH_2)_3$-propylene-$(CH_2)_5$-, -$(CH_2)_3$-propylene-$(CH_2)_6$-, -$(CH_2)_3$-propylene-$(CH_2)_7$-, -$(CH_2)_3$-propylene-$(CH_2)_8$-, -$(CH_2)_4$-propylene-$CH_2$-, -$(CH_2)_4$-propylene-$(CH_2)_2$-, -$(CH_2)_4$-propylene-$(CH_2)_3$-, -$(CH_2)_4$-propylene-$(CH_2)_4$-, -$(CH_2)_4$-propylene-$(CH_2)_5$-, -$(CH_2)_4$-propylene-$(CH_2)_6$-, -$(CH_2)_4$-propylene-$(CH_2)_7$-, -$(CH_2)_4$-propylene-$(CH_2)_8$-, -$(CH_2)_5$-propylene-$(CH_2)_1$-, -$(CH_2)_5$-propylene-$(CH_2)_2$-, -$(CH_2)_5$-propylene-$(CH_2)_3$-, -$(CH_2)_5$-propylene-$(CH_2)_4$-, -$(CH_2)_5$-propylene-$(CH_2)_5$-, -$(CH_2)_5$-propylene-$(CH_2)_6$-, -$(CH_2)_5$-propylene-$(CH_2)_7$-, -$(CH_2)_5$-propylene-$(CH_2)_8$-, -$(CH_2)_6$-propylene-$(CH_2)_1$-, -$(CH_2)_6$-propylene-$(CH_2)_2$-, -$(CH_2)_6$-propylene-$(CH_2)_3$-, -$(CH_2)_6$-propylene-$(CH_2)_4$-, -$(CH_2)_6$-propylene-$(CH_2)_5$-, -$(CH_2)_6$-propylene-$(CH_2)_6$-, -$(CH_2)_6$-propylene-$(CH_2)_7$-, -$(CH_2)_6$-propylene-$(CH_2)_8$-, -$(CH_2)_7$-propylene-$(CH_2)_1$-, -$(CH_2)_7$-propylene-$(CH_2)_2$-, -$(CH_2)_7$-propylene-$(CH_2)_3$-, -$(CH_2)_7$-propylene-$(CH_2)_4$-, -$(CH_2)_7$-propylene-$(CH_2)_8$-, -$(CH_2)_8$-propylene-$CH_2$-, -$(CH_2)_8$-propylene-$(CH_2)_2$-, -$(CH_2)_8$-propylene-$(CH_2)_3$-, -$(CH_2)_8$-propylene-$(CH_2)_4$-, -$(CH_2)_8$-propylene-$(CH_2)_5$-, -$(CH_2)_8$-propylene-$(CH_2)_6$-, -$(CH_2)_8$-propylene-$(CH_2)_7$-, -$(CH_2)_8$-propylene-$(CH_2)_8$-, -$CH_2$-diazacycloheptanylene-$CH_2$-, -$CH_2$-diazacycloheptanylene-$(CH_2)_2$-, -$CH_2$-diazacycloheptanylene-$(CH_2)_3$-, -$CH_2$-diazacycloheptanylene-$(CH_2)_4$-, -$CH_2$-diazacycloheptanylene-$(CH_2)_5$-, -$CH_2$-diazacycloheptanylene-$(CH_2)_6$-, -$CH_2$-diazacycloheptanylene-$(CH_2)_7$-, -$CH_2$-diazacycloheptanylene-$(CH_2)_8$-, -$(CH_2)_2$-diazacycloheptanylene-$(CH_2)_1$-, -$(CH_2)_2$-diazacycloheptanylene-$(CH_2)_2$-, -$(CH_2)_2$-diazacycloheptanylene-$(CH_2)_3$-, -$(CH_2)_2$-diazacycloheptanylene-$(CH_2)_4$-, -$(CH_2)_2$-diazacycloheptanylene-$(CH_2)_5$-, -$(CH_2)_2$-diazacycloheptanylene-$(CH_2)_6$-, -$(CH_2)_2$-diazacycloheptanylene-$(CH_2)_7$-, -$(CH_2)_2$-diazacycloheptanylene-$(CH_2)_8$-, -$(CH_2)_3$-diazacycloheptanylene-$CH_2$-, -$(CH_2)_3$-diazacycloheptanylene-$(CH_2)_2$-, -$(CH_2)_3$-diazacycloheptanylene-$(CH_2)_3$-, -$(CH_2)_3$-diazacycloheptanylene-$(CH_2)_4$-, -$(CH_2)_3$-diazacycloheptanylene-$(CH_2)_5$-, -$(CH_2)_3$-diazacycloheptanylene-$(CH_2)_6$-, -$(CH_2)_3$-diazacycloheptanylene-$(CH_2)_7$-, -$(CH_2)_3$-diazacycloheptanylene-$(CH_2)_8$-, -$(CH_2)_4$-diazacycloheptanylene-$CH_2$-, -$(CH_2)_4$-diazacycloheptanylene-$(CH_2)_2$-, -$(CH_2)_4$-diazacycloheptanylene-$(CH_2)_3$-, -$(CH_2)_4$-diazacycloheptanylene-$(CH_2)_4$-, -$(CH_2)_4$-diazacycloheptanylene-$(CH_2)_5$-, -$(CH_2)_4$-diazacycloheptanylene-$(CH_2)_6$-, -$(CH_2)_4$-diazacycloheptanylene-$(CH_2)_7$-, -$(CH_2)_4$-diazacycloheptanylene-$(CH_2)_8$-, -$(CH_2)_5$-diazacycloheptanylene-$(CH_2)_1$-, -$(CH_2)_5$-diazacycloheptanylene-$(CH_2)_2$-, -$(CH_2)_5$-diazacycloheptanylene-$(CH_2)_3$-, -$(CH_2)_5$-diazacycloheptanylene-$(CH_2)_4$-, -$(CH_2)_5$-diazacycloheptanylene-$(CH_2)_5$-, -$(CH_2)_5$-diazacycloheptanylene-$(CH_2)_6$-, -$(CH_2)_5$-diazacycloheptanylene-$(CH_2)_7$-, -$(CH_2)_5$-diazacycloheptanylene-$(CH_2)_8$-, -$(CH_2)_6$-diazacycloheptanylene-$(CH_2)_1$-, -$(CH_2)_6$-diazacycloheptanylene-$(CH_2)_2$-, -$(CH_2)_6$-diazacycloheptanylene-$(CH_2)_3$-, -$(CH_2)_6$-diazacycloheptanylene-$(CH_2)_4$-, -$(CH_2)_6$-diazacycloheptanylene-$(CH_2)_5$-, -$(CH_2)_6$-diazacycloheptanylene-$(CH_2)_6$-, -$(CH_2)_6$-diazacycloheptanylene-$(CH_2)_7$-, -$(CH_2)_6$-diazacycloheptanylene-$(CH_2)_8$-, -$(CH_2)_7$-diazacycloheptanylene-$(CH_2)_1$-, -$(CH_2)_7$-diazacycloheptanylene-$(CH_2)_2$-, -$(CH_2)_7$-diazacycloheptanylene-$(CH_2)_3$-, -$(CH_2)_7$-diazacycloheptanylene-$(CH_2)_4$-, -$(CH_2)_7$-diazacycloheptanylene-$(CH_2)_8$-, -$(CH_2)_8$-diazacycloheptanylene-$CH_2$-, -$(CH_2)_8$-diazacycloheptanylene-$(CH_2)_2$-, -$(CH_2)_8$-diazacycloheptanylene-$(CH_2)_3$-, -$(CH_2)_8$-diazacycloheptanylene-$(CH_2)_4$-, -$(CH_2)_8$-diazacycloheptanylene-$(CH_2)_5$-, -$(CH_2)_8$-diazacycloheptanylene-$(CH_2)_6$-, -$(CH_2)_8$-diazacycloheptanylene-$(CH_2)_7$-, -$(CH_2)_8$-diazacycloheptanylene-$(CH_2)_8$-, -$CH_2$-diazabicyclo[2.2.1]heptanylene-$CH_2$-, -$CH_2$-diazabicyclo[2.2.1]heptanylene-$(CH_2)_2$-, -$CH_2$-diazabicyclo[2.2.1]heptanylene-$(CH_2)_3$-, -$CH_2$-diazabicyclo[2.2.1]heptanylene-$(CH_2)_4$-, -$CH_2$-diazabicyclo[2.2.1]heptanylene-$(CH_2)_5$-, -$CH_2$-diazabicyclo[2.2.1]heptanylene-$(CH_2)_6$-, -$CH_2$-diazabicyclo[2.2.1]heptanylene-$(CH_2)_7$-, -$CH_2$-diazabicyclo[2.2.1]heptanylene-$(CH_2)_8$-, -$(CH_2)_2$-diazabicyclo[2.2.1]heptanylene-$(CH_2)_1$-, -$(CH_2)_2$-diazabicyclo[2.2.1]heptanylene-$(CH_2)_2$-, -$(CH_2)_2$-diazabicyclo[2.2.1]heptanylene-$(CH_2)_3$-, -$(CH_2)_2$-diazabicyclo[2.2.1]heptanylene-$(CH_2)_4$-, -$(CH_2)_2$-diazabicyclo[2.2.1]heptanylene-$(CH_2)_5$-, -$(CH_2)_2$-diazabicyclo[2.2.1]heptanylene-$(CH_2)_6$-, -$(CH_2)_2$-diazabicyclo[2.2.1]heptanylene-$(CH_2)_7$-, -$(CH_2)_2$-diazabicyclo[2.2.1]heptanylene-$(CH_2)_8$-, -$(CH_2)_3$-diazabicyclo[2.2.1]heptanylene-$CH_2$-, -$(CH_2)_3$-diazabicyclo[2.2.1]heptanylene-$(CH_2)_2$-, -$(CH_2)_3$-diazabicyclo[2.2.1]heptanylene-$(CH_2)_3$-, -$(CH_2)_3$-diazabicyclo[2.2.1]heptanylene-$(CH_2)_4$-, -$(CH_2)_3$-diazabicyclo[2.2.1]heptanylene-$(CH_2)_5$-, -$(CH_2)_3$-diazabicyclo[2.2.1]heptanylene-$(CH_2)_6$-, -$(CH_2)_3$-diazabicyclo[2.2.1]heptanylene-$(CH_2)_7$-, -$(CH_2)_3$-diazabicyclo[2.2.1]heptanylene-$(CH_2)_8$-, -$(CH_2)_4$-diazabicyclo[2.2.1]heptanylene-$CH_2$-, -$(CH_2)_4$-diazabicyclo[2.2.1]heptanylene-$(CH_2)_2$-, -$(CH_2)_4$-diazabicyclo[2.2.1]heptanylene-$(CH_2)_3$-, -$(CH_2)_4$-diazabicyclo[2.2.1]heptanylene-$(CH_2)_4$-, -$(CH_2)_4$-diazabicyclo[2.2.1]heptanylene-$(CH_2)_5$-, -$(CH_2)_4$-diazabicyclo[2.2.1]heptanylene-$(CH_2)_6$-, -$(CH_2)_4$-diazabicyclo[2.2.1]heptanylene-$(CH_2)_7$-, -$(CH_2)_4$-diazabicyclo[2.2.1]heptanylene-$(CH_2)_8$-, -$(CH_2)_5$-diazabicyclo[2.2.1]heptanylene-$(CH_2)_1$-, -$(CH_2)_5$-diazabicyclo[2.2.1]heptanylene-$(CH_2)_2$-, -$(CH_2)_5$-diazabicyclo[2.2.1]heptanylene-$(CH_2)_3$-, -$(CH_2)_5$-

diazabicyclo[2.2.1]heptanylene-$(CH_2)_4$-, -$(CH_2)_5$-diazabicyclo[2.2.1]heptanylene-$(CH_2)_5$-, -$(CH_2)_5$-diazabicyclo[2.2.1]heptanylene-$(CH_2)_6$-, -$(CH_2)_5$-diazabicyclo[2.2.1]heptanylene-$(CH_2)_7$-, -$(CH_2)_5$-diazabicyclo[2.2.1]heptanylene-$(CH_2)_8$-, -$(CH_2)_6$-diazabicyclo[2.2.1]heptanylene-$(CH_2)_i$-, -$(CH_2)_6$-diazabicyclo[2.2.1]heptanylene-$(CH_2)_2$-, -$(CH_2)_6$-diazabicyclo[2.2.1]heptanylene-$(CH_2)_3$-, -$(CH_2)_6$-diazabicyclo[2.2.1]heptanylene-$(CH_2)_4$-, -$(CH_2)_6$-diazabicyclo[2.2.1]heptanylene-$(CH_2)_5$-, -$(CH_2)_6$-diazabicyclo[2.2.1]heptanylene-$(CH_2)_6$-, -$(CH_2)_6$-diazabicyclo[2.2.1]heptanylene-$(CH_2)_7$-, -$(CH_2)_6$-diazabicyclo[2.2.1]heptanylene-$(CH_2)_8$-, -$(CH_2)_7$-diazabicyclo[2.2.1]heptanylene-$(CH_2)_i$-, -$(CH_2)_7$-diazabicyclo[2.2.1]heptanylene-$(CH_2)_2$-, -$(CH_2)_7$-diazabicyclo[2.2.1]heptanylene-$(CH_2)_3$-, -$(CH_2)_7$-diazabicyclo[2.2.1]heptanylene-$(CH_2)_4$-, -$(CH_2)_7$-diazabicyclo[2.2.1]heptanylene-$(CH_2)_8$-, -$(CH_2)_8$-diazabicyclo[2.2.1]heptanylene-$CH_2$-, -$(CH_2)_8$-diazabicyclo[2.2.1]heptanylene-$(CH_2)_2$-, -$(CH_2)_8$-diazabicyclo[2.2.1]heptanylene-$(CH_2)_3$-, -$(CH_2)_8$-diazabicyclo[2.2.1]heptanylene-$(CH_2)_4$-, -$(CH_2)_8$-diazabicyclo[2.2.1]heptanylene-$(CH_2)_5$-, -$(CH_2)_8$-diazabicyclo[2.2.1]heptanylene-$(CH_2)_6$-, -$(CH_2)_8$-diazabicyclo[2.2.1]heptanylene-$(CH_2)_7$-, -$(CH_2)_8$-diazabicyclo[2.2.1]heptanylene-$(CH_2)_8$-, -$CH_2$-diazabicyclo[3.1.1]heptanylene-$CH_2$-, -$CH_2$-diazabicyclo[3.1.1]heptanylene-$(CH_2)_2$-, -$CH_2$-diazabicyclo[3.1.1]heptanylene-$(CH_2)_3$-, -$CH_2$-diazabicyclo[3.1.1]heptanylene-$(CH_2)_4$-, -$CH_2$-diazabicyclo[3.1.1]heptanylene-$(CH_2)_5$-, -$CH_2$-diazabicyclo[3.1.1]heptanylene-$(CH_2)_6$-, -$CH_2$-diazabicyclo[3.1.1]heptanylene-$(CH_2)_7$-, -$CH_2$-diazabicyclo[3.1.1]heptanylene-$(CH_2)_8$-, -$(CH_2)_2$-diazabicyclo[3.1.1]heptanylene-$(CH_2)_1$-, -$(CH_2)_2$-diazabicyclo[3.1.1]heptanylene-$(CH_2)_2$-, -$(CH_2)_2$-diazabicyclo[3.1.1]heptanylene-$(CH_2)_3$-, -$(CH_2)_2$-diazabicyclo[3.1.1]heptanylene-$(CH_2)_4$-, -$(CH_2)_2$-diazabicyclo[3.1.1]heptanylene-$(CH_2)_5$-, -$(CH_2)_2$-diazabicyclo[3.1.1]heptanylene-$(CH_2)_6$-, -$(CH_2)_2$-diazabicyclo[3.1.1]heptanylene-$(CH_2)_7$-, -$(CH_2)_2$-diazabicyclo[3.1.1]heptanylene-$(CH_2)_8$-, -$(CH_2)_3$-diazabicyclo[3.1.1]heptanylene-$CH_2$-, -$(CH_2)_3$-diazabicyclo[3.1.1]heptanylene-$(CH_2)_2$-, -$(CH_2)_3$-diazabicyclo[3.1.1]heptanylene-$(CH_2)_3$-, -$(CH_2)_3$-diazabicyclo[3.1.1]heptanylene-$(CH_2)_4$-, -$(CH_2)_3$-diazabicyclo[3.1.1]heptanylene-$(CH_2)_5$-, -$(CH_2)_3$-diazabicyclo[3.1.1]heptanylene-$(CH_2)_6$-, -$(CH_2)_3$-diazabicyclo[3.1.1]heptanylene-$(CH_2)_7$-, -$(CH_2)_3$-diazabicyclo[3.1.1]heptanylene-$(CH_2)_8$-, -$(CH_2)_4$-diazabicyclo[3.1.1]heptanylene-$CH_2$-, -$(CH_2)_4$-diazabicyclo[3.1.1]heptanylene-$(CH_2)_2$-, -$(CH_2)_4$-diazabicyclo[3.1.1]heptanylene-$(CH_2)_3$-, -$(CH_2)_4$-diazabicyclo[3.1.1]heptanylene-$(CH_2)_4$-, -$(CH_2)_4$-diazabicyclo[3. 1. 1]heptanylene-$(CH_2)_5$-, -$(CH_2)_4$-diazabicyclo[3.1. 1]heptanylene-$(CH_2)_6$-, -$(CH_2)_4$-diazabicyclo[3. 1. 1]heptanylene-$(CH_2)_7$-, -$(CH_2)_4$-diazabicyclo[3.1. 1]heptanylene-$(CH_2)_8$-, -$(CH_2)_5$-diazabicyclo[3.1.1]heptanylene-$(CH_2)_1$-, -$(CH_2)_5$-diazabicyclo[3.1.1]heptanylene-$(CH_2)_2$-, -$(CH_2)_5$-diazabicyclo[3.1.1]heptanylene-$(CH_2)_3$-, -$(CH_2)_5$-diazabicyclo[3.1.1]heptanylene-$(CH_2)_4$-, -$(CH_2)_5$-diazabicyclo[3. 1. 1]heptanylene-$(CH_2)_5$-, -$(CH_2)_5$-diazabicyclo[3.1. 1]heptanylene-$(CH_2)_6$-, -$(CH_2)_5$-diazabicyclo[3. 1. 1]heptanylene-$(CH_2)_7$-, -$(CH_2)_5$-diazabicyclo[3.1. 1]heptanylene-$(CH_2)_8$-, -$(CH_2)_6$-diazabicyclo[3. 1. 1]heptanylene-$(CH_2)_1$-, -$(CH_2)_6$-diazabicyclo[3.1.1]heptanylene-$(CH_2)_2$-, -$(CH_2)_6$-diazabicyclo[3. 1. 1]heptanylene-$(CH_2)_3$-, -$(CH_2)_6$-diazabicyclo[3.1. 1]heptanylene-$(CH_2)_4$-, -$(CH_2)_6$-diazabicyclo[3. 1. 1]heptanylene-$(CH_2)_5$-, -$(CH_2)_6$-diazabicyclo[3.1. 1]heptanylene-$(CH_2)_6$-, -$(CH_2)_6$-diazabicyclo[3. 1. 1]heptanylene-$(CH_2)_7$-, -$(CH_2)_6$-diazabicyclo[3.1. 1]heptanylene-$(CH_2)_8$-, -$(CH_2)_7$-diazabicyclo[3.1.1]heptanylene-$(CH_2)_1$-, -$(CH_2)_7$-diazabicyclo[3.1.1]heptanylene-$(CH_2)_2$-, -$(CH_2)_7$-diazabicyclo[3.1.1]heptanylene-$(CH_2)_3$-, -$(CH_2)_7$-diazabicyclo[3.1.1]heptanylene-$(CH_2)_4$-, -$(CH_2)_7$-diazabicyclo[3.1.1]heptanylene-$(CH_Z)_s$-, -$(CH_2)_8$-diazabicyclo[3.1.1]heptanylene-$CH_2$-, -$(CH_2)_8$-diazabicyclo[3.1.1]heptanylene-$(CH_2)_2$-, -$(CH_2)_8$-diazabicyclo[3.1.1]heptanylene-$(CH_2)_3$-, -$(CH_2)_8$-diazabicyclo[3.1.1]heptanylene-$(CH_2)_4$-, -$(CH_2)_8$-diazabicyclo[3.1.1]heptanylene-$(CH_2)_5$-, -$(CH_2)_8$-diazabicyclo[3.1.1]heptanylene-$(CH_2)_6$-, -$(CH_2)_8$-diazabicyclo[3.1.1]heptanylene-$(CH_2)_7$-, -$(CH_2)_8$-diazabicyclo[3.1.1]heptanylene-$(CH_2)_8$-, -$CH_2$-diazabicyclo[3.2.1]octylene-$CH_2$-, -$CH_2$-diazabicyclo[3.2.1]octylene-$(CH_2)_2$-, -$CH_2$-diazabicyclo[3.2.1]octylene-$(CH_2)_3$-, -$CH_2$-diazabicyclo[3.2.1]octylene-$(CH_2)_4$-, -$CH_2$-diazabicyclo[3.2.1]octylene-$(CH_2)_5$-, -$CH_2$-diazabicyclo[3.2.1]octylene-$(CH_2)_6$-, -$CH_2$-diazabicyclo[3.2.1]octylene-$(CH_2)_7$-, -$CH_2$-diazabicyclo[3.2.1]octylene-$(CH_2)_8$-, -$(CH_2)_2$-diazabicyclo[3.2.1]octylene-$(CH_2)_1$-, -$(CH_2)_2$-diazabicyclo[3.2.1]octylene-$(CH_2)_2$-, -$(CH_2)_2$-diazabicyclo[3.2.1]octylene-$(CH_2)_3$-, -$(CH_2)_2$-diazabicyclo[3.2.1]octylene-$(CH_2)_4$-, -$(CH_2)_2$-diazabicyclo[3.2.1]octylene-$(CH_2)_5$-, -$(CH_2)_2$-diazabicyclo[3.2.1]octylene-$(CH_2)_6$-, -$(CH_2)_2$-diazabicyclo[3.2.1]octylene-$(CH_2)_7$-, -$(CH_2)_2$-diazabicyclo[3.2.1]octylene-$(CH_2)_8$-, -$(CH_2)_3$-diazabicyclo[3.2.1]octylene-$CH_2$-, -$(CH_2)_3$-diazabicyclo[3.2.1]octylene-$(CH_2)_2$-, -$(CH_2)_3$-diazabicyclo[3.2.1]octylene-$(CH_2)_3$-, -$(CH_2)_3$-diazabicyclo[3.2.1]octylene-$(CH_2)_4$-, -$(CH_2)_3$-diazabicyclo[3.2.1]octylene-$(CH_2)_5$-, -$(CH_2)_3$-diazabicyclo[3.2.1]octylene-$(CH_2)_6$-, -$(CH_2)_3$-diazabicyclo[3.2.1]octylene-$(CH_2)_7$-, -$(CH_2)_3$-diazabicyclo[3.2.1]octylene-$(CH_2)_8$-, -$(CH_2)_4$-diazabicyclo[3.2.1]octylene-$CH_2$-, -$(CH_2)_4$-diazabicyclo[3.2.1]octylene-$(CH_2)_2$-, -$(CH_2)_4$-diazabicyclo[3.2.1]octylene-$(CH_2)_3$-, -$(CH_2)_4$-diazabicyclo[3.2.1]octylene-$(CH_2)_4$-, -$(CH_2)_4$-diazabicyclo[3.2.1]octylene-$(CH_2)_5$-, -$(CH_2)_4$-diazabicyclo[3.2.1]octylene-$(CH_2)_6$-, -$(CH_2)_4$-diazabicyclo[3.2.1]octylene-$(CH_2)_7$-, -$(CH_2)_4$-diazabicyclo[3.2.1]octylene-$(CH_2)_8$-, -$(CH_2)_5$-diazabicyclo[3.2.1]octylene-$(CH_2)_1$-, -$(CH_2)_5$-diazabicyclo[3.2.1]octylene-$(CH_2)_2$-, -$(CH_2)_5$-diazabicyclo[3.2.1]octylene-$(CH_2)_3$-, -$(CH_2)_5$-diazabicyclo[3.2.1]octylene-$(CH_2)_4$-, -$(CH_2)_5$-diazabicyclo[3.2.1]octylene-$(CH_2)_5$-, -$(CH_2)_5$-diazabicyclo[3.2.1]octylene-$(CH_2)_6$-, -$(CH_2)_5$-diazabicyclo[3.2.1]octylene-$(CH_2)_7$-, -$(CH_2)_5$-diazabicyclo[3.2.1]octylene-$(CH_2)_8$-, -$(CH_2)_6$-diazabicyclo[3.2.1]octylene-$(CH_2)_1$-, -$(CH_2)_6$-diazabicyclo[3.2.1]octylene-$(CH_2)_2$-, -$(CH_2)_6$-diazabicyclo[3.2.1]octylene-$(CH_2)_3$-, -$(CH_2)_6$-diazabicyclo[3.2.1]octylene-$(CH_2)_4$-, -$(CH_2)_6$-diazabicyclo[3.2.1]octylene-$(CH_2)_5$-, -$(CH_2)_6$-diazabicyclo[3.2.1]octylene-$(CH_2)_6$-, -$(CH_2)_6$-diazabicyclo[3.2.1]octylene-$(CH_2)_7$-, -$(CH_2)_6$-diazabicyclo[3.2.1]octylene-$(CH_2)_8$-, -$(CH_2)_7$-diazabicyclo[3.2.1]octylene-$(CH_2)_1$-, -$(CH_2)_7$-diazabicyclo[3.2.1]octylene-$(CH_2)_2$-, -$(CH_2)_7$-diazabicyclo[3.2.1]octylene-$(CH_2)_3$-, -$(CH_2)_7$-diazabicyclo[3.2.1]octylene-$(CH_2)_4$-, -$(CH_2)_7$-diazabicyclo[3.2.1]octylene-$(CH_2)_8$-, -$(CH_2)_8$-diazabicyclo[3.2. 1]octylene-$CH_2$-, -$(CH_2)_8$-diazabicyclo[3.2.1]octyle-

ne-$(CH_2)_2$-, -$(CH_2)_8$-diazabicyclo[3.2.1]octylene-$(CH_2)_3$-, -$(CH_2)_8$-diazabicyclo[3.2.1]octylene-$(CH_2)_4$-, -$(CH_2)_8$-diazabicyclo[3.2.1]octylene-$(CH_2)_5$-, -$(CH_2)_8$-diazabicyclo[3.2.1]octylene-$(CH_2)_6$-, -$(CH_2)_8$-diazabicyclo[3.2.1]octylene-$(CH_2)_7$-, -$(CH_2)_8$-diazabicyclo[3.2.1]octylene-$(CH_2)_8$-, -$CH_2$-diazabicyclo[2.2.2]octylene-$CH_2$-, -$CH_2$-diazabicyclo[2.2.2]octylene-$(CH_2)_2$-, -$CH_2$-diazabicyclo[2.2.2]octylene-$(CH_2)_3$-, -$CH_2$-diazabicyclo[2.2.2]octylene-$(CH_2)_4$-, -$CH_2$-diazabicyclo[2.2.2]octylene-$(CH_2)_5$-, -$CH_2$-diazabicyclo[2.2.2]octylene-$(CH_2)_6$-, -$CH_2$-diazabicyclo[2.2.2]octylene-$(CH_2)_7$-, -$CH_2$-diazabicyclo[2.2.2]octylene-$(CH_2)_8$-, -$(CH_2)_2$-diazabicyclo[2.2.2]octylene-$(CH_2)_1$-, -$(CH_2)_2$-diazabicyclo[2.2.2]octylene-$(CH_2)_2$-, -$(CH_2)_2$-diazabicyclo[2.2.2]octylene-$(CH_2)_3$-, -$(CH_2)_2$-diazabicyclo[2.2.2]octylene-$(CH_2)_4$-, -$(CH_2)_2$-diazabicyclo[2.2.2]octylene-$(CH_2)_5$-, -$(CH_2)_2$-diazabicyclo[2.2.2]octylene-$(CH_2)_6$-, -$(CH_2)_2$-diazabicyclo[2.2.2]octylene-$(CH_2)_7$-, -$(CH_2)_2$-diazabicyclo[2.2.2]octylene-$(CH_2)_8$-, -$(CH_2)_3$-diazabicyclo[2.2.2]octylene-$CH_2$-, -$(CH_2)_3$-diazabicyclo[2.2.2]octylene-$(CH_2)_2$-, -$(CH_2)_3$-diazabicyclo[2.2.2]octylene-$(CH_2)_3$-, -$(CH_2)_3$-diazabicyclo[2.2.2]octylene-$(CH_2)_4$-, -$(CH_2)_3$-diazabicyclo[2.2.2]octylene-$(CH_2)_5$-, -$(CH_2)_3$-diazabicyclo[2.2.2]octylene-$(CH_2)_6$-, -$(CH_2)_3$-diazabicyclo[2.2.2]octylene-$(CH_2)_7$-, -$(CH_2)_3$-diazabicyclo[2.2.2]octylene-$(CH_2)_8$-, -$(CH_2)_4$-diazabicyclo[2.2.2]octylene-$CH_2$-, -$(CH_2)_4$-diazabicyclo[2.2.2]octylene-$(CH_2)_2$-, -$(CH_2)_4$-diazabicyclo[2.2.2]octylene-$(CH_2)_3$-, -$(CH_2)_4$-diazabicyclo[2.2.2]octylene-$(CH_2)_4$-, -$(CH_2)_4$-diazabicyclo[2.2.2]octylene-$(CH_2)_5$-, -$(CH_2)_4$-diazabicyclo[2.2.2]octylene-$(CH_2)_6$-, -$(CH_2)_4$-diazabicyclo[2.2.2]octylene-$(CH_2)_7$-, -$(CH_2)_4$-diazabicyclo[2.2.2]octylene-$(CH_2)_8$-, -$(CH_2)_5$-diazabicyclo[2.2.2]octylene-$(CH_2)_1$-, -$(CH_2)_5$-diazabicyclo[2.2.2]octylene-$(CH_2)_2$-, -$(CH_2)_5$-diazabicyclo[2.2.2]octylene-$(CH_2)_3$-, -$(CH_2)_5$-diazabicyclo[2.2.2]octylene-$(CH_2)_4$-, -$(CH_2)_5$-diazabicyclo[2.2.2]octylene-$(CH_2)_5$-, -$(CH_2)_5$-diazabicyclo[2.2.2]octylene-$(CH_2)_6$-, -$(CH_2)_5$-diazabicyclo[2.2.2]octylene-$(CH_2)_7$-, -$(CH_2)_5$-diazabicyclo[2.2.2]octylene-$(CH_2)_8$-, -$(CH_2)_6$-diazabicyclo[2.2.2]octylene-$(CH_2)_1$-, -$(CH_2)_6$-diazabicyclo[2.2.2]octylene-$(CH_2)_2$-, -$(CH_2)_6$-diazabicyclo[2.2.2]octylene-$(CH_2)_3$-, -$(CH_2)_6$-diazabicyclo[2.2.2]octylene-$(CH_2)_4$-, -$(CH_2)_6$-diazabicyclo[2.2.2]octylene-$(CH_2)_5$-, -$(CH_2)_6$-diazabicyclo[2.2.2]octylene-$(CH_2)_6$-, -$(CH_2)_6$-diazabicyclo[2.2.2]octylene-$(CH_2)_7$-, -$(CH_2)_6$-diazabicyclo[2.2.2]octylene-$(CH_2)_8$-, -$(CH_2)_7$-diazabicyclo[2.2.2]octylene-$(CH_2)_1$-, -$(CH_2)_7$-diazabicyclo[2.2.2]octylene-$(CH_2)_2$-, -$(CH_2)_7$-diazabicyclo[2.2.2]octylene-$(CH_2)_3$-, -$(CH_2)_7$-diazabicyclo[2.2.2]octylene-$(CH_2)_4$-, -$(CH_2)_7$-diazabicyclo[2.2.2]octylene-$(CH_2)_8$-, -$(CH_2)_8$-diazabicyclo[2.2.2]octylene-$CH_2$-, -$(CH_2)_8$-diazabicyclo[2.2.2]octylene-$(CH_2)_2$-, -$(CH_2)_8$-diazabicyclo[2.2.2]octylene-$(CH_2)_3$-, -$(CH_2)_8$-diazabicyclo[2.2.2]octylene-$(CH_2)_4$-, -$(CH_2)_8$-diazabicyclo[2.2.2]octylene-$(CH_2)_5$-, -$(CH_2)_8$-diazabicyclo[2.2.2]octylene-$(CH_2)_6$-, -$(CH_2)_8$-diazabicyclo[2.2.2]octylene-$(CH_2)_7$-, or -$(CH_2)_8$-diazabicyclo[2.2.2]octylene-$(CH_2)_8$-,

wherein the propylene, the piperidinylene, the piperazinylene, the diazacycloheptanylene, the diazabicyclo[2.2.1]heptanylene, the diazabicyclo[3.1.1]heptanylene, the diazabicyclo[3.2.1]octylene, the diazabicyclo[2.2.2]octylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof; and

each $R_{g11}$ independently represents H or $C_{1-3}$ alkyl.

In some embodiments of the compound of Formula (I-1), $L_2$ represents the structure of the following formula:

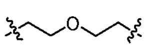 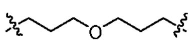 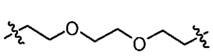 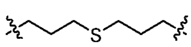 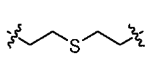 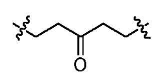

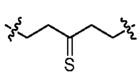 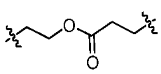 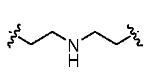 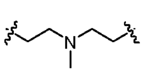 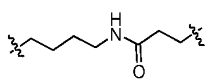 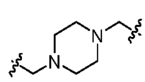 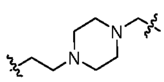

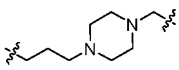 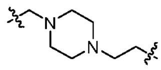 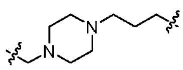 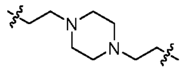 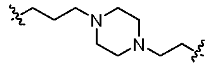 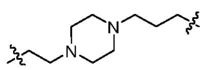

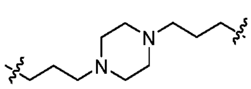 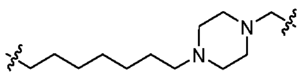 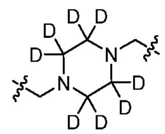 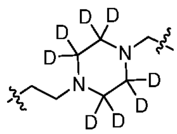 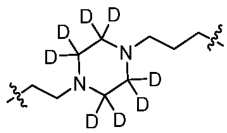

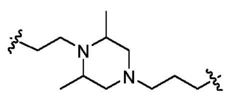 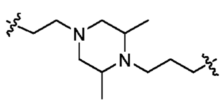 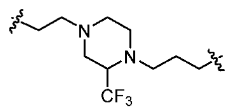 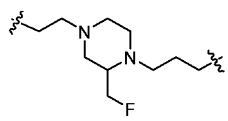 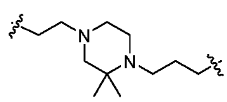

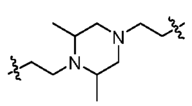 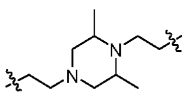 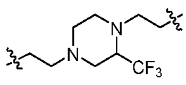 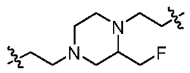 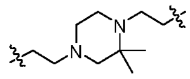 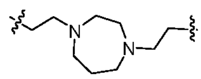

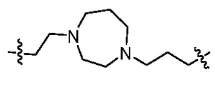 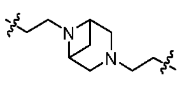 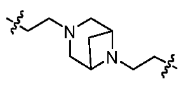 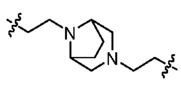 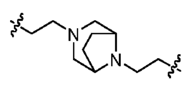 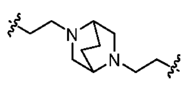

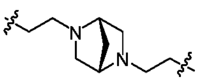 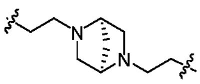 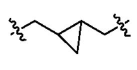 or 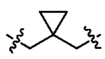

In some embodiments of the compound of Formula (I-1), $R_{b1}$ represents:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, 2-oxobicyclo[2.2.1]heptanyl or bicyclo[2.2.1]heptenyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 3-azaspiro[5.5]undecyl or 7-azaspiro[3.5]nonyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl,

$NH_2$-$C_{1-4}$ alkylene, $C_{1-4}$ alkyl-NHC(O)-, $C_{1-4}$ alkyl-C(O)NH-, or any combination thereof; phenyl or naphthyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl,

$NH_2$-$C_{1-4}$ alkylene, $C_{1-4}$ alkyl-NHC(O)-, $C_{1-4}$ alkyl-C(O)NH-, or any combination thereof; or furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl,

pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl and imidazo[2,1-b]thiazolyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof. In embodiments of the present disclosure, the number of substituents is not theoretically limited in any way or automatically limited by the size of the building units.

In some embodiments of the compound of Formula (I-1), $R_{b1}$ represents:

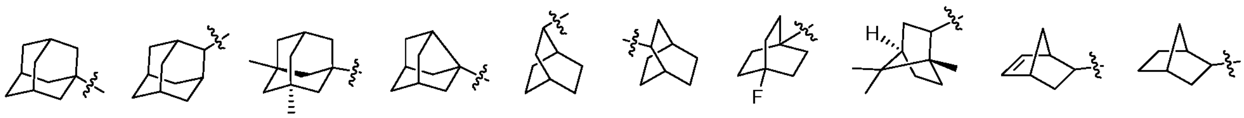

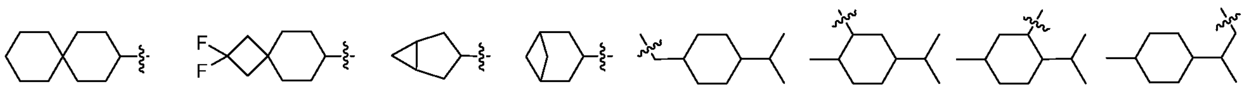

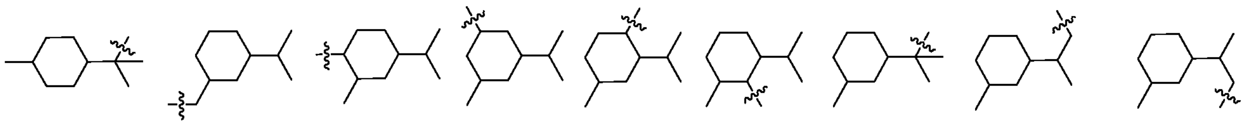

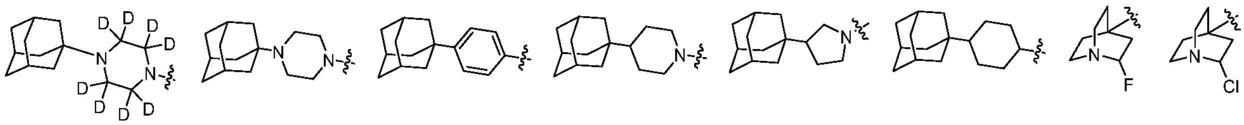

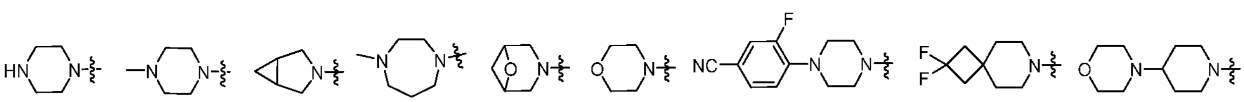

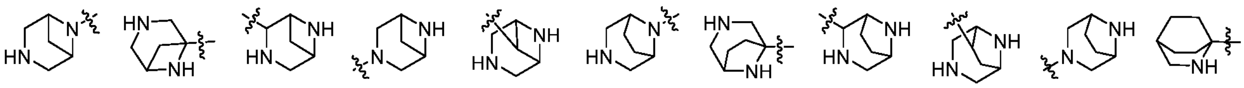

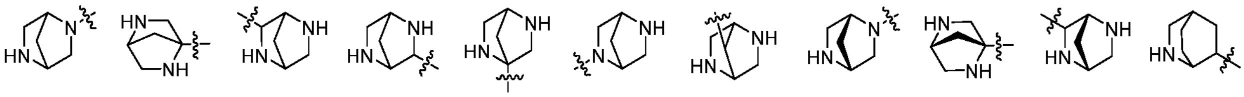

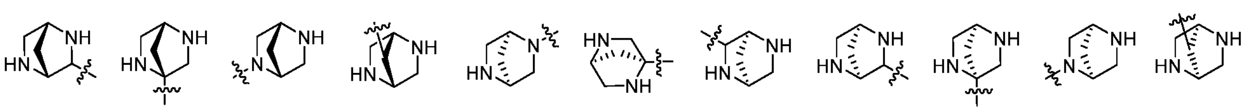

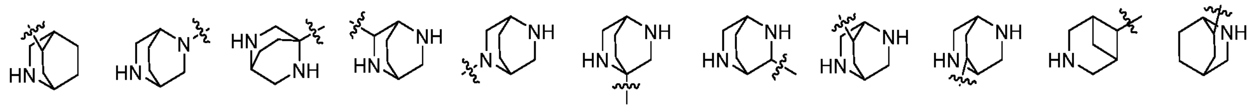

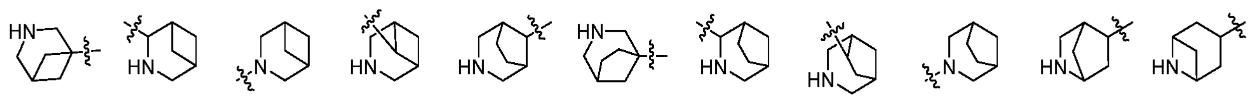

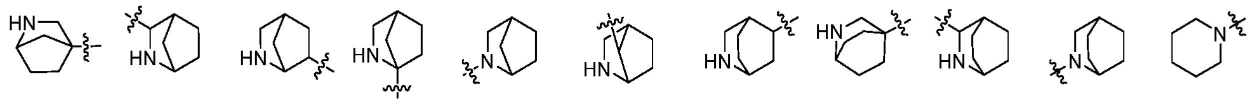

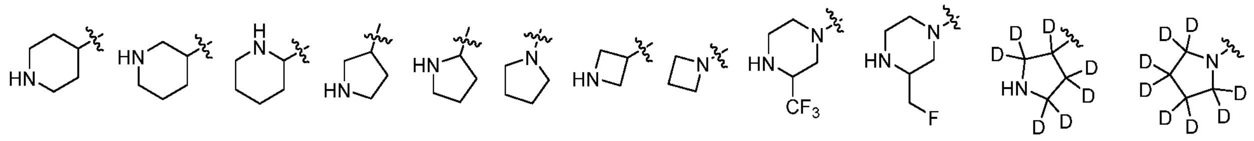

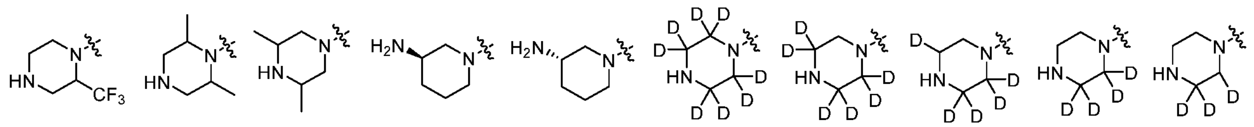

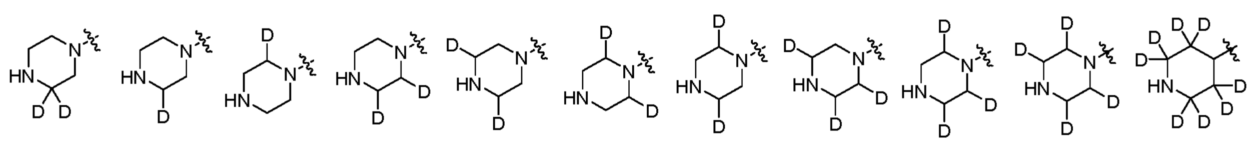

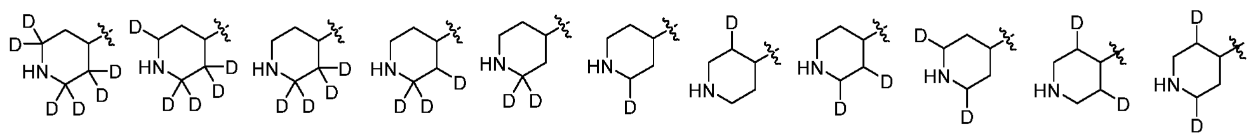

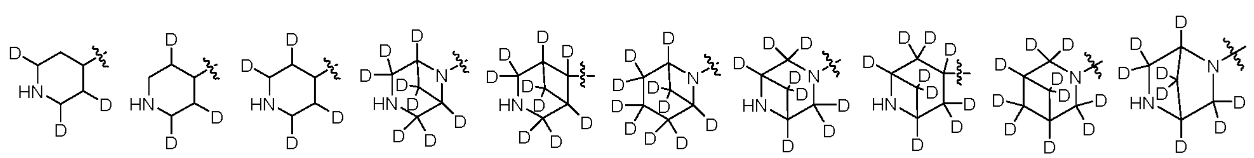

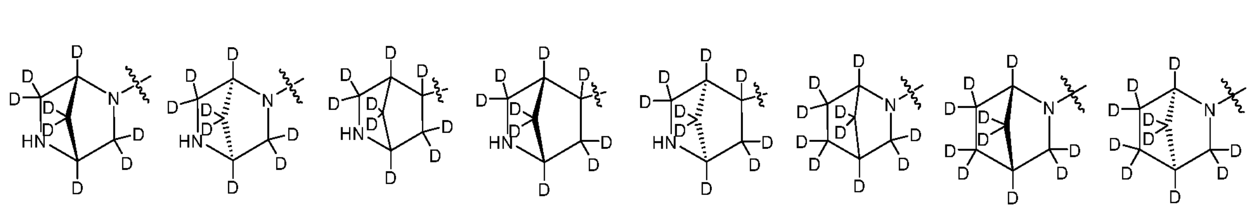

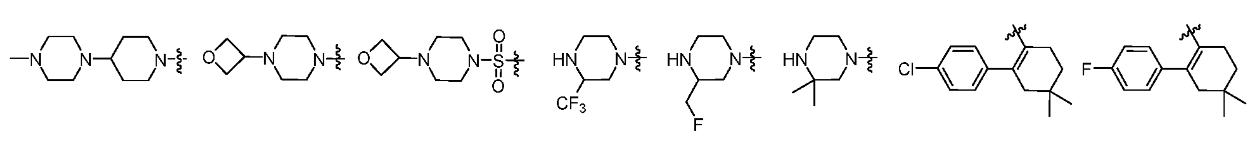

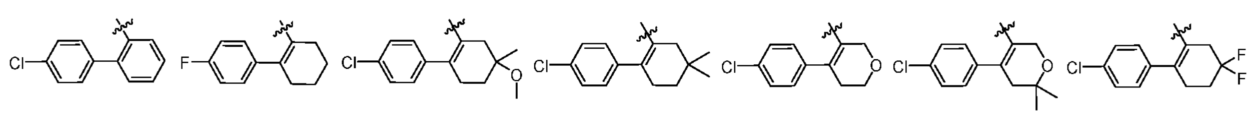

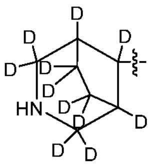 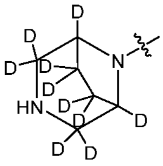 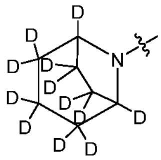 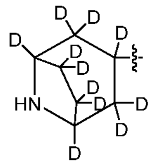 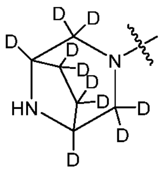 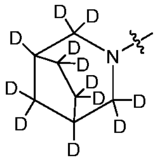 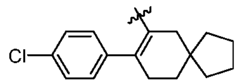

or

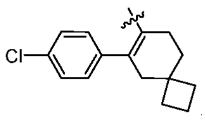

In some embodiments, the compound of Formula (I) is also of Formula (I-2):

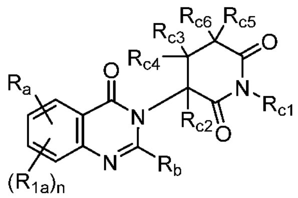

Formula (I-2)

wherein $R_{c1}$, $R_{c2}$, $R_{c3}$, $R_{c4}$, $R_{c5}$, $R_{c6}$, $R_b$, $(R_{1a})_n$, $R_a$, $R_{1a}$ and n are as defined in the compound of Formula (I) above and the embodiments thereof;

wherein $R_a$ represents the following formula:

$$R_{a1}\text{-}X_2\text{-}L_1\text{-}X_1\text{-} ,$$

wherein $X_1$ represents $N(R_{e1})$, O, S, alkynylene (e.g., $C_{2-6}$ alkynylene), alkenylene (e.g., $C_{2-6}$ alkenylene), optionally substituted cycloalkylene (e.g., optionally substituted $C_{3-20}$cycloalkylene or optionally substituted $C_{3-15}$cycloalkylene), optionally substituted heterocyclylene (e.g., optionally substituted 4- to 20-membered heterocyclylene or optionally substituted 4- to 15-membered heterocyclylene), optionally substituted arylene (e.g., optionally substituted $C_{6-10}$ arylene), or optionally substituted heteroarylene (e.g., optionally substituted 5- to 20-membered heteroarylene or optionally substituted 5- to 15-membered heteroarylene), wherein $R_{e1}$ represents H or $C_{1-3}$ alkyl, or $X_1$ represents a bond;

$L_1$ represents:

optionally substituted linear or branched $C_{1-60}$ alkylene (e.g., $C_1$-$C_{40}$ alkylene, $C_1$-$C_{30}$ alkylene, $C_1$-$C_{29}$ alkylene, $C_1$-$C_{28}$ alkylene, $C_1$-$C_{27}$ alkylene, $C_1$-$C_{26}$ alkylene, $C_1$-$C_{25}$ alkylene, $C_1$-$C_{24}$ alkylene, $C_1$-$C_{23}$ alkylene, $C_1$-$C_{22}$ alkylene, $C_1$-$C_{21}$ alkylene, $C_1$-$C_{20}$ alkylene, $C_1$-$C_{19}$ alkylene, $C_1$-$C_{18}$ alkylene, $C_1$-$C_{17}$ alkylene, $C_1$-$C_{16}$ alkylene, $C_1$-$C_{15}$ alkylene, $C_1$-$C_{14}$ alkylene, $C_1$-$C_{13}$ alkylene, $C_1$-$C_{12}$ alkylene, $C_1$-$C_{11}$ alkylene, $C_1$-$C_{10}$ alkylene, $C_1$-$C_9$ alkylene, $C_1$-$C_8$ alkylene, $C_1$-$C_7$ alkylene, $C_1$-$C_6$ alkylene, $C_1$-$C_5$ alkylene, $C_1$-$C_4$ alkylene, $C_1$-$C_3$ alkylene, or $C_1$-$C_2$ alkylene), or

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{e2}$ and/or one or more (e.g., 1-20, or 1-15, 1-10, 1-9, 1-8, 1-7, 1-6, 1-5, 1-4, 1-3 or 1-2, or 1) groups $R_{e3}$ and/or any combination of one or more (e.g., 1-20, or 1-15, 1-10, 1-9, 1-8, 1-7, 1-6, 1-5, 1-4, 1-3 or 1-2, or 1) groups $R_{e2}$ and $R_{e3}$ inserted into its backbone carbon chain, wherein carbon-carbon bond between one or more pairs (e.g., 1-10, 1-9, 1-8, 1-7, 1-6, 1-5, 1-4, 1-3 or 1-2, or 1 pair) of adjacent carbon atoms in the backbone carbon chain is interrupted by the group $R_{e2}$, $R_{e3}$, or a combination of $R_{e2}$ and $R_{e3}$; wherein each $R_{e2}$ is independently selected

from the group consisting of O, S, $N(R_{e4})$, C(O), C(O)O, OC(O), C(O)NH, NHC(O), NHC(O)NH, C(S), S(O), $S(O)_2$, $S(O)_2O$, $OS(O)_2$, $S(O)_2NH$, or $NHS(O)_2$, wherein $R_{e4}$ represents H or $C_{1-3}$ alkyl, and in case that two or more groups $R_{e2}$ are inserted into the backbone carbon chain of the linear or branched $C_{2-60}$ alkylene group, the two or more groups $R_{e2}$ are not directly connected to each other; and wherein each $R_{e3}$ is independently selected from the group consisting of optionally substituted cycloalkylene (e.g., optionally substituted $C_{3-20}$cycloalkylene, or optionally substituted $C_{3-15}$cycloalkylene), optionally substituted heterocyclylene (e.g., optionally substituted 4- to 20-membered heterocyclylene, or optionally substituted 5- to 15-membered heterocyclylene), optionally substituted arylene (e.g., optionally substituted $C_{6-10}$ arylene), optionally substituted heteroarylene (e.g., optionally substituted 5- to 20-membered heteroarylene, or optionally substituted 5- to 15-membered heteroarylene), alkenylene (e.g., $C_{2-6}$ alkenylene), alkynylene (e.g., $C_{2-6}$ alkynylene) or any combination thereof;

$X_2$ represents C(O), S(O) or $S(O)_2$, or $X_2$ represents a bond;
$R_{a1}$ represents the following formula:

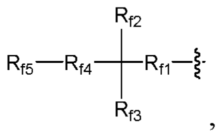
,

wherein $R_{f1}$ represents:

$-NCH_2CH_2N-$;

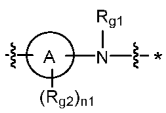
,

wherein symbol * indicates the point of attachment to $X_2$, $R_{g1}$ represents H or $C_{1-3}$ alkyl, ring A represents cycloalkylene (e.g., $C_{3-20}$cycloalkylene, or $C_{3-15}$cycloalkylene) or heterocyclylene (e.g., 4- to 20-membered heterocyclylene, or 5- to 15-membered heterocyclylene), $(R_{g2})_{n1}$ indicates that ring A is optionally substituted with n1 $R_{g2}$, wherein n1 represents an integer of 0 to 8, and each $R_{g2}$, the same or different from each other, is independently selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

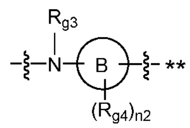
,

wherein symbol ** indicates the point of attachment to $X_2$, $R_{g3}$ represents H or $C_{1-3}$ alkyl, ring B represents cycloalkylene (e.g., $C_{3-20}$cycloalkylene, or $C_{3-15}$cycloalkylene) or heterocyclylene (e.g., 4- to 20-membered heterocyclylene, or 5- to 15-membered heterocyclylene), $(R_{g4})_{n2}$ indicates that ring B is optionally substituted with n2 $R_{g4}$, wherein n2 represents an integer of 0 to 8, and each $R_{g4}$, the same or different from each other, is independently selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof; or

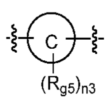
,

wherein ring C represents cycloalkylene (e.g., $C_{3-20}$cycloalkylene, or $C_{3-15}$cycloalkylene) or heterocyclylene (e.g., 4- to 20-membered heterocyclylene, or 5- to 15-membered heterocyclylene), $(R_{g5})_{n3}$ indicates that ring C is optionally substituted with n3 $R_{g5}$, wherein n3 represents an integer of 0 to 8, and each $R_{g5}$, the same or

different from each other, is independently selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

$R_{f2}$ represents H, halogen, $C_{1-3}$ alkyl or halogenated $C_{1-3}$ alkyl;

$R_{f3}$ represents H, halogen, $C_{1-3}$ alkyl, halogenated $C_{1-3}$ alkyl,

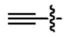, 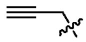, 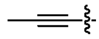

or

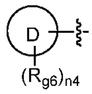,

wherein ring D represents cycloalkylene (e.g., $C_{3-20}$cycloalkylene, or $C_{3-15}$cycloalkylene), heterocyclylene (e.g., 4- to 20-membered heterocyclylene, or 5- to 15-membered heterocyclylene), arylene (e.g., $C_{6-10}$ arylene) or heteroarylene (e.g., 5-to 20-membered heteroarylene, or 5- to 15-membered heteroarylene), $(R_{g6})_{n4}$ indicates that ring D is optionally substituted with n4 $R_{g6}$, wherein n4 represents an integer of 0 to 8, and each $R_{g6}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

$R_{f4}$ represents

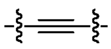, 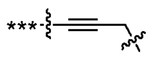, 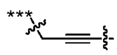 or 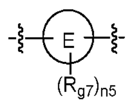,

wherein symbol *** indicates the point of attachment to $R_{f5}$, ring E represents cycloalkylene (e.g., $C_{3-20}$cycloalkylene, or $C_{3-15}$cycloalkylene), heterocyclylene (e.g., 4- to 20-membered heterocyclylene, or 5- to 15-membered heterocyclylene), arylene (e.g., $C_{6-10}$ arylene) or heteroarylene (e.g., 5- to 20-membered heteroarylene, or 5- to 15-membered heteroarylene), $(R_{g7})_{n5}$ indicates that ring E is optionally substituted with n5 $R_{g7}$, wherein n5 represents an integer of 0 to 8, and each $R_{g7}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof; and

$R_{f5}$ represents H, halogen, $C_{1-3}$ alkyl, halogenated $C_{1-3}$ alkyl,

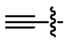, 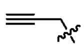, 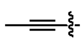 or 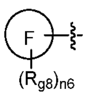,

wherein ring F represents cycloalkyl (e.g., $C_{3-20}$cycloalkyl, or $C_{3-15}$cycloalkyl), heterocyclyl (e.g., 4- to 20-membered heterocyclyl, or 5- to 15-membered heterocyclyl), aryl (e.g., $C_{6-10}$ aryl) or heteroaryl (e.g., 5- to 20-membered heteroaryl, or 5- to 15-membered heteroaryl), $(R_{g8})_{n6}$ indicates that ring F is optionally substituted with n6 $R_{g8}$, wherein n6 represents an integer of 0 to 8, and each $R_{g8}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;
and

$R_b$ represents $C_{1-60}$ alkyl (e.g., $C_1$-$C_{30}$ alkyl, $C_1$-$C_{29}$ alkyl, $C_1$-$C_{28}$ alkyl, $C_1$-$C_{27}$ alkyl, $C_1$-$C_{26}$ alkyl, $C_1$-$C_{25}$ alkyl, $C_1$-$C_{24}$ alkyl, $C_1$-$C_{23}$ alkyl, $C_1$-$C_{22}$ alkyl, $C_1$-$C_{21}$ alkyl, $C_1$-$C_{20}$ alkyl, $C_1$-$C_{19}$ alkyl, $C_1$-$C_{18}$ alkyl, $C_1$-$C_{17}$ alkyl, $C_1$-$C_{16}$

alkyl, $C_1$-$C_{15}$ alkyl, $C_1$-$C_{14}$ alkyl, $C_1$-$C_{13}$ alkyl, $C_1$-$C_{12}$ alkyl, $C_1$-$C_{11}$ alkyl, $C_1$-$C_{10}$ alkyl, $C_1$-$C_9$ alkyl, $C_1$-Cs alkyl, $C_1$-$C_7$ alkyl, $C_1$-$C_6$ alkyl, $C_1$-$C_5$ alkyl, $C_1$-$C_4$ alkyl, $C_1$-$C_3$ alkyl, or $C_1$-$C_2$ alkyl) optionally substituted with D or halogen, or $R_b$ represents the following formula:

$R_{b1}$-$X_4$-$L_2$-,

wherein $L_2$ represents:

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene), or
optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{12}$ and/or one or more (e.g., 1-20, or 1-15, 1-10, 1-9, 1-8, 1-7, 1-6, 1-5, 1-4, 1-3 or 1-2, or 1) groups $R_{13}$ and/or any combination of one or more(e.g., 1-20, or 1-15, 1-10, 1-9, 1-8, 1-7, 1-6, 1-5, 1-4, 1-3 or 1-2, or 1) groups $R_{12}$ and $R_{13}$ inserted into its backbone carbon chain, wherein carbon-carbon bond between one or more pairs (e.g., 1-10, 1-9, 1-8, 1-7, 1-6, 1-5, 1-4, 1-3 or 1-2, or 1 pair) of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, N($R_{14}$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of cycloalkylene (e.g., $C_{3-20}$cycloalkylene, or $C_{3-15}$cycloalkylene), heterocyclylene (e.g., 4- to 20-membered heterocyclylene, or 4- to 15-membered heterocyclylene), arylene (e.g., $C_{6-10}$ arylene), heteroarylene (e.g., 5- to 20-membered heteroarylene, or 5- to 15-membered heteroarylene), alkenylene (e.g., $C_{2-6}$ alkenylene), alkynylene (e.g., $C_{2-6}$ alkynylene) or any combination thereof, wherein the cycloalkylene, the heterocyclylene, arylene and the heteroarylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof;

$X_4$ represents N($R_{15}$), C(O)O, OC(O), C(O)NH, NHC(O), C(O), O, C(=S), S, S(O), $S(O)_2$, $S(O)_2NH$, $NHS(O)_2$, $S(O)_2O$, $OS(O)_2$, optionally substituted cycloalkylene (e.g., optionally substituted $C_{3-20}$cycloalkylene, or optionally substituted $C_{3-15}$cycloalkylene), optionally substituted arylene (e.g., optionally substituted $C_{6-20}$ arylene, or optionally substituted $C_{6-10}$ arylene), optionally substituted heterocyclylene (e.g., optionally substituted 4- to 20-membered heterocyclylene, or optionally substituted 4- to 15-membered heterocyclylene), optionally substituted heteroarylene (e.g., optionally substituted 5- to 20-membered heteroarylene, or optionally substituted 5- to 15-membered heteroarylene), triazolylene-NH-, or -NH-triazolylene, wherein $R_{15}$ represents H or $C_{1-3}$ alkyl, or $X_4$ represents a bond; and
$R_{b1}$ represents optionally substituted cycloalkyl (e.g., optionally substituted $C_{3-20}$cycloalkyl, or optionally substituted $C_{3-15}$cycloalkyl), optionally substituted aryl (e.g., optionally substituted $C_{6-20}$ aryl, or optionally substituted $C_{6-10}$ aryl), optionally substituted heterocyclyl (e.g., optionally substituted 4- to 20-membered heterocyclyl, or optionally substituted 4- to 15-membered heterocyclyl) or optionally substituted heteroaryl (e.g., optionally substituted 5- to 20-membered heteroaryl, or optionally substituted 5- to 15-membered heteroaryl).

In some embodiments of the compound of Formula (I-2), $R_a$ represents the following formula:

$R_{a1}$-$X_2$-$L_1$-$X_1$- ,

wherein $X_1$ represents $N(R_{e1})$, O, S, $C_{2-6}$ alkynylene, $C_{2-6}$ alkenylene, optionally substituted $C_{3-20}$cycloalkylene (e.g., optionally substituted $C_{3-15}$cycloalkylene), optionally substituted 4- to 20-membered heterocyclylene (e.g., optionally substituted 4- to 15-membered heterocyclylene), optionally substituted $C_{6-15}$ arylene (e.g., optionally substituted $C_{6-10}$ arylene), or optionally substituted 5- to 20-membered heteroarylene (e.g., optionally substituted 5- to 15-membered heteroarylene), wherein $R_{e1}$ represents H or $C_{1-3}$ alkyl, or $X_1$ represents a bond;
$L_1$ represents:

optionally substituted linear or branched $C_{1-60}$ alkylene (e.g., $C_1$-$C_{40}$ alkylene, $C_1$-$C_{30}$ alkylene, $C_1$-$C_{29}$ alkylene, $C_1$-$C_{28}$ alkylene, $C_1$-$C_{27}$ alkylene, $C_1$-$C_{26}$ alkylene, $C_1$-$C_{25}$ alkylene, $C_1$-$C_{24}$ alkylene, $C_1$-$C_{23}$ alkylene, $C_1$-$C_{22}$ alkylene, $C_1$-$C_{21}$ alkylene, $C_1$-$C_{20}$ alkylene, $C_1$-$C_{19}$ alkylene, $C_1$-$C_{18}$ alkylene, $C_1$-$C_{17}$ alkylene, $C_1$-$C_{16}$ alkylene, $C_1$-$C_{15}$ alkylene, $C_1$-$C_{14}$ alkylene, $C_1$-$C_{13}$ alkylene, $C_1$-$C_{12}$ alkylene, $C_1$-$C_{11}$ alkylene, $C_1$-$C_{10}$ alkylene, $C_1$-$C_9$ alkylene, $C_1$-$C_8$ alkylene, $C_1$-$C_7$ alkylene, $C_1$-$C_6$ alkylene, $C_1$-$C_5$ alkylene, $C_1$-$C_4$ alkylene, $C_1$-$C_3$ alkylene, or $C_1$-$C_2$ alkylene), or

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{e2}$ and/or one or more(e.g., 1-20, or 1-15, 1-10, 1-9, 1-8, 1-7, 1-6, 1-5, 1-4, 1-3 or 1-2, or 1) groups $R_{e3}$ and/or any combination of one or more (e.g., 1-20, or 1-15, 1-10, 1-9, 1-8, 1-7, 1-6, 1-5, 1-4, 1-3 or 1-2, or 1) groups $R_{e2}$ and $R_{e3}$ inserted into its backbone carbon chain, wherein carbon-carbon bond between one or more pairs (e.g., 1-10, 1-9, 1-8, 1-7, 1-6, 1-5, 1-4, 1-3 or 1-2, or 1 pair) of adjacent carbon atoms in the backbone carbon chain is interrupted by the group $R_{e2}$, $R_{e3}$, or a combination of $R_{e2}$ and $R_{e3}$; wherein each $R_{e2}$ is independently selected from the group consisting of O, S, $N(R_{e4})$, C(O), C(O)O, OC(O), C(O)NH, NHC(O), NHC(O)NH, C(S), S(O), $S(O)_2$, $S(O)_2O$, $OS(O)_2$, $S(O)_2NH$, or $NHS(O)_2$, wherein $R_{e4}$ represents H or $C_{1-3}$ alkyl, and in case that two or more groups $R_{e2}$ are inserted into the backbone carbon chain of the linear or branched $C_{2-60}$ alkylene group, the two or more groups $R_{e2}$ are not directly connected to each other; and wherein each $R_{e3}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the linear or branched $C_{1-60}$ alkylene and the linear or branched $C_{2-60}$ alkylene are each independently optionally substituted with a substituent selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, nitro, halogen, cyano, optionally deuterated $C_{1-6}$ alkoxy (e.g., $C_{1-3}$ alkoxy, such as methoxy, ethoxy, or propoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), or any combination thereof;

$X_2$ represents C(O) or $S(O)_2$, or $X_2$ represents a bond;
$R_{a1}$ represents the following formula:

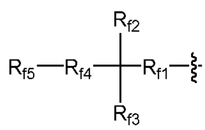,

wherein $R_{f1}$ represents:

$-NCH_2CH_2N-$;

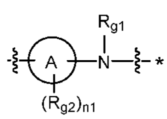,

wherein symbol * indicates the point of attachment to $X_2$, $R_{g1}$ represents H or $C_{1-3}$ alkyl, ring A represents $C_{3-20}$cycloalkylene or 4- to 20-membered heterocyclylene, $(R_{g2})_{n1}$ indicates that ring A is optionally substituted with n1 $R_{g2}$, wherein n1 represents an integer of 0 to 8, and each $R_{g2}$, the same or different from each other, is independently selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, optionally deuterated $C_{1-6}$ alkoxy (e.g., $C_{1-3}$ alkoxy, such as methoxy, ethoxy, or propoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-4}$ alkylene-, such as $NH_2CH_2$-, $NH_2CH_2CH_2$- and $NH_2CH_2CH_2CH_2$-) or any combination thereof;

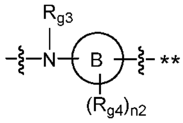,

wherein symbol ** indicates the point of attachment to $X_2$, $R_{g3}$ represents H or $C_{1-3}$ alkyl, ring B represents $C_{3-20}$cycloalkylene or 4- to 20-membered heterocyclylene, $(R_{g4})_{n2}$ indicates that ring B is optionally substituted with n2 $R_{g4}$, wherein n2 represents an integer of 0 to 8, and each $R_{g4}$, the same or different from each other, is independently selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, optionally deuterated $C_{1-6}$ alkoxy (e.g., $C_{1-3}$ alkoxy, such as methoxy, ethoxy, or propoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-4}$ alkylene-, such as $NH_2CH_2$-, $NH_2CH_2CH_2$- and $NH_2CH_2CH_2CH_2$-) or any combination thereof; or

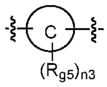,

wherein ring C represents $C_{3-20}$cycloalkylene or 4- to 20-membered heterocyclylene, $(R_{g5})_{n3}$ indicates that ring C is optionally substituted with n3 $R_{g5}$, wherein n3 represents an integer of 0 to 8, and each $R_{g5}$, the same or different from each other, is independently selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, optionally deuterated $C_{1-6}$ alkoxy (e.g., $C_{1-3}$ alkoxy, such as methoxy, ethoxy, or propoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-4}$ alkylene-, such as $NH_2CH_2$-, $NH_2CH_2CH_2$- and $NH_2CH_2CH_2CH_2$-) or any combination thereof;

$R_{12}$ represents H, halogen, $C_{1-3}$ alkyl or halogenated $C_{1-3}$ alkyl (e.g., $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-);

$R_{f3}$ represents H, halogen, $C_{1-3}$ alkyl, halogenated $C_{1-3}$ alkyl (e.g., $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-),

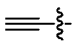, 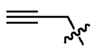, 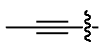 or 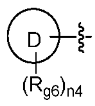,

wherein ring D represents $C_{3-20}$cycloalkylene, 4- to 20-membered heterocyclylene, $C_{6-15}$ arylene or 5- to 20-membered heteroarylene, $(R_{g6})_{n4}$ indicates that ring D is optionally substituted with n4 $R_{g6}$, wherein n4 represents an integer of 0 to 8, and each $R_{g6}$, the same or different from each other, is independently selected

from the group consisting of optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, optionally deuterated $C_{1-6}$ alkoxy (e.g., $C_{1-3}$ alkoxy, such as methoxy, ethoxy, or propoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-4}$ alkylene-, such as $NH_2CH_2$-, $NH_2CH_2CH_2$- and $NH_2CH_2CH_2CH_2$-) or any combination thereof;

$R_{f4}$ represents

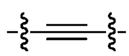, 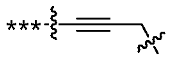, 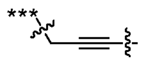 or 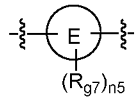,

wherein symbol *** indicates the point of attachment to $R_{f5}$, ring E represents $C_{3-20}$cycloalkylene, 4- to 20-membered heterocyclylene, $C_{6-15}$ arylene or 5- to 20-membered heteroarylene, $(R_{g7})_{n5}$ indicates that ring E is optionally substituted with n5 $R_{g7}$, wherein n5 represents an integer of 0 to 8, and each $R_{g7}$, the same or different from each other, is independently selected from the group consisting of optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, optionally deuterated $C_{1-6}$ alkoxy (e.g., $C_{1-3}$ alkoxy, such as methoxy, ethoxy, or propoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-4}$ alkylene-, such as $NH_2CH_2$-, $NH_2CH_2CH_2$- and $NH_2CH_2CH_2CH_2$-) or any combination thereof; and

$R_{f5}$ represents H, halogen, $C_{1-3}$ alkyl, halogenated $C_{1-3}$ alkyl,

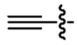, 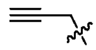, 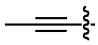

or

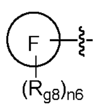,

wherein ring F represents $C_{3-20}$cycloalkyl, 4- to 20-membered heterocyclyl, $C_{6-15}$ aryl or 5- to 20-membered heteroaryl, $(R_{g8})_{n6}$ indicates that ring F is optionally substituted with n6 $R_{g8}$, wherein n6 represents an integer of 0 to 8, and each $R_{g8}$, the same or different from each other, is independently selected from the group consisting of optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, optionally deuterated $C_{1-6}$ alkoxy (e.g., $C_{1-3}$ alkoxy, such as methoxy, ethoxy, or propoxy), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-4}$ alkylene-, such as $NH_2CH_2$-, $NH_2CH_2CH_2$- and $NH_2CH_2CH_2CH_2$-) or any combination thereof;

In some embodiments of the compound of Formula (I-2), $X_1$ represents a bond.

In some embodiments of the compound of Formula (I-2), $X_1$ represents: $N(R_{e1})$, O, S, $C_{2-6}$ alkynylene, $C_{2-6}$ alkenylene, optionally substituted $C_{3-20}$cycloalkylene, optionally substituted 4- to 20-membered heterocyclylene, optionally substituted $C_{6-15}$ arylene, or optionally substituted 5- to 20-membered heteroarylene, wherein $R_{e1}$ represents H or $C_{1-3}$ alkyl, wherein the $C_{3-20}$cycloalkylene, the 4-to 20-membered heterocyclylene, the $C_{6-15}$ arylene, and the 5- to 20-membered heteroarylene are each independently optionally substituted with one or more (e.g., 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally

deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof.

In some embodiments of the compound of Formula (I-2), $X_1$ represents $N(R_{e1})$, O, S, $C_{2-6}$ alkynylene or $C_{2-6}$ alkenylene, wherein $R_{e1}$ represents H or $C_{1-3}$ alkyl.

In some embodiments of the compound of Formula (I-2), $X_1$ represents:

cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, bornylene, bicyclo[2.2.1]heptanylene, 2-oxobicyclo[2.2.1]heptanylene or bicyclo[2.2.1]heptenylene, with each group being optionally substituted with one or more (e.g., 1-10, 1-9, 1-8, 1-7, 1-6, 1-5, 1-4, 1-3 or 1-2, or 1) substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof;

phenylene or naphthylene, wherein the phenylene or naphthylene is optionally substituted with one or more (e.g., 1-5, 1-4, 1-3 or 1-2, or 1) substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof;

azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, azacycloheptanylene, azacyclooctylene, diazacycloheptanylene, diazacyclooctylene, 3-azabicyclo[3.1.0]hexylene, 6-azabicyclo[3.1.1]heptanylene, 2,5-diazabicyclo[2.2.1]heptanyl, 3,6-diazabicyclo[3.1.1]heptanyl, 3-azabicyclo[3.2.1]octylene, 3,8-diazabicyclo[3.2.1]octylene, 2,5-diazabicyclo[2.2.2]octylene, 3-azaspiro[5.5]undecylene or 7-azaspiro[3.5]nonylene, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof; or

furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene or imidazo[2,1-b]thiazolylene, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof. In embodiments of the present disclosure, the number of substituents is not theoretically limited in any way or automatically limited by the size of the building units.

In some embodiments of the compound of Formula (I-2), $X_1$ represents NH, $N(CH_3)$, O, S, ethynylene or vinylene.

In some embodiments of the compound of Formula (I-2), $X_1$ represents:

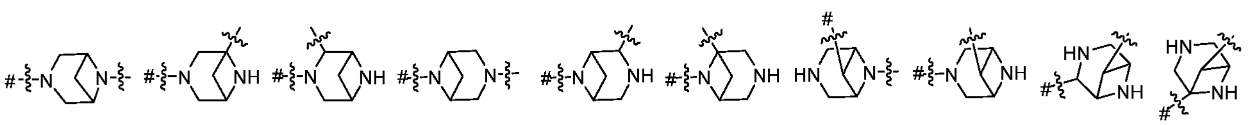

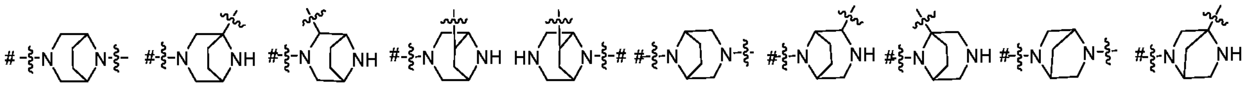

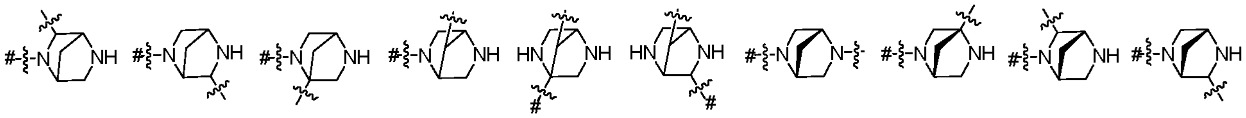

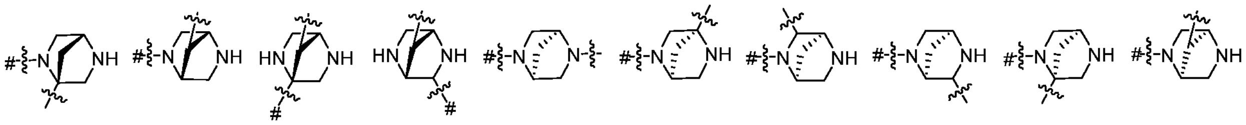

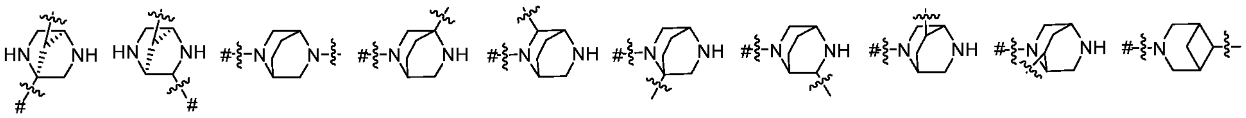

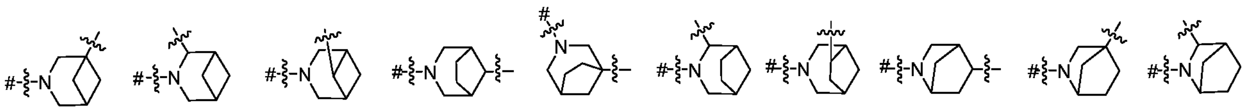

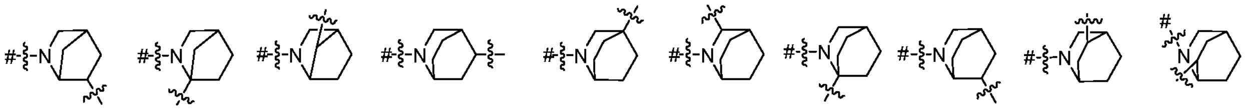

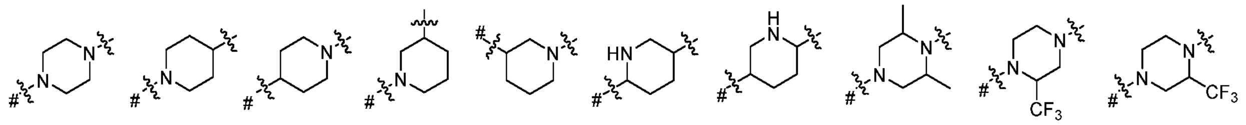

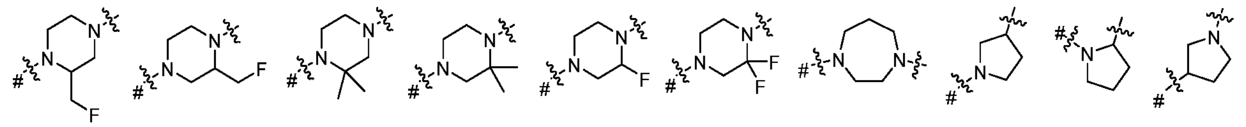

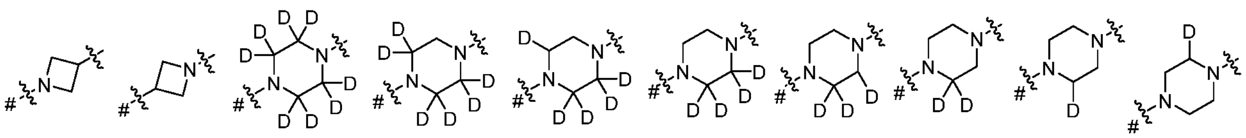

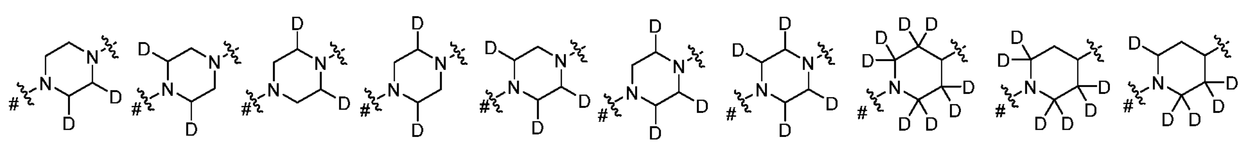

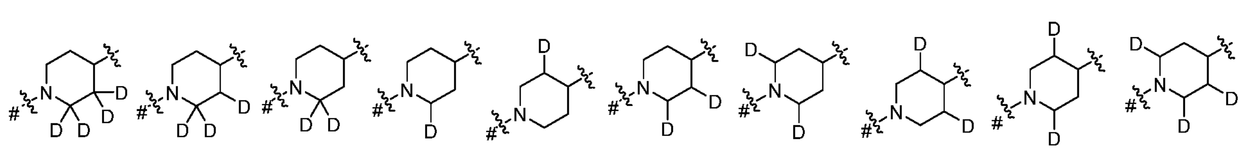

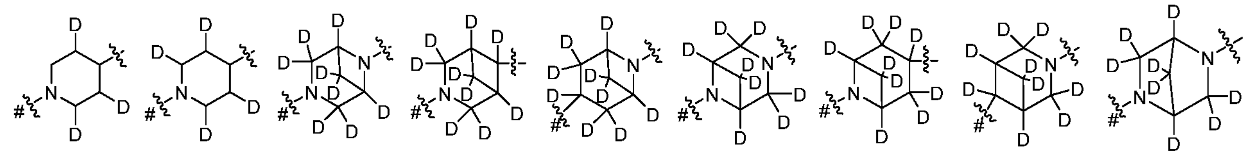

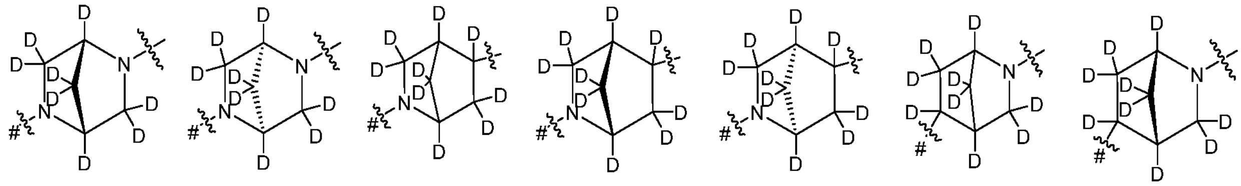

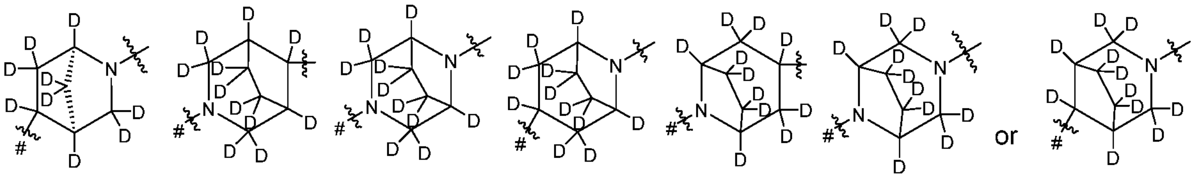

or ,

wherein symbol # indicates the point of attachment to $L_1$ .

In some embodiments of the compound of Formula (I-2), $R_{a1}$ represents the following formula:

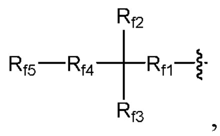

,

wherein $R_{f1}$ represents:

$-NCH_2CH_2N-$;

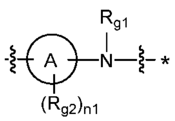

,

wherein symbol * indicates the point of attachment to $X_2$, $R_{g1}$ represents H or $C_{1-3}$ alkyl, and ring A represents: cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, bornylene, bicyclo[2.2.1]heptanylene, 2-oxobicyclo[2.2.1]heptanylene, bicyclo[2.2.1]heptenylene, azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, azacycloheptanylene, azacyclooctylene, diazacycloheptanylene, diazacyclooctylene, 3-azabicyclo[3.1.0]hexylene, 6-azabicyclo[3.1.1]heptanylene, 2,5-diazabicyclo[2.2.1]heptanyl, 3,6-diazabicyclo[3.1.1]heptanyl, 3-azabicyclo[3.2.1]octylene, 3,8-diazabicyclo[3.2.1]octylene, 2,5-diazabicyclo[2.2.2]octylene, 3-azaspiro[5.5]undecylene or 7-azaspiro[3.5]nonylene, and $(R_{g2})_{n1}$ indicates that ring A is optionally substituted with n1 $R_{g2}$, wherein n1 represents an integer of 0 to 8, and each $R_{g2}$, the same or different from each other, is independently selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

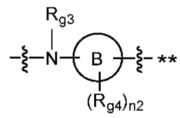,

wherein symbol ** indicates the point of attachment to $X_2$, $R_{g3}$ represents H or $C_{1-3}$ alkyl, and ring B represents: cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, bornylene, bicyclo[2.2.1]heptanylene, 2-oxobicyclo[2.2.1]heptanylene, bicyclo[2.2.1]heptenylene, azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, azacycloheptanylene, azacyclooctylene, diazacycloheptanylene, diazacyclooctylene, 3-azabicyclo[3.1.0]hexylene, 6-azabicyclo[3.1.1]heptanylene, 2,5-diazabicyclo[2.2.1]heptanyl, 3,6-diazabicyclo[3.1.1]heptanyl, 3-azabicyclo[3.2.1]octylene, 3,8-diazabicyclo[3.2.1]octylene, 2,5-diazabicyclo[2.2.2]octylene, 3-azaspiro[5.5]undecylene or 7-azaspiro[3.5]nonylene, and $(R_{g4})_{n2}$ indicates that ring B is optionally substituted with n2 $R_{g4}$, wherein n2 represents an integer of 0 to 8, and each $R_{g4}$, the same or different from each other, is independently selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof; or

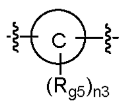,

wherein ring C represents: cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, bornylene, bicyclo[2.2.1]heptanylene, 2-oxobicyclo[2.2.1]heptanylene, bicyclo[2.2.1]heptenylene, azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, azacycloheptanylene, azacyclooctylene, diazacycloheptanylene, diazacyclooctylene, 3-azabicyclo[3.1.0]hexylene, 6-azabicyclo[3.1.1]heptanylene, 2,5-diazabicyclo[2.2.1]heptanyl, 3,6-diazabicyclo[3.1.1]heptanyl, 3-azabicyclo[3.2.1]octylene, 3,8-diazabicyclo[3.2.1]octylene, 2,5-diazabicyclo[2.2.2]octylene, 3-azaspiro[5.5]undecylene or 7-azaspiro[3.5]nonylene, and $(R_{g5})_{n3}$ indicates that ring C is optionally substituted with n3 $R_{g5}$, n3 represents an integer of 0 to 8, and each $R_{g5}$, the same or different from each other, is independently selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

$R_{f2}$ represents H, halogen, $C_{1-3}$ alkyl or halogenated $C_{1-3}$ alkyl;

$R_{f3}$ represents H, halogen, $C_{1-3}$ alkyl, halogenated $C_{1-3}$ alkyl,

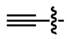, 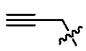, 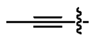 or 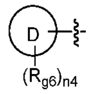,

wherein ring D represents: cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, bornylene, bicyclo[2.2.1]heptanylene, 2-oxobicyclo[2.2.1]heptanylene, bicyclo[2.2.1]heptenylene, azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, azacycloheptanylene, azacyclooctylene, diazacycloheptanylene, diazacyclooctylene, 3-azabicyclo[3.1.0]hexylene, 6-azabicyclo[3.1.1]heptanylene, 2,5-diazabicyclo[2.2.1]heptanyl, 3,6-diazabicyclo[3.1.1]heptanyl, 3-azabicyclo[3.2.1]octylene, 3,8-diazabicyclo[3.2.1]octylene, 2,5-diazabicyclo[2.2.2]octylene, 3-azaspiro[5.5]undecylene, 7-azaspiro[3.5]nonylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene or imidazo[2,1-b]thiazolylene, and

$(R_{g6})_{n4}$ indicates that ring D is optionally substituted with n4 $R_{g6}$, wherein n4 represents an integer of 0 to 8, and each $R_{g6}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

$R_{f4}$ represents

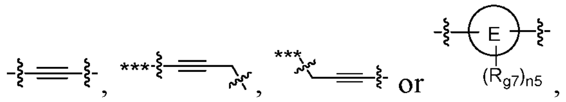

, , or ,

wherein symbol *** indicates the point of attachment to $R_{f5}$, and ring E represents: cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, bornylene, bicyclo[2.2.1]heptanylene, 2-oxobicyclo[2.2.1]heptanylene, bicyclo[2.2.1]heptenylene, azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, azacycloheptanylene, azacyclooctylene, diazacycloheptanylene, diazacyclooctylene, 3-azabicyclo[3.1.0]hexylene, 6-azabicyclo[3.1.1]heptanylene, 2,5-diazabicyclo[2.2.1]heptanyl, 3,6-diazabicyclo[3.1.1]heptanyl, 3-azabicyclo[3.2.1]octylene, 3,8-diazabicyclo[3.2.1]octylene, 2,5-diazabicyclo[2.2.2]octylene, 3-azaspiro[5.5]undecylene, 7-azaspiro[3.5]nonylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene or imidazo[2,1-b]thiazolylene, and $(R_{g7})_{n5}$ indicates that ring E is optionally substituted with n5 $R_{g7}$, wherein n5 represents an integer of 0 to 8, and each $R_{g7}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof; and

$R_{f5}$ represents H, halogen, $C_{1-3}$ alkyl, halogenated $C_{1-3}$ alkyl,

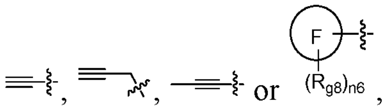

, , or ,

wherein ring F represents: cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, 2-oxobicyclo[2.2.1]heptanyl, bicyclo[2.2.1]heptenyl, azetidinyl, oxetanyl,

pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 3-azaspiro[5.5]undecyl, 7-azaspiro[3.5]nonyl, phenyl, naphthyl, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl or imidazo[2,1-b]thiazolyl, and $(R_{g8})_{n6}$ indicates that ring F is optionally substituted with n6 $R_{g8}$, wherein n6 represents an integer of 0 to 8, and each $R_{g8}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof.

In some embodiments of the compound of Formula (I-2), $R_{a1}$ represents the following formula:

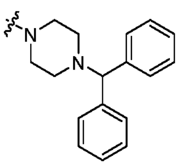 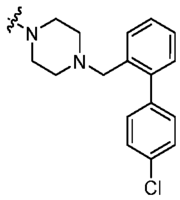 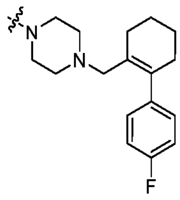 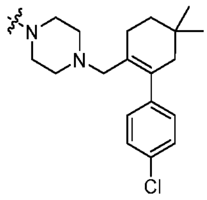 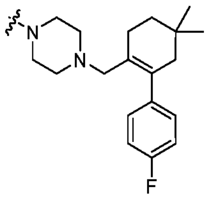 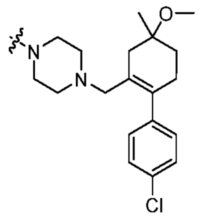

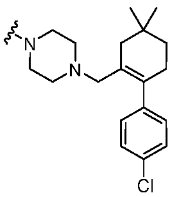 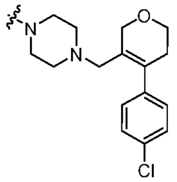 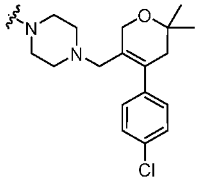 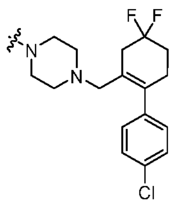 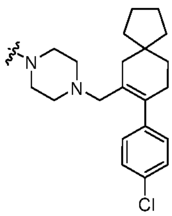 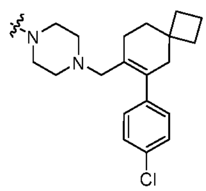

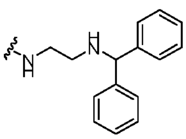 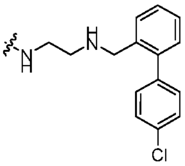 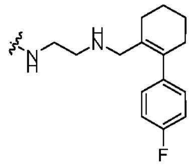 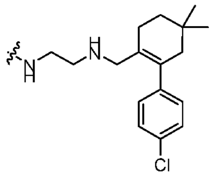 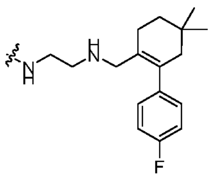 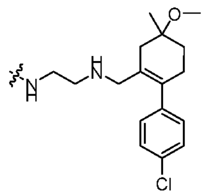

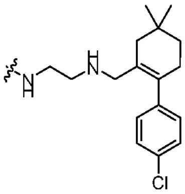 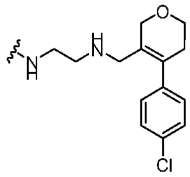 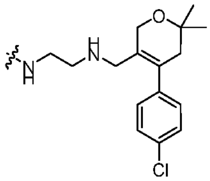 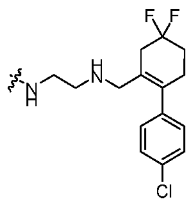 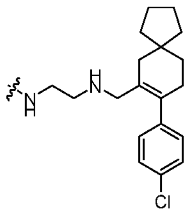 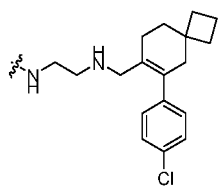

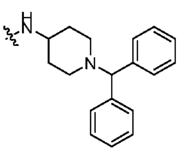 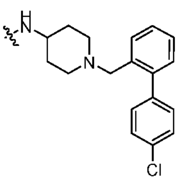 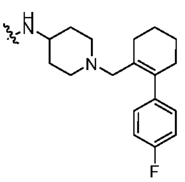 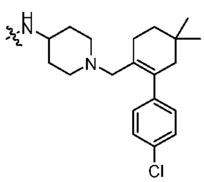 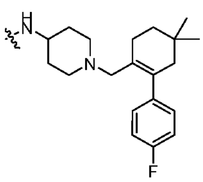 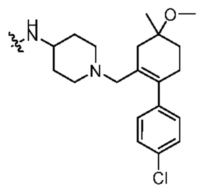

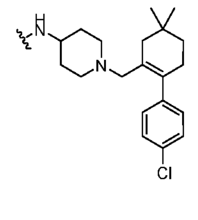 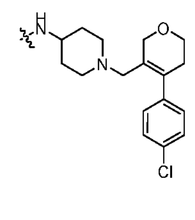 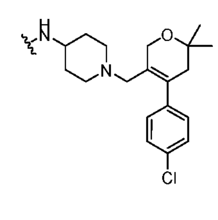 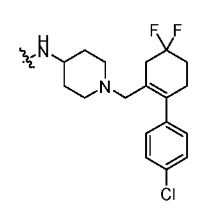 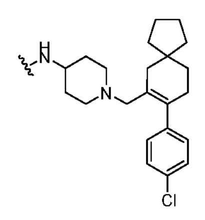 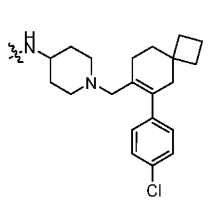

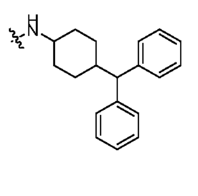 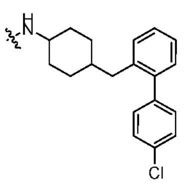 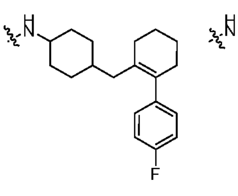 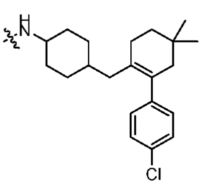 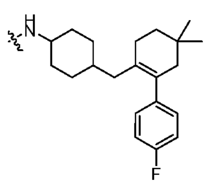 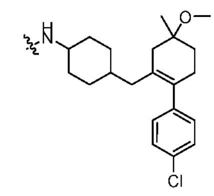

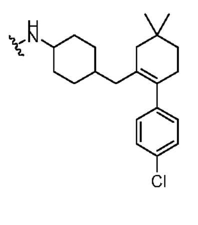 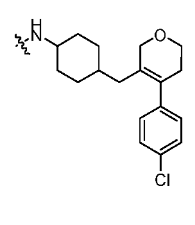 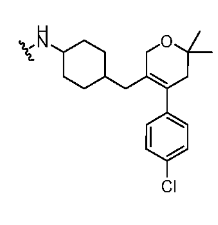 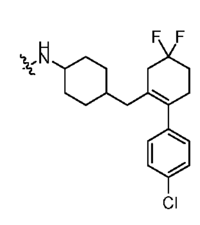 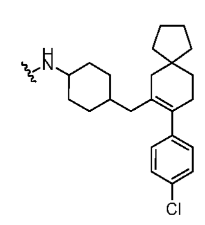 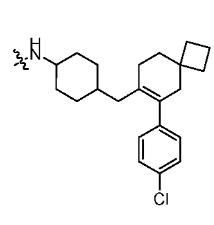

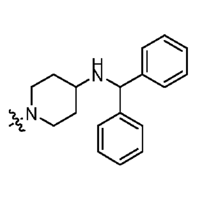 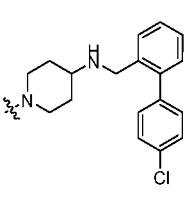 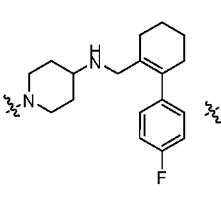 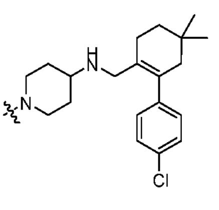 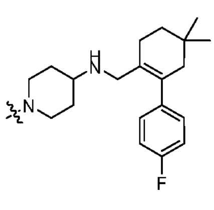 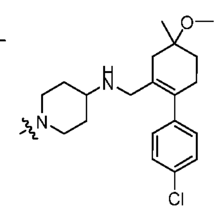

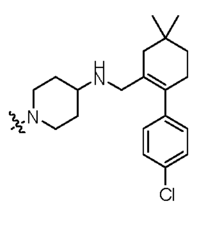 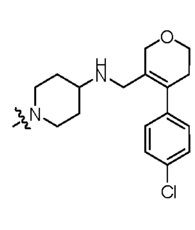 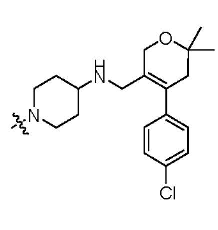 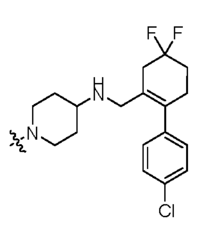 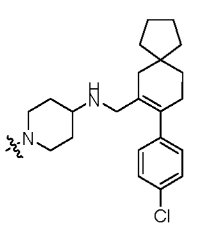 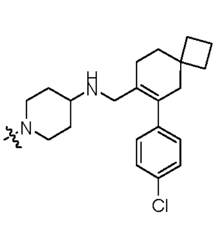

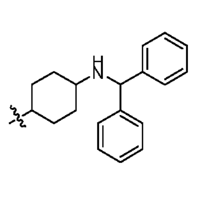 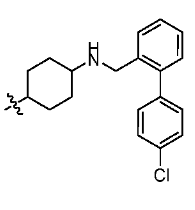 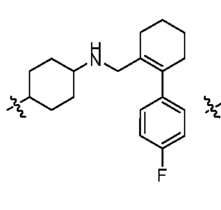 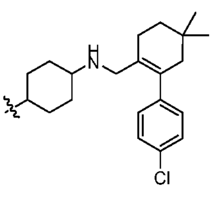 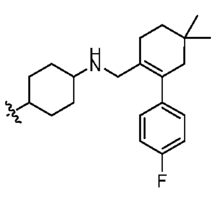 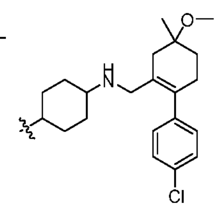

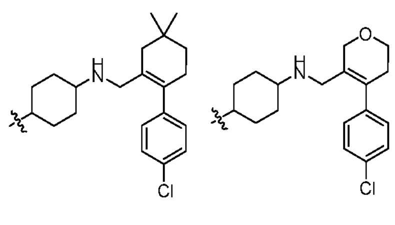 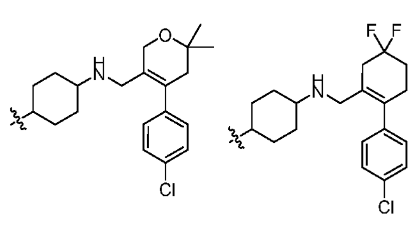 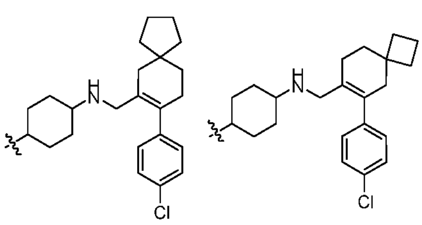

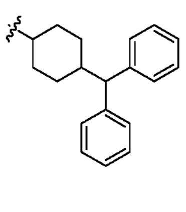
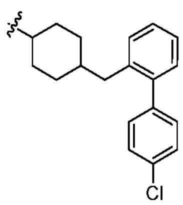
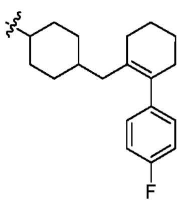
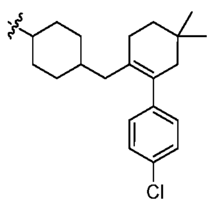
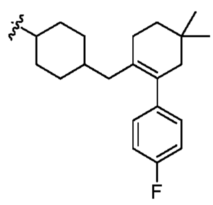
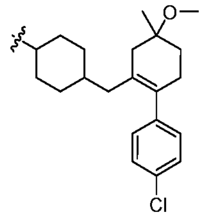
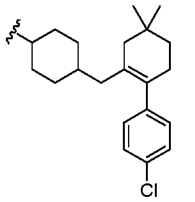
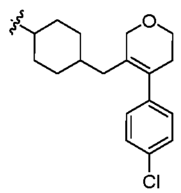
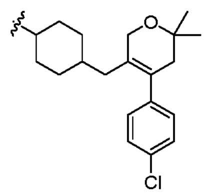
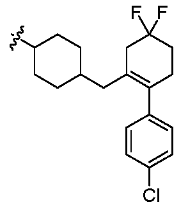
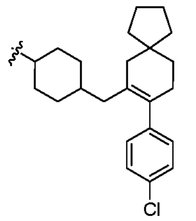
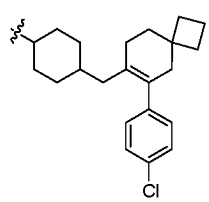
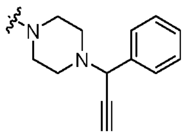
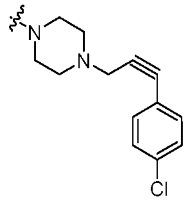
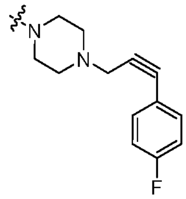
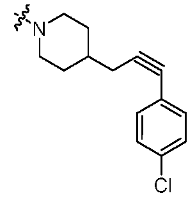
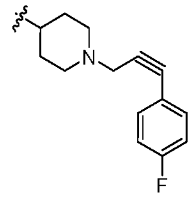
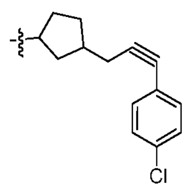
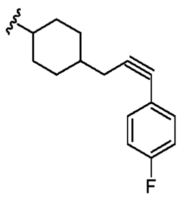
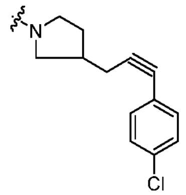
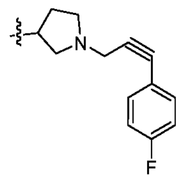
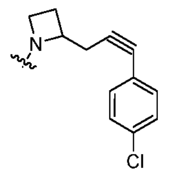
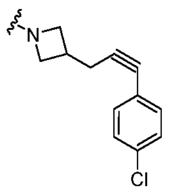
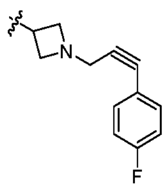
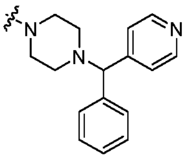
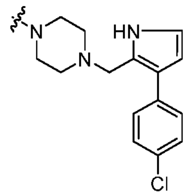
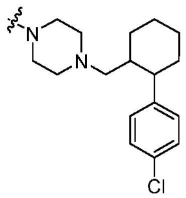
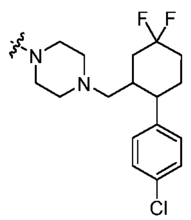
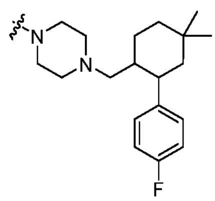
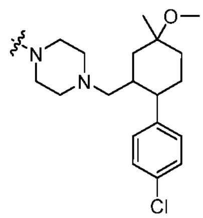
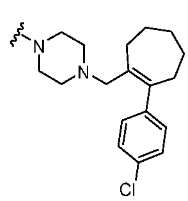
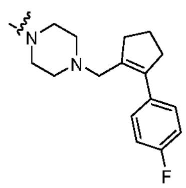
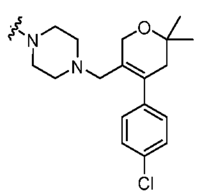
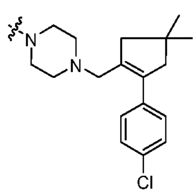
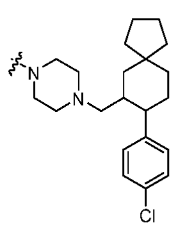
or
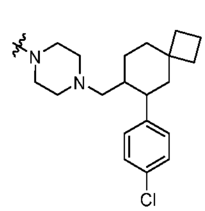

In some embodiments of the compound of Formula (I-2), $L_1$ represents the structure of the following formula:

$$\#\#\text{-}(C(R^{a1})(R^{a2}))_{m1}\text{-}(R_{e2}(C(R^{a3})(R^{a4}))_{m2})_{p1}\text{-};$$

$$\#\#\text{-}(C(R^{a1})(R^{a2}))_{m1}\text{-}(R_{e2}(C(R^{a3})(R^{a4}))_{m2})_{p1}\text{-}(R_{e2}(C(R^{a5})(R^{a6}))_{m3})_{p2}\text{-};$$

$$\#\#\text{-}(C(R^{a1})(R^{a2}))_{m1}\text{-}(R_{e2}(C(R^{a3})(R^{a4}))_{m2})_{p1}\text{-}(R_{e2}(C(R^{a5})(R^{a6}))_{m3})_{p2}\text{-}(Re_2(C(R^{a7})(R^{a8}))_{m4})_{p3}\text{-};$$

$$\#\#\text{-}(C(R^{a1})(R^{a2}))_{m1}\text{-}(R_{e3}(C(R^{a3})(R^{a4}))_{m2})_{p1}\text{-};$$

$$\#\#\text{-}(C(R^{a1})(R^{a2}))_{m1}\text{-}(R_{e3}(C(R^{a3})(R^{a4}))_{m2})_{p1}\text{-}(R_{e3}(C(R^{a5})(R^{a6}))_{m3})_{p2}\text{-};$$

$$\#\#\text{-}(C(R^{a1})(R^{a2}))_{m1}\text{-}(R_{e3}(C(R^{a3})(R^{a4}))_{m2})_{p1}\text{-}(R_{e3}(C(R^{a5})(R^{a6})_{m3})_{p2}\text{-}(R^{e3}(C(R^{a7})(R^{a8}))_{m4})_{p3}\text{-};$$

$$\#\#\text{-}(C(R^{a1})(R^{a2}))_{m1}\text{-}(R_{e3}(C(R^{a3})(R^{a4}))_{m2})_{p1}\text{-}(R_{e3}(C(R^{a5})(R^{a6}))_{m3})_{p2}\text{-}(R_{e3}(C(R^{a7})(R^{a8}))_{m4})_{p3}\text{-};$$

$$\#\#\text{-}(C(R^{a1})(R^{a2}))_{m1}\text{-}(R_{e2}\text{-}R_{e3}\text{-}(C(R^{a3})(R^{a4}))_{m2})_{p1}\text{-};$$

$$\#\#\text{-}(C(R^{a1})(R^{a2}))_{m1}\text{-}(R_{e2}(C(R^{a3})(R^{a4}))_{m2})_{p1}\text{-}(R_{e3}(C(R^{a5})(R^{a6}))_{m3})_{p2}\text{-};$$

$$\#\#\text{-}(C(R^{a1})(R^{a2}))_{m1}\text{-}(R_{e3}\text{-}R_{e2}\text{-}(C(R^{a3})(R^{a4}))_{m2})_{p1}\text{-};\ \text{or}$$

$$\#\#\text{-}(C(R^{a1})(R^{a2}))_{m1}\text{-}(R_{e3}(C(R^{a3})(R^{a4}))_{m2})_{p1}\text{-}(R_{e2}(C(R^{a5})(R^{a6}))_{m3})_{p2}\text{-};$$

wherein each group $R_{e2}$ is independently selected from the group consisting of O, S, N($R_{e4}$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_{e4}$ independently represents H or $C_{1-3}$ alkyl; and each group $R_{e3}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, optionally deuterated $C_{1-6}$ alkoxy (e.g., $C_{1-3}$ alkoxy, such as methoxy, ethoxy, or propoxy), optionally deuterated $C_{1-6}$ alkyl-NH- (e.g., $C_{1-3}$ alkyl-NH-, such as $CH_3NH$-, $CH_3CH_2NH$- or $CH_3CH_2CH_2NH$-), halogenated $C_{1-6}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH-(e.g., $C_{1-4}$ alkyl-C(O)NH-, such as $CH_3$-C(O) NH-, $CH_3CH_2$-C(O)NH-, and $CH_3CH_2CH_2$-C(O)NH-) or any combination thereof;

$R^{a1}$, $R^{a2}$, $R^{a3}$, $R^{a4}$, $R^{a5}$, $R^{a6}$, $R^{a7}$ and $R^{a8}$ each independently represent H, deuterium, hydroxy, amino, mercapto, nitro, halogen, cyano, optionally deuterated $C_{1-6}$ alkyl (e.g., optionally deuterated $C_{1-4}$ alkyl, such as methyl, $CD_3$, ethyl, propyl, isopropyl, butyl, sec-butyl or tert-butyl), halogenated $C_{1-6}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), optionally deuterated $C_{1-6}$ alkoxy (e.g., $C_{1-3}$ alkoxy, such as methoxy, ethoxy, or propoxy), optionally deuterated $C_{1-6}$ alkyl-NH- (e.g., $C_{1-3}$ alkyl-NH-, such as $CH_3NH$-, $CH_3CH_2NH$- or $CH_3CH_2CH_2NH$-), $NH_2$-$C_{1-6}$ alkylene (e.g., $NH_2$-$C_{1-3}$ alkylene-, such as $NH_2CH_2$-, $NH_2CH_2CH_2$- and $NH_2CH_2CH_2CH_2$-), optionally deuterated $C_{1-6}$ alkyl-NHC(O)- (e.g., $C_{1-4}$ alkyl-NHC(O)-, such as $CH_3$-NHC(O)-, $CH_3CH_2$-NHC(O)-, and $CH_3CH_2CH_2$-NHC(O)-), optionally deuterated $C_{1-6}$ alkyl-C(O)NH- (e.g., $C_{1-4}$ alkyl-C(O)NH-, such as $CH_3$-C(O)NH-, $CH_3CH_2$-C(O)NH-, and $CH_3CH_2CH_2$-C(O)NH-) or any combination thereof;

symbol ## indicates the point of attachment to $X_1$; and

m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-2), $L_1$ represents the structure of the following formula:

$$\#\#\text{-}(C(R^{a1})(R^{a2}))_{m1}\text{-}(O(C(R^{a3})(R^{a4}))_{m2})_{p1}\text{-};$$

$$\#\#\text{-}(C(R^{a1})(R^{a2}))_{m1}\text{-}(O(C(R^{a3})(R^{a4}))_{m2})_{p1}\text{-}(O(C(R^{a5})(R^{a6}))_{m3})_{p2}\text{-};$$

$$\#\#\text{-}(C(R^{a1})(R^{a2}))_{m1}\text{-}(O(C(R^{a3})(R^{a4}))_{m2})_{p1}\text{-}(O(C(R^{a5})(R^{a6})_{m3})_{p2}\text{-}(O(C(R^{a7})(R^{a8})_{m4})_{p3}\text{-};$$

$$\#\#\text{-}(C(R^{a1})(R^{a2}))_{m1}\text{-}(N(R_{g9})(C(R^{a3})(R^{a4}))_{m2})_{p1}\text{-};$$

$\#\#\text{-}(C(R^{a1})(R^{a2}))_{m1}\text{-}(N(R_{g9})(C(R^{a3})(R^{a4}))_{m2})_{p1}\text{-}(N(R_{g9})(C(R^{a5})(R^{a6}))_{m3})_{p2}\text{-}$;

$\#\#\text{-}(C(R^{a1})(R^{a2}))_{m1}\text{-}(N(R_{g9})(C(R^{a3})(R^{a4})_{m2})_{p1}\text{-}(N(R_{g9})(C(R^{a5})(R^{a6}))_{m3})_{p2}\text{-}$;

$\#\#\text{-}(C(R^{a1})(R^{a2}))_{m1}\text{-}(N(R_{g9})(C(R^{a3})(R^{a4}))_{m2})_{p1}\text{-}(N(R_{g9})(C(R^{a5})(R^{a6}))_{m3})_{p2}\text{-}(N(R_{g9})(C(R^{a7})(R^{a8}))_{m4})_{p3}\text{-}$;

$\#\#\text{-}(C(R^{a1})(R^{a2})_{m1}\text{-}(C(O)NH\text{-}(C(R^{a3})(R^{a4})_{m2})_{p1}\text{-}$;

$\#\#\text{-}(C(R^{a1})(R^{a2}))_{m1}\text{-}(C(O)NH\text{-}(C(R^{a3})(R^{a4})_{m2})_{p1}\text{-}(C(O)NH\text{-}(C(R^{a5})(R^{a6}))_{m3})_{p2}\text{-}$;

$\#\#\text{-}(C(R^{a1})(R^{a2}))_{m1}\text{-}(C(O)NH\text{-}(C(O)NH\text{-}(C(R^{a3})(R^{a4}))_{m2})_{p1}\text{-}(C(O)NH\text{-}(C(R^{a5})(R^{a6}))_{m3})_{p2}\text{-}(C(O)NH\text{-}(C(R^{a7})(R^{a8}))_{m4})_{p3}\text{-}$;

$\#\#\text{-}(C(R^{a1})(R^{a2}))_{m1}\text{-}(C(O)NH\text{-}(C(R^{a3})(R^{a4})_{m2})_{p1}\text{-}(O\text{-}(C(R^{a5})(R^{a6}))_{m3})_{p2}\text{-}$;

$\#\#\text{-}(C(R^{a1})(R^{a2}))_{m1}\text{-}C(O)NH\text{-}(C(R^{a3})(R^{a4}))_{m2}\text{-}(O(C(R^{a5})(R^{a6}))_{m3})_{p1}\text{-}$;

$\#\#\text{-}(C(R^{a1})(R^{a2}))_{m1}\text{-}(NHC(O)\text{-}(C(R^{a3})(R^{a4}))_{m2})_{p1}\text{-}$;

$\#\#\text{-}(C(R^{a1})(R^{a2}))_{m1}\text{-}(NHC(O)\text{-}(C(R^{a3})(R^{a4}))_{m2})_{p1}\text{-}(O\text{-}(C(R^{a5})(R^{a6}))_{m3})_{p2}\text{-}$;

$\#\#\text{-}(C(R^{a1})(R^{a2}))_{m1}\text{-}(NHC(O)\text{-}(C(R^{a3})(R^{a4}))_{m2})_{p1}\text{-}(O\text{-}(C(R^{a5})(R^{a6}))_{m3})_{p2}\text{-}(O\text{-}(C(R^{a7})(R^{a8}))_{m4})_{p3}\text{-}$;

$\#\#\text{-}(C(R^{a1})(R^{a2}))_{m1}\text{-}NHC(O)\text{-}(C(R^{a3})(R^{a4}))_{m2}\text{-}(O(C(R^{a5})(R^{a6}))_{m3})_{p1}\text{-}$;

$\#\#\text{-}(C(R^{a1})(R^{a2}))_{m1}\text{-}NHC(O)NH\text{-}(C(R^{a3})(R^{a4}))_{m2}\text{-}$;

$\#\#\text{-}(C(R^{a1})(R^{a2}))_{m1}\text{-}C(O)\text{-}(C(R^{a3})(R^{a4}))_{m2}\text{-}$;

$\#\#\text{-}(C(R^{a1})(R^{a2})_{m1}\text{-}C(S)\text{-}(C(R^{a3})(R^{a4}))_{m2}\text{-}$;

$\#\#\text{-}(C(R^{a1})(R^{a2}))_{m1}\text{-}S\text{-}(C(R^{a3})(R^{a4}))_{m2}\text{-}$;

$\#\#\text{-}(C(R^{a1})(R^{a2}))_{m1}\text{-}(C_{6\text{-}10}$ arylene-$(C(R^{a3})(R^{a4}))_{m2})_{p1}\text{-}$;

$\#\#\text{-}(C(R^{a1})(R^{a2}))_{m1}\text{-}(C_{6\text{-}10}$ arylene-$(C(R^{a3})(R^{a4}))_{m2})_{p1}\text{-}C_{6\text{-}10}$ arylene-$(C(R^{a5})(R^{a6}))_{m3}\text{-}$;

$\#\#\text{-}(C(R^{a1})(R^{a2}))_{m1}$-(4- to 15-membered heterocyclylene-$(C(R^{a3})(R^{a4}))_{m2})_{p1}\text{-}$;

$\#\#\text{-}(C(R^{a1})(R^{a2}))_{m1}$-(4- to 15-membered heterocyclylene-$(C(R^{a3})(R^{a4}))_{m2})_{p1}$-(4- to 15-membered heterocyclylene-$(C(R^{a5})(R^{a6}))_{m3})_{p2}\text{-}$;

$\#\#\text{-}(C(R^{a1})(R^{a2}))_{m1}$-(5- to 15-membered heteroarylene-$(C(R^{a3})(R^{a4}))_{m2})_{p1}\text{-}$;

$\#\#\text{-}(C(R^{a1})(R^{a2}))_{m1}$-(5- to 15-membered heteroarylene-$(C(R^{a3})(R^{a4}))_{m2})_{p1}$-(5- to 15-membered heteroarylene-$(C(R^{a5})(R^{a6}))_{m3})_{p2}\text{-}$;

$\#\#\text{-}(C(R^{a1})(R^{a2}))_{m1}\text{-}(C_{3\text{-}15}$cycloalkylene-$(C(R^{a3})(R^{a4}))_{m2})_{p1}\text{-}$; or

$\#\#\text{-}(C(R^{a1})(R^{a2}))_{m1}\text{-}(C_{3\text{-}15}$cycloalkylene-$(C(R^{a3})(R^{a4}))_{m2})_{p1}\text{-}(C_{3\text{-}15}$cycloalkylene-$(C(R^{a5})(R^{a6}))_{m3})_{p2}\text{-}$;

wherein each $R_{g9}$ independently represents H or $C_{1\text{-}3}$ alkyl;
the $C_{3\text{-}15}$cycloalkylene, the $C_{6\text{-}10}$ arylene, the 4- to 15-membered heterocyclylene and the 5- to 15-membered

heteroarylene are each independently optionally substituted with a substituent selected from the group consisting of deuterium, optionally deuterated $C_{1-6}$ alkyl, optionally deuterated $C_{3-6}$cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano or any combination thereof;

$R^{a1}$, $R^{a2}$, $R^{a3}$, $R^{a4}$, $R^{a5}$, $R^{a6}$, $R^{a7}$ and $R^{a8}$ each independently represent H, deuterium, hydroxy, amino, mercapto, nitro, halogen, cyano, optionally deuterated $C_{1-6}$ alkyl, halogenated $C_{1-6}$ alkyl, optionally deuterated $C_{3-6}$cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof;

symbol ## indicates the point of attachment to $X_1$; and

m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-2), $L_1$ represents the following group:
##-$CH_2$-O-$CH_2$-, ##-$CH_2$-O-$(CH_2)_2$-, ##-$(CH_2)_1$-O-$(CH_2)_3$-, ##-$(CH_2)_1$-O-$(CH_2)_4$-, ##-$(CH_2)_1$-O-$(CH_2)_5$-, ##-$(CH_2)_1$-O-$(CH_2)_6$-, ##-$(CH_2)_1$-O-$(CH_2)_7$-, ##-$(CH_2)_1$-O-$(CH_2)_8$-, ##-$(CH_2)_1$-O-$(CH_2)_9$-, ##-$(CH_2)_1$-O-$(CH_2)_{10}$-, ##-$(CH_2)_2$-O-$(CH_2)_1$-, ##-$(CH_2)_2$-O-$(CH_2)_2$-, ##-$(CH_2)_2$-O-$(CH_2)_3$-, ##-$(CH_2)_2$-O-$(CH_2)_4$-, ##-$(CH_2)_2$-O-$(CH_2)_5$-, ##-$(CH_2)_2$-O-$(CH_2)_6$-, ##-$(CH_2)_2$-O-$(CH_2)_7$-, ##-$(CH_2)_2$-O-$(CH_2)_8$-, ##-$(CH_2)_2$-O-$(CH_2)_9$-, ##-$(CH_2)_2$-O-$(CH_2)_{10}$-, ##-$(CH_2)_2$-O-$(CH_2)_{11}$-, ##-$(CH_2)_2$-O-$(CH_2)_{12}$-, ##-$(CH_2)_3$-O-$(CH_2)_1$-, ##-$(CH_2)_3$-O-$(CH_2)_2$-, ##-$(CH_2)_3$-O-$(CH_2)_3$-, ##-$(CH_2)_3$-O-$(CH_2)_4$-, ##-$(CH_2)_3$-O-$(CH_2)_5$-, ##-$(CH_2)_3$-O-$(CH_2)_6$-, ##-$(CH_2)_3$-O-$(CH_2)_7$-, ##-$(CH_2)_4$-O-$(CH_2)_1$-, ##-$(CH_2)_4$-O-$(CH_2)_2$-, ##-$(CH_2)_4$-O-$(CH_2)_3$-, ##-$(CH_2)_4$-O-$(CH_2)_4$-, ##-$(CH_2)_4$-O-$(CH_2)_5$-, ##-$(CH_2)_4$-O-$(CH_2)_6$-, ##-$(CH_2)_5$-O-$(CH_2)_1$-, ##-$(CH_2)_5$-O-$(CH_2)_2$-, ##-$(CH_2)_5$-O-$(CH_2)_3$-, ##-$(CH_2)_5$-O-$(CH_2)_4$-, ##-$(CH_2)_5$-O-$(CH_2)_5$-, ##-$(CH_2)_6$-O-$(CH_2)_1$-, ##-$(CH_2)_6$-O-$(CH_2)_2$-, ##-$(CH_2)_6$-O-$(CH_2)_3$-, ##-$(CH_2)_6$-O-$(CH_2)_4$-, ##-$(CH_2)_7$-O-$(CH_2)_1$-, ##-$(CH_2)_7$-O-$(CH_2)_2$-, ##-$(CH_2)_7$-O-$(CH_2)_3$-, ##-$(CH_2)_8$-O-$(CH_2)_1$-, ##-$(CH_2)_8$-O-$(CH_2)_2$-, ##-$CH(CH_3)$-O-$(CH_2)_1$-, ##-$CH(CH_3)$-O-$(CH_2)_2$-, ##-$CH(CH_3)$-O-$(CH_2)_3$-, ##-$CH(CH_3)$-O-$(CH_2)_4$-, ##-$CH(CH_3)$-O-$(CH_2)_5$-, ##-$CH(CH_3)$-O-$(CH_2)_6$-, ##-$CH(CH_3)$-O-$(CH_2)_7$-, ##-$CH(CH_3)$-O-$(CH_2)_8$-, ##-$CH(CH_3)$-O-$(CH_2)_9$-, ##-$CH(CH_3)$-O-$(CH_2)_{10}$-, ##-$CH_2$-$(O(CH_2)_2)_2$-, ##-$CH_2$-$(O(CH_2)_2)_3$-, ##-$CH_2$-$(O(CH_2)_2)_4$-, ##-$CH_2$-$(O(CH_2)_2)_5$-, ##-$CH_2$-$(O(CH_2)_2)_6$-, ##-$CH_2$-$(O(CH_2)_2)_7$-, ##-$CH_2$-$(O(CH_2)_2)_8$-, ##-$CH_2$-$(O(CH_2)_2)_9$-, ##-$CH_2$-$(O(CH_2)_2)_{10}$-, ##-$CH_2$-$(O(CH_2)_2)_1$-$OCH_2$-, ##-$CH_2$-$(O(CH_2)_2)_2$-$OCH_2$-, ##-$CH_2$-$(O(CH_2)_2)_3$-$OCH_2$-, ##-$CH_2$-$(O(CH_2)_2)_4$-$OCH_2$-, ##-$CH_2$-$(O(CH_2)_2)_5$-$OCH_2$-, ##-$CH_2$-$(O(CH_2)_2)_6$-$OCH_2$-, ##-$CH_2$-$(O(CH_2)_2)_7$-$OCH_2$-, ##-$CH_2$-$(O(CH_2)_2)_8$-$OCH_2$-, ##-$CH_2$-$(O(CH_2)_2)_9$-$OCH_2$-, ##-$CH_2$-$(O(CH_2)_2)_{10}$-$OCH_2$-, ##-$(CH_2)_2$-$(O(CH_2)_2)_2$-, ##-$(CH_2)_2$-$(O(CH_2)_2)_3$-, ##-$(CH_2)_2$-$(O(CH_2)_2)_4$-, ##-$(CH_2)_2$-$(O(CH_2)_2)_5$-, ##-$(CH_2)_2$-$(O(CH_2)_2)_6$-, ##-$(CH_2)_2$-$(O(CH_2)_2)_7$-, ##-$(CH_2)_2$-$(O(CH_2)_2)_8$-, ##-$(CH_2)_2$-$(O(CH_2)_2)_9$-, ##-$(CH_2)_2$-$(O(CH_2)_2)_{10}$-, ##-$(CH_2)_3$-$(O(CH_2)_2)_2$-, ##-$(CH_2)_3$-$(O(CH_2)_2)_3$-, ##-$(CH_2)_3$-$(O(CH_2)_2)_4$-, ##-$(CH_2)_3$-$(O(CH_2)_2)_5$-, ##-$(CH_2)_3$-$(O(CH_2)_2)_6$-, ##-$(CH_2)_3$-$(O(CH_2)_2)_7$-, ##-$(CH_2)_3$-$(O(CH_2)_2)_8$-, ##-$(CH_2)_3$-$(O(CH_2)_2)_9$-, ##-$(CH_2)_3$-$(O(CH_2)_2)_{10}$-, ##-$(CH_2)_4$-$(O(CH_2)_2)_2$-, ##-$(CH_2)_4$-$(O(CH_2)_2)_3$-, ##-$(CH_2)_4$-$(O(CH_2)_2)_4$-, ##-$(CH_2)_4$-$(O(CH_2)_2)_5$-, ##-$(CH_2)_4$-$(O(CH_2)_2)_6$-, ##-$(CH_2)_4$-$(O(CH_2)_2)_7$-, ##-$(CH_2)_4$-$(O(CH_2)_2)_8$-, ##-$(CH_2)_4$-$(O(CH_2)_2)_9$-, ##-$(CH_2)_4$-$(O(CH_2)_2)_{10}$-, ##-$CH_2$-$(O(CH_2)_3)_2$-, ##-$CH_2$-$(O(CH_2)_3)_3$-, ##-$CH_2$-$(O(CH_2)_3)_4$-, ##-$CH_2$-$(O(CH_2)_3)_5$-, ##-$CH_2$-$(O(CH_2)_3)_6$-, ##-$CH_2$-$(O(CH_2)_3)_7$-, ##-$CH_2$-$(O(CH_2)_3)_8$-, ##-$CH_2$-$(O(CH_2)_3)_9$-, ##-$CH_2$-$(O(CH_2)_3)_{10}$-, ##-$(CH_2)_2$-$(O(CH_2)_3)_2$-, ##-$(CH_2)_2$-$(O(CH_2)_3)_3$-, ##-$(CH_2)_2$-$(O(CH_2)_3)_4$-, ##-$(CH_2)_2$-$(O(CH_2)_3)_5$-, ##-$(CH_2)_2$-$(O(CH_2)_3)_6$-, ##-$(CH_2)_2$-$(O(CH_2)_3)_7$-, ##-$(CH_2)_2$-$(O(CH_2)_3)_8$-, ##-$(CH_2)_2$-$(O(CH_2)_3)_9$-, ##-$(CH_2)_2$-$(O(CH_2)_3)_{10}$-, ##-$(CH_2)_3$-$(O(CH_2)_3)_2$-, ##-$(CH_2)_3$-$(O(CH_2)_3)_3$-, ##-$(CH_2)_3$-$(O(CH_2)_3)_4$-, ##-$(CH_2)_3$-$(O(CH_2)_3)_5$-, ##-$(CH_2)_3$-$(O(CH_2)_3)_6$-, ##-$(CH_2)_3$-$(O(CH_2)_3)_7$-, ##-$(CH_2)_3$-$(O(CH_2)_3)_8$-, ##-$(CH_2)_3$-$(O(CH_2)_3)_9$-, ##-$(CH_2)_3$-$(O(CH_2)_3)_{10}$-, ##-$CH_2$-O-$(CH_2)_2$-O-$(CH_2)_3$-, ##-$CH_2$-$(O(CH_2)_2)_2$-$(O(CH_2)_3)_2$-, ##-$CH_2$-$(O(CH_2)_2)_3$-$(O(CH_2)_3)_3$-, ##-$CH_2$-$(O(CH_2)_2)_4$-$(O(CH_2)_3)_4$-, ##-$CH_2$-$(O(CH_2)_2)_5$-$(O(CH_2)_3)_5$-, ##-$CH_2$-$(O(CH_2)_2)_6$-$(O(CH_2)_3)_6$-, ##-$(CH_2)_2$-O-$(CH_2)_2$-O-$(CH_2)_3$-, ##-$(CH_2)_2$-$(O(CH_2)_2)_2$-$(O(CH_2)_3)_2$-, ##-$(CH_2)_2$-$(O(CH_2)_2)_3$-$(O(CH_2)_3)_3$-, ##-$(CH_2)_2$-$(O(CH_2)_2)_4$-$(O(CH_2)_3)_4$-, ##-$(CH_2)_2$-$(O(CH_2)_2)_5$-$(O(CH_2)_3)_5$-, ##-$(CH_2)_2$-$(O(CH_2)_2)_6$-$(O(CH_2)_3)_6$-, ##-$(CH_2)_3$-O-$(CH_2)_2$-O-$(CH_2)_3$-, ##-$(CH_2)_3$-$(O(CH_2)_2)_2$-$(O(CH_2)_3)_2$-, ##-$(CH_2)_3$-$(O(CH_2)_2)_3$-$(O(CH_2)_3)_3$-, ##-$(CH_2)_3$-$(O(CH_2)_2)_4$-$(O(CH_2)_3)_4$-, ##-$(CH_2)_3$-$(O(CH_2)_2)_5$-$(O(CH_2)_3)_5$-, ##-$(CH_2)_3$-$(O(CH_2)_2)_6$-$(O(CH_2)_3)_6$-, ##-$CH_2$-O-$(CH_2)_3$-O-$(CH_2)_2$-, ##-$CH_2$-$(O(CH_2)_3)_2$-$(O(CH_2)_2)_2$-, ##-$CH_2$-$(O(CH_2)_3)_3$-$(O(CH_2)_2)_3$-, ##-$CH_2$-$(O(CH_2)_3)_4$-$(O(CH_2)_2)_4$-, ##-$CH_2$-$(O(CH_2)_3)_5$-$(O(CH_2)_2)_5$-, ##-$CH_2$-$(O(CH_2)_3)_6$-$(O(CH_2)_2)_6$-, ##-$(CH_2)_2$-O-$(CH_2)_3$-O-$(CH_2)_2$-, ##-$(CH_2)_2$-$(O(CH_2)_3)_2$-$(O(CH_2)_2)_2$-, ##-$(CH_2)_2$-$(O(CH_2)_3)_3$-$(O(CH_2)_2)_3$-, ##-$(CH_2)_2$-$(O(CH_2)_3)_4$-$(O(CH_2)_2)_4$-, ##-$(CH_2)_2$-$(O(CH_2)_3)_5$-$(O(CH_2)_2)_5$-, ##-$(CH_2)_2$-$(O(CH_2)_3)_6$-$(O(CH_2)_2)_6$-, ##-$(CH_2)_3$-O-$(CH_2)_3$-O-$(CH_2)_2$-, ##-$(CH_2)_3$-$(O(CH_2)_3)_2$-$(O(CH_2)_2)_2$-, ##-$(CH_2)_3$-$(O(CH_2)_3)_3$-$(O(CH_2)_2)_3$-, ##-$(CH_2)_3$-$(O(CH_2)_3)_4$-$(O(CH_2)_2)_4$-, ##-$(CH_2)_3$-$(O(CH_2)_3)_5$-$(O(CH_2)_2)_5$-, ##-$(CH_2)_3$-$(O(CH_2)_3)_6$-$(O(CH_2)_2)_6$-, ##-$CH_2$-O-$(CH_2)_2$-O-$CH_2$-, ##-$(CH_2)_2$-O-$(CH_2)_2$-O-$CH_2$-, ##-$(CH_2)_2$-$(O(CH_2)_2)_2$-O-$(CH_2)_3$-, ##-$(CH_2)_2$-$(O(CH_2)_2)_3$-O-$(CH_2)_3$-, ##-$(CH_2)_2$-$(O(CH_2)_2)_4$-O-$(CH_2)_3$-, ##-$(CH_2)_5$-$(O(CH_2)_2)_2$-O-$(CH_2)_5$-, ##-$(CH_2)_5$-$(O(CH_2)_2)_2$-O-$(CH_2)_6$-, ##-$CH_2$-C(O)-$CH_2$-, ##-$CH_2$-C(O)-$(CH_2)_2$-, ##-$(CH_2)_1$-C(O)-$(CH_2)_3$-, ##-$(CH_2)_1$-C(O)-$(CH_2)_4$-, ##-$(CH_2)_1$-C(O)-$(CH_2)_5$-, ##-$(CH_2)_1$-C(O)-$(CH_2)_6$-,

##-$(CH_2)_1$-C(O)-$(CH_2)_7$-, ##-$(CH_2)_1$-C(O)-$(CH_2)_8$-, ##-$(CH_2)_1$-C(O)-$(CH_2)_9$-, ##-$(CH_2)_1$-C(O)-$(CH_2)_{10}$-, ##-$(CH_2)_2$-C(O)-$(CH_2)_1$-, ##-$(CH_2)_2$-C(O)-$(CH_2)_2$-, ##-$(CH_2)_2$-C(O)-$(CH_2)_3$-, ##-$(CH_2)_2$-C(O)-$(CH_2)_4$-, ##-$(CH_2)_2$-C(O)-$(CH_2)_5$-, ##-$(CH_2)_2$-C(O)-$(CH_2)_6$-, ##-$(CH_2)_2$-C(O)-$(CH_2)_7$-, ##-$(CH_2)_2$-C(O)-$(CH_2)_8$-, ##-$(CH_2)_2$-C(O)-$(CH_2)_9$-, ##-$(CH_2)_2$-C(O)-$(CH_2)_{10}$-, ##-$(CH_2)_2$-C(O)-$(CH_2)_{11}$-, ##-$(CH_2)_2$-C(O)-$(CH_2)_{12}$-, ##-$(CH_2)_3$-C(O)-$(CH_2)_1$-, ##-$(CH_2)_3$-C(O)-$(CH_2)_2$-, ##-$(CH_2)_3$-C(O)-$(CH_2)_3$-, ##-$(CH_2)_3$-C(O)-$(CH_2)_4$-, ##-$(CH_2)_3$-C(O)-$(CH_2)_5$-, ##-$(CH_2)_3$-C(O)-$(CH_2)_6$-, ##-$(CH_2)_3$-C(O)-$(CH_2)_7$-, ##-$(CH_2)_4$-C(O)-$(CH_2)_1$-, ##-$(CH_2)_4$-C(O)-$(CH_2)_2$-, ##-$(CH_2)_4$-C(O)-$(CH_2)_3$-, ##-$(CH_2)_4$-C(O)-$(CH_2)_4$-, ##-$(CH_2)_4$-C(O)-$(CH_2)_5$-, ##-$(CH_2)_4$-C(O)-$(CH_2)_6$-, ##-$(CH_2)_5$-C(O)-$(CH_2)_1$-, ##-$(CH_2)_5$-C(O)-$(CH_2)_2$-, ##-$(CH_2)_5$-C(O)-$(CH_2)_3$-, ##-$(CH_2)_5$-C(O)-$(CH_2)_4$-, ##-$(CH_2)_5$-C(O)-$(CH_2)_5$-, ##-$(CH_2)_6$-C(O)-$(CH_2)_1$-, ##-$(CH_2)_6$-C(O)-$(CH_2)_2$-, ##-$(CH_2)_6$-C(O)-$(CH_2)_3$-, ##-$(CH_2)_6$-C(O)-$(CH_2)_4$-, ##-$(CH_2)_7$-C(O)-$(CH_2)_1$-, ##-$(CH_2)_7$-C(O)-$(CH_2)_2$-, ##-$(CH_2)_7$-C(O)-$(CH_2)_3$-, ##-$(CH_2)_8$-C(O)-$(CH_2)_l$-, ##-$(CH_2)_8$-C(O)-$(CH_2)_2$-, ##-$CH_2$-S-$CH_2$-, ##-$CH_2$-S-$(CH_2)_2$-, ##-$(CH_2)_1$-S-$(CH_2)_3$-, ##-$(CH_2)_1$-S-$(CH_2)_4$-, ##-$(CH_2)_1$-S-$(CH_2)_5$-, ##-$(CH_2)_1$-S-$(CH_2)_6$-, ##-$(CH_2)_1$-S-$(CH_2)_7$-, ##-$(CH_2)_1$-S-$(CH_2)_8$-, ##-$(CH_2)_1$-S-$(CH_2)_9$-, ##-$(CH_2)_1$-S-$(CH_2)_{10}$-, ##-$(CH_2)_2$-S-$(CH_2)_1$-, ##-$(CH_2)_2$-S-$(CH_2)_2$-, ##-$(CH_2)_2$-S-$(CH_2)_3$-, ##-$(CH_2)_2$-S-$(CH_2)_4$-, ##-$(CH_2)_2$-S-$(CH_2)_5$-, ##-$(CH_2)_2$-S-$(CH_2)_6$-, ##-$(CH_2)_2$-S-$(CH_2)_7$-, ##-$(CH_2)_2$-S-$(CH_2)_8$-, ##-$(CH_2)_2$-S-$(CH_2)_9$-, ##-$(CH_2)_2$-S-$(CH_2)_{10}$-, ##-$(CH_2)_2$-S-$(CH_2)_{11}$-, ##-$(CH_2)_2$-S-$(CH_2)_{12}$-, ##-$(CH_2)_3$-S-$(CH_2)_1$-, ##-$(CH_2)_3$-S-$(CH_2)_2$-, ##-$(CH_2)_3$-S-$(CH_2)_3$-, ##-$(CH_2)_3$-S-$(CH_2)_4$-, ##-$(CH_2)_3$-S-$(CH_2)_5$-, ##-$(CH_2)_3$-S-$(CH_2)_6$-, ##-$(CH_2)_3$-S-$(CH_2)_7$-, ##-$(CH_2)_4$-S-$(CH_2)_1$-, ##-$(CH_2)_4$-S-$(CH_2)_2$-, ##-$(CH_2)_4$-S-$(CH_2)_3$-, ##-$(CH_2)_4$-S-$(CH_2)_4$-, ##-$(CH_2)_4$-S-$(CH_2)_5$-, ##-$(CH_2)_4$-S-$(CH_2)_6$-, ##-$(CH_2)_5$-S-$(CH_2)_1$-, ##-$(CH_2)_5$-S-$(CH_2)_2$-, ##-$(CH_2)_5$-S-$(CH_2)_3$-, ##-$(CH_2)_5$-S-$(CH_2)_4$-, ##-$(CH_2)_5$-S-$(CH_2)_5$-, ##-$(CH_2)_6$-S-$(CH_2)_1$-, ##-$(CH_2)_6$-S-$(CH_2)_2$-, ##-$(CH_2)_6$-S-$(CH_2)_3$-, ##-$(CH_2)_6$-S-$(CH_2)_4$-, ##-$(CH_2)_7$-S-$(CH_2)_1$-, ##-$(CH_2)_7$-S-$(CH_2)_2$-, ##-$(CH_2)_7$-S-$(CH_2)_3$-, ##-$(CH_2)_8$-S-$(CH_2)_1$-, ##-$(CH_2)_8$-S-$(CH_2)_2$-, ##-$CH_2$-C(S)-$CH_2$-, ##-$CH_2$-C(S)-$(CH_2)_2$-, ##-$(CH_2)_1$-C(S)-$(CH_2)_3$-, ##-$(CH_2)_1$-C(S)-$(CH_2)_4$-, ##-$(CH_2)_1$-C(S)-$(CH_2)_5$-, ##-$(CH_2)_1$-C(S)-$(CH_2)_6$-, ##-$(CH_2)_1$-C(S)-$(CH_2)_7$-, ##-$(CH_2)_1$-C(S)-$(CH_2)_8$-, ##-$(CH_2)_1$-C(S)-$(CH_2)_9$-, ##-$(CH_2)_1$-C(S)-$(CH_2)_{10}$-, ##-$(CH_2)_2$-C(S)-$(CH_2)_1$-, ##-$(CH_2)_2$-C(S)-$(CH_2)_2$-, ##-$(CH_2)_2$-C(S)-$(CH_2)_3$-, ##-$(CH_2)_2$-C(S)-$(CH_2)_4$-, ##-$(CH_2)_2$-C(S)-$(CH_2)_5$-, ##-$(CH_2)_2$-C(S)-$(CH_2)_6$-, ##-$(CH_2)_2$-C(S)-$(CH_2)_7$-, ##-$(CH_2)_2$-C(S)-$(CH_2)_8$-, ##-$(CH_2)_2$-C(S)-$(CH_2)_9$-, ##-$(CH_2)_2$-C(S)-$(CH_2)_{10}$-, ##-$(CH_2)_2$-C(S)-$(CH_2)_{11}$-, ##-$(CH_2)_2$-C(S)-$(CH_2)_{12}$-, ##-$(CH_2)_3$-C(S)-$(CH_2)_1$-, ##-$(CH_2)_3$-C(S)-$(CH_2)_2$-, ##-$(CH_2)_3$-C(S)-$(CH_2)_3$-, ##-$(CH_2)_3$-C(S)-$(CH_2)_4$-, ##-$(CH_2)_3$-C(S)-$(CH_2)_5$-, ##-$(CH_2)_3$-C(S)-$(CH_2)_6$-, ##-$(CH_2)_3$-C(S)-$(CH_2)_7$-, ##-$(CH_2)_4$-C(S)-$(CH_2)_1$-, ##-$(CH_2)_4$-C(S)-$(CH_2)_2$-, ##-$(CH_2)_4$-C(S)-$(CH_2)_3$-, ##-$(CH_2)_4$-C(S)-$(CH_2)_4$-, ##-$(CH_2)_4$-C(S)-$(CH_2)_5$-, ##-$(CH_2)_4$-C(S)-$(CH_2)_6$-, ##-$(CH_2)_5$-C(S)-$(CH_2)_1$-, ##-$(CH_2)_5$-C(S)-$(CH_2)_2$-, ##-$(CH_2)_5$-C(S)-$(CH_2)_3$-, ##-$(CH_2)_5$-C(S)-$(CH_2)_4$-, ##-$(CH_2)_5$-C(S)-$(CH_2)_5$-, ##-$(CH_2)_6$-C(S)-$(CH_2)_1$-, ##-$(CH_2)_6$-C(S)-$(CH_2)_2$-, ##-$(CH_2)_6$-C(S)-$(CH_2)_3$-, ##-$(CH_2)_6$-C(S)-$(CH_2)_4$-, ##-$(CH_2)_7$-C(S)-$(CH_2)_1$-, ##-$(CH_2)_7$-C(S)-$(CH_2)_2$-, ##-$(CH_2)_7$-C(S)-$(CH_2)_3$-, ##-$(CH_2)_8$-C(S)-$(CH_2)_1$-, ##-$(CH_2)_8$-C(S)-$(CH_2)_2$-, ##-$CH_2$-C(O)O-$CH_2$-, ##-$CH_2$-C(O)O-$(CH_2)_2$-, ##-$(CH_2)_1$-C(O) O-$(CH_2)_3$-, ##-$(CH_2)_1$-C(O)O-$(CH_2)_4$-, ##-$(CH_2)_1$-C(O)O-$(CH_2)_5$-, ##-$(CH_2)_1$-C(O)O-$(CH_2)_6$-, ##-$(CH_2)_1$-C(O) O-$(CH_2)_7$-, ##-$(CH_2)_1$-C(O)O-$(CH_2)_8$-, ##-$(CH_2)_1$-C(O)O-$(CH_2)_9$-, ##-$(CH_2)_1$-C(O)O-$(CH_2)_{10}$-, ##-$(CH_2)_2$-C(O) O-$(CH_2)_1$-, ##-$(CH_2)_2$-C(O)O-$(CH_2)_2$-, ##-$(CH_2)_2$-C(O)O-$(CH_2)_3$-, ##-$(CH_2)_2$-C(O)O-$(CH_2)_4$-, ##-$(CH_2)_2$-C(O) O-$(CH_2)_5$-, ##-$(CH_2)_2$-C(O)O-$(CH_2)_6$-, ##-$(CH_2)_2$-C(O)O-$(CH_2)_7$-, ##-$(CH_2)_2$-C(O)O-$(CH_2)_8$-, ##-$(CH_2)_2$-C(O) O-$(CH_2)_9$-, ##-$(CH_2)_2$-C(O)O-$(CH_2)_{10}$-, ##-$(CH_2)_2$-C(O)O-$(CH_2)_{11}$-, ##-$(CH_2)_2$-C(O)O-$(CH_2)_{12}$-, ##-$(CH_2)_3$-C(O) O-$(CH_2)_1$-, ##-$(CH_2)_3$-C(O)O-$(CH_2)_2$-, ##-$(CH_2)_3$-C(O)O-$(CH_2)_3$-, ##-$(CH_2)_3$-C(O)O-$(CH_2)_4$-, ##-$(CH_2)_3$-C(O) O-$(CH_2)_5$-, ##-$(CH_2)_3$-C(O)O-$(CH_2)_6$-, ##-$(CH_2)_3$-C(O)O-$(CH_2)_7$-, ##-$(CH_2)_4$-C(O)O-$(CH_2)_1$-, ##-$(CH_2)_4$-C(O) O-$(CH_2)_2$-, ##-$(CH_2)_4$-C(O)O-$(CH_2)_3$-, ##-$(CH_2)_4$-C(O)O-$(CH_2)_4$-, ##-$(CH_2)_4$-C(O)O-$(CH_2)_5$-, ##-$(CH_2)_4$-C(O) O-$(CH_2)_6$-, ##-$(CH_2)_5$-C(O)O-$(CH_2)_1$-, ##-$(CH_2)_5$-C(O)O-$(CH_2)_2$-, ##-$(CH_2)_5$-C(O)O-$(CH_2)_3$-, ##-$(CH_2)_5$-C(O) O-$(CH_2)_4$-, ##-$(CH_2)_5$-C(O)O-$(CH_2)_5$-, ##-$(CH_2)_6$-C(O)O-$(CH_2)_1$-, ##-$(CH_2)_6$-C(O)O-$(CH_2)_2$-, ##-$(CH_2)_6$-C(O) O-$(CH_2)_3$-, ##-$(CH_2)_6$-C(O)O-$(CH_2)_4$-, ##-$(CH_2)_7$-C(O)O-$(CH_2)_1$-, ##-$(CH_2)_7$-C(O)O-$(CH_2)_2$-, ##-$(CH_2)_7$-C(O) O-$(CH_2)_3$-, ##-$(CH_2)_8$-C(O)O-$(CH_2)_1$-, ##-$(CH_2)_8$-C(O)O-$(CH_2)_2$-, ##-$CH_2$-OC(O)-$CH_2$-, ##-$CH_2$-OC(O)-$(CH_2)_2$-, ##-$(CH_2)_1$-OC(O)-$(CH_2)_3$-, ##-$(CH_2)_1$-OC(O)-$(CH_2)_4$-, ##-$(CH_2)_1$-OC(O)-$(CH_2)_5$-, ##-$(CH_2)_1$-OC(O)-$(CH_2)_6$-, ##-$(CH_2)_1$-OC(O)-$(CH_2)_7$-, ##-$(CH_2)_1$-OC(O)-$(CH_2)_8$-, ##-$(CH_2)_1$-OC(O)-$(CH_2)_9$-, ##-$(CH_2)_1$-OC(O)-$(CH_2)_{10}$-, ##-$(CH_2)_2$-OC(O)-$(CH_2)_1$-, ##-$(CH_2)_2$-OC(O)-$(CH_2)_2$-, ##-$(CH_2)_2$-OC(O)-$(CH_2)_3$-, ##-$(CH_2)_2$-OC(O)-$(CH_2)_4$-, ##-$(CH_2)_2$-OC(O)-$(CH_2)_5$-, ##-$(CH_2)_2$-OC(O)-$(CH_2)_6$-, ##-$(CH_2)_2$-OC(O)-$(CH_2)_7$-, ##-$(CH_2)_2$-OC(O)-$(CH_2)_8$-, ##-$(CH_2)_2$-OC(O)-$(CH_2)_9$-, ##-$(CH_2)_2$-OC(O)-$(CH_2)_{10}$-, ##-$(CH_2)_2$-OC(O)-$(CH_2)_{11}$-, ##-$(CH_2)_2$-OC(O)-$(CH_2)_{12}$-, ##-$(CH_2)_3$-OC(O)-$(CH_2)_1$-, ##-$(CH_2)_3$-OC(O)-$(CH_2)_2$-, ##-$(CH_2)_3$-OC(O)-$(CH_2)_3$-, ##-$(CH_2)_3$-OC(O)-$(CH_2)_4$-, ##-$(CH_2)_3$-OC(O)-$(CH_2)_5$-, ##-$(CH_2)_3$-OC(O)-$(CH_2)_6$-, ##-$(CH_2)_3$-OC(O)-$(CH_2)_7$-, ##-$(CH_2)_4$-OC(O)-$(CH_2)_1$-, ##-$(CH_2)_4$-OC(O)-$(CH_2)_2$-, ##-$(CH_2)_4$-OC(O)-$(CH_2)_3$-, ##-$(CH_2)_4$-OC(O)-$(CH_2)_4$-, ##-$(CH_2)_4$-OC(O)-$(CH_2)_5$-, ##-$(CH_2)_4$-OC(O)-$(CH_2)_6$-, ##-$(CH_2)_5$-OC(O)-$(CH_2)_1$-, ##-$(CH_2)_5$-OC(O)-$(CH_2)_2$-, ##-$(CH_2)_5$-OC(O)-$(CH_2)_3$-, ##-$(CH_2)_5$-OC(O)-$(CH_2)_4$-, ##-$(CH_2)_5$-OC(O)-$(CH_2)_5$-, ##-$(CH_2)_6$-OC(O)-$(CH_2)_1$-, ##-$(CH_2)_6$-OC(O)-$(CH_2)_2$-, ##-$(CH_2)_6$-OC(O)-$(CH_2)_3$-, ##-$(CH_2)_6$-OC(O)-$(CH_2)_4$-, ##-$(CH_2)_7$-OC(O)-$(CH_2)_1$-, ##-$(CH_2)_7$-OC(O)-$(CH_2)_2$-, ##-$(CH_2)_7$-OC(O)-$(CH_2)_3$-, ##-$(CH_2)_8$-OC(O)-$(CH_2)_1$-, ##-$(CH_2)_8$-OC(O)-$(CH_2)_2$-, ##-$(CH_2)_1$-N($R_{g10}$)-$(CH_2)_1$-, ##-$(CH_2)_1$-N($R_{g10}$)-$(CH_2)_2$-, ##-$(CH_2)_1$-N($R_{g10}$)-$(CH_2)_3$-, ##-$(CH_2)_1$-N($R_{g10}$)-$(CH_2)_4$-, ##-$(CH_2)_1$-N($R_{g10}$)-$(CH_2)_5$-,

##-$(CH_2)_1$-N($R_{g10}$)-$(CH_2)_6$-, ##-$(CH_2)_1$-N($R_{g10}$)-$(CH_2)_7$-, ##-$(CH_2)_1$-N($R_{g10}$)-$(CH_2)_8$-, ##-$(CH_2)_1$-N($R_{g10}$)-$(CH_2)_9$-, ##-$(CH_2)_1$-N($R_{g10}$)-$(CH_2)_{10}$-, ##-$(CH_2)_2$-N($R_{g10}$)-$(CH_2)_1$-, ##-$(CH_2)_2$-N($R_{g10}$)-$(CH_2)_2$-, ##-$(CH_2)_2$-N($R_{g10}$)-$(CH_2)_3$-, ##-$(CH_2)_2$-N($R_{g10}$)-$(CH_2)_4$-, ##-$(CH_2)_2$-N($R_{g10}$)-$(CH_2)_5$-, ##-$(CH_2)_2$-N($R_{g10}$)-$(CH_2)_6$-, ##-$(CH_2)_2$-N($R_{g10}$)-$(CH_2)_7$-, ##-$(CH_2)_2$-N($R_{g10}$)-$(CH_2)_8$-, ##-$(CH_2)_2$-N($R_{g10}$)-$(CH_2)_9$-, ##-$(CH_2)_2$-N($R_{g10}$)-$(CH_2)_{10}$-, ##-$(CH_2)_2$-N($R_{g10}$)-$(CH_2)_{11}$-, ##-$(CH_2)_2$-N($R_{g10}$)-$(CH_2)_{12}$-, ##-$(CH_2)_3$-N($R_{g10}$)-$(CH_2)_1$-, ##-$(CH_2)_3$-N($R_{g10}$)-$(CH_2)_2$-, ##-$(CH_2)_3$-N($R_{g10}$)-$(CH_2)_3$-, ##-$(CH_2)_4$-N($R_{g10}$)-$(CH_2)_1$-, ##-$(CH_2)_4$-N($R_{g10}$)-$(CH_2)_2$-, ##-$(CH_2)_4$-N($R_{g10}$)-$(CH_2)_3$-, ##-$(CH_2)_4$-N($R_{g10}$)-$(CH_2)_4$-, ##-$(CH_2)_5$-N($R_{g10}$)-$(CH_2)_1$-, ##-$(CH_2)_5$-N($R_{g10}$)-$(CH_2)_2$-, ##-$(CH_2)_5$-N($R_{g10}$)-$(CH_2)_3$-, ##-$(CH_2)_5$-N($R_{g10}$)-$(CH_2)_4$-, ##-$(CH_2)_5$-N($R_{g10}$)-$(CH_2)_5$-, ##-$(CH_2)_6$-N($R_{g10}$)-$(CH_2)_1$-, ##-$(CH_2)_6$-N($R_{g10}$)-$(CH_2)_2$-, ##-$(CH_2)_6$-N($R_{g10}$)-$(CH_2)_3$-, ##-$(CH_2)_7$-N($R_{g10}$)-$(CH_2)_1$-, ##-$(CH_2)_7$-N($R_{g10}$)-$(CH_2)_2$-, ##-$(CH_2)_7$-N($R_{g10}$)-$(CH_2)_3$-, ##-$(CH_2)_8$-N($R_{g10}$)-$(CH_2)_1$-, ##-$(CH_2)_8$-N $R_{g10}$)-$(CH_2)_2$-, ##-$(CH_2)_8$-N($R_{g10}$)-$(CH_2)_3$-, ##-CH($CH_3$)-N($R_{g10}$)-$(CH_2)_1$-, ##-CH($CH_3$)-N($R_{g10}$)-$(CH_2)_2$-, ##-CH($CH_3$)-N($R_{g10}$)-$(CH_2)_3$-, ##-CH($CH_3$)-N($R_{g10}$)-$(CH_2)_4$-, ##-CH($CH_3$)-N($R_{g10}$)-$(CH_2)_5$-, ##-CH($CH_3$)-N($R_{g10}$)-$(CH_2)_6$-, ##-CH($CH_3$)-N($R_{g10}$)-$(CH_2)_7$-, ##-CH($CH_3$)-N($R_{g10}$)-$(CH_2)_8$-, ##-CH($CH_3$)-N($R_{g10}$)-$(CH_2)_9$-, ##-CH($CH_3$)-N($R_{g10}$)-$(CH_2)_{10}$-, ##-$CH_2$C(O)NH$CH_2$-, ##-$(CH_2)_2$C(O)NH$(CH_2)_2$-, ##-$(CH_2)_2$C(O)NH$(CH_2)_3$-, ##-$(CH_2)_2$C(O)NH$(CH_2)_4$-, ##-$(CH_2)_2$C(O)NH$(CH_2)_5$-, ##-$(CH_2)_3$C(O)NH$(CH_2)_3$-, ##-$(CH_2)_3$C(O)NH$(CH_2)_4$-, ##-$(CH_2)_4$C(O)NH$(CH_2)_4$-, ##-$(CH_2)_5$C(O)NH$(CH_2)_5$-, ##-$(CH_2)_6$C(O)NH$(CH_2)_7$-, ##-$(CH_2)_6$C(O)NH$(CH_2)_6$-, ##-$(CH_2)_7$C(O)NH$(CH_2)_7$-, ##-$(CH_2)_8$C(O)NH$(CH_2)_8$, ##-$(CH_2)_9$C(O)NH$(CH_2)_9$-, ##-$(CH_2)_{10}$C(O)NH$(CH_2)_{10}$-, ##-$(CH_2)_2$C(O)NH$(CH_2)_2$-O-$(CH_2)_2$-, ##-$CH_2$NHC(O)$CH_2$-, ##-$(CH_2)_2$NHC(O)$(CH_2)_2$-, ##-$(CH_2)_2$NHC(O)$(CH_2)_3$-, ##-$(CH_2)_2$NHC(O)$(CH_2)_4$-, ##-$(CH_2)_2$NHC(O)$(CH_2)_5$-, ##-$(CH_2)_3$NHC(O)$(CH_2)_3$-, ##-$(CH_2)_3$NHC(O)$(CH_2)_4$-, ##-$(CH_2)_4$NHC(O)$(CH_2)_4$-, ##-$(CH_2)_5$NHC(O)$(CH_2)_5$-, ##-$(CH_2)_6$NHC(O)$(CH_2)_7$-, ##-$(CH_2)_6$NHC(O)$(CH_2)_6$-, ##-$(CH_2)_7$NHC(O)$(CH_2)_7$-, ##-$(CH_2)_8$NHC(O)$(CH_2)_8$, ##-$(CH_2)_9$NHC(O)$(CH_2)_9$-, ##-$(CH_2)_{10}$NHC(O)$(CH_2)_{10}$-, ##-$(CH_2)_4$NHC(O)$(CH_2)_8$-, ##-$(CH_2)_2$NHC(O)$(CH_2)_2$-O-$(CH_2)_2$-, ##-$(CH_2)_4$NHC(O)$CH_2$-, ##-$CH_2$-piperidinylene-$CH_2$-, ##-$CH_2$-piperidinylene-$(CH_2)_2$-, ##-$CH_2$-piperidinylene-$(CH_2)_3$-, ##-$CH_2$-piperidinylene-$(CH_2)_4$-, ##-$CH_2$-piperidinylene-$(CH_2)$s-, ##-$CH_2$-piperidinylene-$(CH_2)_6$-, ##-$CH_2$-piperidinylene-$(CH_2)_7$-, ##-$CH_2$-piperidinylene-$(CH_2)_8$-, ##-$(CH_2)_2$-piperidinylene-$(CH_2)_1$-, ##-$(CH_2)_2$-piperidinylene-$(CH_2)_2$-, ##-$(CH_2)_2$-piperidinylene-$(CH_2)_3$-, ##-$(CH_2)_2$-piperidinylene-$(CH_2)_4$-, ##-$(CH_2)_2$-piperidinylene-$(CH_2)$s-, ##-$(CH_2)_2$-piperidinylene-$(CH_2)_6$-, ##-$(CH_2)_2$-piperidinylene-$(CH_2)_7$-, ##-$(CH_2)_2$-piperidinylene-$(CH_2)_8$-, ##-$(CH_2)_3$-piperidinylene-$CH_2$-, ##-$(CH_2)_3$-piperidinylene-$(CH_2)_2$-, ##-$(CH_2)_3$-piperidinylene-$(CH_2)_3$-, ##-$(CH_2)_3$-piperidinylene-$(CH_2)_4$-, ##-$(CH_2)_3$-piperidinylene-$(CH_2)_5$-, ##-$(CH_2)_3$-piperidinylene-$(CH_2)_6$-, ##-$(CH_2)_3$-piperidinylene-$(CH_2)_7$-, ##-$(CH_2)_3$-piperidinylene-$(CH_2)_8$-, ##-$(CH_2)_4$-piperidinylene-$CH_2$-, ##-$(CH_2)_4$-piperidinylene-$(CH_2)_2$-, ##-$(CH_2)_4$-piperidinylene-$(CH_2)_3$-, ##-$(CH_2)_4$-piperidinylene-$(CH_2)_4$-, ##-$(CH_2)_4$-piperidinylene-$(CH_2)_5$-, ##-$(CH_2)_4$-piperidinylene-$(CH_2)_6$-, ##-$(CH_2)_4$-piperidinylene-$(CH_2)_7$-, ##-$(CH_2)_4$-pipendinylene-$(CH_2)_8$-, ##-$(CH_2)_5$-piperidinylene-$(CH_2)_1$-, ##-$(CH_2)_5$-piperidinylene-$(CH_2)_2$-, ##-$(CH_2)_5$-piperidinylene-$(CH_2)_3$-, ##-$(CH_2)_5$-piperidinylene-$(CH_2)_4$-, ##-$(CH_2)_5$-piperidinylene-$(CH_2)_5$-, ##-$(CH_2)_5$-piperidinylene-$(CH_2)_6$-, ##-$(CH_2)_5$-piperidinylene-$(CH_2)_7$-, ##-$(CH_2)_5$-piperidinylene-$(CH_2)_8$-, ##-$(CH_2)_6$-piperidinylene-$(CH_2)_1$-, ##-$(CH_2)_6$-piperidinylene-$(CH_2)_2$-, ##-$(CH_2)_6$-piperidinylene-$(CH_2)_3$-, ##-$(CH_2)_6$-piperidinylene-$(CH_2)_4$-, ##-$(CH_2)_6$-piperidinylene-$(CH_2)_5$-, ##-$(CH_2)_6$-piperidinylene-$(CH_2)_6$-, ##-$(CH_2)_6$-piperidinylene-$(CH_2)_7$-, ##-$(CH_2)_6$-piperidinylene-$(CH_2)_8$-, ##-$(CH_2)_7$-piperidinylene-$(CH_2)_1$-, ##-$(CH_2)_7$-piperidinylene-$(CH_2)_2$-, ##-$(CH_2)_7$-piperidinylene-$(CH_2)_3$-, ##-$(CH_2)_7$-piperidinylene-$(CH_2)_4$-, ##-$(CH_2)_7$-pipendinylene-$(CH_2)_8$-, ##-$(CH_2)_8$-piperidinylene-$CH_2$-, ##-$(CH_2)_8$-piperidinylene-$(CH_2)_2$-, ##-$(CH_2)_8$-piperidinylene-$(CH_2)_3$-, ##-$(CH_2)_8$-piperidinylene-$(CH_2)_4$-, ##-$(CH_2)_8$-piperidinylene-$(CH_2)_5$-, ##-$(CH_2)_8$-piperidinylene-$(CH_2)_6$-, ##-$(CH_2)_8$-piperidinylene-$(CH_2)_7$-, ##-$(CH_2)_8$-piperidinylene-$(CH_2)_8$-, ##-$CH_2$-piperazinylene-$CH_2$-, ##-$CH_2$-piperazinylene-$(CH_2)_2$-, ##-$CH_2$-piperazinylene-$(CH_2)_3$-, ##-$CH_2$-piperazinylene-$(CH_2)_4$-, ##-$CH_2$-piperazinylene-$(CH_2)_5$-, ##-$CH_2$-piperazinylene-$(CH_2)_6$-, ##-$CH_2$-piperazinylene-$(CH_2)_7$-, ##-$CH_2$-piperazinylene-$(CH_2)_8$-, ##-$(CH_2)_2$-piperazinylene-$(CH_2)_1$-, ##-$(CH_2)_2$-piperazinylene-$(CH_2)_2$-, ##-$(CH_2)_2$-piperazinylene-$(CH_2)_3$-, ##-$(CH_2)_2$-piperazinylene-$(CH_2)_4$-, ##-$(CH_2)_2$-piperazinylene-$(CH_2)_5$-, ##-$(CH_2)_2$-piperazinylene-$(CH_2)_6$-, ##-$(CH_2)_2$-piperazinylene-$(CH_2)_7$-, ##-$(CH_2)_2$-piperazinylene-$(CH_2)_8$-, ##-$(CH_2)_3$-piperazinylene-$CH_2$-, ##-$(CH_2)_3$-piperazinylene-$(CH_2)_2$-, ##-$(CH_2)_3$-piperazinylene-$(CH_2)_3$-, ##-$(CH_2)_3$-piperazinylene-$(CH_2)_4$-, ##-$(CH_2)_3$-piperazinylene-$(CH_2)_5$-, ##-$(CH_2)_3$-piperazinylene-$(CH_2)_6$-, ##-$(CH_2)_3$-piperazinylene-$(CH_2)_7$-, ##-$(CH_2)_3$-piperazinylene-$(CH_2)_8$-, ##-$(CH_2)_4$-piperazinylene-$CH_2$-, ##-$(CH_2)_4$-piperazinylene-$(CH_2)_2$-, ##-$(CH_2)_4$-piperazinylene-$(CH_2)_3$-, ##-$(CH_2)_4$-piperazinylene-$(CH_2)_4$-, ##-$(CH_2)_4$-piperazinylene-$(CH_2)_5$-, ##-$(CH_2)_4$-piperazinylene-$(CH_2)_6$-, ##-$(CH_2)_4$-piperazinylene-$(CH_2)_7$-, ##-$(CH_2)_4$-piperazinylene-$(CH_2)_8$-, ##-$(CH_2)_5$-piperazinylene-$(CH_2)_1$-, ##-$(CH_2)_5$-piperazinylene-$(CH_2)_2$-, ##-$(CH_2)_5$-piperazinylene-$(CH_2)_3$-, ##-$(CH_2)_5$-piperazinylene-$(CH_2)_4$-, ##-$(CH_2)_5$-piperazinylene-$(CH_2)_5$-, ##-$(CH_2)_5$-piperazinylene-$(CH_2)_6$-, ##-$(CH_2)_5$-piperazinylene-$(CH_2)_7$-, ##-$(CH_2)_5$-piperazinylene-$(CH_2)_8$-, ##-$(CH_2)_6$-piperazinylene-$(CH_2)_1$-, ##-$(CH_2)_6$-piperazinylene-$(CH_2)_2$-, ##-$(CH_2)_6$-piperazinylene-$(CH_2)_3$-, ##-$(CH_2)_6$-piperazinylene-$(CH_2)_4$-, ##-$(CH_2)_6$-piperazinylene-$(CH_2)_5$-, ##-$(CH_2)_6$-piperazinylene-$(CH_2)_6$-, ##-$(CH_2)_6$-piperazinylene-$(CH_2)_7$-, ##-$(CH_2)_6$-piperazinylene-$(CH_2)_8$-, ##-$(CH_2)_7$-piperazinylene-$(CH_2)_1$-, ##-$(CH_2)_7$-piperazinylene-$(CH_2)_2$-, ##-$(CH_2)_7$-piperazinylene-$(CH_2)_3$-, ##-$(CH_2)_7$-piperazinylene-$(CH_2)_4$-, ##-$(CH_2)_7$-piperazinylene-$(CH_2)_8$-, ##-$(CH_2)_8$-piperazinylene-$CH_2$-, ##-$(CH_2)_8$-piperazinylene-$(CH_2)_2$-, ##-$(CH_2)_8$-piperazinylene-$(CH_2)_3$-, ##-$(CH_2)_8$-piperazinylene-$(CH_2)_4$-, ##-$(CH_2)_8$-piperazinylene-$(CH_2)_5$-, ##-$(CH_2)_8$-piperazinylene-$(CH_2)_6$-, ##-$(CH_2)_8$-piperazinylene-$(CH_2)_7$-, ##-$(CH_2)_8$-piperazinylene-$(CH_2)_8$-, ##-$CH_2$-propylene-$CH_2$-, ##-$CH_2$-propylene-$(CH_2)_2$-, ##-$CH_2$-propylene-$(CH_2)_3$-,

##-$CH_2$-propylene-$(CH_2)_4$-, ##-$CH_2$-propylene-$(CH_2)_5$-, ##-$CH_2$-propylene-$(CH_2)_6$-, ##-$CH_2$-propylene-$(CH_2)_7$-, ##-$CH_2$-propylene-$(CH_2)_8$-, ##-$(CH_2)_2$-propylene-$(CH_2)_1$-, ##-$(CH_2)_2$-propylene-$(CH_2)_2$-, ##-$(CH_2)_2$-propylene-$(CH_2)_3$-, ##-$(CH_2)_2$-propylene-$(CH_2)_4$-, ##-$(CH_2)_2$-propylene-$(CH_2)_5$-, ##-$(CH_2)_2$-propylene-$(CH_2)_6$-, ##-$(CH_2)_2$-propylene-$(CH_2)_7$-, ##-$(CH_2)_2$-propylene-$(CH_2)_8$-, ##-$(CH_2)_3$-propylene-$CH_2$-, ##-$(CH_2)_3$-propylene-$(CH_2)_2$-, ##-$(CH_2)_3$-propylene-$(CH_2)_3$-, ##-$(CH_2)_3$-propylene-$(CH_2)_4$-, ##-$(CH_2)_3$-propylene-$(CH_2)_5$-, ##-$(CH_2)_3$-propylene-$(CH_2)_6$-, ##-$(CH_2)_3$-propylene-$(CH_2)_7$-, ##-$(CH_2)_3$-propylene-$(CH_2)_8$-, ##-$(CH_2)_4$-propylene-$CH_2$-, ##-$(CH_2)_4$-propylene-$(CH_2)_2$-, ##-$(CH_2)_4$-propylene-$(CH_2)_3$-, ##-$(CH_2)_4$-propylene-$(CH_2)_4$-, ##-$(CH_2)_4$-propylene-$(CH_2)_5$-, ##-$(CH_2)_4$-propylene-$(CH_2)_6$-, ##-$(CH_2)_4$-propylene-$(CH_2)_7$-, ##-$(CH_2)_4$-propylene-$(CH_2)_8$-, ##-$(CH_2)_5$-propylene-$(CH_2)_1$-, ##-$(CH_2)_5$-propylene-$(CH_2)_2$-, ##-$(CH_2)_5$-propylene-$(CH_2)_3$-, ##-$(CH_2)_5$-propylene-$(CH_2)_4$-, ##-$(CH_2)_5$-propylene-$(CH_2)_5$-, ##-$(CH_2)_5$-propylene-$(CH_2)_6$-, ##-$(CH_2)_5$-propylene-$(CH_2)_7$-, ##-$(CH_2)_5$-propylene-$(CH_2)_8$-, ##-$(CH_2)_6$-propylene-$(CH_2)_1$-, ##-$(CH_2)_6$-propylene-$(CH_2)_2$-, ##-$(CH_2)_6$-propylene-$(CH_2)_3$-, ##-$(CH_2)_6$-propylene-$(CH_2)_4$-, ##-$(CH_2)_6$-propylene-$(CH_2)_5$-, ##-$(CH_2)_6$-propylene-$(CH_2)_6$-, ##-$(CH_2)_6$-propylene-$(CH_2)_7$-, ##-$(CH_2)_6$-propylene-$(CH_2)_8$-, ##-$(CH_2)_7$-propylene-$(CH_2)_1$-, ##-$(CH_2)_7$-propylene-$(CH_2)_2$-, ##-$(CH_2)_7$-propylene-$(CH_2)_3$-, ##-$(CH_2)_7$-propylene-$(CH_2)_4$-, ##-$(CH_2)_7$-propylene-$(CH_2)_8$-, ##-$(CH_2)_8$-propylene-$CH_2$-, ##-$(CH_2)_8$-propylene-$(CH_2)_2$-, ##-$(CH_2)_8$-propylene-$(CH_2)_3$-, ##-$(CH_2)_8$-propylene-$(CH_2)_4$-, ##-$(CH_2)_8$-propylene-$(CH_2)_5$-, ##-$(CH_2)_8$-propylene-$(CH_2)_6$-, ##-$(CH_2)_8$-propylene-$(CH_2)_7$-, ##-$(CH_2)_8$-propylene-$(CH_2)_8$-, ##-$CH_2$-diazacycloheptanylene-$CH_2$-, ##-$CH_2$-diazacycloheptanylene-$(CH_2)_2$-, ##-$CH_2$-diazacycloheptanylene-$(CH_2)_3$-, ##-$CH_2$-diazacycloheptanylene-$(CH_2)_4$-, ##-$CH_2$-diazacycloheptanylene-$(CH_2)_5$-, ##-$CH_2$-diazacycloheptanylene-$(CH_2)_6$-, ##-$CH_2$-diazacycloheptanylene-$(CH_2)_7$-, ##-$CH_2$-diazacycloheptanylene-$(CH_2)_8$-, ##-$(CH_2)_2$-diazacycloheptanylene-$(CH_2)_1$-, ##-$(CH_2)_2$-diazacycloheptanylene-$(CH_2)_2$-, ##-$(CH_2)_2$-diazacycloheptanylene-$(CH_2)_3$-, ##-$(CH_2)_2$-diazacycloheptanylene-$(CH_2)_4$-, ##-$(CH_2)_2$-diazacycloheptanylene-$(CH_2)_5$-, ##-$(CH_2)_2$-diazacycloheptanylene-$(CH_2)_6$-, ##-$(CH_2)_2$-diazacycloheptanylene-$(CH_2)_7$-, ##-$(CH_2)_2$-diazacycloheptanylene-$(CH_2)_8$-, ##-$(CH_2)_3$-diazacycloheptanylene-$CH_2$-, ##-$(CH_2)_3$-diazacycloheptanylene-$(CH_2)_2$-, ##-$(CH_2)_3$-diazacycloheptanylene-$(CH_2)_3$-, ##-$(CH_2)_3$-diazacycloheptanylene-$(CH_2)_4$-, ##-$(CH_2)_3$-diazacycloheptanylene-$(CH_2)_5$-, ##-$(CH_2)_3$-diazacycloheptanylene-$(CH_2)_6$-, ##-$(CH_2)_3$-diazacycloheptanylene-$(CH_2)_7$-, ##-$(CH_2)_3$-diazacycloheptanylene-$(CH_2)_8$-, ##-$(CH_2)_4$-diazacycloheptanylene-$CH_2$-, ##-$(CH_2)_4$-diazacycloheptanylene-$(CH_2)_2$-, ##-$(CH_2)_4$-diazacycloheptanylene-$(CH_2)_3$-, ##-$(CH_2)_4$-diazacycloheptanylene-$(CH_2)_4$-, ##-$(CH_2)_4$-diazacycloheptanylene-$(CH_2)_5$-, ##-$(CH_2)_4$-diazacycloheptanylene-$(CH_2)_6$-, ##-$(CH_2)_4$-diazacycloheptanylene-$(CH_2)_7$-, ##-$(CH_2)_4$-diazacycloheptanylene-$(CH_2)_8$-, ##-$(CH_2)_5$-diazacycloheptanylene-$(CH_2)_1$-, ##-$(CH_2)_5$-diazacycloheptanylene-$(CH_2)_2$-, ##-$(CH_2)_5$-diazacycloheptanylene-$(CH_2)_3$-, ##-$(CH_2)_5$-diazacycloheptanylene-$(CH_2)_4$-, ##-$(CH_2)_5$-diazacycloheptanylene-$(CH_2)_5$-, ##-$(CH_2)_5$-diazacycloheptanylene-$(CH_2)_6$-, ##-$(CH_2)_5$-diazacycloheptanylene-$(CH_2)_7$-, ##-$(CH_2)_5$-diazacycloheptanylene-$(CH_2)_8$-, ##-$(CH_2)_6$-diazacycloheptanylene-$(CH_2)_1$-, ##-$(CH_2)_6$-diazacycloheptanylene-$(CH_2)_2$-, ##-$(CH_2)_6$-diazacycloheptanylene-$(CH_2)_3$-, ##-$(CH_2)_6$-diazacycloheptanylene-$(CH_2)_4$-, ##-$(CH_2)_6$-diazacycloheptanylene-$(CH_2)_5$-, ##-$(CH_2)_6$-diazacycloheptanylene-$(CH_2)_6$-, ##-$(CH_2)_6$-diazacycloheptanylene-$(CH_2)_7$-, ##-$(CH_2)_6$-diazacycloheptanylene-$(CH_2)_8$-, ##-$(CH_2)_7$-diazacycloheptanylene-$(CH_2)_1$-, ##-$(CH_2)_7$-diazacycloheptanylene-$(CH_2)_2$-, ##-$(CH_2)_7$-diazacycloheptanylene-$(CH_2)_3$-, ##-$(CH_2)_7$-diazacycloheptanylene-$(CH_2)_4$-, ##-$(CH_2)_7$-diazacycloheptanylene-$(CH_2)_8$-, ##-$(CH_2)_8$-diazacycloheptanylene-$CH_2$-, ##-$(CH_2)_8$-diazacycloheptanylene-$(CH_2)_2$-, ##-$(CH_2)_8$-diazacycloheptanylene-$(CH_2)_3$-, ##-$(CH_2)_8$-diazacycloheptanylene-$(CH_2)_4$-, ##-$(CH_2)_8$-diazacycloheptanylene-$(CH_2)_5$-, ##-$(CH_2)_8$-diazacycloheptanylene-$(CH_2)_6$-, ##-$(CH_2)_8$-diazacycloheptanylene-$(CH_2)_7$-, ##-$(CH_2)_8$-diazacycloheptanylene-$(CH_2)_8$-, ##-$CH_2$-diazabicyclo[2.2.1]heptanylene-$CH_2$-, ##-$CH_2$-diazabicyclo[2.2.1]heptanylene-$(CH_2)_2$-, ##-$CH_2$-diazabicyclo[2.2.1]heptanylene-$(CH_2)_3$-, ##-$CH_2$-diazabicyclo[2.2.1]heptanylene-$(CH_2)_4$-, ##-$CH_2$-diazabicyclo[2.2.1]heptanylene-$(CH_2)_5$-, ##-$CH_2$-diazabicyclo[2.2.1]heptanylene-$(CH_2)_6$-, ##-$CH_2$-diazabicyclo[2.2.1]heptanylene-$(CH_2)_7$-, ##-$CH_2$-diazabicyclo[2.2.1]heptanylene-$(CH_2)_8$-, ##-$(CH_2)_2$-diazabicyclo[2.2.1]heptanylene-$(CH_2)_1$-, ##-$(CH_2)_2$-diazabicyclo[2.2.1]heptanylene-$(CH_2)_2$-, ##-$(CH_2)_2$-diazabicyclo[2.2.1]heptanylene-$(CH_2)_3$-, ##-$(CH_2)_2$-diazabicyclo[2.2.1]heptanylene-$(CH_2)_4$-, ##-$(CH_2)_2$-diazabicyclo[2.2.1]heptanylene-$(CH_2)_5$-, ##-$(CH_2)_2$-diazabicyclo[2.2.1]heptanylene-$(CH_2)_6$-, ##-$(CH_2)_2$-diazabicyclo[2.2.1]heptanylene-$(CH_2)_7$-, ##-$(CH_2)_2$-diazabicyclo[2.2.1]heptanylene-$(CH_2)_8$-, ##-$(CH_2)_3$-diazabicyclo[2.2.1]heptanylene-$CH_2$-, ##-$(CH_2)_3$-diazabicyclo[2.2.1]heptanylene-$(CH_2)_2$-, ##-$(CH_2)_3$-diazabicyclo[2.2.1]heptanylene-$(CH_2)_3$-, ##-$(CH_2)_3$-diazabicyclo[2.2.1]heptanylene-$(CH_2)_4$-, ##-$(CH_2)_3$-diazabicyclo[2.2.1]heptanylene-$(CH_2)_5$-, ##-$(CH_2)_3$-diazabicyclo[2.2.1]heptanylene-$(CH_2)_6$-, ##-$(CH_2)_3$-diazabicyclo[2.2.1]heptanylene-$(CH_2)_7$-, ##-$(CH_2)_3$-diazabicyclo[2.2.1]heptanylene-$(CH_Z)_s$-, ##-$(CH_2)_4$-diazabicyclo[2.2.1]heptanylene-$CH_2$-, ##-$(CH_2)_4$-diazabicyclo[2.2.1]heptanylene-$(CH_2)_2$-, ##-$(CH_2)_4$-diazabicyclo[2.2.1]heptanylene-$(CH_2)_3$-, ##-$(CH_2)_4$-diazabicyclo[2.2.1]heptanylene-$(CH_2)_4$-, ##-$(CH_2)_4$-diazabicyclo[2.2.1]heptanylene-$(CH_2)_5$-, ##-$(CH_2)_4$-diazabicyclo[2.2.1]heptanylene-$(CH_2)_6$-, ##-$(CH_2)_4$-diazabicyclo[2.2.1]heptanylene-$(CH_2)_7$-, ##-$(CH_2)_4$-diazabicyclo[2.2.1]heptanylene-$(CH_2)_8$-, ##-$(CH_2)_5$-diazabicyclo[2.2.1]heptanylene-$(CH_2)_1$-, ##-$(CH_2)_5$-diazabicyclo[2.2.1]heptanylene-$(CH_2)_2$-, ##-$(CH_2)_5$-diazabicyclo[2.2.1]heptanylene-$(CH_2)_3$-, ##-$(CH_2)_5$-diazabicyclo[2.2.1]heptanylene-$(CH_2)_4$-, ##-$(CH_2)_5$-diazabicyclo[2.2.1]heptanylene-$(CH_2)_5$-, ##-$(CH_2)_5$-diazabicyclo[2.2.1]heptanylene-$(CH_2)_6$-, ##-$(CH_2)_5$-diazabicyclo[2.2.1]heptanylene-$(CH_2)_7$-, ##-$(CH_2)_5$-diazabicyclo[2.2.1]heptanylene-$(CH_2)_8$-, ##-$(CH_2)_6$-diazabicyclo[2.2.1]heptanylene-$(CH_2)_1$-, ##-$(CH_2)_6$-diazabicyclo[2.2.1]heptanylene-$(CH_2)_2$-, ##-$(CH_2)_6$-diazabicyclo[2.2.1]heptanylene-$(CH_2)_3$-, ##-$(CH_2)_6$-diazabicyclo[2.2.1]heptanylene-$(CH_2)_4$-, ##-$(CH_2)_6$-diazabicyclo[2.2.1]heptanylene-$(CH_2)_5$-, ##-$(CH_2)_6$-dia-

zabicyclo[2.2.1]heptanylene-$(CH_2)_6$-, ##-$(CH_2)_6$-diazabicyclo[2.2.1]heptanylene-$(CH_2)_7$-, ##-$(CH_2)_6$-diazabicyclo[2.2.1]heptanylene-$(CH_2)_8$-, ##-$(CH_2)_7$-diazabicyclo[2.2.1]heptanylene-$(CH_2)_1$-, ##-$(CH_2)_7$-diazabicyclo[2.2.1]heptanylene-$(CH_2)_2$-, ##-$(CH_2)_7$-diazabicyclo[2.2.1]heptanylene-$(CH_2)_3$-, ##-$(CH_2)_7$-diazabicyclo[2.2.1]heptanylene-$(CH_2)_4$-, ##-$(CH_2)_7$-diazabicyclo[2.2.1]heptanylene-$(CH_2)_8$-, ##-$(CH_2)_8$-diazabicyclo[2.2.1]heptanylene-$CH_2$-, ##-$(CH_2)_8$-diazabicyclo[2.2.1]heptanylene-$(CH_2)_2$-, ##-$(CH_2)_8$-diazabicyclo[2.2.1]heptanylene-$(CH_2)_3$-, ##-$(CH_2)_8$-diazabicyclo[2.2.1]heptanylene-$(CH_2)_4$-, ##-$(CH_2)_8$-diazabicyclo[2.2.1]heptanylene-$(CH_2)_5$-, ##-$(CH_2)_8$-diazabicyclo[2.2.1]heptanylene-$(CH_2)_6$-, ##-$(CH_2)_8$-diazabicyclo[2.2.1]heptanylene-$(CH_2)_7$-, ##-$(CH_2)_8$-diazabicyclo[2.2.1]heptanylene-$(CH_2)_8$-, ##-$CH_2$-diazabicyclo[3.1.1]heptanylene-$CH_2$-, ##-$CH_2$-diazabicyclo[3.1.1]heptanylene-$(CH_2)_2$-, ##-$CH_2$-diazabicyclo[3.1.1]heptanylene-$(CH_2)_3$-, ##-$CH_2$-diazabicyclo[3.1.1]heptanylene-$(CH_2)_4$-, ##-$CH_2$-diazabicyclo[3.1.1]heptanylene-$(CH_2)_5$-, ##-$CH_2$-diazabicyclo[3.1.1]heptanylene-$(CH_2)_6$-, ##-$CH_2$-diazabicyclo[3.1.1]heptanylene-$(CH_2)_7$-, ##-$CH_2$-diazabicyclo[3.1.1]heptanylene-$(CH_2)_8$-, ##-$(CH_2)_2$-diazabicyclo[3.1.1]heptanylene-$(CH_2)_1$-, ##-$(CH_2)_2$-diazabicyclo[3.1.1]heptanylene-$(CH_2)_2$-, ##-$(CH_2)_2$-diazabicyclo[3.1.1]heptanylene-$(CH_2)_3$-, ##-$(CH_2)_2$-diazabicyclo[3.1.1]heptanylene-$(CH_2)_4$-, ##-$(CH_2)_2$-diazabicyclo[3.1.1]heptanylene-$(CH_2)_5$-, ##-$(CH_2)_2$-diazabicyclo[3.1.1]heptanylene-$(CH_2)_6$-, ##-$(CH_2)_2$-diazabicyclo[3.1.1]heptanylene-$(CH_2)_7$-, ##-$(CH_2)_2$-diazabicyclo[3.1.1]heptanylene-$(CH_2)_8$-, ##-$(CH_2)_3$-diazabicyclo[3.1.1]heptanylene-$CH_2$-, ##-$(CH_2)_3$-diazabicyclo[3.1.1]heptanylene-$(CH_2)_2$-, ##-$(CH_2)_3$-diazabicyclo[3.1.1]heptanylene-$(CH_2)_3$-, ##-$(CH_2)_3$-diazabicyclo[3.1.1]heptanylene-$(CH_2)_4$-, ##-$(CH_2)_3$-diazabicyclo[3.1.1]heptanylene-$(CH_2)_5$-, ##-$(CH_2)_3$-diazabicyclo[3.1.1]heptanylene-$(CH_2)_6$-, ##-$(CH_2)_3$-diazabicyclo[3.1.1]heptanylene-$(CH_2)_7$-, ##-$(CH_2)_3$-diazabicyclo[3.1.1]heptanylene-$(CH_2)_8$-, ##-$(CH_2)_4$-diazabicyclo[3.1.1]heptanylene-$CH_2$-, ##-$(CH_2)_4$-diazabicyclo[3.1.1]heptanylene-$(CH_2)_2$-, ##-$(CH_2)_4$-diazabicyclo[3 .1.1]heptanylene-$(CH_2)_3$-, ##-$(CH_2)_4$-diazabicyclo[3.1.1]heptanylene-$(CH_2)_4$-, ##-$(CH_2)_4$-diazabicyclo[3.1.1]heptanylene-$(CH_2)_5$-, ##-$(CH_2)_4$-diazabicyclo[3.1.1]heptanylene-$(CH_2)_6$-, ##-$(CH_2)_4$-diazabicyclo[3.1.1]heptanylene-$(CH_2)_7$-, ##-$(CH_2)_4$-diazabicyclo[3.1.1]heptanylene-$(CH_2)_8$-, ##-$(CH_2)_5$-diazabicyclo[3.1.1]heptanylene-$(CH_2)_1$-, ##-$(CH_2)_5$-diazabicyclo[3.1.1]heptanylene-$(CH_2)_2$-, ##-$(CH_2)_5$-diazabicyclo[3.1.1]heptanylene-$(CH_2)_3$-, ##-$(CH_2)_5$-diazabicyclo[3.1.1]heptanylene-$(CH_2)_4$-, ##-$(CH_2)_5$-diazabicyclo[3.1.1]heptanylene-$(CH_2)_5$-, ##-$(CH_2)_5$-diazabicyclo[3.1.1]heptanylene-$(CH_2)_6$-, ##-$(CH_2)_5$-diazabicyclo[3.1.1]heptanylene-$(CH_2)_7$-, ##-$(CH_2)_5$-diazabicyclo[3.1.1]heptanylene-$(CH_2)_8$-, ##-$(CH_2)_6$-diazabicyclo[3 .1.1]heptanylene-$(CH_2)_1$-, ##-$(CH_2)_6$-diazabicyclo[3.1.1]heptanylene-$(CH_2)_2$-, ##-$(CH_2)_6$-diazabicyclo[3.1.1]heptanylene-$(CH_2)_3$-, ##-$(CH_2)_6$-diazabicyclo[3.1.1]heptanylene-$(CH_2)_4$-, ##-$(CH_2)_6$-diazabicyclo[3.1.1]heptanylene-$(CH_2)_5$-, ##-$(CH_2)_6$-diazabicyclo[3.1.1]heptanylene-$(CH_2)_6$-, ##-$(CH_2)_6$-diazabicyclo[3.1.1]heptanylene-$(CH_2)_7$-, ##-$(CH_2)_6$-diazabicyclo[3 .1.1]heptanylene-$(CH_2)_8$-, ##-$(CH_2)_7$-diazabicyclo[3.1.1]heptanylene-$(CH_2)_1$-, ##-$(CH_2)_7$-diazabicyclo[3.1.1]heptanylene-$(CH_2)_2$-, ##-$(CH_2)_7$-diazabicyclo[3.1.1]heptanylene-$(CH_2)_3$-, ##-$(CH_2)_7$-diazabicyclo[3.1.1]heptanylene-$(CH_2)_4$-, ##-$(CH_2)_7$-diazabicyclo[3.1.1]heptanylene-$(CH_2)_8$-, ##-$(CH_2)_5$-diazabicyclo[3.1.1]heptanylene-$CH_2$-, ##-$(CH_2)_5$-diazabicyclo[3.1.1]heptanylene-$(CH_2)_2$-, ##-$(CH_2)_5$-diazabicyclo[3.1.1]heptanylene-$(CH_2)_3$-, ##-$(CH_2)_8$-diazabicyclo[3 .1.1]heptanylene-$(CH_2)_4$-, ##-$(CH_2)_8$-diazabicyclo[3.1.1]heptanylene-$(CH_2)_5$-, ##-$(CH_2)_5$-diazabicyclo[3.1.1]heptanylene-$(CH_2)_6$-, ##-$(CH_2)_8$-diazabicyclo[3.1.1]heptanylene-$(CH_2)_7$-, ##-$(CH_2)_8$-diazabicyclo[3.1.1]heptanylene-$(CH_2)_8$-, ##-$CH_2$-diazabicyclo[3.2.1]octylene-$CH_2$-, ##-$CH_2$-diazabicyclo[3.2.1]octylene-$(CH_2)_2$-, ##-$CH_2$-diazabicyclo[3.2. 1]octylene-$(CH_2)_3$-, ##-$CH_2$-diazabicyclo[3.2.1]octylene-$(CH_2)_4$-, ##-$CH_2$-diazabicyclo[3.2. 1]octylene-$(CH_2)_5$-, ##-$CH_2$-diazabicyclo[3.2.1]octylene-$(CH_2)_6$-, ##-$CH_2$-diazabicyclo[3.2. 1]octylene-$(CH_2)_7$-, ##-$CH_2$-diazabicyclo[3.2.1]octylene-$(CH_2)_8$-, ##-$(CH_2)_2$-diazabicyclo[3.2.1]octylene-$(CH_2)_1$-, ##-$(CH_2)_2$-diazabicyclo[3.2.1]octylene-$(CH_2)_2$-, ##-$(CH_2)_2$-diazabicyclo[3.2.1]octylene-$(CH_2)_3$-, ##-$(CH_2)_2$-diazabicyclo[3.2.1]octylene-$(CH_2)_4$-, ##-$(CH_2)_2$-diazabicyclo[3.2.1]octylene-$(CH_2)_5$-, ##-$(CH_2)_2$-diazabicyclo[3.2.1]octylene-$(CH_2)_6$-, ##-$(CH_2)_2$-diazabicyclo[3.2.1]octylene-$(CH_2)_7$-, ##-$(CH_2)_2$-diazabicyclo[3.2.1]octylene-$(CH_2)_8$-, ##-$(CH_2)_3$-diazabicyclo[3.2. 1]octylene-$CH_2$-, ##-$(CH_2)_3$-diazabicyclo[3.2.1]octylene-$(CH_2)_2$-, ##-$(CH_2)_3$-diazabicyclo[3.2.1]octylene-$(CH_2)_3$-, ##-$(CH_2)_3$-diazabicyclo[3.2.1]octylene-$(CH_2)_4$-, ##-$(CH_2)_3$-diazabicyclo[3.2. 1]octylene-$(CH_2)_5$-, ##-$(CH_2)_3$-diazabicyclo[3.2.1]octylene-$(CH_2)_6$-, ##-$(CH_2)_3$-diazabicyclo[3.2.1]octylene-$(CH_2)_7$-, ##-$(CH_2)_3$-diazabicyclo[3.2.1]octylene-$(CH_2)_8$-, ##-$(CH_2)_4$-diazabicyclo[3.2. 1]octylene-$CH_2$-, ##-$(CH_2)_4$-diazabicyclo[3.2.1]octylene-$(CH_2)_2$-, ##-$(CH_2)_4$-diazabicyclo[3.2.1]octylene-$(CH_2)_3$-, ##-$(CH_2)_4$-diazabicyclo[3.2.1]octylene-$(CH_2)_4$-, ##-$(CH_2)_4$-diazabicyclo[3.2.1]octylene-$(CH_2)_5$-, ##-$(CH_2)_4$-diazabicyclo[3.2.1]octylene-$(CH_2)_6$-, ##-$(CH_2)_4$-diazabicyclo[3.2.1]octylene-$(CH_2)_7$-, ##-$(CH_2)_4$-diazabicyclo[3.2.1]octylene-$(CH_2)_8$-, ##-$(CH_2)_5$-diazabicyclo[3.2.1]octylene-$(CH_2)_1$-, ##-$(CH_2)_5$-diazabicyclo[3.2.1]octylene-$(CH_2)_2$-, ##-$(CH_2)_5$-diazabicyclo[3.2.l]octylene-$(CH_2)_3$-, ##-$(CH_2)_5$-diazabicyclo[3.2.1]octylene-$(CH_2)_4$-, ##-$(CH_2)_5$-diazabicyclo[3.2. 1]octylene-$(CH_2)_5$-, ##-$(CH_2)_5$-diazabicyclo[3.2.1]octylene-$(CH_2)_6$-, ##-$(CH_2)_5$-diazabicyclo[3.2.1]octylene-$(CH_2)_7$-, ##-$(CH_2)_5$-diazabicyclo[3.2.1]octylene-$(CH_2)_8$-, ##-$(CH_2)_6$-diazabicyclo[3.2. 1]octylene-$(CH_2)_1$-, ##-$(CH_2)_6$-diazabicyclo[3.2.1]octylene-$(CH_2)_2$-, ##-$(CH_2)_6$-diazabicyclo[3.2.1]octylene-$(CH_2)_3$-, ##-$(CH_2)_6$-diazabicyclo[3.2.1]octylene-$(CH_2)_4$-, ##-$(CH_2)_6$-diazabicyclo[3.2.1]octylene-$(CH_2)_5$-, ##-$(CH_2)_6$-diazabicyclo[3.2.1]octylene-$(CH_2)_6$-, ##-$(CH_2)_6$-diazabicyclo[3.2.1]octylene-$(CH_2)_7$-, ##-$(CH_2)_6$-diazabicyclo[3.2.1]octylene-$(CH_2)_8$-, ##-$(CH_2)_7$-diazabicyclo[3.2.1]octylene-$(CH_2)_1$-, ##-$(CH_2)_7$-diazabicyclo[3.2.1]octylene-$(CH_2)_2$-, ##-$(CH_2)_7$-diazabicyclo[3.2.1]octylene-$(CH_2)_3$-, ##-$(CH_2)_7$-diazabicyclo[3.2.1]octylene-$(CH_2)_4$-, ##-$(CH_2)_7$-diazabicyclo[3.2. 1]octylene-$(CH_2)_8$-, ##-$(CH_2)_8$-diazabicyclo[3.2.1]octylene-$CH_2$-, ##-$(CH_2)_8$-diazabicyclo[3.2.1]octylene-$(CH_2)_2$-, ##-$(CH_2)_8$-diazabicyclo[3.2.1]octylene-$(CH_2)_3$-, ##-$(CH_2)_8$-diazabicyclo[3.2.1]octylene-$(CH_2)_4$-, ##-$(CH_2)_8$-diazabicyclo[3.2.1]octyle-

ne-$(CH_2)_5$-, ##-$(CH_2)_8$-diazabicyclo[3.2.1]octylene-$(CH_2)_6$-, ##-$(CH_2)_8$-diazabicyclo[3.2.1]octylene-$(CH_2)_7$-, ##-$(CH_2)_8$-diazabicyclo[3.2.1]octylene-$(CH_2)_8$-, ##-$CH_2$-diazabicyclo[2.2.2]octylene-$CH_2$-, ##-$CH_2$-diazabicyclo[2.2.2]octylene-$(CH_2)_2$-, ##-$CH_2$-diazabicyclo[2.2.2]octylene-$(CH_2)_3$-, ##-$CH_2$-diazabicyclo[2.2.2]octylene-$(CH_2)_4$-, ##-$CH_2$-diazabicyclo[2.2.2]octylene-$(CH_2)_5$-, ##-$CH_2$-diazabicyclo[2.2.2]octylene-$(CH_2)_6$-, ##-$CH_2$-diazabicyclo[2.2.2]octylene-$(CH_2)_7$-, ##-$CH_2$-diazabicyclo[2.2.2]octylene-$(CH_2)_8$-, ##-$(CH_2)_2$-diazabicyclo[2.2.2]octylene-$(CH_2)_1$-, ##-$(CH_2)_2$-diazabicyclo[2.2.2]octylene-$(CH_2)_2$-, ##-$(CH_2)_2$-diazabicyclo[2.2.2]octylene-$(CH_2)_3$-, ##-$(CH_2)_2$-diazabicyclo[2.2.2]octylene-$(CH_2)_4$-, ##-$(CH_2)_2$-diazabicyclo[2.2.2]octylene-$(CH_2)_5$-, ##-$(CH_2)_2$-diazabicyclo[2.2.2]octylene-$(CH_2)_6$-, ##-$(CH_2)_2$-diazabicyclo[2.2.2]octylene-$(CH_2)_7$-, ##-$(CH_2)_2$-diazabicyclo[2.2.2]octylene-$(CH_2)_8$-, ##-$(CH_2)_3$-diazabicyclo[2.2.2]octylene-$CH_2$-, ##-$(CH_2)_3$-diazabicyclo[2.2.2]octylene-$(CH_2)_2$-, ##-$(CH_2)_3$-diazabicyclo[2.2.2]octylene-$(CH_2)_3$-, ##-$(CH_2)_3$-diazabicyclo[2.2.2]octylene-$(CH_2)_4$-, ##-$(CH_2)_3$-diazabicyclo[2.2.2]octylene-$(CH_2)_5$-, ##-$(CH_2)_3$-diazabicyclo[2.2.2]octylene-$(CH_2)_6$-, ##-$(CH_2)_3$-diazabicyclo[2.2.2]octylene-$(CH_2)_7$-, ##-$(CH_2)_3$-diazabicyclo[2.2.2]octylene-$(CH_2)_8$-, ##-$(CH_2)_4$-diazabicyclo[2.2.2]octylene-$CH_2$-, ##-$(CH_2)_4$-diazabicyclo[2.2.2]octylene-$(CH_2)_2$-, ##-$(CH_2)_4$-diazabicyclo[2.2.2]octylene-$(CH_2)_3$-, ##-$(CH_2)_4$-diazabicyclo[2.2.2]octylene-$(CH_2)_4$-, ##-$(CH_2)_4$-diazabicyclo[2.2.2]octylene-$(CH_2)_5$-, ##-$(CH_2)_4$-diazabicyclo[2.2.2]octylene-$(CH_2)_6$-, ##-$(CH_2)_4$-diazabicyclo[2.2.2]octylene-$(CH_2)_7$-, ##-$(CH_2)_4$-diazabicyclo[2.2.2]octylene-$(CH_2)_8$-, ##-$(CH_2)_5$-diazabicyclo[2.2.2]octylene-$(CH_2)_1$-, ##-$(CH_2)_5$-diazabicyclo[2.2.2]octylene-$(CH_2)_2$-, ##-$(CH_2)_5$-diazabicyclo[2.2.2]octylene-$(CH_2)_3$-, ##-$(CH_2)_5$-diazabicyclo[2.2.2]octylene-$(CH_2)_4$-, ##-$(CH_2)_5$-diazabicyclo[2.2.2]octylene-$(CH_2)_5$-, ##-$(CH_2)_5$-diazabicyclo[2.2.2]octylene-$(CH_2)_6$-, ##-$(CH_2)_5$-diazabicyclo[2.2.2]octylene-$(CH_2)_7$-, ##-$(CH_2)_5$-diazabicyclo[2.2.2]octylene-$(CH_2)_8$-, ##-$(CH_2)_6$-diazabicyclo[2.2.2]octylene-$(CH_2)_1$-, ##-$(CH_2)_6$-diazabicyclo[2.2.2]octylene-$(CH_2)_2$-, ##-$(CH_2)_6$-diazabicyclo[2.2.2]octylene-$(CH_2)_3$-, ##-$(CH_2)_6$-diazabicyclo[2.2.2]octylene-$(CH_2)_4$-, ##-$(CH_2)_6$-diazabicyclo[2.2.2]octylene-$(CH_2)_5$-, ##-$(CH_2)_6$-diazabicyclo[2.2.2]octylene-$(CH_2)_6$-, ##-$(CH_2)_6$-diazabicyclo[2.2.2]octylene-$(CH_2)_7$-, ##-$(CH_2)_6$-diazabicyclo[2.2.2]octylene-$(CH_2)_8$-, ##-$(CH_2)_7$-diazabicyclo[2.2.2]octylene-$(CH_2)_1$-, ##-$(CH_2)_7$-diazabicyclo[2.2.2]octylene-$(CH_2)_2$-, ##-$(CH_2)_7$-diazabicyclo[2.2.2]octylene-$(CH_2)_3$-, ##-$(CH_2)_7$-diazabicyclo[2.2.2]octylene-$(CH_2)_4$-, ##-$(CH_2)_7$-diazabicyclo[2.2.2]octylene-$(CH_2)_8$-, ##-$(CH_2)_8$-diazabicyclo[2.2.2]octylene-$CH_2$-, ##-$(CH_2)_8$-diazabicyclo[2.2.2]octylene-$(CH_2)_2$-, ##-$(CH_2)$s-diazabicyclo[2.2.2]octylene-$(CH_2)_3$-, ##-$(CH_2)_8$-diazabicyclo[2.2.2]octylene-$(CH_2)_4$-, ##-$(CH_2)_8$-diazabicyclo[2.2.2]octylene-$(CH_2)_5$-, ##-$(CH_2)_8$-diazabicyclo[2.2.2]octylene-$(CH_2)_6$-, ##-$(CH_2)_8$-diazabicyclo[2.2.2]octylene-$(CH_2)_7$-, or ##-$(CH_2)_8$-diazabicyclo[2.2.2]octylene-$(CH_2)_8$-;

wherein the propylene, the piperidinylene, the piperazinylene, the diazacycloheptanylene, the diazabicyclo[2.2.1]heptanylene, the diazabicyclo[3.1.1]heptanylene, the diazabicyclo[3.2.1]octylene, the diazabicyclo[2.2.2]octylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;

each $R_{g10}$ independently represents H or $C_{1-3}$ alkyl; and

symbol ## indicates the point of attachment to $X_1$.

In some embodiments of the compound of Formula (I-2), $L_1$ represents the following group:

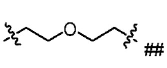 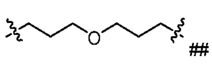 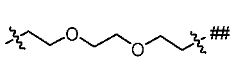 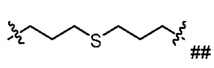 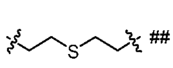 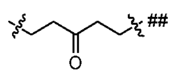

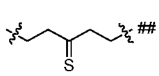 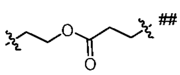 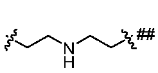 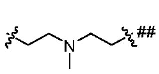 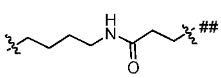 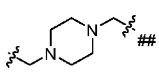 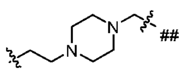

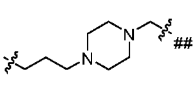 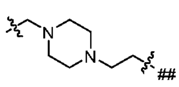 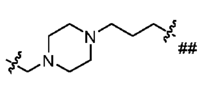 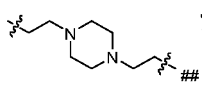 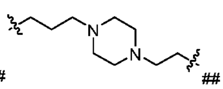 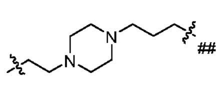

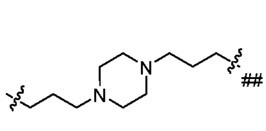 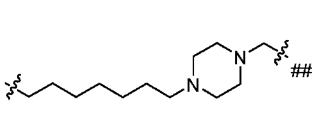 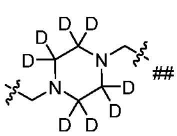 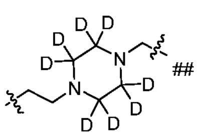 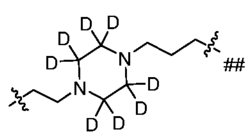

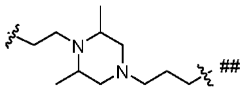 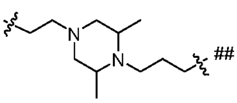 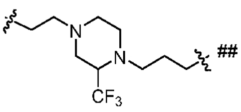 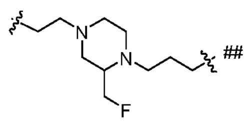 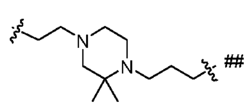

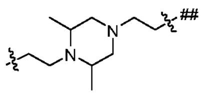 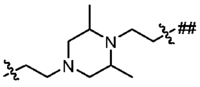 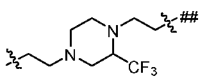 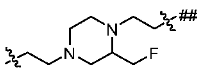 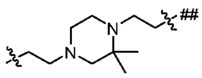 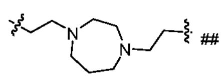

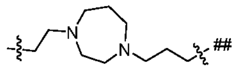 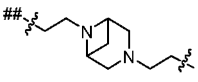 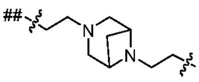 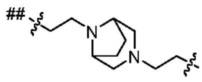 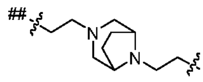 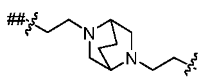

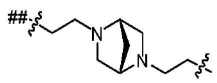 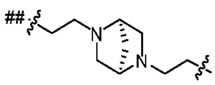 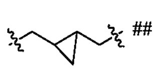 or 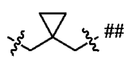

wherein symbol ## indicates the point of attachment to $X_1$.

In some embodiments of the compound of Formula (I-2), $L_1$ represents the structure of the following formula: ##-$C_{1-30}$ alkylene-, wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the $C_{1-30}$ alkylene is optionally replaced with a substituent selected from the group consisting of: D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy or any combination thereof, and symbol ## indicates the point of attachment to $X_1$.

In some embodiments of the compound of Formula (I-2), $L_1$ represents the following group:

##-$CH_2$-, ##-$(CH_2)_2$-, ##-$(CH_2)_3$-, ##-$(CH_2)_4$-, ##-$(CH_2)_5$-, ##-$(CH_2)_2$-$CF_2$-$(CH_2)_2$-, ##-$(CH_2)_6$-, ##-$(CH_2)_7$-, ##-$(CH_2)_8$-, ##-$(CH_2)_9$-, ##-$(CH_2)_{10}$-, ##-$(CH_2)_{11}$-, ##-$(CH_2)_{12}$-, ##-$(CH_2)_{13}$-, ##-$(CH_2)_{14}$-, ##-$(CH_2)_{15}$-, ##-$(CH_2)_{16}$-, ##-$(CH_2)_{17}$-, ##-$(CH_2)_{18}$-, ##-$(CH_2)_{19}$-, ##-$(CH_2)_{20}$-, ##-$(CH_2)_{21}$-, ##-$(CH_2)_{22}$-, ##-$(CH_2)_{25}$-, or ##-$(CH_2)_{30}$-;

wherein a hydrogen atom of one or more (e.g., 1-20, or 1-15, 1-10, 1-9, 1-8, 1-7, 1-6, 1-5, 1-4, 1-3 or 1-2, or 1) $CH_2$ of the group is optionally replaced with a substituent selected from the group consisting of: D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy or any combination thereof, and symbol ## indicates the point of attachment to $X_1$.

In some embodiments of the compound of Formula (I-2), $R_1$ represents $C_{1-60}$ alkyl (e.g., $C_1$-$C_{30}$ alkyl, $C_1$-$C_{29}$ alkyl, $C_1$-$C_{2S}$ alkyl, $C_1$-$C_{27}$ alkyl, $C_1$-$C_{26}$ alkyl, $C_1$-$C_{25}$ alkyl, $C_1$-$C_{24}$ alkyl, $C_1$-$C_{23}$ alkyl, $C_1$-$C_{22}$ alkyl, $C_1$-$C_{21}$ alkyl, $C_1$-$C_{20}$ alkyl, $C_1$-$C_{19}$ alkyl, $C_1$-$C_{18}$ alkyl, $C_1$-$C_{17}$ alkyl, $C_1$-$C_{16}$ alkyl, $C_1$-$C_{15}$ alkyl, $C_1$-$C_{14}$ alkyl, $C_1$-$C_{13}$ alkyl, $C_1$-$C_{12}$ alkyl, $C_1$-$C_{11}$ alkyl, $C_1$-$C_{10}$ alkyl, $C_1$-$C_9$ alkyl, $C_1$-$C_8$ alkyl, $C_1$-$C_7$ alkyl, $C_1$-$C_6$ alkyl, $C_1$-$C_5$ alkyl, $C_1$-$C_4$ alkyl, $C_1$-$C_3$ alkyl, or $C_1$-$C_2$ alkyl) optionally substituted with D or halogen.

In some embodiments of the compound of Formula (I-2), $R_b$ represents the following group: $CH_3$, $CF_3$, $CD_3$, $CH_2CH_3$, -$(CH_2)_2$-$CH_3$, -$(CH_2)_3$-$CH_3$, -$(CH_2)_4$-$CH_3$, -$(CH_2)_5$-$CH_3$, -$(CH_2)_6$-$CH_3$, - $(CH_2)_7$-$CH_3$, -$(CH_2)_8$-$CH_3$, -$(CH_2)_9$-$CH_3$, -$(CH_2)_{10}$-$CH_3$, -$(CH_2)_{11}$-$CH_3$, -$(CH_2)_{12}$-$CH_3$, -$(CH_2)_{13}$-$CH_3$, - $(CH_2)_{14}$-$CH_3$, -$(CH_2)_{15}$-$CH_3$, -$(CH_2)_{16}$-$CH_3$, -$(CH_2)_{17}$-$CH_3$, -$(CH_2)_{18}$-$CH_3$, -$(CH_2)_{19}$-$CH_3$, -$(CH_2)_{20}$-$CH_3$, -$(CH_2)_{21}$-$CH_3$, -$(CH_2)_{22}$-$CH_3$, -$(CH_2)_{23}$-$CH_3$, -$(CH_2)_{24}$-$CH_3$, -$(CH_2)_{25}$-$CH_3$, -$(CH_2)_{26}$-$CH_3$, -$(CH_2)_{27}$-$CH_3$, -$(CH_2)_{28}$-$CH_3$, or -$(CH_2)_{29}$-$CH_3$.

In some embodiments of the compound of Formula (I-2), $R_b$ represents the following formula:

$$R_{11}\text{-}X_4\text{-}L_2\text{-} ,$$

wherein $L_2$ represents:

linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, $C_{1-4}$ alkyl, halogen, $C_{3-6}$cycloalkyl or any combination thereof, or

optionally substituted linear or branched $C_{2-60}$ alkylene (e.g., $C_2$-$C_{40}$ alkylene, $C_2$-$C_{30}$ alkylene, $C_2$-$C_{29}$ alkylene,

$C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{12}$ and/or one or more (e.g., 1-20, or 1-15, 1-10, 1-9, 1-8, 1-7, 1-6, 1-5, 1-4, 1-3 or 1-2, or 1) groups $R_{13}$ and/or any combination of one or more (e.g., 1-20, or 1-15, 1-10, 1-9, 1-8, 1-7, 1-6, 1-5, 1-4, 1-3 or 1-2, or 1) groups $R_{12}$ and $R_{13}$ inserted into its backbone carbon chain, wherein carbon-carbon bond between one or more pairs (e.g., 1-10, 1-9, 1-8, 1-7, 1-6, 1-5, 1-4, 1-3 or 1-2, or 1 pair) of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of $C_{3-15}$cycloalkylene, 4- to 15-membered heterocyclylene, $C_{6-10}$ arylene, 5- to 15-membered heteroarylene, $C_{2-6}$ alkenylene, $C_{2-6}$ alkynylene or any combination thereof, wherein the $C_{3-15}$cycloalkylene, the 4- to 15-membered heterocyclylene, $C_{6-10}$ arylene and the 5- to 15-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O) NH-, and $CH_3CH_2CH_2$-C(O)NH-) or any combination thereof, and the linear or branched $C_{2-60}$ alkylene is optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, $C_{1-4}$ alkyl (e.g., $C_{1-3}$ alkyl, such as methyl, ethyl, propyl, isopropyl, butyl, sec-butyl or tert-butyl), halogen, $C_{3-6}$cycloalkyl or any combination thereof;

$X_4$ represents a bond, or $X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted $C_{3-20}$cycloalkylene, optionally substituted $C_{6-20}$ arylene, optionally substituted 4- to 20-membered heterocyclylene, optionally substituted 5- to 20-membered heteroarylene, triazolylene-NH-, or -NH-triazolylene, wherein $R_{15}$ represents H or $C_{1-3}$ alkyl, and the $C_{3-20}$cycloalkylene, the $C_{6-20}$ arylene, the 4- to 20-membered heterocyclylene, and the 5- to 20-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, halogen, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof; and

$R_{11}$ represents optionally substituted $C_{3-20}$cycloalkyl, optionally substituted $C_{6-20}$ aryl, optionally substituted 4- to 20-membered heterocyclyl or optionally substituted 5- to 20-membered heteroaryl, wherein the $C_{3-20}$cycloalkyl, the $C_{6-20}$ aryl, the 4- to 20-membered heterocyclyl, and the 5- to 20-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-,

and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof.

In some embodiments of the compound of Formula (I-2), $L_2$ represents the structure of the following formula: -$C_{2-30}$ alkylene-, wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the $C_{2-30}$ alkylene is optionally replaced with a substituent selected from the group consisting of: D, $C_{1-4}$ alkyl (e.g., $C_{1-3}$ alkyl, such as methyl, ethyl, propyl, isopropyl, butyl, sec-butyl or tert-butyl), halogen, $C_{3-6}$cycloalkyl or any combination thereof.

In some embodiments of the compound of Formula (I-2), $L_2$ represents the following group:

$-CH_2-$, $-(CH_2)_2-$, $-(CH_2)_3-$, $-(CH_2)_4-$, $-(CH_2)_5-$, $-(CH_2)_2-CF_2-(CH_2)_2-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{15}-$, $-(CH_2)_{16}-$, $-(CH_2)_{17}-$, $-(CH_2)_{18}-$, $-(CH_2)_{19}-$, $-(CH_2)_{20}-$, $-(CH_2)_{21}-$, $-(CH_2)_{22}-$, $-(CH_2)_{25}-$, or $-(CH_2)_{30}-$;

wherein a hydrogen atom of one or more (e.g., 1-20, or 1-15, 1-10, 1-9, 1-8, 1-7, 1-6, 1-5, 1-4, 1-3 or 1-2, or 1) $CH_2$ of the group is optionally replaced with a substituent selected from the group consisting of: D, $C_{1-4}$ alkyl (e.g., $C_{1-3}$ alkyl, such as methyl, ethyl, propyl, isopropyl, butyl, sec-butyl or tert-butyl), halogen, $C_{3-6}$cycloalkyl or any combination thereof.

In some embodiments of the compound of Formula (I-2), $L_2$ represents the structure of the following formula:

$-(C(R^{a9})(R^{a10}))_{m1}-(R_{12}(C(R^{a11})(R^{a12}))_{m2})_{p1}-$;

$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-$;

$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-(R_{12}(C(R^{a15})(R^{a16}))_{m4})_{p3}-$;

$-(C(R^{a9})(R^{a10}))_{m1}-(R_{12}(C(R^{a11})(R^{a12}))_{m2})_{p1}-$;

$-(C(R^{a9})(R^{a10}))_{m1}-(R_{13}(C(R^{a11})(R^{a12}))_{m2})_{p1}-(R_{13}(C(R^{a13})(R^{a14}))_{m3})_{p2}-$;

$-(C(R^{a9})(R^{a10}))_{m1}-(R_{13}(C(R^{a11})(R^{a12}))_{m2})_{p1}-(R_{13}(C(R^{a13})(R^{a14}))_{m3})_{p2}-(R_{13}(C(R^{a15})(R^{a16}))_{m4})_{p3}-$;

$-(C(R^{a9})(R^{a10}))_{m1}-(R_{12}-R_{13}-(C(R^{a11})(R^{a12}))_{m2})_{p1}-$;

$-(C(R^{a9})(R^{a10}))_{m1}-(R_{12}(C(R^{a11})(R^{a12}))_{m2})_{p1}-(R_{13}(C(R^{a13})(R^{a14}))_{m3})_{p2}-$;

$-(C(R^{a9})(R^{a10}))_{m1}-(R_{13}-R_{12}-(C(R^{a11})(R^{a12}))_{m2})_{p1}-$; or

$-(C(R^{a9})(R^{a10}))_{m1}-(R_{13}(C(R^{a11})(R^{a12}))_{m2})_{p1}-(R_{12}(C(R^{a13})(R^{a14}))_{m3})_{p2}-$; -

wherein each group $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_{14}$ independently represents H or $C_{1-3}$ alkyl; and each group $R_{13}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene,

$C_{1-4}$ alkyl-NHC(O)-, $C_{1-4}$ alkyl-C(O)NH- or any combination thereof;
$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and
m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-2), $L_2$ represents the structure of the following formula:

$-(C(R^{a9})(R^{a10}))_{m1}-(O(C(R^{a11})(R^{a12}))_{m2})_{p1}-$;

$-(C(R^{a9})(R^{a10}))_{m1}-(O(C(R^{a11})(R^{a12}))_{m2})_{p1}-(O(C(R^{a13})(R^{a14}))_{m3})_{p2}$;

$-(C(R^{a9})(R^{a10}))_{m1}-(O(C(R^{a11})(R^{a12}))_{m2})_{p1}-(O(C(R^{a13})(R^{a14}))_{m3})_{p2}-(O(C(R^{a15})(R^{a16}))_{m4})_{p3}-$;

$-(C(R^{a9})(R^{a10}))_{m1}-(N(R_{g11})(C(R^{a11})(R^{a12}))_{m2})_{p1}-$;

$-(C(R^{a9})(R^{a10}))_{m1}-(N(R_{g11})(C(R^{a11})(R^{a12}))_{m2})_{p1}-(N(R_{g11})(C(R^{a13})(R^{a14}))_{m3})_{p2}$;

$-(C(R^{a9})(R^{a10}))_{m1}-(N(R_{g11})(C(R^{a11})(R^{a12}))_{m2})_{p1}-(N(R_{g11})(C(R^{a13})(R^{a14}))_{m3})_{p2}-(N(R_{g11})(C(R^{a15})(R^{a16}))_{m4})_{p3}$;

$-(C(R^{a9})(R^{a10}))_{m1}-(C(O)NH-(C(R^{a11})(R^{a12}))_{m2})_{p1}-$;

$-(C(R^{a9})(R^{a10}))_{m1}-(C(O)NH-(C(R^{a11})(R^{a12}))_{m2})_{p1}-(C(O)NH-(C(R^{a13})(R^{a14}))_{m3})_{p2}-$;

$-(C(R^{a9})(R^{a10}))_{m1}-(C(O)NH-(C(R^{a11})(R^{a12}))_{m2})_{p1}-(C(O)NH-(C(R^{a13})(R^{a14}))_{m3})_{p2}-(C(O)NH-(C(R^{a15})(R^{a16}))_{m4})_{p3}$;

$(C(R^{a9})(R^{a10}))_{m1}-(C(O)NH-(C(R^{a11})(R^{a12}))_{m2})_{p1}-(O-(C(R^{a13})(R^{a14}))_{m3})_{p2}-$;

$(C(R^{a9})(R^{a10}))_{m1}-(C(O)NH-(C(R^{a11})(R^{a12}))_{m2})-(O-(C(R^{a13})(R^{a14}))_{m3})_{p1}-$;

$-(C(R^{a9})(R^{a10}))_{m1}-(NHC(O)-(C(R^{a11})(R^{a12}))_{m2})_{p1}-$;

$-(C(R^{a9})(R^{a10}))_{m1}-(NHC(O)-(C(R^{a11})(R^{a12}))_{m2})_{p1}-(O-(C(R^{a13})(R^{a14}))_{m3})_{p2}-$;

$-(C(R^{a9})(R^{a10}))_{m1}-(NHC(O)-(C(R^{a11})(R^{a12}))_{m2})_{p1}-(O-(C(R^{a13})(R^{a14}))_{m3})_{p2}-(O-(C(R^{a15})(R^{a16}))_{m4})_{p3}$;

$-(C(R^{a9})(R^{a10}))_{m1}-NHC(O)-(C(R^{a11})(R^{a12}))_{m2})_{p1}-(O-(C(R^{a13})(R^{a14}))_{m3})_{p1}-$;

$-(C(R^{a9})(R^{a10}))_{m1}-NHC(O)NH-(C(R^{a11})(R^{a12}))_{m2}-$;

$-C(R^{a9})(R^{a10}))_{m1}-C(O)-(C(R^{a11})(R^{a12}))_{m2}-$;

$-C(R^{a9})(R^{a10}))_{m1}-C(S)-(C(R^{a11})(R^{a12}))_{m2}-$;

$-C(R^{a9})(R^{a10}))_{m1}-S-(C(R^{a11})(R^{a12}))_{m2}-$;

$-C(R^{a9})(R^{a10}))_{m1}-C_{6-10}$ arylene(O)-$(C(R^{a11})(R^{a12}))_{m2})_{p1}-$;

$-C(R^{a9})(R^{a10}))_{m1}-C_{6-10}$ arylene(O)-$(C(R^{a11})(R^{a12}))_{m2})_{p1}-C_{6-10}$ arylene(O)-$(C(R^{a13})(R^{a14}))_{m3}-$;

$-(C(R^{a9})(R^{a10}))_{m1}-$(4- to 15-membered heterocyclylene-$(C(R^{a11})(R^{a12}))_{m2})_{p1}-$;

$-(C(R^{a9})(R^{a10}))_{m1}-$(4- to 15-membered heterocyclylene-$(C(R^{a11})(R^{a12}))_{m2})_{p1}-$(4- to 15-membered heterocyclylene-$(C(R^{a13})(R^{a14}))_{m3})_{p2}-$;

$-(C(R^{a9})(R^{a10}))_{m1}-$(5- to 15-membered heterocyclylene-$(C(R^{a11})(R^{a12}))_{m2})_{p1}-$;

$-(C(R^{a9})(R^{a10}))_{m1}$-(5- to 15-membered heterocyclylene-$(C(R^{a11})(R^{a12}))_{m2})_{p1}$-(5- to 15-membered heterocyclylene-$(C(R^{a13})(R^{a14}))_{m3})_{p2}$-;

$-(C(R^{a9})(R^{a10}))_{m1}-(C_{3-15}$cycloalkylene-$(C(R^{a11})(R^{a12}))_{m2})_{p1}$-; or

$-(C(R^{a9})(R^{a10}))_{m1}-(C_{3-15}$cycloalkylene-$(C(R^{a11})(R^{a12}))_{m2})_{p1}-(C_{3-15}$cycloalkylene-$(C(R^{a13})(R^{a14}))_{m3})_{p2}$-;

wherein each $R_{g11}$ independently represents H or $C_{1-3}$ alkyl;
the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;
$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and
m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-2), $L_2$ represents the structure of the following formula: $-CH_2-O-CH_2-$, $-CH_2-O-(CH_2)_2-$, $-(CH_2)_1-O-(CH_2)_3-$, $-(CH_2)_1-O-(CH_2)_4-$, $-(CH_2)_1-O-(CH_2)_5-$, $-(CH_2)_1-O-(CH_2)_6-$, $-(CH_2)_1-O-(CH_2)_7-$, $-(CH_2)_1-O-(CH_2)_8-$, $-(CH_2)_1-O-(CH_2)_9-$, $-(CH_Z)_1-O-(CH_2)_{10}-$, $-(CH_2)_2-O-(CH_2)_1-$, $-(CH_2)_2-O-(CH_2)_2-$, $-(CH_2)_2-O-(CH_2)_3-$, $-(CH_2)_2-O-(CH_2)_4-$, $-(CH_2)_2-O-(CH_2)_5-$, $-(CH_2)_2-O-(CH_2)_6-$, $-(CH_2)_2-O-(CH_2)_7-$, $-(CH_2)_2-O-(CH_2)_8-$, $-(CH_2)_2-O-(CH_2)_9-$, $-(CH_2)_2-O-(CH_2)_{10}-$, $-(CH_2)_2-O-(CH_2)_{11}-$, $-(CH_2)_2-O-(CH_2)_{12}-$, $-(CH_2)_3-O-(CH_2)_1-$, $-(CH_2)_3-O-(CH_2)_2-$, $-(CH_2)_3-O-(CH_2)_3-$, $-(CH_2)_3-O-(CH_2)_4-$, $-(CH_2)_3-O-(CH_2)_5-$, $-(CH_2)_3-O-(CH_2)_6-$, $-(CH_2)_3-O-(CH_2)_7-$, $-(CH_2)_4-O-(CH_2)_1-$, $-(CH_2)_4-O-(CH_2)_2-$, $-(CH_2)_4-O-(CH_2)_3-$, $-(CH_2)_4-O-(CH_2)_4-$, $-(CH_2)_4-O-(CH_2)_5-$, $-(CH_2)_4-O-(CH_2)_6-$, $-(CH_2)_5-O-(CH_2)_1-$, $-(CH_2)_5-O-(CH_2)_2-$, $-(CH_2)_5-O-(CH_2)_3-$, $-(CH_2)_5-O-(CH_2)_4-$, $-(CH_2)_5-O-(CH_2)_5-$, $-(CH_2)_6-O-(CH_2)_1-$, $-(CH_2)_6-O-(CH_2)_2-$, $-(CH_2)_6-O-(CH_2)_3-$, $-(CH_2)_6-O-(CH_2)_4-$, $-(CH_2)_7-O-(CH_2)_1-$, $-(CH_2)_7-O-(CH_2)_2-$, $-(CH_2)_7-O-(CH_2)_3-$, $-(CH_2)_8-O-(CH_2)_1-$, $-(CH_2)_8-O-(CH_2)_2-$, $-CH(CH_3)-O-(CH_2)_1-$, $-CH(CH_3)-O-(CH_2)_2-$, $-CH(CH_3)-O-(CH_2)_3-$, $-CH(CH_3)-O-(CH_2)_4-$, $-CH(CH_3)-O-(CH_2)_5-$, $-CH(CH_3)-O-(CH_2)_6-$, $-CH(CH_3)-O-(CH_2)_7-$, $-CH(CH_3)-O-(CH_2)_8-$, $-CH(CH_3)-O-(CH_2)_9-$, $-CH(CH_3)-O-(CH_2)_{10}-$, $-CH_2-(O(CH_2)_2)_2-$, $-CH_2-(O(CH_2)_2)_3-$, $-CH_2-(O(CH_2)_2)_4-$, $-CH_2-(O(CH_2)_2)_5-$, $-CH_2-(O(CH_2)_2)_6-$, $-CH_2-(O(CH_2)_2)_7-$, $-CH_2-(O(CH_2)_2)_8-$, $-CH_2-(O(CH_2)_2)_9-$, $-CH_2-(O(CH_2)_2)_{10}-$, $-CH_2-(O(CH_2)_2)_1-OCH_2-$, $-CH_2-(O(CH_2)_2)_2-OCH_2-$, $-CH_2-(O(CH_2)_2)_3-OCH_2-$, $-CH_2-(O(CH_2)_2)_4-OCH_2-$, $-CH_2-(O(CH_2)_2)_5-OCH_2-$, $-CH_2-(O(CH_2)_2)_6-OCH_2-$, $-CH_2-(O(CH_2)_2)_7-OCH_2-$, $-CH_2-(O(CH_2)_2)_8-OCH_2-$, $-CH_2-(O(CH_2)_2)_9-OCH_2-$, $-CH_2-(0(CH_2)_2)_{IO}-OCH_2-$, $-(CH_2)_2-(O(CH_2)_2)_2-$, $-(CH_2)_2-(O(CH_2)_2)_3-$, $-(CH_2)_2-(O(CH_2)_2)_4-$, $-(CH_2)_2-(O(CH_2)_2)_5-$, $-(CH_2)_2-(O(CH_2)_2)_6-$, $-(CH_2)_2-(O(CH_2)_2)_7-$, $-(CH_2)_2-(O(CH_2)_2)_8-$, $-(CH_2)_2-(O(CH_2)_2)_9-$, $-(CH_2)_2-(O(CH_2)_2)_{10}-$, $-(CH_2)_3-(O(CH_2)_2)_2-$, $-(CH_2)_3-(O(CH_2)_2)_3-$, $-(CH_2)_3-(O(CH_2)_2)_4-$, $-(CH_2)_3-(O(CH_2)_2)_5-$, $-(CH_2)_3-(O(CH_2)_2)_6-$, $-(CH_2)_3-(O(CH_2)_2)_7-$, $-(CH_2)_3-(O(CH_2)_2)_8-$, $-(CH_2)_3-(O(CH_2)_2)_9-$, $-(CH_2)_3-(O(CH_2)_2)_{10}-$, $-(CH_2)_4-(O(CH_2)_2)_2-$, $-(CH_2)_4-(O(CH_2)_2)_3-$, $-(CH_2)_4-(O(CH_2)_2)_4-$, $-(CH_2)_4-(O(CH_2)_2)_5-$, $-(CH_2)_4-(O(CH_2)_2)_6-$, $-(CH_2)_4-(O(CH_2)_2)_7-$, $-(CH_2)_4-(O(CH_2)_2)_8-$, $-(CH_2)_4-(O(CH_2)_2)_9-$, $-(CH_2)_4-(O(CH_2)_2)_{10}-$, $-CH_2-(O(CH_2)_3)_2-$, $-CH_2-(O(CH_2)_3)_3-$, $-CH_2-(O(CH_2)_3)_4-$, $-CH_2-(O(CH_2)_3)_5-$, $-CH_2-(O(CH_2)_3)_6-$, $-CH_2-(O(CH_2)_3)_7-$, $-CH_2-(O(CH_2)_3)_8-$, $-CH_2-(O(CH_2)_3)_9-$, $-CH_2-(O(CH_2)_3)_{10}-$, $-(CH_2)_2-(O(CH_2)_3)_2-$, $-(CH_2)_2-(O(CH_2)_3)_3-$, $-(CH_2)_2-(O(CH_2)_3)_4-$, $-(CH_2)_2-(O(CH_2)_3)_5-$, $-(CH_2)_2-(O(CH_2)_3)_6-$, $-(CH_2)_2-(O(CH_2)_3)_7-$, $-(CH_2)_2-(O(CH_2)_3)_8-$, $-(CH_2)_2-(O(CH_2)_3)_9-$, $-(CH_2)_2-(0(CH_2)_3)_{IO}-$, $-(CH_2)_3-(O(CH_2)_3)_2-$, $-(CH_2)_3-(O(CH_2)_3)_3-$, $-(CH_2)_3-(O(CH_2)_3)_4-$, $-(CH_2)_3-(O(CH_2)_3)_5-$, $-(CH_2)_3-(O(CH_2)_3)_6-$, $-(CH_2)_3-(O(CH_2)_3)_7-$, $-(CH_2)_3-(O(CH_2)_3)_8-$, $-(CH_2)_3-(O(CH_2)_3)_9-$, $-(CH_2)_3-(0(CH_2)_3)_{IO}-$, $-CH_2-O-(CH_2)_2-O-(CH_2)_3-$, $-CH_2-(O(CH_2)_2)_2-(O(CH_2)_3)_2-$, $-CH_2-(O(CH_2)_2)_3-(O(CH_2)_3)_3-$, $-CH_2-(O(CH_2)_2)_4-(O(CH_2)_3)_4-$, $-CH_2-(O(CH_2)_2)_5-(O(CH_2)_3)_5-$, $-CH_2-(O(CH_2)_2)_6-(O(CH_2)_3)_6-$, $-(CH_2)_2-O-(CH_2)_2-O-(CH_2)_3-$, $-(CH_2)_2-(O(CH_2)_2)_2-(O(CH_2)_3)_2-$, $-(CH_2)_2-(O(CH_2)_2)_3-(O(CH_2)_3)_3-$, $-(CH_2)_2-(O(CH_2)_2)_4-(O(CH_2)_3)_4-$, $-(CH_2)_2-(O(CH_2)_2)_5-(O(CH_2)_3)_5-$, $-(CH_2)_2-(O(CH_2)_2)_6-(O(CH_2)_3)_6-$, $-(CH_2)_3-O-(CH_2)_2-O-(CH_2)_3-$, $-(CH_2)_3-(O(CH_2)_2)_2-(O(CH_2)_3)_2-$, $-(CH_2)_3-(O(CH_2)_2)_3-(O(CH_2)_3)_3-$, $-(CH_2)_3-(O(CH_2)_2)_4-(O(CH_2)_3)_4-$, $-(CH_2)_3-(O(CH_2)_2)_5-(O(CH_2)_3)_5-$, $-(CH_2)_3-(O(CH_2)_2)_6-(O(CH_2)_3)_6-$, $-CH_2-O-(CH_2)_3-O-(CH_2)_2-$, $-CH_2-(O(CH_2)_3)_2-(O(CH_2)_2)_2-$, $-CH_2-(O(CH_2)_3)_3-(O(CH_2)_2)_3-$, $-CH_2-(O(CH_2)_3)_4-(O(CH_2)_2)_4-$, $-CH_2-(O(CH_2)_3)_5-(O(CH_2)_2)_5-$, $-CH_2-(O(CH_2)_3)_6-(O(CH_2)_2)_6-$, $-(CH_2)_2-O-(CH_2)_3-O-(CH_2)_2-$, $-(CH_2)_2-(O(CH_2)_3)_2-(O(CH_2)_2)_2-$, $-(CH_2)_2-(O(CH_2)_3)_3-(O(CH_2)_2)_3-$, $-(CH_2)_2-(O(CH_2)_3)_4-(O(CH_2)_2)_4-$, $-(CH_2)_2-(O(CH_2)_3)_5-(O(CH_2)_2)_5-$, $-(CH_2)_2-(O(CH_2)_3)_6-(O(CH_2)_2)_6-$, $-(CH_2)_3-O-(CH_2)_3-O-(CH_2)_2-$, $-(CH_2)_3-(O(CH_2)_3)_2-(O(CH_2)_2)_2-$, $-(CH_2)_3-(O(CH_2)_3)_3-(O(CH_2)_2)_3-$, $-(CH_2)_3-(O(CH_2)_3)_4-(O(CH_2)_2)_4-$, $-(CH_2)_3-(O(CH_2)_3)_5-(O(CH_2)_2)_5-$, $-(CH_2)_3-(O(CH_2)_3)_6-(O(CH_2)_2)_6-$, $-CH_2-O-(CH_2)_2-O-CH_2-$, $-(CH_2)_2-O-(CH_2)_2-O-CH_2-$,

$-(CH_2)_2-(O(CH_2)_2)_2-O-(CH_2)_3-$, $-(CH_2)_2-(O(CH_2)_2)_3-O-(CH_2)_3-$, $-(CH_2)_2-(O(CH_2)_2)_4-O-(CH_2)_3-$, $-(CH_2)_5-(O(CH_2)_2)_2-O-(CH_2)_5-$, $-(CH_2)_5-(O(CH_2)_2)_2-O-(CH_2)_6-$, $-CH_2-C(O)-CH_2-$, $-CH_2-C(O)-(CH_2)_2-$, $-(CH_2)_1-C(O)-(CH_2)_3-$, $-(CH_2)_1-C(O)-(CH_2)_4-$, $-(CH_2)_1-C(O)-(CH_2)_5-$, $-(CH_2)_1-C(O)-(CH_2)_6-$, $-(CH_2)_1-C(O)-(CH_2)_7-$, $-(CH_2)_1-C(O)-(CH_2)_8-$, $-(CH_2)_1-C(O)-(CH_2)_9-$, $-(CH_2)_1-C(O)-(CH_2)_{10}-$, $-(CH_2)_2-C(O)-(CH_2)_1-$, $-(CH_2)_2-C(O)-(CH_2)_2-$, $-(CH_2)_2-C(O)-(CH_2)_3-$, $-(CH_2)_2-C(O)-(CH_2)_4-$, $-(CH_2)_2-C(O)-(CH_2)_5-$, $-(CH_2)_2-C(O)-(CH_2)_6-$, $-(CH_2)_2-C(O)-(CH_2)_7-$, $-(CH_2)_2-C(O)-(CH_2)_8-$, $-(CH_2)_2-C(O)-(CH_2)_9-$, $-(CH_2)_2-C(O)-(CH_2)_{10}-$, $-(CH_2)_2-C(O)-(CH_2)_{11}-$, $-(CH_2)_2-C(O)-(CH_2)_{12}-$, $-(CH_2)_3-C(O)-(CH_2)_1-$, $-(CH_2)_3-C(O)-(CH_2)_2-$, $-(CH_2)_3-C(O)-(CH_2)_3-$, $-(CH_2)_3-C(O)-(CH_2)_4-$, $-(CH_2)_3-C(O)-(CH_2)_5-$, $-(CH_2)_3-C(O)-(CH_2)_6-$, $-(CH_2)_3-C(O)-(CH_2)_7-$, $-(CH_2)_4-C(O)-(CH_2)_1-$, $-(CH_2)_4-C(O)-(CH_2)_2-$, $-(CH_2)_4-C(O)-(CH_2)_3-$, $-(CH_2)_4-C(O)-(CH_2)_4-$, $-(CH_2)_4-C(O)-(CH_2)_5-$, $-(CH_2)_4-C(O)-(CH_2)_6-$, $-(CH_2)_5-C(O)-(CH_2)_1-$, $-(CH_2)_5-C(O)-(CH_2)_2-$, $-(CH_2)_5-C(O)-(CH_2)_3-$, $-(CH_2)_5-C(O)-(CH_2)_4-$, $-(CH_2)_5-C(O)-(CH_2)_5-$, $-(CH_2)_6-C(O)-(CH_2)_1-$, $-(CH_2)_6-C(O)-(CH_2)_2-$, $-(CH_2)_6-C(O)-(CH_2)_3-$, $-(CH_2)_6-C(O)-(CH_2)_4-$, $-(CH_2)_7-C(O)-(CH_2)_1-$, $-(CH_2)_7-C(O)-(CH_2)_2-$, $-(CH_2)_7-C(O)-(CH_2)_3-$, $-(CH_2)_8-C(O)-(CH_2)_1-$, $-(CH_2)_8-C(O)-(CH_2)_2-$, $-CH_2-S-CH_2-$, $-CH_2-S-(CH_2)_2-$, $-(CH_2)_1-S-(CH_2)_3-$, $-(CH_2)_1-S-(CH_2)_4-$, $-(CH_2)_1-S-(CH_2)_5-$, $-(CH_2)_1-S-(CH_2)_6-$, $-(CH_2)_1-S-(CH_2)_7-$, $-(CH_2)_1-S-(CH_2)_8-$, $-(CH_2)_1-S-(CH_2)_9-$, $-(CH_2)_1-S-(CH_2)_{10}-$, $-(CH_2)_2-S-(CH_2)_1-$, $-(CH_2)_2-S-(CH_2)_2-$, $-(CH_2)_2-S-(CH_2)_3-$, $-(CH_2)_2-S-(CH_2)_4-$, $-(CH_2)_2-S-(CH_2)_5-$, $-(CH_2)_2-S-(CH_2)_6-$, $-(CH_2)_2-S-(CH_2)_7-$, $-(CH_2)_2-S-(CH_2)_8-$, $-(CH_2)_2-S-(CH_2)_9-$, $-(CH_2)_2-S-(CH_2)_{10}-$, $-(CH_2)_2-S-(CH_2)_{11}-$, $-(CH_2)_2-S-(CH_2)_{12}-$, $-(CH_2)_3-S-(CH_2)_1-$, $-(CH_2)_3-S-(CH_2)_2-$, $-(CH_2)_3-S-(CH_2)_3-$, $-(CH_2)_3-S-(CH_2)_4-$, $-(CH_2)_3-S-(CH_2)_5-$, $-(CH_2)_3-S-(CH_2)_6-$, $-(CH_2)_3-S-(CH_2)_7-$, $-(CH_2)_4-S-(CH_2)_1-$, $-(CH_2)_4-S-(CH_2)_2-$, $-(CH_2)_4-S-(CH_2)_3-$, $-(CH_2)_4-S-(CH_2)_4-$, $-(CH_2)_4-S-(CH_2)_5-$, $-(CH_2)_4-S-(CH_2)_6-$, $-(CH_2)_5-S-(CH_2)_1-$, $-(CH_2)_5-S-(CH_2)_2-$, $-(CH_2)_5-S-(CH_2)_3-$, $-(CH_2)_5-S-(CH_2)_4-$, $-(CH_2)_5-S-(CH_2)_5-$, $-(CH_2)_6-S-(CH_2)_1-$, $-(CH_2)_6-S-(CH_2)_2-$, $-(CH_2)_6-S-(CH_2)_3-$, $-(CH_2)_6-S-(CH_2)_4-$, $-(CH_2)_7-S-(CH_2)_1-$, $-(CH_2)_7-S-(CH_2)_2-$, $-(CH_2)_7-S-(CH_2)_3-$, $-(CH_2)_S-S-(CH_2)_1-$, $-(CH_2)_8-S-(CH_2)_2-$, $-CH_2-C(S)-CH_2-$, $-CH_2-C(S)-(CH_2)_2-$, $-(CH_2)_1-C(S)-(CH_2)_3-$, $-(CH_2)_1-C(S)-(CH_2)_4-$, $-(CH_2)_1-C(S)-(CH_2)_5-$, $-(CH_2)_1-C(S)-(CH_2)_6-$, $-(CH_2)_1-C(S)-(CH_2)_7-$, $-(CH_2)_1-C(S)-(CH_2)_8-$, $-(CH_2)_1-C(S)-(CH_2)_9-$, $-(CH_2)_1-C(S)-(CH_2)_{10}-$, $-(CH_2)_2-C(S)-(CH_2)_1-$, $-(CH_2)_2-C(S)-(CH_2)_2-$, $-(CH_2)_2-C(S)-(CH_2)_3-$, $-(CH_2)_2-C(S)-(CH_2)_4-$, $-(CH_2)_2-C(S)-(CH_2)_5-$, $-(CH_2)_2-C(S)-(CH_2)_6-$, $-(CH_2)_2-C(S)-(CH_2)_7-$, $-(CH_2)_2-C(S)-(CH_2)_8-$, $-(CH_2)_2-C(S)-(CH_2)_9-$, $-(CH_2)_2-C(S)-(CH_2)_{10}-$, $-(CH_2)_2-C(S)-(CH_2)_{11}-$, $-(CH_2)_2-C(S)-(CH_2)_{12}-$, $-(CH_2)_3-C(S)-(CH_2)_1-$, $-(CH_2)_3-C(S)-(CH_2)_2-$, $-(CH_2)_3-C(S)-(CH_2)_3-$, $-(CH_2)_3-C(S)-(CH_2)_4-$, $-(CH_2)_3-C(S)-(CH_2)_5-$, $-(CH_2)_3-C(S)-(CH_2)_6-$, $-(CH_2)_3-C(S)-(CH_2)_7-$, $-(CH_2)_4-C(S)-(CH_2)_1-$, $-(CH_2)_4-C(S)-(CH_2)_2-$, $-(CH_2)_4-C(S)-(CH_2)_3-$, $-(CH_2)_4-C(S)-(CH_2)_4-$, $-(CH_2)_4-C(S)-(CH_2)_5-$, $-(CH_2)_4-C(S)-(CH_2)_6-$, $-(CH_2)_5-C(S)-(CH_2)_1-$, $-(CH_2)_5-C(S)-(CH_2)_2-$, $-(CH_2)_5-C(S)-(CH_2)_3-$, $-(CH_2)_5-C(S)-(CH_2)_4-$, $-(CH_2)_5-C(S)-(CH_2)_5-$, $-(CH_2)_6-C(S)-(CH_2)_1-$, $-(CH_2)_6-C(S)-(CH_2)_2-$, $-(CH_2)_6-C(S)-(CH_2)_3-$, $-(CH_2)_6-C(S)-(CH_2)_4-$, $-(CH_2)_7-C(S)-(CH_2)_1-$, $-(CH_2)_7-C(S)-(CH_2)_2-$, $-(CH_2)_7-C(S)-(CH_2)_3-$, $-(CH_2)_8-C(S)-(CH_2)_1-$, $-(CH_2)_8-C(S)-(CH_2)_2-$, $-CH_2-C(O)O-CH_2-$, $-CH_2-C(O)O-(CH_2)_2-$, $-(CH_2)_1-C(O)O-(CH_2)_3-$, $-(CH_2)_1-C(O)O-(CH_2)_4-$, $-(CH_2)_1-C(O)O-(CH_2)_5-$, $-(CH_2)_1-C(O)O-(CH_2)_6-$, $-(CH_2)_1-C(O)O-(CH_2)_7-$, $-(CH_2)_1-C(O)O-(CH_2)_8-$, $-(CH_2)_1-C(O)O-(CH_2)_9-$, $-(CH_2)_1-C(O)O-(CH_2)_{10}-$, $-(CH_2)_2-C(O)O-(CH_2)_1-$, $-(CH_2)_2-C(O)O-(CH_2)_2-$, $-(CH_2)_2-C(O)O-(CH_2)_3-$, $-(CH_2)_2-C(O)O-(CH_2)_4-$, $-(CH_2)_2-C(O)O-(CH_2)_5-$, $-(CH_2)_2-C(O)O-(CH_2)_6-$, $-(CH_2)_2-C(O)O-(CH_2)_7-$, $-(CH_2)_2-C(O)O-(CH_2)_8-$, $-(CH_2)_2-C(O)O-(CH_2)_9-$, $-(CH_2)_2-C(O)O-(CH_2)_{10}-$, $-(CH_2)_2-C(O)O-(CH_2)_{11}-$, $-(CH_2)_2-C(O)O-(CH_2)_{12}-$, $-(CH_2)_3-C(O)O-(CH_2)_1-$, $-(CH_2)_3-C(O)O-(CH_2)_2-$, $-(CH_2)_3-C(O)O-(CH_2)_3-$, $-(CH_2)_3-C(O)O-(CH_2)_4-$, $-(CH_2)_3-C(O)O-(CH_2)_5-$, $-(CH_2)_3-C(O)O-(CH_2)_6-$, $-(CH_2)_3-C(O)O-(CH_2)_7-$, $-(CH_2)_4-C(O)O-(CH_2)_1-$, $-(CH_2)_4-C(O)O-(CH_2)_2-$, $-(CH_2)_4-C(O)O-(CH_2)_3-$, $-(CH_2)_4-C(O)O-(CH_2)_4-$, $-(CH_2)_4-C(O)O-(CH_2)_5-$, $-(CH_2)_4-C(O)O-(CH_2)_6-$, $-(CH_2)_5-C(O)O-(CH_2)_1-$, $-(CH_2)_5-C(O)O-(CH_2)_2-$, $-(CH_2)_5-C(O)O-(CH_2)_3-$, $-(CH_2)_5-C(O)O-(CH_2)_4-$, $-(CH_2)_5-C(O)O-(CH_2)_5-$, $-(CH_2)_6-C(O)O-(CH_2)_1-$, $-(CH_2)_6-C(O)O-(CH_2)_2-$, $-(CH_2)_6-C(O)O-(CH_2)_3-$, $-(CH_2)_6-C(O)O-(CH_2)_4-$, $-(CH_2)_7-C(O)O-(CH_2)_1-$, $-(CH_2)_7-C(O)O-(CH_2)_2-$, $-(CH_2)_7-C(O)O-(CH_2)_3-$, $-(CH_2)_8-C(O)O-(CH_2)_1-$, $-(CH_2)_8-C(O)O-(CH_2)_2-$, $-CH_2-O-C(O)-CH_2-$, $-CH_2-OC(O)-(CH_2)_2-$, $-(CH_2)_1-OC(O)-(CH_2)_3-$, $-(CH_2)_1-OC(O)-(CH_2)_4-$, $-(CH_2)_1-OC(O)-(CH_2)_5-$, $-(CH_2)_1-O-C(O)-(CH_2)_6-$, $-(CH_2)_1-OC(O)-(CH_2)_7-$, $-(CH_2)_1-OC(O)-(CH_2)_8-$, $-(CH_2)_1-OC(O)-(CH_2)_9-$, $-(CH_2)_1-OC(O)-(CH_2)_{10}-$, $-(CH_2)_2-OC(O)-(CH_2)_1-$, $-(CH_2)_2-OC(O)-(CH_2)_2-$, $-(CH_2)_2-OC(O)-(CH_2)_3-$, $-(CH_2)_2-OC(O)-(CH_2)_4-$, $-(CH_2)_2-O-C(O)-(CH_2)_5-$, $-(CH_2)_2-OC(O)-(CH_2)_6-$, $-(CH_2)_2-OC(O)-(CH_2)_7-$, $-(CH_2)_2-OC(O)-(CH_2)_8-$, $-(CH_2)_2-OC(O)-(CH_2)_9-$, $-(CH_2)_2-OC(O)-(CH_2)_{10}-$, $-(CH_2)_2-OC(O)-(CH_2)_{11}-$, $-(CH_2)_2-OC(O)-(CH_2)_{12}-$, $-(CH_2)_3-OC(O)-(CH_2)_1-$, $-(CH_2)_3-O-C(O)-(CH_2)_2-$, $-(CH_2)_3-OC(O)-(CH_2)_3-$, $-(CH_2)_3-OC(O)-(CH_2)_4-$, $-(CH_2)_3-OC(O)-(CH_2)_5-$, $-(CH_2)_3-OC(O)-(CH_2)_6-$, $-(CH_2)_3-OC(O)-(CH_2)_7-$, $-(CH_2)_4-OC(O)-(CH_2)_1-$, $-(CH_2)_4-OC(O)-(CH_2)_2-$, $-(CH_2)_4-OC(O)-(CH_2)_3-$, $-(CH_2)_4-O-C(O)-(CH_2)_4-$, $-(CH_2)_4-OC(O)-(CH_2)_5-$, $-(CH_2)_4-OC(O)-(CH_2)_6-$, $-(CH_2)_5-OC(O)-(CH_2)_1-$, $-(CH_2)_5-OC(O)-(CH_2)_2-$, $-(CH_2)_5-OC(O)-(CH_2)_3-$, $-(CH_2)_5-OC(O)-(CH_2)_4-$, $-(CH_2)_5-OC(O)-(CH_2)_5-$, $-(CH_2)_6-OC(O)-(CH_2)_1-$, $-(CH_2)_6-O-C(O)-(CH_2)_2-$, $-(CH_2)_6-OC(O)-(CH_2)_3-$, $-(CH_2)_6-OC(O)-(CH_2)_4-$, $-(CH_2)_7-OC(O)-(CH_2)_1-$, $-(CH_2)_7-OC(O)-(CH_2)_2-$, $-(CH_2)_7-OC(O)-(CH_2)_3-$, $-(CH_2)_8-OC(O)-(CH_2)_1-$, $-(CH_2)_8-OC(O)-(CH_2)_2-$, $-(CH_2)_1-N(R_{g11})-(CH_2)_1-$, $-(CH_2)_1-N(R_{g11})-(CH_2)_2-$, $-(CH_2)_1-N(R_{g11})-(CH_2)_3-$, $-(CH_2)_1-N(R_{g11})-(CH_2)_4-$, $-(CH_2)_1-N(R_{g11})-(CH_2)_5-$, $-(CH_2)_1-N(R_{g11})-(CH_2)_6-$, $-(CH_2)_1-N(R_{g11})-(CH_2)_7-$, $-(CH_2)_1-N(R_{g11})-(CH_2)_8-$, $-(CH_2)_1-N(R_{g11})-(CH_2)_9-$, $-(CH_2)_1-N(R_{g11})-(CH_2)_{10}-$, $-(CH_2)_2-N(R_{g11})-(CH_2)_1-$, $-(CH_2)_2-N(R_{g11})-(CH_2)_2-$, $-(CH_2)_2-N(R_{g11})-(CH_2)_3-$, $-(CH_2)_2-N(R_{g11})-(CH_2)_4-$, $-(CH_2)_2-N(R_{g11})-(CH_2)_5-$, $-(CH_2)_2-N(R_{g11})-(CH_2)_6-$, $-(CH_2)_2-N(R_{g11})-(CH_2)_7-$, $-(CH_2)_2-N(R_{g11})-(CH_2)_8-$, $-(CH_2)_2-N(R_{g11})-(CH_2)_9-$, $-(CH_2)_2-N(R_{g11})-(CH_2)_{10}-$, $-(CH_2)_2-N(R_{g11})-(CH_2)_{11}-$, $-(CH_2)_2-N(R_{g11})-(CH_2)_{12}-$, $-(CH_2)_3-N(R_{g11})-(CH_2)_1-$, $-(CH_2)_3-N(R_{g11})-(CH_2)_2-$, $-(CH_2)_3-N(R_{g11})-(CH_2)_3-$, $-(CH_2)_4-N(R_{g11})-(CH_2)_1-$, $-(CH_2)_4-N(R_{g11})-(CH_2)_2-$, $-(CH_2)_4-N(R_{g11})-(CH_2)_3-$, $-(CH_2)_4-N(R_{g11})-(CH_2)_4-$,

$-(CH_2)_5-N(R_{g11})-(CH_2)_1-$, $-(CH_2)_5-N(R_{g11})-(CH_2)_2-$, $-(CH_2)_5-N(R_{g11})-(CH_2)_3-$, $-(CH_2)_5-N(R_{g11})-(CH_2)_4-$, $-(CH_2)_5-N(R_{g11})-(CH_2)_5-$, $-(CH_2)_6-N(R_{g11})-(CH_2)_1-$, $-(CH_2)_6-N(R_{g11})-(CH_2)_2-$, $-(CH_2)_6-N(R_{g11})-(CH_2)_3-$, $-(CH_2)_7-N(R_{g11})-(CH_2)_1-$, $-(CH_2)_7-N(R_{g11})-(CH_2)_2-$, $-(CH_2)_7-N(R_{g11})-(CH_2)_3-$, $-(CH_2)_8-N(R_{g11})-(CH_2)_1-$, $-(CH_2)_8-NR_{g11})-(CH_2)_2-$, $-(CH_2)_8-N(R_{g11})-(CH_2)_3-$, $-CH(CH_3)-N(R_{g11})-(CH_2)_1-$, $-CH(CH_3)-N(R_{g11})-(CH_2)_2-$, $-CH(CH_3)-N(R_{g11})-(CH_2)_3-$, $-CH(CH_3)-N(R_{g11})-(CH_2)_4-$, $-CH(CH_3)-N(R_{g11})-(CH_2)_5-$, $-CH(CH_3)-N(R_{g11})-(CH_2)_6-$, $-CH(CH_3)-N(R_{g11})-(CH_2)_7-$, $-CH(CH_3)-N(R_{g11})-(CH_2)_8-$, $-CH(CH_3)-N(R_{g11})-(CH_2)_9-$, $-CH(CH_3)-N(R_{g11})-(CH_2)_{10}-$, $-CH_2C(O)NHCH_2-$, $-(CH_2)_{2C}(O)NH(CH_2)_2-$, $-(CH_2)_2C(O)NH(CH_2)_3-$, - $(CH_2)_2C(O)NH(CH_2)_4-$, $-(CH_2)_2C(O)NH(CH_2)_5-$, $-(CH_2)_3C(O)NH(CH_2)_3-$, $-(CH_2)_3C(O)NH(CH_2)_4-$, - $(CH_2)_4C(O)NH(CH_2)_4-$, $-(CH_2)_5C(O)NH(CH_2)_5-$, $-(CH_2)_6C(O)NH(CH_2)_7-$, $-(CH_2)_6C(O)NH(CH_2)_6-$, - $(CH_2)_7C(O)NH(CH_2)_7-$, $-(CH_2)_8C(O)NH(CH_2)_8$, $-(CH_2)_9C(O)NH(CH_2)_9-$, $-(CH_2)_{10}C(O)NH(CH_2)_{10}-$, - $(CH_2)_2C(O)NH(CH_2)_2-O-(CH_2)_2-$, $-CH_2NHC(O)CH_2-$, $-(CH_2)_2NHC(O)(CH_2)_2-$, $-(CH_2)_2NHC(O)(CH_2)_3-$, $-(CH_2)_2NHC(O)(CH_2)_4-$, $-(CH_2)_2NHC(O)(CH_2)_5-$, $-(CH_2)_3NHC(O)(CH_2)_3-$, - $(CH_2)_3NHC(O)(CH_2)_4-$, $-(CH_2)_4NHC(O)(CH_2)_4-$, $-(CH_2)_5NHC(O)(CH_2)_5-$, $-(CH_2)_6NHC(O)(CH_2)_7-$, - $(CH_2)_6NHC(O)(CH_2)_6-$, $-(CH_2)_7NHC(O)(CH_2)_7-$, $-(CH_2)_8NHC(O)(CH_2)_8$, $-(CH_2)_9NHC(O)(CH_2)_9-$, - $(CH_2)_{10}NHC(O)(CH_2)_{10}-$, $-(CH_2)_4NHC(O)(CH_2)_8-$, $-(CH_2)_2NHC(O)(CH_2)_2-O-(CH_2)_2-$, - $(CH_2)_4NHC(O)CH_2-$, $-CH_2$-piperidinylene-$CH_2-$, $-CH_2$-piperidinylene-$(CH_2)_2-$, $-CH_2$-piperidinylene-$(CH_2)_3-$, $-CH_2$-piperidinylene-$(CH_2)_4-$, $-CH_2$-piperidinylene-$(CH_2)_5-$, $-CH_2$-piperidinylene-$(CH_2)_6-$, - $CH_2$-piperidinylene-$(CH_2)_7-$, $-CH_2$-piperidinylene-$(CH_2)_8-$, $-(CH_2)_2$-piperidinylene-$(CH_2)_1-$, $-(CH_2)_2$-piperidinylene-$(CH_2)_2-$, $-(CH_2)_2$-piperidinylene-$(CH_2)_3-$, $-(CH_2)_2$-piperidinylene-$(CH_2)_4-$, $-(CH_2)_2$-piperidinylene-$(CH_2)_5-$, $-(CH_2)_2$-piperidinylene-$(CH_2)_6-$, $-(CH_2)_2$-piperidinylene-$(CH_2)_7-$, $-(CH_2)_2$-piperidinylene-$(CH_2)_8-$, $-(CH_2)_3$-piperidinylene-$CH_2-$, $-(CH_2)_3$-piperidinylene-$(CH_2)_2-$, $-(CH_2)_3$-piperidinylene-$(CH_2)_3-$, $-(CH_2)_3$-piperidinylene-$(CH_2)_4-$, $-(CH_2)_3$-piperidinylene-$(CH_2)_5-$, $-(CH_2)_3$-piperidinylene-$(CH_2)_6-$, $-(CH_2)_3$-piperidinylene-$(CH_2)_7-$, $-(CH_2)_3$-piperidinylene-$(CH_2)_8-$, $-(CH_2)_4$-piperidinylene-$CH_2-$, $-(CH_2)_4$-piperidinylene-$(CH_2)_2-$, $-(CH_2)_4$-piperidinylene-$(CH_2)_3-$, $-(CH_2)_4$-piperidinylene-$(CH_2)_4-$, $-(CH_2)_4$-piperidinylene-$(CH_2)_5-$, $-(CH_2)_4$-piperidinylene-$(CH_2)_6-$, $-(CH_2)_4$-piperidinylene-$(CH_2)_7-$, $-(CH_2)_4$-piperidinylene-$(CH_2)_8-$, $-(CH_2)_5$-piperidinylene-$(CH_2)_1-$, $-(CH_2)_5$-piperidinylene-$(CH_2)_2-$, $-(CH_2)_5$-piperidinylene-$(CH_2)_3-$, $-(CH_2)_5$-piperidinylene-$(CH_2)_4-$, $-(CH_2)_5$-piperidinylene-$(CH_2)_5-$, $-(CH_2)_5$-piperidinylene-$(CH_2)_6-$, $-(CH_2)_5$-piperidinylene-$(CH_2)_7-$, $-(CH_2)_5$-piperidinylene-$(CH_2)_8-$, $-(CH_2)_6$-piperidinylene-$(CH_2)_1-$, $-(CH_2)_6$-piperidinylene-$(CH_2)_2-$, $-(CH_2)_6$-piperidinylene-$(CH_2)_3-$, $-(CH_2)_6$-piperidinylene-$(CH_2)_4-$, $-(CH_2)_6$-piperidinylene-$(CH_2)_5-$, $-(CH_2)_6$-piperidinylene-$(CH_2)_6-$, $-(CH_2)_6$-piperidinylene-$(CH_2)_7-$, $-(CH_2)_6$-piperidinylene-$(CH_2)_8-$, $-(CH_2)_7$-piperidinylene-$(CH_2)_1-$, $-(CH_2)_7$-piperidinylene-$(CH_2)_2-$, $-(CH_2)_7$-piperidinylene-$(CH_2)_3-$, $-(CH_2)_7$-piperidinylene-$(CH_2)_4-$, $-(CH_2)_7$-piperidinylene-$(CH_2)_8-$, $-(CH_2)_8$-piperidinylene-$CH_2-$, $-(CH_2)_8$-piperidinylene-$(CH_2)_2-$, $-(CH_2)_8$-piperidinylene-$(CH_2)_3-$, $-(CH_2)_8$-piperidinylene-$(CH_2)_4-$, $-(CH_2)_8$-piperidinylene-$(CH_2)_5-$, $-(CH_2)_8$-piperidinylene-$(CH_2)_6-$, $-(CH_2)_8$-piperidinylene-$(CH_2)_7-$, $-(CH_2)_8$-piperidinylene-$(CH_2)_8-$, $-CH_2$-piperazinylene-$CH_2-$, $-CH_2$-piperazinylene-$(CH_2)_2-$, $-CH_2$-piperazinylene-$(CH_2)_3-$, $-CH_2$-piperazinylene-$(CH_2)_4-$, $-CH_2$-piperazinylene-$(CH_2)_5-$, $-CH_2$-piperazinylene-$(CH_2)_6-$, $-CH_2$-piperazinylene-$(CH_2)_7-$, $-CH_2$-piperazinylene-$(CH_2)_8-$, $-(CH_2)_2$-piperazinylene-$(CH_2)_1-$, $-(CH_2)_2$-piperazinylene-$(CH_2)_2-$, $-(CH_2)_2$-piperazinylene-$(CH_2)_3-$, $-(CH_2)_2$-piperazinylene-$(CH_2)_4-$, $-(CH_2)_2$-piperazinylene-$(CH_2)_5-$, $-(CH_2)_2$-piperazinylene-$(CH_2)_6-$, $-(CH_2)_2$-piperazinylene-$(CH_2)_7-$, $-(CH_2)_2$-piperazinylene-$(CH_2)_8-$, $-(CH_2)_3$-piperazinylene-$CH_2-$, $-(CH_2)_3$-piperazinylene-$(CH_2)_2-$, $-(CH_2)_3$-piperazinylene-$(CH_2)_3-$, $-(CH_2)_3$-piperazinylene-$(CH_2)_4-$, $-(CH_2)_3$-piperazinylene-$(CH_2)_5-$, $-(CH_2)_3$-piperazinylene-$(CH_2)_6-$, $-(CH_2)_3$-piperazinylene-$(CH_2)_7-$, $-(CH_2)_3$-piperazinylene-$(CH_2)_8-$, $-(CH_2)_4$-piperazinylene-$CH_2-$, $-(CH_2)_4$-piperazinylene-$(CH_2)_2-$, $-(CH_2)_4$-piperazinylene-$(CH_2)_3-$, $-(CH_2)_4$-piperazinylene-$(CH_2)_4-$, $-(CH_2)_4$-piperazinylene-$(CH_2)_5-$, $-(CH_2)_4$-piperazinylene-$(CH_2)_6-$, $-(CH_2)_4$-piperazinylene-$(CH_2)_7-$, $-(CH_2)_4$-piperazinylene-$(CH_2)_8-$, $-(CH_2)_5$-piperazinylene-$(CH_2)_1-$, $-(CH_2)_5$-piperazinylene-$(CH_2)_2-$, $-(CH_2)_5$-piperazinylene-$(CH_2)_3-$, $-(CH_2)_5$-piperazinylene-$(CH_2)_4-$, $-(CH_2)_5$-piperazinylene-$(CH_2)_5-$, $-(CH_2)_5$-piperazinylene-$(CH_2)_6-$, $-(CH_2)_5$-piperazinylene-$(CH_2)_7-$, $-(CH_2)_5$-piperazinylene-$(CH_2)_8-$, $-(CH_2)_6$-piperazinylene-$(CH_2)i-$, $-(CH_2)_6$-piperazinylene-$(CH_2)_2-$, $-(CH_2)_6$-piperazinylene-$(CH_2)_3-$, $-(CH_2)_6$-piperazinylene-$(CH_2)_4-$, $-(CH_2)_6$-piperazinylene-$(CH_2)_5-$, $-(CH_2)_6$-piperazinylene-$(CH_2)_6-$, $-(CH_2)_6$-piperazinylene-$(CH_2)_7-$, $-(CH_2)_6$-piperazinylene-$(CH_2)_8-$, $-(CH_2)_7$-piperazinylene-$(CH_2)_1-$, $-(CH_2)_7$-piperazinylene-$(CH_2)_2-$, $-(CH_2)_7$-piperazinylene-$(CH_2)_3-$, $-(CH_2)_7$-piperazinylene-$(CH_2)_4-$, $-(CH_2)_7$-piperazinylene-$(CH_2)_8-$, $-(CH_2)_8$-piperazinylene-$CH_2-$, $-(CH_2)_8$-piperazinylene-$(CH_2)_2-$, $-(CH_2)_8$-piperazinylene-$(CH_2)_3-$, $-(CH_2)_8$-piperazinylene-$(CH_2)_4-$, $-(CH_2)_8$-piperazinylene-$(CH_2)_5-$, $-(CH_2)_8$-piperazinylene-$(CH_2)_6-$, $-(CH_2)_8$-piperazinylene-$(CH_2)_7-$, $-(CH_2)_8$-piperazinylene-$(CH_2)_8-$, $-CH_2$-propylene-$CH_2-$, $-CH_2$-propylene-$(CH_2)_2-$, $-CH_2$-propylene-$(CH_2)_3-$, $-CH_2$-propylene-$(CH_2)_4-$, $-CH_2$-propylene-$(CH_2)_5-$, $-CH_2$-propylene-$(CH_2)_6-$, $-CH_2$-propylene-$(CH_2)_7-$, $-CH_2$-propylene-$(CH_2)_8-$, $-(CH_2)_2$-propylene-$(CH_2)_1-$, $-(CH_2)_2$-propylene-$(CH_2)_2-$, $-(CH_2)_2$-propylene-$(CH_2)_3-$, $-(CH_2)_2$-propylene-$(CH_2)_4-$, $-(CH_2)_2$-propylene-$(CH_2)_5-$, $-(CH_2)_2$-propylene-$(CH_2)_6-$, $-(CH_2)_2$-propylene-$(CH_2)_7-$, $-(CH_2)_2$-propylene-$(CH_2)_8-$, $-(CH_2)_3$-propylene-$CH_2-$, $-(CH_2)_3$-propylene-$(CH_2)_2-$, $-(CH_2)_3$-propylene-$(CH_2)_3-$, $-(CH_2)_3$-propylene-$(CH_2)_4-$, - $(CH_2)_3$-propylene-$(CH_2)_5-$, $-(CH_2)_3$-propylene-$(CH_2)_6-$, $-(CH_2)_3$-propylene-$(CH_2)_7-$, $-(CH_2)_3$-propylene-$(CH_2)_8-$, $-(CH_2)_4$-propylene-$CH_2-$, $-(CH_2)_4$-propylene-$(CH_2)_2-$, $-(CH_2)_4$-propylene-$(CH_2)_3-$, $-(CH_2)_4$-propylene-$(CH_2)_4-$, $-(CH_2)_4$-propylene-$(CH_2)_5-$, $-(CH_2)_4$-propylene-$(CH_2)_6-$, $-(CH_2)_4$-propylene-$(CH_2)_7-$, $-(CH_2)_4$-propylene-$(CH_2)_8-$, $-(CH_2)_5$-propylene-$(CH_2)_1-$, $-(CH_2)_5$-propylene-$(CH_2)_2-$, $-(CH_2)_5$-propylene-$(CH_2)_3-$, $-(CH_2)_5$-propylene-$(CH_2)_4-$, $-(CH_2)_5$-propylene-$(CH_2)_5-$, $-(CH_2)_5$-propylene-$(CH_2)_6-$, $-(CH_2)_5$-propylene-$(CH_2)_7-$, $-(CH_2)_5$-propylene-$(CH_2)_8-$, $-(CH_2)_6$-propylene-$(CH_2)_1-$, $-(CH_2)_6$-propylene-$(CH_2)_2-$, $-(CH_2)_6$-propylene-$(CH_2)_3-$, $-(CH_2)_6$-propylene-$(CH_2)_4-$, $-(CH_2)_6$-propylene-$(CH_2)_5-$, $-(CH_2)_6$-propyle-

ne-$(CH_2)_6$-, -$(CH_2)_6$-propylene-$(CH_2)_7$-, -$(CH_2)_6$-propylene-$(CH_2)_8$-, -$(CH_2)_7$-propylene-$(CH_2)_1$-, -$(CH_2)_7$-propylene-$(CH_2)_2$-, -$(CH_2)_7$-propylene-$(CH_2)_3$-, -$(CH_2)_7$-propylene-$(CH_2)_4$-, -$(CH_2)_7$-propylene-$(CH_2)_8$-, -$(CH_2)_8$-propylene-$CH_2$-, -$(CH_2)_8$-propylene-$(CH_2)_2$-, -$(CH_2)_8$-propylene-$(CH_2)_3$-, -$(CH_2)_8$-propylene-$(CH_2)_4$-, -$(CH_2)$s-propylene-$(CH_2)_5$-, -$(CH_2)_8$-propylene-$(CH_2)_6$-, -$(CH_2)_8$-propylene-$(CH_2)_7$-, -$(CH_2)_8$-propylene-$(CH_2)_8$-, -$CH_2$-diazacycloheptanylene-$CH_2$-, -$CH_2$-diazacycloheptanylene-$(CH_2)_2$-, -$CH_2$-diazacycloheptanylene-$(CH_2)_3$-, -$CH_2$-diazacycloheptanylene-$(CH_2)_4$-, -$CH_2$-diazacycloheptanylene-$(CH_2)_5$-, -$CH_2$-diazacycloheptanylene-$(CH_2)_6$-, -$CH_2$-diazacycloheptanylene-$(CH_2)_7$-, -$CH_2$-diazacycloheptanylene-$(CH_2)_8$-, -$(CH_2)_2$-diazacycloheptanylene-$(CH_2)_1$-, -$(CH_2)_2$-diazacycloheptanylene-$(CH_2)_2$-, -$(CH_2)_2$-diazacycloheptanylene-$(CH_2)_3$-, -$(CH_2)_2$-diazacycloheptanylene-$(CH_2)_4$-, -$(CH_2)_2$-diazacycloheptanylene-$(CH_2)_5$-, -$(CH_2)_2$-diazacycloheptanylene-$(CH_2)_6$-, -$(CH_2)_2$-diazacycloheptanylene-$(CH_2)_7$-, -$(CH_2)_2$-diazacycloheptanylene-$(CH_2)_8$-, -$(CH_2)_3$-diazacycloheptanylene-$CH_2$-, -$(CH_2)_3$-diazacycloheptanylene-$(CH_2)_2$-, -$(CH_2)_3$-diazacycloheptanylene-$(CH_2)_3$-, -$(CH_2)_3$-diazacycloheptanylene-$(CH_2)_4$-, -$(CH_2)_3$-diazacycloheptanylene-$(CH_2)_5$-, -$(CH_2)_3$-diazacycloheptanylene-$(CH_2)_6$-, -$(CH_2)_3$-diazacycloheptanylene-$(CH_2)_7$-, -$(CH_2)_3$-diazacycloheptanylene-$(CH_2)_8$-, -$(CH_2)_4$-diazacycloheptanylene-$CH_2$-, -$(CH_2)_4$-diazacycloheptanylene-$(CH_2)_2$-, -$(CH_2)_4$-diazacycloheptanylene-$(CH_2)_3$-, -$(CH_2)_4$-diazacycloheptanylene-$(CH_2)_4$-, -$(CH_2)_4$-diazacycloheptanylene-$(CH_2)_5$-, -$(CH_2)_4$-diazacycloheptanylene-$(CH_2)_6$-, -$(CH_2)_4$-diazacycloheptanylene-$(CH_2)_7$-, -$(CH_2)_4$-diazacycloheptanylene-$(CH_2)_8$-, -$(CH_2)_5$-diazacycloheptanylene-$(CH_2)_1$-, -$(CH_2)_5$-diazacycloheptanylene-$(CH_2)_2$-, -$(CH_2)_5$-diazacycloheptanylene-$(CH_2)_3$-, -$(CH_2)_5$-diazacycloheptanylene-$(CH_2)_4$-, -$(CH_2)_5$-diazacycloheptanylene-$(CH_2)_5$-, -$(CH_2)_5$-diazacycloheptanylene-$(CH_2)_6$-, -$(CH_2)_5$-diazacycloheptanylene-$(CH_2)_7$-, -$(CH_2)_5$-diazacycloheptanylene-$(CH_2)_8$-, -$(CH_2)_6$-diazacycloheptanylene-$(CH_2)_1$-, -$(CH_2)_6$-diazacycloheptanylene-$(CH_2)_2$-, -$(CH_2)_6$-diazacycloheptanylene-$(CH_2)_3$-, -$(CH_2)_6$-diazacycloheptanylene-$(CH_2)_4$-, -$(CH_2)_6$-diazacycloheptanylene-$(CH_2)_5$-, -$(CH_2)_6$-diazacycloheptanylene-$(CH_2)_6$-, -$(CH_2)_6$-diazacycloheptanylene-$(CH_2)_7$-, -$(CH_2)_6$-diazacycloheptanylene-$(CH_2)_8$-, -$(CH_2)_7$-diazacycloheptanylene-$(CH_2)_1$-, -$(CH_2)_7$-diazacycloheptanylene-$(CH_2)_2$-, -$(CH_2)_7$-diazacycloheptanylene-$(CH_2)_3$-, -$(CH_2)_7$-diazacycloheptanylene-$(CH_2)_4$-, -$(CH_2)_7$-diazacycloheptanylene-$(CH_2)_8$-, -$(CH_2)_8$-diazacycloheptanylene-$CH_2$-, -$(CH_2)_8$-diazacycloheptanylene-$(CH_2)_2$-, -$(CH_2)_8$-diazacycloheptanylene-$(CH_2)_3$-, -$(CH_2)_8$-diazacycloheptanylene-$(CH_2)_4$-, -$(CH_2)_8$-chazacycloheptanylene-$(CH_2)_5$-, -$(CH_2)_8$-diazacycloheptanylene-$(CH_2)_6$-, -$(CH_2)_8$-chazacycloheptanylene-$(CH_2)_7$-, -$(CH_2)_8$-diazacycloheptanylene-$(CH_2)_8$-, -$CH_2$-diazabicyclo[2.2.1]heptanylene-$CH_2$-, -$CH_2$-diazabicyclo[2.2.1]heptanylene-$(CH_2)_2$-, -$CH_2$-diazabicyclo[2.2.1]heptanylene-$(CH_2)_3$-, -$CH_2$-diazabicyclo[2.2.1]heptanylene-$(CH_2)_4$-, -$CH_2$-diazabicyclo[2.2.1]heptanylene-$(CH_2)_5$-, -$CH_2$-diazabicyclo[2.2.1]heptanylene-$(CH_2)_6$-, -$CH_2$-diazabicyclo[2.2.1]heptanylene-$(CH_2)_7$-, -$CH_2$-diazabicyclo[2.2.1]heptanylene-$(CH_2)_8$-, -$(CH_2)_2$-diazabicyclo[2.2.1]heptanylene-$(CH_2)_1$-, -$(CH_2)_2$-diazabicyclo[2.2.1]heptanylene-$(CH_2)_2$-, -$(CH_2)_2$-diazabicyclo[2.2.1]heptanylene-$(CH_2)_3$-, -$(CH_2)_2$-diazabicyclo[2.2.1]heptanylene-$(CH_2)_4$-, -$(CH_2)_2$-diazabicyclo[2.2.1 ]heptanylene-$(CH_2)_5$-, -$(CH_2)_2$-diazabicyclo[2.2.1]heptanylene-$(CH_2)_6$-, -$(CH_2)_2$-diazabicyclo[2.2.1]heptanylene-$(CH_2)_7$-, -$(CH_2)_2$-diazabicyclo[2.2.1]heptanylene-$(CH_2)_8$-, -$(CH_2)_3$-diazabicyclo[2.2.1 ]heptanylene-$CH_2$-, -$(CH_2)_3$-diazabicyclo[2.2.1]heptanylene-$(CH_2)_2$-, -$(CH_2)_3$-diazabicyclo[2.2.1]heptanylene-$(CH_2)_3$-, -$(CH_2)_3$-diazabicyclo[2.2.1]heptanylene-$(CH_2)_4$-, -$(CH_2)_3$-diazabicyclo[2.2.1]heptanylene-$(CH_2)_5$-, -$(CH_2)_3$-diazabicyclo[2.2.1]heptanylene-$(CH_2)_6$-, -$(CH_2)_3$-diazabicyclo[2.2.1]heptanylene-$(CH_2)_7$-, -$(CH_2)_3$-diazabicyclo[2.2.1]heptanylene-$(CH_2)_8$-, -$(CH_2)_4$-diazabicyclo[2.2.1]heptanylene-$CH_2$-, -$(CH_2)_4$-diazabicyclo[2.2.1]heptanylene-$(CH_2)_2$-, -$(CH_2)_4$-diazabicyclo[2.2.1]heptanylene-$(CH_2)_3$-, -$(CH_2)_4$-diazabicyclo[2.2.1]heptanylene-$(CH_2)_4$-, -$(CH_2)_4$-diazabicyclo[2.2.1]heptanylene-$(CH_2)_5$-, -$(CH_2)_4$-diazabicyclo[2.2.1]heptanylene-$(CH_2)_6$-, -$(CH_2)_4$-diazabicyclo[2.2.1]heptanylene-$(CH_2)_7$-, -$(CH_2)_4$-diazabicyclo[2.2.1]heptanylene-$(CH_2)_8$-, -$(CH_2)_5$-diazabicyclo[2.2.1]heptanylene-$(CH_2)_1$-, -$(CH_2)_5$-diazabicyclo[2.2.1]heptanylene-$(CH_2)_2$-, -$(CH_2)_5$-diazabicyclo[2.2.1]heptanylene-$(CH_2)_3$-, -$(CH_2)_5$-diazabicyclo[2.2.1]heptanylene-$(CH_2)_4$-, -$(CH_2)_5$-diazabicyclo[2.2.1]heptanylene-$(CH_2)_5$-, -$(CH_2)_5$-diazabicyclo[2.2.1]heptanylene-$(CH_2)_6$-, -$(CH_2)_5$-diazabicyclo[2.2.1]heptanylene-$(CH_2)_7$-, -$(CH_2)_5$-diazabicyclo[2.2.1]heptanylene-$(CH_2)_8$-, -$(CH_2)_6$-diazabicyclo[2.2.1]heptanylene-$(CH_2)_1$-, -$(CH_2)_6$-diazabicyclo[2.2.1]heptanylene-$(CH_2)_2$-, -$(CH_2)_6$-diazabicyclo[2.2.1]heptanylene-$(CH_2)_3$-, -$(CH_2)_6$-diazabicyclo[2.2.1]heptanylene-$(CH_2)_4$-, -$(CH_2)_6$-diazabicyclo[2.2.1]heptanylene-$(CH_2)_5$-, -$(CH_2)_6$-diazabicyclo[2.2.1]heptanylene-$(CH_2)_6$-, -$(CH_2)_6$-diazabicyclo[2.2.1]heptanylene-$(CH_2)_7$-, -$(CH_2)_6$-diazabicyclo[2.2.1]heptanylene-$(CH_2)_8$-, -$(CH_2)_7$-diazabicyclo[2.2.1]heptanylene-$(CH_2)_1$-, -$(CH_2)_7$-diazabicyclo[2.2.1]heptanylene-$(CH_2)_2$-, -$(CH_2)_7$-diazabicyclo[2.2.1]heptanylene-$(CH_2)_3$-, -$(CH_2)_7$-diazabicyclo[2.2.1]heptanylene-$(CH_2)_4$-, -$(CH_2)_7$-diazabicyclo[2.2.1]heptanylene-$(CH_2)_8$-, -$(CH_2)_8$-diazabicyclo[2.2.1]heptanylene-$CH_2$-, -$(CH_2)_8$-diazabicyclo[2.2.1]heptanylene-$(CH_2)_2$-, -$(CH_2)_8$-diazabicyclo[2.2.1]heptanylene-$(CH_2)_3$-, -$(CH_2)_8$-diazabicyclo[2.2.1]heptanylene-$(CH_2)_4$-, -$(CH_2)_8$-diazabicyclo[2.2.1]heptanylene-$(CH_2)_5$-, -$(CH_2)_8$-diazabicyclo[2.2.1]heptanylene-$(CH_2)_6$-, -$(CH_2)_8$-diazabicyclo[2.2.1]heptanylene-$(CH_2)_7$-, -$(CH_2)_8$-diazabicyclo[2.2.1]heptanylene-$(CH_2)_8$-, -$CH_2$-diazabicyclo[3.1.1]heptanylene-$CH_2$-, -$CH_2$-diazabicyclo[3.1.1]heptanylene-$(CH_2)_2$-, -$CH_2$-diazabicyclo[3.1.1]heptanylene-$(CH_2)_3$-, -$CH_2$-diazabicyclo[3.1.1]heptanylene-$(CH_2)_4$-, -$CH_2$-diazabicyclo[3.1.1]heptanylene-$(CH_2)_5$-, -$CH_2$-diazabicyclo[3.1.1]heptanylene-$(CH_2)_6$-, -$CH_2$-diazabicyclo[3.1.1]heptanylene-$(CH_2)_7$-, -$CH_2$-diazabicyclo[3.1.1]heptanylene-$(CH_2)_8$-, -$(CH_2)_2$-diazabicyclo[3.1.1]heptanylene-$(CH_2)_1$-, -$(CH_2)_2$-diazabicyclo[3.1.1]heptanylene-$(CH_2)_2$-, -$(CH_2)_2$-diazabicyclo[3.1.1]heptanylene-$(CH_2)_3$-, -$(CH_2)_2$-diazabicyclo[3.1.1]heptanylene-$(CH_2)_4$-, -$(CH_2)_2$-diazabicyclo[3.1.1]heptanylene-$(CH_2)_5$-, -$(CH_2)_2$-diazabicyclo[3.1.1]heptanylene-$(CH_2)_6$-, -$(CH_2)_2$-diazabicyclo[3.1.1]heptanylene-$(CH_2)_7$-, -$(CH_2)_2$-diazabicyclo[3.1.1]heptanylene-$(CH_2)_8$-, -$(CH_2)_3$-diazabicyclo[3.1.1]heptanylene-$CH_2$-, -$(CH_2)_3$-

diazabicyclo[3.1.1]heptanylene-$(CH_2)_2$-, -$(CH_2)_3$-diazabicyclo[3.1.1]heptanylene-$(CH_2)_3$-, -$(CH_2)_3$-diazabicyclo[3.1.1]heptanylene-$(CH_2)_4$-, -$(CH_2)_3$-diazabicyclo[3.1.1]heptanylene-$(CH_2)_5$-, -$(CH_2)_3$-diazabicyclo[3.1.1]heptanylene-$(CH_2)_6$-, -$(CH_2)_3$-diazabicyclo[3.1.1]heptanylene-$(CH_2)_7$-, -$(CH_2)_3$-diazabicyclo[3.1.1]heptanylene-$(CH_2)_8$-, -$(CH_2)_4$-diazabicyclo[3.1.1]heptanylene-$CH_2$-, -$(CH_2)_4$-diazabicyclo[3.1.1]heptanylene-$(CH_2)_2$-, -$(CH_2)_4$-diazabicyclo[3.1.1]heptanylene-$(CH_2)_3$-, -$(CH_2)_4$-diazabicyclo[3.1.1]heptanylene-$(CH_2)_4$-, -$(CH_2)_4$-diazabicyclo[3.1.1]heptanylene-$(CH_2)_5$-, -$(CH_2)_4$-diazabicyclo[3.1.1]heptanylene-$(CH_2)_6$-, -$(CH_2)_4$-diazabicyclo[3.1.1]heptanylene-$(CH_2)_7$-, -$(CH_2)_4$-diazabicyclo[3.1.1]heptanylene-$(CH_2)_8$-, -$(CH_2)_5$-diazabicyclo[3.1.1]heptanylene-$(CH_2)_1$-, -$(CH_2)_5$-diazabicyclo[3.1.1]heptanylene-$(CH_2)_2$-, -$(CH_2)_5$-diazabicyclo[3.1.1]heptanylene-$(CH_2)_3$-, -$(CH_2)_5$-diazabicyclo[3.1.1]heptanylene-$(CH_2)_4$-, -$(CH_2)_5$-diazabicyclo[3.1.1]heptanylene-$(CH_2)_5$-, -$(CH_2)_5$-diazabicyclo[3.1.1]heptanylene-$(CH_2)_6$-, -$(CH_2)_5$-diazabicyclo[3.1.1]heptanylene-$(CH_2)_7$-, -$(CH_2)_5$-diazabicyclo[3.1.1]heptanylene-$(CH_2)_8$-, -$(CH_2)_6$-diazabicyclo[3.1.1]heptanylene-$(CH_2)_1$-, -$(CH_2)_6$-diazabicyclo[3.1.1]heptanylene-$(CH_2)_2$-, -$(CH_2)_6$-diazabicyclo[3.1.1]heptanylene-$(CH_2)_3$-, -$(CH_2)_6$-diazabicyclo[3.1.1]heptanylene-$(CH_2)_4$-, -$(CH_2)_6$-diazabicyclo[3.1.1]heptanylene-$(CH_2)_5$-, -$(CH_2)_6$-diazabicyclo[3.1.1]heptanylene-$(CH_2)_6$-, -$(CH_2)_6$-diazabicyclo[3.1.1]heptanylene-$(CH_2)_7$-, -$(CH_2)_6$-diazabicyclo[3.1.1]heptanylene-$(CH_2)_8$-, -$(CH_2)_7$-diazabicyclo[3.1.1]heptanylene-$(CH_2)_1$-, -$(CH_2)_7$-diazabicyclo[3.1.1]heptanylene-$(CH_2)_2$-, -$(CH_2)_7$-diazabicyclo[3.1.1]heptanylene-$(CH_2)_3$-, -$(CH_2)_7$-diazabicyclo[3.1.1]heptanylene-$(CH_2)_4$-, -$(CH_2)_7$-diazabicyclo[3.1.1]heptanylene-$(CH_2)_8$-, -$(CH_2)_8$-diazabicyclo[3.1.1]heptanylene-$CH_2$-, -$(CH_2)_8$-diazabicyclo[3.1.1]heptanylene-$(CH_2)_2$-, -$(CH_2)_8$-diazabicyclo[3.1.1]heptanylene-$(CH_2)_3$-, -$(CH_2)_8$-diazabicyclo[3.1.1]heptanylene-$(CH_2)_4$-, -$(CH_2)_8$-diazabicyclo[3.1.1]heptanylene-$(CH_2)_5$-, -$(CH_2)_8$-diazabicyclo[3.1.1]heptanylene-$(CH_2)_6$-, -$(CH_2)_8$-diazabicyclo[3.1.1]heptanylene-$(CH_2)_7$-, -$(CH_2)_8$-diazabicyclo[3.1.1]heptanylene-$(CH_2)_8$-, -$CH_2$-diazabicyclo[3.2.1]octylene-$CH_2$-, -$CH_2$-diazabicyclo[3.2.1]octylene-$(CH_2)_2$-, -$CH_2$-diazabicyclo[3.2.1]octylene-$(CH_2)_3$-, -$CH_2$-diazabicyclo[3.2.1]octylene-$(CH_2)_4$-, -$CH_2$-diazabicyclo[3.2.1]octylene-$(CH_2)_5$-, -$CH_2$-diazabicyclo[3.2.1]octylene-$(CH_2)_6$-, -$CH_2$-diazabicyclo[3.2.1]octylene-$(CH_2)_7$-, -$CH_2$-diazabicyclo[3.2.1]octylene-$(CH_2)_8$-, -$(CH_2)_2$-diazabicyclo[3.2.1]octylene-$(CH_2)_1$-, -$(CH_2)_2$-diazabicyclo[3.2.1]octylene-$(CH_2)_2$-, -$(CH_2)_2$-diazabicyclo[3.2.1]octylene-$(CH_2)_3$-, -$(CH_2)_2$-diazabicyclo[3.2.1]octylene-$(CH_2)_4$-, -$(CH_2)_2$-diazabicyclo[3.2.1]octylene-$(CH_2)_5$-, -$(CH_2)_2$-diazabicyclo[3.2.1]octylene-$(CH_2)_6$-, -$(CH_2)_2$-diazabicyclo[3.2.1]octylene-$(CH_2)_7$-, -$(CH_2)_2$-diazabicyclo[3.2. 1]octylene-$(CH_2)_8$-, -$(CH_2)_3$-diazabicyclo[3.2.1]octylene-$CH_2$-, -$(CH_2)_3$-diazabicyclo[3.2.1]octylene-$(CH_2)_2$-, -$(CH_2)_3$-diazabicyclo[3.2.1]octylene-$(CH_2)_3$-, -$(CH_2)_3$-diazabicyclo[3.2.1]octylene-$(CH_2)_4$-, -$(CH_2)_3$-diazabicyclo[3.2.1]octylene-$(CH_2)_5$-, -$(CH_2)_3$-diazabicyclo[3.2.1]octylene-$(CH_2)_6$-, -$(CH_2)_3$-diazabicyclo[3.2.1]octylene-$(CH_2)_7$-, -$(CH_2)_3$-diazabicyclo[3.2. 1]octylene-$(CH_2)_8$-, -$(CH_2)_4$-diazabicyclo[3.2.1]octylene-$CH_2$-, -$(CH_2)_4$-diazabicyclo[3.2.1]octylene-$(CH_2)_2$-, -$(CH_2)_4$-diazabicyclo[3.2.1]octylene-$(CH_2)_3$-, -$(CH_2)_4$-diazabicyclo[3.2.1]octylene-$(CH_2)_4$-, -$(CH_2)_4$-diazabicyclo[3.2.1]octylene-$(CH_2)_5$-, -$(CH_2)_4$-diazabicyclo[3.2.1]octylene-$(CH_2)_6$-, -$(CH_2)_4$-diazabicyclo[3.2.1]octylene-$(CH_2)_7$-, -$(CH_2)_4$-diazabicyclo[3.2. 1]octylene-$(CH_2)_8$-, -$(CH_2)_5$-diazabicyclo[3.2.1]octylene-$(CH_2)_1$-, -$(CH_2)_5$-diazabicyclo[3.2.1]octylene-$(CH_2)_2$-, -$(CH_2)_5$-diazabicyclo[3.2.1]octylene-$(CH_2)_3$-, -$(CH_2)_5$-diazabicyclo[3.2.1]octylene-$(CH_2)_4$-, -$(CH_2)_5$-diazabicyclo[3.2.1]octylene-$(CH_2)_5$-, -$(CH_2)_5$-diazabicyclo[3.2.1]octylene-$(CH_2)_6$-, -$(CH_2)_5$-diazabicyclo[3.2.1]octylene-$(CH_2)_7$-, -$(CH_2)_5$-diazabicyclo[3.2.1]octylene-$(CH_2)_8$-, -$(CH_2)_6$-diazabicyclo[3.2.1]octylene-$(CH_2)_1$-, -$(CH_2)_6$-diazabicyclo[3.2.1]octylene-$(CH_2)_2$-, -$(CH_2)_6$-diazabicyclo[3.2.1]octylene-$(CH_2)_3$-, -$(CH_2)_6$-diazabicyclo[3.2.1]octylene-$(CH_2)_4$-, -$(CH_2)_6$-diazabicyclo[3.2.1]octylene-$(CH_2)_5$-, -$(CH_2)_6$-diazabicyclo[3.2.1]octylene-$(CH_2)_6$-, -$(CH_2)_6$-diazabicyclo[3.2.1]octylene-$(CH_2)_7$-, -$(CH_2)_6$-diazabicyclo[3.2.1]octylene-$(CH_2)_8$-, -$(CH_2)_7$-diazabicyclo[3.2.1]octylene-$(CH_2)_1$-, -$(CH_2)_7$-diazabicyclo[3.2.1]octylene-$(CH_2)_2$-, -$(CH_2)_7$-diazabicyclo[3.2.1]octylene-$(CH_2)_3$-, -$(CH_2)_7$-diazabicyclo[3.2.1]octylene-$(CH_2)_4$-, -$(CH_2)_7$-diazabicyclo[3.2.1]octylene-$(CH_2)_8$-, -$(CH_2)_8$-diazabicyclo[3.2. 1]octylene-$CH_2$-, -$(CH_2)_8$-diazabicyclo[3.2.1]octylene-$(CH_2)_2$-, -$(CH_2)_8$-diazabicyclo[3.2.1]octylene-$(CH_2)_3$-, -$(CH_2)_8$-diazabicyclo[3.2.1]octylene-$(CH_2)_4$-, -$(CH_2)_8$-diazabicyclo[3.2.1]octylene-$(CH_2)_5$-, -$(CH_2)_8$-diazabicyclo[3.2.1]octylene-$(CH_2)_6$-, -$(CH_2)_8$-diazabicyclo[3.2.1]octylene-$(CH_2)_7$-, -$(CH_2)_8$-diazabicyclo[3.2.1]octylene-$(CH_2)_8$-, -$CH_2$-diazabicyclo[2.2.2]octylene-$CH_2$-, -$CH_2$-diazabicyclo[2.2.2]octylene-$(CH_2)_2$-, -$CH_2$-diazabicyclo[2.2.2]octylene-$(CH_2)_3$-, -$CH_2$-diazabicyclo[2.2.2]octylene-$(CH_2)_4$-, -$CH_2$-diazabicyclo[2.2.2]octylene-$(CH_2)_5$-, -$CH_2$-diazabicyclo[2.2.2]octylene-$(CH_2)_6$-, -$CH_2$-diazabicyclo[2.2.2]octylene-$(CH_2)_7$-, -$CH_2$-diazabicyclo[2.2.2]octylene-$(CH_2)_8$-, -$(CH_2)_2$-diazabicyclo[2.2.2]octylene-$(CH_2)_1$-, -$(CH_2)_2$-diazabicyclo[2.2.2]octylene-$(CH_2)_2$-, -$(CH_2)_2$-diazabicyclo[2.2.2]octylene-$(CH_2)_3$-, -$(CH_2)_2$-diazabicyclo[2.2.2]octylene-$(CH_2)_4$-, -$(CH_2)_2$-diazabicyclo[2.2.2]octylene-$(CH_2)_5$-, -$(CH_2)_2$-diazabicyclo[2.2.2]octylene-$(CH_2)_6$-, -$(CH_2)_2$-diazabicyclo[2.2.2]octylene-$(CH_2)_7$-, -$(CH_2)_2$-diazabicyclo[2.2.2]octylene-$(CH_2)_8$-, -$(CH_2)_3$-diazabicyclo[2.2.2]octylene-$CH_2$-, -$(CH_2)_3$-diazabicyclo[2.2.2]octylene-$(CH_2)_2$-, -$(CH_2)_3$-diazabicyclo[2.2.2]octylene-$(CH_2)_3$-, -$(CH_2)_3$-diazabicyclo[2.2.2]octylene-$(CH_2)_4$-, -$(CH_2)_3$-diazabicyclo[2.2.2]octylene-$(CH_2)_5$-, -$(CH_2)_3$-diazabicyclo[2.2.2]octylene-$(CH_2)_6$-, -$(CH_2)_3$-diazabicyclo[2.2.2]octylene-$(CH_2)_7$-, -$(CH_2)_3$-diazabicyclo[2.2.2]octylene-$(CH_2)_8$-, -$(CH_2)_4$-diazabicyclo[2.2.2]octylene-$CH_2$-, -$(CH_2)_4$-diazabicyclo[2.2.2]octylene-$(CH_2)_2$-, -$(CH_2)_4$-diazabicyclo[2.2.2]octylene-$(CH_2)_3$-, -$(CH_2)_4$-diazabicyclo[2.2.2]octylene-$(CH_2)_4$-, -$(CH_2)_4$-diazabicyclo[2.2.2]octylene-$(CH_2)_5$-, -$(CH_2)_4$-diazabicyclo[2.2.2]octylene-$(CH_2)_6$-, -$(CH_2)_4$-diazabicyclo[2.2.2]octylene-$(CH_2)_7$-, -$(CH_2)_4$-diazabicyclo[2.2.2]octylene-$(CH_2)_8$-, -$(CH_2)_5$-diazabicyclo[2.2.2]octylene-$(CH_2)_1$-, -$(CH_2)_5$-diazabicyclo[2.2.2]octylene-$(CH_2)_2$-, -$(CH_2)_5$-diazabicyclo[2.2.2]octylene-$(CH_2)_3$-, -$(CH_2)_5$-diazabicyclo[2.2.2]octylene-$(CH_2)_4$-, -$(CH_2)_5$-diazabicyclo[2.2.2]octylene-$(CH_2)_5$-, -$(CH_2)_5$-diazabicyclo[2.2.2]octylene-$(CH_2)_6$-, -$(CH_2)_5$-diazabicyclo[2.2.2]octylene-$(CH_2)_7$-, -$(CH_2)_5$-diazabicyclo[2.2.2]octylene-$(CH_2)_8$-, -$(CH_2)_6$-diazabicyclo[2.2.2]octyle-

ne-$(CH_2)_1$-, -$(CH_2)_6$-diazabicyclo[2.2.2]octylene-$(CH_2)_2$-, -$(CH_2)_6$-diazabicyclo[2.2.2]octylene-$(CH_2)_3$-, -$(CH_2)_6$-diazabicyclo[2.2.2]octylene-$(CH_2)_4$-, -$(CH_2)_6$-diazabicyclo[2.2.2]octylene-$(CH_2)_5$-, -$(CH_2)_6$-diazabicyclo[2.2.2]octylene-$(CH_2)_6$-, -$(CH_2)_6$-diazabicyclo[2.2.2]octylene-$(CH_2)_7$-, -$(CH_2)_6$-diazabicyclo[2.2.2]octylene-$(CH_2)_8$-, -$(CH_2)_7$-diazabicyclo[2.2.2]octylene-$(CH_2)_1$-, -$(CH_2)_7$-diazabicyclo[2.2.2]octylene-$(CH_2)_2$-, -$(CH_2)_7$-diazabicyclo[2.2.2]octylene-$(CH_2)_3$-, -$(CH_2)_7$-diazabicyclo[2.2.2]octylene-$(CH_2)_4$-, -$(CH_2)_7$-diazabicyclo[2.2.2]octylene-$(CH_2)_8$-, -$(CH_2)_8$-diazabicyclo[2.2.2]octylene-$CH_2$-, -$(CH_2)_8$-diazabicyclo[2.2.2]octylene-$(CH_2)_2$-, -$(CH_2)_8$-diazabicyclo[2.2.2]octylene-$(CH_2)_3$-, -$(CH_2)_8$-diazabicyclo[2.2.2]octylene-$(CH_2)_4$-, -$(CH_2)_8$-diazabicyclo[2.2.2]octylene-$(CH_2)_5$-, -$(CH_2)_8$-diazabicyclo[2.2.2]octylene-$(CH_2)_6$-, -$(CH_2)_8$-diazabicyclo[2.2.2]octylene-$(CH_2)_7$-, or -$(CH_2)_8$-diazabicyclo[2.2.2]octylene-$(CH_2)_8$-;

wherein the propylene, the piperidinylene, the piperazinylene, the diazacycloheptanylene, the diazabicyclo[2.2.1]heptanylene, the diazabicyclo[3.1.1]heptanylene, the diazabicyclo[3.2.1]octylene, the diazabicyclo[2.2.2]octylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof; and

each $R_{g11}$ independently represents H or $C_{1-3}$ alkyl.

In some embodiments of the compound of Formula (I-2), $L_2$ represents the structure of the following formula:

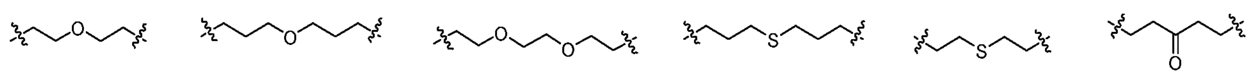

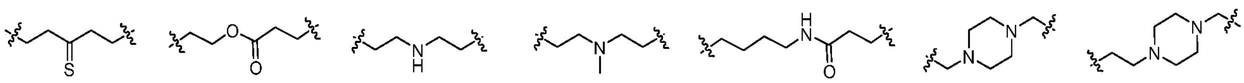

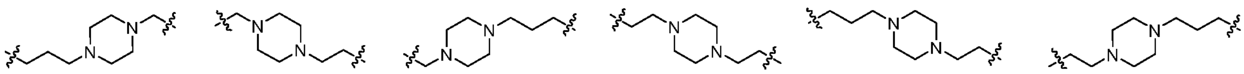

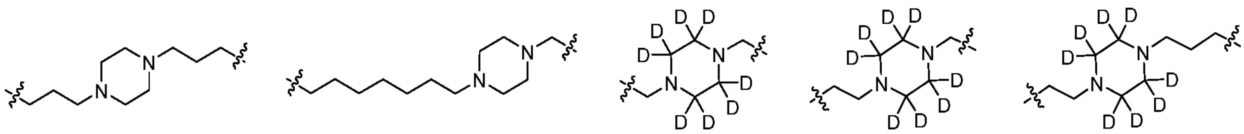

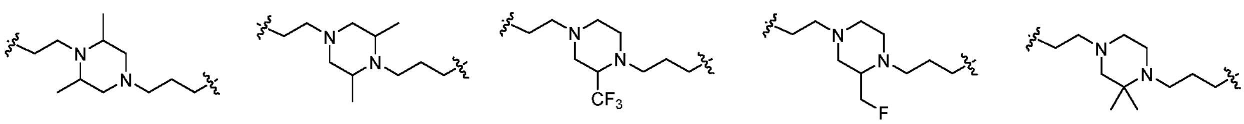

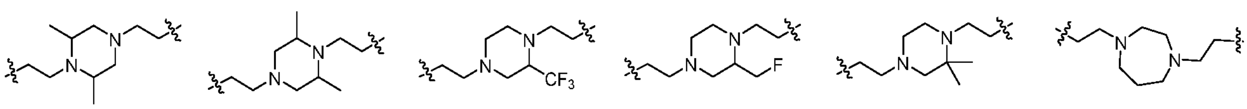

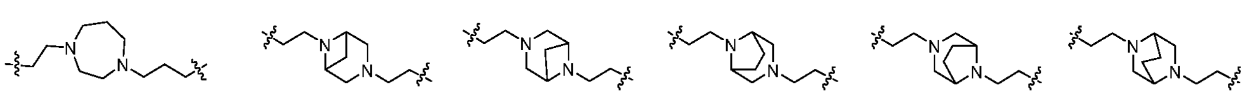

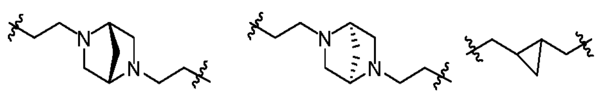 or 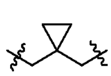

In some embodiments of the compound of Formula (I-2), $R_{b1}$ represents:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl,

bicyclo[2.2.1]heptanyl, 2-oxobicyclo[2.2.1]heptanyl or bicyclo[2.2.1]heptenyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 3-azaspiro[5.5]undecyl or 7-azaspiro[3.5]nonyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

phenyl or naphthyl, with each group being optionally substituted with one or more (e.g., 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl and imidazo[2,1-b]thiazolyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

In some embodiments of the compound of Formula (I-2), $R_{b1}$ represents:

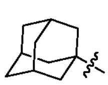 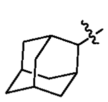 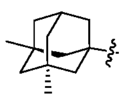 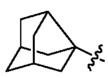 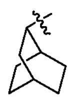 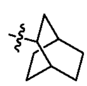 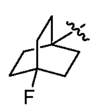 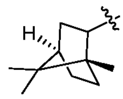 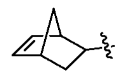 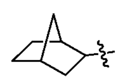

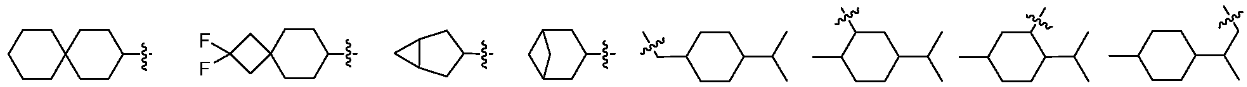

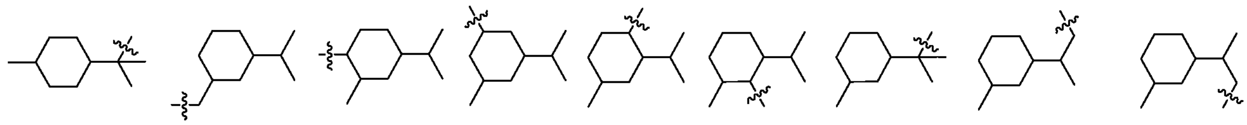

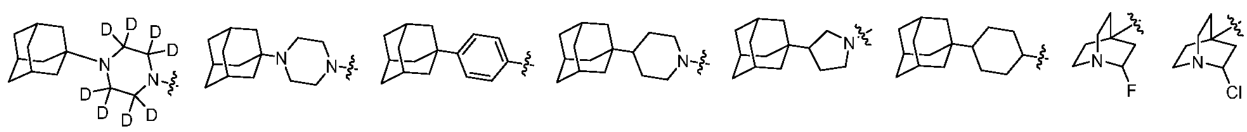

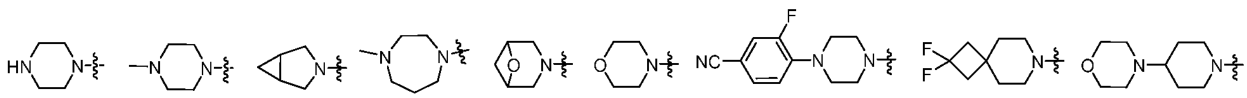

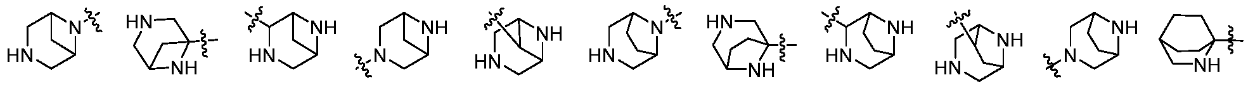

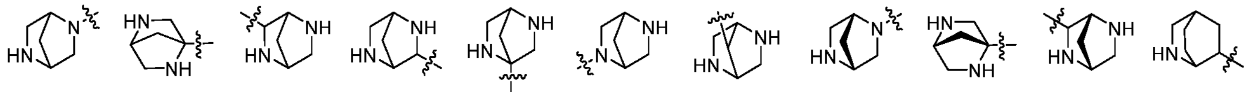

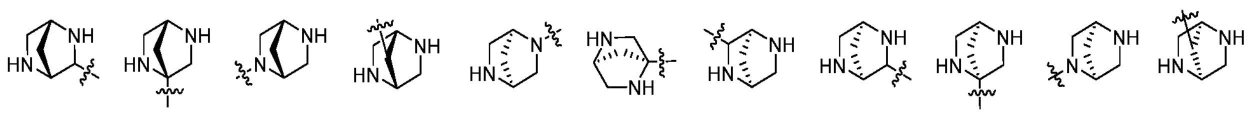

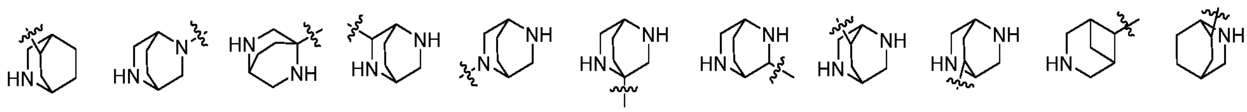

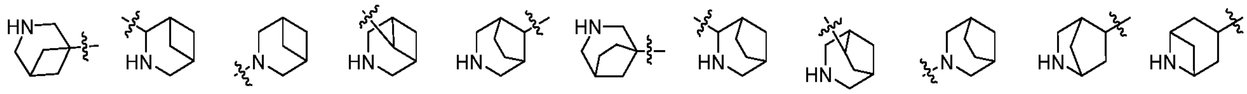

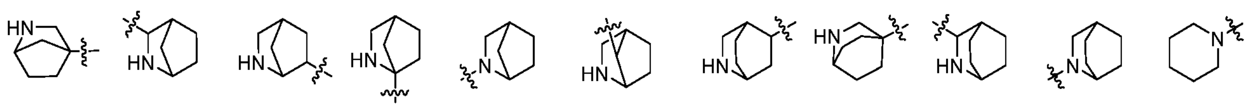

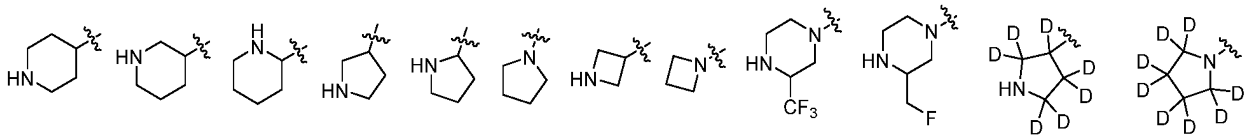

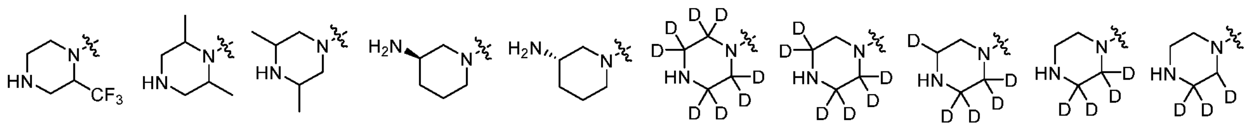

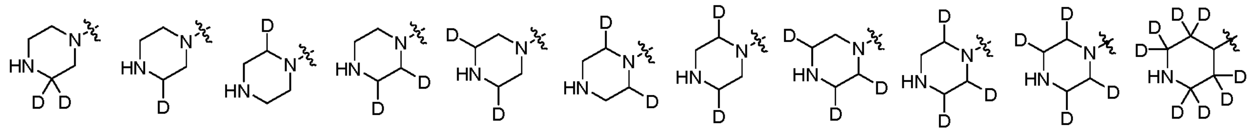

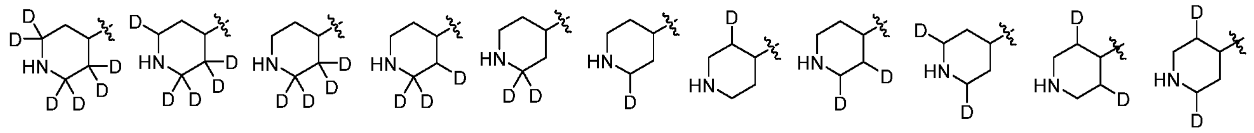

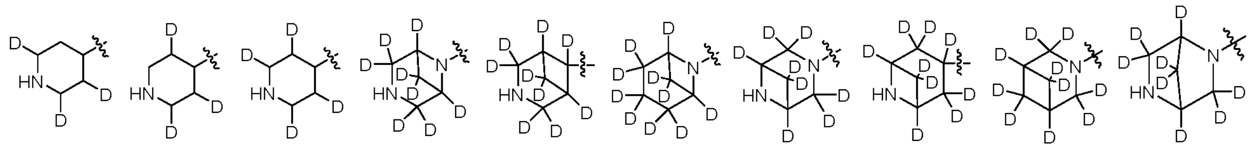

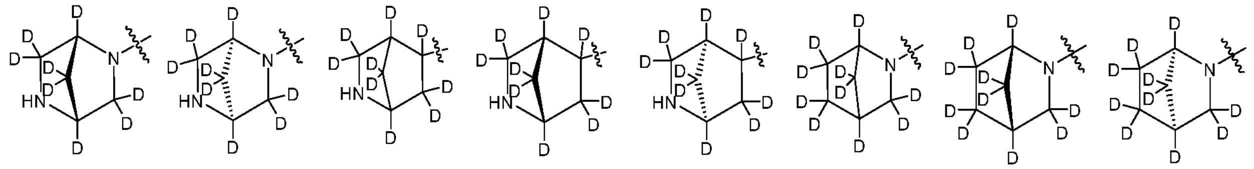

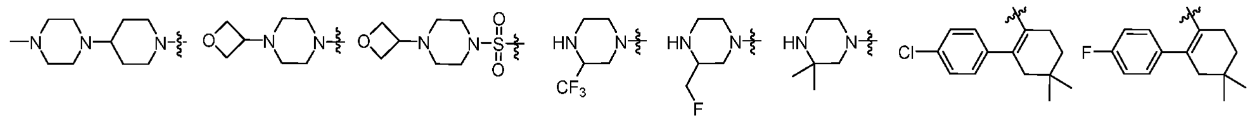

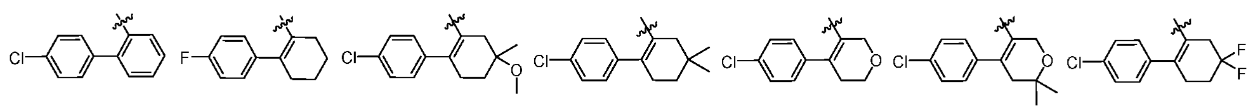

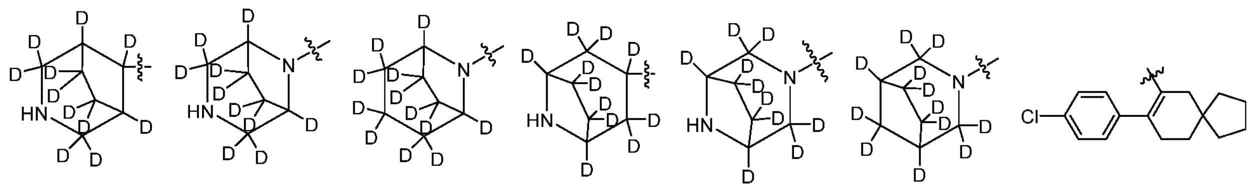

or

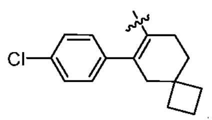

In some embodiments, the compound of Formula (I) is also of Formula (I-3):

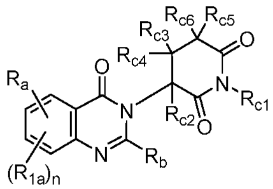

Formula (I-3)

wherein $R_{c1}$, $R_{c2}$, $R_{c3}$, $R_{c4}$, $R_{c5}$, $R_{c6}$, $R_b$, $(R_{1a})_n$, $R_a$, $R_{1a}$ and n are as defined in the compound of Formula (I) above and the embodiments thereof;

wherein $R_a$ represents the following formula:

$$R_{a1}\text{-}X_2\text{-}L_1\text{-}X_1\text{-} ,$$

wherein $X_1$ represents $N(R_1)$, O, S, alkynylene, or alkenylene, wherein $R_1$ represents H or $C_{1-3}$ alkyl, or $X_1$ represents a bond;
$L_1$ represents optionally substituted linear or branched $C_{1-60}$ alkylene (e.g., $C_1$-$C_{30}$ alkylene, $C_1$-$C_{29}$ alkylene, $C_1$-$C_{28}$ alkylene, $C_1$-$C_{27}$ alkylene, $C_1$-$C_{26}$ alkylene, $C_1$-$C_{25}$ alkylene, $C_1$-$C_{24}$ alkylene, $C_1$-$C_{23}$ alkylene, $C_1$-$C_{22}$ alkylene, $C_1$-$C_{21}$ alkylene, $C_1$-$C_{20}$ alkylene, $C_1$-$C_{19}$ alkylene, $C_1$-$C_{18}$ alkylene, $C_1$-$C_{17}$ alkylene, $C_1$-$C_{16}$ alkylene, $C_1$-$C_{15}$ alkylene, $C_1$-$C_{14}$ alkylene, $C_1$-$C_{13}$ alkylene, $C_1$-$C_{12}$ alkylene, $C_1$-$C_{11}$ alkylene, $C_1$-$C_{10}$ alkylene, $C_1$-$C_9$ alkylene, $C_1$-$C_8$ alkylene, $C_1$-$C_7$ alkylene, $C_1$-$C_6$ alkylene, $C_1$-$C_5$ alkylene, $C_1$-$C_4$ alkylene, $C_1$-$C_3$ alkylene, or $C_1$-$C_2$ alkylene);
$X_2$ represents $N(R_2)$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted 4- to 8-membered heterocyclylene containing 1 to 2 nitrogen atoms, triazolylene, triazolylene-NH-, -NH-triazolylene, or optionally substituted phenylene, wherein $R_2$ represents H or $C_{1-3}$ alkyl, or $X_2$ represents a bond; and
$R_{a1}$ represents hydroxy, optionally substituted adamantanyl, optionally substituted noradamantanyl, optionally substituted cubanyl, optionally substituted spirocycloalkyl, optionally substituted bicyclo[2.2.2]octan-1-yl, optionally substituted bicyclo[2.2.2]octan-2-yl, optionally substituted bomyl, optionally substituted bicyclo[2.2.1]heptanyl, optionally substituted bicyclo[2.2.1]heptenyl, optionally substituted p-menthanyl or optionally substituted m-menthanyl, wherein when $R_{a1}$ represents hydroxy, $X_2$ represents a bond;
and
$R_b$ represents $C_{1-60}$ alkyl (e.g., $C_1$-$C_{30}$ alkyl, $C_1$-$C_{29}$ alkyl, $C_1$-$C_{28}$ alkyl, $C_1$-$C_{27}$ alkyl, $C_1$-$C_{26}$ alkyl, $C_1$-$C_{25}$ alkyl, $C_1$-$C_{24}$ alkyl, $C_1$-$C_{23}$ alkyl, $C_1$-$C_{22}$ alkyl, $C_1$-$C_{21}$ alkyl, $C_1$-$C_{20}$ alkyl, $C_1$-$C_{19}$ alkyl, $C_1$-$C_{18}$ alkyl, $C_1$-$C_{17}$ alkyl, $C_1$-$C_{16}$ alkyl, $C_1$-$C_{15}$ alkyl, $C_1$-$C_{14}$ alkyl, $C_1$-$C_{13}$ alkyl, $C_1$-$C_{12}$ alkyl, $C_1$-$C_{11}$ alkyl, $C_1$-$C_{10}$ alkyl, $C_1$-$C_9$ alkyl, $C_1$-$C_8$ alkyl, $C_1$-$C_7$ alkyl, $C_1$-$C_6$ alkyl, $C_1$-$C_5$ alkyl, $C_1$-$C_4$ alkyl, $C_1$-$C_3$ alkyl, or $C_1$-$C_2$ alkyl) optionally substituted with D or halogen, or $R_b$ represents the following formula:

$$R_{b1}\text{-}X_4\text{-}L_2\text{-},$$

wherein $L_2$ represents:

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene), or
optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{12}$ and/or one or more (e.g., 1-20, or 1-15, 1-10, 1-9, 1-8, 1-7, 1-6, 1-5, 1-4, 1-3 or 1-2, or 1) groups $R_{13}$ and/or any combination of one or more (e.g., 1-20, or 1-15, 1-10, 1-9, 1-8, 1-7, 1-6, 1-5, 1-4, 1-3 or 1-2, or 1) groups $R_{12}$ and $R_{13}$ inserted into its backbone carbon chain, wherein carbon-carbon bond between one or more pairs (e.g., 1-10, 1-9, 1-8, 1-7, 1-6, 1-5, 1-4, 1-3 or 1-2, or 1 pair) of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of cycloalkylene (e.g., $C_{3-20}$cycloalkylene, or $C_{3-15}$cycloalkylene), heterocyclylene (e.g., 4- to 20-membered

heterocyclylene, or 5- to 15-membered heterocyclylene), arylene (e.g., $C_{6-10}$ arylene), heteroarylene (e.g., 5- to 20-membered heteroarylene, or 5- to 15-membered heteroarylene), alkenylene (e.g., $C_{2-6}$ alkenylene), alkynylene (e.g., $C_{2-6}$ alkynylene) or any combination thereof, wherein the cycloalkylene, the heterocyclylene, arylene and the heteroarylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof;

$X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted cycloalkylene (e.g., optionally substituted $C_{3-20}$cycloalkylene, or optionally substituted $C_{3-15}$cycloalkylene), optionally substituted arylene (e.g., optionally substituted $C_{6-20}$ arylene, or optionally substituted $C_{6-10}$ arylene), optionally substituted heterocyclylene (e.g., optionally substituted 4- to 20-membered heterocyclylene, or optionally substituted 4- to 15-membered heterocyclylene), optionally substituted heteroarylene (e.g., optionally substituted 5- to 20-membered heteroarylene, or optionally substituted 5- to 15-membered heteroarylene), triazolylene-NH-, or -NH-triazolylene, wherein $R_{15}$ represents H or $C_{1-3}$ alkyl, or $X_4$ represents a bond; and

$R_{b1}$ represents optionally substituted cycloalkyl (e.g., optionally substituted $C_{3-20}$cycloalkyl, or optionally substituted $C_{3-15}$cycloalkyl), optionally substituted aryl (e.g., optionally substituted $C_{6-20}$ aryl, or optionally substituted $C_{6-10}$ aryl), optionally substituted heterocyclyl (e.g., optionally substituted 4- to 20-membered heterocyclyl, or optionally substituted 4- to 15-membered heterocyclyl) or optionally substituted heteroaryl (e.g., optionally substituted 5- to 20-membered heteroaryl, or optionally substituted 5- to 15-membered heteroaryl).

In some embodiments of the compound of Formula (I-3), $R_a$ represents the following formula:

$$R_{a1}\text{-}X_2\text{-}L_1\text{-}X_1\text{-},$$

wherein $X_1$ represents a bond, or $X_1$ represents $N(R_1)$, O, S, $C_{2-6}$ alkynylene or $C_{2-6}$ alkenylene, wherein $R_1$ represents H or $C_{1-3}$ alkyl;

$L_1$ represents linear or branched $C_{1-60}$ alkylene (e.g., $C_1$-$C_{30}$ alkylene, $C_1$-$C_{29}$ alkylene, $C_1$-$C_{28}$ alkylene, $C_1$-$C_{27}$ alkylene, $C_1$-$C_{26}$ alkylene, $C_1$-$C_{25}$ alkylene, $C_1$-$C_{24}$ alkylene, $C_1$-$C_{23}$ alkylene, $C_1$-$C_{22}$ alkylene, $C_1$-$C_{21}$ alkylene, $C_1$-$C_{20}$ alkylene, $C_1$-$C_{19}$ alkylene, $C_1$-$C_{18}$alkylene, $C_1$-$C_{17}$ alkylene, $C_1$-$C_{16}$ alkylene, $C_1$-$C_{15}$ alkylene, $C_1$-$C_{14}$ alkylene, $C_1$-$C_{13}$ alkylene, $C_1$-$C_{12}$ alkylene, $C_1$-$C_{11}$ alkylene, $C_1$-$C_{10}$ alkylene, $C_1$-$C_9$ alkylene, $C_1$-$C_8$ alkylene, $C_1$-$C_7$ alkylene, $C_1$-$C_6$ alkylene, $C_1$-$C_5$ alkylene, $C_1$-$C_4$ alkylene, $C_1$-$C_3$ alkylene, or $C_1$-$C_2$ alkylene) optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy, or any combination thereof;

$X_2$ represents a bond, or $X_2$ represents $N(R_2)$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted 4- to 8-membered heterocyclylene containing 1 to 2 nitrogen atoms, triazolylene, triazolylene-NH-, -NH-triazolylene, or optionally substituted phenylene, wherein $R_2$ represents H or $C_{1-3}$ alkyl, and the 4- to 8-membered heterocyclylene containing 1 to 2 nitrogen atoms is optionally substituted with 1 to 8 substituents selected from the group consisting of D, halogen, optionally deuterated $C_{1-4}$ alkyl, oxo, hydroxy, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano, or any combination thereof, and the phenylene is optionally substituted with 1 to 4 substituents selected from the group consisting of D, halogen, optionally deuterated $C_{1-4}$ alkyl, hydroxy, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano, or any combination thereof; and

$R_{a1}$ represents hydroxy, optionally substituted adamantanyl, optionally substituted noradamantanyl, optionally substituted cubanyl, optionally substituted spirocycloalkyl (e.g., optionally substituted $C_{5-20}$ spirocycloalkyl or optionally substituted $C_{6-15}$ spirocycloalkyl), optionally substituted bicyclo[2.2.2]octan-1-yl, optionally substituted bicyclo[2.2.2]octan-2-yl, optionally substituted bomyl, optionally substituted bicyclo[2.2.1]heptanyl, optionally substituted bicyclo[2.2.1]heptenyl, optionally substituted p-menthanyl or optionally substituted m-menthanyl, wherein when $R_{a1}$ represents hydroxy, $X_2$ represents a bond, and the adamantanyl, noradamantanyl, cubanyl, spirocycloalkyl, bicyclo[2.2.2]octan-1-yl, bicyclo[2.2.2]octan-2-yl, bomyl, bicyclo[2.2.1]heptanyl, bicyclo[2.2.1]heptenyl, p-menthanyl and m-menthanyl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, halogen, optionally deuterated $C_{1-4}$ alkyl, hydroxy, amino, mercapto, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

In some embodiments of the compound of Formula (I-3), $R_{a1}$ represents hydroxy, and $X_2$ represents a bond.

In some embodiments of the compound of Formula (I-3), $R_{a1}$ represents:

adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, adamantan-10-yl, noradamantan-1-yl, noradamantan-2-yl, noradamantan-3-yl, noradamantan-4-yl, noradamantan-5-yl, noradamantan-6-yl, noradamantan-7-yl, noradamantan-8-yl, noradamantan-9-yl, cubanyl, $C_{5-15}$ spirocycloalkyl, bicyclo[2.2.2]octan-1-yl, bicyclo[2.2.2]octan-2-yl, 1,7,7-trimethylbicyclo[2.2.1]hept-2-yl, 1,7,7-trimethylbicyclo[2.2.1]hept-3-yl, 1,7,7-trimethylbicyclo[2.2.1]hept-4-yl, 1,7,7-trimethylbicyclo[2.2.1]hept-5-yl, 1,7,7-trimethylbicyclo[2.2.1]hept-6-yl,

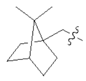 , 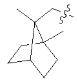 ,

bicyclo[2.2.1]hept-2-yl,bicyclo[2.2.1]hept-3-yl,bicyclo[2.2.1]hept-4-yl,bicyclo[2.2.1]hept-5-yl, bicyclo[2.2.1 ]hept-6-yl, bicyclo[2.2.1]hept-5-en-2-yl, bicyclo[2.2.1]hept-5-en-3-yl, bicyclo[2.2.1]hept-5-en-7-yl, 7,7-dimethyl-2-oxobicyclo[2.2.1]hept-1-yl, 7,7-dimethyl-2-oxobicyclo[2.2.1]hept-3-yl, 7,7-dimethyl-2-oxobicyclo[2.2.1]hept-4-yl, 7,7-dimethyl-2-oxobicyclo[2.2.1]hept-5-yl, 7,7-dimethyl-2-oxobicyclo[2.2.1]hept-6-yl, p-menthanyl or m-menthanyl, with each group being independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, halogen, optionally deuterated $C_{1-4}$ alkyl, hydroxy, amino, mercapto, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

In some embodiments of the compound of Formula (I-3), $R_{a1}$ represents:

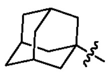 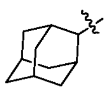 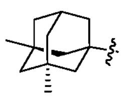 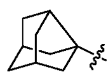 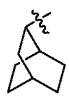 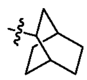 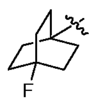 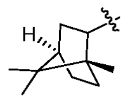 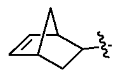 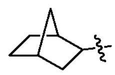

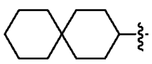 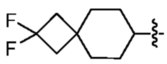 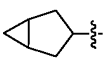 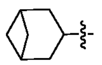 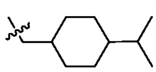 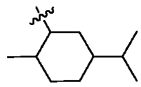 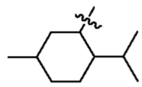 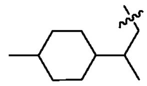

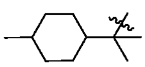 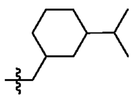 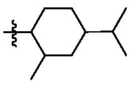 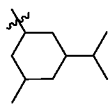 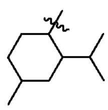 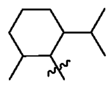 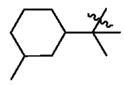 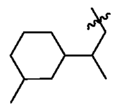 or 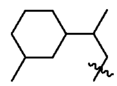

In some embodiments of the compound of Formula (I-3), $L_1$ represents the structure of the following formula: ##-$C_{1-30}$ alkylene-, wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the $C_{1-30}$ alkylene is optionally replaced with a substituent selected from the group consisting of: D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy or any combination thereof, and symbol ## indicates the point of attachment to $X_1$.

In some embodiments of the compound of Formula (I-3), $L_1$ represents the following group:

##-$CH_2$-, ##-$(CH_2)_2$-, ##-$(CH_2)_3$-, ##-$(CH_2)_4$-, ##-$(CH_2)_5$-, ##-$(CH_2)_2$-$CF_2$-$(CH_2)_2$-, ##-$(CH_2)_6$-, ##-$(CH_2)_7$-, ##-$(CH_2)_8$-, ##-$(CH_2)_9$-, ##-$(CH_2)_{10}$-, ##-$(CH_2)_{11}$-, ##-$(CH_2)_{12}$-, ##-$(CH_2)_{13}$-, ##-$(CH_2)_{14}$-, ##-$(CH_2)_{15}$-, ##-$(CH_2)_{16}$-, ##-$(CH_2)_{17}$-, ##-$(CH_2)_{18}$-, ##-$(CH_2)_{19}$-, ##-$(CH_2)_{20}$-, ##-$(CH_2)_{21}$-, ##-$(CH_2)_{22}$-, ##-$(CH_2)_{25}$-, or ##-$(CH_2)_{30}$-;

wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the group is optionally replaced with a substituent selected from the group consisting of: D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy or any combination thereof, and symbol ## indicates the point of attachment to $X_1$.

In some embodiments of the compound of Formula (I-3), $R_1$ represents $C_{1-60}$ alkyl (e.g., $C_1$-$C_{30}$ alkyl, $C_1$-$C_{29}$ alkyl, $C_1$-$C_{28}$ alkyl, $C_1$-$C_{27}$ alkyl, $C_1$-$C_{26}$ alkyl, $C_1$-$C_{25}$ alkyl, $C_1$-$C_{24}$ alkyl, $C_1$-$C_{23}$ alkyl, $C_1$-$C_{22}$ alkyl, $C_1$-$C_{21}$ alkyl, $C_1$-$C_{20}$

alkyl, $C_1$-$C_{19}$ alkyl, $C_1$-$C_{18}$ alkyl, $C_1$-$C_{17}$ alkyl, $C_1$-$C_{16}$ alkyl, $C_1$-$C_{15}$ alkyl, $C_1$-$C_{14}$ alkyl, $C_1$-$C_{13}$ alkyl, $C_1$-$C_{12}$ alkyl, $C_1$-$C_{11}$ alkyl, $C_1$-$C_{10}$ alkyl, $C_1$-$C_9$ alkyl, $C_1$-$C_8$ alkyl, $C_1$-$C_7$ alkyl, $C_1$-$C_6$ alkyl, $C_1$-$C_5$ alkyl, $C_1$-$C_4$ alkyl, $C_1$-$C_3$ alkyl, or $C_1$-$C_2$ alkyl) optionally substituted with D or halogen.

In some embodiments of the compound of Formula (I-3), $R_1$ represents the following group: $CH_3$, $CF_3$, $CD_3$, $CH_2CH_3$, $-(CH_2)_2-CH_3$, $-(CH_2)_3-CH_3$, $-(CH_2)_4-CH_3$, $-(CH_2)_5-CH_3$, $-(CH_2)_6-CH_3$, $-(CH_2)_7-CH_3$, $-(CH_2)_8-CH_3$, $-(CH_2)_9-CH_3$, $-(CH_2)_{10}-CH_3$, $-(CH_2)_{11}-CH_3$, $-(CH_2)_{12}-CH_3$, $-(CH_2)_{13}-CH_3$, $-(CH_2)_{14}-CH_3$, $-(CH_2)_{15}-CH_3$, $-(CH_2)_{16}-CH_3$, $-(CH_2)_{17}-CH_3$, $-(CH_2)_{18}-CH_3$, $-(CH_2)_{19}-CH_3$, $-(CH_2)_{20}-CH_3$, $-(CH_2)_{21}-CH_3$, $-(CH_2)_{22}-CH_3$, $-(CH_2)_{23}-CH_3$, $-(CH_2)_{24}-CH_3$, $-(CH_2)_{25}-CH_3$, $-(CH_2)_{26}-CH_3$, $-(CH_2)_{27}-CH_3$, $-(CH_2)_{28}-CH_3$, or $-(CH_2)_{29}-CH_3$.

In some embodiments of the compound of Formula (I-3), $R_b$ represents the following formula:

$$R_{b1}-X_4-L_2-,$$

wherein $L_2$ represents:

linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, $C_{1-4}$ alkyl, halogen, $C_{3-6}$cycloalkyl or any combination thereof, or optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{12}$ and/or one or more (e.g., 1-20, or 1-15, 1-10, 1-9, 1-8, 1-7, 1-6, 1-5, 1-4, 1-3 or 1-2, or 1) groups $R_{13}$ and/or any combination of one or more (e.g., 1-20, or 1-15, 1-10, 1-9, 1-8, 1-7, 1-6, 1-5, 1-4, 1-3 or 1-2, or 1) groups $R_{12}$ and $R_{13}$ inserted into its backbone carbon chain, wherein carbon-carbon bond between one or more pairs (e.g., 1-20, or 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of $C_{3-15}$cycloalkylene, 4- to 15-membered heterocyclylene, $C_{6-10}$ arylene, 5- to 15-membered heteroarylene or any combination thereof, wherein the $C_{3-15}$cycloalkylene, the 4- to 15-membered heterocyclylene, $C_{6-10}$ arylene and the 5- to 15-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-) or any combination thereof, and the linear or branched $C_{2-60}$ alkylene is optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, $C_{1-4}$ alkyl (e.g., $C_{1-3}$ alkyl, such as methyl, ethyl, propyl, isopropyl, butyl, sec-butyl or tert-butyl), halogen, $C_{3-6}$cycloalkyl or any combination thereof;

$X_4$ represents a bond, or $X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted $C_{3-20}$cycloalkylene, optionally substituted $C_{6-20}$ arylene, optionally substituted 4- to 20-membered heterocyclylene, optionally substituted 5- to 20-membered heteroarylene,

triazolylene-NH-, or -NH-triazolylene, wherein $R_{15}$ represents H or $C_{1-3}$ alkyl, and the $C_{3-20}$cycloalkylene, the $C_{6-20}$ arylene, the 4- to 20-membered heterocyclylene, and the 5- to 20-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, halogen, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof; and

$R_{b1}$ represents optionally substituted $C_{3-20}$cycloalkyl, optionally substituted $C_{6-20}$ aryl, optionally substituted 4- to 20-membered heterocyclyl or optionally substituted 5- to 20-membered heteroaryl, wherein the $C_{3-20}$cycloalkyl, the $C_{6-20}$ aryl, the 4- to 20-membered heterocyclyl, and the 5- to 20-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O) NH-, and $CH_3CH_2CH_2$-C(O)NH-), or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof.

In some embodiments of the compound of Formula (I-3), $L_2$ represents the structure of the following formula: -$C_{2-30}$ alkylene-, wherein ahydrogen atom of one or more (e.g., 1-20, or 1-15, 1-10, 1-9, 1-8, 1-7, 1-6, 1-5, 1-4, 1-3 or 1-2, or 1) $CH_2$ groups of the $C_{2-30}$ alkylene is optionally replaced with a substituent selected from the group consisting of: D, $C_{1-4}$ alkyl, halogen, $C_{3-6}$cycloalkyl or any combination thereof.

In some embodiments of the compound of Formula (I-3), $L_2$ represents the following group:

$-CH_2-$, $-(CH_2)_2-$, $-(CH_2)_3-$, $-(CH_2)_4-$, $-(CH_2)_5-$, $-(CH_2)_2-CF_2-(CH_2)_2-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{15}-$, $-(CH_2)_{16}-$, $-(CH_2)_{17}-$, $-(CH_2)_{18}-$, $-(CH_2)_{19}-$, $-(CH_2)_{20}-$, $-(CH_2)_{21}-$, $-(CH_2)_{22}-$, $-(CH_2)_{25}-$, or $-(CH_2)_{30}-$;

wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the group is optionally replaced with a substituent selected from the group consisting of: D, $C_{1-4}$ alkyl, halogen, $C_{3-6}$cycloalkyl or any combination thereof.

In some embodiments of the compound of Formula (I-3), $L_2$ represents the structure of the following formula:

$$-(C(R^{a9})(R^{a10}))_{m1}-(R_{12}(C(R^{a11})(R^{a12}))_{m2})_{p1}-;$$

$$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-;$$

$$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-(R_{12}(C(R^{a15})(R^{a16}))_{m4})_{p3}-;$$

$-(C(R^{a9})(R^{a10}))_{m1}-(R_{13}(C(R^{a11})(R^{a12}))_{m2})_{p1}-$;

$-(C(R^{a9})(R^{a10}))_{m1}-(R_{13}(C(R^{a11})(R^{a12}))_{m2})_{p1}-(R_{13}(C(R^{a13})(R^{a14}))_{m3})_{p2}-$;

$-(C(R^{a9})(R^{a10}))_{m1}-(R_{13}(C(R^{a11})(R^{a12}))_{m2})_{p1}-(R_{13}(C(R^{a13})(R^{a14}))_{m3})_{p2}-(R_{13}(C(R^{a15})(R^{a16}))_{m4})_{p3}-$;

$-(C(R^{a9})(R^{a10}))_{m1}-(R_{12}-R_{13}-(C(R^{a11})(R^{a12}))_{m2})_{p1}-$;

$-(C(R^{a9})(R^{a10}))_{m1}-(R_{12}(C(R^{a11})(R^{a12}))_{m2})_{p1}-(R_{13}(C(R^{a13})(R^{a14}))_{m3})_{p2}-$;

$-(C(R^{a9})(R^{a10}))_{m1}-(R_{13}-R_{12}-(C(R^{a11})(R^{a12}))_{m2})_{p1}-$; or

$-(C(R^{a9})(R^{a10}))_{m1}-(R_{13}(C(R^{a11})(R^{a12}))_{m2})_{p1}-(R_{12}(C(R^{a13})(R^{a14}))_{m3})_{p2}-$;

wherein each group $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_{14}$ independently represents H or $C_{1-3}$ alkyl; and each group $R_{13}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl, hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH-e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-) or any combination thereof;
$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and
m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-3), $L_2$ represents the structure of the following formula:

$-(C(R^{a9})(R^{a10}))_{m1}-(O(C(R^{a11})(R^{a12}))_{m2})_{p1}-$;

$-(C(R^{a9})(R^{a10}))_{m1}-(O(C(R^{a11})(R^{a12}))_{m2})_{p1}-(O(C(R^{a13})(R^{a14}))_{m3})_{p2}-$;

$-(C(R^{a9})(R^{a10}))_{m1}-(O(C(R^{a11})(R^{a12}))_{m2})_{p1}-(O(C(R^{a13})(R^{a14}))_{m3})_{p2}-(O(C(R^{a15})(R^{a16}))_{m4})_{p3}-$;

$-(C(R^{a9})(R^{a10}))_{m1}-(N(R_{g11})(C(R^{a11})(R^{a12}))_{m2})_{p1}-$;

$-(C(R^{a9})(R^{a10}))_{m1}-(N(R_{g11})(C(R^{a11})(R^{a12}))m_2)_{p1}-(N(R_{g11})(C(R^{a13})(R^{a14}))_{m3})_{p2}-$;

$-(C(R^{a9})(R^{a10}))_{m1}-(N(R_{g11})(C(R^{a11})(R^{a12}))m_2)_{p1}-(N(R_{g11)}(C(R^{a13})(R^{a14}))_{m3})_{p2}-(N(R_{g11})(C(R^{a15})(R^{a16}))_{m4})_{p3}-$;

$-(C(R^{a9})(R^{a10}))_{m1}-(C(O)NH-(C(R^{a11})(R^{a12}))_{m2})_{p1}$

$(C(R^{a9})(R^{a10}))_{m1}-(C(O)NH-(C(R^{a11})(R^{a12}))_{m2})_{p1}-(C(O)NH-(C(Ra^{13})(R^{a14}))_{m3})_{p2}-$;

$(C(R^{a9})(R^{a10}))_{m1}-(C(O)NH-(C(R^{a11})(R^{a12}))_{m2})_{p1}-(C(O)NH-(C(Ra^{13})(R^{a14}))_{m3})_{p2}-C(O)NH-(C(R^{a15})(R^{a16}))_{m4})_{p3}-$;

$(C(R^{a9})(R^{a10}))_{m1}-(C(O)NH-(C(R^{a11})(R^{a12}))_{m2})_{p1}-(O-(C(R^{a13})(R^{a14}))_{m3})_{p2}-$;

$(C(R^{a9})(R^{a10}))_{m1}$-(C(O)NH-$(C(R^{a11})(R^{a12}))_{m2}$-(O-$(C(R^{a13})(R^{a14}))_{m3})_{p1}$-;

$(C(R^{a9})(R^{a10}))_{m1}$-(NHC(O)-$(C(R^{a11})(R^{a12}))_{m2})_{p1}$-;

$C(R^{a9})(R^{a10}))_{m1}$-(NHC(O)-$(C(R^{a11})(R^{a12}))_{m2})_{p1}$-(O-$(C(R^{a13})(R^{a14}))_{m3})_{p2}$-;

$(C(R^{a9})(R^{a10}))_{m1}$-(NHC(O)-$(C(R^{a11})(R^{a12}))_{m2})_{p1}$-(O-$(C(R^{a13})(R^{a14}))_{m3})_{p2}$-(O-$(C(R^{a15})(R^{a16}))_{m4})_{p3}$-;

-$(C(R^{a9})(R^{a10}))_{m1}$-NHC(O)-$(C(Ra^{11})(R^{a12}))_{m2}$-(O$(C(R^{a13})(R^{a14}))_{m3})_{p1}$-;

-$(C(R^{a9})(R^{a10}))_{m1}$-NHC(O)NH-$(C(R^{a11})(R^{a12}))_{m2}$-;

-$(C(R^{a9})(R^{a10}))_{m1}$-C(O)-$(C(R^{a11})(R^{a12}))_{m2}$-;

-$(C(R^{a9})(R^{a10}))_{m1}$-C(S)-$(C(R^{a11})(R^{a12}))_{m2}$-;

-$(C(R^{a9})(R^{a10}))_{m1}$-S-$(C(R^{a11})(R^{a12}))_{m2}$-;

-$(C(R^{a9})(R^{a10}))_{m1}$-($C_{6-10}$ arylene-$(C(R^{a11})(R^{a12}))_{m2})_{p1}$-;

-$(C(R^{a9})(R^{a10}))_{m1}$-($C_{6-10}$ arylene-$(C(R^{a11})(R^{a12}))_{m2})_{p1}$-$C_{6-10}$ arylene-$(C(R^{a13})(R^{a14}))_{m3}$-;

-$(C(R^{a9})(R^{a10}))_{m1}$-(4-to 15-membered heterocyclylene-$(C(R^{a11})(R^{a12}))_{m2})_{p1}$-;

-$(C(R^{a9})(R^{a10}))_{m1}$-(4- to 15-membered heterocyclylene-$(C(R^{a11})(R^{a12}))_{m2})_{p1}$-(4- to 15-membered heterocyclylene-$(C(R^{a13})(R^{a14}))_{m3})_{p2}$-;

-$(C(R^{a9})(R^{a10}))_{m1}$-(5- to 15-membered heteroarylene-$(C(R^{a11})(R^{a12}))_{m2})_{p1}$-;

-$(C(R^{a9})(R^{a10}))_{m1}$-(5- to 15-membered heteroarylene-$(C(R^{a11})(R^{a12}))_{m2})_{p1}$-(5- to 15-membered heteroarylene-$(C(R^{a13})(R^{a14}))_{m3})_{p2}$-;

-$(C(R^{a1})(R^{a10}))_{m1}$-($C_{3-15}$cycloalkylene-$(C(R^{a11})(R^{a12}))_{m2})_{p1}$-; or

-$(C(R^{a1})(R^{a10}))_{m1}$-($C_{3-15}$cycloalkylene-$(C(R^{a11})(R^{a12}))_{m2})_{p1}$-($C_{3-15}$cycloalkylene-$(C(R^{a13})$ $(R^{a14}))_{m3})_{p2}$-;

wherein each $R_{g11}$ independently represents H or $C_{1-3}$ alkyl;
the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;
$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and
m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-3), $L_2$ represents the structure of the following formula: $-CH_2-O-CH_2-$, $-CH_2-O-(CH_2)_2-$, $-(CH_2)_1-O-(CH_2)_3-$, $-(CH_2)_1-O-(CH_2)_4-$, $-(CH_2)_1-O-(CH_2)_5-$, - $(CH_2)_1-O-(CH_2)_6-$, $-(CH_2)_1-O-(CH_2)_7-$, $-(CH_Z)_1-O-(CH_2)_8-$, $-(CH_2)_1-O-(CH_2)_9-$, $-(CH_Z)_1-O-(CH_2)_{10}-$, - $(CH_2)_2-O-(CH_2)_1-$, $-(CH_2)_2-O-(CH_2)_2-$, $-(CH_2)_2-O-(CH_2)_3-$, $-(CH_2)_2-O-(CH_2)_4-$, $-(CH_2)_2-O-(CH_2)_5-$, - $(CH_2)_2-O-(CH_2)_6-$, $-(CH_2)_2-O-(CH_2)_7-$, $-(CH_2)_2-O-(CH_2)_8-$, $-(CH_2)_2-O-(CH_2)_9-$, $-(CH_2)_2-O-(CH_2)_{10}-$, - $(CH_2)_2-O-(CH_2)_{11}-$, $-(CH_2)_2-O-(CH_2)_{12}-$, $-(CH_2)_3-O-(CH_2)_1-$, $-(CH_2)_3-O-(CH_2)_2-$, $-(CH_2)_3-O-(CH_2)_3-$, - $(CH_2)_3-O-(CH_2)_4-$, $-(CH_2)_3-O-(CH_2)_5-$, $-(CH_2)_3-O-(CH_2)_6-$, $-(CH_2)_3-O-(CH_2)_7-$, $-(CH_2)_4-O-(CH_2)_1-$, - $(CH_2)_4-O-(CH_2)_2-$, $-(CH_2)_4-O-(CH_2)_3-$, $-(CH_2)_4-O-(CH_2)_4-$, $-(CH_2)_4-O-(CH_2)_5-$, $-(CH_2)_4-O-(CH_2)_6-$, - $(CH_2)_5-O-(CH_2)_1-$, $-(CH_2)_5-O-(CH_2)_2-$, $-(CH_2)_5-O-(CH_2)_3-$, $-(CH_2)_5-O-(CH_2)_4-$, $-(CH_2)_5-O-(CH_2)_5-$, - $(CH_2)_6-O-(CH_2)_1-$, $-(CH_2)_6-O-(CH_2)_2-$, $-(CH_2)_6-O-(CH_2)_3-$, $-(CH_2)_6-O-(CH_2)_4-$, $-(CH_2)_7-O-(CH_2)_1-$, - $(CH_2)_7-O-(CH_2)_2-$, $-(CH_2)_7-O-(CH_2)_3-$,

$-(CH_2)_8-O-(CH_2)_1-$, $-(CH_2)_8-O-(CH_2)_2-$, $-CH(CH_3)-O-(CH_2)_1-$, $-CH(CH_3)-O-(CH_2)_2-$, $-CH(CH_3)-O-(CH_2)_3-$, $-CH(CH_3)-O-(CH_2)_4-$, $-CH(CH_3)-O-(CH_2)_5-$, $-CH(CH_3)-O-(CH_2)_6-$, $-CH(CH_3)-O-(CH_2)_7-$, $-CH(CH_3)-O-(CH_2)_8-$, $-CH(CH_3)-O-(CH_2)_9-$, $-CH(CH_3)-O-(CH_2)_{10}-$, $-CH_2-(O(CH_2)_2)_2-$, $-CH_2-(O(CH_2)_2)_3-$, $-CH_2-(O(CH_2)_2)_4-$, $-CH_2-(O(CH_2)_2)_5-$, $-CH_2-(O(CH_2)_2)_6-$, $-CH_2-(O(CH_2)_2)_7-$, $-CH_2-(O(CH_2)_2)_8-$, $-CH_2-(O(CH_2)_2)_9-$, $-CH_2-(O(CH_2)_2)_{10}-$, $-CH_2-(O(CH_2)_2)_1-OCH_2-$, $-CH_2-(O(CH_2)_2)_2-OCH_2-$, $-CH_2-(O(CH_2)_2)_3-OCH_2-$, $-CH_2-(O(CH_2)_2)_4-OCH_2-$, $-CH_2-(O(CH_2)_2)_5-OCH_2-$, $-CH_2-(O(CH_2)_2)_6-OCH_2-$, $-CH_2-(O(CH_2)_2)_7-OCH_2-$, $-CH_2-(O(CH_2)_2)_8-OCH_2-$, $-CH_2-(O(CH_2)_2)_9-OCH_2-$, $-CH_2-(O(CH_2)_2)_lo-OCH_2-$, $-(CH_2)_2-(O(CH_2)_2)_2-$, $-(CH_2)_2-(O(CH_2)_2)_3-$, $-(CH_2)_2-(O(CH_2)_2)_4-$, $-(CH_2)_2-(O(CH_2)_2)_5-$, $-(CH_2)_2-(O(CH_2)_2)_6-$, $-(CH_2)_2-(O(CH_2)_2)_7-$, $-(CH_2)_2-(O(CH_2)_2)_8-$, $-(CH_2)_2-(O(CH_2)_2)_9-$, $-(CH_2)_2-(O(CH_2)_2)_{10}-$, $-(CH_2)_3-(O(CH_2)_2)_2-$, $-(CH_2)_3-(O(CH_2)_2)_3-$, $-(CH_2)_3-(O(CH_2)_2)_4-$, $-(CH_2)_3-(O(CH_2)_2)_5-$, $-(CH_2)_3-(O(CH_2)_2)_6-$, $-(CH_2)_3-(O(CH_2)_2)_7-$, $-(CH_2)_3-(O(CH_2)_2)_8-$, $-(CH_2)_3-(O(CH_2)_2)_9-$, $-(CH_2)_3-(O(CH_2)_2)_{10}-$, $-(CH_2)_4-(O(CH_2)_2)_2-$, $-(CH_2)_4-(O(CH_2)_2)_3-$, $-(CH_2)_4-(O(CH_2)_2)_4-$, $-(CH_2)_4-(O(CH_2)_2)_5-$, $-(CH_2)_4-(O(CH_2)_2)_6-$, $-(CH_2)_4-(O(CH_2)_2)_7-$, $-(CH_2)_4-(O(CH_2)_2)_8-$, $-(CH_2)_4-(O(CH_2)_2)_9-$, $-(CH_2)_4-(O(CH_2)_2)_{10}-$, $-CH_2-(O(CH_2)_3)_2-$, $-CH_2-(O(CH_2)_3)_3-$, $-CH_2-(O(CH_2)_3)_4-$, $-CH_2-(O(CH_2)_3)_5-$, $-CH_2-(O(CH_2)_3)_6-$, $-CH_2-(O(CH_2)_3)_7-$, $-CH_2-(O(CH_2)_3)_8-$, $-CH_2-(O(CH_2)_3)_9-$, $-CH_2-(O(CH_2)_3)_{10}-$, $-(CH_2)_2-(O(CH_2)_3)_2-$, $-(CH_2)_2-(O(CH_2)_3)_3-$, $-(CH_2)_2-(O(CH_2)_3)_4-$, $-(CH_2)_2-(O(CH_2)_3)_5-$, $-(CH_2)_2-(O(CH_2)_3)_6-$, $-(CH_2)_2-(O(CH_2)_3)_7-$, $-(CH_2)_2-(O(CH_2)_3)_8-$, $-(CH_2)_2-(O(CH_2)_3)_9-$, $-(CH_2)_2-(O(CH_2)_3)_{10}-$, $-(CH_2)_3-(O(CH_2)_3)_2-$, $-(CH_2)_3-(O(CH_2)_3)_3-$, $-(CH_2)_3-(O(CH_2)_3)_4-$, $-(CH_2)_3-(O(CH_2)_3)_5-$, $-(CH_2)_3-(O(CH_2)_3)_6-$, $-(CH_2)_3-(O(CH_2)_3)_7-$, $-(CH_2)_3-(O(CH_2)_3)_8-$, $-(CH_2)_3-(O(CH_2)_3)_9-$, $-(CH_2)_3-(O(CH_2)_3)_{10}-$, $-CH_2-O-(CH_2)_2-O-(CH_2)_3-$, $-CH_2-(O(CH_2)_2)_2-(O(CH_2)_3)_2-$, $-CH_2-(O(CH_2)_2)_3-(O(CH_2)_3)_3-$, $-CH_2-(O(CH_2)_2)_4-(O(CH_2)_3)_4-$, $-CH_2-(O(CH_2)_2)_5-(O(CH_2)_3)_5-$, $-CH_2-(O(CH_2)_2)_6-(O(CH_2)_3)_6-$, $-(CH_2)_2-O-(CH_2)_2-O-(CH_2)_3-$, $-(CH_2)_2-(O(CH_2)_2)_2-(O(CH_2)_3)_2-$, $-(CH_2)_2-(O(CH_2)_2)_3-(O(CH_2)_3)_3-$, $-(CH_2)_2-(O(CH_2)_2)_4-(O(CH_2)_3)_4-$, $-(CH_2)_2-(O(CH_2)_2)_5-(O(CH_2)_3)_5-$, $-(CH_2)_2-(O(CH_2)_2)_6-(O(CH_2)_3)_6-$, $-(CH_2)_3-O-(CH_2)_2-O-(CH_2)_3-$, $-(CH_2)_3-(O(CH_2)_2)_2-(O(CH_2)_3)_2-$, $-(CH_2)_3-(O(CH_2)_2)_3-(O(CH_2)_3)_3-$, $-(CH_2)_3-(O(CH_2)_2)_4-(O(CH_2)_3)_4-$, $-(CH_2)_3-(O(CH_2)_2)_5-(O(CH_2)_3)_5-$, $-(CH_2)_3-(O(CH_2)_2)_6-(O(CH_2)_3)_6-$, $-CH_2-O-(CH_2)_3-O-(CH_2)_2-$, $-CH_2-(O(CH_2)_3)_2-(O(CH_2)_2)_2-$, $-CH_2-(O(CH_2)_3)_3-(O(CH_2)_2)_3-$, $-CH_2-(O(CH_2)_3)_4-(O(CH_2)_2)_4-$, $-CH_2-(O(CH_2)_3)_5-(O(CH_2)_2)_5-$, $-CH_2-(O(CH_2)_3)_6-(O(CH_2)_2)_6-$, $-(CH_2)_2-O-(CH_2)_3-O-(CH_2)_2-$, $-(CH_2)_2-(O(CH_2)_3)_2-(O(CH_2)_2)_2-$, $-(CH_2)_2-(O(CH_2)_3)_3-(O(CH_2)_2)_3-$, $-(CH_2)_2-(O(CH_2)_3)_4-(O(CH_2)_2)_4-$, $-(CH_2)_2-(O(CH_2)_3)_5-(O(CH_2)_2)_5-$, $-(CH_2)_2-(O(CH_2)_3)_6-(O(CH_2)_2)_6-$, $-(CH_2)_3-O-(CH_2)_3-O-(CH_2)_2-$, $-(CH_2)_3-(O(CH_2)_3)_2-(O(CH_2)_2)_2-$, $-(CH_2)_3-(O(CH_2)_3)_3-(O(CH_2)_2)_3-$, $-(CH_2)_3-(O(CH_2)_3)_4-(O(CH_2)_2)_4-$, $-(CH_2)_3-(O(CH_2)_3)_5-(O(CH_2)_2)_5-$, $-(CH_2)_3-(O(CH_2)_3)_6-(O(CH_2)_2)_6-$, $-CH_2-O-(CH_2)_2-O-CH_2-$, $-(CH_2)_2-O-(CH_2)_2-O-CH_2-$, $-(CH_2)_2-(O(CH_2)_2)_2-O-(CH_2)_3-$, $-(CH_2)_2-(O(CH_2)_2)_3-O-(CH_2)_3-$, $-(CH_2)_2-(O(CH_2)_2)_4-O-(CH_2)_3-$, $-(CH_2)_5-(O(CH_2)_2)_2-O-(CH_2)_5-$, $-(CH_2)_5-(O(CH_2)_2)_2-O-(CH_2)_6-$, $-CH_2-C(O)-CH_2-$, $-CH_2-C(O)-(CH_2)_2-$, $-(CH_2)_1-C(O)-(CH_2)_3-$, $-(CH_2)_1-C(O)-(CH_2)_4-$, $-(CH_2)_1-C(O)-(CH_2)_5-$, $-(CH_2)_1-C(O)-(CH_2)_6-$, $-(CH_2)_1-C(O)-(CH_2)_7-$, $-(CH_2)_1-C(O)-(CH_2)_8-$, $-(CH_2)_1-C(O)-(CH_2)_9-$, $-(CH_2)_1-C(O)-(CH_2)_{10}-$, $-(CH_2)_2-C(O)-(CH_2)_1-$, $-(CH_2)_2-C(O)-(CH_2)_2-$, $-(CH_2)_2-C(O)-(CH_2)_3-$, $-(CH_2)_2-C(O)-(CH_2)_4-$, $-(CH_2)_2-C(O)-(CH_2)_5-$, $-(CH_2)_2-C(O)-(CH_2)_6-$, $-(CH_2)_2-C(O)-(CH_2)_7-$, $-(CH_2)_2-C(O)-(CH_2)_8-$, $-(CH_2)_2-C(O)-(CH_2)_9-$, $-(CH_2)_2-C(O)-(CH_2)_{10}-$, $-(CH_2)_2-C(O)-(CH_2)_{11}-$, $-(CH_2)_2-C(O)-(CH_2)_{12}-$, $-(CH_2)_3-C(O)-(CH_2)_1-$, $-(CH_2)_3-C(O)-(CH_2)_2-$, $-(CH_2)_3-C(O)-(CH_2)_3-$, $-(CH_2)_3-C(O)-(CH_2)_4-$, $-(CH_2)_3-C(O)-(CH_2)_5-$, $-(CH_2)_3-C(O)-(CH_2)_6-$, $-(CH_2)_3-C(O)-(CH_2)_7-$, $-(CH_2)_4-C(O)-(CH_2)_1-$, $-(CH_2)_4-C(O)-(CH_2)_2-$, $-(CH_2)_4-C(O)-(CH_2)_3-$, $-(CH_2)_4-C(O)-(CH_2)_4-$, $-(CH_2)_4-C(O)-(CH_2)_5-$, $-(CH_2)_4-C(O)-(CH_2)_6-$, $-(CH_2)_5-C(O)-(CH_2)_1-$, $-(CH_2)_5-C(O)-(CH_2)_2-$, $-(CH_2)_5-C(O)-(CH_2)_3-$, $-(CH_2)_5-C(O)-(CH_2)_4-$, $-(CH_2)_5-C(O)-(CH_2)_5-$, $-(CH_2)_6-C(O)-(CH_2)_1-$, $-(CH_2)_6-C(O)-(CH_2)_2-$, $-(CH_2)_6-C(O)-(CH_2)_3-$, $-(CH_2)_6-C(O)-(CH_2)_4-$, $-(CH_2)_7-C(O)-(CH_2)_1-$, $-(CH_2)_7-C(O)-(CH_2)_2-$, $-(CH_2)_7-C(O)-(CH_2)_3-$, $-(CH_2)_8-C(O)-(CH_2)_1-$, $-(CH_2)_8-C(O)-(CH_2)_2-$, $-CH_2-S-CH_2-$, $-CH_2-S-(CH_2)_2-$, $-(CH_2)_1-S-(CH_2)_3-$, $-(CH_2)_1-S-(CH_2)_4-$, $-(CH_2)_1-S-(CH_2)_5-$, $-(CH_2)_1-S-(CH_2)_6-$, $-(CH_2)_1-S-(CH_2)_7-$, $-(CH_2)_1-S-(CH_2)_8-$, $-(CH_2)_1-S-(CH_2)_9-$, $-(CH_2)_1-S-(CH_2)_{10}-$, $-(CH_2)_2-S-(CH_2)_1-$, $-(CH_2)_2-S-(CH_2)_2-$, $-(CH_2)_2-S-(CH_2)_3-$, $-(CH_2)_2-S-(CH_2)_4-$, $-(CH_2)_2-S-(CH_2)_5-$, $-(CH_2)_2-S-(CH_2)_6-$, $-(CH_2)_2-S-(CH_2)_7-$, $-(CH_2)_2-S-(CH_2)_8-$, $-(CH_2)_2-S-(CH_2)_9-$, $-(CH_2)_2-S-(CH_2)_{10}-$, $-(CH_2)_2-S-(CH_2)_{11}-$, $-(CH_2)_2-S-(CH_2)_{12}-$, $-(CH_2)_3-S-(CH_2)_1-$, $-(CH_2)_3-S-(CH_2)_2-$, $-(CH_2)_3-S-(CH_2)_3-$, $-(CH_2)_3-S-(CH_2)_4-$, $-(CH_2)_3-S-(CH_2)_5-$, $-(CH_2)_3-S-(CH_2)_6-$, $-(CH_2)_3-S-(CH_2)_7-$, $-(CH_2)_4-S-(CH_2)_1-$, $-(CH_2)_4-S-(CH_2)_2-$, $-(CH_2)_4-S-(CH_2)_3-$, $-(CH_2)_4-S-(CH_2)_4-$, $-(CH_2)_4-S-(CH_2)_5-$, $-(CH_2)_4-S-(CH_2)_6-$, $-(CH_2)_5-S-(CH_2)_1-$, $-(CH_2)_5-S-(CH_2)_2-$, $-(CH_2)_5-S-(CH_2)_3-$, $-(CH_2)_5-S-(CH_2)_4-$, $-(CH_2)_5-S-(CH_2)_5-$, $-(CH_2)_6-S-(CH_2)_1-$, $-(CH_2)_6-S-(CH_2)_2-$, $-(CH_2)_6-S-(CH_2)_3-$, $-(CH_2)_6-S-(CH_2)_4-$, $-(CH_2)_7-S-(CH_2)_1-$, $-(CH_2)_7-S-(CH_2)_2-$, $-(CH_2)_7-S-(CH_2)_3-$, $-(CH_2)_S-S-(CH_2)_1-$, $-(CH_2)_8-S-(CH_2)_2-$, $-CH_2-C(S)-CH_2-$, $-CH_2-C(S)-(CH_2)_2-$, $-(CH_2)_1-C(S)-(CH_2)_3-$, $-(CH_2)_1-C(S)-(CH_2)_4-$, $-(CH_2)_1-C(S)-(CH_2)_5-$, $-(CH_2)_1-C(S)-(CH_2)_6-$, $-(CH_2)_1-C(S)-(CH_2)_7-$, $-(CH_2)_1-C(S)-(CH_2)_8-$, $-(CH_2)_1-C(S)-(CH_2)_9-$, $-(CH_2)_1-C(S)-(CH_2)_{10}-$, $-(CH_2)_2-C(S)-(CH_2)_1-$, $-(CH_2)_2-C(S)-(CH_2)_2-$, $-(CH_2)_2-C(S)-(CH_2)_3-$, $-(CH_2)_2-C(S)-(CH_2)_4-$, $-(CH_2)_2-C(S)-(CH_2)_5-$, $-(CH_2)_2-C(S)-(CH_2)_6-$, $-(CH_2)_2-C(S)-(CH_2)_7-$, $-(CH_2)_2-C(S)-(CH_2)_8-$, $-(CH_2)_2-C(S)-(CH_2)_9-$, $-(CH_2)_2-C(S)-(CH_2)_{10}-$, $-(CH_2)_2-C(S)-(CH_2)_{11}-$, $-(CH_2)_2-C(S)-(CH_2)_{12}-$, $-(CH_2)_3-C(S)-(CH_2)_1-$, $-(CH_2)_3-C(S)-(CH_2)_2-$, $-(CH_2)_3-C(S)-(CH_2)_3-$, $-(CH_2)_3-C(S)-(CH_2)_4-$, $-(CH_2)_3-C(S)-(CH_2)_5-$, $-(CH_2)_3-C(S)-(CH_2)_6-$, $-(CH_2)_3-C(S)-(CH_2)_7-$, $-(CH_2)_4-C(S)-(CH_2)_1-$, $-(CH_2)_4-C(S)-(CH_2)_2-$, $-(CH_2)_4-C(S)-(CH_2)_3-$, $-(CH_2)_4-C(S)-(CH_2)_4-$, $-(CH_2)_4-C(S)-(CH_2)_5-$, $-(CH_2)_4-C(S)-(CH_2)_6-$, $-(CH_2)_5-C(S)-(CH_2)_1-$, $-(CH_2)_5-C(S)-(CH_2)_2-$, $-(CH_2)_5-C(S)-(CH_2)_3-$, $-(CH_2)_5-C(S)-(CH_2)_4-$, $-(CH_2)_5-C(S)-(CH_2)_5-$, $-(CH_2)_6-C(S)-(CH_2)_1-$, $-(CH_2)_6-C(S)-(CH_2)_2-$, $-(CH_2)_6-C(S)-(CH_2)_3-$, $-(CH_2)_6-C(S)-(CH_2)_4-$, $-(CH_2)_7-C(S)-(CH_2)_l-$, $-(CH_2)_7-C(S)-(CH_2)_2-$, $-(CH_2)_7-C(S)-(CH_2)_3-$, $-(CH_2)_S-C(S)-(CH_2)_1-$,

$-(CH_2)_8-C(S)-(CH_2)_2-$, $-CH_2-C(O)O-CH_2-$, $-CH_2-C(O)O-(CH_2)_2-$, $-(CH_2)_1-C(O)O-(CH_2)_3-$, $-(CH_Z)_1-C(O)O-(CH_2)_4-$, $-(CH_2)_1-C(O)O-(CH_2)_5-$, $-(CH_2)_1-C(O)O-(CH_2)_6-$, $-(CH_2)_1-C(O)O-(CH_2)_7-$, $-(CH_2)_1-C(O)O-(CH_2)_8-$, $-(CH_2)_1-C(O)O-(CH_2)_9-$, $-(CH_2)_1-C(O)O-(CH_2)_{10}-$, $-(CH_2)_2-C(O)O-(CH_2)_1-$, $-(CH_2)_2-C(O)O-(CH_2)_2-$, $-(CH_2)_2-C(O)O-(CH_2)_3-$, $-(CH_2)_2-C(O)O-(CH_2)_4-$, $-(CH_2)_2-C(O)O-(CH_2)_5-$, $-(CH_2)_2-C(O)O-(CH_2)_6-$, $-(CH_2)_2-C(O)O-(CH_2)_7-$, $-(CH_2)_2-C(O)O-(CH_2)_8-$, $-(CH_2)_2-C(O)O-(CH_2)_9-$, $-(CH_2)_2-C(O)O-(CH_2)_{10}-$, $-(CH_2)_2-C(O)O-(CH_2)_{11}-$, $-(CH_2)_2-C(O)O-(CH_2)_{12}-$, $-(CH_2)_3-C(O)O-(CH_2)_1-$, $-(CH_2)_3-C(O)O-(CH_2)_2-$, $-(CH_2)_3-C(O)O-(CH_2)_3-$, $-(CH_2)_3-C(O)O-(CH_2)_4-$, $-(CH_2)_3-C(O)O-(CH_2)_5-$, $-(CH_2)_3-C(O)O-(CH_2)_6-$, $-(CH_2)_3-C(O)O-(CH_2)_7-$, $-(CH_2)_4-C(O)O-(CH_2)_1-$, $-(CH_2)_4-C(O)O-(CH_2)_2-$, $-(CH_2)_4-C(O)O-(CH_2)_3-$, $-(CH_2)_4-C(O)O-(CH_2)_4-$, $-(CH_2)_4-C(O)O-(CH_2)_5-$, $-(CH_2)_4-C(O)O-(CH_2)_6-$, $-(CH_2)_5-C(O)O-(CH_2)_1-$, $-(CH_2)_5-C(O)O-(CH_2)_2-$, $-(CH_2)_5-C(O)O-(CH_2)_3-$, $-(CH_2)_5-C(O)O-(CH_2)_4-$, $-(CH_2)_5-C(O)O-(CH_2)_5-$, $-(CH_2)_6-C(O)O-(CH_2)_1-$, $-(CH_2)_6-C(O)O-(CH_2)_2-$, $-(CH_2)_6-C(O)O-(CH_2)_3-$, $-(CH_2)_6-C(O)O-(CH_2)_4-$, $-(CH_2)_7-C(O)O-(CH_2)_1-$, $-(CH_2)_7-C(O)O-(CH_2)_2-$, $-(CH_2)_7-C(O)O-(CH_2)_3-$, $-(CH_2)_8-C(O)O-(CH_2)_1-$, $-(CH_2)_8-C(O)O-(CH_2)_2-$, $-CH_2-O-C(O)-CH_2-$, $-CH_2-OC(O)-(CH_2)_2-$, $-(CH_2)_1-OC(O)-(CH_2)_3-$, $-(CH_2)_1-OC(O)-(CH_2)_4-$, $-(CH_2)_1-OC(O)-(CH_2)_5-$, $-(CH_2)_1-O-C(O)-(CH_2)_6-$, $-(CH_2)_1-OC(O)-(CH_2)_7-$, $-(CH_2)_1-OC(O)-(CH_2)_8-$, $-(CH_2)_1-OC(O)-(CH_2)_9-$, $-(CH_2)_1-OC(O)-(CH_2)_{10}-$, $-(CH_2)_2-OC(O)-(CH_2)_1-$, $-(CH_2)_2-OC(O)-(CH_2)_2-$, $-(CH_2)_2-OC(O)-(CH_2)_3-$, $-(CH_2)_2-OC(O)-(CH_2)_4-$, $-(CH_2)_2-O-C(O)-(CH_2)_5-$, $-(CH_2)_2-OC(O)-(CH_2)_6-$, $-(CH_2)_2-OC(O)-(CH_2)_7-$, $-(CH_2)_2-OC(O)-(CH_2)_8-$, $-(CH_2)_2-OC(O)-(CH_2)_9-$, $-(CH_2)_2-OC(O)-(CH_2)_{10}-$, $-(CH_2)_2-OC(O)-(CH_2)_{11}-$, $-(CH_2)_2-OC(O)-(CH_2)_{12}-$, $-(CH_2)_3-OC(O)-(CH_2)_1-$, $-(CH_2)_3-O-C(O)-(CH_2)_2-$, $-(CH_2)_3-OC(O)-(CH_2)_3-$, $-(CH_2)_3-OC(O)-(CH_2)_4-$, $-(CH_2)_3-OC(O)-(CH_2)_5-$, $-(CH_2)_3-OC(O)-(CH_2)_6-$, $-(CH_2)_3-OC(O)-(CH_2)_7-$, $-(CH_2)_4-OC(O)-(CH_2)_1-$, $-(CH_2)_4-OC(O)-(CH_2)_2-$, $-(CH_2)_4-OC(O)-(CH_2)_3-$, $-(CH_2)_4-O-C(O)-(CH_2)_4-$, $-(CH_2)_4-OC(O)-(CH_2)_5-$, $-(CH_2)_4-OC(O)-(CH_2)_6-$, $-(CH_2)_5-OC(O)-(CH_2)_1-$, $-(CH_2)_5-OC(O)-(CH_2)_2-$, $-(CH_2)_5-OC(O)-(CH_2)_3-$, $-(CH_2)_5-OC(O)-(CH_2)_4-$, $-(CH_2)_5-OC(O)-(CH_2)_5-$, $-(CH_2)_6-OC(O)-(CH_2)_1-$, $-(CH_2)_6-O-C(O)-(CH_2)_2-$, $-(CH_2)_6-OC(O)-(CH_2)_3-$, $-(CH_2)_6-OC(O)-(CH_2)_4-$, $-(CH_2)_7-OC(O)-(CH_2)_1-$, $-(CH_2)_7-OC(O)-(CH_2)_2-$, $-(CH_2)_7-OC(O)-(CH_2)_3-$, $-(CH_2)_8-OC(O)-(CH_2)_1-$, $-(CH_2)_8-OC(O)-(CH_2)_2-$, $-(CH_2)_1-N(R_{g11})-(CH_2)_1-$, $-(CH_2)_1-N(R_{g11})-(CH_2)_2-$, $-(CH_2)_1-N(R_{g11})-(CH_2)_3-$, $-(CH_2)_1-N(R_{g11})-(CH_2)_4-$, $-(CH_2)_1-N(R_{g11})-(CH_2)_5-$, $-(CH_2)_1-N(R_{g11})-(CH_2)_6-$, $-(CH_2)_1-N(R_{g11})-(CH_2)_7-$, $-(CH_2)_1-N(R_{g11})-(CH_2)_8-$, $-(CH_2)_1-N(R_{g11})-(CH_2)_9-$, $-(CH_2)_1-N(R_{g11})-(CH_2)_{10}-$, $-(CH_2)_2-N(R_{g11})-(CH_2)_1-$, $-(CH_2)_2-N(R_{g11})-(CH_2)_2-$, $-(CH_2)_2-N(R_{g11})-(CH_2)_3-$, $-(CH_2)_2-N(R_{g11})-(CH_2)_4-$, $-(CH_2)_2-N(R_{g11})-(CH_2)_5-$, $-(CH_2)_2-N(R_{g11})-(CH_2)_6-$, $-(CH_2)_2-N(R_{g11})-(CH_2)_7-$, $-(CH_2)_2-N(R_{g11})-(CH_2)_8-$, $-(CH_2)_2-N(R_{g11})-(CH_2)_9-$, $-(CH_2)_2-N(R_{g11})-(CH_2)_{10}-$, $-(CH_2)_2-N(R_{g11})-(CH_2)_{11}-$, $-(CH_2)_2-N(R_{g11})-(CH_2)_{12}-$, $-(CH_2)_3-N(R_{g11})-(CH_2)_1-$, $-(CH_2)_3-N(R_{g11})-(CH_2)_2-$, $-(CH_2)_3-N(R_{g11})-(CH_2)_3-$, $-(CH_2)_4-N(R_{g11})-(CH_2)_1-$, $-(CH_2)_4-N(R_{g11})-(CH_2)_2-$, $-(CH_2)_4-N(R_{g11})-(CH_2)_3-$, $-(CH_2)_4-N(R_{g11})-(CH_2)_4-$, $-(CH_2)_5-N(R_{g11})-(CH_2)_1-$, $-(CH_2)_5-N(R_{g11})-(CH_2)_2-$, $-(CH_2)_5-N(R_{g11})-(CH_2)_3-$, $-(CH_2)_5-N(R_{g11})-(CH_2)_4-$, $-(CH_2)_5-N(R_{g11})-(CH_2)_5-$, $-(CH_2)_6-N(R_{g11})-(CH_2)_1-$, $-(CH_2)_6-N(R_{g11})-(CH_2)_2-$, $-(CH_2)_6-N(R_{g11})-(CH_2)_3-$, $-(CH_2)_7-N(R_{g11})-(CH_2)_1-$, $-(CH_2)_7-N(R_{g11})-(CH_2)_2-$, $-(CH_2)_7-N(R_{g11})-(CH_2)_3-$, $-(CH_2)_S-N(R_{g11})-(CH_2)_1-$, $-(CH_2)_8-NR_{g11})-(CH_2)_2-$, $-(CH_2)_8-N(R_{g11})-(CH_2)_3-$, $-CH(CH_3)-N(R_{g11})-(CH_2)_1-$, $-CH(CH_3)-N(R_{g11})-(CH_2)_2-$, $-CH(CH_3)-N(R_{g11})-(CH_2)_3-$, $-CH(CH_3)-N(R_{g11})-(CH_2)_4-$, $-CH(CH_3)-N(R_{g11})-(CH_2)_5-$, $-CH(CH_3)-N(R_{g11})-(CH_2)_6-$, $-CH(CH_3)-N(R_{g11})-(CH_2)_7-$, $-CH(CH_3)-N(R_{g11})-(CH_2)_8-$, $-CH(CH_3)-N(R_{g11})-(CH_2)_9-$, $-CH(CH_3)-N(R_{g11})-(CH_2)_{10}-$, $-CH_2C(O)NHCH_2-$, $-(CH_2)_{2C}(O)NH(CH_2)_2-$, $-(CH_2)_{2C}(O)NH(CH_2)_3-$, $-(CH_2)_2C(O)NH(CH_2)_4-$, $-(CH_2)_2C(O)NH(CH_2)_5-$, $-(CH_2)_3C(O)NH(CH_2)_3-$, $-(CH_2)_3C(O)NH(CH_2)_4-$, $-(CH_2)_4C(O)NH(CH_2)_4-$, $-(CH_2)_5C(O)NH(CH_2)_5-$, $-(CH_2)_6C(O)NH(CH_2)_7-$, $-(CH_2)_6C(O)NH(CH_2)_6-$, $-(CH_2)_7C(O)NH(CH_2)_7-$, $-(CH_2)_8C(O)NH(CH_2)_8$, $-(CH_2)_9C(O)NH(CH_2)_9-$, $-(CH_2)_{10}C(O)NH(CH_2)_{10}-$, $-(CH_2)_2C(O)NH(CH_2)_2-O-(CH_2)_2-$, $-CH_2NHC(O)CH_2-$, $-(CH_2)_2NHC(O)(CH_2)_2-$, $-(CH_2)_2NHC(O)(CH_2)_3-$, $-(CH_2)_2NHC(O)(CH_2)_4-$, $-(CH_2)_2NHC(O)(CH_2)_5-$, $-(CH_2)_3NHC(O)(CH_2)_3-$, $-(CH_2)_3NHC(O)(CH_2)_4-$, $-(CH_2)_4NHC(O)(CH_2)_4-$, $-(CH_2)_5NHC(O)(CH_2)_5-$, $-(CH_2)_6NHC(O)(CH_2)_7-$, $-(CH_2)_6NHC(O)(CH_2)_6-$, $-(CH_2)_7NHC(O)(CH_2)_7-$, $-(CH_2)_8NHC(O)(CH_2)_8$, $-(CH_2)_9NHC(O)(CH_2)_9-$, $-(CH_2)_{lo}NHC(O)(CH_2)_{10}-$, $-(CH_2)_4NHC(O)(CH_2)_8-$, $-(CH_2)_2NHC(O)(CH_2)_2-O-(CH_2)_2-$, $-(CH_2)_4NHC(O)CH_2-$, $-CH_2$-piperidinylene-$CH_2-$, $-CH_2$-piperidinylene-$(CH_2)_2-$, $-CH_2$-piperidinylene-$(CH_2)_3-$, $-CH_2$-piperidinylene-$(CH_2)_4-$, $-CH_2$-piperidinylene-$(CH_2)_5-$, $-CH_2$-piperidinylene-$(CH_2)_6-$, $-CH_2$-piperidinylene-$(CH_2)_7-$, $-CH_2$-piperidinylene-$(CH_2)_8-$, $-(CH_2)_2$-piperidinylene-$(CH_2)_1-$, $-(CH_2)_2$-piperidinylene-$(CH_2)_2-$, $-(CH_2)_2$-piperidinylene-$(CH_2)_3-$, $-(CH_2)_2$-piperidinylene-$(CH_2)_4-$, $-(CH_2)_2$-piperidinylene-$(CH_2)_5-$, $-(CH_2)_2$-piperidinylene-$(CH_2)_6-$, $-(CH_2)_2$-piperidinylene-$(CH_2)_7-$, $-(CH_2)_2$-piperidinylene-$(CH_2)_8-$, $-(CH_2)_3$-piperidinylene-$CH_2-$, $-(CH_2)_3$-piperidinylene-$(CH_2)_2-$, $-(CH_2)_3$-piperidinylene-$(CH_2)_3-$, $-(CH_2)_3$-piperidinylene-$(CH_2)_4-$, $-(CH_2)_3$-piperidinylene-$(CH_2)_5-$, $-(CH_2)_3$-piperidinylene-$(CH_2)_6-$, $-(CH_2)_3$-piperidinylene-$(CH_2)_7-$, $-(CH_2)_3$-piperidinylene-$(CH_2)_8-$, $-(CH_2)_4$-piperidinylene-$CH_2-$, $-(CH_2)_4$-piperidinylene-$(CH_2)_2-$, $-(CH_2)_4$-piperidinylene-$(CH_2)_3-$, $-(CH_2)_4$-piperidinylene-$(CH_2)_4-$, $-(CH_2)_4$-piperidinylene-$(CH_2)_5-$, $-(CH_2)_4$-piperidinylene-$(CH_2)_6-$, $-(CH_2)_4$-piperidinylene-$(CH_2)_7-$, $-(CH_2)_4$-piperidinylene-$(CH_2)_8-$, $-(CH_2)_5$-piperidinylene-$(CH_2)_1-$, $-(CH_2)_5$-piperidinylene-$(CH_2)_2-$, $-(CH_2)_5$-piperidinylene-$(CH_2)_3-$, $-(CH_2)_5$-piperidinylene-$(CH_2)_4-$, $-(CH_2)_5$-piperidinylene-$(CH_2)_5-$, $-(CH_2)_5$-piperidinylene-$(CH_2)_6-$, $-(CH_2)_5$-piperidinylene-$(CH_2)_7-$, $-(CH_2)_5$-piperidinylene-$(CH_2)_8-$, $-(CH_2)_6$-piperidinylene-$(CH_2)_1-$, $-(CH_2)_6$-piperidinylene-$(CH_2)_2-$, $-(CH_2)_6$-piperidinylene-$(CH_2)_3-$, $-(CH_2)_6$-piperidinylene-$(CH_2)_4-$, $-(CH_2)_6$-piperidinylene-$(CH_2)_5-$, $-(CH_2)_6$-piperidinylene-$(CH_2)_6-$, $-(CH_2)_6$-piperidinylene-$(CH_2)_7-$, $-(CH_2)_6$-piperidinylene-$(CH_2)_8-$, $-(CH_2)_7$-piperidinylene-$(CH_2)_1-$, $-(CH_2)_7$-piperidinylene-$(CH_2)_2-$, $-(CH_2)_7$-piperidinylene-$(CH_2)_3-$, $-(CH_2)_7$-piperidinylene-$(CH_2)_4-$, $-(CH_2)_7$-piperidinylene-$(CH_2)_8-$, $-(CH_2)_8$-piperidinylene-$CH_2-$, $-(CH_2)_8$-piperidinylene-$(CH_2)_2-$, $-(CH_2)_8$-piperidinylene-$(CH_2)_3-$, $-(CH_2)_8$-piperidinylene-$(CH_2)_4-$, $-(CH_2)_8$-piperidinylene-$(CH_2)_5-$,

$-(CH_2)_8$-piperidinylene-$(CH_2)_6$-, $-(CH_2)_8$-piperidinylene-$(CH_2)_7$-, $-(CH_2)_8$- piperidinylene-$(CH_2)_8$-, $-CH_2$-piperazinylene-$CH_2$-, $-CH_2$-piperazinylene-$(CH_2)_2$-, $-CH_2$- piperazinylene-$(CH_2)_3$-, $-CH_2$-piperazinylene-$(CH_2)_4$-, $-CH_2$-piperazinylene-$(CH_2)_5$-, $-CH_2$- piperazinylene-$(CH_2)_6$-, $-CH_2$-piperazinylene-$(CH_2)_7$-, $-CH_2$-piperazinylene-$(CH_2)_8$-, $-(CH_2)_2$- piperazinylene-$(CH_2)_1$-, $-(CH_2)_2$-piperazinylene-$(CH_2)_2$-, $-(CH_2)_2$-piperazinylene-$(CH_2)_3$-, $-(CH_2)_2$- piperazinylene-$(CH_2)_4$-, $-(CH_2)_2$-piperazinylene-$(CH_2)_5$-, $-(CH_2)_2$-piperazinylene-$(CH_2)_6$-, $-(CH_2)_2$- piperazinylene-$(CH_2)_7$-, $-(CH_2)_2$-piperazinylene-$(CH_2)_8$-, $-(CH_2)_3$-piperazinylene-$CH_2$-, $-(CH_2)_3$- piperazinylene-$(CH_2)_2$-, $-(CH_2)_3$-piperazinylene-$(CH_2)_3$-, $-(CH_2)_3$-piperazinylene-$(CH_2)_4$-, $-(CH_2)_3$- piperazinylene-$(CH_2)_5$-, $-(CH_2)_3$-piperazinylene-$(CH_2)_6$-, $-(CH_2)_3$-piperazinylene-$(CH_2)_7$-, $-(CH_2)_3$- piperazinylene-$(CH_2)_8$-, $-(CH_2)_4$-piperazinylene-$CH_2$-, $-(CH_2)_4$-piperazinylene-$(CH_2)_2$-, $-(CH_2)_4$-piperazinylene-$(CH_2)_3$-, $-(CH_2)_4$-piperazinylene-$(CH_2)_4$-, $-(CH_2)_4$-piperazinylene-$(CH_2)_5$-, $-(CH_2)_4$- piperazinylene-$(CH_2)_6$-, $-(CH_2)_4$-piperazinylene-$(CH_2)_7$-, $-(CH_2)_4$-piperazinylene-$(CH_2)_8$-, $-(CH_2)_5$- piperazinylene-$(CH_2)_1$-, $-(CH_2)_5$-piperazinylene-$(CH_2)_2$-, $-(CH_2)_5$-piperazinylene-$(CH_2)_3$-, $-(CH_2)_5$- piperazinylene-$(CH_2)_4$-, $-(CH_2)_5$-piperazinylene-$(CH_2)_5$-, $-(CH_2)_5$-piperazinylene-$(CH_2)_6$-, $-(CH_2)_5$-piperazinylene-$(CH_2)_7$-, $-(CH_2)_5$-piperazinylene-$(CH_2)_8$-, $-(CH_2)_6$-piperazinylene-$(CH_2)_1$-, $-(CH_2)_6$-piperazinylene-$(CH_2)_2$-, $-(CH_2)_6$-piperazinylene-$(CH_2)_3$-, $-(CH_2)_6$-piperazinylene-$(CH_2)_4$-, $-(CH_2)_6$-piperazinylene-$(CH_2)_5$-, $-(CH_2)_6$-piperazinylene-$(CH_2)_6$-, $-(CH_2)_6$-piperazinylene-$(CH_2)_7$-, $-(CH_2)_6$-piperazinylene-$(CH_2)_8$-, $-(CH_2)_7$-piperazinylene-$(CH_2)_1$-, $-(CH_2)_7$-piperazinylene-$(CH_2)_2$-, $-(CH_2)_7$-piperazinylene-$(CH_2)_3$-, $-(CH_2)_7$-piperazinylene-$(CH_2)_4$-, $-(CH_2)_7$-piperazinylene-$(CH_2)_8$-, $-(CH_2)_8$-piperazinylene-$CH_2$-, $-(CH_2)_8$-piperazinylene-$(CH_2)_2$-, $-(CH_2)_8$-piperazinylene-$(CH_2)_3$-, $-(CH_2)_8$-piperazinylene-$(CH_2)_4$-, $-(CH_2)_8$-piperazinylene-$(CH_2)_5$-, $-(CH_2)_8$-piperazinylene-$(CH_2)_6$-, $-(CH_2)_8$-piperazinylene-$(CH_2)_7$-, $-(CH_2)_8$-piperazinylene-$(CH_2)_8$-, $-CH_2$-propylene-$CH_2$-, $-CH_2$-propylene-$(CH_2)_2$-, $-CH_2$-propylene-$(CH_2)_3$-, $-CH_2$-propylene-$(CH_2)_4$-, $-CH_2$-propylene-$(CH_2)_5$-, $-CH_2$-propylene-$(CH_2)_6$-, $-CH_2$-propylene-$(CH_2)_7$-, $-CH_2$-propylene-$(CH_2)_8$-, $-(CH_2)_2$-propylene-$(CH_2)_1$-, $-(CH_2)_2$-propylene-$(CH_2)_2$-, $-(CH_2)_2$-propylene-$(CH_2)_3$-, $-(CH_2)_2$-propylene-$(CH_2)_4$-, $-(CH_2)_2$-propylene-$(CH_2)_5$-, $-(CH_2)_2$-propylene-$(CH_2)_6$-, $-(CH_2)_2$-propylene-$(CH_2)_7$-, $-(CH_2)_2$-propylene-$(CH_2)_8$-, $-(CH_2)_3$-propylene-$CH_2$-, $-(CH_2)_3$-propylene-$(CH_2)_2$-, $-(CH_2)_3$-propylene-$(CH_2)_3$-, $-(CH_2)_3$-propylene-$(CH_2)_4$-,$-(CH_2)_3$-propylene-$(CH_2)_5$-, $-(CH_2)_3$-propylene-$(CH_2)_6$-, $-(CH_2)_3$-propylene-$(CH_2)_7$-, $-(CH_2)_3$-propylene-$(CH_2)_8$-, $-(CH_2)_4$-propylene-$CH_2$-, $-(CH_2)_4$-propylene-$(CH_2)_2$-, $-(CH_2)_4$-propylene-$(CH_2)_3$-, $-(CH_2)_4$-propylene-$(CH_2)_4$-, $-(CH_2)_4$-propylene-$(CH_2)_5$-, $-(CH_2)_4$-propylene-$(CH_2)_6$-, $-(CH_2)_4$-propylene-$(CH_2)_7$-, $-(CH_2)_4$-propylene-$(CH_2)_8$-, $-(CH_2)_5$-propylene-$(CH_2)_1$-, $-(CH_2)_5$-propylene-$(CH_2)_2$-, $-(CH_2)_5$-propylene-$(CH_2)_3$-, $-(CH_2)_5$-propylene-$(CH_2)_4$-, $-(CH_2)_5$-propylene-$(CH_2)_5$-, $-(CH_2)_5$-propylene-$(CH_2)_6$-, $-(CH_2)_5$-propylene-$(CH_2)_7$-, $-(CH_2)_5$-propylene-$(CH_2)_8$-, $-(CH_2)_6$-propylene-$(CH_2)_1$-, $-(CH_2)_6$-propylene-$(CH_2)_2$-, $-(CH_2)_6$-propylene-$(CH_2)_3$-, $-(CH_2)_6$-propylene-$(CH_2)_4$-, $-(CH_2)_6$-propylene-$(CH_2)_5$-, $-(CH_2)_6$-propylene-$(CH_2)_6$-, $-(CH_2)_6$-propylene-$(CH_2)_7$-, $-(CH_2)_6$-propylene-$(CH_2)_8$-, $-(CH_2)_7$-propylene-$(CH_2)_1$-, $-(CH_2)_7$-propylene-$(CH_2)_2$-, $-(CH_2)_7$-propylene-$(CH_2)_3$-, $-(CH_2)_7$-propylene-$(CH_2)_4$-, $-(CH_2)_7$-propylene-$(CH_2)_8$-, $-(CH_2)_8$-propylene-$CH_2$-, $-(CH_2)_8$-propylene-$(CH_2)_2$-, $-(CH_2)_8$-propylene-$(CH_2)_3$-, $-(CH_2)_8$-propylene-$(CH_2)_4$-, $-(CH_2)_8$-propylene-$(CH_2)_5$-, $-(CH_2)_8$-propylene-$(CH_2)_6$-, $-(CH_2)_8$-propylene-$(CH_2)_7$-, $-(CH_2)_8$-propylene-$(CH_2)_8$-, $-CH_2$-diazacycloheptanylene-$CH_2$-, $-CH_2$-diazacycloheptanylene-$(CH_2)_2$-, $-CH_2$-diazacycloheptanylene-$(CH_2)_3$-, $-CH_2$-diazacycloheptanylene-$(CH_2)_4$-, $-CH_2$-diazacycloheptanylene-$(CH_2)_5$-, $-CH_2$-diazacycloheptanylene-$(CH_2)_6$-, $-CH_2$-diazacycloheptanylene-$(CH_2)_7$-, $-CH_2$-diazacycloheptanylene-$(CH_2)_8$-, $-(CH_2)_2$-diazacycloheptanylene-$(CH_2)_1$-, $-(CH_2)_2$-diazacycloheptanylene-$(CH_2)_2$-, $-(CH_2)_2$-diazacycloheptanylene-$(CH_2)_3$-, $-(CH_2)_2$-diazacycloheptanylene-$(CH_2)_4$-, $-(CH_2)_2$-diazacycloheptanylene-$(CH_2)_5$-, $-(CH_2)_2$-diazacycloheptanylene-$(CH_2)_6$-, $-(CH_2)_2$-diazacycloheptanylene-$(CH_2)_7$-, $-(CH_2)_2$-diazacycloheptanylene-$(CH_2)_8$-, $-(CH_2)_3$-diazacycloheptanylene-$CH_2$-, $-(CH_2)_3$-diazacycloheptanylene-$(CH_2)_2$-, $-(CH_2)_3$-diazacycloheptanylene-$(CH_2)_3$-, $-(CH_2)_3$-diazacycloheptanylene-$(CH_2)_4$-, $-(CH_2)_3$-diazacycloheptanylene-$(CH_2)_5$-, $-(CH_2)_3$-diazacycloheptanylene-$(CH_2)_6$-, $-(CH_2)_3$-diazacycloheptanylene-$(CH_2)_7$-, $-(CH_2)_3$-diazacycloheptanylene-$(CH_2)_8$-, $-(CH_2)_4$-diazacycloheptanylene-$CH_2$-, $-(CH_2)_4$-diazacycloheptanylene-$(CH_2)_2$-, $-(CH_2)_4$-diazacycloheptanylene-$(CH_2)_3$-, $-(CH_2)_4$-diazacycloheptanylene-$(CH_2)_4$-, $-(CH_2)_4$-diazacycloheptanylene-$(CH_2)_5$-, $-(CH_2)_4$-diazacycloheptanylene-$(CH_2)_6$-, $-(CH_2)_4$-diazacycloheptanylene-$(CH_2)_7$-, $-(CH_2)_4$-diazacycloheptanylene-$(CH_2)_8$-, $-(CH_2)_5$-diazacycloheptanylene-$(CH_2)_1$-, $-(CH_2)_5$-diazacycloheptanylene-$(CH_2)_2$-, $-(CH_2)_5$-diazacycloheptanylene-$(CH_2)_3$-, $-(CH_2)_5$-diazacycloheptanylene-$(CH_2)_4$-, $-(CH_2)_5$-diazacycloheptanylene-$(CH_2)_5$-, $-(CH_2)_5$-diazacycloheptanylene-$(CH_2)_6$-, $-(CH_2)_5$-diazacycloheptanylene-$(CH_2)_7$-, $-(CH_2)_5$-diazacycloheptanylene-$(CH_2)_8$-, $-(CH_2)_6$-diazacycloheptanylene-$(CH_2)_1$-, $-(CH_2)_6$-diazacycloheptanylene-$(CH_2)_2$-, $-(CH_2)_6$-diazacycloheptanylene-$(CH_2)_3$-, $-(CH_2)_6$-diazacycloheptanylene-$(CH_2)_4$-, $-(CH_2)_6$-diazacycloheptanylene-$(CH_2)_5$-, $-(CH_2)_6$-diazacycloheptanylene-$(CH_2)_6$-, $-(CH_2)_6$-diazacycloheptanylene-$(CH_2)_7$-, $-(CH_2)_6$-diazacycloheptanylene-$(CH_2)_8$-, $-(CH_2)_7$-diazacycloheptanylene-$(CH_2)_1$-, $-(CH_2)_7$-diazacycloheptanylene-$(CH_2)_2$-, $-(CH_2)_7$-diazacycloheptanylene-$(CH_2)_3$-, $-(CH_2)_7$-diazacycloheptanylene-$(CH_2)_4$-, $-(CH_2)_7$-diazacycloheptanylene-$(CH_2)_8$-, $-(CH_2)_8$-diazacycloheptanylene-$CH_2$-, $-(CH_2)_8$-diazacycloheptanylene-$(CH_2)_2$-, $-(CH_2)_8$-diazacycloheptanylene-$(CH_2)_3$-, $-(CH_2)_8$-diazacycloheptanylene-$(CH_2)_4$-, $-(CH_2)_8$-diazacycloheptanylene-$(CH_2)_5$-, $-(CH_2)_8$-diazacycloheptanylene-$(CH_2)_6$-, $-(CH_2)_8$-diazacycloheptanylene-$(CH_2)_7$-, $-(CH_2)_8$-diazacycloheptanylene-$(CH_2)_8$-, $-CH_2$-diazabicyclo[2.2.1]heptanylene-$CH_2$-, $-CH_2$-diazabicyclo[2.2.1]heptanylene-$(CH_2)_2$-, $-CH_2$-diazabicyclo[2.2.1]heptanylene-$(CH_2)_3$-, $-CH_2$diazabicyclo[2.2.1]heptanylene-$(CH_2)_4$-, $-CH_2$-diazabicyclo[2.2.1] heptanylene-$(CH_2)_5$-, $-CH_2$diazabicyclo[2.2.1]heptanylene-$(CH_2)_6$-, $-CH_2$-diazabicyclo[2.2.1]heptanylene-$(CH_2)_7$-, $-CH_2$-diazabicyclo[2.2.1]heptanylene-$(CH_2)_8$-, $-(CH_2)_2$-diazabicyclo[2.2.1]heptanylene-$(CH_2)_1$-, $-(CH_2)_2$-diazabicyclo

[2.2.1]heptanylene-$(CH_2)_2$-, -$(CH_2)_2$-diazabicyclo[2.2.1]heptanylene-$(CH_2)_3$-, -$(CH_2)_2$-diazabicyclo[2.2.1]heptanylene-$(CH_2)_4$-, -$(CH_2)_2$-diazabicyclo[2.2.1]heptanylene-$(CH_2)_5$-, -$(CH_2)_2$-diazabicyclo[2.2.1]heptanylene-$(CH_2)_6$-, -$(CH_2)_2$-diazabicyclo[2.2.1]heptanylene-$(CH_2)_7$-, -$(CH_2)_2$-diazabicyclo[2.2.1]heptanylene-$(CH_2)_8$-, -$(CH_2)_3$-diazabicyclo[2.2.1]heptanylene-$CH_2$-, -$(CH_2)_3$-diazabicyclo[2.2.1]heptanylene-$(CH_2)_2$-, -$(CH_2)_3$-diazabicyclo[2.2.1]heptanylene-$(CH_2)_3$-, -$(CH_2)_3$-diazabicyclo[2.2.1]heptanylene-$(CH_2)_4$-, -$(CH_2)_3$-diazabicyclo[2.2.1]heptanylene-$(CH_2)_5$-, -$(CH_2)_3$-diazabicyclo[2.2.1]heptanylene-$(CH_2)_6$-, -$(CH_2)_3$-diazabicyclo[2.2.1]heptanylene-$(CH_2)_7$-, -$(CH_2)_3$-diazabicyclo[2.2.1]heptanylene-$(CH_2)_8$-, -$(CH_2)_4$-diazabicyclo[2.2.1]heptanylene-$CH_2$-, -$(CH_2)_4$-diazabicyclo[2.2.1]heptanylene-$(CH_2)_2$-, -$(CH_2)_4$-diazabicyclo[2.2.1]heptanylene-$(CH_2)_3$-, -$(CH_2)_4$-diazabicyclo[2.2.1]heptanylene-$(CH_2)_4$-, -$(CH_2)_4$-diazabicyclo[2.2.1]heptanylene-$(CH_2)_5$-, -$(CH_2)_4$-diazabicyclo[2.2.1]heptanylene-$(CH_2)_6$-, -$(CH_2)_4$-diazabicyclo[2.2.1]heptanylene-$(CH_2)_7$-, -$(CH_2)_4$-diazabicyclo[2.2.1]heptanylene-$(CH_2)_8$-, -$(CH_2)_5$-diazabicyclo[2.2.1]heptanylene-$(CH_2)_1$-, -$(CH_2)_5$-diazabicyclo[2.2.1]heptanylene-$(CH_2)_2$-, -$(CH_2)_5$-diazabicyclo[2.2.1]heptanylene-$(CH_2)_3$-, -$(CH_2)_5$-diazabicyclo[2.2.1]heptanylene-$(CH_2)_4$-, -$(CH_2)_5$-diazabicyclo[2.2.1]heptanylene-$(CH_2)_5$-, -$(CH_2)_5$-diazabicyclo[2.2.1]heptanylene-$(CH_2)_6$-, -$(CH_2)_5$-diazabicyclo[2.2.1]heptanylene-$(CH_2)_7$-, -$(CH_2)_5$-diazabicyclo[2.2.1]heptanylene-$(CH_2)_8$-, -$(CH_2)_6$-diazabicyclo[2.2.1]heptanylene-$(CH_2)_1$-, -$(CH_2)_6$-diazabicyclo[2.2.1]heptanylene-$(CH_2)_2$-, -$(CH_2)_6$-diazabicyclo[2.2.1]heptanylene-$(CH_2)_3$-, -$(CH_2)_6$-diazabicyclo[2.2.1]heptanylene-$(CH_2)_4$-, -$(CH_2)_6$-diazabicyclo[2.2.1]heptanylene-$(CH_2)_5$-, -$(CH_2)_6$-diazabicyclo[2.2.1]heptanylene-$(CH_2)_6$-, -$(CH_2)_6$-diazabicyclo[2.2.1]heptanylene-$(CH_2)_7$-, -$(CH_2)_6$-diazabicyclo[2.2.1]heptanylene-$(CH_2)_8$-, -$(CH_2)_7$-diazabicyclo[2.2.1]heptanylene-$(CH_2)_1$-, -$(CH_2)_7$-diazabicyclo[2.2.1]heptanylene-$(CH_2)_2$-, -$(CH_2)_7$-diazabicyclo[2.2.1]heptanylene-$(CH_2)_3$-, -$(CH_2)_7$-diazabicyclo[2.2.1]heptanylene-$(CH_2)_4$-, -$(CH_2)_7$-diazabicyclo[2.2.1]heptanylene-$(CH_2)_8$-, -$(CH_2)_8$-diazabicyclo[2.2.1]heptanylene-$CH_2$-, -$(CH_2)_8$-diazabicyclo[2.2.1]heptanylene-$(CH_2)_2$-, -$(CH_2)_8$-diazabicyclo[2.2.1]heptanylene-$(CH_2)_3$-, -$(CH_2)_8$-diazabicyclo[2.2.1]heptanylene-$(CH_2)_4$-, -$(CH_2)_8$-diazabicyclo[2.2.1]heptanylene-$(CH_2)_5$-, -$(CH_2)_8$-diazabicyclo[2.2.1]heptanylene-$(CH_2)_6$-, -$(CH_2)_8$-diazabicyclo[2.2.1]heptanylene-$(CH_2)_7$-, -$(CH_2)_8$-diazabicyclo[2.2.1]heptanylene-$(CH_2)_8$-, -$CH_2$-diazabicyclo[3.1.1]heptanylene-$CH_2$-, -$CH_2$-diazabicyclo[3.1.1]heptanylene-$(CH_2)_2$-, -$CH_2$-diazabicyclo[3.1.1]heptanylene-$(CH_2)_3$-, -$CH_2$-diazabicyclo[3.1.1]heptanylene-$(CH_2)_4$-, -$CH_2$-diazabicyclo[3.1.1]heptanylene-$(CH_2)_5$-, -$CH_2$-diazabicyclo[3.1.1]heptanylene-$(CH_2)_6$-, -$CH_2$-diazabicyclo[3.1.1]heptanylene-$(CH_2)_7$-, -$CH_2$-diazabicyclo[3.1.1]heptanylene-$(CH_2)_8$-, -$(CH_2)_2$-diazabicyclo[3.1.1]heptanylene-$(CH_2)_1$-, -$(CH_2)_2$-diazabicyclo[3.1.1]heptanylene-$(CH_2)_2$-, -$(CH_2)_2$-diazabicyclo[3.1.1]heptanylene-$(CH_2)_3$-, -$(CH_2)_2$-diazabicyclo[3.1.1]heptanylene-$(CH_2)_4$-, -$(CH_2)_2$-diazabicyclo[3.1.1]heptanylene-$(CH_2)_5$-, -$(CH_2)_2$-diazabicyclo[3.1.1]heptanylene-$(CH_2)_6$-, -$(CH_2)_2$-diazabicyclo[3. 1. 1]heptanylene-$(CH_2)_7$-, -$(CH_2)_2$-diazabicyclo[3.1.1]heptanylene-$(CH_2)_8$-, -$(CH_2)_3$-diazabicyclo[3.1.1]heptanylene-$CH_2$-, -$(CH_2)_3$-diazabicyclo[3.1.1]heptanylene-$(CH_2)_2$-, -$(CH_2)_3$-diazabicyclo[3.1.1]heptanylene-$(CH_2)_3$-, -$(CH_2)_3$-diazabicyclo[3.1.1]heptanylene-$(CH_2)_4$-, -$(CH_2)_3$-diazabicyclo[3.1.1]heptanylene-$(CH_2)_5$-, -$(CH_2)_3$-diazabicyclo[3.1.1]heptanylene-$(CH_2)_6$-, -$(CH_2)_3$-diazabicyclo[3.1.1]heptanylene-$(CH_2)_7$-, -$(CH_2)_3$-diazabicyclo[3.1.1]heptanylene-$(CH_2)_8$-, -$(CH_2)_4$-diazabicyclo[3.1.1]heptanylene-$CH_2$-, -$(CH_2)_4$-diazabicyclo[3.1.1]heptanylene-$(CH_2)_2$-, -$(CH_2)_4$-diazabicyclo[3.1.1]heptanylene-$(CH_2)_3$-, -$(CH_2)_4$-diazabicyclo[3.1.1]heptanylene-$(CH_2)_4$-, -$(CH_2)_4$-diazabicyclo[3.1.1]heptanylene-$(CH_2)_5$-, -$(CH_2)_4$-diazabicyclo[3.1.1]heptanylene-$(CH_2)_6$-, -$(CH_2)_4$-diazabicyclo[3.1.1]heptanylene-$(CH_2)_7$-, -$(CH_2)_4$-diazabicyclo[3.1.1]heptanylene-$(CH_2)_8$-, -$(CH_2)_5$-diazabicyclo[3.1.1]heptanylene-$(CH_2)_1$-, -$(CH_2)_5$-diazabicyclo[3.1.1]heptanylene-$(CH_2)_2$-, -$(CH_2)_5$-diazabicyclo[3.1.1]heptanylene-$(CH_2)_3$-, -$(CH_2)_5$-diazabicyclo[3.1.1]heptanylene-$(CH_2)_4$-, -$(CH_2)_5$-diazabicyclo[3.1.1]heptanylene-$(CH_2)_5$-, -$(CH_2)_5$-diazabicyclo[3.1.1]heptanylene-$(CH_2)_6$-, -$(CH_2)_5$-diazabicyclo[3.1.1]heptanylene-$(CH_2)_7$-, -$(CH_2)_5$-diazabicyclo[3.1.1 ]heptanylene-$(CH_2)_8$-, -$(CH_2)_6$-diazabicyclo[3.1.1]heptanylene-$(CH_2)_1$-, -$(CH_2)_6$-diazabicyclo[3.1.1]heptanylene-$(CH_2)_2$-, -$(CH_2)_6$-diazabicyclo[3.1.1]heptanylene-$(CH_2)_3$-, -$(CH_2)_6$-diazabicyclo[3.1.1]heptanylene-$(CH_2)_4$-, -$(CH_2)_6$-diazabicyclo[3. 1.1]heptanylene-$(CH_2)_5$-, -$(CH_2)_6$-diazabicyclo[3.1.1]heptanylene-$(CH_2)_6$-, -$(CH_2)_6$-diazabicyclo[3.1.1]heptanylene-$(CH_2)_7$-, -$(CH_2)_6$-diazabicyclo[3.1.1]heptanylene-$(CH_2)_8$-, -$(CH_2)_7$-diazabicyclo[3.1.1]heptanylene-$(CH_2)_1$-, -$(CH_2)_7$-diazabicyclo[3.1.1]heptanylene-$(CH_2)_2$-, -$(CH_2)_7$-diazabicyclo[3.1.1]heptanylene-$(CH_2)_3$-, -$(CH_2)_7$-diazabicyclo[3.1.1]heptanylene-$(CH_2)_4$-, -$(CH_2)_7$-diazabicyclo[3.1.1]heptanylene-$(CH_2)_8$-, -$(CH_2)_8$-diazabicyclo[3.1.1]heptanylene-$CH_2$-, -$(CH_2)_8$-diazabicyclo[3.1.1]heptanylene-$(CH_2)_2$-, -$(CH_2)_8$-diazabicyclo[3.1.1]heptanylene-$(CH_2)_3$-, -$(CH_2)_8$-diazabicyclo[3.1.1]heptanylene-$(CH_2)_4$-, -$(CH_2)_8$-diazabicyclo[3.1.1]heptanylene-$(CH_2)_5$-, -$(CH_2)_8$-diazabicyclo[3.1.1]heptanylene-$(CH_2)_6$-, -$(CH_2)_8$-diazabicyclo[3.1.1]heptanylene-$(CH_2)_7$-, -$(CH_2)_8$-diazabicyclo[3.1.1]heptanylene-$(CH_2)_8$-, -$CH_2$-diazabicyclo[3.2.1]octylene-$CH_2$-, -$CH_2$-diazabicyclo[3.2.1]octylene-$(CH_2)_2$-, -$CH_2$-diazabicyclo[3.2.1]octylene-$(CH_2)_3$-, -$CH_2$-diazabicyclo[3.2.1]octylene-$(CH_2)_4$-, -$CH_2$-diazabicyclo[3.2.1]octylene-$(CH_2)_5$-, -$CH_2$-diazabicyclo[3.2.1]octylene-$(CH_2)_6$-, -$CH_2$-diazabicyclo[3.2.1]octylene-$(CH_2)_7$-, -$CH_2$-diazabicyclo[3.2.1]octylene-$(CH_2)_8$-, -$(CH_2)_2$-diazabicyclo[3.2.1]octylene-$(CH_2)_1$-, -$(CH_2)_2$-diazabicyclo[3.2.1]octylene-$(CH_2)_2$-, -$(CH_2)_2$-diazabicyclo[3.2.1]octylene-$(CH_2)_3$-, -$(CH_2)_2$-diazabicyclo[3.2.1]octylene-$(CH_2)_4$-, -$(CH_2)_2$-diazabicyclo[3.2.1]octylene-$(CH_2)_5$-, -$(CH_2)_2$-diazabicyclo[3.2.1]octylene-$(CH_2)_6$-, -$(CH_2)_2$-diazabicyclo[3.2.1]octylene-$(CH_2)_7$-, -$(CH_2)_2$-diazabicyclo[3.2.1]octylene-$(CH_2)_8$-, -$(CH_2)_3$-diazabicyclo[3.2.1]octylene-$CH_2$-, -$(CH_2)_3$-diazabicyclo[3.2.1]octylene-$(CH_2)_2$-, -$(CH_2)_3$-diazabicyclo[3.2.1]octylene-$(CH_2)_3$-, -$(CH_2)_3$-diazabicyclo[3.2.1]octylene-$(CH_2)_4$-, -$(CH_2)_3$-diazabicyclo[3.2.1]octylene-$(CH_2)_5$-, -$(CH_2)_3$-diazabicyclo[3.2.1]octylene-$(CH_2)_6$-, -$(CH_2)_3$-diazabicyclo[3.2.1]octylene-$(CH_2)_7$-, -$(CH_2)_3$-diazabicyclo[3.2.1]octylene-$(CH_2)_8$-, -$(CH_2)_4$-diazabicyclo[3.2.1]octylene-$CH_2$-, -$(CH_2)_4$-diazabicyclo[3.2.1]octylene-$(CH_2)_2$-, -$(CH_2)_4$-diazabicyclo[3.2.1]octylene-$(CH_2)_3$-, -$(CH_2)_4$-dia-

zabicyclo[3.2.1]octylene-$(CH_2)_4$-, -$(CH_2)_4$-diazabicyclo[3.2.1]octylene-$(CH_2)_5$-, -$(CH_2)_4$-diazabicyclo[3.2.1]octylene-$(CH_2)_6$-, -$(CH_2)_4$-diazabicyclo[3.2.1]octylene-$(CH_2)_7$-, -$(CH_2)_4$-diazabicyclo[3.2.1]octylene-$(CH_2)_8$-, -$(CH_2)_5$-diazabicyclo[3.2.1]octylene-$(CH_2)_1$-, -$(CH_2)_5$-diazabicyclo[3.2.1]octylene-$(CH_2)_2$-, -$(CH_2)_5$-diazabicyclo[3.2.1]octylene-$(CH_2)_3$-, -$(CH_2)_5$-diazabicyclo[3.2.1]octylene-$(CH_2)_4$-, -$(CH_2)_5$-diazabicyclo[3.2.1]octylene-$(CH_2)_5$-, -$(CH_2)_5$-diazabicyclo[3.2.1]octylene-$(CH_2)_6$-, -$(CH_2)_5$-diazabicyclo[3.2.1]octylene-$(CH_2)_7$-, -$(CH_2)_5$-diazabicyclo[3.2.1]octylene-$(CH_2)_8$-, -$(CH_2)_6$-diazabicyclo[3.2.1]octylene-$(CH_2)_1$-, -$(CH_2)_6$-diazabicyclo[3.2.1]octylene-$(CH_2)_2$-, -$(CH_2)_6$-diazabicyclo[3.2.1]octylene-$(CH_2)_3$-, -$(CH_2)_6$-diazabicyclo[3.2.1]octylene-$(CH_2)_4$-, -$(CH_2)_6$-diazabicyclo[3.2.1]octylene-$(CH_2)_5$-, -$(CH_2)_6$-diazabicyclo[3.2.1]octylene-$(CH_2)_6$-, -$(CH_2)_6$-diazabicyclo[3.2.1]octylene-$(CH_2)_7$-, -$(CH_2)_6$-diazabicyclo[3.2.1]octylene-$(CH_2)_8$-, -$(CH_2)_7$-diazabicyclo[3.2.1]octylene-$(CH_2)_1$-, -$(CH_2)_7$-diazabicyclo[3.2.1]octylene-$(CH_2)_2$-, -$(CH_2)_7$-diazabicyclo[3.2.1]octylene-$(CH_2)_3$-, -$(CH_2)_7$-diazabicyclo[3.2.1]octylene-$(CH_2)_4$-, -$(CH_2)_7$-diazabicyclo[3.2.1]octylene-$(CH_2)_8$-, -$(CH_2)_8$-diazabicyclo[3.2.1]octylene-$CH_2$-, -$(CH_2)_8$-diazabicyclo[3.2.1]octylene-$(CH_2)_2$-, -$(CH_2)_8$-diazabicyclo[3.2.1]octylene-$(CH_2)_3$-, -$(CH_2)_8$-diazabicyclo[3.2.1]octylene-$(CH_2)_4$-, -$(CH_2)_8$-diazabicyclo[3.2.1]octylene-$(CH_2)_5$-, -$(CH_2)_8$-diazabicyclo[3.2.1]octylene-$(CH_2)_6$-, -$(CH_2)_8$-diazabicyclo[3.2.1]octylene-$(CH_2)_7$-, -$(CH_2)_8$-diazabicyclo[3.2.1]octylene-$(CH_2)_8$-, -$CH_2$-diazabicyclo[2.2.2]octylene-$CH_2$-, -$CH_2$-diazabicyclo [2.2.2]octylene-$(CH_2)_2$-, -$CH_2$-diazabicyclo[2.2.2]octylene-$(CH_2)_3$-, -$CH_2$-diazabicyclo[2.2.2]octylene-$(CH_2)_4$-, -$CH_2$-diazabicyclo[2.2.2]octylene-$(CH_2)_5$-, -$CH_2$-diazabicyclo[2.2.2]octylene-$(CH_2)_6$-, -$CH_2$-diazabicyclo[2.2.2]octylene-$(CH_2)_7$-, -$CH_2$-diazabicyclo[2.2.2]octylene-$(CH_2)_8$-, -$(CH_2)_2$-diazabicyclo[2.2.2]octylene-$(CH_2)_1$-, -$(CH_2)_2$-diazabicyclo[2.2.2]octylene-$(CH_2)_2$-, -$(CH_2)_2$-diazabicyclo[2.2.2]octylene-$(CH_2)_3$-, -$(CH_2)_2$-diazabicyclo[2.2.2]octylene-$(CH_2)_4$-, -$(CH_2)_2$-diazabicyclo[2.2.2]octylene-$(CH_2)_5$-, -$(CH_2)_2$-diazabicyclo[2.2.2]octylene-$(CH_2)_6$-, -$(CH_2)_2$-diazabicyclo[2.2.2]octylene-$(CH_2)_7$-, -$(CH_2)_2$-diazabicyclo[2.2.2]octylene-$(CH_2)_8$-, -$(CH_2)_3$-diazabicyclo[2.2.2]octylene-$CH_2$-, -$(CH_2)_3$-diazabicyclo[2.2.2]octylene-$(CH_2)_2$-, -$(CH_2)_3$-diazabicyclo[2.2.2]octylene-$(CH_2)_3$-, -$(CH_2)_3$-diazabicyclo[2.2.2]octylene-$(CH_2)_4$-, -$(CH_2)_3$-diazabicyclo[2.2.2]octylene-$(CH_2)_5$-, -$(CH_2)_3$-diazabicyclo[2.2.2]octylene-$(CH_2)_6$-, -$(CH_2)_3$-diazabicyclo[2.2.2] octylene-$(CH_2)_7$-, -$(CH_2)_3$-diazabicyclo[2.2.2]octylene-$(CH_2)_8$-, -$(CH_2)_4$-diazabicyclo[2.2.2]octylene-$CH_2$-, -$(CH_2)_4$-diazabicyclo[2.2.2]octylene-$(CH_2)_2$-, -$(CH_2)_4$-diazabicyclo[2.2.2]octylene-$(CH_2)_3$-, -$(CH_2)_4$-diazabicyclo[2.2.2]octylene-$(CH_2)_4$-, -$(CH_2)_4$-diazabicyclo[2.2.2]octylene-$(CH_2)_5$-, -$(CH_2)_4$-diazabicyclo[2.2.2]octylene-$(CH_2)_6$-, -$(CH_2)_4$-diazabicyclo[2.2.2]octylene-$(CH_2)_7$-, -$(CH_2)_4$-diazabicyclo[2.2.2]octylene-$(CH_2)_8$-, -$(CH_2)_5$-diazabicyclo[2.2.2]octylene-$(CH_2)_1$-, -$(CH_2)_5$-diazabicyclo[2.2.2]octylene-$(CH_2)_2$-, -$(CH_2)_5$-diazabicyclo[2.2.2]octylene-$(CH_2)_3$-, -$(CH_2)_5$-diazabicyclo[2.2.2]octylene-$(CH_2)_4$-, -$(CH_2)_5$-diazabicyclo[2.2.2]octylene-$(CH_2)_5$-, -$(CH_2)_5$-diazabicyclo[2.2.2]octylene-$(CH_2)_6$-, -$(CH_2)_5$-diazabicyclo[2.2.2]octylene-$(CH_2)_7$-, -$(CH_2)_5$-diazabicyclo[2.2.2]octylene-$(CH_2)_8$-, -$(CH_2)_6$-diazabicyclo[2.2.2]octylene-$(CH_2)_1$-, -$(CH_2)_6$-diazabicyclo[2.2.2]octylene-$(CH_2)_2$-, -$(CH_2)_6$-diazabicyclo[2.2.2]octylene-$(CH_2)_3$-, -$(CH_2)_6$-diazabicyclo[2.2.2]octylene-$(CH_2)_4$-, -$(CH_2)_6$-diazabicyclo[2.2.2]octylene-$(CH_2)_5$-, -$(CH_2)_6$-diazabicyclo[2.2.2]octylene-$(CH_2)_6$-, -$(CH_2)_6$-diazabicyclo[2.2.2]octylene-$(CH_2)_7$-, -$(CH_2)_6$-diazabicyclo[2.2.2] octylene-$(CH_2)_8$-, -$(CH_2)_7$-diazabicyclo[2.2.2]octylene-$(CH_2)_1$-, -$(CH_2)_7$-diazabicyclo[2.2.2]octylene-$(CH_2)_2$-, -$(CH_2)_7$-diazabicyclo[2.2.2]octylene-$(CH_2)_3$-, -$(CH_2)_7$-diazabicyclo[2.2.2]octylene-$(CH_2)_4$-, -$(CH_2)_7$-diazabicyclo[2.2.2]octylene-$(CH_2)_8$-, -$(CH_2)_8$-diazabicyclo[2.2.2]octylene-$CH_2$-, -$(CH_2)_8$-diazabicyclo[2.2.2]octylene-$(CH_2)_2$-, -$(CH_2)_8$-diazabicyclo[2.2.2]octylene-$(CH_2)_3$-, -$(CH_2)_8$-diazabicyclo[2.2.2]octylene-$(CH_2)_4$-, -$(CH_2)_8$-diazabicyclo[2.2.2]octylene-$(CH_2)_5$-, -$(CH_2)_8$-diazabicyclo[2.2.2]octylene-$(CH_2)_6$-, -$(CH_2)_8$-diazabicyclo[2.2.2]octylene-$(CH_2)_7$-, or -$(CH_2)_8$-diazabicyclo [2.2.2]octylene-$(CH_2)_8$-;

wherein the propylene, the piperidinylene, the piperazinylene, the diazacycloheptanylene, the diazabicyclo[2.2.1] heptanylene, the diazabicyclo[3.1.1]heptanylene, the diazabicyclo[3.2.1]octylene, the diazabicyclo[2.2.2]octylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof; and

each $R_{g11}$ independently represents H or $C_{1-3}$ alkyl.

In some embodiments of the compound of Formula (I-3), $L_2$ represents the structure of the following formula:

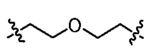 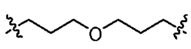 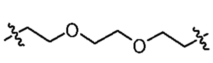 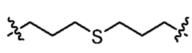 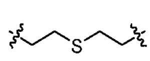 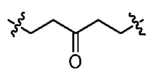

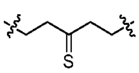 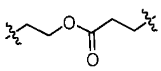 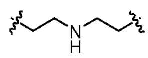 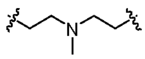 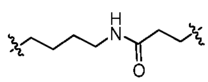 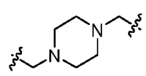 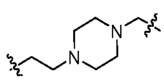

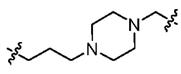 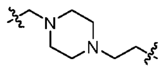 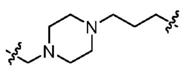 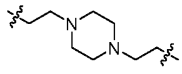 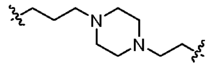 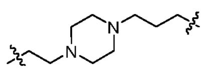

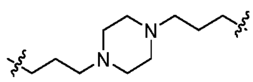 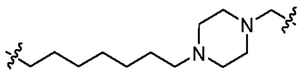 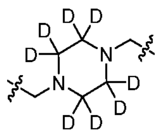 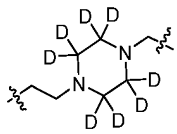 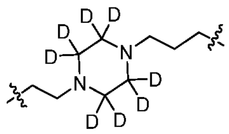

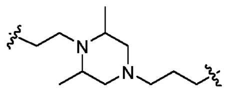 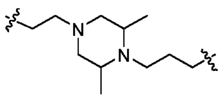 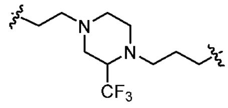 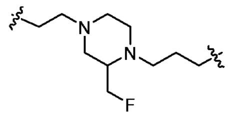 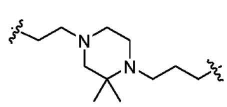

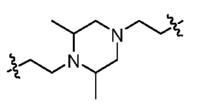 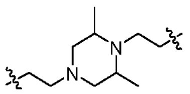 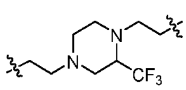 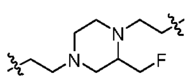 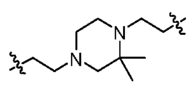 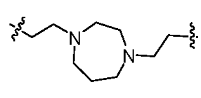

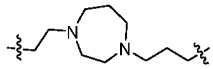 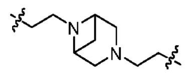 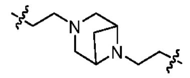 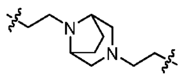 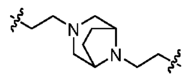 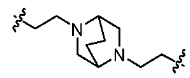

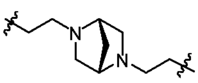 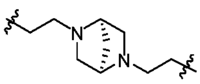 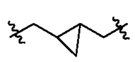 or 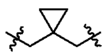

In some embodiments of the compound of Formula (I-3), $R_{b1}$ represents:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, 2-oxobicyclo[2.2.1]heptanyl or bicyclo[2.2.1]heptenyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 3-azaspiro[5.5]undecyl or 7-azaspiro[3.5]nonyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

phenyl or naphthyl, with each group being optionally substituted with one or more (e.g., 1-4, 1-3 or 1-2, or 1)

substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl and imidazo[2,1-b]thiazolyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

In some embodiments of the compound of Formula (I-3), $R_{b1}$ represents:

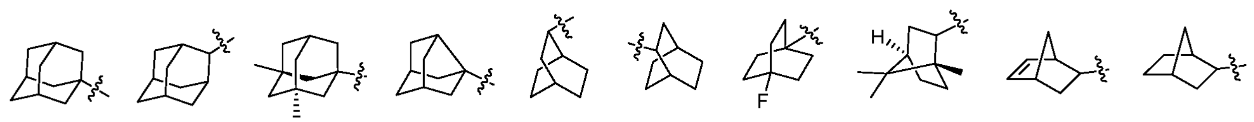

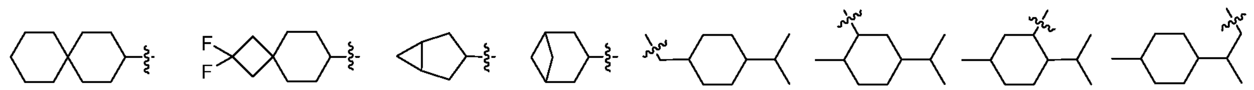

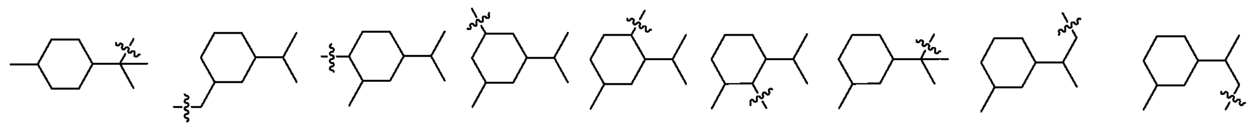

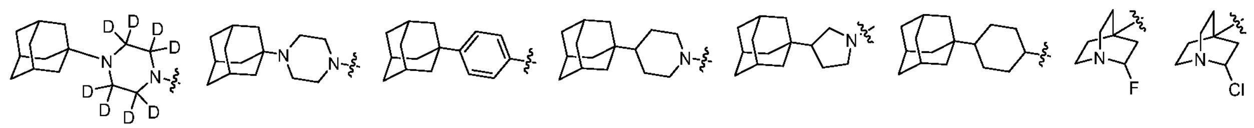

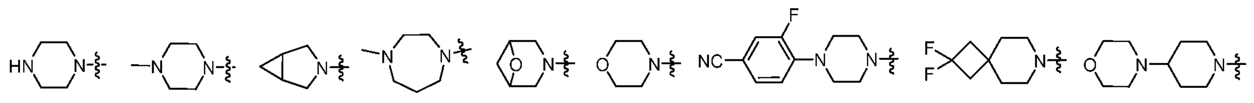

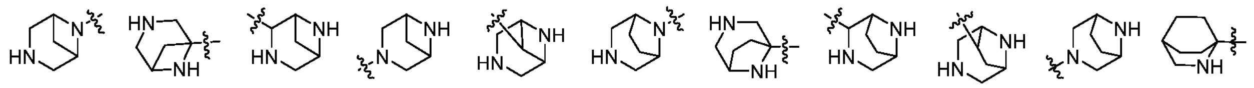

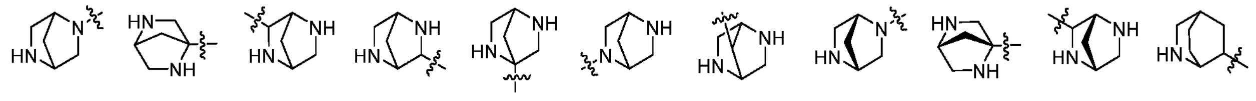

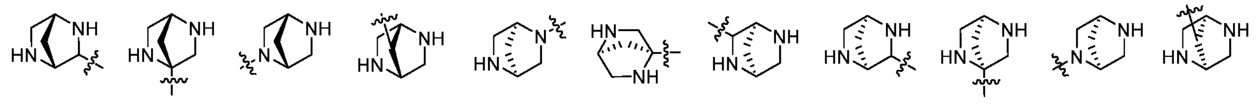

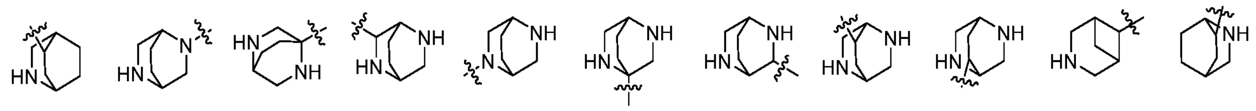

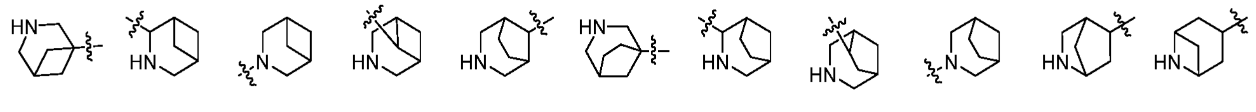

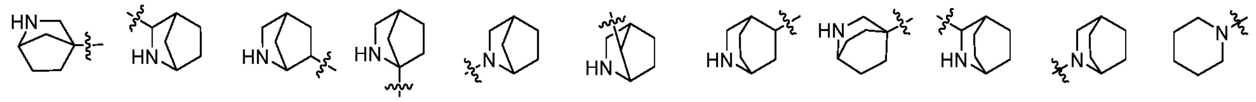

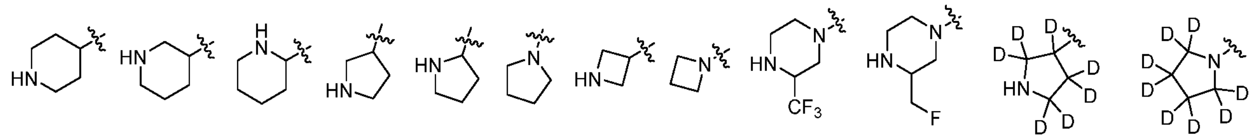

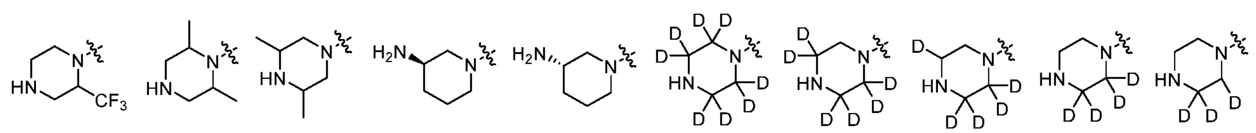

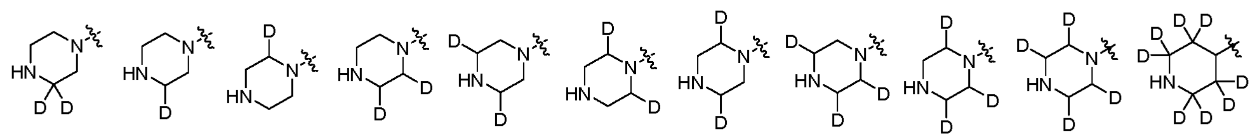

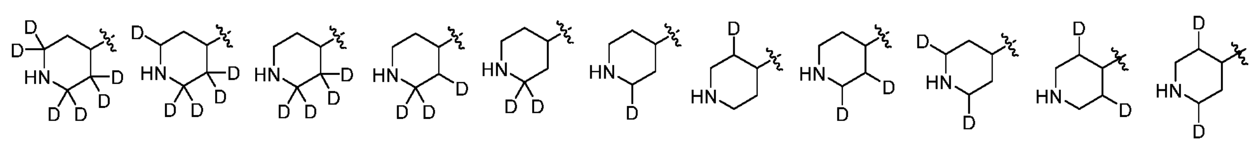

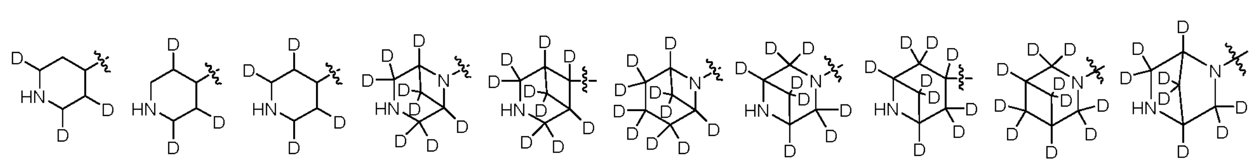

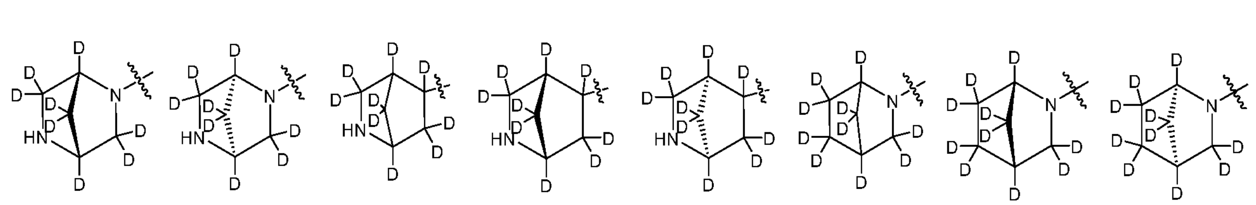

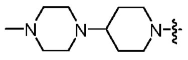 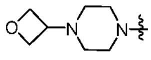 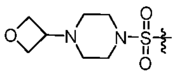 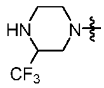 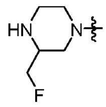 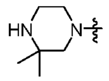 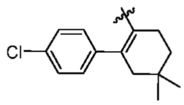 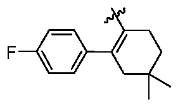

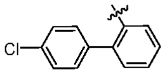 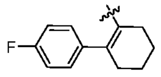 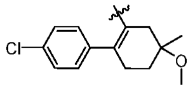 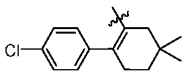 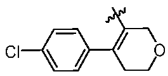 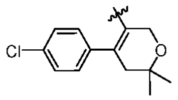 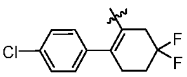

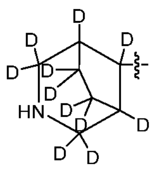 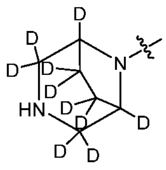 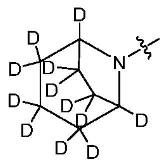 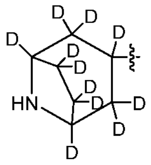 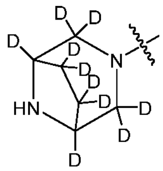 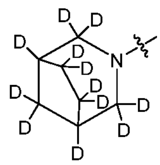 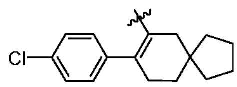

or

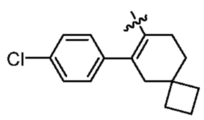

In some embodiments, the compound of Formula (I) is also of Formula (I-4):

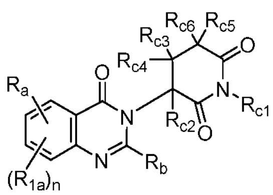

Formula (I-4)

wherein $R_{c1}$, $R_{c2}$, $R_{c3}$, $R_{c4}$, $R_{c5}$, $R_{c6}$, $R_b$, $(R_{1a})_n$, $R_a$, $R_{1a}$ and n are as defined in the compound of Formula (I) above and the embodiments thereof;

wherein $R_a$ represents the following formula:

$$R_{a1}\text{-}X_2\text{-}L_1\text{-}X_1\text{-} ,$$

wherein $X_1$ represents $N(R_3)$, O or S, wherein $R_3$ represents H or $C_{1\text{-}3}$ alkyl;
$L_1$ represents optionally substituted linear or branched $C_{4\text{-}60}$ alkylene (e.g., $C_4$-$C_{30}$ alkylene, $C_4$-$C_{29}$ alkylene, $C_4$-$C_{28}$ alkylene, $C_4$-$C_{27}$ alkylene, $C_4$-$C_{26}$ alkylene, $C_4$-$C_{25}$ alkylene, $C_4$-$C_{24}$ alkylene, $C_4$-$C_{23}$ alkylene, $C_4$-$C_{22}$ alkylene, $C_4$-$C_{21}$ alkylene, $C_4$-$C_{20}$ alkylene, $C_4$-$C_{19}$ alkylene, $C_4$-$C_{18}$ alkylene, $C_4$-$C_{17}$ alkylene, $C_4$-$C_{16}$ alkylene, $C_4$-$C_{15}$ alkylene, $C_4$-$C_{14}$ alkylene, $C_4$-$C_{13}$ alkylene, $C_4$-$C_{12}$ alkylene, $C_4$-$C_{11}$ alkylene, $C_4$-$C_{10}$ alkylene, $C_4$-$C_9$ alkylene, $C_4$-$C_8$ alkylene, $C_4$-$C_7$ alkylene, $C_4$-$C_6$ alkylene, or $C_4$-$C_5$ alkylene);
$X_2$ represents $N(R_4)$, C(O)O, OC(O), C(O)NH, NHC(O), C(O), O, C(S), S, S(O), $S(O)_2$, $S(O)_2NH$, $NHS(O)_2$, $S(O)_2O$, or $OS(O)_2$, wherein $R_4$ represents H or $C_{1\text{-}3}$ alkyl, or $X_2$ represents a bond; and
$R_{a1}$ represents optionally substituted heterocyclyl (e.g., optionally substituted 4- to 20-membered heterocyclylene, or optionally substituted 5- to 15-membered heterocyclylene);
and
$R_b$ represents $C_{1\text{-}60}$ alkyl (e.g., $C_1$-$C_{30}$ alkylene, $C_1$-$C_{29}$ alkylene, $C_1$-$C_{28}$ alkylene, $C_1$-$C_{27}$ alkylene, $C_1$-$C_{26}$ alkylene, $C_1$-$C_{25}$ alkylene, $C_1$-$C_{24}$ alkylene, $C_1$-$C_{23}$ alkylene, $C_1$-$C_{22}$ alkylene, $C_1$-$C_{21}$ alkylene, $C_1$-$C_{20}$ alkylene, $C_1$-$C_{19}$ alkylene, $C_1$-$C_{18}$ alkylene, $C_1$-$C_{17}$ alkylene, $C_1$-$C_{16}$ alkylene, $C_1$-$C_{15}$ alkylene, $C_1$-$C_{14}$ alkylene, $C_1$-$C_{13}$ alkylene, $C_1$-$C_{12}$ alkylene, $C_1$-$C_{11}$ alkylene, $C_1$-$C_{10}$ alkylene, $C_1$-$C_9$ alkylene, $C_1$-$C_8$ alkylene, $C_1$-$C_7$ alkylene, $C_1$-$C_6$ alkylene, $C_1$-$C_5$ alkylene, $C_1$-$C_4$ alkylene, $C_1$-$C_3$ alkylene, or $C_1$-$C_2$ alkylene) optionally substituted with D or halogen, or

$R_b$ represents the following formula:

$R_{b1}$-$X_4$-$L_2$-,

wherein $L_2$ represents:

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene), or
optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{12}$ and/or one or more (e.g., 1-20, or 1-15, 1-10, 1-9, 1-8, 1-7, 1-6, 1-5, 1-4, 1-3 or 1-2, or 1) groups $R_{13}$ and/or any combination of one or more (e.g., 1-20, or 1-15, 1-10, 1-9, 1-8, 1-7, 1-6, 1-5, 1-4, 1-3 or 1-2, or 1) groups $R_{12}$ and $R_{13}$ inserted into its backbone carbon chain, wherein carbon-carbon bond between one or more pairs (e.g., 1-20, or 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, N($R_{14}$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of optionally substituted cycloalkylene (e.g., optionally substituted $C_{3-20}$cycloalkylene, or optionally substituted $C_{3-15}$cycloalkylene), optionally substituted heterocyclylene (e.g., optionally substituted 4- to 20-membered heterocyclylene, or optionally substituted 5- to 15-membered heterocyclylene), optionally substituted arylene (e.g., optionally substituted $C_{6-10}$ arylene), optionally substituted heteroarylene (e.g., optionally substituted 5- to 20-membered heteroarylene, or optionally substituted 5- to 15-membered heteroarylene), alkenylene (e.g., $C_{2-6}$ alkenylene), alkynylene (e.g., $C_{2-6}$ alkynylene) or any combination thereof, wherein the cycloalkylene, the heterocyclylene, arylene and the heteroarylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof;

$X_4$ represents N($R_{15}$), C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted cycloalkylene (e.g., optionally substituted $C_{3-20}$cycloalkylene, or optionally substituted $C_{3-15}$cycloalkylene), optionally substituted arylene (e.g., optionally substituted $C_{6-10}$ arylene), optionally substituted heterocyclylene (e.g., optionally substituted 4- to 20-membered heterocyclylene, or optionally substituted 5-to 15-membered heterocyclylene), optionally substituted heteroarylene (e.g., optionally substituted 5- to 20-membered heteroarylene, or optionally substituted 5- to 15-membered heteroarylene), triazolylene-NH-, or -NH-triazolylene, wherein $R_{15}$ represents H or $C_{1-3}$ alkyl, or $X_4$ represents a bond; and
$R_{b1}$ represents optionally substituted cycloalkyl (e.g., optionally substituted $C_{3-20}$cycloalkyl, or optionally substituted $C_{3-15}$cycloalkyl), optionally substituted aryl (e.g., optionally substituted $C_{6-20}$ aryl or optionally substituted $C_{6-10}$ aryl), optionally substituted heterocyclyl (e.g., optionally substituted 4- to 20-membered heterocyclyl, or optionally substituted 5- to 15-membered heterocyclyl) or optionally substituted heteroaryl (e.g., optionally substituted 5- to 20-membered heteroaryl, or optionally substituted 5- to 15-membered heteroaryl).

In some embodiments of the compound of Formula (I-4), $R_a$ represents the following formula:

$R_{a1}$-$X_2$-$L_1$-$X_1$-,

wherein $X_1$ represents $N(R_3)$, O or S, wherein $R_3$ represents H or $C_{1-3}$ alkyl;

$L_1$ represents linear or branched $C_{4-60}$ alkylene (e.g., $C_4$-$C_{30}$ alkylene, $C_4$-$C_{29}$ alkylene, $C_4$-$C_{28}$ alkylene, $C_4$-$C_{27}$ alkylene, $C_4$-$C_{26}$ alkylene, $C_4$-$C_{25}$ alkylene, $C_4$-$C_{24}$ alkylene, $C_4$-$C_{23}$ alkylene, $C_4$-$C_{22}$ alkylene, $C_4$-$C_{21}$ alkylene, $C_4$-$C_{20}$ alkylene, $C_4$-$C_{19}$ alkylene, $C_4$-$C_{18}$ alkylene, $C_4$-$C_{17}$ alkylene, $C_4$-$C_{16}$ alkylene, $C_4$-$C_{15}$ alkylene, $C_4$-$C_{14}$ alkylene, $C_4$-$C_{13}$ alkylene, $C_4$-$C_{12}$ alkylene, $C_4$-$C_{11}$ alkylene, $C_4$-$C_{10}$ alkylene, $C_4$-$C_9$ alkylene, $C_4$-$C_8$ alkylene, $C_4$-$C_7$ alkylene, $C_4$-$C_6$ alkylene, or $C_4$-$C_5$ alkylene) which is optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-4}$ alkyl, such as methyl, $CD_3$, ethyl, propyl, isopropyl, butyl, sec-butyl or tert-butyl), hydroxy, amino, mercapto, nitro, halogen, cyano, halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), $C_{1-6}$ alkoxy (e.g., $C_{1-3}$ alkoxy, such as methoxy, ethoxy, or propoxy), or any combination thereof;

$X_2$ represents a bond, or $X_2$ represents $N(R_4)$, C(O)O, OC(O), C(O)NH, NHC(O), C(O), O, C(S), S, S(O), $S(O)_2$, $S(O)_2NH$, $NHS(O)_2$, $S(O)_2O$, or $OS(O)_2$, wherein $R_4$ represents H or $C_{1-3}$ alkyl; and

$R_{a1}$ represents optionally substituted 4- to 20-membered heterocyclyl, and the 4- to 20-membered heterocyclyl is optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-4}$ alkyl, such as methyl, $CD_3$, ethyl, propyl, isopropyl, butyl, sec-butyl or tert-butyl), optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-4}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-) (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof.

In some embodiments of the compound of Formula (I-4), $R_{a1}$ represents:
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl, diazacyclooctyl, 1,3-diazacycloheptanyl, 1,4-diazacycloheptanyl, 4,5-diazacycloheptanyl, 5- to 15-memberedbridged heterocyclyl (e.g., 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, diazabicyclo[3.2.1]octyl, diazabicyclo[2.2.2]octyl, 3,8-diazabicyclo[3.2.1]octan-3-yl, and 2,5-diazabicyclo[2.2.2]octan-2-yl), or 5- to 15-memberedazaspirocycloalkyl (e.g., 3-azaspiro[5.5]undecylene and 7-azaspiro[3.5]nonylene), with each group being independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of: D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

In some embodiments of the compound of Formula (I-4), $R_{a1}$ represents:

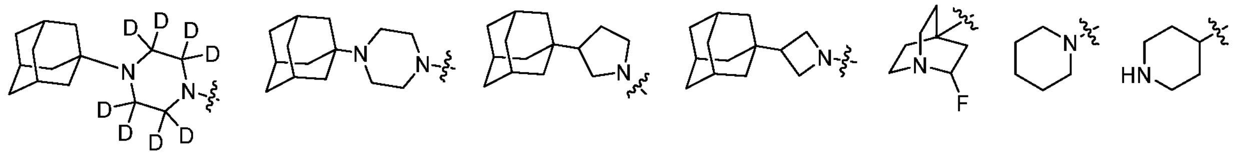

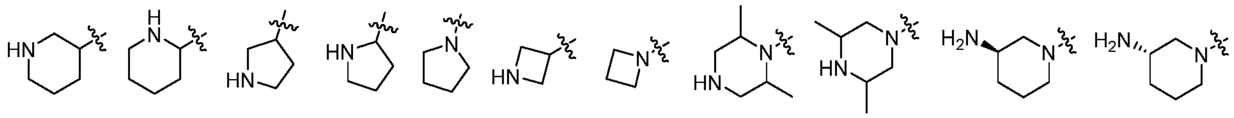

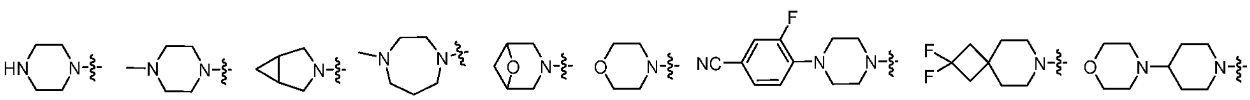

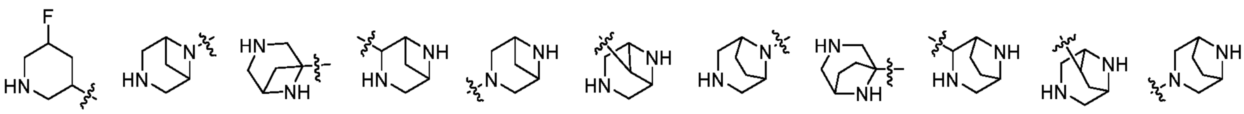

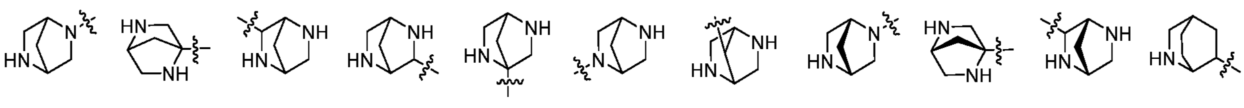

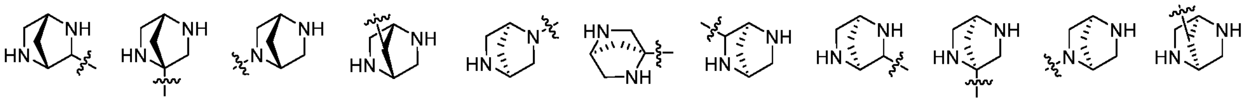

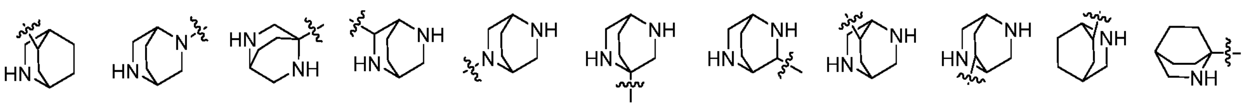

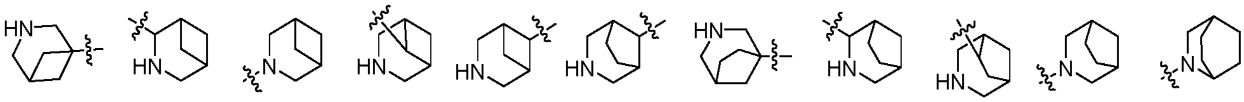

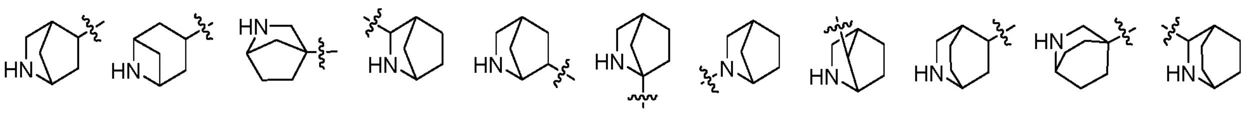

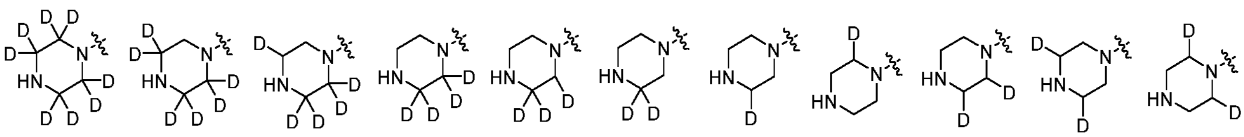

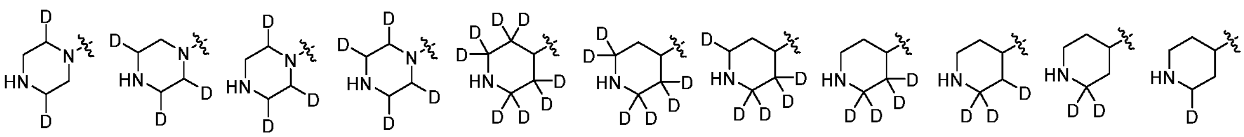

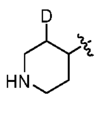 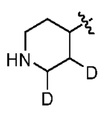 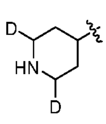 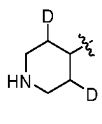 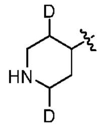 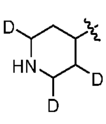 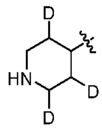 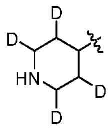 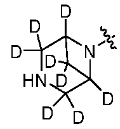 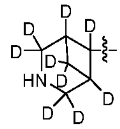 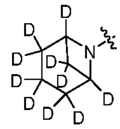

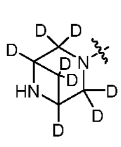 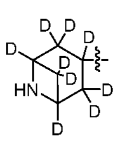 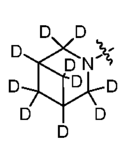 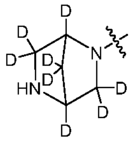 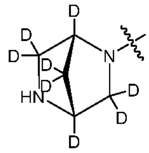 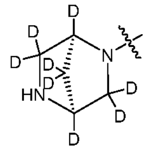 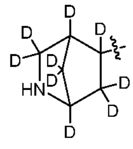 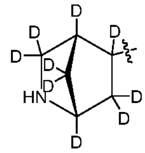 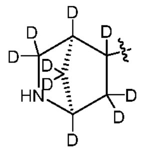

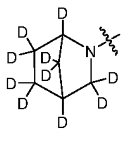 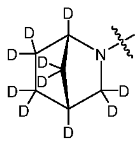 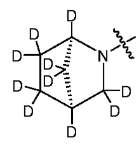 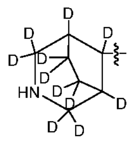 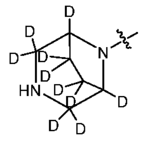 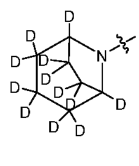 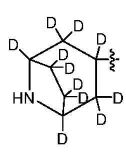 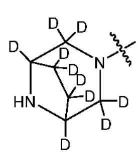 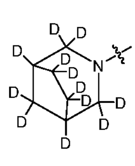

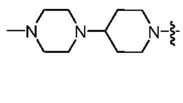 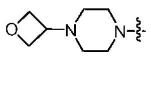 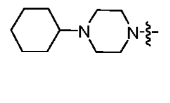 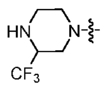 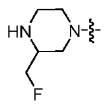 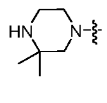 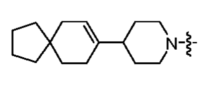 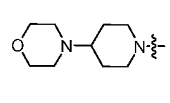

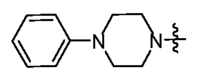 or 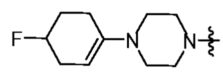

In some embodiments of the compound of Formula (I-4), $L_1$ represents the structure of the following formula: ##-$C_{4-30}$ alkylene-, wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the $C_{4-30}$ alkylene is optionally replaced with a substituent selected from the group consisting of: D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy or any combination thereof, and symbol ## indicates the point of attachment to $X_1$.

In some embodiments of the compound of Formula (I-4), $L_1$ represents the following group: ##-$(CH_2)_4$-, ##-$(CH_2)_5$-, ##-$(CH_2)_2$-$CF_2$-$(CH_2)_2$-, ##-$(CH_2)_6$-, ##-$(CH_2)_7$-, ##-$(CH_2)_8$-, ##-$(CH_2)_9$-, ##-$(CH_2)_{10}$-, ##-$(CH_2)_{11}$-, ##-$(CH_2)_{12}$-, ##-$(CH_2)_{13}$-, ##-$(CH_2)_{14}$-, ##-$(CH_2)_{15}$-, ##-$(CH_2)_{16}$-, ##-$(CH_2)_{17}$-, ##-$(CH_2)_{18}$-, ##-$(CH_2)_{19}$-, ##-$(CH_2)_{20}$-, ##-$(CH_2)_{21}$-, ##-$(CH_2)_{22}$-, ##-$(CH_2)_{25}$-, or ##-$(CH_2)_{30}$-; wherein a hydrogen atom of one or more (e.g., 1-20, or 1-15, 1-10, 1-9, 1-8, 1-7, 1-6, 1-5, 1-4, 1-3 or 1-2, or 1) $CH_2$ of the group is optionally replaced with a substituent selected from the group consisting of: D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy or any combination thereof, and symbol ## indicates the point of attachment to $X_1$.

In some embodiments of the compound of Formula (I-4), $R_b$ represents $C_{1-60}$ alkyl optionally substituted with D or halogen.

In some embodiments of the compound of Formula (I-4), $R_b$ represents the following group: $CH_3$, $CF_3$, $CD_3$, $CH_2CH_3$, -$(CH_2)_2$-$CH_3$, -$(CH_2)_3$-$CH_3$, -$(CH_2)_4$-$CH_3$, -$(CH_2)_5$-$CH_3$, -$(CH_2)_6$-$CH_3$,-$(CH_2)_7$-$CH_3$, -$(CH_2)_8$-$CH_3$, -$(CH_2)_9$-$CH_3$, -$(CH_2)_{10}$-$CH_3$, -$(CH_2)_{11}$-$CH_3$, -$(CH_2)_{12}$-$CH_3$, -$(CH_2)_{13}$-$CH_3$,-$(CH_2)_{14}$-$CH_3$, -$(CH_2)_{15}$-$CH_3$, -$(CH_2)_{16}$-$CH_3$, -$(CH_2)_{17}$-$CH_3$, -$(CH_2)_{18}$-$CH_3$, -$(CH_2)_{19}$-$CH_3$, -$(CH_2)_{20}$-$CH_3$, -$(CH_2)_{21}$-$CH_3$, -$(CH_2)_{22}$-$CH_3$, -$(CH_2)_{23}$-$CH_3$, -$(CH_2)_{24}$-$CH_3$, -$(CH_2)_{25}$-$CH_3$, -$(CH_2)_{26}$-$CH_3$, -$(CH_2)_{27}$-$CH_3$, -$(CH_2)_{28}$-$CH_3$, or -$(CH_2)_{29}$-$CH_3$.

In some embodiments of the compound of Formula (I-4), $R_b$ represents the following formula:

$$R_{b1}\text{-}X_4\text{-}L_2\text{-} ,$$

wherein $L_2$ represents

linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) optionally substituted with one or more (e.g., 1-20, or 1-15, 1-10, 1-9, 1-8, 1-7, 1-6, 1-5, 1-4, 1-3 or 1-2, or 1) substituents

selected from the group consisting of D, $C_{1-4}$ alkyl, halogen, $C_{3-6}$cycloalkyl or any combination thereof, or optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{12}$ and/or one or more (e.g., 1-20, or 1-15, 1-10, 1-9, 1-8, 1-7, 1-6, 1-5, 1-4, 1-3 or 1-2, or 1) groups $R_{13}$ and/or any combination of one or more (e.g., 1-20, or 1-15, 1-10, 1-9, 1-8, 1-7, 1-6, 1-5, 1-4, 1-3 or 1-2, or 1) groups $R_{12}$ and $R_{13}$ inserted into its backbone carbon chain, wherein carbon-carbon bond between one or more pairs (e.g., 1-20, or 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, N($R_{14}$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of $C_{3-15}$cycloalkylene, 4- to 15-membered heterocyclylene, $C_{6-10}$ arylene, 5- to 15-membered heteroarylene, $C_{2-6}$ alkenylene, $C_{2-6}$ alkynylene or any combination thereof, wherein the $C_{3-15}$cycloalkylene, the 4- to 15-membered heterocyclylene, $C_{6-10}$ arylene and the 5- to 15-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH-(e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof, and wherein the linear or branched $C_{2-60}$ alkylene is optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, $C_{1-4}$ alkyl, halogen, $C_{3-6}$cycloalkyl or any combination thereof;

$X_4$ represents a bond, or $X_4$ represents N($R_{15}$), C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted $C_{3-20}$cycloalkylene, optionally substituted $C_{6-20}$ arylene, optionally substituted 4- to 20-membered heterocyclylene, optionally substituted 5- to 20-membered heteroarylene, triazolylene-NH-, or -NH-triazolylene, wherein $R_{15}$ represents H or $C_{1-3}$ alkyl, and the $C_{3-20}$cycloalkylene, the $C_{6-20}$ arylene, the 4- to 20-membered heterocyclylene, and the 5- to 20-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, halogen, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof; and

$R_{b1}$ represents optionally substituted $C_{3-20}$cycloalkyl, optionally substituted $C_{6-20}$ aryl, optionally substituted 4- to 20-membered heterocyclyl or optionally substituted 5- to 20-membered heteroaryl, wherein the $C_{3-20}$cycloalkyl, the $C_{6-20}$ aryl, the 4- to 20-membered heterocyclyl, and the 5- to 20-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy (e.g., $C_{1-3}$ alkoxy, such as methoxy, ethoxy, or

propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof.

In some embodiments of the compound of Formula (I-4), $L_2$ represents the structure of the following formula: -$C_{2-30}$ alkylene-, wherein ahydrogen atom of one or more (e.g., 1-20, or 1-15, 1-10, 1-9, 1-8, 1-7, 1-6, 1-5, 1-4, 1-3 or 1-2, or 1) $CH_2$ groups of the $C_{2-30}$ alkylene is optionally replaced with a substituent selected from the group consisting of: D, $C_{1-4}$ alkyl, halogen, $C_{3-6}$cycloalkyl or any combination thereof.
In some embodiments of the compound of Formula (I-4), $L_2$ represents the following group:

$-CH_2-$, $-(CH_2)_2-$, $-(CH_2)_3-$, $-(CH_2)_4-$, $-(CH_2)_5-$, $-(CH_2)_2-CF_2-(CH_2)_2-$, $-(CH_2)_6-$, $-(CH_2)_7-$, $-(CH_2)_8-$,$-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{15}-$, $-(CH_2)_{16}-$, $-(CH_2)_{17}-$,$-(CH_2)_{18}-$, $-(CH_2)_{19}-$, $-(CH_2)_{20}-$, $-(CH_2)_{21}-$, $-(CH_2)_{22}-$, $-(CH_2)_{25}-$, or $-(CH_2)_{30}-$;
wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the group is optionally replaced with a substituent selected from the group consisting of: D, $C_{1-4}$ alkyl, halogen, $C_{3-6}$cycloalkyl or any combination thereof.

In some embodiments of the compound of Formula (I-4), $L_2$ represents the structure of the following formula:

$$-(C(R^{a9})(R^{a10}))_{m1}-(R_{12}(C(R^{a11})(R^{a12}))_{m2})_{p1}-;$$

$$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-;$$

$$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-(R_{12}(C(R^{a15})(R^{a16}))_{m4})_{p3}-;$$

$$-(C(R^{a9})(R^{a10}))_{m1}-(R_{13}(C(R^{a11})(R^{a12}))_{m2})_{p1}-;$$

$$-(C(R^{a9})(R^{a10}))_{m1}-(R_{13}(C(R^{a11})(R^{a12}))_{m2})_{p1}-(R_{13}(C(R^{a13})(R^{a14}))_{m3})_{p2}-;$$

$$-(C(R^{a9})(R^{a10}))_{m1}-(R_{13}(C(R^{a11})(R^{a12}))_{m2})_{p1}-(R_{13}(C(R^{a13})(R^{a14}))_{m3})_{p2}-(R_{13}(C(R^{a15})(R^{a16}))_{m4})_{p3}-;$$

$$-(C(R^{a9})(R^{a10}))_{m1}-(R_{12}-R_{13}-(C(R^{a11})(R^{a12}))_{m2})_{p1}-;$$

$$-(C(R^{a9})(R^{a10}))_{m1}-(R_{12}(C(R^{a11})(R^{a12}))_{m2})_{p1}-(R_{13}(C(R^{a13})(R^{a14}))_{m3})_{p2}-;$$

$$-(C(R^{a9})(R^{a10}))_{m1}-(R_{13}-R_{12}-(C(R^{a11})(R^{a12}))_{m2})_{p1}-; \text{ or}$$

$$-(C(R^{a9})(R^{a10}))_{m1}-(R_{13}(C(R^{a11})(R^{a12}))_{m2})_{p1}-(R_{12}(C(R^{a13})(R^{a14}))_{m3})_{p2}-;$$

wherein each group $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_{14}$ independently represents H or $C_{1-3}$ alkyl; and each group $R_{13}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;
$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and
m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-4), $L_2$ represents the structure of the following formula:

$-(C(R^{a9})(R^{a10}))_{m1}-(O(C(R^{a11})(R^{a12}))_{m2})_{p1}-$;

$-(C(R^{a9})(R^{a10}))_{m1}-(O(C(R^{a11})(R^{a12}))_{m2})_{p1}-(O(C(R^{a13})(R^{a14}))_{m3})_{p2}-$;

$-(C(R^{a9})(R^{a10}))_{m1}-(O(C(R^{a11})(R^{a12}))_{m2})_{p1}-(O(C(R^{a13})(R^{a14}))_{m3})_{p2}-(O(C(R^{a15})(R^{a16}))_{m4})_{p3}-$;

$-(C(R^{a}9)(R^{a10}))_{m1}-(N(R_{g11})(C(R^{a11})(R^{a12}))_{m2})_{p1}-$;

$-(C(R^{a9})(R^{a10}))_{m1}-(N(R_{g11})(C(R^{a11})(R^{a12}))_{m2})_{p1}-(N(R_{g11})(C(R^{a13})(R^{a14}))_{m3})_{p2}-$;

$-(C(R^{a9})(R^{a10}))_{m1}-(N(R_{g11})(C(R^{a11})(R^{a12}))_{m2})_{p1}-(N(R_{g11})(C(R^{a13})(R^{a14}))_{m3})_{p2}-(N(R_{g11})(C(R^{a15})(R^{a16}))_{m4})_{p3}-$;

$-(C(R^{a9})(R^{a10}))_{m1}-(C(O)NH-(C(R^{a11})(R^{a12}))_{m2})_{p1}-$;

$-(C(R^{a9})(R^{a10}))_{m1}-(C(O)NH-(C(R^{a11})(R^{a12}))_{m2})_{p1}-(C(O)NH-(C(R^{a13})(R^{a14}))_{m3})_{p2}-$;

$-(C(R^{a9})(R^{a10}))_{m1}-(C(O)NH-(C(R^{a11})(R^{a12}))_{m2})_{p1}-(C(O)NH-(C(R^{a13})(R^{a14}))_{m3})_{p2}-(C(O)NH-(C(R^{a15})(R^{a16}))_{m4})_{p3}-$;

$-(C(R^{a9})(R^{a10}))_{m1}-(C(O)NH-(C(R^{a11})(R^{a12}))_{m2})_{p1}-(O-(C(R^{a13})(R^{a1}4))_{m3})_{p2}-$;

$-(C(R^{a9})(R^{a10}))_{m1}-C(O)NH-(C(R^{a11})(R^{a12}))_{m2}-(O(C(R^{a13})(R^{a14}))_{m3})_{p1}-$;

$-(C(R^{a9})(R^{a10}))_{m1}-(NHC(C(R^{a11})m_2)_{p1}-$;

$-(C(R^{a9})(R^{a10}))_{m1}-(NHC(O)-(C(R^{a11})(R^{a12}))_{m2})p1-(O-(C(R^{a13})(R^{a14}))_{m3})_{p2}-$;

$-(C(R^{a9})(R^{a10}))_{m1}-(NHC(O)-(C(R^{a11})(R^{a12}))_{m2})_{p1}-(O-(C(R^{a13})(R^{a14}))_{m3})_{p2}-(O-(C(R^{a15})(R^{a16}))_{m4})_{p3}-$;

$-(C(R^{a9})(R^{a10}))_{m1}-NHC(O)-(C(R^{a11})(R^{a12}))_{m2}-(O(C(R^{a13})(R^{a14}))_{m3})_{p1}-$;

$-(C(R^{a9})(R^{a10}))_{m1}-NHC(O)NH-(C(R^{a11})(R^{a12}))_{m2}-$;

$-(C(R^{a9})(R^{a10}))_{m1}-C(O)-(C(R^{a11})(R^{a12})_{m2}-$;

$-(C(R^{a9})(R^{a10}))_{m1}-C(S)-(C(R^{a11})(R^{a12})_{m2}-$;

$-(C(R^{a9})(R^{a10}))_{m1}-S-(C(R^{a11})(R^{a12}))_{m2})-$;

$-(C(R^{a9})(R^{a10}))_{m1}-(C_{6-10}$ arylene$-(C(R^{a11})(R^{a12}))_{m2})_{p1}-$;

$-(C(R^{a9})(R^{a10}))_{m1}-(C_{6-10}$ arylene$-(C(R^{a11})(R^{a12}))_{m2})_{p1}-C_{6-10}$ arylene$-(C(R^{a13})(R^{a14}))_{m3}-$;

$-(C(R^{a9})(R^{a10}))_{m1}-$(4- to 15-membered heterocyclylene$-(C(R^{a11})(R^{a12}))_{m2})_{p1}-$;

$-(C(R^{a9})(R^{a10}))_{m1}-$(4- to 15-membered heterocyclylene$-(C(R^{a11})(R^{a12}))_{m2})_{p1}-$(4- to 15-membered

heterocyclylene-$(C(R^{a13})(R^{a14}))_{m3})_{p2}$-;

$-(C(R^{a9})(R^{a10}))_{m1}$-(5- to 15-membered heteroarylene-$(C(R^{a11})(R^{a12}))_{m2})_{p1}$-;

$-(C(R^{a9})(R^{a10}))_{m1}$-(5- to 15-membered heteroarylene-$(C(R^{a11})(R^{a12}))_{m2})_{p1}$-(5- to 15-membered heteroarylene-$(C(R^{a13})(R^{a14}))_{m3})_{p2}$-;

$-(C(R^{a9})(R^{a10}))_{m1}-(C_{3-15}$cycloalkylene-$(C(R^{a11})(R^{a12}))_{m2})_{p1}$-; or

$-(C(R^{a9})(R^{a10}))_{m1}-(C_{3-15}$cycloalkylene-$(C(R^{a11})(R^{a12}))_{m2})_{p1}-(C_{3-15}$cycloalkylene-$(C(R^{a13})(R^{a14}))_{m3})_{p2}$-;

wherein each $R_{g11}$ independently represents H or $C_{1-3}$ alkyl;
the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;
$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and
m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-4), $L_2$ represents the structure of the following formula: $-CH_2-O-CH_2-$, $-CH_2-O-(CH_2)_2-$, $-(CH_2)_1-O-(CH_2)_3-$, $-(CH_2)_1-O-(CH_2)_4-$, $-(CH_2)_1-O-(CH_2)_5-$, - $(CH_2)_1-O-(CH_2)_6-$, $-(CH_2)_1-O-(CH_2)_7-$, $-(CH_2)_1-O-(CH_2)_8-$, $-(CH_2)_1-O-(CH_2)_9-$, $-(CH_2)_1-O-(CH_2)_{10}-$, - $(CH_2)_2-O-(CH_2)_1-$, $-(CH_2)_2-O-(CH_2)_2-$, $-(CH_2)_2-O-(CH_2)_3-$, $-(CH_2)_2-O-(CH_2)_4-$, $-(CH_2)_2-O-(CH_2)_5-$, - $(CH_2)_2-O-(CH_2)_6-$, $-(CH_2)_2-O-(CH_2)_7-$, $-(CH_2)_2-O-(CH_2)_8-$, $-(CH_2)_2-O-(CH_2)_9-$, $-(CH_2)_2-O-(CH_2)_{10}-$, - $(CH_2)_2-O-(CH_2)_{11}-$, $-(CH_2)_2-O-(CH_2)_{12}-$, $-(CH_2)_3-O-(CH_2)_1-$, $-(CH_2)_3-O-(CH_2)_2-$, $-(CH_2)_3-O-(CH_2)_3-$, - $(CH_2)_3-O-(CH_2)_4-$, $-(CH_2)_3-O-(CH_2)_5-$, $-(CH_2)_3-O-(CH_2)_6-$, $-(CH_2)_3-O-(CH_2)_7-$, $-(CH_2)_4-O-(CH_2)_1-$, - $(CH_2)_4-O-(CH_2)_2-$, $-(CH_2)_4-O-(CH_2)_3-$, $-(CH_2)_4-O-(CH_2)_4-$, $-(CH_2)_4-O-(CH_2)_5-$, $-(CH_2)_4-O-(CH_2)_6-$, - $(CH_2)_5-O-(CH_2)_1-$, $-(CH_2)_5-O-(CH_2)_2-$, $-(CH_2)_5-O-(CH_2)_3-$, $-(CH_2)_5-O-(CH_2)_4-$, $-(CH_2)_5-O-(CH_2)_5-$, - $(CH_2)_6-O-(CH_2)_1-$, $-(CH_2)_6-O-(CH_2)_2-$, $-(CH_2)_6-O-(CH_2)_3-$, $-(CH_2)_6-O-(CH_2)_4-$, $-(CH_2)_7-O-(CH_2)_1-$, - $(CH_2)_7-O-(CH_2)_2-$, $-(CH_2)_7-O-(CH_2)_3-$, $-(CH_2)_8-O-(CH_2)_1-$, $-(CH_2)_8-O-(CH_2)_2-$, $-CH(CH_3)-O-(CH_2)_1-$, - $CH(CH_3)-O-(CH_2)_2-$, $-CH(CH_3)-O-(CH_2)_3-$, $-CH(CH_3)-O-(CH_2)_4-$, $-CH(CH_3)-O-(CH_2)_5-$, $-CH(CH_3)-O-(CH_2)_6-$, $-CH(CH_3)-O-(CH_2)_7-$, $-CH(CH_3)-O-(CH_2)_8-$, $-CH(CH_3)-O-(CH_2)_9-$, $-CH(CH_3)-O-(CH_2)_{10}-$, $-CH_2-(O(CH_2)_2)_2-$, $-CH_2-(O(CH_2)_2)_3-$, $-CH_2-(O(CH_2)_2)_4-$, $-CH_2-(O(CH_2)_2)_5-$, $-CH_2-(O(CH_2)_2)_6-$, $-CH_2-(O(CH_2)_2)_7-$, $-CH_2-(O(CH_2)_2)_8-$, $-CH_2-(O(CH_2)_2)_9-$, $-CH_2-(O(CH_2)_2)_{10}-$, $-CH_2-(O(CH_2)_2)_1-OCH_2-$, - $CH_2-(O(CH_2)_2)_2-OCH_2-$, $-CH_2-(O(CH_2)_2)_3-OCH_2-$, $-CH_2-(O(CH_2)_2)_4-OCH_2-$, $-CH_2-(O(CH_2)_2)_5-OCH_2-$, $-CH_2-(O(CH_2)_2)_6-OCH_2-$, $-CH_2-(O(CH_2)_2)_7-OCH_2-$, $-CH_2-(O(CH_2)_2)_8-OCH_2-$, $-CH_2-(O(CH_2)_2)_9-OCH_2-$, $-CH_2-(O(CH_2)_2)_{10}-OCH_2-$, $-(CH_2)_2-(O(CH_2)_2)_2-$, $-(CH_2)_2-(O(CH_2)_2)_3-$, $-(CH_2)_2-(O(CH_2)_2)_4-$, - $(CH_2)_2-(O(CH_2)_2)_5-$, $-(CH_2)_2-(O(CH_2)_2)_6-$, $-(CH_2)_2-(O(CH_2)_2)_7-$, $-(CH_2)_2-(O(CH_2)_2)_8-$, $-(CH_2)_2-(O(CH_2)_2)_9-$, $-(CH_2)_2-(O(CH_2)_2)_{10}-$, $-(CH_2)_3-(O(CH_2)_2)_2-$, $-(CH_2)_3-(O(CH_2)_2)_3-$, $-(CH_2)_3-(O(CH_2)_2)_4-$, - $(CH_2)_3-(O(CH_2)_2)_5-$, $-(CH_2)_3-(O(CH_2)_2)_6-$, $-(CH_2)_3-(O(CH_2)_2)_7-$, $-(CH_2)_3-(O(CH_2)_2)_8-$, $-(CH_2)_3-(O(CH_2)_2)_9-$, $-(CH_2)_3-(O(CH_2)_2)_{10}-$, $-(CH_2)_4-(O(CH_2)_2)_2-$, $-(CH_2)_4-(O(CH_2)_2)_3-$, $-(CH_2)_4-(O(CH_2)_2)_4-$, - $(CH_2)_4-(O(CH_2)_2)_5-$, $-(CH_2)_4-(O(CH_2)_2)_6-$, $-(CH_2)_4-(O(CH_2)_2)_7-$, $-(CH_2)_4-(O(CH_2)_2)_8-$, $-(CH_2)_4-(O(CH_2)_2)_9-$, $-(CH_2)_4-(O(CH_2)_2)_{10}-$, $-CH_2-(O(CH_2)_3)_2-$, $-CH_2-(O(CH_2)_3)_3-$, $-CH_2-(O(CH_2)_3)_4-$, $-CH_2-(O(CH_2)_3)_5-$, $-CH_2-(O(CH_2)_3)_6-$, $-CH_2-(O(CH_2)_3)_7-$, $-CH_2-(O(CH_2)_3)_8-$, $-CH_2-(O(CH_2)_3)_9-$, $-CH_2-(O(CH_2)_3)_{10}-$, $-(CH_2)_2-(O(CH_2)_3)_2-$, $-(CH_2)_2-(O(CH_2)_3)_3-$, $-(CH_2)_2-(O(CH_2)_3)_4-$, $-(CH_2)_2-(O(CH_2)_3)_5-$,$-(CH_2)_2-(O(CH_2)_3)_6-$, $-(CH_2)_2-(O(CH_2)_3)_7-$, $-(CH_2)_2-(O(CH_2)_3)_8-$, $-(CH_2)_2-(O(CH_2)_3)_9-$, $-(CH_2)_2-(O(CH_2)_3)_{10}-$, $-(CH_2)_3-(O(CH_2)_3)_2-$, $-(CH_2)_3-(O(CH_2)_3)_3-$, $-(CH_2)_3-(O(CH_2)_3)_4-$, $-(CH_2)_3-(O(CH_2)_3)_5-$,$-(CH_2)_3-(O(CH_2)_3)_6-$, $-(CH_2)_3-(O(CH_2)_3)_7-$, $-(CH_2)_3-(O(CH_2)_3)_8-$, $-(CH_2)_3-(O(CH_2)_3)_9-$, $-(CH_2)_3-(O(CH_2)_3)_{10}-$, $-CH_2-O-(CH_2)_2-O-(CH_2)_3-$, $-CH_2-(O(CH_2)_2)_2-(O(CH_2)_3)_2-$, $-CH_2-(O(CH_2)_2)_3-(O(CH_2)_3)_3-$, $-CH_2-(O(CH_2)_2)_4-(O(CH_2)_3)_4-$, $-CH_2-(O(CH_2)_2)_5-(O(CH_2)_3)_5-$, $-CH_2-(O(CH_2)_2)_6-(O(CH_2)_3)_6-$, $-(CH_2)_2-O-(CH_2)_2-O-(CH_2)_3-$, $-(CH_2)_2-(O(CH_2)_2)_2-(O(CH_2)_3)_2-$, $-(CH_2)_2-(O(CH_2)_2)_3-(O(CH_2)_3)_3-$, $-(CH_2)_2-(O(CH_2)_2)_4-(O(CH_2)_3)_4-$, $-(CH_2)_2-(O(CH_2)_2)_5-(O(CH_2)_3)_5-$, $-(CH_2)_2-(O(CH_2)_2)_6-(O(CH_2)_3)_6-$, $-(CH_2)_3-O-(CH_2)_2-O-(CH_2)_3-$, $-(CH_2)_3-(O(CH_2)_2)_2-(O(CH_2)_3)_2-$, $-(CH_2)_3-(O(CH_2)_2)_3-(O(CH_2)_3)_3-$, $-(CH_2)_3-(O(CH_2)_2)_4-(O(CH_2)_3)_4-$, $-(CH_2)_3-(O(CH_2)_2)_5-(O(CH_2)_3)_5-$, $-(CH_2)_3-(O(CH_2)_2)_6-(O(CH_2)_3)_6-$, $-CH_2-O-(CH_2)_3-O-(CH_2)_2-$, $-CH_2-(O(CH_2)_3)_2-(O(CH_2)_2)_2-$, $-CH_2-(O(CH_2)_3)_3-(O(CH_2)_2)_3-$, $-CH_2-(O(CH_2)_3)_4-(O(CH_2)_2)_4-$, $-CH_2-(O(CH_2)_3)_5-(O(CH_2)_2)_5-$, $-CH_2-(O(CH_2)_3)_6-(O(CH_2)_2)_6-$, $-(CH_2)_2-O-(CH_2)_3-O-(CH_2)_2-$, $-(CH_2)_2-(O(CH_2)_3)_2-(O(CH_2)_2)_2-$, $-(CH_2)_2-(O(CH_2)_3)_3-(O(CH_2)_2)_3-$, $-(CH_2)_2-(O(CH_2)_3)_4-(O(CH_2)_2)_4-$,

$-(CH_2)_2-(O(CH_2)_3)_5-(O(CH_2)_2)_5-$, $-(CH_2)_2-(O(CH_2)_3)_6-(O(CH_2)_2)_6-$, $-(CH_2)_3-O-(CH_2)_3-O-(CH_2)_2-$, $-(CH_2)_3-(O(CH_2)_3)_2-(O(CH_2)_2)_2-$, $-(CH_2)_3-(O(CH_2)_3)_3-(O(CH_2)_2)_3-$, $-(CH_2)_3-(O(CH_2)_3)_4-(O(CH_2)_2)_4-$, $-(CH_2)_3-(O(CH_2)_3)_5-(O(CH_2)_2)_5-$, $-(CH_2)_3-(O(CH_2)_3)_6-(O(CH_2)_2)_6-$, $-CH_2-O-(CH_2)_2-O-CH_2-$, $-(CH_2)_2-O-(CH_2)_2-O-CH_2-$, $-(CH_2)_2-(O(CH_2)_2)_2-O-(CH_2)_3-$, $-(CH_2)_2-(O(CH_2)_2)_3-O-(CH_2)_3-$, $-(CH_2)_2-(O(CH_2)_2)_4-O-(CH_2)_3-$, $-(CH_2)_5-(O(CH_2)_2)_2-O-(CH_2)_5-$, $-(CH_2)_5-(O(CH_2)_2)_2-O-(CH_2)_6-$, $-CH_2-C(O)-CH_2-$, $-CH_2-C(O)-(CH_2)_2-$, $-(CH_2)_1-C(O)-(CH_2)_3-$, $-(CH_2)_1-C(O)-(CH_2)_4-$, $-(CH_2)_1-C(O)-(CH_2)_5-$, $-(CH_2)_1-C(O)-(CH_2)_6-$, $-(CH_2)_1-C(O)-(CH_2)_7-$, $-(CH_2)_1-C(O)-(CH_2)_8-$, $-(CH_2)_1-C(O)-(CH_2)_9-$, $-(CH_2)_1-C(O)-(CH_2)_{10}-$, $-(CH_2)_2-C(O)-(CH_2)_1-$, $-(CH_2)_2-C(O)-(CH_2)_2-$, $-(CH_2)_2-C(O)-(CH_2)_3-$, $-(CH_2)_2-C(O)-(CH_2)_4-$, $-(CH_2)_2-C(O)-(CH_2)_5-$, $-(CH_2)_2-C(O)-(CH_2)_6-$, $-(CH_2)_2-C(O)-(CH_2)_7-$, $-(CH_2)_2-C(O)-(CH_2)_8-$, $-(CH_2)_2-C(O)-(CH_2)_9-$, $-(CH_2)_2-C(O)-(CH_2)_{10}-$, $-(CH_2)_2-C(O)-(CH_2)_{11}-$, $-(CH_2)_2-C(O)-(CH_2)_{12}-$, $-(CH_2)_3-C(O)-(CH_2)_1-$, $-(CH_2)_3-C(O)-(CH_2)_2-$, $-(CH_2)_3-C(O)-(CH_2)_3-$, $-(CH_2)_3-C(O)-(CH_2)_4-$, $-(CH_2)_3-C(O)-(CH_2)_5-$, $-(CH_2)_3-C(O)-(CH_2)_6-$, $-(CH_2)_3-C(O)-(CH_2)_7-$, $-(CH_2)_4-C(O)-(CH_2)_1-$, $-(CH_2)_4-C(O)-(CH_2)_2-$, $-(CH_2)_4-C(O)-(CH_2)_3-$, $-(CH_2)_4-C(O)-(CH_2)_4-$, $-(CH_2)_4-C(O)-(CH_2)_5-$, $-(CH_2)_4-C(O)-(CH_2)_6-$, $-(CH_2)_5-C(O)-(CH_2)_1-$, $-(CH_2)_5-C(O)-(CH_2)_2-$, $-(CH_2)_5-C(O)-(CH_2)_3-$, $-(CH_2)_5-C(O)-(CH_2)_4-$, $-(CH_2)_5-C(O)-(CH_2)_5-$, $-(CH_2)_6-C(O)-(CH_2)_1-$, $-(CH_2)_6-C(O)-(CH_2)_2-$, $-(CH_2)_6-C(O)-(CH_2)_3-$, $-(CH_2)_6-C(O)-(CH_2)_4-$, $-(CH_2)_7-C(O)-(CH_2)_1-$, $-(CH_2)_7-C(O)-(CH_2)_2-$, $-(CH_2)_7-C(O)-(CH_2)_3-$, $-(CH_2)_8-C(O)-(CH_2)_1-$, $-(CH_2)_8-C(O)-(CH_2)_2-$, $-CH_2-S-CH_2-$, $-CH_2-S-(CH_2)_2-$, $-(CH_2)_1-S-(CH_2)_3-$, $-(CH_2)_1-S-(CH_2)_4-$, $-(CH_2)_1-S-(CH_2)_5-$, $-(CH_2)_1-S-(CH_2)_6-$, $-(CH_2)_1-S-(CH_2)_7-$, $-(CH_2)_1-S-(CH_2)_8-$, $-(CH_2)_1-S-(CH_2)_9-$, $-(CH_2)_1-S-(CH_2)_{10}-$, $-(CH_2)_2-S-(CH_2)_1-$, $-(CH_2)_2-S-(CH_2)_2-$, $-(CH_2)_2-S-(CH_2)_3-$, $-(CH_2)_2-S-(CH_2)_4-$, $-(CH_2)_2-S-(CH_2)_5-$, $-(CH_2)_2-S-(CH_2)_6-$, $-(CH_2)_2-S-(CH_2)_7-$, $-(CH_2)_2-S-(CH_2)_8-$, $-(CH_2)_2-S-(CH_2)_9-$, $-(CH_2)_2-S-(CH_2)_{10}-$, $-(CH_2)_2-S-(CH_2)_{11}-$, $-(CH_2)_2-S-(CH_2)_{12}-$, $-(CH_2)_3-S-(CH_2)_1-$, $-(CH_2)_3-S-(CH_2)_2-$, $-(CH_2)_3-S-(CH_2)_3-$, $-(CH_2)_3-S-(CH_2)_4-$, $-(CH_2)_3-S-(CH_2)_5-$, $-(CH_2)_3-S-(CH_2)_6-$, $-(CH_2)_3-S-(CH_2)_7-$, $-(CH_2)_4-S-(CH_2)_1-$, $-(CH_2)_4-S-(CH_2)_2-$, $-(CH_2)_4-S-(CH_2)_3-$, $-(CH_2)_4-S-(CH_2)_4-$, $-(CH_2)_4-S-(CH_2)_5-$, $-(CH_2)_4-S-(CH_2)_6-$, $-(CH_2)_5-S-(CH_2)_1-$, $-(CH_2)_5-S-(CH_2)_2-$, $-(CH_2)_5-S-(CH_2)_3-$, $-(CH_2)_5-S-(CH_2)_4-$, $-(CH_2)_5-S-(CH_2)_5-$, $-(CH_2)_6-S-(CH_2)_1-$, $-(CH_2)_6-S-(CH_2)_2-$, $-(CH_2)_6-S-(CH_2)_3-$, $-(CH_2)_6-S-(CH_2)_4-$, $-(CH_2)_7-S-(CH_2)_1-$, $-(CH_2)_7-S-(CH_2)_2-$, $-(CH_2)_7-S-(CH_2)_3-$, $-(CH_2)_8-S-(CH_2)_1-$, $-(CH_2)_8-S-(CH_2)_2-$, $-CH_2-C(S)-CH_2-$, $-CH_2-C(S)-(CH_2)_2-$, $-(CH_2)_1-C(S)-(CH_2)_3-$, $-(CH_2)_1-C(S)-(CH_2)_4-$, $-(CH_2)_1-C(S)-(CH_2)_5-$, $-(CH_2)_1-C(S)-(CH_2)_6-$, $-(CH_2)_1-C(S)-(CH_2)_7-$, $-(CH_2)_1-C(S)-(CH_2)_8-$, $-(CH_2)_1-C(S)-(CH_2)_9-$, $-(CH_2)_1-C(S)-(CH_2)_{10}-$, $-(CH_2)_2-C(S)-(CH_2)_1-$, $-(CH_2)_2-C(S)-(CH_2)_2-$, $-(CH_2)_2-C(S)-(CH_2)_3-$, $-(CH_2)_2-C(S)-(CH_2)_4-$, $-(CH_2)_2-C(S)-(CH_2)_5-$, $-(CH_2)_2-C(S)-(CH_2)_6-$, $-(CH_2)_2-C(S)-(CH_2)_7-$, $-(CH_2)_2-C(S)-(CH_2)_8-$, $-(CH_2)_2-C(S)-(CH_2)_9-$, $-(CH_2)_2-C(S)-(CH_2)_{10}-$, $-(CH_2)_2-C(S)-(CH_2)_{11}-$, $-(CH_2)_2-C(S)-(CH_2)_{12}-$, $-(CH_2)_3-C(S)-(CH_2)_1-$, $-(CH_2)_3-C(S)-(CH_2)_2-$, $-(CH_2)_3-C(S)-(CH_2)_3-$, $-(CH_2)_3-C(S)-(CH_2)_4-$, $-(CH_2)_3-C(S)-(CH_2)_5-$, $-(CH_2)_3-C(S)-(CH_2)_6-$, $-(CH_2)_3-C(S)-(CH_2)_7-$, $-(CH_2)_4-C(S)-(CH_2)_1-$, $-(CH_2)_4-C(S)-(CH_2)_2-$, $-(CH_2)_4-C(S)-(CH_2)_3-$, $-(CH_2)_4-C(S)-(CH_2)_4-$, $-(CH_2)_4-C(S)-(CH_2)_5-$, $-(CH_2)_4-C(S)-(CH_2)_6-$, $-(CH_2)_5-C(S)-(CH_2)_1-$, $-(CH_2)_5-C(S)-(CH_2)_2-$, $-(CH_2)_5-C(S)-(CH_2)_3-$, $-(CH_2)_5-C(S)-(CH_2)_4-$, $-(CH_2)_5-C(S)-(CH_2)_5-$, $-(CH_2)_6-C(S)-(CH_2)_1-$, $-(CH_2)_6-C(S)-(CH_2)_2-$, $-(CH_2)_6-C(S)-(CH_2)_3-$, $-(CH_2)_6-C(S)-(CH_2)_4-$, $-(CH_2)_7-C(S)-(CH_2)_1-$, $-(CH_2)_7-C(S)-(CH_2)_2-$, $-(CH_2)_7-C(S)-(CH_2)_3-$, $-(CH_2)_8-C(S)-(CH_2)_1-$, $-(CH_2)_8-C(S)-(CH_2)_2-$, $-CH_2-C(O)O-CH_2-$, $-CH_2-C(O)O-(CH_2)_2-$, $-(CH_2)_1-C(O)O-(CH_2)_3-$, $-(CH_2)_1-C(O)O-(CH_2)_4-$, $-(CH_2)_1-C(O)O-(CH_2)_5-$, $-(CH_2)_1-C(O)O-(CH_2)_6-$, $-(CH_2)_1-C(O)O-(CH_2)_7-$, $-(CH_2)_1-C(O)O-(CH_2)_8-$, $-(CH_2)_1-C(O)O-(CH_2)_9-$, $-(CH_2)_1-C(O)O-(CH_2)_{10}-$, $-(CH_2)_2-C(O)O-(CH_2)_1-$, $-(CH_2)_2-C(O)O-(CH_2)_2-$, $-(CH_2)_2-C(O)O-(CH_2)_3-$, $-(CH_2)_2-C(O)O-(CH_2)_4-$, $-(CH_2)_2-C(O)O-(CH_2)_5-$, $-(CH_2)_2-C(O)O-(CH_2)_6-$, $-(CH_2)_2-C(O)O-(CH_2)_7-$, $-(CH_2)_2-C(O)O-(CH_2)_8-$, $-(CH_2)_2-C(O)O-(CH_2)_9-$, $-(CH_2)_2-C(O)O-(CH_2)_{10}-$, $-(CH_2)_2-C(O)O-(CH_2)_{11}-$, $-(CH_2)_2-C(O)O-(CH_2)_{12}-$, $-(CH_2)_3-C(O)O-(CH_2)_1-$, $-(CH_2)_3-C(O)O-(CH_2)_2-$, $-(CH_2)_3-C(O)O-(CH_2)_3-$, $-(CH_2)_3-C(O)O-(CH_2)_4-$, $-(CH_2)_3-C(O)O-(CH_2)_5-$, $-(CH_2)_3-C(O)O-(CH_2)_6-$, $-(CH_2)_3-C(O)O-(CH_2)_7-$, $-(CH_2)_4-C(O)O-(CH_2)_1-$, $-(CH_2)_4-C(O)O-(CH_2)_2-$, $-(CH_2)_4-C(O)O-(CH_2)_3-$, $-(CH_2)_4-C(O)O-(CH_2)_4-$, $-(CH_2)_4-C(O)O-(CH_2)_5-$, $-(CH_2)_4-C(O)O-(CH_2)_6-$, $-(CH_2)_5-C(O)O-(CH_2)_1-$, $-(CH_2)_5-C(O)O-(CH_2)_2-$, $-(CH_2)_5-C(O)O-(CH_2)_3-$, $-(CH_2)_5-C(O)O-(CH_2)_4-$, $-(CH_2)_5-C(O)O-(CH_2)_5-$, $-(CH_2)_6-C(O)O-(CH_2)_1-$, $-(CH_2)_6-C(O)O-(CH_2)_2-$, $-(CH_2)_6-C(O)O-(CH_2)_3-$, $-(CH_2)_6-C(O)O-(CH_2)_4-$, $-(CH_2)_7-C(O)O-(CH_2)_1-$, $-(CH_2)_7-C(O)O-(CH_2)_2-$, $-(CH_2)_7-C(O)O-(CH_2)_3-$, $-(CH_2)_8-C(O)O-(CH_2)_1-$, $-(CH_2)_8-C(O)O-(CH_2)_2-$, $-CH_2-O-C(O)-CH_2-$, $-CH_2-OC(O)-(CH_2)_2-$, $-(CH_2)_1-OC(O)-(CH_2)_3-$, $-(CH_2)_1-OC(O)-(CH_2)_4-$, $-(CH_2)_1-OC(O)-(CH_2)_5-$, $-(CH_2)_1-O-C(O)-(CH_2)_6-$, $-(CH_2)_1-OC(O)-(CH_2)_7-$, $-(CH_2)_1-OC(O)-(CH_2)_8-$, $-(CH_2)_1-OC(O)-(CH_2)_9-$, $-(CH_2)_1-OC(O)-(CH_2)_{10}-$, $-(CH_2)_2-OC(O)-(CH_2)_1-$, $-(CH_2)_2-OC(O)-(CH_2)_2-$, $-(CH_2)_2-OC(O)-(CH_2)_3-$, $-(CH_2)_2-OC(O)-(CH_2)_4-$, $-(CH_2)_2-O-C(O)-(CH_2)_5-$, $-(CH_2)_2-OC(O)-(CH_2)_6-$, $-(CH_2)_2-OC(O)-(CH_2)_7-$, $-(CH_2)_2-OC(O)-(CH_2)_8-$, $-(CH_2)_2-OC(O)-(CH_2)_9-$, $-(CH_2)_2-OC(O)-(CH_2)_{10}-$, $-(CH_2)_2-OC(O)-(CH_2)_{11}-$, $-(CH_2)_2-OC(O)-(CH_2)_{12}-$, $-(CH_2)_3-OC(O)-(CH_2)_1-$, $-(CH_2)_3-O-C(O)-(CH_2)_2-$, $-(CH_2)_3-OC(O)-(CH_2)_3-$, $-(CH_2)_3-OC(O)-(CH_2)_4-$, $-(CH_2)_3-OC(O)-(CH_2)_5-$, $-(CH_2)_3-OC(O)-(CH_2)_6-$, $-(CH_2)_3-OC(O)-(CH_2)_7-$, $-(CH_2)_4-OC(O)-(CH_2)_1-$, $-(CH_2)_4-OC(O)-(CH_2)_2-$, $-(CH_2)_4-OC(O)-(CH_2)_3-$, $-(CH_2)_4-O-C(O)-(CH_2)_4-$, $-(CH_2)_4-OC(O)-(CH_2)_5-$, $-(CH_2)_4-OC(O)-(CH_2)_6-$, $-(CH_2)_5-OC(O)-(CH_2)_1-$, $-(CH_2)_5-OC(O)-(CH_2)_2-$, $-(CH_2)_5-OC(O)-(CH_2)_3-$, $-(CH_2)_5-OC(O)-(CH_2)_4-$, $-(CH_2)_5-OC(O)-(CH_2)_5-$, $-(CH_2)_6-OC(O)-(CH_2)_1-$, $-(CH_2)_6-O-C(O)-(CH_2)_2-$, $-(CH_2)_6-OC(O)-(CH_2)_3-$, $-(CH_2)_6-OC(O)-(CH_2)_4-$, $-(CH_2)_7-OC(O)-(CH_2)_1-$, $-(CH_2)_7-OC(O)-(CH_2)_2-$, $-(CH_2)_7-OC(O)-(CH_2)_3-$, $-(CH_2)_8-OC(O)-(CH_2)_1-$, $-(CH_2)_8-OC(O)-(CH_2)_2-$, $-(CH_2)_1-N(R_{g11})-(CH_2)_1-$, $-(CH_2)_1-N(R_{g11})-(CH_2)_2-$, $-(CH_2)_1-N(R_{g11})-(CH_2)_3-$, $-(CH_2)_1-N(R_{g11})-(CH_2)_4-$, $-(CH_2)_1-N(R_{g11})-(CH_2)_5-$, $-(CH_2)_1-N(R_{g11})-(CH_2)_6-$, $-(CH_2)_1-N(R_{g11})-(CH_2)_7-$, $-(CH_2)_1-N(R_{g11})-(CH_2)_8-$, $-(CH_2)_1-N(R_{g11})-(CH_2)_9-$, $-(CH_2)_1-N(R_{g11})-(CH_2)_{10}-$, $-(CH_2)_2-N(R_{g11})-(CH_2)_1-$, $-(CH_2)_2-N(R_{g11})-(CH_2)_2-$, $-(CH_2)_2-N(R_{g11})-(CH_2)_3-$, $-(CH_2)_2-N(R_{g11})-(CH_2)_4-$, $-(CH_2)_2-N(R_{g11})-(CH_2)_5-$, $-(CH_2)_2-N(R_{g11})-(CH_2)_6-$, $-(CH_2)_2-N(R_{g11})-(CH_2)_7-$,

$-(CH_2)_2-N(R_{g11})-(CH_2)_8-$, $-(CH_2)_2-N(R_{g11})-(CH_2)_9-$, $-(CH_2)_2-N(R_{g11})-(CH_2)_{10}-$, $-(CH_2)_2-N(R_{g11})-(CH_2)_{11}-$, $-(CH_2)_2-N(R_{g11})-(CH_2)_{12}-$, $-(CH_2)_3-N(R_{g11})-(CH_2)_1-$, $-(CH_2)_3-N(R_{g11})-(CH_2)_2-$, $-(CH_2)_3-N(R_{g11})-(CH_2)_3-$, $-(CH_2)_4-N(R_{g11})-(CH_2)_1-$, $-(CH_2)_4-N(R_{g11})-(CH_2)_2-$, $-(CH_2)_4-N(R_{g11})-(CH_2)_3-$, $-(CH_2)_4-N(R_{g11})-(CH_2)_4-$, $-(CH_2)_5-N(R_{g11})-(CH_2)_1-$, $-(CH_2)_5-N(R_{g11})-(CH_2)_2-$, $-(CH_2)_5-N(R_{g11})-(CH_2)_3-$, $-(CH_2)_5-N(R_{g11})-(CH_2)_4-$, $-(CH_2)_5-N(R_{g11})-(CH_2)_5-$, $-(CH_2)_6-N(R_{g11})-(CH_2)_1-$, $-(CH_2)_6-N(R_{g11})-(CH_2)_2-$, $-(CH_2)_6-N(R_{g11})-(CH_2)_3-$, $-(CH_2)_7-N(R_{g11})-(CH_2)_1-$, $-(CH_2)_7-N(R_{g11})-(CH_2)_2-$, $-(CH_2)_7-N(R_{g11})-(CH_2)_3-$, $-(CH_2)_8-N(R_{g11})-(CH_2)_1-$, $-(CH_2)_8-NR_{g11})-(CH_2)_2-$, $-(CH_2)_8-N(R_{g11})-(CH_2)_3-$, $-CH(CH_3)-N(R_{g11})-(CH_2)_1-$, $-CH(CH_3)-N(R_{g11})-(CH_2)_2-$, $-CH(CH_3)-N(R_{g11})-(CH_2)_3-$, $-CH(CH_3)-N(R_{g11})-(CH_2)_4-$, $-CH(CH_3)-N(R_{g11})-(CH_2)_5-$, $-CH(CH_3)-N(R_{g11})-(CH_2)_6-$, $-CH(CH_3)-N(R_{g11})-(CH_2)_7-$, $-CH(CH_3)-N(R_{g11})-(CH_2)_8-$, $-CH(CH_3)-N(R_{g11})-(CH_2)_9-$, $-CH(CH_3)-N(R_{g11})-(CH_2)_{10}-$, $-CH_2C(O)NHCH_2-$, $-(CH_2)_2C(O)NH(CH_2)_2-$, $-(CH_2)_2C(O)NH(CH_2)_3-$, $- (CH_2)_2C(O)NH(CH_2)_4-$, $-(CH_2)_2C(O)NH(CH_2)_5-$, $-(CH_2)_3C(O)NH(CH_2)_3-$, $-(CH_2)_3C(O)NH(CH_2)_4-$, $- (CH_2)_4C(O)NH(CH_2)_4-$, $-(CH_2)_5C(O)NH(CH_2)_5-$, $-(CH_2)_6C(O)NH(CH_2)_7-$, $-(CH_2)_6C(O)NH(CH_2)_6-$, $- (CH_2)_7C(O)NH(CH_2)_7-$, $-(CH_2)_8C(O)NH(CH_2)_8$, $-(CH_2)_9C(O)NH(CH_2)_9-$, $-(CH_2)_{10}C(O)NH(CH_2)_{10}-$, $- (CH_2)_2C(O)NH(CH_2)_2-O-(CH_2)_2-$, $-CH_2NHC(O)CH_2-$, $-(CH_2)_2NHC(O)(CH_2)_2-$, $- (CH_2)_2NHC(O)(CH_2)_3-$, $-(CH_2)_2NHC(O)(CH_2)_4-$, $-(CH_2)_2NHC(O)(CH_2)_5-$, $-(CH_2)_3NHC(O)(CH_2)_3-$, $- (CH_2)_3NHC(O)(CH_2)_4-$, $-(CH_2)_4NHC(O)(CH_2)_4-$, $-(CH_2)_5NHC(O)(CH_2)_5-$, $-(CH_2)_6NHC(O)(CH_2)_7-$, $- (CH_2)_6NHC(O)(CH_2)_6-$, $-(CH_2)_7NHC(O)(CH_2)_7-$, $-(CH_2)_8NHC(O)(CH_2)_8$, $-(CH_2)_9NHC(O)(CH_2)_9-$, $- (CH_2)_{10}NHC(O)(CH_2)_{10}-$, $-(CH_2)_4NHC(O)(CH_2)_8-$, $-(CH_2)_2NHC(O)(CH_2)_2-O-(CH_2)_2-$, $- (CH_2)_4NHC(O)CH_2-$, $-CH_2$-piperidinylene-$CH_2-$, $-CH_2$-piperidinylene-$(CH_2)_2-$, $-CH_2$-piperidinylene-$(CH_2)_3-$, $-CH_2$-piperidinylene-$(CH_2)_4-$, $-CH_2$-piperidinylene-$(CH_2)_5-$, $-CH_2$-piperidinylene-$(CH_2)_6-$, $- CH_2$-piperidinylene-$(CH_2)_7-$, $-CH_2$-piperidinylene-$(CH_2)_8-$, $-(CH_2)_2$-piperidinylene-$(CH_2)_1-$, $-(CH_2)_2$-piperidinylene-$(CH_2)_2-$, $-(CH_2)_2$-piperidinylene-$(CH_2)_3-$, $-(CH_2)_2$-piperidinylene-$(CH_2)_4-$, $-(CH_2)_2$-piperidinylene-$(CH_2)_5-$, $-(CH_2)_2$-piperidinylene-$(CH_2)_6-$, $-(CH_2)_2$-piperidinylene-$(CH_2)_7-$, $-(CH_2)_2$-piperidinylene-$(CH_2)_8-$, $-(CH_2)_3$-piperidinylene-$CH_2-$, $-(CH_2)_3$-piperidinylene-$(CH_2)_2-$, $-(CH_2)_3$-piperidinylene-$(CH_2)_3-$, $-(CH_2)_3$-piperidinylene-$(CH_2)_4-$, $-(CH_2)_3$-piperidinylene-$(CH_2)_5-$, $-(CH_2)_3$-piperidinylene-$(CH_2)_6-$, $-(CH_2)_3$-piperidinylene-$(CH_2)_7-$, $-(CH_2)_3$-piperidinylene-$(CH_2)_8-$, $-(CH_2)_4$-piperidinylene-$CH_2-$, $-(CH_2)_4$-piperidinylene-$(CH_2)_2-$, $-(CH_2)_4$-piperidinylene-$(CH_2)_3-$, $-(CH_2)_4$-piperidinylene-$(CH_2)_4-$, $-(CH_2)_4$-piperidinylene-$(CH_2)_5-$, $-(CH_2)_4$-piperidinylene-$(CH_2)_6-$, $-(CH_2)_4$-piperidinylene-$(CH_2)_7-$, $-(CH_2)_4$-piperidinylene-$(CH_2)_8-$, $-(CH_2)_5$-piperidinylene-$(CH_2)_1-$, $-(CH_2)_5$-piperidinylene-$(CH_2)_2-$, $-(CH_2)_5$-piperidinylene-$(CH_2)_3-$, $-(CH_2)_5$-piperidinylene-$(CH_2)_4-$, $-(CH_2)_5$-piperidinylene-$(CH_2)_5-$, $-(CH_2)_5$-piperidinylene-$(CH_2)_6-$, $-(CH_2)_5$-piperidinylene-$(CH_2)_7-$, $-(CH_2)_5$-piperidinylene-$(CH_2)_8-$, $-(CH_2)_6$-piperidinylene-$(CH_2)_1-$, $-(CH_2)_6$-piperidinylene-$(CH_2)_2-$, $-(CH_2)_6$-piperidinylene-$(CH_2)_3-$, $-(CH_2)_6$-piperidinylene-$(CH_2)_4-$, $-(CH_2)_6$-piperidinylene-$(CH_2)_5-$, $-(CH_2)_6$-piperidinylene-$(CH_2)_6-$, $-(CH_2)_6$-piperidinylene-$(CH_2)_7-$, $-(CH_2)_6$-piperidinylene-$(CH_2)_8-$, $-(CH_2)_7$-piperidinylene-$(CH_2)_1-$, $-(CH_2)_7$-piperidinylene-$(CH_2)_2-$, $-(CH_2)_7$-piperidinylene-$(CH_2)_3-$, $-(CH_2)_7$-piperidinylene-$(CH_2)_4-$, $-(CH_2)_7$-piperidinylene-$(CH_2)_8-$, $-(CH_2)_8$-piperidinylene-$CH_2-$, $-(CH_2)_8$-piperidinylene-$(CH_2)_2-$, $-(CH_2)_8$-piperidinylene-$(CH_2)_3-$, $-(CH_2)_8$-piperidinylene-$(CH_2)_4-$, $-(CH_2)_8$-piperidinylene-$(CH_2)_5-$, $-(CH_2)_8$-piperidinylene-$(CH_2)_6-$, $-(CH_2)_8$-piperidinylene-$(CH_2)_7-$, $-(CH_2)_8$-piperidinylene-$(CH_2)_8-$, $-CH_2$-piperazinylene-$CH_2-$, $-CH_2$-piperazinylene-$(CH_2)_2-$, $-CH_2$-piperazinylene-$(CH_2)_3-$, $-CH_2$-piperazinylene-$(CH_2)_4-$, $-CH_2$-piperazinylene-$(CH_2)_5-$, $-CH_2$-piperazinylene-$(CH_2)_6-$, $-CH_2$-piperazinylene-$(CH_2)_7-$, $-CH_2$-piperazinylene-$(CH_2)_8-$, $-(CH_2)_2$-piperazinylene-$(CH_2)_1-$, $-(CH_2)_2$-piperazinylene-$(CH_2)_2-$, $-(CH_2)_2$-piperazinylene-$(CH_2)_3-$, $-(CH_2)_2$-piperazinylene-$(CH_2)_4-$, $-(CH_2)_2$-piperazinylene-$(CH_2)_5-$, $-(CH_2)_2$-piperazinylene-$(CH_2)_6-$, $-(CH_2)_2$-piperazinylene-$(CH_2)_7-$, $-(CH_2)_2$-piperazinylene-$(CH_2)_8-$, $-(CH_2)_3$-piperazinylene-$CH_2-$, $-(CH_2)_3$-piperazinylene-$(CH_2)_2-$, $-(CH_2)_3$-piperazinylene-$(CH_2)_3-$, $-(CH_2)_3$-piperazinylene-$(CH_2)_4-$, $-(CH_2)_3$-piperazinylene-$(CH_2)_5-$, $-(CH_2)_3$-piperazinylene-$(CH_2)_6-$, $-(CH_2)_3$-piperazinylene-$(CH_2)_7-$, $-(CH_2)_3$-piperazinylene-$(CH_2)_8-$, $-(CH_2)_4$-piperazinylene-$CH_2-$, $-(CH_2)_4$-piperazinylene-$(CH_2)_2-$, $-(CH_2)_4$-piperazinylene-$(CH_2)_3-$, $-(CH_2)_4$-piperazinylene-$(CH_2)_4-$, $-(CH_2)_4$-piperazinylene-$(CH_2)_5-$, $-(CH_2)_4$-piperazinylene-$(CH_2)_6-$, $-(CH_2)_4$-piperazinylene-$(CH_2)_7-$, $-(CH_2)_4$-piperazinylene-$(CH_2)_8-$, $-(CH_2)_5$-piperazinylene-$(CH_2)_1-$, $-(CH_2)_5$-piperazinylene-$(CH_2)_2-$, $-(CH_2)_5$-piperazinylene-$(CH_2)_3-$, $-(CH_2)_5$-piperazinylene-$(CH_2)_4-$, $-(CH_2)_5$-piperazinylene-$(CH_2)_5-$, $-(CH_2)_5$-piperazinylene-$(CH_2)_6-$, $-(CH_2)_5$-piperazinylene-$(CH_2)_7-$, $-(CH_2)_5$-piperazinylene-$(CH_2)_8-$, $-(CH_2)_6$-piperazinylene-$(CH_2)_1-$, $-(CH_2)_6$-piperazinylene-$(CH_2)_2-$, $-(CH_2)_6$-piperazinylene-$(CH_2)_3-$, $-(CH_2)_6$-piperazinylene-$(CH_2)_4-$, $-(CH_2)_6$-piperazinylene-$(CH_2)_5-$, $-(CH_2)_6$-piperazinylene-$(CH_2)_6-$, $-(CH_2)_6$-piperazinylene-$(CH_2)_7-$, $-(CH_2)_6$-piperazinylene-$(CH_2)_8-$, $-(CH_2)_7$-piperazinylene-$(CH_2)_1-$, $-(CH_2)_7$-piperazinylene-$(CH_2)_2-$, $-(CH_2)_7$-piperazinylene-$(CH_2)_3-$, $-(CH_2)_7$-piperazinylene-$(CH_2)_4-$, $-(CH_2)_7$-piperazinylene-$(CH_2)_8-$, $-(CH_2)_8$-piperazinylene-$CH_2-$, $-(CH_2)_8$-piperazinylene-$(CH_2)_2-$, $-(CH_2)_8$-piperazinylene-$(CH_2)_3-$, $-(CH_2)_8$-piperazinylene-$(CH_2)_4-$, $-(CH_2)_8$-piperazinylene-$(CH_2)_5-$, $-(CH_2)_8$-piperazinylene-$(CH_2)_6-$, $-(CH_2)_8$-piperazinylene-$(CH_2)_7-$, $-(CH_2)_8$-piperazinylene-$(CH_2)_8-$, $-CH_2$-propylene-$CH_2-$, $-CH_2$-propylene-$(CH_2)_2-$, $-CH_2$-propylene-$(CH_2)_3-$, $-CH_2$-propylene-$(CH_2)_4-$, $-CH_2$-propylene-$(CH_2)_5-$, $-CH_2$-propylene-$(CH_2)_6-$, $-CH_2$-propylene-$(CH_2)_7-$, $-CH_2$-propylene-$(CH_2)_8-$, $-(CH_2)_2$-propylene-$(CH_2)_1-$, $-(CH_2)_2$-propylene-$(CH_2)_2-$, $-(CH_2)_2$-propylene-$(CH_2)_3-$, $-(CH_2)_2$-propylene-$(CH_2)_4-$, $-(CH_2)_2$-propylene-$(CH_2)_5-$, $-(CH_2)_2$-propylene-$(CH_2)_6-$, $-(CH_2)_2$-propylene-$(CH_2)_7-$, $-(CH_2)_2$-propylene-$(CH_2)_8-$, $-(CH_2)_3$-propylene-$CH_2-$, $-(CH_2)_3$-propylene-$(CH_2)_2-$, $-(CH_2)_3$-propylene-$(CH_2)_3-$, $-(CH_2)_3$-propylene-$(CH_2)_4-$, $- (CH_2)_3$-propylene-$(CH_2)_5-$, $-(CH_2)_3$-propylene-$(CH_2)_6-$, $-(CH_2)_3$-propylene-$(CH_2)_7-$, $-(CH_2)_3$-propylene-$(CH_2)_8-$, $-(CH_2)_4$-propylene-$CH_2-$, $-(CH_2)_4$-propylene-$(CH_2)_2-$, $-(CH_2)_4$-propylene-$(CH_2)_3-$, $-(CH_2)_4$-propylene-$(CH_2)_4-$, $-(CH_2)_4$-propylene-$(CH_2)_5-$, $-(CH_2)_4$-propylene-$(CH_2)_6-$, $-(CH_2)_4$-propylene-$(CH_2)_7-$, $-(CH_2)_4$-propylene-$(CH_2)_8-$, $-(CH_2)_5$-propylene-$(CH_2)_1-$, $-(CH_2)_5$-propyle-

ne-$(CH_2)_2$-, -$(CH_2)_5$-propylene-$(CH_2)_3$-, -$(CH_2)_5$-propylene-$(CH_2)_4$-, -$(CH_2)_5$-propylene-$(CH_2)_5$-, -$(CH_2)_5$-propylene-$(CH_2)_6$-, -$(CH_2)_5$-propylene-$(CH_2)_7$-, -$(CH_2)_5$-propylene-$(CH_2)_8$-, -$(CH_2)_6$-propylene-$(CH_2)_1$-, -$(CH_2)_6$-propylene-$(CH_2)_2$-, -$(CH_2)_6$-propylene-$(CH_2)_3$-, -$(CH_2)_6$-propylene-$(CH_2)_4$-, -$(CH_2)_6$-propylene-$(CH_2)_5$-, -$(CH_2)_6$-propylene-$(CH_2)_6$-, -$(CH_2)_6$-propylene-$(CH_2)_7$-, -$(CH_2)_6$-propylene-$(CH_2)_8$-, -$(CH_2)_7$-propylene-$(CH_2)_1$-, -$(CH_2)_7$-propylene-$(CH_2)_2$-, -$(CH_2)_7$-propylene-$(CH_2)_3$-, -$(CH_2)_7$-propylene-$(CH_2)_4$-, -$(CH_2)_7$-propylene-$(CH_2)_8$-, -$(CH_2)_8$-propylene-$CH_2$-, -$(CH_2)_8$-propylene-$(CH_2)_2$-, -$(CH_2)_8$-propylene-$(CH_2)_3$-, -$(CH_2)_8$-propylene-$(CH_2)_4$-, -$(CH_2)_8$-propylene-$(CH_2)_5$-, -$(CH_2)_8$-propylene-$(CH_2)_6$-, -$(CH_2)_8$-propylene-$(CH_2)_7$-, -$(CH_2)_8$-propylene-$(CH_2)_8$-, -$CH_2$-diazacycloheptanylene-$CH_2$-, -$CH_2$-diazacycloheptanylene-$(CH_2)_2$-, -$CH_2$-diazacycloheptanylene-$(CH_2)_3$-, -$CH_2$-diazacycloheptanylene-$(CH_2)_4$-, -$CH_2$-diazacycloheptanylene-$(CH_2)_5$-, -$CH_2$-diazacycloheptanylene-$(CH_2)_6$-, -$CH_2$-diazacycloheptanylene-$(CH_2)_7$-, -$CH_2$-diazacycloheptanylene-$(CH_2)_8$-, -$(CH_2)_2$-diazacycloheptanylene-$(CH_2)_1$-, -$(CH_2)_2$-diazacycloheptanylene-$(CH_2)_2$-, -$(CH_2)_2$-diazacycloheptanylene-$(CH_2)_3$-, -$(CH_2)_2$-diazacycloheptanylene-$(CH_2)_4$-, -$(CH_2)_2$-diazacycloheptanylene-$(CH_2)_5$-, -$(CH_2)_2$-diazacycloheptanylene-$(CH_2)_6$-, -$(CH_2)_2$-diazacycloheptanylene-$(CH_2)_7$-, -$(CH_2)_2$-diazacycloheptanylene-$(CH_2)_8$-, -$(CH_2)_3$-diazacycloheptanylene-$CH_2$-, -$(CH_2)_3$-diazacycloheptanylene-$(CH_2)_2$-, -$(CH_2)_3$-diazacycloheptanylene-$(CH_2)_3$-, -$(CH_2)_3$-diazacycloheptanylene-$(CH_2)_4$-, -$(CH_2)_3$-diazacycloheptanylene-$(CH_2)_5$-, -$(CH_2)_3$-diazacycloheptanylene-$(CH_2)_6$-, -$(CH_2)_3$-diazacycloheptanylene-$(CH_2)_7$-, -$(CH_2)_3$-diazacycloheptanylene-$(CH_2)_8$-, -$(CH_2)_4$-diazacycloheptanylene-$CH_2$-, -$(CH_2)_4$-diazacycloheptanylene-$(CH_2)_2$-, -$(CH_2)_4$-diazacycloheptanylene-$(CH_2)_3$-, -$(CH_2)_4$-diazacycloheptanylene-$(CH_2)_4$-, -$(CH_2)_4$-diazacycloheptanylene-$(CH_2)_5$-, -$(CH_2)_4$-diazacycloheptanylene-$(CH_2)_6$-, -$(CH_2)_4$-diazacycloheptanylene-$(CH_2)_7$-, -$(CH_2)_4$-diazacycloheptanylene-$(CH_2)_8$-, -$(CH_2)_5$-diazacycloheptanylene-$(CH_2)_i$-, -$(CH_2)_5$-diazacycloheptanylene-$(CH_2)_2$-, -$(CH_2)_5$-diazacycloheptanylene-$(CH_2)_3$-, -$(CH_2)_5$-diazacycloheptanylene-$(CH_2)_4$-, -$(CH_2)_5$-diazacycloheptanylene-$(CH_2)_5$-, -$(CH_2)_5$-diazacycloheptanylene-$(CH_2)_6$-, -$(CH_2)_5$-diazacycloheptanylene-$(CH_2)_7$-, -$(CH_2)_5$-diazacycloheptanylene-$(CH_2)_8$-, -$(CH_2)_6$-diazacycloheptanylene-$(CH_2)_i$-, -$(CH_2)_6$-diazacycloheptanylene-$(CH_2)_2$-, -$(CH_2)_6$-diazacycloheptanylene-$(CH_2)_3$-, -$(CH_2)_6$-diazacycloheptanylene-$(CH_2)_4$-, -$(CH_2)_6$-diazacycloheptanylene-$(CH_2)_5$-, -$(CH_2)_6$-diazacycloheptanylene-$(CH_2)_6$-, -$(CH_2)_6$-diazacycloheptanylene-$(CH_2)_7$-, -$(CH_2)_6$-diazacycloheptanylene-$(CH_2)_8$-, -$(CH_2)_7$-diazacycloheptanylene-$(CH_2)_i$-, -$(CH_2)_7$-diazacycloheptanylene-$(CH_2)_2$-, -$(CH_2)_7$-diazacycloheptanylene-$(CH_2)_3$-, -$(CH_2)_7$-diazacycloheptanylene-$(CH_2)_4$-, -$(CH_2)_7$-diazacycloheptanylene-$(CH_2)_8$-, -$(CH_2)_8$-diazacycloheptanylene-$CH_2$-, -$(CH_2)_8$-diazacycloheptanylene-$(CH_2)_2$-, -$(CH_2)_8$-diazacycloheptanylene-$(CH_2)_3$-, -$(CH_2)_8$-diazacycloheptanylene-$(CH_2)_4$-, -$(CH_2)_8$-diazacycloheptanylene-$(CH_2)_5$-, -$(CH_2)_8$-diazacycloheptanylene-$(CH_2)_6$-, -$(CH_2)_8$-diazacycloheptanylene-$(CH_2)_7$-, -$(CH_2)_8$-diazacycloheptanylene-$(CH_2)_8$-, -$CH_2$-diazabicyclo[2.2.1]heptanylene-$CH_2$-, -$CH_2$-diazabicyclo[2.2.1]heptanylene-$(CH_2)_2$-, -$CH_2$-diazabicyclo[2.2.1]heptanylene-$(CH_2)_3$-, -$CH_2$-diazabicyclo[2.2.1]heptanylene-$(CH_2)_4$-, -$CH_2$-diazabicyclo[2.2.1]heptanylene-$(CH_2)_5$-, -$CH_2$-diazabicyclo[2.2.1]heptanylene-$(CH_2)_6$-, -$CH_2$-diazabicyclo[2.2.1]heptanylene-$(CH_2)_7$-, -$CH_2$-diazabicyclo[2.2.1]heptanylene-$(CH_2)_8$-, -$(CH_2)_2$-diazabicyclo[2.2.1]heptanylene-$(CH_2)_1$-, -$(CH_2)_2$-diazabicyclo[2.2.1]heptanylene-$(CH_2)_2$-, -$(CH_2)_2$-diazabicyclo[2.2.1]heptanylene-$(CH_2)_3$-, -$(CH_2)_2$-diazabicyclo[2.2.1]heptanylene-$(CH_2)_4$-, -$(CH_2)_2$-diazabicyclo[2.2.1]heptanylene-$(CH_2)_5$-, -$(CH_2)_2$-diazabicyclo[2.2.1]heptanylene-$(CH_2)_6$-, -$(CH_2)_2$-diazabicyclo[2.2.1]heptanylene-$(CH_2)_7$-, -$(CH_2)_2$-diazabicyclo[2.2.1]heptanylene-$(CH_2)_8$-, -$(CH_2)_3$-diazabicyclo[2.2.1]heptanylene-$CH_2$-, -$(CH_2)_3$-diazabicyclo[2.2.1]heptanylene-$(CH_2)_2$-, -$(CH_2)_3$-diazabicyclo[2.2.1]heptanylene-$(CH_2)_3$-, -$(CH_2)_3$-diazabicyclo[2.2.1]heptanylene-$(CH_2)_4$-, -$(CH_2)_3$-diazabicyclo[2.2.1]heptanylene-$(CH_2)_5$-, -$(CH_2)_3$-diazabicyclo[2.2.1]heptanylene-$(CH_2)_6$-, -$(CH_2)_3$-diazabicyclo[2.2.1]heptanylene-$(CH_2)_7$-, -$(CH_2)_3$-diazabicyclo[2.2.1]heptanylene-$(CH_Z)_s$-, -$(CH_2)_4$-diazabicyclo[2.2.1]heptanylene-$CH_2$-, -$(CH_2)_4$-diazabicyclo[2.2.1]heptanylene-$(CH_2)_2$-, -$(CH_2)_4$-diazabicyclo[2.2.1]heptanylene-$(CH_2)_3$-, -$(CH_2)_4$-diazabicyclo[2.2.1]heptanylene-$(CH_2)_4$-, -$(CH_2)_4$-diazabicyclo[2.2.1]heptanylene-$(CH_2)_5$-, -$(CH_2)_4$-diazabicyclo[2.2.1]heptanylene-$(CH_2)_6$-, -$(CH_2)_4$-diazabicyclo[2.2.1]heptanylene-$(CH_2)_7$-, -$(CH_2)_4$-diazabicyclo[2.2.1]heptanylene-$(CH_2)_8$-, -$(CH_2)_5$-diazabicyclo[2.2.1]heptanylene-$(CH_2)_1$-, -$(CH_2)_5$-diazabicyclo[2.2.1]heptanylene-$(CH_2)_2$-, -$(CH_2)_5$-diazabicyclo[2.2.1]heptanylene-$(CH_2)_3$-, -$(CH_2)_5$-diazabicyclo[2.2.1]heptanylene-$(CH_2)_4$-, -$(CH_2)_5$-diazabicyclo[2.2.1]heptanylene-$(CH_2)_5$-, -$(CH_2)_5$-diazabicyclo[2.2.1]heptanylene-$(CH_2)_6$-, -$(CH_2)_5$-diazabicyclo[2.2.1]heptanylene-$(CH_2)_7$-, -$(CH_2)_5$-diazabicyclo[2.2.1]heptanylene-$(CH_2)_8$-, -$(CH_2)_6$-diazabicyclo[2.2.1]heptanylene-$(CH_2)_1$-, -$(CH_2)_6$-diazabicyclo[2.2.1]heptanylene-$(CH_2)_2$-, -$(CH_2)_6$-diazabicyclo[2.2.1]heptanylene-$(CH_2)_3$-, -$(CH_2)_6$-diazabicyclo[2.2.1]heptanylene-$(CH_2)_4$-, -$(CH_2)_6$-diazabicyclo[2.2.1]heptanylene-$(CH_2)_5$-, -$(CH_2)_6$-diazabicyclo[2.2.1]heptanylene-$(CH_2)_6$-, -$(CH_2)_6$-diazabicyclo[2.2.1]heptanylene-$(CH_2)_7$-, -$(CH_2)_6$-diazabicyclo[2.2.1]heptanylene-$(CH_2)_8$-, -$(CH_2)_7$-diazabicyclo[2.2.1]heptanylene-$(CH_2)_1$-, -$(CH_2)_7$-diazabicyclo[2.2.1]heptanylene-$(CH_2)_2$-, -$(CH_2)_7$-diazabicyclo[2.2.1]heptanylene-$(CH_2)_3$-, -$(CH_2)_7$-diazabicyclo[2.2.1]heptanylene-$(CH_2)_4$-, -$(CH_2)_7$-diazabicyclo[2.2.1]heptanylene-$(CH_2)_8$-, -$(CH_2)_8$-diazabicyclo[2.2.1]heptanylene-$CH_2$-, -$(CH_2)_8$-diazabicyclo[2.2.1]heptanylene-$(CH_2)_2$-, -$(CH_2)_8$-diazabicyclo[2.2.1]heptanylene-$(CH_2)_3$-, -$(CH_2)_8$-diazabicyclo[2.2.1]heptanylene-$(CH_2)_4$-, -$(CH_2)_8$-diazabicyclo[2.2.1]heptanylene-$(CH_2)_5$-, -$(CH_2)_8$-diazabicyclo[2.2.1]heptanylene-$(CH_2)_6$-, -$(CH_2)_8$-diazabicyclo[2.2.1]heptanylene-$(CH_2)_7$-, -$(CH_2)_8$-diazabicyclo[2.2.1]heptanylene-$(CH_2)_8$-, -$CH_2$-diazabicyclo[3.1.1]heptanylene-$CH_2$-, -$CH_2$-diazabicyclo[3.1.1]heptanylene-$(CH_2)_2$-, -$CH_2$-diazabicyclo[3.1.1]heptanylene-$(CH_2)_3$-, -$CH_2$-diazabicyclo[3.1.1]heptanylene-$(CH_2)_4$-, -$CH_2$-diazabicyclo[3.1.1]heptanylene-$(CH_2)_5$-, -$CH_2$-diazabicyclo[3.1.1]heptanylene-$(CH_2)_6$-, -$CH_2$-diazabicyclo[3.1.1]heptanylene-$(CH_2)_7$-, -$CH_2$-diazabicyclo[3.1.1]heptanylene-$(CH_2)_8$-, -$(CH_2)_2$-diazabicyclo[3.1.1]heptanylene-$(CH_2)_1$-, -$(CH_2)_2$-diazabicyclo[3.1.1]heptanylene-$(CH_2)_2$-,

$-(CH_2)_2$-diazabicyclo[3.1.1]heptanylene-$(CH_2)_3$-, $-(CH_2)_2$-diazabicyclo[3.1.1]heptanylene-$(CH_2)_4$-, $-(CH_2)_2$-diazabicyclo[3.1.1]heptanylene-$(CH_2)_5$-, $-(CH_2)_2$-diazabicyclo[3.1.1]heptanylene-$(CH_2)_6$-, $-(CH_2)_2$-diazabicyclo[3.1.1]heptanylene-$(CH_2)_7$-, $-(CH_2)_2$-diazabicyclo[3.1.1]heptanylene-$(CH_2)_8$-, $-(CH_2)_3$-diazabicyclo[3.1.1]heptanylene-$CH_2$-, $-(CH_2)_3$-diazabicyclo[3.1.1]heptanylene-$(CH_2)_2$-, $-(CH_2)_3$-diazabicyclo[3.1.1]heptanylene-$(CH_2)_3$-, $-(CH_2)_3$-diazabicyclo[3.1.1]heptanylene-$(CH_2)_4$-, $-(CH_2)_3$-diazabicyclo[3.1.1]heptanylene-$(CH_2)_5$-, $-(CH_2)_3$-diazabicyclo[3.1.1]heptanylene-$(CH_2)_6$-, $-(CH_2)_3$-diazabicyclo[3.1.1]heptanylene-$(CH_2)_7$-, $-(CH_2)_3$-diazabicyclo[3.1.1]heptanylene-$(CH_2)_8$-, $-(CH_2)_4$-diazabicyclo[3.1.1]heptanylene-$CH_2$-, $-(CH_2)_4$-diazabicyclo[3.1.1]heptanylene-$(CH_2)_2$-, $-(CH_2)_4$-diazabicyclo[3.1.1]heptanylene-$(CH_2)_3$-, $-(CH_2)_4$-diazabicyclo[3.1.1]heptanylene-$(CH_2)_4$-, $-(CH_2)_4$-diazabicyclo[3.1.1]heptanylene-$(CH_2)_5$-, $-(CH_2)_4$-diazabicyclo[3.1.1]heptanylene-$(CH_2)_6$-, $-(CH_2)_4$-diazabicyclo[3.1.1]heptanylene-$(CH_2)_7$-, $-(CH_2)_4$-diazabicyclo[3.1.1]heptanylene-$(CH_2)_8$-, $-(CH_2)_5$-diazabicyclo[3.1.1]heptanylene-$(CH_2)_1$-, $-(CH_2)_5$-diazabicyclo[3.1.1]heptanylene-$(CH_2)_2$-, $-(CH_2)_5$-diazabicyclo[3.1.1]heptanylene-$(CH_2)_3$-, $-(CH_2)_5$-diazabicyclo[3.1.1]heptanylene-$(CH_2)_4$-, $-(CH_2)_5$-diazabicyclo[3. 1. 1]heptanylene-$(CH_2)_5$-, $-(CH_2)_5$-diazabicyclo[3.1.1]heptanylene-$(CH_2)_6$-, $-(CH_2)_5$-diazabicyclo[3.1.1]heptanylene-$(CH_2)_7$-, $-(CH_2)_5$-diazabicyclo[3.1.1]heptanylene-$(CH_2)_8$-, $-(CH_2)_6$-diazabicyclo[3.1.1]heptanylene-$(CH_2)_1$-, $-(CH_2)_6$-diazabicyclo[3.1.1]heptanylene-$(CH_2)_2$-, $-(CH_2)_6$-diazabicyclo[3.1.1]heptanylene-$(CH_2)_3$-, $-(CH_2)_6$-diazabicyclo[3.1.1]heptanylene-$(CH_2)_4$-, $-(CH_2)_6$-diazabicyclo[3.1.1]heptanylene-$(CH_2)_5$-, $-(CH_2)_6$-diazabicyclo[3.1.1]heptanylene-$(CH_2)_6$-, $-(CH_2)_6$-diazabicyclo[3.1.1]heptanylene-$(CH_2)_7$-, $-(CH_2)_6$-diazabicyclo[3.1.1]heptanylene-$(CH_2)_8$-, $-(CH_2)_7$-diazabicyclo[3.1.1]heptanylene-$(CH_2)_1$-, $-(CH_2)_7$-diazabicyclo[3.1.1]heptanylene-$(CH_2)_2$-, $-(CH_2)_7$-diazabicyclo[3.1.1]heptanylene-$(CH_2)_3$-, $-(CH_2)_7$-diazabicyclo[3.1.1]heptanylene-$(CH_2)_4$-, $-(CH_2)_7$-diazabicyclo[3.1.1]heptanylene-$(CH_2)_8$-, $-(CH_2)_8$-diazabicyclo[3.1.1]heptanylene-$CH_2$-, $-(CH_2)_8$-diazabicyclo[3.1.1]heptanylene-$(CH_2)_2$-, $-(CH_2)_8$-diazabicyclo[3.1.1]heptanylene-$(CH_2)_3$-, $-(CH_2)_8$-diazabicyclo[3.1.1]heptanylene-$(CH_2)_4$-, $-(CH_2)_8$-diazabicyclo[3.1.1]heptanylene-$(CH_2)_5$-, $-(CH_2)_8$-diazabicyclo[3.1.1]heptanylene-$(CH_2)_6$-, $-(CH_2)_8$-diazabicyclo[3.1.1]heptanylene-$(CH_2)_7$-, $-(CH_2)_8$-diazabicyclo[3.1.1]heptanylene-$(CH_2)_8$-, $-CH_2$-diazabicyclo[3.2.1]octylene-$CH_2$-, $-CH_2$-diazabicyclo[3.2.1]octylene-$(CH_2)_2$-, $-CH_2$-diazabicyclo[3.2.1]octylene-$(CH_2)_3$-, $-CH_2$-diazabicyclo[3.2.1]octylene-$(CH_2)_4$-, $-CH_2$-diazabicyclo[3.2.1]octylene-$(CH_2)_5$-, $-CH_2$-diazabicyclo[3.2.1]octylene-$(CH_2)_6$-, $-CH_2$-diazabicyclo[3.2.1]octylene-$(CH_2)_7$-, $-CH_2$-diazabicyclo[3.2.1]octylene-$(CH_2)_8$-, $-(CH_2)_2$-diazabicyclo[3.2.1]octylene-$(CH_2)_1$-, $-(CH_2)_2$-diazabicyclo[3.2.1]octylene-$(CH_2)_2$-, $-(CH_2)_2$-diazabicyclo[3.2.1]octylene-$(CH_2)_3$-, $-(CH_2)_2$-diazabicyclo[3.2.1]octylene-$(CH_2)_4$-, $-(CH_2)_2$-diazabicyclo[3.2.1]octylene-$(CH_2)_5$-, $-(CH_2)_2$-diazabicyclo[3.2.1]octylene-$(CH_2)_6$-, $-(CH_2)_2$-diazabicyclo[3.2.1]octylene-$(CH_2)_7$-, $-(CH_2)_2$-diazabicyclo[3.2.1]octylene-$(CH_2)_8$-, $-(CH_2)_3$-diazabicyclo[3.2.1]octylene-$CH_2$-, $-(CH_2)_3$-diazabicyclo[3.2.1]octylene-$(CH_2)_2$-, $-(CH_2)_3$-diazabicyclo[3.2.1]octylene-$(CH_2)_3$-, $-(CH_2)_3$-diazabicyclo[3.2.1]octylene-$(CH_2)_4$-, $-(CH_2)_3$-diazabicyclo[3.2.1]octylene-$(CH_2)_5$-, $-(CH_2)_3$-diazabicyclo[3.2.1]octylene-$(CH_2)_6$-, $-(CH_2)_3$-diazabicyclo[3.2.1]octylene-$(CH_2)_7$-, $-(CH_2)_3$-diazabicyclo[3.2.1]octylene-$(CH_2)_8$-, $-(CH_2)_4$-diazabicyclo[3.2.1]octylene-$CH_2$-, $-(CH_2)_4$-diazabicyclo[3.2.1]octylene-$(CH_2)_2$-, $-(CH_2)_4$-diazabicyclo[3.2.1]octylene-$(CH_2)_3$-, $-(CH_2)_4$-diazabicyclo[3.2.1]octylene-$(CH_2)_4$-, $-(CH_2)_4$-diazabicyclo[3.2.1]octylene-$(CH_2)_5$-, $-(CH_2)_4$-diazabicyclo[3.2.1]octylene-$(CH_2)_6$-, $-(CH_2)_4$-diazabicyclo[3.2.1]octylene-$(CH_2)_7$-, $-(CH_2)_4$-diazabicyclo[3.2.1]octylene-$(CH_2)_8$-, $-(CH_2)_5$-diazabicyclo[3.2.1]octylene-$(CH_2)_1$-, $-(CH_2)_5$-diazabicyclo[3.2.1]octylene-$(CH_2)_2$-, $-(CH_2)_5$-diazabicyclo[3.2.1]octylene-$(CH_2)_3$-, $-(CH_2)_5$-diazabicyclo[3.2.1]octylene-$(CH_2)_4$-, $-(CH_2)_5$-diazabicyclo[3.2.1]octylene-$(CH_2)_5$-, $-(CH_2)_5$-diazabicyclo[3.2.1]octylene-$(CH_2)_6$-, $-(CH_2)_5$-diazabicyclo[3.2.1]octylene-$(CH_2)_7$-, $-(CH_2)_5$-diazabicyclo[3.2.1]octylene-$(CH_2)_8$-, $-(CH_2)_6$-diazabicyclo[3.2.1]octylene-$(CH_2)_1$-, $-(CH_2)_6$-diazabicyclo[3.2.1]octylene-$(CH_2)_2$-, $-(CH_2)_6$-diazabicyclo[3.2.1]octylene-$(CH_2)_3$-, $-(CH_2)_6$-diazabicyclo[3.2.1]octylene-$(CH_2)_4$-, $-(CH_2)_6$-diazabicyclo[3.2.1]octylene-$(CH_2)_5$-, $-(CH_2)_6$-diazabicyclo[3.2.1]octylene-$(CH_2)_6$-, $-(CH_2)_6$-diazabicyclo[3.2.1]octylene-$(CH_2)_7$-, $-(CH_2)_6$-diazabicyclo[3.2.1]octylene-$(CH_2)_8$-, $-(CH_2)_7$-diazabicyclo[3.2.1]octylene-$(CH_2)_1$-, $-(CH_2)_7$-diazabicyclo[3.2.1]octylene-$(CH_2)_2$-, $-(CH_2)_7$-diazabicyclo[3.2.1]octylene-$(CH_2)_3$-, $-(CH_2)_7$-diazabicyclo[3.2.1]octylene-$(CH_2)_4$-, $-(CH_2)_7$-diazabicyclo[3.2.1]octylene-$(CH_2)_8$-, $-(CH_2)_8$-diazabicyclo[3.2.1]octylene-$CH_2$-, $-(CH_2)_8$-diazabicyclo[3.2.1]octylene-$(CH_2)_2$-, $-(CH_2)_8$-diazabicyclo[3.2.1]octylene-$(CH_2)_3$-, $-(CH_2)_8$-diazabicyclo[3.2.1]octylene-$(CH_2)_4$-, $-(CH_2)_8$-diazabicyclo[3.2.1]octylene-$(CH_2)_5$-, $-(CH_2)_8$-diazabicyclo[3.2.1]octylene-$(CH_2)_6$-, $-(CH_2)_8$-diazabicyclo[3.2.1]octylene-$(CH_2)_7$-, $-(CH_2)_8$-diazabicyclo[3.2.1]octylene-$(CH_2)_8$-, $-CH_2$-diazabicyclo[2.2.2]octylene-$CH_2$-, $-CH_2$-diazabicyclo[2.2.2]octylene-$(CH_2)_2$-, $-CH_2$-diazabicyclo[2.2.2]octylene-$(CH_2)_3$-, $-CH_2$-diazabicyclo[2.2.2]octylene-$(CH_2)_4$-, $-CH_2$-diazabicyclo[2.2.2]octylene-$(CH_2)_5$-, $-CH_2$-diazabicyclo[2.2.2]octylene-$(CH_2)_6$-, $-CH_2$-diazabicyclo[2.2.2]octylene-$(CH_2)_7$-, $-CH_2$-diazabicyclo[2.2.2]octylene-$(CH_2)_8$-, $-(CH_2)_2$-diazabicyclo[2.2.2]octylene-$(CH_2)_1$-, $-(CH_2)_2$-diazabicyclo[2.2.2]octylene-$(CH_2)_2$-, $-(CH_2)_2$-diazabicyclo[2.2.2]octylene-$(CH_2)_3$-, $-(CH_2)_2$-diazabicyclo[2.2.2]octylene-$(CH_2)_4$-, $-(CH_2)_2$-diazabicyclo[2.2.2]octylene-$(CH_2)_5$-, $-(CH_2)_2$-diazabicyclo[2.2.2]octylene-$(CH_2)_6$-, $-(CH_2)_2$-diazabicyclo[2.2.2]octylene-$(CH_2)_7$-, $-(CH_2)_2$-diazabicyclo[2.2.2]octylene-$(CH_2)_8$-, $-(CH_2)_3$-diazabicyclo[2.2.2]octylene-$CH_2$-, $-(CH_2)_3$-diazabicyclo[2.2.2]octylene-$(CH_2)_2$-, $-(CH_2)_3$-diazabicyclo[2.2.2]octylene-$(CH_2)_3$-, $-(CH_2)_3$-diazabicyclo[2.2.2]octylene-$(CH_2)_4$-, $-(CH_2)_3$-diazabicyclo[2.2.2]octylene-$(CH_2)_5$-, $-(CH_2)_3$-diazabicyclo[2.2.2]octylene-$(CH_2)_6$-, $-(CH_2)_3$-diazabicyclo[2.2.2]octylene-$(CH_2)_7$-, $-(CH_2)_3$-diazabicyclo[2.2.2]octylene-$(CH_2)_8$-, $-(CH_2)_4$-diazabicyclo[2.2.2]octylene-$CH_2$-, $-(CH_2)_4$-diazabicyclo[2.2.2]octylene-$(CH_2)_2$-, $-(CH_2)_4$-diazabicyclo[2.2.2]octylene-$(CH_2)_3$-, $-(CH_2)_4$-diazabicyclo[2.2.2]octylene-$(CH_2)_4$-, $-(CH_2)_4$-diazabicyclo[2.2.2]octylene-$(CH_2)_5$-, $-(CH_2)_4$-diazabicyclo[2.2.2]octylene-$(CH_2)_6$-, $-(CH_2)_4$-diazabicyclo[2.2.2]octylene-$(CH_2)_7$-, $-(CH_2)_4$-diazabicyclo[2.2.2]octylene-$(CH_2)_8$-, $-(CH_2)_5$-diazabicyclo[2.2.2]octyle-

ne-$(CH_2)_1$-, -$(CH_2)_5$-diazabicyclo[2.2.2]octylene-$(CH_2)_2$-, -$(CH_2)_5$-diazabicyclo[2.2.2]octylene-$(CH_2)_3$-, -$(CH_2)_5$-diazabicyclo[2.2.2]octylene-$(CH_2)_4$-, -$(CH_2)_5$-diazabicyclo[2.2.2]octylene-$(CH_2)_5$-, -$(CH_2)_5$-diazabicyclo[2.2.2]octylene-$(CH_2)_6$-, -$(CH_2)_5$-diazabicyclo[2.2.2]octylene-$(CH_2)_7$-, -$(CH_2)_5$-diazabicyclo[2.2.2]octylene-$(CH_2)_8$-, -$(CH_2)_6$-diazabicyclo[2.2.2]octylene-$(CH_2)_1$-, -$(CH_2)_6$-diazabicyclo[2.2.2]octylene-$(CH_2)_2$-, -$(CH_2)_6$-diazabicyclo[2.2.2]octylene-$(CH_2)_3$-, -$(CH_2)_6$-diazabicyclo[2.2.2]octylene-$(CH_2)_4$-, -$(CH_2)_6$-diazabicyclo[2.2.2]octylene-$(CH_2)_5$-, -$(CH_2)_6$-diazabicyclo[2.2.2]octylene-$(CH_2)_6$-, -$(CH_2)_6$-diazabicyclo[2.2.2]octylene-$(CH_2)_7$-, -$(CH_2)_6$-diazabicyclo[2.2.2]octylene-$(CH_2)_8$-, -$(CH_2)_7$-diazabicyclo[2.2.2]octylene-$(CH_2)_1$-, -$(CH_2)_7$-diazabicyclo[2.2.2]octylene-$(CH_2)_2$-, -$(CH_2)_7$-diazabicyclo[2.2.2]octylene-$(CH_2)_3$-, -$(CH_2)_7$-diazabicyclo[2.2.2]octylene-$(CH_2)_4$-, -$(CH_2)_7$-diazabicyclo[2.2.2]octylene-$(CH_2)_8$-, -$(CH_2)_8$-diazabicyclo[2.2.2]octylene-$CH_2$-, -$(CH_2)_8$-diazabicyclo[2.2.2]octylene-$(CH_2)_2$-, -$(CH_2)_8$-diazabicyclo[2.2.2]octylene-$(CH_2)_3$-, -$(CH_2)_8$-diazabicyclo[2.2.2]octylene-$(CH_2)_4$-, -$(CH_2)_8$-diazabicyclo[2.2.2]octylene-$(CH_2)_5$-, -$(CH_2)_8$-diazabicyclo[2.2.2]octylene-$(CH_2)_6$-, -$(CH_2)_8$-diazabicyclo[2.2.2]octylene-$(CH_2)_7$-, or -$(CH_2)_8$-diazabicyclo [2.2.2]octylene-$(CH_2)_8$-;

wherein the propylene, the piperidinylene, the piperazinylene, the diazacycloheptanylene, the diazabicyclo[2.2.1] heptanylene, the diazabicyclo[3.1.1]heptanylene, the diazabicyclo[3.2.1]octylene, the diazabicyclo[2.2.2]octylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof; and

each $R_{g11}$ independently represents H or $C_{1-3}$ alkyl.

In some embodiments of the compound of Formula (I-4), $L_2$ represents the structure of the following formula:

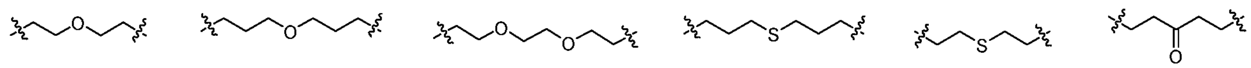

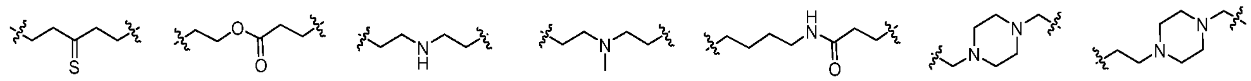

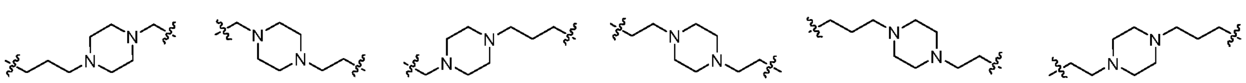

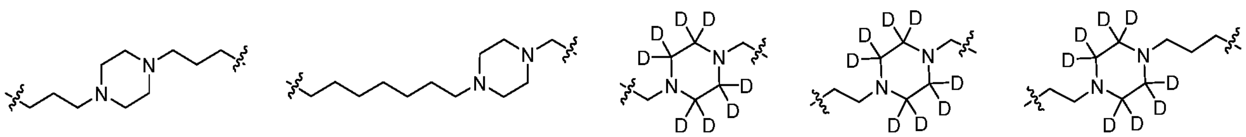

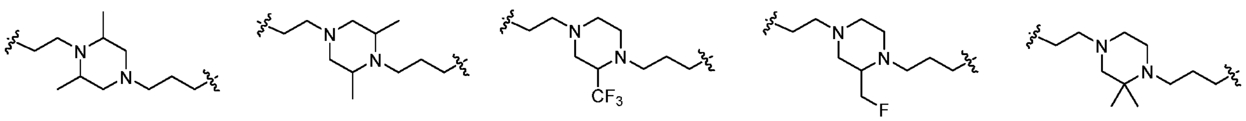

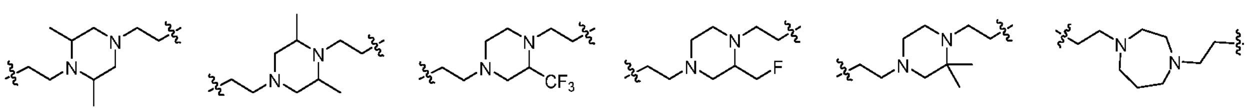

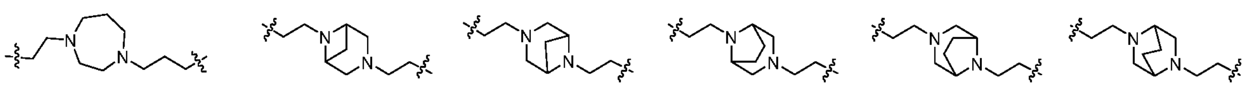

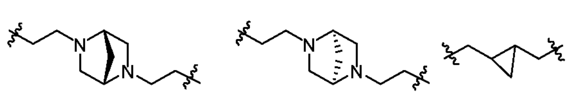 or 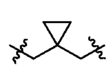

In some embodiments of the compound of Formula (I-4), $R_{b1}$ represents:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, 2-oxobicyclo[2.2.1]heptanyl or bicyclo[2.2.1]heptenyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 3-azaspiro[5.5]undecyl or 7-azaspiro[3.5]nonyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

phenyl or naphthyl, with each group being optionally substituted with one or more (e.g., 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl and imidazo[2,1-b]thiazolyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

In some embodiments of the compound of Formula (I-4), $R_{b1}$ represents:

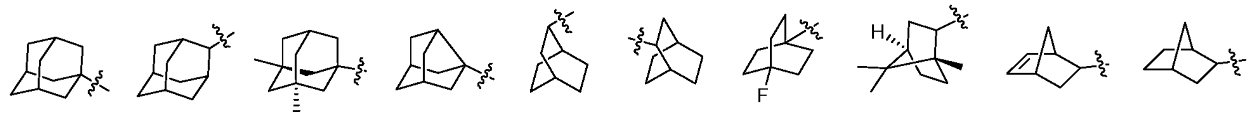

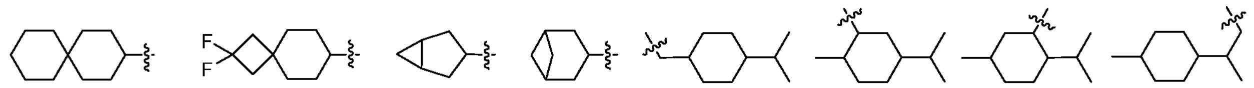

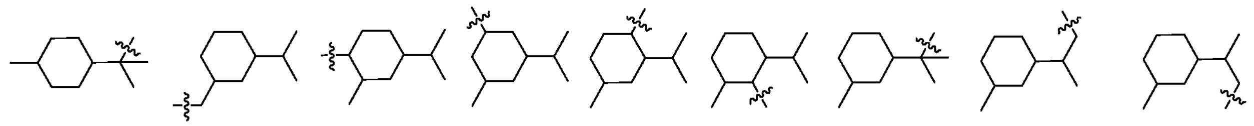

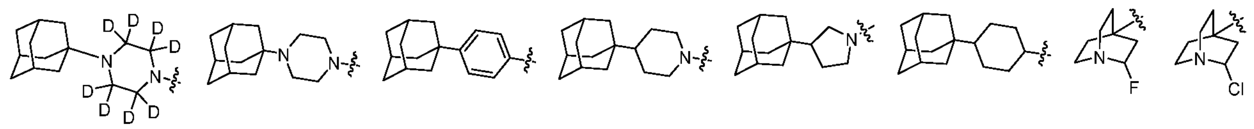

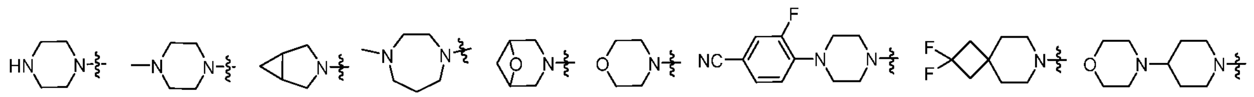

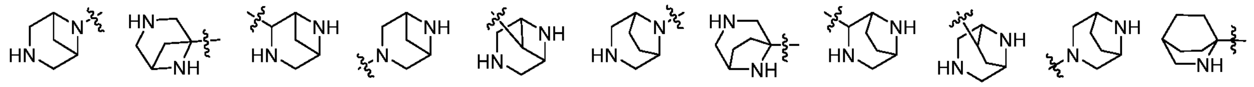

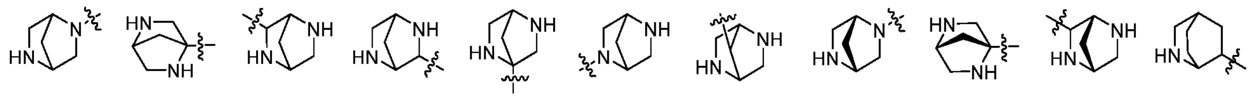

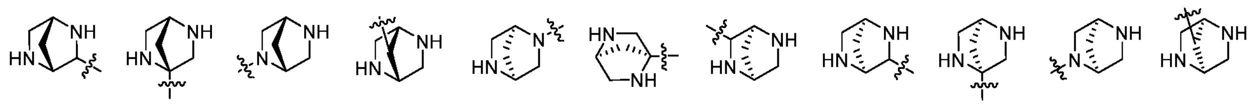

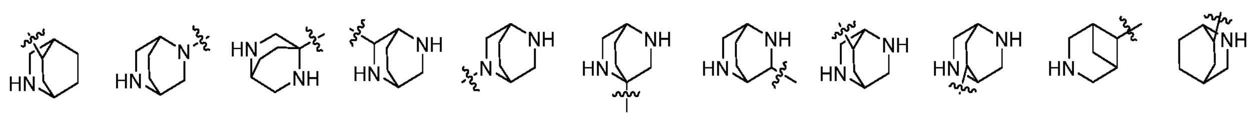

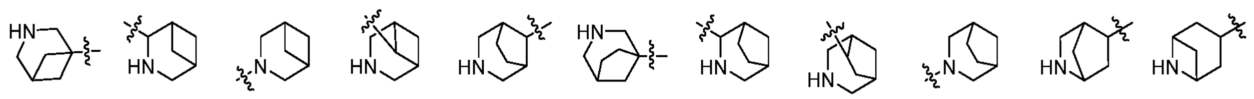

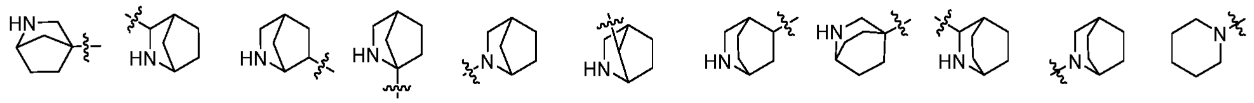

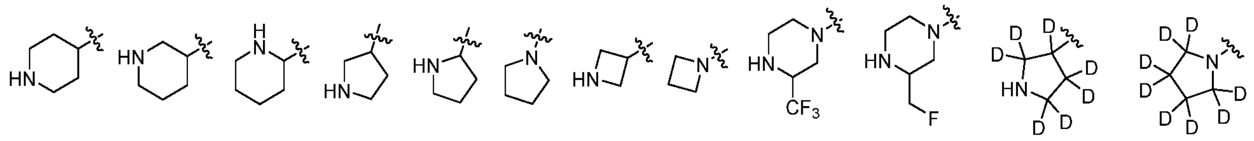

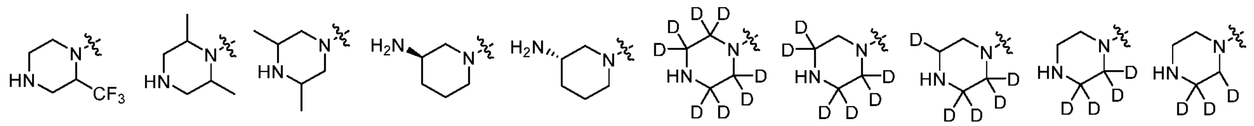

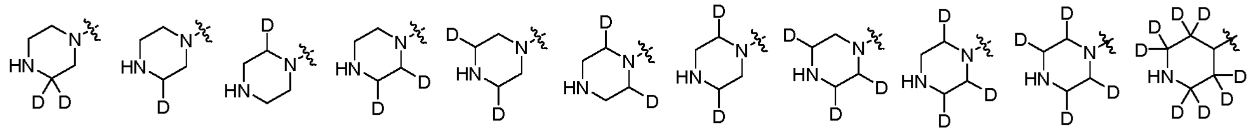

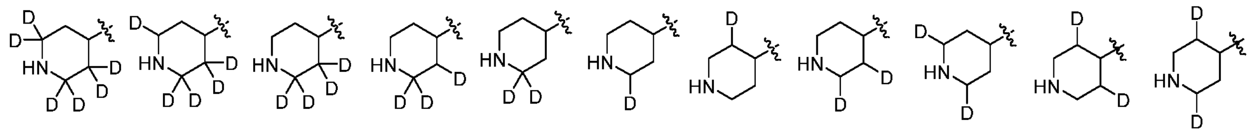

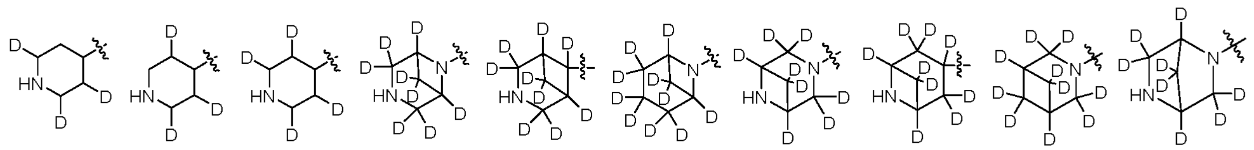

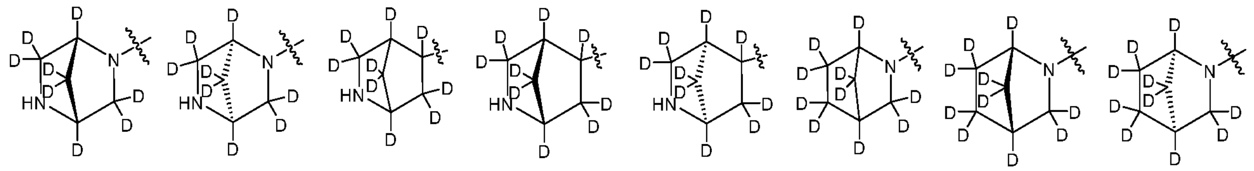

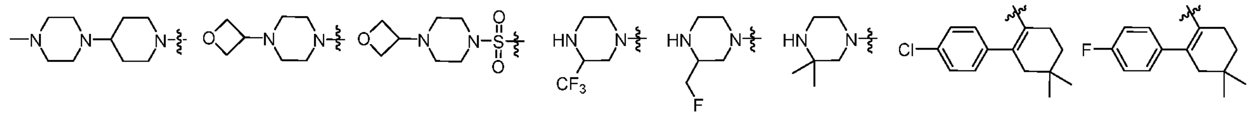

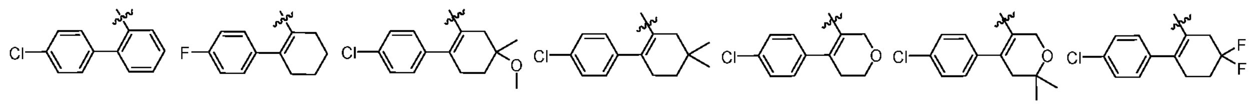

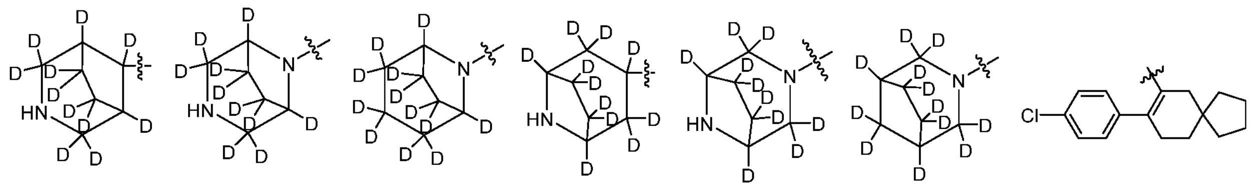

or

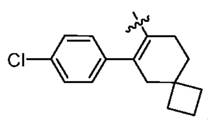

In some embodiments, the compound of Formula (I) is also of Formula (I-5):

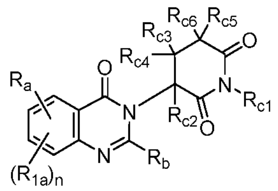

Formula (I-5)

wherein $R_{c1}$, $R_{c2}$, $R_{c3}$, $R_{c4}$, $R_{c5}$, $R_{c6}$, $R_b$, $(R_{1a})_n$, $R_a$, $R_{1a}$ and n are as defined in the compound of Formula (I) above and the embodiments thereof;

wherein $R_a$ represents the following formula:

$$R_{a1}\text{-}X_2\text{-}L_1\text{-}X_1\text{-} ,$$

wherein $X_1$ represents alkynylene (e.g., $C_2$-$C_6$ alkynylene) or alkenylene (e.g., $C_2$-$C_6$ alkenylene), or $X_1$ represents a bond;
$L_1$ represents optionally substituted linear or branched $C_{1-60}$ alkylene (e.g., $C_1$-$C_{30}$ alkylene, $C_1$-$C_{29}$ alkylene, $C_1$-$C_{28}$ alkylene, $C_1$-$C_{27}$ alkylene, $C_1$-$C_{26}$ alkylene, $C_1$-$C_{25}$ alkylene, $C_1$-$C_{24}$ alkylene, $C_1$-$C_{23}$ alkylene, $C_1$-$C_{22}$ alkylene, $C_1$-$C_{21}$ alkylene, $C_1$-$C_{20}$ alkylene, $C_1$-$C_{19}$ alkylene, $C_1$-$C_{18}$ alkylene, $C_1$-$C_{17}$ alkylene, $C_1$-$C_{16}$ alkylene, $C_1$-$C_{15}$ alkylene, $C_1$-$C_{14}$ alkylene, $C_1$-$C_{13}$ alkylene, $C_1$-$C_{12}$ alkylene, $C_1$-$C_{11}$ alkylene, $C_1$-$C_{10}$ alkylene, $C_1$-$C_9$ alkylene, $C_1$-$C_8$ alkylene, $C_1$-$C_7$ alkylene, $C_1$-$C_6$ alkylene, $C_1$-$C_5$ alkylene, $C_1$-$C_4$ alkylene, $C_1$-$C_3$ alkylene, or $C_1$-$C_2$ alkylene); $X_2$ represents $N(R_5)$, C(O)O, OC(O), C(O)NH, NHC(O), C(O), O, C(S), S, S(O), $S(O)_2$, $S(O)_2NH$, $NHS(O)_2$, $S(O)_2O$, or $OS(O)_2$, wherein $R_5$ represents H or $C_{1-3}$ alkyl, or $X_2$ represents a bond; and $R_{a1}$ represents optionally substituted heterocyclyl (e.g., optionally substituted 4- to 20-membered heterocyclyl, or optionally substituted 5- to 15-membered heterocyclyl);
and
$R_b$ represents $C_{1-60}$ alkyl (e.g., $C_1$-$C_{30}$ alkylene, $C_1$-$C_{29}$ alkylene, $C_1$-$C_{28}$ alkylene, $C_1$-$C_{27}$ alkylene, $C_1$-$C_{26}$ alkylene, $C_1$-$C_{25}$ alkylene, $C_1$-$C_{24}$ alkylene, $C_1$-$C_{23}$ alkylene, $C_1$-$C_{22}$ alkylene, $C_1$-$C_{21}$ alkylene, $C_1$-$C_{20}$ alkylene, $C_1$-$C_{19}$ alkylene, $C_1$-$C_{18}$ alkylene, $C_1$-$C_{17}$ alkylene, $C_1$-$C_{16}$ alkylene, $C_1$-$C_{15}$ alkylene, $C_1$-$C_{14}$ alkylene, $C_1$-$C_{13}$ alkylene, $C_1$-$C_{12}$ alkylene, $C_1$-$C_{11}$ alkylene, $C_1$-$C_{10}$ alkylene, $C_1$-$C_9$ alkylene, $C_1$-$C_8$ alkylene, $C_1$-$C_7$ alkylene, $C_1$-$C_6$ alkylene, $C_1$-$C_5$ alkylene, $C_1$-$C_4$ alkylene, $C_1$-$C_3$ alkylene, or $C_1$-$C_2$ alkylene) optionally substituted with D or halogen, or
$R_b$ represents the following formula:

$$R_{b1}\text{-}X_4\text{-}L_2\text{-},$$

wherein $L_2$ represents:

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene), or
optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{12}$ and/or one or more (e.g., 1-20, or 1-15, 1-10, 1-9, 1-8, 1-7, 1-6, 1-5, 1-4, 1-3 or 1-2, or 1) groups $R_{13}$ and/or any combination of one or more (e.g., 1-20, or 1-15, 1-10, 1-9, 1-8, 1-7, 1-6, 1-5, 1-4, 1-3 or 1-2, or 1) groups $R_{12}$ and $R_{13}$ inserted into its backbone carbon chain, wherein carbon-carbon bond between one or more pairs (e.g., 1-20, or 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_{12}$, $R_{13}$, or a combination of

$R_{12}$ and $R_{13}$; wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of cycloalkylene (e.g., $C_{3-20}$cycloalkylene, or $C_{3-15}$cycloalkylene), heterocyclylene (e.g., 4-to 20-membered heterocyclylene, or 4- to 15-membered heterocyclylene), arylene (e.g., $C_{6-10}$ arylene), heteroarylene (e.g., 5- to 20-membered heteroarylene, or 5- to 15-membered heteroarylene), alkenylene (e.g., $C_{2-6}$ alkenylene), alkynylene (e.g., $C_{2-6}$ alkynylene) or any combination thereof, wherein the cycloalkylene, the heterocyclylene, arylene and the heteroarylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof;

$X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted cycloalkylene (e.g., optionally substituted $C_{3-20}$cycloalkylene, or optionally substituted $C_{3-15}$cycloalkylene), optionally substituted arylene (e.g., optionally substituted $C_{6-20}$ arylene, or optionally substituted $C_{6-10}$ arylene), optionally substituted heterocyclylene (e.g., optionally substituted 4- to 20-membered heterocyclylene, or optionally substituted 4- to 15-membered heterocyclylene), optionally substituted heteroarylene (e.g., optionally substituted 5- to 20-membered heteroarylene, or optionally substituted 5- to 15-membered heteroarylene), triazolylene-NH-, or -NH-triazolylene, wherein $R_{15}$ represents H or $C_{1-3}$ alkyl, or $X_4$ represents a bond; and

$R_{b1}$ represents optionally substituted cycloalkyl (e.g., optionally substituted $C_{3-20}$cycloalkyl, or optionally substituted $C_{3-15}$cycloalkyl), optionally substituted aryl (e.g., optionally substituted $C_{6-20}$ aryl, or optionally substituted $C_{6-10}$ aryl), optionally substituted heterocyclyl (e.g., optionally substituted 4- to 20-membered heterocyclyl, or optionally substituted 4- to 15-membered heterocyclyl) or optionally substituted heteroaryl (e.g., optionally substituted 5- to 20-membered heteroaryl, or optionally substituted 5- to 15-membered heteroaryl).

In some embodiments of the compound of Formula (I-5), $R_a$ represents the following formula:

$$R_{a1}\text{-}X_2\text{-}L_1\text{-}X_1\text{-} ,$$

wherein $X_1$ represents a bond, or $X_1$ represents $C_{2-6}$ alkynylene or $C_{2-6}$ alkenylene;

$L_1$ represents linear or branched $C_{1-60}$ alkylene (e.g., $C_1$-$C_{30}$ alkylene, $C_1$-$C_{29}$ alkylene, $C_1$-$C_{28}$ alkylene, $C_1$-$C_{27}$ alkylene, $C_1$-$C_{26}$ alkylene, $C_1$-$C_{25}$ alkylene, $C_1$-$C_{24}$ alkylene, $C_1$-$C_{23}$ alkylene, $C_1$-$C_{22}$ alkylene, $C_1$-$C_{21}$ alkylene, $C_1$-$C_{20}$ alkylene, $C_1$-$C_{19}$ alkylene, $C_1$-$C_{18}$ alkylene, $C_1$-$C_{17}$ alkylene, $C_1$-$C_{16}$ alkylene, $C_1$-$C_{15}$ alkylene, $C_1$-$C_{14}$ alkylene, $C_1$-$C_{13}$ alkylene, $C_1$-$C_{12}$ alkylene, $C_1$-$C_{11}$ alkylene, $C_1$-$C_{10}$ alkylene, $C_1$-$C_9$ alkylene, $C_1$-$C_8$ alkylene, $C_1$-$C_7$ alkylene, $C_1$-$C_6$ alkylene, $C_1$-$C_5$ alkylene, $C_1$-$C_4$ alkylene, $C_1$-$C_3$ alkylene, or $C_1$-$C_2$ alkylene) which is optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy, or any combination thereof; and

$X_2$ represents a bond, or $X_2$ represents $N(R_5)$, C(O)O, OC(O), C(O)NH, NHC(O), C(O), O, C(S), S, S(O), $S(O)_2$, $S(O)_2NH$, $NHS(O)_2$, $S(O)_2O$, or $OS(O)_2$, wherein $R_5$ represents H or $C_{1-3}$ alkyl; and

$R_{a1}$ represents optionally substituted 4- to 20-membered heterocyclyl, and the 4- to 20-membered heterocyclyl is optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH-(e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)-(e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2,

or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$-and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof.

In some embodiments of the compound of Formula (I-5), $R_{a1}$ represents:
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl, diazacyclooctyl, 1,3-diazacycloheptanyl, 1,4-diazacycloheptanyl, 4,5-diazacycloheptanyl, 5- to 15-memberedbridged heterocyclyl (e.g., 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, diazabicyclo[3.2.1]octyl, diazabicyclo[2.2.2]octyl, 3,8-diazabicyclo[3.2.1]octan-3-yl, and 2,5-diazabicyclo[2.2.2]octan-2-yl), or 5- to 15-memberedazaspirocycloalkyl (e.g., 3-azaspiro[5.5]undecylene and 7-azaspiro[3.5]nonylene), with each group being independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of: D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

In some embodiments of the compound of Formula (I-5), $R_{a1}$ represents:

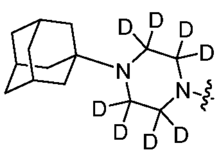 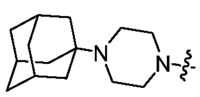 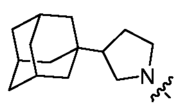 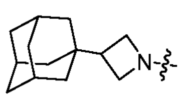 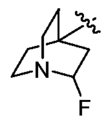 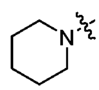 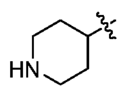

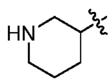 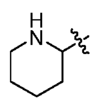 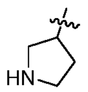 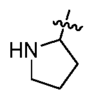 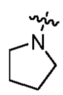 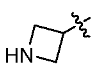 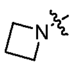 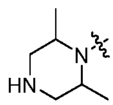 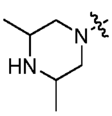 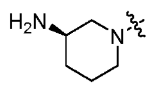 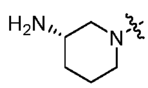

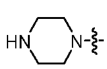 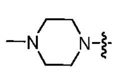 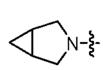 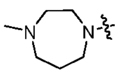 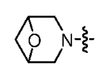 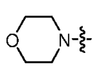 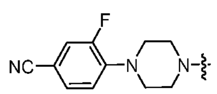 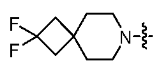 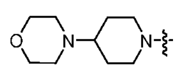

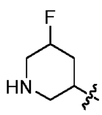 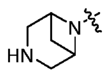 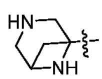 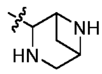 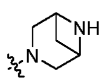 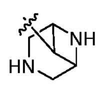 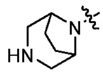 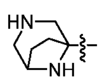 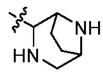 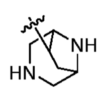 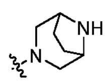

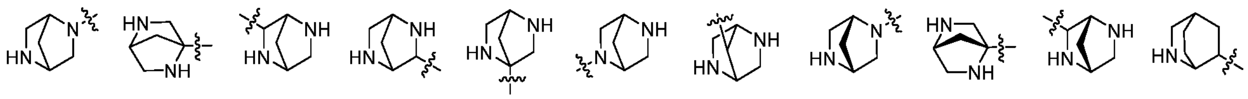

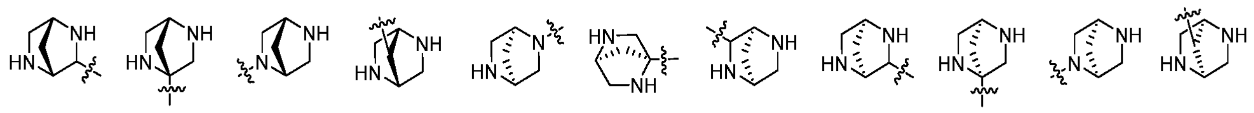

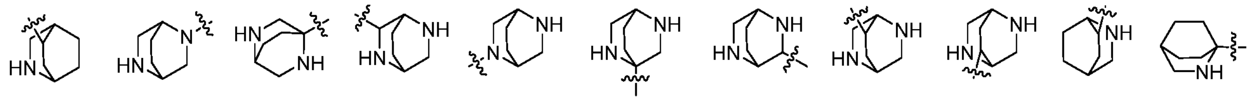

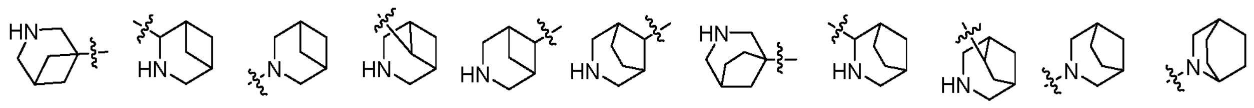

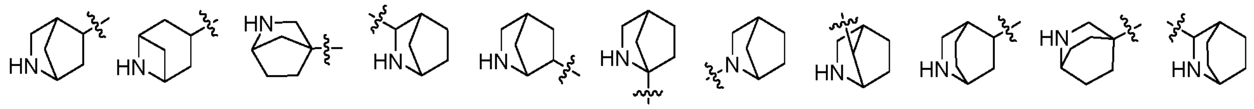

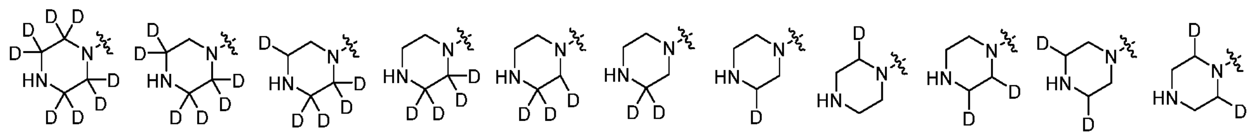

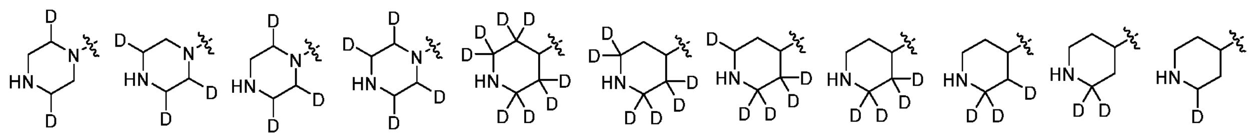

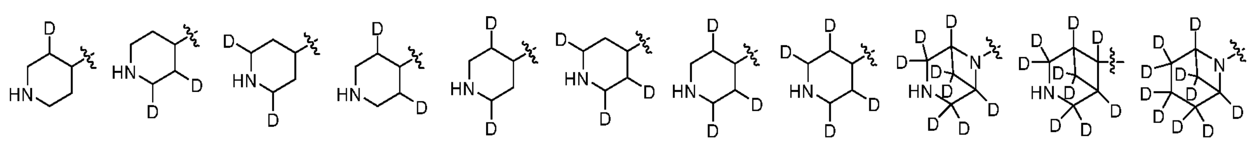

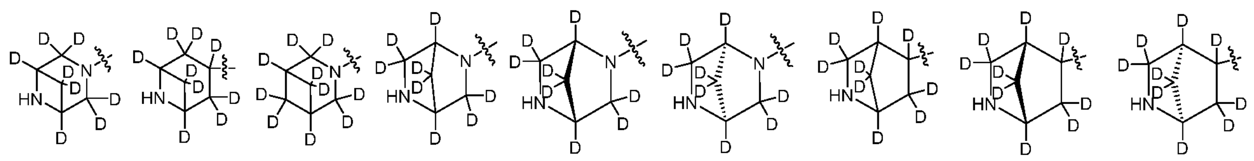

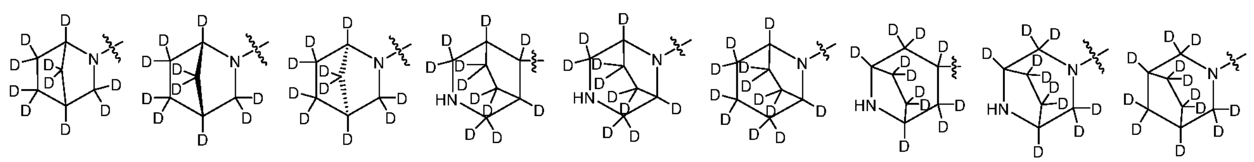

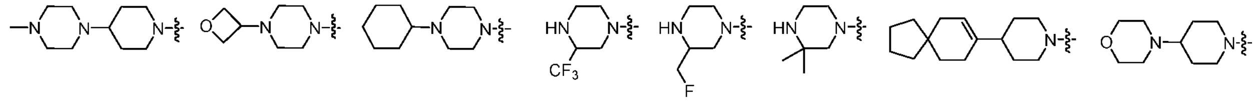

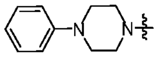 or 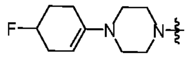

In some embodiments of the compound of Formula (I-5), $L_1$ represents the structure of the following formula: ##-$C_{1-30}$ alkylene-, wherein a hydrogen atom of one or more (e.g., 1-20, or 1-15, 1-10, 1-9, 1-8, 1-7, 1-6, 1-5, 1-4, 1-3 or 1-2,

or 1) $CH_2$ groups of the $C_{1-30}$ alkylene is optionally replaced with a substituent selected from the group consisting of: D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy or any combination thereof, and symbol ## indicates the point of attachment to $X_1$.

In some embodiments of the compound of Formula (I-5), $L_1$ represents the following group:

##-$CH_2$-, ##-$(CH_2)_2$-, ##-$(CH_2)_3$-, ##-$(CH_2)_4$-, ##-$(CH_2)_5$-, ##-$(CH_2)_2$-$CF_2$-$(CH_2)_2$-, ##-$(CH_2)_6$-, ##-$(CH_2)_7$-, ##-$(CH_2)_8$-, ##-$(CH_2)_9$-, ##-$(CH_2)_{10}$-, ##-$(CH_2)_{11}$-, ##-$(CH_2)_{12}$-, ##-$(CH_2)_{13}$-, ##-$(CH_2)_{14}$-, ##-$(CH_2)_{15}$-, ##-$(CH_2)_{16}$-, ##-$(CH_2)_{17}$-, ##-$(CH_2)_{18}$-, ##-$(CH_2)_{19}$-, ##-$(CH_2)_{20}$-, ##-$(CH_2)_{21}$-, ##-$(CH_2)_{22}$-, ##-$(CH_2)_{25}$-, or ##-$(CH_2)_{30}$-;

wherein a hydrogen atom of one or more (e.g., 1-20, or 1-15, 1-10, 1-9, 1-8, 1-7, 1-6, 1-5, 1-4, 1-3 or 1-2, or 1) $CH_2$ of the group is optionally replaced with a substituent selected from the group consisting of: D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy or any combination thereof, and symbol ## indicates the point of attachment to $X_1$.

In some embodiments of the compound of Formula (I-5), $R_b$ represents $C_{1-60}$ alkyl optionally substituted with D or halogen.

In some embodiments of the compound of Formula (I-5), $R_b$ represents the following group: $CH_3$, $CF_3$, $CD_3$, $CH_2CH_3$, -$(CH_2)_2$-$CH_3$, -$(CH_2)_3$-$CH_3$, -$(CH_2)_4$-$CH_3$, -$(CH_2)_5$-$CH_3$, -$(CH_2)_6$-$CH_3$,-$(CH_2)_7$-$CH_3$, -$(CH_2)_8$-$CH_3$, -$(CH_2)_9$-$CH_3$, -$(CH_2)_{10}$-$CH_3$, -$(CH_2)_{11}$-$CH_3$, -$(CH_2)_{12}$-$CH_3$, -$(CH_2)_{13}$-$CH_3$,-$(CH_2)_{14}$-$CH_3$, -$(CH_2)_{15}$-$CH_3$, -$(CH_2)_{16}$-$CH_3$, -$(CH_2)_{17}$-$CH_3$, -$(CH_2)_{18}$-$CH_3$, -$(CH_2)_{19}$-$CH_3$, -$(CH_2)_{20}$-$CH_3$, -$(CH_2)_{21}$-$CH_3$, -$(CH_2)_{22}$-$CH_3$, -$(CH_2)_{23}$-$CH_3$, -$(CH_2)_{24}$-$CH_3$, -$(CH_2)_{25}$-$CH_3$, -$(CH_2)_{26}$-$CH_3$, -$(CH_2)_{27}$-$CH_3$, -$(CH_2)_{28}$-$CH_3$, or -$(CH_2)_{29}$-$CH_3$.

In some embodiments of the compound of Formula (I-5), $R_b$ represents the following formula:

$$R_{b1}\text{-}X_4\text{-}L_2\text{-},$$

wherein $L_2$ represents

linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, $C_{1-4}$ alkyl, halogen, $C_{3-6}$cycloalkyl or any combination thereof, or

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{12}$ and/or one or more (e.g., 1-20, or 1-15, 1-10, 1-9, 1-8, 1-7, 1-6, 1-5, 1-4, 1-3 or 1-2, or 1) groups $R_{13}$ and/or any combination of one or more (e.g., 1-20, or 1-15, 1-10, 1-9, 1-8, 1-7, 1-6, 1-5, 1-4, 1-3 or 1-2, or 1) groups $R_{12}$ and $R_{13}$ inserted into its backbone carbon chain, wherein carbon-carbon bond between one or more pairs (e.g., 1-20, or 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, N($R_{14}$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of $C_{3-15}$cycloalkylene, 4- to 15-membered heterocyclylene, $C_{6-10}$ arylene, 5- to 15-membered heteroarylene, $C_{2-6}$ alkenylene, $C_{2-6}$ alkynylene or any combination thereof, wherein the $C_{3-15}$cycloalkylene, the 4- to 15-membered heterocyclylene, $C_{6-10}$ arylene and the 5- to 15-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-

NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH-(e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof, and the linear or branched $C_{2-60}$ alkylene is optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, $C_{1-4}$ alkyl, halogen, $C_{3-6}$cycloalkyl or any combination thereof;

$X_4$ represents a bond, or $X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted $C_{3-20}$cycloalkylene, optionally substituted $C_{6-20}$ arylene, optionally substituted 4- to 20-membered heterocyclylene, optionally substituted 5- to 20-membered heteroarylene, triazolylene-NH-, or -NH-triazolylene, wherein $R_{15}$ represents H or $C_{1-3}$ alkyl, and the $C_{3-20}$cycloalkylene, the $C_{6-20}$ arylene, the 4- to 20-membered heterocyclylene, and the 5- to 20-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, halogen, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof; and

$R_{b1}$ represents optionally substituted $C_{3-20}$cycloalkyl, optionally substituted $C_{6-20}$ aryl, optionally substituted 4- to 20-membered heterocyclyl or optionally substituted 5- to 20-membered heteroaryl, wherein the $C_{3-20}$cycloalkyl, the $C_{6-20}$ aryl, the 4- to 20-membered heterocyclyl, and the 5- to 20-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally substituted $C_{3-15}$cycloalkyl (e.g., optionally substituted cyclopropyl, optionally substituted cyclobutyl, optionally substituted cyclopentyl or optionally substituted cyclohexyl), optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH-(e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof.

In some embodiments of the compound of Formula (I-5), $L_2$ represents the structure of the following formula: -$C_{2-30}$ alkylene-, wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the $C_{2-30}$ alkylene is optionally replaced with a substituent selected from the group consisting of: D, $C_{1-4}$ alkyl, halogen, $C_{3-6}$cycloalkyl or any combination thereof.

In some embodiments of the compound of Formula (I-5), $L_2$ represents the following group:

$-CH_2-$, $-(CH_2)_2-$, $-(CH_2)_3-$, $-(CH_2)_4-$, $-(CH_2)_5-$, $-(CH_2)_2-CF_2-(CH_2)_2-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{15}-$, $-(CH_2)_{16}-$, $-(CH_2)_{17}-$, $-(CH_2)_{18}-$, $-(CH_2)_{19}-$, $-(CH_2)_{20}-$, $-(CH_2)_{21}-$, $-(CH_2)_{22}-$, $-(CH_2)_{25}-$, or $-(CH_2)_{30}-$;
wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the group is optionally replaced with a substituent selected from the group consisting of: D, $C_{1-4}$ alkyl, halogen, $C_{3-6}$cycloalkyl or any combination thereof.

In some embodiments of the compound of Formula (I-5), $L_2$ represents the structure of the following formula:

$-(C(R^{a9})(R^{a10}))_{m1}-(R_{12}(C(R^{a11})(R^{a12}))_{m2})_{p1}-$;

$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-$;

$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-(R_{12}(C(R^{a15})(R^{a16}))_{m4})_{p3}-$;

$-(C(R^{a9})(R^{a10}))_{m1}-(R_{13}(C(R^{a11})(R^{a12}))_{m2})_{p1}-$;

$-(C(R^{a9})(R^{a10}))_{m1}-(R_{13}(C(R^{a11})(R^{a12}))_{m2})_{p1}-(R_{13}(C(R^{a13})(R^{a14}))_{m3})_{p2}-$;

$-(C(R^{a9})(R^{a10}))_{m1}-(R_{13}(C(R^{a11})(R^{a12}))_{m2})_{p1}-(R_{13}(C(R^{a13})(R^{a14}))_{m3})_{p2}-(R_{13}(C(R^{a15})(R^{a16}))_{m4})_{p3}-$;

$-(C(R^{a9})(R^{a10}))_{m1}-(R_{12}-R_{13}-(C(R^{a11})(R^{a12}))_{m2})_{p1}-$;

$-(C(R^{a9})(R^{a10}))_{m1}-(R_{12}(C(R^{a11})(R^{a12}))_{m2})_{p1}-(R_{13}(C(R^{a13})(R^{a14}))_{m3})_{p2}-$;

$-(C(R^{a9})(R^{a10}))_{m1}-(R_{13}-R_{12}-(C(R^{a11})(R^{a12}))_{m2})_{p1}-$; or

$-(C(R^{a9})(R^{a10}))_{m1}-(R_{13}(C(R^{a11})(R^{a12}))_{m2})_{p1}-(R_{12}(C(R^{a13})(R^{a14}))_{m3})_{p2}-$;

wherein each group $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_{14}$ independently represents H or $C_{1-3}$ alkyl; and each group $R_{13}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl (e.g., optionally substituted cyclopropyl, optionally substituted cyclobutyl, optionally substituted cyclopentyl or optionally substituted cyclohexyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH-$, $CH_3CH_2NH-$ or $CH_3CH_2CH_2NH-$), halogenated $C_{1-4}$ alkyl (e.g., halogenated $C_{1-3}$ alkyl, such as $F_3C-$, $FCH_2-$, $F_2CH-$, $ClCH_2-$, $Cl_2CH-$, $CF_3CF_2-$, $CF_3CHF-$, $CHF_2CF_2-$, $CHF_2CHF-$, $CF_3CH_2-$ or $CH_2ClCH_2-$), $NH_2-C_{1-4}$ alkylene (e.g., $NH_2-C_{1-3}$ alkylene-, such as $NH_2CH_2-$, $NH_2CH_2CH_2-$ and $NH_2CH_2CH_2CH_2-$), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-) or any combination thereof;
$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and
m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-5), $L_2$ represents the structure of the following formula:

$-(C(R^{a9})(R^{a10}))_{m1}-(O(C(R^{a11})(R^{a12}))_{m2})_{p1}-$;

$-(C(R^{a9})(R^{a10}))_{m1}-(O(C(R^{a11})(R^{a12}))_{m2})_{p1}-(O(C(R^{a13})(R^{a14}))_{m3})_{p2}-$;

$-(C(R^{a9})(R^{a10}))_{m1}-(O(C(R^{a11})(R^{a12}))_{m2})_{p1}-(O(C(R^{a13})(R^{a14}))_{m3})_{p2}-(O(C(R^{a15})(R^{a16}))_{m4})_{p3}-$;

$-(C(R^{a9})(R^{a10}))_{m1}-(N(R_{g11})(C(R^{a11})(R^{a12}))_{m2})_{p1}-$;

$-(C(R^{a9})(R^{a10}))_{m1}-(N(R_{g11})(C(R^{a11})(R^{a12}))_{m2})_{p1}-(N(R_{g11})(C(R^{a13})(R^{a14}))_{m3})_{p2}-$;

$-(C(R^{a9})(R^{a10}))_{m1}-(N(R_{g11})(C(R^{a11})(R^{a12}))_{m2})_{p1}-(N(R_{g11})(C(R^{a13})(R^{a14}))_{m3})_{p2}-(N(R_{g11})(C(R^{a15})(R^{a16}))_{m4})_{p3}-$;

$-(C(R^{a9})(R^{a10}))_{m1}-(C(O)NH-(C(R^{a11})(R^{a12}))_{m2})_{p1}-$;

$-(C(R^{a9})(R^{a10}))_{m1}-(C(O)NH-(C(R^{a11})(R^{a12}))_{m2})_{p1}-C(O)NH-(C(R^{a13})(R^{a14}))_{m3})_{p2}-$

$-(C(R^{a9})(R^{a10}))_{m1}-(C(O)NH-(C(R^{a11})(R^{a12}))_{m2})_{p1}-(C(O)NH-(C(R^{a13})(R^{a14}))_{m3})_{p2}-(C(O)NH-(C(R^{a16})(R^{a16}))_{m4})_{p3}-$;

$-(C(R^{a9})(R^{a10}))_{m1}-(C(O)NH-(C(R^{a11})(R^{a12}))_{m2})_{p1}-(O(C(R^{a13})(R^{a14}))_{m3})_{p2}-$

$-(C(R^{a9})(R^{a10}))_{m1}-C(O)NH-(C(R^{a11})(R^{a12}))_{m2}-(O(C(R^{a13})(R^{a14}))_{m3})_{p1}-$;

$-(C(R^{a9})(R^{a10}))_{m1}-(NHC(O)-(C(R^{a11})(R^{a12}))_{m2})_{p1}-$;

$-(C(R^{a9})(R^{a10}))_{m1}-(NHC(O)-(C(R^{a11})(R^{a12}))_{m2})_{p1}-(O-(C(R^{a13})(R^{a14}))_{m3})_{p2}-$;

$-(C(R^{a9})(R^{a10}))_{m1}-(NHC(O)-(C(R^{a11})(R^{a12}))_{m2})_{p1}-(O-(C(R^{a13})(R^{a14}))_{m3})_{p2}-(O-(C(R^{a15})(R^{a16}))_{m4})_{p3}-$;

$-(C(R^{a9})(R^{a10}))_{m1}-NHC(O)-(C(R^{a11})(R^{a12}))_{m2}-(O(C(R^{a13})(R^{a14}))_{m3})_{p1}-$;

$-(C(R^{a9})(R^{a10}))_{m1}-NHC(O)NH-(C(R^{a11})(R^{a12}))_{m2}-$;

$-(C(R^{a9})(R^{a10}))_{m1}-C(O)-C(R^{a11})(R^{a12})_{m2}-$;

$-(C(R^{a9})(R^{a10}))_{m1}-C(S)-(C(R^{a11})(R^{a12}))_{m2}-$;

$-(C(R^{a9})(R^{a10})_{m1}-S-(C(R^{a11})(R^{a12}))_{m2}-$;

$-(C(R^{a9})(R^{a10}))_{m1}-(C_{6-10}$ arylene-$(C(R^{a11})(R^{a12}))_{m2})_{p1}-$;

$-(C(R^{a9})(R^{a10}))_{m1}-(C_{6-10}$ arylene-$(C(R^{a11})(R^{a12}))_{m2})_{p1}-C_{6-10}$ arylene-$(C(R^{a13})(R^{a14}))_{m3}-$;

$-(C(R^{a9})(R^{a10}))_{m1}-$(4- to 15-membered heterocyclylene-$(C(R^{a11})(R^{a12}))_{m2})_{p1}-$;

$-(C(R^{a9})(R^{a10}))_{m1}-$(4- to 15-membered heterocyclylene-$(C(R^{a11})(R^{a12}))_{m2})_{p1}-$(4- to 15-membered heterocyclylene-$(C(R^{a13})(R^{a14}))_{m3})_{p2}-$;

$-(C(R^{a9})(R^{a10}))_{m1}-$(5- to 15-membered heteroarylene-$(C(R^{a11})(R^{a12}))_{m2})_{p1}-$;

$-(C(R^{a9})(R^{a10}))_{m1}-$(5- to 15-membered heteroarylene-$(C(R^{a1})(R^{a12}))_{m2})_{p1}-$(5- to 15-membered heteroarylene-$(C(R^{a13})(R^{a14}))_{m3})_{p2}-$;

$-(C(R^{a9})(R^{a10}))_{m1}-(C_{3-15}$cycloalkylene-$(C(R^{a12})(R^{a12}))_{m2})_{p1}-$; or

$-(C(R^{a9})(R^{a10}))_{m1}-(C_{3-15}$cycloalkylene-$(C(R^{a11})(R^{a12}))_{m2})_{p1}-(C_{3-15}$cycloalkylene-$(C(R^{a13})(R^{a14}))_{m3})_{p2}-$;

wherein each $R_{g11}$ independently represents H or $C_{1-3}$ alkyl;
the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen,

cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;

$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and

m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-5), $L_2$ represents the structure of the following formula: $-CH_2-O-CH_2-$, $-CH_2-O-(CH_2)_2-$, $-(CH_2)_1-O-(CH_2)_3-$, $-(CH_2)_1-O-(CH_2)_4-$, $-(CH_2)_1-O-(CH_2)_5-$, $-(CH_2)_1-$ $O-(CH_2)_6-$, $-(CH_2)_1-O-(CH_2)_7-$, $-(CH_2)_1-O-(CH_2)_8-$, $-(CH_2)_1-O-(CH_2)_9-$, $-(CH_2)_1-O-(CH_2)_{10}-$, $-(CH_2)_2-O-$ $(CH_2)_1-$, $-(CH_2)_2-O-(CH_2)_2-$, $-(CH_2)_2-O-(CH_2)_3-$, $-(CH_2)_2-O-(CH_2)_4-$, $-(CH_2)_2-O-(CH_2)_5-$, $-(CH_2)_2-O-$ $(CH_2)_6-$, $-(CH_2)_2-O-(CH_2)_7-$, $-(CH_2)_2-O-(CH_2)_8-$, $-(CH_2)_2-O-(CH_2)_9-$, $-(CH_2)_2-O-(CH_2)_{10}-$, $-(CH_2)_2-O-$ $(CH_2)_{11}-$, $-(CH_2)_2-O-(CH_2)_{12}-$, $-(CH_2)_3-O-(CH_2)_1-$, $-(CH_2)_3-O-(CH_2)_2-$, $-(CH_2)_3-O-(CH_2)_3-$, $-(CH_2)_3-O-$ $(CH_2)_4-$, $-(CH_2)_3-O-(CH_2)_5-$, $-(CH_2)_3-O-(CH_2)_6-$, $-(CH_2)_3-O-(CH_2)_7-$, $-(CH_2)_4-O-(CH_2)_1-$, $-(CH_2)_4-O-$ $(CH_2)_2-$, $-(CH_2)_4-O-(CH_2)_3-$, $-(CH_2)_4-O-(CH_2)_4-$, $-(CH_2)_4-O-(CH_2)_5-$, $-(CH_2)_4-O-(CH_2)_6-$, $-(CH_2)_5-O-$ $(CH_2)_1-$, $-(CH_2)_5-O-(CH_2)_2-$, $-(CH_2)_5-O-(CH_2)_3-$, $-(CH_2)_5-O-(CH_2)_4-$, $-(CH_2)_5-O-(CH_2)_5-$, $-(CH_2)_6-O-$ $(CH_2)_1-$, $-(CH_2)_6-O-(CH_2)_2-$, $-(CH_2)_6-O-(CH_2)_3-$, $-(CH_2)_6-O-(CH_2)_4-$, $-(CH_2)_7-O-(CH_2)_1-$, $-(CH_2)_7-O-$ $(CH_2)_2-$, $-(CH_2)_7-O-(CH_2)_3-$, $-(CH_2)_8-O-(CH_2)_1-$, $-(CH_2)_8-O-(CH_2)_2-$, $-CH(CH_3)-O-(CH_2)_1-$, $-CH(CH_3)-$ $O-(CH_2)_2-$, $-CH(CH_3)-O-(CH_2)_3-$, $-CH(CH_3)-O-(CH_2)_4-$, $-CH(CH_3)-O-(CH_2)_5-$, $-CH(CH_3)-O-(CH_2)_6-$, - $CH(CH_3)-O-(CH_2)_7-$, $-CH(CH_3)-O-(CH_2)_8-$, $-CH(CH_3)-O-(CH_2)_9-$, $-CH(CH_3)-O-(CH_2)_{10}-$, $-CH_2-(O(CH_2)_2)_2-$, $-CH_2-(O(CH_2)_2)_3-$, $-CH_2-(O(CH_2)_2)_4-$, $-CH_2-(O(CH_2)_2)_5-$, $-CH_2-(O(CH_2)_2)_6-$, $-CH_2-(O(CH_2)_2)_7-$, $-CH_2-(O(CH_2)_2)_8-$, $-CH_2-(O(CH_2)_2)_9-$, $-CH_2-(O(CH_2)_2)_{10}-$, $-CH_2-(O(CH_2)_2)_1-OCH_2-$, - $CH_2-(O(CH_2)_2)_2-OCH_2-$, $-CH_2-(O(CH_2)_2)_3-OCH_2-$, $-CH_2-(O(CH_2)_2)_4-OCH_2-$, $-CH_2-(O(CH_2)_2)_5-OCH_2-$, $-CH_2-(O(CH_2)_2)_6-OCH_2-$, $-CH_2-(O(CH_2)_2)_7-OCH_2-$, $-CH_2-(O(CH_2)_2)_8-OCH_2-$, $-CH_2-(O(CH_2)_2)_9-OCH_2-$, $-CH_2-(O(CH_2)_2)_{10}-OCH_2-$, $-(CH_2)_2-(O(CH_2)_2)_2-$, $-(CH_2)_2-(O(CH_2)_2)_3-$, $-(CH_2)_2-(O(CH_2)_2)_4-$, - $(CH_2)_2-(O(CH_2)_2)_5-$, $-(CH_2)_2-(O(CH_2)_2)_6-$, $-(CH_2)_2-(O(CH_2)_2)_7-$, $-(CH_2)_2-(O(CH_2)_2)_8-$, $-(CH_2)_2-(O(CH_2)_2)_9-$, $-(CH_2)_2-(O(CH_2)_2)_{10}-$, $-(CH_2)_3-(O(CH_2)_2)_2-$, $-(CH_2)_3-(O(CH_2)_2)_3-$, $-(CH_2)_3-(O(CH_2)_2)_4-$, - $(CH_2)_3-(O(CH_2)_2)_5-$, $-(CH_2)_3-(O(CH_2)_2)_6-$, $-(CH_2)_3-(O(CH_2)_2)_7-$, $-(CH_2)_3-(O(CH_2)_2)_8-$, $-(CH_2)_3-$ $(O(CH_2)_2)_9-$, $-(CH_2)_3-(O(CH_2)_2)_{10}-$, $-(CH_2)_4-(O(CH_2)_2)_2-$, $-(CH_2)_4-(O(CH_2)_2)_3-$, $-(CH_2)_4-(O(CH_2)_2)_4-$, - $(CH_2)_4-(O(CH_2)_2)_5-$, $-(CH_2)_4-(O(CH_2)_2)_6-$, $-(CH_2)_4-(O(CH_2)_2)_7-$, $-(CH_2)_4-(O(CH_2)_2)_8-$, $-(CH_2)_4-$ $(O(CH_2)_2)_9-$, $-(CH_2)_4-(O(CH_2)_2)_{10}-$, $-CH_2-(O(CH_2)_3)_2-$, $-CH_2-(O(CH_2)_3)_3-$, $-CH_2-(O(CH_2)_3)_4-$, $-CH_2-$ $(O(CH_2)_3)_5-$, $-CH_2-(O(CH_2)_3)_6-$, $-CH_2-(O(CH_2)_3)_7-$, $-CH_2-(O(CH_2)_3)_8-$, $-CH_2-(O(CH_2)_3)_9-$, $-CH_2-$ $(O(CH_2)_3)_{10}-$, $-(CH_2)_2-(O(CH_2)_3)_2-$, $-(CH_2)_2-(O(CH_2)_3)_3-$, $-(CH_2)_2-(O(CH_2)_3)_4-$, $-(CH_2)_2-(O(CH_2)_3)_5-$, - $(CH_2)_2-(O(CH_2)_3)_6-$, $-(CH_2)_2-(O(CH_2)_3)_7-$, $-(CH_2)_2-(O(CH_2)_3)_8-$, $-(CH_2)_2-(O(CH_2)_3)_9-$, $-(CH_2)_2-$ $(O(CH_2)_3)_{10}-$, $-(CH_2)_3-(O(CH_2)_3)_2-$, $-(CH_2)_3-(O(CH_2)_3)_3-$, $-(CH_2)_3-(O(CH_2)_3)_4-$, $-(CH_2)_3-(O(CH_2)_3)_5-$, - $(CH_2)_3-(O(CH_2)_3)_6-$, $-(CH_2)_3-(O(CH_2)_3)_7-$, $-(CH_2)_3-(O(CH_2)_3)_8-$, $-(CH_2)_3-(O(CH_2)_3)_9-$, $-(CH_2)_3-$ $(O(CH_2)_3)_{10}-$, $-CH_2-O-(CH_2)_2-O-(CH_2)_3-$, $-CH_2-(O(CH_2)_2)_2-(O(CH_2)_3)_2-$, $-CH_2-(O(CH_2)_2)_3-(O(CH_2)_3)_3-$, $-CH_2-(O(CH_2)_2)_4-(O(CH_2)_3)_4-$, $-CH_2-(O(CH_2)_2)_5-(O(CH_2)_3)_5-$, $-CH_2-(O(CH_2)_2)_6-(O(CH_2)_3)_6-$, $-(CH_2)_2-O-(CH_2)_2-O-(CH_2)_3-$, $-(CH_2)_2-(O(CH_2)_2)_2-(O(CH_2)_3)_2-$, $-(CH_2)_2-(O(CH_2)_2)_3-(O(CH_2)_3)_3-$, $-(CH_2)_2-(O(CH_2)_2)_4-(O(CH_2)_3)_4-$, $-(CH_2)_2-(O(CH_2)_2)_5-(O(CH_2)_3)_5-$, $-(CH_2)_2-(O(CH_2)_2)_6-(O(CH_2)_3)_6-$, $-(CH_2)_3-O-(CH_2)_2-O-(CH_2)_3-$, $-(CH_2)_3-(O(CH_2)_2)_2-(O(CH_2)_3)_2-$, $-(CH_2)_3-(O(CH_2)_2)_3-(O(CH_2)_3)_3-$, $-(CH_2)_3-(O(CH_2)_2)_4-(O(CH_2)_3)_4-$, $-(CH_2)_3-(O(CH_2)_2)_5-(O(CH_2)_3)_5-$, $-(CH_2)_3-(O(CH_2)_2)_6-(O(CH_2)_3)_6-$, $-CH_2-O-(CH_2)_3-O-(CH_2)_2-$, $-CH_2-(O(CH_2)_3)_2-(O(CH_2)_2)_2-$, $-CH_2-(O(CH_2)_3)_3-(O(CH_2)_2)_3-$, $-CH_2-(O(CH_2)_3)_4-(O(CH_2)_2)_4-$, $-CH_2-(O(CH_2)_3)_5-(O(CH_2)_2)_5-$, $-CH_2-(O(CH_2)_3)_6-(O(CH_2)_2)_6-$, $-(CH_2)_2-O-(CH_2)_3-O-(CH_2)_2-$, $-(CH_2)_2-(O(CH_2)_3)_2-(O(CH_2)_2)_2-$, $-(CH_2)_2-(O(CH_2)_3)_3-(O(CH_2)_2)_3-$, $-(CH_2)_2-(O(CH_2)_3)_4-(O(CH_2)_2)_4-$, $-(CH_2)_2-(O(CH_2)_3)_5-(O(CH_2)_2)_5-$, $-(CH_2)_2-(O(CH_2)_3)_6-(O(CH_2)_2)_6-$, $-(CH_2)_3-O-(CH_2)_3-O-(CH_2)_2-$, $-(CH_2)_3-(O(CH_2)_3)_2-(O(CH_2)_2)_2-$, $-(CH_2)_3-(O(CH_2)_3)_3-(O(CH_2)_2)_3-$, $-(CH_2)_3-(O(CH_2)_3)_4-(O(CH_2)_2)_4-$, $-(CH_2)_3-(O(CH_2)_3)_5-(O(CH_2)_2)_5-$, $-(CH_2)_3-(O(CH_2)_3)_6-(O(CH_2)_2)_6-$, $-CH_2-O-(CH_2)_2-O-CH_2-$, $-(CH_2)_2-O-(CH_2)_2-O-CH_2-$, $-(CH_2)_2-(O(CH_2)_2)_2-O-(CH_2)_3-$, $-(CH_2)_2-(O(CH_2)_2)_3-O-(CH_2)_3-$, - $(CH_2)_2-(O(CH_2)_2)_4-O-(CH_2)_3-$, $-(CH_2)_5-(O(CH_2)_2)_2-O-(CH_2)_5-$, $-(CH_2)_5-(O(CH_2)_2)_2-O-(CH_2)_6-$, $-CH_2-C(O)-CH_2-$, $-CH_2-C(O)-(CH_2)_2-$, $-(CH_2)_1-C(O)-(CH_2)_3-$, $-(CH_2)_1-C(O)-(CH_2)_4-$, $-(CH_2)_1-C(O)-(CH_2)_5-$, $-(CH_2)_1-C(O)-(CH_2)_6-$, $-(CH_2)_1-C(O)-(CH_2)_7-$, $-(CH_2)_1-C(O)-(CH_2)_8-$, $-(CH_2)_1-C(O)-(CH_2)_9-$, $-(CH_2)_1-C(O)-(CH_2)_{10}-$, $-(CH_2)_2-C(O)-(CH_2)_1-$, $-(CH_2)_2-C(O)-(CH_2)_2-$, $-(CH_2)_2-C(O)-(CH_2)_3-$, $-(CH_2)_2-C(O)-(CH_2)_4-$, $-(CH_2)_2-C(O)-(CH_2)_5-$, $-(CH_2)_2-C(O)-(CH_2)_6-$, $-(CH_2)_2-C(O)-(CH_2)_7-$, $-(CH_2)_2-C(O)-(CH_2)_8-$, - $(CH_2)_2-C(O)-(CH_2)_9-$, $-(CH_2)_2-C(O)-(CH_2)_{10}-$, $-(CH_2)_2-C(O)-(CH_2)_{11}-$, $-(CH_2)_2-C(O)-(CH_2)_{12}-$, - $(CH_2)_3-C(O)-(CH_2)_1-$, $-(CH_2)_3-C(O)-(CH_2)_2-$, $-(CH_2)_3-C(O)-(CH_2)_3-$, $-(CH_2)_3-C(O)-(CH_2)_4-$, $-(CH_2)_3-C(O)-(CH_2)_5-$, $-(CH_2)_3-C(O)-(CH_2)_6-$, $-(CH_2)_3-C(O)-(CH_2)_7-$, $-(CH_2)_4-C(O)-(CH_2)_1-$, $-(CH_2)_4-C(O)-(CH_2)_2-$, $-(CH_2)_4-C(O)-(CH_2)_3-$, $-(CH_2)_4-C(O)-(CH_2)_4-$, $-(CH_2)_4-C(O)-(CH_2)_5-$, $-(CH_2)_4-C(O)-(CH_2)_6-$, - $(CH_2)_5-C(O)-(CH_2)_1-$, $-(CH_2)_5-C(O)-(CH_2)_2-$, $-(CH_2)_5-C(O)-(CH_2)_3-$, $-(CH_2)_5-C(O)-(CH_2)_4-$, $-(CH_2)_5-C(O)-(CH_2)_5-$, $-(CH_2)_6-C(O)-(CH_2)_1-$, $-(CH_2)_6-C(O)-(CH_2)_2-$, $-(CH_2)_6-C(O)-(CH_2)_3-$, $-(CH_2)_6-C(O)-(CH_2)_4-$, $-(CH_2)_7-C(O)-(CH_2)_1-$, $-(CH_2)_7-C(O)-(CH_2)_2-$, $-(CH_2)_7-C(O)-(CH_2)_3-$, $-(CH_2)_8-C(O)-(CH_2)_1-$, - $(CH_2)_8-C(O)-(CH_2)_2-$, $-CH_2-S-CH_2-$, $-CH_2-S-(CH_2)_2-$, $-(CH_2)_1-S-(CH_2)_3-$, $-(CH_2)_1-S-(CH_2)_4-$, $-(CH_2)_1-S-(CH_2)_5-$, $-(CH_2)_1-S-(CH_2)_6-$, $-(CH_2)_1-S-(CH_2)_7-$, $-(CH_2)_1-S-(CH_2)_8-$, $-(CH_2)_1-S-(CH_2)_9-$, $-(CH_2)_1-S-(CH_2)_{10}-$,

$-(CH_2)_2-S-(CH_2)_1-$, $-(CH_2)_2-S-(CH_2)_2-$, $-(CH_2)_2-S-(CH_2)_3-$, $-(CH_2)_2-S-(CH_2)_4-$, $-(CH_2)_2-S-(CH_2)_5-$, $-(CH_2)_2-S-(CH_2)_6-$, $-(CH_2)_2-S-(CH_2)_7-$, $-(CH_2)_2-S-(CH_2)_8-$, $-(CH_2)_2-S-(CH_2)_9-$, $-(CH_2)_2-S-(CH_2)_{10}-$, $-(CH_2)_2-S-(CH_2)_{11}-$, $-(CH_2)_2-S-(CH_2)_{12}-$, $-(CH_2)_3-S-(CH_2)_1-$, $-(CH_2)_3-S-(CH_2)_2-$, $-(CH_2)_3-S-(CH_2)_3-$, $-(CH_2)_3-S-(CH_2)_4-$, $-(CH_2)_3-S-(CH_2)_5-$, $-(CH_2)_3-S-(CH_2)_6-$, $-(CH_2)_3-S-(CH_2)_7-$, $-(CH_2)_4-S-(CH_2)_1-$, $-(CH_2)_4-S-(CH_2)_2-$, $-(CH_2)_4-S-(CH_2)_3-$, $-(CH_2)_4-S-(CH_2)_4-$, $-(CH_2)_4-S-(CH_2)_5-$, $-(CH_2)_4-S-(CH_2)_6-$, $-(CH_2)_5-S-(CH_2)_1-$, $-(CH_2)_5-S-(CH_2)_2-$, $-(CH_2)_5-S-(CH_2)_3-$, $-(CH_2)_5-S-(CH_2)_4-$, $-(CH_2)_5-S-(CH_2)_5-$, $-(CH_2)_6-S-(CH_2)_1-$, $-(CH_2)_6-S-(CH_2)_2-$, $-(CH_2)_6-S-(CH_2)_3-$, $-(CH_2)_6-S-(CH_2)_4-$, $-(CH_2)_7-S-(CH_2)_1-$, $-(CH_2)_7-S-(CH_2)_2-$, $-(CH_2)_7-S-(CH_2)_3-$, $-(CH_2)_8-S-(CH_2)_1-$, $-(CH_2)_8-S-(CH_2)_2-$, $-CH_2-C(S)-CH_2-$, $-CH_2-C(S)-(CH_2)_2-$, $-(CH_2)_1-C(S)-(CH_2)_3-$, $-(CH_2)_1-C(S)-(CH_2)_4-$, $-(CH_2)_1-C(S)-(CH_2)_5-$, $-(CH_2)_1-C(S)-(CH_2)_6-$, $-(CH_2)_1-C(S)-(CH_2)_7-$, $-(CH_2)_1-C(S)-(CH_2)_8-$, $-(CH_2)_1-C(S)-(CH_2)_9-$, $-(CH_2)_1-C(S)-(CH_2)_{10}-$, $-(CH_2)_2-C(S)-(CH_2)_1-$, $-(CH_2)_2-C(S)-(CH_2)_2-$, $-(CH_2)_2-C(S)-(CH_2)_3-$, $-(CH_2)_2-C(S)-(CH_2)_4-$, $-(CH_2)_2-C(S)-(CH_2)_5-$, $-(CH_2)_2-C(S)-(CH_2)_6-$, $-(CH_2)_2-C(S)-(CH_2)_7-$, $-(CH_2)_2-C(S)-(CH_2)_8-$, $-(CH_2)_2-C(S)-(CH_2)_9-$, $-(CH_2)_2-C(S)-(CH_2)_{10}-$, $-(CH_2)_2-C(S)-(CH_2)_{11}-$, $-(CH_2)_2-C(S)-(CH_2)_{12}-$, $-(CH_2)_3-C(S)-(CH_2)_1-$, $-(CH_2)_3-C(S)-(CH_2)_2-$, $-(CH_2)_3-C(S)-(CH_2)_3-$, $-(CH_2)_3-C(S)-(CH_2)_4-$, $-(CH_2)_3-C(S)-(CH_2)_5-$, $-(CH_2)_3-C(S)-(CH_2)_6-$, $-(CH_2)_3-C(S)-(CH_2)_7-$, $-(CH_2)_4-C(S)-(CH_2)_1-$, $-(CH_2)_4-C(S)-(CH_2)_2-$, $-(CH_2)_4-C(S)-(CH_2)_3-$, $-(CH_2)_4-C(S)-(CH_2)_4-$, $-(CH_2)_4-C(S)-(CH_2)_5-$, $-(CH_2)_4-C(S)-(CH_2)_6-$, $-(CH_2)_5-C(S)-(CH_2)_1-$, $-(CH_2)_5-C(S)-(CH_2)_2-$, $-(CH_2)_5-C(S)-(CH_2)_3-$, $-(CH_2)_5-C(S)-(CH_2)_4-$, $-(CH_2)_5-C(S)-(CH_2)_5-$, $-(CH_2)_6-C(S)-(CH_2)_1-$, $-(CH_2)_6-C(S)-(CH_2)_2-$, $-(CH_2)_6-C(S)-(CH_2)_3-$, $-(CH_2)_6-C(S)-(CH_2)_4-$, $-(CH_2)_7-C(S)-(CH_2)_1-$, $-(CH_2)_7-C(S)-(CH_2)_2-$, $-(CH_2)_7-C(S)-(CH_2)_3-$, $-(CH_2)_S-C(S)-(CH_2)_1-$, $-(CH_2)_8-C(S)-(CH_2)_2-$, $-CH_2-C(O)O-CH_2-$, $-CH_2-C(O)O-(CH_2)_2-$, $-(CH_2)_1-C(O)O-(CH_2)_3-$, $-(CH_2)_1-C(O)O-(CH_2)_4-$, $-(CH_2)_1-C(O)O-(CH_2)_5-$, $-(CH_2)_1-C(O)O-(CH_2)_6-$, $-(CH_2)_1-C(O)O-(CH_2)_7-$, $-(CH_2)_1-C(O)O-(CH_2)_8-$, $-(CH_2)_1-C(O)O-(CH_2)_9-$, $-(CH_2)_1-C(O)O-(CH_2)_{10}-$, $-(CH_2)_2-C(O)O-(CH_2)_1-$, $-(CH_2)_2-C(O)O-(CH_2)_2-$, $-(CH_2)_2-C(O)O-(CH_2)_3-$, $-(CH_2)_2-C(O)O-(CH_2)_4-$, $-(CH_2)_2-C(O)O-(CH_2)_5-$, $-(CH_2)_2-C(O)O-(CH_2)_6-$, $-(CH_2)_2-C(O)O-(CH_2)_7-$, $-(CH_2)_2-C(O)O-(CH_2)_8-$, $-(CH_2)_2-C(O)O-(CH_2)_9-$, $-(CH_2)_2-C(O)O-(CH_2)_{10}-$, $-(CH_2)_2-C(O)O-(CH_2)_{11}-$, $-(CH_2)_2-C(O)O-(CH_2)_{12}-$, $-(CH_2)_3-C(O)O-(CH_2)_1-$, $-(CH_2)_3-C(O)O-(CH_2)_2-$, $-(CH_2)_3-C(O)O-(CH_2)_3-$, $-(CH_2)_3-C(O)O-(CH_2)_4-$, $-(CH_2)_3-C(O)O-(CH_2)_5-$, $-(CH_2)_3-C(O)O-(CH_2)_6-$, $-(CH_2)_3-C(O)O-(CH_2)_7-$, $-(CH_2)_4-C(O)O-(CH_2)_1-$, $-(CH_2)_4-C(O)O-(CH_2)_2-$, $-(CH_2)_4-C(O)O-(CH_2)_3-$, $-(CH_2)_4-C(O)O-(CH_2)_4-$, $-(CH_2)_4-C(O)O-(CH_2)_5-$, $-(CH_2)_4-C(O)O-(CH_2)_6-$, $-(CH_2)_5-C(O)O-(CH_2)_1-$, $-(CH_2)_5-C(O)O-(CH_2)_2-$, $-(CH_2)_5-C(O)O-(CH_2)_3-$, $-(CH_2)_5-C(O)O-(CH_2)_4-$, $-(CH_2)_5-C(O)O-(CH_2)_5-$, $-(CH_2)_6-C(O)O-(CH_2)_1-$, $-(CH_2)_6-C(O)O-(CH_2)_2-$, $-(CH_2)_6-C(O)O-(CH_2)_3-$, $-(CH_2)_6-C(O)O-(CH_2)_4-$, $-(CH_2)_7-C(O)O-(CH_2)_1-$, $-(CH_2)_7-C(O)O-(CH_2)_2-$, $-(CH_2)_7-C(O)O-(CH_2)_3-$, $-(CH_2)_8-C(O)O-(CH_2)_1-$, $-(CH_2)_8-C(O)O-(CH_2)_2-$, $-CH_2-O-C(O)-CH_2-$, $-CH_2-OC(O)-(CH_2)_2-$, $-(CH_2)_1-OC(O)-(CH_2)_3-$, $-(CH_2)_1-OC(O)-(CH_2)_4-$, $-(CH_2)_1-OC(O)-(CH_2)_5-$, $-(CH_2)_1-O-C(O)-(CH_2)_6-$, $-(CH_2)_1-OC(O)-(CH_2)_7-$, $-(CH_2)_1-OC(O)-(CH_2)_8-$, $-(CH_2)_1-OC(O)-(CH_2)_9-$, $-(CH_2)_1-OC(O)-(CH_2)_{10}-$, $-(CH_2)_2-OC(O)-(CH_2)_1-$, $-(CH_2)_2-OC(O)-(CH_2)_2-$, $-(CH_2)_2-OC(O)-(CH_2)_3-$, $-(CH_2)_2-OC(O)-(CH_2)_4-$, $-(CH_2)_2-O-C(O)-(CH_2)_5-$, $-(CH_2)_2-OC(O)-(CH_2)_6-$, $-(CH_2)_2-OC(O)-(CH_2)_7-$, $-(CH_2)_2-OC(O)-(CH_2)_8-$, $-(CH_2)_2-OC(O)-(CH_2)_9-$, $-(CH_2)_2-OC(O)-(CH_2)_{10}-$, $-(CH_2)_2-OC(O)-(CH_2)_{11}-$, $-(CH_2)_2-OC(O)-(CH_2)_{12}-$, $-(CH_2)_3-OC(O)-(CH_2)_1-$, $-(CH_2)_3-O-C(O)-(CH_2)_2-$, $-(CH_2)_3-OC(O)-(CH_2)_3-$, $-(CH_2)_3-OC(O)-(CH_2)_4-$, $-(CH_2)_3-OC(O)-(CH_2)_5-$, $-(CH_2)_3-OC(O)-(CH_2)_6-$, $-(CH_2)_3-OC(O)-(CH_2)_7-$, $-(CH_2)_4-OC(O)-(CH_2)_1-$, $-(CH_2)_4-OC(O)-(CH_2)_2-$, $-(CH_2)_4-OC(O)-(CH_2)_3-$, $-(CH_2)_4-O-C(O)-(CH_2)_4-$, $-(CH_2)_4-OC(O)-(CH_2)_5-$, $-(CH_2)_4-OC(O)-(CH_2)_6-$, $-(CH_2)_5-OC(O)-(CH_2)_1-$, $-(CH_2)_5-OC(O)-(CH_2)_2-$, $-(CH_2)_5-OC(O)-(CH_2)_3-$, $-(CH_2)_5-OC(O)-(CH_2)_4-$, $-(CH_2)_5-OC(O)-(CH_2)_5-$, $-(CH_2)_6-OC(O)-(CH_2)_1-$, $-(CH_2)_6-O-C(O)-(CH_2)_2-$, $-(CH_2)_6-OC(O)-(CH_2)_3-$, $-(CH_2)_6-OC(O)-(CH_2)_4-$, $-(CH_2)_7-OC(O)-(CH_2)_1-$, $-(CH_2)_7-OC(O)-(CH_2)_2-$, $-(CH_2)_7-OC(O)-(CH_2)_3-$, $-(CH_2)_8-OC(O)-(CH_2)_1-$, $-(CH_2)_8-OC(O)-(CH_2)_2-$, $-(CH_2)_1-N(R_{g11})-(CH_2)_1-$, $-(CH_2)_1-N(R_{g11})-(CH_2)_2-$, $-(CH_2)_1-N(R_{g11})-(CH_2)_3-$, $-(CH_2)_1-N(R_{g11})-(CH_2)_4-$, $-(CH_2)_1-N(R_{g11})-(CH_2)_5-$, $-(CH_2)_1-N(R_{g11})-(CH_2)_6-$, $-(CH_2)_1-N(R_{g11})-(CH_2)_7-$, $-(CH_2)_1-N(R_{g11})-(CH_2)_8-$, $-(CH_2)_1-N(R_{g11})-(CH_2)_9-$, $-(CH_2)_1-N(R_{g11})-(CH_2)_{10}-$, $-(CH_2)_2-N(R_{g11})-(CH_2)_1-$, $-(CH_2)_2-N(R_{g11})-(CH_2)_2-$, $-(CH_2)_2-N(R_{g11})-(CH_2)_3-$, $-(CH_2)_2-N(R_{g11})-(CH_2)_4-$, $-(CH_2)_2-N(R_{g11})-(CH_2)_5-$, $-(CH_2)_2-N(R_{g11})-(CH_2)_6-$, $-(CH_2)_2-N(R_{g11})-(CH_2)_7-$, $-(CH_2)_2-N(R_{g11})-(CH_2)_8-$, $-(CH_2)_2-N(R_{g11})-(CH_2)_9-$, $-(CH_2)_2-N(R_{g11})-(CH_2)_{10}-$, $-(CH_2)_2-N(R_{g11})-(CH_2)_{11}-$, $-(CH_2)_2-N(R_{g11})-(CH_2)_{12}-$, $-(CH_2)_3-N(R_{g11})-(CH_2)_1-$, $-(CH_2)_3-N(R_{g11})-(CH_2)_2-$, $-(CH_2)_3-N(R_{g11})-(CH_2)_3-$, $-(CH_2)_4-N(R_{g11})-(CH_2)_1-$, $-(CH_2)_4-N(R_{g11})-(CH_2)_2-$, $-(CH_2)_4-N(R_{g11})-(CH_2)_3-$, $-(CH_2)_4-N(R_{g11})-(CH_2)_4-$, $-(CH_2)_5-N(R_{g11})-(CH_2)_1-$, $-(CH_2)_5-N(R_{g11})-(CH_2)_2-$, $-(CH_2)_5-N(R_{g11})-(CH_2)_3-$, $-(CH_2)_5-N(R_{g11})-(CH_2)_4-$, $-(CH_2)_5-N(R_{g11})-(CH_2)_5-$, $-(CH_2)_6-N(R_{g11})-(CH_2)_1-$, $-(CH_2)_6-N(R_{g11})-(CH_2)_2-$, $-(CH_2)_6-N(R_{g11})-(CH_2)_3-$, $-(CH_2)_7-N(R_{g11})-(CH_2)_1-$, $-(CH_2)_7-N(R_{g11})-(CH_2)_2-$, $-(CH_2)_7-N(R_{g11})-(CH_2)_3-$, $-(CH_2)_8-N(R_{g11})-(CH_2)_1-$, $-(CH_2)_8-NR_{g11})-(CH_2)_2-$, $-(CH_2)_8-N(R_{g11})-(CH_2)_3-$, $-CH(CH_3)-N(R_{g11})-(CH_2)_1-$, $-CH(CH_3)-N(R_{g11})-(CH_2)_2-$, $-CH(CH_3)-N(R_{g11})-(CH_2)_3-$, $-CH(CH_3)-N(R_{g11})-(CH_2)_4-$, $-CH(CH_3)-N(R_{g11})-(CH_2)_5-$, $-CH(CH_3)-N(R_{g11})-(CH_2)_6-$, $-CH(CH_3)-N(R_{g11})-(CH_2)_7-$, $-CH(CH_3)-N(R_{g11})-(CH_2)_8-$, $-CH(CH_3)-N(R_{g11})-(CH_2)_9-$, $-CH(CH_3)-N(R_{g11})-(CH_2)_{10}-$, $-CH_2C(O)NHCH_2-$, $-(CH_2)_2C(O)NH(CH_2)_2-$, $-(CH_2)_2C(O)NH(CH_2)_3-$, $-(CH_2)_2C(O)NH(CH_2)_4-$, $-(CH_2)_2C(O)NH(CH_2)_5-$, $-(CH_2)_3C(O)NH(CH_2)_3-$, $-(CH_2)_3C(O)NH(CH_2)_4-$, $-(CH_2)_4C(O)NH(CH_2)_4-$, $-(CH_2)_5C(O)NH(CH_2)_5-$, $-(CH_2)_6C(O)NH(CH_2)_7-$, $-(CH_2)_6C(O)NH(CH_2)_6-$, $-(CH_2)_7C(O)NH(CH_2)_7-$, $-(CH_2)_8C(O)NH(CH_2)_8$, $-(CH_2)_9C(O)NH(CH_2)_9-$, $-(CH_2)_{10}C(O)NH(CH_2)_{10}-$, $-(CH_2)_2C(O)NH(CH_2)_2-O-(CH_2)_2-$, $-CH_2NHC(O)CH_2-$, $-(CH_2)_2NHC(O)(CH_2)_2-$, $-(CH_2)_2NHC(O)(CH_2)_3-$, $-(CH_2)_2NHC(O)(CH_2)_4-$, $-(CH_2)_2NHC(O)(CH_2)_5-$, $-(CH_2)_3NHC(O)(CH_2)_3-$, $-(CH_2)_3NHC(O)(CH_2)_4-$, $-(CH_2)_4NHC(O)(CH_2)_4-$, $-(CH_2)_5NHC(O)(CH_2)_5-$, $-(CH_2)_6NHC(O)(CH_2)_7-$, $-(CH_2)_6NHC(O)(CH_2)_6-$, $-(CH_2)_7NHC(O)(CH_2)_7-$, $-(CH_2)_8NHC(O)(CH_2)_8$, $-(CH_2)_9NHC(O)(CH_2)_9-$, $-(CH_2)_{10}NHC(O)(CH_2)_{10}-$, $-(CH_2)_4NHC(O)$

$(CH_2)_8$-, -$(CH_2)_2NHC(O)(CH_2)_2$-O-$(CH_2)_2$-, - $(CH_2)_4NHC(O)CH_2$-, -$CH_2$-piperidinylene-$CH_2$-, -$CH_2$-piperidinylene-$(CH_2)_2$-, -$CH_2$-piperidinylene-$(CH_2)_3$-, -$CH_2$-piperidinylene-$(CH_2)_4$-, -$CH_2$-piperidinylene-$(CH_2)_5$-, -$CH_2$-piperidinylene-$(CH_2)_6$-, - $CH_2$-piperidinylene-$(CH_2)_7$-, -$CH_2$-piperidinylene-$(CH_2)_8$-, -$(CH_2)_2$-piperidinylene-$(CH_2)_1$-, -$(CH_2)_2$-piperidinylene-$(CH_2)_2$-, -$(CH_2)_2$-piperidinylene-$(CH_2)_3$-, -$(CH_2)_2$-piperidinylene-$(CH_2)_4$-, -$(CH_2)_2$-piperidinylene-$(CH_2)_5$-, -$(CH_2)_2$-piperidinylene-$(CH_2)_6$-, -$(CH_2)_2$-piperidinylene-$(CH_2)_7$-, -$(CH_2)_2$-piperidinylene-$(CH_2)_8$-, -$(CH_2)_3$-piperidinylene-$CH_2$-, -$(CH_2)_3$-piperidinylene-$(CH_2)_2$-, -$(CH_2)_3$-piperidinylene-$(CH_2)_3$-, -$(CH_2)_3$-piperidinylene-$(CH_2)_4$-, -$(CH_2)_3$-piperidinylene-$(CH_2)_5$-, -$(CH_2)_3$-piperidinylene-$(CH_2)_6$-, -$(CH_2)_3$-piperidinylene-$(CH_2)_7$-, -$(CH_2)_3$-piperidinylene-$(CH_2)_8$-, -$(CH_2)_4$-piperidinylene-$CH_2$-, -$(CH_2)_4$-piperidinylene-$(CH_2)_2$-, -$(CH_2)_4$-piperidinylene-$(CH_2)_3$-, -$(CH_2)_4$-piperidinylene-$(CH_2)_4$-, -$(CH_2)_4$-piperidinylene-$(CH_2)_5$-, -$(CH_2)_4$-piperidinylene-$(CH_2)_6$-, -$(CH_2)_4$-piperidinylene-$(CH_2)_7$-, -$(CH_2)_4$-piperidinylene-$(CH_2)_8$-, -$(CH_2)_5$-piperidinylene-$(CH_2)_1$-, -$(CH_2)_5$-piperidinylene-$(CH_2)_2$-, -$(CH_2)_5$-piperidinylene-$(CH_2)_3$-, -$(CH_2)_5$-piperidinylene-$(CH_2)_4$-, -$(CH_2)_5$-piperidinylene-$(CH_2)_5$-, -$(CH_2)_5$-piperidinylene-$(CH_2)_6$-, -$(CH_2)_5$-piperidinylene-$(CH_2)_7$-, -$(CH_2)_5$-piperidinylene-$(CH_2)_8$-, -$(CH_2)_6$-piperidinylene-$(CH_2)_1$-, -$(CH_2)_6$-piperidinylene-$(CH_2)_2$-, -$(CH_2)_6$-piperidinylene-$(CH_2)_3$-, -$(CH_2)_6$-piperidinylene-$(CH_2)_4$-, -$(CH_2)_6$-piperidinylene-$(CH_2)_5$-, -$(CH_2)_6$-piperidinylene-$(CH_2)_6$-, -$(CH_2)_6$-piperidinylene-$(CH_2)_7$-, -$(CH_2)_6$-piperidinylene-$(CH_2)_8$-, -$(CH_2)_7$-piperidinylene-$(CH_2)_1$-, -$(CH_2)_7$-piperidinylene-$(CH_2)_2$-, -$(CH_2)_7$-piperidinylene-$(CH_2)_3$-, -$(CH_2)_7$-piperidinylene-$(CH_2)_4$-, -$(CH_2)_7$-piperidinylene-$(CH_2)_8$-, -$(CH_2)_8$-piperidinylene-$CH_2$-, -$(CH_2)_8$-piperidinylene-$(CH_2)_2$-, -$(CH_2)_8$-piperidinylene-$(CH_2)_3$-, -$(CH_2)_8$-piperidinylene-$(CH_2)_4$-, -$(CH_2)_8$-piperidinylene-$(CH_2)_5$-, -$(CH_2)_8$-piperidinylene-$(CH_2)_6$-, -$(CH_2)_8$-piperidinylene-$(CH_2)_7$-, -$(CH_2)_8$-piperidinylene-$(CH_2)_8$-, -$CH_2$-piperazinylene-$CH_2$-, -$CH_2$-piperazinylene-$(CH_2)_2$-, -$CH_2$-piperazinylene-$(CH_2)_3$-, -$CH_2$-piperazinylene-$(CH_2)_4$-, -$CH_2$-piperazinylene-$(CH_2)_5$-, -$CH_2$-piperazinylene-$(CH_2)_6$-, -$CH_2$-piperazinylene-$(CH_2)_7$-, -$CH_2$-piperazinylene-$(CH_2)_8$-, -$(CH_2)_2$-piperazinylene-$(CH_2)_1$-, -$(CH_2)_2$-piperazinylene-$(CH_2)_2$-, -$(CH_2)_2$-piperazinylene-$(CH_2)_3$-, -$(CH_2)_2$-piperazinylene-$(CH_2)_4$-, -$(CH_2)_2$-piperazinylene-$(CH_2)_5$-, -$(CH_2)_2$-piperazinylene-$(CH_2)_6$-, -$(CH_2)_2$-piperazinylene-$(CH_2)_7$-, -$(CH_2)_2$-piperazinylene-$(CH_2)_8$-, -$(CH_2)_3$-piperazinylene-$CH_2$-, -$(CH_2)_3$-piperazinylene-$(CH_2)_2$-, -$(CH_2)_3$-piperazinylene-$(CH_2)_3$-, -$(CH_2)_3$-piperazinylene-$(CH_2)_4$-, -$(CH_2)_3$-piperazinylene-$(CH_2)_5$-, -$(CH_2)_3$-piperazinylene-$(CH_2)_6$-, -$(CH_2)_3$-piperazinylene-$(CH_2)_7$-, -$(CH_2)_3$-piperazinylene-$(CH_2)_8$-, -$(CH_2)_4$-piperazinylene-$CH_2$-, -$(CH_2)_4$-piperazinylene-$(CH_2)_2$-, -$(CH_2)_4$-piperazinylene-$(CH_2)_3$-, -$(CH_2)_4$-piperazinylene-$(CH_2)_4$-, -$(CH_2)_4$-piperazinylene-$(CH_2)_5$-, -$(CH_2)_4$-piperazinylene-$(CH_2)_6$-, -$(CH_2)_4$-piperazinylene-$(CH_2)_7$-, -$(CH_2)_4$-piperazinylene-$(CH_2)_8$-, -$(CH_2)_5$-piperazinylene-$(CH_2)_1$-, -$(CH_2)_5$-piperazinylene-$(CH_2)_2$-, -$(CH_2)_5$-piperazinylene-$(CH_2)_3$-, -$(CH_2)_5$-piperazinylene-$(CH_2)_4$-, -$(CH_2)_5$-piperazinylene-$(CH_2)_5$-, -$(CH_2)_5$-piperazinylene-$(CH_2)_6$-, -$(CH_2)_5$-piperazinylene-$(CH_2)_7$-, -$(CH_2)_5$-piperazinylene-$(CH_2)_8$-, -$(CH_2)_6$-piperazinylene-$(CH_2)$i-, -$(CH_2)_6$-piperazinylene-$(CH_2)_2$-, -$(CH_2)_6$-piperazinylene-$(CH_2)_3$-, -$(CH_2)_6$-piperazinylene-$(CH_2)_4$-, -$(CH_2)_6$-piperazinylene-$(CH_2)_5$-, -$(CH_2)_6$-piperazinylene-$(CH_2)_6$-, -$(CH_2)_6$-piperazinylene-$(CH_2)_7$-, -$(CH_2)_6$-piperazinylene-$(CH_2)_8$-, -$(CH_2)_7$-piperazinylene-$(CH_2)_1$-, -$(CH_2)_7$-piperazinylene-$(CH_2)_2$-, -$(CH_2)_7$-piperazinylene-$(CH_2)_3$-, -$(CH_2)_7$-piperazinylene-$(CH_2)_4$-, -$(CH_2)_7$-piperazinylene-$(CH_2)_8$-, -$(CH_2)_8$-piperazinylene-$CH_2$-, -$(CH_2)_8$-piperazinylene-$(CH_2)_2$-, -$(CH_2)_8$-piperazinylene-$(CH_2)_3$-, -$(CH_2)_8$-piperazinylene-$(CH_2)_4$-, -$(CH_2)_8$-piperazinylene-$(CH_2)_5$-, -$(CH_2)_8$-piperazinylene-$(CH_2)_6$-, -$(CH_2)_8$-piperazinylene-$(CH_2)_7$-, -$(CH_2)_8$-piperazinylene-$(CH_2)_8$-, -$CH_2$-propylene-$CH_2$-, -$CH_2$-propylene-$(CH_2)_2$-, -$CH_2$-propylene-$(CH_2)_3$-, -$CH_2$-propylene-$(CH_2)_4$-, -$CH_2$-propylene-$(CH_2)_5$-, -$CH_2$-propylene-$(CH_2)_6$-, -$CH_2$-propylene-$(CH_2)_7$-, -$CH_2$-propylene-$(CH_2)_8$-, -$(CH_2)_2$-propylene-$(CH_2)_1$-, -$(CH_2)_2$-propylene-$(CH_2)_2$-, -$(CH_2)_2$-propylene-$(CH_2)_3$-, -$(CH_2)_2$-propylene-$(CH_2)_4$-, -$(CH_2)_2$-propylene-$(CH_2)_5$-, -$(CH_2)_2$-propylene-$(CH_2)_6$-, -$(CH_2)_2$-propylene-$(CH_2)_7$-, -$(CH_2)_2$-propylene-$(CH_2)_8$-, -$(CH_2)_3$-propylene-$CH_2$-, -$(CH_2)_3$-propylene-$(CH_2)_2$-, -$(CH_2)_3$-propylene-$(CH_2)_3$-, -$(CH_2)_3$-propylene-$(CH_2)_4$-, - $(CH_2)_3$-propylene-$(CH_2)_5$-, -$(CH_2)_3$-propylene-$(CH_2)_6$-, -$(CH_2)_3$-propylene-$(CH_2)_7$-, -$(CH_2)_3$-propylene-$(CH_2)_8$-, -$(CH_2)_4$-propylene-$CH_2$-, -$(CH_2)_4$-propylene-$(CH_2)_2$-, -$(CH_2)_4$-propylene-$(CH_2)_3$-, -$(CH_2)_4$-propylene-$(CH_2)_4$-, -$(CH_2)_4$-propylene-$(CH_2)_5$-, -$(CH_2)_4$-propylene-$(CH_2)_6$-, -$(CH_2)_4$-propylene-$(CH_2)_7$-, -$(CH_2)_4$-propylene-$(CH_2)_8$-, -$(CH_2)_5$-propylene-$(CH_2)_1$-, -$(CH_2)_5$-propylene-$(CH_2)_2$-, -$(CH_2)_5$-propylene-$(CH_2)_3$-, -$(CH_2)_5$-propylene-$(CH_2)_4$-, -$(CH_2)_5$-propylene-$(CH_2)_5$-, -$(CH_2)_5$-propylene-$(CH_2)_6$-, -$(CH_2)_5$-propylene-$(CH_2)_7$-, -$(CH_2)_5$-propylene-$(CH_2)_8$-, -$(CH_2)_6$-propylene-$(CH_2)_1$-, -$(CH_2)_6$-propylene-$(CH_2)_2$-, -$(CH_2)_6$-propylene-$(CH_2)_3$-, -$(CH_2)_6$-propylene-$(CH_2)_4$-, -$(CH_2)_6$-propylene-$(CH_2)_5$-, -$(CH_2)_6$-propylene-$(CH_2)_6$-, -$(CH_2)_6$-propylene-$(CH_2)_7$-, -$(CH_2)_6$-propylene-$(CH_2)_8$-, -$(CH_2)_7$-propylene-$(CH_2)_1$-, -$(CH_2)_7$-propylene-$(CH_2)_2$-, -$(CH_2)_7$-propylene-$(CH_2)_3$-, -$(CH_2)_7$-propylene-$(CH_2)_4$-, -$(CH_2)_7$-propylene-$(CH_2)_8$-, -$(CH_2)_8$-propylene-$CH_2$-, -$(CH_2)_8$-propylene-$(CH_2)_2$-, -$(CH_2)_8$-propylene-$(CH_2)_3$-, -$(CH_2)_8$-propylene-$(CH_2)_4$-, -$(CH_2)_8$-propylene-$(CH_2)_5$-, -$(CH_2)_8$-propylene-$(CH_2)_6$-, -$(CH_2)_8$-propylene-$(CH_2)_7$-, -$(CH_2)_8$-propylene-$(CH_2)_8$-, -$CH_2$-diazacycloheptanylene-$CH_2$-, -$CH_2$-diazacycloheptanylene-$(CH_2)_2$-, -$CH_2$-diazacycloheptanylene-$(CH_2)_3$-, -$CH_2$-diazacycloheptanylene-$(CH_2)_4$-, -$CH_2$-diazacycloheptanylene-$(CH_2)_5$-, -$CH_2$-diazacycloheptanylene-$(CH_2)_6$-, -$CH_2$-diazacycloheptanylene-$(CH_2)_7$-, -$CH_2$-diazacycloheptanylene-$(CH_2)_8$-, -$(CH_2)_2$-diazacycloheptanylene-$(CH_2)_1$-, -$(CH_2)_2$-diazacycloheptanylene-$(CH_2)_2$-, -$(CH_2)_2$-diazacycloheptanylene-$(CH_2)_3$-, -$(CH_2)_2$-diazacycloheptanylene-$(CH_2)_4$-, -$(CH_2)_2$-diazacycloheptanylene-$(CH_2)_5$-, -$(CH_2)_2$-diazacycloheptanylene-$(CH_2)_6$-, -$(CH_2)_2$-diazacycloheptanylene-$(CH_2)_7$-, -$(CH_2)_2$-diazacycloheptanylene-$(CH_2)_8$-, -$(CH_2)_3$-diazacycloheptanylene-$CH_2$-, -$(CH_2)_3$-diazacycloheptanylene-$(CH_2)_2$-, -$(CH_2)_3$-diazacycloheptanylene-$(CH_2)_3$-, -$(CH_2)_3$-diazacycloheptanylene-$(CH_2)_4$-, -$(CH_2)_3$-diazacycloheptanylene-$(CH_2)_5$-, -$(CH_2)_3$-diazacycloheptanylene-$(CH_2)_6$-, -$(CH_2)_3$-diazacycloheptanylene-$(CH_2)_7$-, -$(CH_2)_3$-diazacycloheptanylene-$(CH_2)_8$-, -$(CH_2)_4$-diazacycloheptanylene-$CH_2$-, -$(CH_2)_4$-diazacycloheptanylene-$(CH_2)_2$-, -$(CH_2)_4$-diazacycloheptanyle-

ne-$(CH_2)_3$-, -$(CH_2)_4$-diazacycloheptanylene-$(CH_2)_4$-, -$(CH_2)_4$-diazacycloheptanylene-$(CH_2)_5$-, -$(CH_2)_4$-diazacycloheptanylene-$(CH_2)_6$-, -$(CH_2)_4$-diazacycloheptanylene-$(CH_2)_7$-, -$(CH_2)_4$-diazacycloheptanylene-$(CH_2)_8$-, -$(CH_2)_5$-diazacycloheptanylene-$(CH_2)_1$-, -$(CH_2)_5$-diazacycloheptanylene-$(CH_2)_2$-, -$(CH_2)_5$-diazacycloheptanylene-$(CH_2)_3$-, -$(CH_2)_5$-diazacycloheptanylene-$(CH_2)_4$-, -$(CH_2)_5$-diazacycloheptanylene-$(CH_2)_5$-, -$(CH_2)_5$-diazacycloheptanylene-$(CH_2)_6$-, -$(CH_2)_5$-diazacycloheptanylene-$(CH_2)_7$-, -$(CH_2)_5$-diazacycloheptanylene-$(CH_2)_s$-, -$(CH_2)_6$-diazacycloheptanylene-$(CH_2)_1$-, -$(CH_2)_6$-diazacycloheptanylene-$(CH_2)_2$-, -$(CH_2)_6$-diazacycloheptanylene-$(CH_2)_3$-, -$(CH_2)_6$-diazacycloheptanylene-$(CH_2)_4$-, -$(CH_2)_6$-diazacycloheptanylene-$(CH_2)_5$-, -$(CH_2)_6$-diazacycloheptanylene-$(CH_2)_6$-, -$(CH_2)_6$-diazacycloheptanylene-$(CH_2)_7$-, -$(CH_2)_6$-diazacycloheptanylene-$(CH_2)_8$-, -$(CH_2)_7$-diazacycloheptanylene-$(CH_2)_1$-, -$(CH_2)_7$-diazacycloheptanylene-$(CH_2)_2$-, -$(CH_2)_7$-diazacycloheptanylene-$(CH_2)_3$-, -$(CH_2)_7$-diazacycloheptanylene-$(CH_2)_4$-, -$(CH_2)_7$-diazacycloheptanylene-$(CH_2)_8$-, -$(CH_2)_8$-diazacycloheptanylene-$CH_2$-, -$(CH_2)_8$-diazacycloheptanylene-$(CH_2)_2$-, -$(CH_2)_8$-diazacycloheptanylene-$(CH_2)_3$-, -$(CH_2)_8$-diazacycloheptanylene-$(CH_2)_4$-, -$(CH_2)_8$-diazacycloheptanylene-$(CH_2)_5$-, -$(CH_2)_8$-diazacycloheptanylene-$(CH_2)_6$-, -$(CH_2)_8$-diazacycloheptanylene-$(CH_2)_7$-, -$(CH_2)_8$-diazacycloheptanylene-$(CH_2)_8$-, -$CH_2$-diazabicyclo[2.2.1]heptanylene-$CH_2$-, -$CH_2$-diazabicyclo[2.2.1]heptanylene-$(CH_2)_2$-, -$CH_2$-diazabicyclo[2.2.1]heptanylene-$(CH_2)_3$-, -$CH_2$-diazabicyclo[2.2.1]heptanylene-$(CH_2)_4$-, -$CH_2$-diazabicyclo[2.2.1]heptanylene-$(CH_2)_5$-, -$CH_2$-diazabicyclo[2.2.1]heptanylene-$(CH_2)_6$-, -$CH_2$-diazabicyclo[2.2.1]heptanylene-$(CH_2)_7$-, -$CH_2$-diazabicyclo[2.2.1]heptanylene-$(CH_2)_8$-, -$(CH_2)_2$-diazabicyclo[2.2.1]heptanylene-$(CH_2)_1$-, -$(CH_2)_2$-diazabicyclo[2.2.1]heptanylene-$(CH_2)_2$-, -$(CH_2)_2$-diazabicyclo[2.2.1]heptanylene-$(CH_2)_3$-, -$(CH_2)_2$-diazabicyclo[2.2.1]heptanylene-$(CH_2)_4$-, -$(CH_2)_2$-diazabicyclo[2.2.1]heptanylene-$(CH_2)_5$-, -$(CH_2)_2$-diazabicyclo[2.2.1]heptanylene-$(CH_2)_6$-, -$(CH_2)_2$-diazabicyclo[2.2.1]heptanylene-$(CH_2)_7$-, -$(CH_2)_2$-diazabicyclo[2.2.1]heptanylene-$(CH_2)_8$-, -$(CH_2)_3$-diazabicyclo[2.2.1]heptanylene-$CH_2$-, -$(CH_2)_3$-diazabicyclo[2.2.1]heptanylene-$(CH_2)_2$-, -$(CH_2)_3$-diazabicyclo[2.2.1]heptanylene-$(CH_2)_3$-, -$(CH_2)_3$-diazabicyclo[2.2.1]heptanylene-$(CH_2)_4$-, -$(CH_2)_3$-diazabicyclo[2.2.1]heptanylene-$(CH_2)_5$-, -$(CH_2)_3$-diazabicyclo[2.2.1]heptanylene-$(CH_2)_6$-, -$(CH_2)_3$-diazabicyclo[2.2.1]heptanylene-$(CH_2)_7$-, -$(CH_2)_3$-diazabicyclo[2.2.1]heptanylene-$(CH_2)_8$-, -$(CH_2)_4$-diazabicyclo[2.2.1]heptanylene-$CH_2$-, -$(CH_2)_4$-diazabicyclo[2.2.1]heptanylene-$(CH_2)_2$-, -$(CH_2)_4$-diazabicyclo[2.2.1]heptanylene-$(CH_2)_3$-, -$(CH_2)_4$-diazabicyclo[2.2.1]heptanylene-$(CH_2)_4$-, -$(CH_2)_4$-diazabicyclo[2.2.1]heptanylene-$(CH_2)_5$-, -$(CH_2)_4$-diazabicyclo[2.2.1]heptanylene-$(CH_2)_6$-, -$(CH_2)_4$-diazabicyclo[2.2.1]heptanylene-$(CH_2)_7$-, -$(CH_2)_4$-diazabicyclo[2.2.1]heptanylene-$(CH_2)_8$-, -$(CH_2)_5$-diazabicyclo[2.2.1]heptanylene-$(CH_2)_1$-, -$(CH_2)_5$-diazabicyclo[2.2.1]heptanylene-$(CH_2)_2$-, -$(CH_2)_5$-diazabicyclo[2.2.1]heptanylene-$(CH_2)_3$-, -$(CH_2)_5$-diazabicyclo[2.2.1]heptanylene-$(CH_2)_4$-, -$(CH_2)_5$-diazabicyclo[2.2.1]heptanylene-$(CH_2)_5$-, -$(CH_2)_5$-diazabicyclo[2.2.1]heptanylene-$(CH_2)_6$-, -$(CH_2)_5$-diazabicyclo[2.2.1]heptanylene-$(CH_2)_7$-, -$(CH_2)_5$-diazabicyclo[2.2.1]heptanylene-$(CH_2)_8$-, -$(CH_2)_6$-diazabicyclo[2.2.1]heptanylene-$(CH_2)_1$-, -$(CH_2)_6$-diazabicyclo[2.2.1]heptanylene-$(CH_2)_2$-, -$(CH_2)_6$-diazabicyclo[2.2.1]heptanylene-$(CH_2)_3$-, -$(CH_2)_6$-diazabicyclo[2.2.1]heptanylene-$(CH_2)_4$-, -$(CH_2)_6$-diazabicyclo[2.2.1]heptanylene-$(CH_2)_5$-, -$(CH_2)_6$-diazabicyclo[2.2.1]heptanylene-$(CH_2)_6$-, -$(CH_2)_6$-diazabicyclo[2.2.1]heptanylene-$(CH_2)_7$-, -$(CH_2)_6$-diazabicyclo[2.2.1]heptanylene-$(CH_2)_8$-, -$(CH_2)_7$-diazabicyclo[2.2.1]heptanylene-$(CH_2)_1$-, -$(CH_2)_7$-diazabicyclo[2.2.1]heptanylene-$(CH_2)_2$-, -$(CH_2)_7$-diazabicyclo[2.2.1]heptanylene-$(CH_2)_3$-, -$(CH_2)_7$-diazabicyclo[2.2.1]heptanylene-$(CH_2)_4$-, -$(CH_2)_7$-diazabicyclo[2.2.1]heptanylene-$(CH_2)_8$-, -$(CH_2)_8$-diazabicyclo[2.2.1]heptanylene-$CH_2$-, -$(CH_2)_8$-diazabicyclo[2.2.1]heptanylene-$(CH_2)_2$-, -$(CH_2)_8$-diazabicyclo[2.2.1]heptanylene-$(CH_2)_3$-, -$(CH_2)_8$-diazabicyclo[2.2.1]heptanylene-$(CH_2)_4$-, -$(CH_2)_8$-diazabicyclo[2.2.1]heptanylene-$(CH_2)_5$-, -$(CH_2)_8$-diazabicyclo[2.2.1]heptanylene-$(CH_2)_6$-, -$(CH_2)_8$-diazabicyclo[2.2.1]heptanylene-$(CH_2)_7$-, -$(CH_2)_8$-diazabicyclo[2.2.1]heptanylene-$(CH_2)_8$-, -$CH_2$-diazabicyclo[3.1.1]heptanylene-$CH_2$-, -$CH_2$-diazabicyclo[3.1.1]heptanylene-$(CH_2)_2$-, -$CH_2$-diazabicyclo[3.1.1]heptanylene-$(CH_2)_3$-, -$CH_2$-diazabicyclo[3.1.1]heptanylene-$(CH_2)_4$-, -$CH_2$-diazabicyclo[3.1.1]heptanylene-$(CH_2)_5$-, -$CH_2$-diazabicyclo[3.1.1]heptanylene-$(CH_2)_6$-, -$CH_2$-diazabicyclo[3.1.1]heptanylene-$(CH_2)_7$-, -$CH_2$-diazabicyclo[3.1.1]heptanylene-$(CH_2)_8$-, -$(CH_2)_2$-diazabicyclo[3.1.1]heptanylene-$(CH_2)_1$-, -$(CH_2)_2$-diazabicyclo[3.1.1]heptanylene-$(CH_2)_2$-, -$(CH_2)_2$-diazabicyclo[3. 1. 1]heptanylene-$(CH_2)_3$-, -$(CH_2)_2$-diazabicyclo[3.1. 1]heptanylene-$(CH_2)_4$-, -$(CH_2)_2$-diazabicyclo[3. 1. 1]heptanylene-$(CH_2)_5$-, -$(CH_2)_2$-diazabicyclo[3.1. 1]heptanylene-$(CH_2)_6$-, -$(CH_2)_2$-diazabicyclo[3. 1. 1]heptanylene-$(CH_2)_7$-, -$(CH_2)_2$-diazabicyclo[3.1. 1]heptanylene-$(CH_2)_8$-, -$(CH_2)_3$-diazabicyclo[3.1.1]heptanylene-$CH_2$-, -$(CH_2)_3$-diazabicyclo[3.1.1]heptanylene-$(CH_2)_2$-, -$(CH_2)_3$-diazabicyclo[3.1.1]heptanylene-$(CH_2)_3$-, -$(CH_2)_3$-diazabicyclo[3.1.1]heptanylene-$(CH_2)_4$-, -$(CH_2)_3$-diazabicyclo[3.1.1]heptanylene-$(CH_2)_5$-, -$(CH_2)_3$-diazabicyclo[3.1.1]heptanylene-$(CH_2)_6$-, -$(CH_2)_3$-diazabicyclo[3.1.1]heptanylene-$(CH_2)_7$-, -$(CH_2)_3$-diazabicyclo[3.1.1]heptanylene-$(CH_2)_8$-, -$(CH_2)_4$-diazabicyclo[3.1.1]heptanylene-$CH_2$-, -$(CH_2)_4$-diazabicyclo[3.1.1]heptanylene-$(CH_2)_2$-, -$(CH_2)_4$-diazabicyclo[3.1.1]heptanylene-$(CH_2)_3$-, -$(CH_2)_4$-diazabicyclo[3.1.1]heptanylene-$(CH_2)_4$-, -$(CH_2)_4$-diazabicyclo[3. 1.1]heptanylene-$(CH_2)_5$-, -$(CH_2)_4$-diazabicyclo[3.1. 1]heptanylene-$(CH_2)_6$-, -$(CH_2)_4$-diazabicyclo[3. 1. 1]heptanylene-$(CH_2)_7$-, -$(CH_2)_4$-diazabicyclo[3.1. 1]heptanylene-$(CH_2)_8$-, -$(CH_2)_5$-diazabicyclo[3.1.1]heptanylene-$(CH_Z)_1$-, -$(CH_2)_5$-diazabicyclo[3.1.1]heptanylene-$(CH_2)_2$-, -$(CH_2)_5$-diazabicyclo[3.1.1]heptanylene-$(CH_2)_3$-, -$(CH_2)_5$-diazabicyclo[3.1.1]heptanylene-$(CH_2)_4$-, -$(CH_2)_5$-diazabicyclo[3.1.1]heptanylene-$(CH_2)_5$-, -$(CH_2)_5$-diazabicyclo[3.1.1]heptanylene-$(CH_2)_6$-, -$(CH_2)_5$-diazabicyclo[3.1.1]heptanylene-$(CH_2)_7$-, -$(CH_2)_5$-diazabicyclo[3.1.1]heptanylene-$(CH_2)_8$-, -$(CH_2)_6$-diazabicyclo[3.1.1]heptanylene-$(CH_2)_1$-, -$(CH_2)_6$-diazabicyclo[3.1.1]heptanylene-$(CH_2)_2$-, -$(CH_2)_6$-diazabicyclo[3.1.1]heptanylene-$(CH_2)_3$-, -$(CH_2)_6$-diazabicyclo[3.1.1]heptanylene-$(CH_2)_4$-, -$(CH_2)_6$-diazabicyclo[3. 1. 1]heptanylene-$(CH_2)_5$-, -$(CH_2)_6$-diazabicyclo[3.1. 1]heptanylene-$(CH_2)_6$-, -$(CH_2)_6$-diazabicyclo[3. 1. 1]heptanylene-$(CH_2)_7$-, -$(CH_2)_6$-diazabicy-

clo[3.1. 1]heptanylene-$(CH_2)_8$-, -$(CH_2)_7$-diazabicyclo[3.1.1]heptanylene-$(CH_2)_1$-, -$(CH_2)_7$-diazabicyclo[3.1.1]heptanylene-$(CH_2)_2$-, -$(CH_2)_7$-diazabicyclo[3.1.1]heptanylene-$(CH_2)_3$-, -$(CH_2)_7$-diazabicyclo[3.1.1]heptanylene-$(CH_2)_4$-, -$(CH_2)_7$-diazabicyclo[3.1. l]heptanylene-$(CH_2)_8$-, -$(CH_2)_8$-diazabicyclo[3.1.1]heptanylene-$CH_2$-, -$(CH_2)_8$-diazabicyclo[3.1.1]heptanylene-$(CH_2)_2$-, -$(CH_2)_8$-diazabicyclo[3.1.1]heptanylene-$(CH_2)_3$-, -$(CH_2)_8$-diazabicyclo[3.1.1]heptanylene-$(CH_2)_4$-, -$(CH_2)_8$-diazabicyclo[3.1.1]heptanylene-$(CH_2)_5$-, -$(CH_2)_8$-diazabicyclo[3.1.1]heptanylene-$(CH_2)_6$-, -$(CH_2)_8$-diazabicyclo[3.1.1]heptanylene-$(CH_2)_7$-, -$(CH_2)_8$-diazabicyclo[3.1.1]heptanylene-$(CH_2)_8$-, -$CH_2$-diazabicyclo[3.2.1]octylene-$CH_2$-, -$CH_2$-diazabicyclo[3.2.1]octylene-$(CH_2)_2$-, -$CH_2$-diazabicyclo[3.2.1]octylene-$(CH_2)_3$-, -$CH_2$-diazabicyclo[3.2.1]octylene-$(CH_2)_4$-, -$CH_2$-diazabicyclo[3.2.1]octylene-$(CH_2)_5$-, -$CH_2$-diazabicyclo[3.2.1]octylene-$(CH_2)_6$-, -$CH_2$-diazabicyclo [3.2.1]octylene-$(CH_2)_7$-, -$CH_2$-diazabicyclo[3.2.1]octylene-$(CH_2)_8$-, -$(CH_2)_2$-diazabicyclo[3.2.1]octylene-$(CH_2)_1$-, -$(CH_2)_2$-diazabicyclo[3.2.1]octylene-$(CH_2)_2$-, -$(CH_2)_2$-diazabicyclo[3.2.1]octylene-$(CH_2)_3$-, -$(CH_2)_2$-diazabicyclo[3.2.1] octylene-$(CH_2)_4$-, -$(CH_2)_2$-diazabicyclo[3.2.1]octylene-$(CH_2)_5$-, -$(CH_2)_2$-diazabicyclo[3.2.1]octylene-$(CH_2)_6$-, -$(CH_2)_2$-diazabicyclo[3.2.1]octylene-$(CH_2)_7$-, -$(CH_2)_2$-diazabicyclo[3.2.1]octylene-$(CH_2)_8$-, -$(CH_2)_3$-diazabicyclo[3.2.1]octylene-$CH_2$-, -$(CH_2)_3$-diazabicyclo[3.2.1]octylene-$(CH_2)_2$-, -$(CH_2)_3$-diazabicyclo[3.2.1]octylene-$(CH_2)_3$-, -$(CH_2)_3$-diazabicyclo [3.2.1]octylene-$(CH_2)_4$-, -$(CH_2)_3$-diazabicyclo[3.2.1]octylene-$(CH_2)_5$-, -$(CH_2)_3$-diazabicyclo[3.2.1]octylene-$(CH_2)_6$-, -$(CH_2)_3$-diazabicyclo[3.2.1]octylene-$(CH_2)_7$-, -$(CH_2)_3$-diazabicyclo[3.2.1]octylene-$(CH_2)_8$-, -$(CH_2)_4$-diazabicyclo[3.2.1] octylene-$CH_2$-, -$(CH_2)_4$-diazabicyclo[3.2.1]octylene-$(CH_2)_2$-, -$(CH_2)_4$-diazabicyclo[3.2.1]octylene-$(CH_2)_3$-, -$(CH_2)_4$-diazabicyclo[3.2.1]octylene-$(CH_2)_4$-, -$(CH_2)_4$-diazabicyclo[3.2.1]octylene-$(CH_2)_5$-, -$(CH_2)_4$-diazabicyclo[3.2.1]octylene-$(CH_2)_6$-, -$(CH_2)_4$-diazabicyclo[3.2.1]octylene-$(CH_2)_7$-, -$(CH_2)_4$-diazabicyclo[3.2.1]octylene-$(CH_2)_8$-, -$(CH_2)_5$-diazabicyclo[3.2.1]octylene-$(CH_2)_1$-, -$(CH_2)_5$-diazabicyclo[3.2.1]octylene-$(CH_2)_2$-, -$(CH_2)_5$-diazabicyclo[3.2.1]octylene-$(CH_2)_3$-, -$(CH_2)_5$-diazabicyclo[3.2.1]octylene-$(CH_2)_4$-, -$(CH_2)_5$-diazabicyclo[3.2.1]octylene-$(CH_2)_5$-, -$(CH_2)_5$-diazabicyclo[3.2.1]octylene-$(CH_Z)_6$-, -$(CH_2)_5$-diazabicyclo[3.2.1]octylene-$(CH_2)_7$-, -$(CH_2)_5$-diazabicyclo[3.2.1]octylene-$(CH_2)_8$-, -$(CH_2)_6$-diazabicyclo[3.2.1]octylene-$(CH_2)_1$-, -$(CH_2)_6$-diazabicyclo[3.2.1]octylene-$(CH_2)_2$-, -$(CH_2)_6$-diazabicyclo[3.2.1]octylene-$(CH_2)_3$-, -$(CH_2)_6$-diazabicyclo[3.2.1]octylene-$(CH_2)_4$-, -$(CH_2)_6$-diazabicyclo[3.2.1]octylene-$(CH_2)_5$-, -$(CH_2)_6$-diazabicyclo[3.2.1]octylene-$(CH_Z)_6$-, -$(CH_2)_6$-diazabicyclo[3.2.1]octylene-$(CH_2)_7$-, -$(CH_2)_6$-diazabicyclo[3.2.1]octylene-$(CH_2)_8$-, -$(CH_2)_7$-diazabicyclo[3.2.1]octylene-$(CH_2)_1$-, -$(CH_2)_7$-diazabicyclo[3.2.1] octylene-$(CH_2)_2$-, -$(CH_2)_7$-diazabicyclo[3.2.1]octylene-$(CH_2)_3$-, -$(CH_2)_7$-diazabicyclo[3.2.1]octylene-$(CH_2)_4$-, -$(CH_2)_7$-diazabicyclo[3.2.1]octylene-$(CH_2)_8$-, -$(CH_2)_8$-diazabicyclo[3.2.1]octylene-$CH_2$-, -$(CH_2)_8$-diazabicyclo[3.2.1]octylene-$(CH_2)_2$-, -$(CH_2)_8$-diazabicyclo[3.2.1]octylene-$(CH_2)_3$-, -$(CH_2)_8$-diazabicyclo[3.2.1]octylene-$(CH_2)_4$-, -$(CH_2)_8$-diazabicyclo[3.2.1]octylene-$(CH_2)_5$-, -$(CH_2)_8$-diazabicyclo[3.2.1]octylene-$(CH_2)_6$-, -$(CH_2)_8$-diazabicyclo[3.2.1]octylene-$(CH_2)_7$-, -$(CH_2)_8$-diazabicyclo[3.2.1]octylene-$(CH_2)_8$-, -$CH_2$-diazabicyclo[2.2.2]octylene-$CH_2$-, -$CH_2$-diazabicyclo [2.2.2]octylene-$(CH_2)_2$-, -$CH_2$-diazabicyclo[2.2.2]octylene-$(CH_2)_3$-, -$CH_2$-diazabicyclo[2.2.2]octylene-$(CH_2)_4$-, -$CH_2$-diazabicyclo[2.2.2]octylene-$(CH_2)_5$-, -$CH_2$-diazabicyclo[2.2.2]octylene-$(CH_2)_6$-, -$CH_2$-diazabicyclo[2.2.2]octylene-$(CH_2)_7$-, -$CH_2$-diazabicyclo[2.2.2]octylene-$(CH_2)_8$-, -$(CH_2)_2$-diazabicyclo[2.2.2]octylene-$(CH_2)_1$-, -$(CH_2)_2$-diazabicyclo[2.2.2]octylene-$(CH_2)_2$-, -$(CH_2)_2$-diazabicyclo[2.2.2]octylene-$(CH_2)_3$-, -$(CH_2)_2$-diazabicyclo[2.2.2]octylene-$(CH_2)_4$-, -$(CH_2)_2$-diazabicyclo[2.2.2]octylene-$(CH_2)_5$-, -$(CH_2)_2$-diazabicyclo[2.2.2]octylene-$(CH_2)_6$-, -$(CH_2)_2$-diazabicyclo[2.2.2]octylene-$(CH_2)_7$-, -$(CH_2)_2$-diazabicyclo[2.2.2]octylene-$(CH_2)_8$-, -$(CH_2)_3$-diazabicyclo[2.2.2]octylene-$CH_2$-, -$(CH_2)_3$-diazabicyclo[2.2.2]octylene-$(CH_2)_2$-, -$(CH_2)_3$-diazabicyclo[2.2.2]octylene-$(CH_2)_3$-, -$(CH_2)_3$-diazabicyclo[2.2.2]octylene-$(CH_2)_4$-, -$(CH_2)_3$-diazabicyclo[2.2.2]octylene-$(CH_2)_5$-, -$(CH_2)_3$-diazabicyclo[2.2.2]octylene-$(CH_2)_6$-, -$(CH_2)_3$-diazabicyclo[2.2.2] octylene-$(CH_2)_7$-, -$(CH_2)_3$-diazabicyclo[2.2.2]octylene-$(CH_2)_8$-, -$(CH_2)_4$-diazabicyclo[2.2.2]octylene-$CH_2$-, -$(CH_2)_4$-diazabicyclo[2.2.2]octylene-$(CH_2)_2$-, -$(CH_2)_4$-diazabicyclo[2.2.2]octylene-$(CH_2)_3$-, -$(CH_2)_4$-diazabicyclo[2.2.2]octylene-$(CH_2)_4$-, -$(CH_2)_4$-diazabicyclo[2.2.2]octylene-$(CH_2)_5$-, -$(CH_2)_4$-diazabicyclo[2.2.2]octylene-$(CH_2)_6$-, -$(CH_2)_4$-diazabicyclo[2.2.2]octylene-$(CH_2)_7$-, -$(CH_2)_4$-diazabicyclo[2.2.2]octylene-$(CH_2)_8$-, -$(CH_2)_5$-diazabicyclo[2.2.2]octylene-$(CH_2)_1$-, -$(CH_2)_5$-diazabicyclo[2.2.2]octylene-$(CH_2)_2$-, -$(CH_2)_5$-diazabicyclo[2.2.2]octylene-$(CH_2)_3$-, -$(CH_2)_5$-diazabicyclo[2.2.2]octylene-$(CH_2)_4$-, -$(CH_2)_5$-diazabicyclo[2.2.2]octylene-$(CH_2)_5$-, -$(CH_2)_5$-diazabicyclo[2.2.2]octylene-$(CH_2)_6$-, -$(CH_2)_5$-diazabicyclo[2.2.2]octylene-$(CH_2)_7$-, -$(CH_2)_5$-diazabicyclo[2.2.2]octylene-$(CH_2)_8$-, -$(CH_2)_6$-diazabicyclo[2.2.2]octylene-$(CH_2)_1$-, -$(CH_2)_6$-diazabicyclo[2.2.2]octylene-$(CH_2)_2$-, -$(CH_2)_6$-diazabicyclo[2.2.2]octylene-$(CH_2)_3$-, -$(CH_2)_6$-diazabicyclo[2.2.2]octylene-$(CH_2)_4$-, -$(CH_2)_6$-diazabicyclo[2.2.2]octylene-$(CH_2)_5$-, -$(CH_2)_6$-diazabicyclo[2.2.2]octylene-$(CH_2)_6$-, -$(CH_2)_6$-diazabicyclo[2.2.2]octylene-$(CH_2)_7$-, -$(CH_2)_6$-diazabicyclo[2.2.2] octylene-$(CH_2)_8$-, -$(CH_2)_7$-diazabicyclo[2.2.2]octylene-$(CH_2)_1$-, -$(CH_2)_7$-diazabicyclo[2.2.2]octylene-$(CH_2)_2$-, -$(CH_2)_7$-diazabicyclo[2.2.2]octylene-$(CH_2)_3$-, -$(CH_2)_7$-diazabicyclo[2.2.2]octylene-$(CH_2)_4$-, -$(CH_2)_7$-diazabicyclo[2.2.2]octylene-$(CH_2)_8$-, -$(CH_2)_8$-diazabicyclo[2.2.2]octylene-$CH_2$-, -$(CH_2)_8$-diazabicyclo[2.2.2]octylene-$(CH_2)_2$-, -$(CH_2)_8$-diazabicyclo[2.2.2]octylene-$(CH_2)_3$-, -$(CH_2)_8$-diazabicyclo[2.2.2]octylene-$(CH_2)_4$-, -$(CH_2)_8$-diazabicyclo[2.2.2]octylene-$(CH_2)_5$-, -$(CH_2)_8$-diazabicyclo[2.2.2]octylene-$(CH_2)_6$-, -$(CH_2)_8$-diazabicyclo[2.2.2]octylene-$(CH_2)_7$-, or -$(CH_2)_8$-diazabicyclo [2.2.2]octylene-$(CH_2)_8$-;

wherein the propylene, the piperidinylene, the piperazinylene, the diazacycloheptanylene, the diazabicyclo[2.2.1] heptanylene, the diazabicyclo[3.1.1]heptanylene, the diazabicyclo[3.2.1]octylene, the diazabicyclo[2.2.2]octylene are each independently optionally substituted with a substituent selected from the group consisting of deuterium,

optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof; and

each $R_{g11}$ independently represents H or $C_{1-3}$ alkyl.

In some embodiments of the compound of Formula (I-5), $L_2$ represents the structure of the following formula:

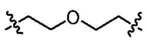 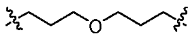 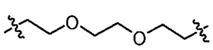 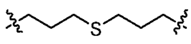 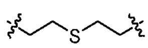 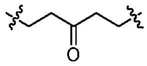

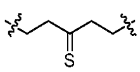 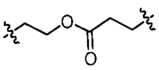 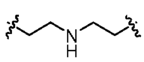 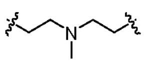 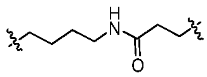 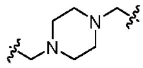 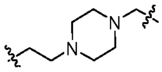

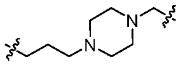 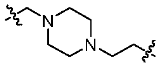 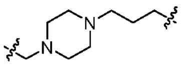 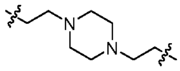 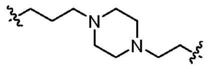 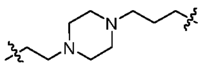

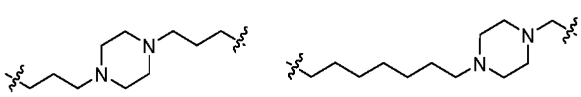 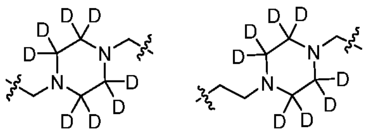 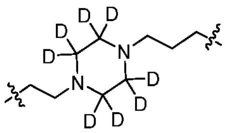

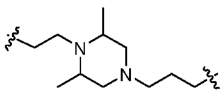 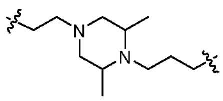 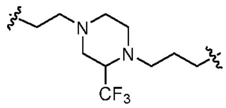 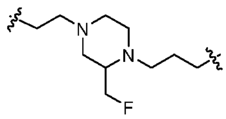 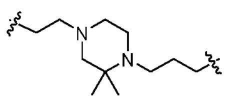

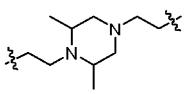 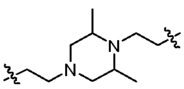 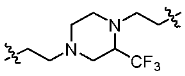 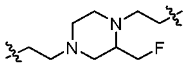 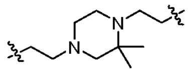 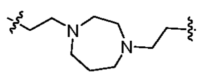

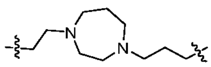 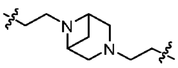 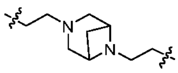 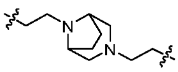 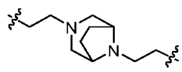 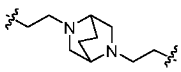

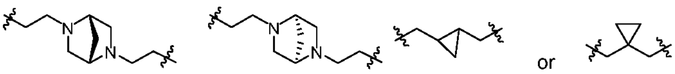 or

In some embodiments of the compound of Formula (I-5), $R_{b1}$ represents:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, 2-oxobicyclo[2.2.1]heptanyl or bicyclo[2.2.1]heptenyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 3-azaspiro[5.5]undecyl or 7-azaspiro[3.5]nonyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

phenyl or naphthyl, with each group being optionally substituted with one or more (e.g., 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl and imidazo[2,1-b]thiazolyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

In some embodiments of the compound of Formula (I-5), $R_{b1}$ represents:

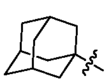 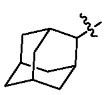 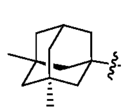 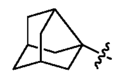 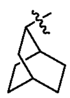 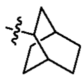 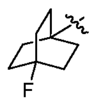 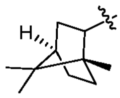 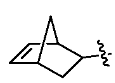 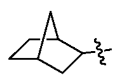

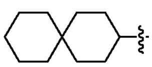 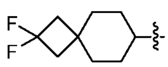 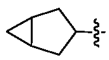 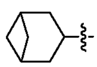 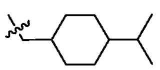 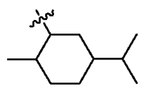 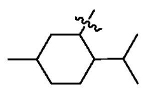 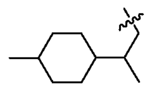

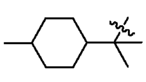 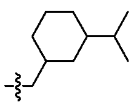 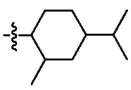 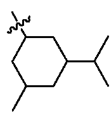 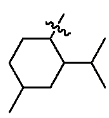 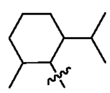 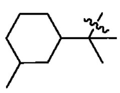 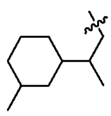 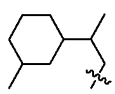

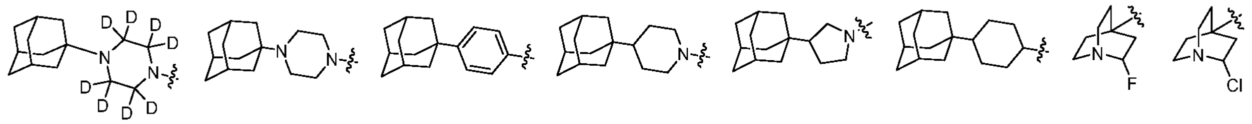

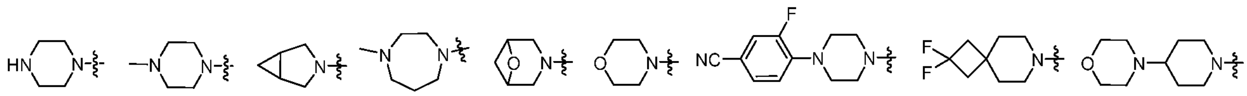

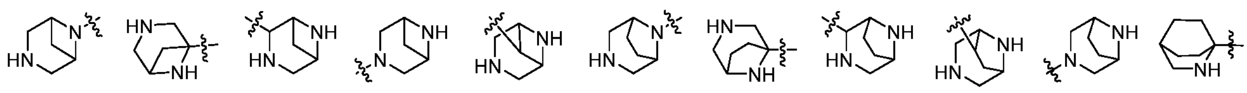

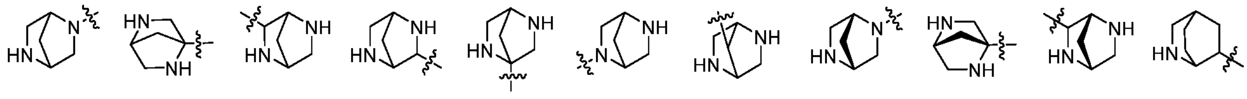

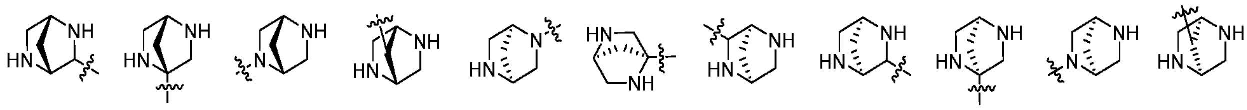

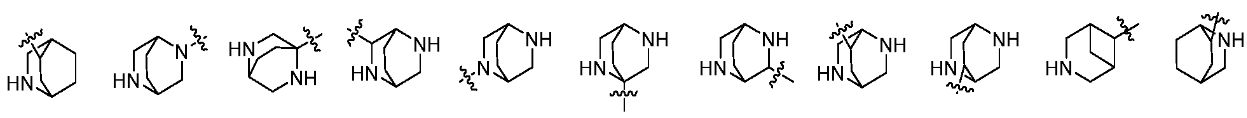

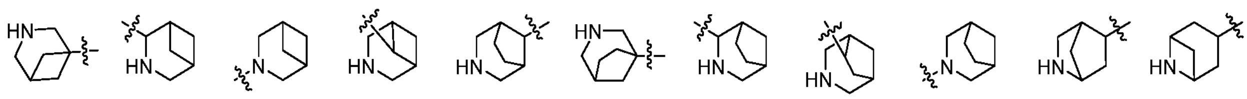

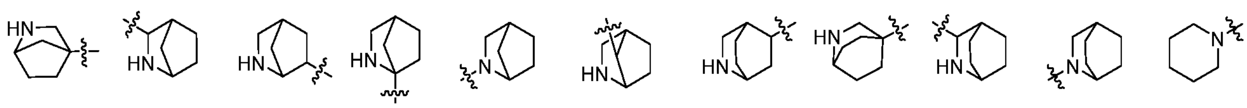

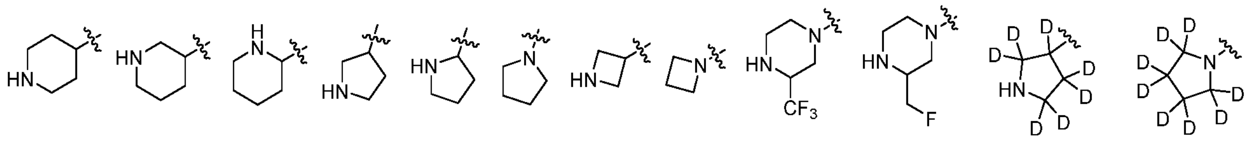

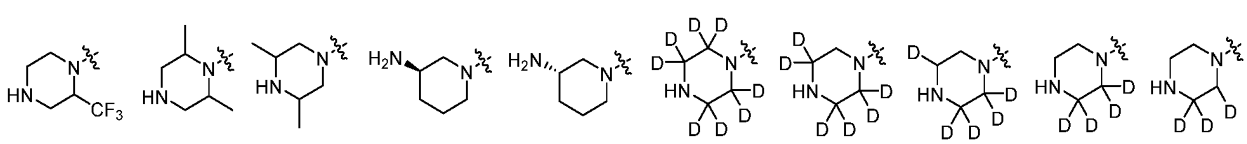

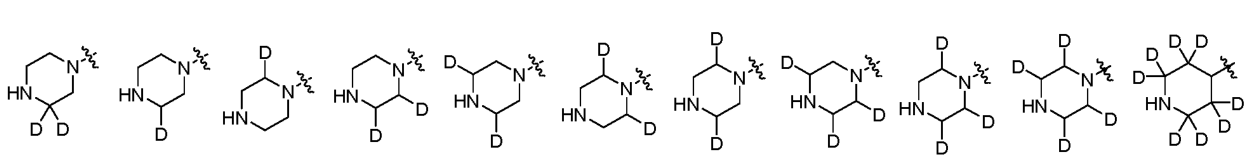

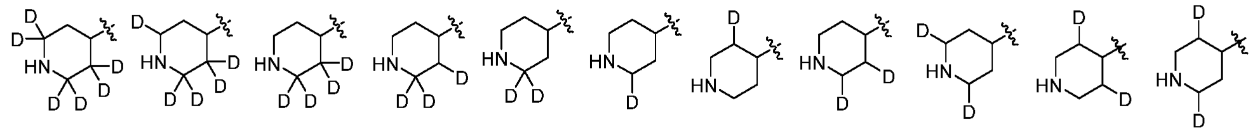

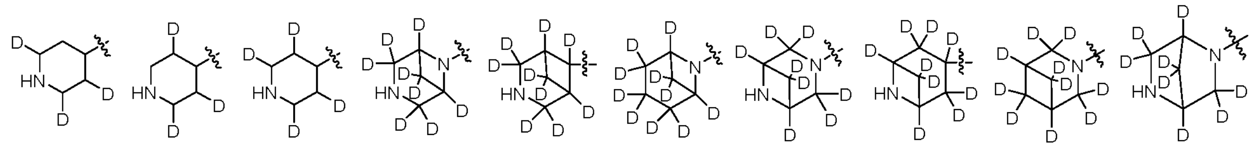

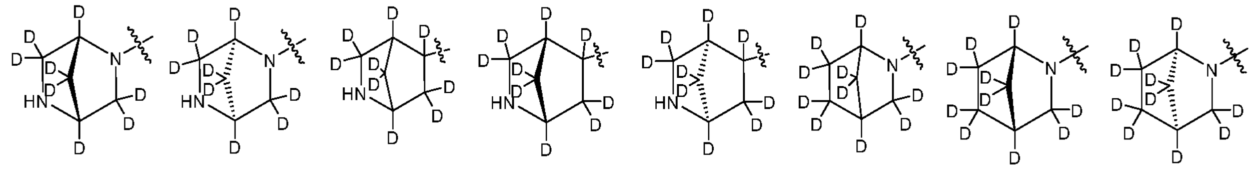

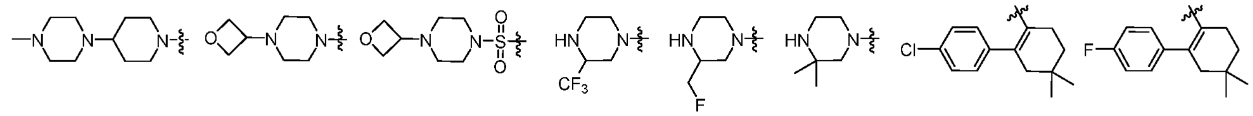

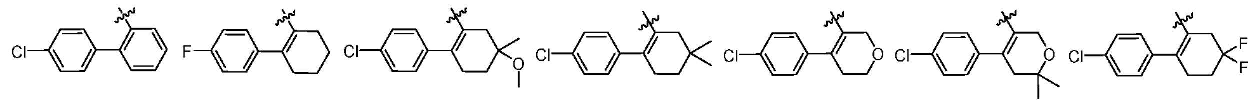

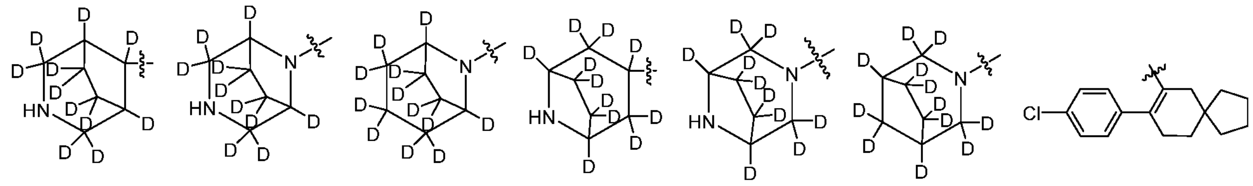

or

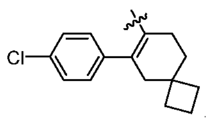

In some embodiments, the compound of Formula (I) is also Formula (I-6):

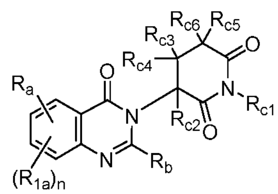

Formula (I-6)

wherein $R_{c1}$, $R_{c2}$, $R_{c3}$, $R_{c4}$, $R_{c5}$, $R_{c6}$, $R_b$, $(R_{1a})_n$, $R_a$, $R_{1a}$ and n are as defined in the compound of Formula (I) above and the embodiments thereof;

wherein $R_a$ represents the following formula:

$R_{a1}$-$X_2$-$L_1$-$X_1$- ,

wherein $X_1$ represents N($R_6$), O, S, alkynylene (e.g., $C_{2-6}$ alkynylene), or alkenylene (e.g., $C_{2-6}$ alkenylene), wherein $R_6$ represents H or $C_{1-3}$ alkyl, or $X_1$ represents a bond;

$L_1$ represents optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_7$ and/or one or more (e.g., 1-20, or 1-15, 1-10, 1-9, 1-8, 1-7, 1-6, 1-5, 1-4, 1-3 or 1-2, or 1) groups $R_8$ and/or any combination of one or more (e.g., 1-20, or 1-15, 1-10, 1-9, 1-8, 1-7, 1-6, 1-5, 1-4, 1-3 or 1-2, or 1) groups $R_7$ and $R_8$ inserted into its backbone carbon chain, wherein carbon-carbon bond between one or more pairs (e.g., 1-20, or 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_7$, $R_8$ or a combination of $R_7$ and Rs; wherein each $R_7$ is independently selected from the group consisting of O, S, N($R_9$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_9$ represents H or $C_{1-3}$ alkyl, and in case that two or more groups $R_7$ are inserted into the backbone carbon chain of the linear or branched $C_{2-60}$ alkylene group, the two or more groups $R_7$ are not directly connected to each other; and wherein each $R_8$ is independently selected from the group consisting of (e.g., optionally substituted $C_{3-20}$cycloalkylene, or optionally substituted $C_{3-15}$cycloalkylene), optionally substituted heterocyclylene (e.g., optionally substituted 4- to 20-membered heterocyclylene, or optionally substituted 5- to 15-membered heterocyclylene), triazolylene, alkenylene (e.g., $C_{2-6}$ alkenylene), alkynylene (e.g., $C_{2-6}$ alkynylene) or any combination thereof, wherein the cycloalkylene, the heterocyclylene and the triazolylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof;

$X_2$ represents N($R_{10}$), C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted 4- to 8-membered heterocyclylene containing 1 to 2 nitrogen atoms, triazolylene, triazolylene-NH-, -NH-triazolylene, or optionally substituted phenylene, wherein $R_{10}$ represents H or $C_{1-3}$ alkyl, or $X_2$ represents a bond; and

$R_{a1}$ represents hydroxy, optionally substituted heterocyclyl (e.g., optionally substituted 4- to 20-membered heterocyclyl, or optionally substituted 5- to 15-membered heterocyclyl) or optionally substituted cycloalkyl (e.g., optionally substituted $C_{3-20}$cycloalkyl, or optionally substituted $C_{3-15}$cycloalkyl), wherein when $R_{a1}$ represents hydroxy, $X_2$ represents a bond;

and

$R_b$ represents $C_{1-60}$ alkyl (e.g., $C_1$-$C_{30}$ alkyl, $C_1$-$C_{29}$ alkyl, $C_1$-$C_{28}$ alkyl, $C_1$-$C_{27}$ alkyl, $C_1$-$C_{26}$ alkyl, $C_1$-$C_{25}$ alkyl, $C_1$-$C_{24}$ alkyl, $C_1$-$C_{23}$ alkyl, $C_1$-$C_{22}$ alkyl, $C_1$-$C_{21}$ alkyl, $C_1$-$C_{20}$ alkyl, $C_1$-$C_{19}$ alkyl, $C_1$-$C_{18}$ alkyl, $C_1$-$C_{17}$ alkyl, $C_1$-$C_{16}$ alkyl, $C_1$-$C_{15}$ alkyl, $C_1$-$C_{14}$ alkyl, $C_1$-$C_{13}$ alkyl, $C_1$-$C_{12}$ alkyl, $C_1$-$C_{11}$ alkyl, $C_1$-$C_{10}$ alkyl, $C_1$-$C_9$ alkyl, $C_1$-$C_8$ alkyl, $C_1$-$C_7$ alkyl, $C_1$-$C_6$ alkyl, $C_1$-$C_5$ alkyl, $C_1$-$C_4$ alkyl, $C_1$-$C_3$ alkyl, or $C_1$-$C_2$ alkyl) optionally substituted with D or halogen, or $R_b$ represents the following formula:

$R_{b1}$-$X_4$-$L_2$-,

wherein $L_2$ represents:

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene), or

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5,

1-4, 1-3 or 1-2, or 1) groups $R_{12}$ and/or one or more (e.g., 1-20, or 1-15, 1-10, 1-9, 1-8, 1-7, 1-6, 1-5, 1-4, 1-3 or 1-2, or 1) groups $R_{13}$ and/or any combination of one or more (e.g., 1-20, or 1-15, 1-10, 1-9, 1-8, 1-7, 1-6, 1-5, 1-4, 1-3 or 1-2, or 1) groups $R_{12}$ and $R_{13}$ inserted into its backbone carbon chain, wherein carbon-carbon bond between one or more pairs (e.g., 1-20, or 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_{12}$, $R_{13}$ or a combination of $R_{12}$ and $R_{13}$; wherein each $R_{12}$ is independently selected from the group consisting of O, S, N($R_{14}$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of cycloalkylene (e.g., $C_{3-20}$cycloalkylene, or $C_{3-15}$cycloalkylene), heterocyclylene (e.g., 4-to 20-membered heterocyclylene, or 4- to 15-membered heterocyclylene), arylene (e.g., $C_{6-10}$ arylene), heteroarylene (e.g., 5- to 20-membered heteroarylene, or 5- to 15-membered heteroarylene), alkenylene (e.g., $C_{2-6}$ alkenylene), alkynylene (e.g., $C_{2-6}$ alkynylene) or any combination thereof, wherein the cycloalkylene, the heterocyclylene, arylene and the heteroarylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof;

$X_4$ represents N($R_{15}$), C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted cycloalkylene (e.g., optionally substituted $C_{3-20}$cycloalkylene, or optionally substituted $C_{3-15}$cycloalkylene), optionally substituted arylene (e.g., optionally substituted $C_{6-20}$ arylene, or optionally substituted $C_{6-10}$ arylene), optionally substituted heterocyclylene (e.g., optionally substituted 4- to 20-membered heterocyclylene, or optionally substituted 4- to 15-membered heterocyclylene), optionally substituted heteroarylene (e.g., optionally substituted 5- to 20-membered heteroarylene, or optionally substituted 5- to 15-membered heteroarylene), triazolylene-NH-, or -NH-triazolylene, wherein $R_{15}$ represents H or $C_{1-3}$ alkyl, or $X_4$ represents a bond; and

$R_{b1}$ represents optionally substituted cycloalkyl (e.g., optionally substituted $C_{3-20}$cycloalkyl, or optionally substituted $C_{3-15}$cycloalkyl), optionally substituted aryl (e.g., optionally substituted $C_{6-20}$ aryl, or optionally substituted $C_{6-10}$ aryl), optionally substituted heterocyclyl (e.g., optionally substituted 4- to 20-membered heterocyclyl, or optionally substituted 4- to 15-membered heterocyclyl) or optionally substituted heteroaryl (e.g., optionally substituted 5- to 20-membered heteroaryl, or optionally substituted 5- to 15-membered heteroaryl).

In some embodiments of the compound of Formula (I-6), $R_a$ represents the following formula:

$$R_{a1}\text{-}X_2\text{-}L_1\text{-}X_1\text{-} ,$$

wherein $X_1$ represents a bond, or $X_1$ represents N($R_6$), O, S, $C_{2-6}$ alkynylene, or $C_{2-6}$ alkenylene, wherein $R_6$ represents H or $C_{1-3}$ alkyl;

$L_1$ represents optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_7$ and/or one or more (e.g., 1-20, or 1-15, 1-10, 1-9, 1-8, 1-7, 1-6, 1-5, 1-4, 1-3 or 1-2, or 1) groups $R_8$ and/or any combination of one or more (e.g., 1-20, or 1-15, 1-10, 1-9, 1-8, 1-7, 1-6, 1-5, 1-4, 1-3 or 1-2, or 1) groups $R_7$ and $R_8$ inserted into its backbone carbon chain, wherein carbon-carbon bond between one or more pairs (e.g., 1-20, or 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_7$, $R_8$ or a combination of $R_7$ and Rs; wherein each $R_7$ is independently selected from the group consisting of O, S, N($R_9$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_9$ represents H or $C_{1-3}$ alkyl, and in case that two or more groups $R_7$ are inserted into the backbone carbon chain of the linear or branched $C_{2-60}$ alkylene group, the two or more groups $R_7$ are not directly connected to each other; and wherein each $R_8$ is independently selected from the group consisting of $C_{3-15}$cycloalkylene, 4- to 15-membered heterocyclylene, triazolylene or any combination thereof, wherein the $C_{3-15}$cycloalkylene, the 4- to 15-membered heterocyclylene and the triazolylene are each independently optionally substituted with a substituent selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl, hydroxy, amino, mercapto,

halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof, and wherein the linear or branched $C_{2-60}$ alkylene is optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, nitro, halogen, cyano, halogenated $C_{1-6}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), optionally deuterated $C_{3-6}$cycloalkyl, $C_{1-6}$ alkoxy (e.g., $C_{1-3}$ alkoxy, such as methoxy, ethoxy, or propoxy), or any combination thereof;

$X_2$ represents a bond, or $X_2$ represents N($R_{10}$), C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted 4- to 8-membered heterocyclylene containing 1 to 2 nitrogen atoms, triazolylene, triazolylene-NH-, -NH-triazolylene, or optionally substituted phenylene, wherein $R_{10}$ represents H or $C_{1-3}$ alkyl, and the 4- to 8-membered heterocyclylene containing 1 to 2 nitrogen atoms is optionally substituted with 1 to 8 substituents selected from the group consisting of D, halogen, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), oxo, hydroxy, amino, mercapto, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof; and the phenylene is optionally substituted with 1 to 4 substituents selected from the group consisting of D, halogen, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene, $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof; and

$R_{a1}$ represents hydroxy, optionally substituted 4- to 20-membered heterocyclyl or optionally substituted $C_{3-20}$cycloalkyl, wherein when $R_{a1}$ represents hydroxy, $X_2$ represents a bond, and the 4- to 20-membered heterocyclyl and the $C_{3-20}$cycloalkyl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof.

In some embodiments of the compound of Formula (I-6), $R_{a1}$ represents hydroxy, and $X_2$ represents a bond.

In some embodiments of the compound of Formula (I-6), $R_{a1}$ represents:

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl, diazacyclooctyl, 1,3-diazacycloheptanyl, 1,4-diazacycloheptanyl, 4,5-diazacycloheptanyl, 5- to 15-memberedbridged heterocyclyl (e.g., 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, diazabicyclo[3.2.1]octyl, diazabicyclo[2.2.2]octyl, 3,8-diazabicyclo[3.2.1]octan-3-yl, and 2,5-diazabicyclo[2.2.2]octan-2-yl), or 5- to 15-memberedazaspirocycloalkyl (e.g., 3-azaspiro[5.5]undecylene and 7-azaspiro[3.5]nonylene), with each group being independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of: D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bomyl, bicyclo[2.2.1]heptanyl, 2-oxobicyclo[2.2.1]heptanyl or bicyclo[2.2.1]heptenyl, with each group being independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of: D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

In some embodiments of the compound of Formula (I-6), $R_{a1}$ represents:

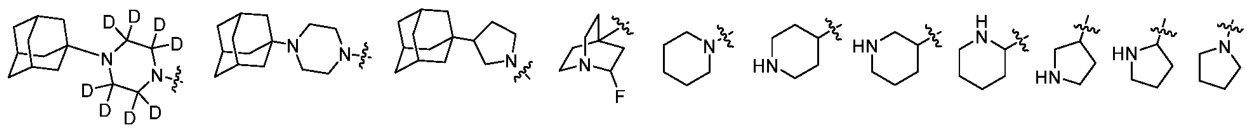

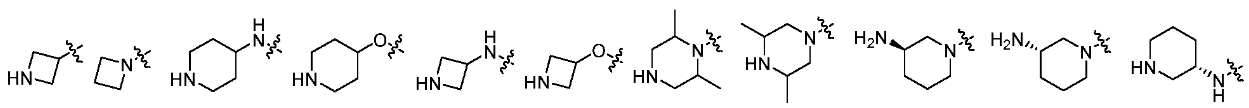

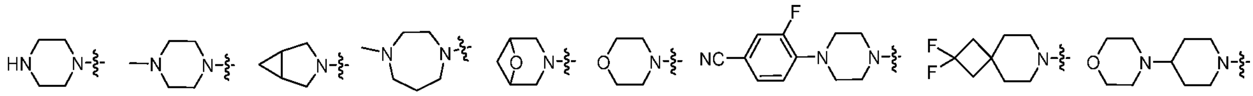

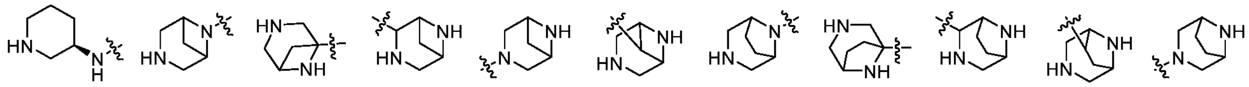

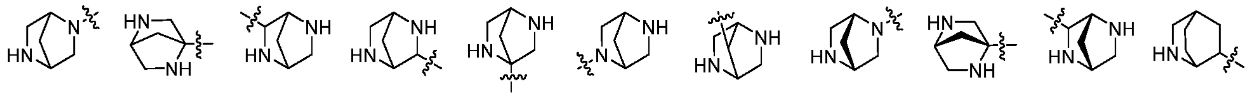

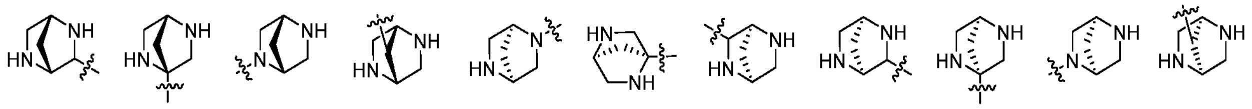

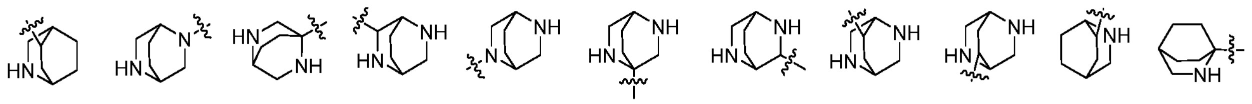

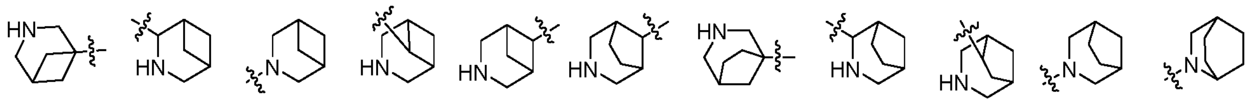

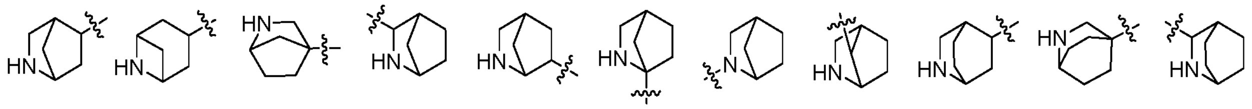

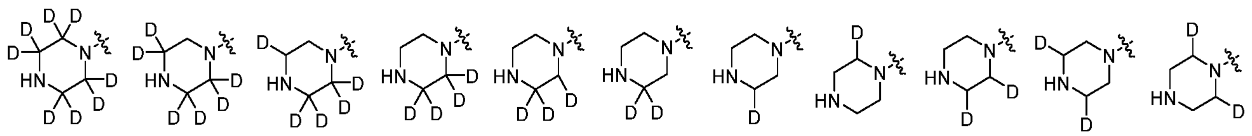

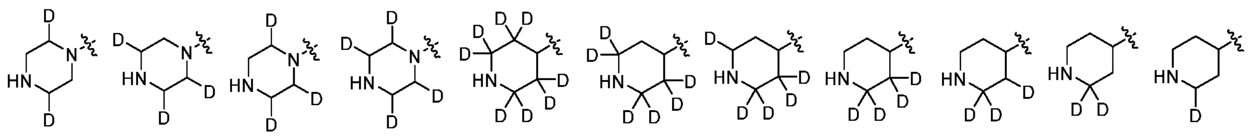

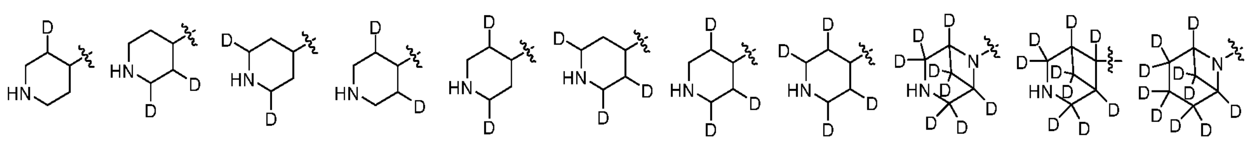

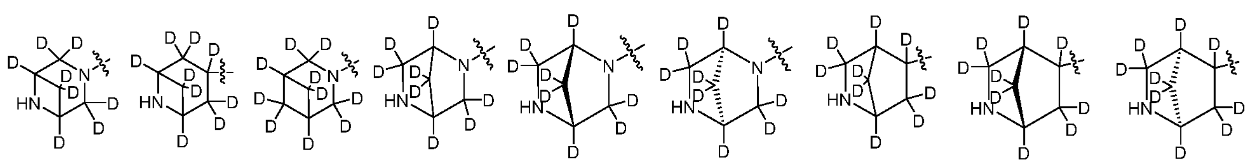

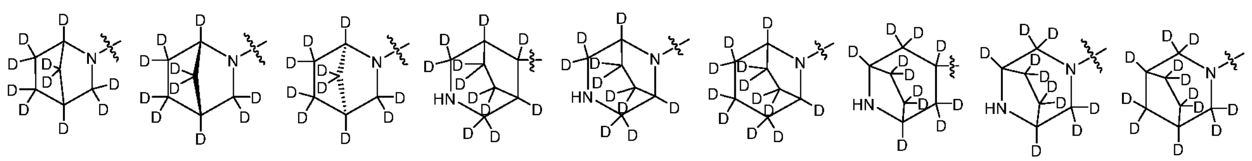

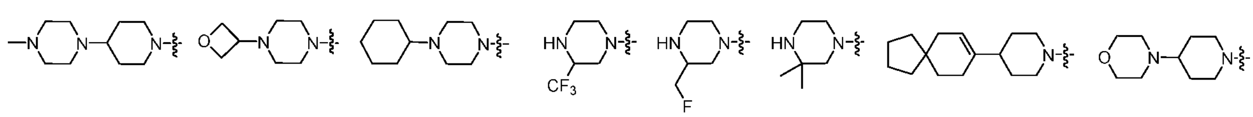

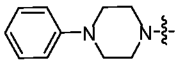 or 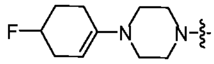

In some embodiments of the compound of Formula (I-6), $L_1$ represents the structure of the following formula:

##-$(C(R^{a1})(R^{a2}))_{m1}-(R_7(C(R^{a3})(R^{a4}))_{m2})_{p1}-$;

##-$(C(R^{a1})(R^{a2}))_{m1}-(R_7(C(R^{a3})(R^{a4}))_{m2})_{p}-(R_7(C(R^{a5})(R^{a6}))_{m3})_{p2}-$;

##-$(C(R^{a1})(R^{a2}))_{m1}-(R_7(C(R^{a3})(R^{a4}))_{m2})_{p1}-(R_7(C(R^{a5})(R^{a6}))_{m3})_{p2}-(R_7(C(R^{a7})(R^{a8}))_{m4})_{p3}-$;

##-$(C(R^{a1})(R^{a2}))_{m1}-(R_8(C(R^{a3})(R^{a4}))_{m2})_{p1}-$;

##-$(C(R^{a1})(R^{a2}))_{m1}-(R_8(C(R^{a3})(R^{a4}))_{m2})_{p1}-(R_8(C(R^{a5})(R^{a6}))_{m3})_{p2}-(R_8(C(R^{a7})(R^{a8}))_{m4})_{p3}-$;

##-$(C(R^{a1})(R^{a2}))_{m1}-(R_8(C(R^{a3})(R^{a4}))_{m2})_{p1}-(R_8(C(R^{a5})(R^{a6}))_{m3})_{p2}-(R_8(C(R^{a7})(R^{a8}))_{m4})_{p3}-$;

##-$(C(R^{a1})(R^{a2}))_{m1}-(R_7-R_8-(C(R^{a3})(R^{a4}))_{m2})_{p1}-$;

##-$(C(R^{a1})(R^{a2}))_{m1}-(R_7(C(R^{a3})(R^{a4}))_{m2})_{p1}(-R_8(C(R^{a5})(R^{a6}))_{m3})_{p2}-$;

##-$(C(R^{a1})(R^{a2}))_{m1}-(R_8-R_7-(C(R^{a3})(R^{a4}))_{m2})_{p1}-$; or

##-$(C(R^{a1})(R^{a2}))_{m1}-(R_8(C(R^{a3})(R^{a4}))_{m2})_{p1}(-R_7(C(R^{a5})(R^{a6}))_{m3})_{p2}-$;

wherein each group $R_7$ is independently selected from the group consisting of O, S, $N(R_9)$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_9$ independently represents H or $C_{1-3}$ alkyl; and each group $R_8$ is independently selected from the group consisting of $C_{3-15}$cycloalkylene, 4- to 15-membered heterocyclylene, triazolylene or any combination thereof, wherein the $C_{3-15}$cycloalkylene, the 4- to 15-membered heterocyclylene and the triazolylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;
$R^{a1}$, $R^{a2}$, $R^{a3}$, $R^{a4}$, $R^{a5}$, $R^{a6}$, $R^{a7}$ and $R^{a8}$ each independently represent H, deuterium, $C_{1-4}$ alkyl, halogen, $C_{3-6}$cycloalkyl or any combination thereof;
symbol ## indicates the point of attachment to $X_1$; and
ml, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-6), $L_1$ represents the structure of the following formula:

##-$(C(R^{a1})(R^{a2}))_{m1}-(O(C(R^{a3})(R^{a4}))_{m2})_{p1}-$;

##-$(C(R^{a1})(R^{a2}))_{m1}-(O(C(R^{a3})(R^{a4}))_{m2})_{p1}-(O(C(R^{a5})(R^{a6}))_{m3})_{p2}-$;

##-$(C(R^{a1})(R^{a2}))_{m1}-(O(C(R^{a3})(R^{a4}))_{m2})_{p1}-(O(C(R^{a5})(R^{a6}))_{m3})_{p2}-(O(C(R^{a7})(R^{a8}))_{m4})_{p3}-$;

##-$(C(R^{a1})(R^{a2}))_{m1}-(N(R_9)(C(Ra^{3})(Ra^{4}))_{m2})_{p1}-$;

##-$(C(R^{a1})(R^{a2}))_{m1}-(N(R_9)(C(R^{a3})(R^{a4}))_{m2})_{p1}-(N(R_9)(C(R^{a5})(R^{a6}))_{m3})_{p2}-$;

##-$(C(R^{a1})(R^{a2}))_{m1}-(N(R_9)(C(R^{a3})(R^{a4}))_{m2})_{p1}-(N(R_9)(C(R^{a5})(R^{a6}))_{m3})_{p2}-(N(R_9)(C(R^{a7})(R^{a8}))_{m4})_{p3}-$;

##-$(C(R^{a1})(R^{a2}))_{m1}-(C(O)NH-(C(R^{a3})R^{a4}))_{m2})_{p1}-$;

##-$(C(R^{a1})(R^{a2}))_{m1}-(C(O)NH-(C(R^{a3})R^{a4}))_{m2})_{p1}-(C(O)NH-(C(R^{a5})(R^{a6}))_{m3})_{p2}-$;

##-$(C(R^{a1})(R^{a2}))_{m1}-(C(O)NH-(C(R^{a3})R^{a4}))_{m2})_{p1}-(C(O)NH-(C(R^{a5})(R^{a6}))_{m3})_{p2}-(C(O)NH-$; $(C(R^{a7})$

$(R^{a8}))_{m4})_{p3}$-;

$\#\#-(C(R^{a1})(R^{a2}))_{m1}-(C(O)NH-(C(R^{a3})R^{a4}))_{m2})_{p1}-(O-(C(R^{a5})(R^{a6}))_{m3})_{p2}$-;

$\#\#-(C(R^{a1})(R^{a2}))_{m1}-(C(O)NH-(C(R^{a3})R^{a4}))_{m2}-(O-(C(R^{a5})(R^{a6}))_{m3})_{p1}$-;

$\#\#-(C(R^{a1})(R^{a2}))_{m1}-(NHC(O)-(C(R^{a3})(R^{a4}))_{m2})_{p1}$-;

$\#\#-(C(R^{a1})(R^{a2}))_{m1}-(NHC(O)-(C(R^{a3})(R^{a4}))_{m2})_{p1}-(O-(C(R^{a5})(R^{a6}))_{m3})_{p2}$-;

$\#\#-(C(R^{a1})(R^{a2}))_{m1}-(NHC(O)-(C(R^{a3})(R^{a4}))_{m2})_{p1}-(O-(C(R^{a5})(R^{a6}))_{m3})_{p2}-(O-(C(R^{a7})(R^{a8}))_{m4})_{p3}$-;

$\#\#-(C(R^{a1})(R^{a2}))_{m1}-(NHC(O)-(C(R^{a3})(R^{a4}))_{m2}(O(C(R^{a5})(R^{a6}))_{m3})_{p1}$-;

$\#\#-(C(R^{a1})(R^{a2}))_{m1}-NHC(O)NH-(C(R^{a3})(R^{a4}))_{m2}$-;

$\#\#-(C(R^{a1})(R^{a2}))_{m1}-C(O)-(C(R^{a3})(R^{a4}))_{m2}$-;

$\#\#-(C(R^{a1})(R^{a2}))_{m1}-C(S)-(C(R^{a3})(R^{a4}))_{m2}$-;

$\#\#-(C(R^{a1})(R^{a2}))_{m1}-C(S)-(C(R^{a3})(R^{a4}))_{m2}$-;

$\#\#-(C(R^{a1})(R^{a2}))_{m1}-S-(C(R^{a3})(R^{a4}))_{m2}$-;

$\#\#-(C(R^{a1})(R^{a2}))_{m1}$-(4- to 15-membered heterocyclylene-$(C(R^{a3})(R^{a4}))_{m2})_{p1}$-;

$\#\#-(C(R^{a1})(R^{a2}))_{m1}$-(4- to 15-membered heterocyclylene-$(C(R^{a3})(R^{a4}))_{m2})_{p1}$-(4- to 15-membered heterocyclylene-$(C(R^{a5})(R^{a6}))_{m3})_{p2}$-;

$\#\#-(C(R^{a1})(R^{a2}))_{m1}$-( triazolylene -$(C(R^{a3})(R^{a4}))_{m2})_{p1}$-;

$\#\#-(C(R^{a1})(R^{a2}))_{m1}$-( triazolylene -$(C(R^{a3})(R^{a4}))_{m2})_{p1}$-( triazolylene -$(C(R^{a5})(R^{a6}))_{m3})_{p2}$-; $\#\#-(C(R^{a1})(R^{a2}))_{m1}-(C_{3-15}$cycloalkylene-$(C(R^{a3})(R^{a4}))_{m2})_{p1}$-; or

$\#\#-(C(R^{a1})(R^{a2}))_{m1}-(C_{3-15}$cycloalkylene-$(C(R^{a3})(R^{a4}))_{m2})_{p1}-(C_{3-15}$cycloalkylene-$(C(R^{a5})(R^{a6}))_{m3})_{p2}$-;

wherein each $R_9$ independently represents H or $C_{1-3}$ alkyl;
the $C_{3-15}$cycloalkylene, the 4- to 15-membered heterocyclylene and the triazolylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;
$R^{a1}$, $R^{a2}$, $R^{a3}$, $R^{a4}$, $R^{as}$, $R^{a6}$, $R^{a7}$ and $R^{a8}$ each independently represent H, deuterium, $C_{1-4}$ alkyl, halogen, $C_{3-6}$cycloalkyl or any combination thereof;
symbol ## indicates the point of attachment to $X_1$; and
m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-6), $L_1$ represents the following group: ##-$CH_2$-O-$CH_2$-, ##-$CH_2$-O-$(CH_2)_2$-, ##-$(CH_2)_1$-O-$(CH_2)_3$-, ##-$(CH_2)_1$-O-$(CH_2)_4$-, ##-$(CH_2)_1$-O-$(CH_2)_5$-, ##-$(CH_2)_1$-O-$(CH_2)_6$-, ##-$(CH_2)_1$-O-$(CH_2)_7$-, ##-$(CH_2)_1$-O-$(CH_2)_8$-, ##-$(CH_2)_1$-O-$(CH_2)_9$-, ##-$(CH_2)_1$-O-$(CH_2)_{10}$-, ##-$(CH_2)_2$-O-$(CH_2)_1$-, ##-$(CH_2)_2$-O-$(CH_2)_2$-, ##-$(CH_2)_2$-O-$(CH_2)_3$-, ##-$(CH_2)_2$-O-$(CH_2)_4$-, ##-$(CH_2)_2$-O-$(CH_2)_5$-, ##-$(CH_2)_2$-O-$(CH_2)_6$-, ##-$(CH_2)_2$-O-$(CH_2)_7$-, ##-$(CH_2)_2$-O-$(CH_2)_8$-, ##-$(CH_2)_2$-O-$(CH_2)_9$-, ##-$(CH_2)_2$-O-$(CH_2)_{1O}$-, ##-$(CH_2)_2$-O-$(CH_2)_{ll}$-, ##-$(CH_2)_2$-O-$(CH_2)_{12}$-, ##-$(CH_2)_3$-O-$(CH_2)_1$-, ##-$(CH_2)_3$-O-$(CH_2)_2$-, ##-$(CH_2)_3$-O-$(CH_2)_3$-, ##-$(CH_2)_3$-O-$(CH_2)_4$-, ##-$(CH_2)_3$-O-$(CH_2)_5$-, ##-$(CH_2)_3$-O-$(CH_2)_6$-, ##-$(CH_2)_3$-O-$(CH_2)_7$-, ##-$(CH_2)_4$-O-$(CH_2)_1$-, ##-$(CH_2)_4$-O-$(CH_2)_2$-, ##-$(CH_2)_4$-O-$(CH_2)_3$-, ##-$(CH_2)_4$-O-$(CH_2)_4$-, ##-$(CH_2)_4$-O-$(CH_2)_5$-, ##-$(CH_2)_4$-O-$(CH_2)_6$-, ##-$(CH_2)_5$-O-$(CH_2)_1$-, ##-$(CH_2)_5$-O-$(CH_2)_2$-, ##-$(CH_2)_5$-O-$(CH_2)_3$-, ##-$(CH_2)_5$-O-$(CH_2)_4$-, ##-$(CH_2)_5$-O-$(CH_2)_5$-, ##-$(CH_2)_6$-O-$(CH_2)_1$-, ##-$(CH_2)_6$-O-$(CH_2)_2$-, ##-$(CH_2)_6$-O-$(CH_2)_3$-, ##-$(CH_2)_6$-O-$(CH_2)_4$-, ##-$(CH_2)_7$-O-$(CH_2)_1$-,

##-$(CH_2)_7-O-(CH_2)_2$-, ##-$(CH_2)_7-O-(CH_2)_3$-, ##-$(CH_2)_8-O-(CH_2)_1$-, ##-$(CH_2)_8-O-(CH_2)_2$-, ##-$CH(CH_3)-O-(CH_2)_1$-, ##-$CH(CH_3)-O-(CH_2)_2$-, ##-$CH(CH_3)-O-(CH_2)_3$-, ##-$CH(CH_3)-O-(CH_2)_4$-, ##-$CH(CH_3)-O-(CH_2)_5$-, ##-$CH(CH_3)-O-(CH_2)_6$-, ##-$CH(CH_3)-O-(CH_2)_7$-, ##-$CH(CH_3)-O-(CH_2)_8$-, ##-$CH(CH_3)-O-(CH_2)_9$-, ##-$CH(CH_3)-O-(CH_2)_{10}$-, ##-$CH_2-(O(CH_2)_2)_2$-, ##-$CH_2-(O(CH_2)_2)_3$-, ##-$CH_2-(O(CH_2)_2)_4$-, ##-$CH_2-(O(CH_2)_2)_5$-, ##-$CH_2-(O(CH_2)_2)_6$-, 99-$CH_2-(O(CH_2)_2)_7$-, 99-$CH_2-(O(CH_2)_2)_8$-, ##-$CH_2-(O(CH_2)_2)_9$-, ##-$CH_2-(O(CH_2)_2)_{10}$-, ##-$CH_2-(O(CH_2)_2)_1-OCH_2$-, ##-$CH_2-(O(CH_2)_2)_2-OCH_2$-, ##-$CH_2-(O(CH_2)_2)_3-OCH_2$-, ##-$CH_2-(O(CH_2)_2)_4-OCH_2$-, ##-$CH_2-(O(CH_2)_2)_5-OCH_2$-, ##-$CH_2-(O(CH_2)_2)_6-OCH_2$-, ##-$CH_2-(O(CH_2)_2)_7-OCH_2$-, ##-$CH_2-(O(CH_2)_2)_8-OCH_2$-, ##-$CH_2-(O(CH_2)_2)_9-OCH_2$-, ##-$CH_2-(O(CH_2)_2)_{10}-OCH_2$-, ##-$(CH_2)_2-(O(CH_2)_2)_2$-, ##-$(CH_2)_2-(O(CH_2)_2)_3$-, ##-$(CH_2)_2-(O(CH_2)_2)_4$-, ##-$(CH_2)_2-(O(CH_2)_2)_5$-, ##-$(CH_2)_2-(O(CH_2)_2)_6$-, ##-$(CH_2)_2-(O(CH_2)_2)_7$-, ##-$(CH_2)_2-(O(CH_2)_2)_8$-, ##-$(CH_2)_2-(O(CH_2)_2)_9$-, ##-$(CH_2)_2-(O(CH_2)_2)_{10}$-, ##-$(CH_2)_3-(O(CH_2)_2)_2$-, ##-$(CH_2)_3-(O(CH_2)_2)_3$-, ##-$(CH_2)_3-(O(CH_2)_2)_4$-, ##-$(CH_2)_3-(O(CH_2)_2)_5$-, ##-$(CH_2)_3-(O(CH_2)_2)_6$-, ##-$(CH_2)_3-(O(CH_2)_2)_7$-, ##-$(CH_2)_3-(O(CH_2)_2)_8$-, ##-$(CH_2)_3-(O(CH_2)_2)_9$-, ##-$(CH_2)_3-(O(CH_2)_2)_{10}$-, ##-$(CH_2)_4-(O(CH_2)_2)_2$-, ##-$(CH_2)_4-(O(CH_2)_2)_3$-, ##-$(CH_2)_4-(O(CH_2)_2)_4$-, ##-$(CH_2)_4-(O(CH_2)_2)_5$-, ##-$(CH_2)_4-(O(CH_2)_2)_6$-, ##-$(CH_2)_4-(O(CH_2)_2)_7$-, ##-$(CH_2)_4-(O(CH_2)_2)_8$-, ##-$(CH_2)_4-(O(CH_2)_2)_9$-, ##-$(CH_2)_4-(O(CH_2)_2)_{10}$-, ##-$CH_2-(O(CH_2)_3)_2$-, ##-$CH_2-(O(CH_2)_3)_3$-, ##-$CH_2-(O(CH_2)_3)_4$-, ##-$CH_2-(O(CH_2)_3)_5$-, ##-$CH_2-(O(CH_2)_3)_6$-, ##-$CH_2-(O(CH_2)_3)_7$-, ##-$CH_2-(O(CH_2)_3)_8$-, ##-$CH_2-(O(CH_2)_3)_9$-, ##-$CH_2-(O(CH_2)_3)_{10}$-, ##-$(CH_2)_2-(O(CH_2)_3)_2$-, ##-$(CH_2)_2-(O(CH_2)_3)_3$-, ##-$(CH_2)_2-(O(CH_2)_3)_4$-, ##-$(CH_2)_2-(O(CH_2)_3)_5$-, ##-$(CH_2)_2-(O(CH_2)_3)_6$-, ##-$(CH_2)_2-(O(CH_2)_3)_7$-, ##-$(CH_2)_2-(O(CH_2)_3)_8$-, ##-$(CH_2)_2-(O(CH_2)_3)_9$-, ##-$(CH_2)_2-(O(CH_2)_3)_{10}$-, ##-$(CH_2)_3-(O(CH_2)_3)_2$-, ##-$(CH_2)_3-(O(CH_2)_3)_3$-, ##-$(CH_2)_3-(O(CH_2)_3)_4$-, ##-$(CH_2)_3-(O(CH_2)_3)_5$-, ##-$(CH_2)_3-(O(CH_2)_3)_6$-, ##-$(CH_2)_3-(O(CH_2)_3)_7$-, ##-$(CH_2)_3-(O(CH_2)_3)_8$-, ##-$(CH_2)_3-(O(CH_2)_3)_9$-, ##-$(CH_2)_3-(O(CH_2)_3)_{10}$-, ##-$CH_2-O-(CH_2)_2-O-(CH_2)_3$-, ##-$CH_2-(O(CH_2)_2)_2-(O(CH_2)_3)_2$-, ##-$CH_2-(O(CH_2)_2)_3-(O(CH_2)_3)_3$-, ##-$CH_2-(O(CH_2)_2)_4-(O(CH_2)_3)_4$-, ##-$CH_2-(O(CH_2)_2)_5-(O(CH_2)_3)_5$-, ##-$CH_2-(O(CH_2)_2)_6-(O(CH_2)_3)_6$-, ##-$(CH_2)_2-O-(CH_2)_2-O-(CH_2)_3$-, ##-$(CH_2)_2-(O(CH_2)_2)_2-(O(CH_2)_3)_2$-, ##-$(CH_2)_2-(O(CH_2)_2)_3-(O(CH_2)_3)_3$-, ##-$(CH_2)_2-(O(CH_2)_2)_4-(O(CH_2)_3)_4$-, ##-$(CH_2)_2-(O(CH_2)_2)_5-(O(CH_2)_3)_5$-, ##-$(CH_2)_2-(O(CH_2)_2)_6-(O(CH_2)_3)_6$-, ##-$(CH_2)_3-O-(CH_2)_2-O-(CH_2)_3$-, ##-$(CH_2)_3-(O(CH_2)_2)_2-(O(CH_2)_3)_2$-, ##-$(CH_2)_3-(O(CH_2)_2)_3-(O(CH_2)_3)_3$-, ##-$(CH_2)_3-(O(CH_2)_2)_4-(O(CH_2)_3)_4$-, ##-$(CH_2)_3-(O(CH_2)_2)_5-(O(CH_2)_3)_5$-, ##-$(CH_2)_3-(O(CH_2)_2)_6-(O(CH_2)_3)_6$-, ##-$CH_2-O-(CH_2)_3-O-(CH_2)_2$-, ##-$CH_2-(O(CH_2)_3)_2-(O(CH_2)_2)_2$-, ##-$CH_2-(O(CH_2)_3)_3-(O(CH_2)_2)_3$-, ##-$CH_2-(O(CH_2)_3)_4-(O(CH_2)_2)_4$-, ##-$CH_2-(O(CH_2)_3)_5-(O(CH_2)_2)_5$-, ##-$CH_2-(O(CH_2)_3)_6-(O(CH_2)_2)_6$-, ##-$(CH_2)_2-O-(CH_2)_3-O-(CH_2)_2$-, ##-$(CH_2)_2-(O(CH_2)_3)_2-(O(CH_2)_2)_2$-, ##-$(CH_2)_2-(O(CH_2)_3)_3-(O(CH_2)_2)_3$-, ##-$(CH_2)_2-(O(CH_2)_3)_4-(O(CH_2)_2)_4$-, ##-$(CH_2)_2-(O(CH_2)_3)_5-(O(CH_2)_2)_5$-, ##-$(CH_2)_2-(O(CH_2)_3)_6-(O(CH_2)_2)_6$-, ##-$(CH_2)_3-O-(CH_2)_3-O-(CH_2)_2$-, ##-$(CH_2)_3-(O(CH_2)_3)_2-(O(CH_2)_2)_2$-, ##-$(CH_2)_3-(O(CH_2)_3)_3-(O(CH_2)_2)_3$-, ##-$(CH_2)_3-(O(CH_2)_3)_4-(O(CH_2)_2)_4$-, ##-$(CH_2)_3-(O(CH_2)_3)_5-(O(CH_2)_2)_5$-, ##-$(CH_2)_3-(O(CH_2)_3)_6-(O(CH_2)_2)_6$-, ##-$CH_2-O-(CH_2)_2-O-CH_2$-, ##-$(CH_2)_2-O-(CH_2)_2-O-CH_2$-, ##-$(CH_2)_2-(O(CH_2)_2)_2-O-(CH_2)_3$-, ##-$(CH_2)_2-(O(CH_2)_2)_3-O-(CH_2)_3$-, ##-$(CH_2)_2-(O(CH_2)_2)_4-O-(CH_2)_3$-, ##-$(CH_2)_5-(O(CH_2)_2)_2-O-(CH_2)_5$-, ##-$(CH_2)_5-(O(CH_2)_2)_2-O-(CH_2)_6$-, ##-$CH_2-C(O)-CH_2$-, ##-$CH_2-C(O)-(CH_2)_2$-, ##-$(CH_2)_1-C(O)-(CH_2)_3$-, ##-$(CH_2)_1-C(O)-(CH_2)_4$-, ##-$(CH_2)_1-C(O)-(CH_2)_5$-, ##-$(CH_2)_1-C(O)-(CH_2)_6$-, ##-$(CH_2)_1-C(O)-(CH_2)_7$-, ##-$(CH_2)_1-C(O)-(CH_2)_8$-, ##-$(CH_2)_1-C(O)-(CH_2)_9$-, ##-$(CH_2)_1-C(O)-(CH_2)_{10}$-, ##-$(CH_2)_2-C(O)-(CH_2)_1$-, ##-$(CH_2)_2-C(O)-(CH_2)_2$-, ##-$(CH_2)_2-C(O)-(CH_2)_3$-, ##-$(CH_2)_2-C(O)-(CH_2)_4$-, ##-$(CH_2)_2-C(O)-(CH_2)_5$-, ##-$(CH_2)_2-C(O)-(CH_2)_6$-, ##-$(CH_2)_2-C(O)-(CH_2)_7$-, ##-$(CH_2)_2-C(O)-(CH_2)_8$-, ##-$(CH_2)_2-C(O)-(CH_2)_9$-, ##-$(CH_2)_2-C(O)-(CH_2)_{10}$-, ##-$(CH_Z)_2-C(O)-(CH_Z)_{11}$-, ##-$(CH_2)_2-C(O)-(CH_2)_{12}$-, ##-$(CH_2)_3-C(O)-(CH_2)_1$-, ##-$(CH_2)_3-C(O)-(CH_2)_2$-, ##-$(CH_2)_3-C(O)-(CH_2)_3$-, ##-$(CH_2)_3-C(O)-(CH_2)_4$-, ##-$(CH_2)_3-C(O)-(CH_2)_5$-, ##-$(CH_2)_3-C(O)-(CH_2)_6$-, ##-$(CH_2)_3-C(O)-(CH_2)_7$-, ##-$(CH_2)_4-C(O)-(CH_2)_1$-, ##-$(CH_2)_4-C(O)-(CH_2)_2$-, ##-$(CH_2)_4-C(O)-(CH_2)_3$-, ##-$(CH_2)_4-C(O)-(CH_2)_4$-, ##-$(CH_2)_4-C(O)-(CH_2)_5$-, ##-$(CH_2)_4-C(O)-(CH_2)_6$-, ##-$(CH_2)_5-C(O)-(CH_2)_1$-, ##-$(CH_2)_5-C(O)-(CH_2)_2$-, ##-$(CH_2)_5-C(O)-(CH_2)_3$-, ##-$(CH_2)_5-C(O)-(CH_2)_4$-, ##-$(CH_2)_5-C(O)-(CH_2)_5$-, ##-$(CH_2)_6-C(O)-(CH_2)_1$-, ##-$(CH_2)_6-C(O)-(CH_2)_2$-, ##-$(CH_2)_6-C(O)-(CH_2)_3$-, ##-$(CH_2)_6-C(O)-(CH_2)_4$-, ##-$(CH_2)_7-C(O)-(CH_2)_1$-, ##-$(CH_2)_7-C(O)-(CH_2)_2$-, ##-$(CH_2)_7-C(O)-(CH_2)_3$-, ##-$(CH_2)_8-C(O)-(CH_2)_1$-, ##-$(CH_2)_8-C(O)-(CH_2)_2$-, ##-$CH_2-S-CH_2$-, ##-$CH_2-S-(CH_2)_2$-, ##-$(CH_2)_1-S-(CH_2)_3$-, ##-$(CH_2)_1-S-(CH_2)_4$-, ##-$(CH_2)_1-S-(CH_2)_5$-, ##-$(CH_2)_1-S-(CH_2)_6$-, ##-$(CH_2)_1-S-(CH_2)_7$-, ##-$(CH_2)_1-S-(CH_2)_8$-, ##-$(CH_2)_1-S-(CH_2)_9$-, ##-$(CH_2)_1-S-(CH_2)_{10}$-, ##-$(CH_2)_2-S-(CH_2)_1$-, ##-$(CH_2)_2-S-(CH_2)_2$-, ##-$(CH_2)_2-S-(CH_2)_3$-, ##-$(CH_2)_2-S-(CH_2)_4$-, ##-$(CH_2)_2-S-(CH_2)_5$-, ##-$(CH_2)_2-S-(CH_2)_6$-, ##-$(CH_2)_2-S-(CH_2)_7$-, ##-$(CH_2)_2-S-(CH_2)_8$-, ##-$(CH_2)_2-S-(CH_2)_9$-, ##-$(CH_2)_2-S-(CH_2)_{10}$-, ##-$(CH_2)_2-S-(CH_2)_{11}$-, ##-$(CH_2)_2-S-(CH_2)_{12}$-, ##-$(CH_2)_3-S-(CH_2)_1$-, ##-$(CH_2)_3-S-(CH_2)_2$-, ##-$(CH_2)_3-S-(CH_2)_3$-, ##-$(CH_2)_3-S-(CH_2)_4$-, ##-$(CH_2)_3-S-(CH_2)_5$-, ##-$(CH_2)_3-S-(CH_2)_6$-, ##-$(CH_2)_3-S-(CH_2)_7$-, ##-$(CH_2)_4-S-(CH_2)_1$-, ##-$(CH_2)_4-S-(CH_2)_2$-, ##-$(CH_2)_4-S-(CH_2)_3$-, ##-$(CH_2)_4-S-(CH_2)_4$-, ##-$(CH_2)_4-S-(CH_2)_5$-, ##-$(CH_2)_4-S-(CH_2)_6$-, ##-$(CH_2)_5-S-(CH_2)_1$-, ##-$(CH_2)_5-S-(CH_2)_2$-, ##-$(CH_2)_5-S-(CH_2)_3$-, ##-$(CH_2)_5-S-(CH_2)_4$-, ##-$(CH_2)_5-S-(CH_2)_5$-, ##-$(CH_2)_6-S-(CH_2)_1$-, ##-$(CH_2)_6-S-(CH_2)_2$-, ##-$(CH_2)_6-S-(CH_2)_3$-, ##-$(CH_2)_6-S-(CH_2)_4$-, ##-$(CH_2)_7-S-(CH_2)_1$-, ##-$(CH_2)_7-S-(CH_2)_2$-, ##-$(CH_2)_7-S-(CH_2)_3$-, ##-$(CH_2)_8-S-(CH_2)_1$-, ##-$(CH_2)_8-S-(CH_2)_2$-, ##-$CH_2-C(S)-CH_2$-, ##-$CH_2-C(S)-(CH_2)_2$-, ##-$(CH_2)_1-C(S)-(CH_2)_3$-, ##-$(CH_2)_1-C(S)-(CH_2)_4$-, ##-$(CH_2)_1-C(S)-(CH_2)_5$-, ##-$(CH_2)_1-C(S)-(CH_2)_6$-, ##-$(CH_2)_1-C(S)-(CH_2)_7$-, ##-$(CH_Z)_1-C(S)-(CH_Z)_s$-, ##-$(CH_2)_1-C(S)-(CH_2)_9$-, ##-$(CH_2)_1-C(S)-(CH_2)_{10}$-, ##-$(CH_2)_2-C(S)-(CH_2)_1$-,

##-$(CH_2)_2$-C(S)-$(CH_2)_2$-, ##-$(CH_2)_2$-C(S)-$(CH_2)_3$-, ##-$(CH_2)_2$-C(S)-$(CH_2)_4$-, ##-$(CH_2)_2$-C(S)-$(CH_2)_5$-, ##-$(CH_2)_2$-C(S)-$(CH_2)_6$-, ##-$(CH_2)_2$-C(S)-$(CH_2)_7$-, ##-$(CH_2)_2$-C(S)-$(CH_2)_8$-, ##-$(CH_2)_2$-C(S)-$(CH_2)_9$-, ##-$(CH_2)_2$-C(S)-$(CH_2)_{10}$-, ##-$(CH_2)_2$-C(S)-$(CH_2)_{11}$-, ##-$(CH_2)_2$-C(S)-$(CH_2)_{12}$-, ##-$(CH_2)_3$-C(S)-$(CH_2)_1$-, ##-$(CH_2)_3$-C(S)-$(CH_2)_2$-, ##-$(CH_2)_3$-C(S)-$(CH_2)_3$-, ##-$(CH_2)_3$-C(S)-$(CH_2)_4$-, ##-$(CH_2)_3$-C(S)-$(CH_2)_5$-, ##-$(CH_2)_3$-C(S)-$(CH_2)_6$-, ##-$(CH_2)_3$-C(S)-$(CH_2)_7$-, ##-$(CH_2)_4$-C(S)-$(CH_2)_1$-, ##-$(CH_2)_4$-C(S)-$(CH_2)_2$-, ##-$(CH_2)_4$-C(S)-$(CH_2)_3$-, ##-$(CH_2)_4$-C(S)-$(CH_2)_4$-, ##-$(CH_2)_4$-C(S)-$(CH_2)_5$-, ##-$(CH_2)_4$-C(S)-$(CH_2)_6$-, ##-$(CH_2)_5$-C(S)-$(CH_2)_1$-, ##-$(CH_2)_5$-C(S)-$(CH_2)_2$-, ##-$(CH_2)_5$-C(S)-$(CH_2)_3$-, ##-$(CH_2)_5$-C(S)-$(CH_2)_4$-, ##-$(CH_2)_5$-C(S)-$(CH_2)_5$-, ##-$(CH_2)_6$-C(S)-$(CH_2)_1$-, ##-$(CH_2)_6$-C(S)-$(CH_2)_2$-, ##-$(CH_2)_6$-C(S)-$(CH_2)_3$-, ##-$(CH_2)_6$-C(S)-$(CH_2)_4$-, ##-$(CH_2)_7$-C(S)-$(CH_2)_1$-, ##-$(CH_2)_7$-C(S)-$(CH_2)_2$-, ##-$(CH_2)_7$-C(S)-$(CH_2)_3$-, ##-$(CH_2)_8$-C(S)-$(CH_2)_1$-, ##-$(CH_2)_8$-C(S)-$(CH_2)_2$-, ##-$CH_2$-C(O)O-$CH_2$-, ##-$CH_2$-C(O)O-$(CH_2)_2$-, ##-$(CH_2)_1$-C(O)O-$(CH_2)_3$-, ##-$(CH_2)_1$-C(O)O-$(CH_2)_4$-, ##-$(CH_2)_1$-C(O)O-$(CH_2)_5$-, ##-$(CH_2)_1$-C(O)O-$(CH_2)_6$-, ##-$(CH_2)_1$-C(O)O-$(CH_2)_7$-, ##-$(CH_2)_1$-C(O)O-$(CH_2)_8$-, ##-$(CH_2)_1$-C(O)O-$(CH_2)_9$-, ##-$(CH_2)_1$-C(O)O-$(CH_2)_{10}$-, ##-$(CH_2)_2$-C(O)O-$(CH_2)_1$-, ##-$(CH_2)_2$-C(O)O-$(CH_2)_2$-, ##-$(CH_2)_2$-C(O)O-$(CH_2)_3$-, ##-$(CH_2)_2$-C(O)O-$(CH_2)_4$-, ##-$(CH_2)_2$-C(O)O-$(CH_2)_5$-, ##-$(CH_2)_2$-C(O)O-$(CH_2)_6$-, ##-$(CH_2)_2$-C(O)O-$(CH_2)_7$-, ##-$(CH_2)_2$-C(O)O-$(CH_2)_8$-, ##-$(CH_2)_2$-C(O)O-$(CH_2)_9$-, ##-$(CH_2)_2$-C(O)O-$(CH_2)_{10}$-, ##-$(CH_2)_2$-C(O)O-$(CH_2)_{11}$-, ##-$(CH_2)_2$-C(O)O-$(CH_2)_{12}$-, ##-$(CH_2)_3$-C(O)O-$(CH_2)_1$-, ##-$(CH_2)_3$-C(O)O-$(CH_2)_2$-, ##-$(CH_2)_3$-C(O)O-$(CH_2)_3$-, ##-$(CH_2)_3$-C(O)O-$(CH_2)_4$-, ##-$(CH_2)_3$-C(O)O-$(CH_2)_5$-, ##-$(CH_2)_3$-C(O)O-$(CH_2)_6$-, ##-$(CH_2)_3$-C(O)O-$(CH_2)_7$-, ##-$(CH_2)_4$-C(O)O-$(CH_2)_1$-, ##-$(CH_2)_4$-C(O)O-$(CH_2)_2$-, ##-$(CH_2)_4$-C(O)O-$(CH_2)_3$-, ##-$(CH_2)_4$-C(O)O-$(CH_2)_4$-, ##-$(CH_2)_4$-C(O)O-$(CH_2)_5$-, ##-$(CH_2)_4$-C(O)O-$(CH_2)_6$-, ##-$(CH_2)_5$-C(O)O-$(CH_2)_1$-, ##-$(CH_2)_5$-C(O)O-$(CH_2)_2$-, ##-$(CH_2)_5$-C(O)O-$(CH_2)_3$-, ##-$(CH_2)_5$-C(O)O-$(CH_2)_4$-, ##-$(CH_2)_5$-C(O)O-$(CH_2)_5$-, ##-$(CH_2)_6$-C(O)O-$(CH_2)_1$-, ##-$(CH_2)_6$-C(O)O-$(CH_2)_2$-, ##-$(CH_2)_6$-C(O)O-$(CH_2)_3$-, ##-$(CH_2)_6$-C(O)O-$(CH_2)_4$-, ##-$(CH_2)_7$-C(O)O-$(CH_2)_1$-, ##-$(CH_2)_7$-C(O)O-$(CH_2)_2$-, ##-$(CH_2)_7$-C(O)O-$(CH_2)_3$-, ##-$(CH_2)_8$-C(O)O-$(CH_2)_1$-, ##-$(CH_2)_8$-C(O)O-$(CH_2)_2$-, ##-$CH_2$-OC(O)-$CH_2$-, ##-$CH_2$-OC(O)-$(CH_2)_2$-, ##-$(CH_2)_1$-OC(O)-$(CH_2)_3$-, ##-$(CH_2)_1$-OC(O)-$(CH_2)_4$-, ##-$(CH_2)_1$-OC(O)-$(CH_2)_5$-, ##-$(CH_2)_1$-OC(O)-$(CH_2)_6$-, ##-$(CH_2)_1$-OC(O)-$(CH_2)_7$-, ##-$(CH_2)_1$-OC(O)-$(CH_2)_8$-, ##-$(CH_2)_1$-OC(O)-$(CH_2)_9$-, ##-$(CH_2)_1$-OC(O)-$(CH_2)_{10}$-, ##-$(CH_2)_2$-OC(O)-$(CH_2)_1$-, ##-$(CH_2)_2$-OC(O)-$(CH_2)_2$-, ##-$(CH_2)_2$-OC(O)-$(CH_2)_3$-, ##-$(CH_2)_2$-OC(O)-$(CH_2)_4$-, ##-$(CH_2)_2$-OC(O)-$(CH_2)_5$-, ##-$(CH_2)_2$-OC(O)-$(CH_2)_6$-, ##-$(CH_2)_2$-OC(O)-$(CH_2)_7$-, ##-$(CH_2)_2$-OC(O)-$(CH_2)_8$-, ##-$(CH_2)_2$-OC(O)-$(CH_2)_9$-, ##-$(CH_2)_2$-OC(O)-$(CH_2)_{10}$-, ##-$(CH_2)_2$-OC(O)-$(CH_2)_{11}$-, ##-$(CH_2)_2$-OC(O)-$(CH_2)_{12}$-, ##-$(CH_2)_3$-OC(O)-$(CH_2)_1$-, ##-$(CH_2)_3$-OC(O)-$(CH_2)_2$-, ##-$(CH_2)_3$-OC(O)-$(CH_2)_3$-, ##-$(CH_2)_3$-OC(O)-$(CH_2)_4$-, ##-$(CH_2)_3$-OC(O)-$(CH_2)_5$-, ##-$(CH_2)_3$-OC(O)-$(CH_2)_6$-, ##-$(CH_2)_3$-OC(O)-$(CH_2)_7$-, ##-$(CH_2)_4$-OC(O)-$(CH_2)_1$-, ##-$(CH_2)_4$-OC(O)-$(CH_2)_2$-, ##-$(CH_2)_4$-OC(O)-$(CH_2)_3$-, ##-$(CH_2)_4$-OC(O)-$(CH_2)_4$-, ##-$(CH_2)_4$-OC(O)-$(CH_2)_5$-, ##-$(CH_2)_4$-OC(O)-$(CH_2)_6$-, ##-$(CH_2)_5$-OC(O)-$(CH_2)_1$-, ##-$(CH_2)_5$-OC(O)-$(CH_2)_2$-, ##-$(CH_2)_5$-OC(O)-$(CH_2)_3$-, ##-$(CH_2)_5$-OC(O)-$(CH_2)_4$-, ##-$(CH_2)_5$-OC(O)-$(CH_2)_5$-, ##-$(CH_2)_6$-OC(O)-$(CH_2)_1$-, ##-$(CH_2)_6$-OC(O)-$(CH_2)_2$-, ##-$(CH_2)_6$-OC(O)-$(CH_2)_3$-, ##-$(CH_2)_6$-OC(O)-$(CH_2)_4$-, ##-$(CH_2)_7$-OC(O)-$(CH_2)_1$-, ##-$(CH_2)_7$-OC(O)-$(CH_2)_2$-, ##-$(CH_2)_7$-OC(O)-$(CH_2)_3$-, ##-$(CH_2)_8$-OC(O)-$(CH_2)_1$-, ##-$(CH_2)_8$-OC(O)-$(CH_2)_2$-, ##-$(CH_2)_1$-N($R_{g10}$)-$(CH_2)_1$-, ##-$(CH_2)_1$-N($R_{g10}$)-$(CH_2)_2$-, ##-$(CH_2)_1$-N($R_{g10}$)-$(CH_2)_3$-, ##-$(CH_2)_1$-N($R_{g10}$)-$(CH_2)_4$-, ##-$(CH_2)_1$-N($R_{g10}$)-$(CH_2)_5$-, ##-$(CH_2)_1$-N($R_{g10}$)-$(CH_2)_6$-, ##-$(CH_2)_1$-N($R_{g10}$)-$(CH_2)_7$-, ##-$(CH_2)_1$-N($R_{g10}$)-$(CH_2)_8$-, ##-$(CH_2)_1$-N($R_{g10}$)-$(CH_2)_9$-, ##-$(CH_2)_1$-N($R_{g10}$)-$(CH_2)_{10}$-, ##-$(CH_2)_2$-N($R_{g10}$)-$(CH_2)_1$-, ##-$(CH_2)_2$-N($R_{g10}$)-$(CH_2)_2$-, ##-$(CH_2)_2$-N($R_{g10}$)-$(CH_2)_3$-, ##-$(CH_2)_2$-N($R_{g10}$)-$(CH_2)_4$-, ##-$(CH_2)_2$-N($R_{g10}$)-$(CH_2)_5$-, ##-$(CH_2)_2$-N($R_{g10}$)-$(CH_2)_6$-, ##-$(CH_2)_2$-N($R_{g10}$)-$(CH_2)_7$-, ##-$(CH_2)_2$-N($R_{g10}$)-$(CH_2)_8$-, ##-$(CH_2)_2$-N($R_{g10}$)-$(CH_2)_9$-, ##-$(CH_2)_2$-N($R_{g10}$)-$(CH_2)_{10}$-, ##-$(CH_2)_2$-N($R_{g10}$)-$(CH_2)_{11}$-, ##-$(CH_2)_2$-N($R_{g10}$)-$(CH_2)_{12}$-, ##-$(CH_2)_3$-N($R_{g10}$)-$(CH_2)_i$-, ##-$(CH_2)_3$-N($R_{g10}$)-$(CH_2)_2$-, ##-$(CH_2)_3$-N($R_{g10}$)-$(CH_2)_3$-, ##-$(CH_2)_4$-N($R_{g10}$)-$(CH_2)_1$-, ##-$(CH_2)_4$-N($R_{g10}$)-$(CH_2)_2$-, ##-$(CH_2)_4$-N($R_{g10}$)-$(CH_2)_3$-, ##-$(CH_2)_4$-N($R_{g10}$)-$(CH_2)_4$-, ##-$(CH_2)_5$-N($R_{g10}$)-$(CH_2)_1$-, ##-$(CH_2)_5$-N($R_{g10}$)-$(CH_2)_2$-, ##-$(CH_2)_5$-N($R_{g10}$)-$(CH_2)_3$-, ##-$(CH_2)_5$-N($R_{g10}$)-$(CH_2)_4$-, ##-$(CH_2)_5$-N($R_{g10}$)-$(CH_2)_5$-, ##-$(CH_2)_6$-N($R_{g10}$)-$(CH_2)_1$-, ##-$(CH_2)_6$-N($R_{g10}$)-$(CH_2)_2$-, ##-$(CH_2)_6$-N($R_{g10}$)-$(CH_2)_3$-, ##-$(CH_2)_7$-N($R_{g10}$)-$(CH_2)_1$-, ##-$(CH_2)_7$-N($R_{g10}$)-$(CH_2)_2$-, ##-$(CH_2)_7$-N($R_{g10}$)-$(CH_2)_3$-, ##-$(CH_2)_8$-N($R_{g10}$)-$(CH_2)_1$-, ##-$(CH_2)_8$-N $R_{g10}$)-$(CH_2)_2$-, ##-$(CH_2)_8$-N($R_{g10}$)-$(CH_2)_3$-, ##-CH($CH_3$)-N($R_{g10}$)-$(CH_2)_1$-, ##-CH($CH_3$)-N($R_{g10}$)-$(CH_2)_2$-, ##-CH($CH_3$)-N($R_{g10}$)-$(CH_2)_3$-, ##-CH($CH_3$)-N($R_{g10}$)-$(CH_2)_4$-, ##-CH($CH_3$)-N($R_{g10}$)-$(CH_2)_5$-, ##-CH($CH_3$)-N($R_{g10}$)-$(CH_2)_6$-, ##-CH($CH_3$)-N($R_{g10}$)-$(CH_2)_7$-, ##-CH($CH_3$)-N($R_{g10}$)-$(CH_2)_8$-, ##-CH($CH_3$)-N($R_{g10}$)-$(CH_2)_9$-, ##-CH($CH_3$)-N($R_{g10}$)-$(CH_2)_{10}$-, ##-$CH_2$C(O)NH$CH_2$-, ##-$(CH_2)_2$C(O)NH$(CH_2)_2$-, ##-$(CH_2)_2$C(O)NH$(CH_2)_3$-, ##-$(CH_2)_2$C(O)NH$(CH_2)_4$-, ##-$(CH_2)_2$C(O)NH$(CH_2)_5$-, ##-$(CH_2)_3$C(O)NH$(CH_2)_3$-, ##-$(CH_2)_3$C(O)NH$(CH_2)_4$-, ##-$(CH_2)_4$C(O)NH$(CH_2)_4$-, ##-$(CH_2)_5$C(O)NH$(CH_2)_5$-, ##-$(CH_2)_6$C(O)NH$(CH_2)_7$-, ##-$(CH_2)_6$C(O)NH$(CH_2)_6$-, ##-$(CH_2)_7$C(O)NH$(CH_2)_7$-, ##-$(CH_2)_8$C(O)NH$(CH_2)_8$, ##-$(CH_2)_9$C(O)NH$(CH_2)_9$-, ##-$(CH_2)_{10}$C(O)NH$(CH_2)_{10}$-, ##-$(CH_2)_2$C(O)NH$(CH_2)_2$-O-$(CH_2)_2$-, ##-$CH_2$NHC(O)$CH_2$-, ##-$(CH_2)_2$NHC(O)$(CH_2)_2$-, ##-$(CH_2)_2$NHC(O)$(CH_2)_3$-, ##-$(CH_2)_2$NHC(O)$(CH_2)_4$-, ##-$(CH_2)_2$NHC(O)$(CH_2)_5$-, ##-$(CH_2)_3$NHC(O)$(CH_2)_3$-, ##-$(CH_2)_3$NHC(O)$(CH_2)_4$-, ##-$(CH_2)_4$NHC(O)$(CH_2)_4$-, ##-$(CH_2)_5$NHC(O)$(CH_2)_5$-, ##-$(CH_2)_6$NHC(O)$(CH_2)_7$-, ##-$(CH_2)_6$NHC(O)$(CH_2)_6$-, ##-$(CH_2)_7$NHC(O)$(CH_2)_7$-, ##-$(CH_2)_8$NHC(O)$(CH_2)_8$, ##-$(CH_2)_9$NHC(O)$(CH_2)_9$-, ##-$(CH_2)_{10}$NHC(O)$(CH_2)_{10}$-, ##-$(CH_2)_4$NHC(O)$(CH_2)_8$-, ##-$(CH_2)_2$NHC(O)$(CH_2)_2$-O-$(CH_2)_2$-, ##-$(CH_2)_4$NHC(O)$CH_2$-, ##-$CH_2$-piperidinylene-$CH_2$-, ##-$CH_2$-piperidinylene-$(CH_2)_2$-, ##-$CH_2$-piperidinylene-$(CH_2)_3$-, ##-$CH_2$-piperidinylene-$(CH_2)_4$-, ##-$CH_2$-piperidinylene-$(CH_2)_5$-, ##-$CH_2$-piperidinylene-$(CH_2)_6$-, ##-$CH_2$-piperidinylene-$(CH_2)_7$-, ##-$CH_2$-

piperidinylene-$(CH_2)_8$-, ##-$(CH_2)_2$-piperidinylene-$(CH_2)_1$-, ##-$(CH_2)_2$-piperidinylene-$(CH_2)_2$-, ##-$(CH_2)_2$-piperidinylene-$(CH_2)_3$-, ##-$(CH_2)_2$-piperidinylene-$(CH_2)_4$-, ##-$(CH_2)_2$-piperidinylene-$(CH_2)_5$-, ##-$(CH_2)_2$-piperidinylene-$(CH_2)_6$-, ##-$(CH_2)_2$-piperidinylene-$(CH_2)_7$-, ##-$(CH_2)_2$-piperidinylene-$(CH_2)_8$-, ##-$(CH_2)_3$-piperidinylene-$CH_2$-, ##-$(CH_2)_3$-piperidinylene-$(CH_2)_2$-, ##-$(CH_2)_3$-piperidinylene-$(CH_2)_3$-, ##-$(CH_2)_3$-piperidinylene-$(CH_2)_4$-, ##-$(CH_2)_3$-piperidinylene-$(CH_2)_5$-, ##-$(CH_2)_3$-piperidinylene-$(CH_2)_6$-, ##-$(CH_2)_3$-piperidinylene-$(CH_2)_7$-, ##-$(CH_2)_3$-piperidinylene-$(CH_2)_8$-, ##-$(CH_2)_4$-piperidinylene-$CH_2$-, ##-$(CH_2)_4$-piperidinylene-$(CH_2)_2$-, ##-$(CH_2)_4$-piperidinylene-$(CH_2)_3$-, ##-$(CH_2)_4$-piperidinylene-$(CH_2)_4$-, ##-$(CH_2)_4$-piperidinylene-$(CH_2)_5$-, ##-$(CH_2)_4$-piperidinylene-$(CH_2)_6$-, ##-$(CH_2)_4$-piperidinylene-$(CH_2)_7$-, ##-$(CH_2)_4$-piperidinylene-$(CH_2)_8$-, ##-$(CH_2)_5$-piperidinylene-$(CH_2)_1$-, ##-$(CH_2)_5$-piperidinylene-$(CH_2)_2$-, ##-$(CH_2)_5$-piperidinylene-$(CH_2)_3$-, ##-$(CH_2)_5$-piperidinylene-$(CH_2)_4$-, ##-$(CH_2)_5$-piperidinylene-$(CH_2)_5$-, ##-$(CH_2)_5$-piperidinylene-$(CH_2)_6$-, ##-$(CH_2)_5$-piperidinylene-$(CH_2)_7$-, ##-$(CH_2)_5$-piperidinylene-$(CH_2)_8$-, ##-$(CH_2)_6$-piperidinylene-$(CH_2)_1$-, ##-$(CH_2)_6$-pipendinylene-$(CH_2)_2$-, ##-$(CH_2)_6$-piperidinylene-$(CH_2)_3$-, ##-$(CH_2)_6$-piperidinylene-$(CH_2)_4$-, ##-$(CH_2)_6$-piperidinylene-$(CH_2)_5$-, ##-$(CH_2)_6$-piperidinylene-$(CH_2)_6$-, ##-$(CH_2)_6$-piperidinylene-$(CH_2)_7$-, ##-$(CH_2)_6$-piperidinylene-$(CH_2)_8$-, ##-$(CH_2)_7$-piperidinylene-$(CH_2)_1$-, ##-$(CH_2)_7$-piperidinylene-$(CH_2)_2$-, ##-$(CH_2)_7$-piperidinylene-$(CH_2)_3$-, ##-$(CH_2)_7$-piperidinylene-$(CH_2)_4$-, ##-$(CH_2)_7$-piperidinylene-$(CH_2)_8$-, ##-$(CH_2)_8$-piperidinylene-$CH_2$-, ##-$(CH_2)_8$-piperidinylene-$(CH_2)_2$-, ##-$(CH_2)_8$-piperidinylene-$(CH_2)_3$-, ##-$(CH_2)_8$-piperidinylene-$(CH_2)_4$-, ##-$(CH_2)_8$-piperidinylene-$(CH_2)_5$-, ##-$(CH_2)_8$-piperidinylene-$(CH_2)_6$-, ##-$(CH_2)_8$-piperidinylene-$(CH_2)_7$-, ##-$(CH_2)_8$-piperidinylene-$(CH_2)_8$-, ##-$CH_2$-piperazinylene-$CH_2$-, ##-$CH_2$-piperazinylene-$(CH_2)_2$-, ##-$CH_2$-piperazinylene-$(CH_2)_3$-, ##-$CH_2$-piperazinylene-$(CH_2)_4$-, ##-$CH_2$-piperazinylene-$(CH_2)_5$-, ##-$CH_2$-piperazinylene-$(CH_2)_6$-, ##-$CH_2$-piperazinylene-$(CH_2)_7$-, ##-$CH_2$-piperazinylene-$(CH_2)_8$-, ##-$(CH_2)_2$-piperazinylene-$(CH_2)_1$-, ##-$(CH_2)_2$-piperazinylene-$(CH_2)_2$-, ##-$(CH_2)_2$-piperazinylene-$(CH_2)_3$-, ##-$(CH_2)_2$-piperazinylene-$(CH_2)_4$-, ##-$(CH_2)_2$-piperazinylene-$(CH_2)_5$-, ##-$(CH_2)_2$-piperazinylene-$(CH_2)_6$-, ##-$(CH_2)_2$-piperazinylene-$(CH_2)_7$-, ##-$(CH_2)_2$-piperazinylene-$(CH_2)_8$-, ##-$(CH_2)_3$-piperazinylene-$CH_2$-, ##-$(CH_2)_3$-piperazinylene-$(CH_2)_2$-, ##-$(CH_2)_3$-piperazinylene-$(CH_2)_3$-, ##-$(CH_2)_3$-piperazinylene-$(CH_2)_4$-, ##-$(CH_2)_3$-piperazinylene-$(CH_2)_5$-, ##-$(CH_2)_3$-piperazinylene-$(CH_2)_6$-, ##-$(CH_2)_3$-piperazinylene-$(CH_2)_7$-, ##-$(CH_2)_3$-piperazinylene-$(CH_2)_8$-, ##-$(CH_2)_4$-piperazinylene-$CH_2$-, ##-$(CH_2)_4$-piperazinylene-$(CH_2)_2$-, ##-$(CH_2)_4$-piperazinylene-$(CH_2)_3$-, ##-$(CH_2)_4$-piperazinylene-$(CH_2)_4$-, ##-$(CH_2)_4$-piperazinylene-$(CH_2)_5$-, ##-$(CH_2)_4$-piperazinylene-$(CH_2)_6$-, ##-$(CH_2)_4$-piperazinylene-$(CH_2)_7$-, ##-$(CH_2)_4$-piperazinylene-$(CH_2)_8$-, ##-$(CH_2)_5$-piperazinylene-$(CH_2)_1$-, ##-$(CH_2)_5$-piperazinylene-$(CH_2)_2$-, ##-$(CH_2)_5$-piperazinylene-$(CH_2)_3$-, ##-$(CH_2)_5$-piperazinylene-$(CH_2)_4$-, ##-$(CH_2)_5$-piperazinylene-$(CH_2)_5$-, ##-$(CH_2)_5$-piperazinylene-$(CH_2)_6$-, ##-$(CH_2)_5$-piperazinylene-$(CH_2)_7$-, ##-$(CH_2)_5$-piperazinylene-$(CH_2)_8$-, ##-$(CH_2)_6$-piperazinylene-$(CH_2)_1$-, ##-$(CH_2)_6$-piperazinylene-$(CH_2)_2$-, ##-$(CH_2)_6$-piperazinylene-$(CH_2)_3$-, ##-$(CH_2)_6$-piperazinylene-$(CH_2)_4$-, ##-$(CH_2)_6$-piperazinylene-$(CH_2)_5$-, ##-$(CH_2)_6$-piperazinylene-$(CH_2)_6$-, ##-$(CH_2)_6$-piperazinylene-$(CH_2)_7$-, ##-$(CH_2)_6$-piperazinylene-$(CH_2)_8$-, ##-$(CH_2)_7$-piperazinylene-$(CH_2)_1$-, ##-$(CH_2)_7$-piperazinylene-$(CH_2)_2$-, ##-$(CH_2)_7$-piperazinylene-$(CH_2)_3$-, ##-$(CH_2)_7$-piperazinylene-$(CH_2)_4$-, ##-$(CH_2)_7$-piperazinylene-$(CH_2)_8$-, ##-$(CH_2)_8$-piperazinylene-$CH_2$-, ##-$(CH_2)_8$-piperazinylene-$(CH_2)_2$-, ##-$(CH_2)_8$-piperazinylene-$(CH_2)_3$-, ##-$(CH_2)_8$-piperazinylene-$(CH_2)_4$-, ##-$(CH_2)_8$-piperazinylene-$(CH_2)_5$-, ##-$(CH_2)_8$-piperazinylene-$(CH_2)_6$-, ##-$(CH_2)_8$-piperazinylene-$(CH_2)_7$-, ##-$(CH_2)_8$-piperazinylene-$(CH_2)_8$-, ##-$CH_2$-propylene-$CH_2$-, ##-$CH_2$-propylene-$(CH_2)_2$-, ##-$CH_2$-propylene-$(CH_2)_3$-, ##-$CH_2$-propylene-$(CH_2)_4$-, ##-$CH_2$-propylene-$(CH_2)_5$-, ##-$CH_2$-propylene-$(CH_2)_6$-, ##-$CH_2$-propylene-$(CH_2)_7$-, ##-$CH_2$-propylene-$(CH_2)_8$-, ##-$(CH_2)_2$-propylene-$(CH_2)_1$-, ##-$(CH_2)_2$-propylene-$(CH_2)_2$-, ##-$(CH_2)_2$-propylene-$(CH_2)_3$-, ##-$(CH_2)_2$-propylene-$(CH_2)_4$-, ##-$(CH_2)_2$-propylene-$(CH_2)_5$-, ##-$(CH_2)_2$-propylene-$(CH_2)_6$-, ##-$(CH_2)_2$-propylene-$(CH_2)_7$-, ##-$(CH_2)_2$-propylene-$(CH_2)_8$-, ##-$(CH_2)_3$-propylene-$CH_2$-, ##-$(CH_2)_3$-propylene-$(CH_2)_2$-, ##-$(CH_2)_3$-propylene-$(CH_2)_3$-, ##-$(CH_2)_3$-propylene-$(CH_2)_4$-, ##-$(CH_2)_3$-propylene-$(CH_2)_5$-, ##-$(CH_2)_3$-propylene-$(CH_2)_6$-, ##-$(CH_2)_3$-propylene-$(CH_2)_7$-, ##-$(CH_2)_3$-propylene-$(CH_2)_8$-, ##-$(CH_2)_4$-propylene-$CH_2$-, ##-$(CH_2)_4$-propylene-$(CH_2)_2$-, ##-$(CH_2)_4$-propylene-$(CH_2)_3$-, ##-$(CH_2)_4$-propylene-$(CH_2)_4$-, ##-$(CH_2)_4$-propylene-$(CH_2)_5$-, ##-$(CH_2)_4$-propylene-$(CH_2)_6$-, ##-$(CH_2)_4$-propylene-$(CH_2)_7$-, ##-$(CH_2)_4$-propylene-$(CH_2)_8$-, ##-$(CH_2)_5$-propylene-$(CH_2)_1$-, ##-$(CH_2)_5$-propylene-$(CH_2)_2$-, ##-$(CH_2)_5$-propylene-$(CH_2)_3$-, ##-$(CH_2)_5$-propylene-$(CH_2)_4$-, ##-$(CH_2)_5$-propylene-$(CH_2)_5$-, ##-$(CH_2)_5$-propylene-$(CH_2)_6$-, ##-$(CH_2)_5$-propylene-$(CH_2)_7$-, ##-$(CH_2)_5$-propylene-$(CH_2)_8$-, ##-$(CH_2)_6$-propylene-$(CH_2)_1$-, ##-$(CH_2)_6$-propylene-$(CH_2)_2$-, ##-$(CH_2)_6$-propylene-$(CH_2)_3$-, ##-$(CH_2)_6$-propylene-$(CH_2)_4$-, ##-$(CH_2)_6$-propylene-$(CH_2)_5$-, ##-$(CH_2)_6$-propylene-$(CH_2)_6$-, ##-$(CH_2)_6$-propylene-$(CH_2)_7$-, ##-$(CH_2)_6$-propylene-$(CH_2)_8$-, ##-$(CH_2)_7$-propylene-$(CH_2)_1$-, ##-$(CH_2)_7$-propylene-$(CH_2)_2$-, ##-$(CH_2)_7$-propylene-$(CH_2)_3$-, ##-$(CH_2)_7$-propylene-$(CH_2)_4$-, ##-$(CH_2)_7$-propylene-$(CH_2)_8$-, ##-$(CH_2)_8$-propylene-$CH_2$-, ##-$(CH_2)_8$-propylene-$(CH_2)_2$-, ##-$(CH_2)_8$-propylene-$(CH_2)_3$-, ##-$(CH_2)_8$-propylene-$(CH_2)_4$-, ##-$(CH_2)_8$-propylene-$(CH_2)_5$-, ##-$(CH_2)_8$-propylene-$(CH_2)_6$-, ##-$(CH_2)_8$-propylene-$(CH_2)_7$-, ##-$(CH_2)_8$-propylene-$(CH_2)_8$-, ##-$CH_2$-diazacycloheptanylene-$CH_2$-, ##-$CH_2$-diazacycloheptanylene-$(CH_2)_2$-, ##-$CH_2$-diazacycloheptanylene-$(CH_2)_3$-, ##-$CH_2$-diazacycloheptanylene-$(CH_2)_4$-, ##-$CH_2$-diazacycloheptanylene-$(CH_2)_5$-, ##-$CH_2$-diazacycloheptanylene-$(CH_2)_6$-, ##-$CH_2$-diazacycloheptanylene-$(CH_2)_7$-, ##-$CH_2$-diazacycloheptanylene-$(CH_2)_8$-, ##-$(CH_2)_2$-diazacycloheptanylene-$(CH_2)_1$-, ##-$(CH_2)_2$-diazacycloheptanylene-$(CH_2)_2$-, ##-$(CH_2)_2$-diazacycloheptanylene-$(CH_2)_3$-, ##-$(CH_2)_2$-diazacycloheptanylene-$(CH_2)_4$-, ##-$(CH_2)_2$-diazacycloheptanylene-$(CH_2)_5$-, ##-$(CH_2)_2$-diazacycloheptanylene-$(CH_2)_6$-, ##-$(CH_2)_2$-diazacycloheptanylene-$(CH_2)_7$-, ##-$(CH_2)_2$-diazacycloheptanylene-$(CH_2)_8$-, ##-$(CH_2)_3$-diazacycloheptanylene-$CH_2$-, ##-$(CH_2)_3$-diazacycloheptanylene-$(CH_2)_2$-, ##-$(CH_2)_3$-diazacycloheptanylene-$(CH_2)_3$-, ##-$(CH_2)_3$-diazacycloheptanylene-$(CH_2)_4$-, ##-$(CH_2)_3$-diazacycloheptanylene-$(CH_2)_5$-,

##-$(CH_2)_3$-diazacycloheptanylene-$(CH_2)_6$-, ##-$(CH_2)_3$-diazacycloheptanylene-$(CH_2)_7$-, ##-$(CH_2)_3$-diazacycloheptanylene-$(CH_2)_8$-, ##-$(CH_2)_4$-diazacycloheptanylene-$CH_2$-, ##-$(CH_2)_4$-diazacycloheptanylene-$(CH_2)_2$-, ##-$(CH_2)_4$-diazacycloheptanylene-$(CH_2)_3$-, ##-$(CH_2)_4$-diazacycloheptanylene-$(CH_2)_4$-, ##-$(CH_2)_4$-diazacycloheptanylene-$(CH_2)_5$-, ##-$(CH_2)_4$-diazacycloheptanylene-$(CH_2)_6$-, ##-$(CH_2)_4$-diazacycloheptanylene-$(CH_2)_7$-, ##-$(CH_2)_4$-diazacycloheptanylene-$(CH_2)_8$-, ##-$(CH_2)_5$-diazacycloheptanylene-$(CH_2)_1$-, ##-$(CH_2)_5$-diazacycloheptanylene-$(CH_2)_2$-, ##-$(CH_2)_5$-diazacycloheptanylene-$(CH_2)_3$-, ##-$(CH_2)_5$-diazacycloheptanylene-$(CH_2)_4$-, ##-$(CH_2)_5$-diazacycloheptanylene-$(CH_2)_5$-, ##-$(CH_2)_5$-diazacycloheptanylene-$(CH_2)_6$-, ##-$(CH_2)_5$-diazacycloheptanylene-$(CH_2)_7$-, ##-$(CH_2)_5$-diazacycloheptanylene-$(CH_2)_8$-, ##-$(CH_2)_6$-diazacycloheptanylene-$(CH_2)_1$-, ##-$(CH_2)_6$-diazacycloheptanylene-$(CH_2)_2$-, ##-$(CH_2)_6$-diazacycloheptanylene-$(CH_2)_3$-, ##-$(CH_2)_6$-diazacycloheptanylene-$(CH_2)_4$-, ##-$(CH_2)_6$-diazacycloheptanylene-$(CH_2)_5$-, ##-$(CH_2)_6$-diazacycloheptanylene-$(CH_2)_6$-, ##-$(CH_2)_6$-diazacycloheptanylene-$(CH_2)_7$-, ##-$(CH_2)_6$-diazacycloheptanylene-$(CH_2)_8$-, ##-$(CH_2)_7$-diazacycloheptanylene-$(CH_2)_1$-, ##-$(CH_2)_7$-diazacycloheptanylene-$(CH_2)_2$-, ##-$(CH_2)_7$-diazacycloheptanylene-$(CH_2)_3$-, ##-$(CH_2)_7$-diazacycloheptanylene-$(CH_2)_4$-, ##-$(CH_2)_7$-diazacycloheptanylene-$(CH_2)_8$-, ##-$(CH_2)_8$-diazacycloheptanylene-$CH_2$-, ##-$(CH_2)_8$-diazacycloheptanylene-$(CH_2)_2$-, ##-$(CH_2)_8$-diazacycloheptanylene-$(CH_2)_3$-, ##-$(CH_2)_8$-diazacycloheptanylene-$(CH_2)_4$-, ##-$(CH_2)_8$-diazacycloheptanylene-$(CH_2)_5$-, ##-$(CH_2)_8$-diazacycloheptanylene-$(CH_2)_6$-, ##-$(CH_2)_8$-diazacycloheptanylene-$(CH_2)_7$-, ##-$(CH_2)_8$-diazacycloheptanylene-$(CH_2)_8$-, ##-$CH_2$-diazabicyclo[2.2.1]heptanylene-$CH_2$-, ##-$CH_2$-diazabicyclo[2.2.1]heptanylene-$(CH_2)_2$-, ##-$CH_2$-diazabicyclo[2.2.1]heptanylene-$(CH_2)_3$-, ##-$CH_2$-diazabicyclo[2.2.1]heptanylene-$(CH_2)_4$-, ##-$CH_2$-diazabicyclo[2.2.1]heptanylene-$(CH_2)_5$-, ##-$CH_2$-diazabicyclo[2.2.1]heptanylene-$(CH_2)_6$-, ##-$CH_2$-diazabicyclo[2.2.1]heptanylene-$(CH_2)_7$-, ##-$CH_2$-diazabicyclo[2.2.1]heptanylene-$(CH_2)_8$-, ##-$(CH_2)_2$-diazabicyclo[2.2.1]heptanylene-$(CH_2)_1$-, ##-$(CH_2)_2$-diazabicyclo[2.2.1]heptanylene-$(CH_2)_2$-, ##-$(CH_2)_2$-diazabicyclo[2.2.1]heptanylene-$(CH_2)_3$-, ##-$(CH_2)_2$-diazabicyclo[2.2.1]heptanylene-$(CH_2)_4$-, ##-$(CH_2)_2$-diazabicyclo[2.2.1]heptanylene-$(CH_2)_5$-, ##-$(CH_2)_2$-diazabicyclo[2.2.1]heptanylene-$(CH_2)_6$-, ##-$(CH_2)_2$-diazabicyclo[2.2.1]heptanylene-$(CH_2)_7$-, ##-$(CH_2)_2$-diazabicyclo[2.2.1]heptanylene-$(CH_2)_8$-, ##-$(CH_2)_3$-diazabicyclo[2.2.1]heptanylene-$CH_2$-, ##-$(CH_2)_3$-diazabicyclo[2.2.1]heptanylene-$(CH_2)_2$-, ##-$(CH_2)_3$-diazabicyclo[2.2.1]heptanylene-$(CH_2)_3$-, ##-$(CH_2)_3$-diazabicyclo[2.2.1]heptanylene-$(CH_2)_4$-, ##-$(CH_2)_3$-diazabicyclo[2.2.1]heptanylene-$(CH_2)_5$-, ##-$(CH_2)_3$-diazabicyclo[2.2.1]heptanylene-$(CH_2)_6$-, ##-$(CH_2)_3$-diazabicyclo[2.2.1]heptanylene-$(CH_2)_7$-, ##-$(CH_2)_3$-diazabicyclo[2.2.1]heptanylene-$(CH_2)_8$-, ##-$(CH_2)_4$-diazabicyclo[2.2.1]heptanylene-$CH_2$-, ##-$(CH_2)_4$-diazabicyclo[2.2.1]heptanylene-$(CH_2)_2$-, ##-$(CH_2)_4$-diazabicyclo[2.2.1]heptanylene-$(CH_2)_3$-, ##-$(CH_2)_4$-diazabicyclo[2.2.1]heptanylene-$(CH_2)_4$-, ##-$(CH_2)_4$-diazabicyclo[2.2.1]heptanylene-$(CH_2)_5$-, ##-$(CH_2)_4$-diazabicyclo[2.2.1]heptanylene-$(CH_2)_6$-, ##-$(CH_2)_4$-diazabicyclo[2.2.1]heptanylene-$(CH_2)_7$-, ##-$(CH_2)_4$-diazabicyclo[2.2.1]heptanylene-$(CH_2)_8$-, ##-$(CH_2)_5$-diazabicyclo[2.2.1]heptanylene-$(CH_2)_1$-, ##-$(CH_2)_5$-diazabicyclo[2.2.1]heptanylene-$(CH_2)_2$-, ##-$(CH_2)_5$-diazabicyclo[2.2.1]heptanylene-$(CH_2)_3$-, ##-$(CH_2)_5$-diazabicyclo[2.2.1]heptanylene-$(CH_2)_4$-, ##-$(CH_2)_5$-diazabicyclo[2.2.1]heptanylene-$(CH_2)_5$-, ##-$(CH_2)_5$-diazabicyclo[2.2.1]heptanylene-$(CH_2)_6$-, ##-$(CH_2)_5$-diazabicyclo[2.2.1]heptanylene-$(CH_2)_7$-, ##-$(CH_2)_5$-diazabicyclo[2.2.1]heptanylene-$(CH_2)_8$-, ##-$(CH_2)_6$-diazabicyclo[2.2.1]heptanylene-$(CH_2)_1$-, ##-$(CH_2)_6$-diazabicyclo[2.2.1]heptanylene-$(CH_2)_2$-, ##-$(CH_2)_6$-diazabicyclo[2.2.1]heptanylene-$(CH_2)_3$-, ##-$(CH_2)_6$-diazabicyclo[2.2.1]heptanylene-$(CH_2)_4$-, ##-$(CH_2)_6$-diazabicyclo[2.2.1]heptanylene-$(CH_2)_5$-, ##-$(CH_2)_6$-diazabicyclo[2.2.1]heptanylene-$(CH_2)_6$-, ##-$(CH_2)_6$-diazabicyclo[2.2.1]heptanylene-$(CH_2)_7$-, ##-$(CH_2)_6$-diazabicyclo[2.2.1]heptanylene-$(CH_2)_8$-, ##-$(CH_2)_7$-diazabicyclo[2.2.1]heptanylene-$(CH_2)_1$-, ##-$(CH_2)_7$-diazabicyclo[2.2.1]heptanylene-$(CH_2)_2$-, ##-$(CH_2)_7$-diazabicyclo[2.2.1]heptanylene-$(CH_2)_3$-, ##-$(CH_2)_7$-diazabicyclo[2.2.1]heptanylene-$(CH_2)_4$-, ##-$(CH_2)_7$-diazabicyclo[2.2.1]heptanylene-$(CH_2)_8$-, ##-$(CH_2)_8$-diazabicyclo[2.2.1]heptanylene-$CH_2$-, ##-$(CH_2)_8$-diazabicyclo[2.2.1]heptanylene-$(CH_2)_2$-, ##-$(CH_2)_8$-diazabicyclo[2.2.1]heptanylene-$(CH_2)_3$-, ##-$(CH_2)_8$-diazabicyclo[2.2.1]heptanylene-$(CH_2)_4$-, ##-$(CH_2)_8$-diazabicyclo[2.2.1]heptanylene-$(CH_2)_5$-, ##-$(CH_2)_8$-diazabicyclo[2.2.1]heptanylene-$(CH_2)_6$-, ##-$(CH_2)_8$-diazabicyclo[2.2.1]heptanylene-$(CH_2)_7$-, ##-$(CH_2)_8$-diazabicyclo[2.2.1]heptanylene-$(CH_2)_8$-, ##-$CH_2$-diazabicyclo[3.1.1]heptanylene-$CH_2$-, ##-$CH_2$-diazabicyclo[3.1.1]heptanylene-$(CH_2)_2$-, ##-$CH_2$-diazabicyclo[3.1.1]heptanylene-$(CH_2)_3$-, ##-$CH_2$-diazabicyclo[3.1.1]heptanylene-$(CH_2)_4$-, 99-$CH_2$-diazabicyclo[3.1.1]heptanylene-$(CH_2)_5$-, ##-$CH_2$-diazabicyclo[3.1.1]heptanylene-$(CH_2)_6$-, ##-$CH_2$-diazabicyclo[3.1.1]heptanylene-$(CH_2)_7$-, ##-$CH_2$-diazabicyclo[3.1.1]heptanylene-$(CH_2)_8$-, ##-$(CH_2)_2$-diazabicyclo[3.1.1]heptanylene-$(CH_2)_1$-, ##-$(CH_2)_2$-diazabicyclo[3.1.1]heptanylene-$(CH_2)_2$-, ##-$(CH_2)_2$-diazabicyclo[3.1.1]heptanylene-$(CH_2)_3$-, ##-$(CH_2)_2$-diazabicyclo[3.1.1]heptanylene-$(CH_2)_4$-, ##-$(CH_2)_2$-diazabicyclo[3.1.1]heptanylene-$(CH_2)_5$-, ##-$(CH_2)_2$-diazabicyclo[3.1.1]heptanylene-$(CH_2)_6$-, ##-$(CH_2)_2$-diazabicyclo[3.1.1]heptanylene-$(CH_2)_7$-, ##-$(CH_2)_2$-diazabicyclo[3.1.1]heptanylene-$(CH_2)_8$-, ##-$(CH_2)_3$-diazabicyclo[3.1.1]heptanylene-$CH_2$-, ##-$(CH_2)_3$-diazabicyclo[3.1.1]heptanylene-$(CH_2)_2$-, ##-$(CH_2)_3$-diazabicyclo[3.1.1]heptanylene-$(CH_2)_3$-, ##-$(CH_2)_3$-diazabicyclo[3.1.1]heptanylene-$(CH_2)_4$-, ##-$(CH_2)_3$-diazabicyclo[3.1.1]heptanylene-$(CH_2)_5$-, ##-$(CH_2)_3$-diazabicyclo[3.1.1]heptanylene-$(CH_2)_6$-, ##-$(CH_2)_3$-diazabicyclo[3.1.1]heptanylene-$(CH_2)_7$-, ##-$(CH_2)_3$-diazabicyclo[3.1.1]heptanylene-$(CH_2)_8$-, ##-$(CH_2)_4$-diazabicyclo[3.1.1]heptanylene-$CH_2$-, ##-$(CH_2)_4$-diazabicyclo[3.1.1]heptanylene-$(CH_2)_2$-, ##-$(CH_2)_4$-diazabicyclo[3.1.1]heptanylene-$(CH_2)_3$-, ##-$(CH_2)_4$-diazabicyclo[3.1.1]heptanylene-$(CH_2)_4$-, ##-$(CH_2)_4$-diazabicyclo[3.1.1]heptanylene-$(CH_2)_5$-, ##-$(CH_2)_4$-diazabicyclo[3.1.1]heptanylene-$(CH_2)_6$-, ##-$(CH_2)_4$-diazabicyclo[3.1.1]heptanylene-$(CH_2)_7$-, ##-$(CH_2)_4$-diazabicyclo[3.1.1]heptanylene-$(CH_2)_8$-, ##-$(CH_2)_5$-diazabicyclo[3.1.1]heptanylene-$(CH_2)_1$-, ##-$(CH_2)_5$-diazabicyclo[3.1.1]heptanylene-$(CH_2)_2$-, ##-$(CH_2)_5$-diazabicyclo[3.1.1]heptanylene-$(CH_2)_3$-, ##-$(CH_2)_5$-diazabicyclo[3.1.1]heptanylene-$(CH_2)_4$-, ##-$(CH_2)_5$-dia-

zabicyclo[3.1.1]heptanylene-$(CH_2)_5$-, ##-$(CH_2)_5$-diazabicyclo[3.1.1]heptanylene-$(CH_2)_6$-, ##-$(CH_2)_5$-diazabicyclo[3.1.1]heptanylene-$(CH_2)_7$-, ##-$(CH_2)_5$-diazabicyclo[3.1.1]heptanylene-$(CH_2)_8$-, ##-$(CH_2)_6$-diazabicyclo[3 .1.1]heptanylene-$(CH_2)_1$-, ##-$(CH_2)_6$-diazabicyclo[3.1.1]heptanylene-$(CH_2)_2$-, ##-$(CH_2)_6$-diazabicyclo[3.1.1]heptanylene-$(CH_2)_3$-, ##-$(CH_2)_6$-diazabicyclo[3.1.1]heptanylene-$(CH_2)_4$-, ##-$(CH_2)_6$-diazabicyclo[3.1.1]heptanylene-$(CH_2)_5$-, ##-$(CH_2)_6$-diazabicyclo[3.1.1]heptanylene-$(CH_2)_6$-, ##-$(CH_2)_6$-diazabicyclo[3.1.1]heptanylene-$(CH_2)_7$-, ##-$(CH_2)_6$-diazabicyclo[3 .1.1]heptanylene-$(CH_2)_8$-, ##-$(CH_2)_7$-diazabicyclo[3.1.1]heptanylene-$(CH_2)_1$-, ##-$(CH_2)_7$-diazabicyclo[3.1.1]heptanylene-$(CH_2)_2$-, ##-$(CH_2)_7$-diazabicyclo[3.1.1]heptanylene-$(CH_2)_3$-, ##-$(CH_2)_7$-diazabicyclo[3.1.1]heptanylene-$(CH_2)_4$-, ##-$(CH_2)_7$-diazabicyclo[3.1.1]heptanylene-$(CH_2)_8$-, ##-$(CH_2)_8$-diazabicyclo[3.1.1]heptanylene-$CH_2$-, ##-$(CH_2)_8$-diazabicyclo[3.1.1]heptanylene-$(CH_2)_2$-, ##-$(CH_2)_8$-diazabicyclo[3.1.1]heptanylene-$(CH_2)_3$-, ##-$(CH_2)_8$-diazabicyclo[3.1.1]heptanylene-$(CH_2)_4$-, ##-$(CH_2)_8$-diazabicyclo[3.1.1]heptanylene-$(CH_2)_5$-, ##-$(CH_Z)$s-diazabicyclo[3.1.1]heptanylene-$(CH_Z)_6$-, ##-$(CH_2)_8$-diazabicyclo[3.1.1]heptanylene-$(CH_2)_7$-, ##-$(CH_2)_8$-diazabicyclo[3.1.1]heptanylene-$(CH_2)_8$-, ##-$CH_2$-diazabicyclo[3.2.1]octylene-$CH_2$-, ##-$CH_2$-diazabicyclo[3.2.1]octylene-$(CH_2)_2$-, ##-$CH_2$-diazabicyclo[3.2.1]octylene-$(CH_2)_3$-, ##-$CH_2$-diazabicyclo[3.2.1]octylene-$(CH_2)_4$-, ##-$CH_2$-diazabicyclo[3.2.1]octylene-$(CH_2)_5$-, ##-$CH_2$-diazabicyclo[3.2.1]octylene-$(CH_2)_6$-, ##-$CH_2$-diazabicyclo[3.2.1]octylene-$(CH_2)_7$-, ##-$CH_2$-diazabicyclo[3.2.1]octylene-$(CH_2)_8$-, ##-$(CH_2)_2$-diazabicyclo[3.2.1]octylene-$(CH_2)_1$-, ##-$(CH_2)_2$-diazabicyclo[3.2.1]octylene-$(CH_2)_2$-, ##-$(CH_2)_2$-diazabicyclo[3.2.1]octylene-$(CH_2)_3$-, ##-$(CH_2)_2$-diazabicyclo[3.2.1]octylene-$(CH_2)_4$-, ##-$(CH_2)_2$-diazabicyclo[3.2.1]octylene-$(CH_2)_5$-, ##-$(CH_2)_2$-diazabicyclo[3.2.1]octylene-$(CH_2)_6$-, ##-$(CH_2)_2$-diazabicyclo[3.2.1]octylene-$(CH_2)_7$-, ##-$(CH_2)_2$-diazabicyclo[3.2.1]octylene-$(CH_2)_8$-, ##-$(CH_2)_3$-diazabicyclo[3.2.1]octylene-$CH_2$-, ##-$(CH_2)_3$-diazabicyclo[3.2.1]octylene-$(CH_2)_2$-, ##-$(CH_2)_3$-diazabicyclo[3.2.1]octylene-$(CH_2)_3$-, ##-$(CH_2)_3$-diazabicyclo[3.2.1]octylene-$(CH_2)_4$-, ##-$(CH_2)_3$-diazabicyclo[3.2.1]octylene-$(CH_2)_5$-, ##-$(CH_2)_3$-diazabicyclo[3.2.1]octylene-$(CH_2)_6$-, ##-$(CH_2)_3$-diazabicyclo[3.2.1]octylene-$(CH_2)_7$-, ##-$(CH_2)_3$-diazabicyclo[3.2.1]octylene-$(CH_2)_8$-, ##-$(CH_2)_4$-diazabicyclo[3.2.1]octylene-$CH_2$-, ##-$(CH_2)_4$-diazabicyclo[3.2.1]octylene-$(CH_2)_2$-, ##-$(CH_2)_4$-diazabicyclo[3.2.1]octylene-$(CH_2)_3$-, ##-$(CH_2)_4$-diazabicyclo[3.2.1]octylene-$(CH_2)_4$-, ##-$(CH_2)_4$-diazabicyclo[3.2.1]octylene-$(CH_2)_5$-, ##-$(CH_2)_4$-diazabicyclo[3.2.1]octylene-$(CH_2)_6$-, ##-$(CH_2)_4$-diazabicyclo[3.2.1]octylene-$(CH_2)_7$-, ##-$(CH_2)_4$-diazabicyclo[3.2.1]octylene-$(CH_2)_8$-, ##-$(CH_2)_5$-diazabicyclo[3.2.1]octylene-$(CH_2)_1$-, ##-$(CH_2)_5$-diazabicyclo[3.2.1]octylene-$(CH_2)_2$-, ##-$(CH_2)_5$-diazabicyclo[3.2.1]octylene-$(CH_2)_3$-, ##-$(CH_2)_5$-diazabicyclo[3.2.1]octylene-$(CH_2)_4$-, ##-$(CH_2)_5$-diazabicyclo[3.2.1]octylene-$(CH_2)_5$-, ##-$(CH_2)_5$-diazabicyclo[3.2.1]octylene-$(CH_Z)_6$-, ##-$(CH_2)_5$-diazabicyclo[3.2.1]octylene-$(CH_2)_7$-, ##-$(CH_2)_5$-diazabicyclo[3.2.1]octylene-$(CH_2)_8$-, ##-$(CH_2)_6$-diazabicyclo[3.2. 1]octylene-$(CH_2)_1$-, ##-$(CH_2)_6$-diazabicyclo[3.2.1]octylene-$(CH_2)_2$-, ##-$(CH_2)_6$-diazabicyclo[3.2.1]octylene-$(CH_2)_3$-, ##-$(CH_2)_6$-diazabicyclo[3.2.1]octylene-$(CH_2)_4$-, ##-$(CH_2)_6$-diazabicyclo[3.2.1]octylene-$(CH_2)_5$-, ##-$(CH_2)_6$-diazabicyclo[3.2.1]octylene-$(CH_2)_6$-, ##-$(CH_2)_6$-diazabicyclo[3.2.1]octylene-$(CH_2)_7$-, ##-$(CH_2)_6$-diazabicyclo[3.2.1]octylene-$(CH_2)_8$-, ##-$(CH_2)_7$-diazabicyclo[3.2.1]octylene-$(CH_2)_1$-, ##-$(CH_2)_7$-diazabicyclo[3.2.1]octylene-$(CH_2)_2$-, ##-$(CH_2)_7$-diazabicyclo[3.2.1]octylene-$(CH_2)_3$-, ##-$(CH_2)_7$-diazabicyclo[3.2.1]octylene-$(CH_2)_4$-, ##-$(CH_2)_7$-diazabicyclo[3.2.1]octylene-$(CH_2)_8$-, ##-$(CH_2)_8$-diazabicyclo[3.2.1]octylene-$CH_2$-, ##-$(CH_2)_8$-diazabicyclo[3.2.1]octylene-$(CH_2)_2$-, ##-$(CH_2)_8$-diazabicyclo[3.2.1]octylene-$(CH_2)_3$-, ##-$(CH_2)_8$-diazabicyclo[3.2.1]octylene-$(CH_2)_4$-, ##-$(CH_2)_8$-diazabicyclo[3.2.1]octylene-$(CH_2)_5$-, ##-$(CH_2)_8$-diazabicyclo[3.2.1]octylene-$(CH_Z)_6$-, ##-$(CH_2)_8$-diazabicyclo[3.2.1]octylene-$(CH_2)_7$-, ##-$(CH_2)_8$-diazabicyclo[3.2.1]octylene-$(CH_2)_8$-, ##-$CH_2$-diazabicyclo[2.2.2]octylene-$CH_2$-, ##-$CH_2$-diazabicyclo[2.2.2]octylene-$(CH_2)_2$-, ##-$CH_2$-diazabicyclo[2.2.2]octylene-$(CH_2)_3$-, ##-$CH_2$-diazabicyclo[2.2.2]octylene-$(CH_2)_4$-, ##-$CH_2$-diazabicyclo[2.2.2]octylene-$(CH_2)_5$-, ##-$CH_2$-diazabicyclo[2.2.2]octylene-$(CH_2)_6$-, ##-$CH_2$-diazabicyclo[2.2.2]octylene-$(CH_2)_7$-, ##-$CH_2$-diazabicyclo[2.2.2]octylene-$(CH_2)_8$-, ##-$(CH_2)_2$-diazabicyclo[2.2.2]octylene-$(CH_2)_1$-, ##-$(CH_2)_2$-diazabicyclo[2.2.2]octylene-$(CH_2)_2$-, ##-$(CH_2)_2$-diazabicyclo[2.2.2]octylene-$(CH_2)_3$-, ##-$(CH_2)_2$-diazabicyclo[2.2.2]octylene-$(CH_2)_4$-, ##-$(CH_2)_2$-diazabicyclo[2.2.2]octylene-$(CH_2)_5$-, ##-$(CH_2)_2$-diazabicyclo[2.2.2]octylene-$(CH_2)_6$-, ##-$(CH_2)_2$-diazabicyclo[2.2.2]octylene-$(CH_2)_7$-, ##-$(CH_2)_2$-diazabicyclo[2.2.2]octylene-$(CH_2)_8$-, ##-$(CH_2)_3$-diazabicyclo[2.2.2]octylene-$CH_2$-, ##-$(CH_2)_3$-diazabicyclo[2.2.2]octylene-$(CH_2)_2$-, ##-$(CH_2)_3$-diazabicyclo[2.2.2]octylene-$(CH_2)_3$-, ##-$(CH_2)_3$-diazabicyclo[2.2.2]octylene-$(CH_2)_4$-, ##-$(CH_2)_3$-diazabicyclo[2.2.2]octylene-$(CH_2)_5$-, ##-$(CH_2)_3$-diazabicyclo[2.2.2]octylene-$(CH_2)_6$-, ##-$(CH_2)_3$-diazabicyclo[2.2.2]octylene-$(CH_2)_7$-, ##-$(CH_2)_3$-diazabicyclo[2.2.2]octylene-$(CH_2)_8$-, ##-$(CH_2)_4$-diazabicyclo[2.2.2]octylene-$CH_2$-, ##-$(CH_2)_4$-diazabicyclo[2.2.2]octylene-$(CH_2)_2$-, ##-$(CH_2)_4$-diazabicyclo[2.2.2]octylene-$(CH_2)_3$-, ##-$(CH_2)_4$-diazabicyclo[2.2.2]octylene-$(CH_2)_4$-, ##-$(CH_2)_4$-diazabicyclo[2.2.2]octylene-$(CH_2)_5$-, ##-$(CH_2)_4$-diazabicyclo[2.2.2]octylene-$(CH_2)_6$-, ##-$(CH_2)_4$-diazabicyclo[2.2.2]octylene-$(CH_2)_7$-, ##-$(CH_2)_4$-diazabicyclo[2.2.2]octylene-$(CH_2)_8$-, ##-$(CH_2)_5$-diazabicyclo[2.2.2]octylene-$(CH_2)_1$-, ##-$(CH_2)_5$-diazabicyclo[2.2.2]octylene-$(CH_2)_2$-, ##-$(CH_2)_5$-diazabicyclo[2.2.2]octylene-$(CH_2)_3$-, ##-$(CH_2)_5$-diazabicyclo[2.2.2]octylene-$(CH_2)_4$-, ##-$(CH_2)_5$-diazabicyclo[2.2.2]octylene-$(CH_2)_5$-, ##-$(CH_2)_5$-diazabicyclo[2.2.2]octylene-$(CH_2)_6$-, ##-$(CH_2)_5$-diazabicyclo[2.2.2]octylene-$(CH_2)_7$-, ##-$(CH_2)_5$-diazabicyclo[2.2.2]octylene-$(CH_2)_8$-, ##-$(CH_2)_6$-diazabicyclo[2.2.2]octylene-$(CH_2)_1$-, ##-$(CH_2)_6$-diazabicyclo[2.2.2]octylene-$(CH_2)_2$-, ##-$(CH_2)_6$-diazabicyclo[2.2.2]octylene-$(CH_2)_3$-, ##-$(CH_2)_6$-diazabicyclo[2.2.2]octylene-$(CH_2)_4$-, ##-$(CH_2)_6$-diazabicyclo[2.2.2]octylene-$(CH_2)_5$-, ##-$(CH_2)_6$-diazabicyclo[2.2.2]octylene-$(CH_2)_6$-, ##-$(CH_2)_6$-diazabicyclo[2.2.2]octylene-$(CH_2)_7$-, ##-$(CH_2)_6$-diazabicyclo[2.2.2]octylene-$(CH_2)_8$-, ##-$(CH_2)_7$-diazabicyclo[2.2.2]octylene-$(CH_2)_1$-, ##-$(CH_2)_7$-diazabicyclo[2.2.2]octylene-$(CH_2)_2$-, ##-$(CH_2)_7$-diazabicyclo[2.2.2]octylene-$(CH_2)_3$-, ##-$(CH_2)_7$-diazabicyclo[2.2.2]octylene-$(CH_2)_4$-, ##-$(CH_2)_7$-diazabicyclo[2.2.2]octylene-$(CH_2)_8$-, ##-$(CH_2)_8$-

diazabicyclo[2.2.2]octylene-$CH_2$-, ##-$(CH_2)_8$-diazabicyclo[2.2.2]octylene-$(CH_2)_2$-, ##-$(CH_2)_8$-diazabicyclo[2.2.2]octylene-$(CH_2)_3$-, ##-$(CH_2)_8$-diazabicyclo[2.2.2]octylene-$(CH_2)_4$-, ##-$(CH_2)_8$-diazabicyclo[2.2.2]octylene-$(CH_2)_5$-, ##-$(CH_2)_8$-diazabicyclo[2.2.2]octylene-$(CH_2)_6$-, ##-$(CH_2)_8$-diazabicyclo[2.2.2]octylene-$(CH_2)_7$-, or ##-$(CH_2)_8$-diazabicyclo[2.2.2]octylene-$(CH_2)_8$-;

wherein the propylene, the piperidinylene, the piperazinylene, the diazacycloheptanylene, the diazabicyclo[2.2.1]heptanylene, the diazabicyclo[3.1.1]heptanylene, the diazabicyclo[3.2.1]octylene, the diazabicyclo[2.2.2]octylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;
each $R_{g10}$ independently represents H or $C_{1-3}$ alkyl; and
symbol ## indicates the point of attachment to $X_1$.

In some embodiments of the compound of Formula (I-6), $L_1$ represents the following group:

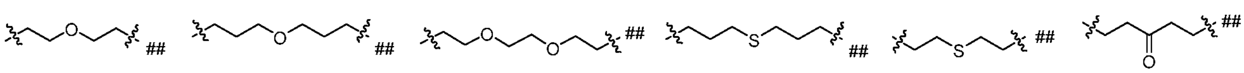

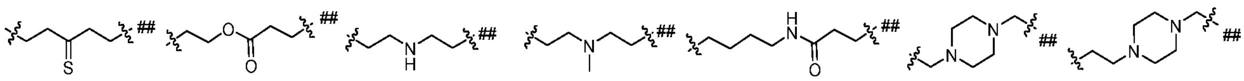

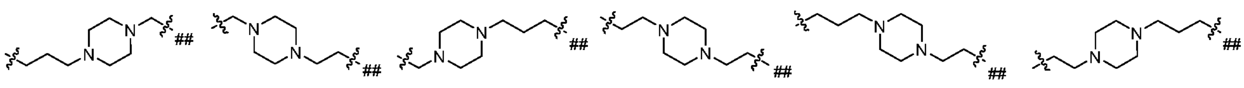

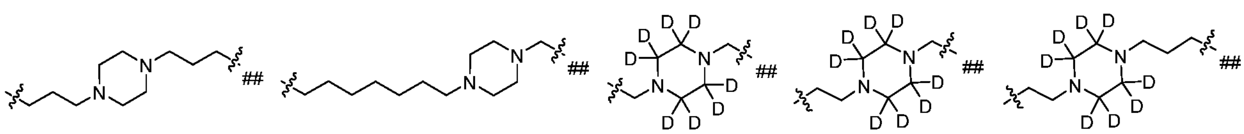

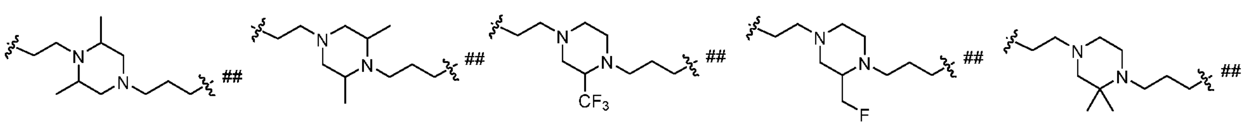

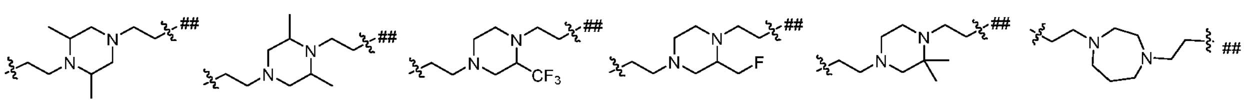

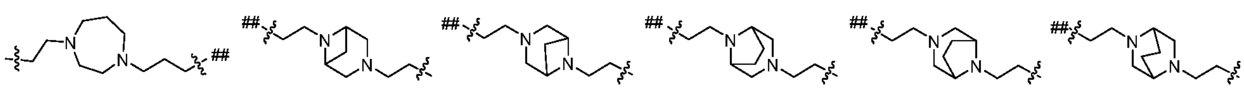

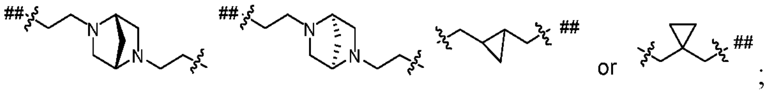

or ;

wherein symbol ## indicates the point of attachment to $X_1$.

In some embodiments of the compound of Formula (I-6), $R_b$ represents $C_{1-60}$ alkyl optionally substituted with D or halogen.

In some embodiments of the compound of Formula (I-6), $R_b$ represents the following group: $CH_3$, $CF_3$, $CD_3$, $CH_2CH_3$, -$(CH_2)_2$-$CH_3$, -$(CH_2)_3$-$CH_3$, -$(CH_2)_4$-$CH_3$, -$(CH_2)_5$-$CH_3$, -$(CH_2)_6$-$CH_3$, -$(CH_2)_7$-$CH_3$, -$(CH_2)_8$-$CH_3$, -$(CH_2)_9$-$CH_3$, -$(CH_2)_{10}$-$CH_3$, -$(CH_2)_{11}$-$CH_3$, -$(CH_2)_{12}$-$CH_3$, -$(CH_2)_{13}$-$CH_3$, -$(CH_2)_{14}$-$CH_3$, -$(CH_2)_{15}$-$CH_3$, -$(CH_2)_{16}$-$CH_3$, -$(CH_2)_{17}$-$CH_3$, -$(CH_2)_{18}$-$CH_3$, -$(CH_2)_{19}$-$CH_3$, -$(CH_2)_{20}$-$CH_3$, -$(CH_2)_{21}$-$CH_3$, -$(CH_2)_{22}$-$CH_3$, -$(CH_2)_{23}$-$CH_3$, -$(CH_2)_{24}$-$CH_3$, -$(CH_2)_{25}$-$CH_3$, -$(CH_2)_{26}$-$CH_3$, -$(CH_2)_{27}$-$CH_3$, -$(CH_2)_{28}$-$CH_3$, or -$(CH_2)_{29}$-$CH_3$.

In some embodiments of the compound of Formula (I-6), $R_b$ represents the following formula:

$R_{b1}$-$X_4$-$L_2$-,

wherein $L_2$ represents:

linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, $C_{1-4}$ alkyl, halogen, $C_{3-6}$cycloalkyl or any combination thereof, or optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{12}$ and/or one or more (e.g., 1-20, or 1-15, 1-10, 1-9, 1-8, 1-7, 1-6, 1-5, 1-4, 1-3 or 1-2, or 1) groups $R_{13}$ and/or any combination of one or more (e.g., 1-20, or 1-15, 1-10, 1-9, 1-8, 1-7, 1-6, 1-5, 1-4, 1-3 or 1-2, or 1) groups $R_{12}$ and $R_{13}$ inserted into its backbone carbon chain, wherein carbon-carbon bond between one or more pairs (e.g., 1-20, or 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_{12}$, $R_{13}$ or a combination of $R_{12}$ and $R_{13}$; wherein each $R_{12}$ is independently selected from the group consisting of O, S, N($R_{14}$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of $C_{3-15}$cycloalkylene, 4- to 15-membered heterocyclylene, $C_{6-10}$ arylene, 5- to 15-membered heteroarylene, $C_{2-6}$ alkenylene, $C_{2-6}$ alkynylene or any combination thereof, wherein the $C_{3-15}$cycloalkylene, the 4- to 15-membered heterocyclylene, $C_{6-10}$ arylene and the 5- to 15-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH-(e.g., $C_{1-3}$ alkyl-C(O)NH-, such as $CH_3$-C(O) NH-, $CH_3CH_2$-C(O)NH-, and $CH_3CH_2CH_2$-C(O)NH-), or any combination thereof, and the linear or branched $C_{2-60}$ alkylene is optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, $C_{1-4}$ alkyl, halogen, $C_{3-6}$cycloalkyl or any combination thereof;

$X_4$ represents a bond, or $X_4$ represents N($R_{15}$), C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted $C_{3-20}$cycloalkylene, optionally substituted $C_{6-20}$ arylene, optionally substituted 4- to 20-membered heterocyclylene, optionally substituted 5- to 20-membered heteroarylene, triazolylene-NH-, or -NH-triazolylene, wherein $R_{15}$ represents H or $C_{1-3}$ alkyl, and the $C_{3-20}$cycloalkylene, the $C_{6-20}$ arylene, the 4- to 20-membered heterocyclylene, and the 5- to 20-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, halogen, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-),

$NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof; and

$R_{b1}$ represents optionally substituted $C_{3-20}$cycloalkyl, optionally substituted $C_{6-20}$ aryl, optionally substituted 4- to 20-membered heterocyclyl or optionally substituted 5- to 20-membered heteroaryl, wherein the $C_{3-20}$cycloalkyl, the $C_{6-20}$ aryl, the 4- to 20-membered heterocyclyl, and the 5- to 20-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, $C_{1-3}$ alkoxy (e.g., methoxy, ethoxy, or propoxy), $C_{1-3}$ alkyl-NH- (e.g., $CH_3NH$-, $CH_3CH_2NH$- or $CH_3CH_2CH_2NH$-), halogenated $C_{1-3}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-3}$ alkylene (e.g., $NH_2CH_2$-, $NH_2CH_2CH_2$- and $NH_2CH_2CH_2CH_2$-), $C_{1-3}$ alkyl-NHC(O)- (e.g., $CH_3$-NHC(O)-, $CH_3CH_2$-NHC(O)-, and $CH_3CH_2CH_2$-NHC(O)-), $C_{1-3}$ alkyl-C(O)NH- (e.g., $CH_3$-C(O)NH-, $CH_3CH_2$-C(O)NH-, and $CH_3CH_2CH_2$-C(O)NH-), cyano or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH-(e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof.

In some embodiments of the compound of Formula (I-6), $L_2$ represents the structure of the following formula: -$C_{2-30}$ alkylene-, wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the $C_{2-30}$ alkylene is optionally replaced with a substituent selected from the group consisting of: D, $C_{1-4}$ alkyl, halogen, $C_{3-6}$cycloalkyl or any combination thereof.

In some embodiments of the compound of Formula (I-6), $L_2$ represents the following group:

$-CH_2-$, $-(CH_2)_2-$, $-(CH_2)_3-$, $-(CH_2)_4-$, $-(CH_2)_5-$, $-(CH_2)_2-CF_2-(CH_2)_2-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{15}-$, $-(CH_2)_{16}-$, $-(CH_2)_{17}-$, $-(CH_2)_{18}-$, $-(CH_2)_{19}-$, $-(CH_2)_{20}-$, $-(CH_2)_{21}-$, $-(CH_2)_{22}-$, $-(CH_2)_{25}-$, or $-(CH_2)_{30}-$;

wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the group is optionally replaced with a substituent selected from the group consisting of: D, $C_{1-4}$ alkyl, halogen, $C_{3-6}$cycloalkyl or any combination thereof.

In some embodiments of the compound of Formula (I-6), $L_2$ represents the structure of the following formula:

$$-(C(R^{a9})(R^{a10}))_{m1}-(R_{12}(C(R^{a11})(R^{a12}))_{m2})_{p1}-;$$

$$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-;$$

$$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-(R_{12}(C(R^{a15})(R^{a16}))_{m4})_{p3}-;$$

$$-(C(R^{a9})(R^{a10}))_{m1}-(R_{13}(C(R^{a11})(R^{a12}))_{m2})_{p1}-;$$

$$-(C(R^{a9})(R^{a10}))_{m1}-(R_{13}(C(R^{a11})(R^{a12}))_{m2})_{p1}-(R_{13}(C(R^{a13})(R^{a14}))_{m3})_{p2}-;$$

$$-(C(R^{a9})(R^{a10}))_{m1}-(R_{13}(C(R^{a11})(R^{a13}))_{m2})_{p1}-(R_{13}(C(R^{a13})(R^{a14}))_{m3})_{p2}-(R_{13}(C(R^{a15})(R^{a16}))_{m4})_{p3}-;$$

$$-(C(R^{a9})(R^{a10}))_{m1}-(R_{12}-R_{13}-(C(R^{a11})(R^{a12}))_{m2})_{p1}-;$$

$$-(C(R^{a9})(R^{a10}))_{m1}-(R_{12}(C(R^{a11})(R^{a12}))_{m2})_{p1}-(R_{13}(C(R^{a13})(R^{a14}))_{m3})_{p2}-;$$

$-(C(R^{a9})(R^{a10}))_{m1}-(R_{13}-R_{12}-(C(R^{a11})(R^{a12}))_{m2})_{p1}-$; or

$-(C(R^{a9})(R^{a10}))_{m1}-(R_{13}(C(R^{a11})(R^{a12}))_{m2})_{p1}-(R_{12}(C(R^{a13})(R^{al14}))_{m3})_{p2}-$;

wherein each group $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_{14}$ independently represents H or $C_{1-3}$ alkyl; and each group $R_{13}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;
$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and
m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-6), $L_2$ represents the structure of the following formula:

$-(C(R^{a9})(R^{a10}))_{m1}-(O(C(R^{a11})(R^{a12}))_{m2})_{p1}-$;

$-(C(R^{a9})(R^{a10}))_{m1}-(O(C(R^{a11})(R^{a12}))_{m2})_{p1}-(O(C(R^{a13})(R^{a14}))_{m3})_{p2}-$;

$-(C(R^{a9})(R^{a10}))_{m1}-(O(C(R^{a11})(R^{a12}))_{m2})_{p1}-(O(C(R^{a13})(R^{a14}))_{m3})_{p2}-(O(C(R^{a15})(R^{a16}))_{m4})_{p3}-$;

$-(C(R^{a9})(R^{a10}))_{m1}-(N(R_{g11})(C(R^{a11})(R^{a12}))_{m2})_{p1}-$;

$-(C(R^{a9})(R^{a10}))_{m1}-(N(R_{g11})(C(R^{a11})(R^{a12}))_{m2})_{p1}-(N(R_{g11})(C(R^{a13})(R^{a14}))_{m3})_{p2}-$;

$-(C(R^{a9})(R^{a10}))_{m1}-(N(R_{g11})(C(R^{a11})(R^{a12}))_{m2})_{p1}-(N(R_{g11})(C(R^{a13})(R^{a14}))_{m3})_{p2}-(N(R_{g11})(C(R^{a15})(R^{a16}))_{m4})_{p3}-$;

$-(C(R^{a9})(R^{a10}))_{m1}-(C(O)NH-(C(R^{a11})(R^{a12}))_{m2})_{p1}-$;

$-(C(R^{a9})(R^{a10}))_{m1}-(C(O)NH-(C(R^{a11})(R^{a12}))_{m2})_{p1}-(C(O)NH-(C(R^{a13})(R^{a14}))_{m3})_{p2}-$;

$-(C(R^{a9})(R^{a10}))_{m1}-(C(O)NH-(C(R^{a11})(R^{a12}))_{m2})_{p1}-(C(O)NH-(C(R^{a13})(R^{a14}))_{m3})_{p2}-(C(O)NH-(C(R^{a15})(R^{a16}))_{m4})_{p3}-$;

$-(C(R^{a9})(R^{a10}))_{m1}-(C(O)NH-(C(R^{a11})(R^{a12}))_{m2})_{p1}-(O-(C(R^{a13})(R^{a14}))_{m3})_{p2}-$;

$-(C(R^{a9})(R^{a10}))_{m1}-C(O)NH-(C(R^{a11})(R^{a12}))_{m2}-(O(C(R^{a13})(R^{a14}))_{m3})_{p1}-$;

$-(C(R^{a9})(R^{a10}))_{m1}-(NHC(O)-(C(R^{a11})(R^{a12})_{m2})_{p1}-$;

$-(C(R^{a9})(R^{a10}))_{m1}-(NHC(O)-(C(R^{a11})(R^{a12}))_{m2})_{p1}-(O-(C(R^{a13})(R^{a14}))_{m3})_{p2}-$;

$-(C(R^{a9})(R^{a10}))_{m1}-(NHC(O)-(C(R^{a11})(R^{a12}))_{m2})_{p1}-(O-(C(R^{a13})(R^{a14}))_{m2})_{p2}-(O-(C(R^{a15})(R^{a16}))_{m4})_{p3}-$;

$-(C(R^{a9})(R^{a10}))_{m1}-NHC(O)-(C(R^{a11})(R^{a12}))_{m2}-(O(C(R^{a13})(R^{a14}))_{m3})_{p1}-$;

$-(C(R^{a9})(R^{a10}))_{m1}-NHC(O)NH-(C(R^{a11})(R^{a12}))_{m2}-$;

$-(C(R^{a9})(R^{a10}))_{m1}-C(O)-(C(R^{a11})(R^{a12}))_{m2}-$;

$-(C(R^{a9})(Ra^{10}))_{m1}-C(S)-(C(R^{a11})(R^{a12}))_{m2}-$;

$-(C(R^{a9})(R^{a10}))_{m1}-S-(C(R^{a11})(R^{a12}))_{m2}-$;

$-(C(R^{a9})(R^{a10}))_{m1}-(C_{6-10}$ arylene-$(C(R^{a11})(R^{a12}))_{m2})_{p1}-$;

$-(C(R^{a9})(R^{a10}))_{m1}-(C_{6-10}$ arylene-$(C(R^{a11})(R^{a12}))_{m2})_{p1}-C_{6-10}$ arylene-$(C(R^{a13})(R^{a14}))_{m3}-$;

$-(C(R^{a9})(R^{a10}))_{m1}-$(4- to 15-membered heterocyclylene-$(C(R^{a11})(R^{a12}))_{m2})_{p1}-$;

$-(C(R^{a9})(R^{a10}))_{m1}-$(4- to 15-membered heterocyclylene-$(C(R^{a11})(R^{a12}))_{m2})_{p1}-$(4- to 15-membered heterocyclylene-$(C(R^{a13})(R^{a14}))_{m3})_{p2}-$;

$-(C(R^{a9})(R^{a10}))_{m1}-$(5- to 15-membered heteroarylene-$(C(R^{a11})(R^{a12}))_{m2})_{p1}-$;

$-(C(R^{a9})(R^{a10}))_{m1}-$(5- to 15-membered heteroarylene-$(C(R^{a11})(R^{a12}))_{m2})_{p1}-$(5- to 15-membered heteroarylene-$(C(R^{a13})(R^{a14}))_{m3})_{p2}-$;

$-(C(R^{a9})(R^{a10}))_{m1}-(C_{3-15}$cycloalkylene-$(C(R^{a11})(R^{a12}))_{m2})_{p1}-$; or

$-(C(R^{a9})(R^{a10}))_{m1}-(C_{3-15}$cycloalkylene-$(C(R^{a11})(R^{a12}))_{m2})_{p1}-(C_{3-15}$cycloalkylene-$(C(R^{a13})(R^{a14}))_{m3})_{p2}-$;

wherein each $R_{g11}$ independently represents H or $C_{1-3}$ alkyl;
the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;
$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and
m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-6), $L_2$ represents the structure of the following formula: $-CH_2-O-CH_2-$, $-CH_2-O-(CH_2)_2-$, $-(CH_2)_1-O-(CH_2)_3-$, $-(CH_2)_1-O-(CH_2)_4-$, $-(CH_2)_1-O-(CH_2)_5-$, $-(CH_2)_1-O-(CH_2)_6-$, $-(CH_2)_1-O-(CH_2)_7-$, $-(CH_2)_1-O-(CH_2)_8-$, $-(CH_2)_1-O-(CH_2)_9-$, $-(CH_2)_1-O-(CH_2)_{10}-$, $-(CH_2)_2-O-(CH_2)_1-$, $-(CH_2)_2-O-(CH_2)_2-$, $-(CH_2)_2-O-(CH_2)_3-$, $-(CH_2)_2-O-(CH_2)_4-$, $-(CH_2)_2-O-(CH_2)_5-$, $-(CH_2)_2-O-(CH_2)_6-$, $-(CH_2)_2-O-(CH_2)_7-$, $-(CH_2)_2-O-(CH_2)_8-$, $-(CH_2)_2-O-(CH_2)_9-$, $-(CH_2)_2-O-(CH_2)_{10}-$, $-(CH_2)_2-O-(CH_2)_{11}-$, $-(CH_2)_2-O-(CH_2)_{12}-$, $-(CH_2)_3-O-(CH_2)_1-$, $-(CH_2)_3-O-(CH_2)_2-$, $-(CH_2)_3-O-(CH_2)_3-$, $-(CH_2)_3-O-(CH_2)_4-$, $-(CH_2)_3-O-(CH_2)_5-$, $-(CH_2)_3-O-(CH_2)_6-$, $-(CH_2)_3-O-(CH_2)_7-$, $-(CH_2)_4-O-(CH_2)_1-$, $-(CH_2)_4-O-(CH_2)_2-$, $-(CH_2)_4-O-(CH_2)_3-$, $-(CH_2)_4-O-(CH_2)_4-$, $-(CH_2)_4-O-(CH_2)_5-$, $-(CH_2)_4-O-(CH_2)_6-$, $-(CH_2)_5-O-(CH_2)_1-$, $-(CH_2)_5-O-(CH_2)_2-$, $-(CH_2)_5-O-(CH_2)_3-$, $-(CH_2)_5-O-(CH_2)_4-$, $-(CH_2)_5-O-(CH_2)_5-$, $-(CH_2)_6-O-(CH_2)_1-$, $-(CH_2)_6-O-(CH_2)_2-$, $-(CH_2)_6-O-(CH_2)_3-$, $-(CH_2)_6-O-(CH_2)_4-$, $-(CH_2)_7-O-(CH_2)_1-$, $-(CH_2)_7-O-(CH_2)_2-$, $-(CH_2)_7-O-(CH_2)_3-$, $-(CH_2)_8-O-(CH_2)_1-$, $-(CH_2)_8-O-(CH_2)_2-$, $-CH(CH_3)-O-(CH_2)_1-$, $-CH(CH_3)-O-(CH_2)_2-$, $-CH(CH_3)-O-(CH_2)_3-$, $-CH(CH_3)-O-(CH_2)_4-$, $-CH(CH_3)-O-(CH_2)_5-$, $-CH(CH_3)-O-(CH_2)_6-$, $-CH(CH_3)-O-(CH_2)_7-$, $-CH(CH_3)-O-(CH_2)_8-$, $-CH(CH_3)-O-(CH_2)_9-$, $-CH(CH_3)-O-(CH_2)_{10}-$, $-CH_2-(O(CH_2)_2)_2-$, $-CH_2-(O(CH_2)_2)_3-$, $-CH_2-(O(CH_2)_2)_4-$, $-CH_2-(O(CH_2)_2)_5-$, $-CH_2-(O(CH_2)_2)_6-$, $-CH_2-(O(CH_2)_2)_7-$, $-CH_2-(O(CH_2)_2)_8-$, $-CH_2-(O(CH_2)_2)_9-$, $-CH_2-(O(CH_2)_2)_{10}-$, $-CH_2-(O(CH_2)_2)_1-OCH_2-$, $-CH_2-(O(CH_2)_2)_2-OCH_2-$, $-CH_2-(O(CH_2)_2)_3-OCH_2-$, $-CH_2-(O(CH_2)_2)_4-OCH_2-$, $-CH_2-(O(CH_2)_2)_5-OCH_2-$, $-CH_2-(O(CH_2)_2)_6-OCH_2-$, $-CH_2-(O(CH_2)_2)_7-OCH_2-$, $-CH_2-(O(CH_2)_2)_8-OCH_2-$, $-CH_2-(O(CH_2)_2)_9-OCH_2-$, $-CH_2-(O(CH_2)_2)_{10}-OCH_2-$, $-(CH_2)_2-(O(CH_2)_2)_2-$, $-(CH_2)_2-(O(CH_2)_2)_3-$, $-(CH_2)_2-(O(CH_2)_2)_4-$, $-(CH_2)_2-(O(CH_2)_2)_5-$, $-(CH_2)_2-(O(CH_2)_2)_6-$, $-(CH_2)_2-(O(CH_2)_2)_7-$, $-(CH_2)_2-(O(CH_2)_2)_8-$, $-(CH_2)_2-(O(CH_2)_2)_9-$, $-(CH_2)_2-(O(CH_2)_2)_{10}-$, $-(CH_2)_3-(O(CH_2)_2)_2-$, $-(CH_2)_3-(O(CH_2)_2)_3-$, $-(CH_2)_3-(O(CH_2)_2)_4-$, $-(CH_2)_3-(O(CH_2)_2)_5-$, $-(CH_2)_3-(O(CH_2)_2)_6-$, $-(CH_2)_3-(O(CH_2)_2)_7-$, $-(CH_2)_3-(O(CH_2)_2)_8-$, $-(CH_2)_3-(O(CH_2)_2)_9-$, $-(CH_2)_3-(O(CH_2)_2)_{10}-$, $-(CH_2)_4-(O(CH_2)_2)_2-$, $-(CH_2)_4-(O(CH_2)_2)_3-$, $-(CH_2)_4-(O(CH_2)_2)_4-$, $-(CH_2)_4-(O(CH_2)_2)_5-$, $-(CH_2)_4-(O(CH_2)_2)_6-$, $-(CH_2)_4-(O(CH_2)_2)_7-$, $-(CH_2)_4-(O(CH_2)_2)_8-$, $-(CH_2)_4-(O(CH_2)_2)_9-$, $-(CH_2)_4-(O(CH_2)_2)_{10}-$, $-CH_2-(O(CH_2)_3)_2-$, $-CH_2-(O(CH_2)_3)_3-$, $-CH_2-(O(CH_2)_3)_4-$, $-CH_2-(O(CH_2)_3)_5-$, $-CH_2-(O(CH_2)_3)_6-$, $-CH_2-(O(CH_2)_3)_7-$, $-CH_2-(O(CH_2)_3)_8-$, $-CH_2-(O(CH_2)_3)_9-$, $-CH_2-(O(CH_2)_3)_{10}-$, $-(CH_2)_2-(O(CH_2)_3)_2-$, $-(CH_2)_2-(O(CH_2)_3)_3-$, $-(CH_2)_2-(O(CH_2)_3)_4-$, $-(CH_2)_2-(O(CH_2)_3)_5-$, $-(CH_2)_2-(O(CH_2)_3)_6-$, $-(CH_2)_2-(O(CH_2)_3)_7-$,

$-(CH_2)_2-(O(CH_2)_3)_8-$, $-(CH_2)_2-(O(CH_2)_3)_9-$, $-(CH_2)_2-(O(CH_2)_3)_{10}-$, $-(CH_2)_3-(O(CH_2)_3)_2-$, $-(CH_2)_3-(O(CH_2)_3)_3-$, $-(CH_2)_3-(O(CH_2)_3)_4-$, $-(CH_2)_3-(O(CH_2)_3)_5-$,$-(CH_2)_3-(O(CH_2)_3)_6-$, $-(CH_2)_3-(O(CH_2)_3)_7-$, $-(CH_2)_3-(O(CH_2)_3)_8-$, $-(CH_2)_3-(O(CH_2)_3)_9-$, $-(CH_2)_3-(O(CH_2)_3)_{10}-$, $-CH_2-O-(CH_2)_2-O-(CH_2)_3-$, $-CH_2-(O(CH_2)_2)_2-(O(CH_2)_3)_2-$, $-CH_2-(O(CH_2)_2)_3-(O(CH_2)_3)_3-$, $-CH_2-(O(CH_2)_2)_4-(O(CH_2)_3)_4-$, $-CH_2-(O(CH_2)_2)_5-(O(CH_2)_3)_5-$, $-CH_2-(O(CH_2)_2)_6-(O(CH_2)_3)_6-$, $-(CH_2)_2-O-(CH_2)_2-O-(CH_2)_3-$, $-(CH_2)_2-(O(CH_2)_2)_2-(O(CH_2)_3)_2-$, $-(CH_2)_2-(O(CH_2)_2)_3-(O(CH_2)_3)_3-$, $-(CH_2)_2-(O(CH_2)_2)_4-(O(CH_2)_3)_4-$, $-(CH_2)_2-(O(CH_2)_2)_5-(O(CH_2)_3)_5-$, $-(CH_2)_2-(O(CH_2)_2)_6-(O(CH_2)_3)_6-$, $-(CH_2)_3-O-(CH_2)_2-O-(CH_2)_3-$, $-(CH_2)_3-(O(CH_2)_2)_2-(O(CH_2)_3)_2-$, $-(CH_2)_3-(O(CH_2)_2)_3-(O(CH_2)_3)_3-$, $-(CH_2)_3-(O(CH_2)_2)_4-(O(CH_2)_3)_4-$, $-(CH_2)_3-(O(CH_2)_2)_5-(O(CH_2)_3)_5-$, $-(CH_2)_3-(O(CH_2)_2)_6-(O(CH_2)_3)_6-$, $-CH_2-O-(CH_2)_3-O-(CH_2)_2-$, $-CH_2-(O(CH_2)_3)_2-(O(CH_2)_2)_2-$, $-CH_2-(O(CH_2)_3)_3-(O(CH_2)_2)_3-$, $-CH_2-(O(CH_2)_3)_4-(O(CH_2)_2)_4-$, $-CH_2-(O(CH_2)_3)_5-(O(CH_2)_2)_5-$, $-CH_2-(O(CH_2)_3)_6-(O(CH_2)_2)_6-$, $-(CH_2)_2-O-(CH_2)_3-O-(CH_2)_2-$, $-(CH_2)_2-(O(CH_2)_3)_2-(O(CH_2)_2)_2-$, $-(CH_2)_2-(O(CH_2)_3)_3-(O(CH_2)_2)_3-$, $-(CH_2)_2-(O(CH_2)_3)_4-(O(CH_2)_2)_4-$, $-(CH_2)_2-(O(CH_2)_3)_5-(O(CH_2)_2)_5-$, $-(CH_2)_2-(O(CH_2)_3)_6-(O(CH_2)_2)_6-$, $-(CH_2)_3-O-(CH_2)_3-O-(CH_2)_2-$, $-(CH_2)_3-(O(CH_2)_3)_2-(O(CH_2)_2)_2-$, $-(CH_2)_3-(O(CH_2)_3)_3-(O(CH_2)_2)_3-$, $-(CH_2)_3-(O(CH_2)_3)_4-(O(CH_2)_2)_4-$, $-(CH_2)_3-(O(CH_2)_3)_5-(O(CH_2)_2)_5-$, $-(CH_2)_3-(O(CH_2)_3)_6-(O(CH_2)_2)_6-$, $-CH_2-O-(CH_2)_2-O-CH_2-$, $-(CH_2)_2-O-(CH_2)_2-O-CH_2-$, $-(CH_2)_2-(O(CH_2)_2)_2-O-(CH_2)_3-$, $-(CH_2)_2-(O(CH_2)_2)_3-O-(CH_2)_3-$,$-(CH_2)_2-(O(CH_2)_2)_4-O-(CH_2)_3-$, $-(CH_2)_5-(O(CH_2)_2)_2-O-(CH_2)_5-$, $-(CH_2)_5-(O(CH_2)_2)_2-O-(CH_2)_6-$, $-CH_2-C(O)-CH_2-$, $-CH_2-C(O)-(CH_2)_2-$, $-(CH_2)_1-C(O)-(CH_2)_3-$, $-(CH_2)_1-C(O)-(CH_2)_4-$, $-(CH_2)_1-C(O)-(CH_2)_5-$, $-(CH_2)_1-C(O)-(CH_2)_6-$, $-(CH_2)_1-C(O)-(CH_2)_7-$, $-(CH_2)_1-C(O)-(CH_2)_8-$, $-(CH_2)_1-C(O)-(CH_2)_9-$, $-(CH_2)_1-C(O)-(CH_2)_{10}-$, $-(CH_2)_2-C(O)-(CH_2)_1-$, $-(CH_2)_2-C(O)-(CH_2)_2-$, $-(CH_2)_2-C(O)-(CH_2)_3-$, $-(CH_2)_2-C(O)-(CH_2)_4-$, $-(CH_2)_2-C(O)-(CH_2)_5-$, $-(CH_2)_2-C(O)-(CH_2)_6-$, $-(CH_2)_2-C(O)-(CH_2)_7-$, $-(CH_2)_2-C(O)-(CH_2)_8-$,$-(CH_2)_2-C(O)-(CH_2)_9-$, $-(CH_2)_2-C(O)-(CH_2)_{10}-$, $-(CH_2)_2-C(O)-(CH_2)_{11}-$, $-(CH_2)_2-C(O)-(CH_2)_{12}-$,$-(CH_2)_3-C(O)-(CH_2)_1-$, $-(CH_2)_3-C(O)-(CH_2)_2-$, $-(CH_2)_3-C(O)-(CH_2)_3-$, $-(CH_2)_3-C(O)-(CH_2)_4-$, $-(CH_2)_3-C(O)-(CH_2)_5-$, $-(CH_2)_3-C(O)-(CH_2)_6-$, $-(CH_2)_3-C(O)-(CH_2)_7-$, $-(CH_2)_4-C(O)-(CH_2)_1-$, $-(CH_2)_4-C(O)-(CH_2)_2-$, $-(CH_2)_4-C(O)-(CH_2)_3-$, $-(CH_2)_4-C(O)-(CH_2)_4-$, $-(CH_2)_4-C(O)-(CH_2)_5-$, $-(CH_2)_4-C(O)-(CH_2)_6-$,$-(CH_2)_5-C(O)-(CH_2)_1-$, $-(CH_2)_5-C(O)-(CH_2)_2-$, $-(CH_2)_5-C(O)-(CH_2)_3-$, $-(CH_2)_5-C(O)-(CH_2)_4-$, $-(CH_2)_5-C(O)-(CH_2)_5-$, $-(CH_2)_6-C(O)-(CH_2)_1-$, $-(CH_2)_6-C(O)-(CH_2)_2-$, $-(CH_2)_6-C(O)-(CH_2)_3-$, $-(CH_2)_6-C(O)-(CH_2)_4-$, $-(CH_2)_7-C(O)-(CH_2)_1-$, $-(CH_2)_7-C(O)-(CH_2)_2-$, $-(CH_2)_7-C(O)-(CH_2)_3-$, $-(CH_2)_8-C(O)-(CH_2)_1-$,$-(CH_2)_8-C(O)-(CH_2)_2-$, $-CH_2-S-CH_2-$, $-CH_2-S-(CH_2)_2-$, $-(CH_2)_1-S-(CH_2)_3-$, $-(CH_2)_1-S-(CH_2)_4-$, $-(CH_2)_1-S-(CH_2)_5-$, $-(CH_2)_1-S-(CH_2)_6-$, $-(CH_2)_1-S-(CH_2)_7-$, $-(CH_2)_1-S-(CH_2)_8-$, $-(CH_2)_1-S-(CH_2)_9-$, $-(CH_2)_1-S-(CH_2)_{10}-$, $-(CH_2)_2-S-(CH_2)_1-$, $-(CH_2)_2-S-(CH_2)_2-$, $-(CH_2)_2-S-(CH_2)_3-$, $-(CH_2)_2-S-(CH_2)_4-$, $-(CH_2)_2-S-(CH_2)_5-$, $-(CH_2)_2-S-(CH_2)_6-$, $-(CH_2)_2-S-(CH_2)_7-$, $-(CH_2)_2-S-(CH_2)_8-$, $-(CH_2)_2-S-(CH_2)_9-$, $-(CH_2)_2-S-(CH_2)_{10}-$, $-(CH_2)_2-S-(CH_2)_{11}-$, $-(CH_2)_2-S-(CH_2)_{12}-$, $-(CH_2)_3-S-(CH_2)_1-$, $-(CH_2)_3-S-(CH_2)_2-$, $-(CH_2)_3-S-(CH_2)_3-$, $-(CH_2)_3-S-(CH_2)_4-$, $-(CH_2)_3-S-(CH_2)_5-$, $-(CH_2)_3-S-(CH_2)_6-$, $-(CH_2)_3-S-(CH_2)_7-$, $-(CH_2)_4-S-(CH_2)_1-$, $-(CH_2)_4-S-(CH_2)_2-$, $-(CH_2)_4-S-(CH_2)_3-$, $-(CH_2)_4-S-(CH_2)_4-$, $-(CH_2)_4-S-(CH_2)_5-$, $-(CH_2)_4-S-(CH_2)_6-$, $-(CH_2)_5-S-(CH_2)_1-$, $-(CH_2)_5-S-(CH_2)_2-$, $-(CH_2)_5-S-(CH_2)_3-$, $-(CH_2)_5-S-(CH_2)_4-$, $-(CH_2)_5-S-(CH_2)_5-$, $-(CH_2)_6-S-(CH_2)_1-$, $-(CH_2)_6-S-(CH_2)_2-$, $-(CH_2)_6-S-(CH_2)_3-$, $-(CH_2)_6-S-(CH_2)_4-$, $-(CH_2)_7-S-(CH_2)_1-$, $-(CH_2)_7-S-(CH_2)_2-$, $-(CH_2)_7-S-(CH_2)_3-$, $-(CH_2)_8-S-(CH_2)_1-$, $-(CH_2)_8-S-(CH_2)_2-$, $-CH_2-C(S)-CH_2-$, $-CH_2-C(S)-(CH_2)_2-$, $-(CH_2)_1-C(S)-(CH_2)_3-$, $-(CH_2)_1-C(S)-(CH_2)_4-$, $-(CH_2)_1-C(S)-(CH_2)_5-$,$-(CH_2)_1-C(S)-(CH_2)_6-$, $-(CH_2)_1-C(S)-(CH_2)_7-$, $-(CH_2)_1-C(S)-(CH_2)_8-$, $-(CH_2)_1-C(S)-(CH_2)_9-$, $-(CH_2)_1-C(S)-(CH_2)_{10}-$, $-(CH_2)_2-C(S)-(CH_2)_1-$, $-(CH_2)_2-C(S)-(CH_2)_2-$, $-(CH_2)_2-C(S)-(CH_2)_3-$, $-(CH_2)_2-C(S)-(CH_2)_4-$, $-(CH_2)_2-C(S)-(CH_2)_5-$, $-(CH_2)_2-C(S)-(CH_2)_6-$, $-(CH_2)_2-C(S)-(CH_2)_7-$, $-(CH_2)_2-C(S)-(CH_2)_8-$,$-(CH_2)_2-C(S)-(CH_2)_9-$, $-(CH_2)_2-C(S)-(CH_2)_{10}-$ , $-(CH_2)_2-C(S)-(CH_2)_{11}-$, $-(CH_2)_2-C(S)-(CH_2)_{12}-$, $-(CH_2)_3-C(S)-(CH_2)_1-$, $-(CH_2)_3-C(S)-(CH_2)_2-$, $-(CH_2)_3-C(S)-(CH_2)_3-$, $-(CH_2)_3-C(S)-(CH_2)_4-$, $-(CH_2)_3-C(S)-(CH_2)_5-$, $-(CH_2)_3-C(S)-(CH_2)_6-$, $-(CH_2)_3-C(S)-(CH_2)_7-$, $-(CH_2)_4-C(S)-(CH_2)_1-$, $-(CH_2)_4-C(S)-(CH_2)_2-$,$-(CH_2)_4-C(S)-(CH_2)_3-$, $-(CH_2)_4-C(S)-(CH_2)_4-$, $-(CH_2)_4-C(S)-(CH_2)_5-$, $-(CH_2)_4-C(S)-(CH_2)_6-$, $-(CH_2)_5-C(S)-(CH_2)_1-$, $-(CH_2)_5-C(S)-(CH_2)_2-$, $-(CH_2)_5-C(S)-(CH_2)_3-$, $-(CH_2)_5-C(S)-(CH_2)_4-$, $-(CH_2)_5-C(S)-(CH_2)_5-$, $-(CH_2)_6-C(S)-(CH_2)_1-$, $-(CH_2)_6-C(S)-(CH_2)_2-$, $-(CH_2)_6-C(S)-(CH_2)_3-$, $-(CH_2)_6-C(S)-(CH_2)_4-$,$-(CH_2)_7-C(S)-(CH_2)_1-$, $-(CH_2)_7-C(S)-(CH_2)_2-$, $-(CH_2)_7-C(S)-(CH_2)_3-$, $-(CH_2)_8-C(S)-(CH_2)_1-$, $-(CH_2)_8-C(S)-(CH_2)_2-$, $-CH_2-C(O)O-CH_2-$, $-CH_2-C(O)O-(CH_2)_2-$, $-(CH_2)_1-C(O)O-(CH_2)_3-$, $-(CH_2)_1-C(O)O-(CH_2)_4-$, $-(CH_2)_1-C(O)O-(CH_2)_5-$, $-(CH_2)_1-C(O)O-(CH_2)_6-$, $-(CH_2)_1-C(O)O-(CH_2)_7-$, $-(CH_2)_1-C(O)O-(CH_2)_8-$, $-(CH_2)_1-C(O)O-(CH_2)_9-$, $-(CH_2)_1-C(O)O-(CH_2)_{10}-$, $-(CH_2)_2-C(O)O-(CH_2)_1-$, $-(CH_2)_2-C(O)O-(CH_2)_2-$, $-(CH_2)_2-C(O)O-(CH_2)_3-$, $-(CH_2)_2-C(O)O-(CH_2)_4-$, $-(CH_2)_2-C(O)O-(CH_2)_5-$, $-(CH_2)_2-C(O)O-(CH_2)_6-$, $-(CH_2)_2-C(O)O-(CH_2)_7-$, $-(CH_2)_2-C(O)O-(CH_2)_8-$, $-(CH_2)_2-C(O)O-(CH_2)_9-$, $-(CH_2)_2-C(O)O-(CH_2)_{10}-$, $-(CH_2)_2-C(O)O-(CH_2)_{11}-$, $-(CH_2)_2-C(O)O-(CH_2)_{12}-$, $-(CH_2)_3-C(O)O-(CH_2)_1-$, $-(CH_2)_3-C(O)O-(CH_2)_2-$, $-(CH_2)_3-C(O)O-(CH_2)_3-$, $-(CH_2)_3-C(O)O-(CH_2)_4-$, $-(CH_2)_3-C(O)O-(CH_2)_5-$, $-(CH_2)_3-C(O)O-(CH_2)_6-$, $-(CH_2)_3-C(O)O-(CH_2)_7-$, $-(CH_2)_4-C(O)O-(CH_2)_1-$, $-(CH_2)_4-C(O)O-(CH_2)_2-$, $-(CH_2)_4-C(O)O-(CH_2)_3-$, $-(CH_2)_4-C(O)O-(CH_2)_4-$, $-(CH_2)_4-C(O)O-(CH_2)_5-$, $-(CH_2)_4-C(O)O-(CH_2)_6-$, $-(CH_2)_5-C(O)O-(CH_2)_1-$, $-(CH_2)_5-C(O)O-(CH_2)_2-$, $-(CH_2)_5-C(O)O-(CH_2)_3-$, $-(CH_2)_5-C(O)O-(CH_2)_4-$, $-(CH_2)_5-C(O)O-(CH_2)_5-$, $-(CH_2)_6-C(O)O-(CH_2)_1-$, $-(CH_2)_6-C(O)O-(CH_2)_2-$, $-(CH_2)_6-C(O)O-(CH_2)_3-$, $-(CH_2)_6-C(O)O-(CH_2)_4-$, $-(CH_2)_7-C(O)O-(CH_2)_1-$, $-(CH_2)_7-C(O)O-(CH_2)_2-$, $-(CH_2)_7-C(O)O-(CH_2)_3-$, $-(CH_2)_8-C(O)O-(CH_2)_1-$, $-(CH_2)_8-C(O)O-(CH_2)_2-$, $-CH_2-O-C(O)-CH_2-$, $-CH_2-OC(O)-(CH_2)_2-$, $-(CH_2)_1-OC(O)-(CH_2)_3-$,$-(CH_2)_1-OC(O)-(CH_2)_4-$, $-(CH_2)_1-OC(O)-(CH_2)_5-$, $-(CH_2)_1-O-C(O)-(CH_2)_6-$, $-(CH_2)_1-OC(O)-(CH_2)_7-$,$-(CH_2)_1-OC(O)-(CH_2)_8-$, $-(CH_2)_1-OC(O)-(CH_2)_9-$, $-(CH_2)_1-OC(O)-(CH_2)_{10}-$, $-(CH_2)_2-OC(O)-(CH_2)_1-$,$-(CH_2)_2-OC(O)-(CH_2)_2-$, $-(CH_2)_2-OC(O)-(CH_2)_3-$, $-(CH_2)_2-OC(O)-(CH_2)_4-$, $-(CH_2)_2-O-$

$C(O)-(CH_2)_5-$,$-(CH_2)_2-OC(O)-(CH_2)_6-$, $-(CH_2)_2-OC(O)-(CH_2)_7-$, $-(CH_2)_2-OC(O)-(CH_2)_8-$, $-(CH_2)_2-O-C(O)-(CH_2)_9-$,$-(CH_2)_2-OC(O)-(CH_2)_{10}-$, $-(CH_2)_2-OC(O)-(CH_2)_{11}-$, $-(CH_2)_2-OC(O)-(CH_2)_{12}-$, $-(CH_2)_3-O-C(O)-(CH_2)_1-$,$-(CH_2)_3-OC(O)-(CH_2)_2-$, $-(CH_2)_3-OC(O)-(CH_2)_3-$, $-(CH_2)_3-OC(O)-(CH_2)_4-$, $-(CH_2)_3-O-C(O)-(CH_2)_5-$,$-(CH_2)_3-OC(O)-(CH_2)_6-$, $-(CH_2)_3-OC(O)-(CH_2)_7-$, $-(CH_2)_4-OC(O)-(CH_2)_1-$, $-(CH_2)_4-O-C(O)-(CH_2)_2-$,$-(CH_2)_4-OC(O)-(CH_2)_3-$, $-(CH_2)_4-OC(O)-(CH_2)_4-$, $-(CH_2)_4-OC(O)-(CH_2)_5-$, $-(CH_2)_4-O-C(O)-(CH_2)_6-$,$-(CH_2)_5-OC(O)-(CH_2)_1-$, $-(CH_2)_5-OC(O)-(CH_2)_2-$, $-(CH_2)_5-OC(O)-(CH_2)_3-$, $-(CH_2)_5-O-C(O)-(CH_2)_4-$,$-(CH_2)_5-OC(O)-(CH_2)_5-$, $-(CH_2)_6-OC(O)-(CH_2)_1-$, $-(CH_2)_6-OC(O)-(CH_2)_2-$, $-(CH_2)_6-O-C(O)-(CH_2)_3-$,$-(CH_2)_6-OC(O)-(CH_2)_4-$, $-(CH_2)_7-OC(O)-(CH_2)_1-$, $-(CH_2)_7-OC(O)-(CH_2)_2-$, $-(CH_2)_7-O-C(O)-(CH_2)_3-$,$-(CH_2)_8-OC(O)-(CH_2)_1-$, $-(CH_2)_8-OC(O)-(CH_2)_2-$, $-(CH_2)_1-N(R_{g11})-(CH_2)_1-$, $-(CH_2)_1-N(R_{g11})-(CH_2)_2-$,$-(CH_2)_1-N(R_{g11})-(CH_2)_3-$, $-(CH_2)_1-N(R_{g11})-(CH_2)_4-$, $-(CH_2)_1-N(R_{g11})-(CH_2)_5-$, $-(CH_2)_1-N(R_{g11})-(CH_2)_6-$,$-(CH_2)_1-N(R_{g11})-(CH_2)_7-$, $-(CH_2)_1-N(R_{g11})-(CH_2)_8-$, $-(CH_2)_1-N(R_{g11})-(CH_2)_9-$, $-(CH_2)_1-N(R_{g11})-(CH_2)_{10}-$, $-(CH_2)_2-N(R_{g11})-(CH_2)_1-$, $-(CH_2)_2-N(R_{g11})-(CH_2)_2-$, $-(CH_2)_2-N(R_{g11})-(CH_2)_3-$, $-(CH_2)_2-N(R_{g11})-(CH_2)_4-$, $-(CH_2)_2-N(R_{g11})-(CH_2)_5-$, $-(CH_2)_2-N(R_{g11})-(CH_2)_6-$, $-(CH_2)_2-N(R_{g11})-(CH_2)_7-$, $-(CH_2)_2-N(R_{g11})-(CH_2)_8-$, $-(CH_2)_2-N(R_{g11})-(CH_2)_9-$, $-(CH_2)_2-N(R_{g11})-(CH_2)_{10}-$, $-(CH_2)_2-N(R_{g11})-(CH_2)_{11}-$, $-(CH_2)_2-N(R_{g11})-(CH_2)_{12}-$, $-(CH_2)_3-N(R_{g11})-(CH_2)_1-$, $-(CH_2)_3-N(R_{g11})-(CH_2)_2-$, $-(CH_2)_3-N(R_{g11})-(CH_2)_3-$, $-(CH_2)_4-N(R_{g11})-(CH_2)_1-$, $-(CH_2)_4-N(R_{g11})-(CH_2)_2-$, $-(CH_2)_4-N(R_{g11})-(CH_2)_3-$, $-(CH_2)_4-N(R_{g11})-(CH_2)_4-$, $-(CH_2)_5-N(R_{g11})-(CH_2)_1-$, $-(CH_2)_5-N(R_{g11})-(CH_2)_2-$, $-(CH_2)_5-N(R_{g11})-(CH_2)_3-$, $-(CH_2)_5-N(R_{g11})-(CH_2)_4-$, $-(CH_2)_5-N(R_{g11})-(CH_2)_5-$, $-(CH_2)_6-N(R_{g11})-(CH_2)_1-$, $-(CH_2)_6-N(R_{g11})-(CH_2)_2-$, $-(CH_2)_6-N(R_{g11})-(CH_2)_3-$, $-(CH_2)_7-N(R_{g11})-(CH_2)_1-$, $-(CH_2)_7-N(R_{g11})-(CH_2)_2-$, $-(CH_2)_7-N(R_{g11})-(CH_2)_3-$, $-(CH_2)_8-N(R_{g11})-(CH_2)_1-$, $-(CH_2)_8-NR_{g11})-(CH_2)_2-$, $-(CH_2)_8-N(R_{g11})-(CH_2)_3-$, $-CH(CH_3)-N(R_{g11})-(CH_2)_1-$, $-CH(CH_3)-N(R_{g11})-(CH_2)_2-$,$-CH(CH_3)-N(R_{g11})-(CH_2)_3-$, $-CH(CH_3)-N(R_{g11})-(CH_2)_4-$, $-CH(CH_3)-N(R_{g11})-(CH_2)_5-$, $-CH(CH_3)-N(R_{g11})-(CH_2)_6-$, $-CH(CH_3)-N(R_{g11})-(CH_2)_7-$, $-CH(CH_3)-N(R_{g11})-(CH_2)_8-$, $-CH(CH_3)-N(R_{g11})-(CH_2)_9-$, $-CH(CH_3)-N(R_{g11})-(CH_2)_{10}-$, $-CH_2C(O)NHCH_2-$, $-(CH_2)_2C(O)NH(CH_2)_2-$, $-(CH_2)_2C(O)NH(CH_2)_3-$,$-(CH_2)_2C(O)NH(CH_2)_4-$, $-(CH_2)_2C(O)NH(CH_2)_5-$, $-(CH_2)_3C(O)NH(CH_2)_3-$, $-(CH_2)_3C(O)NH(CH_2)_4-$,$-(CH_2)_4C(O)NH(CH_2)_4-$, $-(CH_2)_5C(O)NH(CH_2)_5-$, $-(CH_2)_6C(O)NH(CH_2)_7-$, $-(CH_2)_6C(O)NH(CH_2)_6-$,$-(CH_2)_7C(O)NH(CH_2)_7-$, $-(CH_2)_8C(O)NH(CH_2)_8$, $-(CH_2)_9C(O)NH(CH_2)_9-$, $-(CH_2)_{10}C(O)NH(CH_2)_{10}-$,$-(CH_2)_2C(O)NH(CH_2)_2-O-(CH_2)_2-$, $-CH_2NHC(O)CH_2-$, $-(CH_2)_2NHC(O)(CH_2)_2-$,$-(CH_2)_2NHC(O)(CH_2)_3-$, $-(CH_2)_2NHC(O)(CH_2)_4-$, $-(CH_2)_2NHC(O)(CH_2)_5-$, $-(CH_2)_3NHC(O)(CH_2)_3-$,$-(CH_2)_3NHC(O)(CH_2)_4-$, $-(CH_2)_4NHC(O)(CH_2)_4-$, $-(CH_2)_5NHC(O)(CH_2)_5-$, $-(CH_2)_6NHC(O)(CH_2)_7-$,$-(CH_2)_6NHC(O)(CH_2)_6-$, $-(CH_2)_7NHC(O)(CH_2)_7-$, $-(CH_2)_8NHC(O)(CH_2)_8$, $-(CH_2)_9NHC(O)(CH_2)_9-$,$-(CH_2)_{10}NHC(O)(CH_2)_{10}-$, $-(CH_2)_4NHC(O)(CH_2)_8-$, $-(CH_2)_2NHC(O)(CH_2)_2-O-(CH_2)_2-$,$-(CH_2)_4NHC(O)CH_2-$, $-CH_2$-piperidinylene-$CH_2-$, $-CH_2$-piperidinylene-$(CH_2)_2-$, $-CH_2$-piperidinylene-$(CH_2)_3-$, $-CH_2$-piperidinylene-$(CH_2)_4-$, $-CH_2$-piperidinylene-$(CH_2)_5-$, $-CH_2$-piperidinylene-$(CH_2)_6-$,$-CH_2$-piperidinylene-$(CH_2)_7-$, $-CH_2$-piperidinylene-$(CH_2)_8-$, $-(CH_2)_2$-piperidinylene-$(CH_2)_1-$, $-(CH_2)_2$-piperidinylene-$(CH_2)_2-$, $-(CH_2)_2$-piperidinylene-$(CH_2)_3-$, $-(CH_2)_2$-piperidinylene-$(CH_2)_4-$, $-(CH_2)_2$-piperidinylene-$(CH_2)_5-$, $-(CH_2)_2$-piperidinylene-$(CH_2)_6-$, $-(CH_2)_2$-piperidinylene-$(CH_2)_7-$, $-(CH_2)_2$-piperidinylene-$(CH_2)_8-$, $-(CH_2)_3$-piperidinylene-$CH_2-$, $-(CH_2)_3$-piperidinylene-$(CH_2)_2-$, $-(CH_2)_3$-piperidinylene-$(CH_2)_3-$, $-(CH_2)_3$-piperidinylene-$(CH_2)_4-$, $-(CH_2)_3$-piperidinylene-$(CH_2)_5-$, $-(CH_2)_3$-piperidinylene-$(CH_2)_6-$, $-(CH_2)_3$-piperidinylene-$(CH_2)_7-$, $-(CH_2)_3$-piperidinylene-$(CH_2)_8-$, $-(CH_2)_4$-piperidinylene-$CH_2-$, $-(CH_2)_4$-piperidinylene-$(CH_2)_2-$, $-(CH_2)_4$-piperidinylene-$(CH_2)_3-$, $-(CH_2)_4$-piperidinylene-$(CH_2)_4-$, $-(CH_2)_4$-piperidinylene-$(CH_2)_5-$, $-(CH_2)_4$-piperidinylene-$(CH_2)_6-$, $-(CH_2)_4$-piperidinylene-$(CH_2)_7-$, $-(CH_2)_4$-piperidinylene-$(CH_2)_8-$, $-(CH_2)_5$-piperidinylene-$(CH_2)_1-$, $-(CH_2)_5$-piperidinylene-$(CH_2)_2-$, $-(CH_2)_5$-piperidinylene-$(CH_2)_3-$, $-(CH_2)_5$-piperidinylene-$(CH_2)_4-$, $-(CH_2)_5$-piperidinylene-$(CH_2)_5-$, $-(CH_2)_5$-piperidinylene-$(CH_2)_6-$, $-(CH_2)_5$-piperidinylene-$(CH_2)_7-$, $-(CH_2)_5$-piperidinylene-$(CH_2)_8-$, $-(CH_2)_6$-piperidinylene-$(CH_2)_1-$, $-(CH_2)_6$-piperidinylene-$(CH_2)_2-$, $-(CH_2)_6$-piperidinylene-$(CH_2)_3-$, $-(CH_2)_6$-piperidinylene-$(CH_2)_4-$, $-(CH_2)_6$-piperidinylene-$(CH_2)_5-$, $-(CH_2)_6$-piperidinylene-$(CH_2)_6-$, $-(CH_2)_6$-piperidinylene-$(CH_2)_7-$, $-(CH_2)_6$-piperidinylene-$(CH_2)_8-$, $-(CH_2)_7$-piperidinylene-$(CH_2)_1-$, $-(CH_2)_7$-piperidinylene-$(CH_2)_2-$, $-(CH_2)_7$-piperidinylene-$(CH_2)_3-$, $-(CH_2)_7$-piperidinylene-$(CH_2)_4-$, $-(CH_2)_7$-piperidinylene-$(CH_2)_8-$, $-(CH_2)_8$-piperidinylene-$CH_2-$, $-(CH_2)_8$-piperidinylene-$(CH_2)_2-$, $-(CH_2)_8$-piperidinylene-$(CH_2)_3-$, $-(CH_2)_8$-piperidinylene-$(CH_2)_4-$, $-(CH_2)_8$-piperidinylene-$(CH_2)_5-$, $-(CH_2)_8$-piperidinylene-$(CH_2)_6-$, $-(CH_2)_8$-piperidinylene-$(CH_2)_7-$, $-(CH_2)_8$-piperidinylene-$(CH_2)_8-$, $-CH_2$-piperazinylene-$CH_2-$, $-CH_2$-piperazinylene-$(CH_2)_2-$, $-CH_2$-piperazinylene-$(CH_2)_3-$, $-CH_2$-piperazinylene-$(CH_2)_4-$, $-CH_2$-piperazinylene-$(CH_2)_5-$, $-CH_2$-piperazinylene-$(CH_2)_6-$, $-CH_2$-piperazinylene-$(CH_2)_7-$, $-CH_2$-piperazinylene-$(CH_2)_8-$, $-(CH_2)_2$-piperazinylene-$(CH_2)_1-$, $-(CH_2)_2$-piperazinylene-$(CH_2)_2-$, $-(CH_2)_2$-piperazinylene-$(CH_2)_3-$, $-(CH_2)_2$-piperazinylene-$(CH_2)_4-$, $-(CH_2)_2$-piperazinylene-$(CH_2)_5-$, $-(CH_2)_2$-piperazinylene-$(CH_2)_6-$, $-(CH_2)_2$-piperazinylene-$(CH_2)_7-$, $-(CH_2)_2$-piperazinylene-$(CH_2)_8-$, $-(CH_2)_3$-piperazinylene-$CH_2-$, $-(CH_2)_3$-piperazinylene-$(CH_2)_2-$, $-(CH_2)_3$-piperazinylene-$(CH_2)_3-$, $-(CH_2)_3$-piperazinylene-$(CH_2)_4-$, $-(CH_2)_3$-piperazinylene-$(CH_2)_5-$, $-(CH_2)_3$-piperazinylene-$(CH_2)_6-$, $-(CH_2)_3$-piperazinylene-$(CH_2)_7-$, $-(CH_2)_3$-piperazinylene-$(CH_2)_8-$, $-(CH_2)_4$-piperazinylene-$CH_2-$, $-(CH_2)_4$-piperazinylene-$(CH_2)_2-$, $-(CH_2)_4$-piperazinylene-$(CH_2)_3-$, $-(CH_2)_4$-piperazinylene-$(CH_2)_4-$, $-(CH_2)_4$-piperazinylene-$(CH_2)_5-$, $-(CH_2)_4$-piperazinylene-$(CH_2)_6-$, $-(CH_2)_4$-piperazinylene-$(CH_2)_7-$, $-(CH_2)_4$-piperazinylene-$(CH_2)_8-$, $-(CH_2)_5$-piperazinylene-$(CH_2)_1-$, $-(CH_2)_5$-piperazinylene-$(CH_2)_2-$, $-(CH_2)_5$-piperazinylene-$(CH_2)_3-$, $-(CH_2)_5$-piperazinylene-$(CH_2)_4-$, $-(CH_2)_5$-piperazinylene-$(CH_2)_5-$, $-(CH_2)_5$-piperazinylene-$(CH_2)_6-$, $-(CH_2)_5$-piperazinylene-$(CH_2)_7-$, $-(CH_2)_5$-piperazinylene-$(CH_2)_8-$, $-(CH_2)_6$-piperazinylene-$(CH_2)_1-$, $-(CH_2)_6$-piperazinylene-$(CH_2)_2-$, $-(CH_2)_6$-

piperazinylene-$(CH_2)_3$-, -$(CH_2)_6$-piperazinylene-$(CH_2)_4$-, -$(CH_2)_6$-piperazinylene-$(CH_2)_5$-, -$(CH_2)_6$-piperazinylene-$(CH_2)_6$-, -$(CH_2)_6$-piperazinylene-$(CH_2)_7$-, -$(CH_2)_6$-piperazinylene-$(CH_2)_8$-, -$(CH_2)_7$-piperazinylene-$(CH_2)_1$-, -$(CH_2)_7$-piperazinylene-$(CH_2)_2$-, -$(CH_2)_7$-piperazinylene-$(CH_2)_3$-, -$(CH_2)_7$-piperazinylene-$(CH_2)_4$-, -$(CH_2)_7$-piperazinylene-$(CH_2)_8$-, -$(CH_2)_8$-piperazinylene-$CH_2$-, -$(CH_2)_8$-piperazinylene-$(CH_2)_2$-, -$(CH_2)_8$-piperazinylene-$(CH_2)_3$-, -$(CH_2)_8$-piperazinylene-$(CH_2)_4$-, -$(CH_2)_8$-piperazinylene-$(CH_2)_5$-, -$(CH_2)_8$-piperazinylene-$(CH_2)_6$-, -$(CH_2)_8$-piperazinylene-$(CH_2)_7$-, -$(CH_2)_8$-piperazinylene-$(CH_2)_8$-, -$CH_2$-propylene-$CH_2$-, -$CH_2$-propylene-$(CH_2)_2$-, -$CH_2$-propylene-$(CH_2)_3$-, -$CH_2$-propylene-$(CH_2)_4$-, -$CH_2$-propylene-$(CH_2)_5$-, -$CH_2$-propylene-$(CH_2)_6$-, -$CH_2$-propylene-$(CH_2)_7$-, -$CH_2$-propylene-$(CH_2)_8$-, -$(CH_2)_2$-propylene-$(CH_2)_1$-, -$(CH_2)_2$-propylene-$(CH_2)_2$-, -$(CH_2)_2$-propylene-$(CH_2)_3$-, -$(CH_2)_2$-propylene-$(CH_2)_4$-, -$(CH_2)_2$-propylene-$(CH_2)_5$-, -$(CH_2)_2$-propylene-$(CH_2)_6$-, -$(CH_2)_2$-propylene-$(CH_2)_7$-, -$(CH_2)_2$-propylene-$(CH_2)_8$-, -$(CH_2)_3$-propylene-$CH_2$-, -$(CH_2)_3$-propylene-$(CH_2)_2$-, -$(CH_2)_3$-propylene-$(CH_2)_3$-, -$(CH_2)_3$-propylene-$(CH_2)_4$-, - $(CH_2)_3$-propylene-$(CH_2)_5$-, -$(CH_2)_3$-propylene-$(CH_2)_6$-, -$(CH_2)_3$-propylene-$(CH_2)_7$-, -$(CH_2)_3$-propylene-$(CH_2)_8$-, -$(CH_2)_4$-propylene-$CH_2$-, -$(CH_2)_4$-propylene-$(CH_2)_2$-, -$(CH_2)_4$-propylene-$(CH_2)_3$-, -$(CH_2)_4$-propylene-$(CH_2)_4$-, -$(CH_2)_4$-propylene-$(CH_2)_5$-, -$(CH_2)_4$-propylene-$(CH_2)_6$-, -$(CH_2)_4$-propylene-$(CH_2)_7$-, -$(CH_2)_4$-propylene-$(CH_2)_8$-, -$(CH_2)_5$-propylene-$(CH_2)_1$-, -$(CH_2)_5$-propylene-$(CH_2)_2$-, -$(CH_2)_5$-propylene-$(CH_2)_3$-, -$(CH_2)_5$-propylene-$(CH_2)_4$-, -$(CH_2)_5$-propylene-$(CH_2)_5$-, -$(CH_2)_5$-propylene-$(CH_2)_6$-, -$(CH_2)_5$-propylene-$(CH_2)_7$-, -$(CH_2)_5$-propylene-$(CH_2)_8$-, -$(CH_2)_6$-propylene-$(CH_2)_1$-, -$(CH_2)_6$-propylene-$(CH_2)_2$-, -$(CH_2)_6$-propylene-$(CH_2)_3$-, -$(CH_2)_6$-propylene-$(CH_2)_4$-, -$(CH_2)_6$-propylene-$(CH_2)_5$-, -$(CH_2)_6$-propylene-$(CH_2)_6$-, -$(CH_2)_6$-propylene-$(CH_2)_7$-, -$(CH_2)_6$-propylene-$(CH_2)_8$-, -$(CH_2)_7$-propylene-$(CH_2)_1$-, -$(CH_2)_7$-propylene-$(CH_2)_2$-, -$(CH_2)_7$-propylene-$(CH_2)_3$-, -$(CH_2)_7$-propylene-$(CH_2)_4$-, -$(CH_2)_7$-propylene-$(CH_2)_8$-, -$(CH_2)_8$-propylene-$CH_2$-, -$(CH_2)_8$-propylene-$(CH_2)_2$-, -$(CH_2)_8$-propylene-$(CH_2)_3$-, -$(CH_2)_8$-propylene-$(CH_2)_4$-, -$(CH_2)_8$-propylene-$(CH_2)_5$-, -$(CH_2)_8$-propylene-$(CH_2)_6$-, -$(CH_2)_8$-propylene-$(CH_2)_7$-, -$(CH_2)_8$-propylene-$(CH_2)_8$-, -$CH_2$-diazacycloheptanylene-$CH_2$-, -$CH_2$-diazacycloheptanylene-$(CH_2)_2$-, -$CH_2$-diazacycloheptanylene-$(CH_2)_3$-, -$CH_2$-diazacycloheptanylene-$(CH_2)_4$-, -$CH_2$-diazacycloheptanylene-$(CH_2)_5$-, -$CH_2$-diazacycloheptanylene-$(CH_2)_6$-, -$CH_2$-diazacycloheptanylene-$(CH_2)_7$-, -$CH_2$-diazacycloheptanylene-$(CH_2)_8$-, -$(CH_2)_2$-diazacycloheptanylene-$(CH_2)_1$-, -$(CH_2)_2$-diazacycloheptanylene-$(CH_2)_2$-, -$(CH_2)_2$-diazacycloheptanylene-$(CH_2)_3$-, -$(CH_2)_2$-diazacycloheptanylene-$(CH_2)_4$-, -$(CH_2)_2$-diazacycloheptanylene-$(CH_2)_5$-, -$(CH_2)_2$-diazacycloheptanylene-$(CH_2)_6$-, -$(CH_2)_2$-diazacycloheptanylene-$(CH_2)_7$-, -$(CH_2)_2$-diazacycloheptanylene-$(CH_2)_8$-, -$(CH_2)_3$-diazacycloheptanylene-$CH_2$-, -$(CH_2)_3$-diazacycloheptanylene-$(CH_2)_2$-, -$(CH_2)_3$-diazacycloheptanylene-$(CH_2)_3$-, -$(CH_2)_3$-diazacycloheptanylene-$(CH_2)_4$-, -$(CH_2)_3$-diazacycloheptanylene-$(CH_2)_5$-, -$(CH_2)_3$-diazacycloheptanylene-$(CH_2)_6$-, -$(CH_2)_3$-diazacycloheptanylene-$(CH_2)_7$-, -$(CH_2)_3$-diazacycloheptanylene-$(CH_2)_8$-, -$(CH_2)_4$-diazacycloheptanylene-$CH_2$-, -$(CH_2)_4$-diazacycloheptanylene-$(CH_2)_2$-, -$(CH_2)_4$-diazacycloheptanylene-$(CH_2)_3$-, -$(CH_2)_4$-diazacycloheptanylene-$(CH_2)_4$-, -$(CH_2)_4$-diazacycloheptanylene-$(CH_2)_5$-, -$(CH_2)_4$-diazacycloheptanylene-$(CH_2)_6$-, -$(CH_2)_4$-diazacycloheptanylene-$(CH_2)_7$-, -$(CH_2)_4$-diazacycloheptanylene-$(CH_2)_8$-, -$(CH_2)_5$-diazacycloheptanylene-$(CH_2)_1$-, -$(CH_2)_5$-diazacycloheptanylene-$(CH_2)_2$-, -$(CH_2)_5$-diazacycloheptanylene-$(CH_2)_3$-, -$(CH_2)_5$-diazacycloheptanylene-$(CH_2)_4$-, -$(CH_2)_5$-diazacycloheptanylene-$(CH_2)_5$-, -$(CH_2)_5$-diazacycloheptanylene-$(CH_2)_6$-, -$(CH_2)_5$-diazacycloheptanylene-$(CH_2)_7$-, -$(CH_2)_5$-diazacycloheptanylene-$(CH_2)_8$-, -$(CH_2)_6$-diazacycloheptanylene-$(CH_2)_1$-, -$(CH_2)_6$-diazacycloheptanylene-$(CH_2)_2$-, -$(CH_2)_6$-diazacycloheptanylene-$(CH_2)_3$-, -$(CH_2)_6$-diazacycloheptanylene-$(CH_2)_4$-, -$(CH_2)_6$-diazacycloheptanylene-$(CH_2)_5$-, -$(CH_2)_6$-diazacycloheptanylene-$(CH_2)_6$-, -$(CH_2)_6$-diazacycloheptanylene-$(CH_2)_7$-, -$(CH_2)_6$-diazacycloheptanylene-$(CH_2)_8$-, -$(CH_2)_7$-diazacycloheptanylene-$(CH_2)_1$-, -$(CH_2)_7$-diazacycloheptanylene-$(CH_2)_2$-, -$(CH_2)_7$-diazacycloheptanylene-$(CH_2)_3$-, -$(CH_2)_7$-diazacycloheptanylene-$(CH_2)_4$-, -$(CH_2)_7$-diazacycloheptanylene-$(CH_2)_8$-, -$(CH_2)_8$-diazacycloheptanylene-$CH_2$-, -$(CH_2)_8$-diazacycloheptanylene-$(CH_2)_2$-, -$(CH_2)_8$-diazacycloheptanylene-$(CH_2)_3$-, -$(CH_2)_8$-diazacycloheptanylene-$(CH_2)_4$-, -$(CH_2)_8$-diazacycloheptanylene-$(CH_2)_5$-, -$(CH_2)_8$-diazacycloheptanylene-$(CH_2)_6$-, -$(CH_2)_8$-diazacycloheptanylene-$(CH_2)_7$-, -$(CH_2)_8$-diazacycloheptanylene-$(CH_2)_8$-, -$CH_2$-diazabicyclo[2.2.1]heptanylene-$CH_2$-, -$CH_2$-diazabicyclo[2.2.1]heptanylene-$(CH_2)_2$-, -$CH_2$-diazabicyclo[2.2.1]heptanylene-$(CH_2)_3$-, -$CH_2$-diazabicyclo[2.2.1]heptanylene-$(CH_2)_4$-, -$CH_2$-diazabicyclo[2.2.1]heptanylene-$(CH_2)_5$-, -$CH_2$-diazabicyclo[2.2.1]heptanylene-$(CH_2)_6$-, -$CH_2$-diazabicyclo[2.2.1]heptanylene-$(CH_2)_7$-, -$CH_2$-diazabicyclo[2.2.1]heptanylene-$(CH_2)_8$-, -$(CH_2)_2$-diazabicyclo[2.2.1]heptanylene-$(CH_2)_1$-, -$(CH_2)_2$-diazabicyclo[2.2.1]heptanylene-$(CH_2)_2$-, -$(CH_2)_2$-diazabicyclo[2.2.1]heptanylene-$(CH_2)_3$-, -$(CH_2)_2$-diazabicyclo[2.2.1]heptanylene-$(CH_2)_4$-, -$(CH_2)_2$-diazabicyclo[2.2.1]heptanylene-$(CH_2)_5$-, -$(CH_2)_2$-diazabicyclo[2.2.1]heptanylene-$(CH_2)_6$-, -$(CH_2)_2$-diazabicyclo[2.2.1]heptanylene-$(CH_2)_7$-, -$(CH_2)_2$-diazabicyclo[2.2.1]heptanylene-$(CH_2)_8$-, -$(CH_2)_3$-diazabicyclo[2.2.1]heptanylene-$CH_2$-, -$(CH_2)_3$-diazabicyclo[2.2.1]heptanylene-$(CH_2)_2$-, -$(CH_2)_3$-diazabicyclo[2.2.1]heptanylene-$(CH_2)_3$-, -$(CH_2)_3$-diazabicyclo[2.2.1]heptanylene-$(CH_2)_4$-, -$(CH_2)_3$-diazabicyclo[2.2.1]heptanylene-$(CH_2)_5$-, -$(CH_2)_3$-diazabicyclo[2.2.1]heptanylene-$(CH_2)_6$-, -$(CH_2)_3$-diazabicyclo[2.2.1]heptanylene-$(CH_2)_7$-, -$(CH_2)_3$-diazabicyclo[2.2.1]heptanylene-$(CH_2)_8$-, -$(CH_2)_4$-diazabicyclo[2.2.1]heptanylene-$CH_2$-, -$(CH_2)_4$-diazabicyclo[2.2.1]heptanylene-$(CH_2)_2$-, -$(CH_2)_4$-diazabicyclo[2.2.1]heptanylene-$(CH_2)_3$-, -$(CH_2)_4$-diazabicyclo[2.2.1]heptanylene-$(CH_2)_4$-, -$(CH_2)_4$-diazabicyclo[2.2.1]heptanylene-$(CH_2)_5$-, -$(CH_2)_4$-diazabicyclo[2.2.1]heptanylene-$(CH_2)_6$-, -$(CH_2)_4$-diazabicyclo[2.2.1]heptanylene-$(CH_2)_7$-, -$(CH_2)_4$-diazabicyclo[2.2.1]heptanylene-$(CH_2)_8$-, -$(CH_2)_5$-diazabicyclo[2.2.1]heptanylene-$(CH_2)_1$-, -$(CH_2)_5$-diazabicyclo[2.2.1]heptanylene-$(CH_2)_2$-, -$(CH_2)_5$-diazabicyclo[2.2.1]heptanylene-$(CH_2)_3$-, -$(CH_2)_5$-diazabicyclo[2.2.1]heptanylene-$(CH_2)_4$-, -$(CH_2)_5$-diazabicyclo[2.2.1]heptanylene-$(CH_2)_5$-, -$(CH_2)_5$-diazabicyclo[2.2.1]heptanylene-$(CH_2)_6$-, -$(CH_2)_5$-diazabicyclo[2.2.1]heptanyle-

ne-$(CH_2)_7$-, -$(CH_2)_5$-diazabicyclo[2.2.1]heptanylene-$(CH_2)_8$-, -$(CH_2)_6$-diazabicyclo[2.2.1]heptanylene-$(CH_2)_1$-, -$(CH_2)_6$-diazabicyclo[2.2.1]heptanylene-$(CH_2)_2$-, -$(CH_2)_6$-diazabicyclo[2.2.1]heptanylene-$(CH_2)_3$-, -$(CH_2)_6$-diazabicyclo[2.2.1]heptanylene-$(CH_2)_4$-, -$(CH_2)_6$-diazabicyclo[2.2.1]heptanylene-$(CH_2)_5$-, -$(CH_2)_6$-diazabicyclo[2.2.1]heptanylene-$(CH_2)_6$-, -$(CH_2)_6$-diazabicyclo[2.2.1]heptanylene-$(CH_2)_7$-, -$(CH_2)_6$-diazabicyclo[2.2.1]heptanylene-$(CH_2)_8$-, -$(CH_2)_7$-diazabicyclo[2.2.1]heptanylene-$(CH_2)$1-, -$(CH_2)_7$-diazabicyclo[2.2.1]heptanylene-$(CH_2)_2$-, -$(CH_2)_7$-diazabicyclo[2.2.1]heptanylene-$(CH_2)_3$-, -$(CH_2)_7$-diazabicyclo[2.2.1]heptanylene-$(CH_2)_4$-, -$(CH_2)_7$-diazabicyclo[2.2.1]heptanylene-$(CH_2)_8$-, -$(CH_2)_8$-diazabicyclo[2.2.1]heptanylene-$CH_2$-, -$(CH_2)_8$-diazabicyclo[2.2.1]heptanylene-$(CH_2)_2$-, -$(CH_2)_8$-diazabicyclo[2.2.1]heptanylene-$(CH_2)_3$-, -$(CH_2)_8$-diazabicyclo[2.2.1]heptanylene-$(CH_2)_4$-, -$(CH_2)_8$-diazabicyclo[2.2.1]heptanylene-$(CH_2)_5$-, -$(CH_2)_8$-diazabicyclo[2.2.1]heptanylene-$(CH_2)_6$-, -$(CH_2)_8$-diazabicyclo[2.2.1]heptanylene-$(CH_2)_7$-, -$(CH_2)_8$-diazabicyclo[2.2.1]heptanylene-$(CH_2)_8$-, -$CH_2$-diazabicyclo[3.1.1]heptanylene-$CH_2$-, -$CH_2$-diazabicyclo[3.1.1]heptanylene-$(CH_2)_2$-, -$CH_2$-diazabicyclo[3.1.1]heptanylene-$(CH_2)_3$-, -$CH_2$-diazabicyclo[3.1.1]heptanylene-$(CH_2)_4$-, -$CH_2$-diazabicyclo[3.1.1]heptanylene-$(CH_2)_5$-, -$CH_2$-diazabicyclo[3.1.1]heptanylene-$(CH_2)_6$-, -$CH_2$-diazabicyclo[3.1.1]heptanylene-$(CH_2)_7$-, -$CH_2$-diazabicyclo[3.1.1]heptanylene-$(CH_2)_8$-, -$(CH_2)_2$-diazabicyclo[3.1.1]heptanylene-$(CH_2)_1$-, -$(CH_2)_2$-diazabicyclo[3.1.1]heptanylene-$(CH_2)_2$-, -$(CH_2)_2$-diazabicyclo[3.1.1]heptanylene-$(CH_2)_3$-, -$(CH_2)_2$-diazabicyclo[3.1.1]heptanylene-$(CH_2)_4$-, -$(CH_2)_2$-diazabicyclo[3.1.1]heptanylene-$(CH_2)_5$-, -$(CH_2)_2$-diazabicyclo[3.1.1]heptanylene-$(CH_2)_6$-, -$(CH_2)_2$-diazabicyclo[3.1.1]heptanylene-$(CH_2)_7$-, -$(CH_2)_2$-diazabicyclo[3.1.1]heptanylene-$(CH_2)_8$-, -$(CH_2)_3$-diazabicyclo[3.1.1]heptanylene-$CH_2$-, -$(CH_2)_3$-diazabicyclo[3.1.1]heptanylene-$(CH_2)_2$-, -$(CH_2)_3$-diazabicyclo[3.1.1]heptanylene-$(CH_2)_3$-, -$(CH_2)_3$-diazabicyclo[3.1.1 ]heptanylene-$(CH_2)_4$-, -$(CH_2)_3$-diazabicyclo[3.1.1]heptanylene-$(CH_2)_5$-, -$(CH_2)_3$-diazabicyclo[3.1.1]heptanylene-$(CH_2)_6$-, -$(CH_2)_3$-diazabicyclo[3.1.1]heptanylene-$(CH_2)_7$-, -$(CH_2)_3$-diazabicyclo[3.1.1]heptanylene-$(CH_2)_8$-, -$(CH_2)_4$-diazabicyclo[3.1.1]heptanylene-$CH_2$-, -$(CH_2)_4$-diazabicyclo[3.1.1]heptanylene-$(CH_2)_2$-, -$(CH_2)_4$-diazabicyclo[3.1.1]heptanylene-$(CH_2)_3$-, -$(CH_2)_4$-diazabicyclo[3.1.1]heptanylene-$(CH_2)_4$-, -$(CH_2)_4$-diazabicyclo[3.1.1]heptanylene-$(CH_2)_5$-, -$(CH_2)_4$-diazabicyclo[3.1.1]heptanylene-$(CH_2)_6$-, -$(CH_2)_4$-diazabicyclo[3.1.1]heptanylene-$(CH_2)_7$-, -$(CH_2)_4$-diazabicyclo[3.1.1]heptanylene-$(CH_2)_8$-, -$(CH_2)_5$-diazabicyclo[3.1.1]heptanylene-$(CH_2)_1$-, -$(CH_2)_5$-diazabicyclo[3.1.1]heptanylene-$(CH_2)_2$-, -$(CH_2)_5$-diazabicyclo[3.1.1]heptanylene-$(CH_2)_3$-, -$(CH_2)_5$-diazabicyclo[3.1.1]heptanylene-$(CH_2)_4$-, -$(CH_2)_5$-diazabicyclo[3.1.1]heptanylene-$(CH_2)_5$-, -$(CH_2)_5$-diazabicyclo[3.1.1]heptanylene-$(CH_2)_6$-, -$(CH_2)_5$-diazabicyclo[3.1.1]heptanylene-$(CH_2)_7$-, -$(CH_2)_5$-diazabicyclo[3.1.1]heptanylene-$(CH_2)_8$-, -$(CH_2)_6$-diazabicyclo[3.1.1]heptanylene-$(CH_2)_1$-, -$(CH_2)_6$-diazabicyclo[3.1.1]heptanylene-$(CH_2)_2$-, -$(CH_2)_6$-diazabicyclo[3.1.1]heptanylene-$(CH_2)_3$-, -$(CH_2)_6$-diazabicyclo[3.1.1]heptanylene-$(CH_2)_4$-, -$(CH_2)_6$-diazabicyclo[3.1.1]heptanylene-$(CH_2)_5$-, -$(CH_2)_6$-diazabicyclo[3.1.1]heptanylene-$(CH_2)_6$-, -$(CH_2)_6$-diazabicyclo[3. 1. 1]heptanylene-$(CH_2)_7$-, -$(CH_2)_6$-diazabicyclo[3.1.1]heptanylene-$(CH_2)_8$-, -$(CH_2)_7$-diazabicyclo[3. 1. 1]heptanylene-$(CH_2)_1$-, -$(CH_2)_7$-diazabicyclo[3.1.1]heptanylene-$(CH_2)_2$-, -$(CH_2)_7$-diazabicyclo[3.1.1]heptanylene-$(CH_2)_3$-, -$(CH_2)_7$-diazabicyclo[3.1.1]heptanylene-$(CH_2)_4$-, -$(CH_2)_7$-diazabicyclo[3.1.1]heptanylene-$(CH_2)_8$-, -$(CH_2)_8$-diazabicyclo[3.1.1]heptanylene-$CH_2$-, -$(CH_2)_8$-diazabicyclo[3.1.1]heptanylene-$(CH_2)_2$-, -$(CH_2)_8$-diazabicyclo[3.1.1]heptanylene-$(CH_2)_3$-, -$(CH_2)_8$-diazabicyclo[3.1.1]heptanylene-$(CH_2)_4$-, -$(CH_2)_8$-diazabicyclo[3.1.1]heptanylene-$(CH_2)_5$-, -$(CH_2)_8$-diazabicyclo[3.1.1]heptanylene-$(CH_2)_6$-, -$(CH_2)_8$-diazabicyclo[3.1.1]heptanylene-$(CH_2)_7$-, -$(CH_2)_8$-diazabicyclo[3.1.1]heptanylene-$(CH_2)_8$-, -$CH_2$-diazabicyclo[3.2.1]octylene-$CH_2$-, -$CH_2$-diazabicyclo[3.2.1]octylene-$(CH_2)_2$-, -$CH_2$-diazabicyclo[3.2.1]octylene-$(CH_2)_3$-, -$CH_2$-diazabicyclo[3.2.1]octylene-$(CH_2)_4$-, -$CH_2$-diazabicyclo[3.2.1]octylene-$(CH_2)_5$-, -$CH_2$-diazabicyclo[3.2.1]octylene-$(CH_2)_6$-, -$CH_2$-diazabicyclo[3.2.1]octylene-$(CH_2)_7$-, -$CH_2$-diazabicyclo[3.2.1]octylene-$(CH_2)_8$-, -$(CH_2)_2$-diazabicyclo[3.2.1]octylene-$(CH_2)_1$-, -$(CH_2)_2$-diazabicyclo[3.2.1]octylene-$(CH_2)_2$-, -$(CH_2)_2$-diazabicyclo[3.2.1]octylene-$(CH_2)_3$-, -$(CH_2)_2$-diazabicyclo[3.2.1]octylene-$(CH_2)_4$-, -$(CH_2)_2$-diazabicyclo[3.2.1]octylene-$(CH_2)_5$-, -$(CH_2)_2$-diazabicyclo[3.2.1]octylene-$(CH_2)_6$-, -$(CH_2)_2$-diazabicyclo[3.2.1]octylene-$(CH_2)_7$-, -$(CH_2)_2$-diazabicyclo[3.2.1]octylene-$(CH_2)_8$-, -$(CH_2)_3$-diazabicyclo[3.2.1]octylene-$CH_2$-, -$(CH_2)_3$-diazabicyclo[3.2.1]octylene-$(CH_2)_2$-, -$(CH_2)_3$-diazabicyclo[3.2.1]octylene-$(CH_2)_3$-, -$(CH_2)_3$-diazabicyclo[3.2.1]octylene-$(CH_2)_4$-, -$(CH_2)_3$-diazabicyclo[3.2.1]octylene-$(CH_2)_5$-, -$(CH_2)_3$-diazabicyclo[3.2.1]octylene-$(CH_2)_6$-, -$(CH_2)_3$-diazabicyclo[3.2.1]octylene-$(CH_2)_7$-, -$(CH_2)_3$-diazabicyclo[3.2.1]octylene-$(CH_2)_8$-, -$(CH_2)_4$-diazabicyclo[3.2.1]octylene-$CH_2$-, -$(CH_2)_4$-diazabicyclo[3.2.1]octylene-$(CH_2)_2$-, -$(CH_2)_4$-diazabicyclo[3.2.1]octylene-$(CH_2)_3$-, -$(CH_2)_4$-diazabicyclo[3.2.1]octylene-$(CH_2)_4$-, -$(CH_2)_4$-diazabicyclo[3.2.1]octylene-$(CH_2)_5$-, -$(CH_2)_4$-diazabicyclo[3.2.1]octylene-$(CH_2)_6$-, -$(CH_2)_4$-diazabicyclo[3.2.1]octylene-$(CH_2)_7$-, -$(CH_2)_4$-diazabicyclo[3.2.1]octylene-$(CH_2)_8$-, -$(CH_2)_5$-diazabicyclo[3.2.1]octylene-$(CH_2)_1$-, -$(CH_2)_5$-diazabicyclo[3.2.1]octylene-$(CH_2)_2$-, -$(CH_2)_5$-diazabicyclo[3.2.1]octylene-$(CH_2)_3$-, -$(CH_2)_5$-diazabicyclo[3.2.1]octylene-$(CH_2)_4$-, -$(CH_2)_5$-diazabicyclo[3.2.1]octylene-$(CH_2)_5$-, -$(CH_2)_5$-diazabicyclo[3.2.1]octylene-$(CH_2)_6$-, -$(CH_2)_5$-diazabicyclo[3.2.1]octylene-$(CH_2)_7$-, -$(CH_2)_5$-diazabicyclo[3.2.1]octylene-$(CH_2)_8$-, -$(CH_2)_6$-diazabicyclo[3.2.1]octylene-$(CH_2)_1$-, -$(CH_2)_6$-diazabicyclo[3.2.1]octylene-$(CH_2)_2$-, -$(CH_2)_6$-diazabicyclo[3.2.1]octylene-$(CH_2)_3$-, -$(CH_2)_6$-diazabicyclo[3.2.1]octylene-$(CH_2)_4$-, -$(CH_2)_6$-diazabicyclo[3.2.1]octylene-$(CH_2)_5$-, -$(CH_2)_6$-diazabicyclo[3.2.1]octylene-$(CH_2)_6$-, -$(CH_2)_6$-diazabicyclo[3.2.1]octylene-$(CH_2)_7$-, -$(CH_2)_6$-diazabicyclo[3.2.1]octylene-$(CH_2)_8$-, -$(CH_2)_7$-diazabicyclo[3.2.1]octylene-$(CH_2)_1$-, -$(CH_2)_7$-diazabicyclo[3.2.1]octylene-$(CH_2)_2$-, -$(CH_2)_7$-diazabicyclo[3.2.1]octylene-$(CH_2)_3$-, -$(CH_2)_7$-diazabicyclo[3.2.1]octylene-$(CH_2)_4$-, -$(CH_2)_7$-diazabicyclo[3.2.1]octylene-$(CH_2)_8$-, -$(CH_2)_8$-diazabicyclo[3.2.1]octylene-$CH_2$-, -$(CH_2)_8$-diazabicyclo[3.2.1]octylene-$(CH_2)_2$-, -$(CH_2)_8$-diazabicyclo[3.2.1]octylene-$(CH_2)_3$-, -$(CH_2)_8$-diazabicyclo[3.2.1]octylene-$(CH_2)_4$-, -$(CH_2)_8$-diazabicyclo[3.2.1]octylene-$(CH_2)_5$-,

$-(CH_2)_8$-diazabicyclo[3.2.1]octylene-$(CH_2)_6$-, $-(CH_2)_8$-diazabicyclo[3.2.1]octylene-$(CH_2)_7$-, $-(CH_2)_8$-diazabicyclo[3.2.1]octylene-$(CH_2)_8$-, $-CH_2$-diazabicyclo[2.2.2]octylene-$CH_2$-, $-CH_2$-diazabicyclo[2.2.2]octylene-$(CH_2)_2$-, $-CH_2$-diazabicyclo[2.2.2]octylene-$(CH_2)_3$-, $-CH_2$-diazabicyclo[2.2.2]octylene-$(CH_2)_4$-, $-CH_2$-diazabicyclo[2.2.2]octylene-$(CH_2)_5$-, $-CH_2$-diazabicyclo[2.2.2]octylene-$(CH_2)_6$-, $-CH_2$-diazabicyclo[2.2.2]octylene-$(CH_2)_7$-, $-CH_2$-diazabicyclo[2.2.2]octylene-$(CH_2)_8$-, $-(CH_2)_2$-diazabicyclo[2.2.2]octylene-$(CH_2)_1$-, $-(CH_2)_2$-diazabicyclo[2.2.2]octylene-$(CH_2)_2$-, $-(CH_2)_2$-diazabicyclo[2.2.2]octylene-$(CH_2)_3$-, $-(CH_2)_2$-diazabicyclo[2.2.2]octylene-$(CH_2)_4$-, $-(CH_2)_2$-diazabicyclo[2.2.2]octylene-$(CH_2)_5$-, $-(CH_2)_2$-diazabicyclo[2.2.2]octylene-$(CH_2)_6$-, $-(CH_2)_2$-diazabicyclo[2.2.2]octylene-$(CH_2)_7$-, $-(CH_2)_2$-diazabicyclo[2.2.2]octylene-$(CH_2)_8$-, $-(CH_2)_3$-diazabicyclo[2.2.2]octylene-$CH_2$-, $-(CH_2)_3$-diazabicyclo[2.2.2]octylene-$(CH_2)_2$-, $-(CH_2)_3$-diazabicyclo[2.2.2]octylene-$(CH_2)_3$-, $-(CH_2)_3$-diazabicyclo[2.2.2]octylene-$(CH_2)_4$-, $-(CH_2)_3$-diazabicyclo[2.2.2]octylene-$(CH_2)_5$-, $-(CH_2)_3$-diazabicyclo[2.2.2]octylene-$(CH_2)_6$-, $-(CH_2)_3$-diazabicyclo[2.2.2]octylene-$(CH_2)_7$-, $-(CH_2)_3$-diazabicyclo[2.2.2]octylene-$(CH_2)_8$-, $-(CH_2)_4$-diazabicyclo[2.2.2]octylene-$CH_2$-, $-(CH_2)_4$-diazabicyclo[2.2.2]octylene-$(CH_2)_2$-, $-(CH_2)_4$-diazabicyclo[2.2.2]octylene-$(CH_2)_3$-, $-(CH_2)_4$-diazabicyclo[2.2.2]octylene-$(CH_2)_4$-, $-(CH_2)_4$-diazabicyclo[2.2.2]octylene-$(CH_2)_5$-, $-(CH_2)_4$-diazabicyclo[2.2.2]octylene-$(CH_2)_6$-, $-(CH_2)_4$-diazabicyclo[2.2.2]octylene-$(CH_2)_7$-, $-(CH_2)_4$-diazabicyclo[2.2.2]octylene-$(CH_2)_8$-, $-(CH_2)_5$-diazabicyclo[2.2.2]octylene-$(CH_2)_1$-, $-(CH_2)_5$-diazabicyclo[2.2.2]octylene-$(CH_2)_2$-, $-(CH_2)_5$-diazabicyclo[2.2.2]octylene-$(CH_2)_3$-, $-(CH_2)_5$-diazabicyclo[2.2.2]octylene-$(CH_2)_4$-, $-(CH_2)_5$-diazabicyclo[2.2.2]octylene-$(CH_2)_5$-, $-(CH_2)_5$-diazabicyclo[2.2.2]octylene-$(CH_2)_6$-, $-(CH_2)_5$-diazabicyclo[2.2.2]octylene-$(CH_2)_7$-, $-(CH_2)_5$-diazabicyclo[2.2.2]octylene-$(CH_2)_8$-, $-(CH_2)_6$-diazabicyclo[2.2.2]octylene-$(CH_2)_1$-, $-(CH_2)_6$-diazabicyclo[2.2.2]octylene-$(CH_2)_2$-, $-(CH_2)_6$-diazabicyclo[2.2.2]octylene-$(CH_2)_3$-, $-(CH_2)_6$-diazabicyclo[2.2.2]octylene-$(CH_2)_4$-, $-(CH_2)_6$-diazabicyclo[2.2.2]octylene-$(CH_2)_5$-, $-(CH_2)_6$-diazabicyclo[2.2.2]octylene-$(CH_2)_6$-, $-(CH_2)_6$-diazabicyclo[2.2.2]octylene-$(CH_2)_7$-, $-(CH_2)_6$-diazabicyclo[2.2.2]octylene-$(CH_2)_8$-, $-(CH_2)_7$-diazabicyclo[2.2.2]octylene-$(CH_2)_1$-, $-(CH_2)_7$-diazabicyclo[2.2.2]octylene-$(CH_2)_2$-, $-(CH_2)_7$-diazabicyclo[2.2.2]octylene-$(CH_2)_3$-, $-(CH_2)_7$-diazabicyclo[2.2.2]octylene-$(CH_2)_4$-, $-(CH_2)_7$-diazabicyclo[2.2.2]octylene-$(CH_2)_8$-, $-(CH_2)_8$-diazabicyclo[2.2.2]octylene-$CH_2$-, $-(CH_2)_8$-diazabicyclo[2.2.2]octylene-$(CH_2)_2$-, $-(CH_2)_8$-diazabicyclo[2.2.2]octylene-$(CH_2)_3$-, $-(CH_2)_8$-diazabicyclo[2.2.2]octylene-$(CH_2)_4$-, $-(CH_2)_8$-diazabicyclo[2.2.2]octylene-$(CH_2)_5$-, $-(CH_2)_8$-diazabicyclo[2.2.2]octylene-$(CH_2)_6$-, $-(CH_2)_8$-diazabicyclo[2.2.2]octylene-$(CH_2)_7$-, or $-(CH_2)_8$-diazabicyclo[2.2.2]octylene-$(CH_2)_8$-,

wherein the propylene, the piperidinylene, the piperazinylene, the diazacycloheptanylene, the diazabicyclo[2.2.1]heptanylene, the diazabicyclo[3.1.1]heptanylene, the diazabicyclo[3.2.1]octylene, the diazabicyclo[2.2.2]octylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof; and

each $R_{g11}$ independently represents H or $C_{1-3}$ alkyl.

In some embodiments of the compound of Formula (I-6), $L_2$ represents the structure of the following formula:

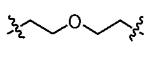 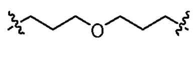 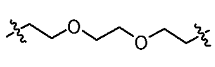 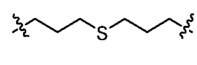 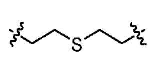 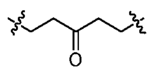

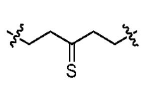 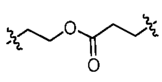 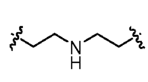 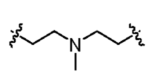 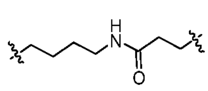 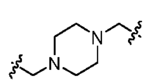 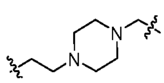

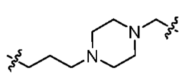 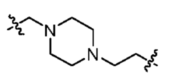 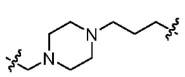 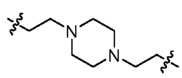 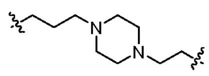 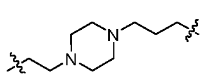

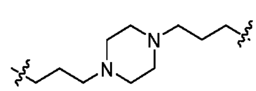 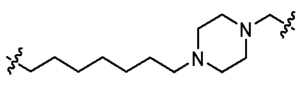 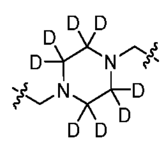 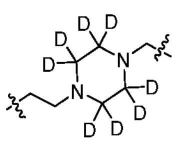 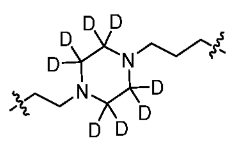

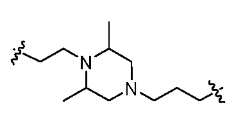 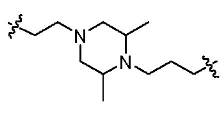 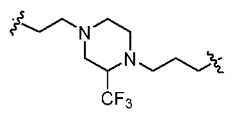 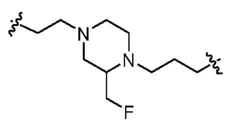 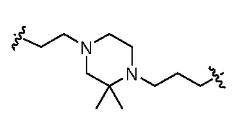

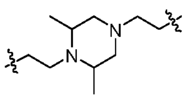 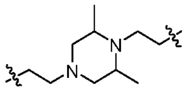 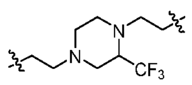 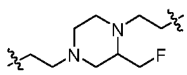 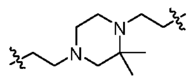 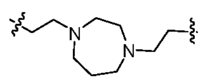

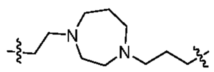 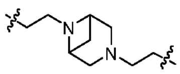 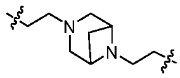 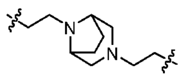 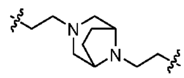 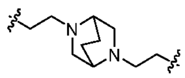

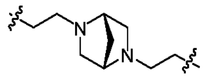 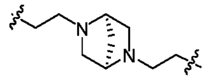 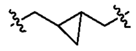 or 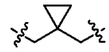

In some embodiments of the compound of Formula (I-6), $R_{b1}$ represents:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, 2-oxobicyclo[2.2.1]heptanyl or bicyclo[2.2.1]heptenyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 3-azaspiro[5.5]undecyl or 7-azaspiro[3.5]nonyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

phenyl or naphthyl, with each group being optionally substituted with one or more (e.g., 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo [2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl and imidazo[2,1-b]thiazolyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

In some embodiments of the compound of Formula (I-6), $R_{b1}$ represents:

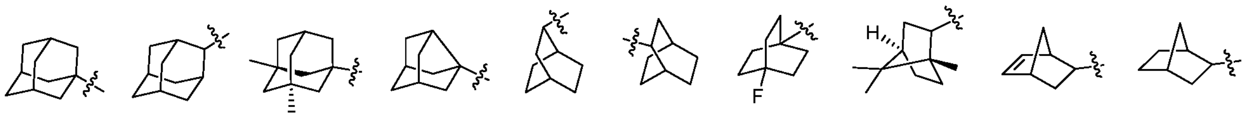

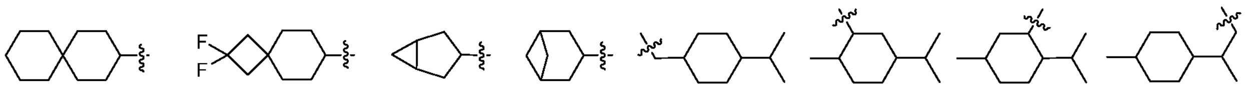

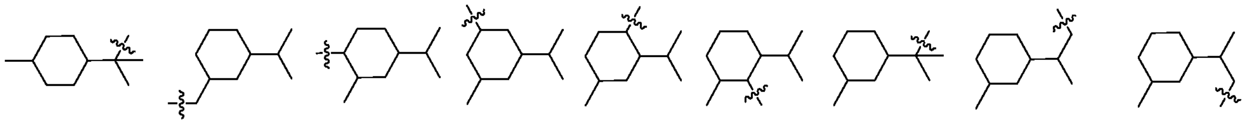

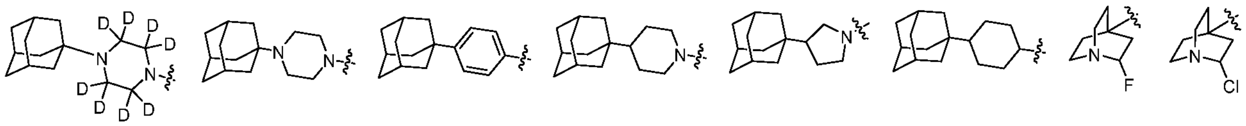

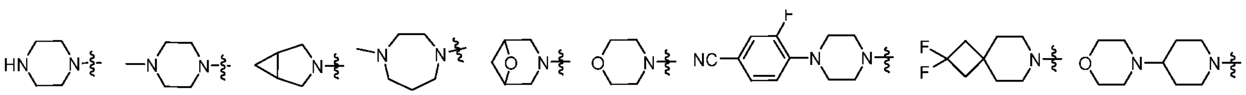

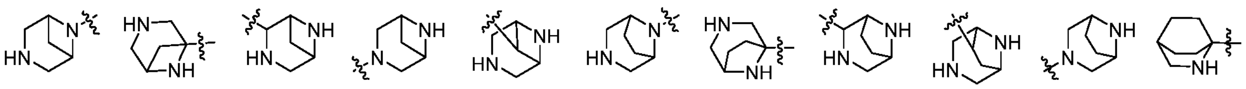

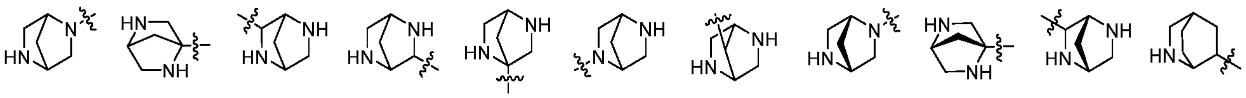

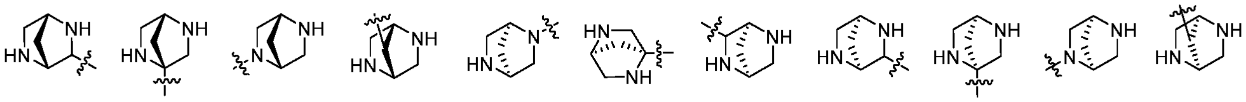

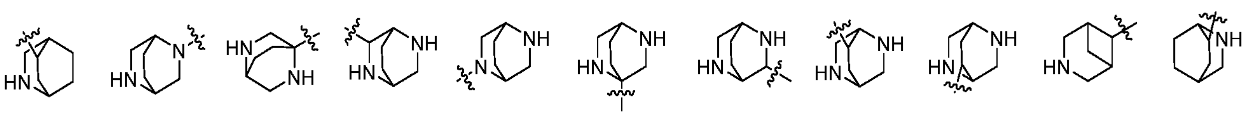

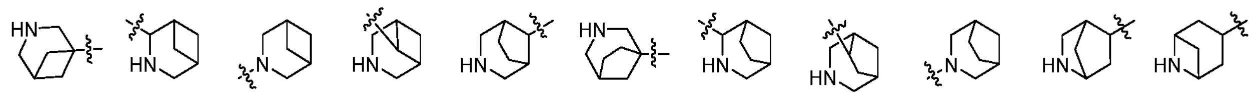

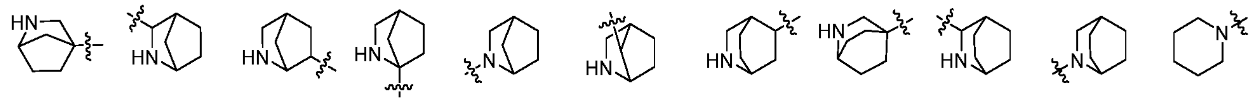

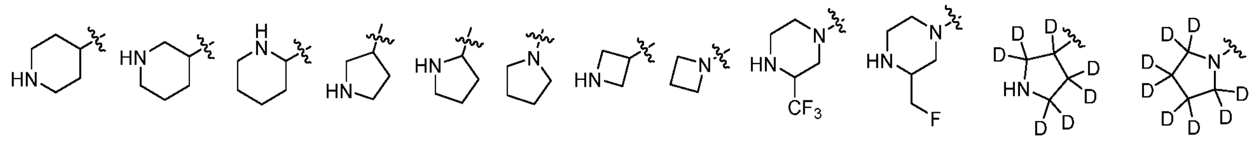

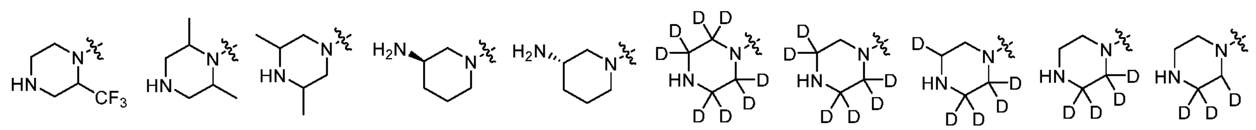

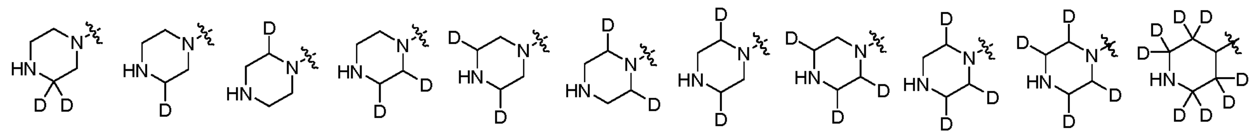

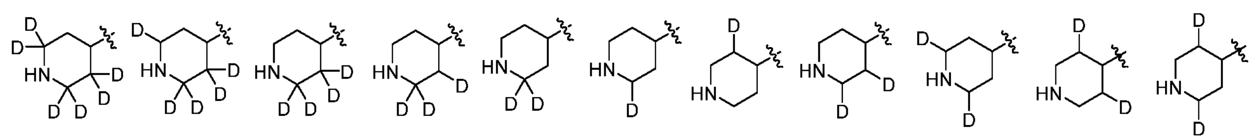

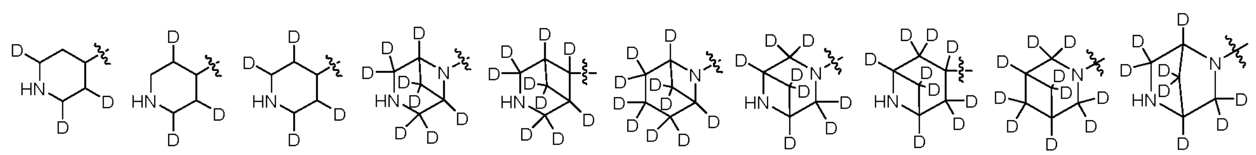

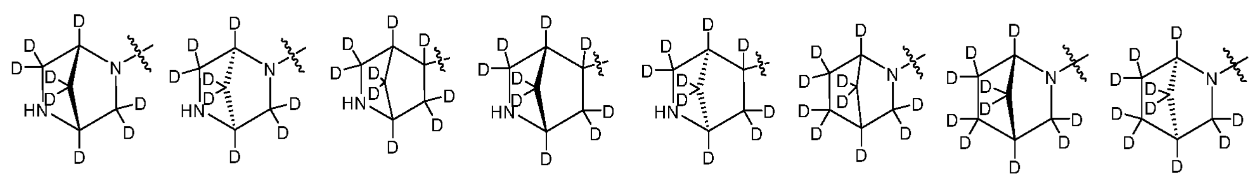

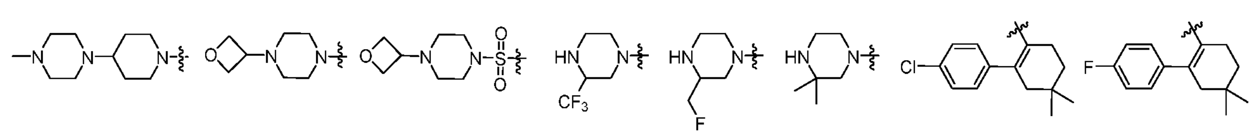

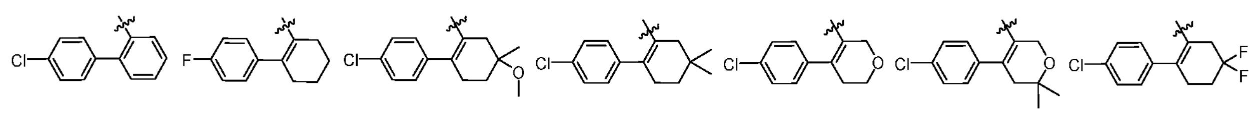

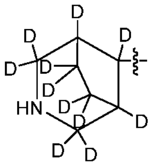 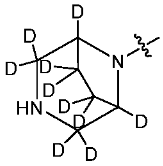 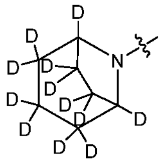 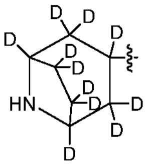 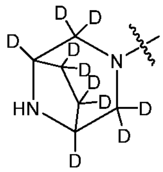 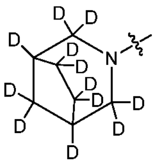 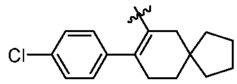

or

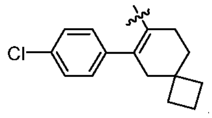

In some embodiments, the compound of Formula (I) is also of Formula (I-7):

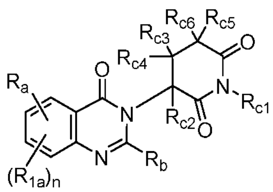

Formula (I-7)

wherein $R_{c1}$, $R_{c2}$, $R_{c3}$, $R_{c4}$, $R_{c5}$, $R_{c6}$, $R_b$, $(R_{1a})_n$, $R_a$, $R_{1a}$ and n are as defined in the compound of Formula (I) above and the embodiments thereof;

wherein $R_a$ represents the following formula:

$$R_{a2}\text{-}X_3\text{-} ,$$

wherein $X_3$ represents $N(R_{11})$, C(O)O, OC(O), C(O)NH, NHC(O), O, S, optionally substituted cycloalkylene (e.g., optionally substituted $C_{3-20}$cycloalkylene or optionally substituted $C_{3-15}$cycloalkylene), optionally substituted arylene (e.g., optionally substituted $C_{6-10}$ arylene), optionally substituted heteroarylene (e.g., optionally substituted 5- to 20-membered heteroarylene, or optionally substituted 5- to 15-membered heteroarylene) or optionally substituted heterocyclylene (e.g., optionally substituted 4- to 20-membered heterocyclylene, or optionally substituted 5- to 15-membered heterocyclylene), wherein $R_{11}$ represents H or $C_{1-3}$ alkyl, and

$R_{a2}$ represents optionally substituted cycloalkyl (e.g., optionally substituted $C_{3-20}$cycloalkyl, or optionally substituted $C_{3-15}$cycloalkyl), optionally substituted heterocyclyl (e.g., optionally substituted 4- to 20-membered heterocyclyl, or optionally substituted 4- to 15-membered heterocyclyl), optionally substituted heteroaryl (e.g., optionally substituted 5- to 20-membered heteroaryl, or optionally substituted 5- to 15-membered heteroaryl) or optionally substituted aryl (e.g., optionally substituted $C_{6-10}$ aryl);

and

$R_b$ represents $C_{1-60}$ alkyl (e.g., $C_1$-$C_{30}$ alkyl, $C_1$-$C_{29}$ alkyl, $C_1$-$C_{28}$ alkyl, $C_1$-$C_{27}$ alkyl, $C_1$-$C_{26}$ alkyl, $C_1$-$C_{25}$ alkyl, $C_1$-$C_{24}$ alkyl, $C_1$-$C_{23}$ alkyl, $C_1$-$C_{22}$ alkyl, $C_1$-$C_{21}$ alkyl, $C_1$-$C_{20}$ alkyl, $C_1$-$C_{19}$ alkyl, $C_1$-$C_{18}$ alkyl, $C_1$-$C_{17}$ alkyl, $C_1$-$C_{16}$ alkyl, $C_1$-$C_{15}$ alkyl, $C_1$-$C_{14}$ alkyl, $C_1$-$C_{13}$ alkyl, $C_1$-$C_{12}$ alkyl, $C_1$-$C_{11}$ alkyl, $C_1$-$C_{10}$ alkyl, $C_1$-$C_9$ alkyl, $C_1$-$C_8$ alkyl, $C_1$-$C_7$ alkyl, $C_1$-$C_6$ alkyl, $C_1$-$C_5$ alkyl, $C_1$-$C_4$ alkyl, $C_1$-$C_3$ alkyl, or $C_1$-$C_2$ alkyl) optionally substituted with D or halogen, or $R_b$ represents the following formula:

$$R_{b1}\text{-}X_4\text{-}L_2\text{-},$$

wherein $L_2$ represents:

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$

alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene), or

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{12}$ and/or one or more (e.g., 1-20, or 1-15, 1-10, 1-9, 1-8, 1-7, 1-6, 1-5, 1-4, 1-3 or 1-2, or 1) groups $R_{13}$ and/or any combination of one or more (e.g., 1-20, or 1-15, 1-10, 1-9, 1-8, 1-7, 1-6, 1-5, 1-4, 1-3 or 1-2, or 1) groups $R_{12}$ and $R_{13}$ inserted into its backbone carbon chain, wherein carbon-carbon bond between one or more pairs (e.g., 1-20, or 1-15, 1-10, 1-9, 1-8, 1-7, 1-6, 1-5, 1-4, 1-3 or 1-2, or 1 pair) of adj acent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of cycloalkylene (e.g., $C_{3-20}$cycloalkylene or $C_{3-15}$cycloalkylene)), heterocyclylene (e.g., 4- to 20-membered heterocyclylene, or 4- to 15-membered heterocyclylene), arylene (e.g., $C_{6-15}$ arylene or $C_{6-10}$ arylene), heteroarylene (e.g., 5-to 20-membered heteroarylene, or 5- to 15-membered heteroarylene), alkenylene (e.g., $C_{2-6}$ alkenylene), alkynylene (e.g., $C_{2-6}$ alkynylene) or any combination thereof, wherein the cycloalkylene, the heterocyclylene, arylene and the heteroarylene are each independently optionally substituted with a substituent selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $C1_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), cyano or any combination thereof;

$X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted cycloalkylene (e.g., optionally substituted $C_{3-20}$cycloalkylene, or optionally substituted $C_{3-15}$cycloalkylene), optionally substituted arylene (e.g., optionally substituted $C_{6-20}$ arylene, or optionally substituted $C_{6-10}$ arylene), optionally substituted heterocyclylene (e.g., optionally substituted 4- to 20-membered heterocyclylene, or optionally substituted 4- to 15-membered heterocyclylene), optionally substituted heteroarylene (e.g., optionally substituted 5- to 20-membered heteroarylene, or optionally substituted 5- to 15-membered heteroarylene), triazolylene-NH-, or -NH-triazolylene, wherein $R_{15}$ represents H or $C_{1-3}$ alkyl, or $X_4$ represents a bond; and

$R_{b1}$ represents (e.g., optionally substituted $C_{3-20}$cycloalkyl, or optionally substituted $C_{3-15}$cycloalkyl), optionally substituted aryl (e.g., optionally substituted $C_{6-20}$ aryl, or optionally substituted $C_{6-10}$ aryl), optionally substituted heterocyclyl (e.g., optionally substituted 4- to 20-membered heterocyclyl, or optionally substituted 4- to 15-membered heterocyclyl) or optionally substituted heteroaryl (e.g., optionally substituted 5- to 20-membered heteroaryl, or optionally substituted 5- to 15-membered heteroaryl).

In some embodiments of the compound of Formula (I-7), $R_a$ represents the following formula:

$$R_{a2}\text{-}X_3\text{-} ,$$

wherein $X_3$ represents $N(R_{11})$, C(O)O, OC(O), C(O)NH, NHC(O), O, S, optionally substituted $C_{3-20}$cycloalkylene, optionally substituted $C_{6-20}$ aryl, optionally substituted 5- to 20-membered heteroaryl or optionally substituted 4- to 20-membered heterocyclylene, wherein $R_{11}$ represents H or $C_{1-3}$ alkyl, and wherein the $C_{3-20}$cycloalkylene and the 4- to 20-membered heterocyclylene are each independently optionally substituted with 1 to 8 substituents selected from

the group consisting of D, halogen, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), oxo, or any combination thereof, and the $C_{6-20}$ aryl and the 5- to 20-membered heteroaryl are each independently optionally substituted with 1 to 8 substituents selected from the group consisting of D, halogen, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), or any combination thereof, and

$R_{a2}$ represents optionally substituted $C_{3-20}$cycloalkyl, optionally substituted 4- to 20-membered heterocyclyl, optionally substituted 5- to 20-membered heteroaryl or optionally substituted $C_{6-20}$ aryl, wherein the $C_{3-20}$cycloalkyl, the $C_{6-20}$ aryl, the 4- to 20-membered heterocyclyl, and the 5-to 20-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH-(e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof.

In some embodiments of the compound of Formula (I-7), $R_{a2}$ represents:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornmyl, bicyclo[2.2.1]heptanyl, 2-oxobicyclo[2.2.1]heptanyl or bicyclo[2.2.1]heptenyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 3-azaspiro[5.5]undecyl or 7-azaspiro[3.5]nonyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4,

1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

phenyl or naphthyl, with each group being optionally substituted with one or more (e.g., 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo [2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl and imidazo [2,1-b]thiazolyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

In some embodiments of the compound of Formula (I-7), $R_{a2}$-$X_3$- represents:

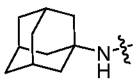 or 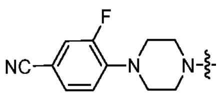

In some embodiments of the compound of Formula (I-7), $R_b$ represents $C_{1-60}$ alkyl (e.g., $C_1$-$C_{30}$ alkyl, $C_1$-$C_{29}$ alkyl, $C_1$-$C_{28}$ alkyl, $C_1$-$C_{27}$ alkyl, $C_1$-$C_{26}$ alkyl, $C_1$-$C_{25}$ alkyl, $C_1$-$C_{24}$ alkyl, $C_1$-$C_{23}$ alkyl, $C_1$-$C_{22}$ alkyl, $C_1$-$C_{21}$ alkyl, $C_1$-$C_{20}$ alkyl, $C_1$-$C_{19}$ alkyl, $C_1$-$C_{18}$ alkyl, $C_1$-$C_{17}$ alkyl, $C_1$-$C_{16}$ alkyl, $C_1$-$C_{15}$ alkyl, $C_1$-$C_{14}$ alkyl, $C_1$-$C_{13}$ alkyl, $C_1$-$C_{12}$ alkyl, $C_1$-$C_{11}$ alkyl, $C_1$-$C_{10}$ alkyl, $C_1$-$C_9$ alkyl, $C_1$-$C_8$ alkyl, $C_1$-$C_7$ alkyl, $C_1$-$C_6$ alkyl, $C_1$-$C_5$ alkyl, $C_1$-$C_4$ alkyl, $C_1$-$C_3$ alkyl, or $C_1$-$C_2$ alkyl) optionally substituted with D or halogen.

In some embodiments of the compound of Formula (I-7), $R_b$ represents the following group: $CH_3$, $CF_3$, $CD_3$, $CH_2CH_3$, $-(CH_2)_2-CH_3$, $-(CH_2)_3-CH_3$, $-(CH_2)_4-CH_3$, $-(CH_2)_5-CH_3$, $-(CH_2)_6-CH_3$, $-(CH_2)_7-CH_3$, $-(CH_2)_8-CH_3$, $-(CH_2)_9-CH_3$, $-(CH_2)_{10}-CH_3$, $-(CH_2)_{11}-CH_3$, $-(CH_2)_{12}-CH_3$, $-(CH_2)_{13}-CH_3$, $-(CH_2)_{14}-CH_3$, $-(CH_2)_{15}-CH_3$, $-(CH_2)_{16}-CH_3$, $-(CH_2)_{17}-CH_3$, $-(CH_2)_{18}-CH_3$, $-(CH_2)_{19}-CH_3$, $-(CH_2)_{20}-CH_3$, $-(CH_2)_{21}-CH_3$, $-(CH_2)_{22}-CH_3$, $-(CH_2)_{23}-CH_3$, $-(CH_2)_{24}-CH_3$, $-(CH_2)_{25}-CH_3$, $-(CH_2)_{26}-CH_3$, $-(CH_2)_{27}-CH_3$, $-(CH_2)_{28}-CH_3$, or $-(CH_2)_{29}-CH_3$.

In some embodiments of the compound of Formula (I-7), $R_b$ can represents the following formula:

$R_{b1}$-$X_4$-$L_2$- ,

wherein $L_2$ represents:

linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) optionally substituted with one or more (e.g., 1-20, or 1-15, 1-10, 1-9, 1-8, 1-7, 1-6, 1-5, 1-4, 1-3 or 1-2, or 1) substituents

selected from the group consisting of D, $C_{1-4}$ alkyl, halogen, $C_{3-6}$cycloalkyl or any combination thereof, or optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{12}$ and/or one or more (e.g., 1-20, or 1-15, 1-10, 1-9, 1-8, 1-7, 1-6, 1-5, 1-4, 1-3 or 1-2, or 1) groups $R_{13}$ and/or any combination of one or more (e.g., 1-20, or 1-15, 1-10, 1-9, 1-8, 1-7, 1-6, 1-5, 1-4, 1-3 or 1-2, or 1) groups $R_{12}$ and $R_{13}$ inserted into its backbone carbon chain, wherein carbon-carbon bond between one or more pairs (e.g., 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of $C_{3-15}$cycloalkylene, 4- to 15-membered heterocyclylene, $C_{6-10}$ arylene, 5- to 15-membered heteroarylene, $C_{2-6}$ alkenylene, $C_{2-6}$ alkynylene or any combination thereof, wherein the $C_{3-15}$cycloalkylene, the 4- to 15-membered heterocyclylene, $C_{6-10}$ arylene and the 5- to 15-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH-(e.g., $C_{1-3}$ alkyl-C(O)NH-, such as $CH_3$-C(O) NH-, $CH_3CH_2$-C(O)NH-, and $CH_3CH_2CH_2$-C(O)NH-), or any combination thereof, and the linear or branched $C_{2-60}$ alkylene is optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, $C_{1-4}$ alkyl, halogen, $C_{3-6}$cycloalkyl or any combination thereof;

$X_4$ represents a bond, or $X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted $C_{3-20}$cycloalkylene, optionally substituted $C_{6-20}$ arylene, optionally substituted 4- to 20-membered heterocyclylene, optionally substituted 5- to 20-membered heteroarylene, triazolylene-NH-, or -NH-triazolylene, wherein $R_{15}$ represents H or $C_{1-3}$ alkyl, and the $C_{3-20}$cycloalkylene, the $C_{6-20}$ arylene, the 4- to 20-membered heterocyclylene, and the 5- to 20-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, halogen, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof; and

$R_{b1}$ represents optionally substituted $C_{3-20}$cycloalkyl, optionally substituted $C_{6-20}$ aryl, optionally substituted 4- to 20-membered heterocyclyl or optionally substituted 5- to 20-membered heteroaryl, wherein the $C_{3-20}$cycloalkyl, the $C_{6-20}$ aryl, the 4- to 20-membered heterocyclyl, and the 5- to 20-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy (e.g., $C_{1-3}$ alkoxy, such as methoxy, ethoxy, or

propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof.

In some embodiments of the compound of Formula (I-7), $L_2$ represents the structure of the following formula: -$C_{2-30}$ alkylene-, wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the $C_{2-30}$ alkylene is optionally replaced with a substituent selected from the group consisting of: D, $C_{1-4}$ alkyl, halogen, $C_{3-6}$cycloalkyl or any combination thereof.

In some embodiments of the compound of Formula (I-7), $L_2$ represents the following group:

$-CH_2-$, $-(CH_2)_2-$, $-(CH_2)_3-$, $-(CH_2)_4-$, $-(CH_2)_5-$, $-(CH_2)_2-CF_2-(CH_2)_2-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{15}-$, $-(CH_2)_{16}-$, $-(CH_2)_{17}-$, $-(CH_2)_{18}-$, $-(CH_2)_{19}-$, $-(CH_2)_{20}-$, $-(CH_2)_{21}-$, $-(CH_2)_{22}-$, $-(CH_2)_{25}-$, or $-(CH_2)_{30}-$;

wherein a hydrogen atom of one or more (e.g., 1-20, or 1-15, 1-10, 1-9, 1-8, 1-7, 1-6, 1-5, 1-4, 1-3 or 1-2, or 1) $CH_2$ of the group is optionally replaced with a substituent selected from the group consisting of: D, $C_{1-4}$ alkyl, halogen, $C_{3-6}$cycloalkyl or any combination thereof.

In some embodiments of the compound of Formula (I-7), $L_2$ represents the structure of the following formula:

$-(C(R^{a9})(R^{a10}))_{m1}-(R_{12}(C(R^{a11})(R^{a12}))_{m2})_{p1}-$;

$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-$;

$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-(R_{12}(C(R^{a15})(R^{a16}))_{m4})_{p3}-$;

$-(C(R^{a9})(R^{a10}))_{m1}-(R_{13}(C(R^{a11})(R^{a12}))_{m2})_{p1}-$;

$-(C(R^{a9})(R^{a10}))_{m1}-(R_{13}(C(R^{a11})(R^{a12}))_{m2})_{p1}-(R_{13}(C(R^{a13})(R^{a14}))_{m3})_{p2}-$;

$-(C(R^{a9})(R^{a10}))_{m1}-(R_{13}(C(R^{a11})(R^{a12}))_{m2})_{p1}-(R_{13}(C(R^{a13})(R^{a14}))_{m3})_{p2}-(R_{13}(C(R^{a15})(R^{a16}))_{m4})_{p3}-$;

$-(C(R^{a9})(R^{a10}))_{m1}-(R_{12}-R_{13}-(C(R^{a11})(R^{a12}))_{m2})_{p1}-$;

$-(C(R^{a9})(R^{a10}))_{m1}-(R_{12}(C(R^{a11})(R^{a12}))_{m2})_{p1}-(R_{13}(C(R^{a13})(R^{a14}))_{m3})_{p2}-$;

$-(C(R^{a9})(R^{a10}))_{m1}-(R_{13}-R_{12}-(C(R^{a11})(R^{a12}))_{m2})_{p1}-$; or

$-(C(R^{a9})(R^{a10}))_{m1}-(R_{13}(C(R^{a11})(R^{a12}))_{m2})_{p1}-(R_{12}(C(R^{a13})(R^{a14}))_{m3})_{p2}-$;

wherein each group $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_{14}$ independently represents H or $C_{1-3}$ alkyl; and each group $R_{13}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of deuterium, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O) NH-, and $CH_3CH_2CH_2$-C(O)NH-) or any combination thereof;

$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl (e.g., $C_{1-3}$ alkyl, such as methyl, ethyl, propyl, isopropyl, butyl, sec-butyl or tert-butyl), $C_{3-6}$cycloalkyl (e.g., cyclopropyl, cyclobutyl, cyclopentyl or cyclohexyl) or any combination thereof; and

m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-7), $L_2$ represents the structure of the following formula:

$-(C(R^{a9})(R^{a10}))_{m1}-(O(C(R^{a11})(R^{a12}))_{m2})_{p1}-$;

$-(C(R^{a9})(R^{a10}))_{m1}-(O(C(R^{a11})(R^{a12}))_{m2})_{p1}-(O(C(R^{a13})(R^{a14}))_{m3})_{p2}-$;

$-(C(R^{a9})(R^{a10}))_{m1}-(O(C(R^{a11})(R^{a12}))_{m2})_{p1}-(O(C(R^{a13})(R^{a14}))_{m3})_{p2}-(O(C(R^{a15})(R^{a16}))_{m4})_{p3}-$;

$-(C(R^{a}9)(R^{a10}))_{m1}-(N(R_{g11})(C(R^{a11})(R^{a12}))_{m2})_{p1}-$;

$-(C(R^{a9})(R^{a10}))_{m1}-(N(R^{a11})(C(R^{a12})_{m2})_{p1}-(N(R^{g11})(C(R^{a13})(R^{a14})_{m3})_{p2}-$;

$-(C(R^{a9})(R^{a10}))m_1-(N(R_{g11})(C(R^{a11})(R^{a12}))_{m2})_{p1}-(N(R_{g11})(C(R^{a13})(R^{a14}))_{m3})_{p2}-(N(R_{g11})(C(R^{al5})(R^{a16}))_{m4})_{p3}-$;

$-(C(R^{a9})(R^{a10}))_{m1}-(C(O)NH-(C(R^{a11})(R^{a12}))_{m2})_{p1}-$;

$-(C(R^{a9})(R^{a10}))_{m1}-(C(O)NH-(C(R^{a11})(R^{a12}))_{m2})_{p1}-(C(O)NH-(C(R^{a13})(R^{a14}))_{m3})_{p2}-$;

$-(C(R^{a9})(R^{a10})_{m1}-(C(O)NH-(C(R^{a11})(R^{a12}))_{m2})_{p1}-(C(O)NH-(C(R^{a13})(R^{a14}))_{m3})_{p2}-(C(O)NH-(C(R^{a15})(R^{a16}))_{m4})_{p3}-$;

$-(C(R^{a9})(R^{a10}))_{m1}-(C(O)NH-(C(R^{a11})(R^{a12}))_{m2})_{p1}-(O-(C(R^{a13})(R^{a14}))_{m3})_{p2}-$;

$-(C(R^{a9})(R^{a10}))_{m1}-C(O)NH-(C(R^{a11})(R^{al1}))_{m2}-(O(C(R^{a13})(R^{a14}))_{m3})_{p1}-$;

$-(C(R^{a9})(R^{a10}))_{m1}-(NHC(C(R^{a11})(R^{a12}))_{m2})_{p1}-$;

$-(C(R^{a9})(R^{a10}))_{m1}-(NHC(O)-C(R^{a11})(R^{a12})_{m2})_{p1}-(O-(C(R^{a13})(R^{a14})_{m3})_{p2}-$;

$-(C(R^{a9})(R^{a10}))_{m1}-(NHC(O)-(C(R^{a11})(R^{a12}))_{m2})_{p1}-(O-(C(R^{a13})(R^{a14})_{m3})_{p2}-(O-(C(R^{a15})(R^{a16}))_{m4})_{p3}-$;

$-(C(R^{a9})(R^{a10}))_{m1}-NHC(O)-(C(R^{a11})(R^{a12}))_{m2}-(O(C(R^{a13})(R^{a14}))_{m3})_{p1}-$;

$-(C(R^{a9})(R^{a10}))_{m1}-NHC(O)NH-(C(R^{a11})(R^{a12}))_{m2}-$;

$-(C(R^{a9})(R^{a10})_{m1}-C(O)-(C(R^{a11})(R^{a12}))_{m2}-$;

$-C(R^{a9})(R^{a10})_{m1}-C(S)-(C(R^{a11})(R^{a12}))_{m2}-$;

$-(C(R^{a9})(R^{a10}))_{m1}-S-(C(R^{a11})(R^{a12}))_{m2}-$;

$-(C(R^{a9})(R^{a10}))_{m1}-(C_{6-10}$ arylene$-(C(R^{a11})(R^{a12}))_{m2})_{p1}-$;

$-(C(R^{a9})(R^{a10}))_{m1}-(C_{6-10}$ arylene$-(C(R^{a11})(R^{a12}))_{m2})_{p1}-C_{6-10}$ arylene$-(C(R^{a13})(R^{a14}))_{m3}-$;

$-(C(R^{a9})(R^{a10}))_{m1}-$(4- to 15-membered heterocyclylene$-(C(R^{a11})(R^{a12}))_{m2})_{p1}-$;

$-(C(R^{a9})(R^{a10}))_{m1}-$(4- to 15-membered heterocyclylene$-(C(R^{a11})(R^{a12}))_{m2})_{p1}-$(4- to 15-membered heterocyclylene$-(C(R^{a13})(R^{a14}))_{m3})_{p2}-$;

$-(C(R^{a9})(R^{a10}))_{m1}-$(5- to 15-membered heteroarylene$-(C(R^{a11})(R^{a12}))_{m2})_{p1}-$;

$-(C(R^{a9})(R^{a10}))_{m1}-$(5- to 15-membered heteroarylene$-(C(R^{a11})(R^{a12}))_{m2})_{p1}-$(5- to 15-membered heteroarylene$-(C(R^{a13})(R^{a14}))_{m3})_{p2}-$;

$-(C(R^{a9})(R^{a10}))_{m1}-(C_{3-15}$cycloalkylene$-(C(R^{a11})(R^{a12}))_{m2})_{p1}-$; or

$-(C(R^{a9})(R^{a10}))_{m1}-(C_{3-15}$cycloalkylene$-(C(R^{a11})(R^{a12}))_{m2})_{p1}-(C_{3-15}$cycloalkylene- $(C(R^{a13})(R^{a14}))_{m3})_{p2}-$;

wherein each $R_{g11}$ independently represents H or $C_{1-3}$ alkyl;
the $C_{3-15}$cycloalkylene, the $C_{6-10}$ arylene, the 4- to 15-membered heterocyclylene and the 5- to 15-membered heteroarylene are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2-C_{1-4}$ alkylene, $C_{1-4}$ alkyl-NHC(O)-, $C_{1-4}$ alkyl-C(O)NH- or any combination thereof;
$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and
m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-7), $L_2$ represents the structure of the following formula: $-CH_2-O-CH_2-$, $-CH_2-O-(CH_2)_2-$, $-(CH_2)_1-O-(CH_2)_3-$, $-(CH_2)_1-O-(CH_2)_4-$, $-(CH_2)_1-O-(CH_2)_5-$, $-(CH_2)_1-O-(CH_2)_6-$, $-(CH_2)_1-O-(CH_2)_7-$, $-(CH_2)_1-O-(CH_2)_8-$, $-(CH_2)_1-O-(CH_2)_9-$, $-(CH_2)_1-O-(CH_2)_{10}-$, $-(CH_2)_2-O-(CH_2)_1-$, $-(CH_2)_2-O-(CH_2)_2-$, $-(CH_2)_2-O-(CH_2)_3-$, $-(CH_2)_2-O-(CH_2)_4-$, $-(CH_2)_2-O-(CH_2)_5-$, $-(CH_2)_2-O-(CH_2)_6-$, $-(CH_2)_2-O-(CH_2)_7-$, $-(CH_2)_2-O-(CH_2)_8-$, $-(CH_2)_2-O-(CH_2)_9-$, $-(CH_2)_2-O-(CH_2)_{10}-$, $-(CH_2)_2-O-(CH_2)_{11}-$, $-(CH_2)_2-O-(CH_2)_{12}-$, $-(CH_2)_3-O-(CH_2)_1-$, $-(CH_2)_3-O-(CH_2)_2-$, $-(CH_2)_3-O-(CH_2)_3-$, $-(CH_2)_3-O-(CH_2)_4-$, $-(CH_2)_3-O-(CH_2)_5-$, $-(CH_2)_3-O-(CH_2)_6-$, $-(CH_2)_3-O-(CH_2)_7-$, $-(CH_2)_4-O-(CH_2)_1-$, $-(CH_2)_4-O-(CH_2)_2-$, $-(CH_2)_4-O-(CH_2)_3-$, $-(CH_2)_4-O-(CH_2)_4-$, $-(CH_2)_4-O-(CH_2)_5-$, $-(CH_2)_4-O-(CH_2)_6-$, $-(CH_2)_5-O-(CH_2)_1-$, $-(CH_2)_5-O-(CH_2)_2-$, $-(CH_2)_5-O-(CH_2)_3-$, $-(CH_2)_5-O-(CH_2)_4-$, $-(CH_2)_5-O-(CH_2)_5-$, $-(CH_2)_6-O-(CH_2)_1-$, $-(CH_2)_6-O-(CH_2)_2-$, $-(CH_2)_6-O-(CH_2)_3-$, $-(CH_2)_6-O-(CH_2)_4-$, $-(CH_2)_7-O-(CH_2)_1-$, $-(CH_2)_7-O-(CH_2)_2-$, $-(CH_2)_7-O-(CH_2)_3-$, $-(CH_2)_8-O-(CH_2)_1-$, $-(CH_2)_8-O-(CH_2)_2-$, $-CH(CH_3)-O-(CH_2)_1-$, $-CH(CH_3)-O-(CH_2)_2-$, $-CH(CH_3)-O-(CH_2)_3-$, $-CH(CH_3)-O-(CH_2)_4-$, $-CH(CH_3)-O-(CH_2)_5-$, $-CH(CH_3)-O-(CH_2)_6-$, $-CH(CH_3)-O-(CH_2)_7-$, $-CH(CH_3)-O-(CH_2)_8-$, $-CH(CH_3)-O-(CH_2)_9-$, $-CH(CH_3)-O-(CH_2)_{10}-$, $-CH_2-(O(CH_2)_2)_2-$, $-CH_2-(O(CH_2)_2)_3-$, $-CH_2-(O(CH_2)_2)_4-$, $-CH_2-(O(CH_2)_2)_5-$, $-CH_2-(O(CH_2)_2)_6-$, $-CH_2-(O(CH_2)_2)_7-$, $-CH_2-(O(CH_2)_2)_8-$, $-CH_2-(O(CH_2)_2)_9-$, $-CH_2-(O(CH_2)_2)_{10}-$, $-CH_2-(O(CH_2)_2)_1-OCH_2-$, $-CH_2-(O(CH_2)_2)_2-OCH_2-$, $-CH_2-(O(CH_2)_2)_3-OCH_2-$, $-CH_2-(O(CH_2)_2)_4-OCH_2-$, $-CH_2-(O(CH_2)_2)_5-OCH_2-$, $-CH_2-(O(CH_2)_2)_6-OCH_2-$, $-CH_2-(O(CH_2)_2)_7-OCH_2-$, $-CH_2-(O(CH_2)_2)_8-OCH_2-$, $-CH_2-(O(CH_2)_2)_9-OCH_2-$, $-CH_2-(O(CH_2)_2)_{10}-OCH_2-$, $-(CH_2)_2-(O(CH_2)_2)_2-$, $-(CH_2)_2-(O(CH_2)_2)_3-$, $-(CH_2)_2-(O(CH_2)_2)_4-$, $-(CH_2)_2-(O(CH_2)_2)_5-$, $-(CH_2)_2-(O(CH_2)_2)_6-$, $-(CH_2)_2-(O(CH_2)_2)_7-$, $-(CH_2)_2-(O(CH_2)_2)_8-$, $-(CH_2)_2-(O(CH_2)_2)_9-$, $-(CH_2)_2-(O(CH_2)_2)_{10}-$, $-(CH_2)_3-(O(CH_2)_2)_2-$, $-(CH_2)_3-(O(CH_2)_2)_3-$, $-(CH_2)_3-(O(CH_2)_2)_4-$, $-(CH_2)_3-(O(CH_2)_2)_5-$, $-(CH_2)_3-(O(CH_2)_2)_6-$, $-(CH_2)_3-(O(CH_2)_2)_7-$, $-(CH_2)_3-(O(CH_2)_2)_8-$, $-(CH_2)_3-(O(CH_2)_2)_9-$, $-(CH_2)_3-(O(CH_2)_2)_{10}-$, $-(CH_2)_4-(O(CH_2)_2)_2-$, $-(CH_2)_4-(O(CH_2)_2)_3-$, $-(CH_2)_4-(O(CH_2)_2)_4-$, $-(CH_2)_4-(O(CH_2)_2)_5-$, $-(CH_2)_4-(O(CH_2)_2)_6-$, $-(CH_2)_4-(O(CH_2)_2)_7-$, $-(CH_2)_4-(O(CH_2)_2)_8-$, $-(CH_2)_4-(O(CH_2)_2)_9-$, $-(CH_2)_4-(O(CH_2)_2)_{10}-$, $-CH_2-(O(CH_2)_3)_2-$, $-CH_2-(O(CH_2)_3)_3-$, $-CH_2-(O(CH_2)_3)_4-$, $-CH_2-(O(CH_2)_3)_5-$, $-CH_2-(O(CH_2)_3)_6-$, $-CH_2-(O(CH_2)_3)_7-$, $-CH_2-(O(CH_2)_3)_8-$, $-CH_2-(O(CH_2)_3)_9-$, $-CH_2-(O(CH_2)_3)_{10}-$, $-(CH_2)_2-(O(CH_2)_3)_2-$, $-(CH_2)_2-(O(CH_2)_3)_3-$, $-(CH_2)_2-(O(CH_2)_3)_4-$, $-(CH_2)_2-(O(CH_2)_3)_5-$, $-(CH_2)_2-(O(CH_2)_3)_6-$, $-(CH_2)_2-(O(CH_2)_3)_7-$, $-(CH_2)_2-(O(CH_2)_3)_8-$, $-(CH_2)_2-(O(CH_2)_3)_9-$, $-(CH_2)_2-(O(CH_2)_3)_{10}-$, $-(CH_2)_3-(O(CH_2)_3)_2-$, $-(CH_2)_3-(O(CH_2)_3)_3-$, $-(CH_2)_3-(O(CH_2)_3)_4-$, $-(CH_2)_3-(O(CH_2)_3)_5-$, $-(CH_2)_3-(O(CH_2)_3)_6-$, $-(CH_2)_3-(O(CH_2)_3)_7-$, $-(CH_2)_3-(O(CH_2)_3)_8-$, $-(CH_2)_3-(O(CH_2)_3)_9-$, $-(CH_2)_3-(O(CH_Z)_3)_{10}-$,

$-CH_2-O-(CH_2)_2-O-(CH_2)_3-$, $-CH_2-(O(CH_2)_2)_2-(O(CH_2)_3)_2-$, $-CH_2-(O(CH_2)_2)_3-(O(CH_2)_3)_3-$, $-CH_2-(O(CH_2)_2)_4-(O(CH_2)_3)_4-$, $-CH_2-(O(CH_2)_2)_5-(O(CH_2)_3)_5-$, $-CH_2-(O(CH_2)_2)_6-(O(CH_2)_3)_6-$, $-(CH_2)_2-O-(CH_2)_2-O-(CH_2)_3-$, $-(CH_2)_2-(O(CH_2)_2)_2-(O(CH_2)_3)_2-$, $-(CH_2)_2-(O(CH_2)_2)_3-(O(CH_2)_3)_3-$, $-(CH_2)_2-(O(CH_2)_2)_4-(O(CH_2)_3)_4-$, $-(CH_2)_2-(O(CH_2)_2)_5-(O(CH_2)_3)_5-$, $-(CH_2)_2-(O(CH_2)_2)_6-(O(CH_2)_3)_6-$, $-(CH_2)_3-O-(CH_2)_2-O-(CH_2)_3-$, $-(CH_2)_3-(O(CH_2)_2)_2-(O(CH_2)_3)_2-$, $-(CH_2)_3-(O(CH_2)_2)_3-(O(CH_2)_3)_3-$, $-(CH_2)_3-(O(CH_2)_2)_4-(O(CH_2)_3)_4-$, $-(CH_2)_3-(O(CH_2)_2)_5-(O(CH_2)_3)_5-$, $-(CH_2)_3-(O(CH_2)_2)_6-(O(CH_2)_3)_6-$, $-CH_2-O-(CH_2)_3-O-(CH_2)_2-$, $-CH_2-(O(CH_2)_3)_2-(O(CH_2)_2)_2-$, $-CH_2-(O(CH_2)_3)_3-(O(CH_2)_2)_3-$, $-CH_2-(O(CH_2)_3)_4-(O(CH_2)_2)_4-$, $-CH_2-(O(CH_2)_3)_5-(O(CH_2)_2)_5-$, $-CH_2-(O(CH_2)_3)_6-(O(CH_2)_2)_6-$, $-(CH_2)_2-O-(CH_2)_3-O-(CH_2)_2-$, $-(CH_2)_2-(O(CH_2)_3)_2-(O(CH_2)_2)_2-$, $-(CH_2)_2-(O(CH_2)_3)_3-(O(CH_2)_2)_3-$, $-(CH_2)_2-(O(CH_2)_3)_4-(O(CH_2)_2)_4-$, $-(CH_2)_2-(O(CH_2)_3)_5-(O(CH_2)_2)_5-$, $-(CH_2)_2-(O(CH_2)_3)_6-(O(CH_2)_2)_6-$, $-(CH_2)_3-O-(CH_2)_3-O-(CH_2)_2-$, $-(CH_2)_3-(O(CH_2)_3)_2-(O(CH_2)_2)_2-$, $-(CH_2)_3-(O(CH_2)_3)_3-(O(CH_2)_2)_3-$, $-(CH_2)_3-(O(CH_2)_3)_4-(O(CH_2)_2)_4-$, $-(CH_2)_3-(O(CH_2)_3)_5-(O(CH_2)_2)_5-$, $-(CH_2)_3-(O(CH_2)_3)_6-(O(CH_2)_2)_6-$, $-CH_2-O-(CH_2)_2-O-CH_2-$, $-(CH_2)_2-O-(CH_2)_2-O-CH_2-$, $-(CH_2)_2-(O(CH_2)_2)_2-O-(CH_2)_3-$, $-(CH_2)_2-(O(CH_2)_2)_3-O-(CH_2)_3-$, $-(CH_2)_2-(O(CH_2)_2)_4-O-(CH_2)_3-$, $-(CH_2)_5-(O(CH_2)_2)_2-O-(CH_2)_5-$, $-(CH_2)_5-(O(CH_2)_2)_2-O-(CH_2)_6-$, $-CH_2-C(O)-CH_2-$, $-CH_2-C(O)-(CH_2)_2-$, $-(CH_2)_1-C(O)-(CH_2)_3-$, $-(CH_2)_1-C(O)-(CH_2)_4-$, $-(CH_2)_1-C(O)-(CH_2)_5-$, $-(CH_2)_1-C(O)-(CH_2)_6-$, $-(CH_2)_1-C(O)-(CH_2)_7-$, $-(CH_2)_1-C(O)-(CH_2)_8-$, $-(CH_2)_1-C(O)-(CH_2)_9-$, $-(CH_2)_1-C(O)-(CH_Z)_{lo}-$, $-(CH_2)_2-C(O)-(CH_2)_1-$, $-(CH_2)_2-C(O)-(CH_2)_2-$, $-(CH_2)_2-C(O)-(CH_2)_3-$, $-(CH_2)_2-C(O)-(CH_2)_4-$, $-(CH_2)_2-C(O)-(CH_2)_5-$, $-(CH_2)_2-C(O)-(CH_2)_6-$, $-(CH_2)_2-C(O)-(CH_2)_7-$, $-(CH_2)_2-C(O)-(CH_2)_8-$, $-(CH_2)_2-C(O)-(CH_2)_9-$, $-(CH_2)_2-C(O)-(CH_2)_{10}-$, $-(CH_2)_2-C(O)-(CH_2)_{11}-$, $-(CH_2)_2-C(O)-(CH_2)_{12}-$, $-(CH_2)_3-C(O)-(CH_2)_1-$, $-(CH_2)_3-C(O)-(CH_2)_2-$, $-(CH_2)_3-C(O)-(CH_2)_3-$, $-(CH_2)_3-C(O)-(CH_2)_4-$, $-(CH_2)_3-C(O)-(CH_2)_5-$, $-(CH_2)_3-C(O)-(CH_2)_6-$, $-(CH_2)_3-C(O)-(CH_2)_7-$, $-(CH_2)_4-C(O)-(CH_2)_1-$, $-(CH_2)_4-C(O)-(CH_2)_2-$, $-(CH_2)_4-C(O)-(CH_2)_3-$, $-(CH_2)_4-C(O)-(CH_2)_4-$, $-(CH_2)_4-C(O)-(CH_2)_5-$, $-(CH_2)_4-C(O)-(CH_2)_6-$, $-(CH_2)_5-C(O)-(CH_2)_1-$, $-(CH_2)_5-C(O)-(CH_2)_2-$, $-(CH_2)_5-C(O)-(CH_2)_3-$, $-(CH_2)_5-C(O)-(CH_2)_4-$, $-(CH_2)_5-C(O)-(CH_2)_5-$, $-(CH_2)_6-C(O)-(CH_2)_1-$, $-(CH_2)_6-C(O)-(CH_2)_2-$, $-(CH_2)_6-C(O)-(CH_2)_3-$, $-(CH_2)_6-C(O)-(CH_2)_4-$, $-(CH_2)_7-C(O)-(CH_2)_1-$, $-(CH_2)_7-C(O)-(CH_2)_2-$, $-(CH_2)_7-C(O)-(CH_2)_3-$, $-(CH_2)_8-C(O)-(CH_2)_1-$, $-(CH_2)_8-C(O)-(CH_2)_2-$, $-CH_2-S-CH_2-$, $-CH_2-S-(CH_2)_2-$, $-(CH_2)_1-S-(CH_2)_3-$, $-(CH_2)_1-S-(CH_2)_4-$, $-(CH_2)_1-S-(CH_2)_5-$, $-(CH_2)_1-S-(CH_2)_6-$, $-(CH_2)_1-S-(CH_2)_7-$, $-(CH_2)_l-S-(CH_2)_8-$, $-(CH_2)_1-S-(CH_2)_9-$, $-(CH_2)_1-S-(CH_2)_{1O}-$, $-(CH_2)_2-S-(CH_2)_1-$, $-(CH_2)_2-S-(CH_2)_2-$, $-(CH_2)_2-S-(CH_2)_3-$, $-(CH_2)_2-S-(CH_2)_4-$, $-(CH_2)_2-S-(CH_2)_5-$, $-(CH_2)_2-S-(CH_2)_6-$, $-(CH_2)_2-S-(CH_2)_7-$, $-(CH_2)_2-S-(CH_2)_8-$, $-(CH_2)_2-S-(CH_2)_9-$, $-(CH_2)_2-S-(CH_2)_{10}-$, $-(CH_2)_2-S-(CH_2)_{11}-$, $-(CH_2)_2-S-(CH_2)_{12}-$, $-(CH_2)_3-S-(CH_2)_1-$, $-(CH_2)_3-S-(CH_2)_2-$, $-(CH_2)_3-S-(CH_2)_3-$, $-(CH_2)_3-S-(CH_2)_4-$, $-(CH_2)_3-S-(CH_2)_5-$, $-(CH_2)_3-S-(CH_2)_6-$, $-(CH_2)_3-S-(CH_2)_7-$, $-(CH_2)_4-S-(CH_2)_l-$, $-(CH_2)_4-S-(CH_2)_2-$, $-(CH_2)_4-S-(CH_2)_3-$, $-(CH_2)_4-S-(CH_2)_4-$, $-(CH_2)_4-S-(CH_2)_5-$, $-(CH_2)_4-S-(CH_2)_6-$, $-(CH_2)_5-S-(CH_2)_1-$, $-(CH_2)_5-S-(CH_2)_2-$, $-(CH_2)_5-S-(CH_2)_3-$, $-(CH_2)_5-S-(CH_2)_4-$, $-(CH_2)_5-S-(CH_2)_5-$, $-(CH_2)_6-S-(CH_2)_1-$, $-(CH_2)_6-S-(CH_2)_2-$, $-(CH_2)_6-S-(CH_2)_3-$, $-(CH_2)_6-S-(CH_2)_4-$, $-(CH_2)_7-S-(CH_2)_1-$, $-(CH_2)_7-S-(CH_2)_2-$, $-(CH_2)_7-S-(CH_2)_3-$, $-(CH_2)_8-S-(CH_2)_1-$, $-(CH_2)_8-S-(CH_2)_2-$, $-CH_2-C(S)-CH_2-$, $-CH_2-C(S)-(CH_2)_2-$, $-(CH_2)_1-C(S)-(CH_2)_3-$, $-(CH_2)_1-C(S)-(CH_2)_4-$, $-(CH_2)_1-C(S)-(CH_2)_5-$, $-(CH_2)_1-C(S)-(CH_2)_6-$, $-(CH_2)_1-C(S)-(CH_2)_7-$, $-(CH_2)_1-C(S)-(CH_2)_8-$, $-(CH_2)_1-C(S)-(CH_2)_9-$, $-(CH_2)_1-C(S)-(CH_2)_{10}-$, $-(CH_2)_2-C(S)-(CH_2)_1-$, $-(CH_2)_2-C(S)-(CH_2)_2-$, $-(CH_2)_2-C(S)-(CH_2)_3-$, $-(CH_2)_2-C(S)-(CH_2)_4-$, $-(CH_2)_2-C(S)-(CH_2)_5-$, $-(CH_2)_2-C(S)-(CH_2)_6-$, $-(CH_2)_2-C(S)-(CH_2)_7-$, $-(CH_2)_2-C(S)-(CH_2)_8-$, $-(CH_2)_2-C(S)-(CH_2)_9-$, $-(CH_2)_2-C(S)-(CH_2)_{10}-$, $-(CH_2)_2-C(S)-(CH_2)_{11}-$, $-(CH_2)_2-C(S)-(CH_2)_{12}-$, $-(CH_2)_3-C(S)-(CH_2)_1-$, $-(CH_2)_3-C(S)-(CH_2)_2-$, $-(CH_2)_3-C(S)-(CH_2)_3-$, $-(CH_2)_3-C(S)-(CH_2)_4-$, $-(CH_2)_3-C(S)-(CH_2)_5-$, $-(CH_2)_3-C(S)-(CH_2)_6-$, $-(CH_2)_3-C(S)-(CH_2)_7-$, $-(CH_2)_4-C(S)-(CH_2)_1-$, $-(CH_2)_4-C(S)-(CH_2)_2-$, $-(CH_2)_4-C(S)-(CH_2)_3-$, $-(CH_2)_4-C(S)-(CH_2)_4-$, $-(CH_2)_4-C(S)-(CH_2)_5-$, $-(CH_2)_4-C(S)-(CH_2)_6-$, $-(CH_2)_5-C(S)-(CH_2)_1-$, $-(CH_2)_5-C(S)-(CH_2)_2-$, $-(CH_2)_5-C(S)-(CH_2)_3-$, $-(CH_2)_5-C(S)-(CH_2)_4-$, $-(CH_2)_5-C(S)-(CH_2)_5-$, $-(CH_2)_6-C(S)-(CH_2)_1-$, $-(CH_2)_6-C(S)-(CH_2)_2-$, $-(CH_2)_6-C(S)-(CH_2)_3-$, $-(CH_2)_6-C(S)-(CH_2)_4-$, $-(CH_2)_7-C(S)-(CH_2)_l-$, $-(CH_2)_7-C(S)-(CH_2)_2-$, $-(CH_2)_7-C(S)-(CH_2)_3-$, $-(CH_2)_8-C(S)-(CH_2)_1-$, $-(CH_2)_8-C(S)-(CH_2)_2-$, $-CH_2-C(O)O-CH_2-$, $-CH_2-C(O)O-(CH_2)_2-$, $-(CH_2)_l-C(O)O-(CH_2)_3-$, $-(CH_2)_1-C(O)O-(CH_2)_4-$, $-(CH_2)_1-C(O)O-(CH_2)_5-$, $-(CH_2)_1-C(O)O-(CH_2)_6-$, $-(CH_2)_1-C(0)0-(CH_2)_7-$, $-(CH_2)_1-C(O)O-(CH_2)_8-$, $-(CH_2)_1-C(O)O-(CH_2)_9-$, $-(CH_2)_1-C(O)O-(CH_2)_{10}-$, $-(CH_2)_2-C(O)O-(CH_2)_1-$, $-(CH_2)_2-C(O)O-(CH_2)_2-$, $-(CH_2)_2-C(O)O-(CH_2)_3-$, $-(CH_2)_2-C(O)O-(CH_2)_4-$, $-(CH_2)_2-C(O)O-(CH_2)_5-$, $-(CH_2)_2-C(O)O-(CH_2)_6-$, $-(CH_2)_2-C(O)O-(CH_2)_7-$, $-(CH_2)_2-C(O)O-(CH_2)_8-$, $-(CH_2)_2-C(O)O-(CH_2)_9-$, $-(CH_2)_2-C(O)O-(CH_2)_{10}-$, $-(CH_2)_2-C(O)O-(CH_2)_{11}-$, $-(CH_2)_2-C(O)O-(CH_2)_{12}-$, $-(CH_2)_3-C(O)O-(CH_2)_1-$, $-(CH_2)_3-C(O)O-(CH_2)_2-$, $-(CH_2)_3-C(O)O-(CH_2)_3-$, $-(CH_2)_3-C(O)O-(CH_2)_4-$, $-(CH_2)_3-C(O)O-(CH_2)_5-$, $-(CH_2)_3-C(O)O-(CH_2)_6-$, $-(CH_2)_3-C(O)O-(CH_2)_7-$, $-(CH_2)_4-C(O)O-(CH_2)_1-$, $-(CH_2)_4-C(O)O-(CH_2)_2-$, $-(CH_2)_4-C(O)O-(CH_2)_3-$, $-(CH_2)_4-C(O)O-(CH_2)_4-$, $-(CH_2)_4-C(O)O-(CH_2)_5-$, $-(CH_2)_4-C(O)O-(CH_2)_6-$, $-(CH_2)_5-C(O)O-(CH_2)_1-$, $-(CH_2)_5-C(O)O-(CH_2)_2-$, $-(CH_2)_5-C(O)O-(CH_2)_3-$, $-(CH_2)_5-C(O)O-(CH_2)_4-$, $-(CH_2)_5-C(O)O-(CH_2)_5-$, $-(CH_2)_6-C(O)O-(CH_2)_1-$, $-(CH_2)_6-C(O)O-(CH_2)_2-$, $-(CH_2)_6-C(O)O-(CH_2)_3-$, $-(CH_2)_6-C(O)O-(CH_2)_4-$, $-(CH_2)_7-C(O)O-(CH_2)_1-$, $-(CH_2)_7-C(O)O-(CH_2)_2-$, $-(CH_2)_7-C(O)O-(CH_2)_3-$, $-(CH_2)_8-C(O)O-(CH_2)_1-$, $-(CH_2)_8-C(O)O-(CH_2)_2-$, $-CH_2-O-C(O)-CH_2-$, $-CH_2-OC(O)-(CH_2)_2-$, $-(CH_2)_1-OC(O)-(CH_2)_3-$, $-(CH_2)_1-OC(O)-(CH_2)_4-$, $-(CH_2)_1-OC(O)-(CH_2)_5-$, $-(CH_2)_1-O-C(O)-(CH_2)_6-$, $-(CH_2)_1-OC(O)-(CH_2)_7-$, $-(CH_2)_1-OC(O)-(CH_2)_8-$, $-(CH_2)_1-OC(O)-(CH_2)_9-$, $-(CH_2)_1-OC(O)-(CH_2)_{10}-$, $-(CH_2)_2-OC(O)-(CH_2)_1-$, $-(CH_2)_2-OC(O)-(CH_2)_2-$, $-(CH_2)_2-OC(O)-(CH_2)_3-$, $-(CH_2)_2-OC(O)-(CH_2)_4-$, $-(CH_2)_2-O-C(O)-(CH_2)_5-$, $-(CH_2)_2-OC(O)-(CH_2)_6-$, $-(CH_2)_2-OC(O)-(CH_2)_7-$, $-(CH_2)_2-OC(O)-(CH_2)_8-$, $-(CH_2)_2-OC(O)-(CH_2)_9-$, $-(CH_2)_2-OC(O)-(CH_2)_{10}-$, $-(CH_2)_2-OC(O)-(CH_2)_{11}-$, $-(CH_2)_2-OC(O)-(CH_2)_{12}-$, $-(CH_2)_3-OC(O)-(CH_2)_1-$, $-(CH_2)_3-O-C(O)-(CH_2)_2-$, $-(CH_2)_3-OC(O)-(CH_2)_3-$, $-(CH_2)_3-OC(O)-(CH_2)_4-$, $-(CH_2)_3-OC(O)-(CH_2)_5-$, $-(CH_2)_3-OC(O)-(CH_2)_6-$,

$-(CH_2)_3-OC(O)-(CH_2)_7-$, $-(CH_2)_4-OC(O)-(CH_2)_1-$, $-(CH_2)_4-OC(O)-(CH_2)_2-$, $- (CH_2)_4-OC(O)-(CH_2)_3-$, $-(CH_2)_4-O-C(O)-(CH_2)_4-$, $-(CH_2)_4-OC(O)-(CH_2)_5-$, $-(CH_2)_4-OC(O)-(CH_2)_6-$, $- (CH_2)_5-OC(O)-(CH_2)_1-$, $-(CH_2)_5-OC(O)-(CH_2)_2-$, $-(CH_2)_5-OC(O)-(CH_2)_3-$, $-(CH_2)_5-OC(O)-(CH_2)_4-$, $- (CH_2)_5-OC(O)-(CH_2)_5-$, $-(CH_2)_6-OC(O)-(CH_2)_1-$, $-(CH_2)_6-O-C(O)-(CH_2)_2-$, $-(CH_2)_6-OC(O)-(CH_2)_3-$, $- (CH_2)_6-OC(O)-(CH_2)_4-$, $-(CH_2)_7-OC(O)-(CH_2)_1-$, $-(CH_2)_7-OC(O)-(CH_2)_2-$, $-(CH_2)_7-OC(O)-(CH_2)_3-$, $- (CH_2)_8-OC(O)-(CH_2)_1-$, $-(CH_2)_8-OC(O)-(CH_2)_2-$, $-(CH_2)_1-N(R_{g11})-(CH_2)_1-$, $-(CH_2)_1-N(R_{g11})-(CH_2)_2-$, $- (CH_2)_1-N(R_{g11})-(CH_2)_3-$, $-(CH_2)_1-N(R_{g11})-(CH_2)_4-$, $-(CH_2)_1-N(R_{g11})-(CH_2)_5-$, $-(CH_2)_1-N(R_{g11})-(CH_2)_6-$, $- (CH_2)_1-N(R_{g11})-(CH_2)_7-$, $-(CH_2)_1-N(R_{g11})-(CH_2)_8-$, $-(CH_2)_1-N(R_{g11})-(CH_2)_9-$, $-(CH_2)_1-N(R_{g11})-(CH_2)_{10}-$, $-(CH_2)_2-N(R_{g11})-(CH_2)_1-$, $-(CH_2)_2-N(R_{g11})-(CH_2)_2-$, $-(CH_2)_2-N(R_{g11})-(CH_2)_3-$, $-(CH_2)_2-N(R_{g11})-(CH_2)_4-$, $-(CH_2)_2-N(R_{g11})-(CH_2)_5-$, $-(CH_2)_2-N(R_{g11})-(CH_2)_6-$, $-(CH_2)_2-N(R_{gn})-(CH_2)_7-$, $-(CH_2)_2-N(R_{g11})-(CH_2)_8-$, $-(CH_2)_2-N(R_{g11})-(CH_2)_9-$, $-(CH_2)_2-N(R_{g11})-(CH_2)_{10}-$, $-(CH_2)_2-N(R_{g11})-(CH_2)_{11}-$, $-(CH_2)_2-N(R_{g11})-(CH_2)_{12}-$, $-(CH_2)_3-N(R_{g11})-(CH_2)_1-$, $-(CH_2)_3-N(R_{g11})-(CH_2)_2-$, $-(CH_2)_3-N(R_{g11})-(CH_2)_3-$, $-(CH_2)_4-N(R_{g11})-(CH_2)_1-$, $-(CH_2)_4-N(R_{g11})-(CH_2)_2-$, $-(CH_2)_4-N(R_{g11})-(CH_2)_3-$, $-(CH_2)_4-N(R_{g11})-(CH_2)_4-$, $-(CH_2)_5-N(R_{g11})-(CH_2)_1-$, $-(CH_2)_5-N(R_{g11})-(CH_2)_2-$, $-(CH_2)_5-N(R_{g11})-(CH_2)_3-$, $-(CH_2)_5-N(R_{g11})-(CH_2)_4-$, $-(CH_2)_5-N(R_{g11})-(CH_2)_5-$, $-(CH_2)_6-N(R_{g11})-(CH_2)_1-$, $-(CH_2)_6-N(R_{g11})-(CH_2)_2-$, $-(CH_2)_6-N(R_{g11})-(CH_2)_3-$, $-(CH_2)_7-N(R_{g11})-(CH_2)_1-$, $-(CH_2)_7-N(R_{g11})-(CH_2)_2-$, $-(CH_2)_7-N(R_{g11})-(CH_2)_3-$, $-(CH_2)_8-N(R_{g11})-(CH_2)_1-$, $-(CH_2)_8-NR_{g11})-(CH_2)_2-$, $-(CH_2)_8-N(R_{g11})-(CH_2)_3-$, $-CH(CH_3)-N(R_{g11})-(CH_2)_1-$, $-CH(CH_3)-N(R_{g11})-(CH_2)_2-$, $-CH(CH_3)-N(R_{g11})-(CH_2)_3-$, $-CH(CH_3)-N(R_{g11})-(CH_2)_4-$, $-CH(CH_3)-N(R_{g11})-(CH_2)_5-$, $-CH(CH_3)-N(R_{g11})-(CH_2)_6-$, $-CH(CH_3)- N(R_{gll})-(CH_2)_7-$, $-CH(CH_3)-N(R_{g11})-(CH_2)_8-$, $-CH(CH_3)-N(R_{g11})-(CH_2)_9-$, $-CH(CH_3)-N(R_{g11})-(CH_2)_{10}-$, $-CH_{2C}(O)NHCH_2-$, $-(CH_2)_{2C}(O)NH(CH_2)_2-$, $-(CH_2)_{2C}(O)NH(CH_2)_3-$, $- (CH_2)_2C(O)NH(CH_2)_4-$, $-(CH_2)_2C(O)NH(CH_2)_5-$, $-(CH_2)_3C(O)NH(CH_2)_3-$, $-(CH_2)_3C(O)NH(CH_2)_4-$, $- (CH_2)_4C(O)NH(CH_2)_4-$, $-(CH_2)_5C(O)NH(CH_2)_5-$, $-(CH_2)_6C(O)NH(CH_2)_7-$, $-(CH_2)_6C(O)NH(CH_2)_6-$, $- (CH_2)_7C(O)NH(CH_2)_7-$, $-(CH_2)_8C(O)NH(CH_2)_8$, $-(CH_2)_9C(O)NH(CH_2)_9-$, $-(CH_2)_{10}C(O)NH(CH_2)_{10}-$, $- (CH_2)_2C(O)NH(CH_2)_2-O-(CH_2)_2-$, $-CH_2NHC(O)CH_2-$, $-(CH_2)_2NHC(O)(CH_2)_2-$, $- (CH_2)_2NHC(O)(CH_2)_3-$, $-(CH_2)_2NHC(O)(CH_2)_4-$, $-(CH_2)_2NHC(O)(CH_2)_5-$, $-(CH_2)_3NHC(O)(CH_2)_3-$, $- (CH_2)_3NHC(O)(CH_2)_4-$, $-(CH_2)_4NHC(O)(CH_2)_4-$, $-(CH_2)_5NHC(O)(CH_2)_5-$, $-(CH_2)_6NHC(O)(CH_2)_7-$, $- (CH_2)_6NHC(O)(CH_2)_6-$, $-(CH_2)_7NHC(O)(CH_2)_7-$, $-(CH_2)_8NHC(O)(CH_2)_8$, $-(CH_2)_9NHC(O)(CH_2)_9-$, - (CH2)ioNHC(0)(CH2)io-, $-(CH_2)_4NHC(O)(CH_2)_8-$, $-(CH_2)_2NHC(O)(CH_2)_2-O-(CH_2)_2-$, $- (CH_2)_4NHC(O)CH_2-$, -$CH_2$-piperidinylene-$CH_2$-, -$CH_2$-piperidinylene-$(CH_2)_2$-, -$CH_2$-piperidinylene-$(CH_2)_3$-, -$CH_2$-piperidinylene-$(CH_2)_4$-, -$CH_2$-piperidinylene-$(CH_2)_5$-, -$CH_2$-piperidinylene-$(CH_2)_6$-, - $CH_2$-piperidinylene-$(CH_2)_7$-, -$CH_2$-piperidinylene-$(CH_2)_8$-, -$(CH_2)_2$-piperidinylene-$(CH_2)_1$-, -$(CH_2)_2$-piperidinylene-$(CH_2)_2$-, -$(CH_2)_2$-piperidinylene-$(CH_2)_3$-, -$(CH_2)_2$-piperidinylene-$(CH_2)_4$-, -$(CH_2)_2$-piperidinylene-$(CH_2)_5$-, -$(CH_2)_2$-piperidinylene-$(CH_2)_6$-, -$(CH_2)_2$-piperidinylene-$(CH_2)_7$-, -$(CH_2)_2$-piperidinylene-$(CH_2)_8$-, -(CH2)3-piperidinylene-$CH_2$-, -$(CH_2)_3$-piperidinylene-$(CH_2)_2$-, -$(CH_2)_3$-piperidinylene-$(CH_2)_3$-, -$(CH_2)_3$-piperidinylene-$(CH_2)_4$-, -$(CH_2)_3$-piperidinylene-$(CH_2)_5$-, -$(CH_2)_3$-piperidinylene-$(CH_2)_6$-, -$(CH_2)_3$-piperidinylene-$(CH_2)_7$-, -$(CH_2)_3$-piperidinylene-$(CH_2)_8$-, -$(CH_2)_4$-piperidinylene-$CH_2$-, -$(CH_2)_4$-piperidinylene-$(CH_2)_2$-, -$(CH_2)_4$-piperidinylene-$(CH_2)_3$-, -$(CH_2)_4$-piperidinylene-$(CH_2)_4$-, -$(CH_2)_4$-piperidinylene-$(CH_2)_5$-, -$(CH_2)_4$-piperidinylene-$(CH_2)_6$-, -$(CH_2)_4$-piperidinylene-$(CH_2)_7$-, -$(CH_2)_4$-piperidinylene-$(CH_2)_8$-, -$(CH_2)_5$-piperidinylene-$(CH_2)_1$-, -$(CH_2)_5$-piperidinylene-$(CH_2)_2$-, -$(CH_2)_5$-piperidinylene-$(CH_2)_3$-, -$(CH_2)_5$-piperidinylene-$(CH_2)_4$-, -$(CH_2)_5$-piperidinylene-$(CH_2)_5$-, -$(CH_2)_5$-piperidinylene-$(CH_2)_6$-, -$(CH_2)_5$-piperidinylene-$(CH_2)_7$-, -$(CH_2)_5$-piperidinylene-$(CH_2)_8$-, -$(CH_2)_6$-piperidinylene-$(CH_2)_1$-, -$(CH_2)_6$-piperidinylene-$(CH_2)_2$-, -$(CH_2)_6$-piperidinylene-$(CH_2)_3$-, -$(CH_2)_6$-piperidinylene-$(CH_2)_4$-, -$(CH_2)_6$-piperidinylene-$(CH_2)_5$-, -$(CH_2)_6$-piperidinylene-$(CH_2)_6$-, -$(CH_2)_6$-piperidinylene-$(CH_2)_7$-, -$(CH_2)_6$-piperidinylene-$(CH_2)_8$-, -$(CH_2)_7$-piperidinylene-$(CH_2)_1$-, -$(CH_2)_7$-piperidinylene-$(CH_2)_2$-, -$(CH_2)_7$-piperidinylene-$(CH_2)_3$-, -$(CH_2)_7$-piperidinylene-$(CH_2)_4$-, -$(CH_2)_7$-piperidinylene-$(CH_2)_8$-, -$(CH_2)_8$-piperidinylene-$CH_2$-, -$(CH_2)_8$-piperidinylene-$(CH_2)_2$-, -$(CH_2)_8$-piperidinylene-$(CH_2)_3$-, -$(CH_2)_8$-piperidinylene-$(CH_2)_4$-, -$(CH_2)_8$-piperidinylene-$(CH_2)_5$-, -$(CH_2)_8$-piperidinylene-$(CH_2)_6$-, -$(CH_2)_8$-piperidinylene-$(CH_2)_7$-, -$(CH_2)_8$-piperidinylene-$(CH_2)_8$-, -$CH_2$-piperazinylene-$CH_2$-, -$CH_2$-piperazinylene-$(CH_2)_2$-, -$CH_2$-piperazinylene-$(CH_2)_3$-, -$CH_2$-piperazinylene-$(CH_2)_4$-, -$CH_2$-piperazinylene-$(CH_2)_5$-, -$CH_2$-piperazinylene-$(CH_2)_6$-, -$CH_2$-piperazinylene-$(CH_2)_7$-, -$CH_2$-piperazinylene-$(CH_2)_8$-, -$(CH_2)_2$-piperazinylene-$(CH_2)_1$-, -$(CH_2)_2$-piperazinylene-$(CH_2)_2$-, -$(CH_2)_2$-piperazinylene-$(CH_2)_3$-, -$(CH_2)_2$-piperazinylene-$(CH_2)_4$-, -$(CH_2)_2$-piperazinylene-$(CH_2)_5$-, -$(CH_2)_2$-piperazinylene-$(CH_2)_6$-, -$(CH_2)_2$-piperazinylene-$(CH_2)_7$-, -$(CH_2)_2$-piperazinylene-$(CH_2)_8$-, -$(CH_2)_3$-piperazinylene-$CH_2$-, -$(CH_2)_3$-piperazinylene-$(CH_2)_2$-, -$(CH_2)_3$-piperazinylene-$(CH_2)_3$-, -$(CH_2)_3$-piperazinylene-$(CH_2)_4$-, -$(CH_2)_3$-piperazinylene-$(CH_2)_5$-, -$(CH_2)_3$-piperazinylene-$(CH_2)_6$-, -$(CH_2)_3$-piperazinylene-$(CH_2)_7$-, -$(CH_2)_3$-piperazinylene-$(CH_2)_8$-, -$(CH_2)_4$-piperazinylene-$CH_2$-, -$(CH_2)_4$-piperazinylene-$(CH_2)_2$-, -$(CH_2)_4$-piperazinylene-$(CH_2)_3$-, -$(CH_2)_4$-piperazinylene-$(CH_2)_4$-, -$(CH_2)_4$-piperazinylene-$(CH_2)_5$-, -$(CH_2)_4$-piperazinylene-$(CH_2)_6$-, -$(CH_2)_4$-piperazinylene-$(CH_2)_7$-, -$(CH_2)_4$-piperazinylene-$(CH_2)_8$-, -$(CH_2)_5$-piperazinylene-$(CH_2)_1$-, -$(CH_2)_5$-piperazinylene-$(CH_2)_2$-, -$(CH_2)_5$-piperazinylene-$(CH_2)_3$-, -$(CH_2)_5$-piperazinylene-$(CH_2)_4$-, -$(CH_2)_5$-piperazinylene-$(CH_2)_5$-, -$(CH_2)_5$-piperazinylene-$(CH_2)_6$-, -$(CH_2)_5$-piperazinylene-$(CH_2)_7$-, -$(CH_2)_5$-piperazinylene-$(CH_2)_8$-, -$(CH_2)_6$-piperazinylene-(CH2)i-, -$(CH_2)_6$-piperazinylene-$(CH_2)_2$-, -$(CH_2)_6$-piperazinylene-$(CH_2)_3$-, -$(CH_2)_6$-piperazinylene-$(CH_2)_4$-, -$(CH_2)_6$-piperazinylene-$(CH_2)_5$-, -$(CH_2)_6$-piperazinylene-$(CH_2)_6$-, -$(CH_2)_6$-piperazinylene-$(CH_2)_7$-, -$(CH_2)_6$-piperazinylene-$(CH_2)_8$-, -$(CH_2)_7$-piperazinylene-$(CH_2)_1$-, -$(CH_2)_7$-piperazinylene-$(CH_2)_2$-, -$(CH_2)_7$-piperazinylene-$(CH_2)_3$-, -$(CH_2)_7$-piperazinylene-$(CH_2)_4$-, -$(CH_2)_7$-piperazinylene-$(CH_2)_8$-, -$(CH_2)_8$-piperazinylene-$CH_2$-, -$(CH_2)_8$-piperazinylene-$(CH_2)_2$-, -$(CH_2)_8$-piperazinylene-$(CH_2)_3$-, -$(CH_2)_8$-piperazinylene-$(CH_2)_4$-,

$-(CH_2)_8$-piperazinylene-$(CH_2)_5$-, $-(CH_2)_8$-piperazinylene-$(CH_2)_6$-, $-(CH_2)_8$-piperazinylene-$(CH_2)_7$-, $-(CH_2)_8$-piperazinylene-$(CH_2)_8$-, $-CH_2$-propylene-$CH_2$-, $-CH_2$-propylene-$(CH_2)_2$-, $-CH_2$-propylene-$(CH_2)_3$-, $-CH_2$-propylene-$(CH_2)_4$-, $-CH_2$-propylene-$(CH_2)_5$-, $-CH_2$-propylene-$(CH_2)_6$-, $-CH_2$-propylene-$(CH_2)_7$-, $-CH_2$-propylene-$(CH_2)_8$-, $-(CH_2)_2$-propylene-$(CH_2)_1$-, $-(CH_2)_2$-propylene-$(CH_2)_2$-, $-(CH_2)_2$-propylene-$(CH_2)_3$-, $-(CH_2)_2$-propylene-$(CH_2)_4$-, $-(CH_2)_2$-propylene-$(CH_2)_5$-, $-(CH_2)_2$-propylene-$(CH_2)_6$-, $-(CH_2)_2$-propylene-$(CH_2)_7$-, $-(CH_2)_2$-propylene-$(CH_2)_8$-, $-(CH_2)_3$-propylene-$CH_2$-, $-(CH_2)_3$-propylene-$(CH_2)_2$-, $-(CH_2)_3$-propylene-$(CH_2)_3$-, $-(CH_2)_3$-propylene-$(CH_2)_4$-, - $(CH_2)_3$-propylene-$(CH_2)_5$-, $-(CH_2)_3$-propylene-$(CH_2)_6$-, $-(CH_2)_3$-propylene-$(CH_2)_7$-, $-(CH_2)_3$-propylene-$(CH_2)_8$-, $-(CH_2)_4$-propylene-$CH_2$-, $-(CH_2)_4$-propylene-$(CH_2)_2$-, $-(CH_2)_4$-propylene-$(CH_2)_3$-, $-(CH_2)_4$-propylene-$(CH_2)_4$-, $-(CH_2)_4$-propylene-$(CH_2)_5$-, $-(CH_2)_4$-propylene-$(CH_2)_6$-, $-(CH_2)_4$-propylene-$(CH_2)_7$-, $-(CH_2)_4$-propylene-$(CH_2)_8$-, $-(CH_2)_5$-propylene-$(CH_2)_1$-, $-(CH_2)_5$-propylene-$(CH_2)_2$-, $-(CH_2)_5$-propylene-$(CH_2)_3$-, $-(CH_2)_5$-propylene-$(CH_2)_4$-, $-(CH_2)_5$-propylene-$(CH_2)_5$-, $-(CH_2)_5$-propylene-$(CH_2)_6$-, $-(CH_2)_5$-propylene-$(CH_2)_7$-, $-(CH_2)_5$-propylene-$(CH_2)_8$-, $-(CH_2)_6$-propylene-$(CH_2)_1$-, $-(CH_2)_6$-propylene-$(CH_2)_2$-, $-(CH_2)_6$-propylene-$(CH_2)_3$-, $-(CH_2)_6$-propylene-$(CH_2)_4$-, $-(CH_2)_6$-propylene-$(CH_2)_5$-, $-(CH_2)_6$-propylene-$(CH_2)_6$-, $-(CH_2)_6$-propylene-$(CH_2)_7$-, $-(CH_2)_6$-propylene-$(CH_2)_8$-, $-(CH_2)_7$-propylene-$(CH_2)_1$-, $-(CH_2)_7$-propylene-$(CH_2)_2$-, $-(CH_2)_7$-propylene-$(CH_2)_3$-, $-(CH_2)_7$-propylene-$(CH_2)_4$-, $-(CH_2)_7$-propylene-$(CH_2)_8$-, $-(CH_2)_8$-propylene-$CH_2$-, $-(CH_2)_8$-propylene-$(CH_2)_2$-, $-(CH_2)_8$-propylene-$(CH_2)_3$-, $-(CH_2)_8$-propylene-$(CH_2)_4$-, $-(CH_2)_8$-propylene-$(CH_2)_5$-, $-(CH_2)_8$-propylene-$(CH_2)_6$-, $-(CH_2)_8$-propylene-$(CH_2)_7$-, $-(CH_2)_8$-propylene-$(CH_2)_8$-, $-CH_2$-diazacycloheptanylene-$CH_2$-, $-CH_2$-diazacycloheptanylene-$(CH_2)_2$-, $-CH_2$-diazacycloheptanylene-$(CH_2)_3$-, $-CH_2$-diazacycloheptanylene-$(CH_2)_4$-, $-CH_2$-diazacycloheptanylene-$(CH_2)_5$-, $-CH_2$-diazacycloheptanylene-$(CH_2)_6$-, $-CH_2$-diazacycloheptanylene-$(CH_2)_7$-, $-CH_2$-diazacycloheptanylene-$(CH_2)_8$-, $-(CH_2)_2$-diazacycloheptanylene-$(CH_2)_1$-, $-(CH_2)_2$-diazacycloheptanylene-$(CH_2)_2$-, $-(CH_2)_2$-diazacycloheptanylene-$(CH_2)_3$-, $-(CH_2)_2$-diazacycloheptanylene-$(CH_2)_4$-, $-(CH_2)_2$-diazacycloheptanylene-$(CH_2)_5$-, $-(CH_2)_2$-diazacycloheptanylene-$(CH_2)_6$-, $-(CH_2)_2$-diazacycloheptanylene-$(CH_2)_7$-, $-(CH_2)_2$-diazacycloheptanylene-$(CH_2)_8$-, $-(CH_2)_3$-diazacycloheptanylene-$CH_2$-, $-(CH_2)_3$-diazacycloheptanylene-$(CH_2)_2$-, $-(CH_2)_3$-diazacycloheptanylene-$(CH_2)_3$-, $-(CH_2)_3$-diazacycloheptanylene-$(CH_2)_4$-, $-(CH_2)_3$-diazacycloheptanylene-$(CH_2)_5$-, $-(CH_2)_3$-diazacycloheptanylene-$(CH_2)_6$-, $-(CH_2)_3$-diazacycloheptanylene-$(CH_2)_7$-, $-(CH_2)_3$-diazacycloheptanylene-$(CH_2)_8$-, $-(CH_2)_4$-diazacycloheptanylene-$CH_2$-, $-(CH_2)_4$-diazacycloheptanylene-$(CH_2)_2$-, $-(CH_2)_4$-diazacycloheptanylene-$(CH_2)_3$-, $-(CH_2)_4$-diazacycloheptanylene-$(CH_2)_4$-, $-(CH_2)_4$-diazacycloheptanylene-$(CH_2)_5$-, $-(CH_2)_4$-diazacycloheptanylene-$(CH_2)_6$-, $-(CH_2)_4$-diazacycloheptanylene-$(CH_2)_7$-, $-(CH_2)_4$-diazacycloheptanylene-$(CH_2)_8$-, $-(CH_2)_5$-diazacycloheptanylene-$(CH_2)_1$-, $-(CH_2)_5$-diazacycloheptanylene-$(CH_2)_2$-, $-(CH_2)_5$-diazacycloheptanylene-$(CH_2)_3$-, $-(CH_2)_5$-diazacycloheptanylene-$(CH_2)_4$-, $-(CH_2)_5$-diazacycloheptanylene-$(CH_2)_5$-, $-(CH_2)_5$-diazacycloheptanylene-$(CH_2)_6$-, $-(CH_2)_5$-diazacycloheptanylene-$(CH_2)_7$-, $-(CH_2)_5$-diazacycloheptanylene-$(CH_2)_s$-, $-(CH_2)_6$-diazacycloheptanylene-$(CH_2)_1$-, $-(CH_2)_6$-diazacycloheptanylene-$(CH_2)_2$-, $-(CH_2)_6$-diazacycloheptanylene-$(CH_2)_3$-, $-(CH_2)_6$-diazacycloheptanylene-$(CH_2)_4$-, $-(CH_2)_6$-diazacycloheptanylene-$(CH_2)_5$-, $-(CH_2)_6$-diazacycloheptanylene-$(CH_2)_6$-, $-(CH_2)_6$-diazacycloheptanylene-$(CH_2)_7$-, $-(CH_2)_6$-diazacycloheptanylene-$(CH_2)_8$-, $-(CH_2)_7$-diazacycloheptanylene-$(CH_2)_1$-, $-(CH_2)_7$-diazacycloheptanylene-$(CH_2)_2$-, $-(CH_2)_7$-diazacycloheptanylene-$(CH_2)_3$-, $-(CH_2)_7$-diazacycloheptanylene-$(CH_2)_4$-, $-(CH_2)_7$-diazacycloheptanylene-$(CH_2)_8$-, $-(CH_2)_8$-diazacycloheptanylene-$CH_2$-, $-(CH_2)_8$-diazacycloheptanylene-$(CH_2)_2$-, $-(CH_2)_8$-diazacycloheptanylene-$(CH_2)_3$-, $-(CH_2)_8$-diazacycloheptanylene-$(CH_2)_4$-, $-(CH_2)_8$-diazacycloheptanylene-$(CH_2)_5$-, $-(CH_2)_8$-diazacycloheptanylene-$(CH_2)_6$-, $-(CH_2)_8$-diazacycloheptanylene-$(CH_2)_7$-, $-(CH_2)_8$-diazacycloheptanylene-$(CH_2)_8$-, $-CH_2$-diazabicyclo[2.2.1]heptanylene-$CH_2$-, $-CH_2$-diazabicyclo[2.2.1]heptanylene-$(CH_2)_2$-, $-CH_2$-diazabicyclo[2.2.1]heptanylene-$(CH_2)_3$-, $-CH_2$-diazabicyclo[2.2.1]heptanylene-$(CH_2)_4$-, $-CH_2$-diazabicyclo[2.2.1]heptanylene-$(CH_2)_5$-, $-CH_2$-diazabicyclo[2.2.1]heptanylene-$(CH_2)_6$-, $-CH_2$-diazabicyclo[2.2.1]heptanylene-$(CH_2)_7$-, $-CH_2$-diazabicyclo[2.2.1]heptanylene-$(CH_2)_8$-, $-(CH_2)_2$-diazabicyclo[2.2.1]heptanylene-$(CH_2)_1$-, $-(CH_2)_2$-diazabicyclo[2.2.1]heptanylene-$(CH_2)_2$-, $-(CH_2)_2$-diazabicyclo[2.2.1]heptanylene-$(CH_2)_3$-, $-(CH_2)_2$-diazabicyclo[2.2.1]heptanylene-$(CH_2)_4$-, $-(CH_2)_2$-diazabicyclo[2.2.1]heptanylene-$(CH_2)_5$-, $-(CH_2)_2$-diazabicyclo[2.2.1]heptanylene-$(CH_2)_6$-, $-(CH_2)_2$-diazabicyclo[2.2.1]heptanylene-$(CH_2)_7$-, $-(CH_2)_2$-diazabicyclo[2.2.1]heptanylene-$(CH_2)_8$-, $-(CH_2)_3$-diazabicyclo[2.2.1]heptanylene-$CH_2$-, $-(CH_2)_3$-diazabicyclo[2.2.1]heptanylene-$(CH_2)_2$-, $-(CH_2)_3$-diazabicyclo[2.2.1]heptanylene-$(CH_2)_3$-, $-(CH_2)_3$-diazabicyclo[2.2.1]heptanylene-$(CH_2)_4$-, $-(CH_2)_3$-diazabicyclo[2.2.1]heptanylene-$(CH_2)_5$-, $-(CH_2)_3$-diazabicyclo[2.2.1]heptanylene-$(CH_2)_6$-, $-(CH_2)_3$-diazabicyclo[2.2.1]heptanylene-$(CH_2)_7$-, $-(CH_2)_3$-diazabicyclo[2.2.1]heptanylene-$(CH_2)_8$-, $-(CH_2)_4$-diazabicyclo[2.2.1]heptanylene-$CH_2$-, $-(CH_2)_4$-diazabicyclo[2.2.1]heptanylene-$(CH_2)_2$-, $-(CH_2)_4$-diazabicyclo[2.2.1]heptanylene-$(CH_2)_3$-, $-(CH_2)_4$-diazabicyclo[2.2.1]heptanylene-$(CH_2)_4$-, $-(CH_2)_4$-diazabicyclo[2.2.1]heptanylene-$(CH_2)_5$-, $-(CH_2)_4$-diazabicyclo[2.2.1]heptanylene-$(CH_2)_6$-, $-(CH_2)_4$-diazabicyclo[2.2. I]heptanylene-$(CH_2)_7$-, $-(CH_2)_4$-diazabicyclo[2.2.1]heptanylene-$(CH_2)_8$-, $-(CH_2)_5$-diazabicyclo[2.2.1]heptanylene-$(CH_2)_1$-, $-(CH_2)_5$-diazabicyclo[2.2.1]heptanylene-$(CH_2)_2$-, $-(CH_2)_5$-diazabicyclo[2.2.1]heptanylene-$(CH_2)_3$-, $-(CH_2)_5$-diazabicyclo[2.2.1]heptanylene-$(CH_2)_4$-, $-(CH_2)_5$-diazabicyclo[2.2.1]heptanylene-$(CH_2)_5$-, $-(CH_2)_5$-diazabicyclo[2.2.1]heptanylene-$(CH_2)_6$-, $-(CH_2)_5$-diazabicyclo[2.2.1]heptanylene-$(CH_2)_7$-, $-(CH_2)_5$-diazabicyclo[2.2.1]heptanylene-$(CH_2)_8$-, $-(CH_2)_6$-diazabicyclo[2.2.1]heptanylene-$(CH_2)_1$-, $-(CH_2)_6$-diazabicyclo[2.2.1]heptanylene-$(CH_2)_2$-, $-(CH_2)_6$-diazabicyclo[2.2.1]heptanylene-$(CH_2)_3$-, $-(CH_2)_6$-diazabicyclo[2.2.1]heptanylene-$(CH_2)_4$-, $-(CH_2)_6$-diazabicyclo[2.2.1]heptanylene-$(CH_2)_5$-, $-(CH_2)_6$-diazabicyclo[2.2.1]heptanylene-$(CH)_6$-, $-(CH_2)_6$-diazabicyclo[2.2.1]heptanylene-$(CH_2)_7$-, $-(CH_2)_6$-diazabicyclo[2.2.1]heptanylene-$(CH_2)_8$-, $-(CH_2)_7$-diazabicyclo[2.2.1]heptanylene-$(CH_2)_1$-,

$-(CH_2)_7-$diazabicyclo[2.2.1]heptanylene-$(CH_2)_2-$, $-(CH_2)_7-$diazabicyclo[2.2.1]heptanylene-$(CH_2)_3-$, $-(CH_2)_7-$diazabicyclo [2.2.1]heptanylene-$(CH_2)_4-$, $-(CH_2)_7-$diazabicyclo[2.2.1]heptanylene-$(CH_2)_8-$, $-(CH_2)_8-$diazabicyclo[2.2.1]heptanylene-$CH_2-$, $-(CH_2)_8-$diazabicyclo[2.2.1]heptanylene-$(CH_2)_2-$, $-(CH_2)_8-$diazabicyclo[2.2.1]heptanylene-$(CH_2)_3-$, $-(CH_2)_8-$diazabicyclo[2.2.1]heptanylene-$(CH_2)_4-$, $-(CH_2)_8-$diazabicyclo[2.2.1]heptanylene-$(CH_2)_5-$, $-(CH_2)_8-$diazabicyclo[2.2.1]heptanylene-$(CH_2)_6-$, $-(CH_2)_8-$diazabicyclo[2.2.1]heptanylene-$(CH_2)_7-$, $-(CH_2)_8-$diazabicyclo[2.2.1]heptanylene-$(CH_2)_8-$, $-CH_2-$diazabicyclo[3.1.1]heptanylene-$CH_2-$, $-CH_2-$diazabicyclo[3.1.1]heptanylene-$(CH_2)_2-$, $-CH_2-$diazabicyclo[3.1.1] heptanylene-$(CH_2)_3-$, $-CH_2-$diazabicyclo[3.1.1]heptanylene-$(CH_2)_4-$, $-CH_2-$diazabicyclo[3.1.1]heptanylene-$(CH_2)_5-$, $-CH_2-$diazabicyclo[3.1.1]heptanylene-$(CH_2)_6-$, $-CH_2-$diazabicyclo[3.1.1]heptanylene-$(CH_2)_7-$, $-CH_2-$diazabicyclo[3.1.1] heptanylene-$(CH_2)_8-$, $-(CH_2)_2-$diazabicyclo[3.1.1]heptanylene-$(CH_2)_1-$, $-(CH_2)_2-$diazabicyclo[3.1.1]heptanylene-$(CH_2)_2-$, $-(CH_2)_2-$diazabicyclo[3. 1. 1]heptanylene-$(CH_2)_3-$, $-(CH_2)_2-$diazabicyclo[3.1.1]heptanylene-$(CH_2)_4-$, $-(CH_2)_2-$diazabicyclo[3.1.1]heptanylene-$(CH_2)_5-$, $-(CH_2)_2-$diazabicyclo[3.1.1]heptanylene-$(CH_2)_6-$, $-(CH_2)_2-$diazabicyclo[3.1.1]heptanylene-$(CH_2)_7-$, $-(CH_2)_2-$diazabicyclo[3.1.1]heptanylene-$(CH_2)_8-$, $-(CH_2)_3-$diazabicyclo[3.1.1]heptanylene-$CH_2-$, $-(CH_2)_3-$diazabicyclo[3.1.1]heptanylene-$(CH_2)_2-$, $-(CH_2)_3-$diazabicyclo[3.1.1]heptanylene-$(CH_2)_3-$, $-(CH_2)_3-$diazabicyclo[3.1.1]heptanylene-$(CH_2)_4-$, $-(CH_2)_3-$diazabicyclo[3.1.1]heptanylene-$(CH_2)_5-$, $-(CH_2)_3-$diazabicyclo[3.1.1] heptanylene-$(CH_2)_6-$, $-(CH_2)_3-$diazabicyclo[3.1.1]heptanylene-$(CH_2)_7-$, $-(CH_2)_3-$diazabicyclo[3.1.1]heptanylene-$(CH_2)_8-$, $-(CH_2)_4-$diazabicyclo[3.1.1]heptanylene-$CH_2-$, $-(CH_2)_4-$diazabicyclo[3.1.1]heptanylene-$(CH_2)_2-$, $-(CH_2)_4-$diazabicyclo[3.1.1]heptanylene-$(CH_2)_3-$, $-(CH_2)_4-$diazabicyclo[3.1.1]heptanylene-$(CH_2)_4-$, $-(CH_2)_4-$diazabicyclo[3. 1. 1] heptanylene-$(CH_2)_5-$, $-(CH_2)_4-$diazabicyclo[3.1.1]heptanylene-$(CH_2)_6-$, $-(CH_2)_4-$diazabicyclo[3. 1. 1]heptanylene-$(CH_2)_7-$, $-(CH_2)_4-$diazabicyclo[3.1.1]heptanylene-$(CH_2)_8-$, $-(CH_2)_5-$diazabicyclo[3.1.1]heptanylene-$(CH_2)_1-$, $-(CH_2)_5-$diazabicyclo[3.1.1]heptanylene-$(CH_2)_2-$, $-(CH_2)_5-$diazabicyclo[3.1.1]heptanylene-$(CH_2)_3-$, $-(CH_2)_5-$diazabicyclo[3.1.1]heptanylene-$(CH_2)_4-$, $-(CH_2)_5-$diazabicyclo[3.1.1]heptanylene-$(CH_2)_5-$, $-(CH_2)_5-$diazabicyclo[3.1.1]heptanylene-$(CH_2)_6-$, $-(CH_2)_5-$diazabicyclo[3.1.1]heptanylene-$(CH_2)_7-$, $-(CH_2)_5-$diazabicyclo[3.1.1]heptanylene-$(CH_2)_8-$, $-(CH_2)_6-$diazabicyclo[3.1.1]heptanylene-$(CH_2)_1-$, $-(CH_2)_6-$diazabicyclo[3.1.1]heptanylene-$(CH_2)_2-$, $-(CH_2)_6-$diazabicyclo[3.1.1] heptanylene-$(CH_2)_3-$, $-(CH_2)_6-$diazabicyclo[3.1.1]heptanylene-$(CH_2)_4-$, $-(CH_2)_6-$diazabicyclo[3.1.1]heptanylene-$(CH_2)_5-$, $-(CH_2)_6-$diazabicyclo[3.1.1]heptanylene-$(CH_2)_6-$, $-(CH_2)_6-$diazabicyclo[3.1.1]heptanylene-$(CH_2)_7-$, $-(CH_2)_6-$diazabicyclo[3.1.1]heptanylene-$(CH_2)_8-$, $-(CH_2)_7-$diazabicyclo[3.1.1]heptanylene-$(CH_2)_1-$, $-(CH_2)_7-$diazabicyclo[3.1.1]heptanylene-$(CH_2)_2-$, $-(CH_2)_7-$diazabicyclo[3.1.1]heptanylene-$(CH_2)_3-$, $-(CH_2)_7-$diazabicyclo[3.1.1]heptanylene-$(CH_2)_4-$, $-(CH_2)_7-$diazabicyclo[3.1.1]heptanylene-$(CH_2)_8-$, $-(CH_2)_8-$diazabicyclo[3.1.1]heptanylene-$CH_2-$, $-(CH_2)_8-$diazabicyclo[3.1.1]heptanylene-$(CH_2)_2-$, $-(CH_2)_8-$diazabicyclo[3.1.1]heptanylene-$(CH_2)_3-$, $-(CH_2)_8-$diazabicyclo[3.1.1] heptanylene-$(CH_2)_4-$, $-(CH_2)_8-$diazabicyclo[3.1.1]heptanylene-$(CH_2)_5-$, $-(CH_2)_8-$diazabicyclo[3.1.1]heptanylene-$(CH_2)_6-$, $-(CH_2)_8-$diazabicyclo[3.1.1]heptanylene-$(CH_2)_7-$, $-(CH_2)_8-$diazabicyclo[3.1.1]heptanylene-$(CH_2)_8-$, $-CH_2-$diazabicyclo[3.2.1]octylene-$CH_2-$, $-CH_2-$diazabicyclo[3.2.1]octylene-$(CH_2)_2-$, $-CH_2-$diazabicyclo[3.2.1]octylene-$(CH_2)_3-$, $-CH_2-$diazabicyclo[3.2.1]octylene-$(CH_2)_4-$, $-CH_2-$diazabicyclo[3.2.1]octylene-$(CH_2)_5-$, $-CH_2-$diazabicyclo[3.2.1]octylene-$(CH_2)_6-$, $-CH_2-$diazabicyclo[3.2.1]octylene-$(CH_2)_7-$, $-CH_2-$diazabicyclo[3.2.1]octylene-$(CH_2)_8-$, $-(CH_2)_2-$diazabicyclo [3.2.1]octylene-$(CH_2)_1-$, $-(CH_2)_2-$diazabicyclo[3.2.1]octylene-$(CH_2)_2-$, $-(CH_2)_2-$diazabicyclo[3.2.1]octylene-$(CH_2)_3-$, $-(CH_2)_2-$diazabicyclo[3.2.1]octylene-$(CH_2)_4-$, $-(CH_2)_2-$diazabicyclo[3.2.1]octylene-$(CH_2)_5-$, $-(CH_2)_2-$diazabicyclo[3.2.1] octylene-$(CH_2)_6-$, $-(CH_2)_2-$diazabicyclo[3.2.1]octylene-$(CH_2)_7-$, $-(CH_2)_2-$diazabicyclo[3.2.1]octylene-$(CH_2)_8-$, $-(CH_2)_3-$diazabicyclo[3.2.1]octylene-$CH_2-$, $-(CH_2)_3-$diazabicyclo[3.2.1]octylene-$(CH_2)_2-$, $-(CH_2)_3-$diazabicyclo[3.2.1]octylene-$(CH_2)_3-$, $-(CH_2)_3-$diazabicyclo[3.2.1]octylene-$(CH_2)_4-$, $-(CH_2)_3-$diazabicyclo[3.2.1]octylene-$(CH_2)_5-$, $-(CH_2)_3-$diazabicyclo[3.2.1]octylene-$(CH_2)_6-$, $-(CH_2)_3-$diazabicyclo[3.2.1]octylene-$(CH_2)_7-$, $-(CH_2)_3-$diazabicyclo[3.2.1]octylene-$(CH_2)_8-$, $-(CH_2)_4-$diazabicyclo[3.2.1]octylene-$CH_2-$, $-(CH_2)_4-$diazabicyclo[3.2.1]octylene-$(CH_2)_2-$, $-(CH_2)_4-$diazabicyclo[3.2.1]octylene-$(CH_2)_3-$, $-(CH_2)_4-$diazabicyclo[3.2.1]octylene-$(CH_2)_4-$, $-(CH_2)_4-$diazabicyclo[3.2.1]octylene-$(CH_2)_5-$, $-(CH_2)_4-$diazabicyclo[3.2.1]octylene-$(CH_2)_6-$, $-(CH_2)_4-$diazabicyclo[3.2.1]octylene-$(CH_2)_7-$, $-(CH_2)_4-$diazabicyclo[3.2.1] octylene-$(CH_2)_8-$, $-(CH_2)_5-$diazabicyclo[3.2.1]octylene-$(CH_2)_1-$, $-(CH_2)_5-$diazabicyclo[3.2.1]octylene-$(CH_2)_2-$, $-(CH_2)_5-$diazabicyclo[3.2.1]octylene-$(CH_2)_3-$, $-(CH_2)_5-$diazabicyclo[3.2.1]octylene-$(CH_2)_4-$, $-(CH_2)_5-$diazabicyclo[3.2.1]octylene-$(CH_2)_5-$, $-(CH_2)_5-$diazabicyclo[3.2.1]octylene-$(CH_2)_6-$, $-(CH_2)_5-$diazabicyclo[3.2.1]octylene-$(CH_2)_7-$, $-(CH_2)_5-$diazabicyclo[3.2.1]octylene-$(CH_2)_8-$, $-(CH_2)_6-$diazabicyclo[3.2.1]octylene-$(CH_2)_1-$, $-(CH_2)_6-$diazabicyclo[3.2.1]octylene-$(CH_2)_2-$, $-(CH_2)_6-$diazabicyclo[3.2.1]octylene-$(CH_2)_3-$, $-(CH_2)_6-$diazabicyclo[3.2.1]octylene-$(CH_2)_4-$, $-(CH_2)_6-$diazabicyclo[3.2.1]octylene-$(CH_2)_5-$, $-(CH_2)_6-$diazabicyclo[3.2.1]octylene-$(CH_2)_6-$, $-(CH_2)_6-$diazabicyclo[3.2.1]octylene-$(CH_2)_7-$, $-(CH_2)_6-$diazabicyclo[3.2.1]octylene-$(CH_2)_8-$, $-(CH_2)_7-$diazabicyclo[3.2.1]octylene-$(CH_2)_1-$, $-(CH_2)_7-$diazabicyclo[3.2.1]octylene-$(CH_2)_2-$, $-(CH_2)_7-$diazabicyclo[3.2.1]octylene-$(CH_2)_3-$, $-(CH_2)_7-$diazabicyclo[3.2.1]octylene-$(CH_2)_4-$, $-(CH_2)_7-$diazabicyclo[3.2.1]octylene-$(CH_2)_8-$, $-(CH_2)_8-$diazabicyclo[3.2.1l]octylene-$CH_2-$, $-(CH_2)_8-$diazabicyclo[3.2.1]octylene-$(CH_2)_2-$, $-(CH_2)_8-$diazabicyclo[3.2.1]octylene-$(CH_2)_3-$, $-(CH_2)_8-$diazabicyclo[3.2.1]octylene-$(CH_2)_4-$, $-(CH_2)_8-$diazabicyclo[3.2.1]octylene-$(CH_2)_5-$, $-(CH_2)_8-$diazabicyclo[3.2.1]octylene-$(CH_2)_6-$, $-(CH_2)_8-$diazabicyclo[3.2.1] octylene-$(CH_2)_7-$, $-(CH_2)_8-$diazabicyclo[3.2.1]octylene-$(CH_2)_8-$, $-CH_2-$diazabicyclo[2.2.2]octylene-$CH_2-$, $-CH_2-$diazabicyclo[2.2.2]octylene-$(CH_2)_2-$, $-CH_2-$diazabicyclo[2.2.2]octylene-$(CH_2)_3-$, $-CH_2-$diazabicyclo[2.2.2]octylene-$(CH_2)_4-$, $-CH_2-$diazabicyclo[2.2.2]octylene-$(CH_2)_5-$, $-CH_2-$diazabicyclo[2.2.2]octylene-$(CH_2)_6-$, $-CH_2-$diazabicyclo[2.2.2]octylene-$(CH_2)_7-$, $-CH_2-$diazabicyclo[2.2.2]octylene-$(CH_2)_8-$, $-(CH_2)_2-$diazabicyclo[2.2.2]octylene-$(CH_2)_1-$, $-(CH_2)_2-$diazabicy-

clo[2.2.2]octylene-$(CH_2)_2$-, -$(CH_2)_2$-diazabicyclo[2.2.2]octylene-$(CH_2)_3$-, -$(CH_2)_2$-diazabicyclo[2.2.2]octylene-$(CH_2)_4$-, -$(CH_2)_2$-diazabicyclo[2.2.2]octylene-$(CH_2)_5$-, -$(CH_2)_2$-diazabicyclo[2.2.2]octylene-$(CH_2)_6$-, -$(CH_2)_2$-diazabicyclo[2.2.2]octylene-$(CH_2)_7$-, -$(CH_2)_2$-diazabicyclo[2.2.2]octylene-$(CH_2)_8$-, -$(CH_2)_3$-diazabicyclo[2.2.2]octylene-$CH_2$-, -$(CH_2)_3$-diazabicyclo[2.2.2]octylene-$(CH_2)_2$-, -$(CH_2)_3$-diazabicyclo[2.2.2]octylene-$(CH_2)_3$-, -$(CH_2)_3$-diazabicyclo[2.2.2]octylene-$(CH_2)_4$-, -$(CH_2)_3$-diazabicyclo[2.2.2]octylene-$(CH_2)_5$-, -$(CH_2)_3$-diazabicyclo[2.2.2]octylene-$(CH_2)_6$-, -$(CH_2)_3$-diazabicyclo[2.2.2]octylene-$(CH_2)_7$-, -$(CH_2)_3$-diazabicyclo[2.2.2]octylene-$(CH_2)_8$-, -$(CH_2)_4$-diazabicyclo[2.2.2]octylene-$CH_2$-, -$(CH_2)_4$-diazabicyclo[2.2.2]octylene-$(CH_2)_2$-, -$(CH_2)_4$-diazabicyclo[2.2.2]octylene-$(CH_2)_3$-, -$(CH_2)_4$-diazabicyclo[2.2.2]octylene-$(CH_2)_4$-, -$(CH_2)_4$-diazabicyclo[2.2.2]octylene-$(CH_2)_5$-, -$(CH_2)_4$-diazabicyclo[2.2.2]octylene-$(CH_2)_6$-, -$(CH_2)_4$-diazabicyclo[2.2.2]octylene-$(CH_2)_7$-, -$(CH_2)_4$-diazabicyclo[2.2.2]octylene-$(CH_2)_8$-, -$(CH_2)_5$-diazabicyclo[2.2.2]octylene-$(CH_2)_1$-, -$(CH_2)_5$-diazabicyclo[2.2.2]octylene-$(CH_2)_2$-, -$(CH_2)_5$-diazabicyclo[2.2.2]octylene-$(CH_2)_3$-, -$(CH_2)_5$-diazabicyclo[2.2.2]octylene-$(CH_2)_4$-, -$(CH_2)_5$-diazabicyclo[2.2.2]octylene-$(CH_2)_5$-, -$(CH_2)_5$-diazabicyclo[2.2.2]octylene-$(CH_2)_6$-, -$(CH_2)_5$-diazabicyclo[2.2.2]octylene-$(CH_2)_7$-, -$(CH_2)_5$-diazabicyclo[2.2.2]octylene-$(CH_2)_8$-, -$(CH_2)_6$-diazabicyclo[2.2.2]octylene-$(CH_2)_1$-, -$(CH_2)_6$-diazabicyclo[2.2.2]octylene-$(CH_2)_2$-, -$(CH_2)_6$-diazabicyclo[2.2.2]octylene-$(CH_2)_3$-, -$(CH_2)_6$-diazabicyclo[2.2.2]octylene-$(CH_2)_4$-, -$(CH_2)_6$-diazabicyclo[2.2.2]octylene-$(CH_2)_5$-, -$(CH_2)_6$-diazabicyclo[2.2.2]octylene-$(CH_2)_6$-, -$(CH_2)_6$-diazabicyclo[2.2.2]octylene-$(CH_2)_7$-, -$(CH_2)_6$-diazabicyclo[2.2.2]octylene-$(CH_2)_8$-, -$(CH_2)_7$-diazabicyclo[2.2.2]octylene-$(CH_2)_1$-, -$(CH_2)_7$-diazabicyclo[2.2.2]octylene-$(CH_2)_2$-, -$(CH_2)_7$-diazabicyclo[2.2.2]octylene-$(CH_2)_3$-, -$(CH_2)_7$-diazabicyclo[2.2.2]octylene-$(CH_2)_4$-, -$(CH_2)_7$-diazabicyclo[2.2.2]octylene-$(CH_2)_8$-, -$(CH_2)_8$-diazabicyclo[2.2.2]octylene-$CH_2$-, -$(CH_2)_8$-diazabicyclo[2.2.2]octylene-$(CH_2)_2$-, -$(CH_2)_8$-diazabicyclo[2.2.2]octylene-$(CH_2)_3$-, -$(CH_2)_8$-diazabicyclo[2.2.2]octylene-$(CH_2)_4$-, -$(CH_2)_8$-diazabicyclo[2.2.2]octylene-$(CH_2)_5$-, -$(CH_2)_8$-diazabicyclo[2.2.2]octylene-$(CH_2)_6$-, -$(CH_2)_8$-diazabicyclo[2.2.2]octylene-$(CH_2)_7$-, or -$(CH_2)_8$-diazabicyclo[2.2.2]octylene-$(CH_2)_8$-;

wherein the propylene, the piperidinylene, the piperazinylene, the diazacycloheptanylene, the diazabicyclo[2.2.1]heptanylene, the diazabicyclo[3.1.1]heptanylene, the diazabicyclo[3.2.1]octylene, the diazabicyclo[2.2.2]octylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof; and

each $R_{g11}$ independently represents H or $C_{1-3}$ alkyl.

In some embodiments of the compound of Formula (I-7), $L_2$ represents the structure of the following formula:

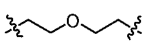 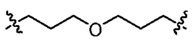 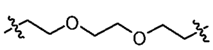 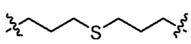 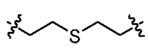 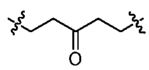

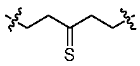 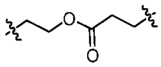 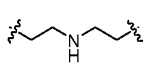 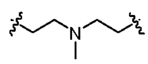 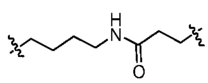 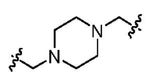 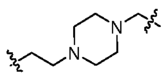

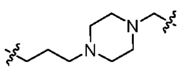 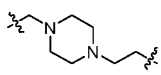 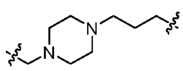 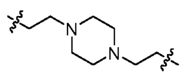 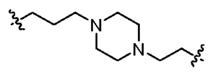 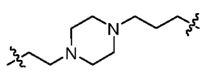

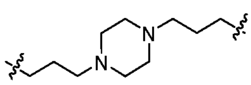 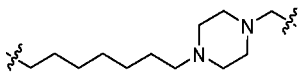 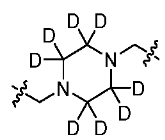 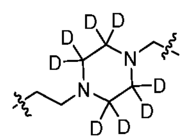 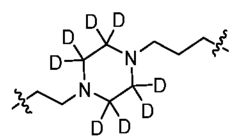

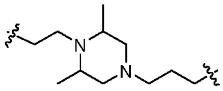 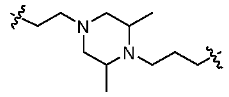 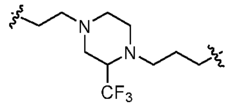 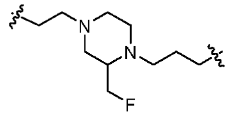 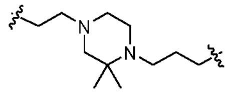

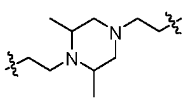 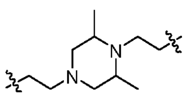 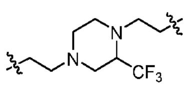 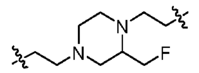 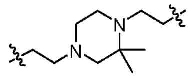 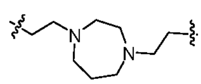

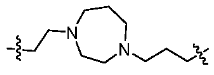 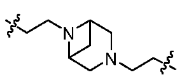 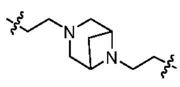 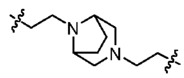 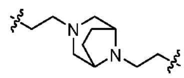 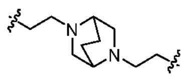

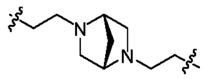 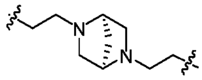 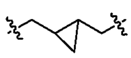 or 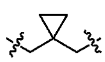

In some embodiments of the compound of Formula (I-7), $R_{b1}$ represents:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, 2-oxobicyclo[2.2.1]heptanyl or bicyclo[2.2.1]heptenyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 3-azaspiro[5.5]undecyl or 7-azaspiro[3.5]nonyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

phenyl or naphthyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl and imidazo[2,1-b]thiazolyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$

alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

In some embodiments of the compound of Formula (I-7), $R_{b1}$ represents:

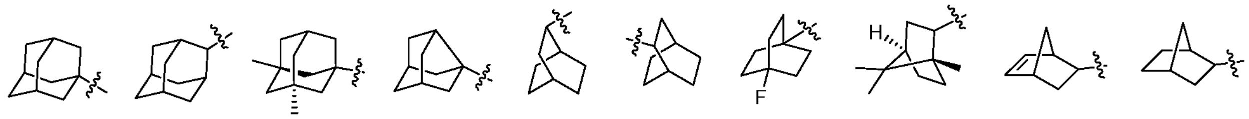

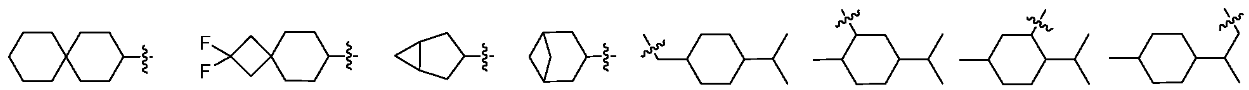

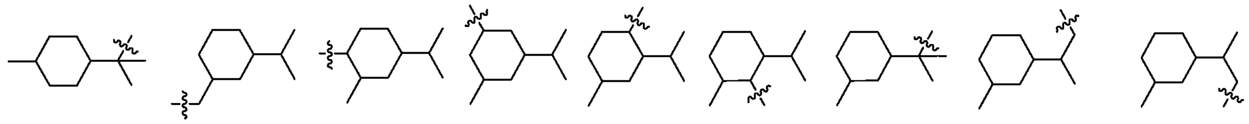

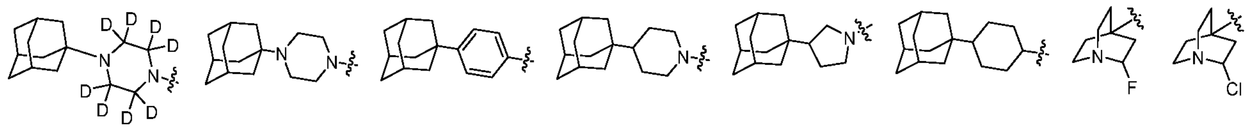

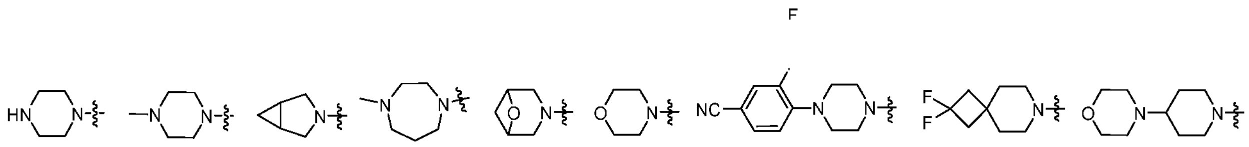

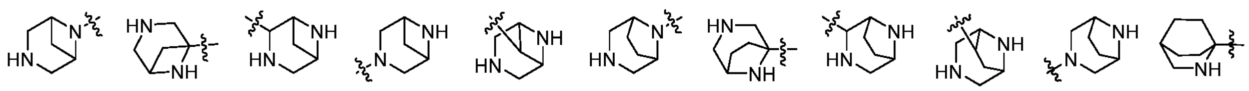

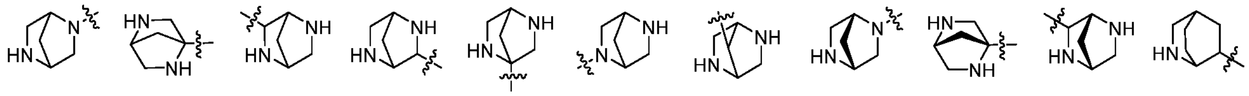

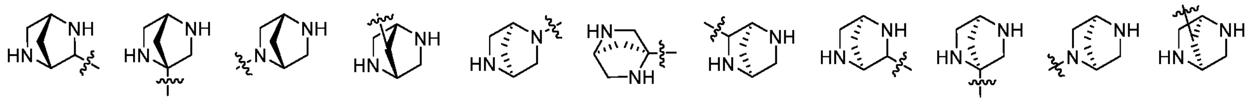

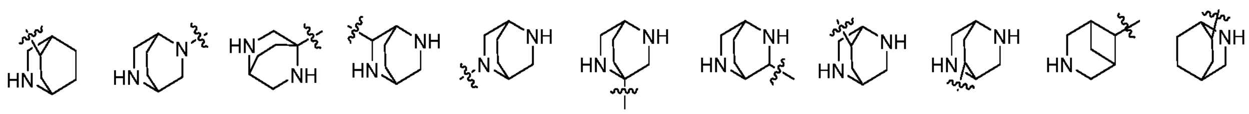

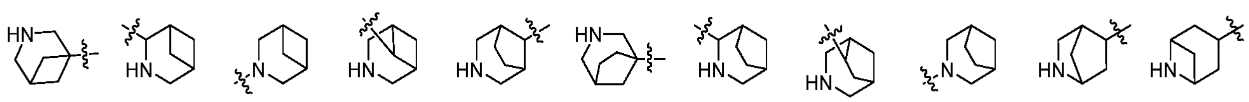

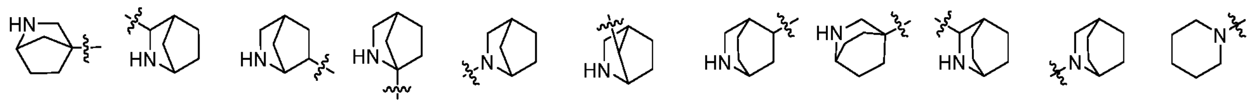

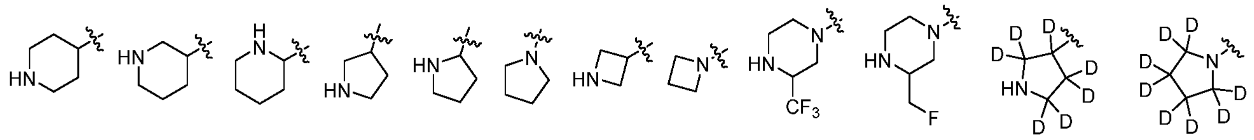

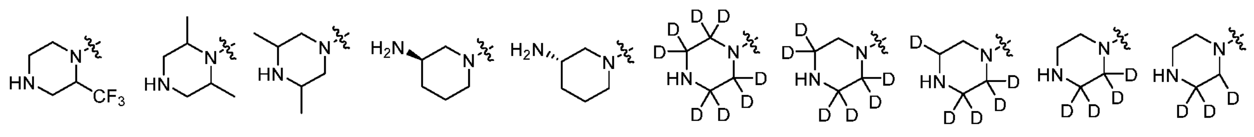

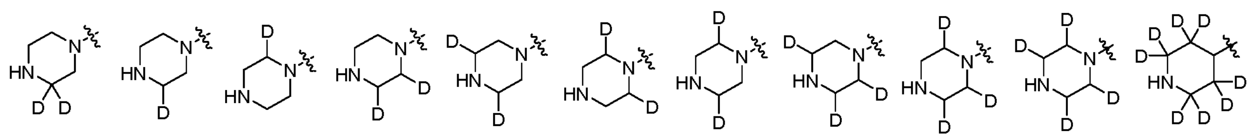

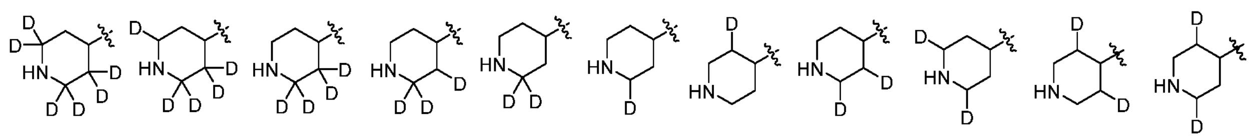

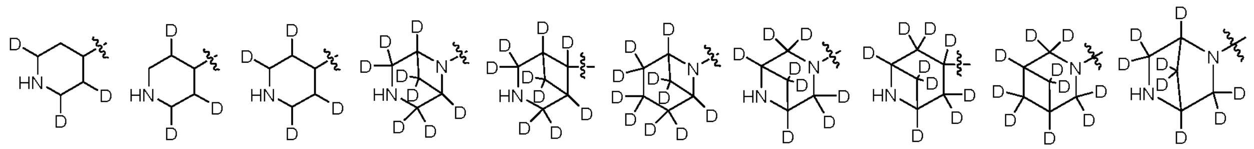

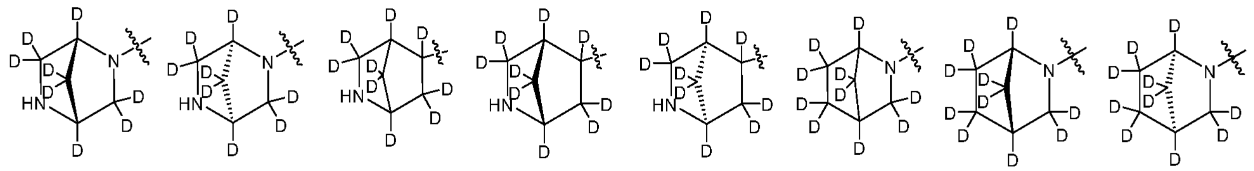

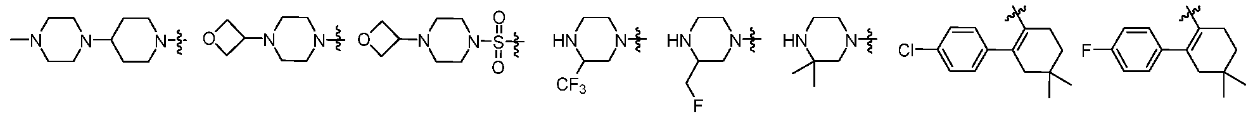

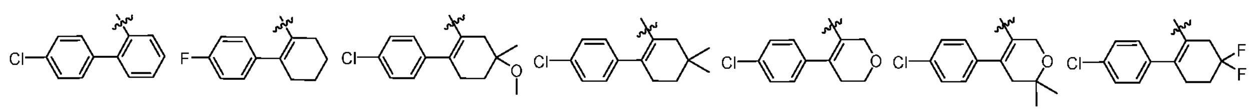

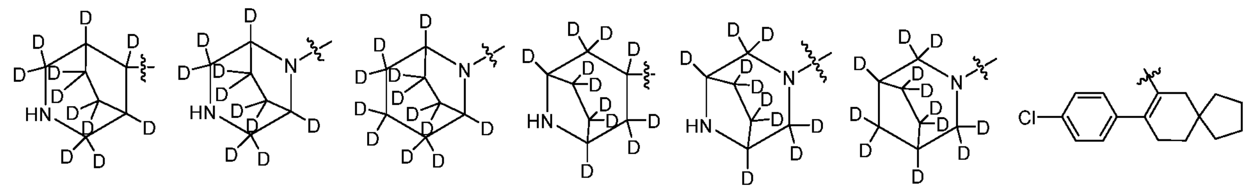

or

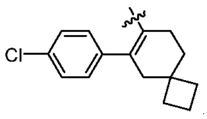

In some embodiments, the compound of Formula (I) is also of Formula (I-8):

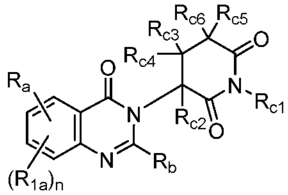

Formula (I-8)

wherein $R_{c1}$, $R_{c2}$, $R_{c3}$, $R_{c4}$, $R_{c5}$, $R_{c6}$, $R_b$, $(R_{1a})_n$, $R_a$, $R_{1a}$ and n are as defined in the compound of Formula (I) above and the embodiments thereof;
wherein $R_a$ represents halogen or cyano, and
$R_b$ represents the following formula:

$R_{b2}$-$X_5$-$L_3$-,

wherein $L_3$ represents:

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene), or

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{16}$ and/or one or more (e.g., 1-20, or 1-15, 1-10, 1-9, 1-8, 1-7, 1-6, 1-5, 1-4, 1-3 or 1-2, or 1) groups $R_{17}$ and/or any combination of one or more (e.g., 1-20, or 1-15, 1-10, 1-9, 1-8, 1-7, 1-6, 1-5, 1-4, 1-3 or 1-2, or 1) groups $R_{16}$ and $R_{17}$ inserted into its backbone carbon chain, wherein carbon-carbon bond between one or more pairs (e.g., 1-20, or 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_{16}$, $R_{17}$ or a combination of $R_{16}$ and $R_{17}$; wherein each $R_{16}$ is independently selected from the group consisting of O, S, N($R_{18}$), C(O), C(O)O, OC(O), C(O)NH, NHC(O), NHC(O)NH, C(=S), S(O), $S(O)_2$, $S(O)_2O$, $OS(O)_2$, $S(O)_2NH$ or $NHS(O)_2$, wherein $R_{18}$ represents H or $C_{1-3}$ alkyl, and in case that two or more groups $R_{16}$ are inserted into the backbone carbon chain of the linear or branched $C_{2-60}$ alkylene group, the two or more groups $R_{16}$ are not directly connected to each other; and wherein each $R_{17}$ is independently selected from the group consisting of cycloalkylene (e.g., $C_{3-20}$cycloalkylene, or $C_{3-15}$cycloalkylene), heterocyclylene e.g., 4- to 20-membered heterocyclylene, or 5- to 15-membered heterocyclylene), arylene (e.g., $C_{6-10}$ arylene), heteroarylene (e.g., 5- to 20-membered heteroarylene, or 5- to 15-membered heteroarylene), alkenylene (e.g., $C_{2-6}$ alkenylene), alkynylene (e.g., $C_{2-6}$ alkynylene) or any combination thereof, wherein the cycloalkylene, the heterocyclylene, arylene and the heteroarylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof;

$X_5$ represents N($R_{19}$), C(O)O, OC(O), C(O)NH, NHC(O), C(O), O, C(S), S, S(O), $S(O)_2$, $S(O)_2NH$, $NHS(O)_2$,

$S(O)_2O$, or $OS(O)_2$, wherein $R_{19}$ represents H or $C_{1-3}$ alkyl, or $X_5$ represents a bond; and

$R_{b2}$ represents optionally substituted cycloalkyl (e.g., optionally substituted $C_{3-20}$cycloalkyl, or optionally substituted $C_{3-15}$cycloalkyl), optionally substituted aryl (e.g., optionally substituted $C_{6-20}$ aryl, or optionally substituted $C_{6-10}$ aryl), optionally substituted heterocyclyl (e.g., optionally substituted 4- to 20-membered heterocyclyl, or optionally substituted 4- to 15-membered heterocyclyl) or optionally substituted heteroaryl (e.g., optionally substituted 5- to 20-membered heteroaryl, or optionally substituted 5- to 15-membered heteroaryl), or $R_{b2}$ represents the following formula:

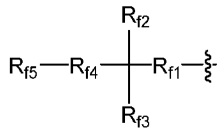

,

wherein $R_{f1}$, $R_{f2}$, $R_{f3}$, $R_{f4}$ and $R_{f5}$ are as defined in the compound of Formula (I) or Formula (I-2) above and their embodiments.

In some embodiments of the compound of Formula (I-8), $R_a$ represents halogen.

In some embodiments of the compound of Formula (I-8), $R_a$ represents cyano.

In some embodiments of the compound of Formula (I-8), $R_a$ represents cyano.

In some embodiments of the compound of Formula (I-8), $R_b$ represents the following formula:

$R_{b2}$-$X_5$-$L_3$-,

wherein $L_3$ represents:

linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, $C_{1-4}$ alkyl, halogen, $C_{3-6}$cycloalkyl or any combination thereof, or

optionally substituted linear or branched $C_{2-60}$ alkylene (e.g., $C_2$-$C_{40}$ alkylene, $C_2$-$C_{30}$ alkylene, $C_2$-$C_{29}$ alkylene, $C_2$-$C_{28}$ alkylene, $C_2$-$C_{27}$ alkylene, $C_2$-$C_{26}$ alkylene, $C_2$-$C_{25}$ alkylene, $C_2$-$C_{24}$ alkylene, $C_2$-$C_{23}$ alkylene, $C_2$-$C_{22}$ alkylene, $C_2$-$C_{21}$ alkylene, $C_2$-$C_{20}$ alkylene, $C_2$-$C_{19}$ alkylene, $C_2$-$C_{18}$ alkylene, $C_2$-$C_{17}$ alkylene, $C_2$-$C_{16}$ alkylene, $C_2$-$C_{15}$ alkylene, $C_2$-$C_{14}$ alkylene, $C_2$-$C_{13}$ alkylene, $C_2$-$C_{12}$ alkylene, $C_2$-$C_{11}$ alkylene, $C_2$-$C_{10}$ alkylene, $C_2$-$C_9$ alkylene, $C_2$-$C_8$ alkylene, $C_2$-$C_7$ alkylene, $C_2$-$C_6$ alkylene, $C_2$-$C_5$ alkylene, $C_2$-$C_4$ alkylene, $C_2$-$C_3$ alkylene, or ethylene) with one or more (e.g., 1-20, or 1-15, 1-10, 1-9, 1-8, 1-7, 1-6, 1-5, 1-4, 1-3 or 1-2, or 1) groups $R_{16}$ and/or one or more (e.g., 1-20, or 1-15, 1-10, 1-9, 1-8, 1-7, 1-6, 1-5, 1-4, 1-3 or 1-2, or 1) groups $R_{17}$ and/or any combination of one or more (e.g., 1-20, or 1-15, 1-10, 1-9, 1-8, 1-7, 1-6, 1-5, 1-4, 1-3 or 1-2, or 1) groups $R_{16}$ and $R_{17}$ inserted into its backbone carbon chain; wherein carbon-carbon bond between one or more pairs (e.g., 1-20, or 1-15, 1-10, 1-9, 1-8, 1-7, 1-6, 1-5, 1-4, 1-3 or 1-2, or 1 pair) of adjacent carbon atoms in the backbone carbon chain is interrupted by the group $R_{16}$, $R_{17}$, or a combination of $R_{16}$ and $R_{17}$; wherein each $R_{16}$ is independently selected from the group consisting of O, S, $N(R_{18})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{18}$ represents H or $C_{1-3}$ alkyl, and in case that two or more groups $R_{16}$ are inserted into the backbone carbon chain of the linear or branched $C_{2-60}$ alkylene group, the two or more groups $R_{16}$ are not directly connected to each other; and wherein each $R_{17}$ is independently selected from the group consisting of $C_{3-15}$cycloalkylene, 4- to 15-membered heterocyclylene, $C_{6-10}$ arylene, 5- to 15-membered heteroarylene, $C_{2-6}$ alkenylene, $C_{2-6}$ alkynylene or any combination thereof, wherein the $C_{3-15}$cycloalkylene, the 4- to 15-membered heterocyclylene, $C_{6-10}$ arylene and the 5- to 15-membered heteroarylene are each independently optionally substituted with a substituent selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl (e.g., optionally perdeuterated cyclopropyl, optionally perdeuterated cyclobutyl, optionally perdeuterated cyclopentyl or optionally perdeuterated cyclohexyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-,

$CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$-and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof, and the linear or branched $C_{2-60}$ alkylene is optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, $C_{1-4}$ alkyl, halogen, $C_{3-6}$cycloalkyl or any combination thereof;

$X_5$ represents N($R_{19}$), C(O)O, OC(O), C(O)NH, NHC(O), C(O), O, C(S), S, S(O), $S(O)_2$, $S(O)_2NH$, $NHS(O)_2$, $S(O)_2O$, or $OS(O)_2$, wherein $R_{19}$ represents H or $C_{1-3}$ alkyl, or $X_5$ represents a bond; and

$R_{b2}$ represents optionally substituted $C_{3-20}$cycloalkyl, optionally substituted $C_{6-20}$ aryl, optionally substituted 4- to 20-membered heterocyclyl or optionally substituted 5- to 20-membered heteroaryl, wherein the $C_{3-20}$cycloalkyl, the $C_{6-20}$ aryl, the 4- to 20-membered heterocyclyl, and the 5- to 20-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-), or any combination thereof; or

$R_{b2}$ represents the following formula:

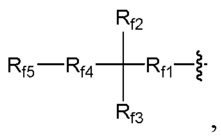

,

wherein $R_{f1}$, $R_{f2}$, $R_{f3}$, $R_{f4}$ and $R_{f5}$ are as defined in the compound of Formula (I) or Formula (I-2) above and their embodiments.

In some embodiments of the compound of Formula (I-8), $L_3$ represents the structure of the following formula: -$C_{2-30}$ alkylene-, wherein a hydrogen atom of one or more (e.g., 1-20, or 1-15, 1-10, 1-9, 1-8, 1-7, 1-6, 1-5, 1-4, 1-3 or 1-2, or 1) $CH_2$ groups of the $C_{2-30}$ alkylene is optionally replaced with a substituent selected from the group consisting of: D, $C_{1-4}$ alkyl, halogen, $C_{3-6}$cycloalkyl or any combination thereof.

In some embodiments of the compound of Formula (I-8), $L_3$ represents the following group:

-$CH_2$-, -$(CH_2)_2$-, -$(CH_2)_3$-, -$(CH_2)_4$-, -$(CH_2)_5$-, -$(CH_2)_2$-$CF_2$-$(CH_2)_2$-, -$(CH_2)_6$-, -$(CH_2)_7$-, -$(CH_2)_8$-, - $(CH_2)_9$-, -$(CH_2)_{10}$-, -$(CH_2)_{11}$-, -$(CH_2)_{12}$-, -$(CH_2)_{13}$-, -$(CH_2)_{14}$-, -$(CH_2)_{15}$-, -$(CH_2)_{16}$-, -$(CH_2)_{17}$-, - $(CH_2)_{18}$-, -$(CH_2)_{19}$-, -$(CH_2)_{20}$-, -$(CH_2)_{21}$-, -$(CH_2)_{22}$-, -$(CH_2)_{25}$-, or -$(CH_2)_{30}$-;

wherein a hydrogen atom of one or more (e.g., 1-20, or 1-15, 1-10, 1-9, 1-8, 1-7, 1-6, 1-5, 1-4, 1-3 or 1-2, or 1) $CH_2$ of the group is optionally replaced with a substituent selected from the group consisting of: D, $C_{1-4}$ alkyl, halogen, $C_{3-6}$cycloalkyl or any combination thereof.

In some embodiments of the compound of Formula (I-8), $L_3$ represents the structure of the following formula:

$-(C(R^{a9})(R^{a10}))_{m1}-(R_{16}(C(R^{a11})(R^{a12}))_{m2})_{p1}-$;

$-(C(R^{a9})(R^{a10}))_{m1}-(R_{16}(C(R^{a11})(R^{a12}))_{m2})_{p1}-(R_{16}(C(R^{a13})(R^{a14}))_{m3})_{p2}-$;

$-(C(R^{a9})(R^{a10}))_{m1}-(R_{16}(C(R^{a11})(R^{a12}))_{m2})_{p1}-(R_{16}(C(R^{a13})(R^{a14}))_{m3})_{p2}-(R_{16}(C(R^{a15})(R^{a16}))_{m4})_{p3}-$;

$-(C(R^{a9})(R^{a10}))_{m1}-(R_{17}(C(R^{a11})(R^{a12}))_{m2})_{p1}-$;

$-(C(R^{a9})(R^{a10}))_{m1}-(R_{17}(C(R^{a11})(R^{a12}))_{m2})_{p1}-(R_{17}(C(R^{a13})(R^{a14}))_{m3})_{p2}-$;

$-(C(R^{a9})(R^{a10}))_{m1}-(R_{17}(C(R^{a11})(R^{a12}))_{m2})_{p1}-(R_{17}(C(R^{a13})(R^{a14}))_{m3})_{p2}-(R_{17}(C(R^{a15})(R^{a16}))_{m4})_{p3}-$;

$-(C(R^{a9})(R^{a10}))_{m1}-(R_{16}-R_{17}-(C(R^{a11})(R^{a12}))_{m2})_{p1}-$;

$-(C(R^{a9})(R^{a10}))_{m1}-(R_{16}(C(R^{a11})(R^{a12})_{m2})_{p1}-(R_{17}(C(R^{a13})(R^{a14}))_{m3})_{p2}-$;

$-(C(R^{a9})(R^{a10}))_{m1}-(R_{17}-R_{16}-(C(R^{a11})(R^{a12})_{p1}-$; or

$-(C(R^{a9})(R^{a10}))_{m1}-(R_{17}(C(R^{a11})(R^{a12}))_{m2})_{p1}-(R_{16}(C(R^{a13})(R^{a14}))_{m3})_{p2}-$;

wherein each group $R_{16}$ is independently selected from the group consisting of O, S, $N(R_{18})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_{18}$ independently represents H or $C_{1-3}$ alkyl; and each group $R_{17}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl (e.g., optionally deuterated $C_{1-3}$ alkyl, such as methyl, $CD_3$, ethyl, propyl, isopropyl, butyl, sec-butyl or tert-butyl), optionally deuterated $C_{3-6}$cycloalkyl, hydroxy, amino, mercapto, halogen, cyano, $C_{1-4}$ alkoxy (e.g., $C_{1-3}$ alkoxy, such as methoxy, ethoxy, or propoxy), $C_{1-4}$ alkyl-NH- (e.g., $C_{1-3}$ alkyl-NH-, such as $CH_3NH$-, $CH_3CH_2NH$- or $CH_3CH_2CH_2NH$-), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $NH_2$-$C_{1-4}$ alkylene (e.g., $NH_2$-$C_{1-3}$ alkylene-, such as $NH_2CH_2$-, $NH_2CH_2CH_2$- and $NH_2CH_2CH_2CH_2$-), $C_{1-4}$ alkyl-NHC(O)- (e.g., $C_{1-3}$ alkyl-NHC(O)-, such as $CH_3$-NHC(O)-, $CH_3CH_2$-NHC(O)-, and $CH_3CH_2CH_2$-NHC(O)-), $C_{1-4}$ alkyl-C(O)NH- (e.g., $C_{1-3}$ alkyl-C(O)NH-, such as $CH_3$-C(O)NH-, $CH_3CH_2$-C(O)NH-, and $CH_3CH_2CH_2$-C(O)NH-) or any combination thereof;

$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and

m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-8), $L_3$ represents the structure of the following formula:

$-(C(R^{a9})(R^{a10}))_{m1}-(O(C(R^{a11})(R^{a12}))_{m2})_{p1}-$;

$-(C(R^{a9})(R^{a10}))_{m1}-(O(C(R^{a11})(R^{a12}))_{m2})_{p1}-(O(C(R^{a13})(R^{a14}))_{m3})_{p2}-$;

$-(C(R^{a9})(R^{a10}))_{m1}-(O(C(R^{a11})(R^{a12}))_{m2})_{p1}-(O(C(R^{a13})(R^{a14}))_{m3})_{p2}-(O(C(R^{a15})(R^{a16}))_{m4})_{p3}-$;

$-(C(R^{a9})(R^{a10}))_{m1}-(N(R_{g11})(C(R^{a11})(R^{a12}))_{m2})_{p1}-$;

$-(C(R^{a9})(R^{a10}))_{m1}-(N(R_{g11})(C(R^{a11})(R^{a12}))m_2)_{p1}-(N(R_{g11})(C(R^{a13})(R^{a14}))_{m3})_{p2}-$;

$-(C(R^{a9})(R^{a10}))_{m1}-(N(R_{g11})(C(R^{a11})(R^{a12}))_{m2})_{p1}-(N(R_{g11})(C(R^{a13})(R^{a14}))_{m3})_{p2}-(N(R_{g11})(C(R^{a15})$ $(R^{a16}))_{m4})_{p3}-$;

$-(C(R^{a9})(R^{a10}))_{m1}-(C(O)NH-(C(R^{a11})(R^{a12}))_{m2})_{p1}-$;

$-(C(R^{a9})(R^{a10}))_{m1}-(C(O)NH-(C(R^{a11})(R^{a12}))_{m2})_{p1}-(C(O)NH-(C(R^{a13})(R^{a14}))_{m3})_{p2}-$;

$-(C(R^{a9})(R^{a10}))_{m1}-(C(O)NH-(C(R^{a11})(R^{a12}))_{m2})_{p1}-(C(O)NH-(C(R^{a13})(R^{a14}))_{m3})_{p2}-(C(O)NH-(C(R^{a15})(R^{a16}))_{m4})_{p3}-$;

$-(C(R^{a9})(R^{a10}))_{m1}-(C(O)NH-(C(R^{a11})(R^{a12}))_{m2})_{p1}-(O-(C(R^{a13})(R^{a14}))_{m3})_{p2}-$;

$-(C(R^{a9})(R^{a10}))_{m1}-C(O)NH-(C(R^{a11})(R^{a12}))_{m2}-(O(C(R^{a13})(R^{a14}))_{m3})_{p1}-$;

$-(C(R^{a9})(R^{a10}))_{m1}-(NHC(O)-(C(R^{a11})(R^{a12})_{m2})_{p1}-$;

$-(C(R^{a9})(R^{a10}))_{m1}-(NHC(O)-(C(R^{a11})(R^{a12}))_{m2})_{p1}-(O-(C(R^{a13})(R^{a14}))_{m3})_{p2}-$;

$-(C(R^{a9})(R^{a10}))_{m1}-(NHC(O)-(C(R^{a11})(R^{a12}))_{m2})_{p1}-(O-(C(R^{a13})(R^{a14}))_{m3})_{p2}-(O-(C(R^{a15})(R^{a16}))_{m4})_{p3}-$;

$-(C(R^{a9})(R^{a10}))_{m1}-NHC(O)-(C(R^{a11})(R^{a12}))_{m2}-(O(C(R^{a13})(R^{a14}))_{m3})_{p1}-$;

$-(C(R^{a9})(R^{a10}))_{m1}-NHC(O)NH-(C(R^{a11})(R^{a12}))_{m2}-$;

$-(C(R^{a9})(R^{a10}))_{m1}-C(O)-(C(R^{a11})(R^{a12}))_{m2}-$;

$-(C(R^{a9})(R^{a10}))_{m1}-C(S)-(C(R^{a11})(R^{a12})_{m2}-$;

$-(C(R^{a9})(R^{a10}))_{m1}-S-(C(R^{a11})(R^{a12}))_{m2}-$;

$-(C(R^{a9})(R^{a10}))_{m1}-(C_{6-10}$ arylene$-(C(R^{a111})(R^{a12}))_{m2})_{p1}-$;

$-(C(R^{a9})(R^{a10}))_{m1}-(C_{6-10}$ arylene$-(C(R^{a11})(R^{a12}))_{m2})_{p1}-C_{6-10}$ arylene$-(C(R^{a13})(R^{a14}))_{m3}-$;

$-(C(R^{a9})(R^{a10}))_{m1}-$(4- to 15-membered heterocyclylene$-(C(R^{a11})(R^{a12}))_{m2})_{p1}-$;

$-(C(R^{a9})(R^{a10}))_{m1}-$(4- to 15-membered heterocyclylene$-(C(R^{a11})(R^{a12}))_{m2})_{p1}-$(4- to 15-membered heterocyclylene$-(C(R^{a13})(R^{a14}))_{m3})_{p2}-$;

$-(C(R^{a9})(R^{a10}))_{m1}-$(5- to 15-membered heteroarylene$-(C(R^{a11})(R^{a12}))_{m2})_{p1}-$;

$-(C(R^{a9})(R^{a10}))_{m1}-$(5- to 15-membered heteroarylene$-(C(R^{a11})(R^{a12}))_{m2})_{p1}-$(5- to 15-membered heteroarylene$-(C(R^{a13})(R^{a14}))_{m3})_{p2}-$;

$-(C(R^{a9})(R^{a10}))_{m1}-(C_{3-15}$cycloalkylene$-(C(R^{a11})(R^{a12}))_{m2})_{p1}-$; or

$-(C(R^{a9})(R^{a10}))_{m1}-(C_{3-15}$cycloalkylene$-(C(R^{a11})(R^{a12}))_{m2})_{p1}-(C_{3-15}$cycloalkylene$-(C(R^{a13})(R^{a14}))_{m3})_{p2}-$;

wherein each $R_{g11}$ independently represents H or $C_{1-3}$ alkyl;
the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;
$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and
m1, m2, m3, m4, p1, p2, p3 are each independently selected from the group consisting of an integer of 1, 2, 3, 4, 5, 6, 7, 8, 9, 10, 11, 12, 13, 14, or 15.

In some embodiments of the compound of Formula (I-8), $L_3$ represents the structure of the following formula:

$-CH_2-O-CH_2-$, $-CH_2-O-(CH_2)_2-$, $-(CH_2)_1-O-(CH_2)_3-$, $-(CH_2)_1-O-(CH_2)a-$, $-(CH_2)_1-O-(CH_2)5-$,$-(CH_2)_1-O-(CH_2)_6-$, $-(CH_2)_1-O-(CH_2)_7-$, $-(CH_2)_1-O-(CH_2)_8-$, $-(CH_2)_1-O-(CH_2)_9-$, $-(CH_2)_1-O-(CH_2)_{10}-$,$-(CH_2)_2-O-(CH_2)_1-$, $-(CH_2)_2-O-(CH_2)_2-$, $-(CH_2)_2-O-(CH_2)_3-$, $-(CH_2)_2-O-(CH_2)_4-$, $-(CH_2)_2-O-(CH_2)_5-$,$-(CH_2)_2-O-(CH_2)_6-$, $-(CH_2)_2-O-(CH_2)_7-$, $-(CH_2)_2-O-(CH_2)_8-$, $-(CH_2)_2-O-(CH_2)_9-$, $-(CH_2)_2-O-(CH_2)_{10}-$,$-(CH_2)_2-O-(CH_2)_{11}-$, $-(CH_2)_2-O-(CH_2)_{12}-$, $-(CH_2)_3-O-(CH_2)_1-$, $-(CH_2)_3-O-(CH_2)_2-$, $-(CH_2)_3-O-(CH_2)_3-$,$-(CH_2)_3-O-(CH_2)_4-$, $-(CH_2)_3-O-(CH_2)_5-$, $-(CH_2)_3-O-(CH_2)_6-$, $-(CH_2)_3-O-(CH_2)_7-$, $-(CH_2)_4-O-(CH_2)_1-$,$-(CH_2)_4-O-(CH_2)_2-$, $-(CH_2)_4-O-(CH_2)_3-$, $-(CH_2)_4-O-(CH_2)_4-$, $-(CH_2)_4-O-(CH_2)_5-$, $-(CH_2)_4-O-(CH_2)_6-$,$-(CH_2)_5-O-(CH_2)_1-$, $-(CH_2)_5-O-(CH_2)_2-$, $-(CH_2)_5-O-(CH_2)_3-$, $-(CH_2)_5-O-(CH_2)_4-$, $-(CH_2)_5-O-(CH_2)_5-$,$-(CH_2)_6-O-(CH_2)_1-$, $-(CH_2)_6-O-(CH_2)_2-$, $-(CH_2)_6-O-(CH_2)_3-$, $-(CH_2)_6-O-(CH_2)_4-$, $-(CH_2)_7-O-(CH_2)_1-$,$-(CH_2)_7-O-(CH_2)_2-$, $-(CH_2)_7-O-(CH_2)_3-$, $-(CH_2)_8-O-(CH_2)_1-$, $-(CH_2)_8-O-(CH_2)_2-$, $-CH(CH_3)-O-(CH_2)_1-$,$-CH(CH_3)-O-(CH_2)_2-$, $-CH(CH_3)-O-(CH_2)_3-$, $-CH(CH_3)-O-(CH_2)_4-$, $-CH(CH_3)-O-(CH_2)_5-$, $-CH(CH_3)-O-(CH_2)_6-$, $-CH(CH_3)-O-(CH_2)_7-$, $-CH(CH_3)-O-(CH_2)_8-$, $-CH(CH_3)-O-(CH_2)_9-$, $-CH(CH_3)-O-(CH_2)_{10}-$, $-CH_2-(O(CH_2)_2)_2-$, $-CH_2-(O(CH_2)_2)_3-$, $-CH_2-(O(CH_2)_2)_4-$, $-CH_2-(O(CH_2)_2)_5-$, $-CH_2-(O(CH_2)_2)_6-$, $-CH_2-(O(CH_2)_2)_7-$, $-CH_2-(O(CH_2)_2)_8-$, $-CH_2-(O(CH_2)_2)_9-$, $-CH_2-(O(CH_2)_2)_{10}-$, $-CH_2-(O(CH_2)_2)_1-OCH_2-$,$-CH_2-(O(CH_2)_2)_2-OCH_2-$, $-CH_2-(O(CH_2)_2)_3-OCH_2-$, $-CH_2-(O(CH_2)_2)_4-OCH_2-$, $-CH_2-(O(CH_2)_2)_5-OCH_2-$, $-CH_2-(O(CH_2)_2)_6-OCH_2-$, $-CH_2-(O(CH_2)_2)_7-OCH_2-$, $-CH_2-(O(CH_2)_2)_8-OCH_2-$, $-CH_2-(O(CH_2)_2)_9-OCH_2-$, $-CH_2-(O(CH_2)_2)_lo-OCH_2-$, $-(CH_2)_2-(O(CH_2)_2)_2-$, $-(CH_2)_2-(O(CH_2)_2)_3-$, $-(CH_2)_2-(O(CH_2)_2)_4-$,$-(CH_2)_2-(O(CH_2)_2)_5-$, $-(CH_2)_2-(O(CH_2)_2)_6-$, $-(CH_2)_2-(O(CH_2)_2)_7-$, $-(CH_2)_2-(O(CH_2)_2)_8-$, $-(CH_2)_2-(O(CH_2)_2)_9-$, $-(CH_2)_2-(O(CH_2)_2)_{10}-$, $-(CH_2)_3-(O(CH_2)_2)_2-$, $-(CH_2)_3-(O(CH_2)_2)_3-$, $-(CH_2)_3-(O(CH_2)_2)_4-$,$-(CH_2)_3-(O(CH_2)_2)_5-$, $-(CH_2)_3-(O(CH_2)_2)_6-$, $-(CH_2)_3-(O(CH_2)_2)_7-$, $-(CH_2)_3-(O(CH_2)_2)_8-$, $-(CH_2)_3-(O(CH_2)_2)_9-$, $-(CH_2)_3-(O(CH_2)_2)_{10}-$, $-(CH_2)_4-(O(CH_2)_2)_2-$, $-(CH_2)_4-(O(CH_2)_2)_3-$, $-(CH_2)_4-(O(CH_2)_2)_4-$,$-(CH_2)_4-(O(CH_2)_2)_5-$, $-(CH_2)_4-(O(CH_2)_2)_6-$, $-(CH_2)_4-(O(CH_2)_2)_7-$, $-(CH_2)_4-(O(CH_2)_2)_8-$, $-(CH_2)_4-(O(CH_2)_2)_9-$, $-(CH_2)_4-(O(CH_2)_2)_{10}-$, $-CH_2-(O(CH_2)_3)_2-$, $-CH_2-(O(CH_2)_3)_3-$, $-CH_2-(O(CH_2)_3)_4-$, $-CH_2-(O(CH_2)_3)_5-$, $-CH_2-(O(CH_2)_3)_6-$, $-CH_2-(O(CH_2)_3)_7-$, $-CH_2-(O(CH_2)_3)_8-$, $-CH_2-(O(CH_2)_3)_9-$, $-CH_2-(O(CH_2)_3)_{10}-$, $-(CH_2)_2-(O(CH_2)_3)_2-$, $-(CH_2)_2-(O(CH_2)_3)_3-$, $-(CH_2)_2-(O(CH_2)_3)_4-$, $-(CH_2)_2-(O(CH_2)_3)_5-$,$-(CH_2)_2-(O(CH_2)_3)_6-$, $-(CH_2)_2-(O(CH_2)_3)_7-$, $-(CH_2)_2-(O(CH_2)_3)_8-$, $-(CH_2)_2-(O(CH_2)_3)_9-$, $-(CH_2)_2-(O(CH_2)_3)_{10}-$, $-(CH_2)_3-(O(CH_2)_3)_2-$, $-(CH_2)_3-(O(CH_2)_3)_3-$, $-(CH_2)_3-(O(CH_2)_3)_4-$, $-(CH_2)_3-(O(CH_2)_3)_5-$,$-(CH_2)_3-(O(CH_2)_3)_6-$, $-(CH_2)_3-(O(CH_2)_3)_7-$, $-(CH_2)_3-(O(CH_2)_3)_8-$, $-(CH_2)_3-(O(CH_2)_3)_9-$, $-(CH_2)_3-(O(CH_2)_3)_{10}-$, $-CH_2-O-(CH_2)_2-O-(CH_2)_3-$, $-CH_2-(O(CH_2)_2)_2-(O(CH_2)_3)_2-$, $-CH_2-(O(CH_2)_2)_3-(O(CH_2)_3)_3-$, $-CH_2-(O(CH_2)_2)_4-(O(CH_2)_3)_4-$, $-CH_2-(O(CH_2)_2)_5-(O(CH_2)_3)_5-$, $-CH_2-(O(CH_2)_2)_6-(O(CH_2)_3)_6-$, $-(CH_2)_2-O-(CH_2)_2-O-(CH_2)_3-$, $-(CH_2)_2-(O(CH_2)_2)_2-(O(CH_2)_3)_2-$, $-(CH_2)_2-(O(CH_2)_2)_3-(O(CH_2)_3)_3-$, $-(CH_2)_2-(O(CH_2)_2)_4-(O(CH_2)_3)_4-$, $-(CH_2)_2-(O(CH_2)_2)_5-(O(CH_2)_3)_5-$, $-(CH_2)_2-(O(CH_2)_2)_6-(O(CH_2)_3)_6-$, $-(CH_2)_3-O-(CH_2)_2-O-(CH_2)_3-$, $-(CH_2)_3-(O(CH_2)_2)_2-(O(CH_2)_3)_2-$, $-(CH_2)_3-(O(CH_2)_2)_3-(O(CH_2)_3)_3-$, $-(CH_2)_3-(O(CH_2)_2)_4-(O(CH_2)_3)_4-$, $-(CH_2)_3-(O(CH_2)_2)_5-(O(CH_2)_3)_5-$, $-(CH_2)_3-(O(CH_2)_2)_6-(O(CH_2)_3)_6-$, $-CH_2-O-(CH_2)_3-O-(CH_2)_2-$, $-CH_2-(O(CH_2)_3)_2-(O(CH_2)_2)_2-$, $-CH_2-(O(CH_2)_3)_3-(O(CH_2)_2)_3-$, $-CH_2-(O(CH_2)_3)_4-(O(CH_2)_2)_4-$, $-CH_2-(O(CH_2)_3)_5-(O(CH_2)_2)_5-$, $-CH_2-(O(CH_2)_3)_6-(O(CH_2)_2)_6-$, $-(CH_2)_2-O-(CH_2)_3-O-(CH_2)_2-$, $-(CH_2)_2-(O(CH_2)_3)_2-(O(CH_2)_2)_2-$, $-(CH_2)_2-(O(CH_2)_3)_3-(O(CH_2)_2)_3-$, $-(CH_2)_2-(O(CH_2)_3)_4-(O(CH_2)_2)_4-$, $-(CH_2)_2-(O(CH_2)_3)_5-(O(CH_2)_2)_5-$, $-(CH_2)_2-(O(CH_2)_3)_6-(O(CH_2)_2)_6-$, $-(CH_2)_3-O-(CH_2)_3-O-(CH_2)_2-$, $-(CH_2)_3-(O(CH_2)_3)_2-(O(CH_2)_2)_2-$, $-(CH_2)_3-(O(CH_2)_3)_3-(O(CH_2)_2)_3-$, $-(CH_2)_3-(O(CH_2)_3)_4-(O(CH_2)_2)_4-$, $-(CH_2)_3-(O(CH_2)_3)_5-(O(CH_2)_2)_5-$, $-(CH_2)_3-(O(CH_2)_3)_6-(O(CH_2)_2)_6-$, $-CH_2-O-(CH_2)_2-O-CH_2-$, $-(CH_2)_2-O-(CH_2)_2-O-CH_2-$, $-(CH_2)_2-(O(CH_2)_2)_2-O-(CH_2)_3-$, $-(CH_2)_2-(O(CH_2)_2)_3-O-(CH_2)_3-$,$-(CH_2)_2-(O(CH_2)_2)_4-O-(CH_2)_3-$, $-(CH_2)_5-(O(CH_2)_2)_2-O-(CH_2)_5-$, $-(CH_2)_5-(O(CH_2)_2)_2-O-(CH_2)_6-$, $-CH_2-C(O)-CH_2-$, $-CH_2-C(O)-(CH_2)_2-$, $-(CH_2)_1-C(O)-(CH_2)_3-$, $-(CH_2)_1-C(O)-(CH_2)_4-$, $-(CH_Z)_1-C(O)-(CH_Z)s-$, $-(CH_2)_1-C(O)-(CH_2)_6-$, $-(CH_2)_1-C(O)-(CH_2)_7-$, $-(CH_2)_1-C(O)-(CH_2)_8-$, $-(CH_2)_1-C(O)-(CH_2)_9-$, $-(CH_2)_1-C(O)-(CH_2)_{10}-$, $-(CH_2)_2-C(O)-(CH_2)_1-$, $-(CH_2)_2-C(O)-(CH_2)_2-$, $-(CH_2)_2-C(O)-(CH_2)_3-$, $-(CH_2)_2-C(O)-(CH_2)_4-$, $-(CH_2)_2-C(O)-(CH_2)_5-$, $-(CH_2)_2-C(O)-(CH_2)_6-$, $-(CH_2)_2-C(O)-(CH_2)_7-$, $-(CH_2)_2-C(O)-(CH_2)_8-$,$-(CH_2)_2-C(O)-(CH_2)_9-$, $-(CH_2)_2-C(O)-(CH_2)_{10}-$, $-(CH_2)_2-C(O)-(CH_2)_{11}-$, $-(CH_2)_2-C(O)-(CH_2)_{12}-$,$-(CH_2)_3-C(O)-(CH_2)_1-$, $-(CH_2)_3-C(O)-(CH_2)_2-$, $-(CH_2)_3-C(O)-(CH_2)_3-$, $-(CH_2)_3-C(O)-(CH_2)_4-$, $-(CH_2)_3-C(O)-(CH_2)_5-$, $-(CH_2)_3-C(O)-(CH_2)_6-$, $-(CH_2)_3-C(O)-(CH_2)_7-$, $-(CH_2)_4-C(O)-(CH_2)_1-$, $-(CH_2)_4-C(O)-(CH_2)_2-$, $-(CH_2)_4-C(O)-(CH_2)_3-$, $-(CH_2)_4-C(O)-(CH_2)_4-$, $-(CH_2)_4-C(O)-(CH_2)_5-$, $-(CH_2)_4-C(O)-(CH_2)_6-$,$-(CH_2)_5-C(O)-(CH_2)_1-$, $-(CH_2)_5-C(O)-(CH_2)_2-$, $-(CH_2)_5-C(O)-(CH_2)_3-$, $-(CH_2)_5-C(O)-(CH_2)_4-$, $-(CH_2)_5-C(O)-(CH_2)_5-$, $-(CH_2)_6-C(O)-(CH_2)_1-$, $-(CH_2)_6-C(O)-(CH_2)_2-$, $-(CH_2)_6-C(O)-(CH_2)_3-$, $-(CH_2)_6-C(O)-(CH_2)_4-$, $-(CH_2)_7-C(O)-(CH_2)_1-$, $-(CH_2)_7-C(O)-(CH_2)_2-$, $-(CH_2)_7-C(O)-(CH_2)_3-$, $-(CH_2)_8-C(O)-(CH_2)_1-$,$-(CH_2)_8-C(O)-(CH_2)_2-$, $-CH_2-S-CH_2-$, $-CH_2-S-(CH_2)_2-$, $-(CH_2)_1-S-(CH_2)_3-$, $-(CH_2)_1-S-(CH_2)_4-$, $-(CH_2)_1-S-(CH_2)_5-$, $-(CH_2)_1-S-(CH_2)_6-$, $-(CH_2)_1-S-(CH_2)_7-$, $-(CH_2)_1-S-(CH_2)_8-$, $-(CH_2)_1-S-(CH_2)_9-$, $-(CH_2)_1-S-(CH_2)_{10}-$, $-(CH_2)_2-S-(CH_2)_1-$, $-(CH_2)_2-S-(CH_2)_2-$, $-(CH_2)_2-S-(CH_2)_3-$, $-(CH_2)_2-S-(CH_2)_4-$, $-(CH_2)_2-S-(CH_2)_5-$, $-(CH_2)_2-S-(CH_2)_6-$, $-(CH_2)_2-S-(CH_2)_7-$, $-(CH_2)_2-S-(CH_2)_8-$, $-(CH_2)_2-S-(CH_2)_9-$, $-(CH_2)_2-S-(CH_2)_{10}-$, $-(CH_2)_2-S-(CH_2)_{11}-$, $-(CH_2)_2-S-(CH_2)_{12}-$, $-(CH_2)_3-S-(CH_2)_1-$, $-(CH_2)_3-S-(CH_2)_2-$, $-(CH_2)_3-S-(CH_2)_3-$, $-(CH_2)_3-S-(CH_2)_4-$, $-(CH_2)_3-S-(CH_2)_5-$, $-(CH_2)_3-S-(CH_2)_6-$, $-(CH_2)_3-S-(CH_2)_7-$, $-(CH_2)_4-S-(CH_2)_1-$, $-(CH_2)_4-S-(CH_2)_2-$, $-(CH_2)_4-S-(CH_2)_3-$, $-(CH_2)_4-S-(CH_2)_4-$, $-(CH_2)_4-S-(CH_2)_5-$, $-(CH_2)_4-S-(CH_2)_6-$, $-(CH_2)_5-S-(CH_2)_1-$, $-(CH_2)_5-S-(CH_2)_2-$, $-(CH_2)_5-S-(CH_2)_3-$, $-(CH_2)_5-S-(CH_2)_4-$, $-(CH_2)_5-S-(CH_2)_5-$, $-(CH_2)_6-S-(CH_2)_1-$, $-(CH_2)_6-S-(CH_2)_2-$, $-(CH_2)_6-S-(CH_2)_3-$, $-(CH_2)_6-S-(CH_2)_4-$, $-(CH_2)_7-S-(CH_2)_1-$, $-(CH_2)_7-S-(CH_2)_2-$, $-(CH_2)_7-S-(CH_2)_3-$, $-(CH_2)_8-S-(CH_2)_l-$, $-(CH_2)_8-S-(CH_2)_2-$, $-CH_2-C(S)-CH_2-$, $-CH_2-C(S)-(CH_2)_2-$, $-(CH_2)_1-C(S)-(CH_2)_3-$, $-(CH_2)_1-C(S)-(CH_2)_4-$,

$-(CH_2)_1-C(S)-(CH_2)_5-$,$-(CH_2)_1-C(S)-(CH_2)_6-$, $-(CH_2)_1-C(S)-(CH_2)_7-$, $-(CH_2)_1-C(S)-(CH_2)_8-$, $-(CH_2)_1-C(S)-(CH_2)_9-$, $-(CH_2)_1-C(S)-(CH_2)_{10}-$, $-(CH_2)_2-C(S)-(CH_2)_1-$, $-(CH_2)_2-C(S)-(CH_2)_2-$, $-(CH_2)_2-C(S)-(CH_2)_3-$, $-(CH_2)_2-C(S)-(CH_2)_4-$, $-(CH_2)_2-C(S)-(CH_2)_5-$, $-(CH_2)_2-C(S)-(CH_2)_6-$, $-(CH_2)_2-C(S)-(CH_2)_7-$, $-(CH_2)_2-C(S)-(CH_2)_8-$, $-$ $(CH_2)_2-C(S)-(CH_2)_9-$, $-(CH_2)_2-C(S)-(CH_2)_{10}-$, $-(CH_2)_2-C(S)-(CH_2)_{11}-$, $-(CH_2)_2-C(S)-(CH_2)_{12}-$, $-(CH_2)_3-C(S)-(CH_2)_1-$, $-(CH_2)_3-C(S)-(CH_2)_2-$, $-(CH_2)_3-C(S)-(CH_2)_3-$, $-(CH_2)_3-C(S)-(CH_2)_4-$, $-(CH_2)_3-C(S)-(CH_2)_5-$, $-(CH_2)_3-C(S)-(CH_2)_6-$, $-(CH_2)_3-C(S)-(CH_2)_7-$, $-(CH_2)_4-C(S)-(CH_2)_1-$, $-(CH_2)_4-C(S)-(CH_2)_2-$,$-(CH_2)_4-C(S)-(CH_2)_3-$, $-(CH_2)_4-C(S)-(CH_2)_4-$, $-(CH_2)_4-C(S)-(CH_2)_5-$, $-(CH_2)_4-C(S)-(CH_2)_6-$, $-(CH_2)_5-C(S)-(CH_2)_1-$, $-(CH_2)_5-C(S)-(CH_2)_2-$, $-(CH_2)_5-C(S)-(CH_2)_3-$, $-(CH_2)_5-C(S)-(CH_2)_4-$, $-(CH_2)_5-C(S)-(CH_2)_5-$, $-(CH_2)_6-C(S)-(CH_2)_1-$, $-(CH_2)_6-C(S)-(CH_2)_2-$, $-(CH_2)_6-C(S)-(CH_2)_3-$, $-(CH_2)_6-C(S)-(CH_2)_4-$,$-(CH_2)_7-C(S)-(CH_2)_1-$, $-(CH_2)_7-C(S)-(CH_2)_2-$, $-(CH_2)_7-C(S)-(CH_2)_3-$, $-(CH_2)_8-C(S)-(CH_2)_1-$, $-(CH_2)_8-C(S)-(CH_2)_2-$, $-CH_2-C(O)O-CH_2-$, $-CH_2-C(O)O-(CH_2)_2-$, $-(CH_Z)_1-C(O)O-(CH_Z)_3-$, $-(CH_2)_1-C(O)O-(CH_2)_4-$, $-(CH_2)_1-C(O)O-(CH_2)_5-$, $-(CH_2)_1-C(O)O-(CH_2)_6-$, $-(CH_2)_1-C(O)O-(CH_2)_7-$, $-(CH_2)_1-C(O)O-(CH_2)_8-$, $-(CH_2)_1-C(O)O-(CH_2)_9-$, $-(CH_2)_1-C(O)O-(CH_2)_{10}-$, $-(CH_2)_2-C(O)O-(CH_2)_1-$, $-(CH_2)_2-C(O)O-(CH_2)_2-$, $-(CH_2)_2-C(O)O-(CH_2)_3-$, $-(CH_2)_2-C(O)O-(CH_2)_4-$, $-(CH_2)_2-C(O)O-(CH_2)_5-$, $-(CH_2)_2-C(O)O-(CH_2)_6-$, $-(CH_2)_2-C(O)O-(CH_2)_7-$, $-(CH_2)_2-C(O)O-(CH_2)_8-$, $-(CH_2)_2-C(O)O-(CH_2)_9-$, $-(CH_2)_2-C(O)O-(CH_2)_{10}-$, $-(CH_2)_2-C(O)O-(CH_2)_{11}-$, $-(CH_2)_2-C(O)O-(CH_2)_{12}-$, $-(CH_2)_3-C(O)O-(CH_2)_1-$, $-(CH_2)_3-C(O)O-(CH_2)_2-$, $-(CH_2)_3-C(O)O-(CH_2)_3-$, $-(CH_2)_3-C(O)O-(CH_2)_4-$, $-(CH_2)_3-C(O)O-(CH_2)_5-$, $-(CH_2)_3-C(O)O-(CH_2)_6-$, $-(CH_2)_3-C(O)O-(CH_2)_7-$, $-(CH_2)_4-C(O)O-(CH_2)_1-$, $-(CH_2)_4-C(O)O-(CH_2)_2-$, $-(CH_2)_4-C(O)O-(CH_2)_3-$, $-(CH_2)_4-C(O)O-(CH_2)_4-$, $-(CH_2)_4-C(O)O-(CH_2)_5-$, $-(CH_2)_4-C(O)O-(CH_2)_6-$, $-(CH_2)_5-C(O)O-(CH_2)_1-$, $-(CH_2)_5-C(O)O-(CH_2)_2-$, $-(CH_2)_5-C(O)O-(CH_2)_3-$, $-(CH_2)_5-C(O)O-(CH_2)_4-$, $-(CH_2)_5-C(O)O-(CH_2)_5-$, $-(CH_Z)_6-C(O)O-(CH_Z)_1-$, $-(CH_2)_6-C(O)O-(CH_2)_2-$, $-(CH_2)_6-C(O)O-(CH_2)_3-$, $-(CH_2)_6-C(O)O-(CH_2)_4-$, $-(CH_2)_7-C(O)O-(CH_2)_1-$, $-(CH_2)_7-C(O)O-(CH_2)_2-$, $-(CH_2)_7-C(O)O-(CH_2)_3-$, $-(CH_2)_8-C(O)O-(CH_2)_1-$, $-(CH_2)_8-C(O)O-(CH_2)_2-$, $-CH_2-O-C(O)-CH_2-$, $-CH_2-OC(O)-(CH_2)_2-$, $-(CH_2)_1-OC(O)-(CH_2)_3-$,$-(CH_2)_1-OC(O)-(CH_2)_4-$, $-(CH_2)_1-OC(O)-(CH_2)_5-$, $-(CH_2)_1-O-C(O)-(CH_2)_6-$, $-(CH_2)_1-OC(O)-(CH_2)_7-$,$-(CH_2)_1-OC(O)-(CH_2)_8-$, $-(CH_2)_1-OC(O)-(CH_2)_9-$, $-(CH_2)_1-OC(O)-(CH_2)_{10}-$, $-(CH_2)_2-OC(O)-(CH_2)_1-$,$-(CH_2)_2-OC(O)-(CH_2)_2-$, $-(CH_2)_2-OC(O)-(CH_2)_3-$, $-(CH_2)_2-OC(O)-(CH_2)_4-$, $-(CH_2)_2-O-C(O)-(CH_2)_5-$,$-(CH_2)_2-OC(O)-(CH_2)_6-$, $-(CH_2)_2-OC(O)-(CH_2)_7-$, $-(CH_2)_2-OC(O)-(CH_2)_8-$, $-(CH_2)_2-O-C(O)-(CH_2)_9-$,$-(CH_2)_2-OC(O)-(CH_2)_{10}-$, $-(CH_2)_2-OC(O)-(CH_2)_{11}-$, $-(CH_2)_2-OC(O)-(CH_2)_{12}-$, $-(CH_2)_3-O-C(O)-(CH_2)_1-$,$-(CH_2)_3-OC(O)-(CH_2)_2-$, $-(CH_2)_3-OC(O)-(CH_2)_3-$, $-(CH_2)_3-OC(O)-(CH_2)_4-$, $-(CH_2)_3-O-C(O)-(CH_2)_5-$,$-(CH_2)_3-OC(O)-(CH_2)_6-$, $-(CH_2)_3-OC(O)-(CH_2)_7-$, $-(CH_2)_4-OC(O)-(CH_2)_1-$, $-(CH_2)_4-O-C(O)-(CH_2)_2-$,$-(CH_2)_4-OC(O)-(CH_2)_3-$, $-(CH_2)_4-OC(O)-(CH_2)_4-$, $-(CH_2)_4-OC(O)-(CH_2)_5-$, $-(CH_2)_4-O-C(O)-(CH_2)_6-$,$-(CH_2)_5-OC(O)-(CH_2)_1-$, $-(CH_2)_5-OC(O)-(CH_2)_2-$, $-(CH_2)_5-OC(O)-(CH_2)_3-$, $-(CH_2)_5-O-C(O)-(CH_2)_4-$,$-(CH_2)_5-OC(O)-(CH_2)_5-$, $-(CH_2)_6-OC(O)-(CH_2)_1-$, $-(CH_2)_6-OC(O)-(CH_2)_2-$, $-(CH_2)_6-O-C(O)-(CH_2)_3-$,$-(CH_2)_6-OC(O)-(CH_2)_4-$, $-(CH_2)_7-OC(O)-(CH_2)_1-$, $-(CH_2)_7-OC(O)-(CH_2)_2-$, $-(CH_2)_7-O-C(O)-(CH_2)_3-$,$-(CH_2)_8-OC(O)-(CH_2)_1-$, $-(CH_2)_8-OC(O)-(CH_2)_2-$, $-(CH_2)_1-N(R_{g11})-(CH_2)_1-$, $-(CH_2)_1-N(R_{g11})-(CH_2)_2-$,$-(CH_2)_1-N(R_{g11})-(CH_2)_3-$, $-(CH_2)_1-N(R_{g11})-(CH_2)_4-$, $-(CH_2)_1-N(R_{g11})-(CH_2)_5-$, $-(CH_2)_1-N(R_{g11})-(CH_2)_6-$,$-(CH_2)_1-$ $N(R_{g11})-(CH_2)_7-$, $-(CH_2)_1-N(R_{g11})-(CH_2)_S-$, $-(CH_2)_1-N(R_{g11})-(CH_2)_9-$,$-(CH_2)_1-N(R_{g11})-(CH_2)_{10}-$, $-(CH_2)_2-N(R_{g11})-(CH_2)_1-$, $-(CH_2)_2-N(R_{g11})-(CH_2)_2-$, $-(CH_2)_2-N(R_{g11})-(CH_2)_3-$, $-(CH_2)_2-N(R_{g11})-(CH_2)_4-$, $-(CH_2)_2-N(R_{g11})-(CH_2)_5-$, $-(CH_2)_2-N(R_{g11})-(CH_2)_6-$, $-(CH_2)_2-N(R_{g11})-(CH_2)_7-$, $-(CH_2)_2-N(R_{g11})-(CH_2)_8-$, $-(CH_2)_2-N(R_{g11})-(CH_2)_9-$, $-(CH_2)_2-N(R_{g11})-(CH_2)_{10}-$, $-(CH_2)_2-N(R_{g11})-(CH_2)_{11}-$, $-(CH_2)_2-N(R_{g11})-(CH_2)_{12}-$, $-(CH_2)_3-N(R_{g11})-(CH_2)_1-$, $-(CH_2)_3-N(R_{g11})-(CH_2)_2-$, $-(CH_2)_3-N(R_{g11})-(CH_2)_3-$, $-(CH_2)_4-N(R_{g11})-(CH_2)_1-$, $-(CH_2)_4-N(R_{g11})-(CH_2)_2-$, $-(CH_2)_4-N(R_{g11})-(CH_2)_3-$, $-(CH_2)_4-N(R_{g11})-(CH_2)_4-$, $-(CH_2)_5-N(R_{g11})-(CH_2)_1-$, $-(CH_2)_5-N(R_{g11})-(CH_2)_2-$, $-(CH_2)_5-N(R_{g11})-(CH_2)_3-$, $-(CH_2)_5-N(R_{g11})-(CH_2)_4-$, $-(CH_2)_5-N(R_{g11})-(CH_2)_5-$, $-(CH_2)_6-N(R_{g11})-(CH_2)_1-$, $-(CH_2)_6-N(R_{g11})-(CH_2)_2-$, $-(CH_2)_6-N(R_{g11})-(CH_2)_3-$, $-(CH_2)_7-N(R_{g11})-(CH_2)_1-$, $-(CH_2)_7-N(R_{g11})-(CH_2)_2-$, $-(CH_2)_7-N(R_{g11})-(CH_2)_3-$, $-(CH_2)_8-N(R_{g11})-(CH_2)_1-$, $-(CH_2)_8-NR_{g11})-(CH_2)_2-$, $-(CH_2)_8-N(R_{g11})-(CH_2)_3-$, $-CH(CH_3)-N(R_{g11})-(CH_2)_1-$, $-CH(CH_3)-N(R_{g11})-(CH_2)_2-$, $-$ $CH(CH_3)-N(R_{g11})-(CH_2)_3-$, $-CH(CH_3)-N(R_{g11})-(CH_2)_4-$, $-CH(CH_3)-N(R_{g11})-(CH_2)_5-$, $-CH(CH_3)-N(R_{g11})-(CH_2)_6-$, $-CH(CH_3)-N(R_{g11})-(CH_2)_7-$, $-CH(CH_3)-N(R_{g11})-(CH_2)_8-$, $-CH(CH_3)-N(R_{g11})-(CH_2)_9-$, $-CH(CH_3)-N(R_{g11})-(CH_2)_{10}-$, $-CH_2C(O)NHCH_2-$, $-(CH_2)_{2C}(O)NH(CH_2)_2-$, $-(CH_2)_{2C}(O)NH(CH_2)_3-$, $-$ $(CH_2)_2C(O)NH(CH_2)_4-$, $-(CH_2)_2C(O)NH(CH_2)_5-$, $-(CH_2)_3C(O)NH(CH_2)_3-$, $-(CH_2)_3C(O)NH(CH_2)_4-$, $-$ $(CH_2)_4C(O)NH(CH_2)_4-$, $-(CH_2)_5C(O)NH(CH_2)_5-$, $-(CH_2)_6C(O)NH(CH_2)_7-$, $-(CH_2)_6C(O)NH(CH_2)_6-$, $-$ $(CH_2)_7C(O)NH(CH_2)_7-$, $-(CH_2)_8C(O)NH(CH_2)_8$, $-(CH_2)_9C(O)NH(CH_2)_9-$, $-(CH_2)_{10}C(O)NH(CH_2)_{10}-$, $-$ $(CH_2)_2C(O)NH(CH_2)_2-O-(CH_2)_2-$, $-CH_2NHC(O)CH_2-$, $-(CH_2)_2NHC(O)(CH_2)_2-$, $-$ $(CH_2)_2NHC(O)(CH_2)_3-$, $-(CH_2)_2NHC(O)(CH_2)_4-$, $-(CH_2)_2NHC(O)(CH_2)_5-$, $-(CH_2)_3NHC(O)(CH_2)_3-$, $-$ $(CH_2)_3NHC(O)(CH_2)_4-$, $-(CH_2)_4NHC(O)(CH_2)_4-$, $-(CH_2)_5NHC(O)(CH_2)_5-$, $-(CH_2)_6NHC(O)(CH_2)_7-$, $-$ $(CH_2)_6NHC(O)(CH_2)_6-$, $-(CH_2)_7NHC(O)(CH_2)_7-$, $-(CH_2)_8NHC(O)(CH_2)_8$, $-(CH_2)_9NHC(O)(CH_2)_9-$, $-$ $(CH_2)_{lo}NHC(O)(CH_2)_{10}-$, $-(CH_2)_4NHC(O)(CH_2)_8-$, $-(CH_2)_2NHC(O)(CH_2)_2-O-(CH_2)_2-$, $-$ $(CH_2)_4NHC(O)CH_2-$, $-CH_2-$piperidinylene$-CH_2-$, $-CH_2-$piperidinylene$-(CH_2)_2-$, $-CH_2-$piperidinylene$-(CH_2)_3-$, $-CH_2-$piperidinylene$-(CH_2)_4-$, $-CH_2-$piperidinylene$-(CH_2)_5-$, $-CH_2-$piperidinylene$-(CH_2)_6-$, $-$ $CH_2-$piperidinylene$-(CH_2)_7-$, $-CH_2-$piperidinylene$-(CH_2)_8-$, $-(CH_2)_2-$piperidinylene$-(CH_2)_1-$, $-(CH_2)_2-$piperidinylene$-(CH_2)_2-$, $-(CH_2)_2-$piperidinylene$-(CH_2)_3-$, $-(CH_2)_2-$piperidinylene$-(CH_2)_4-$, $-(CH_2)_2-$piperidinylene$-(CH_2)_5-$, $-(CH_2)_2-$piperidinylene$-(CH_2)_6-$, $-(CH_2)_2-$piperidinylene$-(CH_2)_7-$, $-(CH_2)_2-$piperidinylene$-(CH_2)_8-$, $-(CH_2)_3-$piperidinylene$-CH_2-$, $-(CH_2)_3-$piperidinylene$-(CH_2)_2-$, $-(CH_2)_3-$piperidinylene$-(CH_2)_3-$, $-(CH_2)_3-$piperidinyle-

ne-$(CH_2)_4$-, -$(CH_2)_3$-piperidinylene-$(CH_2)_5$-, -$(CH_2)_3$-piperidinylene-$(CH_2)_6$-, -$(CH_2)_3$-piperidinylene-$(CH_2)_7$-, -$(CH_2)_3$-piperidinylene-$(CH_2)_8$-, -$(CH_2)_4$-piperidinylene-$CH_2$-, -$(CH_2)_4$-piperidinylene-$(CH_2)_2$-, -$(CH_2)_4$-piperidinylene-$(CH_2)_3$-, -$(CH_2)_4$-piperidinylene-$(CH_2)_4$-, -$(CH_2)_4$-piperidinylene-$(CH_2)_5$-, -$(CH_2)_4$-piperidinylene-$(CH_2)_6$-, -$(CH_2)_4$-piperidinylene-$(CH_2)_7$-, -$(CH_2)_4$-piperidinylene-$(CH_2)_8$-, -$( CH_2)_5$-piperidinylene-$( CH_2)_1$-, -$(CH_2)_5$-piperidinylene-$(CH_2)_2$-, -$(CH_2)_5$-piperidinylene-$(CH_2)_3$-, -$(CH_2)_5$-piperidinylene-$(CH_2)_4$-, -$(CH_2)_5$-piperidinylene-$(CH_2)_5$-, -$(CH_2)_5$-piperidinylene-$(CH_2)_6$-, -$(CH_2)_5$-piperidinylene-$(CH_2)_7$-, -$(CH_2)_5$-piperidinylene-$(CH_2)_8$-, -$(CH_2)_6$-piperidinylene-$(CH_2)_1$-, -$(CH_2)_6$-piperidinylene-$(CH_2)_2$-, -$(CH_2)_6$-piperidinylene-$(CH_2)_3$-, -$(CH_2)_6$-piperidinylene-$(CH_2)_4$-, -$(CH_2)_6$-piperidinylene-$(CH_2)_5$-, -$(CH_2)_6$-piperidinylene-$(CH_2)_6$-, -$(CH_2)_6$-piperidinylene-$(CH_2)_7$-, -$(CH_2)_6$-piperidinylene-$(CH_2)_8$-, -$(CH_2)_7$-piperidinylene-$(CH_Z)_1$-, -$(CH_2)_7$-piperidinylene-$(CH_2)_2$-, -$(CH_2)_7$-piperidinylene-$(CH_2)_3$-, -$(CH_2)_7$-piperidinylene-$(CH_2)_4$-, -$(CH_2)_7$-piperidinylene-$(CH_2)_8$-, -$(CH_Z)$s-piperidinylene-$CH_Z$-, -$(CH_2)_8$-piperidinylene-$(CH_2)_2$-, -$(CH_2)_8$-piperidinylene-$(CH_2)_3$-, -$(CH_2)_8$-piperidinylene-$(CH_2)_4$-, -$(CH_2)_8$-piperidinylene-$(CH_2)_5$-, -$(CH_2)_8$-piperidinylene-$(CH_2)_6$-, -$(CH_2)_8$-piperidinylene-$(CH_2)_7$-, -$(CH_2)_8$-piperidinylene-$(CH_2)_8$-, -$CH_2$-piperazinylene-$CH_2$-, -$CH_2$-piperazinylene-$(CH_2)_2$-, -$CH_2$-piperazinylene-$(CH_2)_3$-, -$CH_2$-piperazinylene-$(CH_2)_4$-, -$CH_2$-piperazinylene-$(CH_2)_5$-, -$CH_2$-piperazinylene-$(CH_2)_6$-, -$CH_2$-piperazinylene-$(CH_2)_7$-, -$CH_2$-piperazinylene-$(CH_2)_8$-, -$(CH_2)_2$-piperazinylene-$(CH_2)_1$-, -$(CH_2)_2$-piperazinylene-$(CH_2)_2$-, -$(CH_2)_2$-piperazinylene-$(CH_2)_3$-, -$(CH_2)_2$-piperazinylene-$(CH_2)_4$-, -$(CH_2)_2$-piperazinylene-$(CH_2)_5$-, -$(CH_2)_2$-piperazinylene-$(CH_2)_6$-, -$(CH_2)_2$-piperazinylene-$(CH_2)_7$-, -$(CH_2)_2$-piperazinylene-$(CH_2)_8$-, -$(CH_2)_3$-piperazinylene-$CH_2$-, -$(CH_2)_3$-piperazinylene-$(CH_2)_2$-, -$(CH_2)_3$-piperazinylene-$(CH_2)_3$-, -$(CH_2)_3$-piperazinylene-$(CH_2)_4$-, -$(CH_2)_3$-piperazinylene-$(CH_2)_5$-, -$(CH_2)_3$-piperazinylene-$(CH_2)_6$-, -$(CH_2)_3$-piperazinylene-$(CH_2)_7$-, -$(CH_2)_3$-piperazinylene-$(CH_2)_8$-, -$(CH_2)_4$-piperazinylene-$CH_2$-, -$(CH_2)_4$-piperazinylene-$(CH_2)_2$-, -$(CH_2)_4$-piperazinylene-$(CH_2)_3$-, -$(CH_2)_4$-piperazinylene-$(CH_2)_4$-, -$(CH_2)_4$-piperazinylene-$(CH_2)_5$-, -$(CH_2)_4$-piperazinylene-$(CH_2)_6$-, -$(CH_2)_4$-piperazinylene-$(CH_2)_7$-, -$(CH_2)_4$-piperazinylene-$(CH_2)_8$-, -$(CH_2)_5$-piperazinylene-$(CH_2)_1$-, -$(CH_2)_5$-piperazinylene-$(CH_2)_2$-, -$(CH_2)_5$-piperazinylene-$(CH_2)_3$-, -$(CH_2)_5$-piperazinylene-$(CH_2)_4$-, -$(CH_2)_5$-piperazinylene-$(CH_2)_5$-, -$(CH_2)_5$-piperazinylene-$(CH_2)_6$-, -$(CH_2)_5$-piperazinylene-$(CH_2)_7$-, -$(CH_2)_5$-piperazinylene-$(CH_2)_5$-, -$(CH_2)_6$-piperazinylene-$(CH_2)_1$-, -$(CH_2)_6$-piperazinylene-$(CH_2)_2$-, -$(CH_2)_6$-piperazinylene-$(CH_2)_3$-, -$(CH_2)_6$-piperazinylene-$(CH_2)_4$-, -$(CH_2)_6$-piperazinylene-$(CH_2)_5$-, -$(CH_2)_6$-piperazinylene-$(CH_2)_6$-, -$(CH_2)_6$-piperazinylene-$(CH_2)_7$-, -$(CH_2)_6$-piperazinylene-$(CH_2)_8$-, -$(CH_2)_7$-piperazinylene-$(CH_2)$i-, -$(CH_2)_7$-piperazinylene-$(CH_2)_2$-, -$(CH_2)_7$-piperazinylene-$(CH_2)_3$-, -$(CH_2)_7$-piperazinylene-$(CH_2)_4$-, -$(CH_2)_7$-piperazinylene-$(CH_2)_8$-, -$(CH_2)_8$-piperazinylene-$CH_2$-, -$(CH_2)_8$-piperazinylene-$(CH_2)_2$-, -$(CH_2)_8$-piperazinylene-$(CH_2)_3$-, -$(CH_2)_8$-piperazinylene-$(CH_2)_4$-, -$(CH_2)_5$-piperazinylene-$(CH_2)_5$-, -$(CH_2)_8$-piperazinylene-$(CH_2)_6$-, -$(CH_2)_8$-piperazinylene-$(CH_2)_7$-, -$(CH_2)_8$-piperazinylene-$(CH_2)_8$-, -$CH_2$-propylene-$CH_2$-, -$CH_2$-propylene-$(CH_2)_2$-, -$CH_2$-propylene-$(CH_2)_3$-, -$CH_2$-propylene-$(CH_2)_4$-, -$CH_2$-propylene-$(CH_2)_5$-, -$CH_2$-propylene-$(CH_2)_6$-, -$CH_2$-propylene-$(CH_2)_7$-, -$CH_2$-propylene-$(CH_2)_8$-, -$(CH_2)_2$-propylene-$(CH_2)_1$-, -$(CH_2)_2$-propylene-$(CH_2)_2$-, -$(CH_2)_2$-propylene-$(CH_2)_3$-, -$(CH_2)_2$-propylene-$(CH_2)_4$-, -$(CH_2)_2$-propylene-$(CH_2)_5$-, -$(CH_2)_2$-propylene-$(CH_2)_6$-, -$(CH_2)_2$-propylene-$(CH_2)_7$-, -$(CH_2)_2$-propylene-$(CH_2)_8$-, -$(CH_2)_3$-propylene-$CH_2$-, -$(CH_2)_3$-propylene-$(CH_2)_2$-, -$(CH_2)_3$-propylene-$(CH_2)_3$-, -$(CH_2)_3$-propylene-$(CH_2)_4$-,-$(CH_2)_3$-propylene-$(CH_2)_5$-, -$(CH_2)_3$-propylene-$(CH_2)_6$-, -$(CH_2)_3$-propylene-$(CH_2)_7$-, -$(CH_2)_3$-propylene-$(CH_2)_8$-, -$(CH_2)_4$-propylene-$CH_2$-, -$(CH_2)_4$-propylene-$(CH_2)_2$-, -$(CH_2)_4$-propylene-$(CH_2)_3$-, -$(CH_2)_4$-propylene-$(CH_2)_4$-, -$(CH_2)_4$-propylene-$(CH_2)_5$-, -$(CH_2)_4$-propylene-$(CH_2)_6$-, -$(CH_2)_4$-propylene-$(CH_2)_7$-, -$(CH_2)_4$-propylene-$(CH_2)$s-, -$(CH_2)_5$-propylene-$(CH_2)_1$-, -$(CH_2)_5$-propylene-$(CH_2)_2$-, -$(CH_2)_5$-propylene-$(CH_2)_3$-, -$(CH_2)_5$-propylene-$(CH_2)_4$-, -$(CH_2)_5$-propylene-$(CH_2)_5$-, -$(CH_2)_5$-propylene-$(CH_2)_6$-, -$(CH_2)_5$-propylene-$(CH_2)_7$-, -$(CH_2)_5$-propylene-$(CH_2)_8$-, -$(CH_2)_6$-propylene-$(CH_2)_1$-, -$(CH_2)_6$-propylene-$(CH_2)_2$-, -$(CH_2)_6$-propylene-$(CH_2)_3$-, -$(CH_2)_6$-propylene-$(CH_2)_4$-, -$(CH_2)_6$-propylene-$(CH_2)_5$-, -$(CH_2)_6$-propylene-$(CH_2)_6$-, -$(CH_2)_6$-propylene-$(CH_2)_7$-, -$(CH_2)_6$-propylene-$(CH_2)$8-, -$(CH_2)_7$-propylene-$(CH_2)_1$-, -$(CH_2)_7$-propylene-$(CH_2)_2$-, -$(CH_2)_7$-propylene-$(CH_2)_3$-, -$(CH_2)_7$-propylene-$(CH_2)_4$-, -$(CH_2)_7$-propylene-$(CH_2)$s-, -$(CH_2)_8$-propylene-$CH_2$-, -$(CH_2)_8$-propylene-$(CH_2)_2$-, -$(CH_2)_8$-propylene-$(CH_2)_3$-, -$(CH_2)_8$-propylene-$(CH_2)_4$-, -$(CH_2)_8$-propylene-$(CH_2)_5$-, -$(CH_2)_8$-propylene-$(CH_2)_6$-, -$(CH_2)_8$-propylene-$(CH_2)_7$-, -$(CH_2)_8$-propylene-$(CH_2)_8$-, -$CH_2$-diazacycloheptanylene-$CH_2$-, -$CH_2$-diazacycloheptanylene-$(CH_2)_2$-, -$CH_2$-diazacycloheptanylene-$(CH_2)_3$-, -$CH_2$-diazacycloheptanylene-$(CH_2)_4$-, -$CH_2$-diazacycloheptanylene-$(CH_2)_5$-, -$CH_2$-diazacycloheptanylene-$(CH_2)_6$-, -$CH_2$-diazacycloheptanylene-$(CH_2)_7$-, -$CH_2$-diazacycloheptanylene-$(CH_2)_8$-, -$(CH_2)_2$-diazacycloheptanylene-$(CH_2)_1$-, -$(CH_2)_2$-diazacycloheptanylene-$(CH_2)_2$-, -$(CH_2)_2$-diazacycloheptanylene-$(CH_2)_3$-, -$(CH_2)_2$-diazacycloheptanylene-$(CH_2)_4$-, -$(CH_2)_2$-diazacycloheptanylene-$(CH_2)_5$-, -$(CH_2)_2$-diazacycloheptanylene-$(CH_2)_6$-, -$(CH_2)_2$-diazacycloheptanylene-$(CH_2)_7$-, -$(CH_2)_2$-diazacycloheptanylene-$(CH_2)_8$-, -$(CH_2)_3$-diazacycloheptanylene-$CH_2$-, -$(CH_2)_3$-diazacycloheptanylene-$(CH_2)_2$-, -$(CH_2)_3$-diazacycloheptanylene-$(CH_2)_3$-, -$(CH_2)_3$-diazacycloheptanylene-$(CH_2)_4$-, -$(CH_2)_3$-diazacycloheptanylene-$(CH_2)_5$-, -$(CH_2)_3$-diazacycloheptanylene-$(CH_2)_6$-, -$(CH_2)_3$-diazacycloheptanylene-$(CH_2)_7$-, -$(CH_2)_3$-diazacycloheptanylene-$(CH_2)_8$-, -$(CH_2)_4$-diazacycloheptanylene-$CH_2$-, -$(CH_2)_4$-diazacycloheptanylene-$(CH_2)_2$-, -$(CH_2)_4$-diazacycloheptanylene-$(CH_2)_3$-, -$(CH_2)_4$-diazacycloheptanylene-$(CH_2)_4$-, -$(CH_2)_4$-diazacycloheptanylene-$(CH_2)_5$-, -$(CH_2)_4$-diazacycloheptanylene-$(CH_2)_6$-, -$(CH_2)_4$-diazacycloheptanylene-$(CH_2)_7$-, -$(CH_2)_4$-diazacycloheptanylene-$(CH_2)_8$-, -$(CH_2)_5$-diazacycloheptanylene-$(CH_2)_1$-, -$(CH_2)_5$-diazacycloheptanylene-$(CH_2)_2$-, -$(CH_2)_5$-diazacycloheptanylene-$(CH_2)_3$-, -$(CH_2)_5$-diazacycloheptanylene-$(CH_2)_4$-, -$(CH_2)_5$-diazacycloheptanylene-$(CH_2)_5$-, -$(CH_2)_5$-diazacycloheptanylene-$(CH_2)_6$-, -$(CH_2)_5$-diazacycloheptanylene-$(CH_2)_7$-, -$(CH_2)_5$-diazacycloheptanylene-$(CH_2)_8$-, -$(CH_2)_6$-diazacycloheptanylene-$(CH_2)_1$-, -$(CH_2)_6$-diazacycloheptanyle-

ne-$(CH_2)_2$-, -$(CH_2)_6$-diazacycloheptanylene-$(CH_2)_3$-, -$(CH_2)_6$-diazacycloheptanylene-$(CH_2)_4$-, -$(CH_2)_6$-diazacycloheptanylene-$(CH_2)_5$-, -$(CH_2)_6$-diazacycloheptanylene-$(CH_2)_6$-, -$(CH_2)_6$-diazacycloheptanylene-$(CH_2)_7$-, -$(CH_2)_6$-diazacycloheptanylene-$(CH_2)_8$-, -$(CH_2)_7$-diazacycloheptanylene-$(CH_2)_1$-, -$(CH_2)_7$-diazacycloheptanylene-$(CH_2)_2$-, -$(CH_2)_7$-diazacycloheptanylene-$(CH_2)_3$-, -$(CH_2)_7$-diazacycloheptanylene-$(CH_2)_4$-, -$(CH_2)_7$-diazacycloheptanylene-$(CH_2)_8$-, -$(CH_2)_8$-diazacycloheptanylene-$CH_2$-, -$(CH_2)_8$-diazacycloheptanylene-$(CH_2)_2$-, -$(CH_2)_8$-diazacycloheptanylene-$(CH_2)_3$-, -$(CH_2)_8$-diazacycloheptanylene-$(CH_2)_4$-, -$(CH_2)_8$-diazacycloheptanylene-$(CH_2)_5$-, -$(CH_2)_8$-diazacycloheptanylene-$(CH_2)_6$-, -$(CH_2)_8$-diazacycloheptanylene-$(CH_2)_7$-, -$(CH_2)_8$-diazacycloheptanylene-$(CH_2)_8$-, -$CH_2$-diazabicyclo [2.2.1]heptanylene-$CH_2$-, -$CH_2$-diazabicyclo[2.2.1]heptanylene-$(CH_2)_2$-, -$CH_2$-diazabicyclo[2.2.1]heptanylene-$(CH_2)_3$-, -$CH_2$-diazabicyclo[2.2.1]heptanylene-$(CH_2)_4$-, -$CH_2$-diazabicyclo[2.2.1]heptanylene-$(CH_2)_5$-, -$CH_2$-diazabicyclo[2.2.1] heptanylene-$(CH_2)_6$-, -$CH_2$-diazabicyclo[2.2.1]heptanylene-$(CH_2)_7$-, -$CH_2$-diazabicyclo[2.2.1]heptanylene-$(CH_2)_8$-, -$(CH_2)_2$-diazabicyclo[2.2.1]heptanylene-$(CH_2)_1$-, -$(CH_2)_2$-diazabicyclo[2.2.1]heptanylene-$(CH_2)_2$-, -$(CH_2)_2$-diazabicyclo[2.2.1]heptanylene-$(CH_2)_3$-, -$(CH_2)_2$-diazabicyclo[2.2.1]heptanylene-$(CH_2)_4$-, -$(CH_2)_2$-diazabicyclo[2.2.1]heptanylene-$(CH_2)_5$-, -$(CH_2)_2$-diazabicyclo[2.2.1]heptanylene-$(CH_2)_6$-, -$(CH_2)_2$-diazabicyclo[2.2.1]heptanylene-$(CH_2)_7$-, -$(CH_2)_2$-diazabicyclo[2.2.1]heptanylene-$(CH_2)_8$-, -$(CH_2)_3$-diazabicyclo[2.2.1]heptanylene-$CH_2$-, -$(CH_2)_3$-diazabicyclo[2.2.1]heptanylene-$(CH_2)_2$-, -$(CH_2)_3$-diazabicyclo[2.2.1]heptanylene-$(CH_2)_3$-, -$(CH_2)_3$-diazabicyclo[2.2.1]heptanylene-$(CH_2)_4$-, -$(CH_2)_3$-diazabicyclo[2.2.1]heptanylene-$(CH_2)_3$-, -$(CH_2)_3$-diazabicyclo[2.2.1]heptanylene-$(CH_2)_6$-, -$(CH_2)_3$-diazabicyclo[2.2.1]heptanylene-$(CH_2)_7$-, -$(CH_2)_3$-diazabicyclo[2.2.1]heptanylene-$(CH_2)_8$-, -$(CH_2)_4$-diazabicyclo[2.2.1]heptanylene-$CH_2$-, -$(CH_2)_4$-diazabicyclo[2.2.1]heptanylene-$(CH_2)_2$-, -$(CH_2)_4$-diazabicyclo[2.2.1]heptanylene-$(CH_2)_3$-, -$(CH_2)_4$-diazabicyclo[2.2.1]heptanylene-$(CH_2)_4$-, -$(CH_2)_4$-diazabicyclo[2.2.1]heptanylene-$(CH_2)_5$-, -$(CH_2)_4$-diazabicyclo[2.2.1] heptanylene-$(CH_2)_6$-, -$(CH_2)_4$-diazabicyclo[2.2.1]heptanylene-$(CH_2)_7$-, -$(CH_2)_4$-diazabicyclo[2.2.1]heptanylene-$(CH_2)_8$-, -$(CH_2)_5$-diazabicyclo[2.2.1]heptanylene-$(CH_2)_1$-, -$(CH_2)_5$-diazabicyclo[2.2.1]heptanylene-$(CH_2)_2$-, -$(CH_2)_5$-diazabicyclo[2.2.1]heptanylene-$(CH_2)_3$-, -$(CH_2)_5$-diazabicyclo[2.2.1]heptanylene-$(CH_2)_4$-, -$(CH_2)_5$-diazabicyclo[2.2.1]heptanylene-$(CH_2)_5$-, -$(CH_2)_5$-diazabicyclo[2.2.1]heptanylene-$(CH_2)_6$-, -$(CH_2)_5$-diazabicyclo[2.2.1]heptanylene-$(CH_2)_7$-, -$(CH_2)_5$-diazabicyclo[2.2.1]heptanylene-$(CH_2)_8$-, -$(CH_2)_6$-diazabicyclo[2.2.1]heptanylene-$(CH_2)_1$-, -$(CH_2)_6$-diazabicyclo[2.2.1]heptanylene-$(CH_2)_2$-, -$(CH_2)_6$-diazabicyclo[2.2.1]heptanylene-$(CH_2)_3$-, -$(CH_2)_6$-diazabicyclo[2.2.1] heptanylene-$(CH_2)_4$-, -$(CH_2)_6$-diazabicyclo[2.2.1]heptanylene-$(CH_2)_5$-, -$(CH_2)_6$-diazabicyclo[2.2.1]heptanylene-$(CH_2)_6$-, -$(CH_2)_6$-diazabicyclo[2.2.1]heptanylene-$(CH_2)_7$-, -$(CH_2)_6$-diazabicyclo[2.2.1]heptanylene-$(CH_2)_8$-, -$(CH_2)_7$-diazabicyclo[2.2.1]heptanylene-$(CH_2)_1$-, -$(CH_2)_7$-diazabicyclo[2.2.1]heptanylene-$(CH_2)_2$-, -$(CH_2)_7$-diazabicyclo[2.2.1]heptanylene-$(CH_2)_3$-, -$(CH_2)_7$-diazabicyclo[2.2.1]heptanylene-$(CH_2)_4$-, -$(CH_2)_7$-diazabicyclo[2.2.1]heptanylene-$(CH_2)_8$-, -$(CH_2)_8$-diazabicyclo[2.2.1]heptanylene-$CH_2$-, -$(CH_2)_8$-diazabicyclo[2.2.1]heptanylene-$(CH_2)_2$-, -$(CH_2)_8$-diazabicyclo[2.2.1]heptanylene-$(CH_2)_3$-, -$(CH_2)_8$-diazabicyclo[2.2.1]heptanylene-$(CH_2)_4$-, -$(CH_2)_8$-diazabicyclo[2.2.1] heptanylene-$(CH_2)_5$-, -$(CH_2)_8$-diazabicyclo[2.2.1]heptanylene-$(CH_2)_6$-, -$(CH_2)_8$-diazabicyclo[2.2. l]heptanylene-$(CH_2)_7$-, -$(CH_2)_8$-diazabicyclo[2.2.1]heptanylene-$(CH_2)_8$-, -$CH_2$-diazabicyclo[3.1.1]heptanylene-$CH_2$-, -$CH_2$-diazabicyclo[3.1.1]heptanylene-$(CH_2)_2$-, -$CH_2$-diazabicyclo[3.1.1]heptanylene-$(CH_2)_3$-, -$CH_2$-diazabicyclo[3.1.1]heptanylene-$(CH_2)_4$-, -$CH_2$-diazabicyclo[3.1.1]heptanylene-$(CH_2)_5$-, -$CH_2$-diazabicyclo[3.1.1]heptanylene-$(CH_2)_6$-, -$CH_2$-diazabicyclo[3.1.1]heptanylene-$(CH_2)_7$-, -$CH_2$-diazabicyclo[3.1.1]heptanylene-$(CH_2)_8$-, -$(CH_2)_2$-diazabicyclo[3.1.1]heptanylene-$(CH_2)_1$-, -$(CH_2)_2$-diazabicyclo[3.1.1]heptanylene-$(CH_2)_2$-, -$(CH_2)_2$-diazabicyclo[3. 1.1]heptanylene-$(CH_2)_3$-, -$(CH_2)_2$-diazabicyclo[3.1.1]heptanylene-$(CH_2)_4$-, -$(CH_2)_2$-diazabicyclo[3. 1. 1]heptanylene-$(CH_2)_5$-, -$(CH_2)_2$-diazabicyclo[3.1.1] heptanylene-$(CH_2)_6$-, -$(CH_2)_2$-diazabicyclo[3.1.1]heptanylene-$(CH_2)_7$-, -$(CH_2)_2$-diazabicyclo[3.1.1]heptanylene-$(CH_2)_8$-, -$(CH_2)_3$-diazabicyclo[3.1.1]heptanylene-$CH_2$-, -$(CH_2)_3$-diazabicyclo[3.1.1]heptanylene-$(CH_2)_2$-, -$(CH_2)_3$-diazabicyclo[3.1.1]heptanylene-$(CH_2)_3$-, -$(CH_2)_3$-diazabicyclo[3.1.1]heptanylene-$(CH_2)_4$-, -$(CH_2)_3$-diazabicyclo[3.1.1] heptanylene-$(CH_2)_5$-, -$(CH_2)_3$-diazabicyclo[3.1.1]heptanylene-$(CH_2)_6$-, -$(CH_2)_3$-diazabicyclo[3.1.1]heptanylene-$(CH_2)_7$-, -$(CH_2)_3$-diazabicyclo[3.1.1]heptanylene-$(CH_2)_8$-, -$(CH_2)_4$-diazabicyclo[3.1.1]heptanylene-$CH_2$-, -$(CH_2)_4$-diazabicyclo[3.1.1]heptanylene-$(CH_2)_2$-, -$(CH_2)_4$-diazabicyclo[3.1.1]heptanylene-$(CH_2)_3$-, -$(CH_2)_4$-diazabicyclo[3.1.1] heptanylene-$(CH_2)_4$-, -$(CH_2)_4$-diazabicyclo[3.1.1]heptanylene-$(CH_2)_5$-, -$(CH_2)_4$-diazabicyclo[3.1.1]heptanylene-$(CH_2)_6$-, -$(CH_2)_4$-diazabicyclo[3.1.1]heptanylene-$(CH_2)_7$-, -$(CH_2)_4$-diazabicyclo[3.1.1]heptanylene-$(CH_2)_8$-, -$(CH_2)_5$-diazabicyclo[3.1.1]heptanylene-$(CH_2)_1$-, -$(CH_2)_5$-diazabicyclo[3.1.1]heptanylene-$(CH_2)_2$-, -$(CH_2)_5$-diazabicyclo[3.1.1]heptanylene-$(CH_2)_3$-, -$(CH_2)_5$-diazabicyclo[3.1.1]heptanylene-$(CH_2)_4$-, -$(CH_2)_5$-diazabicyclo[3.1.1]heptanylene-$(CH_2)_5$-, -$(CH_2)_5$-diazabicyclo[3.1.1]heptanylene-$(CH_2)_6$-, -$(CH_2)_5$-diazabicyclo[3.1.1]heptanylene-$(CH_2)_7$-, -$(CH_2)_5$-diazabicyclo[3.1.1]heptanylene-$(CH_2)_8$-, -$(CH_2)_6$-diazabicyclo[3.1.1]heptanylene-$(CH_2)_1$-, -$(CH_2)_6$-diazabicyclo[3.1.1] heptanylene-$(CH_2)_2$-, -$(CH_2)_6$-diazabicyclo[3.1.1]heptanylene-$(CH_2)_3$-, -$(CH_2)_6$-diazabicyclo[3.1.1]heptanylene-$(CH_2)_4$-, -$(CH_2)_6$-diazabicyclo[3.1.1]heptanylene-$(CH_2)_5$-, -$(CH_2)_6$-diazabicyclo[3.1.1]heptanylene-$(CH_2)_6$-, -$(CH_2)_6$-diazabicyclo[3.1.1]heptanylene-$(CH_2)_7$-, -$(CH_2)_6$-diazabicyclo[3.1.1]heptanylene-$(CH_2)_8$-, -$(CH_2)_7$-diazabicyclo[3.1.1]heptanylene-$(CH_2)_1$-, -$(CH_2)_7$-diazabicyclo[3.1.1]heptanylene-$(CH_2)_2$-, -$(CH_2)_7$-diazabicyclo[3.1.1]heptanylene-$(CH_2)_3$-, -$(CH_2)_7$-diazabicyclo[3.1.1]heptanylene-$(CH_2)_4$-, -$(CH_2)_7$-diazabicyclo[3.1.1]heptanylene-$(CH_2)_8$-, -$(CH_2)_8$-diazabicyclo[3.1.1]heptanylene-$CH_2$-, -$(CH_2)_8$-diazabicyclo[3.1.1]heptanylene-$(CH_2)_2$-, -$(CH_2)_8$-diazabicyclo[3.1.1]heptanylene-$(CH_2)_3$-, -$(CH_2)_8$-diazabicyclo[3.1.1]heptanylene-$(CH_2)_4$-, -$(CH_2)_8$-diazabicyclo[3.1.1]heptanylene-$(CH_2)_5$-, -$(CH_2)_8$-diazabicyclo[3.1.1]heptanylene-$(CH_2)_6$-, -$(CH_2)_8$-diazabicyclo[3.1.1]heptanylene-$(CH_2)_7$-, -$(CH_2)_8$-diazabicy-

clo[3.1.1]heptanylene-$(CH_2)_8$-, -$CH_2$-diazabicyclo[3.2.1]octylene-$CH_2$-, -$CH_2$-diazabicyclo[3.2.1]octylene-$(CH_2)_2$-, -$CH_2$-diazabicyclo[3.2.1]octylene-$(CH_2)_3$-, -$CH_2$-diazabicyclo[3.2.1]octylene-$(CH_2)_4$-, -$CH_2$-diazabicyclo[3.2.1]octylene-$(CH_2)_5$-, -$CH_2$-diazabicyclo[3.2.1]octylene-$(CH_2)_6$-, -$CH_2$-diazabicyclo[3.2.1]octylene-$(CH_2)_7$-, -$CH_2$-diazabicyclo[3.2.1]octylene-$(CH_2)_8$-, -$(CH_2)_2$-diazabicyclo[3.2.1]octylene-$(CH_2)_1$-, -$(CH_2)_2$-diazabicyclo[3.2.1]octylene-$(CH_2)_2$-, -$(CH_2)_2$-diazabicyclo[3.2.1]octylene-$(CH_2)_3$-, -$(CH_2)_2$-diazabicyclo[3.2.1]octylene-$(CH_2)_4$-, -$(CH_2)_2$-diazabicyclo[3.2.1]octylene-$(CH_2)_5$-, -$(CH_2)_2$-diazabicyclo[3.2.1]octylene-$(CH_2)_6$-, -$(CH_2)_2$-diazabicyclo[3.2.1]octylene-$(CH_2)_7$-, -$(CH_2)_2$-diazabicyclo[3.2.1]octylene-$(CH_2)s$-, -$(CH_2)_3$-diazabicyclo[3.2.1]octylene-$CH_2$-, -$(CH_2)_3$-diazabicyclo[3.2.1]octylene-$(CH_2)_2$-, -$(CH_2)_3$-diazabicyclo[3.2.1]octylene-$(CH_2)_3$-, -$(CH_2)_3$-diazabicyclo[3.2.1]octylene-$(CH_2)_4$-, -$(CH_2)_3$-diazabicyclo[3.2.1]octylene-$(CH_2)_5$-, -$(CH_2)_3$-diazabicyclo[3.2.1]octylene-$(CH_2)_6$-, -$(CH_2)_3$-diazabicyclo[3.2.1]octylene-$(CH_2)_7$-, -$(CH_2)_3$-diazabicyclo[3.2.1]octylene-$(CH_2)_8$-, -$(CH_2)_4$-diazabicyclo[3.2.1]octylene-$CH_2$-, -$(CH_2)_4$-diazabicyclo[3.2.1]octylene-$(CH_2)_2$-, -$(CH_2)_4$-diazabicyclo[3.2.1]octylene-$(CH_2)_3$-, -$(CH_2)_4$-diazabicyclo[3.2.1]octylene-$(CH_2)_4$-, -$(CH_2)_4$-diazabicyclo[3.2.1]octylene-$(CH_2)_5$-, -$(CH_2)_4$-diazabicyclo[3.2.1]octylene-$(CH_2)_6$-, -$(CH_2)_4$-diazabicyclo[3.2.1]octylene-$(CH_2)_7$-, -$(CH_2)_4$-diazabicyclo[3.2.1]octylene-$(CH_2)_8$-, -$(CH_2)_5$-diazabicyclo[3.2.1]octylene-$(CH_2)_1$-, -$(CH_2)_5$-diazabicyclo[3.2.1]octylene-$(CH_2)_2$-, -$(CH_2)_5$-diazabicyclo[3.2.1]octylene-$(CH_2)_3$-, -$(CH_2)_5$-diazabicyclo[3.2.1]octylene-$(CH_2)_4$-, -$(CH_2)_5$-diazabicyclo[3.2.1]octylene-$(CH_2)_5$-, -$(CH_2)_5$-diazabicyclo[3.2.1]octylene-$(CH_2)_6$-, -$(CH_2)_5$-diazabicyclo[3.2.1]octylene-$(CH_2)_7$-, -$(CH_2)_5$-diazabicyclo[3.2.1]octylene-$(CH_2)_8$-, -$(CH_2)_6$-diazabicyclo[3.2.1]octylene-$(CH_2)_1$-, -$(CH_2)_6$-diazabicyclo[3.2.1]octylene-$(CH_2)_2$-, -$(CH_2)_6$-diazabicyclo[3.2.1]octylene-$(CH_2)_3$-, -$(CH_2)_6$-diazabicyclo[3.2.1]octylene-$(CH_2)_4$-, -$(CH_2)_6$-diazabicyclo[3.2.1]octylene-$(CH_2)_5$-, -$(CH_2)_6$-diazabicyclo[3.2.1]octylene-$(CH_2)_6$-, -$(CH_2)_6$-diazabicyclo[3.2.1]octylene-$(CH_2)_7$-, -$(CH_2)_6$-diazabicyclo[3.2.1]octylene-$(CH_2)_8$-, -$(CH_2)_7$-diazabicyclo[3.2.1]octylene-$(CH_2)_1$-, -$(CH_2)_7$-diazabicyclo[3.2.1]octylene-$(CH_2)_2$-, -$(CH_2)_7$-diazabicyclo[3.2.1]octylene-$(CH_2)_3$-, -$(CH_2)_7$-diazabicyclo[3.2.1]octylene-$(CH_2)_4$-, -$(CH_2)_7$-diazabicyclo[3.2.1]octylene-$(CH_2)_8$-, -$(CH_2)_8$-diazabicyclo[3.2.1]octylene-$CH_2$-, -$(CH_2)_8$-diazabicyclo[3.2.1]octylene-$(CH_2)_2$-, -$(CH_2)_8$-diazabicyclo[3.2.1]octylene-$(CH_2)_3$-, -$(CH_2)_8$-diazabicyclo[3.2.1]octylene-$(CH_2)_4$-, -$(CH_2)_8$-diazabicyclo[3.2.1]octylene-$(CH_2)_5$-, -$(CH_2)_8$-diazabicyclo[3.2.1]octylene-$(CH_2)_6$-, -$(CH_2)_8$-diazabicyclo[3.2.1]octylene-$(CH_2)_7$-, -$(CH_2)_8$-diazabicyclo[3.2.1]octylene-$(CH_2)_8$-, -$CH_2$-diazabicyclo[2.2.2]octylene-$CH_2$-, -$CH_2$-diazabicyclo[2.2.2]octylene-$(CH_2)_2$-, -$CH_2$-diazabicyclo[2.2.2]octylene-$(CH_2)_3$-, -$CH_2$-diazabicyclo[2.2.2]octylene-$(CH_2)_4$-, -$CH_2$-diazabicyclo[2.2.2]octylene-$(CH_2)_5$-, -$CH_2$-diazabicyclo[2.2.2]octylene-$(CH_2)_6$-, -$CH_2$-diazabicyclo[2.2.2]octylene-$(CH_2)_7$-, -$CH_2$-diazabicyclo[2.2.2]octylene-$(CH_2)_8$-, -$(CH_2)_2$-diazabicyclo[2.2.2]octylene-$(CH_2)_1$-, -$(CH_2)_2$-diazabicyclo[2.2.2]octylene-$(CH_2)_2$-, -$(CH_2)_2$-diazabicyclo[2.2.2]octylene-$(CH_2)_3$-, -$(CH_2)_2$-diazabicyclo[2.2.2]octylene-$(CH_2)_4$-, -$(CH_2)_2$-diazabicyclo[2.2.2]octylene-$(CH_2)_5$-, -$(CH_2)_2$-diazabicyclo[2.2.2]octylene-$(CH_2)_6$-, -$(CH_2)_2$-diazabicyclo[2.2.2]octylene-$(CH_2)_7$-, -$(CH_2)_2$-diazabicyclo[2.2.2]octylene-$(CH_2)_8$-, -$(CH_2)_3$-diazabicyclo[2.2.2]octylene-$CH_2$-, -$(CH_2)_3$-diazabicyclo[2.2.2]octylene-$(CH_2)_2$-, -$(CH_2)_3$-diazabicyclo[2.2.2]octylene-$(CH_2)_3$-, -$(CH_2)_3$-diazabicyclo[2.2.2]octylene-$(CH_2)_4$-, -$(CH_2)_3$-diazabicyclo[2.2.2]octylene-$(CH_2)_5$-, -$(CH_2)_3$-diazabicyclo[2.2.2]octylene-$(CH_2)_6$-, -$(CH_2)_3$-diazabicyclo[2.2.2]octylene-$(CH_2)_7$-, -$(CH_2)_3$-diazabicyclo[2.2.2]octylene-$(CH_2)_8$-, -$(CH_2)_4$-diazabicyclo[2.2.2]octylene-$CH_2$-, -$(CH_2)_4$-diazabicyclo[2.2.2]octylene-$(CH_2)_2$-, -$(CH_2)_4$-diazabicyclo[2.2.2]octylene-$(CH_2)_3$-, -$(CH_2)_4$-diazabicyclo[2.2.2]octylene-$(CH_2)_4$-, -$(CH_2)_4$-diazabicyclo[2.2.2]octylene-$(CH_2)_5$-, -$(CH_2)_4$-diazabicyclo[2.2.2]octylene-$(CH_2)_6$-, -$(CH_2)_4$-diazabicyclo[2.2.2]octylene-$(CH_2)_7$-, -$(CH_2)_4$-diazabicyclo[2.2.2]octylene-$(CH_2)_8$-, -$(CH_2)_5$-diazabicyclo[2.2.2]octylene-$(CH_2)_1$-, -$(CH_2)_5$-diazabicyclo[2.2.2]octylene-$(CH_2)_2$-, -$(CH_2)_5$-diazabicyclo[2.2.2]octylene-$(CH_2)_3$-, -$(CH_2)_5$-diazabicyclo[2.2.2]octylene-$(CH_2)_4$-, -$(CH_2)_5$-diazabicyclo[2.2.2]octylene-$(CH_2)_5$-, -$(CH_2)_5$-diazabicyclo[2.2.2]octylene-$(CH_2)_6$-, -$(CH_2)_5$-diazabicyclo[2.2.2]octylene-$(CH_2)_7$-, -$(CH_2)_5$-diazabicyclo[2.2.2]octylene-$(CH_2)_8$-, -$(CH_2)_6$-diazabicyclo[2.2.2]octylene-$(CH_2)_1$-, -$(CH_2)_6$-diazabicyclo[2.2.2]octylene-$(CH_2)_2$-, -$(CH_2)_6$-diazabicyclo[2.2.2]octylene-$(CH_2)_3$-, -$(CH_2)_6$-diazabicyclo[2.2.2]octylene-$(CH_2)_4$-, -$(CH_2)_6$-diazabicyclo[2.2.2]octylene-$(CH_2)_5$-, -$(CH_2)_6$-diazabicyclo[2.2.2]octylene-$(CH_2)_6$-, -$(CH_2)_6$-diazabicyclo[2.2.2]octylene-$(CH_2)_7$-, -$(CH_2)_6$-diazabicyclo[2.2.2]octylene-$(CH_2)_8$-, -$(CH_2)_7$-diazabicyclo[2.2.2]octylene-$(CH_2)_1$-, -$(CH_2)_7$-diazabicyclo[2.2.2]octylene-$(CH_2)_2$-, -$(CH_2)_7$-diazabicyclo[2.2.2]octylene-$(CH_2)_3$-, -$(CH_2)_7$-diazabicyclo[2.2.2]octylene-$(CH_2)_4$-, -$(CH_2)_7$-diazabicyclo[2.2.2]octylene-$(CH_2)_8$-, -$(CH_2)_8$-diazabicyclo[2.2.2]octylene-$CH_2$-, -$(CH_2)_8$-diazabicyclo[2.2.2]octylene-$(CH_2)_2$-, -$(CH_2)_8$-diazabicyclo[2.2.2]octylene-$(CH_2)_3$-, -$(CH_2)_8$-diazabicyclo[2.2.2]octylene-$(CH_2)_4$-, -$(CH_2)_8$-diazabicyclo[2.2.2]octylene-$(CH_2)_5$-, -$(CH_2)_8$-diazabicyclo[2.2.2]octylene-$(CH_2)_6$-, -$(CH_2)_8$-diazabicyclo[2.2.2]octylene-$(CH_2)_7$-, or -$(CH_2)_8$-diazabicyclo[2.2.2]octylene-$(CH_2)_8$-;

wherein the propylene, the piperidinylene, the piperazinylene, the diazacycloheptanylene, the diazabicyclo[2.2.1]heptanylene, the diazabicyclo[3.1.1]heptanylene, the diazabicyclo[3.2.1]octylene, the diazabicyclo[2.2.2]octylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof; and

each $R_{g11}$ independently represents H or $C_{1-3}$ alkyl.

In some embodiments of the compound of Formula (I-8), $L_3$ represents the structure of the following formula:

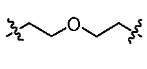 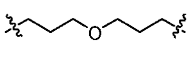 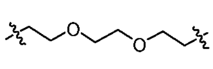 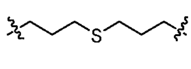 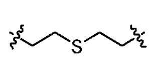 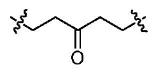

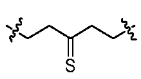 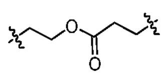 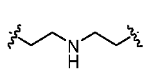 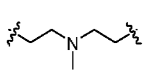 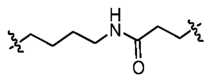 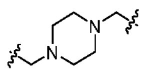 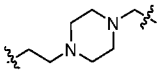

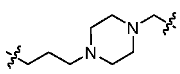 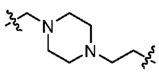 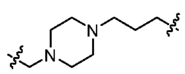 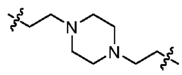 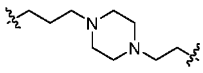 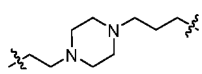

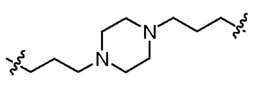 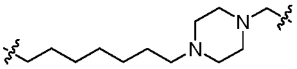 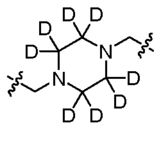 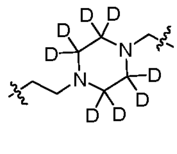 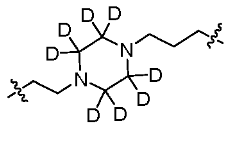

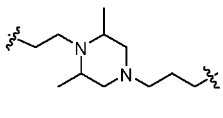 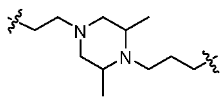 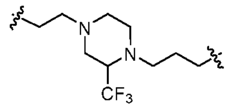 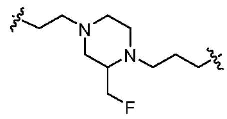 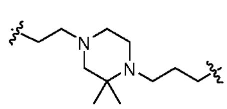

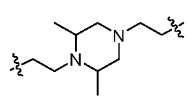 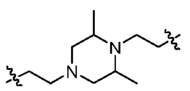 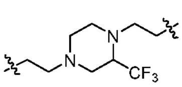 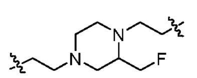 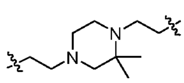 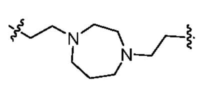

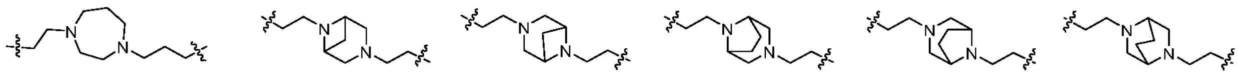

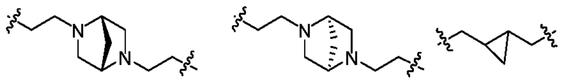 or 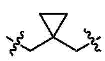

In some embodiments of the compound of Formula (I-8), $R_{b2}$ represents:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, 2-oxobicyclo[2.2.1]heptanyl or bicyclo[2.2.1]heptenyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 3-azaspiro[5.5]undecyl or 7-azaspiro[3.5]nonyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and

wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

phenyl or naphthyl, with each group being optionally substituted with one or more (e.g., 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo [2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl and imidazo [2,1-b]thiazolyl, with each group being optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, wherein the $C_{3-15}$cycloalkyl, the 4- to 15-membered heterocyclyl, the $C_{6-15}$ aryl, and the 5- to 15-membered heteroaryl are each independently optionally substituted with one or more (e.g., 1-20, or 1-15, 1-10, 1-9, 1-8, 1-7, 1-6, 1-5, 1-4, 1-3 or 1-2, or 1) substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

In some embodiments of the compound of Formula (I-8), $R_{b2}$ represents the following formula:

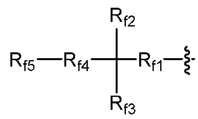,

wherein $R_{f1}$, $R_{f2}$, $R_{f3}$, $R_{f4}$ and $R_{f5}$ are as defined in the compound of Formula (I-2) and the embodiments thereof.

In some embodiments of the compound of Formula (I-8), $R_{b2}$ represents:

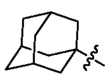 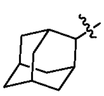 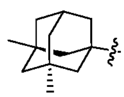 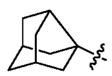 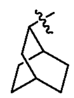 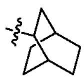 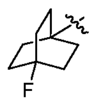 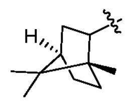 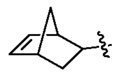 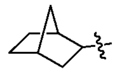

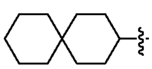 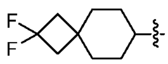 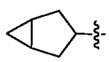 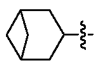 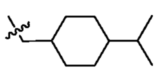 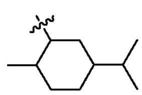 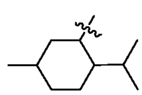 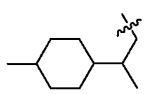

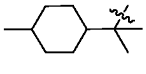 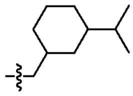 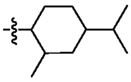 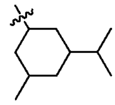 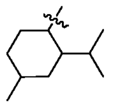 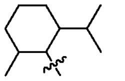 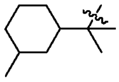 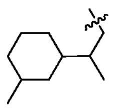 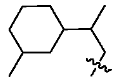

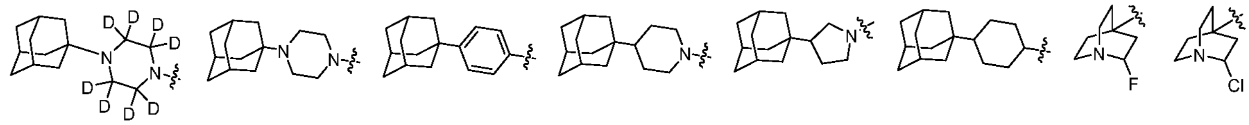

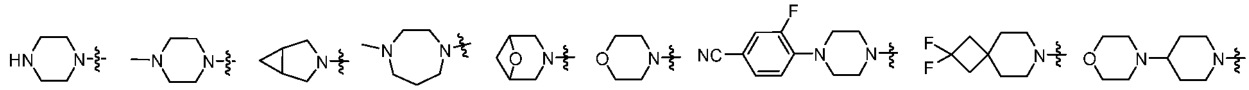

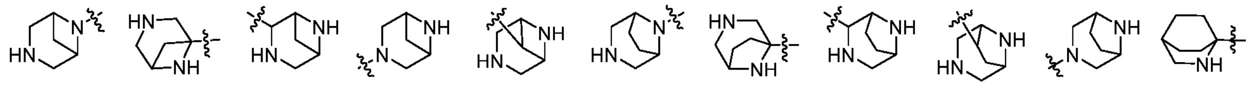

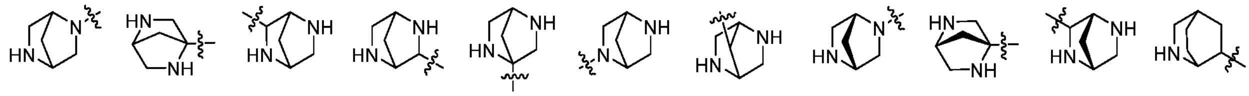

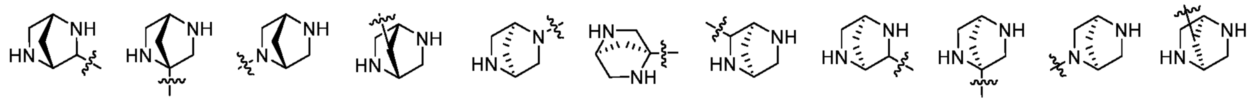

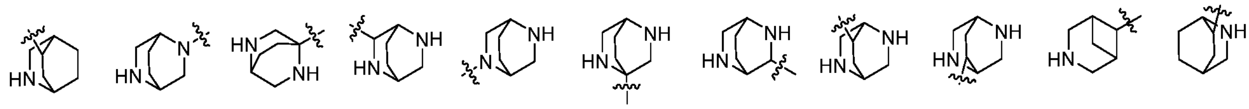

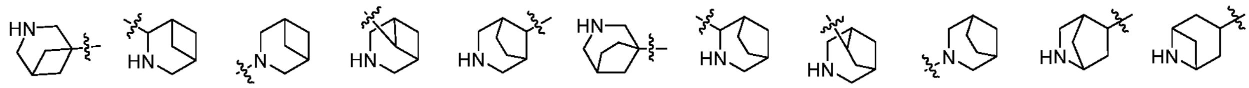

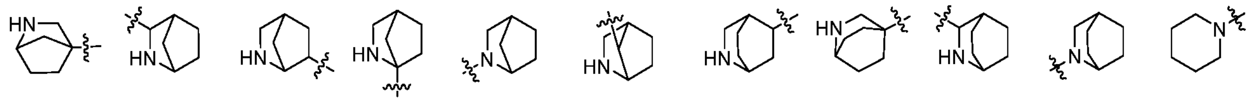

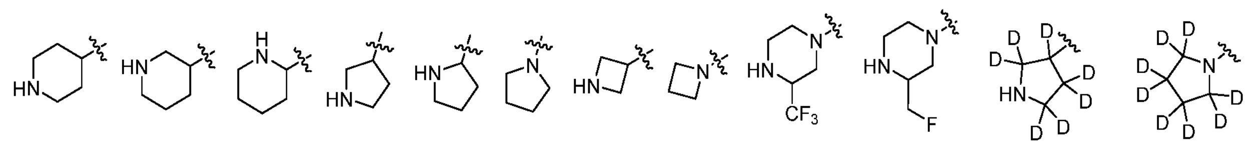

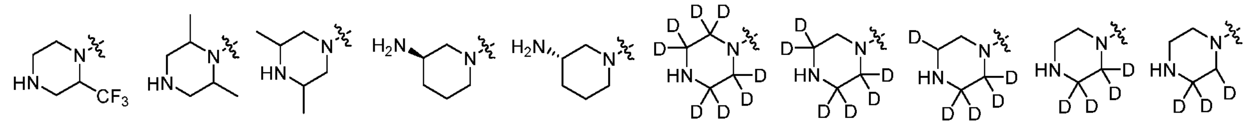

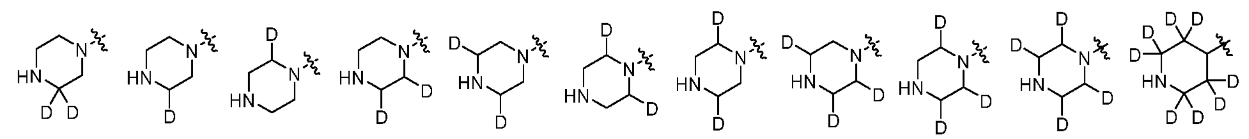

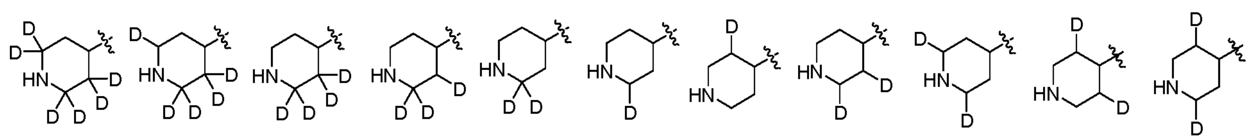

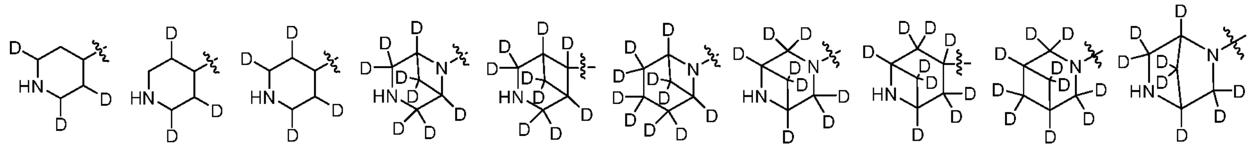

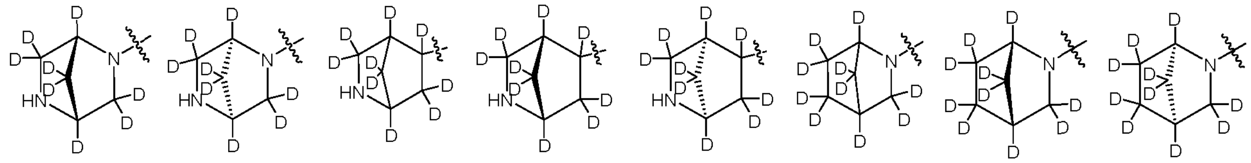

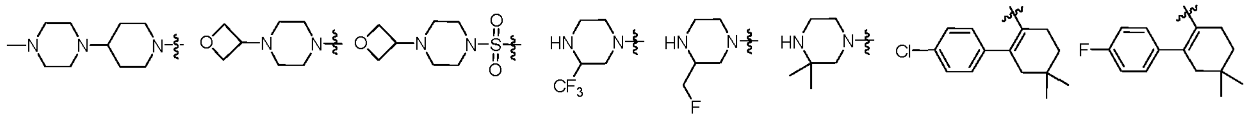

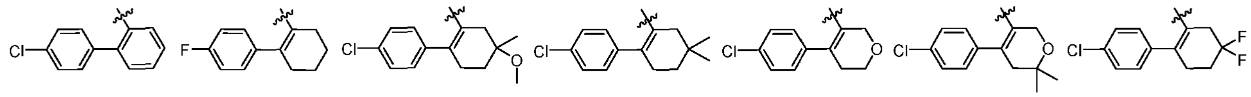

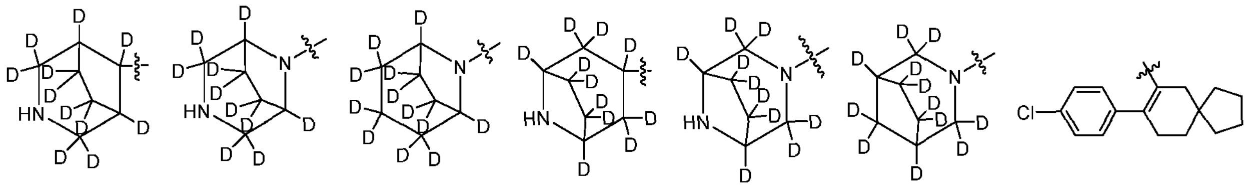

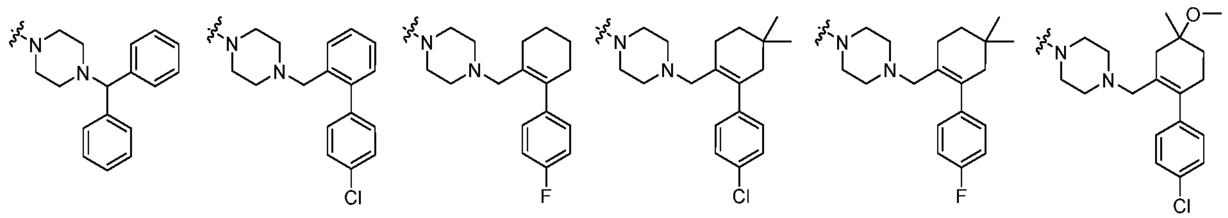

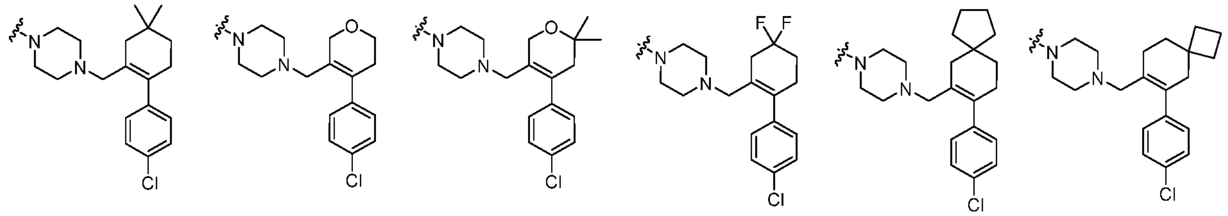

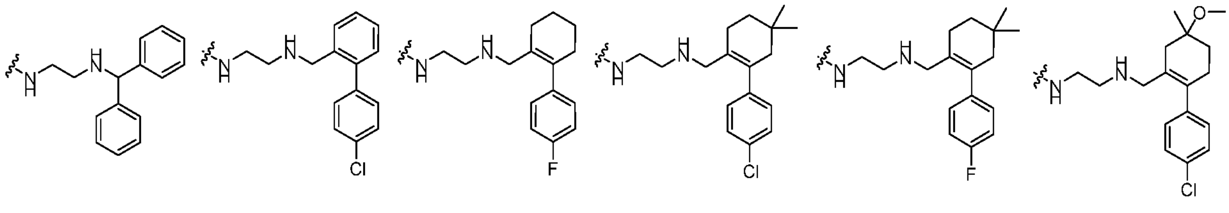

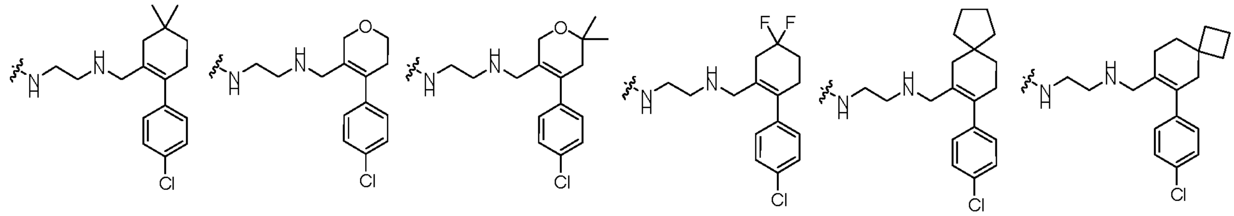

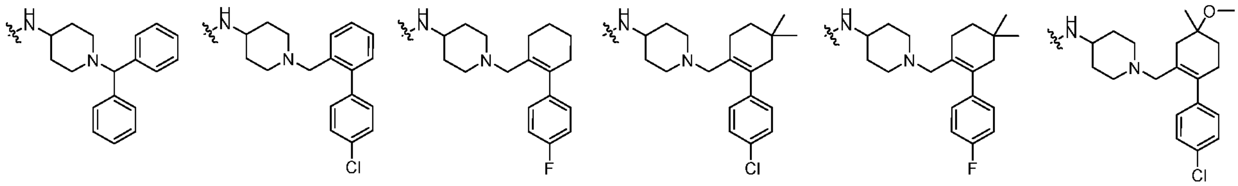

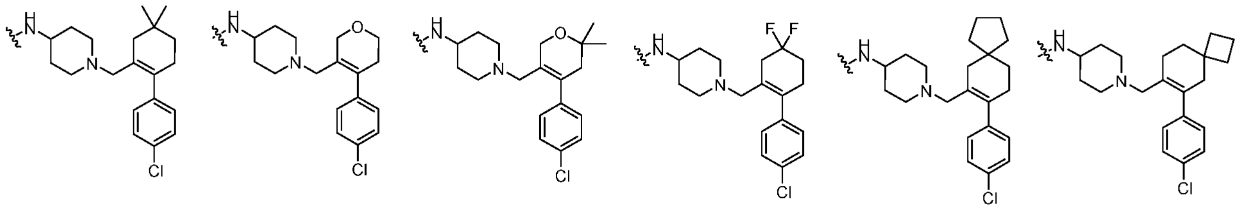

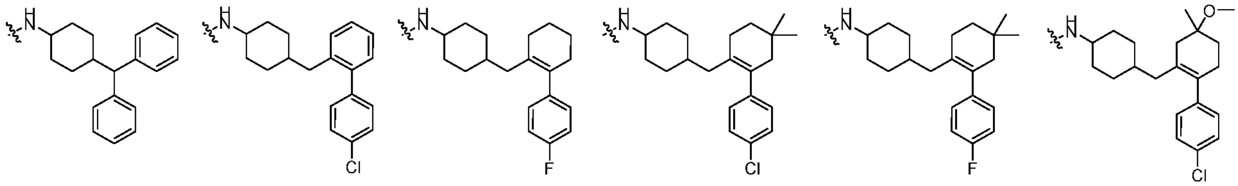

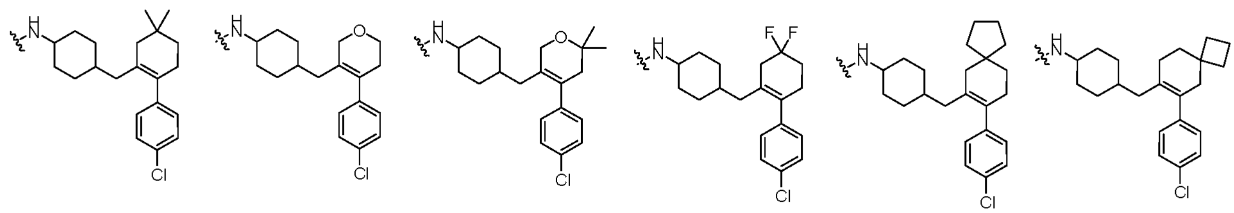

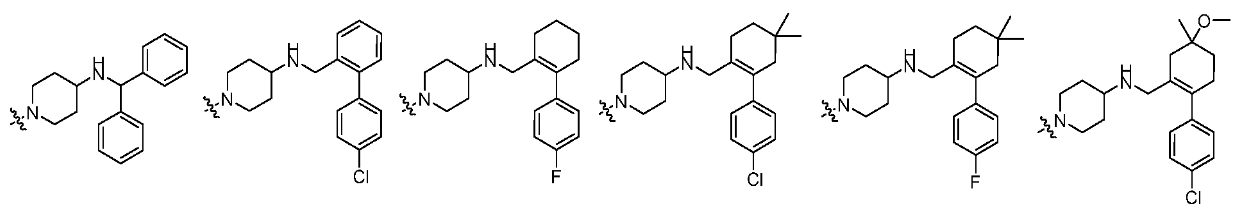

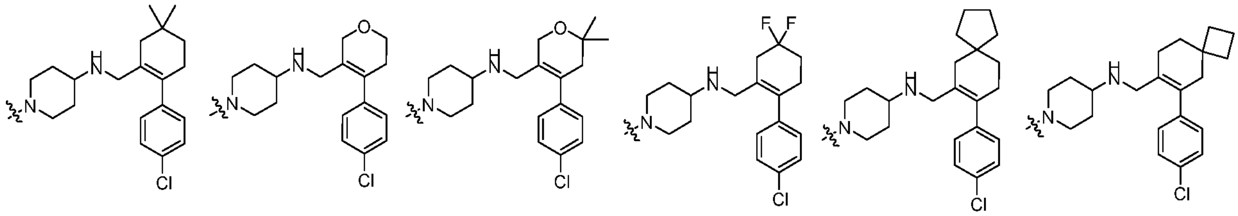

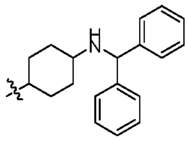 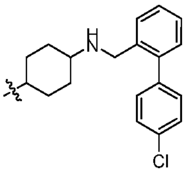 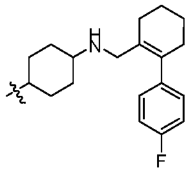 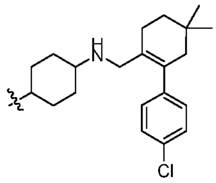 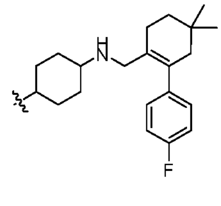 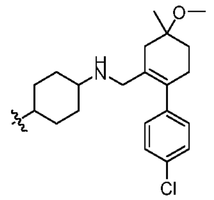

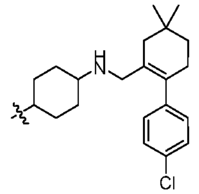 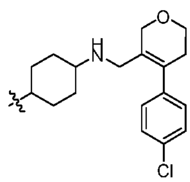 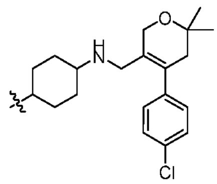 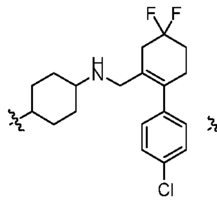 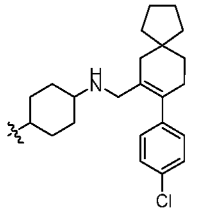 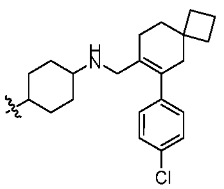

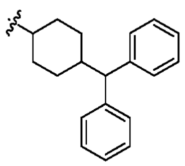 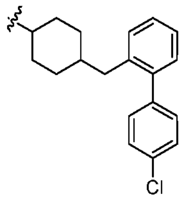 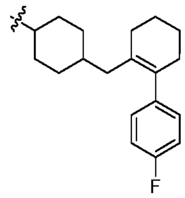 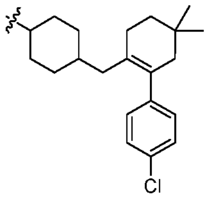 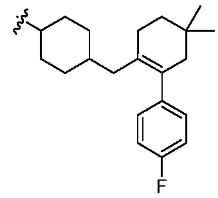 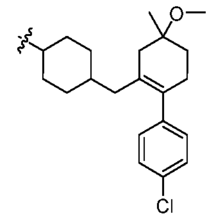

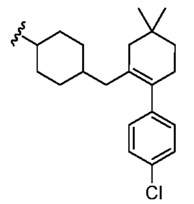 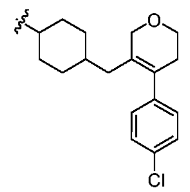 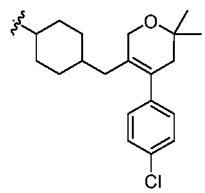 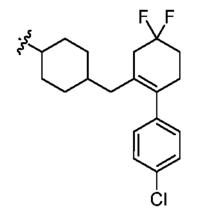 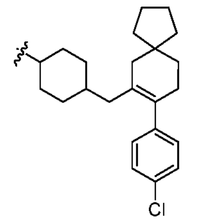 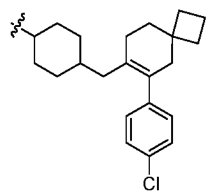

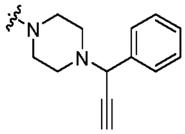 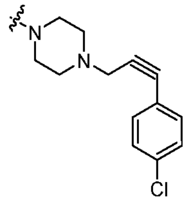 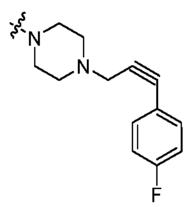 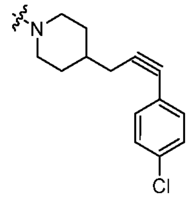 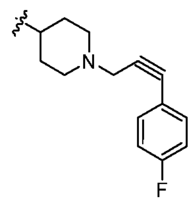 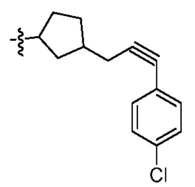

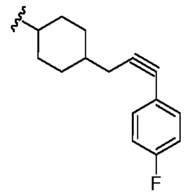 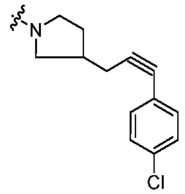 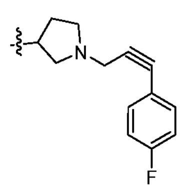 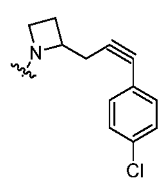 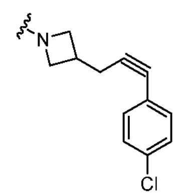 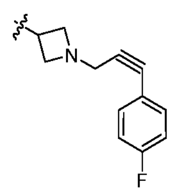

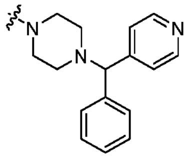 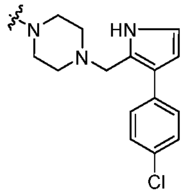 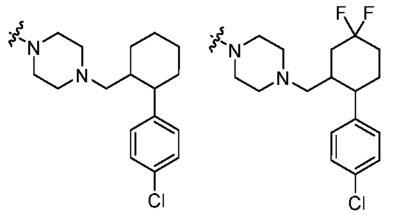 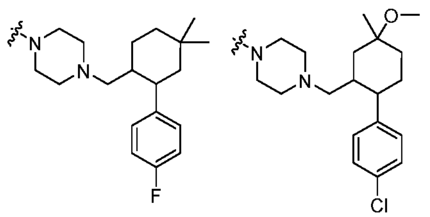

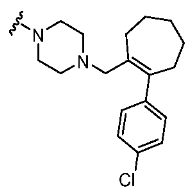 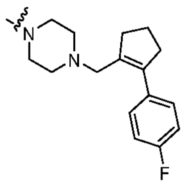 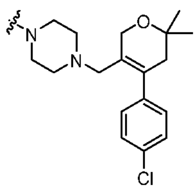 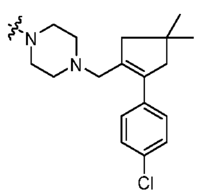 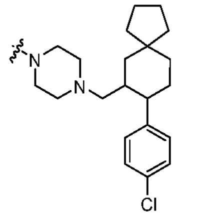 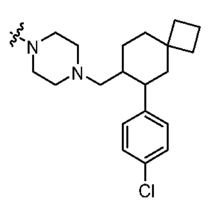

or

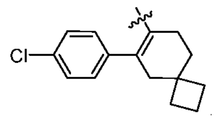

In some embodiments, the compound of Formula (I) of the present disclosure is also of Formula (II) or Formula (III):

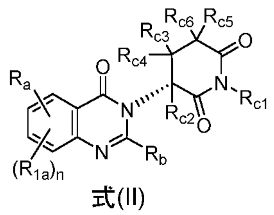
式(II)

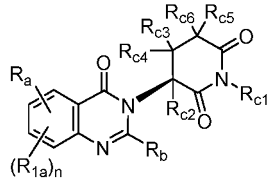
式(III)

wherein groups $R_{c1}$, $R_{c2}$, $R_{c3}$, $R_{c4}$, $R_{c5}$, $R_{c6}$, $R_a$, $(R_{1a})_n$ and $R_b$ are as defined in the compound of Formula (I) above and the embodiments thereof.

In some embodiments, the compound of Formula (I) of the present disclosure is also of Formula (Ia), Formula (Ib), Formula (Ic), Formula (Id), Formula (IIa), Formula (IIb), Formula (IIc), Formula (IId), Formula (IIIa), Formula (IIIb), Formula (IIIc), or Formula (IIId):

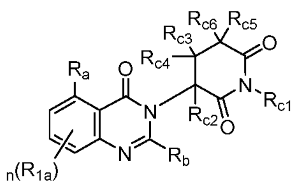
Formula (Ia)

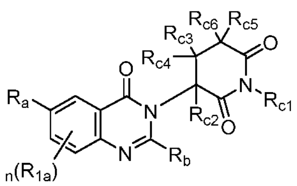
Formula (Ib)

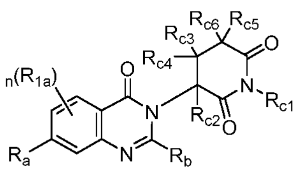
Formula (Ic)

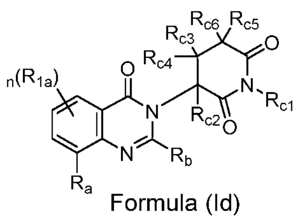
Formula (Id)

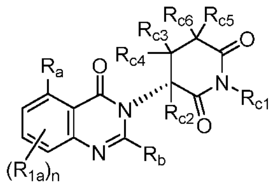
Formula (IIa)

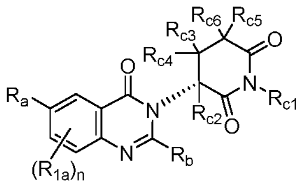
Formula (IIb)

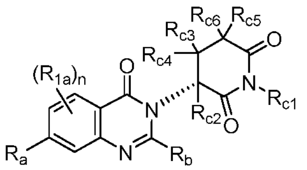
Formula (IIc)

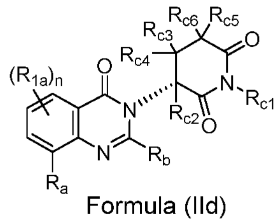
Formula (IId)

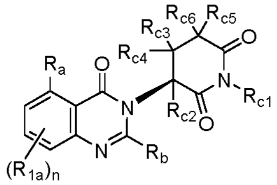
Formula (IIIa)

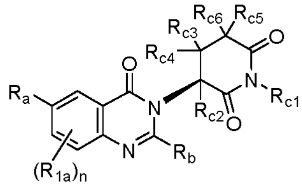
Formula (IIIb)

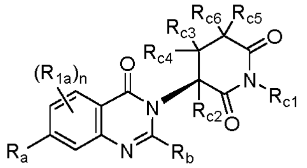
Formula (IIIc)

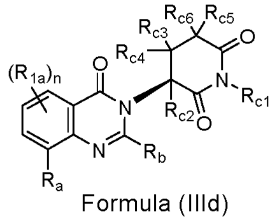
Formula (IIId)

wherein groups $R_{c1}$, $R_{c2}$, $R_{c3}$, $R_{c4}$, $R_{c5}$, $R_{c6}$, $R_a$, $(R_{1a})_n$ and $R_b$ are as defined in the compound of Formula (I) above and the

embodiments thereof.

Preferably the compounds of the present invention and their salts (especially pharmaceutically acceptable salts, such as hydrochloride, etc.), enantiomers, diastereomers, solvates, or polymorphs thereof in Table 1 below are provided.

Table 1. The compounds of the present invention

| Compou nd No. | Structure of the compounds | The compounds' name in English | Chinese translation of the compounds' name |
|---|---|---|---|
| GT-01818 | 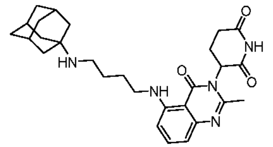 | 3-(5-((4-(((3s,5s,7s)-adamantan-1-yl)amino)butyl)amino)-2-methyl-4-oxoquinazolin-3(4H)-yl)piperidine-2,6-dione | 3-(5-((4-(((3s,5s,7s)-adamantan-1-yl)amino)butyl)amino)-2-methyl-4-oxoquinazolin-3(4H)-yl)piperidine-2,6-dione |
| GT-02280 | 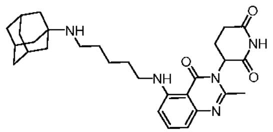 | 3-(5-((5-(((1s,3s)-adamantan-1-yl)amino)pentyl)amino)-2-methyl-4-oxoquinazolin-3(4H)-yl)piperidine-2,6-dione | 3-(5-((5-(((1s,3s)-adamantan-1-yl)amino)pentyl)amino)-2-methyl-4-oxoquinazolin-3(4H)-yl)piperidine-2,6-dione |
| GT-02235 | 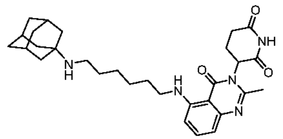 | 3-(5-((6-(((3s,5s,7s)-adamantan-1-yl)amino)hexyl)amino)-2-methyl-4-oxoquinazolin-3(4H)-yl)piperidine-2,6-dione | 3-(5-((6-(((3s,5s,7s)-adamantan-1-yl)amino)hexyl)amino)-2-methyl-4-oxoquinazolin-3(4H)-yl)piperidine-2,6-dione |
| GT-02237 | 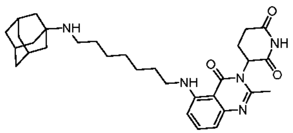 | 3-(5-((7-(((1s,3s)-adamantan-1-yl)amino)heptyl)amino)-2-methyl-4-oxoquinazolin-3(4H)-yl)piperidine-2,6-dione | 3-(5-((7-(((1s,3s)-adamantan-1-yl)amino)heptyl)amino)-2-methyl-4-oxoquinazolin-3(4H)-yl)piperidine-2,6-dione |
| GT-02234 | 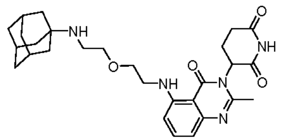 | 3-(5-((2-(2-(((1s,3s)-adamantan-1-yl)amino)ethoxy)ethyl) amino) -2-methyl-4-oxoquinazolin-3(4H)-yl)piperidine-2,6-dione | 3-(5-((2-(2-(((1s,3s)-adamantan-1-yl)amino)ethoxy)ethyl) amino) -2-methyl-4-oxoquinazolin-3(4H)-yl)piperidine-2,6-dione |
| GT-02279 | 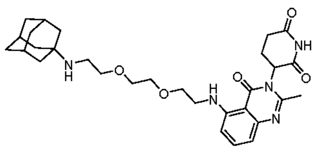 | 3-(5-((2-(2-(2-(((3s,5s,7s)-adamantan-1-yl) amino) ethoxy)ethoxy)ethyl)a mino)-2-methyl-4-oxoquinazolin-3 (4H)-yl)piperidine-2,6-dione | 3-(5-((2-(2-(2-(((3s,5s,7s)-adamantan-1-yl) amino) ethoxy)ethoxy)ethyl)a mino)-2-methyl-4-oxoquinazolin-3 (4H)-yl)piperidine-2,6-dione |

(continued)

| Compound No. | Structure of the compounds | The compounds' name in English | Chinese translation of the compounds' name |
|---|---|---|---|
| GT-02325 | 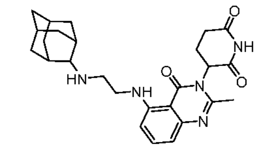 | 3-(5-((2-(((1r,3r,5r,7r)-adamantan-2-yl)amino)ethyl)amino)-2-methyl-4-oxoquinazolin-3(4H)-yl)piperidine-2,6-dione | 3-(5-((2-(((1r,3r,5r,7r)-adamantan-2-yl)amino)ethyl)amino)-2-methyl-4-oxoquinazolin-3(4H)-yl)piperidine-2,6-dione |
| GT-02326 | 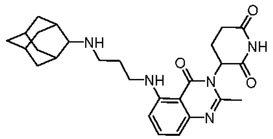 | 3-(5-((3-(((1r,3r,5r,7r)-adamantan-2-yl)amino)propyl)amino)-2-methyl-4-oxoquinazolin-3(4H)-yl)piperidine-2,6-dione | 3-(5-((3-(((1r,3r,5r,7r)-adamantan-2-yl)amino)propyl)amino)-2-methyl-4-oxoquinazolin-3(4H)-yl)piperidine-2,6-dione |
| GT-02392 | 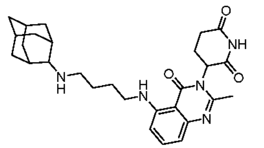 | 3-(5-((4-(((1r,3r,5r,7r)-adamantan-2-yl)amino)butyl)amino)-2-methyl-4-oxoquinazolin-3(4H)-yl)piperidine-2,6-dione | 3-(5-((4-(((1r,3r,5r,7r)-adamantan-2-yl)amino)butyl)amino)-2-methyl-4-oxoquinazolin-3(4H)-yl)piperidine-2,6-dione |
| GT-02393 | 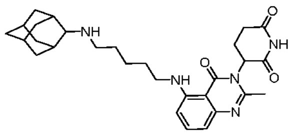 | 3-(5-((5-(((1r,3r,5r,7r)-adamantan-2-yl)amino)pentyl)amino)-2-methyl-4-oxoquinazolin-3(4H)-yl)piperidine-2,6-dione | 3-(5-((5-(((1r,3r,5r,7r)-adamantan-2-yl)amino)pentyl)amino)-2-methyl-4-oxoquinazolin-3(4H)-yl)piperidine-2,6-dione |
| GT-02496 | 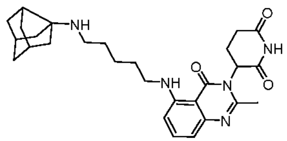 | 3-(5-((5-(((3as,6as)-hexahydro-2,5-methanopentalen-3a(1H)-yl)amino)pentyl)amino)-2-methyl-4-oxoquinazolin-3(4H)-yl)piperidine-2,6-dione | 3-(5-((5-(((3as,6as)-hexahydro-2,5-methanopentalen-3a(1H)-yl)amino)pentyl)amino)-2-methyl-4-oxoquinazolin-3(4H)-yl)piperidine-2,6-dione |
| GT-02494 | 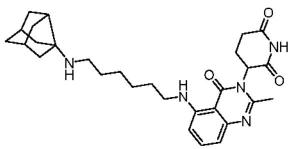 | 3-(5-((6-(((2R,3as,5S,6as)-hexahydro-2,5-methanopentalen-3a(1H)-yl)amino)hexyl)amino)-2-methyl-4-oxoquinazolin-3(4H)-yl)piperidine-2,6-dione | 3-(5-((6-(((2R,3as,5S,6as)-hexahydro-2,5-methanopentalen-3a(1H)-yl)amino)hexyl)amino)-2-methyl-4-oxoquinazolin-3(4H)-yl)piperidine-2,6-dione |

(continued)

| Compou nd No. | Structure of the compounds | The compounds' name in English | Chinese translation of the compounds' name |
|---|---|---|---|
| GT-02495 | 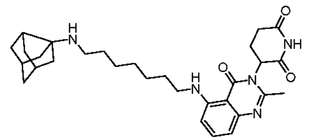 | 3-(5-((7-(((3as,6as)-hexahydro-2,5-methanopentalen-3a(1H)-yl)amino)heptyl)amino)-2-methyl-4-oxoquinazolin-3(4H)-yl)piperidine-2,6-dione | 3-(5-((7-(((3as,6as)-hexahydro-2,5-methanopentalen-3a(1H)-yl)amino)heptyl)amino)-2-methyl-4-oxoquinazolin-3(4H)-yl)piperidine-2,6-dione |
| GT-02389 | 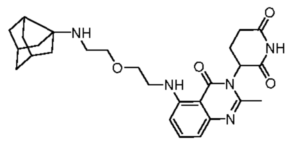 | 3-(5-((2-(2-(((3as,6as)-hexahydro-2,5-methanopentalen-3a(1H)-yl)amino)ethoxy)ethyl)amino) -2-methyl-4-oxoquinazolin-3(4H)-yl)piperidine-2,6-dione | 3-(5-((2-(2-(((3as,6as)-hexahydro-2,5-methanopentalen-3a(1H)-yl)amino)ethoxy)ethyl)amino) -2-methyl-4-oxoquinazolin-3(4H)-yl)piperidine-2,6-dione |
| GT-02497 | 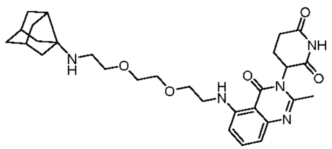 | 3-(5-((2-(2-(2-(((2R,3 as,5S,6as)-hexahydro-2,5 -methanopentalen-3a(1H)-yl)amino)ethoxy)ethoxy)ethyl) amino)-2-methyl-4-oxoquinazolin-3(4H)-yl)piperidine-2,6-dione | 3-(5-((2-(2-(2-(((2R,3 as,5S,6as)-hexahydro-2,5-methanopentalen-3a(1H)-yl)amino)ethoxy)ethoxy)ethyl) amino)-2-methyl-4-oxoquinazolin-3 (4H)-yl)piperidine-2,6-dione |
| GT-02500 | 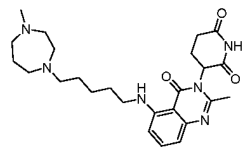 | 3-(2-methyl-5-((5-(4-methyl-1,4-diazepan-1-yl)pentyl) amino)-4-oxoquinazolin-3(4H)-yl)piperidine-2,6-dione | 3 -(2-methyl-5 -((5 -(4-methyl-1,4-diazepan-1-yl)pentyl) amino)-4-oxoquinazolin-3 (4H)-yl)piperidine-2,6-dione |
| GT-02502 | 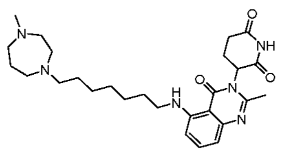 | 3-(2-methyl-5-((7-(4-methyl-1,4-diazepan-1-yl)heptyl) amino)-4-oxoquinazolin-3(4H)-yl)piperidine-2,6-dione | 3-(2-methyl-5-((7-(4-methyl-1,4-diazepan-1-yl)heptyl) amino)-4-oxoquinazolin-3 (4H)-yl)piperidine-2,6-dione |
| GT-02493 | 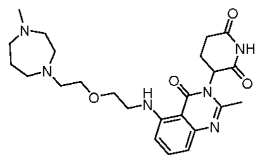 | 3-(2-methyl-5-((2-(2-(4-methyl-1,4-diazepan-1-yl)ethoxy) ethyl)amino)-4-oxoquinazolin-3(4H)-yl)piperidine-2,6-dione | 3-(2-methyl-5-((2-(2-(4-methyl-1,4-diazepan-1-yl)ethoxy) ethyl)amino)-4-oxoquinazolin-3 (4H)-yl)piperidine-2,6-dione |

(continued)

| Compou nd No. | Structure of the compounds | The compounds' name in English | Chinese translation of the compounds' name |
|---|---|---|---|
| GT-02607 | 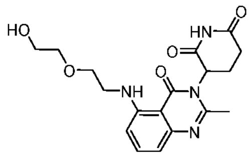 | 3-(5-((2-(2-hydroxyethoxy)ethyl)amino)-2-methyl-4-oxoquinazolin-3(4H)-yl)piperidine-2,6-dione | 3-(5-((2-(2-hydroxyethoxy)ethyl)amino)-2-methyl-4-oxoquinazolin-3(4H)-yl)piperidine-2,6-dione |
| GT-02608 | 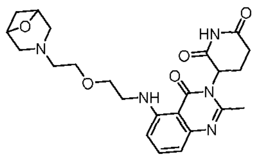 | 3-(5-((2-(2-(6-oxa-3-azabicyclo[3.1.1]heptan-3-yl)ethoxy) ethyl)amino)-2-methyl-4-oxoquinazolin-3(4H)-yl)piperidine-2,6-dione | 3-(5-((2-(2-(6-oxa-3-azabicyclo[3.1.1]heptan-3-yl)ethoxy) ethyl)amino)-2-methyl-4-oxoquinazolin-3(4H)-yl)piperidine-2,6-dione |
| GT-02617 | 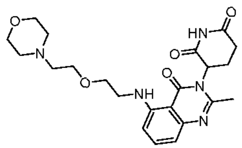 | 3-(2-methyl-5-((2-(2-morpholinoethoxy)ethyl)amin o)-4-oxoquinazolin-3(4H)-yl)piperidine-2,6-dione | 3-(2-methyl-5-((2-(2-morpholinoethoxy)ethyl)amin o)-4-oxoquinazolin-3(4H)-yl)piperidine-2,6-dione |
| GT-02618 | 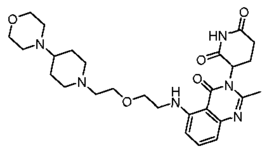 | 3-(2-methyl-5-((2-(2-(4-morpholinopiperidin-1-yl)ethoxy) ethyl)amino)-4-oxoquinazolin-3(4H)-yl)piperidine-2,6-dione | 3-(2-methyl-5-((2-(2-(4-morpholinopiperidin-1-yl)ethoxy) ethyl)amino)-4-oxoquinazolin-3(4H)-yl)piperidine-2,6-dione |
| GT-02619 | 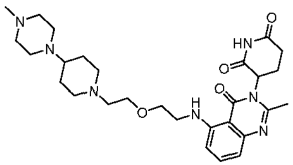 | 3-(2-methyl-5-(2-(2-(4-(4-methylpiperazin-1-yl)piperidin-1-yl)ethoxy)ethyl)amino)-4-oxoquinazolin-3(4H)-yl)piperidine-2,6-dione | 3-(2-methyl-5-((2-(2-(4-(4-methylpiperazin-1-yl)piperidin-1-yl)ethoxy)ethyl)amino)-4-oxoquinazolin-3(4H)-yl)piperidine-2,6-dione |
| GT-02620 | 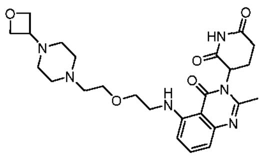 | 3-(2-methyl-5-((2-(2-(4-(oxetan-3-yl)piperazin-1-yl) ethoxy)ethyl)amino)-4-oxoquinazolin-3(4H)-yl)piperidine-2,6-dione | 3-(2-methyl-5-((2-(2-(4-(oxetan-3-yl)piperazin-1-yl) ethoxy)ethyl)amino)-4-oxoquinazolin-3(4H)-yl)piperi-dine-2,6-dione |

(continued)

| Compound No. | Structure of the compounds | The compounds' name in English | Chinese translation of the compounds' name |
|---|---|---|---|
| GT-02598 | 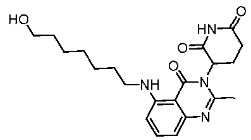 | 3-(5-((7-hydroxyheptyl)amino)-2-methyl-4-oxoquinazolin-3(4H)-yl)piperidine-2,6-dione | 3-(5-((7-hydroxyheptyl)amino)-2-methyl-4-oxoquinazolin-3(4H)-yl)piperidine-2,6-dione |
| GT-02621 | 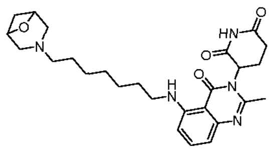 | 3-(5-((7-(6-oxa-3-azabicyclo[3.1.1]heptan-3-yl)heptyl)amino)-2-methyl-4-oxoquinazolin-3(4H)-yl)piperidine-2,6-dione | 3-(5-((7-(6-oxa-3-azabicyclo[3.1.1]heptan-3-yl)heptyl)amino)-2-methyl-4-oxoquinazolin-3(4H)-yl)piperidine-2,6-dione |
| GT-02622 | 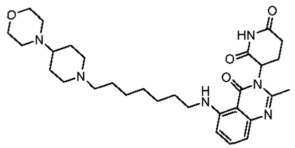 | 3-(2-methyl-5-((7-(4-morpholinopiperidin-1-yl)heptyl)amino)-4-oxoquinazolin-3(4H)-yl)piperidine-2,6-dione | 3-(2-methyl-5-((7-(4-morpholinopiperidin-1-yl)heptyl)amino)-4-oxoquinazolin-3(4H)-yl)piperidine-2,6-dione |
| GT-02623 | 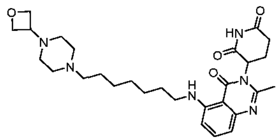 | 3-(2-methyl-5-((7-(4-(oxetan-3-yl)piperazin-1-yl)heptyl)amino)-4-oxoquinazolin-3(4H)-yl)piperidine-2,6-dione | 3-(2-methyl-5-((7-(4-(oxetan-3-yl)piperazin-1-yl)heptyl)amino)-4-oxoquinazolin-3(4H)-yl)piperidine-2,6-dione |
| GT-02767 | 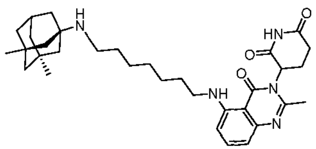 | 3-(5-((7-(((1SR,3RS,5SR,7r)-3,5-dimethyladamantan-1-yl)amino)heptyl)amino)-2-methyl-4-oxoquinazolin-3(4H)-yl)piperidine-2,6-dione | 3-(5-((7-(((1SR,3RS,5SR,7r)-3,5-dimethyladamantan-1-yl)amino)heptyl)amino)-2-methyl-4-oxoquinazolin-3(4H)-yl)piperidine-2,6-dione |
| GT-02768 | 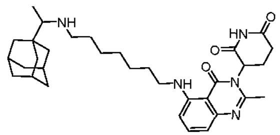 | 3-(5-((7-((1-((3r,5r,7r)-adamantan-1-yl)ethyl)amino)heptyl)amino)-2-methyl-4-oxoquinazolin-3(4H)-yl)piperidine-2,6-dione | 3-(S-((7-((1-((3r,5r,7r)-adamantan-1-yl)ethyl)amino)heptyl)amino)-2-methyl-4-oxoquinazolin-3(4H)-yl)piperidine-2,6-dione |

(continued)

| Compou nd No. | Structure of the compounds | The compounds' name in English | Chinese translation of the compounds' name |
|---|---|---|---|
| GT-02772 | 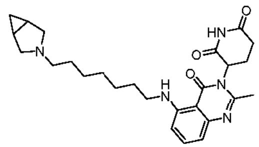 | 3-(5-((7-(3-azabicyclo[3.1.0]hexan-3-yl)heptyl)amino)-2-methyl-4-oxoquinazolin-3 (4H)-yl)piperidine-2,6-dione | 3-(5-((7-(3-azabicyclo[3.1.0]hexan-3-yl)heptyl)amino)-2-methyl-4-oxoquinazolin-3 (4H)-yl)piperidine-2,6-dione |
| GT-02773 | 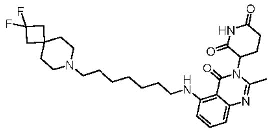 | 3-(5-((7-(2,2-difluoro-7-azaspiro[3.5]nonan-7-yl)heptyl) amino)-2-methyl-4-oxoquinazolin-3 (4H)-yl)piperidine-2,6-dione | 3-(5-((7-(2,2-difluoro-7-azaspiro[3.5]nonan-7-yl)heptyl) amino)-2-methyl-4-oxoquinazolin-3 (4H)-yl)piperidine-2,6-dione |
| GT-02774 | 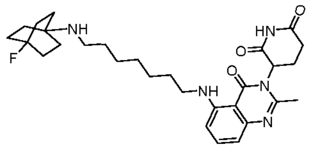 | 3-(5-((7-((4-fluorobicyclo[2.2.2]octan-1-yl)amino)heptyl) amino)-2-methyl-4-oxoquinazolin-3 (4H)-yl)piperidine-2,6-dione | 3-(5-((7-((4-fluorobicyclo[2.2.2]octan-1-yl)amino)heptyl) amino)-2-methyl-4-oxoquinazolin-3(4H)-yl)piperi-dine-2,6-dione |
| | 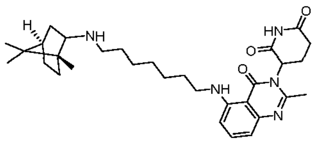 | 3-(2-methyl-4-oxo-5-((7-(((1R,2S,4R)-1,7,7-trimethylbicyclo[2.2.1]heptan-2-yl)amino)heptyl)amino)quinaz olin-3 (4H)-yl)piperidine-2,6-dione | 3-(2-methyl-4-oxo-5-((7-(((1R,2S,4R)-1,7,7-trimethylbicyclo[2.2.1]heptan-2-yl)amino)heptyl)amino)quinaz olin-3(4H)-yl)piperidine-2,6-dione |
| | 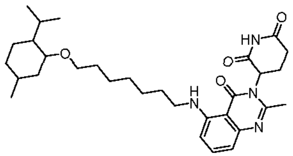 | 3-(5-((7-((2-isopropyl-5-methylcyclohexyl)oxy)heptyl) amino)-2-methyl-4-oxoquinazolin-3 (4H)-yl)piperidine-2,6-dione | 3-(5 -((7-((2-isopropyl-5-methylcyclohexyl)oxy)heptyl) amino)-2-methyl-4-oxoquinazolin-3 (4H)-yl)piperi-dine-2,6-dione |
| | 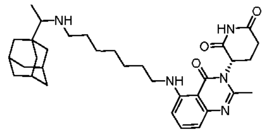 | (3S)-3-(5-((7-((1-((3r,5r,7r)-adamantan-1-yl)ethyl)amino) heptyl)amino)-2-methyl-4-oxoquinazolin-3 (4H)-yl)piperidine-2,6-dione | (3S)-3-(5-((7-((1-((3r,5r,7r)-adamantan-1-yl)ethyl)amino) heptyl)amino)-2-methyl-4-oxoquinazolin-3(4H)-yl)piperi-dine-2,6-dione |

(continued)

| Compound No. | Structure of the compounds | The compounds' name in English | Chinese translation of the compounds' name |
|---|---|---|---|
| | 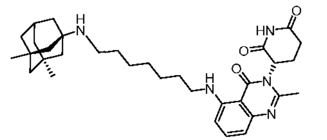 | (S)-3-(5-((7-(((1SR,3RS,5SR,7r)-3,5-dimethyladamantan-1-yl)amino)heptyl)amino)-2-methyl-4-oxoquinazolin-3 (4H)-yl)piperidine-2,6-dione | (S)-3-(5-((7-(((1SR,3RS,5SR,7r)-3,5-dimethyladamantan-1-yl)amino)heptyl)amino)-2-methyl-4-oxoquinazolin-3(4H)-yl)piperidine-2,6-dione |
| | 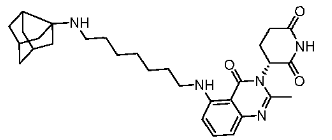 | (3R)-3-(5-((7-(((3as,6as)-hexahydro-2,5-methanopentalen-3a(1H)-yl)amino)heptyl)amino)-2-methyl-4-oxoquinazolin-3(4H)-yl)piperidine-2,6-dione | (3R)-3-(5-((7-(((3as,6as)-hexahydro-2,5-methanopentalen-3a(1H)-yl)amino)heptyl)amino)-2-methyl-4-oxoquinazolin-3(4H)-yl)piperidine-2,6-dione |
| | 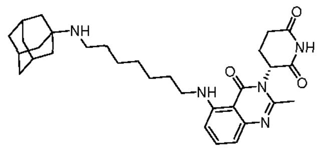 | (3R)-3-(5-((7-(((1s,3s)-adamantan-1-yl)amino)heptyl) amino)-2-methyl-4-oxoquinazolin-3 (4H)-yl)piperidine-2,6-dione | (3R)-3-(5-((7-(((1s,3s)-adamantan-1-yl)amino)heptyl) amino)-2-methyl-4-oxoquinazolin-3 (4H)-yl)piperidine-2,6-dione |
| | 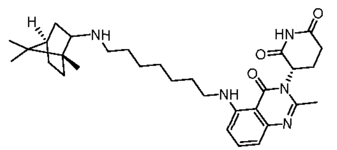 | (S)-3-(2-methyl-4-oxo-5 -((7-(((1R,2S,4R)-1,7,7-trimethylbicyclo[2.2.1]heptan-2-yl)amino)heptyl)amino)quinaz olin-3 (4H)-yl)piperidine-2,6-dione | (S)-3-(2-methyl-4-oxo-5-((7-(((1R,2S,4R)-1,7,7-trimethylbicyclo[2.2.1]heptan-2-yl)amino)heptyl)amino)quinaz olin-3(4H)-yl)piperidine-2,6-dione |
| | 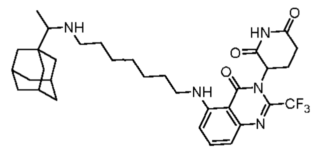 | 3-(5-((7-((1-((3r,5r,7r)-adamantan-1-yl)ethyl)amino)heptyl)amino)-4-oxo-2-(trifluoromethyl)quinazolin-3(4H)-yl) piperidine-2,6-dione | 3-(S-((7-((1-((3r,5r,7r)-adamantan-1-yl)ethyl)amino)heptyl)amino)-4-oxo-2-(trifluoromethyl)quinazolin-3(4H)-yl) piperidine-2,6-dione |
| | 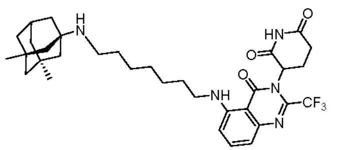 | 3-(S-((7-(((1SR,3RS,5SR,7r)-3,5-dimethyladamantan-1-yl)amino)heptyl)amino)-4-oxo-2-(trifluoromethyl)quinazolin-3(4H)-yl)piperidine-2,6-dione | 3-(5-((7-(1SR,3RS,5SR,7r)-3,5-dimethyladamantan-1-yl) amino)heptyl)amino)-4-oxo-2-(trifluoromethyl)quinazolin-3(4H)-yl)piperidine-2,6-dione |

(continued)

| Compou nd No. | Structure of the compounds | The compounds' name in English | Chinese translation of the compounds' name |
|---|---|---|---|
| | 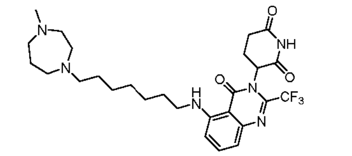 | 3-(5-((7-(4-methyl-1,4-diazepan-1-yl)heptyl)amino)-4-oxo-2-(trifluoromethyl)quinazolin-3(4H)-yl)piperidine-2,6-dione | 3-(5-((7-(4-methyl-1,4-diazepan-1-yl)heptyl)amino)-4-oxo-2-(trifluoromethyl)quinazolin-3(4H)-yl)piperidine-2,6-dione |
| | 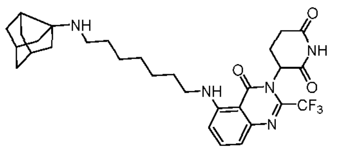 | 3-(5-((7-(((3as,6as)-hexahydro-2,5-methanopentalen-3a(1H)-yl)amino)heptyl)amino)-4-oxo-2-(trifluoromethyl)quinazolin-3(4H)-yl)piperidine-2,6-dione | 3-(5-((7-(((3as,6as)-hexahydro-2,5-methanopentalen-3a(1H)-yl)amino)heptyl)amino)-4-oxo-2-(trifluoromethyl)quinazolin-3 (4H)-yl)piperidine-2,6-dione |
| | 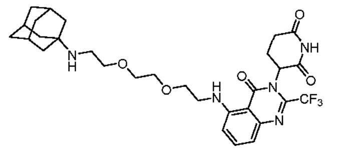 | 3-(5-((2-(2-(2-(((3s,5s,7s)-adamantan-1-yl)amino) ethoxy)ethoxy)ethyl) amino)-4-oxo-2-(trifluoromethyl)quinazolin-3(4H)-yl)piperidine-2,6-dione | 3-(5-((2-(2-(2-(((3s,5s,7s)-adamantan-1-yl)amino) ethoxy)ethoxy)ethyl) amino)-4-oxo-2-(trifluoromethyl)quinazolin-3(4H)-yl)piperidine-2,6-dione |
| | 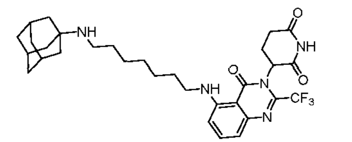 | 3-(5-((7-(((1s,3s)-adamantan-1-yl)amino)heptyl)amino)-4-oxo-2-(trifluoromethyl)quinazolin-3(4H)-yl)piperidine-2,6-dione | 3-(5-((7-(((1s,3s)-adamantan-1-yl)amino)heptyl)amino)-4-oxo-2-(trifluoromethyl)quinazolin-3(4H)-yl)piperidine-2,6-dione |
| | 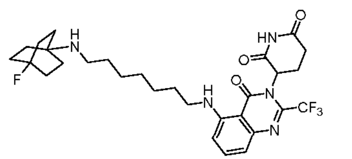 | 3-(5-((7-((4-fluorobicyclo [2.2.2]octan-1-yl)amino)heptyl) amino)-4-oxo-2-(trifluoromethyl)quinazolin-3(4H)-yl)piperidine-2,6-dione | 3-(5-((7-((4-fluorobicyclo [2.2.2]octan-1-yl)amino)heptyl) amino)-4-oxo-2-(trifluoromethyl)quinazolin-3(4H)-yl)piperidine-2,6-dione |
| | 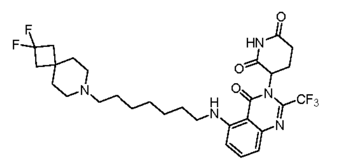 | 3-(5-((7-(2,2-difluoro-7-azaspiro[3.5]nonan-7-yl)heptyl) amino)-4-oxo-2-(trifluoromethyl)quinazolin-3(4H)-yl)piperidine-2,6-dione | 3-(5-((7-(2,2-difluoro-7-azaspiro[3.5]nonan-7-yl)heptyl) amino)-4-oxo-2-(trifluoromethyl)quinazolin-3(4H)-yl)piperidine-2,6-dione |

(continued)

| Compound No. | Structure of the compounds | The compounds' name in English | Chinese translation of the compounds' name |
|---|---|---|---|
| | 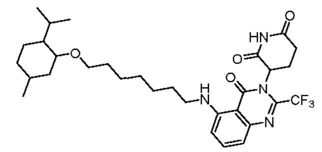 | 3-(5-((7-((2-isopropyl-5-methylcyclohexyl)oxy)heptyl) amino)-4-oxo-2-(trifluoromethyl)quinazolin-3(4H)-yl)piperidine-2,6-dione | 3-(5-((7-((2-isopropyl-5-methylcyclohexyl)oxy)heptyl) amino)-4-oxo-2-(trifluoromethyl)quinazolin-3(4H)-yl)piperidine-2,6-dione |
| | 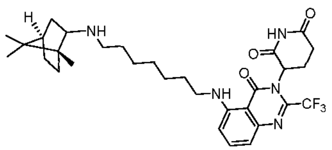 | 3-(4-oxo-2-(trifluoromethyl)-5-((7-(((1R,2S,4R)-1,7,7-trimethylbicyclo[2.2.1]heptan-2-yl)amino)heptyl)amino)quinaz olin-3(4H)-yl)piperidine-2,6-dione | 3-(4-oxo-2-(trifluoromethyl)-5-((7-(((1R,2S,4R)-1,7,7-trimethylbicyclo[2.2. 1]heptan-2-yl)amino)heptyl)amino) quinaz olin-3(4H)-yl)piperidine-2,6-dione |
| | 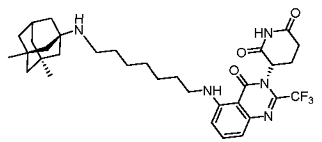 | (S)-3-(5-((7-(((1SR,3RS,5SR,7r)-3,S-dimethyladamantan-1-yl)amino)heptyl)amino)-4-oxo-2-(trifluoromethyl) quinazolin-3(4H)-yl)piperidine-2,6-dione | (S)-3-(5-((7-(((1SR,3RS,5SR,7r)-3,5-dimethyladamantan-1-yl)amino)heptyl)amino)-4-oxo-2-(trifluoromethyl) quinazolin-3(4H)-yl)piperidine-2,6-dione |
| | 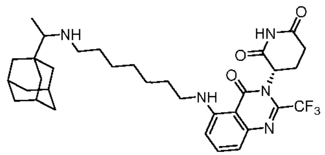 | (3S)-3-(S-((7-((1-((3r,5r,7r)-adamantan-1-yl)ethyl)amino) heptyl)amino)-4-oxo-2-(trifluoromethyl)quinazolin-3(4H)-yl)piperidine-2,6-dione | (3S)-3-(5-((7-((1-((3r,5r,7r)-adamantan-1-yl)ethyl)amino) heptyl)amino)-4-oxo-2-(trifluoromethyl)quinazolin-3(4H)-yl)piperidine-2,6-dione |
| | 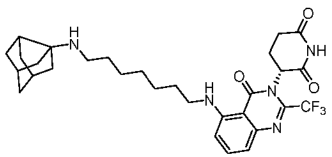 | (3R)-3-(5-((7-(((3as,6as)-hexahydro-2,5-methanopentalen-3a(1H)-yl)amino)heptyl)amino)-4-oxo-2-(trifluoromethyl)quinazolin-3(4H)-yl)piperidine-2,6-dione | (3R)-3-(5-((7-(((3as,6as)-hexahydro-2,5-methanopentalen-3a(1H)-yl)amino)heptyl)amino)-4-oxo-2-(trifluoromethyl)quinazolin-3(4H)-yl)piperidine-2,6-dione |
| | 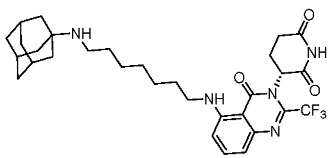 | (3R)-3-(5-((7-(((1s,3s)-adamantan-1-yl)amino)heptyl) amino)-4-oxo-2-(trifluoromethyl)quinazolin-3(4H)-yl)piperidine-2,6-dione | (3R)-3-(5-((7-(((1s,3s)-adamantan-1-yl)amino)heptyl) amino)-4-oxo-2-(trifluoromethyl)quinazolin-3(4H)-yl)piperidine-2,6-dione |

(continued)

| Compound No. | Structure of the compounds | The compounds' name in English | Chinese translation of the compounds' name |
|---|---|---|---|
| | 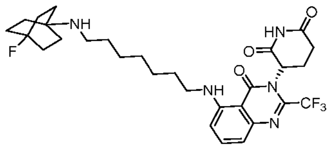 | (S)-3-(5-((7-((4-fluorobicyclo[2.2.2]octan-1-yl)amino)heptyl)amino)-4-oxo-2-(trifluoromethyl)quinazolin-3(4H)-yl) piperidine-2,6-dione | (S)-3-(5-((7-((4-fluorobicyclo [2.2.2]octan-1-yl)amino) heptyl)amino)-4-oxo-2-(trifluoromethyl)quinazolin-3(4H)-yl)piperidine-2,6-dione |
| | 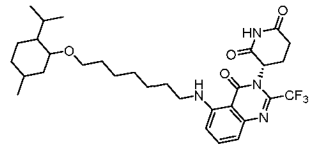 | (3S)-3-(5-((7-((2-isopropyl-5-methylcyclohexyl)oxy)heptyl) amino)-4-oxo-2-(trifluoromethyl)quinazolin-3(4H)-yl) piperidine-2,6-dione | (3S)-3-(5-((7-((2-isopropyl-5-methylcyclohexyl)oxy)heptyl) amino)-4-oxo-2-(trifluoromethyl)quinazolin-3(4H)-yl) piperidine-2,6-dione |
| | 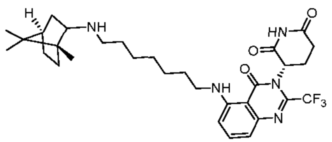 | (S)-3-(4-oxo-2-(trifluoromethyl)-5 -((7-(((1R,2S,4R)-1,7,7-trimethylbicyclo[2.2.1]heptan-2-yl)amino)heptyl)amino)quinaz olin-3 (4H)-yl)piperidine-2,6-dione | (S)-3-(4-oxo-2-(trifluoromethyl)-5 -((7-(((1R,2S,4R)-1,7,7-trimethylbicyclo[2.2.1]heptan-2-yl)amino)heptyl)amino) quinaz olin-3(4H)-yl)piperidine-2,6-dione |
| | 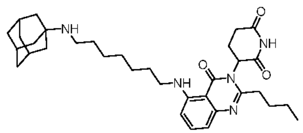 | 3-(5-((7-(((1s,3s)-adamantan-1-yl)amino)heptyl)amino)-2-butyl-4-oxoquinazolin-3(4H)-yl)piperidine-2,6-dione | 3-(5-((7-(((1s,3s)-adamantan-1-yl)amino)heptyl)amino)-2-butyl-4-oxoquinazolin-3(4H)-yl) piperidine-2,6-dione |
| | 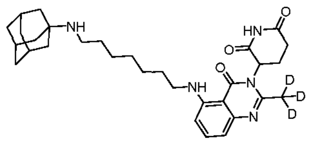 | 3-(5-((7-(((1s,3s)-adamantan-1-yl)amino)heptyl)amino)-2-(methyl-d3)-4-oxoquinazolin-3(4H)-yl)piperidine-2,6-dione | 3-(5-((7-(((1s,3s)-adamantan-1-yl)amino)heptyl)amino)-2-(methyl-d3)-4-oxoquinazolin-3(4H)-yl)piperidine-2,6-dione |
| | 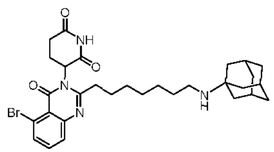 | 3-(2-(7-(((3s,5s,7s)-adamantan-1-yl)amino)heptyl)-5-bromo-4-oxoquinazolin-3 (4H)-yl)piperidine-2,6-dione | 3-(2-(7-(((3s,5s,7s)-adamantan-1-yl)amino)heptyl)-5 -bromo-4-oxoquinazolin-3(4H)-yl)piperidine-2,6-dione |

(continued)

| Compou nd No. | Structure of the compounds | The compounds' name in English | Chinese translation of the compounds' name |
|---|---|---|---|
| | 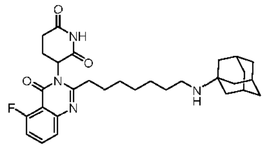 | 3-(2-(7-(((3s,5s,7s)-adamantan-1-yl)amino)heptyl)-5-fluoro-4-oxoquinazolin-3(4H)-yl)piperidine-2,6-dione | 3-(2-(7-(((3s,5s,7s)-adamantan-1-yl)amino)heptyl)-5-fluoro-4-oxoquinazolin-3(4H)-yl)piperidine-2,6-dione |
| | 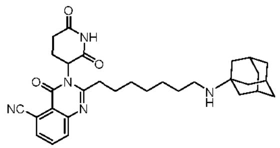 | 2-(7-(((3s,5s,7s)-adamantan-1-yl)amino)heptyl)-3-(2,6-dioxopiperidin-3-yl)-4-oxo-3,4-dihydroquinazoline-5-carbonitrile | 2-(7-(((3s,5s,7s)-adamantan-1-yl)amino)heptyl)-3-(2,6-dioxopiperidin-3-yl)-4-oxo-3,4-dihydroquinazoline-5-carbonitrile |
| | 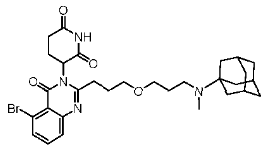 | 3-(2-(3-(3-(((3s,5s,7s)-adamantan-1-yl)(methyl)amino) propoxy)pro pyl)-5-bromo-4-oxoquinazolin-3(4H)-yl)piperidine-2,6-dione | 3-(2-(3-(3-(((3s,5s,7s)-adamantan-1-yl)(methyl)amino) propoxy)pro pyl)-5-bromo-4-oxoquinazolin-3(4H)-yl)piperidine-2,6-dione |
| GT-02601 | 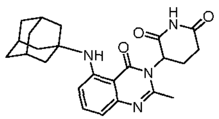 | 3 -(5 -(((1s,3s)-adamantan-1-yl)amino)-2-methyl-4-oxoquinazolin-3 (4H)-yl)piperidine-2,6-dione | 3-(5-(((1s,3s)-adamantan-1-yl)amino)-2-methyl-4-oxoquinazolin-3(4H)-yl)piperidine-2,6-dione |
| | 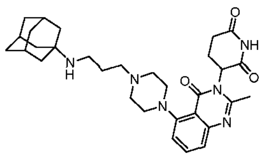 | 3-(5-(4-(3-(((3s,5s,7s)-adamantan-1-yl)amino)propyl)piperazin-1-yl)-2-methyl-4-oxoquinazolin-3(4H)-yl)piperidine-2,6-dione | 3-(5-(4-(3-(((3s,5s,7s)-adamantan-1-yl)amino)propyl)piperazin-1-yl)-2-methyl-4-oxoquinazolin-3(4H)-yl)piperidine-2,6-dione |
| | 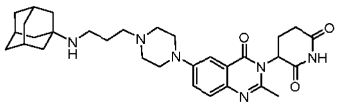 | 3-(6-(4-(3-(((3s,5s,7s)-adamantan-1-yl)amino)propyl)piperazin-1-yl)-2-methyl-4-oxoquinazolin-3(4H)-yl)piperidine-2,6-dione | 3-(6-(4-(3-(((3s,5s,7s)-adamantan-1-yl)amino)propyl)piperazin-1-yl)-2-methyl-4-oxoquinazolin-3(4H)-yl)piperidine-2,6-dione |
| | 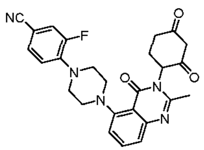 | 4-(4-(3-(2,4-dioxocyclohexyl)-2-methyl-4-oxo-3,4-dihydroquinazolin-5-yl)piperazin-1-yl)-3-fluorobenzonitrile | 4-(4-(3-(2,4-dioxocyclohexyl)-2-methyl-4-oxo-3,4-dihydroquinazolin-5-yl)piperazin-1-yl)-3-fluorobenzonitrile |

(continued)

| Compound No. | Structure of the compounds | The compounds' name in English | Chinese translation of the compounds' name |
|---|---|---|---|
| | 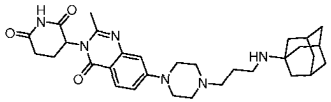 | 3-(7-(4-(3-(((3s,5s,7s)-adamantan-1-yl)amino)propyl)piperazin-1-yl)-2-methyl-4-oxoquinazolin-3(4H)-yl)piperidine-2,6-dione | 3-(7-(4-(3-(((3s,5s,7s)-adamantan-1-yl)amino)propyl)piperazin-1-yl)-2-methyl-4-oxoquinazolin-3(4H)-yl)piperidine-2,6-dione |
| | 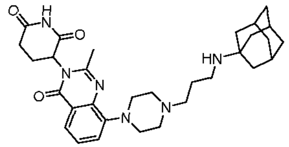 | 3-(8-(4-(3-(((3s,5s,7s)-adamantan-1-yl)amino)propyl)piperazin-1-yl)-2-methyl-4-oxoquinazolin-3(4H)-yl)piperidine-2,6-dione | 3-(8-(4-(3-(((3s,5s,7s)-adamantan-1-yl)amino)propyl)piperazin-1-yl)-2-methyl-4-oxoquinazolin-3(4H)-yl)piperidine-2,6-dione |
| | 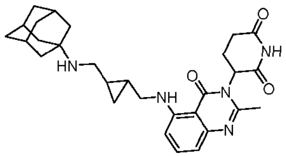 | 3-(5-(((2-((((3s,5s,7s)-adamantan-1-yl)amino)methyl)cyclopropyl) methyl)amino)-2-methyl-4-oxoquinazolin-3(4H)-yl)piperidine-2,6-dione | 3-(5-(((2-((((3s,5s,7s)-adamantan-1-yl)amino)methyl)cyclopropyl) methyl)amino)-2-methyl-4-oxoquinazolin-3(4H)-yl)piperidine-2,6-dione |
| | 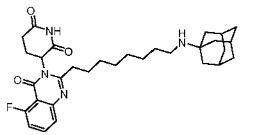 | 3-(2-(8-(((3s,5s,7s)-adamantan-1-yl)amino)octyl)-5-fluoro-4-oxoquinazolin-3(4H)-yl)piperidine-2,6-dione | 3-(2-(8-(((3s,5s,7s)-adamantan-1-yl)amino)octyl)-5-fluoro-4-oxoquinazolin-3(4H)-yl)piperidine-2,6-dione |
| | 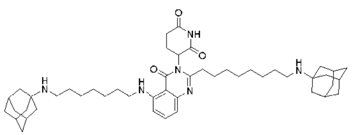 | 3-(5-((7-(((1s,3s)-adamantan-1-yl)amino)heptyl)amino)-2-(8-(((3s,5s,7s)-adamantan-1-yl)amino)octyl)-4-oxoquinazolin-3(4H)-yl)piperidine-2,6-dione | 3-(5-((7-(((1s,3s)-adamantan-1-yl)amino)heptyl)amino)-2-(8-(((3s,5s,7s)-adamantan-1-yl)amino)octyl)-4-oxoquinazolin-3(4H)-yl)piperidine-2,6-dione |
| | 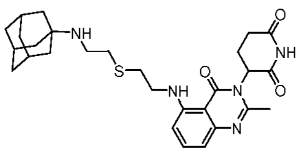 | 3-(5-((2-((2-(((1s,3s)-adamantan-1-yl)amino)ethyl)thio) ethyl)ami no)-2-methyl-4-oxoquinazolin-3(4H)-yl)piperidine-2,6-dione | 3-(5-((2-((2-(((1s,3s)-adamantan-1-yl)amino)ethyl)thio) ethyl)ami no)-2-methyl-4-oxoquinazolin-3(4H)-yl)piperidine-2,6-dione |
| | 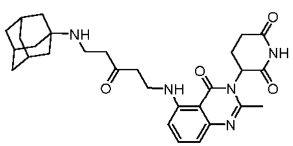 | 3-(5-((5-(((1s,3s)-adamantan-1-yl)amino)-3-oxopentyl) amino)-2-methyl-4-oxoquinazolin-3 (4H)-yl)piperidine-2,6-dione | 3-(5-((5-(((1s,3s)-adamantan-1-yl)amino)-3-oxopentyl) amino)-2-methyl-4-oxoquinazolin-3 (4H)-yl)piperidine-2,6-dione |

(continued)

| Compound No. | Structure of the compounds | The compounds' name in English | Chinese translation of the compounds' name |
|---|---|---|---|
| | 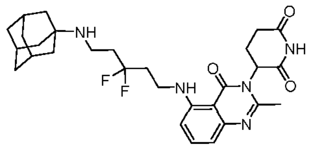 | 3-(5-((5-(((1s,3s)-adamantan-1-yl)amino)-3,3-difluoropentyl)amino)-2-methyl-4-oxoquinazolin-3 (4H)-yl)piperidine-2,6-dione | 3-(5-((5-(((1s,3s)-adamantan-1-yl)amino)-3,3-difluoropentyl)amino)-2-methyl-4-oxoquinazolin-3(4H)-yl)piperidine-2,6-dione |
| | 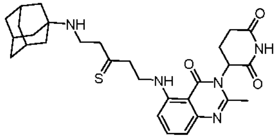 | 3-(5-((5-(((1s,3s)-adamantan-1-yl)amino)-3-thioxopentyl) amino)-2-methyl-4-oxoquinazolin-3(4H)-yl)piperidine-2,6-dione | 3-(5-((5-(((1s,3s)-adamantan-1-yl)amino)-3-thioxopentyl) amino)-2-methyl-4-oxoquinazolin-3(4H)-yl)piperidine-2,6-dione |
| | 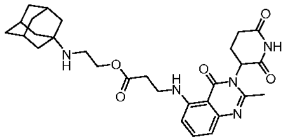 | 2-(((3s,5s,7s)-adamantan-1-yl)amino)ethyl 3-((3-(2,6-dioxopiperidin-3-yl)-2-methyl-4-oxo-3,4-dihydroquinazolin-5 -yl)amino)propanoate | 2-(((3s,5s,7s)-adamantan-1-yl)amino)ethyl 3-((3-(2,6-dioxopiperidin-3-yl)-2-methyl-4-oxo-3,4-dihydroquinazolin-5-yl)amino)propanoate |
| | 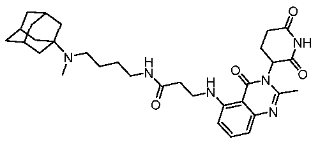 | N-(4-(((3s,5s,7s)-adamantan-1-yl)(methyl)amino)butyl)-3-((3 -(2,6-dioxopiperidin-3 -yl)-2-methyl-4-oxo-3,4-dihydroquinazolin-5-yl)amino)propanamide | N-(4-(((3s,5s,7s)-adamantan-1-yl)(methyl)amino)butyl)-3-((3-(2,6-dioxopiperidin-3-yl)-2-methyl-4-oxo-3,4-dihydroquinazolin-5-vl)amino)propanamide |
| | 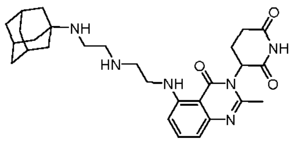 | 3-(5-((2-((2-(((1s,3s)-adamantan-1-yl)amino)ethyl)amino)ethyl)a mino)-2-methyl-4-oxoquinazolin-3 (4H)-yl)piperidine-2,6-dione | 3-(5-((2-((2-(((1s,3s)-adamantan-1-yl)amino)ethyl)amino)ethyl)a mino)-2-methyl-4-oxoquinazolin-3 (4H)-yl)piperidine-2,6-dione |
| | 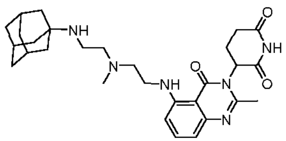 | 3-(5-((2-((2-(((1s,3s)-adamantan-1-yl)amino)ethyl) (methyl)amino )ethyl)amino)-2-methyl-4-oxoquinazolin-3 (4H)-yl)piperidine-2,6-dione | 3-(5-((2-((2-(((1s,3s)-adamantan-1-yl)amino)ethyl) (methyl)amino )ethyl)amino)-2-methyl-4-oxoquinazolin-3 (4H)-vl)piperidine-2,6-dione |
| GT-02663 | 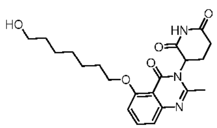 | 3-(5-((7-hydroxyheptyl)oxy)-2-methyl-4-oxoquinazolin-3(4H)-yl)piperidine-2,6-dione | 3-(5-((7-hydroxyheptyl)oxy)-2-methyl-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| GT-02769 | 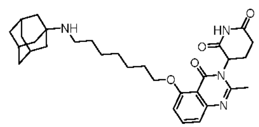 | 3-(5-((7-(((1s,3s)-adamantan-1-yl)amino)heptyl)oxy)-2-methyl-4-oxoquinazolin-3(4H)-yl)piperidine-2,6-dione | 3-(5-((7-(((1s,3s)-adamantan-1-yl)amino)heptyl)oxy)-2-methyl-4-oxoquinazolin-3(4H)-yl)piperidine-2,6-dione |
| GT-02770 | 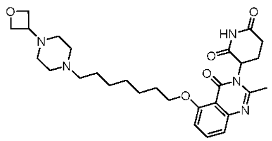 | 3-(2-methyl-5-((7-(4-(oxetan-3-yl)piperazin-1-yl)heptyl)oxy)-4-oxoquinazolin-3(4H)-yl)piperidine-2,6-dione | 3-(2-methyl-5-((7-(4-(oxetan-3-yl)piperazin-1-yl)heptyl)oxy)-4-oxoquinazolin-3(4H)-yl)piperidine-2,6-dione |
| GT-02771 | 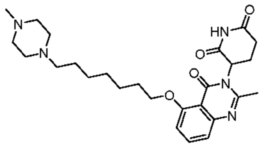 | 3-(2-methyl-5-((7-(4-methylpiperazin-1-yl)heptyl)oxy)-4-oxoquinazolin-3(4H)-yl)piperidine-2,6-dione | 3-(2-methyl-5-((7-(4-methylpiperazin-1-yl)heptyl)oxy)-4-oxoquinazolin-3(4H)-yl)piperidine-2,6-dione |
| | 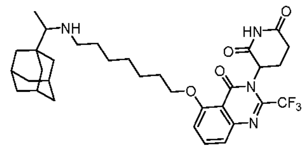 | 3-(5-((7-((1-((3r,5r,7r)-adamantan-1-yl)ethyl)amino)heptyl)oxy)-4-oxo-2-(trifluoromethyl)quinazolin-3(4H)-yl)piperidine-2,6-dione | 3-(5-((7-((1-((3r,5r,7r)-adamantan-1-yl)ethyl)amino)heptyl)oxy)-4-oxo-2-(trifluoromethyl)quinazolin-3(4H)-yl)piperidine-2,6-dione |
| | 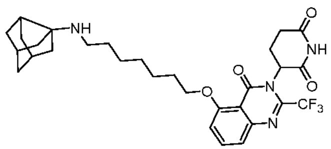 | 3-(5-((7-(((3as,6as)-hexahydro-2,5-methanopentalen-3a(1H)-yl)amino)heptyl)oxy)-4-oxo-2-(trifluoromethyl)quinazolin-3(4H)-yl)piperidine-2,6-dione | 3-(5-((7-(((3as,6as)-hexahydro-2,5-methanopentalen-3a(1H)-yl)amino)heptyl)oxy)-4-oxo-2-(trifluoromethyl)quinazolin-3(4H)-yl)piperidine-2,6-dione |
| | 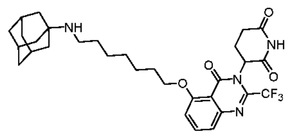 | 3-(5-((7-(((1s,3s)-adamantan-1-yl)amino)heptyl)oxy)-4-oxo-2-(trifluoromethyl)quinazolin-3(4H)-yl)piperidine-2,6-dione | 3-(5-((7-(((1s,3s)-adamantan-1-yl)amino)heptyl)oxy)-4-oxo-2-(trifluoromethyl)quinazolin-3(4H)-yl)piperidine-2,6-dione |
| | 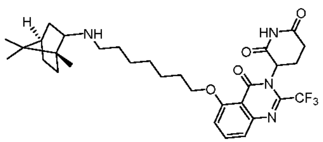 | 3-(4-oxo-2-(trifluoromethyl)-5-((7-(((1R,2S,4R)-1,7,7-trimethylbicyclo[2.2.1]heptan-2-yl)amino)heptyl)oxy)quinazoli n-3(4H)-yl)piperidine-2,6-dione | 3-(4-oxo-2-(trifluoromethyl)-5-((7-(((1R,2S,4R)-1,7,7-trimethylbicyclo[2.2. 1]heptan-2-yl)amino)heptyl)oxy)quinazoli n-3(4H)-yl)piperidine-2,6-dione |

(continued)

| Compound No. | Structure of the compounds | The compounds' name in English | Chinese translation of the compounds' name |
|---|---|---|---|
| | 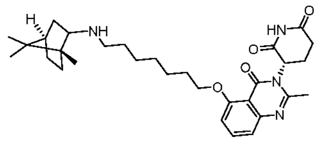 | (S)-3-(2-methyl-4-oxo-5-((7-(((1R,2S,4R)-1,7,7-trimethylbicyclo[2.2.1]heptan-2-yl)amino)heptyl)oxy)quinazolin-3(4H)-yl)piperidine-2,6-dione | (S)-3-(2-methyl-4-oxo-5-((7-(((1R,2S,4R)-1,7,7-trimethylbicyclo[2.2.1]heptan-2-yl)amino)heptyl)oxy)quinazolin-3(4H)-yl)piperidine-2,6-dione |
| | 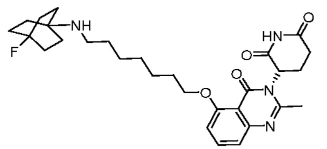 | (S)-3-(5-((7-((4-fluorobicyclo [2.2.2]octan-1-yl)amino)heptyl)oxy)-2-methyl-4-oxoquinazolin-3(4H)-yl)piperidine-2,6-dione | (S)-3-(5-((7-((4-fluorobicyclo [2.2.2]octan-1-yl)amino)heptyl)oxy)-2-methyl-4-oxoquinazolin-3(4H)-yl)piperidine-2,6-dione |
| | 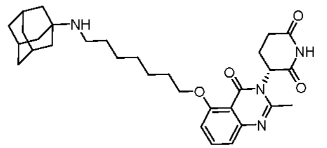 | (3R)-3-(5-((7-(((1s,3s)-adamantan-1-yl)amino)heptyl)oxy)-2-methyl-4-oxoquinazolin-3(4H)-yl)piperidine-2,6-dione | (3R)-3-(5-((7-(((1s,3s)-adamantan-1-yl)amino)heptyl)oxy)-2-methyl-4-oxoquinazolin-3(4H)-yl)piperidine-2,6-dione |
| | 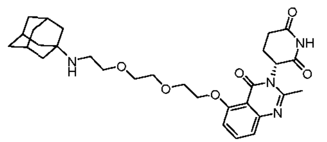 | (R)-3-(5-(2-(2-(2-(((3s,5s,7s)-adamantan-1-yl)amino)ethoxy)ethoxy)ethox y)-2-methyl-4-oxoquinazolin-3(4H)-yl)piperidine-2,6-dione | (R)-3-(5-(2-(2-(2-(((3s,5s,7s)-adamantan-1-yl)amino)ethoxy)ethoxy)ethox y)-2-methyl-4-oxoquinazolin-3(4H)-yl)piperidine-2,6-dione |
| | 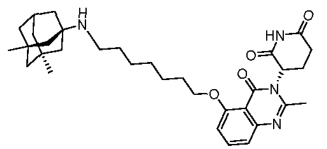 | (S)-3-(5-((7-(((1SR,3RS,SSR,7r)-3,5-dimethyladamantan-1-yl)amino)heptyl)oxy)-2-methyl-4-oxoquinazolin-3(4H)-yl)piperidine-2,6-dione | (S)-3-(5-((7-(((1SR,3RS,5SR,7r)-3,5-dimethyladamantan-1-yl)amino)heptyl)oxy)-2-methyl-4-oxoquinazolin-3(4H)-yl)piperidine-2,6-dione |
| | 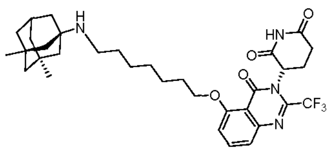 | (S)-3-(5-((7-(((1SR,3RS,SSR,7r)-3,5-dimethyladamantan-1-yl)amino)heptyl)oxy)-4-oxo-2-(trifluoromethyl)quinazolin-3(4H)-yl)piperidine-2,6-dione | (S)-3-(5-((7-(((1SR,3RS,SSR,7r)-3,5-dimethyladamantan-1-yl)amino)heptyl)oxy)-4-oxo-2-(trifluoromethyl)quinazolin-3(4H)-yl)piperidine-2,6-dione |

(continued)

| Compou nd No. | Structure of the compounds | The compounds' name in English | Chinese translation of the compounds' name |
|---|---|---|---|
| | 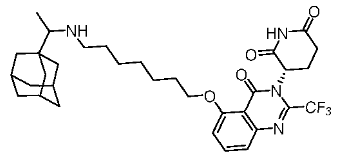 | (3S)-3-(5-((7-((1-((3r,Sr,7r)-adamantan-1-yl)ethyl)amino) heptyl)oxy)-4-oxo-2-(trifluoromethyl)quinazolin-3(4H)-yl) piperidine-2,6-dione | (3S)-3-(5-((7-((1-((3r,5r,7r)-adamantan-1-yl)ethyl)amino) heptyl)oxy)-4-oxo-2-(trifluoromethyl)quinazolin-3(4H)-yl) piperidine-2,6-dione |
| | 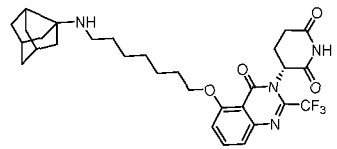 | (3R)-3-(5-((7-(((3as,6as)-hexahydro-2,5-methanopentalen-3a(1H)-yl)amino)heptyl)oxy)-4-oxo-2-(trifluoromethyl)quinazolin-3(4H)-yl)piperidine-2,6-dione | (3R)-3-(5-((7-(((3as,6as)-hexahydro-2,5-methanopentalen-3a(1H)-yl)amino)heptyl)oxy)-4-oxo-2-(trifluoromethyl) quinazolin-3(4H)-yl)piperidine-2,6-dione |
| | 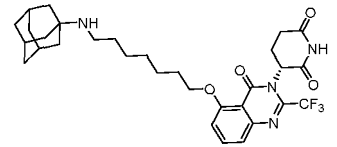 | (3R)-3-(5-((7-(((1s,3s)-adamantan-1-yl)amino)heptyl) oxy)-4-oxo-2-(trifluoromethyl)quinazolin-3(4H)-yl)piperidine-2,6-dione | (3R)-3-(5-((7-(((1s,3s)-adamantan-1-yl)amino)heptyl) oxy)-4-oxo-2-(trifluoromethyl)quinazolin-3(4H)-yl)piperi-dine-2,6-dione |
| | 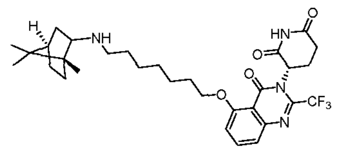 | (S)-3-(4-oxo-2-(trifluoromethyl)-5-((7-(((1R,2S,4R)-1,7,7-trimethylbicyclo[2.2.1]heptan-2-yl)amino)heptyl)oxy)quinazoli n-3(4H)-yl)piperidine-2,6-dione | (S)-3-(4-oxo-2-(trifluoromethyl)-5-((7-(((1R,2S,4R)-1,7,7-trimethylbicyclo[2.2.1]heptan-2-yl)amino)heptyl)oxy)quinazoli n-3(4H)-yl)piperidine-2,6-dione |
| GT-02802 | 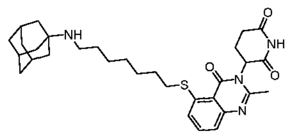 | 3-(5-((7-(((1s,3s)-adamantan-1-yl)amino)heptyl)thio)-2-methyl-4-oxoquinazolin-3(4H)-yl)piperidine-2,6-dione | 3-(5-((7-(((1s,3s)-adamantan-1-yl)amino)heptyl)thio)-2-methyl-4-oxoquinazolin-3(4H)-yl)piperidine-2,6-dione |
| | 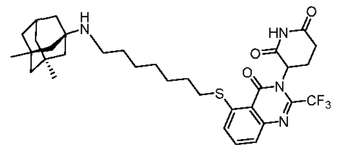 | 3-(5-((7-(((1SR,3RS,5SR,7r)-3,5-dimethyladamantan-1-yl)amino)heptyl)thio)-4-oxo-2-(trifluoromethyl)quinazolin-3(4H)-yl)piperidine-2,6-dione | 3-(5-((7-(((1SR,3RS,5SR,7r)-3,5-dimethyladamantan-1-yl)amino)heptyl)thio)-4-oxo-2-(trifluoromethyl)quinazolin-3(4H)-yl)piperidine-2,6-dione |

(continued)

| Compou nd No. | Structure of the compounds | The compounds' name in English | Chinese translation of the compounds' name |
|---|---|---|---|
| | 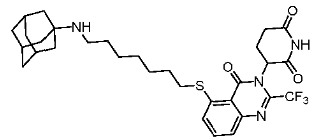 | 3-(5-((7-(((1s,3s)-adamantan-1-yl)amino)heptyl)thio)-4-oxo-2-(trifluoromethyl)quinazolin-3(4H)-yl)piperidine-2,6-dione | 3-(5-((7-(((1s,3s)-adamantan-1-yl)amino)heptyl)thio)-4-oxo-2-(trifluoromethyl)quinazolin-3(4H)-yl)piperidine-2,6-dione |
| | 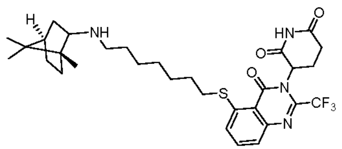 | 3-(4-oxo-2-(trifluoromethyl)-5-((7-(((1R,2S,4R)-1,7,7-trimethylbicyclo[2.2.1]heptan-2-yl)amino)heptyl)thio)quinazoli n-3(4H)-yl)piperidine-2,6-dione | 3-(4-oxo-2-(trifluoromethyl)-5-((7-(((1R,2S,4R)-1,7,7-trimethylbicyclo[2.2. 1]heptan-2-yl)amino)heptyl)thio)quinazoli n-3(4H)-yl)piperidine-2,6-dione |
| | 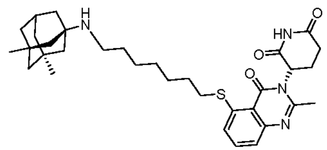 | (S)-3-(5-((7-(((1SR,3RS,5SR,7r)-3,5-dimethyladamantan-1-yl)amino)heptyl)thio)-2-methyl-4-oxoquinazolin-3(4H)-yl)piperidine-2,6-dione | (S)-3-(5-((7-(((1SR,3RS,5SR,7r)-3,5-dimethyladamantan-1-yl)amino)heptyl)thio)-2-methyl-4-oxoquinazolin-3(4H)-yl)piperidine-2,6-dione |
| | 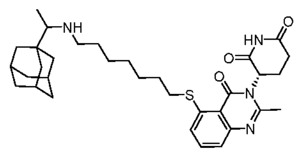 | (3S)-3-(5-((7-((1-((3r,Sr,7r)-adamantan-1-yl)ethyl)amino)heptyl)thio)-2-methyl-4-oxoquinazolin-3(4H)-yl)piperidine-2,6-dione | (3S)-3-(5-((7-((1-((3r,5r,7r)-adamantan-1-yl)ethyl)amino)heptyl)thio)-2-methyl-4-oxoquinazolin-3(4H)-yl)piperidine-2,6-dione |
| | 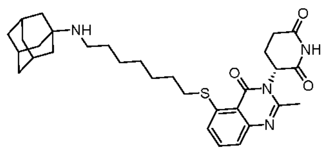 | (3R)-3-(5-((7-(((1s,3s)-adamantan-1-yl)amino)heptyl)thio)-2-methyl-4-oxoquinazolin-3(4H)-yl)piperidine-2,6-dione | (3R)-3-(5-((7-(((1s,3s)-adamantan-1-yl)amino)heptyl)thio)-2-methyl-4-oxoquinazolin-3(4H)-yl)piperidine-2,6-dione |
| | 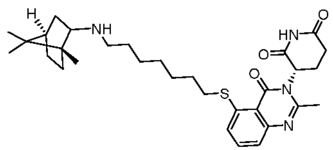 | (S)-3-(2-methyl-4-oxo-5-((7-(((1R,2S,4R)-1,7,7-trimethylbicyclo[2.2.1]heptan-2-yl)amino)heptyl)thio)quinazoli n-3(4H)-yl)piperidine-2,6-dione | (S)-3-(2-methyl-4-oxo-5-((7-(((1R,2S,4R)-1,7,7-trimethylbicyclo[2.2.1]heptan-2-yl)amino)heptyl)thio)quinazoli n-3(4H)-yl)piperidine-2,6-dione |

(continued)

| Compound No. | Structure of the compounds | The compounds' name in English | Chinese translation of the compounds' name |
|---|---|---|---|
| | 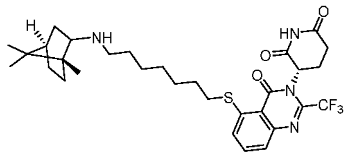 | (S)-3-(4-oxo-2-(trifluoromethyl)-5-((7-(((1R,2S,4R)-1,7,7-trimethylbicyclo[2.2.1]heptan-2-yl)amino)heptyl)thio)quinazoli n-3(4H)-yl)piperidine-2,6-dione | (S)-3-(4-oxo-2-(trifluoromethyl)-5 -((7-(((1R,2S,4R)-1,7,7-trimethylbicyclo[2.2.1]heptan-2-yl)amino)heptyl)thio)quinazoli n-3(4H)-yl)piperidine-2,6-dione |
| | 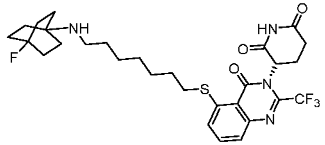 | (S)-3-(5-((7-((4-fluorobicyclo[2.2.2]octan-1-yl)amino)heptyl)thio)-4-oxo-2-(trifluoromethyl)quinazolin-3(4H)-yl)piperidine-2,6-dione | (S)-3-(5-((7-((4-fluorobicyclo [2.2.2]octan-1-yl)amino) heptyl)thio)-4-oxo-2-(trifluoromethyl)quinazolin-3(4H)-yl) piperidine-2,6-dione |
| | 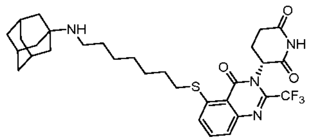 | (3R)-3-(5-((7-(((1s,3s)-adamantan-1-yl)amino)heptyl) thio)-4-oxo-2-(trifluoromethyl)quinazolin-3(4H)-yl)piperidine-2,6-dione | (3R)-3-(5-((7-(((1s,3s)-adamantan-1-yl)amino)heptyl) thio)-4-oxo-2-(trifluoromethyl)quinazolin-3(4H)-yl)piperidine-2,6-dione |
| | 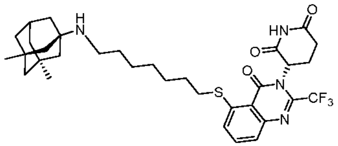 | (S)-3-(5-((7-(((1SR,3RS,5SR,7r)-3,5-dimethyladamantan-1-yl)amino)heptyl)thio)-4-oxo-2-(trifluoromethyl)quinazolin-3(4H)-yl)piperidine-2,6-dione | (S)-3-(5-((7-(((1SR,3RS,5SR,7r)-3,5-dimethyladamantan-1-yl)amino)heptyl)thio)-4-oxo-2-(trifluoromethyl)quinazolin-3(4H)-yl)piperidine-2,6-dione |
| GT-02814 | 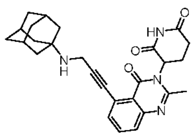 | 3-(5-(3-(((3s,5s,7s)-adamantan-1-yl)amino)prop-1-yn-1-yl)-2-methyl-4-oxoquinazolin-3 (4H)-yl)piperidine-2,6-dione | 3-(5-(3-(((3s,5s,7s)-adamantan-1-yl)amino)prop-1-yn-1-yl)-2-methyl-4-oxoquinazolin-3 (4H)-yl)piperidine-2,6-dione |
| GT-02597 | 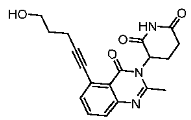 | 3-(5-(5-hydroxypent-1-yn-1-yl)-2-methyl-4-oxoquinazolin-3(4H)-yl)piperidine-2,6-dione | 3-(5-(5-hydroxypent-1-yn-1-yl)-2-methyl-4-oxoquinazolin-3(4H)-yl)piperidine-2,6-dione |
| GT-02632 | 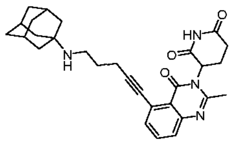 | 3-(5-(5-(((3s,5s,7s)-adamantan-1-yl)amino)pent-1-yn-1-yl)-2-methyl-4-oxoquinazolin-3 (4H)-yl)piperidine-2,6-dione | 3-(5-(5-(((3s,5s,7s)-adamantan-1-yl)amino)pent-1-yn-1-yl)-2-methyl-4-oxoquinazolin-3 (4H)-yl)piperidine-2,6-dione |

(continued)

| Compound No. | Structure of the compounds | The compounds' name in English | Chinese translation of the compounds' name |
|---|---|---|---|
| GT-02633 | 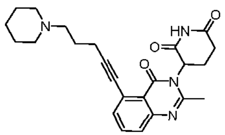 | 3-(2-methyl-4-oxo-5-(5-(piperidin-1-yl)pent-1-yn-1-yl)quinazolin-3(4H)-yl)piperidine-2,6-dione | 3-(2-methyl-4-oxo-5-(5-(piperidin-1-yl)pent-1-yn-1-yl)quinazolin-3 (4H)-yl)piperidine-2,6-dione |
| GT-02634 | 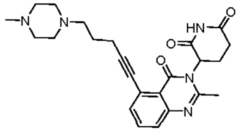 | 3-(2-methyl-5-(5-(4-methylpiperazin-1-yl)pent-1-yn-1-yl)-4-oxoquinazolin-3(4H)-yl)piperidine-2,6-dione | 3-(2-methyl-5-(5-(4-methylpiperazin-1-yl)pent-1-yn-1-yl)-4-oxoquinazolin-3(4H)-yl)piperidine-2,6-dione |
| GT-02635 | 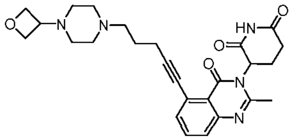 | 3-(2-methyl-5-(5-(4-(oxetan-3-yl)piperazin-1-yl)pent-1-yn-1-yl)-4-oxoquinazolin-3(4H)-yl)piperidine-2,6-dione | 3-(2-methyl-5-(5-(4-(oxetan-3-yl)piperazin-1-yl)pent-1-yn-1-yl)-4-oxoquinazolin-3 (4H)-yl)piperidine-2,6-dione |
| GT-02636 | 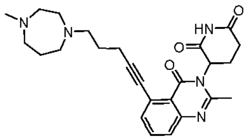 | 3-(2-methyl-5-(5-(4-methyl-1,4-diazepan-1-yl)pent-1-yn-1-yl)-4-oxoquinazolin-3(4H)-yl)piperidine-2,6-dione | 3-(2-methyl-5-(5-(4-methyl-1,4-diazepan-1-yl)pent-1-yn-1-yl)-4-oxoquinazolin-3(4H)-yl)piperidine-2,6-dione |
| GT-02815 | 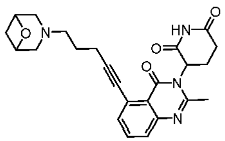 | 3-(5-(5-(6-oxa-3-azabicyclo[3.1.1]heptan-3-yl)pent-1-yn-1-yl)-2-methyl-4-oxoquinazolin-3 (4H)-yl)piperidine-2,6-dione | 3-(5-(5-(6-oxa-3-azabicyclo[3.1.1]heptan-3-yl)pent-1-yn-1-yl)-2-methyl-4-oxoquinazolin-3(4H)-yl)piperidine-2,6-dione |
| GT-02816 | 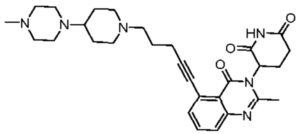 | 3-(2-methyl-5 -(5-(4-(4-methylpiperazin-1-yl)piperidin-1-yl)pent-1-yn-1-yl)-4-oxoquinazolin-3(4H)-yl)piperidine-2,6-dione | 3-(2-methyl-5-(5-(4-(4-methylpiperazin-1-yl)piperidin-1-yl)pent-1-yn-1-yl)-4-oxoquinazolin-3(4H)-yl)piperidine-2,6-dione |
| GT-02817 | 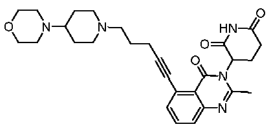 | 3-(2-methyl-5-(5-(4-morpholinopiperidin-1-yl)pent-1-yn-1-yl)-4-oxoquinazolin-3(4H)-yl)piperidine-2,6-dione | 3-(2-methyl-5-(5-(4-morpholinopiperidin-1-yl)pent-1-yn-1-yl)-4-oxoquinazolin-3 (4H)-yl)piperidine-2,6-dione |

(continued)

| Compound No. | Structure of the compounds | The compounds' name in English | Chinese translation of the compounds' name |
|---|---|---|---|
| GT-02818 | 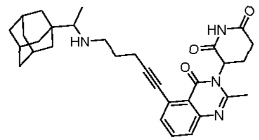 | 3-(5-(5-((1-((1s,3s)-adamantan-1-yl)ethyl)amino)pent-1-yn-1-yl)-2-methyl-4-oxoquinazolin-3(4H)-yl)piperidine-2,6-dione | 3-(5-(5-((1-((1s,3s)-adamamtan-1-yl)ethyl)amino)pent-1-yn-1-yl)-2-methyl-4-oxoquinazolin-3(4H)-yl)piperidine-2,6-dione |
| GT-02637 | 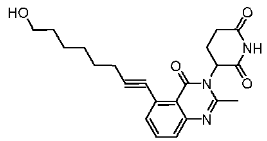 | 3-(5-(8-hydroxyoct-1-yn-1-yl)-2-methyl-4-oxoquinazolin-3 (4H)-yl)piperidine-2,6-dione | 3-(5-(8-hydroxyoct-1-yn-1-yl)-2-methyl-4-oxoquinazolin-3(4H)-yl)piperidine-2,6-dione |
| GT-02638 | 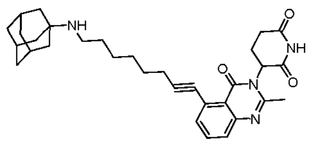 | 3-(5-(8-(((1s,3s)-adamantan-1-yl)amino)oct-1-yn-1-yl)-2-methyl-4-oxoquinazolin-3(4H)-yl)piperidine-2,6-dione | 3-(5-(8-(((1s,3s)-adamantan-1-yl)amino)oct-1-yn-1-yl)-2-methyl-4-oxoquinazolin-3(4H)-yl)piperidine-2,6-dione |
| GT-02639 | 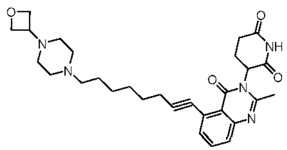 | 3-(2-methyl-5-(8-(4-(oxetan-3-yl)piperazin-1-yl)oct-1-yn-1-yl)-4-oxoquinazolin-3(4H)-yl)piperidine-2,6-dione | 3-(2-methyl-5-(8-(4-(oxetan-3-yl)piperazin-1-yl)oct-1-yn-1-yl)-4-oxoquinazolin-3(4H)-yl)piperidine-2,6-dione |
| GT-02645 | 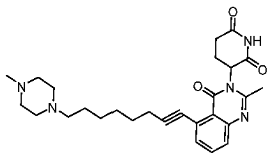 | 3-(2-methyl-5-(8-(4-methylpiperazin-1-yl)oct-1-yn-1-yl)-4-oxoquinazolin-3 (4H)-yl)piperidine-2,6-dione | 3-(2-methyl-5-(8-(4-methylpiperazin-1-yl)oct-1-yn-1-yl)-4-oxoquinazolin-3(4H)-yl)piperidine-2,6-dione |
| GT-02653 | 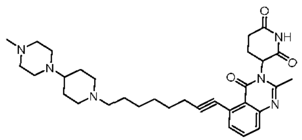 | 3-(2-methyl-5-(8-(4-(4-methylpiperazin-1-yl)piperidin-1-yl)oct-1-yn-1-yl)-4-oxoquinazolin-3(4H)-yl)piperidine-2,6-dione | 3-(2-methyl-5-(8-(4-(4-methylpiperazin-1-yl)piperidin-1-yl)oct-1-yn-1-yl)-4-oxoquinazolin-3(4H)-yl)piperidine-2,6-dione |

(continued)

| Compou nd No. | Structure of the compounds | The compounds' name in English | Chinese translation of the compounds' name |
|---|---|---|---|
| GT-02654 | 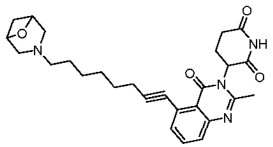 | 3-(5-(8-(6-oxa-3-azabicyclo[3.1.1]heptan-3-yl)oct-1-yn-1-yl)-2-methyl-4-oxoquinazolin-3(4H)-yl)piperidine-2,6-dione | 3-(5-(8-(6-oxa-3-azabicyclo[3.1.1]heptan-3-yl)oct-1-yn-1-yl)-2-methyl-4-oxoquinazolin-3(4H)-yl)piperidine-2,6-dione |
| GT-02640 | 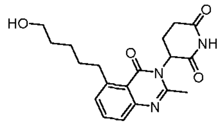 | 3-(5-(5-hydroxypentyl)-2-methyl-4-oxoquinazolin-3(4H)-yl)piperidine-2,6-dione | 3-(5-(5-hydroxypentyl)-2-methyl-4-oxoquinazolin-3(4H)-yl)piperidine-2,6-dione |
| GT-02759 | 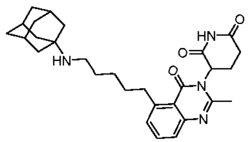 | 3-(5-(5-(((3s,5s,7s)-adamantan-1-yl)amino)pentyl)-2-methyl-4-oxoquinazolin-3(4H)-yl)piperidine-2,6-dione | 3-(5-(5-(((3s,5s,7s)-adamantan-1-yl)amino)pentyl)-2-methyl-4-oxoquinazolin-3(4H)-yl)piperidine-2,6-dione |
| GT-02761 | 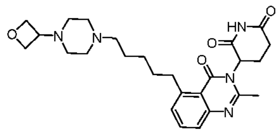 | 3-(2-methyl-5-(5-(4-(oxetan-3-yl)piperazin-1-yl)pentyl)-4-oxoquinazolin-3(4H)-yl)piperidine-2,6-dione | 3-(2-methyl-5-(5-(4-(oxetan-3-yl)piperazin-1-yl)pentyl)-4-oxoquinazolin-3(4H)-yl)piperidine-2,6-dione |
| GT-02762 | 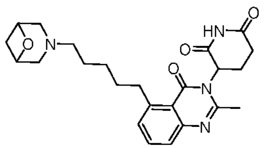 | 3-(5-(5-(6-oxa-3-azabicyclo[3.1.1]heptan-3-yl)pentyl)-2-methyl-4-oxoquinazolin-3(4H)-yl)piperidine-2,6-dione | 3-(5-(5-(6-oxa-3-azabicyclo[3.1.1]heptan-3-yl)pentyl)-2-methyl-4-oxoquinazolin-3 (4H)-yl)piperidine-2,6-dione |
| GT-02763 | 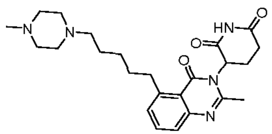 | 3-(2-methyl-5-(5-(4-methylpiperazin-1-yl)pentyl)-4-oxoquinazolin-3(4H)-yl)piperidine-2,6-dione | 3-(2-methyl-5-(5-(4-methylpiperazin-1-yl)pentyl)-4-oxoquinazolin-3(4H)-yl)piperidine-2,6-dione |
| GT-02764 | 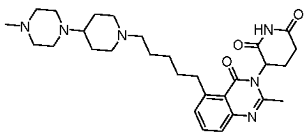 | 3-(2-methyl-5-(5-(4-(4-methylpiperazin-1-yl)piperidin-1-yl)pentyl)-4-oxoquinazolin-3(4H)-yl)piperidine-2,6-dione | 3-(2-methyl-5-(5-(4-(4-methylpiperazin-1-yl)piperidin-1-yl)pentyl)-4-oxoquinazolin-3 (4H)-yl)piperidine-2,6-dione |

(continued)

| Compound No. | Structure of the compounds | The compounds' name in English | Chinese translation of the compounds' name |
|---|---|---|---|
| GT-02760 | 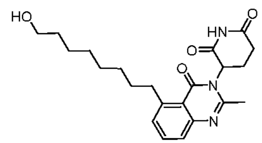 | 3-(5-(8-hydroxyoctyl)-2-methyl-4-oxoquinazolin-3(4H)-yl) piperidine-2,6-dione | 3-(5-(8-hydroxyoctyl)-2-methyl-4-oxoquinazolin-3(4H)-yl) piperidine-2,6-dione |
| GT-02797 | 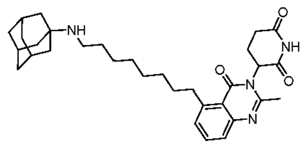 | 3-(5-(8-(((1s,3s)-adamantan-1-yl)amino)octyl)-2-methyl-4-oxoquinazolin-3(4H)-yl)piperidine-2,6-dione | 3-(5-(8-(((1s,3s)-adamantan-1-yl)amino)octyl)-2-methyl-4-oxoquinazolin-3(4H)-yl)piperidine-2,6-dione |
| GT-02798 | 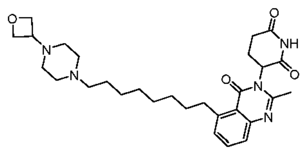 | 3-(2-methyl-5-(8-(4-(oxetan-3-yl)piperazin-1-yl)octyl)-4-oxoquinazolin-3(4H)-yl)piperidine-2,6-dione | 3-(2-methyl-5-(8-(4-(oxetan-3-yl)piperazin-1-yl)octyl)-4-oxoquinazolin-3(4H)-yl)piperidine-2,6-dione |
| GT-02799 | 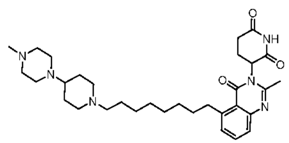 | 3-(2-methyl-5-(8-(4-(4-methylpiperazin-1-yl)piperidin-1-yl)octyl)-4-oxoquinazolin-3(4H)-yl)piperidine-2,6-dione | 3-(2-methyl-5-(8-(4-(4-methylpiperazin-1-yl)piperidin-1-yl)octyl)-4-oxoquinazolin-3(4H)-yl)piperidine-2,6-dione |
| GT-02800 | 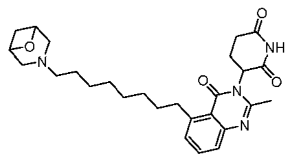 | 3-(5-(8-(6-oxa-3-azabicyclo[3.1.1]heptan-3-yl)octyl)-2-methyl-4-oxoquinazolin-3(4H)-yl)piperidine-2,6-dione | 3-(5-(8-(6-oxa-3-azabicyclo[3.1.1]heptan-3-yl)octyl)-2-methyl-4-oxoquinazolin-3(4H)-yl)piperidine-2,6-dione |
| GT-02813 | 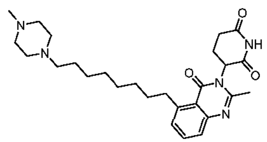 | 3-(2-methyl-5-(8-(4-methylpiperazin-1-yl)octyl)-4-oxoquinazolin-3(4H)-yl)piperidine-2,6-dione | 3-(2-methyl-5-(8-(4-methylpiperazin-1-yl)octyl)-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| | 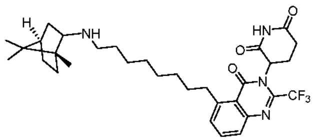 | 3-(4-oxo-2-(trifluoromethyl)-5-(8-(((1R,2S,4R)-1,7,7-trimethylbicyclo[2.2.1]heptan-2-yl)amino)octyl)quinazolin-3(4H)-yl)piperidine-2,6-dione | 3-(4-oxo-2-(trifluoromethyl)-5-(8-(((1R,2S,4R)-1,7,7-trimethylbicyclo[2.2.1]heptan-2-yl)amino)octyl)quinazolin-3(4H)-yl)piperidine-2,6-dione |
| | 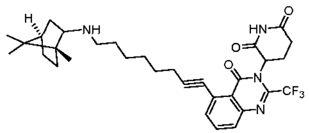 | 3-(4-oxo-2-(trifluoromethyl)-5-(8-(((1R,2S,4R)-1,7,7-trimethylbicyclo[2.2.1]heptan-2-yl)amino)oct-1-yn-1-yl)quinazolin-3(4H)-yl)piperidine-2,6-dione | 3-(4-oxo-2-(trifluoromethyl)-5-(8-(((1R,2S,4R)-1,7,7-trimethylbicyclo[2.2.1]heptan-2-yl)amino)oct-1-yn-1-yl)quinazolin-3(4H)-yl)piperidine-2,6-dione |
| | 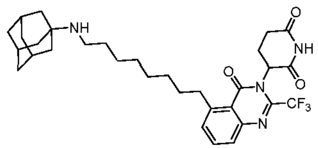 | 3-(5-(8-(((1s,3s)-adamantan-1-yl)amino)octyl)-4-oxo-2-(trifluoromethyl)quinazolin-3 (4H)-yl)piperidine-2,6-dione | 3-(5-(8-(((1s,3s)-adamantan-1-yl)amino)octyl)-4-oxo-2-(trifluoromethyl)quinazolin-3 (4H)-yl)piperidine-2,6-dione |
| | 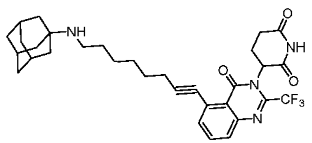 | 3-(5-(8-(((1s,3s)-adamantan-1-yl)amino)oct-1-yn-1-yl)-4-oxo-2-(trifluoromethyl)quinazolin-3(4H)-yl)piperidine-2,6-dione | 3-(5-(8-(((1s,3s)-adamantan-1-yl)amino)oct-1-yn-1-yl)-4-oxo-2-(trifluoromethyl)quinazolin-3(4H)-yl)piperidine-2,6-dione |
| | 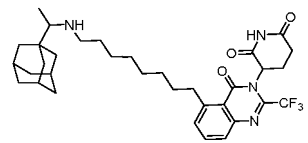 | 3-(5-(8-((1-((3r,5r,7r)-adamantan-1-yl)ethyl)amino)octyl)-4-oxo-2-(trifluoromethyl)quinazolin-3(4H)-yl)piperidine-2,6-dione | 3-(5-(8-((1-((3r,Sr,7r)-adamantan-1-yl)ethyl)amino)octyl)-4-oxo-2-(trifluoromethyl)quinazolin-3(4H)-yl)piperidine-2,6-dione |
| | 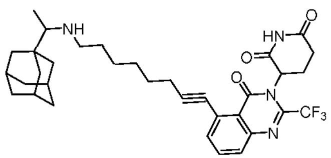 | 3-(5-(8-((1-((3r,5r,7r)-adamantan-1-yl)ethyl)amino)oct-1-yn-1-yl)-4-oxo-2-(trifluoromethyl)quinazolin-3(4H)-yl)piperidine-2,6-dione | 3-(5-(8-((1-((3r,5r,7r)-adamantan-1-yl)ethyl)amino)oct-1-yn-1-yl)-4-oxo-2-(trifluoromethyl)quinazolin-3(4H)-yl)piperidine-2,6-dione |

(continued)

| Compou nd No. | Structure of the compounds | The compounds' name in English | Chinese translation of the compounds' name |
|---|---|---|---|
| | 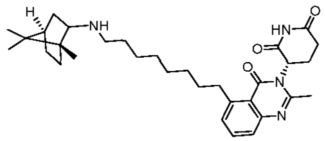 | (S)-3 -(2-methyl-4-oxo-5-(8-(((1R,2S,4R)-1,7,7-trimethylbicyclo[2.2.1]heptan-2-yl)amino)octyl)quinazolin-3(4H)-yl)piperidine-2,6-dione | (S)-3-(2-methyl-4-oxo-5-(8-(((1R,2S,4R)-1,7,7-trimethylbicyclo[2.2.1]heptan-2-yl)amino)octyl)quinazolin-3(4H)-yl)piperidine-2,6-dione |
| | 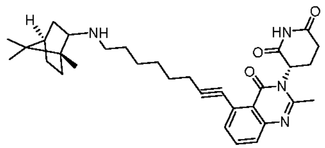 | (S)-3-(2-methyl-4-oxo-5-(8-(((1R,2S,4R)-1,7,7-trimethylbicyclo[2.2.1]heptan-2-yl)amino)oct-1-yn-1-yl)quinazolin-3(4H)-yl)piperidine-2,6-dione | (S)-3-(2-methyl-4-oxo-5-(8-(((1R,2S,4R)-1,7,7-trimethylbicyclo[2.2.1]heptan-2-yl)amino)oct-1-yn-1-yl)quinazolin-3(4H)-yl)piperidine-2,6-dione |
| | 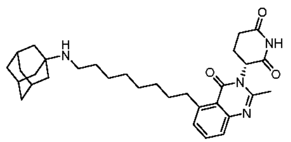 | (3R)-3-(5-(8-(((1s,3s)-adamantan-1-yl)amino)octyl)-2-methyl-4-oxoquinazolin-3(4H)-yl)piperidine-2,6-dione | (3R)-3-(5-(8-(((1s,3s)-adamantan-1-yl)amino)octyl)-2-methyl-4-oxoquinazolin-3(4H)-yl)piperidine-2,6-dione |
| | 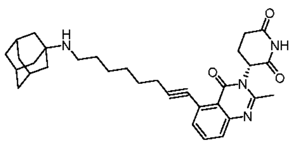 | (3R)-3-(5-(8-(((1s,3s)-adamantan-1-yl)amino)oct-1-yn-1-yl)-2-methyl-4-oxoquinazolin-3(4H)-yl)piperidine-2,6-dione | (3R)-3-(5-(8-(((1s,3s)-adamantan-1-yl)amino)oct-1-yn-1-yl)-2-methyl-4-oxoquinazolin-3(4H)-yl)piperidine-2,6-dione |
| | 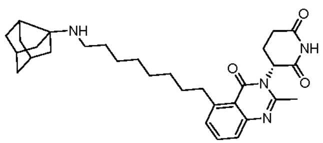 | (3R)-3-(5-(8-(((3as,6as)-hexahydro-2,5-methanopentalen-3a(1H)-yl)amino)octyl)-2-methyl-4-oxoquinazolin-3(4H)-yl)piperidine-2,6-dione | (3R)-3-(5-(8-(((3as,6as)-hexahydro-2,5-methanopentalen-3a(1H)-yl)amino)octyl)-2-methyl-4-oxoquinazolin-3(4H)-yl)piperidine-2,6-dione |
| | 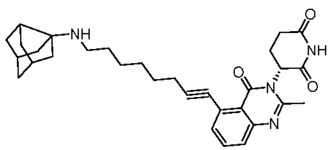 | (3R)-3-(5-(8-(((3as,6as)-hexahydro-2,5-methanopentalen-3a(1H)-yl)amino)oct-1-yn-1-yl)-2-methyl-4-oxoquinazolin-3(4H)-yl)piperidine-2,6-dione | (3R)-3-(5-(8-(((3as,6as)-hexahydro-2,5-methanopentalen-3a(1H)-yl)amino)oct-1-yn-1-yl)-2-methyl-4-oxoquinazolin-3(4H)-yl)piperidine-2,6-dione |

(continued)

| Compou nd No. | Structure of the compounds | The compounds' name in English | Chinese translation of the compounds' name |
|---|---|---|---|
| | 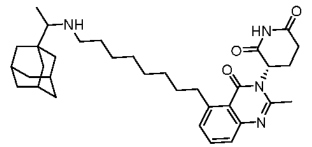 | (3S)-3-(5-(8-((1-((3r,5r,7r)-adamantan-1-yl)ethyl)amino) octyl)-2-methyl-4-oxoquinazolin-3(4H)-yl)piperidine-2,6-dione | (3S)-3-(5-(8-((1-((3r,5r,7r)-adamantan-1-yl)ethyl)amino) octyl)-2-methyl-4-oxoquinazolin-3(4H)-yl)piperidine-2,6-dione |
| | 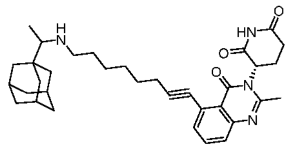 | (3S)-3-(5-(8-((1-((3r,5r,7r)-adamantan-1-yl)ethyl)amino) oct-1-yn-1-yl)-2-methyl-4-oxoquinazolin-3(4H)-yl)piperidine-2,6-dione | (3S)-3-(5-(8-((1-((3r,5r,7r)-adamantan-1-yl)ethyl)amino) oct-1-yn-1-yl)-2-methyl-4-oxoquinazolin-3(4H)-yl)piperidine-2,6-dione |
| | 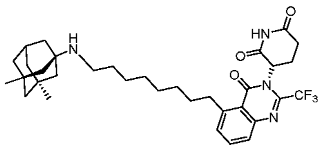 | (S)-3-(5-(8-(((1SR,3RS,5SR,7r)-3,5-dimethyladamantan-1-yl)amino)octyl)-4-oxo-2-(trifluoromethyl)quinazolin-3(4H)-yl)piperidine-2,6-dione | (S)-3-(5-(8-(((1SR,3RS,5SR,7r)-3,5-dimethyladamantan-1-yl)amino)octyl)-4-oxo-2-(trifluoromethyl)quinazolin-3(4H)-yl)piperidine-2,6-dione |
| | 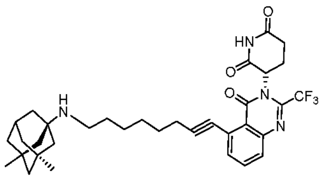 | (S)-3-(5-(8-(((1SR,3RS,5SR,7r)-3,5-dimethyladamantan-1-yl)amino)oct-1-yn-1-yl)-4-oxo-2-(trifluoromethyl) quinazolin-3(4H)-yl)piperidine-2,6-dione | (S)-3-(5-(8-(((1SR,3RS,5SR,7r)-3,5-dimethyladamantan-1-yl)amino)oct-1-yn-1-yl)-4-oxo-2-(trifluoromethyl) quinazolin-3(4H)-yl)piperidine-2,6-dione |
| | 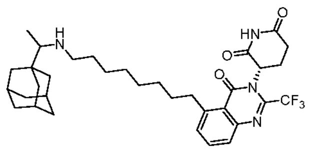 | (3S)-3-(5-(8-((1-((3r,5r,7r)-adamantan-1-yl)ethyl)amino) octyl)-4-oxo-2-(trifluoromethyl)quinazolin-3(4H)-yl)piperidine-2,6-dione | (3S)-3-(5-(8-((1-((3r,5r,7r)-adamantan-1-yl)ethyl)amino) octyl)-4-oxo-2-(trifluoromethyl)quinazolin-3(4H)-yl)piperidine-2,6-dione |
| | 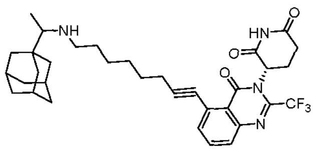 | (3S)-3-(5-(8-((1-((3r,5r,7r)-adamantan-1-yl)ethyl)amino) oct-1-yn-1-yl)-4-oxo-2-(trifluoromethyl)quinazolin-3(4H)-yl)piperidine-2,6-dione | (3 S)-3-(5-(8-((1-((3r,5r,7r)-adamantan-1-yl)ethyl)amino) oct-1-yn-1-yl)-4-oxo-2-(trifluoromethyl)quinazolin-3(4H)-yl)piperidine-2,6-dione |

(continued)

| Compound No. | Structure of the compounds | The compounds' name in English | Chinese translation of the compounds' name |
|---|---|---|---|
| | 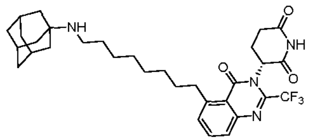 | (3R)-3-(5-(8-(((1s,3s)-adamantan-1-yl)amino)octyl)-4-oxo-2-(trifluoromethyl)quinazolin-3(4H)-yl)piperidine-2,6-dione | (3R)-3-(5-(8-(((1s,3s)-adamantan-1-yl)amino)octyl)-4-oxo-2-(trifluoromethyl)quinazolin-3(4H)-yl)piperidine-2,6-dione |
| | 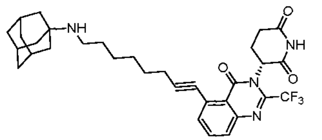 | (3R)-3-(5-(8-(((1s,3s)-adamantan-1-yl)amino)oct-1-yn-1-yl)-4-oxo-2-(trifluoromethyl)quinazolin-3(4H)-yl)piperidine-2,6-dione | (3R)-3-(5-(8-(((1s,3s)-adamantan-1-yl)amino)oct-1-yn-1-yl)-4-oxo-2-(trifluoromethyl)quinazolin-3(4H)-yl)piperidine-2,6-dione |
| | 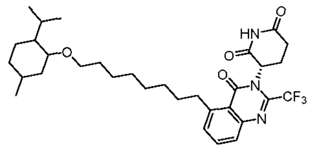 | (3S)-3-(5-(8-((2-isopropyl-5-methylcyclohexyl)oxy)octyl)-4-oxo-2-(trifluoromethyl)quinazolin-3(4H)-yl)piperidine-2,6-dione | (3S)-3-(5-(8-((2-isopropyl-5-methylcyclohexyl)oxy)octyl)-4-oxo-2-(trifluoromethyl)quinazolin-3(4H)-yl)piperidine-2,6-dione |
| | 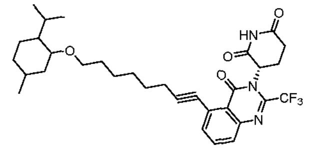 | (3S)-3-(5-(8-((2-isopropyl-5-methylcyclohexyl)oxy)oct-1-yn-1-yl)-4-oxo-2-(trifluoromethyl)quinazolin-3(4H)-yl)piperidine-2,6-dione | (3S)-3-(5-(8-((2-isopropyl-5-methylcyclohexyl)oxy)oct-1-yn-1-yl)-4-oxo-2-(trifluoromethyl)quinazolin-3(4H)-yl)piperidine-2,6-dione |
| | 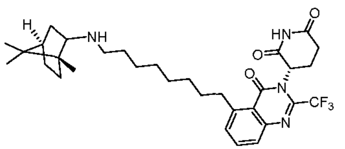 | (S)-3-(4-oxo-2-(trifluoromethyl)-5-(8-(((1R,2S,4R)-1,7,7-trimethylbicyclo[2.2.1]heptan-2-yl)amino)octyl)quinazolin-3(4H)-yl)piperidine-2,6-dione | (S)-3-(4-oxo-2-(trifluoromethyl)-5-(8-(((1R,2S,4R)-1,7,7-trimethylbicyclo[2.2.1]heptan-2-yl)amino)octyl)quinazolin-3(4H)-yl)piperidine-2,6-dione |
| | 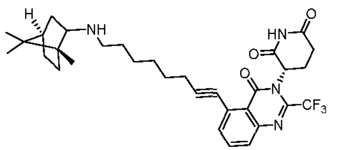 | (S)-3-(4-oxo-2-(trifluoromethyl)-5 -(8-(((1R,2S,4R)-1,7,7-trimethylbicyclo[2.2.1]heptan-2-yl)amino)oct-1-yn-1-yl) quinazolin-3(4H)-yl)piperidine-2,6-dione | (S)-3-(4-oxo-2-(trifluoromethyl)-5-(8-(((1R,2S,4R)-1,7,7-trimethylbicyclo[2.2.1] heptan-2-yl)amino)oct-1-yn-1-yl) quinazolin-3(4H)-yl)piperidine-2,6-dione |

(continued)

| Compound No. | Structure of the compounds | The compounds' name in English | Chinese translation of the compounds' name |
|---|---|---|---|
| | 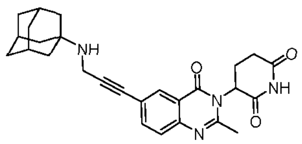 | 3-(6-(3-(((1s,3s)-adamantan-1-yl)amino)prop-1-yn-1-yl)-2-methyl-4-oxoquinazolin-3(4H)-yl)piperidine-2,6-dione | 3-(6-(3-(((1s,3s)-adamantan-1-yl)amino)prop-1-yn-1-yl)-2-methyl-4-oxoquinazolin-3(4H)-yl)piperidine-2,6-dione |
| | 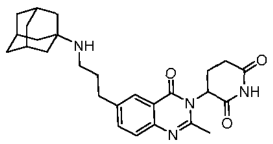 | 3-(6-(3-(((1s,3s)-adamantan-1-yl)amino)propyl)-2-methyl-4-oxoquinazolin-3 (4H)-yl)piperidine-2,6-dione | 3-(6-(3-(((1s,3s)-adamantan-1-yl)amino)propyl)-2-methyl-4-oxoquinazolin-3 (4H)-yl)piperidine-2,6-dione |
| | 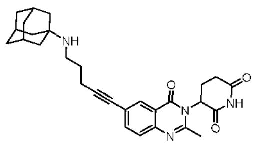 | 3-(6-(5-(((1s,3s)-adamantan-1-yl)amino)pent-1-yn-1-yl)-2-methyl-4-oxoquinazolin-3 (4H)-yl)piperidine-2,6-dione | 3-(6-(5-(((1s,3s)-adamantan-1-yl)amino-yn-1-yl)-2-methyl-4-oxoquinazolin-3(4H)-yl)piperidine-2,6-dione |
| | 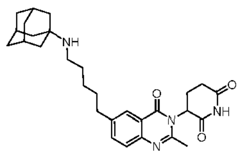 | 3-(6-(5-(((1s,3s)-adamantan-1-yl)amino)pentyl)-2-methyl-4-oxoquinazolin-3 (4H)-yl)piperidine-2,6-dione | 3-(6-(5-(((1s,3s)-adamantan-1-yl)amino)pentyl)-2-methyl-4-oxoquinazolin-3 (4H)-yl)piperidine-2,6-dione |
| | 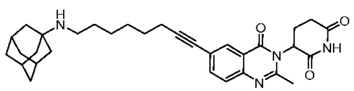 | 3-(6-(8-(((3s,5s,7s)-adamantan-1-yl)amino)oct-1-yn-1-yl)-2-methyl-4-oxoquinazolin-3(4H)-yl)piperidine-2,6-dione | 3-(6-(8-(((3s,5s,7s)-adamantan-1-yl)amino)oct-1-yn-1-yl)-2-methyl-4-oxoquinazolin-3(4H)-yl)piperidine-2,6-dione |
| | 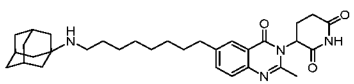 | 3-(6-(8-(((3s,5s,7s)-adamantan-1-yl)amino)octyl)-2-methyl-4-oxoquinazolin-3(4H)-yl)piperidine-2,6-dione | 3-(6-(8-(((3s,5s,7s)-adamantan-1-yl)amino)octyl)-2-methyl-4-oxoquinazolin-3(4H)-yl)piperidine-2,6-dione |
| | 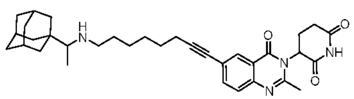 | 3-(6-(8-((1-((1s,3s)-adamantan-1-yl)ethyl)amino)oct-1-yn-1-yl)-2-methyl-4-oxoquinazolin-3(4H)-yl)piperidine-2,6-dione | 3-(6-(8-((1-((1s,3s)-adamantan-1-yl)ethyl)amino)oct-1-yn-1-yl)-2-methyl-4-oxoquinazolin-3(4H)-yl)piperidine-2,6-dione |
| | 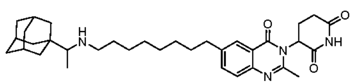 | 3-(6-(8-((1-((1s,3s)-adamantan-1-yl)ethyl)amino)octyl)-2-methyl-4-oxoquinazolin-3(4H)-yl)piperidine-2,6-dione | 3-(6-(8-((1-((1s,3s)-adamantan-1-yl)ethyl)amino)octyl)-2-methyl-4-oxoquinazolin-3(4H)-yl)piperidine-2,6-dione |

(continued)

| Compou nd No. | Structure of the compounds | The compounds' name in English | Chinese translation of the compounds' name |
|---|---|---|---|
| | 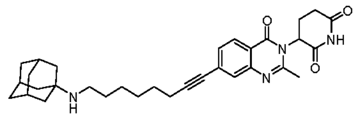 | 3-(7-(8-(((3s,5s,7s)-adamantan-1-yl)amino)oct-1-yn-1-yl)-2-methyl-4-oxoquinazolin-3(4H)-yl)piperidine-2,6-dione | 3-(7-(8-(((3s,5s,7s)-adamantan-1-yl)amino)oct-1-yn-1-yl)-2-methyl-4-oxoquinazolin-3(4H)-yl)piperidine-2,6-dione |
| | 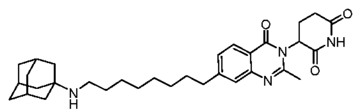 | 3-(7-(8-(((3s,5s,7s)-adamantan-1-yl)amino)octyl)-2-methyl-4-oxoquinazolin-3(4H)-yl)piperidine-2,6-dione | 3-(7-(8-(((3s,5s,7s)-adamantan-1-yl)amino)octyl)-2-methyl-4-oxoquinazolin-3(4H)-yl)piperidine-2,6-dione |
| | 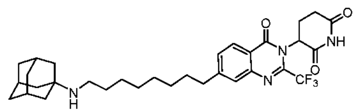 | 3-(7-(8-(((3s,5s,7s)-adamantan-1-yl)amino)octyl)-4-oxo-2-(trifluoromethyl)quinazolin-3(4H)-yl)piperidine-2,6-dione | 3-(7-(8-(((3s,5s,7s)-adamantan-1-yl)amino)octyl)-4-oxo-2-(trifluoromethyl)quinazolin-3(4H)-yl)piperidine-2,6-dione |
| | 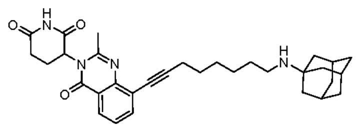 | 3-(8-(8-(((3s,5s,7s)-adamantan-1-yl)amino)oct-1-yn-1-yl)-2-methyl-4-oxoquinazolin-3 (4H)-yl)piperidine-2,6-dione | 3-(8-(8-(((3s,5s,7s)-adamantan-1-yl)amino)oct-1-yn-1-yl)-2-methyl-4-oxoquinazolin-3 (4H)-yl)piperidine-2,6-dione |
| | 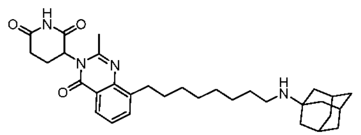 | 3-(8-(8-(((3s,5s,7s)-adamantan-1-yl)amino)octyl)-2-methyl-4-oxoquinazolin-3(4H)-yl)piperidine-2,6-dione | 3-(8-(8-(((3s,5s,7s)-adamantan-1-yl)amino)octyl)-2-methyl-4-oxoquinazolin-3(4H)-yl)piperidine-2,6-dione |
| | 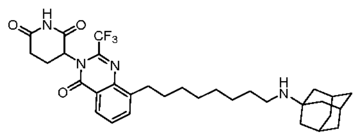 | 3-(8-(8-(((3s,5s,7s)-adamantan-1-yl)amino)octyl)-4-oxo-2-(trifluoromethyl)quinazolin-3(4H)-yl)piperidine-2,6-dione | 3-(8-(8-(((3s,5s,7s)-adamantan-1-yl)amino)octyl)-4-oxo-2-(trifluoromethyl)quinazolin-3(4H)-yl)piperidine-2,6-dione |
| | 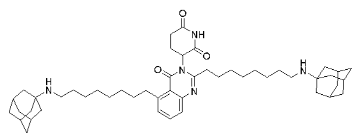 | 3-(5-(8-(((1s,3s)-adamantan-1-yl)amino)octyl)-2-(8-(((3s,5s,7s)-adamantan-1-yl)amino)octyl)-4-oxoquinazolin-3 (4H)-yl)piperidine-2,6-dione | 3-(5-(8-(((1s,3s)-adamantan-1-yl)amino)octyl)-2-(8-(((3s,5s,7s)-adamantan-1-yl)amino)octyl)-4-oxoquinazolin-3 (4H)-yl)piperidine-2,6-dione |

(continued)

| Compound No. | Structure of the compounds | The compounds' name in English | Chinese translation of the compounds' name |
|---|---|---|---|
| GT-02323 | 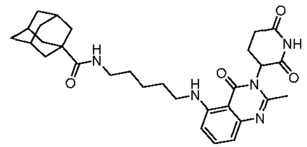 | (3r,5r,7r)-N-(5-((3-(2,6-dioxopiperidin-3-yl)-2-methyl-4-oxo-3,4-dihydroquinazolin-5-yl)amino)pentyl)adamantane-1-carboxamide | (3r,5r,7r)-N-(5-((3-(2,6-dioxopiperidin-3-yl)-2-methyl-4-oxo-3,4-dihydroquinazolin-5-yl)amino)pentyl)adamantane-1-carboxamide |
| | 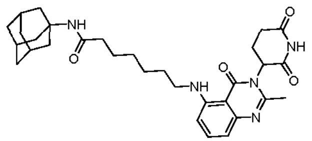 | N-((1s,3s)-adamantan-1-yl)-7-((3 -(2,6-dioxopiperidin-3 -yl)-2-methyl-4-oxo-3,4-dihydroquinazolin-5-yl)amino) heptanamide | N-((1s,3s)-adamantan-1-yl)-7-((3-(2,6-dioxopiperidin-3-yl)-2-methyl-4-oxo-3,4-dihydroquinazolin-5-yl)amino) heptanamide |
| | 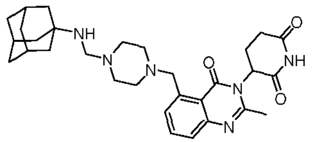 | 3-(5-((4-((((1s,3s)-adamantan-1-yl)amino)methyl)piperazin-1 -yl)methyl)-2-methyl-4-oxoquinazolin-3 (4H)-yl)piperidine-2,6-dione | 3-(5-((4-((((1s,3s)-adamantan-1-yl)amino)methyl)piperazin-1-yl)methyl)-2-methyl-4-oxoquinazolin-3(4H)-yl)piperidine-2,6-dione |
| | 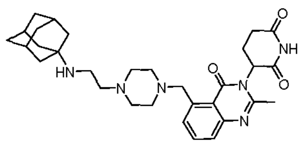 | 3-(5-((4-(2-(((3s,5s,7s)-adamantan-1-yl)amino)ethyl)piperazin-1-yl)methyl)-2-methyl-4-oxoquinazolin-3(4H)-yl) piperidine-2,6-dione | 3-(5-((4-(2-(((3s,5s,7s)-adamantan-1-yl)amino)ethyl)piperazin-1-yl)methyl)-2-methyl-4-oxoquinazolin-3(4H)-yl) piperidine-2,6-dione |
| GT-03226 | 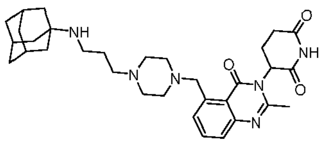 | 3-(5-((4-(3-(((1s,3s)-adamantan-1-yl)amino)propyl)piperazin-1-yl)methyl)-2-methyl-4-oxoquinazolin-3(4H)-yl)piperidine-2,6-dione | 3-(5-((4-(3-(((1s,3s)-adamantan-1-yl)amino)propyl)piperazin-1-yl)methyl)-2-methyl-4-oxoquinazolin-3(4H)-yl)piperidine-2,6-dione |
| | 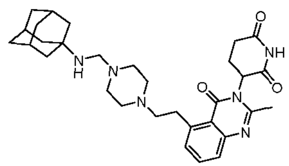 | 3-(5-(2-(4-((((3s,5s,7s)-adamantan-1-yl)amino)methyl)piperazin-1-yl)ethyl)-2-methyl-4-oxoquinazolin-3(4H)-yl)piperidine-2,6-dione | 3-(5-(2-(4-((((3s,5s,7s)-adamantan-1-yl)amino)methyl)piperazin-1-yl)ethyl)-2-methyl-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| | 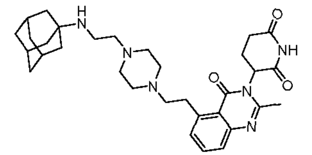 | 3-(5-(2-(4-(2-(((1s,3s)-adamantan-1-yl)amino)ethyl)piperazin-1-yl)ethyl)-2-methyl-4-oxoquinazolin-3 (4H)-yl) piperidine-2,6-dione | 3-(5-(2-(4-(2-(((1s,3s)-adamantan-1-yl)amino)ethyl)piperazin-1-yl)ethyl)-2-methyl-4-oxoquinazolin-3 (4H)-yl)piperidine-2,6-dione |
| | 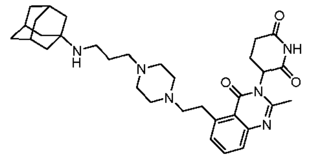 | 3-(5-(2-(4-(3-(((3s,5s,7s)-adamantan-1-yl)amino)propyl) piperazin-1-yl)ethyl)-2-methyl-4-oxoquinazolin-3 (4H)-yl) piperidine-2,6-dione | 3-(5-(2-(4-(3-(((3s,5s,7s)-adamantan-1-yl)amino)propyl) piperazin-1-yl)ethyl)-2-methyl-4-oxoquinazolin-3 (4H)-yl) piperidine-2,6-dione |
| | 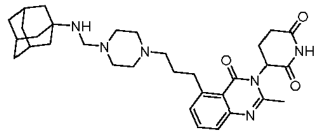 | 3-(5-(3-(4-((((1s,3s)-adamantan-1-yl)amino)methyl)piperazin-1-yl)propyl)-2-methyl-4-oxoquinazolin-3 (4H)-yl) piperidine-2,6-dione | 3-(5-(3-(4-((((1s,3s)-adamantan-1-yl)amino)methyl)piperazin-1-yl)propyl)-2-methyl-4-oxoquinazolin-3 (4H)-yl)piperidine-2,6-dione |
| | 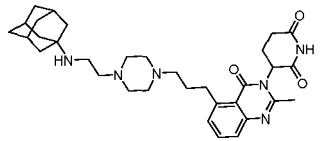 | 3-(5-(3-(4-(2-(((3s,5s,7s)-adamantan-1-yl)amino)ethyl)piperazin-1-yl)propyl)-2-methyl-4-oxoquinazolin-3 (4H)-yl) piperidine-2,6-dione | 3-(5-(3-(4-(2-(((3s,5s,7s)-adamantan-1-yl)amino)ethyl)piperazin-1-yl)propyl)-2-methyl-4-oxoquinazolin-3 (4H)-yl) piperidine-2,6-dione |
| | 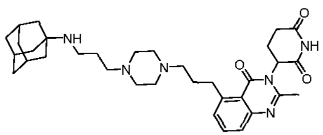 | 3-(5-(3-(4-(3-(((1s,3s)-adamantan-1-yl)amino)propyl)piperazin-1-yl)propyl)-2-methyl-4-oxoquinazolin-3 (4H)-yl) piperidine-2,6-dione | 3-(5-(3-(4-(3-(((1s,3s)-adamantan-1-yl)amino)propyl)piperazin-1-yl)propyl)-2-methyl-4-oxoquinazolin-3 (4H)-yl) piperidine-2,6-dione |
| GT-03227 | 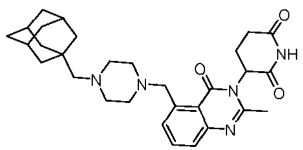 | 3-(5-((4-(((3r,5r,7r)-adamantan-1-yl)methyl)piperazin-1-yl)methyl)-2-methyl-4-oxoquinazolin-3 (4H)-yl)piperidine-2,6-dione | 3-(5-((4-(((3r,5r,7r)-adamantan-1-yl)methyl)piperazin-1-yl)methyl)-2-methyl-4-oxoquinazolin-3 (4H)-yl)piperidine-2,6-dione |
| GT-03228 | 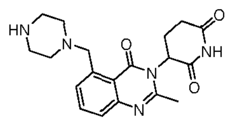 | 3-(2-methyl-4-oxo-5-(piperazin-1-ylmethyl)quinazolin-3 (4H)-yl)piperidine-2,6-dione | 3-(2-methyl-4-oxo-5-(piperazin-1-ylmethyl)quinazolin-3(4H)-yl)piperidine-2,6-dione |

(continued)

| Compound No. | Structure of the compounds | The compounds' name in English | Chinese translation of the compounds' name |
|---|---|---|---|
| GT-03229 | 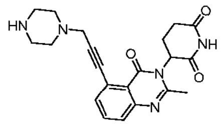 | 3-(2-methyl-4-oxo-5-(3-(piperazin-1-yl)prop-1-yn-1-yl) quinazolin-3(4H)-yl)piperidine-2,6-dione | 3-(2-methyl-4-oxo-5-(3-(piperazin-1-yl)prop-1-yn-1-yl) quinazolin-3 (4H)-yl)piperidine-2,6-dione |
| GT-03258 | 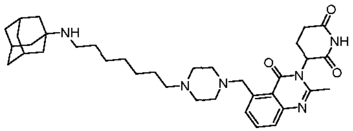 | 3-(5-((4-(7-(((1s,3s)-adamantan-1-yl)amino)heptyl)piperazin-1-yl)methyl)-2-methyl-4-oxoquinazolin-3 (4H)-yl)piperidine-2,6-dione | 3-(5-((4-(7-(((1s,3s)-adamantan-1-yl)amino)heptyl)piperazin-1-yl)methyl)-2-methyl-4-oxoquinazolin-3(4H)-yl)piperidine-2,6-dione |
| | 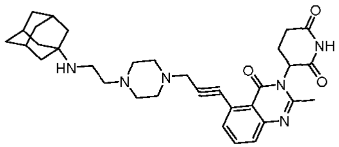 | 3-(5-(3-(4-(2-(((3s,5s,7s)-adamantan-1-yl)amino)ethyl)piperazin-1-yl)prop-1-yn-1-yl)-2-methyl-4-oxoquinazolin-3 (4H)-yl)piperidine-2,6-dione | 3-(5-(3-(4-(2-(((3s,5s,7s)-adamantan-1-yl)amino)ethyl)piperazin-1-yl)prop-1-yn-1-yl)-2-methyl-4-oxoquinazolin-3 (4H)-yl)piperidine-2,6-dione |
| | 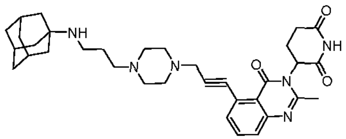 | 3-(5-(3-(4-(3-(((1s,3s)-adamantan-1-yl)amino)propyl)piperazin-1-yl)prop-1-yn-1-yl)-2-methyl-4-oxoquinazolin-3 (4H)-yl)piperidine-2,6-dione | 3-(5-(3-(4-(3-(((1s,3s)-adamantan-1-yl)amino)propyl)piperazin-1-yl)prop-1-yn-1-yl)-2-methyl-4-oxoquinazolin-3 (4H)-yl)piperidine-2,6-dione |
| GT-03231 | 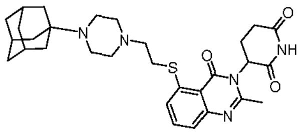 | 3-(5-((2-(4-((1s,3s)-adamantan-1-yl)piperazin-1-yl)ethyl) thio)-2-methyl-4-oxoquinazolin-3 (4H)-yl)piperidine-2,6-dione | 3-(5-((2-(4-((1s,3s)-adamantan-1-yl)piperazin-1-yl)ethyl) thio)-2-methyl-4-oxoquinazolin-3 (4H)-yl)piperidine-2,6-dione |
| GT-03232 | 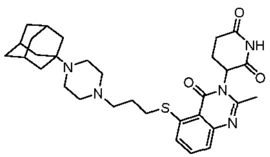 | 3-(5-((3-(4-((3s,5s,7s)-adamantan-1-yl)piperazin-1-yl) propyl)thio)-2-methyl-4-oxoquinazolin-3 (4H)-yl)piperidine-2,6-dione | 3-(5-((3-(4-((3s,5s,7s)-adamantan-1-yl)piperazin-1-yl) propyl)thio)-2-methyl-4-oxoquinazolin-3 (4H)-yl)piperidine-2,6-dione |
| GT-03284 | 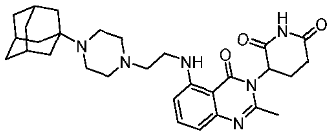 | 3-(5-((2-(4-((1s,3s)-adamantan-1-yl)piperazin-1-yl)ethyl) amino)-2-methyl-4-oxoquinazolin-3 (4H)-yl)piperidine-2,6-dione | 3-(5-((2-(4-((1s,3s)-adamantan-1-yl)piperazin-1-yl)ethyl) amino)-2-methyl-4-oxoquinazolin-3 (4H)-yl)piperidine-2,6-dione |

(continued)

| Compou nd No. | Structure of the compounds | The compounds' name in English | Chinese translation of the compounds' name |
|---|---|---|---|
| | 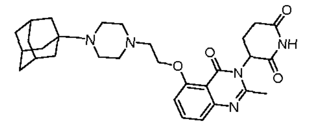 | 3-(5-(2-(4-((1s,3s)-adamantan-1-yl)piperazin-1-yl) ethoxy)-2-methyl-4-oxoquinazolin-3(4H)-yl)piperidine-2,6-dione | 3-(5-(2-(4-((1s,3s)-adamantan-1-yl)piperazin-1-yl) ethoxy)-2-methyl-4-oxoquinazolin-3(4H)-yl)piperi-dine-2,6-dione |
| | 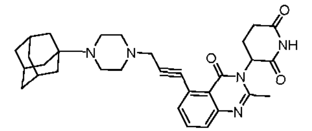 | 3-(5-(3-(4-((1s,3s)-adamantan-1-yl)piperazin-1-yl) prop-1-yn-1-yl)-2-methyl-4-oxoquinazolin-3(4H)-yl)piperidine-2,6-dione | 3-(5-(3-(4-((1s,3s)-adamantan-1-yl)piperazin-1-yl) prop-1-yn-1-yl)-2-methyl-4-oxoquinazolin-3(4H)-yl)piperi-dine-2,6-dione |
| | 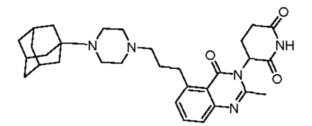 | 3-(5-(3-(4-((1s,3s)-adamantan-1-yl)piperazin-1-yl)propyl)-2-methyl-4-oxoquinazolin-3(4H)-yl)piperidine-2,6-dione | 3-(5-(3-(4-((1s,3s)-adamantan-1-yl)piperazin-1-yl)propyl)-2-methyl-4-oxoquinazolin-3(4H)-yl)piperidine-2,6-dione |
| GT-03259 | 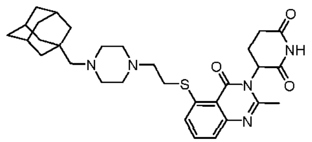 | 3-(5-((2-(4-(((3r,5r,7r)-adamantan-1-yl)methyl)piperazin-1-yl)ethyl)thio)-2-methyl-4-oxoquinazolin-3(4H)-yl)piperidine-2,6-dione | 3-(5-((2-(4-(((3r,5r,7r)-adamantan-1-yl)methyl)piperazin-1-yl)ethyl)thio)-2-methyl-4-oxoquinazolin-3(4H)-yl)piperidine-2,6-dione |
| GT-03235 | 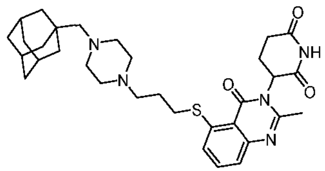 | 3-(5-((3-(4-(((1s,3s)-adamantan-1-yl)methyl)piperazin-1-yl)propyl)thio)-2-methyl-4-oxoquinazolin-3(4H)-yl)piperidine-2,6-dione | 3-(5-((3-(4-(((1s,3s)-adamantan-1-yl)methyl)piperazin-1-yl)propyl)thio)-2-methyl-4-oxoquinazolin-3(4H)-yl)piperi-dine-2,6-dione |
| | 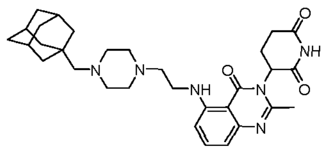 | 3-(5-((2-(4-(((3r,5r,7r)-adamantan-1-yl)methyl)piperazin-1-yl)ethyl)amino)-2-methyl-4-oxoquinazolin-3(4H)-yl) piperidine-2,6-dione | 3-(5-((2-(4-(((3r,5r,7r)-adamantan-1-yl)methyl)piperazin-1-yl)ethyl)amino)-2-methyl-4-oxoquinazolin-3(4H)-yl) piperidine-2,6-dione |

(continued)

| Compound No. | Structure of the compounds | The compounds' name in English | Chinese translation of the compounds' name |
|---|---|---|---|
| | 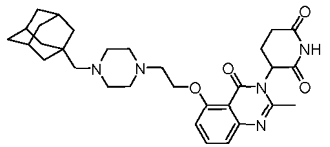 | 3-(5-(2-(4-(((3r,5r,7r)-adamantan-1-yl)methyl)piperazin-1-yl)ethoxy)-2-methyl-4-oxoquinazolin-3(4H)-yl)piperidine-2,6-dione | 3-(5-(2-(4-(((3r,5r,7r)-adamantan-1-yl)methyl)piperazin-1-yl)ethoxy)-2-methyl-4-oxoquinazolin-3(4H)-yl)piperidine-2,6-dione |
| GT-03302 | 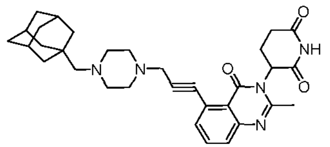 | 3-(5-(3-(4-(((3r,5r,7r)-adamantan-1-yl)methyl)piperazin-1-yl)prop-1-yn-1-yl)-2-methyl-4-oxoquinazolin-3(4H)-yl)piperidine-2,6-dione | 3-(5-(3-(4-(((3r,5r,7r)-adamantan-1-yl)methyl)piperazin-1-yl)prop-1-yn-1-yl)-2-methyl-4-oxoquinazolin-3 (4H)-yl)piperidine-2,6-dione |
| | 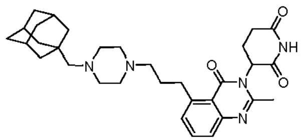 | 3-(5-(3-(4-(((3r,5r,7r)-adamantan-1-yl)methyl)piperazin-1-yl)propyl)-2-methyl-4-oxoquinazolin-3(4H)-yl)piperidine-2,6-dione | 3-(5-(3-(4-(((3r,5r,7r)-adamantan-1-yl)methyl)piperazin-1-yl)propyl)-2-methyl-4-oxoquinazolin-3(4H)-yl)piperidine-2,6-dione |
| GT-03305 | 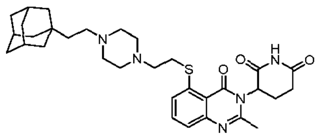 | 3-(5-((2-(4-(2-((3r,5r,7r)-adamantan-1-yl)ethyl)piperazin-1-yl)ethyl)thio)-2-methyl-4-oxoquinazolin-3(4H)-yl)piperidine-2,6-dione | 3-(5-((2-(4-(2-((3r,5r,7r)-adamantan-1-yl)ethyl)piperazin-1-yl)ethyl)thio)-2-methyl-4-oxoquinazolin-3(4H)-yl)piperidine-2,6-dione |
| GT-03301 | 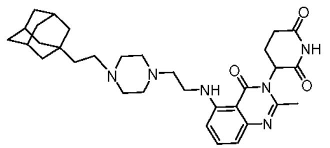 | 3-(5-((2-(4-(2-((3r,5r,7r)-adamantan-1-yl)ethyl)piperazin-1-yl)ethyl)amino)-2-methyl-4-oxoquinazolin-3(4H)-yl)piperidine-2,6-dione | 3-(5-((2-(4-(2-((3r,5r,7r)-adamantan-1-yl)ethyl)piperazin-1-yl)ethyl)amino)-2-methyl-4-oxoquinazolin-3(4H)-yl)piperidine-2,6-dione |
| | 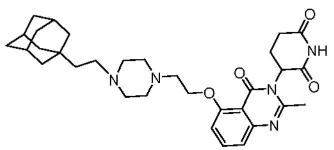 | 3-(5-(2-(4-(2-((3r,5r,7r)-adamantan-1-yl)ethyl)piperazin-1-yl)ethoxy)-2-methyl-4-oxoquinazolin-3(4H)-yl)piperidine-2,6-dione | 3-(5-(2-(4-(2-((3r,5r,7r)-adamantan-1-yl)ethyl)piperazin-1-yl)ethoxy)-2-methyl-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| | 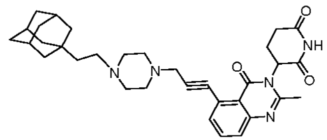 | 3-(5-(3-(4-(2-((3r,5r,7r)-adamantan-1-yl)ethyl)piperazin-1-yl)prop-1-yn-1-yl)-2-methyl-4-oxoquinazolin-3(4H)-yl)piperidine-2,6-dione | 3-(5-(3 -(4-(2-((3r,5r,7r)-adamantan-1-yl)ethyl)piperazin-1-yl)prop-1-yn-1-yl)-2-methyl-4-oxoquinazolin-3(4H)-yl)piperidine-2,6-dione |
| | 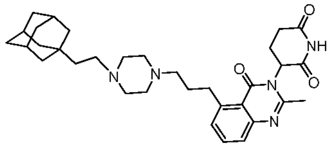 | 3-(5-(3-(4-(2-((3r,5r,7r)-adamantan-1-yl)ethyl)piperazin-1-yl)propyl)-2-methyl-4-oxoquinazolin-3(4H)-yl)piperidine-2,6-dione | 3-(5-(3-(4-(2-((3r,5r,7r)-adamantan-1-yl)ethyl)piperazin-1-yl)propyl)-2-methyl-4-oxoquinazolin-3(4H)-yl)piperidine-2,6-dione |
| GT-03230 | 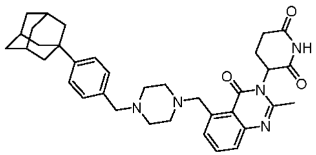 | 3-(5-((4-(4-((3r,5r,7r)-adamantan-1-yl)benzyl)piperazin-1-yl)methyl)-2-methyl-4-oxoquinazolin-3(4H)-yl)piperidine-2,6-dione | 3-(5-((4-(4-((3r,5r,7r)-adamantan-1-yl)benzyl)piperazin-1-yl)methyl)-2-methyl-4-oxoquinazolin-3(4H)-yl)piperidine-2,6-dione |
| | 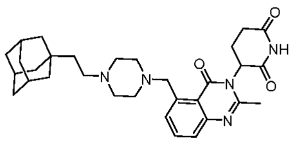 | 3-(5-((4-(2-((1s,3s)-adamantan-1-yl)ethyl)piperazin-1-yl)methyl)-2-methyl-4-oxoquinazolin-3(4H)-yl)piperidine-2,6-dione | 3-(5-((4-(2-((1s,3s)-adamantan-1-yl)ethyl)piperazin-1-yl)methyl)-2-methyl-4-oxoquinazolin-3(4H)-yl)piperidine-2,6-dione |
| GT-03233 | 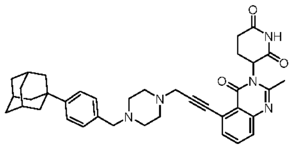 | 3-(5-(3-(4-(4-((3r,5r,7r)-adamantan-1-yl)benzyl)piperazin-1-yl)prop-1-yn-1-yl)-2-methyl-4-oxoquinazolin-3(4H)-yl)piperidine-2,6-dione | 3-(5-(3-(4-(4-((3r,5r,7r)-adamantan-1-yl)benzyl)piperazin-1-yl)prop-1-yn-1-yl)-2-methyl-4-oxoquinazolin-3(4H)-yl)piperidine-2,6-dione |
| | 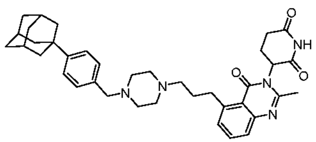 | 3-(5-(3-(4-(4-((3r,5r,7r)-adamantan-1-yl)benzyl)piperazin-1-yl)propyl)-2-methyl-4-oxoquinazolin-3(4H)-yl)piperidine-2,6-dione | 3-(5-(3-(4-(4-((3r,5r,7r)-adamantan-1-yl)benzyl)piperazin-1-yl)propyl)-2-methyl-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| | 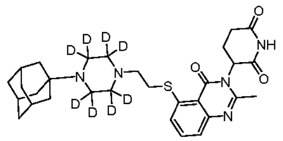 | 3-(5-((2-(4-((1s,3s)-adamantan-1-yl)piperazin-1-yl-2,2,3,3,5,5,6,6-d8)ethyl)thio)-2-methyl-4-oxoquinazolin-3(4H)-yl)piperidine-2,6-dione | 3-(5-((2-(4-((1s,3s)-adamantan-1-yl)piperazin-1-yl-2,2,3,3,5,5,6,6-d8)ethyl)thio)-2-methyl-4-oxoquinazolin-3(4H)-yl)piperidine-2,6-dione |
| | 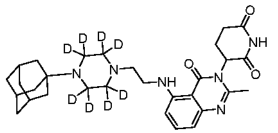 | 3-(5-((2-(4-((1s,3s)-adamantan-1-yl)piperazin-1-yl-2,2,3,3,5,5,6,6-d8)ethyl)amino)-2-methyl-4-oxoquinazolin-3 (4H)-yl)piperidine-2,6-dione | 3-(5-((2-(4-((1s,3s)-adamantan-1-yl)piperazin-1-yl-2,2,3,3,5,5,6,6-d8)ethyl)amino)-2-methyl-4-oxoquinazolin-3 (4H)-yl)piperidine-2,6-dione |
| | 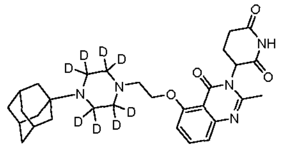 | 3-(5-(2-(4-((1s,3s)-adamantan-1-yl)piperazin-1-yl-2,2,3,3,5,5,6,6-d8)ethoxy)-2-methyl-4-oxoquinazolin-3 (4H)-yl)piperidine-2,6-dione | 3-(5-(2-(4-((1s,3s)-adamantan-1-yl)piperazin-1-yl-2,2,3,3,5,5,6,6-d8)ethoxy)-2-methyl-4-oxoquinazolin-3(4H)-yl)piperidine-2,6-dione |
| | 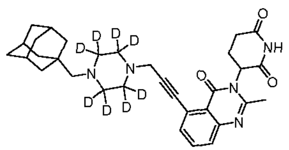 | 3-(5-(3-(4-(((3r,5r,7r)-adamantan-1-yl)methyl)piperazin-1-yl-2,2,3,3,5,5,6,6-d8)prop-1-yn-1-yl)-2-methyl-4-oxoquinazolin-3(4H)-yl)piperidine-2,6-dione | 3-(5-(3-(4-(((3r,5r,7r)-adamantan-1-yl)methyl)piperazin-1-yl-2,2,3,3,5,5,6,6-d8)prop-1-yn-1-yl)-2-methyl-4-oxoquinazolin-3(4H)-yl)piperidine-2,6-dione |
| | 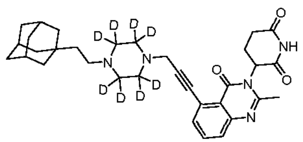 | 3-(5-(3-(4-(2-((1s,3s)-adamantan-1-yl)ethyl)piperazin-1-yl-2,2,3,3,5,5,6,6-d8)prop-1-yn-1-yl)-2-methyl-4-oxoquinazolin-3 (4H)-yl)piperidine-2,6-dione | 3-(5-(3-(4-(2-((1s,3s)-adamantan-1-yl)ethyl)piperazin-1-yl-2,2,3,3,5,5,6,6-d8)prop-1-yn-1-yl)-2-methyl-4-oxoquinazolin-3 (4H)-yl)piperidine-2,6-dione |
| | 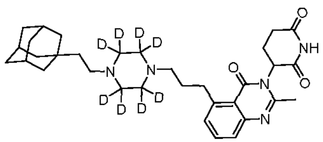 | 3-(5-(3-(4-(2-((1s,3s)-adamantan-1-yl)ethyl)piperazin-1-yl-2,2,3,3,5,5,6,6-d8)propyl)-2-methyl-4-oxoquinazolin-3 (4H)-yl)piperidine-2,6-dione | 3-(5-(3-(4-(2-((1s,3s)-adamantan-1-yl)ethyl)piperazin-1-yl-2,2,3,3,5,5,6,6-d8)propyl)-2-methyl-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| | 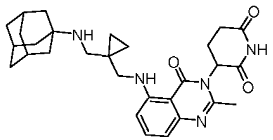 | 3-(5-(((1-((((1s,3s)-adamantan-1-yl)amino)methyl)cyclopropyl) methyl)amino)-2-methyl-4-oxoquinazolin-3(4H)-yl)piperidine-2,6-dione | 3-(5-(((1-((((1s,3s)-adamantan-1-yl)amino)methyl)cyclopropyl) methyl)amino)-2-methyl-4-oxoquinazolin-3(4H)-yl)piperidine-2,6-dione |
| | 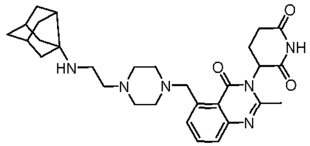 | 3-(5-((4-(2-(((2R,3as,5S,6as)-hexahydro-2,5-methanopentalen-3a(1H)-yl)amino)ethyl)piperazin-1-yl)methyl)-2-methyl-4-oxoquinazolin-3 (4H)-yl)piperidine-2,6-dione | 3-(5-((4-(2-(((2R,3as,5S,6as)-hexahydro-2,5-methanopentalen-3a(1H)-yl)amino)ethyl)piperazin-1-yl)methyl)-2-methyl-4-oxoquinazolin-3 (4H)-yl)piperidine-2,6-dione |
| | 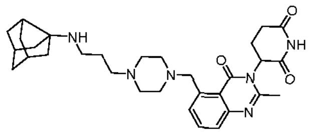 | 3-(5-((4-(3-(((3as,6as)-hexahydro-2,5-methanopentalen-3a(1H)-yl)amino)propyl)piperazin-1-yl)methyl)-2-methyl-4-oxoquinazolin-3 (4H)-yl)piperidine-2,6-dione | 3-(5-((4-(3-(((3as,6as)-hexahydro-2,5-methanopentalen-3a(1H)-yl)amino)propyl)piperazin-1-yl)methyl)-2-methyl-4-oxoquinazolin-3 (4H)-yl)piperidine-2,6-dione |
| | 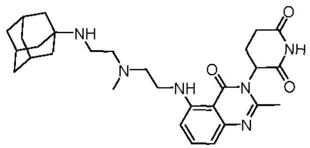 | 3-(5-((2-((2-(((1s,3s)-adamantan-1-yl)amino)ethyl) (methyl)amino )ethyl)amino)-2-methyl-4-oxoquinazolin-3(4H)-yl)piperidine-2,6-dione | 3-(5-((2-((2-(((1s,3s)-adamantan-1-yl)amino)ethyl) (methyl)amino )ethyl)amino)-2-methyl-4-oxoquinazolin-3(4H)-yl)piperidine-2,6-dione |
| | 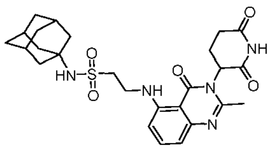 | N-((3s,5s,7s)-adamantan-1-yl)-2-((3-(2,6-dioxopiperidin-3-yl)-2-methyl-4-oxo-3,4-dihydroquinazolin-5-yl)amino)ethane-1-sulfonamide | N-((3s,5s,7s)-adamantan-1-yl)-2-((3-(2,6-dioxopiperidin-3-yl)-2-methyl-4-oxo-3,4-dihydroquinazolin-5-yl)amino)ethane-1-sulfonamide |
| | 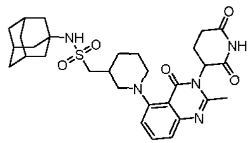 | N-((1s,3s)-adamantan-1-yl)-1-(1-(3-(2,6-dioxopiperidin-3-yl)-2-methyl-4-oxo-3,4-dihydroquinazolin-5-yl)piperidin-3-yl)methanesulfonamide | N-((1s,3s)-adamantan-1-yl)-1-(1-(3-(2,6-dioxopiperidin-3-yl)-2-methyl-4-oxo-3,4-dihydroquinazolin-5-yl)piperidin-3-yl)methanesulfonamide |

(continued)

| Compound No. | Structure of the compounds | The compounds' name in English | Chinese translation of the compounds' name |
|---|---|---|---|
| | 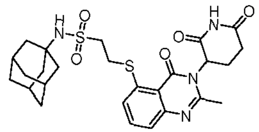 | N-((3s,5s,7s)-adamantan-1-yl)-2-((3-(2,6-dioxopiperidin-3-yl)-2-methyl-4-oxo-3,4-dihydroquinazolin-5-yl)thio)ethane-1-sulfonamide | N-((3s,5s,7s)-adamantan-1-yl)-2-((3-(2,6-dioxopiperidin-3-yl)-2-methyl-4-oxo-3,4-dihydroquinazolin-5-yl)thio)ethane-1-sulfonamide |
| | 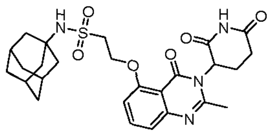 | N-((3s,5s,7s)-adamantan-1-yl)-2-((3-(2,6-dioxopiperidin-3-yl)-2-methyl-4-oxo-3,4-dihydroquinazolin-5-yl)oxy)ethane-1-sulfonamide | N-((3s,5s,7s)-adamantan-1-yl)-2-((3-(2,6-dioxopiperidin-3-yl)-2-methyl-4-oxo-3,4-dihydroquinazolin-5-yl)oxy)ethane-1-sulfonamide |
| | 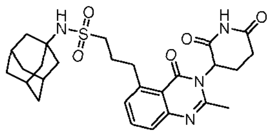 | N-((3s,5s,7s)-adamantan-1-yl)-3-(3-(2,6-dioxopiperidin-3-yl)-2-methyl-4-oxo-3,4-dihydroquinazolin-5-yl)propane-1-sulfonamide | N-((3s,5s,7s)-adamantan-1-yl)-3-(3-(2,6-dioxopiperidin-3-yl)-2-methyl-4-oxo-3,4-dihydroquinazolin-5-vl)propane-1-sulfonamide |
| | 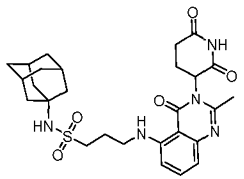 | N-((3s,5s,7s)-adamantan-1-yl)-3-((3-(2,6-dioxopiperidin-3-yl)-2-methyl-4-oxo-3,4-dihydroquinazolin-5-yl)amino)propane-1-sulfonamide | N-((3s,5s,7s)-adamantan-1-yl)-3-((3-(2,6-dioxopiperidin-3-yl)-2-methyl-4-oxo-3,4-dihydroquinazolin-5-yl)amino)propane-1-sulfonamide |
| | 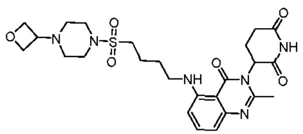 | 3-(2-methyl-5-((4-((4-(oxetan-3-yl)piperazin-1-yl)sulfonyl)butyl)amino)-4-oxoquinazolin-3(4H)-yl)piperidine-2,6-dione | 3-(2-methyl-5-((4-((4-(oxetan-3-yl)piperazin-1-yl)sulfonyl)butyl)amino)-4-oxoquinazolin-3(4H)-yl)piperidine-2,6-dione |
| | 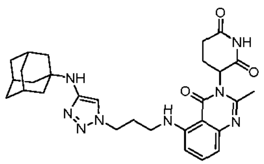 | 3-(5-((3-(4-(((3s,5s,7s)-adamantan-1-yl)amino)-1H-1,2,3-triazol-1-yl)propyl)amino)-2-methyl-4-oxoquinazolin-3(4H)-yl)piperidine-2,6-dione | 3-(5-((3-(4-(((3s,5s,7s)-adamantan-1-yl)amino)-1H-1,2,3-triazol-1-yl)propyl)amino)-2-methyl-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| GT-03388 | 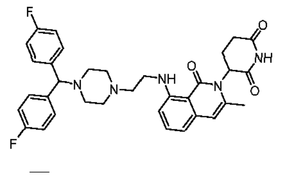 | 3-(8-((2-(4-(bis(4-fluorophenyl)methyl)piperazin -1-yl) ethyl)amino)-3-methyl-1-oxoisoquinolin-2(1H)-yl)piperidine-2,6-dione | 3-(8-((2-(4-(bis(4-fluorophenyl)methyl)piperazin -1-yl) ethyl)amino)-3-methyl-1-oxoisoquinolin-2(1H)-yl)piperidine-2,6-dione |
| GT-03306 | 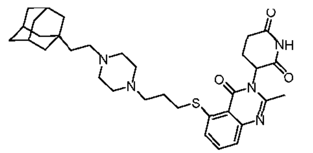 | 3-(5-((3-(4-(2-((3r,5r,7r)-adamantan-1-yl)ethyl)piperazin-1-yl)propyl)thio)-2-methyl-4-oxoquinazolin-3 (4H)-yl) piperidine-2,6-dione | 3-(5-((3-(4-(2-((3r,5r,7r)-adamantan-1-yl)ethyl)piperazin-1-yl)propyl)thio)-2-methyl-4-oxoquinazolin-3 (4H)-yl) piperidine-2,6-dione |
| GT-03384 | 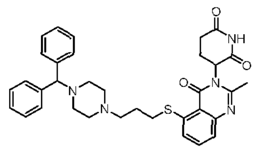 | 3-(5-((3-(4-benzhydrylpiperazin-1-yl)propyl)thio)-2-methyl-4-oxoquinazolin-3(4H)-yl)piperidine-2,6-dione | 3-(5-((3-(4-benzhydrylpiperazin-1-yl)propyl)thio)-2-methyl-4-oxoquinazolin-3(4H)-yl)piperidine-2,6-dione |
| GT-03387 | 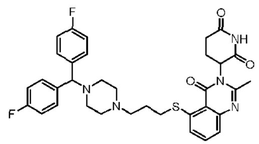 | 3-(5-((3-(4-(bis(4-fluorophenyl)methyl)piperazin -1-yl) propyl)thio)-2-methyl-4-oxoquinazolin-3(4H)-yl)piperidine-2,6-dione | 3-(5-((3-(4-(bis(4-fluorophenyl)methyl)piperazin -1-yl) propyl)thio)-2-methyl-4-oxoquinazolin-3(4H)-yl)piperidine-2,6-dione |
| GT-03697 | 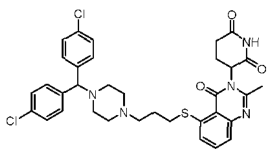 | 3-(5-((3-(4-(bis(4-chlorophenyl)methyl)piperazin -1-yl) propyl)thio)-2-methyl-4-oxoquinazolin-3(4H)-yl)piperidine-2,6-dione | 3-(5-((3-(4-(bis(4-chlorophenyl)methyl)piperazin -1-yl) propyl)thio)-2-methyl-4-oxoquinazolin-3(4H)-yl)piperidine-2,6-dione |
| | 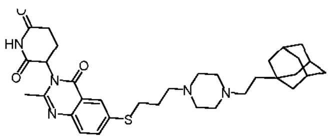 | 3-(6-((3-(4-(2-((3r,5r,7r)-adamantan-1-yl)ethyl)piperazin-1-yl)propyl)thio)-2-methyl-4-oxoquinazolin-3(4H)-yl) piperidine-2,6-dione | 3-(6-((3-(4-(2-((3r,5r,7r)-adamantan-1-yl)ethyl)piperazin-1-yl)propyl)thio)-2-methyl-4-oxoquinazolin-3(4H)-yl) piperidine-2,6-dione |
| | 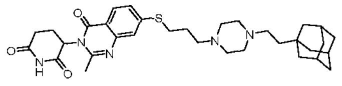 | 3-(7-((3-(4-(2-((3r,5r,7r)-adamantan-1-yl)ethyl)piperazin-1-yl)propyl)thio)-2-methyl-4-oxoquinazolin-3 (4H)-yl) piperidine-2,6-dione | 3-(7-((3-(4-(2-((3r,5r,7r)-adamantan-1-yl)ethyl)piperazin-1-yl)propyl)thio)-2-methyl-4-oxoquinazolin-3 (4H)-yl) piperidine-2,6-dione |

(continued)

| Compound No. | Structure of the compounds | The compounds' name in English | Chinese translation of the compounds' name |
|---|---|---|---|
| | 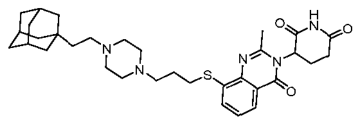 | 3-(8-((3-(4-(2-((3r,5r,7r)-adamantan-1-yl)ethyl)piperazin-1-yl)propyl)thio)-2-methyl-4-oxoquinazolin-3 (4H)-yl) piperidine-2,6-dione | 3-(8-((3-(4-(2-((3r,5r,7r)-adamantan-1-yl)ethyl)piperazin-1-yl)propyl)thio)-2-methyl-4-oxoquinazolin-3 (4H)-yl) piperidine-2,6-dione |
| | 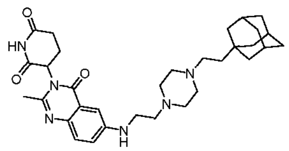 | 3 -(6-((2-(4-(2-((3r,5r,7r)-adamantan-1-yl)ethyl)piperazin-1-yl)ethyl)amino)-2-methyl-4-oxoquinazolin-3 (4H)-yl) piperidine-2,6-dione | 3-(6-((2-(4-(2-((3r,5r,7r)-adamantan-1-yl)ethyl)piperazin-1-yl)ethyl)amino)-2-methyl-4-oxoquinazolin-3 (4H)-yl) piperidine-2,6-dione |
| | 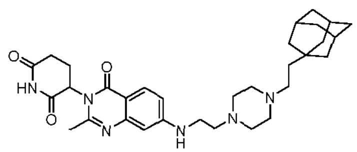 | 3-(7-((2-(4-(2-((3r,5r,7r)-adamantan-1-yl)ethyl)piperazin-1-yl)ethyl)amino)-2-methyl-4-oxoquinazolin-3(4H)-yl) piperidine-2,6-dione | 3-(7-((2-(4-(2-((3r,5r,7r)-adamantan-1-yl)ethyl)piperazin-1-yl)ethyl)amino)-2-methyl-4-oxoquinazolin-3(4H)-yl) piperidine-2,6-dione |
| | 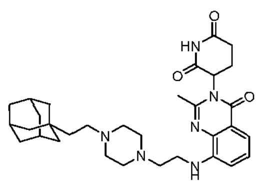 | 3-(8-((2-(4-(2-((3r,5r, 7r)-adamantan-1-yl)ethyl)piperazin-1-yl)ethyl)amino)-2-methyl-4-oxoquinazolin-3(4H)-yl) piperidine-2,6-dione | 3-(8-((2-(4-(2-((3r,5r,7r)-adamantan-1-yl)ethyl)piperazin-1-yl)ethyl)amino)-2-methyl-4-oxoquinazolin-3(4H)-yl) piperidine-2,6-dione |
| GT-04231 | 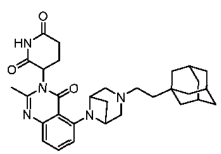 | 3-(5-(3-(2-((3r,5r,7r)-adamantan-1-yl)ethyl)-3,6-diazabicyclo[3.1.1]heptan-6-yl)-2-methyl-4-oxoquinazolin-3(4H)-yl)piperidine-2,6-dione | 3-(5-(3-(2-((3r,5r,7r)-adamantan-1-yl)ethyl)-3,6-diazabicyclo[3.1.1]heptan-6-yl)-2-methyl-4-oxoquinazolin-3(4H)-yl)piperidine-2,6-dione |
| GT-04230 | 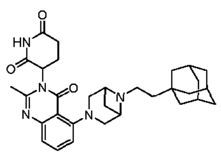 | 3-(5-(6-(2-((3r,5r,7r)-adamantan-1-yl)ethyl)-3,6-diazabicyclo[3.1.1]heptan-3-yl)-2-methyl-4-oxoquinazolin-3(4H)-yl)piperidine-2,6-dione | 3-(5-(6-(2-((3r,5r,7r)-adamantan-1-yl)ethyl)-3,6-diazabicyclo[3.1.1]heptan-3-yl)-2-methyl-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| GT-04253 | 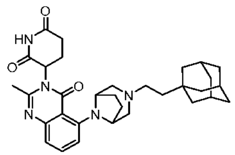 | 3-(5-(3-(2-((3r,5r,7r)-adamantan-1-yl)ethyl)-3,8-diazabicyclo[3.2.1]octan-8-yl)-2-methyl-4-oxoquinazolin-3(4H)-yl)piperidine-2,6-dione | 3-(5-(3-(2-((3r,5r,7r)-adamantan-1-yl)ethyl)-3,8-diazabicyclo[3.2.1]octan-8-yl)-2-methyl-4-oxoquinazolin-3(4H)-yl)piperidine-2,6-dione |
| GT-03727 | 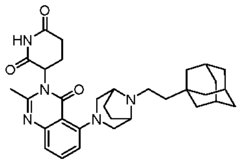 | 3-(5-(8-(2-((3r,5r,7r)-adamantan-1-yl)ethyl)-3,8-diazabicyclo[3.2.1]octan-3-yl)-2-methyl-4-oxoquinazolin-3(4H)-yl)piperidine-2,6-dione | 3-(5-(8-(2-((3r,5r,7r)-adamantan-1-yl)ethyl)-3,8-diazabicyclo[3.2.1]octan-3-yl)-2-methyl-4-oxoquinazolin-3(4H)-yl)piperidine-2,6-dione |
| GT-04228 | 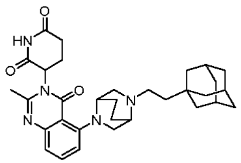 | 3-(5-(5-(2-((3r,5r,7r)-adamantan-1-yl)ethyl)-2,5-diazabicyclo[2.2.2]octan-2-yl)-2-methyl-4-oxoquinazolin-3(4H)-yl)piperidine-2,6-dione | 3-(5-(5-(2-((3r,5r,7r)-adamantan-1-yl)ethyl)-2,5-diazabicyclo[2.2.2]octan-2-yl)-2-methyl-4-oxoquinazolin-3(4H)-yl)piperidine-2,6-dione |
| GT-04232 | 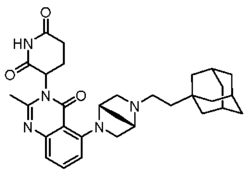 | 3-(5-((1S,4S)-5-(2-((3S,5S,7S)-adamantan-1-yl)ethyl)-2,5-diazabicyclo[2.2.1]heptan-2-yl)-2-methyl-4-oxoquinazolin-3(4H)-yl)piperidine-2,6-dione | 3-(5-((1S,4S)-5-(2-((S,5S,7S)-adamantan-1-yl)ethyl)-2,5-diazabicyclo[2.2.1]heptan-2-yl)-2-methyl-4-oxoquinazolin-3(4H)-yl)piperidine-2,6-dione |
| GT-04233 | 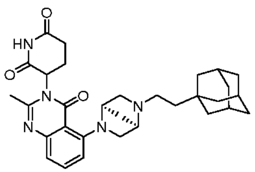 | 3-(5-((1R,4R)-5-(2-((3R,5R,7R)-adamantan-1-yl)ethyl)-2,5-diazabicyclo[2.2.1]heptan-2-yl)-2-methyl-4-oxoquinazolin-3(4H)-yl)piperidine-2,6-dione | 3-(5-((1R,4R)-5-(2-((3R,5R,7R)-adamantan-1-yl)ethyl)-2,5-diazabicyclo[2.2.1]heptan-2-yl)-2-methyl-4-oxoquinazolin-3(4H)-yl)piperidine-2,6-dione |
| GT-04246 | 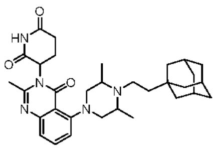 | 3-(5-(4-(2-((3r,5r,7r)-adamantan-1-yl)ethyl)-3,5-dimethylpiperazin-1-yl)-2-methyl-4-oxoquinazolin-3(4H)-yl)piperidine-2,6-dione | 3-(5-(4-(2-((3r,5r,7r)-adamantan-1-yl)ethyl)-3,5-dimethylpiperazin-1-yl)-2-methyl-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| | 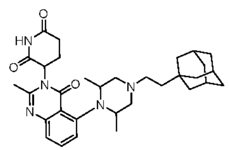 | 3-(5-(4-(2-((3r,5r,7r)-adamantan-1-yl)ethyl)-2,6-dimethylpiperazin-1-yl)-2-methyl-4-oxoquinazolin-3(4H)-yl)piperidine-2,6-dione | 3-(5-(4-(2-((3r,5r,7r)-adamantan-1-yl)ethyl)-2,6-dimethylpiperazin-1-yl)-2-methyl-4-oxoquinazolin-3(4H)-yl)piperi-dine-2,6-dione |
| | 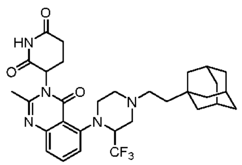 | 3-(5-(4-(2-((3r,5r,7r)-adamantan-1-yl)ethyl)-2-(trifluoromethyl)piperazin-1-yl)-2-methyl-4-oxoquinazolin-3(4H)-yl)piperidine-2,6-dione | 3-(5-(4-(2-((3r,5r,7r)-adamantan-1-yl)ethyl)-2-(trifluoromethyl)piperazin-1-yl)-2-methyl-4-oxoquinazolin-3(4H)-yl)piperidine-2,6-dione |
| | 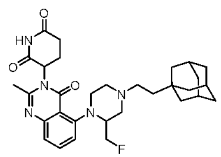 | 3-(5-(4-(2-((3r,5r,7r)-adamantan-1-yl)ethyl)-2-(fluoromethyl)piperazin-1-yl)-2-methyl-4-oxoquinazolin-3(4H)-yl)piperidine-2,6-dione | 3-(5-(4-(2-((3r,5r,7r)-adamantan-1-yl)ethyl)-2-(fluoromethyl)piperazin-1-yl)-2-methyl-4-oxoquinazolin-3(4H)-yl)piperidine-2,6-dione |
| | 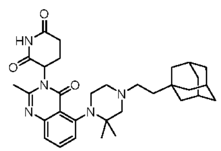 | 3-(5-(4-(2-((3r,5r,7r)-adamantan-1-yl)ethyl)-2,2-dimethylpiperazin-1-yl)-2-methyl-4-oxoquinazolin-3(4H)-yl)piperidine-2,6-dione | 3-(5-(4-(2-((3r,5r,7r)-adamantan-1-yl)ethyl)-2,2-dimethylpiperazin-1-yl)-2-methyl-4-oxoquinazolin-3(4H)-yl)piperidine-2,6-dione |
| GT-04229 | 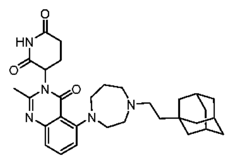 | 3-(5-(4-(2-((3r,5r,7r)-adamantan-1-yl)ethyl)-1,4-diazepan-1-yl)-2-methyl-4-oxoquinazolin-3 (4H)-yl)piperidine-2,6-dione | 3-(5-(4-(2-((3r,5r,7r)-adamantan-1-yl)ethyl)-1,4-diazepan-1-yl)-2-methyl-4-oxoquinazolin-3 (4H)-yl)piperi-dine-2,6-dione |
| | 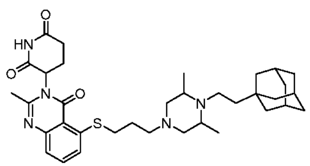 | 3-(5-((3-(4-(2-((3r,5r,7r)-adamantan-1-yl)ethyl)-3,5-dimethylpiperazin-1-yl)propyl)thio)-2-methyl-4-oxoquinazolin-3 (4H)-yl)piperidine-2,6-dione | 3-(5-((3-(4-(2-((3r,5r,7r)-adamantan-1-yl)ethyl)-3,5-dimethylpiperazin-1-yl)propyl)thio)-2-methyl-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| | 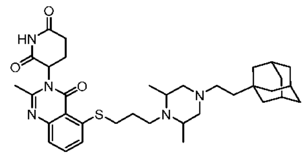 | 3-(5-((3-(4-(2-((3r,5r,7r)-adamantan-1-yl)ethyl)-2,6-dimethylpiperazin-1-yl)propyl)thio)-2-methyl-4-oxoquinazolin-3(4H)-yl)piperidine-2,6-dione | 3-(5-((3-(4-(2-((3r,5r,7r)-adamantan-1-yl)ethyl)-2,6-dimethylpiperazin-1-yl)propyl)thio)-2-methyl-4-oxoquinazolin-3(4H)-yl)piperidine-2,6-dione |
| | 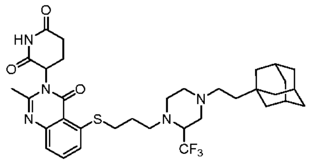 | 3-(5-((3-(4-(2-((3r,5r,7r)-adamantan-1-yl)ethyl)-2-(trifluoromethyl)piperazin-1-yl)propyl)thio)-2-methyl-4-oxoquinazolin-3(4H)-yl)piperidine-2,6-dione | 3-(5-((3-(4-(2-((3r,5r,7r)-adamantan-1-yl)ethyl)-2-(trifluoromethyl)piperazin-1-yl)propyl)thio)-2-methyl-4-oxoquinazolin-3(4H)-yl)piperidine-2,6-dione |
| | 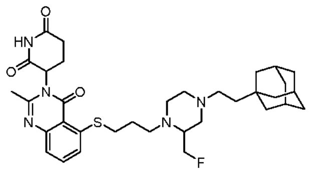 | 3-(5-((3-(4-(2-((3r,5r,7r)-adamantan-1-yl)ethyl)-2-(fluoromethyl)piperazin-1-yl)propyl)thio)-2-methyl-4-oxoquinazolin-3(4H)-yl)piperidine-2,6-dione | 3-(5-((3-(4-(2-((3r,5r,7r)-adamantan-1-yl)ethyl)-2-(fluoromethyl)piperazin-1-yl)propyl)thio)-2-methyl-4-oxoquinazolin-3(4H)-yl)piperidine-2,6-dione |
| | 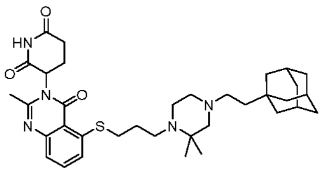 | 3-(5-((3-(4-(2-((3r,5r,7r)-adamantan-1-yl)ethyl)-2,2-dimethylpiperazin-1-yl)propyl)thio)-2-methyl-4-oxoquinazolin-3(4H)-yl)piperidine-2,6-dione | 3-(5-((3-(4-(2-((3r,5r,7r)-adamantan-1-yl)ethyl)-2,2-dimethylpiperazin-1-yl)propyl)thio)-2-methyl-4-oxoquinazolin-3(4H)-yl)piperidine-2,6-dione |
| | 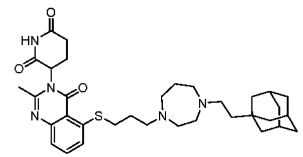 | 3-(5-((3-(4-(2-((3r,5r,7r)-adamantan-1-yl)ethyl)-1,4-diazepan-1-yl)propyl)thio)-2-methyl-4-oxoquinazolin-3(4H)-yl)piperidine-2,6-dione | 3-(5-((3-(4-(2-((3r,5r,7r)-adamantan-1-yl)ethyl)-1,4-diazepan-1-yl)propyl)thio)-2-methyl-4-oxoquinazolin-3(4H)-yl) piperidine-2,6-dione |
| | 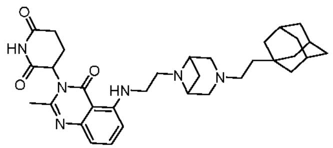 | 3-(5-((2-(3-(2-((3r,5r,7r)-adamantan-1-yl)ethyl)-3,6-diazabicyclo[3.1.1]heptan-6-yl)ethyl)amino)-2-methyl-4-oxoquinazolin-3(4H)-yl)piperidine-2,6-dione | 3-(5-((2-(3-(2-((3r,5r,7r)-adamantan-1-yl)ethyl)-3,6-diazabicyclo[3.1.1]heptan-6-yl)ethyl)amino)-2-methyl-4-oxoquinazolin-3(4H)-yl)piperidine-2,6-dione |

(continued)

| Compou nd No. | Structure of the compounds | The compounds' name in English | Chinese translation of the compounds' name |
|---|---|---|---|
| | 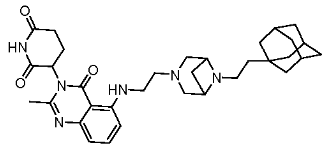 | 3-(5-((2-(6-(2-((3r,5r,7r)-adamantan-1-yl)ethyl)-3,6-diazabicyclo[3.1.1]heptan-3-yl)ethyl)amino)-2-methyl-4-oxoquinazolin-3(4H)-yl)piperidine-2,6-dione | 3-(5-((2-(6-(2-((3r,5r,7r)-adamantan-1-yl)ethyl)-3,6-diazabicyclo[3.1.1]heptan-3-yl)ethyl)amino)-2-methyl-4-oxoquinazolin-3(4H)-yl)piperidine-2,6-dione |
| | 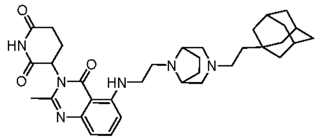 | 3-(5-((2-(3-(2-((3r,5r,7r)-adamantan-1-yl)ethyl)-3,8-diazabicyclo[3.2.1]octan-8-yl)ethyl)amino)-2-methyl-4-oxoquinazolin-3(4H)-yl)piperidine-2,6-dione | 3-(5-((2-(3-(2-((3r,5r,7r)-adamantan-1-yl)ethyl)-3,8-diazabicyclo[3.2.1]octan-8-yl)ethyl)amino)-2-methyl-4-oxoquinazolin-3(4H)-yl)piperidine-2,6-dione |
| | 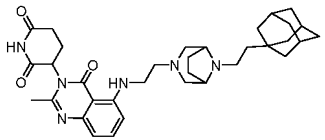 | 3-(5-((2-(8-(2-((3r,5r,7r)-adamantan-1-yl)ethyl)-3,8-diazabicyclo[3.2.1]octan-3-yl)ethyl)amino)-2-methyl-4-oxoquinazolin-3(4H)-yl)piperidine-2,6-dione | 3-(5-((2-(8-(2-((3r,5r,7r)-adamantan-1-yl)ethyl)-3,8-diazabicyclo[3.2.1]octan-3-yl)ethyl)amino)-2-methyl-4-oxoquinazolin-3(4H)-yl)piperidine-2,6-dione |
| | 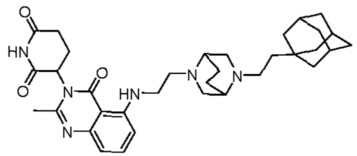 | 3-(5-((2-(5-(2-((3r,5r,7r)-adamantan-1-yl)ethyl)-2,5-diazabicyclo[2.2.2]octan-2-yl)ethyl)amino)-2-methyl-4-oxoquinazolin-3(4H)-yl)piperidine-2,6-dione | 3-(5-((2-(5-(2-((3r,5r,7r)-adamantan-1-yl)ethyl)-2,5-diazabicyclo[2.2.2]octan-2-yl)ethyl)amino)-2-methyl-4-oxoquinazolin-3(4H)-yl)piperidine-2,6-dione |
| | 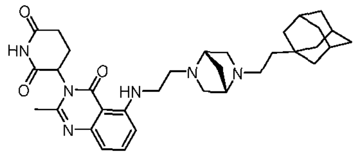 | 3-(5-((2-((1S,4S)-5-(2-((3S,5S,7S)-adamantan-1-yl) ethyl)-2,5-diazabicyclo[2.2.1]heptan-2-yl)ethyl)amino)-2-methyl-4-oxoquinazolin-3(4H)-yl)piperidine-2,6-dione | 3-(5-((2-((1S,4S)-5-(2-((3S,5S,7S)-adamantan-1-yl) ethyl)-2,5-diazabicyclo[2.2.1]heptan-2-yl)ethyl)amino)-2-methyl-4-oxoquinazolin-3(4H)-yl)piperidine-2,6-dione |
| | 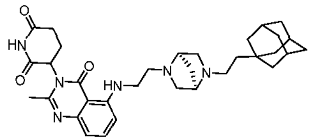 | 3-(5-((2-((1R,4R)-5-(2-((3R,5R,7R)-adamantan-1-yl) ethyl)-2,5-diazabicyclo[2.2.1]heptan-2-yl)ethyl)amino)-2-methyl-4-oxoquinazolin-3 (4H)-yl)piperidine-2,6-dione | 3-(5-((2-((1R,4R)-5-(2-((3R,5R,7R)-adamantan-1-yl) ethyl)-2,5-diazabicyclo[2.2.1]heptan-2-yl)ethyl)amino)-2-methyl-4-oxoquinazolin-3 (4H)-yl)piperidine-2,6-dione |

(continued)

| Compound No. | Structure of the compounds | The compounds' name in English | Chinese translation of the compounds' name |
|---|---|---|---|
| | 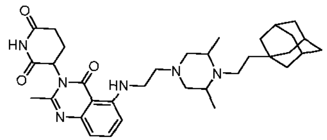 | 3-(5-((2-(4-(2-((3r,5r,7r)-adamantan-1-yl)ethyl)-3,5-dimethylpiperazin-1-yl)ethyl)amino)-2-methyl-4-oxoquinazolin-3(4H)-yl)piperidine-2,6-dione | 3-(5-((2-(4-(2-((3r,5r,7r)-adamantan-1-yl)ethyl)-3,5-dimethylpiperazin-1-yl)ethyl)amino)-2-methyl-4-oxoquinazolin-3(4H)-yl)piperidine-2,6-dione |
| | 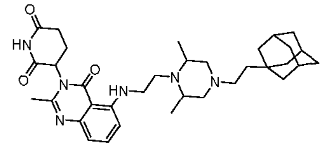 | 3-(5-((2-(4-(2-((3r,5r,7r)-adamantan-1-yl)ethyl)-2,6-dimethylpiperazin-1-yl)ethyl)amino)-2-methyl-4-oxoquinazolin-3(4H)-yl)piperidine-2,6-dione | 3-(5-((2-(4-(2-((3r,5r,7r)-adamantan-1-yl)ethyl)-2,6-dimethylpiperazin-1-yl)ethyl)amino)-2-methyl-4-oxoquinazolin-3(4H)-vl)piperidine-2,6-dione |
| | 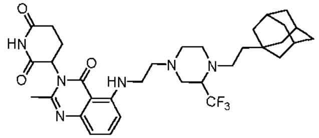 | 3-(5-((2-(4-(2-((3r,5r,7r)-adamantan-1-yl)ethyl)-3-(trifluoromethyl)piperazin-1-yl)ethyl)amino)-2-methyl-4-oxoquinazolin-3(4H)-yl)piperidine-2,6-dione | 3-(5-((2-(4-(2-((3r,5r,7r)-adamantan-1-yl)ethyl)-3-(trifhioromethyl)piperazin-1-yl)ethyl)amino)-2-methyl-4-oxoquinazolin-3(4H)-yl)piperidine-2,6-dione |
| | 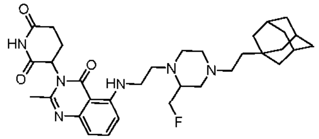 | 3-(5-((2-(4-(2-((3r,5r,7r)-adamantan-1-yl)ethyl)-2-(fluoromethyl)piperazin-1-yl)ethyl)amino)-2-methyl-4-oxoquinazolin-3(4H)-yl)piperidine-2,6-dione | 3-(5-((2-(4-(2-((3r,5r,7r)-adamantan-1-yl)ethyl)-2-(fluoromethyl)piperazin-1-yl)ethyl)amino)-2-methyl-4-oxoquinazolin-3 (4H)-yl)piperidine-2,6-dione |
| | 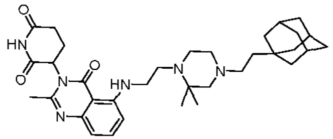 | 3-(5-((2-(4-(2-((3r,5r,7r)-adamantan-1-yl)ethyl)-2,2-dimethylpiperazin-1-yl)ethyl)amino)-2-methyl-4-oxoquinazolin-3(4H)-yl)piperidine-2,6-dione | 3-(5-((2-(4-(2-((3r,5r,7r)-adamantan-1-yl)ethyl)-2,2-dimethylpiperazin-1-yl)ethyl)amino)-2-methyl-4-oxoquinazolin-3(4H)-yl)piperidine-2,6-dione |
| | 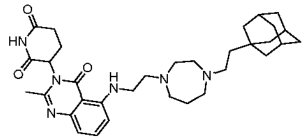 | 3-(5-((2-(4-(2-((3r,5r,7r)-adamantan-1 -yl)ethyl)-1,4-diazepan-1-yl)ethyl)amino)-2-methyl-4-oxoquinazolin-3 (4H)-yl)piperidine-2,6-dione | 3-(5-((2-(4-(2-((3r,5r,7r)-adamantan-1-yl)ethyl)-1,4-diazepan-1-yl)ethyl)amino)-2-methyl-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| GT-03385 | 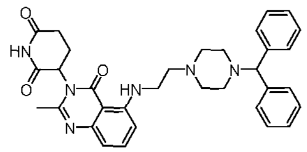 | 3-(5-((2-(4-benzhydrylpiperazin-1-yl)ethyl)amino)-2-methyl-4-oxoquinazolin-3(4H)-yl)piperidine-2,6-dione | 3-(5-((2-(4-benzhydrylpiperazin-1-yl)ethyl)amino)-2-methyl-4-oxoquinazolin-3(4H)-yl)piperidine-2,6-dione |
| | 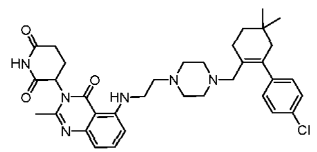 | 3-(5-((2-(4-((4'-chloro-5,5-dimethyl-3,4,5,6-tetrahydro-[1,1'-biphenyl]-2-yl)methyl)piperazin-1-yl)ethyl)amino)-2-methyl-4-oxoquinazolin-3(4H)-yl)piperidine-2,6-dione | 3-(5-((2-(4-((4'-chloro-5,5-dimethyl-3,4,5,6-tetrahydro-[1,1'-biphenyl]-2-yl)methyl)piperazin-1-yl)ethyl)amino)-2-methyl-4-oxoquinazolin-3(4H)-yl)piperidine-2,6-dione |
| | 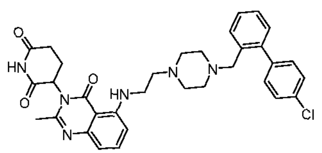 | 3-(5-((2-(4-((4'-chloro-[1,1'-biphenyl]-2-yl)methyl)piperazin-1-yl)ethyl)amino)-2-methyl-4-oxoquinazolin-3(4H)-yl) piperidine-2,6-dione | 3-(5-((2-(4-((4'-chloro-[1,1'-biphenyl]-2-yl)methyl)piperazin-1-yl)ethyl)amino)-2-methyl-4-oxoquinazolin-3(4H)-yl) piperidine-2,6-dione |
| | 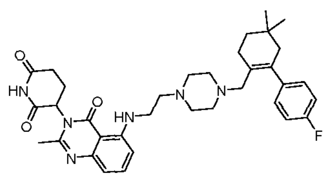 | 3-(5-((2-(4-((4'-fluoro-5,5-dimethyl-3,4,5,6-tetrahydro-[1,1'-biphenyl]-2-yl)methyl)piperazin-1-yl)ethyl)amino)-2-methyl-4-oxoquinazolin-3(4H)-yl)piperidine-2,6-dione | 3-(5-((2-(4-((4'-fluoro-5,5-dimethyl-3,4,5,6-tetrahydro-[1,1'-biphenyl]-2-yl)methyl)piperazin-1-yl)ethyl)amino)-2-methyl-4-oxoquinazolin-3(4H)-vl)piperidine-2,6-dione |
| | 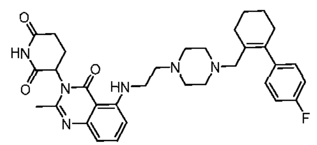 | 3-(5-((2-(4-((4'-fluoro-3,4,5,6-tetrahydro-[1,1'-biphenyl]-2-yl)methyl)piperazin-1-yl)ethyl)amino)-2-methyl-4-oxoquinazolin-3(4H)-yl)piperidine-2,6-dione | 3-(5-((2-(4-((4'-fluoro-3,4,5,6-tetrahydro-[1,1'-biphenyl]-2-yl)methyl)piperazin-1-yl)ethyl)amino)-2-methyl-4-oxoquinazolin-3(4H)-yl)piperidine-2,6-dione |
| | 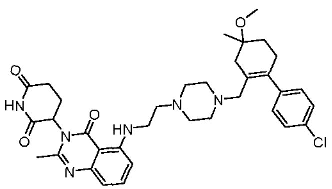 | 3-(5-((2-(4-((4'-chloro-4-methoxy-4-methyl-3,4,5,6-tetrahydro-[1,1'-biphenyl]-2-yl)methyl)piperazin-1-yl)ethyl) amino)-2-methyl-4-oxoquinazolin-3(4H)-yl)piperidine-2,6-dione | 3-(5-((2-(4-((4'-chloro-4-methoxy-4-methyl-3,4,5,6-tetrahydro-[1,1'-biphenyl]-2-yl)methyl)piperazin-1-yl)ethyl) amino)-2-methyl-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| | 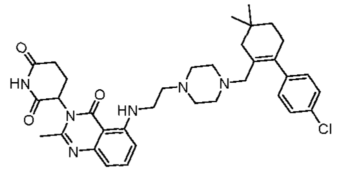 | 3-(5-((2-(4-((4'-chloro-4,4-dimethyl-3,4,5 ,6-tetrahydro-[1,1'-biphenyl]-2-yl)methyl)piperazin-1-yl)ethyl)amino)-2-methyl-4-oxoquinazolin-3 (4H)-yl)piperidine-2,6-dione | 3-(5-((2-(4-((4'-chloro-4,4-dimethyl-3,4,5 ,6-tetrahydro-[1,1'-biphenyl]-2-yl)methyl)piperazin-1-yl)ethyl)amino)-2-methyl-4-oxoquinazolin-3 (4H)-yl)piperidine-2,6-dione |
| | 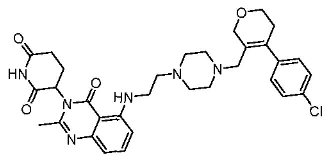 | 3-(5-((2-(4-((4-(4-chlorophenyl)-5,6-dihydro-2H-pyran-3-yl)methyl)piperazin-1-yl)ethyl)amino)-2-methyl-4-oxoquinazolin-3 (4H)-yl)piperidine-2,6-dione | 3-(5-((2-(4-((4-(4-chlorophenyl)-5,6-dihydro-2H-pyran-3-yl)methyl)piperazin-1-yl)ethyl)amino)-2-methyl-4-oxoquinazolin-3 (4H)-yl)piperidine-2,6-dione |
| | 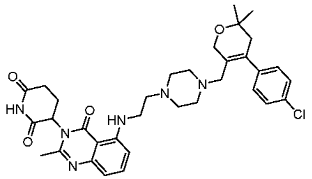 | 3-(5-((2-(4-((4-(4-chlorophenyl)-6,6-dimethyl-5,6-dihydro-2H-pyran-3-yl)methyl)piperazin-1-yl)ethyl)amino)-2-methyl-4-oxoquinazolin-3(4H)-yl)piperidine-2,6-dione | 3-(5-((2-(4-((4-(4-chlorophenyl)-6,6-dimethyl-5,6-dihydro-2H-pyran-3-yl)methyl)piperazin-1-yl)ethyl)amino)-2-methyl-4-oxoquinazolin-3(4H)-yl)piperidine-2,6-dione |
| | 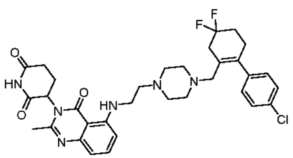 | 3-(5-((2-(4-((4'-chloro-4,4-difluoro-3,4,5,6-tetrahydro-[1,1'-biphenyl]-2-yl)methyl)piperazin-1-yl)ethyl)amino)-2-methyl-4-oxoquinazolin-3(4H)-yl)piperidine-2,6-dione | 3-(5-((2-(4-((4'-chloro-4,4-difluoro-3,4,5 ,6-tetrahydro-[1,1'-biphenyl]-2-yl)methyl)piperazin-1-yl)ethyl)amino)-2-methyl-4-oxoquinazolin-3(4H)-yl)piperidine-2,6-dione |
| | 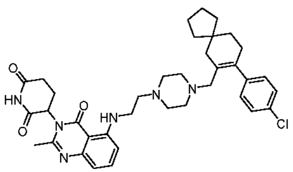 | 3-(5-((2-(4-((8-(4-chlorophenyl)spiro[4.5]dec-7-en-7-yl)methyl)piperazin-1-yl)ethyl)amino)-2-methyl-4-oxoquinazolin-3(4H)-yl)piperidine-2,6-dione | 3-(5-((2-(4-((8-(4-chlorophenyl)spiro[4.5]dec-7-en-7-yl)methyl)piperazin-1-yl)ethyl)amino)-2-methyl-4-oxoquinazolin-3(4H)-yl)piperidine-2,6-dione |
| | 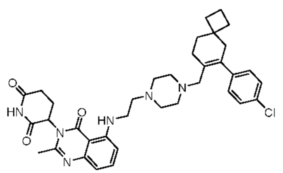 | 3-(5-((2-(4-((6-(4-chlorophenyl)spiro[3.5]non-6-en-7-yl)methyl)piperazin-1-yl)ethyl)amino)-2-methyl-4-oxoquinazolin-3(4H)-yl)piperidine-2,6-dione | 3-(5-((2-(4-((6-(4-chlorophenyl)spiro[3.5]non-6-en-7-yl)methyl)piperazin-1-yl)ethyl)amino)-2-methyl-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| | 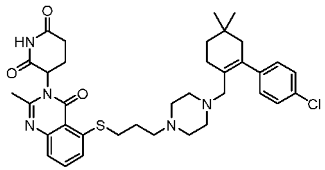 | 3-(5-((3-(4-((4'-chloro-5,5-dimethyl-3,4,5,6-tetrahydro-[1,1'-biphenyl]-2-yl)methyl)piperazin-1-yl)propyl)thio)-2-methyl-4-oxoquinazolin-3(4H)-yl)piperidine-2,6-dione | 3-(5-((3-(4-((4'-chloro-5,5-dimethyl-3,4,5,6-tetrahydro-[1,1'-biphenyl]-2-yl)methyl)piperazin-1-yl)propyl)thio)-2-methyl-4-oxoquinazolin-3(4H)-yl)piperidine-2,6-dione |
| | 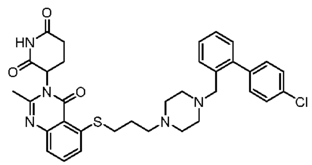 | 3-(5-((3-(4-((4'-chloro-[1,1'-biphenyl]-2-yl)methyl)piperazin-1-yl)propyl)thio)-2-methyl-4-oxoquinazolin-3(4H)-yl)piperidine-2,6-dione | 3-(5-((3-(4-((4'-chloro-[1,1'-biphenyl]-2-yl)methyl)piperazin-1-yl)propyl)thio)-2-methyl-4-oxoquinazolin-3(4H)-yl)piperidine-2,6-dione |
| | 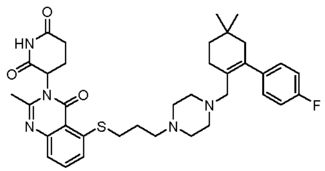 | 3-(5-((3-(4-((4'-fluoro-5,5-dimethyl-3,4,5,6-tetrahydro-[1,1'-biphenyl]-2-yl)methyl)piperazin-1-yl)propyl)thio)-2-methyl-4-oxoquinazolin-3(4H)-yl)piperidine-2,6-dione | 3-(5-((3-(4-((4'-fluoro-5,5-dimethyl-3,4,5,6-tetrahydro-[1,1'-biphenyl]-2-yl)methyl)piperazin-1-yl)propyl)thio)-2-methyl-4-oxoquinazolin-3(4H)-yl)piperidine-2,6-dione |
| | 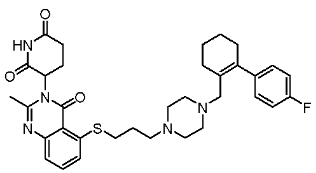 | 3-(5-((3-(4-((4'-fluoro-3,4,5,6-tetrahydro-[1,1'-biphenyl]-2-yl)methyl)piperazin-1-yl)propyl)thio)-2-methyl-4-oxoquinazolin-3(4H)-yl)piperidine-2,6-dione | 3-(5-((3-(4-((4'-fluoro-3,4,5,6-tetrahydro-[1,1'-biphenyl]-2-yl)methyl)piperazin-1-yl)propyl)thio)-2-methyl-4-oxoquinazolin-3(4H)-yl)piperidine-2,6-dione |
| | 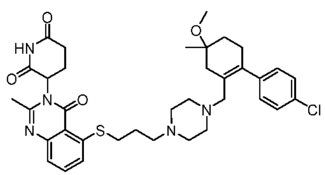 | 3-(5-((3-(4-((4'-chloro-4-methoxy-4-methyl-3,4,5,6-tetrahydro-[1,1'-biphenyl]-2-yl)methyl)piperazin-1-yl)propyl)thio)-2-methyl-4-oxoquinazolin-3(4H)-yl)piperidine-2,6-dione | 3-(5-((3-(4-((4'-chloro-4-methoxy-4-methyl-3,4,5,6-tetrahydro-[1,1'-biphenyl]-2-yl)methyl)piperazin-1-yl)propyl)thio)-2-methyl-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| | 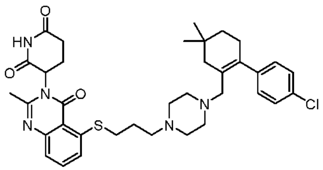 | 3-(5-((3-(4-((4'-chloro-4,4-dimethyl-3,4,5 ,6-tetrahydro-[1,1'-biphenyl]-2-yl)methyl)piperazin-1-yl)propyl) thio)-2-methyl-4-oxoquinazolin-3(4H)-yl)piperidine-2,6-dione | 3-(5-((3-(4-((4'-chloro-4,4-dimethyl-3,4,5 ,6-tetrahydro-[1,1'-biphenyl]-2-yl)methyl)piperazin-1-yl)propyl) thio)-2-methyl-4-oxoquinazolin-3(4H)-yl)piperidine-2,6-dione |
| | 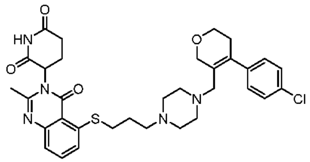 | 3-(5-((3-(4-((4-(4-chlorophenyl)-5,6-dihydro-2H-pyran-3-yl)methyl)piperazin-1-yl)propyl)thio)-2-methyl-4-oxoquinazolin-3(4H)-yl)piperidine-2,6-dione | 3-(5-((3-(4-((4-(4-chlorophenyl)-5,6-dihydro-2H-pyran-3-yl)methyl)piperazin-1-yl)propyl)thio)-2-methyl-4-oxoquinazolin-3(4H)-yl)piperidine-2,6-dione |
| | 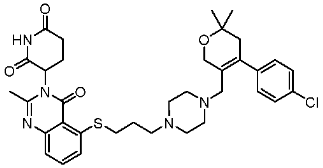 | 3-(5-((3-(4-((4-(4-chlorophenyl)-6,6-dimethyl-5,6-dihydro-2H-pyran-3-yl)methyl)piperazin-1-yl)propyl)thio)-2-methyl-4-oxoquinazolin-3(4H)-yl)piperidine-2,6-dione | 3-(5-((3-(4-((4-(4-chlorophenyl)-6,6-dimethyl-5,6-dihydro-2H-pyran-3-yl)methyl)piperazin-1-yl)propyl)thio)-2-methyl-4-oxoquinazolin-3(4H)-yl)piperidine-2,6-dione |
| | 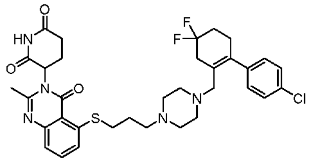 | 3-(5-((3-(4-((4'-chloro-4,4-difluoro-3,4,5,6-tetrahydro-[1,1'-biphenyl]-2-yl)methyl)piperazin-1-yl)propyl) thio)-2-methyl-4-oxoquinazolin-3(4H)-yl)piperidine-2,6-dione | 3 -(5-((3-(4-((4'-chloro-4,4-difluoro-3,4,5,6-tetrahydro-[1,1'-biphenyl]-2-yl)methyl)piperazin-1-yl)propyl) thio)-2-methyl-4-oxoquinazolin-3(4H)-yl)piperidine-2,6-dione |
| | 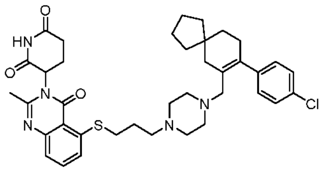 | 3-(5-((3-(4-((8-(4-chlorophenyl)spiro[4.5]dec-7-en-7-yl) methyl)piperazin-1-yl)propyl)thio)-2-methyl-4-oxoquinazolin-3(4H)-yl)piperidine-2,6-dione | 3-(5-((3-(4-((8-(4-chlorophenyl)spiro[4.5]dec-7-en-7-yl) methyl)piperazin-1-yl)propyl)thio)-2-methyl-4-oxoquinazolin-3(4H)-yl)piperidine-2,6-dione |
| | 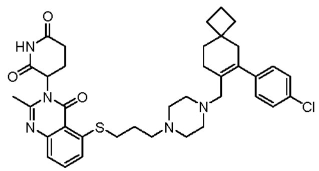 | 3-(5-((3-(4-((6-(4-chlorophenyl)spiro[3.5]non-6-en-7-yl) methyl)piperazin-1-yl)propyl)thio)-2-methyl-4-oxoquinazolin-3(4H)-yl)piperidine-2,6-dione | 3-(5-((3-(4-((6-(4-chlorophenyl)spiro[3.5]non-6-en-7-yl) methyl)piperazin-1-yl)propyl)thio)-2-methyl-4-oxoquinazolin-3(4H)-yl)piperidine-2,6-dione |

(continued)

| Compound No. | Structure of the compounds | The compounds' name in English | Chinese translation of the compounds' name |
|---|---|---|---|
| GT-03314 | 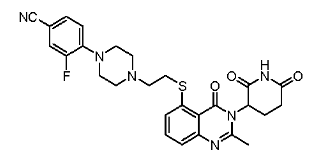 | 4-(4-(2-((3-(2,6-dioxopiperidin-3-yl)-2-methyl-4-oxo-3,4-dihydroquinazolin-5-yl)thio)ethyl)piperazin-1-yl)-3-fluorobenzonitrile | 4-(4-(2-((3-(2,6-dioxopiperidin-3-yl)-2-methyl-4-oxo-3,4-dihydroquinazolin-5-yl)thio)ethyl)piperazin-1-yl)-3-fluoro-benzonitrile |
| GT-03315 | 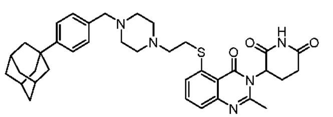 | 3-(5-((2-(4-(4-((1s,3s)-adamantan-1-yl)benzyl)piperazin-1-yl)ethyl)thio)-2-methyl-4-oxoquinazolin-3(4H)-yl)piperidine-2,6-dione | 3-(5-((2-(4-(4-((1s,3s)-adamantan-1-yl)benzyl)piperazin-1-yl)ethyl)thio)-2-methyl-4-oxoquinazolin-3(4H)-yl)piperidine-2,6-dione |
| GT-03285 | 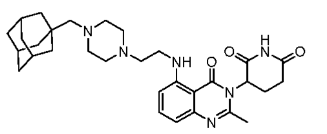 | 3-(5-((2-(4-(((1s,3s)-adamantan-1-yl)methyl)piperazin-1-yl)ethyl)amino)-2-methyl-4-oxoquinazolin-3(4H)-yl)piperidine-2,6-dione | 3-(5-((2-(4-(((1s,3s)-adamantan-1-yl)methyl)piperazin-1-yl)ethyl)amino)-2-methyl-4-oxoquinazolin-3(4H)-yl)piperidine-2,6-dione |
| GT-03316 | 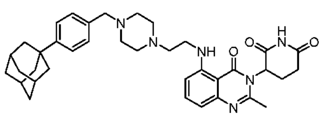 | 3-(5-((2-(4-(4-((1s,3s)-adamantan-1-yl)benzyl)piperazin-1-yl)ethyl)amino)-2-methyl-4-oxoquinazolin-3 (4H)-yl) piperidine-2,6-dione | 3-(5-((2-(4-(4-((1s,3s)-adamantan-1-yl)benzyl)piperazin-1-yl)ethyl)amino)-2-methyl-4-oxoquinazolin-3 (4H)-yl) piperidine-2,6-dione |
| GT-03313 | 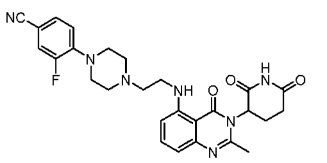 | 4-(4-(2-((3-(2,6-dioxopiperidin-3-yl)-2-methyl-4-oxo-3,4-dihydroquinazolin-5-yl)amino)ethyl)piperazin-1-yl)-3-fluorobenzonitrile | 4-(4-(2-((3-(2,6-dioxopiperidin-3-yl)-2-methyl-4-oxo-3,4-dihydroquinazolin-5-yl)amino)ethyl)piperazin-1-yl)-3-fluorobenzonitrile |
| GT-03310 | 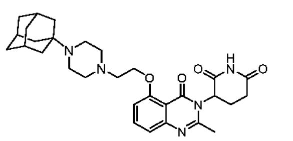 | 3-(5-(2-(4-((3s,5s,7s)-adamantan-1-yl)piperazin-1-yl) ethoxy)-2-methyl-4-oxoquinazolin-3(4H)-yl)piperidine-2,6-dione | 3-(5-(2-(4-((3s,5s,7s)-adamantan-1-yl)piperazin-1-yl) ethoxy)-2-methyl-4-oxoquinazolin-3(4H)-yl)piperidine-2,6-dione |
| GT-03311 | 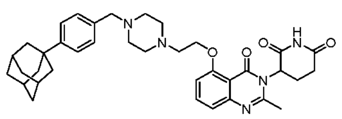 | 3-(5-(2-(4-(4-((1s,3s)-adamantan-1-yl)benzyl)piperazin-1-yl)ethoxy)-2-methyl-4-oxoquinazolin-3 (4H)-yl)piperidine-2,6-dione | 3-(5-(2-(4-(4-((1s,3s)-adamantan-1-yl)benzyl)piperazin-1-yl)ethoxy)-2-methyl-4-oxoquinazolin-3 (4H)-yl)pi-peridine-2,6-dione |

(continued)

| Compound No. | Structure of the compounds | The compounds' name in English | Chinese translation of the compounds' name |
|---|---|---|---|
| GT-03312 | 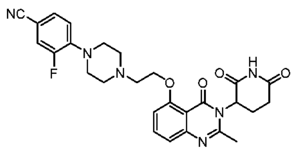 | 4-(4-(2-((3-(2,6-dioxopiperidin-3-yl)-2-methyl-4-oxo-3,4-dihydroquinazolin-5-yl)oxy)ethyl)piperazin-1-yl)-3-fluorobenzonitrile | 4-(4-(2-((3-(2,6-dioxopiperidin-3-yl)-2-methyl-4-oxo-3,4-dihydroquinazolin-5-yl)oxy)ethyl)piperazin-1-yl)-3-fluoro-benzonitrile |
| GT-03261 | 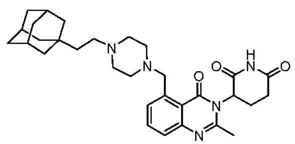 | 3-(5-((4-(2-((3r,5r,7r)-adamantan-1-yl)ethyl)piperazin-1-yl)methyl)-2-methyl-4-oxoquinazolin-3(4H)-yl)piperidine-2,6-dione | 3-(5-((4-(2-((3r,5r,7r)-adamantan-1-yl)ethyl)piperazin-1-yl)methyl)-2-methyl-4-oxoquinazolin-3(4H)-yl)piperidine-2,6-dione |
| GT-03317 | 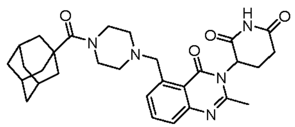 | 3-(5-((4-((1s,3s)-adamantane-1-carbonyl)piperazin-1-yl) methyl)-2-methyl-4-oxoquinazolin-3 (4H)-yl)piperidine-2,6-dione | 3-(5-((4-((1s,3s)-adamantane-1-carbonyl)piperazin-1-yl) methyl)-2-methyl-4-oxoquinazolin-3 (4H)-yl)piperidine-2,6-dione |
| GT-03262 | 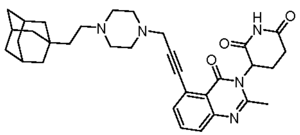 | 3-(5-(3-(4-(2-((1s,3s)-adamantan-1-yl)ethyl)piperazin-1-yl)prop-1-yn-1-yl)-2-methyl-4-oxoquinazolin-3(4H)-yl)piperidine-2,6-dione | 3-(5-(3-(4-(2-((1s,3s)-adamantan-1-yl)ethyl)piperazin-1-yl)prop-1-yn-1-yl)-2-methyl-4-oxoquinazolin-3(4H)-yl)piperidine-2,6-dione |
| GT-03318 | 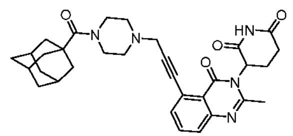 | 3-(5-(3-(4-((3r,5r,7r)-adamantane-1-carbonyl)piperazin-1-yl)prop-1-yn-1-yl)-2-methyl-4-oxoquinazolin-3(4H)-yl)piperidine-2,6-dione | 3-(5-(3-(4-((3r,5r,7r)-adamantane-1-carbonyl)piperazin-1-yl)prop-1-yn-1-yl)-2-methyl-4-oxoquinazolin-3(4H)-yl)piperidine-2,6-dione |

**II. Other Forms of Compounds** (including salts, enantiomers, stereoisomers, solvates, isotopically enriched analogs, prodrugs, or polymorphs of compounds)

The compounds of the present disclosure have the structures of any one of Formula (I), Formula (I-1), Formula (I-2), Formula (I-3), Formula (I-4), Formula (I-5), Formula (I-6), Formula (I-7), Formula (I-8), Formula (II), Formula (II), Formula (III), Formula (Ia), Formula (Ib), Formula (Ic), Formula (Id), Formula (IIa), Formula (IIb), Formula (IIc), Formula (IId), Formula (IIIa), Formula (IIIb), Formula (IIIc), or Formula (IIId). Unless otherwise specified, all references to the compounds of the present disclosure also include compounds of any one of Formula (I), Formula (I-1), Formula (I-2), Formula (I-3), Formula (I-4), Formula (I-5), Formula (I-6), Formula (I-7), Formula (I-8), Formula (II), Formula (II), Formula (III), Formula (Ia), Formula (Ib), Formula (Ic), Formula (Id), Formula (IIa), Formula (IIb), Formula (IIc), Formula (IId), Formula (IIIa), Formula (IIIb), Formula (IIIc), or Formula (IIId) and specific compounds within the scope of these general formulae.

It should be recognized that compounds of the present disclosure (including compounds of Formula (I), Formula (I-1), Formula (I-2), Formula (I-3), Formula (I-4), Formula (I-5), Formula (I-6), Formula (I-7), Formula (I-8), Formula (II), Formula (II), Formula (III), Formula (Ia), Formula (Ib), Formula (Ic), Formula (Id), Formula (IIa), Formula (IIb), Formula (IIc), Formula (IId), Formula (IIIa), Formula (IIIb), Formula (IIIc), or Formula (IIId)) may have a stereo-configuration and thus can exist in more than one stereoisomeric form. The present disclosure also relates to optically enriched compounds having a stereo-configuration, e.g., greater than about 90% enantiomeric/diastereomeric excess ("ee"), such as about 95% ee or 97% ee, or greater than 99% ee, and mixtures thereof, including racemic mixtures. As used herein, "optically enriched" means that a mixture of enantiomers consists of a significantly greater proportion of one enantiomer, and can be described by enantiomeric excess (ee%). Purification of isomers and separation of mixtures of isomers can be accomplished by standard techniques known in the art (e.g., column chromatography, preparative TLC, preparative HPLC, asymmetric synthesis (e.g., by using chiral intermediates) and/or or chiral resolution, etc.).

In some embodiments, polymorph forms or salts of the compounds of the present disclosure are also provided. Salts of the compounds of the present disclosure can be pharmaceutically acceptable salts including, but not limited to, hydrochlorides, sulfates, citrates, maleates, sulfonates, citrates, lactates, tartrates, fumarates, phosphates, dihydrophosphates, pyrophosphates, metaphosphates, oxalates, malonates, benzoates, mandelates, succinates, trifluoroacetates, hydroxyacetates, or p-toluenesulfonates, etc. The compounds of the present disclosure can exist as non-solvated or solvated forms in pharmaceutically acceptable solvents such as water, ethanol, and the like. In some embodiments, compounds of the present disclosure can be prepared as prodrugs or precursor drugs. Prodrugs can be converted into parent drugs in the body to play their role. In some embodiments, isotopically-labeled compounds of the present disclosure are also provided, examples of which include deuterium (D or $^2H$).

**III. Compositions/Formulations**

In some embodiments, the present disclosure provides a pharmaceutical composition comprising as an active ingredient the compound of the present disclosure or a pharmaceutically acceptable salt, solvate, isotopically enriched analog, polymorph, prodrug, stereoisomer (including enantiomer), or mixture of stereoisomers thereof, and at least one pharmaceutically acceptable carrier.

In some embodiments, pharmaceutically acceptable carriers include, but are not limited to, fillers, stabilizers, dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that can be used as pharmaceutically acceptable carriers include: sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common non-toxic compatible substances used in pharmaceutical formulations.

The pharmaceutical composition of the present disclosure can further comprise at least one second therapeutic agent, e.g., an anticancer agent. The second therapeutic agent may be used in combination with the compounds of Formula (I) described in the present disclosure to treat the diseases or disorders as disclosed herein. The second therapeutic agent includes, but is not limited to, chemotherapeutic agents, immunotherapeutic agents, gene therapy agents, and the like.

The pharmaceutical composition of the present disclosure comprising, as an active ingredient, the compounds of Formula (I) of the present disclosure or a pharmaceutically acceptable salt thereof can be formulated into any suitable

formulations such as sprays, patches, tablets (such as conventional tablets, dispersible tablets, orally disintegrating tablets), capsules (such as soft capsules, hard capsules, enteric-coated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of Formula (I) of the present disclosure into conventional, dispersible, chewable, orally disintegrating or rapidly dissolving formulations, or sustained-release capsules or controlled-release capsules as needed.

The compounds of Formula (I) of the present disclosure, as an active ingredient, is contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25mg per kilogram body weight of the subject per day.

The compounds of Formula (I) of the present disclosure or pharmaceutically acceptable salts thereof can be conveniently administered in any suitable unit dosage form. Suitable unit dosage form specifications include, but are not limited to, less than 1mg, 1mg to 3000mg, 5mg to 1000mg, for example, 5 to 500mg, 25 to 250mg of active ingredient per unit dosage form.

## IV. **Kits/Packaged Products**

The compounds of Formula (I) or a pharmaceutically acceptable salt, solvate, isotopically enriched analog, polymorph, prodrug, stereoisomer (including enantiomer), or mixture of stereoisomers thereof is used as a medicament. The medicament of the present disclosure or the pharmaceutical composition of the present disclosure may be presented in a kit/packaged product. The kit/packaged product may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/packaged product may contain instructions for use of the product.

## V. Methods and Uses

The compounds of Formula (I) or a pharmaceutically acceptable salt, solvate, isotopically enriched analog, polymorph, prodrug, stereoisomer (including enantiomer), or mixture of stereoisomers thereof can be used as a medicament. Especially the compounds of Formula (I) or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of diseases or disorders associated with cereblon protein. The diseases or disorders associated with cereblon protein comprise: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes. In some embodiments, the diseases or disorders associated with cereblon protein include, but are not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer;

central nervous system tumor; neuroblastoma; neuroglioma; Peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

The present disclosure provides a method for preventing and/or treating diseases or disorders associated with cereblon protein in a subject, comprising administering to the subject a therapeutically effective amount of the compounds of Formula (I) of the present disclosure or a pharmaceutically acceptable salt thereof, or the pharmaceutical composition of the present disclosure comprising as an active ingredient the compounds of Formula (I) of the present disclosure or a pharmaceutically acceptable salt thereof. In some embodiments, the diseases or disorders associated with cereblon protein comprise: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes. In some embodiments, the diseases or disorders associated with cereblon protein include, but are not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; neuroglioma; Peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

In the method for preventing and/or treating diseases or disorders associated with cereblon protein in a subject, the compound of Formula (I) of the present disclosure or the pharmaceutical composition comprising as an active

ingredient the compound of Formula (I) of the present disclosure of the present disclosure is administered to the subject through at least one mode of administration selected from the group consisting of nasal administration, inhalation administration, topical administration, oral administration, oral mucosal administration, rectal administration, pleural cavity administration, peritoneal administration, vaginal administration, intramuscular administration, subcutaneous, transdermal, epidural, intrathecal, and intravenous administration.

The term "treatment" or "treating" refers to the administration of the compound of Formula (I) or a pharmaceutically acceptable salt thereof according to the present disclosure, or the pharmaceutical composition containing, as an active ingredient, the compound of Formula (I) or a pharmaceutically acceptable salt thereof, to a subject to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

It is to be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see e.g., Jun Li (chief editor), "Clinical Pharmacology", 4th edition, People's Public Health Press, 2008).

As used herein, the term "patient" or "subject" to be treated refers to animal, for example mammal, including but not limited to primate (such as human being), cow, sheep, goat, horse, dog, cat, rabbit, guinea pig, rat, mice, etc.

## VI. **Definitions**

Unless otherwise specified, the following words, phrases and symbols used herein generally have the meanings as described below.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of" are also provided.

As used herein, the term "about" used alone or in combination refers to approximately, roughly, nearly, or around. When the term "about" is used in conjunction with a numerical range, it modifies that range by extending the boundaries above and below the stated numerical value. In general, the term "about" can modify a numerical value above and below the stated value by an upward or downward (increasing or decreasing) variation, e.g., 10%, 5%, 2%, or 1%.

As used herein, the wording "...represents a bond" used alone or in combination means that the referenced group is a bond linker (that is, the referenced group is absent). For example, the wording "$X_2$ represents a bond" means that $X_2$ is a bond linker. In other words, when $X_2$ represents a bond, the group $R_{a1}$ in the structure of Formula (I) is directly connected to $L_1$ in the structure of Formula (I). For example, the wording "$X_1$ represents a bond" means that $X_1$ is a bond linker. In other words, when $X_1$ represents a bond, the group $L_1$ in the structure of Formula (I) is directly connected to quinazoline ring in the structure of Formula (I).

In this document, the term "optionally substituted" used alone or in combination means that the referenced group may be unsubstituted or substituted with one or more substituents as defined here. Herein, the wording "optionally substituted with..." and "unsubstituted or substituted" can be used interchangeably. The term "substituted" generally indicates that one or more hydrogens in the referenced structure are replaced by the same or different specific substituents. The number of substituents is not theoretically limited in any way, or is automatically limited by the size of the building units (i.e., the total number of replaceable hydrogen atoms in the building units), or as clearly defined herein.

As used herein, the term "inserted" of the expression "one or more groups Rai and/or one or more groups $R_{d2}$ and/or any combination of one or more groups $R_{d1}$ and $R_{d2}$ are inserted into the backbone carbon chain of the linear or branched $C_{2-60}$ alkylene group", used alone or in combination, has a known definition in the art, which can mean that

carbon-carbon bond between one or more pairs of adjacent carbon atoms in the referenced backbone carbon chain is interrupted by the groups $R_{d1}$, $R_{d2}$, or a combination of $R_{d1}$ and $R_{d2}$. Herein, examples of the above-mentioned expression "one or more groups...... are inserted into" may include, but are not limited to, that one or more (1-30, 1-20, or 1-15, 1-10, 1-9, 1-8, 1-7, 1-6, 1-5, 1-4, 1-3 or 1-2, or 1) groups Rai as defined herein and/or one or more (1-30, 1-20, or 1-15, 1-10, 1-9, 1-8, 1-7, 1-6, 1-5, 1-4, 1-3 or 1-2, or 1) groups $R_{d2}$ as defined herein and/or one or more (1-30, 1-20, or 1-15, 1-10, 1-9, 1-8, 1-7, 1-6, 1-5, 1-4, 1-3 or 1-2, or 1) combinations of $R_{d1}$ with $R_{d2}$ are inserted into the backbone carbon chain, and the resulting backbone chain group conforms to the covalent bond theory. For example, the expression "one or more groups $R_{e2}$ and/or one or more groups $R_{e3}$ and/or any combination of one or more groups $R_{e2}$ and $R_{e3}$ are inserted into the backbone carbon chain of the linear or branched $C_{2-30}$ alkylene group" can refer to that one or more (e.g., 1-30, 1-20, 1-15, 1-10, 1-8, 1-7, 1-6, 1-5, 1-4, 1-3, 1-2 or 1) groups $R_{e2}$ and/or $R_{e3}$ and/or any combination of one or more groups $R_{e2}$ with $R_{e3}$ are inserted between one or more pairs of any two adjacent carbon atoms of the backbone carbon chain of the linear or branched $C_{2-30}$ alkylene group, resulting in the formation of a backbone chain group containing one or more (e.g., 1-30, 1-20, 1-15, 1-10, 1-8, 1-7, 1-6, 1-5, 1-4, 1-3, 1-2 or 1) fragments "$-CH_2-R_{e2}-CH_2-$" and/or one or more (e.g., 1-30, 1-20, 1-15, 1-10, 1-8, 1-7, 1-6, 1-5, 1-4, 1-3, 1-2 or 1) fragments "$-CH_2-R_{e3}-CH_2-$" and/or one or more (e.g., 1-30, 1-20, 1-15, 1-10, 1-8, 1-7, 1-6, 1-5, 1-4, 1-3, 1-2 or 1) fragments "$-CH_2-R_{e2}-R_{e3}-CH_2-$", where each $R_{e2}$ are the same or different, each $R_{e3}$ are the same or different, and are as defined herein.

Herein, it should be understood that the expression "one or more groups $R_{e2}$ and/or one or more groups $R_{e3}$ or any combination of one or more groups $R_{e2}$ and $R_{e3}$ are optionally inserted into the backbone carbon chain of the linear or branched $C_{2-30}$ alkylene group" includes embodiments where "one or more groups $R_{e2}$ and/or one or more groups $R_{e3}$ or any combination of one or more groups $R_{e2}$ and $R_{e3}$ are inserted into the backbone carbon chain of the linear or branched $C_{2-30}$ alkylene group", as well as embodiments where "none of one or more groups $R_{e2}$ and/or one or more groups $R_{e3}$ or any combination of one or more groups $R_{e2}$ and $R_{e3}$ are inserted into the backbone carbon chain of the linear or branched $C_{2-30}$ alkylene group".

Herein, a bond interrupted by a wavy line shows the point of attachment of the depicted group to the rest of the molecule. For example, the group $R_{a1}$ depicted below

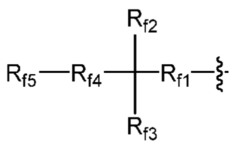

shows the point of attachment of $R_{f1}$ in said group to $X_2$. For example, the group $R_{f1}$ depicted below:

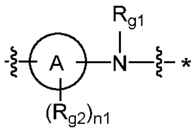

,

symbol * indicates the point of attachment of the atom N to $X_2$, while ring A is connected to the methylene group of $R_{a1}$. Herein, when the attachment point of the group is not specified, e.g., for the group $-(CH_2)_6-C(O)-(CH_2)_1-$ represented by $L_2$, either end of the group (e.g., $(CH_2)_1$) can be attached to the quinazoline ring of the compound of Formula (I), while the other end is attached to $X_4$.

As used herein, the expression "a hydrogen atom of one or more $CH_2$ of the linear or branched $C_{x-y}$ alkylene is replaced with......", used alone or in combination, means that a hydrogen atom of any one or more $CH_2$ of the linear or branched $C_{x-y}$ alkylene is replaced by a substituent(s) as defined herein. Herein, the term "one or more" of "a hydrogen atom of one or more $CH_2$ of groups $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{15}-$, $-(CH_2)_{16}-$, $-(CH_2)_{17}-$, $-(CH_2)_{18}-$, $-(CH_2)_{19}-$, $-(CH_2)_{20}-$, $-(CH_2)_{21}-$, $-(CH_2)_{22}-$, $-(CH_2)_{25}-$, or $-(CH_2)_{30}-$" may refer to part or all hydrogen atoms of each referenced alkylene group, including but not limited to 1-60 hydrogens. In some embodiments, the expression "a hydrogen atom of one or more $CH_2$" may refer to part or all of the hydrogen atoms of the referenced alkylene group, including but not limited to 1-30, such as 1-25, 1-20, 1-15, 1-10, 1-5, 1-4, 1-3, 1-2 or 1 hydrogen atoms. In some embodiments, the expression "a hydrogen atom of one or more $CH_2$" may include 1-3 of the plurality of hydrogen atoms of the referenced alkylene group. The number of hydrogens to be replaced is in principle not limited in any way, or is automatically limited by the size of the building unit.

As used herein, the term "halogen atom" or "halogen", used alone or in combination, refers to fluorine, chlorine, bromine, or iodine.

As used herein, the term "alkyl", used alone or in combination, refers to a linear or branched alkyl group. The term "$C_x$-$C_y$ alkyl" or "$C_{x-y}$ alkyl" (x and y each being an integer) refers to a linear or branched alkyl group containing from x to y

carbon atoms. The term "$C_{1-60}$ alkyl" used alone or in combination in the present disclosure refers to a linear or branched alkyl group containing from 1 to 60 carbon atoms, examples of which include $C_1$-$C_{60}$ alkyl, $C_1$-$C_{40}$ alkyl, and $C_1$-$C_{30}$ alkyl and the like. The term "$C_{1-30}$ alkyl" used alone or in combination in the present disclosure refers to a linear or branched alkyl group containing from 1 to 30 carbon atoms. Examples of the $C_{1-60}$ alkyl of the present disclosure may include a $C_1$-$C_{40}$ alkyl, $C_1$-$C_{30}$ alkyl, $C_1$-$C_{29}$ alkyl, $C_1$-$C_{28}$ alkyl, $C_1$-$C_{27}$ alkyl, $C_1$-$C_{26}$ alkyl, $C_1$-$C_{25}$ alkyl, $C_1$-$C_{24}$ alkyl, $C_1$-$C_{23}$ alkyl, $C_1$-$C_{22}$ alkyl, $C_1$-$C_{21}$ alkyl, $C_1$-$C_{20}$ alkyl, $C_1$-$C_{19}$ alkyl, $C_1$-$C_{18}$ alkyl, $C_1$-$C_{17}$ alkyl, $C_1$-$C_{16}$ alkyl, $C_1$-$C_{15}$ alkyl, $C_1$-$C_{14}$ alkyl, $C_1$-$C_{13}$ alkyl, $C_1$-$C_{12}$ alkyl, $C_1$-$C_{11}$ alkyl, $C_1$-$C_{10}$ alkyl, $C_1$-$C_9$ alkyl, $C_1$-$C_8$ alkyl, $C_1$-$C_7$ alkyl, $C_1$-$C_6$ alkyl, $C_1$-$C_5$ alkyl, $C_1$-$C_4$ alkyl, $C_1$-$C_3$ alkyl, and $C_1$-$C_2$ alkyl. The term "$C_{1-10}$ alkyl" used alone or in combination in the present disclosure refers to a linear or branched alkyl group containing from 1 to 10 carbon atoms. Examples of the $C_{1-10}$ alkyl of the present disclosure may include a $C_{1-9}$ alkyl, $C_{1-8}$ alkyl, $C_{2-8}$ alkyl, $C_{1-7}$ alkyl, $C_{1-6}$ alkyl, $C_{1-5}$ alkyl, $C_{1-4}$ alkyl, and $C_{1-3}$ alkyl. Representative examples include methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, and decyl. The term "$C_{1-3}$ alkyl" or "$C_1$-$C_3$ alkyl" in the present disclosure refers to an alkyl group containing from 1 to 3 carbon atoms, and representative examples of which include methyl, ethyl, n-propyl, and isopropyl. In the present disclosure, the "alkyl" is optionally substituted with one or more substituents optionally selected from the group consisting of halogen, hydroxy, cyano, $C_{1-3}$ alkyl, $C_{1-3}$ alkoxy, trifluoromethyl, heterocyclyl, or a combination thereof.

As used herein, the term "halogenated alkyl" or "haloalkyl", used alone or in combination, refers to a linear or branched alkyl group substituted with one or more halogens, wherein one or more hydrogen atom(s) of the alkyl group are replaced with one or more halogens. The term "halogenated $C_{x-Cy}$ alkyl" or "halogenated $C_{x-y}$ alkyl" (x and y are each an integer) refers to a linear or branched alkyl containing from x to y carbon atoms substituted with one or more halogens. The term "halogenated $C_{1-10}$ alkyl" used alone or in combination in the present invention refers to a linear or branched alkyl group containing from 1 to 10 carbon atoms substituted with one or more halogens. Examples of the halogenated $C_{1-10}$ alkyl group of the present disclosure include halogenated $C_{1-9}$ alkyl group, e.g., halogenated $C_{1-8}$ alkyl group, halogenated $C_{2-8}$ alkyl group, halogenated $C_{1-7}$ alkyl group, halogenated $C_{1-6}$ alkyl, halogenated $C_{1-5}$ alkyl, or halogenated $C_{1-4}$ alkyl. Representative examples include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, halohexyl, haloheptyl, halooctyl, halononyl, and halodecyl. The term "halo-$C_{1-3}$ alkyl" or "halo-$C_{1-}C_3$ alkyl" of the present disclosure refers to an alkyl group containing from 1 to 3 carbon atoms substituted with one or more halogens, and its representative examples include halomethyl, haloethyl, halo-n-propyl and haloisopropyl.

As used herein, the term "deuterated alkyl", used alone or in combination, refers to a linear or branched alkyl group substituted with one or more deuterium atoms, wherein one or more hydrogen atom(s) of the alkyl group are replaced with one or more deuterium atoms. The term "deuterated $C_{x-Cy}$ alkyl" or "deuterated $C_{x-y}$ alkyl" (x and y are each an integer) refers to a linear or branched alkyl containing from x to y carbon atoms substituted with one or more deuterium atoms. The term "deuterated $C_{1-10}$ alkyl" used alone or in combination in the present invention refers to a linear or branched alkyl group containing from 1 to 10 carbon atoms substituted with one or more deuterium atoms. Examples of the deuterated $C_{1-10}$ alkyl group of the present disclosure include deuterated $C_{1-9}$ alkyl group, e.g., deuterated $C_{1-8}$ alkyl group, deuterated $C_{2-8}$ alkyl group, deuterated $C_{1-7}$ alkyl group, deuterated $C_{1-6}$ alkyl, deuterated $C_{1-5}$ alkyl, or deuterated $C_{1-4}$ alkyl. Representative examples include perdeuterated methyl ($CD_3$), perdeuterated ethyl ($CD_3CD_2$), perdeuterated n-propyl, perdeuterated isopropyl, perdeuterated n-butyl, perdeuterated isobutyl, perdeuterated sec-butyl, perdeuterated tert-butyl, perdeuterated pentyl, perdeuterated isopentyl, perdeuterated neopentyl, perdeuterated tert-pentyl, perdeuterated hexyl, perdeuterated heptyl, perdeuterated octyl, perdeuterated nonyl, and perdeuterated decyl. The term "deuterated $C_{1-3}$ alkyl" or "deuterated $C_1$-$C_3$ alkyl" of the present disclosure refers to an alkyl group containing from 1 to 3 carbon atoms substituted with one or more deuterium atoms, and its representative examples include perdeuterated methyl ($CD_3$) and perdeuterated ethyl ($CD_3CD_2$).

As used herein, the term "alkylene" (which is used interchangeably with "alkylene chain"), used alone or in combination, refers to a linear or branched divalent saturated hydrocarbon group composed of carbon and hydrogen atoms. The term "$C_x$-$C_y$ alkylene" or "$C_{x-y}$ alkylene" (x and y each being an integer) refers to a linear or branched alkylene group containing from x to y carbon atoms. The term "$C_1$-$C_{60}$ alkylene" or "$C_{1-60}$ alkylene" refers to a linear or branched alkylene group containing from 1 to 60 carbon atoms, examples of which include $C_1$-$C_{60}$ alkylene, $C_1$-$C_{40}$ alkylene, and $C_1$-$C_{30}$ alkylene and the like. Examples of the $C_1$-$C_{30}$ alkylene in the present disclosure may include $C_1$-$C_{30}$ alkylene, $C_1$-$C_{29}$ alkylene, $C_1$-$C_{28}$ alkylene, $C_1$-$C_{27}$ alkylene, $C_1$-$C_{26}$ alkylene, $C_1$-$C_{25}$ alkylene, $C_1$-$C_{24}$ alkylene, $C_1$-$C_{23}$ alkylene, $C_1$-$C_{22}$ alkylene, $C_1$-$C_{21}$ alkylene, $C_1$-$C_{20}$ alkylene, $C_1$-$C_{19}$ alkylene, $C_1$-$C_{18}$ alkylene, $C_1$-$C_{17}$ alkylene, $C_1$-$C_{16}$ alkylene, $C_1$-$C_{15}$ alkylene, $C_1$-$C_{14}$ alkylene, $C_1$-$C_{13}$ alkylene, $C_1$-$C_{12}$ alkylene, $C_1$-$C_{11}$ alkylene, $C_1$-$C_{10}$ alkylene, $C_1$-$C_9$ alkylene, $C_1$-$C_8$ alkylene, $C_1$-$C_7$ alkylene, $C_1$-$C_6$ alkylene, $C_1$-$C_5$ alkylene, $C_1$-$C_4$ alkylene, $C_1$-$C_3$ alkylene, or $C_1$-$C_2$ alkylene. Representative examples include, but are not limited to, methylene, ethylene, propylene, isopropylene, butylene, isobutylene, sec-butylene, tert-butylene, n-pentylene, isopentylene, neopentylidene, tert-pentylene, hexylene, heptylene, octylene, nonylene, decylene, undecylene, dodecylene, tridecylene, tetradecylene, pentadecylene, hexadecylene, heptadecylene, octadecylene, nonadecylene, eicosylene, heneicosylene, docosylene, tricosylene, tetracosylene, pentacosylene, hexacosylene, heptacosylene, octacosylene, nonacosylene, and triacontylene. In the present disclosure, the "alkylene" is optionally substituted with one or more substituents optionally selected from $C_{1-3}$ alkyl, $C_{3-6}$ cycloalkyl, hydroxyl, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "alkoxy", used alone or in combination, refers to a linear or branched alkoxy group having structural formula of -O-alkyl. Optionally, the alkyl portion of the alkoxy group may contain 1-10 carbon atoms. Representative examples of "alkoxy" include, but are not limited to, methoxy, ethoxy, propoxy, isopropoxy, n-butoxy, isobutoxy, tert-butoxy, pentyloxy, 2-pentyloxy, isopentyloxy, neopentyloxy, hexyloxy, 2-hexyloxy, 3-hexyloxy, 3-methylpentyloxy, etc. The term "$C_1$-$C_3$ alkoxy" or "$C_{1-3}$ alkoxy" refers to a linear or branched alkoxy group containing from 1 to 3 carbon atoms. Representative examples of $C_{1-3}$ alkoxy include, but are not limited to, methoxy, ethoxy, n-propoxy, and isopropoxy.

As used herein, the term "halogenated alkoxy", used alone or in combination, refers to a alkoxy group substituted with one or more halogen atoms. Optionally, the alkyl portion of the alkoxy group may contain 1-10 carbon atoms. Examples of "halogenated alkoxy" include, but are not limited to, halogenated $C_{1-6}$ alkoxy or halogenated $C_{1-4}$ alkoxy. Representative examples include, but are not limited to, $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-.

As used herein, the term "alkylamino", used alone or in combination, refers to a linear or branched alkyl-NH-, wherein alkyl is as defined above. Optionally, the alkyl portion of the alkylamino group may contain 1-10 carbon atoms, i.e., $C_{1-10}$ alkyl-NH-. Representative examples of "alkylamino" include, but are not limited to, methyl-NH-, ethyl-NH-, n-propyl-NH-, isopropyl-NH-, n-butyl-NH-, isobutyl-NH-, tert-butyl-NH-, pentyl-NH-, and hexyl-NH-, etc. The term "$C_1$-$C_3$ alkyl-NH-" or "$C_{1-3}$ alkyl-NH-" refers to a linear or branched alkyl-NH- group containing from 1 to 3 carbon atoms. Representative examples of $C_{1-3}$ alkyl-NH- include, but are not limited to, methyl-NH-, ethyl-NH-, n-propyl-NH-, and isopropyl-NH-.

As used herein, the term "$NH_2$-alkylene", used alone or in combination, refers to a linear or branched alkylene group substituted with amino, wherein alkyl is as defined above. Optionally, the alkylene portion of the "$NH_2$-alkylene" group may contain 1-10 carbon atoms. The term "$NH_2$-$C_{1-3}$ alkylene" or "amino-$C_{1-3}$ alkylene-" refers to a linear or branched alkylene group containing from 1 to 3 carbon atoms which is substituted with amino. Representative examples of $NH_2$-$C_{1-3}$ alkylene include, but are not limited to, $NH_2$-$CH_2$-, $NH_2$-$CH_2CH_2$-, and $NH_2$-$CH_2CH_2CH_2$-.

As used herein, the term "alkyl-NHC(O)-", used alone or in combination, refers to a linear or branched alkyl-NHC(O)- group, wherein alkyl is as defined above. Optionally, the alkyl portion of the alkyl-NHC(O)- group may contain 1-10 carbon atoms. The term "$C_{1-3}$ alkyl-NHC(O)-" or "$C_1$-$C_3$ alkyl-NHC(O)-" refers to a linear or branched alkyl-NHC(O)- group containing from 1 to 3 carbon atoms. Representative examples of $C_{1-3}$ alkyl-NHC(O)- include, but are not limited to, $CH_3$-NHC(O)-, $CH_3CH_2$-NHC(O)-, and $CH_3CH_2CH_2$-NHC(O)-.

As used herein, the term "alkyl-C(O)NH-", used alone or in combination, refers to a linear or branched alkyl-C(O) NH- group, wherein alkyl is as defined above. Optionally, the alkyl portion of the alkyl-C(O)NH- group may contain 1-10 carbon atoms. The term "$C_{1-3}$ alkyl-C(O)NH-" or "$C_1$-$C_3$ alkyl-C(O)NH-" refers to a linear or branched alkyl-C(O)NH- group containing from 1 to 3 carbon atoms. Representative examples of $C_{1-3}$ alkyl-C(O)NH- include, but are not limited to, $CH_3$-C(O)NH-, $CH_3CH_2$-C(O)NH-, and $CH_3CH_2CH_2$-C(O)NH-.

As used herein, the term "heteroaryl", used alone or in combination, refers to a 5- to 20-membered (e.g., 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) monocyclic, bicyclic, or polycyclic cyclic aromatic ring radical containing one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic or tetracyclic heteroaryl groups, which contain one aromatic ring having one or more heteroatoms independently selected from O, S and N, and the remaining rings which may be a saturated, partially unsaturated or aromatic ring and can be carbocyclic ring or contain one or more heteroatoms independently selected from O, S and N. Examples of monocyclic heteroaryl groups include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, tetrazolyl, and triazinyl. Examples of bicyclic heteroaryl groups include, but are not limited to, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, oxazolopyridyl, furopyridyl, pteridyl, purinyl, pyridopyridyl, pyrazolo[1,5-a]pyridyl, pyrazolo[1,5-a]pyrimidinyl, imidazo[1,2-a]pyridyl, 1H-pyrrolo[3,2-b] pyridyl, 1H-pyrrolo[2,3-b]pyridyl, pyrrolo[2,1-b]thiazolyl and imidazo[2,1-b]thiazolyl. Examples of tricyclic heteroaryl groups include, but are not limited to, acridinyl, benzindolyl, carbazolyl, dibenzofuranyl, and xanthyl. The heteroaryl group may be unsubstituted or substituted. A substituted heteroaryl refers to heteroaryl substituted one or more times (e.g., 1-4, 1-3, or 1-2 times) by a substituent(s) optionally selected from the group consisting of $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "heteroarylene", used alone or in combination, refers to a 5- to 20-membered (e.g., 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) bivalent monocyclic, bicyclic, or polycyclic cyclic aromatic ring radical containing one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic or tetracyclic heteroarylene groups, which contain one aromatic ring having one or more heteroatoms independently selected from O, S and N, and the remaining ring(s) which may be a saturated, partially unsaturated or aromatic ring and can be carbocyclic ring or contain one or more heteroatoms independently selected from O, S and N. Examples of monocyclic heteroarylene groups include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, and triazinylene. Examples of bicyclic heteroarylene groups include, but are not limited to, indolylene, isoindolylene, isoindolinylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, oxazolopyridylene, furopyridylene, pteridylene, purinylene, pyridopyridylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene. Examples of tricyclic heteroarylene groups include, but are not limited to, acridinylene, benzindolylene, carbazolylene, dibenzofuranylene, and xanthylene. The heteroarylene group may be unsubstituted or substituted. A substituted heteroarylene refers to heteroarylene substituted one or more times (e.g., 1-4, 1-3, or 1-2 times) by a substituent(s) optionally selected from the group consisting of $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "aryl", used alone or in combination, refers to a monovalent aromatic hydrocarbon group containing from 5 to 14 carbon atoms and optionally one or more fused rings, such as phenyl group, naphthyl group, or fluorenyl group. As used herein, the "aryl" is optionally substituted. A substituted aryl group refers to an aryl group substituted one or more times (e.g., 1-4, 1-3, or 1-2 times) with a substituent(s). For example, aryl is mono-, di-, or tri-substituted with a substituent(s) optionally selected from e.g., $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "arylene", used alone or in combination, refers to a divalent aromatic hydrocarbon group containing from 5 to 14 carbon atoms and optionally one or more fused rings, such as phenylene, naphthylene, or fluorenylene. As used herein, the "arylene" is optionally substituted. A substituted arylene refers to an arylene group optionally substituted one or more times (e.g., 1-4, 1-3, or 1-2 times) with a substituent(s). For example, arylene is mono-, di-, or tri-substituted with a substituent(s) optionally selected from e.g., $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "cycloalkyl", used alone or in combination, refers to a saturated or partially unsaturated (i.e., containing one or more double bonds, but not having a fully conjugated $\pi$-electron system) monocyclic or bicyclic or polycyclic cyclic hydrocarbon radical, which in some embodiments contains from 3 to 20 carbon atoms (i.e., $C_{3-20}$ cycloalkyl), or from 3 to 15 carbon atoms (i.e., $C_{3-15}$ cycloalkyl), or from 3 to 12 carbon atoms (i.e., $C_{3-12}$ cycloalkyl), or from 3 to 11 carbon atoms (i.e., $C_{3-11}$ cycloalkyl), or from 3 to 10 carbon atoms (i.e., $C_{3-10}$ cycloalkyl), or from 3 to 8 carbon atoms (i.e., $C_{3-8}$ cycloalkyl), or from 3 to 7 carbon atoms (i.e., $C_{3-7}$ cycloalkyl), or from 3 to 6 carbon atoms (i.e., $C_{3-6}$ cycloalkyl). The term "cycloalkyl" includes monocyclic, bicyclic, or tricyclic cyclic hydrocarbon radical having from 3 to 20 carbon atoms. Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Bicyclic and tricyclic cycloalkyl groups include bridged cycloalkyl (e.g., $C_{5-15}$ bridged cycloalkyl), fused cycloalkyl and spiro-cycloalkyl groups (e.g., $C_{5-15}$ spiro-cycloalkyl) such as, but not limited to, decalinyl, octahydropentalenyl, octahydro-1H-indenyl, spiro-cycloalkyl (e.g., $C_{5-11}$ spiro-cycloalkyl), adamantanyl, noradamantanyl, bornyl, norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" can be optionally one or more (e.g., 1-5, 1-4, 1-3, 1-2, or 1) substituents selected from $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof. Examples of "$C_{3-6}$ cycloalkyl" include, but are not limited to, cyclopropyl, cyclobutyl, cyclopentyl, cyclopentenyl, and cyclohexyl.

As used herein, the term "cycloalkylene", used alone or in combination, refers to a saturated or partially unsaturated (i.e., containing one or more double bonds, but not having a fully conjugated $\pi$-electron system) monocyclic or bicyclic or polycyclic divalent cyclic hydrocarbon radical containing from 3 to 12 carbon atoms (e.g., 3-12, 3-11, 3-10, 3-8,

3-7, 3-6 carbon atoms). The term "cycloalkylene" includes monocyclic, bicyclic or tricyclic cycloalkylene having from 3 to 12 carbon atoms. Representative examples of monocyclic cycloalkylene groups include, but are not limited to, cyclopropylene, cyclobutylene, cyclopentylene, cyclopentenylene, cyclohexylene, cyclohexenylene, cycloheptylene, and cyclooctylene. Bicyclic and tricyclic cycloalkylene groups include bridged cycloalkylene (e.g., $C_{5-15}$ bridged cycloalkylene), fused cycloalkylene and spiro-cycloalkylene groups (e.g., $C_{5-15}$ spiro-cycloalkylene) such as, but not limited to, decalinylene, octahydropentalenylene, octahydro-1H-indenylene, 2,3-dihydro-1H-indenylene, spiro-cycloalkylene (e.g., $C_{5-11}$ spiro-cycloalkylene), adamantanylene, noradamantanylene, and norbomylene (also named as bicyclo[2.2.1] heptylene by the IUPAC system). As used herein, the "cycloalkylene" is optionally mono- or poly-substituted, such as, but not limited to, 2,2-, 2,3-, 2,4-, 2,5-, or 2,6-disubstituted cyclohexylene. The substituents of the substituted "cycloalkylene" can be optionally one or more substituents selected from $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "$C_{x-y}$ spiro-cycloalkyl" (x and y each being an integer), used alone or in combination, refers to a spiro-cycloalkyl group containing from x to y carbon atoms. As used herein, the term "$C_{7-11}$ spiro-cycloalkyl", used alone or in combination, refers to a spiro-cycloalkyl group containing from 7 to 11 carbon atoms (e.g., 7-10, 7-9 carbon atoms). The term "$C_{5-15}$ spiro-cycloalkyl" includes "$C_{7-15}$ spiro-cycloalkyl" and "$C_{5-11}$ spiro-cycloalkyl". Representative examples of "$C_{7-11}$ spiro-cycloalkyl" include, but are not limited to, spiro[3.3]heptyl, spiro[2.5]octyl, spiro[3.5]nonyl, spiro [4.4]nonyl, spiro[4.5]decyl, or spiro[5.5]undecyl. Herein, the term "$C_{x-y}$ spiro-cycloalkyl" is optionally substituted. The "$C_{x-y}$ spiro-cycloalkyl" is optionally further substituted with one or more (e.g., 1-10, 1-8, 1-6, 1-4, 1-3 or 1-2) substituents selected from the group consisting of $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "$C_{x-y}$ spiro-cycloalkylene" (x and y each being an integer), used alone or in combination, refers to a spiro-cycloalkylene group containing from x to y carbon atoms. As used herein, the term "$C_{7-11}$ spiro-cycloalkylene", used alone or in combination, refers to a spiro-cycloalkylene group containing from 7 to 11 carbon atoms (e.g., 7-10, 7-9 carbon atoms). The term "$C_{5-15}$ spiro-cycloalkylene" includes "$C_{7-15}$ spiro-cycloalkylene" and "$C_{5-11}$ spiro-cycloalkylene". Representative examples of "$C_{7-11}$ spiro-cycloalkylene" include, but are not limited to, spiro[3.3]heptylene, spiro[2.5]octylene, spiro[3.5]nonylene, spiro[4.4]nonylene, spiro[4.5]decylene, or spiro[5.5]undecylene. Herein, the term "$C_{x-y}$ spiro-cycloalkylene" is optionally substituted. The "$C_{x-y}$ spiro-cycloalkylene" is optionally further substituted with one or more (e.g., 1-10, 1-8, 1-6, 1-4, 1-3 or 1-2) substituents selected from the group consisting of $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "$C_{x-y}$ bridged cycloalkyl" (x and y each being an integer), used alone or in combination, refers to a bridged cycloalkyl group containing from x to y carbon atoms. As used herein, the term "$C_{5-15}$ bridged cycloalkyl", used alone or in combination, refers to a bridged cycloalkyl group containing from 5 to 15 carbon atoms, examples of which include "$C_{7-15}$ bridged cycloalkyl" and "$C_{5-11}$ bridged cycloalkyl". Representative examples of "$C_{5-15}$ bridged cycloalkyl" include, but are not limited to, adamantanyl, noradamantanyl, norbornyl (also named as bicyclo[2.2.1] heptyl by the IUPAC system), and cubanyl. Herein, the term "$C_{x-y}$ bridged cycloalkyl" is optionally substituted. The "$C_{x-y}$ bridged cycloalkyl" is optionally further substituted with one or more (e.g., 1-10, 1-8, 1-6, 1-4, 1-3 or 1-2) substituents selected from the group consisting of $C_{1-3}$ alkyl, deuterated $C_{1-3}$ alkyl, $C_{1-3}$ alkoxy, halogen, halogenated $C_{1-3}$ alkyl, $C_{1-3}$ alkylamino, amino, hydroxy, cyano, oxo, or any combination thereof.

As used herein, the term "$C_{x-y}$ bridged cycloalkylene" (x and y each being an integer), used alone or in combination, refers to a bridged cycloalkylene group containing from x to y carbon atoms. As used herein, the term "$C_{5-15}$ bridged cycloalkylene", used alone or in combination, refers to a bridged cycloalkylene group containing from 5 to 15 carbon atoms, examples of which include "$C_{7-15}$ bridged cycloalkylene" and "$C_{5-11}$ bridged cycloalkylene". Representative examples of "$C_{5-15}$ bridged cycloalkylene" include, but are not limited to, adamantanylene, noradamantanylene, norbomylene (also named as bicyclo[2.2.1]heptylene by the IUPAC system), and cubanylene. Herein, the term "$C_{x-y}$ bridged cycloalkylene" is optionally substituted. The "$C_{x-y}$ bridged cycloalkylene" is optionally further substituted with one or more (e.g., 1-10, 1-8, 1-6, 1-4, 1-3 or 1-2) substituents selected from the group consisting of $C_{1-3}$ alkyl, deuterated $C_{1-3}$ alkyl, $C_{1-3}$ alkoxy, halogen, halogenated $C_{1-3}$ alkyl, $C_{1-3}$ alkylamino, amino, hydroxy, cyano, oxo, or any combination thereof.

As used herein, the term "heterocyclyl" or "heterocyclic group", used alone or in combination, refers to a 3- to 20-membered saturated or partially unsaturated (i.e., containing one or more double bonds, but not having a fully conjugated $\pi$-electron system) monocyclic, bicyclic, or tricyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclyl" may refer to a 3- to 15-membered (e.g., 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not

having a fully conjugated π-electron system) monocyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Representative examples of the monocyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl) and diazacyclooctyl. Bicyclic and tricyclic heterocyclyl groups include bridged heterocyclyl, fused heterocyclyl and spiro-heterocyclyl groups such as, but not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, and azaspirocycloalkyl (e.g., 3-azaspiro[5.5]undecan-3-yl). The heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "nitrogen-containing monocyclic heterocyclyl", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) monocyclic monovalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Representative examples of the nitrogen-containing monocyclic heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl (e.g., 1,4-diazacycloheptan-1-yl), and diazacyclooctyl. The nitrogen-containing monocyclic heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "nitrogen-containing bridged heterocyclyl", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) monovalent tricyclic cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Tricyclic heterocyclyl includes bridged heterocyclyl, e.g., but not limited to, 6-azabicyclo[3.1.1]hept-3-yl, 2,5-diazabicyclo[2.2.1]hept-2-yl, 3,6-diazabicyclo[3.1.1]hept-3-yl, 3-azabicyclo[3.2.1]octan-8-yl, 3,8-diazabicyclo[3.2.1]octan-8-yl, 3,8-diazabicyclo[3.2.1]octan-3-yl and 2,5-diazabicyclo[2.2.2]octan-2-yl. The nitrogen-containing bridged heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "-O-optionally substituted heterocyclyl", used alone or in combination, refers to a group formed by optionally substituted heterocyclyl connected to oxygen. Optionally, the heterocyclyl of "-O-optionally substituted heterocyclyl" is e.g., 4- to 20-membered monocyclic or bicyclic cyclic heterocyclyl containing one or more (e.g., from 1 to 4, or 1, 2, 3 or 4) heteroatoms independently selected from sulfur, oxygen, and nitrogen, including but not limited to azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, diazacycloheptanyl (e.g., 1,4-diazacycloheptan-1-yl), azacyclooctyl, diazacyclooctyl, azabicyclo[3.1.1]heptanyl, azabicyclo[2.2.1]heptanyl, azabicyclo[3.2.1]octanyl, azabicyclo[2.2.2]octanyl, diazabicyclo[3.1.1]heptanyl, diazabicyclo[2.2.1]heptanyl, diazabicyclo[3.2.1]octanyl (e.g., 3,8-diazabicyclo[3.2.1]octan-3-yl), and diazabicyclo[2.2.2]octanyl (e.g., 2,5-diazabicyclo[2.2.2]octan-2-yl). As used herein, the heterocyclyl is optionally substituted with a substituent selected from deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "heterocyclylene", used alone or in combination, refers to a 3- to 20-membered saturated or partially unsaturated (i.e., containing one or more double bonds, but not having a fully conjugated π-electron

system) monocyclic, bicyclic, or tricyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. In some embodiments, "heterocyclylene" may refer to e.g., a 3- to 15-membered (optionally 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) monocyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Representative examples of the monocyclic heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, and diazacycloheptanylene (e.g., 1,4-diazacycloheptanylene, 4,5-diazacycloheptanylene, 1,3-diazacycloheptanylene). Bicyclic and tricyclic heterocyclylene groups include bridged heterocyclylene, fused heterocyclylene and spiro-heterocyclylene groups such as, but not limited to, 6-azabicyclo[3.1.1]heptanylene, 2,5-diazabicyclo[2.2.1]heptanylene, 3,6-diazabicyclo[3.1.1]heptanylene, 3-azabicyclo [3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 3-azaspiro[5.5]undecanylene). The heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "nitrogen-containing monocyclic heterocyclylene", used alone or in combination, refers to 3- to 20-membered (e.g., 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) monocyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Representative examples of the nitrogen-containing monocyclic heterocyclylene include, but are not limited to, piperidinylene, piperazinylene, morpholinylene, azetidinylene, pyrrolidinylene, imidazolidinylene, pyrazolidylene, oxazolidinylene, thiazolidinylene, thiomorpholinylene, azacycloheptylene, diazacycloheptanylene, azacyclooctylene, and diazacyclooctylene. The nitrogen-containing monocyclic heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "nitrogen-containing bridged heterocyclylene", used alone or in combination, refers to 3- to 20-membered (optionally 3- to 15-membered, 3- to 14-membered, 3- to 12-membered, 3- to 11-membered, 3- to 10-membered, 3- to 9-membered, 3- to 8-membered, 3- to 7-membered, 3- to 6-membered, 3- to 5-membered, or 4- to 9-membered) saturated or partially unsaturated (i.e., containing one or more double bonds, but not having a fully conjugated π-electron system) tricyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Tricyclic heterocyclylene groups include bridged heterocyclylene, such as but not limited to, 6-azabicyclo[3.1.1] heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, and 2,5-diazabicyclo[2.2.2]octanylene. The nitrogen-containing bridged heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof.

As used herein, the term "alkynylene", used alone or in combination, refers to a linear or branched bivalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynylene include, but are not limited to, ethynylene, 1 -propynylene, 1-butynylene, and 1,3-diynylene.

As used herein, the term "alkynyl", used alone or in combination, refers to a linear or branched monovalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of $C_{2-6}$ alkynylene include, but are not limited to, ethynyl, 1-propynyl, 1-butynyl, and 1,3-diynyl.

As used herein, the term "alkenylene", used alone or in combination, refers to a linear or branched divalent

hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of alkenylene groups include, but are not limited to, vinylene (e.g., - CH=CH-), 1-propenylene, allylidene, 1-butenylene, 2-butenylene, 3-butenylene, isobutenylene, pentenylene, n-pent-2,4-dienylene, 1-methyl-but-1-enylene, 2-methyl-but-1-enylene, 3-methyl-but-1-enylene, 1-methyl-but-2-enylene, 2-methyl-but-2-enylene, 3-methyl-but-2-enylene, 1-methyl-but-3-enylene, 2-methyl-but-3-enylene, 3-methyl-but-3-enylene, and hexenylene.

As used herein, the term "alkenyl", used alone or in combination, refers to a linear or branched monovalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of $C_{2-6}$ alkenyl group include, but are not limited to, vinyl (e.g., $CH_2$=CH-), 1-propenyl, allyl, 1-butenyl, 2-butenyl, 3-butenyl, isobutenyl, pentenyl, n-pent-2,4-dienyl, 1-methyl-but-1-enyl, 2-methyl-but-1-enyl, 3-methyl-but-1-enyl, 1-methyl-but-2-enyl, 2-methyl-but-2-enyl, 3-methyl-but-2-enyl, 1-methyl-but-3-enyl, 2-methyl-but-3-enyl, 3-methyl-but-3-enyl, and hexenyl.

As used herein, "bornylane" or "bornane" (also known as 1,7,7-trimethylbicyclo[2.2.1]heptane; camphane; bornylane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2. 1]heptan-5-yl, 1,7,7-trimethylbicyclo[2.2.1]heptan-6-yl,

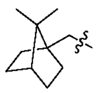
, or
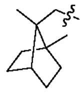
.

As used herein, "bicyclo[2.2.1]heptane" also known as "norbornane", has a definition known to those skilled in the art. As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en-3-yl, or bicyclo[2.2.1]hept-5-en-7-yl.

As used herein, "adamantane" (also known as tricyclo[3.3.1.1$^{3,7}$]decane) has a definition known to those skilled in the art, and its structural formula is e.g., as follows:

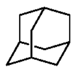

As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" (also known as octahydro-2,5-methanopentalen) has a definition known to those skilled in the art, and its structural formula is e.g., as follows:

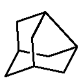
or
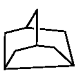

As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

As used herein, "adamantanamine" has the definitions known to those skilled in the art, namely referring to an adamantane having an amino substituent, wherein the amino substituent can replace a hydrogen on a carbon at any position in the adamantane. An example of "adamantanamine" can be adamantan-1-amine (corresponding English chemical name is adamantan-1-amine or Tricyclo[3.3.1.1$^{3,7}$]decan-1-amine; CAS No.: 768-94-5), with the following

structural Formula:

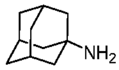

In the present disclosure, the term "leaving group" used alone or in combination is well known to those skilled in the art, which is a leaving molecular fragment (ion or neutral molecule) that carries a pair of electrons from a reactant in chemical reactions, as is a term used in nucleophilic substitution and elimination reactions. Common ionic leaving groups include $Cl^-$, $Br^-$, $I^-$ and sulfonate (such as p-toluenesulfonate, $TsO^-$), and neutral molecular leaving groups include water, ammonia and alcohol. In the present disclosure, those skilled in the art can select an appropriate leaving group as needed, such as but not limited to $-N_3$, halogen, methanesulfonyloxy ($MsO^-$), trifluoromethanesulfonyloxy ($TfO^-$) or p-toluenesulfonyloxy ($TsO^-$), etc.

Salts or pharmaceutically acceptable salts, enantiomers, stereoisomers, solvates, polymorphs of the compounds of Formula (I), Formula (II) or Formula (III) of the present disclosure are also encompassed within the scope of the present invention.

In all embodiments of the present disclosure, the salts or pharmaceutically acceptable salts of the compounds of Formula (I), Formula (II) or Formula (III) refer to non-toxic inorganic or organic acid and/or base addition salts. Examples include: sulfates, hydrohalides (including hydrochlorides and hydrobromates), maleates, sulfonates, citrates, lactates, lactobionates, L-tartrates, fumarates, L-malates, L-lactates, α-ketoglutarates, hippurates, D-glucuronates, D-gluconates, α-D-glucoheptates, glycolates, mucates, L-ascorbates, orotates, picrates, glycinates, alaninates, argininates, cinnamates, laurates, pamoates, sebacates, benzenesulfonates, methanesulfonates, ethanesulfonates, ethanedisulfonates, formates, acetates, 2,2-dichloroacetates, trimethylacetates, propionates, valerates, palmitates, triphenylacetates, 2-ethyl-butanedioates, iodates, nicotiniates, L-pyroglutamates, L-prolinates, ferulates, 2-hydroxyethanesulfonates, nitrates, gentisates, cholates, salicylate, terephthalates, glutarates, adipates, stearates, oleoates, undecylenates, camphorates, camphorsulfonates, dodecyl sulfonates, phosphates, thiocyanates, dihydrophosphates, pyrophosphates, metaphosphates, oxalates, carbonates, malonates, benzoates, mandelates, succinates, pyruvates, p-chlorobenzenesulfonates, 1,5-naphthalenedisulfonates, 3-hydroxy-2-naphthoates, 1-hydroxy-2-naphthoates, naphthalenesulfonates, glycolates, or p-toluenesulfonates, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

As used herein, "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "solvate" refers to an association or complex formed by the interaction between one or more solvent molecules and compounds of the present invention. Examples of solvents include water, isopropanol, ethanol, methanol, DMSO, ethyl acetate, acetic acid and ethanolamine. The term "hydrate" means that a complex formed with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are nonsuperimposable mirror images.

As used herein, the term "diastereomers" refers to stereoisomers that have two or more chiral centers but are not mirror images of each other. The diastereomers have different physical properties, such as melting point, boiling point, spectral properties and reactivity. The diastereomeric mixture can be separated by high-resolution analytical operations such as electrophoresis and chromatography, such as HPLC.

As used herein, the term "oxo", used alone or in combination, refers to = O.

As used herein, the term "C(O)" or "C(=O)" or "C=O", used alone or in combination, refers to carbonyl.

As used herein, the term "C(S)" or "C(=S)" or "C=S", used alone or in combination, refers to thiocarbonyl.

As used herein, "p-menthane" has the definitions known to those skilled in the art, and its structural formula is e.g., as follows:

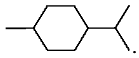.

As used herein, "p-menthanyl" refers to a monovalent group of menthane, that is, the group remaining after any hydrogen in p-menthane is removed. Representative examples of "p-menthanyl" include, but are not limited to,

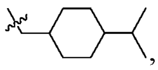, 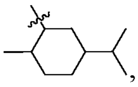,

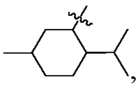, 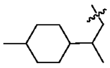 or 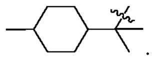.

As used herein, "m-menthane" has the definitions known to those skilled in the art, and its structural formula is e.g., as follows:

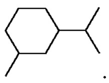.

As used herein, "m-menthanyl" refers to a monovalent group of m-menthane, that is, the group remaining after any hydrogen in m-menthane is removed. Representative examples of "m-menthanyl" include, but are not limited to,

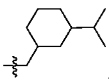, 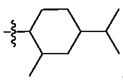, 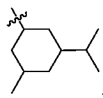,

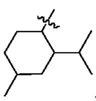, 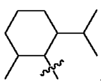, 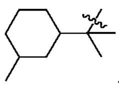, 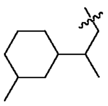 or 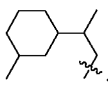.

As used herein, "Quinuclidine" (also known as 1-azabicyclo[2.2.2]octane) has the definitions known to those skilled in the art, and its structural formula is e.g., as follows:

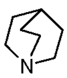

As used herein, "quinuclidinyl" refers to a monovalent group of Quinuclidine, that is, the group remaining after any hydrogen in Quinuclidine is removed. Representative examples of "quinuclidinyl" include, but are not limited to,

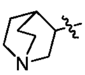, 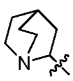, 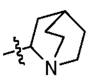, 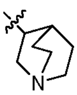, 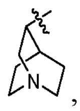, 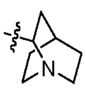, or 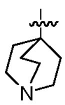

## Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present

disclosure. Although the present disclosure will be described in conjunction with specific embodiments, it should be understood that this is not intended to limit the present disclosure to these embodiments.

The following abbreviations are used throughout the specification and examples:

| | |
|---|---|
| AcOH | acetic acid |
| Boc | t-Butyloxy carbonyl |
| BINAP | 2,2'-Bis(diphenylphosphino)-1,1'-binaphthalene |
| Con. | Concentration |
| DCM | dichloromethane |
| DIAD | Diisopropyl azodicarboxylate |
| DIEA | N, N-diisopropylethylamine |
| DMAP | 4-Dimethylaminopyridine |
| DMF | N,N-dimethylformamide |
| DMSO | dimethyl sulfoxide |
| DIPEA or DIEA | N, N-diisopropylethylamine |
| EA | Ethyl acetate |
| EDCI | 1-(3-dimethylaminopropyl)-3-ethylcarbodiimide hydrochloride |
| ESI | electrospray ionization |
| equiv | equivalent |
| EtOH | ethanol |
| EtOAc | Ethyl acetate |
| HATU | 2-(7-Azabenzotriazol-1-yl)-N,N,N',N'-tetramethyluronium hexafluorophosphate |
| HPLC | high performance liquid chromatography |
| HRMS | high resolution mass spectrometry |
| LC-MS | liquid chromatography-mass spectrometry |
| LRMS | low resolution mass spectrometry |
| LC | liquid chromatography |
| Me | methyl |
| MeCN | acetonitrile |
| MeOH | Methanol |
| MeO- | Methoxy |
| MS | mass spectrometry |
| MsCl | Methanesulfonyl chloride |
| MsO- | Methanesulfonyloxy |
| $^1$H NMR | Proton nuclear magnetic resonance |
| HFIP | Hexafluoroisopropanol |
| NMP | N-methylpyrrolidone |
| rt | room temperature |
| tBu | tert-butyl |
| TEA | triethylamine |
| TFA | trifluoroacetic acid |
| $T_fO$- | Trifluoromethanesulfonate |
| TLC | thin layer chromatography |
| TMS | tetramethylsilane |
| TsCl | Tosyl chloride |
| TsO- | Tosyloxy |
| Xantphos | 4,5-Bis(diphenylphosphino)-9,9-dimethylxanthene |

In the present disclosure, the $^1$H NMR spectrum was recorded on a Bruker-500MHz nuclear magnetic resonance instrument, by using, as a solvent, $CD_3OD$ ($\delta$ = 3.31 ppm) containing 0.1% TMS (as an internal standard); or using, as a solvent, $CDCl_3$ ($\delta$ = 7.26 ppm) containing 0.1% TMS (as an internal standard); or using, as a solvent, DMSO-$d_6$ ($\delta$ = 2.50 ppm) containing 0.03% TMS (as an internal standard). LC-MS spectrum was recorded on a Sciex API 2000 mass spectrometer equipped with an Agilent 1100 binary pump and DAD, as well as an ELSD, or on an Agilent 1260-6125B single quadrupole liquid-mass spectrometer equipped with an Agilent 1260 quadrupole pump, DAD, and ELSD; HPLC preparation was measured on a SHIMADZU LC-20AP type instrument, and HPLC purity was measured on a SHIMADZU LC-30AP or Waters 1525 type instrument. Unless otherwise specified, all reactions were performed in the air atmosphere. The reactions were monitored via TLC or LC-MS.

Solvents and reagents are processed as follows:

the solvents used in the reactions such as DCM, DMF, anhydrous EtOH, and anhydrous MeOH were all purchased from Sinopharm Group; HPLC preparation uses preparation grade $CH_3CN$ and deionized water; Other reaction substrates, reagents and drugs can be commercially available without special instructions, or can be synthesized using or according to methods known in the art.

**General synthesis methods**

Compounds and/or pharmaceutically acceptable salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, racemates, enantiomers, phosphates, sulfates, hydrochlorides and prodrug forms of the compounds of Formula (I) and Formula (II) of the present disclosure according to routine techniques in the art.

Scheme 1:

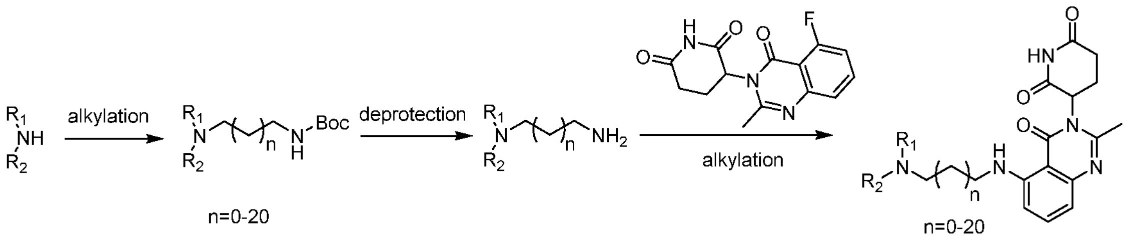


Scheme 1

In Scheme 1, the group $NR_1R_2$ corresponds to the group $R_{a1}$ or $R_{a1}$-$X_2$- in the compound of Formula (I).

Scheme 2:

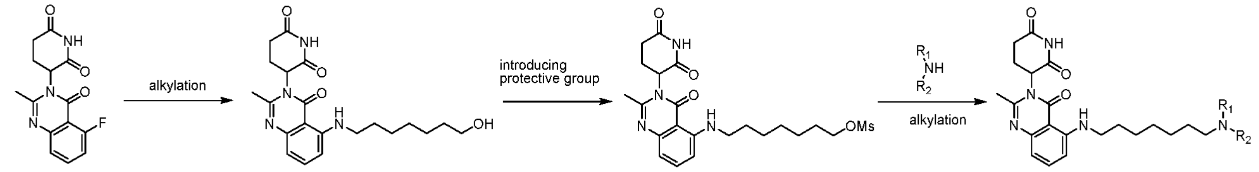


Scheme 2

In Scheme 2, the group $NR_1R_2$ corresponds to the group $R_{a1}$ or $R_{a1}$-$X_2$- in the compound of Formula (I).

In Scheme 2, the alkanolamine substrate of the alkylation reaction in step 1 may be varied depending on the target compound, e.g., by changing the carbon number to obtain the desired target compound.

Scheme 3

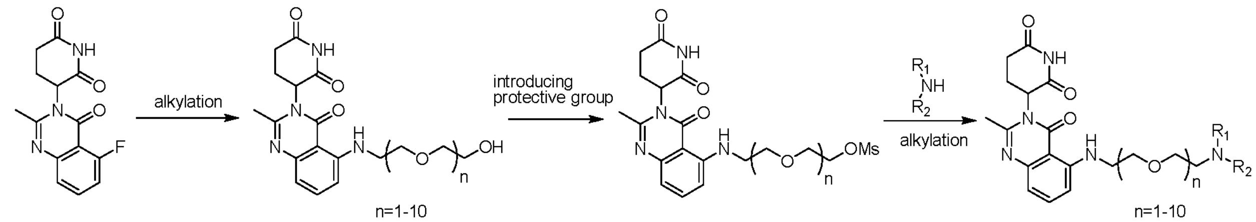


Scheme 3

In Scheme 3, the group $NR_1R_2$ corresponds to the group $R_{a1}$ or $R_{a1}$-$X_2$- in the compound of Formula (I).

Scheme 4:

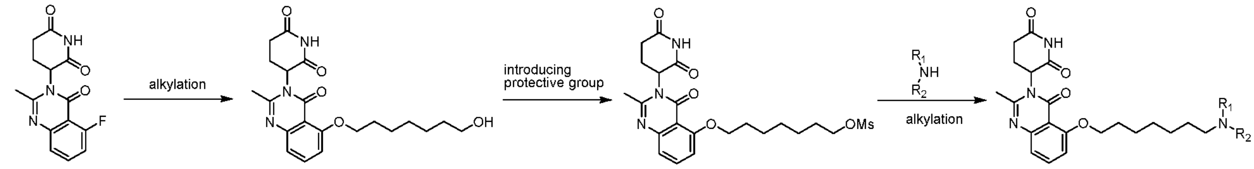


Scheme 4

In Scheme 4, the group $NR_1R_2$ corresponds to the group $R_{a1}$ or $R_{a1}$-$X_2$- in the compound of Formula (I). In Scheme 4, the diol substrate of the alkylation reaction in step 1 may be varied depending on the target compound, e.g., by changing the carbon number to obtain the desired target compound.

Scheme 5:

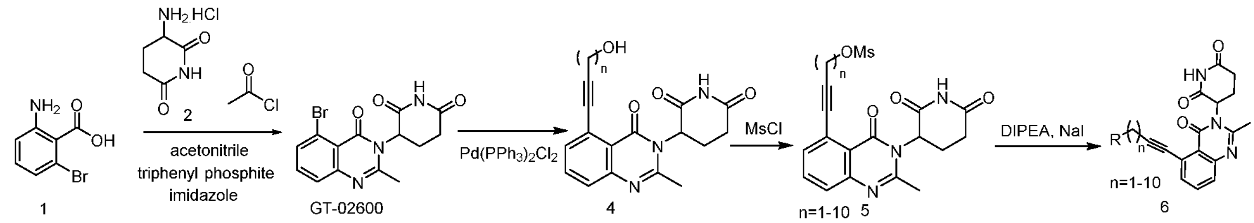


Scheme 5

In Scheme 5, n= 1-10, and the group R corresponds to the group $R_{a1}$ in the compound of Formula (I).

**Scheme 6:**

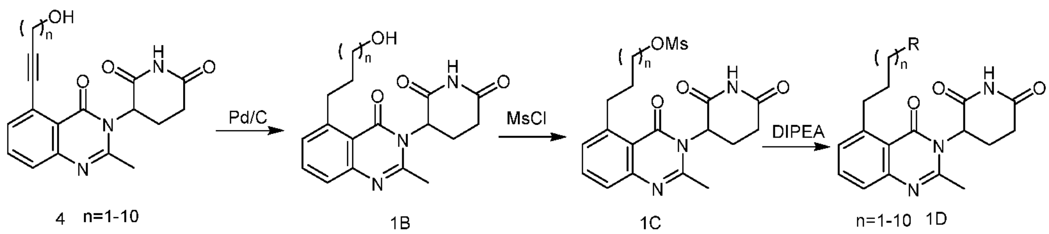


Scheme 6

In Scheme 6, n= 1-10, and the group R corresponds to the group $R_{a1}$ in the compound of Formula (I).

**Scheme 7:**

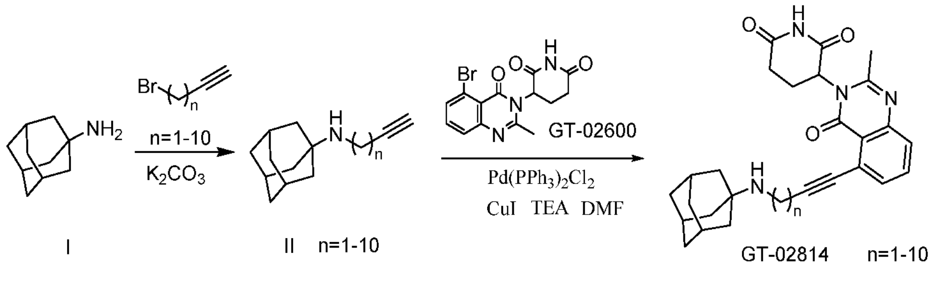


Scheme 7

## Scheme 8:

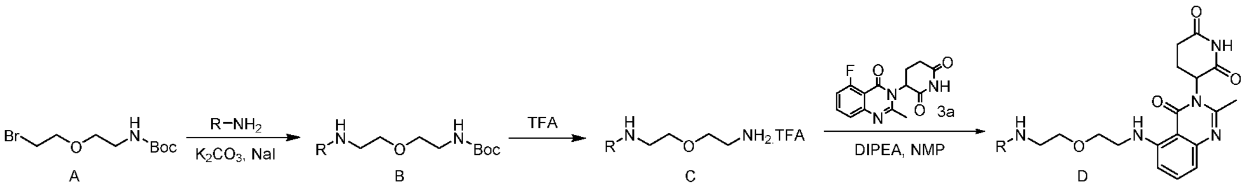


Scheme 8

In Scheme 8, the group R corresponds to the group $R_{a1}$ in the compound of Formula (I).

## Scheme 9:

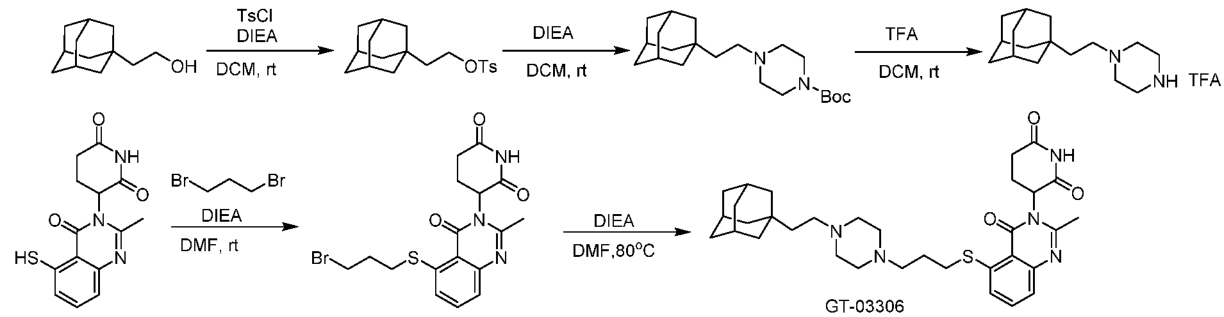


Scheme 9

## Scheme 10

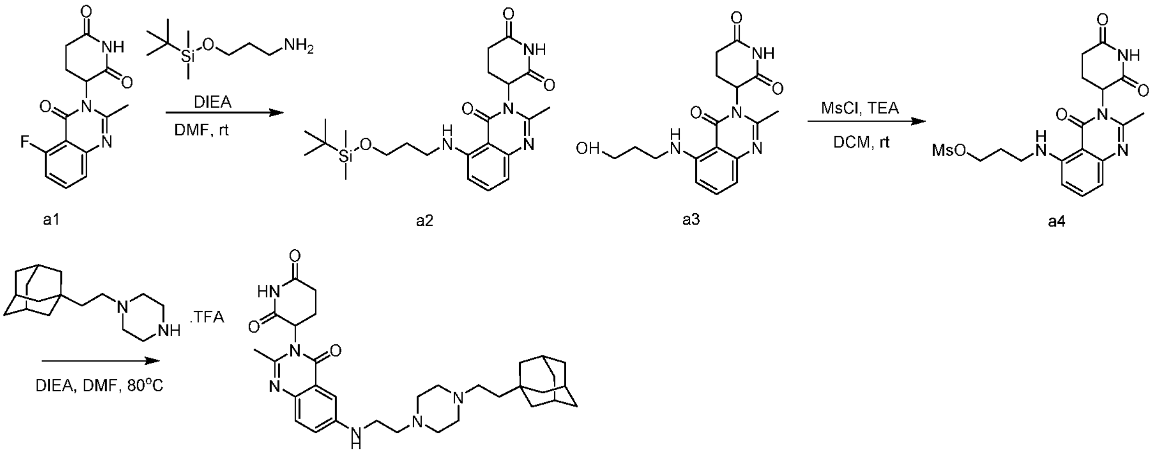


Scheme 10

## Scheme 11

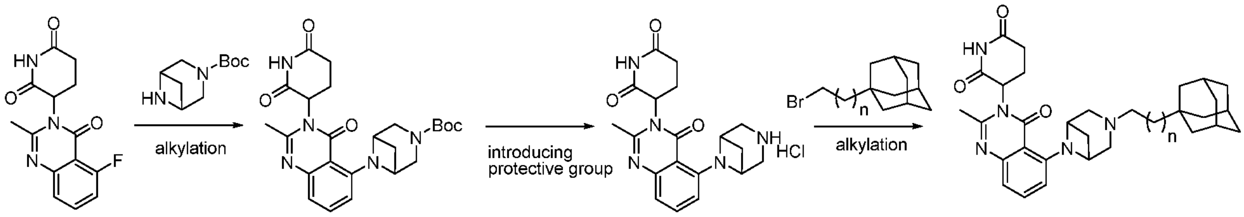


Scheme 11

In Scheme 11, n= 0-10.

**Scheme 12**

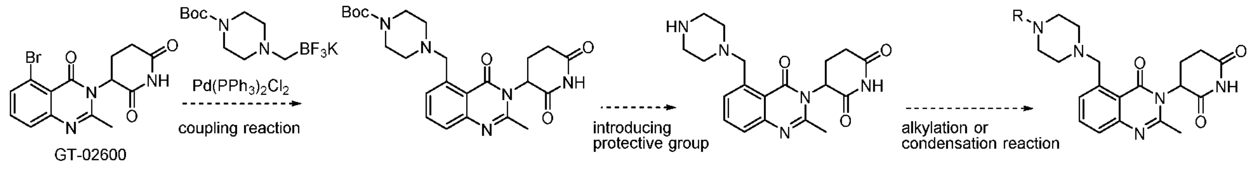


Scheme 12

Depending upon the target compound, the reaction substrate, reaction conditions (including reaction dosage, temperature, time, etc.), and work up, etc. in Scheme 12 can be appropriately modified and adjusted using techniques and methods well known to those skilled in the art to obtain the desired target compound. The group R of the product of Step 3 of Scheme 12 can be modified and adjusted depending on the target compound and the reaction used, e.g., it can be a halogenated substrate for alkylation reactions, e.g.,

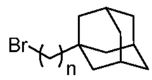
;

or a carboxylic acid substrate for condensation reaction

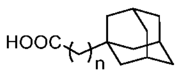
;

wherein n = 0-10.

For example, An exemplary procedure for Scheme 12 is shown below:

Step 1: to a solution of 3-(5-bromo-2-methyl-4-oxoquinazolin-3(4H)-yl)piperidine-2,6-dione (1.0 eq.) and potassium Boc-protected piperazine trifluoroborate (1.3 eq.) in dioxane were added palladium catalyst (0.2 eq.) and potassium carbonate (2.0 eq.). The reaction solution was heated to 80°C and stirred under nitrogen protection for 18 hours. After monitoring the reaction via TLC and confirming its completion, the reaction solution was cooled to room temperature, and concentrated under vacuum. The residue was washed with water, extracted with dichloromethane. The organic phases were combined, dried over anhydrous sodium sulfate, filtered and concentrated. The residue was subjected to silica gel column chromatography for purification to give tert-butyl 4-((3-(2,6-dioxopiperidin-3-yl)-2-methyl-4-oxo-3,4-dihydroquinazolin-5-yl)methyl)piperazine-1-carboxylate as a pale yellow solid.

Step 2: to a solution of the product from Step 1 in dichloromethane was added trifluoroacetic acid. The reaction solution was stirred at room temperature for 2 hours. After monitoring the reaction via LCMS and confirming its completion, the reaction solution was concentrated under vacuum to give 3-(2-methyl-4-oxo-5-(piperazin-1-ylmethyl)quinazolin-3(4H)-yl)piperidine-2,6-dione.

Step 3 (alkylation): to a solution of 3-(2-methyl-4-oxo-5-(piperazin-1-ylmethyl)quinazolin-3(4H)-yl)piperidine-2,6-dione (1.0 eq.) and halogenated substrate (1.3 eq.) in anhydrous DMF were added triethylamine (3.0 eq.) and sodium iodide (1.0 eq.). The reaction solution was stirred at 50 °C for 2 hours. After monitoring the reaction via LCMS and confirming its completion, the reaction solution was filtered. The filtrate was subjected to HPLC for purification to give the target compound.

Alternatively, Step 3 (condensation reaction): to a solution of 3-(2-methyl-4-oxo-5-(piperazin-1-ylmethyl)quinazolin-3(4H)-yl)piperidine-2,6-dione (1.0 eq.) and carboxylic acid substrate (1.3 eq.) in anhydrous DMF were added triethylamine (3.0 eq.) and HATU (1.2 eq.). The reaction solution was stirred at 50 °C for 2 hours. After monitoring the reaction via LCMS and confirming its completion, the reaction solution was filtered. The filtrate was subjected to HPLC for purification to give the target compound.

Depending upon the target compounds, the above schemes and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

## Examples

**Intermediate example 1: preparation of 3-(5-bromo-2-methyl-4-oxoquinazolin-3(4H)-yl)piperidine-2,6-dione (GT-02600)**

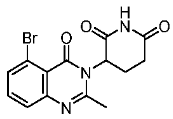

The target compound 3-(5-bromo-2-methyl-4-oxoquinazolin-3(4H)-yl)piperidine-2,6-dione (GT-02600) was prepared by referring to the method of step 1 of Scheme 5.

At room temperature, in a 500 mL single-neck flask, 2-amino-6-bromobenzoic acid (10 g, 46.29 mmol), imidazole (3.78 g, 55.55 mmol), acetonitrile (150 mL), and acetyl chloride (3.96 mL, 55.55 mmol) were sequentially added. Subsequently, the three-necked flask was vacuumed and backfilled with nitrogen. The reaction mixture was stirred overnight at room temperature, and then 3-aminopiperidine-2,6-dione hydrochloride (8.38 g, 50.92 mmol), another batch of imidazole (3.78 g, 55.548 mmol), and triphenyl phosphite (14.557 mL, 55.548 mmol) were added to the reaction mixture. The resulting reaction mixture was stirred at 80°C for 18 hours. After the reaction was completed as detected by LCMS, the mixture was added to 500 mL of water, resulting in the precipitation of a solid. The precipitated solid was washed with water and ethyl acetate, dried to give GT-02600 (off-white solid, 12 g, yield 74.0%). $^{1}$H NMR (400 MHz, DMSO) δ 11. 04 (s, 1H), 7. 73 (dt, *J* = 13.5, 6. 7 Hz, 1H), 7. 69 - 7. 53 (m, 2H), 5. 24 (dd, *J* = 11. 4, 5. 7 Hz, 1H), 2. 90 - 2. 77 (m, 1H), 2. 69 - 2. 60 (m, 4H), 2. 60 - 2.53 (m, 1H), 2. 20 - 2. 11 (m, 1H). LCMS (ESI) m/z: calcd for $C_{14}H_{13}BrN_3O_3^+$ [M+H]$^+$, 350. 01/352. 01; found, 350. 0/352. 0.

**Intermediate example 2: preparation of 3-(5-(5-hydroxypent-1-yn-1-yl)-2-methyl-4-oxoquinazolin-3(4H)-yl)piperidine-2,6-dione (GT-02597)**

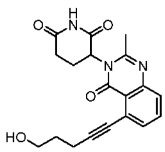

The target compound 3-(5-(5-hydroxypent-1-yn-1-yl)-2-methyl-4-oxoquinazolin-3(4H)-yl)piperidine-2,6-dione (GT-02597) was prepared by referring to the method of step 2 of Scheme 5.

A 100 mL reaction flask was charged sequentially with the compound GT-02600 (1 eq.), $Pd(PPh_3)Cl_2$ (0. 1 eq.) and cuprous iodide (1 eq.), and then under nitrogen protection, DMF (25 mL), TEA (3 mL), and pent-4-yn-1-ol (3 eq.). After the addition was complete, the mixture was heated to 90°C and stirred for 18 hours. After monitoring the reaction via TLC and confirming its completion, the reaction mixture was concentrated, and subjected to column chromatography (dichloromethane: methanol = 20:1) for purification to give the target compound GT-02597 (grayish white solid, 1.7 g, yield 93.59%). $^{1}$H NMR (400 MHz, DMSO) δ 11. 02 (s, 1H), 8. 94 (s, 1H), 7. 71 (dd, J = 8. 1, 7. 6 Hz, 1H), 7. 51 (ddd, J = 16. 4, 7. 8, 1. 1 Hz, 2H), 5. 22 (dd, J = 11. 6, 5. 7 Hz, 1H), 3.54 (dd, J = 11. 5, 6. 1 Hz, 2H), 2. 84 (ddd, J = 16. 0, 13. 2, 4. 3 Hz, 1H), 2. 72 - 2.57 (m, 5H), 2.55 - 2.52 (m, 2H), 2. 15 (dd, J = 10. 9, 5. 0 Hz, 1H), 1. 75 - 1. 61 (m, 2H). LCMS (ESI) m/z: calcd for $C_{19}H_{20}N_3O_4^+$ [M+H]$^+$, 354. 14; found, 354. 2.

**Intermediate example 3: preparation of 3-(5-(8-hydroxyoct-1-yn-1-yl)-2-methyl-4-oxoquinazolin-3(4H)-yl)piperidine-2,6-dione (GT-02637)**

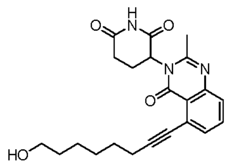

Following the method of Scheme 5 and the method of Intermediate Example 2, under appropriate conditions understood in the field, the target product GT-02637 was prepared (pale yellow solid, 1.1 g, yield 49.85%). LCMS (ESI) m/z: calcd for $C_{22}H_{26}N_3O_4^+$ [M+H]$^+$, 396. 19; found, 396. 2.

**Intermediate example 4: preparation of 5-(3-(2,6-dioxopiperidin-3-yl)-2-methyl-4-oxo-3,4-dihydroquinazolin-5-yl)pent-4-yn-1-yl methanesulfonate (729092)**

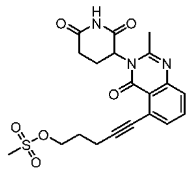

The target compound 5-(3-(2,6-dioxopiperidin-3-yl)-2-methyl-4-oxo-3,4-dihydroquinazolin-5-yl)pent-4-yn-1-yl methanesulfonate (729092) was prepared by referring to the method of step 3 of Scheme 5.

A 100 mL reaction flask was charged sequentially with 3-(5-(5-hydroxypent-1-yn-1-yl)-2-methyl-4-oxoquinazolin-3(4H)-yl)piperidine-2,6-dione (GT-02597) (1 eq.) and DCM (50 mL), and then under nitrogen protection at room temperature, TEA (3 eq.) and MsCl (2 eq.). After the addition was complete, the mixture was stirred at room temperature for 1 hour. After monitoring the reaction via TLC and confirming its completion, the reaction mixture was washed with water twice and brine once, concentrated, and then slurried (petroleum ether: ethyl acetate = 1: 1) to give the target compound **(729092)** (pale yellow solid, 170 mg, yield 84.23%). LCMS (ESI) m/z: calcd for $C_{20}H_{22}N_3O_6S^+$ [M+H]$^+$, 432. 12; found, 432. 1.

**Intermediate example 5: preparation of 8-(3-(2,6-dioxopiperidin-3-yl)-2-methyl-4-oxo-3,4-dihydroquinazolin-5-yl)oct-7-yn-1-yl methanesulfonate (729101)**

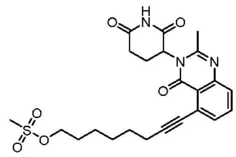

Following the method of Scheme 5 and the method of Intermediate Example 4, under appropriate conditions understood in the field, the target product 729101 was prepared (pale yellow solid, 120 mg, yield 50.11%). LCMS (ESI) m/z: calcd for $C_{23}H_{28}N_3O_6S^+$ [M+H]$^+$, 474. 17; found, 474. 2.

**Intermediate example 6: preparation of 3-(5-(5-hydroxypentyl)-2-methyl-4-oxoquinazolin-3(4H)-yl)piperidine-2,6-dione (GT-02640)**

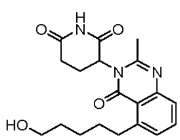

The target compound 3-(5-(5-hydroxypentyl)-2-methyl-4-oxoquinazolin-3(4H)-yl)piperidine-2,6-dione

(GT-02640) was prepared by referring to the method of step 1 of Scheme 6.

A 250 mL reaction flask was charged sequentially with 3-(5-(5-hydroxypent-1-yn-1-yl)-2-methyl-4-oxoquinazolin-3(4H)-yl)piperidine-2,6-dione (GT-02597) (1 eq.) and ethanol (80 mL), and then under nitrogen protection, 10% wet Pd/C (10 eq.). The mixture was purged with a hydrogen balloon three times, and heated to 50°C and stirred for 18h. After the reaction was completed as detected by LCMS, the reaction mixture was filtered and concentrated to give the target compound 3-(5-(5-hydroxypentyl)-2-methyl-4-oxoquinazolin-3(4H)-yl)piperidine-2,6-dione (GT-02640) (off-white solid, 200 mg, yield 88.99%). LCMS (ESI) m/z: calcd for $C_{19}H_{24}N_3O_4^+$ [M+H]$^+$, 358. 18; found, 358. 2.

**Intermediate example 7: preparation of 3-(5-(8-hydroxyoctyl)-2-methyl-4-oxoquinazolin-3(4H)-yl)piperidine-2,6-dione (GT-02760)**

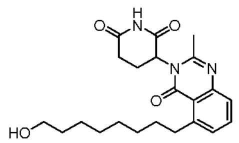

Following the method of Scheme 6 and the method of Intermediate Example 6, under appropriate conditions understood in the field, the target product GT-02760 was prepared (grayish white solid, 250 mg, yield 74.24%). LCMS (ESI) m/z: calcd for $C_{22}H_{30}N_3O_4^+$ [M+H]$^+$, 400. 22; found, 400. 2.

**Intermediate example 8: preparation of 5-(3-(2,6-dioxopiperidin-3-yl)-2-methyl-4-oxo-3,4-dihydroquinazolin-5-yl)pentyl methanesulfonate (729109)**

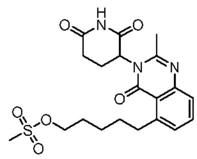

The target compound 5-(3-(2,6-dioxopiperidin-3-yl)-2-methyl-4-oxo-3,4-dihydroquinazolin-5-yl)pentyl methanesulfonate (729109) was prepared by referring to the method of step 2 of Scheme 6.

A 100 mL reaction flask was charged sequentially with 3-(5-(5-hydroxypentyl)-2-methyl-4-oxoquinazolin-3(4H)-yl)piperidine-2,6-dione (GT-02640) (1 eq.) and DCM (50 mL), and then under nitrogen protection at room temperature, TEA (3 eq.) and MsCl (2 eq.). After the addition was complete, the mixture was stirred at room temperature for 1 hour. After monitoring the reaction via TLC and confirming its completion, the reaction mixture was washed with water twice and brine once, concentrated, and then slurried (petroleum ether: ethyl acetate = 1:1) to give the target compound

**(729109)** (pale yellow solid, 150 mg, yield 64.79 %). LCMS (ESI) m/z: calcd for $C_{20}H_{26}N_3O_6S^+$ [M+H]$^+$, 436. 15; found, 436. 2.

**Intermediate example 9: preparation of 8-(3-(2,6-dioxopiperidin-3-yl)-2-methyl-4-oxo-3,4-dihydroquinazolin-5-yl)octyl methanesulfonate (729119)**

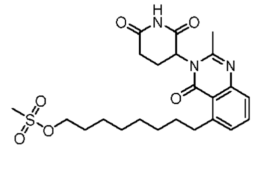

Following the method of Scheme 6 and the method of Intermediate Example 8, under appropriate conditions understood in the field, the target product **729119** was prepared (pale yellow solid, 100 mg, yield 38.02%). LCMS (ESI) m/z: calcd for $C_{23}H_{32}N_3O_6S^+$ [M+H]$^+$, 478. 20; found, 478. 2.

**Intermediate example 10: preparation of (1s,3s)-N-(prop-2-yn-1-yl)adamantan-1-amine (GT-M-24)**

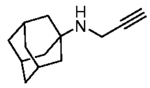

The target compound **GT-M-24** was prepared by referring to the method of step 1 of Scheme 7.

Adamantanamine (1 eq.) was added to a 250 mL reaction flask, followed by the addition of 100 mL of DMF, potassium carbonate (2 eq.), and 3-bromoprop-1-yne (1 eq.). The mixture was then stirred at room temperature under nitrogen protection for 18 hours. After the reaction was completed as detected by TLC, the reaction mixture was added to water and extracted with ethyl acetate. The organic phase was washed with saturated brine, dried over anhydrous sodium sulfate, concentrated, and subjected to column chromatography (ethyl acetate: petroleum ether = 1:1) for purification to give the target compound GT-M-24 (colorless oil, 2.3 g, yield 36.77%). $^1$H NMR (400 MHz, DMSO) $\delta$ 3. 27 (s, 2H), 2. 93 (s, 1H), 1. 99 (s, 3H), 1. 62 - 1. 47 (m, 12H). LCMS (ESI) calcd., 190. 1; found, 190. 2.

**Intermediate example 11: preparation of tert-butyl (2-(2-(((1s,3s)-adamantan-1-yl)amino)ethoxy)ethyl)carbamate (729022)**

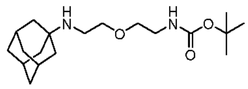

The target compound 729022 was prepared by referring to the method of step 1 of Scheme 8.

Tert-butyl (2-(2-bromoethoxy)ethyl)carbamate (1 eq.) was added into a 50 mL reaction flask, followed by the addition of 10 mL of DMF, adamantanamine (1.3 eq.), potassium carbonate (3 eq.), and sodium iodide (1 eq.). The mixture was then stirred at 80°C under nitrogen protection for 3 hours. After the reaction was completed as detected by TLC, the reaction mixture was added to water and extracted with ethyl acetate. The organic phase was washed with saturated brine, dried over anhydrous sodium sulfate, concentrated, and subjected to column chromatography (dichloromethane: ethanol = 20:1) for purification to give the target compound tert-butyl (2-(2-(((1s,3s)-adamantan-1-yl)amino)ethoxy)ethyl)carbamate (729022) (colorless oil, 250 mg, yield 65.79%).

**Intermediate example 12: preparation oftert-butyl (2-(2-(((3as,6as)-hexahydro-2,5-methanopentalen-3a(1H)-yl) amino)ethoxy)ethyl)carbamate (729031)**

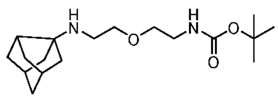

Following the method of Scheme 8 and the method of Intermediate Example 11, under appropriate conditions understood in the field, the target product **729031** was prepared (colorless oil, 310 mg, yield 86.11%).

**Intermediate example 13: preparation of tert-butyl (2-(2-(4-methyl-1,4-diazepan-1-yl)ethoxy)ethyl)carbamate (729032)**

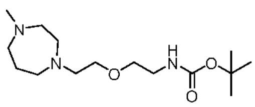

Following the method of Scheme 8 and the method of Intermediate Example 11, under appropriate conditions understood in the field, the target product 729032 was prepared (colorless oil, 270 mg, yield 79. 41%).

**Intermediate example 14: preparation of tert-butyl (2-(2-(4-methyl-1,4-diazepan-1-yl)ethoxy)ethyl)carbamate (730030)**

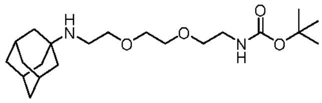

Following the method of Scheme 8 and the method of Intermediate Example 11, under appropriate conditions understood in the field, the target product **730030** was prepared (colorless oil, 80 mg, yield 61.54%). $^1$H NMR (500 MHz, $CDCl_3$) δ 3. 69 - 3. 60 (m, 6H), 3.55 (t, *J* = 5. 2 Hz, 2H), 3.35 - 3. 29 (m, 2H), 2. 90 - 2. 79 (m, 2H), 2. 12 - 2. 07 (m, 3H), 1. 73 - 1. 68 (m, 6H), 1. 66 - 1. 60 (m, 6H), 1. 44 (s, 9H).

**Intermediate example 15: preparation of tert-butyl (5-(((1*s*,3*s*)-adamantan-1-yl)amino) pentyl)carbamate (730037)**

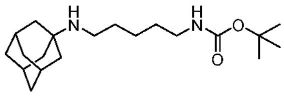

Following the method of Scheme 8 and the method of Intermediate Example 11, under appropriate conditions understood in the field, the target product **730037** was prepared (colorless oil, 600 mg, yield 54. 04%).

**Intermediate example 16: preparation of tert-butyl (5-(((3as,6as)-hexahydro-2,5-methanopentalen-3a(1H)-yl) amino)pentyl)carbamate (730055)**

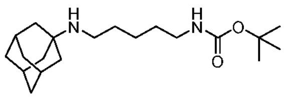

Following the method of Scheme 8 and the method of Intermediate Example 11, under appropriate conditions understood in the field, the target product **730055** was prepared (yellow oil, 100 mg, yield 21.28%).

**Intermediate example 17: preparation of tert-butyl (2-(2-(2-(((3as,6as)-hexahydro-2,5-methanopentalen-3a(1H)-yl)amino)ethoxy)ethoxy)ethyl)carbamate (730056)**

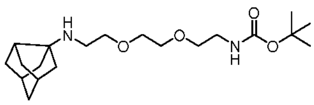

Following the method of Scheme 8 and the method of Intermediate Example 11, under appropriate conditions understood in the field, the target product **730056** was prepared (yellow oil, 50 mg, yield 16.67%).

**Intermediate example 18: preparation of tert-butyl (5-(4-methyl-1,4-diazepan-1-yl)pentyl)carbamate (730057)**

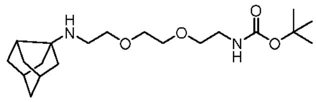

Following the method of Scheme 8 and the method of Intermediate Example 11, under appropriate conditions understood in the field, the target product 730057 was prepared (yellow oil, 262 mg, yield 49.95%).

**Intermediate example 19: preparation of (1s,3s)-N-(2-(2-aminoethoxy)ethyl)adamantan-1-amine (729023)**

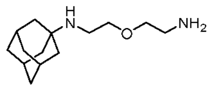

The target compound **729023** was prepared by referring to the method of step 2 of Scheme 8.

Tert-butyl (2-(2-(((1s,3s)-adamantan-1-yl)amino)ethoxy)ethyl)carbamate (729022) (1 eq.) was added into a 20 mL reaction flask, followed by the addition of 3 mL of DCM and 1 mL of TFA. The mixture was then stirred at room temperature for 1 hour. After the reaction was completed as detected by TLC, the reaction mixture was concentrated and lyophilized to give the target compound (729023) (colorless oil, 420 mg, yield 97.91%).

**Intermediate example 20: preparation of (3as,6as)-N-(2-(2-aminoethoxy)ethyl)hexahydro-2,5-methanopentalen-3a(1H)-amine (729033)**

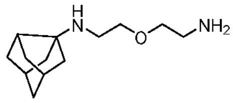

Following the method of step 2 of Scheme 8 and the method of Intermediate Example 19, under appropriate conditions understood in the field, the target product 729033 was prepared (colorless oil, 540 mg, yield 99. 83%).

**Intermediate example 21: preparation of 2-(2-(4-methyl-1,4-diazepan-1-yl)ethoxy)ethan-1-amine (729035)**

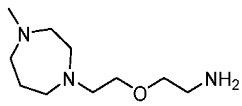

Following the method of step 2 of Scheme 8 and the method of Intermediate Example 19, under appropriate conditions understood in the field, the target product 729035 was prepared (colorless oil, 580 mg, yield 98. 47%).

**Intermediate example 22: preparation of (1s,3s)-N-(2-(2-(2-aminoethoxy)ethoxy)ethyl)adamantan-1-amine (730039)**

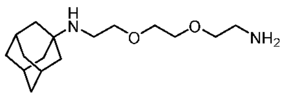

Following the method of step 2 of Scheme 8 and the method of Intermediate Example 19, under appropriate conditions understood in the field, the target product 730039 was prepared (colorless oil, 100 mg, yield 76. 62%).

**Intermediate example 23: preparation of N1-((1s,3s)-adamantan-1-yl)pentane-1,5-**diamine (730041)

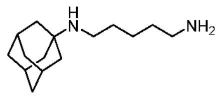

Following the method of step 2 of Scheme 8 and the method of Intermediate Example 19, under appropriate conditions understood in the field, the target product 730041 was prepared (yellow oil, 130 mg).

**Intermediate example 24: preparation of *N1*-((3as,6as)-hexahydro-2,5-methanopentalen-3a(1H)-yl)pentane-1,5-diamine (730064)**

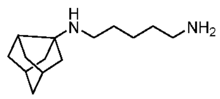

Following the method of step 2 of Scheme 8 and the method of Intermediate Example 19, under appropriate conditions understood in the field, the target product **730064** was prepared (yellow oil, 60 mg, yield 42. 96%).

**Intermediate example 25: preparation of (3as,6as)-N-(2-(2-(2-aminoethoxy)ethoxy)ethyl)hexahydro-2,5-methanopentalen-3a(1H)-amine (730060)**

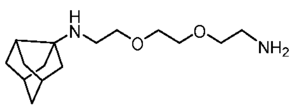

Following the method of step 2 of Scheme 8 and the method of Intermediate Example 19, under appropriate conditions understood in the field, the target product 730060 was prepared (yellow oil, 50 mg, yield 74. 23%).

**Intermediate example** 26: **preparation of 5-(4-methyl-1,4-diazepan-1-yl)pentan-1-amine (730065)**

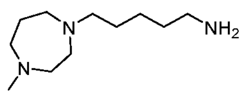

Following the method of step 2 of Scheme 8 and the method of Intermediate Example 19, under appropriate conditions understood in the field, the target product **730065** was prepared (yellow oil, 200 mg, yield 53.49%).

**Example 1: preparation of 3-(5-((3-(4-(2-((3r,5r,7r)-adamantan-1-yl)ethyl)piperazin-1-yl)propyl)thio)-2-methyl-4-oxoquinazolin-3(4H)-yl)piperidine-2,6-dione (GT-03306)**

The target compound **GT-03306** was prepared by referring to the method of Scheme 9.

Preparation of 2-((3r,5r,7r)-adamantan-1-yl)ethyl 4-methylbenzenesulfonate:

To a solution of 2-((3r,5r,7r)-adamantan-1-yl)ethan-1-ol (1 g, 5.55 mmol) in dichloromethane (30 mL) were added diisopropylethylamine (1.5 mL, 11.09 mmol) and p-toluenesulfonyl chloride (1.3 g, 6.66 mmol). The reaction mixture was stirred at room temperature for 18 hours. After monitoring the reaction via TLC and confirming its completion, the reaction solution was washed with water (60 mL) and extracted with dichloromethane (50 mL x 3). The organic phases were

combined and washed with saturated brine (50 mL x 2), dried over anhydrous sodium sulfate, and filtered. The filtrate was concentrated under reduced pressure, and the residue was subj ected to column chromatography (eluent (v/v): PE/EtOAc = 20/1) for purification to give 2-((3r,5r,7r)-adamantan-1-yl)ethyl 4-methylbenzenesulfonate as a colorless oily liquid (890 mg, yield: 48%).

Preparation of tert-butyl 4-(2-((3r,5r,7r)-adamantan-1-yl)ethyl)piperazine-1-carboxylate:

To a solution of 2-((3r,5r,7r)-adamantan-1-yl)ethyl 4-methylbenzenesulfonate (500 mg, 1.5 mmol) in acetonitrile (10 mL) were added potassium carbonate (620 mg, 4.5 mmol) and tert-butyl piperazine-1-carboxylate (556 mg, 3.0 mmol). The reaction mixture was stirred at 60°C for 18 hours. After monitoring the reaction via TLC and confirming its completion, the reaction solution was filtered, and the filtrate was concentrated under reduced pressure. The residue was washed with water (30 mL) and extracted with dichloromethane (50 mL x 3). The organic phases were combined, washed with saturated brine (50 mL x 1), dried over anhydrous sodium sulfate, and filtered. The filtrate was concentrated under reduced pressure, and the residue was subjected to column chromatography (eluent: dichloromethane/methanol = 20/1) for purification to give tert-butyl 4-(2-((3r,5r,7r)-adamantan-1-yl)ethyl)piperazine-1-carboxylate as a colorless oily liquid (180 mg, yield: 35%). LC/MS (ESI, *m/z)* 349. 4 $[M + H]^+$.

Preparation of 1-(2-((3r,5r,7r)-adamantan-1-yl)ethyl)piperazine 2,2,2-trifluoroacetaldehyde:

To a solution of tert-butyl 4-(2-((3r,5r,7r)-adamantan-1-yl)ethyl)piperazine-1-carboxylate (180 mg, 0.52 mmol) in dichloromethane (10 mL) was added trifluoroacetic acid (0.38 mL, 5.17 mmol). The reaction solution was stirred at room temperature for 2 hours. After monitoring the reaction via TLC and confirming its completion, the reaction solution was concentrated under reduced pressure to give 1-(2-((3r,5r,7r)-adamantan-1-yl)ethyl)piperazine 2,2,2-trifluoroacetaldehyde as a pale yellow solid (200 mg, yield: 107%). LC/MS (ESI, *m/z)* 249. 3 $[M + H]^+$.

Preparation of 3-(5-((3-bromopropyl)thio)-2-methyl-4-oxoquinazolin-3(4H)-yl)piperidine-2,6-dione:

To a solution of 3-(5-mercapto-2-methyl-4-oxoquinazolin-3(4H)-yl)piperidine-2,6-dione (200 mg, 0.66 mmol) in N,N-dimethylformamide (20 mL) were added diisopropylethylamine (0.33 mL, 1.98 mmol) and 1,3-dibromopropane (173 mg, 0.86 mmol). The reaction solution was stirred at room temperature for 18 hours. After monitoring the reaction via TLC and confirming its completion, the reaction solution was washed with water (50 mL) and extracted with ethyl acetate (50 mL x 3). The organic phases were combined, washed with saturated brine (50 mL x 1), dried over anhydrous sodium sulfate, and filtered. The filtrate was concentrated under reduced pressure, and the residue was slurried with ethyl acetate/petroleum ether (1/2, 20 mL). After filtration, the solid was collected to give 3-(5-((3-bromopropyl)thio)-2-methyl-4-oxoquinazolin-3(4H)-yl)piperidine-2,6-dione as white solid (161 mg, yield: 58%). LC/MS (ESI, *m/z)* 424. 1, 426. 1 $[M + H]^+$.

Preparation of 3-(5-((3-(4-(2-((3r,5r,7r)-adamantan-1-yl)ethyl)piperazin-1-yl)propyl)thio)-2-methyl-4-oxoquinazolin-3(4H)-yl)piperidine-2,6-dione (GT-03306):

To a solution of 1-(2-((3r,5r,7r)-adamantan-1-yl)ethyl)piperazine trifluoroacetate (20 mg, 0.05 mmol) in N,N-dimethylformamide (2 mL) were added diisopropylethylamine (0.03 mL, 0.19 mmol) and 3-(5-((3-bromopropyl)thio)-2-methyl-4-oxoquinazolin-3(4H)-yl)piperidine-2,6-dione (13 mg, 0.05 mmol). The reaction solution was stirred at 80 °C for 18 hours. After the reaction was completed as detected by LC/MS, the reaction solution was filtered, and the filtrate was subjected to HPLC for purification to give the white solid compound GT-03306 (6 mg, yield: 16%). $^1$H NMR (400 MHz, MeOD) δ 7. 70 (t, *J* = 8. 0 Hz, 1H), 7. 35 (d, *J* = 8. 0 Hz, 2H), 5. 24 - 5. 28 (m, 1H), 3. 74 (t, *J* = 6. 0 Hz, 1H), 3. 21 - 3. 29 (m, 2H), 3. 14 - 3. 18 (m, 2H), 3. 01 - 3. 08 (m, 4H), 2. 92 - 2. 97 ( m, 3H), 2. 78 - 2. 87 (m, 2H), 2. 70 - 2. 73 (m, 2H), 2. 22 - 2. 26 (m, 1H), 2. 04 - 2. 08 (m, 2H), 2. 00 (brs, 4H), 1. 78 - 1. 81 (m, 4H), 1. 70 - 1. 74 (m, 4H), 1. 60 (brs, 8H). LC/MS (ESI) m/z: calcd for $C_{33}H_{46}N_5O_3S+$ $[M+H]^+$, 592. 3; found, 592. 3.

**Example 2: preparation of 3-(5-((3-(4-benzhydrylpiperazin-1-yl)propyl)thio)-2-methyl-4-oxoquinazolin-3(4H)-yl) piperidine-2,6-dione (GT-03384)**

Following the method of Scheme 9 and the method of Example 1, under appropriate conditions understood in the field, the target compound GT-03384 was prepared (pale yellow solid, 20 mg, yield: 47. 48%). $^1$H NMR (400 MHz, MeOD) δ 7. 78 (s, 1H), 7. 62 (brs, 4H), 7. 49 (s, 1H), 7. 30 - 7. 34 (m, 5H), 7. 24 - 7. 27 (m, 2H), 5. 32 (br, 1H), 5. 02 - 5. 19 (m, 1H), 3.58 - 3. 62 (m, 4H), 3.33 - 3.37 (m, 4H), 3. 05 - 3. 09 (m, 4H), 2. 67 - 2. 89 (m, 6H), 2. 16 - 2. 27 (m, 3H). LC/MS (ESI) m/z: calcd for $C_{34}H_{38}N_5O_3S^+$ $[M+H]^+$, 596. 3; found, 596. 3.

Example 3: preparation of 3-(5-((3-(4-(bis(4-fluorophenyl)methyl)piperazin-1-**yl)propyl)thio)-2-methyl-4-oxoquinazolin-3(4H)-yl)piperidine-2,6-dione** (GT-03387)

Following the method of Scheme 9 and the method of Example 1, under appropriate conditions understood in the field, the target compound **GT-03387** was prepared (pale yellow solid, 24 mg, yield: 53.52%). $^1$H NMR (400 MHz, MeOD) δ 7. 76 (t, *J*= 8. 0 Hz, 1H), 7. 68 (dd, *J* = 8. 0, 4. 0 Hz, 1H), 7. 45 (t, *J* = 8. 0 Hz, 3H), 7. 32 (d, *J* = 8. 0 Hz, 1H), 7. 16 (t, *J* = 8. 0 Hz, 1H), 6. 98 (t, *J* = 8. 0 Hz, 4H), 5. 28 (dd, *J* = 11. 3, 5. 6 Hz, 1H), 4.54 (s, 1H), 3. 45 (s, 2H), 3.34 - 3. 24 (m, 4H), 3. 13 - 2. 90 (m, 5H), 2. 88 - 2. 75 (m, 4H), 2. 73 - 2. 63 (m, 2H), 2. 42 (s, 1H), 2. 27 - 2. 17 (m, 1H), 2. 16 - 2. 05 (m, 2H). LC/MS (ESI) m/z: cal. $C_{34}H_{36}F_2N_5O_3S^+$ [M+H]$^+$, 632. 2; found, 632. 2.

**Example 4: preparation of 3-(5-((2-(4-(2-((3r,5r,7r)-adamantan-1-yl)ethyl)piperazin-1-yl)ethyl)amino)-2-methyl-4-oxoquinazolin-3(4H)-yl)piperidine-2,6-dione (GT-03301)**

The target compound GT-03301 was prepared by referring to the method of Scheme 10.

Preparation of 3-(5-((3-((tert-butyldimethylsilyl)oxy)propyl)amino)-2-methyl-4-oxoquinazolin-3(4H)-yl)piperidine-2,6-dione (a2) and 3-(5-((3-hydroxypropyl)amino)-2-methyl-4-oxoquinazolin-3(4H)-yl)piperidine-2,6-dione (a3):

To a solution of 3-(5-fluoro-2-methyl-4-oxoquinazolin-3(4H)-yl)piperidine-2,6-dione (1 g, 3.46 mmol) in N-methylpyrrolidone (20 mL) were added triethylamine (1.92 mL, 13.83 mmol) and 2-((tert-butyldimethylsilyl)oxy)-1-amino-ethane (1.21 g, 6.91 mmol). The reaction solution was stirred at 90°C for 18 hours. After the reaction was completed as detected by TLC, the reaction mixture was cooled to room temperature, washed with water (100 mL), and extracted with ethyl acetate (100 mL x 3). The organic phases were combined, washed with saturated brine (50 mL x 2), dried over anhydrous sodium sulfate, and filtered. The filtrate was concentrated under reduced pressure, and the residue was subjected to column chromatography (eluent: dichloromethane/methanol = 20/1) for purification to give pale yellow solid 3-(5-((3-((tert-butyldimethylsilyl)oxy)propyl)amino)-2-methyl-4-oxoquinazolin-3(4H)-yl)piperidine-2,6-dione (890 mg, yield: 58%)(LC/MS (ESI, m/z) 445.3 [M + H]$^+$), and pale yellow solid 3-(5-((3-hydroxypropyl)amino)-2-methyl-4-oxoquinazolin-3(4H)-yl)piperidine-2,6-dione (210 mg, yield: 19%) (LC/MS (ESI, m/z) 331.2 [M + H]$^+$).

Preparation of 3-((3-(2,6-dioxopiperidin-3-yl)-2-methyl-4-oxo-3,4-dihydroquinazolin-5-yl)amino)propyl methanesulfonate:

To a solution of 3-(5-((3-hydroxypropyl)amino)-2-methyl-4-oxoquinazolin-3(4H)-yl)piperidine-2,6-dione (210 mg, 0.64 mmol) in dichloromethane (20 mL) were added triethylamine (0.27 mL, 1.91 mmol) and methanesulfonyl chloride (0.098 mL, 1.27 mmol). The reaction solution was stirred at room temperature for 1 hour. After monitoring the reaction via TLC and confirming its completion, the reaction solution was washed with water (50 mL) and extracted with dichloromethane (50 mL x 3). The organic phases were combined, washed with saturated brine (30 mL), dried over anhydrous sodium sulfate, and filtered. The filtrate was concentrated under reduced pressure, and the residue was subjected to column chromatography (eluent: dichloromethane/methanol = 20/1) for purification to give pale yellow solid 3-((3-(2,6-dioxopiperidin-3-yl)-2-methyl-4-oxo-3,4-dihydroquinazolin-5-yl)amino)propyl methanesulfonate (180 mg, yield: 69%). LC/MS (ESI, m/z) 409.2 [M + H]$^+$.

Preparation of the target compound GT-03301:

To a solution of 3-((3-(2,6-dioxopiperidin-3-yl)-2-methyl-4-oxo-3,4-dihydroquinazolin-5-yl)amino)propyl methanesulfonate (20 mg, 0.049 mmol) in N,N-dimethylformamide (2 mL) were added diisopropylethylamine (0.03 mL, 0.19 mmol) and 1-(2-((3r,5r,7r)-adamantan-1-yl)ethyl)piperazine 2,2,2-trifluoroacetaldehyde (13 mg, 0.054 mmol). The reaction solution was stirred at 80°C for 18 hours. After monitoring the reaction via LC/MS and confirming its completion, the reaction solution was filtered, and the filtrate was subjected to HPLC for purification to give the white solid compound GT-03301 (8 mg, yield: 25%). $^1$H NMR (400 MHz, MeOD) δ 7. 75 (t, *J* = 8. 0 Hz, 1H), 6. 93 (d, *J* = 8. 0 Hz, 1H), 6. 82 (d, *J* = 8. 0 Hz, 1H), 5. 37 - 5. 41 (m, 1H), 4.47 - 4.56 (m, 1H), 3. 49 - 3.53 (m, 3H), 3. 21 - 3. 29 (m, 5H), 3. 08 - 3. 16 (m, 4H), 3. 01 - 3. 07 (m, 1H), 2. 92 - 2. 97 (m, 3H), 2. 22 - 2. 26 (m, 1H), 2. 03 (brs, 4H), 1. 78 - 1. 81 (m, 4H), 1. 70 - 1. 74 (m, 4H), 1. 60 (brs, 8H). LC/MS (ESI) m/z: calcd for $C_{32}H_{45}N_6O_3$+ [M+H]$^+$, 561. 4; found, 561. 4.

Example 5: **preparation** of **3-(8-((2-(4-(bis(4-fluorophenyl)methyl)piperazin-1-yl)ethyl)amino)-3-methyl-1-oxoisoquinolin-2(1H)-yl)piperidine-2,6-dione (GT-03388)**

Following the method of Scheme 10 and the method of Example 4, under appropriate conditions understood in

the field, the target compound GT-03388 was prepared (pale yellow solid, 15 mg, yield: 33. 80%). $^1$H NMR (400 MHz, DMSO) δ 11. 07 (s, 1H), 8. 04 (s, 1H), 7. 97 (brs, 3H), 7. 60 (t, *J* = 8. 0 Hz, 1H), 7. 31 (t, *J* = 8. 0 Hz, 3H), 7. 25 - 7. 16 (m, 3H), 6. 84 (t, *J* = 8. 0 Hz, 1H), 5. 66 - 5. 72 (m, 1H), 5. 26 - 5. 31 (m, 1H), 3. 79 - 3. 83 (m, 2H), 3. 71 - 3. 74 (m, 2H), 3.51 - 3.58 (m, 2H), 3.39 - 3. 44 (m, 2H), 3. 18 - 3. 29 (m, 3H), 2. 82 - 3. 02 (m, 3H), 2. 71 (s, 3H), 2.58 - 2. 61 (m, 2H), 2. 18 - 2. 21 (m, 1H). LC/MS (ESI) m/z: calcd for $C_{34}H_{36}F_2N_5O_3^+$ [M+H]$^+$, 600. 3; found, 600. 3.

**Example 6: preparation of 3-(5-((4-(((3s,5s,7s)-adamantan-1-yl)amino)butyl)amino)-2-methyl-4-oxoquinazolin-3(4H)-yl)piperidine-2,6-dione (GT-01818)**

The target compound was prepared by referring to the method of step 3 of Scheme 1.

To a solution of the compound 3-(5-fluoro-2-methyl-4-oxo-3,4-dihydroquinazolin-3-yl)piperidine-2,6-dione (80 mg, 0.28 mmol) in anhydrous NMP were added sequentially DIEA (0.14 mL, 0.83 mmol) and N1-(adamantan-1-yl) butane-1,4-diamine (61.5 mg, 0.28 mmol). The reaction solution was stirred at 130°C for 8 hours. After monitoring the reaction via TLC and confirming its completion, the reaction solution was cooled to room temperature and filtered. The filtrate was subjected to HPLC for purification to give the target compound (pale yellow solid, 18 mg, yield: 12%). $^1$H NMR (500 MHz, MeOD) δ 7. 63 (t, *J* = 5. 0 Hz, 1H), 6. 81 (d, *J* = 5. 0 Hz, 1H), 6. 68 (d, *J* = 5. 0 Hz, 1H), 5. 30 (dd, *J* = 5. 0, 10. 0 Hz, 1H), 3. 29 - 3.32 (m, 2H), 2. 92 - 2. 95 (m, 2H), 2. 84 (s, 3H), 2. 65 - 2. 73 (m, 3H), 2. 22 - 2. 26 (m, 1H), 2. 11 (s, 3H), 1. 82 - 1. 85 (m, 6H), 1. 68 - 1. 673 (m, 7H), 1. 62 - 1. 64 (m, 3H). LC/MS (ESI) calcd for $C_{28}H_{38}N_5O_3^+$ [M+H]$^+$, 492.30; found, 492.4.

**Example 7: preparation of 3-(5-((6-(((3s,5s,7s)-adamantan-1-yl)amino)hexyl)amino)-2-methyl-4-oxoquinazolin-3(4H)-yl)piperidine-2,6-dione (GT-02235)**

Following the method of Scheme 1 and the method of Example 6, under appropriate conditions understood in the field, the target compound **GT-02235** was prepared (pale yellow solid, 15 mg, yield 45%). $^1$H NMR (400 MHz, DMSO) δ 11. 01 (s, 1H), 8. 34 (brs, 2H), 7. 51 (t, *J* = 8. 0 Hz, 1H), 6.66 (d, *J* = 8. 0 Hz, 1H), 6. 53 (d, *J* = 8. 0 Hz, 1H), 5. 23-5. 20 (m, 1H), 2. 89-2. 81 (m, 2H), 2. 61-2.58 (m, 2H), 2. 12 (s, 3H), 1. 85-1. 81 (m, 7H), 1. 69-1. 57 (m, 14H), 1. 42-1. 38 (m, 6H). LC/MS (ESI) calcd for $C_{30}H_{42}N_5O_3$+ [M+H]$^+$, 520. 33; found, 520. 3.

**Example 8: preparation of 3-(5-((7-(((1s,3s)-adamantan-1-yl)amino)heptyl)amino)-2-methyl-4-oxoquinazolin-3(4H)-yl)piperidine-2,6-dione (GT-02237)**

Following the method of Scheme 1 and the method of Example 6, under appropriate conditions understood in the field, the target compound **GT-02237** was prepared (pale yellow solid, 14 mg, yield 54%). $^1$H NMR (400 MHz, DMSO) δ 11. 12 (s, 1H), 8. 71 (brs, 2H), 7. 61 (t, *J* = 8. 0 Hz, 1H), 6. 84 (d, *J* = 8. 0 Hz, 1H), 6. 68 (d, *J* = 8. 0 Hz, 1H), 5. 33-5. 29 (m, 1H), 3. 22 (t, *J* = 8. 0 Hz, 2H), 2. 88 - 2. 75 (m, 6H), 2. 68 - 2.56 (m, 2H), 2. 28 - 2. 17 (m, 1H), 2. 11 (s, 3H), 1. 87 (s, 6H), 1. 68-1. 57 (m, 10H), 1. 34 (s, 6H). LC/MS (ESI) calcd for $C_{31}H_{44}N_5O_3^+$ [M+H]$^+$, 534. 34; found, 534.4.

**Example 9: preparation of 3-(5-((2-(((1r,3r,5r,7r)-adamantan-2-yl)amino)ethyl)amino)-2-methyl-4-oxoquinazolin-3(4H)-yl)piperidine-2,6-dione (GT-02325)**

Following the method of Scheme 1 and the method of Example 6, under appropriate conditions understood in the field, the target compound **GT-02325** was prepared (pale yellow solid, 12 mg, yield 56%). $^1$H NMR (400 MHz, DMSO) δ 11. 03 (s, 1H), 8. 66 (brs, 2H), 7. 57 (t, *J* = 8. 0 Hz, 1H), 6. 76 (d, *J*= 8. 0 Hz, 1H), 6. 73 (d, *J*= 8. 0 Hz, 1H), 5. 26-5. 22 (m, 1H), 3. 89-3. 80 (m, 1H), 3. 69-3. 65 (m, 2H), 3. 19-3. 15 (m, 2H), 2. 86-2. 83 (m, 1H), 2. 62 (s, 3H), 2. 16-2. 12 (m, 3H), 2. 05-2. 02 (m, 3H), 1. 86-1. 82 (m, 6H), 1. 72-1. 68 (m, 6H). LC/MS (ESI) calcd for $C_{26}H_{34}N_5O_3^+$ [M+H]$^+$, 464.59; found, 464.7.

**Example 10: preparation of 3-(5-((3-(((1r,3r,5r,7r)-adamantan-2-yl)amino)propyl)amino)-2-methyl-4-oxoquinazolin-3(4H)-yl)piperidine-2,6-dione (GT-02326)**

Following the method of Scheme 1 and the method of Example 6, under appropriate conditions understood in the field, the target compound **GT-02326** was prepared (pale yellow solid, 15 mg, yield 46%). $^1$H NMR (400 MHz, DMSO) δ 11. 01 (s, 1H), 8. 42 (brs, 2H), 7. 54 (t, *J* = 8. 0 Hz, 1H), 6. 71 (d, *J= 8.* 0 Hz, 1H), 6. 61 (d, *J* = 8. 0 Hz, 1H), 5. 25-5. 21 (m, 1H), 3.57-3.55 (m, 1H), 3.54-3.51 (m, 2H), 3. 06-3. 00 (m, 2H), 2. 86-2. 83 (m, 1H), 2. 60 (s, 3H), 2. 36-2. 32 (m, 2H), 2. 17-2. 11 (m, 3H), 2. 03-1. 99 (m, 2H), 1. 89-1. 83 (m, 6H), 1. 77-1. 71 (m, 6H). LC/MS (ESI) calcd for $C_{27}H_{36}N_5O_3^+$ [M+H]$^+$, 478. 62; found, 478. 6.

**Example 11: preparation of 3-(5-((6-(((2R,3as,5S,6as)-hexahydro-2,5-methanopentalen-3a(1H)-yl)amino)hexyl) amino)-2-methyl-4-oxoquinazolin-3(4H)-yl)piperidine-2,6-dione (GT-02494)**

Following the method of Scheme 1 and the method of Example 6, under appropriate conditions understood in the field, the target compound **GT-02494** was prepared (pale yellow solid, 50 mg, yield 51%). $^1$H NMR (400 MHz, DMSO) δ 11. 07 (s, 1H), 9. 15 (brs, 2H), 7. 57 (t, *J* = 8. 0 Hz, 1H), 6. 75 (d, *J* = 8. 0 Hz, 1H), 6. 62 (d, *J* = 8. 0 Hz, 1H), 5. 29-5. 24 (m, 1H), 3. 21 (t, *J* = 8. 0 Hz, 2H), 2. 86-2. 82 (m, 4H), 2. 68 (s, 3H), 2. 63-2. 61 (m, 1H), 2. 60-2.58 (m, 1H), 2. 42-2. 39 (m, 1H), 2. 32-2. 29 (m, 3H), 2. 22-2. 16 (m, 1H), 1. 95-1. 85 (m, 6H), 1. 64-1. 61 (m, 5H), 1. 49-1. 40 (m, 6H). LC/MS (ESI) calcd for $C_{29}H_{40}N_5O_3^+$ [M+H]$^+$, 506. 67; found, 506. 8.

**Example 12: preparation of 3-(5-((7-(((3as,6as)-hexahydro-2,5-methanopentalen-3a(1H)-yl)amino)heptyl)amino)-2-methyl-4-oxoquinazolin-3(4H)-yl)piperidine-2,6-dione (GT-02495)**

Following the method of Scheme 1 and the method of Example 6, under appropriate conditions understood in the field, the target compound **GT-02495** was prepared (pale yellow solid, 54 mg, yield 67%). $^1$H NMR (400 MHz, DMSO) δ 11. 03 (s, 1H), 8. 94 (brs, 2H), 7. 53 (t, *J* = 8. 0 Hz, 1H), 6. 69 (d, *J* = 8. 0 Hz, 1H), 6. 56 (d, *J* = 8. 0 Hz, 1H), 5. 25-5. 20 (m, 1H), 3. 18 (t, *J* = 8. 0 Hz, 2H), 2. 90-2. 80 (m, 3H), 2. 69-2. 67 (m, 1H), 2. 65-2. 63(m, 1H), 2. 61 (s, 3H), 2. 60-2.57 (m, 1H), 2. 40-2. 37 (m, 1H), 2. 34-2. 28 (m, 3H), 2. 21-2. 14(m, 1H), 1. 94-1. 84 (m, 7H), 1. 67-1. 58 (m, 6H), 1. 41-1. 34 (m, 6H). LC/MS (ESI) calcd for $C_{30}H_{42}N_5O_3^+$ [M+H]$^+$, 520. 69; found, 520. 8.

**Example 13: preparation of 3-(2-methyl-5-((7-(4-methyl-1,4-diazepan-1-yl)heptyl)amino)-4-oxoquinazolin-3(4H)-yl)piperidine-2,6-dione (GT-02502)**

Following the method of Scheme 1 and the method of Example 6, under appropriate conditions understood in the field, the target compound **GT-02502** was prepared (pale yellow solid, 15 mg, yield 45%). LC/MS (ESI) calcd for $C_{25}H_{37}N_6O_3^+$ [M+H]$^+$, 469. 61; found, 469. 6.

**Example 14: preparation of 3-(5-(((1s,3s)-adamantan-1-yl)amino)-2-methyl-4-oxoquinazolin-3(4H)-yl)piperidine-2,6-dione (GT-02601)**

Following the method of Scheme 1 and the method of Example 6, under appropriate conditions understood in the field, the target compound **GT-02601** was prepared (white solid, 10 mg, yield 46%). LC/MS (ESI) calcd for $C_{24}H_{29}N_4O_3^+$ [M+H]$^+$, 421. 52; found,421. 5.

**Example 15: preparation of 3-(5-((7-hydroxyheptyl)amino)-2-methyl-4-oxoquinazolin-3(4H)-yl)piperidine-2,6-dione (GT-02598)**

Following the method of Scheme 2, under appropriate conditions understood in the field, the target compound **GT-02598** was prepared (pale yellow solid, 280 mg, yield 40%). LC/MS (ESI) calcd for $C_{21}H_{29}N_4O_4^+$ [M+H]$^+$, 401. 22; found, 401. 3.

**Example 16: preparation of 3-(5-((2-(2-hydroxyethoxy)ethyl)amino)-2-methyl-4-oxoquinazolin-3(4H)-yl)piperidine-2,6-dione (GT-02607)**

Following the method of Scheme 3, under appropriate conditions understood in the field, the target compound **GT-02607** was prepared (pale yellow solid, 1.1 g, yield 58%). LC/MS (ESI) calcd for $C_{18}H_{23}N_4O_5^+$ [M+H]$^+$, 375.17; found, 375. 2.

**Example 17: preparation of 3-(5-((2-(2-(6-oxa-3-azabicyclo[3.1.1]heptan-3-yl)ethoxy)ethyl)amino)-2-methyl-4-oxoquinazolin-3(4H)-yl)piperidine-2,6-dione (GT-02608)**

Following the method of Scheme 3, under appropriate conditions understood in the field, the target compound **GT-02608** was prepared (pale yellow solid, 11 mg, yield 46%). $^1$H NMR (400 MHz, DMSO) δ 11. 04 (s, 1H), 9. 78 (brs, 1H), 7. 55 (t, *J* = 8. 0 Hz, 1H), 6. 74 (d, *J* = 8. 0 Hz, 1H), 6. 64 (d, *J* = 8. 0 Hz, 1H), 5. 27-5. 23 (m, 1H), 3. 85-3. 80 (m, 4H), 3. 72-3. 68 (m, 3H), 3. 18 - 3. 08 (m, 2H), 3. 03-2. 97 (m, 1H), 2. 87-2. 83 (m, 2H), 2. 71-2. 69 (m, 1H), 2. 64 (s, 3H), 2. 34-2. 69 (m, 1H), 2. 30-2. 28 (m, 1H), 2. 20-2. 16 (m, 2H), 1. 94-1. 89 (m, 1H), 1. 86-1. 75 (m, 1H), 1. 30-1. 26 (m, 1H). LC/MS (ESI) calcd for $C_{23}H_{30}N_5O_5^+$ [M+H]$^+$, 456. 22; found, 456. 2.

**Example 18: preparation of 3-(2-methyl-5-((2-(2-morpholinoethoxy)ethyl)amino)-4-oxoquinazolin-3(4H)-yl)piperidine-2,6-dione (GT-02617)**

Following the method of Scheme 3, under appropriate conditions understood in the field, the target compound **GT-02617** was prepared (pale yellow solid, 5 mg, yield 23%). $^1$H NMR (400 MHz, DMSO) δ 11. 09 (s, 1H), 10. 91 (s, 1H), 7. 60 (t, *J* = 8. 8 Hz, 1H), 6. 82 (d, *J* = 8. 8 Hz, 1H), 6. 71 (d, *J= 8.* 8 Hz, 1H), 5. 32-5. 27 (m, 1H), 3. 77 - 3. 69 (m, 6H), 3. 46 - 3.38 (m, 4H), 3. 18 - 3. 01 (m, 4H), 2. 88 - 2. 92 (m, 2H), 2. 66 - 2.56 (m, 2H), 2.52 (s, 3H), 2. 27 - 2. 14 (m, 2H). LC/MS (ESI) calcd for $C_{22}H_{30}N_5O_5^+$ [M+H]$^+$, 444. 22; found, 444. 2.

**Example 19: preparation of 3-(2-methyl-5-((2-(2-(4-morpholinopiperidin-1-yl)ethoxy)ethyl)amino)-4-oxoquinazolin-3(4H)-yl)piperidine-2,6-dione (GT-02618)**

Following the method of Scheme 3, under appropriate conditions understood in the field, the target compound **GT-02618** was prepared (pale yellow solid, 10 mg, yield 38%). $^1$H NMR (400 MHz, DMSO) δ 11. 15 (s, 1H), 10. 67 (s, 1H), 7. 63 (t, *J* = 8. 0 Hz, 1H), 6. 88 (d, *J* = 8. 0 Hz, 1H), 6. 76 (d, *J= 8.* 0 Hz, 1H), 5. 35-5. 31 (m, 1H), 4. 04 - 3. 82 (m, 6H), 3.50 - 3.35 (m, 8H), 3. 16 - 2. 93 (m, 6H), 2. 87-2. 84 (m, 1H), 2. 64-2. 61 (m, 2H), 2.52 (s, 3H), 2. 40 - 2. 24 (m, 3H), 2. 14-2. 10 (m, 2H). LC/MS (ESI) calcd for $C_{27}H_{39}N_6O_5^+$ [M+H]$^+$, 527. 30; found, 527. 3.

**Example 20: preparation of 3-(2-methyl-5-((2-(2-(4-(4-methylpiperazin-1-yl)piperidin-1-yl)ethoxy)ethyl)amino)-4-oxoquinazolin-3(4H)-yl)piperidine-2,6-dione (GT-02619)**

Following the method of Scheme 3, under appropriate conditions understood in the field, the target compound **GT-02619** was prepared (pale yellow solid, 10 mg, yield 35%). $^1$H NMR (400 MHz, DMSO) δ 11. 08 (s, 1H), 10. 37 (brs, 1H), 8. 40 br(s, 1H), 7. 56 (t, *J* = 8. 0 Hz, 1H), 6. 75 (d, *J* = 8. 0 Hz, 1H), 6. 65 (d, *J*= 8. 0 Hz, 1H), 5. 28-5. 24 (m, 1H), 3. 84-3. 82 (m, 2H), 3. 71-3. 69 (m, 3H), 3. 63-3. 60 (m, 5H), 3.33-3. 27 (m, 6H), 2. 98-2. 95 (m, 3H), 2. 86-2. 85 (m, 1H), 2. 82 (s, 3H), 2. 68-2. 65 (m, 1H), 2. 65 (s, 3H), 2. 62-2. 60 (m, 2H), 2. 27-2. 20 (m, 3H), 2. 08-1. 96 (m, 2H), 1. 31-1. 26 (m, 1H). LC/MS (ESI) calcd for $C_{28}H_{42}N_7O_4^+$ [M+H]$^+$, 540. 33; found, 540. 3.

**Example 21: preparation of 3-(2-methyl-5-((2-(2-(4-(oxetan-3-yl)piperazin-1-yl)ethoxy)ethyl)amino)-4-oxoquinazolin-3(4H)-yl)piperidine-2,6-dione (GT-02620)**

Following the method of Scheme 3, under appropriate conditions understood in the field, the target compound **GT-02620** was prepared (pale yellow solid, 8 mg, yield 32%). $^1$H NMR (400 MHz, DMSO) δ 11. 14 (s, 1H), 7. 63 (t, *J* = 8. 0 Hz, 1H), 6. 88 (d, *J* = 8. 0 Hz, 1H), 6. 77 (d, *J* = 8. 0 Hz, 1H), 5. 36-5. 31 (m, 1H), 4.49-4.44 (m, 1H), 3. 90-3. 86 (m, 4H), 3.51-3. 47 (m, 6H), 3. 42-3.38 (m, 4H), 3.32-3. 29 (m, 2H), 2. 91-2. 86 (m, 1H), 2. 79 (s, 3H), 2. 70 (s, 1H), 2. 68-2. 64 (m, 1H), 2. 64-2. 61 (m, 1H), 2. 25-2. 16 (m, 2H), 1. 94-1. 89 (m, 1H), 1. 32-1. 26 (m, 1H). LC/MS (ESI) calcd for $C_{25}H_{35}N_6O_5^+$ [M+H]$^+$, 499. 27; found, 499. 3.

**Example 22: preparation of 3-(5-((7-(6-oxa-3-azabicyclo[3.1.1]heptan-3-yl)heptyl)amino)-2-methyl-4-oxoquinazolin-3(4H)-yl)piperidine-2,6-dione ( GT-02621)**

Following the method of Scheme 2, under appropriate conditions understood in the field, the target compound **GT-02621** was prepared (pale yellow solid, 10 mg, yield 46%). $^1$H NMR (400 MHz, DMSO) δ 11. 05 (s, 1H), 7. 55 (t, *J* = 8. 0 Hz, 1H), 6. 71 (d, *J* = 8. 0 Hz, 1H), 6. 58 (t, *J* = 8. 0 Hz, 1H), 5. 26-5. 22 (m, 1H), 4.59 (d, *J* = 8. 0 Hz, 1H), 3. 70-3. 62 (m, 3H), 3. 21-3. 17 (m, 5H), 3. 08-3. 06 (m, 2H), 2. 87-2. 84 (m, 2H), 2. 67-2. 61 (m, 5H), 2.59-2.57 (m, 1H), 2. 36-2. 26 (m, 1H), 2. 19-2. 14 (m, 1H), 1. 70 -1. 54 (m, 3H), 1. 40 -1. 27 (m, 6H). LC/MS (ESI) calcd for $C_{26}H_{36}N_5O_4^+$ [M+H]$^+$, 482. 28; found, 482. 3.

**Example 23: preparation of 3-(2-methyl-5-((7-(4-morpholinopiperidin-1-yl)heptyl)amino)-4-oxoquinazolin-3(4H)-yl)piperidine-2,6-dione (GT-02622)**

Following the method of Scheme 2, under appropriate conditions understood in the field, the target compound **GT-02622** was prepared (pale yellow solid, 14 mg, yield 57%). $^1$H NMR (400 MHz, DMSO) *δ* 11. 58 (brs, 1H), 11. 09 (s, 1H), 10. 58 (brs, 1H), 7. 59 (t, *J* = 8. 0 Hz, 1H), 6. 79 (d, *J* = 8. 0 Hz, 1H), 6. 63 (d, *J* = 8. 0 Hz, 1H), 5. 31-5. 27 (m, 1H), 4. 02-3. 97 (m, 3H), 3. 91-3. 85 (m, 3H), 3. 22-3. 19 (m, 3H), 3. 14-3. 07 (m, 3H), 2. 94-2. 83 (m, 3H), 2. 72 (s, 3H), 2. 67-2.56 (m, 2H), 2. 35-2. 32 (m, 2H), 2. 22 - 2. 12 (m, 3H), 1. 75-1. 56 (m, 5H), 1. 41-1. 24 (m, 8H). LC/MS (ESI) calcd for $C_{30}H_{45}N_6O_4^+$ [M+H]$^+$, 553.35; found, 553. 4.

**Example 23: preparation of 3-(2-methyl-5-((7-(4-(oxetan-3-yl)piperazin-1-yl)heptyl)amino)-4-oxoquinazolin-3(4H)-yl)piperidine-2,6-dione (GT-02623)**

Following the method of Scheme 2, under appropriate conditions understood in the field, the target compound **GT-02623** was prepared (pale yellow solid, 8 mg, yield 32%). $^1$H NMR (400 MHz, DMSO) $\delta$ 11. 09 (s, 1H), 7. 58 (t, $J$ = 8. 0 Hz, 1H), 6. 77 (d, $J$ = 8. 0 Hz, 1H), 6. 63 (d, $J$ = 8. 0 Hz, 1H), 5. 30-5. 26 (m, 1H), 4. 69-4. 61 (m, 2H), 3. 77-3. 67 (m, 5H), 3. 26 -3. 14 (m, 5H), 3. 11-3. 05 (m, 3H), 2. 92-2. 83 (m, 2H), 2. 71 (s, 3H), 2. 64-2. 62 (m, 1H), 2.59-2.57 (m, 1H), 2. 25-2. 13 (m, 1H), 1. 74-1. 58 (m, 4H), 1. 42-1. 23 (m, 7H). LC/MS (ESI) calcd for $C_{28}H_{41}N_6O_4^+$ [M+H]$^+$, 525. 32; found, 525. 3.

**Example 24: preparation of 3-(5-((7-hydroxyheptyl)oxy)-2-methyl-4-oxoquinazolin-3(4H)-yl)piperidine-2,6-dione (GT-02663)**

Following the method of Scheme 4, under appropriate conditions understood in the field, the target compound **GT-02623** was prepared (pale yellow solid, 14 mg, yield 36%). LC/MS (ESI) calcd for $C_{21}H_{28}N_3O_5^+$ [M+H]$^+$, 402. 20; found, 402. 2.

**Example 25: preparation of 3-(5-((7-(((1s,3s)-adamantan-1-yl)amino)heptyl)oxy)-2-methyl-4-oxoquinazolin-3(4H)-yl)piperidine-2,6-dione (GT-02769)**

Following the method of Scheme 4, under appropriate conditions understood in the field, the target compound **GT-02669** was prepared (pale yellow solid, 12 mg, yield 54%). $^1$H NMR (400 MHz, DMSO) $\delta$ 10. 98 (s, 1H), 8. 30 (brs, 2H), 7. 67 (t, $J$ = 8. 0 Hz, 1H), 7. 11 (d, $J$ = 8. 0 Hz, 1H), 6. 99 (d, $J$ = 8. 0 Hz, 1H), 5. 17-5. 15 (m, 1H), 4. 08 - 4. 01 (m, 2H), 2. 90-2. 81 (m, 4H), 2.59 (s, 3H), 2. 14-2. 09 (m, 5H), 1. 85-1. 80 (m, 6H), 1. 69-1. 64 (m, 4H), 1. 50-1. 44 (m, 2H), 1. 40-1. 36 (m, 6H), 1. 24-1. 20 (m, 4H). LC/MS (ESI) calcd for $C_{31}H_{43}N_4O_4^+$ [M+H]$^+$, 535. 33; found, 535. 3.

**Example 26: preparation of 3-(2-methyl-5-((7-(4-(oxetan-3-yl)piperazin-1-yl)heptyl)oxy)-4-oxoquinazolin-3(4H)-yl)piperidine-2,6-dione (GT-02770)**

Following the method of Scheme 4, under appropriate conditions understood in the field, the target compound **GT-02770** was prepared (white solid, 13 mg, yield 59%). LC/MS (ESI) calcd for $C_{28}H_{40}N_5O_5^+$ [M+H]$^+$, 526. 30; found, 526. 3.

**Example 27: preparation of 3-(2-methyl-5-((7-(4-methylpiperazin-1-yl)heptyl)oxy)-4-oxoquinazolin-3(4H)-yl)piperidine-2,6-dione (GT-02771)**

Following the method of Scheme 4, under appropriate conditions understood in the field, the target compound **GT-02771** was prepared (white solid, 10 mg, yield 49%). LC/MS (ESI) calcd for $C_{26}H_{38}N_5O_4^+$ [M+H]$^+$, 484.29; found, 484.3.

**Example 28: preparation of 3-(5-((7-(((1SR,3RS,SSR,7r)-3,5-dimethyladamantan-1-yl)amino)heptyl)amino)-2-methyl-4-oxoquinazolin-3(4H)-yl)piperidine-2,6-dione (GT-02767)**

Following the method of Scheme 1, under appropriate conditions understood in the field, the target compound **GT-02767** was prepared (white solid, 15 mg, yield 38%). $^1$H NMR (400 MHz, DMSO) $\delta$ 11. 01 (s, 1H), 8. 41 (brs, 2H), 7. 50 (t, $J$ = 8. 0 Hz, 1H), 6. 65 (d, $J$ = 8. 0 Hz, 1H), 6. 52 (d, $J$ = 8. 0 Hz, 1H), 5. 22-5. 18 (m, 1H), 3. 21 - 3. 14 (m, 3H), 2. 91-2. 80 (m, 4H), 2.57 (s, 3H), 2.56 (s, 2H), 2.54 (s, 3H), 2. 34-2. 33 (m, 2H), 2. 22-2. 17 (m, 3H), 1. 71-1. 69 (m, 3H), 1. 65-1. 56 (m, 4H), 1. 20 -1. 09 (m, 5H), 0. 87 (brs, 9H). LC/MS (ESI) calcd for $C_{33}H_{48}N_5O_3^+$ [M+H]$^+$, 562.38; found, 562.4.

**Example 29: preparation of 3-(5-((7-((1-((3r,5r,7r)-adamantan-1-yl)ethyl)amino)heptyl)amino)-2-methyl-4-oxoquinazolin-3(4H)-yl)piperidine-2,6-dione (GT-02768)**

Following the method of Scheme 1, under appropriate conditions understood in the field, the target compound **GT-02768** was prepared (white solid, 23 mg, yield 58%). $^1$H NMR (400 MHz, DMSO) $\delta$ 10. 93 (s, 1H), 8. 24 (brs, 2H), 7. 43 (t, $J$= 8. 0 Hz, 1H), 7. 27 (brs, 1H), 6. 58 (d, $J$= 8. 0 Hz, 1H), 6. 44 (d, $J$ = 8. 0 Hz, 1H), 5. 15-5. 11 (m, 1H), 3. 13-3. 06 (m, 4H), 2. 95-2. 77 (m, 5H), 2,70-2. 65 (m, 2H), 2. 61-2. 60 (m, 1H), 2. 49 (s, 3H), 2. 28-2. 25 (m, 1H), 2. 15-2. 05 (m, 2H), 1. 95-1. 92 (m, 3H), 1. 61-1. 55 (m, 5H), 1. 48-1. 40 (m, 5H), 1. 36 -1. 25 (m, 6H), 1. 09 - 1. 02 (m, 4H). LC/MS (ESI) calcd for $C_{33}H_{48}N_5O_3^+$ [M+H]$^+$, 562.38; found, 562.4.

**Example 30: preparation of 3-(5-((7-(3-azabicyclo[3.1.0]hexan-3-yl)heptyl)amino)-2-methyl-4-oxoquinazolin-3(4H)-yl)piperidine-2,6-dione (GT-02772)**

Following the method of Scheme 1, under appropriate conditions understood in the field, the target compound **GT-02772** was prepared (white solid, 15 mg, yield 47%). $^1$H NMR (400 MHz, DMSO) δ 11. 02 (s, 1H), 10. 04 (brs, 1H), 8. 29 br(s, 1H), 7. 52 (t, *J* = 8. 0 Hz, 1H), 6. 67 (d, *J* = 8. 0 Hz, 1H), 6. 54 (d, *J* = 8. 0 Hz, 1H), 5. 23-5. 19 (m, 1H), 3.54-3.50 (m, 2H), 3. 23-3. 15 (m, 5H), 3. 07-3. 02 (m, 2H), 2. 86-2. 82 (m, 1H), 2. 72-2. 67 (m, 1H), 2. 63 - 2.58 (m, 5H), 2. 19-2. 14 (m, 1H), 1. 76-1. 70 (m, 2H), 1. 64-1. 57 (m, 4H), 1. 40-1. 34 (m, 4H), 0. 92-0. 88 (m, 1H), 0. 67-0. 61 (m, 1H). LC/MS (ESI) calcd for $C_{26}H_{36}N_5O_3^+$ [M+H]$^+$, 466. 28; found, 466. 3.

**Example 31: preparation of 3-(5-((7-(2,2-difluoro-7-azaspiro[3.5]nonan-7-yl)heptyl)amino)-2-methyl-4-oxoquinazolin-3(4H)-yl)piperidine-2,6-dione (GT-02773)**

Following the method of Scheme 1, under appropriate conditions understood in the field, the target compound **GT-02773** was prepared (white solid, 13 mg, yield 34%). $^1$H NMR (400 MHz, DMSO) *δ* 11. 04 (s, 1H), 9. 99 (brs, 1H), 7. 54 (t, *J* = 8. 0 Hz, 1H), 6. 70 (d, *J* = 8. 0 Hz, 1H), 6. 57 (d, *J* = 8. 0 Hz, 1H), 5. 26-5. 21 (m, 1H), 3.34-3. 29 (m, 5H), 3. 20-3. 16 (m, 3H), 3. 01-2. 97 (m, 3H), 2. 88-2. 82 (m, 4H), 2. 63 (s, 3H), 2.59-2.54 (m, 2H), 2. 20-2. 16 (m, 1H), 1. 98-1. 92 (m, 2H), 1. 82-1. 79 (m, 3H), 1. 67-1. 58 (m, 5H), 1. 53-1. 50 (m, 1H). LC/MS (ESI) calcd for $C_{29}H_{40}F_2N_5O_3^+$ [M+H]$^+$, 544. 31; found, 544. 3.

**Example 32: preparation of 3-(5-((7-((4-fluorobicyclo[2.2.2]octan-1-yl)amino)heptyl)amino)-2-methyl-4-oxoquinazolin-3(4H)-yl)piperidine-2,6-dione (GT-02774)**

Following the method of Scheme 1, under appropriate conditions understood in the field, the target compound **GT-02774** was prepared (white solid, 15 mg, yield 41%). $^1$H NMR (400 MHz, DMSO) *δ* 11. 01 (s, 1H), 8. 61 (brs, 2H), 7. 51 (t, *J* = 8. 0 Hz, 1H), 6. 66 (d, *J* = 8. 0 Hz, 1H), 6. 53 (d, *J* = 8. 0 Hz, 1H), 5. 23-5. 20 (m, 1H), 3. 21-3. 15 (m, 4H), 2. 89-2. 81 (m, 4H), 2. 68-2. 64 (m, 2H), 2.58 (s, 3H), 2. 34-2. 32 (m, 1H), 2. 20-2. 13 (m, 2H), 1. 99-1. 95 (m, 6H), 1. 64-1. 56 (m, 6H), 1. 29-1. 24 (m, 5H). LC/MS (ESI) calcd for $C_{29}H_{41}FN_5O_3^+$ [M+H]$^+$, 526. 31; found, 526. 3.

**Example 33: preparation of 3-(5-(5-(((3s,5s,7s)-adamantan-1-yl)amino)pent-1-yn-1-yl)-2-methyl-4-oxoquinazolin-3(4H)-yl)piperidine-2,6-dione (GT-02632)**

The target compound 3-(5-(5-(((3s,5s,7s)-adamantan-1-yl)amino)pent-1-yn-1-yl)-2-methyl-4-oxoquinazolin-3(4H)-yl)piperidine-2,6-dione (GT-02632) was prepared by referring to the method of step 4 of Scheme 5.

5-(3-(2,6-dioxopiperidin-3-yl)-2-methyl-4-oxo-3,4-dihydroquinazolin-5-yl)pent-4-yn-1-yl methanesulfonate (729092) (1 eq.) and adamantaneamine (2 eq.) were added into a 10 mL reaction flask, followed by the addition of NMP (1 mL), and then under nitrogen protection, sodium iodide (1.5 eq.) and DIPEA (3 eq.). After the addition was complete, the mixture was heated to 85°C and stirred for 3 hours. After monitoring the reaction via LCMS and confirming its completion, the reaction mixture was subjected to preparative HPLC (0.05% HCl aq./acetonitrile) to give the target compound (GT-02632) (pale yellow solid, 10.0 mg, yield 39.5%). $^1$H NMR (400 MHz, MeOD) δ 7. 89 (t, J= 7. 9 Hz, 1H), 7. 69 (dd, *J*= 23. 7, 7. 9 Hz, 2H), 5. 41 (dd, *J*= 11. 1, 5. 9 Hz, 1H), 3.30 - 3. 23 (m, 3H), 3. 03 - 2. 82 (m, 5H), 2. 75 (t, *J* = 6. 8 Hz, 2H), 2. 33 (d, *J* = 5. 7 Hz, 1H), 2. 23 (s, 3H), 2. 02 (dd, *J*= 14.4, 7. 0 Hz, 2H), 1. 95 (s, 6H), 1. 78 (dd, *J* = 31. 3, 12. 6 Hz, 6H). LCMS (ESI) calcd for $C_{29}H_{35}N_4O_3^+$ [M+H]$^+$, 487.27; found, 487.3.

**Example 34: preparation of 3-(2-methyl-4-oxo-5-(5-(piperidin-1-yl)pent-1-yn-1-yl)quinazolin-3(4H)-yl)piperidine-2,6-dione (GT-02633)**

Following the method of Scheme 5 and the method of example 33, under appropriate conditions understood in the field, the target compound **GT-02633** was prepared (pale yellow solid, 10.0 mg, yield 39.5%). $^1$H NMR (400 MHz, MeOD) δ 7. 80 (t, *J*= 7. 9 Hz, 1H), 7. 68 - 7. 57 (m, 2H), 5. 36 (dd, *J* = 11. 2, 5. 9 Hz, 1H), 3. 60 (d, *J* = 11. 6 Hz, 2H), 3.51 (dd, *J* = 9. 0, 7. 0 Hz, 2H), 3. 16 (s, 1H), 2. 93 (dd, *J*= 22. 1, 10. 6 Hz, 4H), 2. 82 - 2. 70 (m, 5H), 2. 30 (d, *J* = 4. 9 Hz, 1H), 2. 11 - 1. 98 (m, 4H), 1. 91 - 1. 73 (m, 4H). LCMS (ESI) calcd for $C_{24}H_{29}N_4O_3^+$ [M+H]$^+$, 421. 22; found, 421. 2.

**Example 35: preparation of 3-(2-methyl-5-(5-(4-methylpiperazin-1-yl)pent-1-yn-1-yl)-4-oxoquinazolin-3(4H)-yl) piperidine-2,6-dione (GT-02634)**

Following the method of Scheme 5 and the method of example 33, under appropriate conditions understood in the field, the target compound **GT-02634** was prepared (pale yellow solid, 6.0 mg, yield 35.38%). $^1$H NMR (400 MHz, MeOD) δ 7. 88 (t, *J* = 7. 9 Hz, 1H), 7. 69 (dd, *J*= 25. 3, 7. 8 Hz, 2H), 5. 42 (d, *J* = 10. 1 Hz, 1H), 3. 63 (dd, *J*= 16. 1, 8. 1 Hz, 11H),

3. 05 (s, 3H), 2. 92 (ddd, *J* = 29. 2, 15. 3, 10. 3 Hz, 5H), 2. 77 (t, *J* = 6. 5 Hz, 2H), 2. 37 - 2. 27 (m, 1H), 2. 18 (dd, *J*= 14. 1, 7. 2 Hz, 2H). LCMS (ESI) calcd for $C_{24}H_{30}N_5O_3^+$ $[M+H]^+$, 436.23; found, 436.2.

**Example 36: preparation of 3-(2-methyl-5-(5-(4-(oxetan-3-yl)piperazin-1-yl)pent-1-yn-1-yl)-4-oxoquinazolin-3(4H)-yl)piperidine-2,6-dione (GT-02635)**

Following the method of Scheme 5 and the method of example 33, under appropriate conditions understood in the field, the target compound **GT-02635** was prepared (pale yellow solid, 8. 0 mg, yield 39. 96%). $^1H$ NMR (400 MHz, MeOD) δ 7. 88 (t, *J*= 8. 0 Hz, 1H), 7. 69 (dd, *J*= 20. 8, 7. 8 Hz, 2H), 5. 45 (d, *J*= 5. 6 Hz, 1H), 4. 86 - 4. 70 (m, 4H), 3. 97 - 3. 41 (m, 8H), 3. 18 (d, *J*= 21. 6 Hz, 3H), 3. 04 - 2.51 (m, 8H), 2. 36 (d, *J* = 10. 3 Hz, 1H), 2. 11 (s, 2H). LCMS (ESI) calcd for $C_{26}H_{32}N_5O_4^+$ $[M+H]^+$, 478. 24; found, 478. 2.

**Example 37: preparation of 3-(2-methyl-5-(5-(4-methyl-1,4-diazepan-1-yl)pent-1-yn-1-yl)-4-oxoquinazolin-3(4H)-yl)piperidine-2,6-dione (GT-02636)**

Following the method of Scheme 5 and the method of example 33, under appropriate conditions understood in the field, the target compound **GT-02636** was prepared (pale yellow solid, 3. 0 mg, yield 15. 02%). LCMS (ESI) calcd for $C_{25}H_{32}N_5O_3^+$ $[M+H]^+$, 450. 25; found, 450. 3.

**Example 38: preparation of 3-(5-(5-(6-oxa-3-azabicyclo[3.1.1]heptan-3-yl)pent-1-yn-1-yl)-2-methyl-4-oxoquinazolin-3(4H)-yl)piperidine-2,6-dione (GT-02815)**

Following the method of Scheme 5 and the method of example 33, under appropriate conditions understood in the field, the target compound **GT-02815** was prepared (pale yellow solid, 9. 0 mg, yield 39. 47%). $^1H$ NMR (400 MHz, DMSO) δ 11. 00 (d, *J* = 11. 6 Hz, 1H), 7. 81 - 7. 67 (m, 1H), 7. 64 - 7. 51 (m, 2H), 5. 26 (d, *J* = 6. 1 Hz, 1H), 4. 61 (s, 1H), 3. 76 - 3. 69 (m, 1H), 3.53 (s, 1H), 3.30 (d, *J*= 7. 1 Hz, 2H), 3. 18 (d, *J*= 9. 9 Hz, 2H), 2. 92 - 2. 81 (m, 1H), 2. 75 - 2.56 (m, 8H), 2. 44 (d, *J* = 9. 9 Hz, 1H), 2. 22 - 2. 12 (m, 2H), 2. 12 - 1. 99 (m, 2H). LCMS (ESI) calcd for $C_{24}H_{27}N_4O_4^+$ $[M+H]^+$, 435. 20; found, 435. 2.

**Example 39: preparation of 3-(2-methyl-5-(5-(4-(4-methylpiperazin-1-yl)piperidin-1-yl)pent-1-yn-1-yl)-4-oxoquinazolin-3(4H)-yl)piperidine-2,6-dione (GT-02816)**

Following the method of Scheme 5 and the method of example 33, under appropriate conditions understood in the field, the target compound **GT-02816** was prepared (pale yellow solid, 5. 0 mg, yield 18. 07%). $^1H$ NMR (400 MHz, DMSO) δ 11. 11 (s, 1H), 10. 41 (s, 1H), 7. 81 - 7. 68 (m, 1H), 7. 63 - 7. 48 (m, 2H), 5. 30 (dd, *J* = 11. 6, 5. 6 Hz, 1H), 3. 65 (s, 8H), 2. 82 (s, 8H), 2. 73 - 2.58 (m, 9H), 2. 11 (dd, *J*= 68. 3, 5. 7 Hz, 8H). LCMS (ESI) calcd for $C_{29}H_{39}N_6O_3^+$ $[M+H]^+$, 519. 31; found, 519. 3.

**Example 40: preparation of 3-(2-methyl-5-(5-(4-morpholinopiperidin-1-yl)pent-1-yn-1-yl)-4-oxoquinazolin-3(4H)-yl)piperidine-2,6-dione (GT-02817)**

Following the method of Scheme 5 and the method of example 33, under appropriate conditions understood in the field, the target compound **GT-02817** was prepared (pale yellow solid, 8. 0 mg, yield 30. 48%). $^1H$ NMR (400 MHz, DMSO) δ 11. 53 (s, 1H), 11. 09 (d, *J*= 25. 4 Hz, 1H), 10. 74 (s, 1H), 7. 78 - 7. 72 (m, 1H), 7. 57 (dd, *J*= 16. 7, 7. 8 Hz, 2H), 5. 31 (dd, *J* = 11. 7, 5. 4 Hz, 1H), 4. 00 (s, 2H), 3. 89 (d, *J*= 11. 5 Hz, 2H), 3. 69 (d, *J*= 11. 4 Hz, 2H), 3.37 - 3.32 (m, 2H), 3. 08 (s, 4H), 2. 86 (s, 4H), 2. 71 - 2. 61 (m, 9H), 2.35 (d, *J* = 11. 4 Hz, 1H), 2. 17 (d, *J* = 8. 7 Hz, 2H), 2. 02 (s, 2H). LCMS (ESI) calcd for $C_{28}H_{36}N_5O_4^+$ $[M+H]^+$, 506. 28; found, 506. 3.

**Example 41: preparation of 3-(5-(5-((1-((3r,5r,7r)-adamantan-1-yl)ethyl)amino)pent-1-yn-1-yl)-2-methyl-4-oxoquinazolin-3(4H)-yl)piperidine-2,6-dione (GT-02818)**

Following the method of Scheme 5 and the method of example 33, under appropriate conditions understood in the field, the target compound **GT-02818** was prepared (pale yellow solid, 9. 0 mg, yield 33.37%). $^1H$ NMR (400 MHz, DMSO) δ 10. 93 (s, 1H), 8. 38 (s, 1H), 7. 74 - 7. 64 (m, 1H), 7. 52 - 7. 45 (m, 2H), 5. 19 (dd, *J*= 11. 3, 5. 2 Hz, 1H), 3. 21 - 3. 10 (m, 1H), 3. 04 (s, 1H), 2. 89 - 2. 74 (m, 1H), 2. 72 - 2. 65 (m, 1H), 2. 65 - 2.53 (m, 7H), 2. 10 (d, *J* = 6. 8 Hz, 1H), 2. 01 - 1. 81 (m, 5H), 1. 57 (t, *J*= 12. 3 Hz, 9H), 1. 44 (d, *J*= 11. 5 Hz, 3H), 1. 07 (d, *J* = 12. 4 Hz, 3H). LCMS (ESI) calcd for $C_{31}H_{39}N_4O_3^+$ $[M+H]^+$, 515. 30; found, 515. 3.

**Example 42: preparation of 3-(5-(8-(((3s,5s,7s)-adamantan-1-yl)amino)oct-1-yn-1-yl))-2-methyl-4-oxoquinazolin-3(4H)-yl)piperidine-2,6-dione (GT-02638)**

The target compound 3-(5-(8-(((3s,5s,7s)-adamantan-1-yl)amino)oct-1-yn-1-yl))-2-methyl-4-oxoquinazolin-3(4H)-yl)piperidine-2,6-dione (GT-02638) was prepared by referring to the method of step 4 of Scheme 5 and the method of example 33.

8-(3-(2,6-dioxopiperidin-3-yl)-2-methyl-4-oxo-3,4-dihydroquinazolin-5-yl)oct-7-yn-1-yl methanesulfonate (n=6: 729101) (1 eq.) and adamantaneamine (2 eq.) were added into a 10 mL reaction flask, followed by the addition of NMP (1 mL), and then under nitrogen protection, sodium iodide (1.5 eq.) and DIPEA (3 eq.). After the addition was complete, the mixture was heated to 85°C and stirred for 3 hours. After monitoring the reaction via LCMS and confirming its completion, the reaction mixture was subjected to preparative HPLC (0.05% HCl aq./acetonitrile) to give the target compound (GT-02638) (pale yellow solid, 8. 0 mg, yield 35. 39%). $^1$H NMR (400 MHz, MeOD) δ 7. 87 (t, *J* = 8. 0 Hz, 1H), 7. 65 (dd, *J*= 25. 1, 7. 3 Hz, 2H), 5. 46 - 5. 33 (m, 1H), 3. 10 - 2. 69 (m, 8H), 2.57 (d, *J* = 6. 1 Hz, 2H), 2. 37 - 2. 28 (m, 1H), 2. 20 (s, 3H), 1. 92 (d, J= 2. 2 Hz, 6H), 1. 84 - 1. 66 (m, 12H), 1. 52 (d, *J* = 7. 9 Hz, 2H). LCMS (ESI) calcd for $C_{32}H_{41}N_4O_3^+$ [M+H]$^+$, 529.32; found, 529.3.

**Example 43: preparation of 3-(2-methyl-5-(8-(4-(oxetan-3-yl)piperazin-1-yl)oct-1-yn-1-yl)-4-oxoquinazolin-3(4H)-yl)piperidine-2,6-dione (GT-02639)**

Following the method of Scheme 5 and the method of example 42, under appropriate conditions understood in the field, the target compound **GT-02639** was prepared (pale yellow solid, 9. 0 mg, yield 40. 46%). $^1$H NMR (400 MHz, MeOD) *δ* 7. 79 (t, *J* = 8. 0 Hz, 1H), 7. 57 (dd, *J*= 31. 0, 7. 9 Hz, 2H), 5. 32 (dd, *J* = 11. 3, 5. 8 Hz, 1H), 4. 75 - 4.50 (m, 4H), 4. 29 - 3. 27 (m, 9H), 3. 16 - 3. 10 (m, 2H), 2. 90 - 2. 64 (m, 6H), 2. 46 (s, 2H), 2. 29 - 2. 15 (m, 1H), 1. 70 (dd, *J* = 11. 8, 7. 9 Hz, 2H), 1. 59 (s, 4H), 1. 38 (d, *J* = 7. 1 Hz, 2H). LCMS (ESI) calcd for $C_{29}H_{38}N_5O_4^+$ [M+H]$^+$, 520. 29; found, 520. 3.

**Example 44: preparation of 3-(2-methyl-5-(8-(4-methylpiperazin-1-yl)oct-1-yn-1-yl)-4-oxoquinazolin-3(4H)-yl)piperidine-2,6-dione (GT-02645)**

Following the method of Scheme 5 and the method of example 42, under appropriate conditions understood in the field, the target compound **GT-02645** was prepared (pale yellow solid, 9. 0 mg, yield 43. 77%). $^1$H NMR (400 MHz, MeOD) δ 7. 87 (t, *J* = 7. 9 Hz, 1H), 7. 66 (dd, *J*= 23. 2, 7. 8 Hz, 2H), 5. 40 (dd, *J* = 11. 4, 5. 8 Hz, 1H), 3. 75 (tt, *J*= 13. 2, 6. 7 Hz, 8H), 3. 27 (d, *J* = 7. 4 Hz, 2H), 3. 04 (s, 3H), 3. 02 - 2. 65 (m, 6H), 2.57 (s, 2H), 2. 34 (d, *J* = 5.5 Hz, 1H), 1. 85 (dd, *J*= 19. 6, 12. 3 Hz, 2H), 1. 70 (s, 4H), 1. 50 (s, 2H). LCMS (ESI) calcd for $C_{27}H_{36}N_5O_3^+$ [M+H]$^+$, 478. 28; found, 478. 3.

**Example 45: preparation of 3-(2-methyl-5-(8-(4-(4-methylpiperazin-1-yl)piperidin-1-yl)oct-1-yn-1-yl)-4-oxoquinazolin-3(4H)-yl)piperidine-2,6-dione (GT-02653)**

Following the method of Scheme 5 and the method of example 42, under appropriate conditions understood in the field, the target compound **GT-02653** was prepared (pale yellow solid, 15.0 mg, yield 62.79%). $^1$H NMR (400 MHz, MeOD) δ 7. 93 *(t, J*= 8. 0 Hz, 1H), 7. 70 (dd, J= 32. 8, 7. 5 Hz, 2H), 5.53 - 5. 36 (m, 1H), 4. 04 - 3. 41 (m, 12H), 3. 22 - 3. 06 (m, 4H), 3. 06 - 2. 88 (m, 6H), 2. 82 (dd, *J* = 21. 2, 13. 6 Hz, 2H), 2.57 (d, *J* = 5. 6 Hz, 2H), 2.51 - 2. 31 (m, 3H), 2. 24 - 2. 08 (m, 2H), 1. 78 (dd, *J* = 17. 2, 9. 3 Hz, 2H), 1. 70 (s, 4H), 1. 48 (d, *J* = 7. 3 Hz, 2H). LCMS (ESI) calcd for $C_{32}H_{45}N_6O_3^+$ [M+H]$^+$, 561.35; found, 561.4.

**Example 46: preparation of 3-(5-(8-(6-oxa-3-azabicyclo[3.1.1]heptan-3-yl)oct-1-yn-1-yl)-2-methyl-4-oxoquinazolin-3(4H)-yl)piperidine-2,6-dione (GT-02654)**

Following the method of Scheme 5 and the method of example 42, under appropriate conditions understood in the field, the target compound **GT-02654** was prepared (pale yellow solid, 10. 0 mg, yield 48. 73%). $^1$H NMR (400 MHz, MeOD) δ 7. 75 (t, *J* = 8. 0 Hz, 1H), 7. 62 - 7. 45 (m, 2H), 5. 29 (d, *J*= 11. 6 Hz, 1H), 4.55 (d, *J*= 6. 5 Hz, 2H), 3. 72 (dt, *J*= 34. 6, 17. 3 Hz, 2H), 3.52 - 3.34 (m, 2H), 3. 29 (dd, *J* = 20. 1, 9. 6 Hz, 2H), 3. 04 (dd, *J*= 19. 9, 11. 4 Hz, 2H), 2. 92 - 2.56 (m, 6H), 2. 47 (s, 2H), 2. 21 (dd, *J* = 13. 0, 8. 9 Hz, 1H), 1. 80 - 1. 68 (m, 2H), 1. 59 (s, 4H), 1. 40 (s, 2H). LCMS (ESI) calcd for $C_{27}H_{33}N_4O_4^+$ [M+H]$^+$, 477. 25; found, 477. 3.

**Example 47: preparation of 3-(5-(5-(((3s,5s,7s)-adamantan-1-yl)amino)pentyl)-2-methyl-4-oxoquinazolin-3(4H)-yl)piperidine-2,6-dione (GT-02759)**

The target compound 3-(5-(5-(((3s,5s,7s)-adamantan-1-yl)amino)pentyl)-2-methyl-4-oxoquinazolin-3(4H)-yl)

piperidine-2,6-dione (GT-02759) was prepared by referring to the method of step 3 of Scheme 6.

5-(3-(2,6-dioxopiperidin-3-yl)-2-methyl-4-oxo-3,4-dihydroquinazolin-5-yl)pentyl methanesulfonate (729109) (1 eq.) and adamantaneamine (2 eq.) were added into a 10 mL reaction flask, followed by the addition of NMP (1 mL), and then under nitrogen protection, sodium iodide (1.5 eq.) and DIPEA (3 eq.). After the addition was complete, the mixture was heated to 85°C and stirred for 3 hours. After monitoring the reaction via LCMS and confirming its completion, the reaction mixture was subjected to preparative HPLC (0.05% HCl aq./acetonitrile) to give the target compound GT-02759 (pale yellow solid, 9.0 mg, yield 35.26%). $^1$H NMR (400 MHz, MeOD) $\delta$ 7. 75 (t, *J* = 7. 9 Hz, 1H), 7. 47 (d, *J* = 7. 3 Hz, 1H), 7. 39 (d, *J* = 7. 4 Hz, 1H), 5. 27 (dd, *J* = 11. 1, 5. 8 Hz, 1H), 3. 25 (d, *J*= 12. 3 Hz, 1H), 3. 09 (dt, *J*= 16. 5, 8. 7 Hz, 1H), 2. 97 - 2. 81 (m, 3H), 2. 79 (s, 3H), 2. 75 - 2. 60 (m, 2H), 2. 22 (t, *J* = 7. 6 Hz, 1H), 2. 11 (s, 3H), 1. 81 (d, *J* = 2. 4 Hz, 7H), 1. 61 (ddd, *J* = 24. 7, 22.5, 10. 3 Hz, 11H), 1. 49 -1. 37 (m, 2H). LCMS (ESI) calcd for $C_{29}H_{39}N_4O_3^+$ [M+H]$^+$, 491.30; found, 491.3.

**Example 48: preparation of 3-(2-methyl-5-(5-(4-(oxetan-3-yl)piperazin-1-yl)pentyl)-4-oxoquinazolin-3(4H)-yl)piperidine-2,6-dione (GT-02761)**

Following the method of Scheme 6 and the method of example 47, under appropriate conditions understood in the field, the target compound **GT-02761** was prepared (pale yellow solid, 10. 0 mg, yield 39. 86%). $^1$H NMR (400 MHz, MeOD) $\delta$ 7. 95 (t, *J*= 7. 9 Hz, 1H), 7. 64 (dd, *J*= 14. 6, 7. 9 Hz, 2H), 5. 47 (dd, *J* = 11. 3, 5. 8 Hz, 1H), 4. 87 - 4. 73 (m, 3H), 4. 31 (ddd, *J* = 95. 3, 10. 4, 5. 1 Hz, 1H), 3. 90 - 3.35 (m, 10H), 3.31 - 3. 23 (m, 2H), 3. 22 - 3. 14 (m, 1H), 3. 02 (d, *J* = 5. 1 Hz, 3H), 2. 99 - 2. 78 (m, 3H), 2. 43 - 2. 28 (m, 1H), 1. 90 (dt, *J* = 15. 6, 7. 7 Hz, 2H), 1. 68 (tt, *J* = 14. 1, 7. 2 Hz, 2H), 1. 62 - 1. 47 (m, 2H). LCMS (ESI) calcd for $C_{26}H_{36}N_5O_4^+$ [M+H]$^+$, 482. 28; found, 482. 3.

**Example 49: preparation of 3-(5-(5-(6-oxa-3-azabicyclo[3.1.1]heptan-3-yl)pentyl)-2-methyl-4-oxoquinazolin-3(4H)-yl)piperidine-2,6-dione (GT-02762)**

Following the method of Scheme 6 and the method of example 47, under appropriate conditions understood in the field, the target compound **GT-02762** was prepared (pale yellow solid, 9. 0 mg, yield 39. 13%). $^1$H NMR (400 MHz, MeOD) δ 7. 87 (td, *J* = 7. 9, 4. 1 Hz, 1H), 7. 60 (d, *J* = 8. 2 Hz, 1H), 7. 52 (t, *J* = 7. 8 Hz, 1H), 5. 40 (dd, *J* = 11. 0, 5. 8 Hz, 1H), 4. 80 - 4. 21 (m, 2H), 3. 88 - 3. 70 (m, 1H), 3. 65 - 3.34 (m, 4H), 3. 18 (ddd, *J* = 28. 5, 24. 2, 20. 0 Hz, 4H), 3. 01 - 2. 73 (m, 6H), 2.50 - 2. 26 (m, 2H), 1. 90 (ddd, *J* = 37. 8, 19. 2, 7. 2 Hz, 2H), 1. 74 - 1. 45 (m, 4H). LCMS (ESI) calcd for $C_{24}H_{31}N_4O_4^+$ [M+H]$^+$, 439. 23; found, 439. 2.

**Example 50: preparation of 3-(2-methyl-5-(5-(4-methylpiperazin-1-yl)pentyl)-4-oxoquinazolin-3(4H)-yl)piperidine-2,6-dione (GT-02763)**

Following the method of Scheme 6 and the method of example 47, under appropriate conditions understood in the field, the target compound **GT-02763** was prepared (pale yellow solid, 9. 0 mg, yield 38. 22%). $^1$H NMR (400 MHz, MeOD) δ 7. 94 (t, *J*= 7. 9 Hz, 1H), 7. 69 - 7. 54 (m, 2H), 5. 45 (dd, *J*= 11. 0, 5. 7 Hz, 1H), 3. 71 (dd, *J* = 64.5, 18. 1 Hz, 8H), 3. 45 - 3.34 (m, 2H), 3.31 (d, *J* = 12.5 Hz, 2H), 3. 17 (ddd, *J* = 12.5, 9. 4, 6. 3 Hz, 1H), 3. 04 (d, *J* = 3. 9 Hz, 3H), 3. 02 - 2. 78 (m, 5H), 2. 44 - 2. 28 (m, 1H), 1. 99 - 1. 83 (m, 2H), 1. 76 - 1. 62 (m, 2H), 1. 56 (dd, *J*= 14. 7, 7. 2 Hz, 2H). LCMS (ESI) calcd for $C_{24}H_{34}N_5O_3^+$ [M+H]$^+$, 440. 27; found, 440. 3.

**Example 51: preparation of 3-(2-methyl-5-(5-(4-(4-methylpiperazin-1-yl)piperidin-1-yl)pentyl)-4-oxoquinazolin-3(4H)-yl)piperidine-2,6-dione (GT-02764)**

Following the method of Scheme 6 and the method of example 47, under appropriate conditions understood in the field, the target compound **GT-02764** was prepared (pale yellow solid, 10. 0 mg, yield 36. 16%). $^1$H NMR (400 MHz, MeOD) δ 7. 96 (t, *J* = 7. 9 Hz, 1H), 7. 64 (dd, *J* = 14. 3, 7. 9 Hz, 2H), 5. 48 (dd, *J* = 11. 1, 5. 7 Hz, 1H), 3. 67 (dd, *J* = 85. 3, 32. 7 Hz, 11H), 3. 45 - 3.35 (m, 2H), 3. 24 - 3. 08 (m, 5H), 3. 08 - 2. 92 (m, 6H), 2. 92 - 2. 80 (m, 2H), 2. 49 (d, *J* = 13. 0 Hz, 2H), 2. 40 (dd, *J* = 10. 6, 5. 2 Hz, 1H), 2. 27 - 2. 11 (m, 2H), 1. 95 - 1. 80 (m, 2H), 1. 67 (s, 2H), 1. 56 (dd, *J* = 14. 1, 6. 9 Hz, 2H). LCMS (ESI) calcd for $C_{29}H_{43}N_6O_3^+$ [M+H]$^+$, 523.34; found, 523.3.

**Example 52: preparation of 3-(5-(8-(((1s,3s)-adamantan-1-yl)amino)octyl)-2-methyl-4-oxoquinazolin-3(4H)-yl) piperidine-2,6-dione (GT-02797)**

The target compound 3-(5-(8-(((1s,3s)-adamantan-1-yl)amino)octyl)-2-methyl-4-oxoquinazolin-3(4H)-yl)piperidine-2,6-dione (GT-02797) was prepared by referring to the method of step 3 of Scheme 6.

8-(3-(2,6-dioxopiperidin-3-yl)-2-methyl-4-oxo-3,4-dihydroquinazolin-5-yl)octyl methanesulfonate (729119) (1 eq.) and adamantaneamine (2 eq.) were added into a 10 mL reaction flask, followed by the addition of NMP (1 mL),

and then under nitrogen protection, sodium iodide (1.5 eq.) and DIPEA (3 eq.). After the addition was complete, the mixture was heated to 85°C and stirred for 3 hours. After monitoring the reaction via LCMS and confirming its completion, the reaction mixture was subjected to preparative HPLC (0.05% HCl aq./acetonitrile) to give the target compound **GT-02797** (pale yellow solid, 9. 0 mg, yield 35. 77%). $^1$H NMR (400 MHz, MeOD) δ 7. 77 (t, *J* = 7. 9 Hz, 1H), 7. 48 (d, *J* = 7. 4 Hz, 1H), 7. 41 (d, *J* = 7. 5 Hz, 1H), 5. 29 (dd, *J* = 11. 0, 5. 9 Hz, 1H), 3. 19 - 3. 03 (m, 2H), 2. 93 - 2. 60 (m, 8H), 2. 27 - 2. 17 (m, 1H), 2. 11 (s, 3H), 1. 82 (d, *J* = 2.5 Hz, 6H), 1. 67 (dd, *J*= 32. 3, 12. 0 Hz, 6H), 1. 50 (td, *J*= 14. 7, 7. 3 Hz, 4H), 1. 31 (s, 8H). LCMS (ESI) calcd for $C_{32}H_{45}N_4O_3^+$ [M+H]$^+$, 533.35; found, 533.3.

**Example 53: preparation of 3-(2-methyl-5-(8-(4-(oxetan-3-yl)piperazin-1-yl)octyl)-4-oxoquinazolin-3(4H)-yl)piperidine-2,6-dione (GT-02798)**

Following the method of Scheme 6 and the method of example 47, under appropriate conditions understood in the field, the target compound **GT-02798** was prepared (pale yellow solid, 12.0 mg, yield 48.46%). $^1$H NMR (400 MHz, MeOD) δ 7. 82 (t, *J* = 7. 9 Hz, 1H), 7. 49 (dd, *J*= 15. 9, 7. 5 Hz, 2H), 5. 34 (dd, *J* = 10. 9, 5. 8 Hz, 1H), 4. 67 (dt, *J* = 13. 0, 7.3 Hz, 4H), 4. 28 - 3. 22 (m, 9H), 3. 19 - 2. 99 (m, 4H), 2.96 - 2.53 (m, 6H), 2.26 (dt, *J*= 11. 1, 5.4 Hz, 1H), 1.69 (s, 2H), 1. 54 - 1. 41 (m, 2H), 1. 31 (s, 8H). LCMS (ESI) calcd for $C_{29}H_{42}N_5O_4^+$ [M+H]$^+$, 524.32; found, 524.3.

**Example 54: preparation of 3-(2-methyl-5-(8-(4-methylpiperazin-1-yl)octyl)-4-oxoquinazolin-3(4H)-yl)piperidine-2,6-dione (GT-02813)**

Following the method of Scheme 6 and the method of example 47, under appropriate conditions understood in the field, the target compound **GT-02813** was prepared (pale yellow solid, 14.0 mg, yield 61.12%). $^1$H NMR (400 MHz, MeOD) δ 7. 79 (t, *J* = 7. 9 Hz, 1H), 7. 46 (dd, *J* = 21. 5, 7. 4 Hz, 2H), 5. 31 (dd, *J* = 11. 2, 5. 7 Hz, 1H), 3. 99 - 3. 22 (m, 12H), 3. 19 - 3. 13 (m, 2H), 3. 04 (dt, *J*= 12. 2, 7. 7 Hz, 1H), 2. 92 (s, 3H), 2. 85 - 2. 79 (m, 1H), 2. 77 - 2. 65 (m, 2H), 2. 30 - 2. 19 (m, 1H), 1. 71 (s, 2H), 1. 58 - 1. 43 (m, 2H), 1. 30 (d, *J* = 15. 0 Hz, 8H). LCMS (ESI) calcd for $C_{27}H_{40}N_5O_3^+$ [M+H]$^+$, 482.31; found, 482.3.

**Example 55: preparation of 3-(2-methyl-5-(8-(4-(4-methylpiperazin-1-yl)piperidin-1-yl)octyl)-4-oxoquinazolin-3(4H)-yl)piperidine-2,6-dione (GT-02799)**

Following the method of Scheme 6 and the method of example 47, under appropriate conditions understood in the field, the target compound **GT-02799** was prepared (pale yellow solid, 13.0 mg, yield 48.91%). $^1$H NMR (400 MHz, MeOD) δ 7. 94 (t, *J* = 7. 9 Hz, 1H), 7. 70 - 7. 54 (m, 2H), 5. 48 (dd, *J* = 11. 2, 5. 7 Hz, 1H), 3. 97 - 3.37 (m, 12H), 3.32 - 3. 24 (m, 2H), 3. 23 - 3. 07 (m, 5H), 3. 05 - 2. 92 (m, 5H), 2. 92 - 2. 77 (m, 2H), 2.50 (d, *J* = 13.5 Hz, 2H), 2. 44 - 2. 35 (m, 1H), 2. 29 - 2. 10 (m, 2H), 1. 79 (s, 2H), 1. 59 (dt, *J* = 27. 3, 13.5 Hz, 2H), 1. 41 (d, *J* = 16. 5 Hz, 8H). LCMS (ESI) calcd for $C_{32}H_{49}N_6O_3^+$ [M+H]$^+$, 565.39; found, 565.4.

**Example 56: preparation of 3-(5-(8-(6-oxa-3-azabicyclo[3.1.1]heptan-3-yl)octyl)-2-methyl-4-oxoquinazolin-3(4H)-yl)piperidine-2,6-dione (GT-02800)**

Following the method of Scheme 6 and the method of example 47, under appropriate conditions understood in the field, the target compound **GT-02800** was prepared (pale yellow solid, 13.0 mg, yield 56.78%). $^1$H NMR (400 MHz, MeOD) δ 7. 76 (td, *J*= 8. 0, 2. 4 Hz, 1H), 7. 48 (d, *J*= 7. 5 Hz, 1H), 7. 40 (dd, *J* = 7. 4, 4. 0 Hz, 1H), 5. 29 (dd, *J* = 11. 3, 5. 8 Hz, 1H), 4. 67 - 4. 15 (m, 2H), 3. 90 - 3.53 (m, 2H), 3. 48 - 3.33 (m, 2H), 3.32 - 3. 22 (m, 2H), 3. 20 - 2. 97 (m, 4H), 2. 92 - 2.56 (m, 5H), 2. 24 (dd, *J* = 10. 2, 7. 6 Hz, 2H), 1. 83 - 1. 63 (m, 2H), 1. 49 (d, *J* = 6. 8 Hz, 2H), 1. 32 (s, 8H). LCMS (ESI) calcd for $C_{27}H_{37}N_4O_4^+$ [M+H]$^+$, 481.28; found, 481.3.

**Example 57: preparation of 3-(5-(3-(((1s,3s)-adamantan-1-yl)amino)prop-1-yn-1-yl)-2-methyl-4-oxoquinazolin-3(4H)-yl)piperidine-2,6-dione (GT-02814)**

Referring to the method of step 2 of Scheme 7, compound GT-02600 (1 eq.) was added to a 100 mL reaction flask, followed by the addition of $Pd(PPh_3)Cl_2$ (0. 1 eq.) and copper iodide (1 eq.), and then under nitrogen protection, DMF (25 mL), TEA (3 mL) and (1s,3s)-N-(prop-2-yn-1-yl)adamantan-1-amine (GT-M-24) (3 eq.). After the addition was complete, the mixture was heated to 90°C and stirred for 18 hours. After monitoring the reaction via LCMS and confirming its completion, the reaction mixture was filtered and subjected to preparative HPLC (0.05% HCl aq. / acetonitrile) to give the target compound GT-02814 (pale yellow solid, 30. 0 mg, yield 40. 26%). $^1$H NMR (400 MHz, DMSO) δ 10. 93 (d, *J* = 27. 4 Hz, 1H), 9. 30 (s, 2H), 7. 76 (t, *J* = 7. 8 Hz, 1H), 7. 61 (ddd, *J* = 10. 2, 5. 4, 4. 3 Hz, 2H), 5. 23 (dd, *J* = 11. 5, 5. 6 Hz, 1H), 4. 13 (t, *J* = 5. 6 Hz, 2H), 2. 84 - 2. 77 (m, 1H), 2. 63 - 2.55 (m, 5H), 2. 15 - 2. 09 (m, 1H), 2. 06 (s, 3H), 1. 83 (d, *J* = 24. 9 Hz, 6H), 1. 62 - 1. 51 (m, 6H). LCMS (ESI) calcd for $C_{27}H_{31}N_4O_3^+$ [M+H]$^+$, 459. 24; found, 459. 2.

**Example 58: preparation of 3-(5-((2-(2-(((1s,3s)-adamantan-1-yl)amino)ethoxy)ethyl)amino)-2-methyl-4-oxoquinazolin-3(4H)-yl)piperidine-2,6-dione (GT-**02234)

Referring to the method of step 3 of Scheme 8, (1s,3s)-N-(2-(2-aminoethoxy)ethyl)adamantan-1-amine (729023) (1 eq.) was added to a 10 mL reaction flask, followed by the addition of 2 mL DMSO, 3-(5-fluoro-2-methyl-4-oxoquinazolin-3(4H)-yl)piperidine-2,6-dione (1.1 eq.), and DIPEA (3 eq.). The mixture was then stirred at 130°C for 10 h. After monitoring the reaction via LCMS and confirming its completion, the reaction mixture was subjected to preparative HPLC (0.05% HCl aq./acetonitrile) to give the target compound **GT-02234** (pale yellow solid, 13.0 mg, yield 22. 04%). $^1$H NMR (400 MHz, MeOD) δ 7. 78 - 7. 68 (m, 1H), 6. 93 (d, *J* = 8. 7 Hz, 1H), 6. 80 (d, *J* = 7. 8 Hz, 1H), 5. 41 (dd, *J* = 11. 1, 5. 6 Hz, 1H), 3. 82 (dt, *J*= 12. 9, 5. 1 Hz, 4H), 3.58 (t, *J*= 5. 2 Hz, 2H), 3. 26 - 3. 21 (m, 2H), 3. 01 - 2. 85 (m, 4H), 2. 81 (td, *J* = 16. 5, 8. 1 Hz, 2H), 2. 41 - 2. 31 (m, 1H), 2. 23 (s, 3H), 1. 95 (d, *J* = 2. 4 Hz, 6H), 1. 82 - 1. 74 (m, 6H). LCMS (ESI) calcd for $C_{28}H_{38}N_5O_4^+$ $[M+H]^+$, 508. 29; found, 509. 0.

**Example 59: preparation of 3-(5-((2-(2-(((3as,6as)-hexahydro-2,5-methanopentalen-3a(1H)-yl)amino)ethoxy) ethyl)amino)-2-methyl-4-oxoquinazolin-3(4H)-yl)piperidine-2,6-dione (GT-02389)**

Following the method of Scheme 8 and the method of example 58, under appropriate conditions understood in the field, the target compound **GT-02389** was prepared (pale yellow solid, 10. 0 mg, yield 16. 92%). $^1$H NMR (400 MHz, MeOD) δ 7. 76 - 7. 64 (m, 1H), 6. 88 (d, *J* = 8. 6 Hz, 1H), 6. 79 (d, *J* = 7. 5 Hz, 1H), 5. 38 (dd, *J*= 11. 4, 5. 8 Hz, 1H), 3. 84 (dt, *J* = 10. 4, 5. 3 Hz, 4H), 3.57 (t, *J* = 5. 2 Hz, 2H), 3.30 - 3. 20 (m, 2H), 3. 05 - 2. 70 (m, 6H), 2. 43 (d, *J* = 5. 2 Hz, 3H), 2. 37 - 2. 28 (m, 1H), 2. 01 (q, *J* = 10. 3 Hz, 6H), 1. 73 (d, *J* = 8. 8 Hz, 3H), 1. 63 (d, *J* = 13. 1 Hz, 1H). LCMS (ESI) calcd for $C_{27}H_{36}N_5O_4^+$ $[M+H]^+$, 494. 28; found, 494. 7.

**Example 60: preparation of 3-(2-methyl-5-((2-(2-(4-methyl-1,4-diazepan-1-yl)ethoxy)ethyl)amino)-4-oxoquinazolin-3(4H)-yl)piperidine-2,6-dione (GT-02493)**

Following the method of Scheme 8 and the method of example 58, under appropriate conditions understood in the field, the target compound **GT-02493** was prepared (pale yellow solid, 20.0 mg, yield 31.24%). $^1$H NMR (400 MHz, MeOD) δ 7. 78 - 7. 67 (m, 1H), 6. 94 (d, *J* = 8. 7 Hz, 1H), 6. 82 (d, *J* = 7. 7 Hz, 1H), 5. 43 (dd, *J* = 11. 3, 5.5 Hz, 1H), 3. 89 (ddd, *J* = 18. 8, 9. 4, 4.4 Hz, 10H), 3. 65 - 3.50 (m, 6H), 3. 03 - 2. 78 (m, 9H), 2. 38 (dd, *J* = 9. 2, 6. 3 Hz, 3H). LCMS (ESI) calcd for $C_{24}H_{35}N_6O_4^+$ $[M+H]^+$, 471.27; found, 471.6.

**Example 61: preparation of (3r,5r,7r)-N-(5-((3-(2,6-dioxopiperidin-3-yl)-2-methyl-4-oxo-3,4-dihydroquinazolin-5-yl)amino)pentyl)adamantane-1-carboxamide (GT-02323)**

Following the method of Scheme 8 and the method of example 58, under appropriate conditions understood in the field, the target compound **GT-02323** was prepared (pale yellow solid, 26.0 mg, yield 35.03%). $^1$H NMR (400 MHz, MeOD) δ 7. 61 - 7. 49 (m, 1H), 6. 68 (d, *J* = 8. 6 Hz, 1H), 6. 61 (d, *J* = 7. 7 Hz, 1H), 5. 21 (dd, *J* = 10. 9, 5. 6 Hz, 1H), 3. 19 (t, *J* = 7. 1 Hz, 2H), 3. 12 - 3. 05 (m, 2H), 2. 89 - 2.53 (m, 6H), 2. 18 (dd, *J*= 8. 7, 4. 2 Hz, 1H), 1. 91 (s, 3H), 1. 74 (d, *J* = 2. 6 Hz, 6H), 1. 64 (dd, *J* = 14.4, 10. 5 Hz, 8H), 1. 45 (dd, *J* = 14.4, 7. 3 Hz, 2H), 1. 38 - 1. 27 (m, 2H). LCMS (ESI) calcd for $C_{30}H_{40}N_5O_4^+$ $[M+H]^+$, 534.3; found, 535.0.

**Example 62: preparation of 3-(5-((2-(2-(2-(((1s,3s)-adamantan-1-yl)amino)ethoxy)ethoxy)ethyl)amino)-2-methyl-4-oxoquinazolin-3(4H)-yl)piperidine-2,6-dione (GT-02279)**

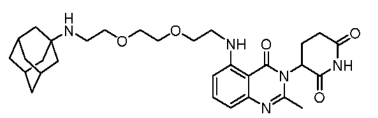

Following the method of Scheme 8 and the method of example 58, under appropriate conditions understood in the field, the target compound **GT-02279** was prepared (pale yellow solid, 10. 0 mg, yield 5. 94%). LCMS (ESI) calcd for $C_{30}H_{42}N_5O_5^+$ $[M+H]^+$, 552. 32; found, 552. 3. $^1$H NMR (500 MHz, DMSO) δ 11. 02 (s, 1H), 8. 45 (s, 3H), 7. 53 (t, *J* = 8. 1 Hz, 1H), 6. 70 (d, *J* = 7. 8 Hz, 1H), 6. 59 (d, *J* = 8. 4 Hz, 1H), 5. 22 (dd, *J*= 11. 6, 5. 6 Hz, 1H), 3. 66 (t, *J* = *5.* 1 Hz, 5H), 3. 61 (s, 5H), 3. 02 - 2. 96 (m, 2H), 2. 89 - 2. 80 (m, 1H), 2. 61 (s, 3H), 2. 19 - 2. 15 (m, 1H), 2. 11 - 2. 08 (m, 3H), 1. 83 - 1. 80 (m, 6H), 1. 66 - 1. 53 (m, 8H).

**Example 63: preparation of 3-(5-((5-(((3s,5s,7s)-adamantan-1-yl)amino)pentyl)amino)-2-methyl-4-oxoquinazolin-3(4H)-yl)piperidine-2,6-dione (GT-02280)**

Following the method of Scheme 8 and the method of example 58, under appropriate conditions understood in the field, the target compound **GT-02280** was prepared (pale yellow solid, 2.5 mg, yield 2. 13%). LC/MS (ESI) calcd for $C_{29}H_{40}N_5O_3^+$ $[M+H]^+$, 506.31; found, 506.3.

**Example 64: preparation of 3-(5-((4-(((1r,3r,5r,7r)-adamantan-2-yl)amino)butyl)amino)-2-methyl-4-oxoquinazolin-3(4H)-yl)piperidine-2,6-dione (GT-02392)**

Following the method of Scheme 8 and the method of example 58, under appropriate conditions understood in the field, the target compound **GT-02392** was prepared (pale yellow solid, 10 mg, yield 5. 81%). LCMS (ESI) calcd for $C_{28}H_{38}N_5O_3^+$ $[M+H]^+$, 492.30; found, 492.6. $^1$H NMR (500 MHz, DMSO) δ 11. 01 (s, 1H), 8. 37 (s, 2H), 7. 53 (t, *J*= 8. 1 Hz, 1H), 6. 69 (d, *J*= 7. 9 Hz, 1H), 6. 58 (d, *J* = 8. 4 Hz, 1H), 5. 22 (dd, *J* = 11. 4, 5. 6 Hz, 1H), 2. 99 - 2. 96 (m, 2H), 2. 90 - 2. 82 (m, 2H), 2. 77 (d, *J*= 4. 8 Hz, 1H), 2. 60 (s, 3H), 2. 16 - 2. 10 (m, 4H), 1. 75 - 1. 68 (m, 10H), 1. 59 - 1. 50 (m, 4H).

**Example 65: preparation of 3-(5-((5-(((1r,3r,5r,7r)-adamantan-2-yl)amino)pentyl)amino)-2-methyl-4-oxoquinazolin-3(4H)-yl)piperidine-2,6-dione (GT-02393)**

Following the method of Scheme 8 and the method of example 58, under appropriate conditions understood in the field, the target compound **GT-02393** was prepared (pale yellow solid, 10 mg, yield 9. 28%). LCMS (ESI) calcd for $C_{29}H_{40}N_5O_3^+$ $[M+H]^+$, 506.31; found, 506.8. $^1$H NMR (400 MHz, MeOD) δ 7. 69 - 7. 59 (m, 1H), 6. 81 - 6. 70 (m, 2H), 5. 30 (dd, *J* = 11. 2, 5. 9 Hz, 1H), 3.36 (d, *J* = 21. 6 Hz, 2H), 3. 10 - 2. 99 (m, 2H), 2. 92 - 2. 85 (m, 1H), 2. 80 (s, 3H), 2. 78 - 2. 71 (m, 1H), 2. 32 - 2. 25 (m, 1H), 2. 22 - 2. 14 (m, 2H), 2. 02 - 1. 91 (m, 6H), 1. 90 - 1. 71 (m, 12H), 1. 61 - 1. 50 (m, 2H).

**Example 66: preparation of 3-(5-((5-(((2R,3as,5S,6as)-hexahydro-2,5-methanopentalen-3a(1H)-yl)amino)pentyl)amino)-2-methyl-4-oxoquinazolin-3(4H)-yl)piperidine-2,6-dione (GT-02496)**

Following the method of Scheme 8 and the method of example 58, under appropriate conditions understood in the field, the target compound **GT-02496** was prepared (pale yellow solid, 10 mg, yield 6. 74%). LCMS (ESI) calcd for $C_{28}H_{38}N_5O_3^+$ $[M+H]^+$, 492.30; found, 492.8. $^1$H NMR (400 MHz, MeOD) δ 7. 73 - 7. 65 (m, 1H), 6. 84 (d, *J*= 8. 6 Hz, 1H), 6. 74 (d, *J*= 7. 4 Hz, 1H), 5. 36 (dd, *J*= 11. 0, 5. 9 Hz, 1H), 3.39 - 3.33 (m, 2H), 3. 05 - 2. 98 (m, 2H), 2. 88 (s, 3H), 2. 85 - 2. 73 (m, 2H), 2. 48 - 2. 38 (m, 3H), 2. 37 - 2. 27 (m, 1H), 2. 07 - 1. 92 (m, 6H), 1. 84 - 1. 68 (m, 7H), 1. 64 - 1. 53 (m, 3H).

**Example 67: preparation of 3-(5-((2-(2-(2-(((3as,6as)-hexahydro-2,5-methanopentalen-3a(1H)-yl)amino)ethoxy)ethoxy)ethyl)amino)-2-methyl-4-oxoquinazolin-3(4H)-yl)piperidine-2,6-dione (GT-02497)**

Following the method of Scheme 8 and the method of example 58, under appropriate conditions understood in the field, the target compound **GT-02497** was prepared (pale yellow solid, 10 mg, yield 8. 79%). LCMS (ESI) calcd for C29H40N5O5+ [M+H]+, 538.30; found, 538.8. $^1$H NMR (400 MHz, MeOD) δ 7. 75 - 7. 68 (m, 1H), 6. 89 (d, J = 8. 6 Hz, 1H), 6. 77 (d, J = 7. 6 Hz, 1H), 5. 39 (dd, J = 11. 3, 5. 7 Hz, 1H), 3. 82 - 3. 74 (m, 6H), 3.56 - 3. 46 (m, 4H), 3. 22 - 3. 18 (m, 2H), 2. 92 (s, 3H), 2. 85 - 2. 74 (m, 2H), 2. 38 - 2. 31 (m, 2H), 2. 08 - 1. 91 (m, 10H), 1. 67 - 1. 54 (m, 2H).

**Example 68: preparation of 3-(2-methyl-5-((5-(4-methyl-1,4-diazepan-1-yl)pentyl)amino)-4-oxoquinazolin-3(4H)-yl)piperidine-2,6-dione (GT-02500)**

Following the method of Scheme 8 and the method of example 58, under appropriate conditions understood in the field, the target compound **GT-02500** was prepared (pale yellow solid, 10 mg, yield 6. 58%). LC/MS (ESI) calcd for $C_{25}H_{37}N_6O_3^+$ $[M+H]^+$, 469.29; found, 469.3.

**Example 69: preparation of 3-(5-((3-(4-(bis(4-chlorophenyl)methyl)piperazin-1-yl)propyl)thio)-2-methyl-4-oxoquinazolin-3(4H)-yl)piperidine-2,6-dione (GT-03697)**

Following the method of Scheme 9 and the method of example 1, under appropriate conditions understood in the field, the target compound **GT-03697** was prepared (19 mg, yield: 36%, white solid). $^1$H NMR (400 MHz, DMSO) δ 11. 03 (s, 1H), 7. 70 (t, J = 8. 0 Hz, 1H), 7. 57-7. 48 (m, 4H), 7. 46-7. 39 (m, 6H), 7. 34 (d, J = 8. 0 Hz, 1H), 7. 29 (d, J = 8. 0 Hz, 1H), 5. 27-5. 22 (m, 1H), 3.54-3.38 (m, 3H), 3.30-3. 22 (m, 3H), 3. 18-3. 08 (m, 2H), 3. 06-2. 96 (m, 3H), 2. 91-2. 82 (m, 3H), 2. 63 (s, 3H), 2. 26-2. 13 (m, 2H), 2. 10-2. 06 (m, 3H). LC/MS (ESI) calcd for $C_{34}H_{36}Cl_2N_5O_3S^+$ $[M+H]^+$, 664.19; found, 664.2.

**Example 70: preparation of 3-(5-(3-(2-((3r,5r,7r)-adamantan-1-yl)ethyl)-3,6-diazabicyclo[3.1.1]heptan-6-yl)-2-methyl-4-oxoquinazolin-3(4H)-yl)piperidine-2,6-dione (GT-**04231)

The target compound **GT-04231** was prepared by referring to the method of Scheme 11.

Step 1: to a solution of 3-(5-fluoro-2-methyl-4-oxoquinazolin-3(4H)-yl)piperidine-2,6-dione (1.0 eq.) and tert-butyl 3,6-diazabicyclo[3.1.1]hept-3-carboxylate (1.5 eq.) in dimethyl sulfoxide was added triethylamine (3.0 eq.). The reaction solution was heated to 120 °C and stirred for 18 hours. After monitoring the reaction via TLC and confirming its completion, the reaction mixture was cooled to room temperature, washed with water, and extracted with dichloromethane. The organic phases were combined, dried over anhydrous sodium sulfate, filtered, and concentrated. The residue was purified by silica gel column chromatography to give tert-butyl 6-(3-(2,6-dioxopiperidin-3-yl)-2-methyl-4-oxo-3,4-dihydroquinazolin-5-yl)-3,6-diazabicyclo[3.1.1]hept-3-carboxylate.

Step 2: to a solution of the product obtained from the previous step in dichloromethane was added trifluoroacetic acid. The reaction solution was stirred at room temperature for 2 hours. After monitoring the reaction via LCMS and confirming its completion, the reaction solution was concentrated under reduced pressure to give 3-(5-(3,6-diazabicyclo [3.1.1]hept-6-yl)-2-methyl-4-oxoquinazolin-3(4H)-yl)piperidine-2,6-dione hydrochloride.

Step 3: to a solution of 3-(5-(3,6-diazabicyclo[3.1.1]hept-6-yl)-2-methyl-4-oxoquinazolin-3(4H)-yl)piperidine-2,6-dione hydrochloride (1.0 eq.) obtained from Step 2 and (3r,5r,7r)-1-(2-bromoethyl)adamantane (1.3 eq.) in anhydrous DMF (3 mL) were added triethylamine (3.0 eq.) and sodium iodide (1.0 eq.). The reaction solution was stirred at 50 °C for 18 hours. After monitoring the reaction via LCMS and confirming its completion, the reaction solution was filtered, and the filtrate was subjected to HPLC for purification to give the target compound GT-04231 (8 mg, yield: 17%, white solid). LC/MS (ESI) calcd for $C_{31}H_{40}N_5O_3^+$ [M+H]$^+$, 530.31; found, 530. 3.

**Example 71: preparation of 3-(5-(6-(2-((3r,5r,7r)-adamantan-1-yl)ethyl)-3,6-diazabicyclo[3.1.1]heptan-3-yl)-2-methyl-4-oxoquinazolin-3(4H)-yl)piperidine-2,6-dione (GT-04230)**

Following the method of Scheme 11 and the method of example 70, under appropriate conditions understood in the field, the target compound **GT-04230** was prepared (16 mg, yield: 26%, white solid). LC/MS (ESI) calcd for $C_{31}H_{40}N_5O_3^+$ [M+H]$^+$, 530.31; found, 530. 3.

**Example 72: preparation of 3-(5-(3-(2-((3r,5r,7r)-adamantan-1-yl)ethyl)-3,8-diazabicyclo[3.2.1]octan-8-yl)-2-methyl-4-oxoquinazolin-3(4H)-yl)piperidine-2,6-dione (GT-04253)**

Following the method of Scheme 11 and the method of example 70, under appropriate conditions understood in the field, the target compound **GT-04253** was prepared (17 mg, yield: 29%, white solid). $^1$H NMR (400 MHz, DMSO) δ 6. 98 (t, *J* = 8. 2 Hz, 1H), 6. 46 (d, *J* = 8. 2 Hz, 1H), 6. 40 (d, *J* = 8. 2 Hz, 1H), 4.56 - 4.52 (m, 1H), 3.50 (d, *J* = 6. 0 Hz, 1H), 3. 25 (brs, 1H), 2. 91 - 2. 77 (m, 2H), 2. 77 - 2. 65 (m, 2H), 2. 47 - 2. 43 (m, 2H), 2. 16 - 1. 91 (m, 5H), 1. 57 - 1. 53 (m, 1H), 1. 44 - 1. 38 (m, 1H), 1. 35 - 1. 12 (m, 6H), 0. 96 (dd, *J*= 31. 2, 12. 0 Hz, 6H), 0. 81 (s, 9H). LC/MS (ESI) calcd for $C_{32}H_{42}N_5O_3^+$ [M+H]$^+$, 544.33; found, 544.4.

**Example 73: preparation of 3-(5-(8-(2-((3r,5r,7r)-adamantan-1-yl)ethyl)-3,8-diazabicyclo[3.2.1]octan-3-yl)-2-methyl-4-oxoquinazolin-3(4H)-yl)piperidine-2,6-dione (GT-03727)**

Following the method of Scheme 11 and the method of example 70, under appropriate conditions understood in the field, the target compound GT-03727 was prepared (18 mg, yield: 31%, white solid). LC/MS (ESI) calcd for $C_{32}H_{42}N_5O_3^+$ [M+H]$^+$, 544.33; found, 544.4.

**Example 74: preparation of 3-(5-(5-(2-((3r,5r,7r)-adamantan-1-yl)ethyl)-2,5-diazabicyclo [2.2.2] octan-2-yl)-2-methyl-4-oxoquinazolin-3(4H)-yl)piperidine-2,6-dione (GT-04228)**

Following the method of Scheme 11 and the method of example 70, under appropriate conditions understood in the field, the target compound GT-04228 was prepared (20 mg, yield: 34%, white solid). LC/MS (ESI) calcd for $C_{32}H_{42}N_5O_3^+$ [M+H]$^+$, 544.33; found, 544.4.

**Example 75: preparation of 3-(5-((1S,4S)-5-(2-((3S,SS,7S)-adamantan-1-yl)ethyl)-2,5-diazabicyclo[2.2.1]heptan-2-yl)-2-methyl-4-oxoquinazolin-3(4H)-yl)piperidine-2,6-dione (GT-04232)**

Following the method of Scheme 11 and the method of example 70, under appropriate conditions understood in the field, the target compound GT-04232 was prepared (12 mg, yield: 20%, white solid). LC/MS (ESI) calcd for

$C_{31}H_{40}N_5O_3^+$ [M+H]+, 530.31; found, 530.3.

**Example 76: preparation of 3-(5-((1R,4R)-5-(2-((3R,SR,7R)-adamantan-1-yl)ethyl)-2,5-diazabicyclo[2.2.1]heptan-2-yl)-2-methyl-4-oxoquinazolin-3(4H)-yl)piperidine-2,6-dione (GT-04233)**

Following the method of Scheme 11, under appropriate conditions understood in the field, the target compound GT-04233 was prepared (14 mg, yield: 23%, white solid). LC/MS (ESI) calcd for $C_{31}H_{40}N_5O_3^+$ [M+H]+, 530.31; found, 530.3.

**Example 77: preparation of 3-(5-(4-(2-((3r,5r,7r)-adamantan-1-yl)ethyl)-3,5-dimethylpiperazin-1-yl)-2-methyl-4-oxoquinazolin-3(4H)-yl)piperidine-2,6-dione (GT-04246)**

Following the method of Scheme 11, under appropriate conditions understood in the field, the target compound GT-04246 was prepared (5 mg, yield: 10%, white solid). LC/MS (ESI) calcd for $C_{32}H_{44}N_5O_3^+$ [M+H]+, 546.34; found, 546.3.

**Example 78: preparation of 3-(5-(4-(2-((3r,5r,7r)-adamantan-1-yl)ethyl)-1,4-diazepan-1-yl)-2-methyl-4-oxoquinazolin-3(4H)-yl)piperidine-2,6-dione (GT-04229)**

Following the method of Scheme 11, under appropriate conditions understood in the field, the target compound GT-04229 was prepared (10 mg, yield: 15%, white solid). LC/MS (ESI) calcd for $C_{31}H_{42}N_5O_3^+$ [M+H]+, 532.33; found, 532.3.

**Example 79: preparation of 3-(5-((2-(4-(2-((3r,5r,7r)-adamantan-1-yl)ethyl)piperazin-1-yl)ethyl)thio)-2-methyl-4-oxoquinazolin-3(4H)-yl)piperidine-2,6-dione (GT-03305)**

Following the method of Scheme 9 and the method of example 1, under appropriate conditions understood in the field, the target compound GT-03305 was prepared (8 mg, yield: 20 %, white solid). $^1$H NMR (400 MHz, MeOD) $\delta$ 7. 70 (t, J = 8. 0 Hz, 1H), 7. 38-7. 32 (m, 2H), 5. 30-5. 22 (m, 1H), 3.52-3. 49 (m, 1H), 3. 46-3.39 (m, 3H), 3. 24-3. 16 (m, 5H), 2. 96-2. 86 (m, 3H), 2. 86-2. 80 (m, 1H), 2. 80-2. 76 (m, 1H), 2. 75-2. 69 (m, 1H), 2. 69 (s, 3H), 2. 28-2. 20 (m, 1H), 2. 05-1. 97 (m, 3H), 1. 85-1. 77 (m, 3H), 1. 76-1. 68 (m, 3H), 1. 65-1. 57 (m, 6H), 1. 54-1. 47 (m, 2H). LC/MS (ESI) calcd for $C_{32}H_{44}N_5O_3S^+$ [M+H]+, 578.31; found, 578.3.

**Example 80: preparation of 4-(4-(2-((3-(2,6-dioxopiperidin-3-yl)-2-methyl-4-oxo-3,4-dihydroquinazolin-5-yl)thio) ethyl)piperazin-1-yl)-3-fluorobenzonitrile (GT-03314)**

Following the method of Scheme 9 and the method of example 1, under appropriate conditions understood in the field, the target compound GT-03314 was prepared (12 mg, yield: 45 %, white solid). LC/MS (ESI) calcd for $C_{27}H_{28}FN_6O_3S^+$ [M+H]+, 535.19; found, 535.2.

**Example 81: preparation of 3-(5-((2-(4-(4-((1s,3s)-adamantan-1-yl)benzyl)piperazin-1-yl)ethyl)thio)-2-methyl-4-oxoquinazolin-3(4H)-yl)piperidine-2,6-dione (GT-03315)**

Following the method of Scheme 9 and the method of example 1, under appropriate conditions understood in the field, the target compound GT-03315 was prepared (8 mg, yield: 23 %, white solid). $^1$H NMR (400 MHz, MeOD) $\delta$ 7. 71 (t, *J* = 8. 0 Hz, 1H), 7. 42 - 7. 36 (m, 5H), 7. 30 (d, *J* = 8. 0 Hz, 1H), 5. 26 - 5. 21 (m, 1H), 4. 24 (brs, 2H), 3. 45-3.32 (m, 4H), 3. 13-3. 08 (m, 2H), 2. 87-2. 73 (m, 2H), 2. 72(s, 3H), 2. 76-2. 61 (m, 2H), 2. 28-2. 12 (m, 1H), 2. 00 (brs, 4H), 1. 85 (brs, 7H), 1. 77-1. 69 (m, 9H). LC/MS (ESI) calcd for $C_{37}H_{46}N_5O_3S^+$ [M+H]+, 640.33; found, 640.3.

**Example 82: preparation of 3-(5-((2-(4-((1s,3s)-adamantan-1-yl)piperazin-1-yl)ethyl)amino)-2-methyl-4-oxoquinazolin-3(4H)-yl)piperidine-2,6-dione (GT-03284)**

Following the method of Scheme 10 and the method of example 4, under appropriate conditions understood in the field, the target compound GT-03284 was prepared (8 mg, yield: 27 %, white solid). $^1$H NMR (400 MHz, MeOD) $\delta$ 7. 66 (t, *J* = 8. 4 Hz, 1H), 6. 88 (d, *J* = 8. 4 Hz, 1H), 6. 74 (d, *J* = 8. 4 Hz, 1H), 5. 31-5. 26 (m, 1H), 3. 78-3. 61 (m, 6H), 3. 49-3.36 (m, 2H), 2. 82 (s, 3H), 2. 79-2.59 (m, 3H), 2. 25-2. 20 (m, 4H), 1. 98 (brs, 7H), 1. 73-1. 64 (m, 7H), 1. 29-1. 27 (m, 1H), 1. 24-1. 20 (m, 1H). LC/MS (ESI) calcd for $C_{30}H_{41}N_6O_3^+$ [M+H]+, 533.32; found, 533.3.

**Example 82: preparation of 3-(5-((2-(4-(((1s,3s)-adamantan-1-yl)methyl)piperazin-1-yl)ethyl)amino)-2-methyl-4-oxoquinazolin-3(4H)-yl)piperidine-2,6-dione (GT-03285)**

Following the method of Scheme 10 and the method of example 4, under appropriate conditions understood in the field, the target compound GT-03285 was prepared (9 mg, yield: 32 %, white solid). $^1$H NMR (400 MHz, MeOD) $\delta$ 7. 79 (t, $J$ = 8. 2 Hz, 1H), 7. 01 (d, $J$ = 8. 2 Hz, 1H), 6. 86 (d, $J$ = 8. 2 Hz, 1H), 5. 43-5. 39 (m, 1H), 3. 82-3. 76 (m, 3H), 3. 69-3.56 (m, 6H), 3. 02-2. 98 (m, 2H), 2. 95 (s, 3H), 2. 91-2. 76 (m, 3H), 2. 38-2. 34 (m, 1H), 2. 07 (s, 3H), 1. 79 (s, 15H). LC/MS (ESI) calcd for $C_{31}H_{43}N_6O_3^+$ [M+H]$^+$, 547.37; found, 547.3.

**Example 82: preparation of 3-(5-((2-(4-(4-((1s,3s)-adamantan-1-yl)benzyl)piperazin-1-yl)ethyl)amino)-2-methyl-4-oxoquinazolin-3(4H)-yl)piperidine-2,6-dione (GT-03316)**

Following the method of Scheme 10 and the method of example 4, under appropriate conditions understood in the field, the target compound GT-03316 was prepared (5 mg, yield: 15 %, white solid). $^1$H NMR (400 MHz, MeOD) $\delta$ 7. 63 (t, $J$ = 8. 4 Hz, 1H), 7. 46-7. 34 (m, 4H), 6. 81 (d, $J$ = 8. 4 Hz, 1H), 6. 69 (d, $J$ = 8. 4 Hz, 1H), 5. 31-5. 27 (m, 1H), 4. 26-4. 23 (m, 2H), 3.53-3. 47 (m, 3H), 3. 43-3. 29 (m, 4H), 2. 73-2. 68 (m, 3H), 2. 31-2. 19 (m, 1H), 2. 00 (brs, 3H), 1. 85 (brs, 7H), 1. 77-1. 69 (m, 8H). LC/MS (ESI) calcd for $C_{37}H_{47}N_6O_3^+$ [M+H]$^+$, 623.37; found, 623.3.

**Example 83: preparation of 4-(4-(2-((3-(2,6-dioxopiperidin-3-yl)-2-methyl-4-oxo-3,4-dihydroquinazolin-5-yl)amino)ethyl)piperazin-1-yl)-3-fluorobenzonitrile (GT-03313)**

Following the method of Scheme 10 and the method of example 4, under appropriate conditions understood in the field, the target compound GT-03313 was prepared (14 mg, yield: 52 %, white solid). H NMR (400 MHz, MeOD) $\delta$ 7. 66 (t, $J$= 8. 4 Hz, 1H), 7. 44-7. 41 (m, 2H), 7. 11 (t, $J$= 8. 4 Hz, 1H), 6. 88 (d, $J$= 8. 4 Hz, 1H), 6. 78 (d, $J$= 8. 4 Hz, 1H), 5. 28-5. 24 (m, 1H), 3. 79 (t, $J$= 6. 4 Hz, 2H), 3. 68 (brs, 4H), 3. 43 (t, $J$= 6. 4 Hz, 3H), 3.38-3. 23 (m, 4H), 2. 88-2. 79 (m, 1H), 2. 77 (s, 3H), 2. 75-2. 62 (m, 2H), 2. 24-2. 20 (m, 1H). LC/MS (ESI) calcd for $C_{27}H_{29}FN_7O_3^+$ [M+H]$^+$, 518.23; found, 518. 3.

**Example 84: preparation of 3-(5-(2-(4-((3s,5s,7s)-adamantan-1-yl)piperazin-1-yl)ethoxy)-2-methyl-4-oxoquinazolin-3(4H)-yl)piperidine-2,6-dione (GT-03310)**

Following the method of Scheme 10 and the method of example 4, under appropriate conditions understood in the field, the target compound GT-03310 was prepared (6 mg, yield: 20 %, white solid). $^1$H NMR (400 MHz, MeOD) $\delta$ 7. 85 (t, $J$= 8. 0 Hz, 1H), 7. 36 (d, $J$= 8. 0 Hz, 1H), 7. 22 (d, $J$ = 8. 0 Hz, 1H), 5. 36-5. 29 (m, 1H), 4.53-4.50 (m, 2H), 4. 13-4. 04 (m, 2H), 3. 97-3. 87 (m, 2H), 3. 71-3. 62 (m, 2H), 3.52-3. 48 (m, 2H), 3. 17-3. 14 (m, 1H), 2. 76 (s, 3H), 2. 76-2. 71 (m, 1H), 2. 38-2. 29 (m, 5H), 2. 08 (s, 6H), 1. 82 (s, 9H). LC/MS (ESI) calcd for $C_{30}H_{39}N_5O_4^+$ [M+H]$^+$, 534.31; found, 534. 3.

**Example 85: preparation of 3-(5-(2-(4-(4-((1s,3s)-adamantan-1-yl)benzyl)piperazin-1-yl)ethoxy)-2-methyl-4-oxoquinazolin-3(4H)-yl)piperidine-2,6-dione (GT-03311)**

Following the method of Scheme 10 and the method of example 4, under appropriate conditions understood in the field, the target compound GT-03311 was prepared (8 mg, yield: 23 %, white solid). LC/MS (ESI) calcd for $C_{37}H_{46}N_5O_4^+$ [M+H]$^+$, 624.35; found, 624.4.

**Example 86: preparation of 4-(4-(2-((3-(2,6-dioxopiperidin-3-yl)-2-methyl-4-oxo-3,4-dihydroquinazolin-5-yl)oxy)ethyl)piperazin-1-yl)-3-fluorobenzonitrile (GT-03312)**

Following the method of Scheme 10 and the method of example 4, under appropriate conditions understood in the field, the target compound GT-03312 was prepared (12 mg, yield: 47 %, white solid). $^1$H NMR (400 MHz, MeOD) $\delta$ 7. 87 (t, $J$= 8. 0 Hz, 1H), 7. 58-7. 54 (m, 2H), 7. 40 (d, $J$ = 8. 0 Hz, 1H), 7. 27-7. 21 (m, 2H), 5. 37-5. 34 (m, 1H), 4. 64-4. 61 (m, 2H), 4. 01-3. 97 (m, 1H), 3. 84-3. 77 (m, 6H), 3. 62-3. 48 (m, 3H), 2. 97-2. 92 (m, 2H), 2. 78 (s, 3H), 2. 75-2. 70 (m, 1H), 2. 33-2. 27 (m, 1H). LC/MS (ESI) calcd for $C_{27}H_{28}FN_6O_4^+$ [M+H]$^+$, 519.22; found, 519.3.

**Example 87: preparation of 3-(5-((4-(2-((3r,5r,7r)-adamantan-1-yl)ethyl)piperazin-1-yl)methyl)-2-methyl-4-oxoquinazolin-3(4H)-yl)piperidine-2,6-dione (GT-03261)**

Following the method of Scheme 12, under appropriate conditions understood in the field, the target compound GT-03261 was prepared (6 mg, yield: 24 %, white solid). LC/MS (ESI) calcd for $C_{31}H_{42}N_5O_3^+$ [M+H]$^+$, 532.33; found, 532.3.

**Example 88: preparation of 3-(5-((4-((1s,3s)-adamantane-1-carbonyl)piperazin-1-yl)methyl)-2-methyl-4-oxoquinazolin-3(4H)-yl)piperidine-2,6-dione (GT-03317)**

Following the method of Scheme 12, under appropriate conditions understood in the field, the target compound GT-03317 was prepared (7 mg, yield: 20 %, white solid). LC/MS (ESI) calcd for $C_{30}H_{38}N_5O_4^+$ $[M+H]^+$, 532.29; found, 532.3.

**Example 89: preparation of 3-(5-(3-(4-(((3r,5r,7r)-adamantan-1-yl)methyl)piperazin-1-yl)prop-1-yn-1-yl)-2-methyl-4-oxoquinazolin-3(4H)-yl)piperidine-2,6-dione (GT-03302)**

Following the method of steps 1-3 of Scheme 5 and the method of step 3 of Scheme 10, under appropriate conditions understood in the field, the target compound GT-03302 was prepared (8 mg, yield: 26 %, white solid). $^1H$ NMR (400 MHz, MeOD) δ 7. 79 (t, *J* = 8. 0 Hz, 1H), 7. 67 (d, *J* = 8. 0 Hz, 1H), 7. 63 (d, *J* = 8. 0 Hz, 1H), 5. 30-5. 25 (m, 1H), 4. 25 (s, 2H), 3. 79 (brs, 4H), 3. 64-3. 42 (m, 4H), 2. 98-2. 90 (m, 2H), 2. 86-2. 81 (m, 1H), 2. 74 (s, 3H), 2. 68-2. 65 (m, 1H), 2. 64-2. 61 (m, 1H), 2. 28-2. 14 (m, 1H), 1. 97 (s, 3H), 1. 70 (s, 12H). LC/MS (ESI) calcd for $C_{32}H_{40}N_5O_3^+$ $[M+H]^+$, 542.31; found, 542.3.

**Example 90: preparation of 3-(5-(3-(4-(2-((1s,3s)-adamantan-1-yl)ethyl)piperazin-1-yl)prop-1-yn-1-yl)-2-methyl-4-oxoquinazolin-3(4H)-yl)piperidine-2,6-dione (GT-03262)**

Following the method of example 89, under appropriate conditions understood in the field, the target compound GT-03262 was prepared (12 mg, yield: 19 %, white solid). $^1H$ NMR (400 MHz, MeOD) δ 7. 88 (t, *J* = 8. 0 Hz, 1H), 7. 73 (t, *J* = 8. 0 Hz, 2H), 5. 40-5. 34 (m, 1H), 4. 18 (s, 2H), 3. 99-3. 72 (m, 4H), 3. 49-3. 40 (m, 3H), 2. 99-2. 94 (m, 2H), 2. 83(s, 3H), 2. 82-2. 77 (m, 2H), 2. 40-2. 28 (m, 1H), 2. 01 (brs, 4H), 1. 82-1. 72 (m, 6H), 1. 65 (s, 9H). LC/MS (ESI) calcd for $C_{33}H_{42}N_5O_3^+$ $[M+H]^+$, 556.33; found, 556.3.

**Example 91: preparation of 3-(5-(3-(4-((3r,5r,7r)-adamantane-1-carbonyl)piperazin-1-yl)prop-1-yn-1-yl)-2-methyl-4-oxoquinazolin-3(4H)-yl)piperidine-2,6-dione (GT-03318)**

Following the method of example 89, under appropriate conditions understood in the field, the target compound GT-03318 was prepared (8 mg, yield: 26 %, white solid). $^1H$ NMR (400 MHz, MeOD) δ 7. 75 (t, *J*= 8. 0 Hz, 1H), 7. 63-7. 60 (m, 2H), 5. 25-5. 16 (m, 1H), 4. 39-4. 26 (m, 2H), 4. 02-3. 82 (m, 1H), 3. 81-3.58 (m, 1H), 2. 86-2. 75 (m, 1H), 2. 66 (s, 3H), 2. 66-2. 62(m, 1H), 2. 26-2. 18 (m, 1H), 1. 99-1. 91 (m, 7H), 1. 70 (s, 6H), 1. 20 (s, 9H). LC/MS (ESI) calcd for $C_{32}H_{38}N_5O_4^+$ $[M+H]^+$, 556.29; found, 556.3.

**Example 92: preparation of 3-(5-((2-(4-benzhydrylpiperazin-1-yl)ethyl)amino)-2-methyl-4-oxoquinazolin-3(4H)-yl)piperidine-2,6-dione (GT-03385)**

Following the method of Scheme 10 and the method of Example 4, under appropriate conditions understood in the field, the target compound GT-03385 was prepared (12 mg, yield: 25 %, white solid). LC/MS (ESI) calcd for $C_{34}H_{38}N_5O_3^+$ $[M+H]^+$, 564.30; found, 564.3.

**Example 93: preparation of 3-(5-((7-(((1s,3s)-adamantan-1-yl)amino)heptyl)thio)-2-methyl-4-oxoquinazolin-3(4H)-yl)piperidine-2,6-dione (GT-02802)**

Following the method of Scheme 9, under appropriate conditions understood in the field, the target compound GT-02802 was prepared (1. 6 mg, yield: 20 %, white solid). LC/MS (ESI) calcd for $C_{31}H_{43}N_4O_3S^+$ $[M+H]^+$, 551.31; found, 551.3.

**Example 94: preparation of 3-(2-methyl-4-oxo-5-(piperazin-1-ylmethyl)quinazolin-3(4H)-yl)piperidine-2,6-dione (GT-03228)**

Following the method of steps 1-2 of Scheme 12, under appropriate conditions understood in the field, the target compound GT-03228 was prepared (540 mg, yield: 91 %, white solid). LC/MS (ESI) calcd for $C_{19}H_{24}N_5O_3^+$ $[M+H]^+$, 370.19; found, 370.2.

**Example 95: preparation of 3-(5-((4-(3-(((1s,3s)-adamantan-1-yl)amino)propyl)piperazin-1-yl)methyl)-2-methyl-4-oxoquinazolin-3(4H)-yl)piperidine-2,6-dione (GT-03226)**

Following the method of Scheme 12, under appropriate conditions understood in the field, the target compound GT-03226 was prepared (8 mg, yield: 24 %, white solid). LC/MS (ESI) calcd for $C_{32}H_{45}N_6O_3^+$ [M+H]$^+$, 561.35; found, 561.4.

**Example 96: preparation of 3-(5-((4-(((3r,5r,7r)-adamantan-1-yl)methyl)piperazin-1-yl)methyl)-2-methyl-4-oxoquinazolin-3(4H)-yl)piperidine-2,6-dione (GT-03227)**

Following the method of Scheme 12, under appropriate conditions understood in the field, the target compound GT-03227 was prepared (1 mg, yield: 3 %, white solid). LC/MS (ESI) calcd for $C_{30}H_{40}N_5O_3^+$ [M+H]$^+$, 518.31; found, 518.3.

**Example 97: preparation of 3-(5-((4-(4-((3r,5r,7r)-adamantan-1-yl)benzyl)piperazin-1-yl)methyl)-2-methyl-4-oxoquinazolin-3(4H)-yl)piperidine-2,6-dione (GT-03230)**

Following the method of Scheme 12, under appropriate conditions understood in the field, the target compound GT-03230 was prepared (4 mg, yield: 15 %, white solid). $^1$H NMR (400 MHz, CD3OD) δ 7. 96-7. 90 (m, 1H), 7. 83 (d, *J* = 8. 0 Hz, 1H), 7. 70 (d, *J* = 8. 0 Hz, 1H), 7. 52-7. 49 (m, 5H), 5. 45-5. 33 (m, 1H), 4.46-4. 31 (m, 2H), 3. 80-3.56 (m, 7H), 2. 97-2. 87 (m, 2H), 2. 83(s, 3H), 2. 31-2. 27 (m, 1H), 2. 09 (brs, 4H), 1. 96-1. 91 (m, 6H), 1. 86-1. 77 (m, 9H). LC/MS (ESI) calcd for $C_{36}H_{44}N_5O_3^+$ [M+H]$^+$, 594.34; found, 594.3.

**Example 98: preparation of 3-(5-((4-(7-(((1s,3s)-adamantan-1-yl)amino)heptyl)piperazin-1-yl)methyl)-2-methyl-4-oxoquinazolin-3(4H)-yl)piperidine-2,6-dione (GT-03258)**

Following the method of Scheme 12, under appropriate conditions understood in the field, the target compound GT-03258 was prepared (4 mg, yield: 22 %, white solid). LC/MS (ESI) calcd for $C_{35}H_{51}N_6O_3^+$ [M+H]$^+$, 603.4; found, 603.4.

**Example 99: preparation of 3-(5-((2-(4-((1s,3s)-adamantan-1-yl)piperazin-1-yl)ethyl)thio)-2-methyl-4-oxoquinazolin-3(4H)-yl)piperidine-2,6-dione (GT-03231)**

Following the method of Scheme 9 and the method of Example 1, under appropriate conditions understood in the field, the target compound GT-03231 was prepared (6 mg, yield: 21 %, white solid). $^1$H NMR (400 MHz, MeOD) δ 7. 87 (t, J = 8. 0 Hz, 1H), 7. 61 (d, J = 8. 0 Hz, 1H), 7. 46 (d, J = 8. 0 Hz, 1H), 5. 39-5. 35 (m, 1H), 3. 91-3. 83 (m, 4H), 3.56-3. 42 (m, 6H), 2. 86(s, 3H), 2. 99-2. 75 (m, 2H), 2. 31 (brs, 4H), 2. 10 (brs, 7H), 1. 85-1. 76 (m, 8H). LC/MS (ESI) calcd for $C_{30}H_{40}N_5O_3S^+$ [M+H]$^+$, 550.28; found, 550.3.

**Example 100: preparation of 3-(5-((3-(4-((3s,5s,7s)-adamantan-1-yl)piperazin-1-yl)propyl)thio)-2-methyl-4-oxoquinazolin-3(4H)-yl)piperidine-2,6-dione (GT-03232)**

Following the method of Scheme 9 and the method of Example 1, under appropriate conditions understood in the field, the target compound GT-03232 was prepared (8 mg, yield: 28 %, white solid). 1 H NMR (400 MHz, CD3OD_SPE) δ 7. 83 (t, *J*=8. 0 Hz, 1H), 7. 49 (d, *J*=8. 0 Hz, 1H), 7. 43 (d, *J*=8. 0 Hz, 1H), 5. 35-5. 30 (m, 1H), 4. 00-3. 85 (m, 4H), 3. 61-3. 43 (m, 6H), 3. 20-3. 11 (m, 2H), 2. 93-2. 89 (m, 1H), 2. 81(s, 3H), 2. 85-2. 67 (m, 2H), 2. 30-2. 26 (m, 3H), 2. 07 (s, 7H), 1. 83-1. 74 (m, 8H). LC/MS (ESI) calcd for $C_{31}H_{42}N_5O_3S^+$ [M+H]$^+$, 564.30; found, 564.3.

**Example 101: preparation of 3-(5-((3-(4-(((1s,3s)-adamantan-1-yl)methyl)piperazin-1-yl)propyl)thio)-2-methyl-4-oxoquinazolin-3(4H)-yl)piperidine-2,6-dione (GT-03235)**

Following the method of Scheme 9 and the method of Example 1, under appropriate conditions understood in the field, the target compound GT-03235 was prepared (8 mg, yield: 28 %, white solid). $^1$H NMR (400 MHz, MeOD) δ 7. 90 (t, *J* = 8. 0 Hz, 1H), 7. 58 (d, *J* = 8. 0 Hz, 1H), 7. 48 (d, *J* = 8. 0 Hz, 1H), 5. 42-5. 38 (m, 1H), 3. 89-3. 73 (m, 6H), 3.54-3. 45 (m, 2H), 3. 29-3. 14 (m, 3H), 3. 12-3. 02 (m, 2H), 2. 96-2. 94 (m, 1H), 2. 90 (s, 3H), 2. 82-2. 76 (m, 2H), 2. 66-2. 65(m, 2H), 2. 38-2. 24 (m, 3H), 2. 09 (s, 3H), 1. 80 (s, 12H), 1. 42-1. 39(m, 2H). LC/MS (ESI) calcd for $C_{32}H_{44}N_5O_3S^+$ [M+H]$^+$, 578.32; found, 578.3.

**Example 102: preparation of 3-(5-((2-(4-(((3r,5r,7r)-adamantan-1-yl)methyl)piperazin-1-yl)ethyl)thio)-2-methyl-4-oxoquinazolin-3(4H)-yl)piperidine-2,6-dione (GT-03259)**

Following the method of Scheme 9 and the method of Example 1, under appropriate conditions understood in the field, the target compound GT-03259 was prepared (8 mg, yield: 27 %, white solid). $^1$H NMR (400 MHz, MeOD) δ 7. 79 (t, *J*= 8. 0 Hz, 1H), 7. 54 (d, *J* = 8. 0 Hz, 1H), 7. 36 (d, *J*= 8. 0 Hz, 1H), 5. 31-5. 26 (m, 1H), 4. 05-3. 95 (m, 1H), 3. 67-3.55 (m, 5H), 3.38 (s, 3H), 2. 97-2. 83 (m, 4H), 2. 85-2. 69 (m, 7H), 2. 22-2. 17 (m, 1H), 1. 95 (s, 6H), 1. 67 (s, 9H). LC/MS (ESI) calcd for $C_{31}H_{42}N_5O_3S^+$ [M+H]$^+$, 564.30; found, 564.3.

**Example 103: preparation of 3-(2-methyl-4-oxo-5-(3-(piperazin-1-yl)prop-1-yn-1-yl)quinazolin-3(4H)-yl)piperidine-2,6-dione (GT-03229)**

Following the method of Scheme 5 and the method of Example 33, under appropriate conditions understood in the field, the target compound GT-03229 was prepared (320 mg, yield: 112 %, white solid). LC/MS (ESI) calcd for $C_{21}H_{24}N_5O_3^+$ [M+H]$^+$, 394.19; found, 394.2.

**Example 104: preparation of 3-(5-(3-(4-(4-((3r,5r,7r)-adamantan-1-yl)benzyl)piperazin-1-yl)prop-1-yn-1-yl)-2-methyl-4-oxoquinazolin-3(4H)-yl)piperidine-2,6-dione (GT-03233)**

Following the method of Scheme 5 and the method of Example 33, under appropriate conditions understood in the field, the target compound GT-03233 was prepared (20 mg, yield: 42 %, white solid). $^1$H NMR (400 MHz, MeOD) δ 7. 81 (t, J = 8. 0 Hz, 1H), 7. 70 (d, J = 8. 0 Hz, 1H), 7. 64 (d, J = 8. 0 Hz, 1H), 7. 49-7. 40 (m, 4H), 5. 31-5. 27 (m, 1H), 4. 33 (q, J = 13. 2 Hz, 2H), 4. 23 (s, 2H), 3. 64-3. 41 (m, 6H), 2. 92-2. 64 (m, 6H), 2. 26-2. 18 (m, 1H), 2. 01 (s, 3H), 1. 86 (s, 7H), 1. 77-1. 69 (m, 8H). LC/MS (ESI) calcd for $C_{38}H_{44}N_5O_3^+$ [M+H]$^+$, 618.34; found, 618.3.

**Biological activity assay**

**Materials:**

| Reagents and materials | Suppliers or Source |
|---|---|
| Human Burkitt's lymphoma cell line: Daudi | Dalian Meilun Biotech Co., Ltd. |
| Human acute lymphoblastic leukemia cell line: Kasumi-1 | ATCC (American Type Culture Collection) |
| Human colon cancer cell: SW620 | ATCC |
| Human multiple myeloma cells: MM.1S | ATCC |
| Human B-lymphoma cell: Ramos | Dalian Meilun Biotech Co., Ltd. |
| Human dexamethasone-resistant multiple myeloma cell line: MM.1R | ATCC |
| Human diffuse large B-cell lymphoma cell line: TMD8 | Kyinno Biotechnology Co., Ltd |
| RPMI-1640 Medium | Dalian Meilun Biotech Co., Ltd. |
| L-15 Medium | ATCC |
| Meilun Cell Counting Kit-8 | Dalian Meilun Biotech Co., Ltd. |
| Fetal bovine serum (FBS) | Sunrise Science Products company |
| Penicillin-Streptomycin | Beijing TransGen Biotech Co., Ltd. |
| Mycoplasma detection kit | Beijing TransGen Biotech Co., Ltd. |
| STR genotype detection | Shanghai Biowing Applied Biotechnology Co. Ltd. |

Antibody:

Most of the antibodies used in the experiment were purchased from Cell Signaling Technology, wherein IKZF1 (#9034S), IKZF3 (#15103S), and CK1α (#ab108296) were included, and the GAPDH antibody was purchased from Abcam.

**Cell cultures**

The tumor cells used herein were cultured in a cell culture medium listed in table 2 at 37°C in an incubator with 5% $CO_2$. Prior to experimentation, all cells used were correctly identified by STR profiling, and tested negative for mycoplasma through routine screenings.

Table 2. Tumor cells and cell culture medium used in the present disclosure

| Cell Line | Cell culture medium used |
|---|---|
| Daudi | RPMI 1640 culture medium supplemented with 10% FBS, as well as penicillin at a final concentration of 100 U/mL and streptomycin at a final concentration of 100 μg/mL |
| Kasumi-1 | |
| MM. 1S | |
| Ramos | |
| MM. 1R | |
| TMD8 | |
| SW620 | L-15 culture medium supplemented with 10% FBS, as well as penicillin at a final concentration of 100 U/mL and streptomycin at a final concentration of 100 μg/mL |

**Determination of half inhibitory concentration ($IC_{50}$) of the compounds against tumor cells:**

$IC_{50}$ values of the compounds of the present disclosure (including the compounds in Table 1, and compounds of examples 1-104) were measured using the Meilun Cell Counting Kit-8 kit from Dalian Meilun Biotech Co., Ltd. Assay details are as follows: tumor cells were seeded in a 96-well plate at a density listed in Table 3. After 24h, 100 μL of the inoculated cells were treated with 0.5μL of the compounds of the present disclosure (including the compounds in Table 1, and compounds of examples 1-104) at 10 different successively decreasing concentrations (starting at the highest concentration of 10μM; 5-fold serial dilutions). After the cells were treated with the compounds of the present disclosure for a period of time, cell viability was measured according to the instructions provided with the CCK-8 kit. DMSO served as the negative control, and corresponding commercial immunomodulatory drug Avadomide (CC-122) was used as the positive control, both of which treated the cells using the same protocol as that of the compounds of the present disclosure. The growth inhibition of the compounds of the present disclosure on cells was plotted by Prism Graphpad software, and the $IC_{50}$ values of the compounds of the present disclosure were calculated therefrom. Results were shown in Table 4 below.

Table 3. Seeding protocol and treatment time for tumor cells

| Cells | Cells seeding protocol | The times for treating cells with the compounds of the present disclosure (hours) |
|---|---|---|
| Kasumi-1 | cells were seeded in 100 μL RPMI1640 medium containing serum at a density of 10,000 cells/well | 72h |
| MM. 1S | | |
| MM. 1R | | |
| TMD8 | cells were seeded in 100 μL RPMI1640 medium containing serum at a density of 10,000 cells/well | 96h |
| Ramos | | |
| Daudi | cells were seeded in 100 μL RPMI1640 medium containing serum at a density of 4000 cells/well | 96h |
| SW620 | cells were seeded in 100 μL L-15 medium containing serum at a density of 4000 cells/well | 96h |

**Western Blotting assay**

Tumor cells were plated in a 24-well plate at a cell seeding density of $3\times10^5$ cells/mL, with 1mL culture media per well. After 24h, the cells were treated with different concentrations of the compounds of the present disclosure. After 16 hours, the cells were collected, and washed with PBS. The supernatant was discarded, and the cells were placed on ice, and treated with RIPA protein lysate containing Halt protease and phosphatase inhibitor. The lysate was centrifuged at

10000RPM at 4°C for 10 minutes, and the supernatant was collected. An equal amount of proteins were loaded in 4X SDS sample solution, denatured at 95°C for 5 minutes, and then freezed to -20°C or directly subjected to protein electrophoresis (4-15% gradient gel, Bio-rad). Electrophoresis apparatus and related components were purchased from Bio-rad company, and electrophoresis set at a constant pressure of 120V for 1h. Then transferring membrane was conducted by using PVDF (polyvinylidene fluoride) at 400mA for 1h on ice. Afterwards, the membranes were block for 30 minutes by using the TarKara Blocking Buffer at room temperature. Western blotting was conducted according to the antibody product manual of Cell Signaling Technology Company.

DCso value (the drug concentration required for degrading proteins by 50%, abbreviated as $DC_{50}$) reads method: comparing the gray values of the Western blotting bands for the drug treatment with the gray values of the Western blotting band for the DMSO control, and reading the drug concentration range corresponding to the gray value of the Western blotting bands for the drug treatment which is equal to half of the gray value of the Western blotting band for the DMSO control.

$DC_{50}$ value could also be calculated as follows: using software ImageJ to quantify the gray values of the Western blotting bands for the drug treatment, fitting the relationship curve between drug concentrations and gray values, and from the fitted curve, calculating the drug concentration corresponding to half of the gray value of the Western blotting band for the DMSO control.

**Evaluation of TNF-α Inhibition Effect**

PBMC cells were cultured at 37°C in 5% $CO_2$ atmosphere, and then seeded in 96-well plates at $1\times10^7$ cells/well. Compounds (including compounds in Table 1 and Examples 1-104 compounds) were dissolved in DMSO and diluted to corresponding concentrations so that the final concentration of DMSO added to the cell culture did not exceed 0.5%. Cells were incubated in medium with or without compounds for 1 h, then stimulated with lipopolysaccharide (LPS; 1 ng/ml), and continually cultured for 18-20 h. Then, the supernatant was collected, diluted with serum-free medium, and tested for TNF-α level by ELISA kit. $IC_{50}$ was then calculated by Graphpad Prism 7.0.

**Determination of CRBN-binding affinity of compounds**

The CEREBLON BINDING kits (specification: 10,000 tests; product number: Catalog # 64BDCRBNPEH; CISBIO company) was used to determine the CRBN binding ability of the compounds to be tested through a HTRF method (homogeneous time-resolved fluorescence). The specific methods are as follows:

**1.** According to the instructions of the CEREBLON BINDING kits, the compounds to be tested of the present invention and lenalidomide were serially diluted using diluent #9 (1X) solution to obtain a final concentration of 2 μM for both the tested compounds and lenalidomide solution.
**2.** 2.5 μL of the above 2 μM of the tested compounds and lenalidomide solution, as well as the same volume of diluent #9 (1X) solution (solvent control group, Std0) were taken and added to each well of a 96-well plate, respectively. Then, 2.5 μL of (human Cereblon WT GST-tagged protein) solution was added to each well. Finally, 5 μL of the uniformly mixed thalidomide-Red reagent and GST Eu antibody working solution were added to each of the above wells. The final concentration of the tested compounds and lenalidomide in each well is 0.5 μM.
**3.** The blank control wells were sequentially added with 2.5 μL of diluent #9 (1X) solution, 2.5 μL of PROTAC binding buffer, and 5 μL of uniformly mixed thalidomide-Red reagent and GST Eu antibody working solution.
**4.** After sealing and incubating the solutions in the above wells at room temperature for 3 hours, the absorbance values at emission wavelengths of 620 nm and 665 nm were detected by the HTRF method using a Spark microplate reader (V3.1 SP1).

The corresponding CRBN binding inhibition rate was calculated using the following method:

$$R_{compound} = OD\ 665\ nm_{compound} / OD\ 620\ nm_{compound} - OD\ 665\ nm_{control} / OD\ 620\ nm_{control}$$

$$R_{Std0} = OD\ 665\ nm_{Std0} / OD\ 620\ nm_{Std0} - OD\ 665\ nm_{control} / OD\ 620\ nm_{control}$$

$$\text{inhibition rate } (\%) = (1 - R_{compound} / R_{Std0}) * 100$$

Note: OD 665 nm $_{compound}$ is the absorbance value of each well of the compounds to be tested of the present disclosure at an emission wavelength of 665 nm.

OD 620 nm $_{compound}$ is the absorbance value of each well of the compounds to be tested of the present disclosure at an emission wavelength of 620 nm.
OD 665 nm $_{control}$ is the absorbance value of the blank control well at an emission wavelength of 665 nm.
OD 620 nm $_{control}$ is the absorbance value of the blank control well at an emission wavelength of 620 nm.
OD 665 nm $_{Std0}$ is the absorbance value of the solvent control well at an emission wavelength of 665 nm.
OD 620 nm $_{Std0}$ is the absorbance value of the solvent control well at an emission wavelength of 620 nm.

**Experimental results**

Based on immunomodulatory drugs, this study developed the compounds of the present invention. By selecting the compounds designed and developed based on immunomodulatory drugs including avadomide (CC-122) (including the compounds in Table 1 and compounds in Examples 1-104) for cellular inhibition studies, it was found that many compounds of the present invention exhibited better inhibitory activity against multiple myeloma cells MM.1S, MM. 1R, and human diffuse large B-cell lymphoma cells TMD8, etc. The compounds of the present invention not only exhibit activity in inhibiting the proliferation of tumor cells but also have the ability to promote the degradation of IKZF proteins and inhibit the production of TNF-α, thus making them potential therapeutic agents for patients with immune-related tumors. The specific experimental data are shown below.

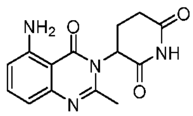

Structure of CC-122

Proliferation inhibition of the compounds of the present invention based on Avadomide (CC-122) in tumor cells

We tested the activities of the compounds of the present invention (including the compounds in Table 1 and Examples 1-104 compounds) in tumor cells through a dose-dependent manner. The cells were treated with the compounds of the present invention at 10 different successively decreasing concentrations (starting at the highest concentration of 10μM; 5-fold serial dilutions) for 72h or 96h, and then the cell viability determination was performed in accordance with CCK-8 reagent operating instructions. Results were shown in Table 4. It showed that the compounds of the present invention inhibited tumor cells to different degrees, and some compounds of the present invention exhibited significantly stronger inhibitory effects as compared with CC-122.

Table 4. $IC_{50}$ values (half inhibitory concentration) of the compounds of the present invention in tumor cells ($IC_{50}$, nM)

| Compound No. | MM. 1S/ $IC_{50}$(nM) | MM. 1R/ $IC_{50}$(nM) | TMD8/ $IC_{50}$(nM) | SW620/ $IC_{50}$(nM) | Ramos/ $IC_{50}$(nM) | Daudi/ $IC_{50}$(nM) | Kasumi-1/ $IC_{50}$(nM) |
|---|---|---|---|---|---|---|---|
| CC-122 | 46.9 | 464.4 | 890.3 | >10000 | 1326.0 | 1653.2 | >10000 |
| GT-03306 | 29.3 | 12. 7 | 86.5 | 18.6 | 89.3 | 44.9 | 22.3 |
| GT-03387 | - | 275.8 | 204.0 | - | 489.7 | 285.6 | - |
| GT-03301 | 79.6 | 32.8 | 185.8 | 35.8 | 164.7 | 155.4 | 104.1 |
| GT-03388 | - | 352.3 | - | - | - | - | - |
| GT-01818 | - | - | 553.6 | - | - | - | - |
| GT-02235 | 39.5 | - | 145.9 | - | - | - | - |
| GT-02237 | 3.3 | - | 8.2 | - | - | - | - |
| GT-02494 | - | - | 184.5 | - | - | - | - |
| GT-02495 | - | 9.8 | 21.4 | - | - | - | - |
| GT-02598 | 17.2 | - | - | - | - | - | - |
| GT-02767 | 69.9 | - | 309.4 | - | - | - | - |
| GT-02768 | 98.1 | - | 293.7 | - | - | - | - |
| GT-02772 | - | - | 448.2 | - | - | - | - |

(continued)

| Compound No. | MM. 1S/ $IC_{50}$(nM) | MM. 1R/ $IC_{50}$(nM) | TMD8/ $IC_{50}$(nM) | SW620/ $IC_{50}$(nM) | Ramos/ $IC_{50}$(nM) | Daudi/ $IC_{50}$(nM) | Kasumi-1/ $IC_{50}$(nM) |
|---|---|---|---|---|---|---|---|
| GT-02773 | - | - | 440.4 | - | - | - | - |
| GT-02774 | - | - | 102.3 | - | - | - | - |
| GT-02797 | - | - | 311.5 | - | - | - | - |
| GT-02323 | - | - | 651.7 | - | - | - | - |
| GT-02279 | - | - | 437.8 | - | - | - | - |
| GT-02280 | - | - | 409.1 | - | - | - | - |
| GT-02393 | - | - | 457.8 | - | - | - | - |
| GT-02496 | - | - | 474.4 | - | - | - | - |
| GT-03697 | - | 204.3 | - | - | 652.5 | 545.5 | 693.6 |
| GT-04246 | - | - | 412.7 | 386.8 | - | - | - |
| GT-03305 | - | - | - | 717.0 | - | - | - |
| GT-03314 | - | - | - | 708.8 | - | - | - |
| GT-03235 | - | - | - | - | 398.0 | - | - |
| symbol "-" indicates undetected. | | | | | | | |

The results of the binding affinity assay for the compounds of the present invention to CRBN were listed in Table 5 below.

Table 5. HTRF inhibitory activity ($IC_{50}$, $\mu M$) of the compounds of the present invention (including the compounds in Table 1 and Examples 1-104 compounds)

| Compound No. | HTRF $IC_{50}$ ($\mu M$) | Compound No. | HTRF $IC_{50}$ ($\mu M$) |
|---|---|---|---|
| CC-122 | 1.07 | GT-02645 | 2.78 |
| GT-02237 | 0.95 | GT-03306 | 1.57 |
| GT-02326 | 0.70 | GT-02759 | 0.05 |
| GT-02494 | 0.46 | GT-02797 | 0.18 |
| GT-02495 | 0.32 | GT-02799 | 2.47 |
| GT-02502 | 1.80 | GT-02800 | 3.40 |
| GT-02598 | 3.66 | GT-02234 | 3.34 |
| GT-02601 | 2.82 | GT-02389 | 0.78 |
| GT-02621 | 1.04 | GT-03301 | 1.72 |
| GT-02622 | 2.48 | GT-02496 | 0.42 |
| GT-02623 | 2.52 | GT-02392 | 0.99 |
| GT-02767 | 1.05 | GT-02393 | 0.42 |
| GT-02768 | 0.68 | GT-02774 | 2.61 |
| GT-02773 | 2.80 | GT-02497 | 0.66 |
| GT-04231 | 3.24 | GT-03305 | 1.20 |
| GT-03315 | 1.98 | GT-03261 | 1.00 |
| GT-03317 | 3.58 | GT-03262 | 1.00 |
| GT-03227 | 2.24 | GT-03228 | 1.84 |
| GT-03259 | 2.15 | GT-03235 | 2.40 |

(continued)

| Compound No. | HTRF $IC_{50}$ ($\mu$M) | Compound No. | HTRF $IC_{50}$ ($\mu$M) |
|---|---|---|---|
| GT-03230 | 2.75 | GT-03233 | 2.24 |

The experimental results indicated that the compounds of the present invention exhibit lower or comparable $IC_{50}$ values compared to CC-122, demonstrating stronger or equivalent binding affinity to CRBN. The differences in binding affinity may lead to the recruitment of distinct substrate proteins upon binding to CRBN and induce their degradation, resulting in different target selectivity and potentially treating different diseases.

The basic principles, main features and advantages of the present disclosure are shown and described above. Those skilled in the art should understand that the present disclosure is not limited by the foregoing embodiments, and they can make various changes, substitutions and alterations herein without departing from the spirit and scope of the present disclosure. These changes, substitutions and alterations fall within the scope of the present disclosure. The claimed scope of the present disclosure is defined by the appended claims and their equivalents.

## Claims

1. A compound of Formula (I)

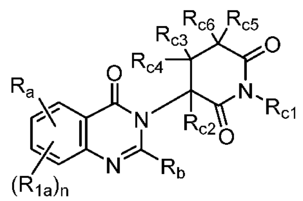

Formula (I)

or a salt, enantiomer, diastereoisomer, isotopically enriched analog, solvate, prodrug, or polymorph thereof,

wherein $R_{c1}$ represents H or $C_{1-3}$ alkyl, $R_{c2}$, $R_{c3}$, $R_{c4}$, $R_{c5}$ and $R_{c6}$ are the same or different and each independently represent H, D or $C_{1-3}$ alkyl;
$(R_{1a})_n$ indicates that quinazoline ring of Formula (I) is optionally substituted with n $R_{1a}$, wherein $R_{1a}$ represents halogen, and n represents an integer of 0, 1, 2, or 3; and
$R_a$ represents the following formula:

$$R_{a1}\text{-}X_2\text{-}L_1\text{-}X_1\text{-} ,$$

wherein $X_1$ represents optionally substituted cycloalkylene, optionally substituted heterocyclylene, optionally substituted arylene, or optionally substituted heteroarylene;
Li represents optionally substituted linear or branched $C_{1-60}$ alkylene, or optionally substituted linear or branched $C_{2-60}$ alkylene with one or more groups Rai and/or one or more groups $R_{d2}$ and/or any combination of one or more groups Rai and $R_{d2}$ inserted into its backbone carbon chain, wherein carbon-carbon bond between one or more pairs of adjacent carbon atoms in the backbone carbon chain is interrupted by the group Rai, $R_{d2}$, or a combination of $R_{d1}$ and $R_{d2}$; wherein each Rai is independently selected from the group consisting of O, S, $N(R_{d3})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, where $R_{d3}$ represents H or $C_{1-3}$ alkyl, and in case that two or more groups Rai are inserted into the backbone carbon chain of the linear or branched $C_{2-60}$ alkylene group, the two or more groups Rai are not directly connected to each other; and wherein each $R_{d2}$ is independently selected from the group consisting of optionally substituted cycloalkylene, optionally substituted heterocyclylene, optionally substituted arylene, optionally substituted heteroarylene, alkenylene, alkynylene, or any combination thereof;
$X_2$ represents a bond; and
$R_{a1}$ represents $NR_{d4}R_{d5}$, $C(O)NR_{d6}R_{d7}$, NHC(O)Ras, $NHC(O)NR_{d9}R_{d10}$, $C(O)OR_{d11}$, $OC(O)R_{d12}$,

$OR_{d13}$, $SR_{d14}$, $C(O)R_{d15}$, $C(S)R_{d16}$, $S(O)R_{d17}$, $S(O)_2R_{d18}$, $S(O)_2NHR_{d19}$, $NHS(O)_2R_{d20}$, $S(O)_2OR_{d21}$, $OS(O)_2R_{d22}$, optionally substituted linear or branched $C_{1-10}$ alkyl, optionally substituted cycloalkyl, optionally substituted heterocyclyl, optionally substituted aryl or optionally substituted heteroaryl, wherein $R_{d4}$, Ras, $R_{d6}$, $R_{d7}$, $R_{d9}$, $R_{d10}$, $R_{d11}$, $R_{d13}$, $R_{d14}$, $R_{d19}$ and $R_{d21}$ each independently represent H, optionally substituted linear or branched Ci-io alkyl, optionally substituted cycloalkyl, optionally substituted aryl, optionally substituted heterocyclyl or optionally substituted heteroaryl, and Ras, $R_{d12}$, $R_{d15}$, $R_{d16}$, $R_{d17}$, $R_{d18}$, $R_{d20}$ and $R_{d22}$ represents optionally substituted linear or branched $C_{1-10}$ alkyl, optionally substituted cycloalkyl, optionally substituted heterocyclyl, optionally substituted aryl or optionally substituted heteroaryl;

or

$X_1$ represents $N(R_{e1})$, O, S, alkynylene, alkenylene, optionally substituted cycloalkylene, optionally substituted heterocyclylene, optionally substituted arylene, or optionally substituted heteroarylene, wherein $R_{e1}$ represents H or $C_{1-3}$ alkyl, or $X_1$ represents a bond;

$L_1$ represents optionally substituted linear or branched $C_{1-60}$ alkylene, or optionally substituted linear or branched $C_{2-60}$ alkylene with one or more groups $R_{e2}$ and/or one or more groups $R_{e3}$ and/or any combination of one or more groups $R_{e2}$ and $R_{e3}$ inserted into its backbone carbon chain, wherein carbon-carbon bond between one or more pairs of adjacent carbon atoms in the backbone carbon chain is interrupted by the group $R_{e2}$, $R_{e3}$, or a combination of $R_{e2}$ and $R_{e3}$; wherein each $R_{e2}$ is independently selected from the group consisting of O, S, $N(R_{e4})$, C(O), C(O)O, OC(O), C(O)NH, NHC(O), NHC(O)NH, C(S), S(O), $S(O)_2$, $S(O)_2O$, $OS(O)_2$, $S(O)_2NH$, or $NHS(O)_2$, wherein $R_{e4}$ represents H or $C_{1-3}$ alkyl, and in case that two or more groups $R_{e2}$ are inserted into the backbone carbon chain of the linear or branched $C_{2-60}$ alkylene group, the two or more groups $R_{e2}$ are not directly connected to each other; and wherein each $R_{e3}$ is independently selected from the group consisting of optionally substituted cycloalkylene, optionally substituted heterocyclylene, optionally substituted arylene, optionally substituted heteroarylene, alkenylene, alkynylene, or any combination thereof;

$X_2$ represents C(O), S(O) or $S(O)_2$, or $X_2$ represents a bond;

$R_{a1}$ represents the following formula:

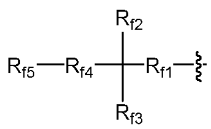

,

wherein $R_{f1}$ represents:

$-NCH_2CH_2N-$;

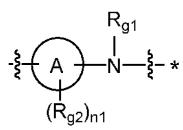

,

wherein symbol * indicates the point of attachment to $X_2$, $R_{g1}$ represents H or $C_{1-3}$ alkyl, ring A represents cycloalkylene or heterocyclylene, and $(R_{g2})_{n1}$ indicates that ring A is optionally substituted with n1 $R_{g2}$ groups, wherein n1 represents an integer of 0 to 8, and each $R_{g2}$, the same or different from each other, is independently selected from the group consisting of deuterium, optionally deuterated $C_{1-6}$ alkyl, optionally deuterated $C_{3-6}$ cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

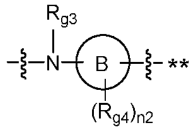

,

wherein symbol ** indicates the point of attachment to $X_2$, $R_{g3}$ represents H or $C_{1-3}$ alkyl, ring B represents cycloalkylene or heterocyclylene, $(R_{g4})_{n2}$ indicates that ring B is optionally substituted with n2 $R_{g4}$, wherein n2 represents an integer of 0 to 8, and each $R_{g4}$, the same or different from each other, is independently selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof; or

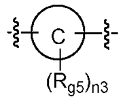,

wherein ring C represents cycloalkylene or heterocyclylene, $(R_{g5})_{n3}$ indicates that ring C is optionally substituted with n3 $R_{g5}$ groups, wherein n3 represents an integer of 0 to 8, and each $R_{g5}$, the same or different from each other, is independently selected from the group consisting of deuterium, optionally deuterated $C_{1-6}$ alkyl, optionally deuterated $C_{3-6}$ cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

$R_{f2}$ represents H, halogen, $C_{1-3}$ alkyl or halogenated $C_{1-3}$ alkyl;
$R_{f3}$ represents H, halogen, $C_{1-3}$ alkyl, halogenated $C_{1-3}$ alkyl,

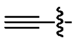, 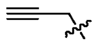, 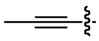

or

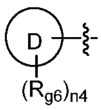,

wherein ring D represents cycloalkylene, heterocyclylene, arylene or heteroarylene, $(R_{g6})_{n4}$ indicates that ring D is optionally substituted with n4 $R_{g6}$ groups, wherein n4 represents an integer of 0 to 8, and each $R_{g6}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;
$R_{f4}$ represents

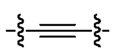, 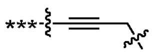, 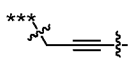 or 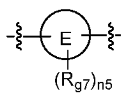,

wherein symbol *** indicates the point of attachment to $R_{f5}$, ring E represents cycloalkylene, heterocyclylene, arylene or heteroarylene, $(R_{g7})_{n5}$ indicates that ring E is optionally substituted with n5 $R_{g7}$, wherein n5 represents an integer of 0 to 8, and each $R_{g7}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof; and
$R_{f5}$ represents H, halogen, $C_{1-3}$ alkyl, halogenated $C_{1-3}$ alkyl,

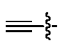, 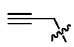, 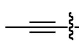

or

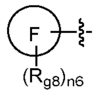
,

wherein ring F represents cycloalkyl, heterocyclyl, aryl or heteroaryl, $(R_{g8})_{n6}$ indicates that ring F is optionally substituted with n6 $R_{g8}$, wherein n6 represents an integer of 0 to 8, and each $R_{g8}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;
or

$X_1$ represents $N(R_1)$, O, S, alkynylene, or alkenylene, wherein $R_1$ represents H or $C_{1-3}$ alkyl, or $X_1$ represents a bond;
$L_1$ represents optionally substituted linear or branched $C_{1-60}$ alkylene;
$X_2$ represents $N(R_2)$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted 4- to 8-membered heterocyclylene containing 1 to 2 nitrogen atoms, triazolylene, triazolylene-NH-, -NH-triazolylene, or optionally substituted phenylene, wherein $R_2$ represents H or $C_{1-3}$ alkyl, or $X_2$ represents a bond; and
$R_{a1}$ represents hydroxy, optionally substituted adamantanyl, optionally substituted noradamantanyl, optionally substituted cubanyl, optionally substituted spirocycloalkyl, optionally substituted bicyclo[2.2.2]octan-1-yl, optionally substituted bicyclo[2.2.2]octan-2-yl, optionally substituted bornyl, optionally substituted bicyclo[2.2.1]heptanyl, optionally substituted bicyclo[2.2.1]heptenyl, optionally substituted p-menthanyl or optionally substituted m-menthanyl, wherein when $R_{a1}$ represents hydroxy, $X_2$ represents a bond;
or
$X_1$ represents $N(R_3)$, O or S, wherein $R_3$ represents H or $C_{1-3}$ alkyl;
$L_1$ represents optionally substituted linear or branched $C_{4-60}$ alkylene;
$X_2$ represents $N(R_4)$, C(O)O, OC(O), C(O)NH, NHC(O), C(O), O, C(S), S, S(O), $S(O)_2$, $S(O)_2NH$, $NHS(O)_2$, $S(O)_2O$, or $OS(O)_2$, wherein $R_4$ represents H or $C_{1-3}$ alkyl, or $X_2$ represents a bond; and
$R_{a1}$ represents optionally substituted heterocyclyl;
or
$X_1$ represents alkynylene or alkenylene, or $X_1$ represents a bond;
$L_1$ represents optionally substituted linear or branched $C_{1-60}$ alkylene; and
$X_2$ represents $N(R_5)$, C(O)O, OC(O), C(O)NH, NHC(O), C(O), O, C(S), S, S(O), $S(O)_2$, $S(O)_2NH$, $NHS(O)_2$, $S(O)_2O$, or $OS(O)_2$, wherein $R_5$ represents H or $C_{1-3}$ alkyl, or $X_2$ represents a bond; and
$R_{a1}$ represents optionally substituted heterocyclyl;
or
$X_1$ represents $N(R_6)$, O, S, alkynylene, or alkenylene, wherein $R_6$ represents H or $C_{1-3}$ alkyl, or $X_1$ represents a bond;
$L_1$ represents optionally substituted linear or branched $C_{2-60}$ alkylene with one or more groups $R_7$ and/or one or more groups $R_8$ and/or any combination of one or more groups $R_7$ and $R_8$ inserted into its backbone carbon chain, wherein carbon-carbon bond between one or more pairs of adjacent carbon atoms in the backbone carbon chain is interrupted by the group $R_7$, $R_8$, or a combination of $R_7$ and $R_8$; wherein each $R_7$ is independently selected from the group consisting of O, S, $N(R_9)$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_9$ represents H or $C_{1-3}$ alkyl, and in case that two or more groups $R_7$ are inserted into the backbone carbon chain of the linear or branched $C_{2-60}$ alkylene group, the two or more groups $R_7$ are not directly connected to each other; and wherein each $R_8$ is independently selected from the group consisting of cycloalkylene, heterocyclylene, triazolylene, alkenylene, alkynylene or any combination thereof, wherein the cycloalkylene, heterocyclylene and triazolylene are each independently optionally substituted with a substituent selected from the group consisting of D, optionally deuterated $C_{1-4}$ alkyl, optionally deuterated $C_{3-6}$ cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof;
$X_2$ represents $N(R_{10})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted 4- to 8-membered heterocyclylene containing 1 to 2 nitrogen atoms, triazolylene, triazolylene-NH-, -NH-triazolylene, or optionally substituted phenylene,

wherein $R_{10}$ represents H or $C_{1-3}$ alkyl, or $X_2$ represents a bond; and
$R_{a1}$ represents hydroxy, optionally substituted heterocyclyl or optionally substituted cycloalkyl, wherein when $R_{a1}$ represents hydroxy, $X_2$ represents a bond;
or

$R_a$ represents the following formula:

$$R_{a2}\text{-}X_3\text{-},$$

wherein $X_3$ represents $N(R_{11})$, C(O)O, OC(O), C(O)NH, NHC(O), O, S, optionally substituted cycloalkylene, optionally substituted arylene, optionally substituted heteroarylene or optionally substituted heterocyclylene, wherein $R_{11}$ represents H or $C_{1-3}$ alkyl, and
$R_{a2}$ represents optionally substituted cycloalkyl, optionally substituted heterocyclyl, optionally substituted heteroaryl or optionally substituted aryl;
and

$R_b$ represents $C_{1-60}$ alkyl optionally substituted with D or halogen, or
$R_b$ represents the following formula:

$$R_{b1}\text{-}X_4\text{-}L_2\text{-},$$

wherein $L_2$ represents optionally substituted linear or branched $C_{2-60}$ alkylene, or optionally substituted linear or branched $C_{2-60}$ alkylene with one or more groups $R_{12}$ and/or one or more groups $R_{13}$ and/or any combination of one or more groups $R_{12}$ and $R_{13}$ inserted into its backbone carbon chain, wherein carbon-carbon bond between one or more pairs of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of cycloalkylene, heterocyclylene, arylene, heteroarylene, alkenylene, alkynylene or any combination thereof, wherein the cycloalkylene, heterocyclylene, arylene and heteroarylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof;
$X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted cycloalkylene, optionally substituted arylene, optionally substituted heterocyclylene, optionally substituted heteroarylene, triazolylene-NH-, or -NH-triazolylene, wherein $R_{15}$ represents H or $C_{1-3}$ alkyl, or $X_4$ represents a bond; and
$R_{b1}$ represents optionally substituted cycloalkyl, optionally substituted aryl, optionally substituted heterocyclyl or optionally substituted heteroaryl;
or

$R_a$ represents halogen or cyano, and
$R_b$ represents the following formula:

$$R_{b2}\text{-}X_5\text{-}L_3\text{-},$$

wherein $L_3$ represents optionally substituted linear or branched $C_{2-60}$ alkylene, or optionally substituted linear or branched $C_{2-60}$ alkylene with one or more groups $R_{16}$ and/or one or more groups $R_{17}$ and/or any

combination of one or more groups $R_{16}$ and $R_{17}$ inserted into its backbone carbon chain, wherein carbon-carbon bond between one or more pairs of adjacent carbon atoms in the backbone carbon chain is interrupted by the group $R_{16}$, $R_{17}$, or a combination of $R_{16}$ and $R_{17}$; wherein each $R_{16}$ is independently selected from the group consisting of O, S, N($R_{18}$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{18}$ represents H or $C_{1-3}$ alkyl, and in case that two or more groups $R_{16}$ are inserted into the backbone carbon chain of the linear or branched $C_{2-60}$ alkylene group, the two or more groups $R_{16}$ are not directly connected to each other; and wherein each $R_{17}$ is independently selected from the group consisting of cycloalkylene, heterocyclylene, arylene, heteroarylene, alkenylene, alkynylene or any combination thereof, wherein the cycloalkylene, heterocyclylene, arylene and heteroarylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof;
$X_5$ represents N($R_{19}$), C(O)O, OC(O), C(O)NH, NHC(O), C(O), O, C(S), S, S(O), $S(O)_2$, $S(O)_2NH$, $NHS(O)_2$, $S(O)_2O$, or $OS(O)_2$, wherein $R_{19}$ represents H or $C_{1-3}$ alkyl, or $X_5$ represents a bond; and
$R_{b2}$ represents optionally substituted cycloalkyl, optionally substituted aryl, optionally substituted heterocyclyl or optionally substituted heteroaryl, or represents the following formula:

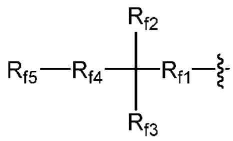
,

wherein $R_{f1}$, $R_{f2}$, $R_{f3}$, $R_{f4}$ and $R_{f5}$ are defined as above.

2. The compound of Formula (I) or salts, enantiomers, diastereoisomer, isotopically enriched analogs, solvates, prodrugs, or polymorphs thereof as claimed in claim 1, wherein the compound of Formula (I) is also of Formula (II) or Formula (III):

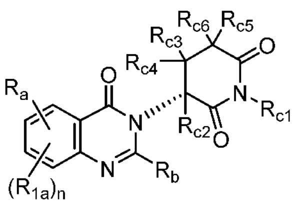
Formula (II)

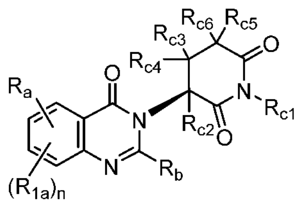
Formula (III)

wherein groups $R_{c1}$, $R_{c2}$, $R_{c3}$, $R_{c4}$, $R_{c5}$, $R_{c6}$, $R_a$, $(R_{1a})_n$ and $R_b$ are as defined in claim 1.

3. The compound of Formula (I) or salts, enantiomers, diastereoisomer, isotopically enriched analogs, solvates, prodrugs, or polymorphs thereof as claimed in claim 1, wherein the compound of Formula (I) is also of Formula (Ia), Formula (Ib), Formula (Ic), Formula (Id), Formula (IIa), Formula (IIb), Formula (IIc), Formula (IId), Formula (IIIa), Formula (IIIb), Formula (IIIc), or Formula (IIId):

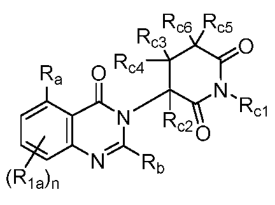
Formula (Ia)

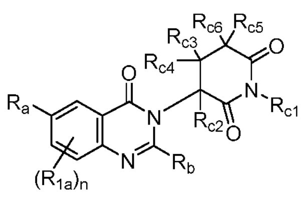
Formula (Ib)

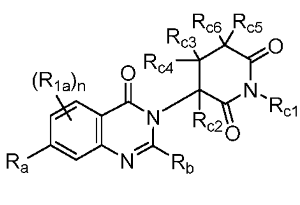
Formula (Ic)

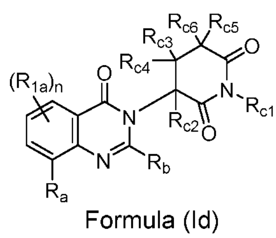
Formula (Id)

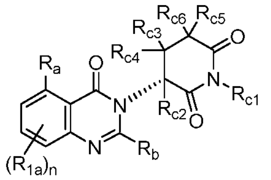
Formula (IIa)

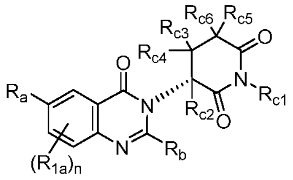
Formula (IIb)

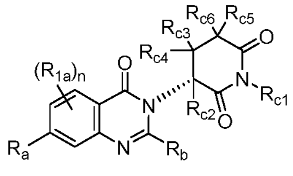
Formula (IIc)

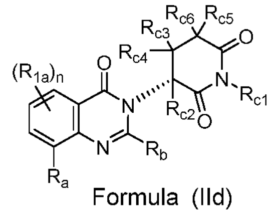
Formula (IId)

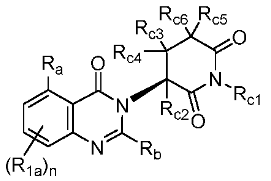
Formula (IIIa)

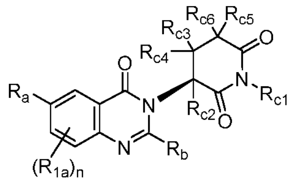
Formula (IIIb)

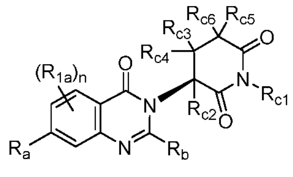
Formula (IIIc)

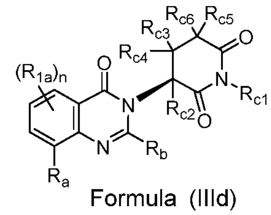
Formula (IIId)

wherein groups $R_{c1}$, $R_{c2}$, $R_{c3}$, $R_{c4}$, $R_{c5}$, $R_{c6}$, $R_a$, $(R_{1a})_n$ and $R_b$ are as defined in claim 1.

**4.** The compound of Formula (I) or salts, enantiomers, diastereoisomer, isotopically enriched analogs, solvates, prodrugs, or polymorphs thereof as claimed in any one of claims 1-3, wherein

$R_a$ represents the following formula:

$$R_{a1}\text{-}X_2\text{-}L_1\text{-}X_1\text{-},$$

wherein $X_1$ represents optionally substituted $C_{3-20}$ cycloalkylene, optionally substituted 4- to 20-membered nitrogen-containing heterocyclylene, optionally substituted $C_{6-10}$ arylene, or optionally substituted 5- to 20-membered heteroarylene;

$L_1$ represents optionally substituted linear or branched $C_{1-60}$ alkylene, or optionally substituted linear or branched $C_{2-60}$ alkylene with one or more groups Rai and/or one or more groups $R_{d2}$ and/or any combination of one or more groups Rai and $R_{d2}$ inserted into its backbone carbon chain, wherein carbon-carbon bond between one or more pairs of adjacent carbon atoms in the backbone carbon chain is interrupted by the group Rai, $R_{d2}$, or a combination of $R_{d1}$ and $R_{d2}$; wherein each Rai is independently selected from the group consisting of O, S, $N(R_{d3})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{d3}$ independently represents H or $C_{1-3}$ alkyl, and in case that two or more groups Rai are inserted into the backbone carbon chain of the linear or branched $C_{2-60}$ alkylene group, the two or more groups Rai are not directly connected to each other; and wherein each $R_{d2}$ is independently selected from the group consisting of optionally substituted $C_{3-15}$ cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH- or any combination thereof, wherein the linear or branched $C_{1-60}$ alkylene and the linear or branched $C_{2-60}$ alkylene are each independently optionally substituted with a substituent selected from the group consisting of deuterium, optionally deuterated $C_{1-6}$ alkyl, optionally deuterated $C_{3-6}$cycloalkyl, hydroxy, amino, mercapto, nitro, halogen, cyano, optionally deuterated $C_{1-6}$ alkoxy, halogenated $C_{1-6}$ alkyl, or any combination thereof;

$X_2$ represents a bond; and

$R_{a1}$ represents $NR_{d4}R_{d5}$, $C(O)NR_{d6}R_{d7}$, NHC(O)Ras, $NHC(O)NR_{d9}R_{d10}$, $C(O)OR_{d11}$, $OC(O)R_{d12}$, $OR_{d13}$, $SR_{d14}$, $C(O)R_{d15}$, $C(S)R_{d16}$, $S(O)R_{d17}$, $S(O)_2R_{d18}$, $S(O)_2NHR_{d19}$, $NHS(O)_2R_{d20}$, $S(O)_2OR_{d21}$, $OS(O)_2R_{d22}$, optionally substituted linear or branched $C_{1-10}$ alkyl, optionally substituted $C_{3-20}$cycloalkyl, optionally substituted 4- to 20-membered heterocyclyl, optionally substituted $C_{6-20}$ aryl or optionally substituted 5- to 20-membered heteroaryl, wherein $R_{d4}$, $R_{d5}$, $R_{d6}$, $R_{d7}$, $R_{d9}$, $R_{d10}$, $R_{d11}$, $R_{d13}$, $R_{d14}$, $R_{d19}$ and $R_{d21}$ are the

same or different and each independently represent H, optionally substituted linear or branched $C_{1-10}$ alkyl, optionally substituted $C_{3-20}$cycloalkyl, optionally substituted $C_{6-20}$ aryl, optionally substituted 4- to 20-membered heterocyclyl or optionally substituted 5- to 20-membered heteroaryl, and Ras, $R_{d12}$, $R_{d15}$, $R_{d16}$, $R_{d17}$, $R_{d18}$, $R_{d20}$ and $R_{d22}$ each independently represent optionally substituted linear or branched $C_{1-10}$ alkyl, optionally substituted $C_{3-20}$cycloalkyl, optionally substituted 4- to 20-membered heterocyclyl, optionally substituted $C_{6-20}$ aryl or optionally substituted 5- to 20-membered heteroaryl;
and

$R_b$ represents $C_{1-60}$ alkyl optionally substituted with D or halogen, or
$R_b$ represents the following formula:

$$R_{b1}\text{-}X_4\text{-}L_2\text{-} ,$$

wherein $L_2$ represents:

linear or branched $C_{2-60}$ alkylene optionally substituted with one or more substituents selected from the group consisting of D, $C_{1-4}$ alkyl, halogen, $C_{3-6}$cycloalkyl or any combination thereof, or
optionally substituted linear or branched $C_{2-60}$ alkylene with one or more groups $R_{12}$ and/or one or more groups $R_{13}$ and/or any combination of one or more groups $R_{12}$ and $R_{13}$ inserted into its backbone carbon chain, wherein carbon-carbon bond between one or more pairs of adj acent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of $C_{3-15}$ cycloalkylene, 4- to 15-membered heterocyclylene, $C_{6-10}$ arylene, 5- to 15-membered heteroarylene, $C_{2-6}$ alkenylene, $C_{2-6}$ alkynylene or any combination thereof, wherein the $C_{3-15}$ cycloalkylene, the 4- to 15-membered heterocyclylene, $C_{6-10}$ arylene and the 5- to 15-membered heteroarylene are each independently optionally substituted with one or more substituents selected from the group consisting of D, optionally deuterated $C_{1-4}$ alkyl, optionally deuterated $C_{3-6}$ cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof, and the linear or branched $C_{2-60}$ alkylene is optionally substituted with one or more substituents selected from the group consisting of D, $C_{1-4}$ alkyl, halogen, $C_{3-6}$ cycloalkyl or any combination thereof;

$X_4$ represents a bond, or $X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted $C_{3-20}$ cycloalkylene, optionally substituted $C_{6-20}$ arylene, optionally substituted 4- to 20-membered heterocyclylene, optionally substituted 5- to 20-membered heteroarylene, triazolylene-NH-, or -NH-triazolylene, wherein $R_{15}$ represents H or $C_{1-3}$ alkyl, and the $C_{3-20}$ cycloalkylene, the $C_{6-20}$ arylene, the 4- to 20-membered heterocyclylene, and the 5- to 20-membered heteroarylene are each independently optionally substituted with one or more substituents selected from the group consisting of D, halogen, optionally deuterated $C_{1-4}$ alkyl, hydroxy, amino, mercapto, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; and
$R_{b1}$ represents optionally substituted $C_{3-20}$ cycloalkyl, optionally substituted $C_{6-20}$ aryl, optionally substituted 4- to 20-membered heterocyclyl or optionally substituted 5- to 20-membered heteroaryl, wherein the $C_{3-20}$ cycloalkyl, the $C_{6-20}$ aryl, the 4- to 20-membered heterocyclyl, and the 5- to 20-membered heteroaryl are each independently optionally substituted with one or more substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$ cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy,

$C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

**5.** The compound of Formula (I) or salts, enantiomers, diastereoisomer, isotopically enriched analogs, solvates, prodrugs, or polymorphs thereof as claimed in any one of claims 1-3, wherein

$R_a$ represents the following formula:

$$R_{a1}\text{-}X_2\text{-}L_1\text{-}X_1\text{-} ,$$

wherein $X_1$ represents $N(R_{e1})$, O, S, $C_{2-6}$ alkynylene, $C_{2-6}$ alkenylene, optionally substituted $C_{3-20}$ cycloalkylene, optionally substituted 4- to 20-membered heterocyclylene, optionally substituted $C_{6-15}$ arylene, or optionally substituted 5- to 20-membered heteroarylene, wherein $R_{e1}$ represents H or $C_{1-3}$ alkyl, or $X_1$ represents a bond;

$L_1$ represents optionally substituted linear or branched $C_{1-60}$ alkylene, or optionally substituted linear or branched $C_{2-60}$ alkylene with one or more groups $R_{e2}$ and/or one or more groups $R_{e3}$ and/or any combination of one or more groups $R_{e2}$ and $R_{e3}$ inserted into its backbone carbon chain, wherein carbon-carbon bond between one or more pairs of adjacent carbon atoms in the backbone carbon chain is interrupted by the group $R_{e2}$, $R_{e3}$, or a combination of $R_{e2}$ and $R_{e3}$; wherein each $R_{e2}$ is independently selected from the group consisting of O, S, $N(R_{e4})$, C(O), C(O)O, OC(O), C(O)NH, NHC(O), NHC(O)NH, C(S), S(O), $S(O)_2$, $S(O)_2O$, $OS(O)_2$, $S(O)_2NH$, or $NHS(O)_2$, wherein $R_{e4}$ represents H or $C_{1-3}$ alkyl, and in case that two or more groups $R_{e2}$ are inserted into the backbone carbon chain of the linear or branched $C_{2-60}$ alkylene group, the two or more groups $R_{e2}$ are not directly connected to each other; and wherein each $R_{e3}$ is independently selected from the group consisting of optionally substituted $C_{3-15}$ cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the linear or branched $C_{1-60}$ alkylene and the linear or branched $C_{2-60}$ alkylene are each independently optionally substituted with a substituent selected from the group consisting of deuterium, optionally deuterated $C_{1-6}$ alkyl, optionally deuterated $C_{3-6}$ cycloalkyl, hydroxy, amino, mercapto, nitro, halogen, cyano, optionally deuterated $C_{1-6}$ alkoxy, halogenated $C_{1-6}$ alkyl, or any combination thereof;

$X_2$ represents C(O) or $S(O)_2$, or $X_2$ represents a bond;

$R_{a1}$ represents the following formula:

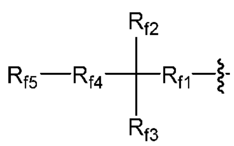

,

wherein $R_{f1}$ represents:

$$\text{-}NCH_2CH_2N\text{-};$$

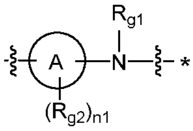

,

wherein symbol * indicates the point of attachment to $X_2$, $R_{g1}$ represents H or $C_{1-3}$ alkyl, ring A represents $C_{3-20}$ cycloalkylene or 4- to 20-membered heterocyclylene, $(R_{g2})_{n1}$ indicates that ring A is optionally substituted with n1 $R_{g2}$, wherein n1 represents an integer of 0 to 8, and each

$R_{g2}$, the same or different from each other, is independently selected from the group consisting of deuterium, optionally deuterated $C_{1-6}$ alkyl, optionally deuterated $C_{3-6}$ cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

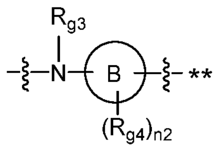
,

wherein symbol ** indicates the point of attachment to $X_2$, $R_{g3}$ represents H or $C_{1-3}$ alkyl, ring B represents $C_{3-20}$ cycloalkylene or 4- to 20-membered heterocyclylene, and $(R_{g4})_{n2}$ indicates that ring B is optionally substituted with n2 $R_{g4}$, wherein n2 represents an integer of 0 to 8, and each $R_{g4}$, the same or different from each other, is independently selected from the group consisting of deuterium, optionally deuterated $C_{1-6}$ alkyl, optionally deuterated $C_{3-6}$ cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof; or

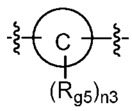
,

wherein ring C represents $C_{3-20}$ cycloalkylene or 4- to 20-membered heterocyclylene, $(R_{g5})_{n3}$ indicates that ring C is optionally substituted with n3 $R_{g5}$, n3 represents an integer of 0 to 8, and each $R_{g5}$, the same or different from each other, is independently selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

$R_{f2}$ represents H, halogen, $C_{1-3}$ alkyl or halogenated $C_{1-3}$ alkyl;
$R_{f3}$ represents H, halogen, $C_{1-3}$ alkyl, halogenated $C_{1-3}$ alkyl,

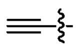
,
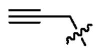
,
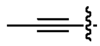

or

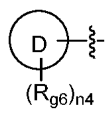
,

wherein ring D represents $C_{3-20}$cycloalkylene, 4- to 20-membered heterocyclylene, $C_{6-15}$ arylene or 5- to 20-membered heteroarylene, $(R_{g6})_{n4}$ indicates that ring D is optionally substituted with n4 $R_{g6}$, n4 represents an integer of 0 to 8, and each $R_{g6}$, the same or different from each other, is independently selected from the group consisting of optionally deuterated $C_{1-6}$ alkyl, optionally deuterated $C_{3-6}$ cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;
$R_{f4}$ represents

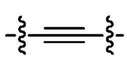
,
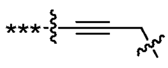
,
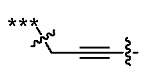
or
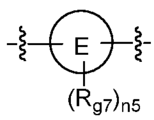
,

wherein symbol *** indicates the point of attachment to $R_{f5}$, ring E represents $C_{3-20}$ cycloalkylene, 4-

to 20-membered heterocyclylene, $C_{6-15}$ arylene or 5- to 20-membered heteroarylene, $(R_{g7})_{n5}$ indicates that ring E is optionally substituted with n5 $R_{g7}$, wherein n5 represents an integer of 0 to 8, and each $R_{g7}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof; and

$R_{f5}$ represents H, halogen, $C_{1-3}$ alkyl, halogenated $C_{1-3}$ alkyl,

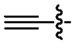, 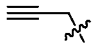, 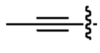

or

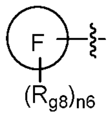,

wherein ring F represents $C_{3-20}$ cycloalkyl, 4- to 20-membered heterocyclyl, $C_{6-15}$ aryl or 5- to 20-membered heteroaryl, $(R_{g8})_{n6}$ indicates that ring F is optionally substituted with n6 $R_{g8}$, wherein n6 represents an integer of 0 to 8, and each $R_{g8}$, the same or different from each other, is independently selected from the group consisting of optionally deuterated $C_{1-6}$ alkyl, optionally deuterated $C_{3-6}$ cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof:

and

$R_b$ represents $C_{1-60}$ alkyl optionally substituted with D or halogen, or
$R_b$ represents the following formula:

$R_{b1}$-$X_4$-$L_2$- ,

wherein $L_2$ represents

linear or branched $C_{2-60}$ alkylene optionally substituted with one or more substituents selected from the group consisting of D, $C_{1-4}$ alkyl, halogen, $C_{3-6}$ cycloalkyl or any combination thereof, or

optionally substituted linear or branched $C_{2-60}$ alkylene with one or more groups $R_{12}$ and/or one or more groups $R_{13}$ and/or any combination of one or more groups $R_{12}$ and $R_{13}$ inserted into its backbone carbon chain, wherein carbon-carbon bond between one or more pairs of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of $C_{3-15}$ cycloalkylene, 4- to 15-membered heterocyclylene, $C_{6-10}$ arylene, 5- to 15-membered heteroarylene, $C_{2-6}$ alkenylene, $C_{2-6}$ alkynylene or any combination thereof, wherein the $C_{3-15}$ cycloalkylene, the 4- to 15-membered heterocyclylene, $C_{6-10}$ arylene and the 5- to 15-membered heteroarylene are each independently optionally substituted with one or more substituents selected from the group consisting of D, optionally deuterated $C_{1-4}$ alkyl, optionally deuterated $C_{3-6}$ cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof, and the linear or branched $C_{2-60}$ alkylene is optionally substituted with one or more substituents selected from the group consisting of D, $C_{1-4}$ alkyl, halogen, $C_{3-6}$ cycloalkyl or any combination thereof;

$X_4$ represents a bond, or $X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted $C_{3-20}$ cycloalkylene, optionally

substituted $C_{6-20}$ arylene, optionally substituted 4- to 20-membered heterocyclylene, optionally substituted 5- to 20-membered heteroarylene, triazolylene-NH-, or -NH-triazolylene, wherein $R_{15}$ represents H or $C_{1-3}$ alkyl, and the $C_{3-20}$cycloalkylene, the $C_{6-20}$ arylene, the 4- to 20-membered heterocyclylene, and the 5- to 20-membered heteroarylene are each independently optionally substituted with one or more substituents selected from the group consisting of D, halogen, optionally deuterated $C_{1-4}$ alkyl, hydroxy, amino, mercapto, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; and

$R_{b1}$ represents optionally substituted $C_{3-20}$ cycloalkyl, optionally substituted $C_{6-20}$ aryl, optionally substituted 4- to 20-membered heterocyclyl or optionally substituted 5- to 20-membered heteroaryl, wherein the $C_{3-20}$ cycloalkyl, the $C_{6-20}$ aryl, the 4- to 20-membered heterocyclyl, and the 5- to 20-membered heteroaryl are each independently optionally substituted with one or more substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

**6.** The compound of Formula (I) or salts, enantiomers, diastereoisomer, isotopically enriched analogs, solvates, prodrugs, or polymorphs thereof as claimed in any one of claims 1-3, wherein

$R_a$ represents the following formula:

$$R_{a1}\text{-}X_2\text{-}L_1\text{-}X_1\text{-},$$

wherein $X_1$ represents a bond, or $X_1$ represents $N(R_1)$, O, S, $C_{2-6}$ alkynylene or $C_{2-6}$ alkenylene, wherein $R_1$ represents H or $C_{1-3}$ alkyl;

$L_1$ represents linear or branched $C_{1-60}$ alkylene optionally substituted with one or more substituents selected from the group consisting of D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy, or any combination thereof;

$X_2$ represents a bond, or $X_2$ represents $N(R_2)$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted 4- to 8-membered heterocyclylene containing 1 to 2 nitrogen atoms, triazolylene, triazolylene-NH-, -NH-triazolylene, or optionally substituted phenylene, wherein $R_2$ represents H or $C_{1-3}$ alkyl, and the 4- to 8-membered heterocyclylene containing 1 to 2 nitrogen atoms is optionally substituted with 1 to 8 substituents selected from the group consisting of D, halogen, optionally deuterated $C_{1-4}$ alkyl, oxo, hydroxy, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano, or any combination thereof, and the phenylene is optionally substituted with 1 to 4 substituents selected from the group consisting of D, halogen, optionally deuterated $C_{1-4}$ alkyl, hydroxy, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano, or any combination thereof; and

$R_{a1}$ represents hydroxy, optionally substituted adamantanyl, optionally substituted noradamantanyl, optionally substituted cubanyl, optionally substituted spirocycloalkyl, optionally substituted bicyclo[2.2.2]octan-1-yl, optionally substituted bicyclo[2.2.2]octan-2-yl, optionally substituted bornyl, optionally substituted bicyclo[2.2.1]heptanyl, optionally substituted bicyclo[2.2.1]heptenyl, optionally substituted p-menthanyl or optionally substituted m-menthanyl, wherein when $R_{a1}$ represents hydroxy, $X_2$ represents a bond, and the adamantanyl, noradamantanyl, cubanyl, spirocycloalkyl, bicyclo[2.2.2]octan-1-yl, bicyclo[2.2.2]octan-2-yl, bornyl, bicyclo[2.2.1]heptanyl, bicyclo[2.2.1]heptenyl, p-menthanyl and m-menthanyl are each independently optionally substituted with one or more substituents selected from the group consisting of D, halogen, optionally deuterated $C_{1-4}$ alkyl, hydroxy, amino, mercapto, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;
and

$R_b$ represents $C_{1-60}$ alkyl optionally substituted with D or halogen, or

$R_b$ represents the following formula:

$R_{b1}$-$X_4$-$L_2$-,

wherein $L_2$ represents

linear or branched $C_{2-60}$ alkylene optionally substituted with one or more substituents selected from the group consisting of D, $C_{1-4}$ alkyl, halogen, $C_{3-6}$ cycloalkyl or any combination thereof, or
optionally substituted linear or branched $C_{2-60}$ alkylene with one or more groups $R_{12}$ and/or one or more groups $R_{13}$ and/or any combination of one or more groups $R_{12}$ and $R_{13}$ inserted into its backbone carbon chain, wherein carbon-carbon bond between one or more pairs of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, N($R_{14}$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of $C_{3-15}$ cycloalkylene, 4- to 15-membered heterocyclylene, $C_{6-10}$ arylene, 5- to 15-membered heteroarylene or any combination thereof, wherein the $C_{3-15}$ cycloalkylene, the 4- to 15-membered heterocyclylene, $C_{6-10}$ arylene and the 5- to 15-membered heteroarylene are each independently optionally substituted with one or more substituents selected from the group consisting of D, optionally deuterated $C_{1-4}$ alkyl, optionally deuterated $C_{3-6}$ cycloalkyl, hydroxy, amino, mercapto, halogen, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, cyano or any combination thereof, and the linear or branched $C_{2-60}$ alkylene is optionally substituted with one or more substituents selected from the group consisting of D, $C_{1-4}$ alkyl, halogen, $C_{3-6}$ cycloalkyl or any combination thereof;

$X_4$ represents a bond, or $X_4$ represents N($R_{15}$), C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted $C_{3-20}$ cycloalkylene, optionally substituted $C_{6-20}$ arylene, optionally substituted 4- to 20-membered heterocyclylene, optionally substituted 5- to 20-membered heteroarylene, triazolylene-NH-, or -NH-triazolylene, wherein $R_{15}$ represents H or $C_{1-3}$ alkyl, and the $C_{3-20}$ cycloalkylene, the $C_{6-20}$ arylene, the 4- to 20-membered heterocyclylene, and the 5- to 20-membered heteroarylene are each independently optionally substituted with one or more substituents selected from the group consisting of D, halogen, optionally deuterated $C_{1-4}$ alkyl, hydroxy, amino, mercapto, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; and
$R_{b1}$ represents optionally substituted $C_{3-20}$ cycloalkyl, optionally substituted $C_{6-20}$ aryl, optionally substituted 4- to 20-membered heterocyclyl or optionally substituted 5- to 20-membered heteroaryl, wherein the $C_{3-20}$ cycloalkyl, the $C_{6-20}$ aryl, the 4- to 20-membered heterocyclyl, and the 5- to 20-membered heteroaryl are each independently optionally substituted with one or more substituents selected from the group consisting of D, optionally deuterated $C_{1-4}$ alkyl, optionally substituted $C_{3-15}$ cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the $C_{3-15}$ cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

**7.** The compound of Formula (I) or salts, enantiomers, diastereoisomer, isotopically enriched analogs, solvates, prodrugs, or polymorphs thereof as claimed in any one of claims 1-3, wherein

$R_a$ represents the following formula:

$R_{a1}$-$X_2$-$L_1$-$X_1$-,

wherein $X_1$ represents N($R_3$), O or S, wherein $R_3$ represents H or $C_{1-3}$ alkyl;

$L_1$ represents linear or branched $C_{4-60}$ alkylene optionally substituted with one or more substituents selected

from the group consisting of D, optionally deuterated $C_{1-4}$ alkyl, hydroxy, amino, mercapto, nitro, halogen, cyano, halogenated $C_{1-6}$ alkyl, optionally deuterated $C_{3-6}$ cycloalkyl, $C_{1-6}$ alkoxy, or any combination thereof;
$X_2$ represents a bond, or $X_2$ represents $N(R_4)$, C(O)O, OC(O), C(O)NH, NHC(O), C(O), O, C(S), S, S(O), $S(O)_2$, $S(O)_2NH$, $NHS(O)_2$, $S(O)_2O$, or $OS(O)_2$, wherein $R_4$ represents H or $C_{1-3}$ alkyl; and
$R_{a1}$ represents optionally substituted 4- to 20-membered heterocyclyl, and the 4- to 20-membered heterocyclyl is optionally substituted with one or more substituents selected from the group consisting of D, optionally deuterated $C_{1-4}$ alkyl, optionally substituted $C_{3-15}$ cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the $C_{3-15}$ cycloalkyl, the 4-to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;
and

$R_b$ represents $C_{1-60}$ alkyl optionally substituted with D or halogen, or
$R_b$ represents the following formula:

$$R_{b1}\text{-}X_4\text{-}L_2\text{-} ,$$

wherein $L_2$ represents

linear or branched $C_{2-60}$ alkylene optionally substituted with one or more substituents selected from the group consisting of D, $C_{1-4}$ alkyl, halogen, $C_{3-6}$ cycloalkyl or any combination thereof, or
optionally substituted linear or branched $C_{2-60}$ alkylene with one or more groups $R_{12}$ and/or one or more groups $R_{13}$ and/or any combination of one or more groups $R_{12}$ and $R_{13}$ inserted into its backbone carbon chain, wherein carbon-carbon bond between one or more pairs of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of $C_{3-15}$ cycloalkylene, 4- to 15-membered heterocyclylene, $C_{6-10}$ arylene, 5- to 15-membered heteroarylene, $C_{2-6}$ alkenylene, $C_{2-6}$ alkynylene or any combination thereof, wherein the $C_{3-15}$ cycloalkylene, the 4- to 15-membered heterocyclylene, $C_{6-10}$ arylene and the 5- to 15-membered heteroarylene are each independently optionally substituted with one or more substituents selected from the group consisting of D, optionally deuterated $C_{1-4}$ alkyl, optionally deuterated $C_{3-6}$ cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof, and the linear or branched $C_{2-60}$ alkylene is optionally substituted with one or more substituents selected from the group consisting of D, $C_{1-4}$ alkyl, halogen, $C_{3-6}$ cycloalkyl or any combination thereof;
$X_4$ represents a bond, or $X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted $C_{3-20}$ cycloalkylene, optionally substituted $C_{6-20}$ arylene, optionally substituted 4- to 20-membered heterocyclylene, optionally substituted 5- to 20-membered heteroarylene, triazolylene-NH-, or -NH-triazolylene, wherein $R_{15}$ represents H or $C_{1-3}$ alkyl, and the $C_{3-20}$ cycloalkylene, the $C_{6-20}$ arylene, the 4- to 20-membered heterocyclylene, and the 5- to 20-membered heteroarylene are each independently optionally substituted with one or more substituents selected from the group consisting of D, halogen, optionally deuterated $C_{1-4}$ alkyl, hydroxy, amino, mercapto, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; and
$R_{b1}$ represents optionally substituted $C_{3-20}$ cycloalkyl, optionally substituted $C_{6-20}$ aryl, optionally substituted 4- to 20-membered heterocyclyl or optionally substituted 5- to 20-membered heteroaryl, wherein the $C_{3-20}$ cycloalkyl, the $C_{6-20}$ aryl, the 4- to 20-membered heterocyclyl, and the 5- to 20-membered heteroaryl are each independently optionally substituted with one or more substituents selected from the group consisting of D, optionally deuterated $C_{1-4}$ alkyl, optionally substituted $C_{3-15}$ cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the $C_{3-15}$ cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

**8.** The compound of Formula (I) or salts, enantiomers, diastereoisomer, isotopically enriched analogs, solvates, prodrugs, or polymorphs thereof as claimed in any one of claims 1-3, wherein

$R_a$ represents the following formula:

$$R_{a1}\text{-}X_2\text{-}L_1\text{-}X_1\text{-} ,$$

wherein $X_1$ represents a bond, or $X_1$ represents $C_{2-6}$ alkynylene or $C_{2-6}$ alkenylene;

$L_1$ represents linear or branched $C_{1-60}$ alkylene optionally substituted with one or more substituents selected from the group consisting of D, optionally deuterated $C_{1-4}$ alkyl, hydroxy, amino, mercapto, nitro, halogen, cyano, halogenated $C_{1-6}$ alkyl, optionally deuterated $C_{3-6}$ cycloalkyl, $C_{1-6}$ alkoxy, or any combination thereof; and

$X_2$ represents a bond, or $X_2$ represents N(Rs), C(O)O, OC(O), C(O)NH, NHC(O), C(O), O, C(S), S, S(O), $S(O)_2$, $S(O)_2NH$, $NHS(O)_2$, $S(O)_2O$, or $OS(O)_2$, wherein $R_5$ represents H or $C_{1-3}$ alkyl; and

$R_{a1}$ represents optionally substituted 4- to 20-membered heterocyclyl, wherein the 4- to 20-membered heterocyclyl is optionally substituted with one or more substituents selected from the group consisting of D, optionally deuterated $C_{1-4}$ alkyl, optionally substituted $C_{3-15}$ cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the $C_{3-15}$ cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

and

$R_b$ represents $C_{1-60}$ alkyl optionally substituted with D or halogen, or
$R_b$ represents the following formula:

$$R_{b1}\text{-}X_4\text{-}L_2\text{-},$$

wherein $L_2$ represents

linear or branched $C_{2-60}$ alkylene optionally substituted with one or more substituents selected from the group consisting of D, $C_{1-4}$ alkyl, halogen, $C_{3-6}$ cycloalkyl or any combination thereof, or

optionally substituted linear or branched $C_{2-60}$ alkylene with one or more groups $R_{12}$ and/or one or more groups $R_{13}$ and/or any combination of one or more groups $R_{12}$ and $R_{13}$ inserted into its backbone carbon chain, wherein carbon-carbon bond between one or more pairs of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, N($R_{14}$), C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of $C_{3-15}$ cycloalkylene, 4- to 15-membered heterocyclylene, $C_{6-10}$ arylene, 5- to 15-membered heteroarylene, $C_{2-6}$ alkenylene, $C_{2-6}$ alkynylene or any combination thereof, wherein the $C_{3-15}$ cycloalkylene, the 4- to 15-membered heterocyclylene, $C_{6-10}$ arylene and the 5- to 15-membered heteroarylene are each independently optionally

substituted with one or more substituents selected from the group consisting of D, optionally deuterated $C_{1-4}$ alkyl, optionally deuterated $C_{3-6}$ cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof, and the linear or branched $C_{2-60}$ alkylene is optionally substituted with one or more substituents selected from the group consisting of D, $C_{1-4}$ alkyl, halogen, $C_{3-6}$ cycloalkyl or any combination thereof;

$X_4$ represents a bond, or $X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted $C_{3-20}$ cycloalkylene, optionally substituted $C_{6-20}$ arylene, optionally substituted 4- to 20-membered heterocyclylene, optionally substituted 5- to 20-membered heteroarylene, triazolylene-NH-, or -NH-triazolylene, wherein $R_{15}$ represents H or $C_{1-3}$ alkyl, and the $C_{3-20}$ cycloalkylene, the $C_{6-20}$ arylene, the 4- to 20-membered heterocyclylene, and the 5- to 20-membered heteroarylene are each independently optionally substituted with one or more substituents selected from the group consisting of D, halogen, optionally deuterated $C_{1-4}$ alkyl, hydroxy, amino, mercapto, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; and

$R_{b1}$ represents optionally substituted $C_{3-20}$ cycloalkyl, optionally substituted $C_{6-20}$ aryl, optionally substituted 4- to 20-membered heterocyclyl or optionally substituted 5- to 20-membered heteroaryl, wherein the $C_{3-20}$ cycloalkyl, the $C_{6-20}$ aryl, the 4- to 20-membered heterocyclyl, and the 5- to 20-membered heteroaryl are each independently optionally substituted with one or more substituents selected from the group consisting of D, optionally deuterated $C_{1-4}$ alkyl, optionally substituted $C_{3-15}$ cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$ cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

**9.** The compound of Formula (I) or salts, enantiomers, diastereoisomer, isotopically enriched analogs, solvates, prodrugs, or polymorphs thereof as claimed in any one of claims 1-3, wherein

$R_a$ represents the following formula:

$$R_{a1}\text{-}X_2\text{-}L_1\text{-}X_1\text{-} ,$$

wherein $X_1$ represents a bond, or $X_1$ represents $N(R_6)$, O, S, $C_{2-6}$ alkynylene, or $C_{2-6}$ alkenylene, wherein $R_6$ represents H or $C_{1-3}$ alkyl;

$L_1$ represents optionally substituted linear or branched $C_{2-60}$ alkylene with one or more groups $R_7$ and/or one or more groups Rs and/or any combination of one or more groups $R_7$ and $R_8$ inserted into its backbone carbon chain, wherein carbon-carbon bond between one or more pairs of adjacent carbon atoms in the backbone carbon chain is interrupted by the group $R_7$, $R_8$, or a combination of $R_7$ and Rs; wherein each $R_7$ is independently selected from the group consisting of O, S, $N(R_9)$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_9$ represents H or $C_{1-3}$ alkyl, and in case that two or more groups $R_7$ are inserted into the backbone carbon chain of the linear or branched $C_{2-60}$ alkylene group, the two or more groups $R_7$ are not directly connected to each other; and wherein each Rs is independently selected from the group consisting of $C_{3-15}$ cycloalkylene, 4- to 15-membered heterocyclylene, triazolylene or any combination thereof, wherein the $C_{3-15}$ cycloalkylene, the 4- to 15-membered heterocyclylene and the triazolylene are each independently optionally substituted with a substituent selected from the group consisting of D, optionally deuterated $C_{1-4}$ alkyl, optionally deuterated $C_{3-6}$ cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the linear or branched $C_{2-60}$ alkylene is optionally substituted with one or more substituents selected from the group consisting of D, optionally deuterated $C_{1-4}$ alkyl, hydroxy, amino, mercapto, nitro, halogen, cyano, halogenated $C_{1-6}$ alkyl, optionally deuterated $C_{3-6}$ cycloalkyl, $C_{1-6}$ alkoxy, or any combination thereof;

$X_2$ represents a bond, or $X_2$ represents $N(R_{10})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted 4- to 8-membered heterocyclylene

containing 1 to 2 nitrogen atoms, triazolylene, triazolylene-NH-, -NH-triazolylene, or optionally substituted phenylene, wherein $R_{10}$ represents H or $C_{1-3}$ alkyl, and the 4- to 8-membered heterocyclylene containing 1 to 2 nitrogen atoms is optionally substituted with 1 to 8 substituents selected from the group consisting of D, halogen, optionally deuterated $C_{1-4}$ alkyl, oxo, hydroxy, amino, mercapto, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the phenylene is optionally substituted with 1 to 4 substituents selected from the group consisting of D, halogen, optionally deuterated $C_{1-4}$ alkyl, hydroxy, amino, mercapto, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

and

$R_{a1}$ represents hydroxy, optionally substituted 4- to 20-membered heterocyclyl or optionally substituted $C_{3-20}$ cycloalkyl, wherein when $R_{a1}$ represents hydroxy, $X_2$ represents a bond, and the 4- to 20-membered heterocyclyl and the $C_{3-20}$ cycloalkyl are each independently optionally substituted with one or more substituents selected from the group consisting of D, optionally deuterated $C_{1-4}$ alkyl, optionally substituted $C_{3-15}$ cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$ cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

and

$R_b$ represents $C_{1-60}$ alkyl optionally substituted with D or halogen, or
$R_b$ represents the following formula:

$$R_{b1}\text{-}X_4\text{-}L_2\text{-},$$

wherein $L_2$ represents

linear or branched $C_{2-60}$ alkylene optionally substituted with one or more substituents selected from the group consisting of D, $C_{1-4}$ alkyl, halogen, $C_{3-6}$ cycloalkyl or any combination thereof, or

optionally substituted linear or branched $C_{2-60}$ alkylene with one or more groups $R_{12}$ and/or one or more groups $R_{13}$ and/or any combination of one or more groups $R_{12}$ and $R_{13}$ inserted into its backbone carbon chain, wherein carbon-carbon bond between one or more pairs of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of $C_{3-15}$ cycloalkylene, 4- to 15-membered heterocyclylene, $C_{6-10}$ arylene, 5- to 15-membered heteroarylene, $C_{2-6}$ alkenylene, $C_{2-6}$ alkynylene or any combination thereof, wherein the $C_{3-15}$ cycloalkylene, the 4- to 15-membered heterocyclylene, $C_{6-10}$ arylene and the 5- to 15-membered heteroarylene are each independently optionally substituted with one or more substituents selected from the group consisting of D, optionally deuterated $C_{1-4}$ alkyl, optionally deuterated $C_{3-6}$ cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the linear or branched $C_{2-60}$ alkylene is optionally substituted with one or more substituents selected from the group consisting of D, $C_{1-4}$ alkyl, halogen, $C_{3-6}$ cycloalkyl or any combination thereof;

$X_4$ represents a bond, or $X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted $C_{3-20}$ cycloalkylene, optionally substituted $C_{6-20}$ arylene, optionally substituted 4- to 20-membered heterocyclylene, optionally substituted 5- to 20-membered heteroarylene, triazolylene-NH-, or -NH-triazolylene, wherein $R_{15}$ represents H or $C_{1-3}$ alkyl, and the $C_{3-20}$ cycloalkylene, the $C_{6-20}$ arylene, the 4- to 20-membered heterocyclylene, and the 5- to 20-membered heteroarylene are each independently optionally substituted with one or more substituents selected from the group consisting of D, halogen, optionally deuterated $C_{1-4}$ alkyl, hydroxy, amino, mercapto,

cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; and

$R_{b1}$ represents optionally substituted $C_{3-20}$ cycloalkyl, optionally substituted $C_{6-20}$ aryl, optionally substituted 4- to 20-membered heterocyclyl or optionally substituted 5- to 20-membered heteroaryl, wherein the $C_{3-20}$ cycloalkyl, the $C_{6-20}$ aryl, the 4- to 20-membered heterocyclyl, and the 5- to 20-membered heteroaryl are each independently optionally substituted with one or more substituents selected from the group consisting of D, optionally deuterated $C_{1-4}$ alkyl, optionally substituted $C_{3-15}$ cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, $C_{1-3}$ alkoxy, $C_{1-3}$ alkyl-NH-, halogenated $C_{1-3}$ alkyl, $NH_2$-$C_{1-3}$ alkylene, $C_{1-3}$ alkyl-NHC(O)-, $C_{1-3}$ alkyl-C(O)NH-, cyano or any combination thereof, and wherein the $C_{3-15}$ cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

**10.** The compound of Formula (I) or salts, enantiomers, diastereoisomer, isotopically enriched analogs, solvates, prodrugs, or polymorphs thereof as claimed in any one of claims 1-3, wherein

$R_a$ represents the following formula:

$$R_{a2}\text{-}X_3\text{-} ,$$

wherein $X_3$ represents $N(R_{11})$, C(O)O, OC(O), C(O)NH, NHC(O), O, S, optionally substituted $C_{3-20}$ cycloalkylene, optionally substituted $C_{6-20}$ arylene, optionally substituted 5- to 20-membered heteroarylene or optionally substituted 4- to 20-membered heterocyclylene, wherein $R_{11}$ represents H or $C_{1-3}$ alkyl, wherein the $C_{3-20}$ cycloalkylene and the 4- to 20-membered heterocyclylene are each independently optionally substituted with 1 to 8 substituents selected from the group consisting of D, halogen, optionally deuterated $C_{1-4}$ alkyl, oxo, or any combination thereof, and the $C_{6-20}$ arylene and the 5- to 20-membered heteroarylene are each independently optionally substituted with 1 to 8 substituents selected from the group consisting of D, halogen, optionally deuterated $C_{1-4}$ alkyl, or any combination thereof, and

$R_{a2}$ represents optionally substituted $C_{3-20}$ cycloalkyl, optionally substituted 4- to 20-membered heterocyclyl, optionally substituted 5- to 20-membered heteroaryl or optionally substituted $C_{6-20}$ aryl, wherein the $C_{3-20}$ cycloalkyl, the $C_{6-20}$ aryl, the 4- to 20-membered heterocyclyl, and the 5- to 20-membered heteroaryl are each independently optionally substituted with one or more substituents selected from the group consisting of D, optionally deuterated $C_{1-4}$ alkyl, optionally substituted $C_{3-15}$ cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the $C_{3-15}$ cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

and

$R_b$ represents $C_{1-60}$ alkyl optionally substituted with D or halogen, or
$R_b$ represents the following formula:

$$R_{b1}\text{-}X_4\text{-}L_2\text{-} ,$$

wherein $L_2$ represents

linear or branched $C_{2-60}$ alkylene optionally substituted with one or more substituents selected from the group consisting of D, $C_{1-4}$ alkyl, halogen, $C_{3-6}$ cycloalkyl or any combination thereof, or

optionally substituted linear or branched $C_{2-60}$ alkylene with one or more groups $R_{12}$ and/or one or more

groups $R_{13}$ and/or any combination of one or more groups $R_{12}$ and $R_{13}$ inserted into its backbone carbon chain, wherein carbon-carbon bond between one or more pairs of adjacent carbon atoms in the backbone carbon chain is interrupted by the group $R_{12}$, $R_{13}$, or a combination of $R_{12}$ and $R_{13}$; wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{14}$ independently represents H or $C_{1-3}$ alkyl, and in case that two or more groups $R_{12}$ are inserted into the backbone carbon chain of the linear or branched $C_{2-60}$ alkylene group, the two or more groups $R_{12}$ are not directly connected to each other; and wherein each $R_{13}$ is independently selected from the group consisting of $C_{3-15}$ cycloalkylene, 4- to 15-membered heterocyclylene, $C_{6-10}$ arylene, 5- to 15-membered heteroarylene, $C_{2-6}$ alkenylene, $C_{2-6}$ alkynylene or any combination thereof, wherein the $C_{3-15}$ cycloalkylene, the 4- to 15-membered heterocyclylene, $C_{6-10}$ arylene and the 5- to 15-membered heteroarylene are each independently optionally substituted with one or more substituents selected from the group consisting of D, optionally deuterated $C_{1-4}$ alkyl, optionally deuterated $C_{3-6}$ cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the linear or branched $C_{2-60}$ alkylene is optionally substituted with one or more substituents selected from the group consisting of D, $C_{1-4}$ alkyl, halogen, $C_{3-6}$ cycloalkyl or any combination thereof;

$X_4$ represents a bond, or $X_4$ represents $N(R_{15})$, C(O)O, OC(O), C(O)NH, NHC(O), C(O), O, C(S), S, S(O), $S(O)_2$, $S(O)_2NH$, $NHS(O)_2$, $S(O)_2O$, $OS(O)_2$, optionally substituted $C_{3-20}$ cycloalkylene, optionally substituted $C_{6-20}$ arylene, optionally substituted 4- to 20-membered heterocyclylene, optionally substituted 5- to 20-membered heteroarylene, triazolylene-NH-, or -NH-triazolylene, wherein $R_{15}$ represents H or $C_{1-3}$ alkyl, and the $C_{3-20}$ cycloalkylene, the $C_{6-20}$ arylene, the 4- to 20-membered heterocyclylene, and the 5- to 20-membered heteroarylene are each independently optionally substituted with one or more substituents selected from the group consisting of D, halogen, optionally deuterated $C_{1-4}$ alkyl, hydroxy, amino, mercapto, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; and

$R_{b1}$ represents optionally substituted $C_{3-20}$ cycloalkyl, optionally substituted $C_{6-20}$ aryl, optionally substituted 4- to 20-membered heterocyclyl or optionally substituted 5- to 20-membered heteroaryl, wherein the $C_{3-20}$ cycloalkyl, the $C_{6-20}$ aryl, the 4- to 20-membered heterocyclyl, and the 5- to 20-membered heteroaryl are each independently optionally substituted with one or more substituents selected from the group consisting of D, optionally deuterated $C_{1-4}$ alkyl, optionally substituted $C_{3-15}$ cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$ cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

**11.** The compound of Formula (I) or salts, enantiomers, diastereoisomer, isotopically enriched analogs, solvates, prodrugs, or polymorphs thereof as claimed in any one of claims 1-3, wherein

$R_a$ represents halogen or cyano, and
$R_b$ represents the following formula:

$$R_{b2}\text{-}X_5\text{-}L_3\text{-},$$

wherein $L_3$ represents

linear or branched $C_{2-60}$ alkylene optionally substituted with one or more substituents selected from the group consisting of D, $C_{1-4}$ alkyl, halogen, $C_{3-6}$ cycloalkyl or any combination thereof, or
optionally substituted linear or branched $C_{2-60}$ alkylene with one or more groups $R_{16}$ and/or one or more groups $R_{17}$ and/or any combination of one or more groups $R_{16}$ and $R_{17}$ inserted into its backbone carbon chain, wherein carbon-carbon bond between one or more pairs of adjacent carbon atoms in the backbone carbon chain is interrupted by the group $R_{16}$, $R_{17}$, or a combination of $R_{16}$ and $R_{17}$; wherein

each $R_{16}$ is independently selected from the group consisting of O, S, $N(R_{18})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein $R_{18}$ represents H or $C_{1-3}$ alkyl, and in case that two or more groups $R_{16}$ are inserted into the backbone carbon chain of the linear or branched $C_{2-60}$ alkylene group, the two or more groups $R_{16}$ are not directly connected to each other; and wherein each $R_{17}$ is independently selected from the group consisting of $C_{3-15}$ cycloalkylene, 4- to 15-membered heterocyclylene, $C_{6-10}$ arylene, 5- to 15-membered heteroarylene, $C_{2-6}$ alkenylene, $C_{2-6}$ alkynylene or any combination thereof, wherein the $C_{3-15}$ cycloalkylene, the 4- to 15-membered heterocyclylene, $C_{6-10}$ arylene and the 5- to 15-membered heteroarylene are each independently optionally substituted with a substituent selected from the group consisting of D, optionally deuterated $C_{1-4}$ alkyl, optionally deuterated $C_{3-6}$ cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the linear or branched $C_{2-60}$ alkylene is optionally substituted with one or more substituents selected from the group consisting of D, $C_{1-4}$ alkyl, halogen, $C_{3-6}$ cycloalkyl or any combination thereof;

$X_5$ represents $N(R_{19})$, C(O)O, OC(O), C(O)NH, NHC(O), C(O), O, C(S), S, S(O), $S(O)_2$, $S(O)_2NH$, $NHS(O)_2$, $S(O)_2O$, or $OS(O)_2$, wherein $R_{19}$ represents H or $C_{1-3}$ alkyl, or $X_5$ represents a bond; and

$R_{b2}$ represents optionally substituted $C_{3-20}$ cycloalkyl, optionally substituted $C_{6-20}$ aryl, optionally substituted 4- to 20-membered heterocyclyl or optionally substituted 5- to 20-membered heteroaryl, wherein the $C_{3-20}$ cycloalkyl, the $C_{6-20}$ aryl, the 4- to 20-membered heterocyclyl, and the 5- to 20-membered heteroaryl are each independently optionally substituted with one or more substituents selected from the group consisting of D, optionally deuterated $C_{1-4}$ alkyl, optionally substituted $C_{3-15}$ cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$ cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

$R_{b2}$ represents the following formula:

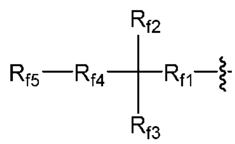

,

wherein $R_{f1}$, $R_{f2}$, $R_{f3}$, $R_{f4}$ and $R_{f5}$ are as defined in claim 1.

**12.** The compound of Formula (I) or salts, enantiomers, diastereoisomer, isotopically enriched analogs, solvates, prodrugs, or polymorphs thereof as claimed in claim 4, wherein

$X_1$ represents optionally substituted $C_{3-20}$ cycloalkylene, optionally substituted 4- to 20-membered nitrogen-containing monocyclic heterocyclylene, optionally substituted 5- to 20-membered nitrogen-containing bridged heterocyclylene, optionally substituted 5- to 20-membered nitrogen-containing spiro-heterocyclylene, optionally substituted 5- to 20-membered nitrogen-containing fused heterocyclylene, optionally substituted $C_{6-10}$ arylene, or optionally substituted 5- to 20-membered heteroarylene, wherein the $C_{3-20}$ cycloalkylene, the 4- to 20-membered nitrogen-containing monocyclic heterocyclylene, the 5- to 20-membered nitrogen-containing bridged heterocyclylene, the 5- to 20-membered nitrogen-containing spiro-heterocyclylene, the 5- to 20-membered nitrogen-containing fused heterocyclylene, the $C_{6-10}$ arylene, and the 5- to 20-membered heteroarylene are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

$R_{a1}$ represents $NR_{d4}R_{d5}$, $C(O)NR_{d6}R_{d7}$, NHC(O)Ras, $NHC(O)NR_{d9}R_{a10}$, $C(O)OR_{d11}$, $OC(O)R_{d12}$, $OR_{d13}$, $SR_{d14}$, $C(O)R_{d15}$, $C(S)R_{d16}$, $S(O)R_{d17}$, $S(O)_2R_{d18}$, $S(O)_2NHR_{d19}$, $NHS(O)_2R_{d20}$, $S(O)_2OR_{d21}$, $OS(O)_2R_{d22}$, optionally substituted linear or branched $C_{1-10}$ alkyl, optionally substituted $C_{3-20}$ cycloalkyl, optionally substituted

4- to 20-membered heterocyclyl, optionally substituted $C_{6-20}$ aryl or optionally substituted 5- to 20-membered heteroaryl, wherein the linear or branched $C_{1-10}$ alkyl, the $C_{3-20}$ cycloalkyl, the 4- to 20-membered heterocyclyl, the $C_{6-20}$ aryl or the 5- to 20-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$ cycloalkyl, optionally substituted $C_{6-10}$ aryl, optionally substituted 4- to 15-membered heterocyclyl, optionally substituted 5- to 15-membered heteroaryl or any combination thereof;

wherein $R_{d4}$, $R_{d5}$, $R_{d6}$, $R_{d7}$, $R_{d9}$, $R_{d10}$, $R_{d11}$, $R_{d13}$, $R_{d14}$, $R_{d19}$ and $K_{d21}$ each independently represent H, optionally substituted linear or branched $C_{1-10}$ alkyl, optionally substituted $C_{3-20}$ cycloalkyl, optionally substituted $C_{6-20}$ aryl, optionally substituted 4- to 20-membered heterocyclyl or optionally substituted 5- to 20-membered heteroaryl, wherein the linear or branched $C_{1-10}$ alkyl, the $C_{3-20}$ cycloalkyl, the 4- to 20-membered heterocyclyl, the $C_{6-20}$ aryl or the 5- to 20-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$ cycloalkyl, optionally substituted $C_{6-10}$ aryl, optionally substituted 4- to 15-membered heterocyclyl, optionally substituted 5- to 15-membered heteroaryl or any combination thereof; and

$R_{d8}$, $R_{d12}$, $R_{d15}$, $R_{d16}$, $R_{d17}$, $R_{d18}$, $R_{d20}$ and $R_{d22}$ each independently represent optionally substituted linear or branched $C_{1-10}$ alkyl, optionally substituted $C_{3-20}$ cycloalkyl, optionally substituted 4- to 20-membered heterocyclyl, optionally substituted $C_{6-20}$ aryl or optionally substituted 5- to 20-membered heteroaryl, wherein the linear or branched $C_{1-10}$ alkyl, the $C_{3-20}$ cycloalkyl, the 4- to 20-membered heterocyclyl, the $C_{6-20}$ aryl or the 5- to 20-membered heteroaryl are each independently optionally substituted with one or more substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, optionally substituted $C_{3-15}$ cycloalkyl, optionally substituted $C_{6-10}$ aryl, optionally substituted 4- to 15-membered heterocyclyl, optionally substituted 5- to 15-membered heteroaryl or any combination thereof;

preferably, wherein $X_1$ represents:

cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, bornylene, bicyclo[2.2.1]heptanylene, 2-oxobicyclo[2.2.1]heptanylene or bicyclo[2.2.1]heptenylene, with each group being optionally substituted with one or more substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof;

phenylene or naphthylene, wherein the phenylene or naphthylene is optionally substituted with one or more substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof;

azetidinylene, pyrrolidinylene, imidazolidylene, pyrazolidylene, oxazolidinylene, thiazolidinylene, piperidinylene, piperazinylene, morpholinylene, thiomorpholinylene, azepanylene, azacyclooctylene, diazacycloheptanylene, diazacyclooctylene, 3-azabicyclo[3.1.0]hexylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octylene, 3,8-diazabicyclo[3.2.1]octylene, 2,5-diazabicyclo[2.2.2]octylene, 3-azaspiro[5.5]undecylene or 7-azaspiro[3.5]nonylene, with each group being optionally substituted with one or more substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof; or

furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene,

quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pynmidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene, and imidazo[2,1-b]thiazolylene, with each group being optionally substituted with one or more substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof;
or

$R_{a1}$ represents $NR_{d4}R_{d5}$, $C(O)NR_{d6}R_{d7}$, $NHC(O)R_{d8}$, $NHC(O)NR_{d9}R_{d10}$, $C(O)OR_{d11}$, $OC(O)R_{d12}$, $OR_{d13}$, $SR_{d14}$, $C(O)R_{d15}$, $C(S)R_{d16}$, $S(O)R_{d17}$, $S(O)_2R_{d18}$, $S(O)_2NHR_{d19}$, $NHS(O)_2R_{d20}$, $S(O)_2OR_{d21}$, or $OS(O)_2R_{d22}$,

wherein $R_{d4}$, $R_{d5}$, $R_{d6}$, $R_{d7}$, $R_{d9}$, $R_{d10}$, $R_{d11}$, $R_{d13}$, $R_{d14}$, $R_{d19}$ and $R_{d21}$ are the same or different and each independently represent:

H, or
methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl or decyl, with each group being optionally substituted with one or more substituents selected from the group consisting of D, optionally deuterated $C_{1-4}$ alkyl, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$ cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$ cycloalkyl, optionally substituted $C_{6-10}$ aryl, optionally substituted 4- to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or
cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bomyl, bicyclo[2.2.1]heptanyl, or bicyclo[2.2.1]heptenyl, with each group being optionally substituted with one or more substituents selected from the group consisting of D, optionally deuterated $C_{1-4}$ alkyl, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$ cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$ cycloalkyl, optionally substituted $C_{6-10}$ aryl, optionally substituted 4- to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or
phenyl or naphthyl, wherein the phenyl or naphthyl is optionally substituted with one or more substituents selected from the group consisting of optionally deuterated $C_{1-4}$ alkyl, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$ cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$ cycloalkyl, optionally substituted $C_{6-10}$ aryl, optionally substituted 4- to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl, diazacyclooctyl, 3-azabicyclo[3. 1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, or azaspirocycloalkyl (e.g., 3-azaspiro[5.5]undecyl or 7-azaspiro[3.5]nonyl), with each group being optionally substituted with one or more substituents selected from the group consisting of D, optionally

deuterated $C_{1-4}$ alkyl, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$ cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$ cycloalkyl, optionally substituted $C_{6-10}$ aryl, optionally substituted 4- to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof;

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl or imidazo[2,1-b]thiazolyl, with each group being optionally substituted with one or more substituents selected from the group consisting of D, optionally deuterated $C_{1-4}$ alkyl, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$ cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$ cycloalkyl, optionally substituted $C_{6-10}$ aryl, optionally substituted 4- to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; and

$R_{d8}$, $R_{d12}$, $R_{d15}$, $R_{d16}$, $R_{d17}$, $R_{d18}$, $R_{d20}$ and $R_{d22}$ each independently represent:

methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl or decyl, with each group being optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$ cycloalkyl, optionally substituted $C_{6-10}$ aryl, optionally substituted 4- to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, or bicyclo[2.2.1]heptenyl, with each group being optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4-to 15-membered heterocyclyl, optionally substituted 5- to 15-membered heteroaryl or any combination thereof, wherein the optionally substituted $C_{3-15}$ cycloalkyl, optionally substituted $C_{6-10}$ aryl, optionally substituted 4- to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

phenyl or naphthyl, wherein the phenyl or naphthyl is optionally substituted with one or more substituents selected from the group consisting of halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$ cycloalkyl, optionally substituted $C_{6-10}$ aryl, optionally substituted 4- to 15-membered

heterocyclyl, optionally substituted 5- to 15-membered heteroaryl or any combination thereof, wherein the optionally substituted $C_{3-15}$ cycloalkyl, optionally substituted $C_{6-10}$ aryl, optionally substituted 4- to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl, diazacyclooctyl, 3-azabicyclo[3. 1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, or azaspirocycloalkyl (e.g., 3-azaspiro[5.5]undecyl or 7-azaspiro[3.5]nonyl), with each group being optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$ cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof;

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl or imidazo[2,1-b]thiazolyl, with each group being optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$ cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$ cycloalkyl, optionally substituted $C_{6-10}$ aryl, optionally substituted 4- to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof;

or

$R_{a1}$ represents:

methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl or decyl, with each group being optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$ cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$ cycloalkyl, optionally substituted $C_{6-10}$ aryl, optionally substituted 4- to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, or bicyclo[2.2.1]heptenyl, with each group being optionally substituted with one or more substituents selected from the group consisting of D, halogen, hydroxy, cyano,

amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$ cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$ cycloalkyl, optionally substituted $C_{6-10}$ aryl, optionally substituted 4- to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

phenyl or naphthyl, wherein the phenyl or naphthyl is optionally substituted with one or more substituents selected from the group consisting of halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$ cycloalkyl, optionally substituted $C_{6-10}$ aryl, optionally substituted 4- to 15-membered heterocyclyl, optionally substituted 5- to 15-membered heteroaryl or any combination thereof, wherein the optionally substituted $C_{3-15}$ cycloalkyl, optionally substituted $C_{6-10}$ aryl, optionally substituted 4- to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, or azaspirocycloalkyl (e.g., 3-azaspiro[5.5]undecyl or 7-azaspiro[3.5]nonyl), with each group being optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5-to 15-membered heteroaryl or any combination thereof, wherein the optionally substituted $C_{3-15}$cycloalkyl, optionally substituted $C_{6-10}$ aryl, optionally substituted 4-to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl or imidazo[2,1-b]thiazolyl, with each group being optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, optionally substituted $C_{3-15}$cycloalkyl, optionally substituted $C_{6-10}$ aryl, optionally substituted 4- to 15-membered heterocyclyl, optionally substituted 5-to 15-membered heteroaryl or any combination thereof, wherein the optionally substituted $C_{3-15}$cycloalkyl, optionally substituted $C_{6-10}$ aryl, optionally substituted 4- to 15-membered heterocyclyl and optionally substituted 5- to 15-membered heteroaryl are each independently optionally substituted with one or more substituents selected from the group consisting of D, halogen, hydroxy, cyano, amino, mercapto, $C_{1-4}$ alkoxy, $C_{1-4}$ alkyl-NH-, halogenated $C_{1-4}$ alkyl, $NH_2$-$C_{1-4}$ alkylene or any combination thereof.

**13.** The compound of Formula (I) or salts, enantiomers, diastereoisomer, isotopically enriched analogs, solvates, prodrugs, or polymorphs thereof as claimed in claim 5, wherein

(i) $X_1$ represents a bond, or $X_1$ represents $N(R_{e1})$, O, S, $C_{2-6}$ alkynylene, $C_{2-6}$ alkenylene, optionally substituted $C_{3-20}$cycloalkylene, optionally substituted 4- to 20-membered heterocyclylene, optionally substituted $C_{6-15}$ arylene, or optionally substituted 5- to 20-membered heteroarylene, wherein $R_{e1}$ represents H or $C_{1-3}$ alkyl, wherein the $C_{3-20}$ cycloalkylene, the 4- to 20-membered heterocyclylene, the $C_{6-15}$ arylene, and the 5- to 20-membered heteroarylene are each independently optionally substituted with one or more substituents selected

from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

preferably, wherein $X_1$ represents a bond, or
$X_1$ represents $N(R_{e1})$, O, S, $C_{2-6}$ alkynylene or $C_{2-6}$ alkenylene, wherein $R_{e1}$ represents H or $C_{1-3}$ alkyl; or
$X_1$ represents:

cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro [3.3]heptylene, spiro[2.5]octylene, spiro[3.5]nonylene, spiro[4.4]nonylene, spiro[4.5]decylene, spiro [5.5]undecylene, p-menthanylene, m-menthanylene, quinuclidinylene, adamantanylene, noradamantanylene, bornylene, bicyclo[2.2.1]heptanylene, 2-oxobicyclo[2.2.1]heptanylene or bicyclo[2.2.1]heptenylene, with each group being optionally substituted with one or more substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof;
phenylene or naphthylene, wherein the phenylene or naphthylene is optionally substituted with one or more substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof;
azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, azacycloheptanylene, azacyclooctylene, diazacycloheptanylene, diazacyclooctylene, 3-azabicyclo[3.1.0] hexylene, 6-azabicyclo[3.1.1]heptanylene, 2,5-diazabicyclo[2.2.1]heptanyl, 3,6-diazabicyclo[3.1.1] heptanyl, 3-azabicyclo[3.2.1]octylene, 3,8-diazabicyclo[3.2.1]octylene, 2,5-diazabicyclo[2.2.2]octylene, 3-azaspiro[5.5]undecylene or 7-azaspiro[3.5]nonylene, with each group being optionally substituted with one or more substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof; or
furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo [1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene or imidazo[2,1-b]thiazolylene, with each group being optionally substituted with one or more substituents selected from the group consisting of D, optionally deuterated $C_{1-4}$ alkyl, halogenated $C_{1-4}$ alkyl, hydroxy, amino, mercapto, halogen, cyano, $C_{1-4}$ alkoxy, $C_{1-4}$ alkyl-NH-, $NH_2$-$C_{1-4}$ alkylene, $C_{1-4}$ alkyl-NHC(O)-, $C_{1-4}$ alkyl-C(O)NH-, or any combination thereof;
or

(ii) $R_{a1}$ represents the following formula:

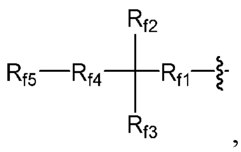
,

wherein $R_{f1}$ represents:

$-NCH_2CH_2N-$;

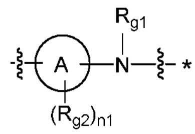

,

wherein symbol * indicates the point of attachment to $X_2$, $R_{g1}$ represents H or $C_{1-3}$ alkyl, ring A represents: cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, bornylene, bicyclo[2.2.1]heptanylene, 2-oxobicyclo[2.2.1]heptanylene, bicyclo[2.2.1]heptenylene, azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, azacycloheptanylene, azacyclooctylene, diazacycloheptanylene, diazacyclooctylene, 3-azabicyclo[3.1.0]hexylene, 6-azabicyclo[3.1.1]heptanylene, 2,5-diazabicyclo[2.2.1]heptanyl, 3,6-diazabicyclo[3.1.1]heptanyl, 3-azabicyclo[3.2.1]octylene, 3,8-diazabicyclo[3.2.1]octylene, 2,5-diazabicyclo[2.2.2]octylene, 3-azaspiro[5.5]undecylene or 7-azaspiro[3.5]nonylene, and
$(R_{g2})_{n1}$ indicates that ring A is optionally substituted with n1 $R_{g2}$, n1 represents an integer of 0 to 8, and each $R_{g2}$, the same or different from each other, is independently selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

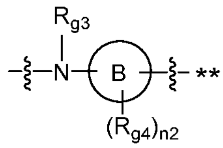

,

wherein symbol ** indicates the point of attachment to $X_2$, $R_{g3}$ represents H or $C_{1-3}$ alkyl, ring B represents: cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, bornylene, bicyclo[2.2.1]heptanylene, 2-oxobicyclo[2.2.1]heptanylene, bicyclo[2.2.1]heptenylene, azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, azacycloheptanylene, azacyclooctylene, diazacycloheptanylene, diazacyclooctylene, 3-azabicyclo[3.1.0]hexylene, 6-azabicyclo[3.1.1]heptanylene, 2,5-diazabicyclo[2.2.1]heptanyl, 3,6-diazabicyclo[3.1.1]heptanyl, 3-azabicyclo[3.2.1]octylene, 3,8-diazabicyclo[3.2.1]octylene, 2,5-diazabicyclo[2.2.2]octylene, 3-azaspiro[5.5]undecylene or 7-azaspiro[3.5]nonylene, and
$(R_{g4})_{n2}$ indicates that ring B is optionally substituted with n2 $R_{g4}$, n2 represents an integer of 0 to 8, and each $R_{g4}$, the same or different from each other, is independently selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof; or

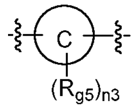

,

wherein ring C represents: cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, bornylene, bicyclo[2.2.1]heptanylene, 2-oxobicyclo[2.2.1]heptanylene, bicyclo[2.2.1]heptenylene, azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, azacycloheptanylene, azacyclooctylene, diazacycloheptanylene, diazacyclooctylene, 3-azabicyclo[3.1.0]hexylene, 6-azabicyclo[3.1.1]heptanylene, 2,5-diazabicyclo[2.2.1]heptanyl, 3,6-diazabicyclo[3.1.1]heptanyl, 3-azabicyclo[3.2.1]octylene, 3,8-diazabicyclo[3.2.1]octylene, 2,5-diazabicyclo[2.2.2]octylene, 3-azaspiro[5.5]undecylene or 7-azaspiro[3.5]nonylene, and $(R_{g5})_{n3}$ indicates that ring C is optionally substituted with n3 $R_{g5}$, n3 represents an integer of 0 to 8, and each $R_{g5}$, the same or different from each other, is independently selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;

$R_{f2}$ represents H, halogen, $C_{1-3}$ alkyl or halogenated $C_{1-3}$ alkyl;
$R_{f3}$ represents H, halogen, $C_{1-3}$ alkyl, halogenated $C_{1-3}$ alkyl,

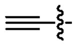 , 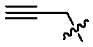 , 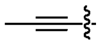

or

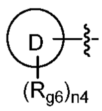 ,

wherein ring D represents: cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, bornylene, bicyclo[2.2.1]heptanylene, 2-oxobicyclo[2.2.1]heptanylene, bicyclo[2.2.1]heptenylene, azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, azacycloheptanylene, azacyclooctylene, diazacycloheptanylene, diazacyclooctylene, 3-azabicyclo[3.1.0]hexylene, 6-azabicyclo[3.1.1]heptanylene, 2,5-diazabicyclo[2.2.1]heptanyl, 3,6-diazabicyclo[3.1.1]heptanyl, 3-azabicyclo[3.2.1]octylene, 3,8-diazabicyclo[3.2.1]octylene, 2,5-diazabicyclo[2.2.2]octylene, 3-azaspiro[5.5]undecylene, 7-azaspiro[3.5]nonylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene or imidazo[2,1-b]thiazolylene, and $(R_{g6})_{n4}$ indicates that ring D is optionally substituted with n4 $R_{g6}$, n4 represents an integer of 0 to 8, and each $R_{g6}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof;
$R_{f4}$ represents

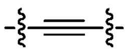, 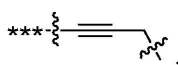, 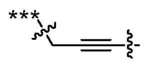 or 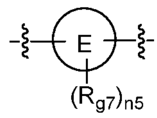,

wherein symbol *** indicates the point of attachment to $R_{f5}$, ring E represents: cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro[3.3]heptylene, spiro[2.5]octylene, spiro[3.5]nonylene, spiro[4.4]nonylene, spiro[4.5]decylene, spiro[5.5]undecylene, p-menthanylene, m-menthanylene, quinuclidinylene, adamantanylene, noradamantanylene, bornylene, bicyclo[2.2.1]heptanylene, 2-oxobicyclo[2.2.1]heptanylene, bicyclo[2.2.1]heptenylene, azetidinylene, oxetanylene, pyrrolidinylene, imidazolidylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, azacycloheptanylene, azacyclooctylene, diazacycloheptanylene, diazacyclooctylene, 3-azabicyclo[3.1.0]hexylene, 6-azabicyclo[3.1.1]heptanylene, 2,5-diazabicyclo[2.2.1]heptanyl, 3,6-diazabicyclo[3.1.1]heptanyl, 3-azabicyclo[3.2.1]octylene, 3,8-diazabicyclo[3.2.1]octylene, 2,5-diazabicyclo[2.2.2]octylene, 3-azaspiro[5.5]undecylene, 7-azaspiro[3.5]nonylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene or imidazo[2,1-b]thiazolylene, and

$(R_{g7})_{n5}$ indicates that ring E is optionally substituted with n5 $R_{g7}$, n5 represents an integer of 0 to 8, and each $R_{g7}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof; and $R_{f5}$ represents H, halogen, $C_{1-3}$ alkyl, halogenated $C_{1-3}$ alkyl,

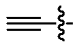, 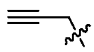, 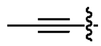

or

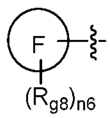,

wherein ring F represents: cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, 2-oxobicyclo[2.2.1]heptanyl, bicyclo[2.2.1]heptenyl, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, 3,8-diazabicyclo[3.2.1]octyl, 2,5-diazabicyclo[2.2.2]octyl, 3-azaspiro[5.5]undecyl, 7-azaspiro[3.5]nonyl, phenyl, naphthyl, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl

or imidazo[2,1-b]thiazolyl, and
$(R_{g8})_{n6}$ indicates that ring F is optionally substituted with n6 $R_{g8}$, n6 represents an integer of 0 to 8, and each $R_{g8}$, the same or different from each other, is independently selected from the group consisting of optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, halogenated $C_{1-6}$ alkyl, $NH_2$-$C_{1-6}$ alkylene or any combination thereof.

**14.** The compound of Formula (I) or salts, enantiomers, diastereoisomer, isotopically enriched analogs, solvates, prodrugs, or polymorphs thereof as claimed in claim 6, wherein $R_{a1}$ represents:

hydroxy, wherein when $R_{a1}$ represents hydroxy, $X_2$ represents a bond; or
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, adamantan-10-yl, noradamantan-1-yl, noradamantan-2-yl, noradamantan-3-yl, noradamantan-4-yl, noradamantan-5-yl, noradamantan-6-yl, noradamantan-7-yl, noradamantan-8-yl, noradamantan-9-yl, cubanyl, $C_{5-15}$ spirocycloalkyl, bicyclo[2.2.2]octan-1-yl, bicyclo[2.2.2]octan-2-yl, 1,7,7-trimethylbicyclo[2.2.1]hept-2-yl, 1,7,7-trimethylbicyclo[2.2.1]hept-3-yl, 1,7,7-trimethylbicyclo[2.2.1]hept-4-yl, 1,7,7-trimethylbicyclo[2.2. 1]hept-5-yl, 1,7,7-trimethylbicyclo[2.2. 1]hept-6-yl,

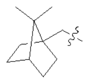 , 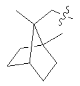 ,

bicyclo[2.2.1]hept-2-yl, bicyclo[2.2.1]hept-3-yl, bicyclo[2.2.1]hept-4-yl, bicyclo[2.2.1]hept-5-yl, bicyclo[2.2.1]hept-6-yl, bicyclo[2.2.1]hept-5-en-2-yl, bicyclo[2.2.1]hept-5-en-3-yl, bicyclo[2.2.1]hept-5-en-7-yl, 7,7-dimethyl-2-oxobicyclo[2.2.1]hept-1-yl, 7,7-dimethyl-2-oxobicyclo[2.2.1]hept-3-yl, 7,7-dimethyl-2-oxobicyclo[2.2.1]hept-4-yl, 7,7-dimethyl-2-oxobicyclo[2.2.1]hept-5-yl, 7,7-dimethyl-2-oxobicyclo[2.2.1]hept-6-yl, p-menthanyl or m-menthanyl, with each group being independently optionally substituted with one or more substituents selected from the group consisting of D, halogen, optionally deuterated $C_{1-4}$ alkyl, hydroxy, amino, mercapto, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

**15.** The compound of Formula (I) or salts, enantiomers, diastereoisomer, isotopically enriched analogs, solvates, prodrugs, or polymorphs thereof as claimed in claim 7 or 8, wherein $R_{a1}$ represents:
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl, diazacyclooctyl, 1,3-diazacycloheptanyl, 1,4-diazacycloheptanyl, 4,5-diazacycloheptanyl, 5- to 15-memberedbridged heterocyclyl (e.g., 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, diazabicyclo[3.2.1]octyl, diazabicyclo[2.2.2]octyl, 3,8-diazabicyclo[3.2.1]octan-3-yl, and 2,5-diazabicyclo[2.2.2]octan-2-yl), or 5- to 15-memberedazaspirocycloalkyl (e.g., 3-azaspiro[5.5]undecylene and 7-azaspiro[3.5]nonylene), with each group being independently optionally substituted with one or more substituents selected from the group consisting of: D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, wherein the $C_{3-15}$cycloalkyl, the 4-to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

**16.** The compound of Formula (I) or salts, enantiomers, diastereoisomer, isotopically enriched analogs, solvates, prodrugs, or polymorphs thereof as claimed in claim 9, wherein $R_{a1}$ represents:
$R_{a1}$ represents hydroxy, and $X_2$ represents a bond; or $R_{a1}$ represents:

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl, diazacyclooctyl, 1,3-diazacycloheptanyl, 1,4-diazacycloheptanyl, 4,5-diazacycloheptanyl, 5- to 15-memberedbridged heterocyclyl (e.g., 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo[3.2.1]octyl, diazabicyclo[3.2.1]octyl, diazabicyclo[2.2.2]octyl, 3,8-diazabicyclo[3.2.1]octan-3-yl, and 2,5-diazabicyclo[2.2.2]octan-2-yl), or 5- to 15-memberedazaspirocycloalkyl (e.g., 3-azaspiro[5.5]undecylene and 7-azaspiro[3.5]nonylene), with each group being independently optionally substituted with one or more substituents selected from the group consisting of: D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, wherein the $C_{3-15}$cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bomyl, bicyclo[2.2.1]heptanyl, 2-oxobicyclo[2.2.1]heptanyl or bicyclo[2.2.1]heptenyl, with each group being independently optionally substituted with one or more substituents selected from the group consisting of: D, optionally deuterated $C_{1-4}$ alkyl, optionally substituted $C_{3-}$iscycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, wherein the $C_{3-15}$cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

**17.** The compound of Formula (I) or salts, enantiomers, diastereoisomer, isotopically enriched analogs, solvates, prodrugs, or polymorphs thereof as claimed in claim 10, wherein $R_{a2}$ represents:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, 2-oxobicyclo[2.2.1]heptanyl or bicyclo[2.2.1]heptenyl, with each group being optionally substituted with one or more substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the $C_{3-15}$cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo [3.2.1]octyl, 3,8-diazabicyclo[3.2.1]octyl, 2,5-diazabicyclo[2.2.2]octyl, 3-azaspiro[5.5]undecyl or 7-azaspiro [3.5]nonyl, with each group being optionally substituted with one or more substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the $C_{3-15}$cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

phenyl or naphthyl, wherein the phenyl or naphthyl is optionally substituted with one or more substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the $C_{3-15}$cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl and imidazo[2,1-b]thiazolyl, with each group being optionally substituted with one or more substituents selected from the group consisting of D, optionally deuterated $C_{1-4}$ alkyl, optionally substituted $C_{3-15}$cycloalkyl, optionally substituted 4-to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and the $C_{3-15}$cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof.

**18.** The compound of Formula (I) or salts, enantiomers, diastereoisomer, isotopically enriched analogs, solvates, prodrugs, or polymorphs thereof as claimed in claim 4, wherein

$X_1$ represents:

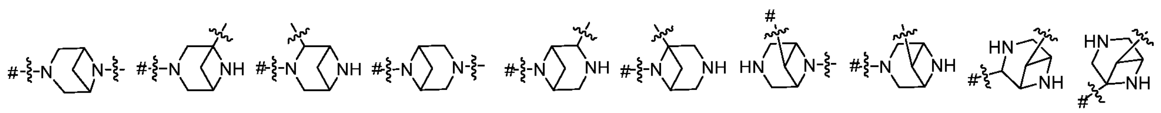

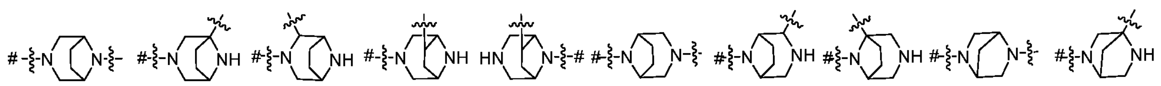

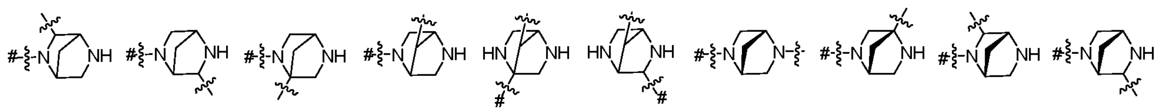

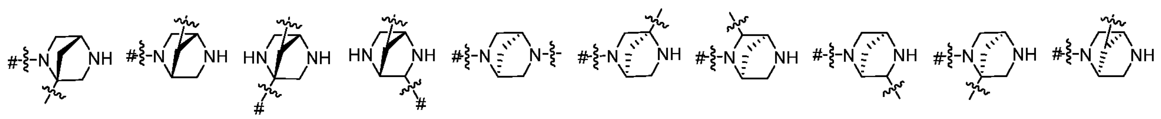

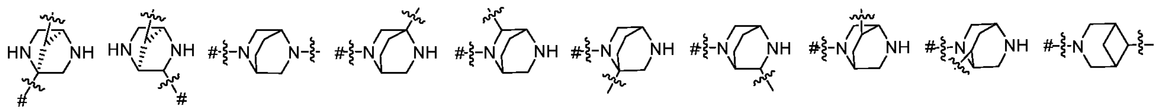

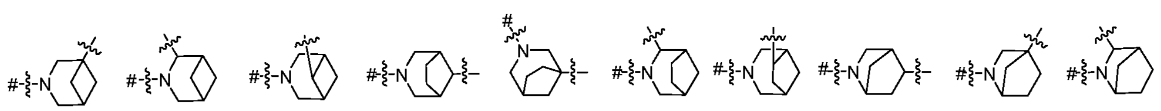

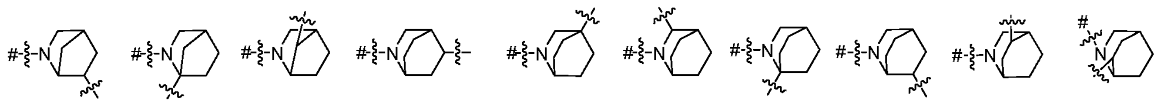

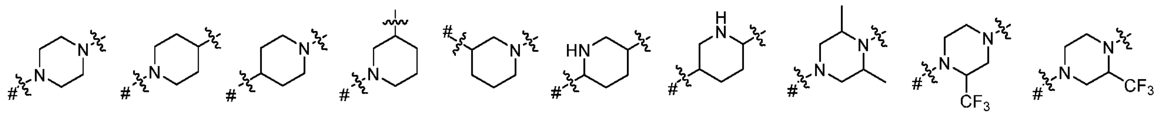

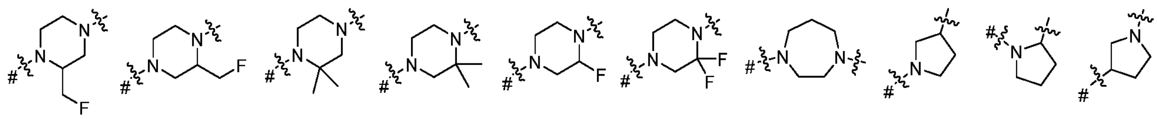

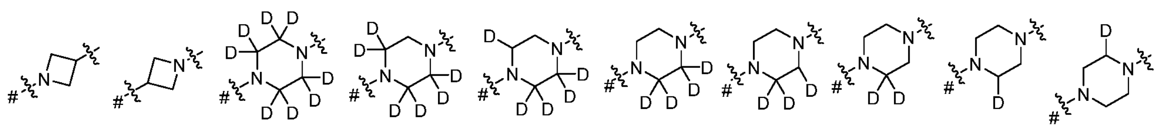

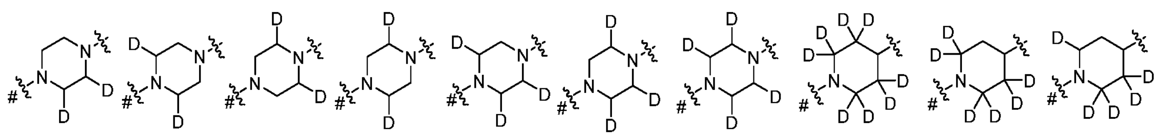

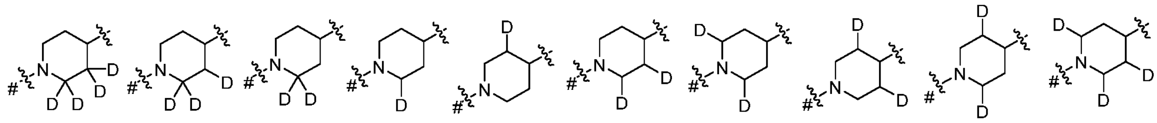

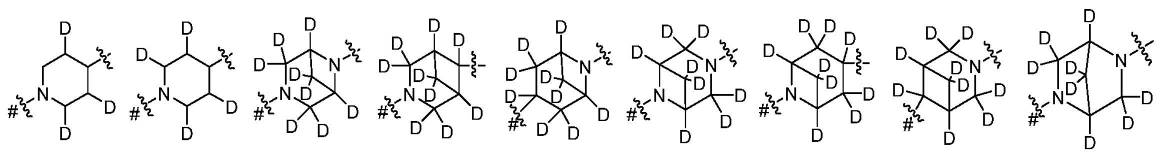

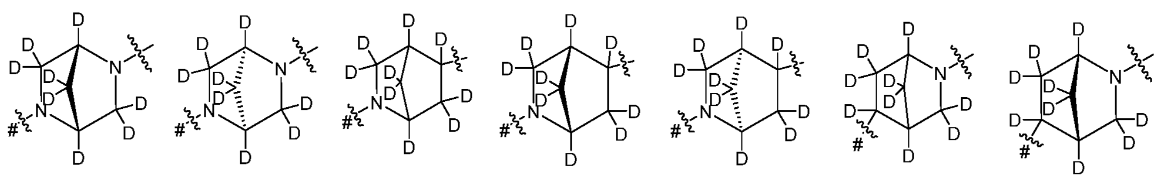

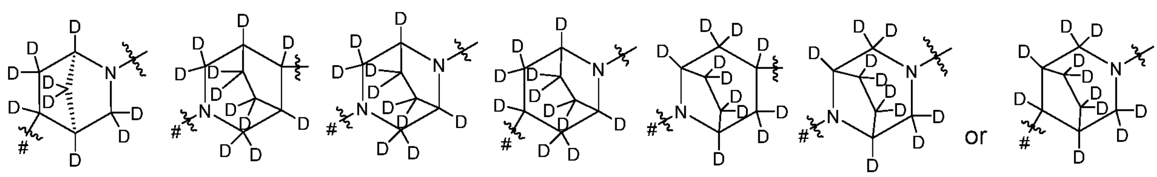

wherein symbol # indicates the point of attachment to $L_1$;
and/or
$R_{a1}$ represents hydroxy, or $R_{a1}$ represents:

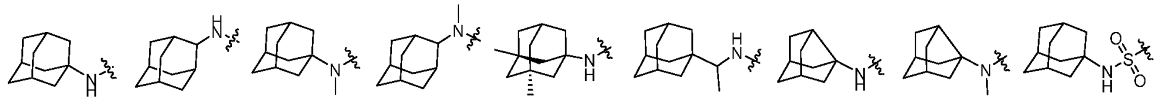

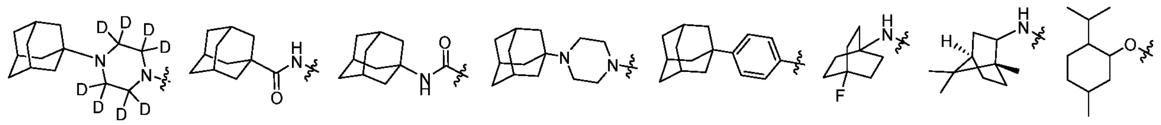

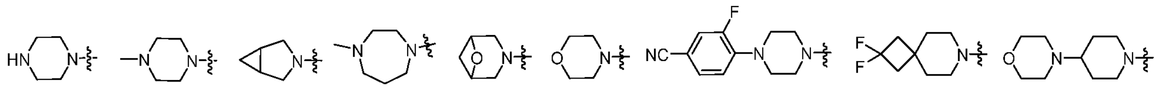

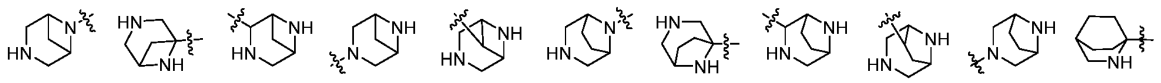

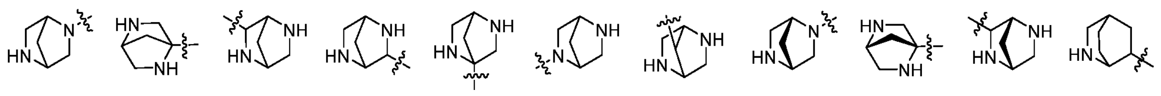

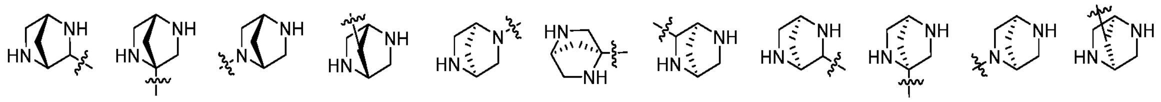

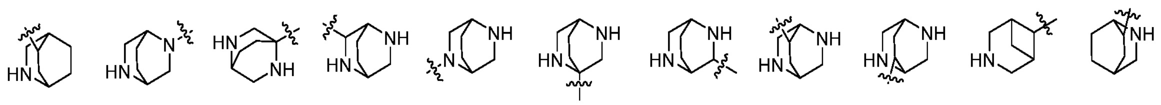

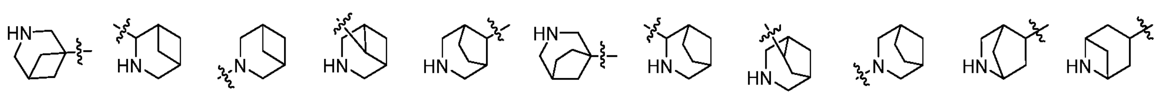

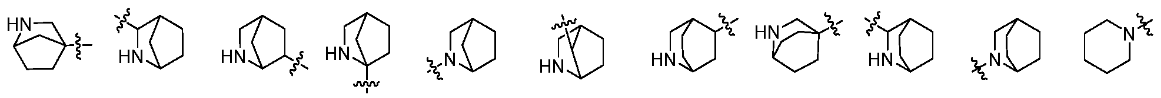

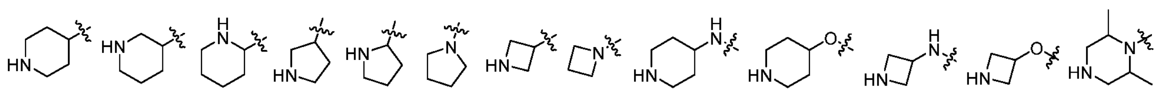

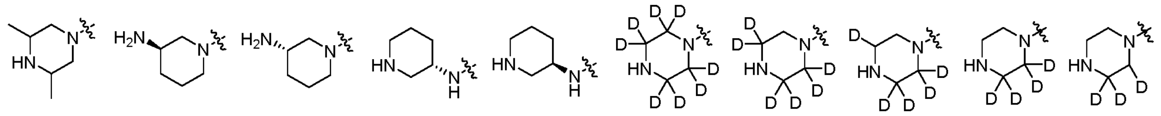

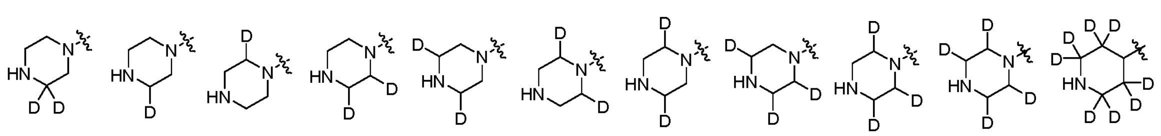

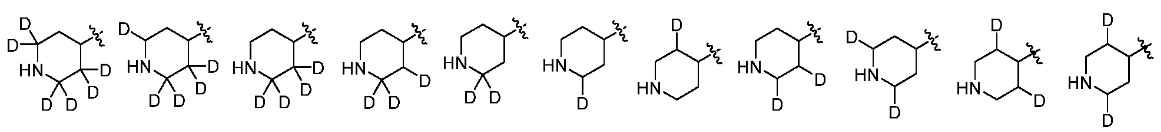

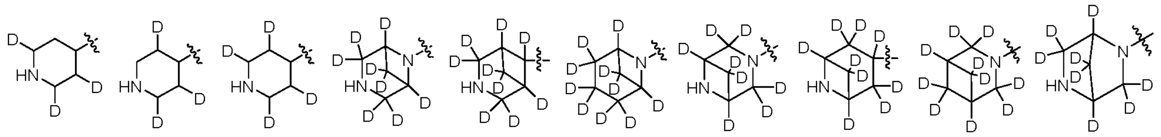

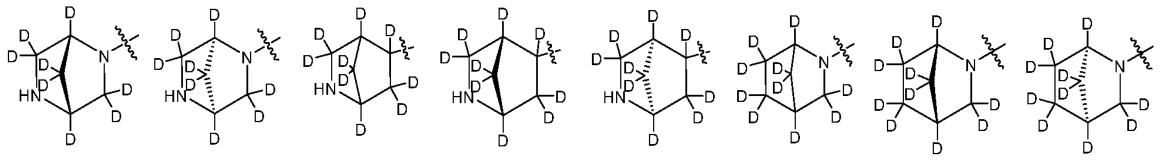

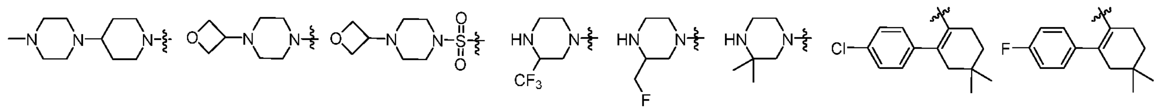

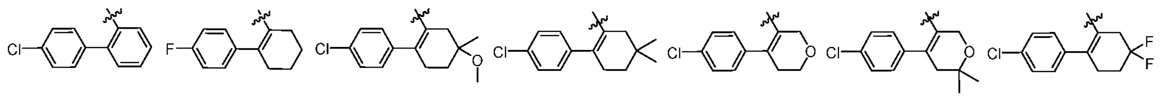

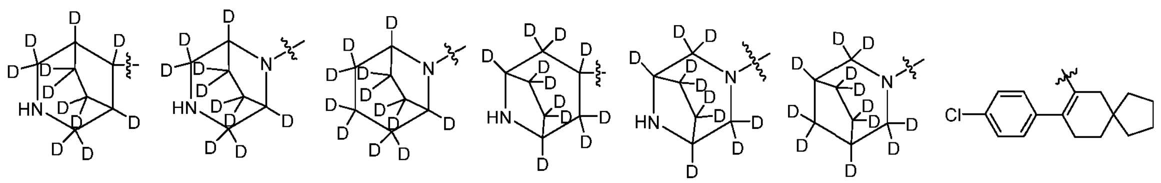

or

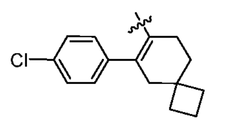

**19.** The compound of Formula (I) or salts, enantiomers, diastereoisomer, isotopically enriched analogs, solvates, prodrugs, or polymorphs thereof as claimed in claim 5, wherein

$X_1$ represents a bond, or $X_1$ represents NH, $N(CH_3)$, O, S, ethynylene or vinylene, or
$X_1$ represents:

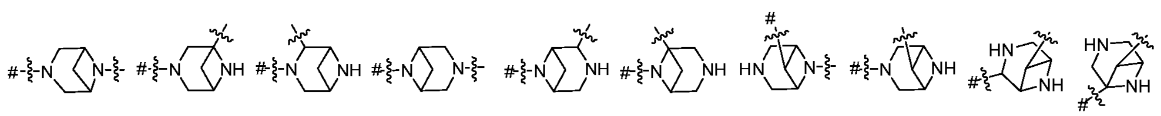

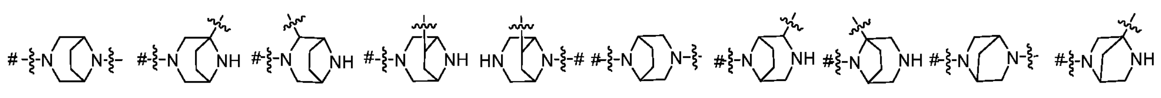

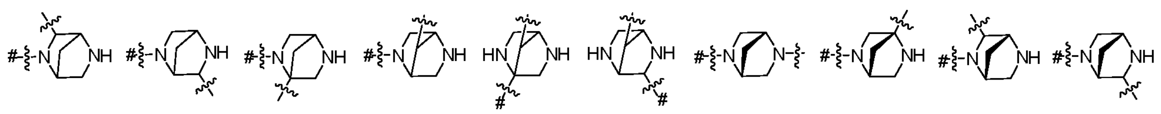

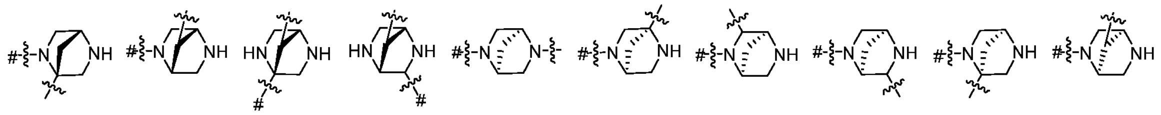

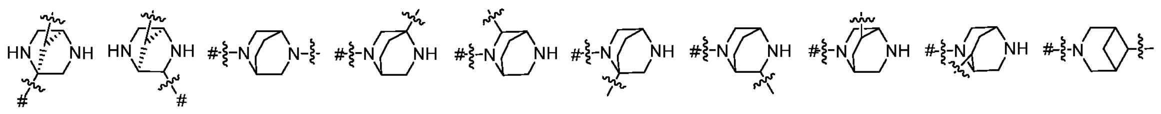

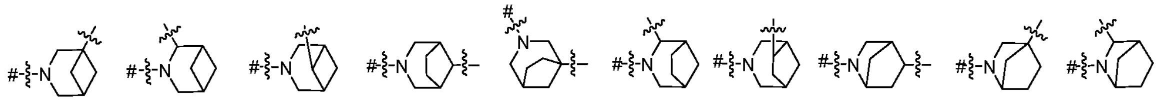

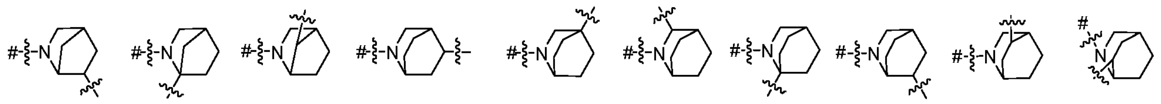

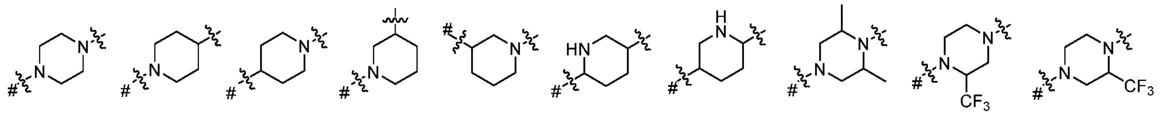

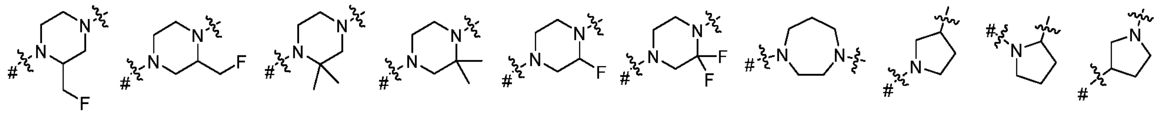

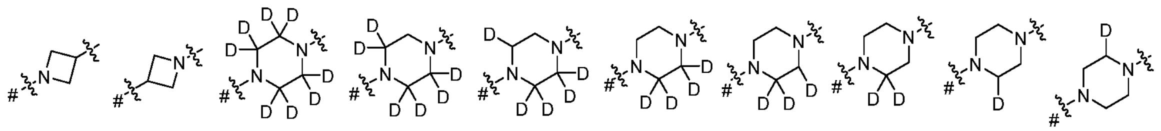

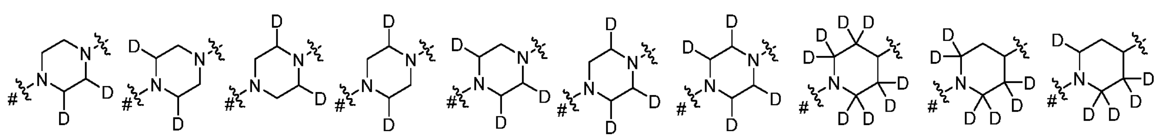

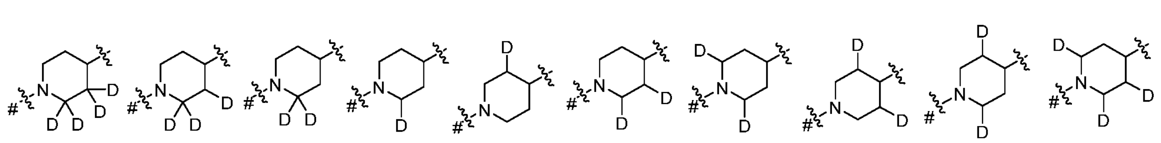

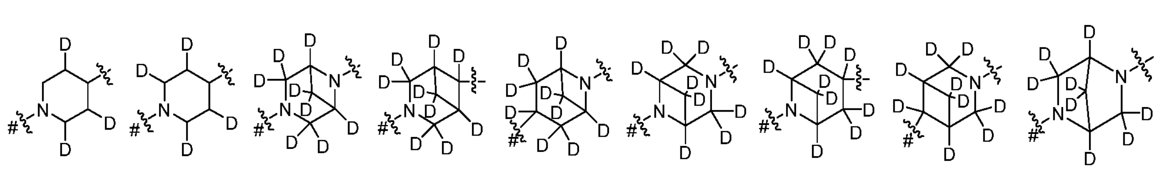

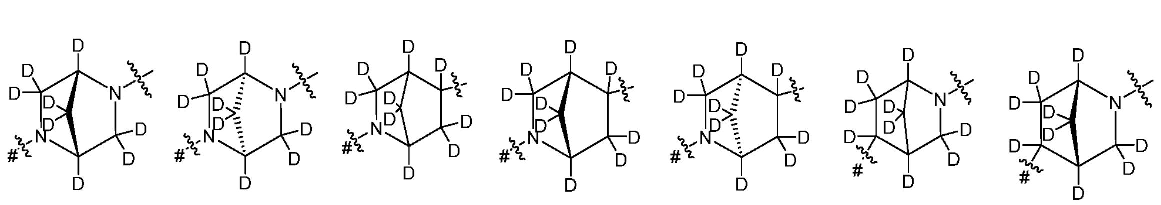

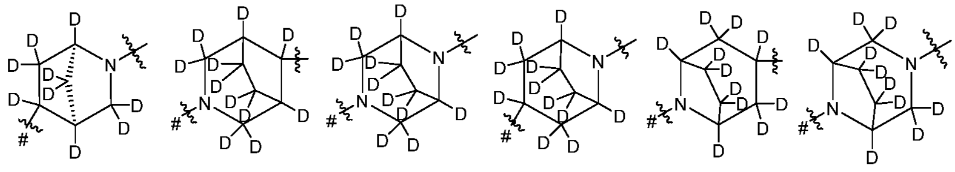 or 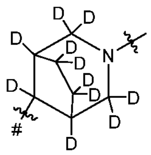

wherein symbol # indicates the point of attachment to $L_1$;
and/or
$R_{a1}$ represents:

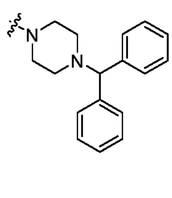 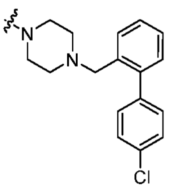 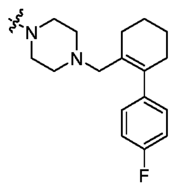 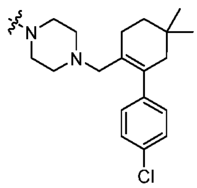 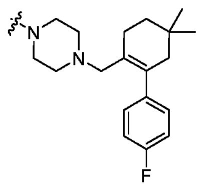 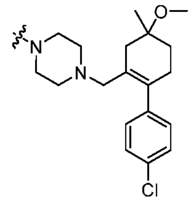

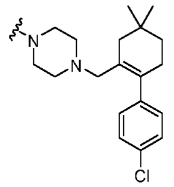 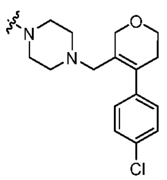 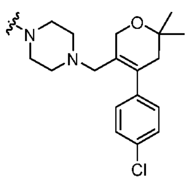 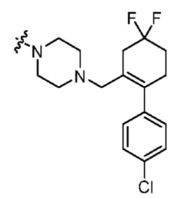 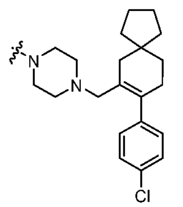 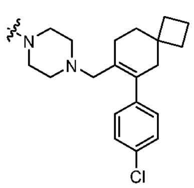

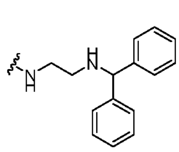 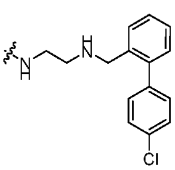 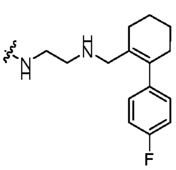 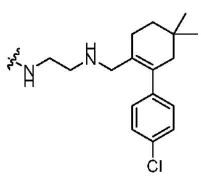 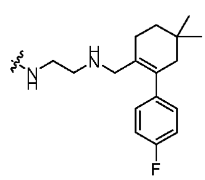 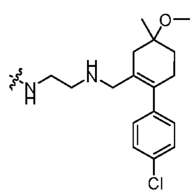

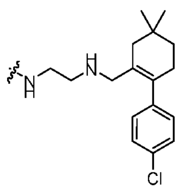 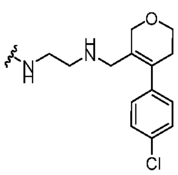 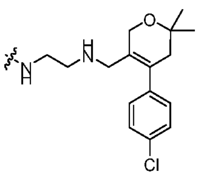 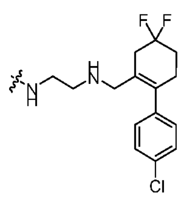 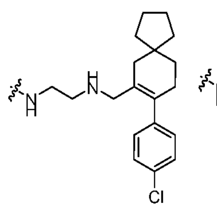 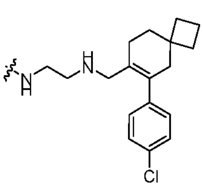

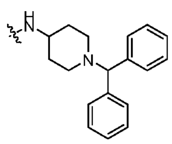 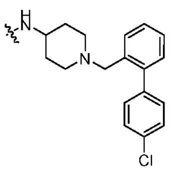 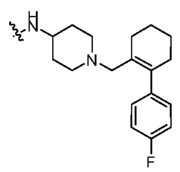 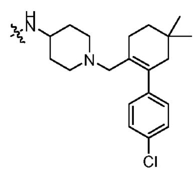 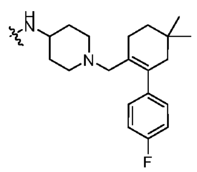 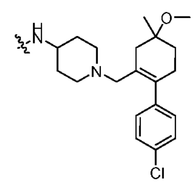

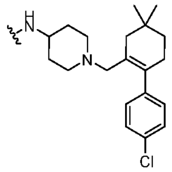 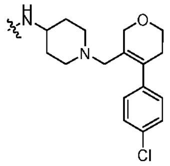 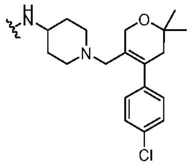 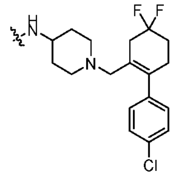 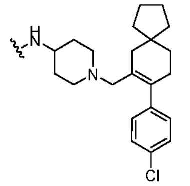 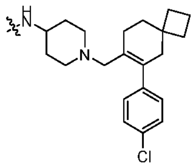

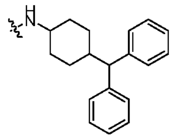 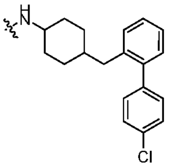 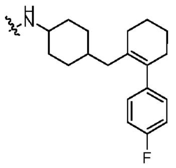 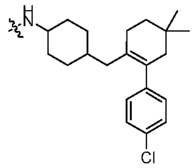 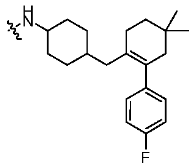 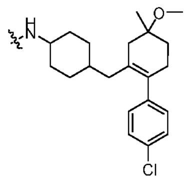

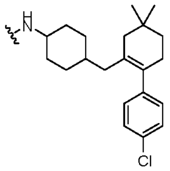 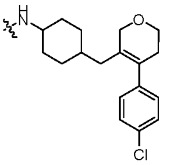 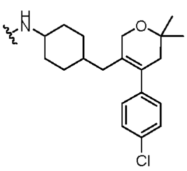 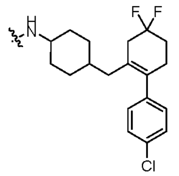 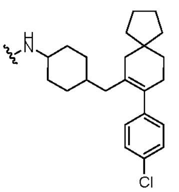 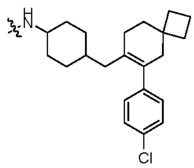

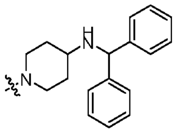 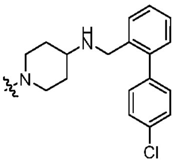 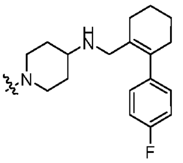 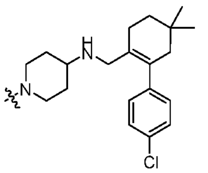 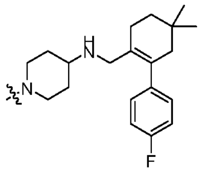 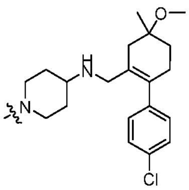

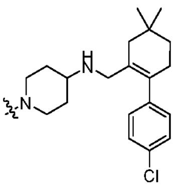 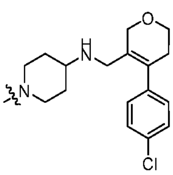 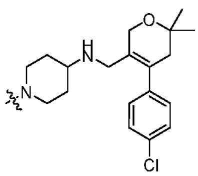 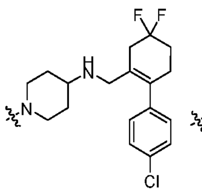 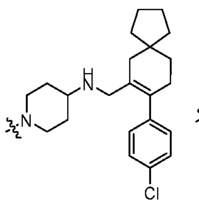 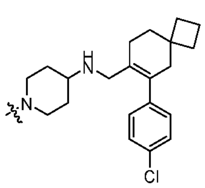

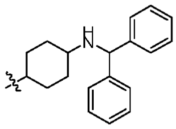 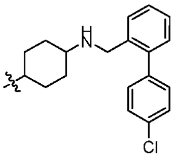 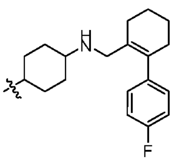 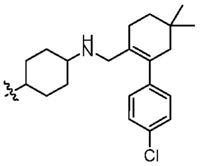 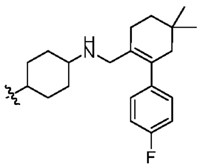 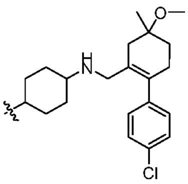

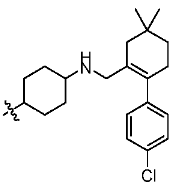 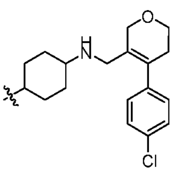 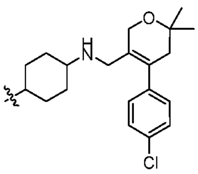 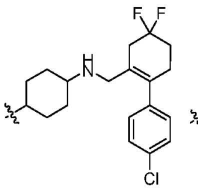 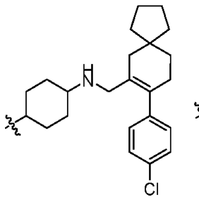 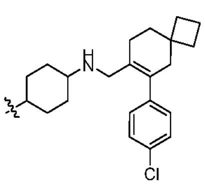

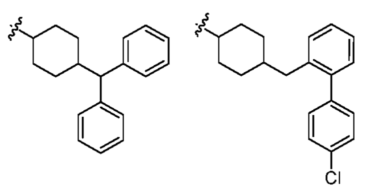 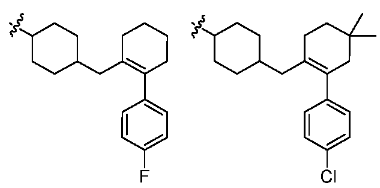 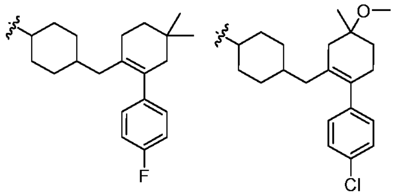

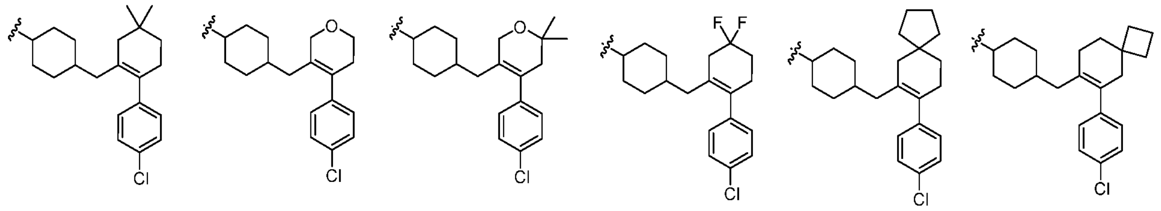

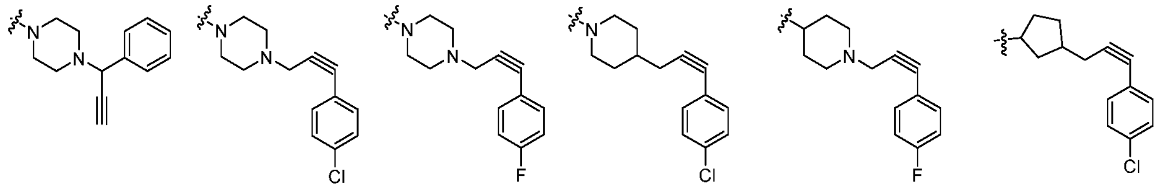

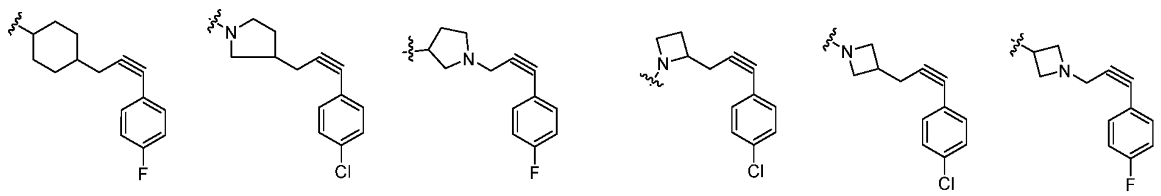

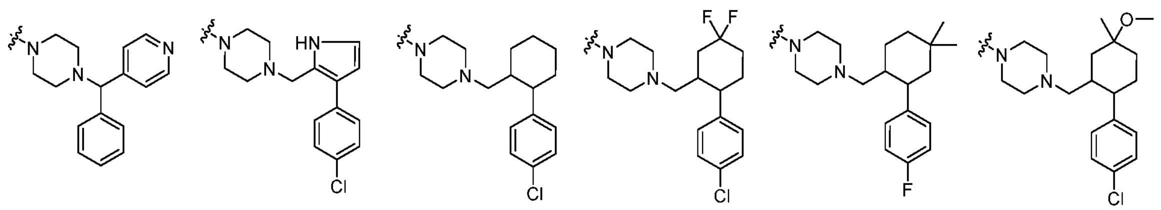

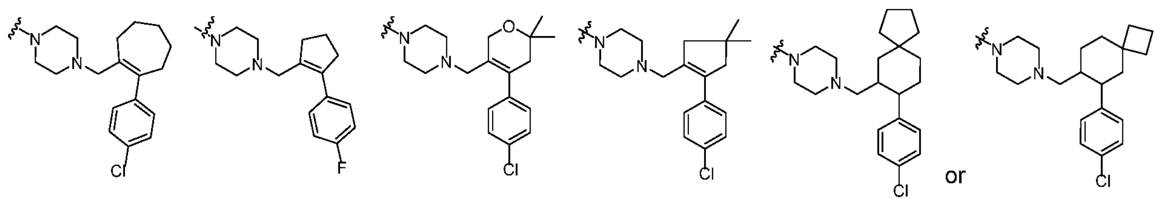

or

20. The compound of Formula (I) or salts, enantiomers, diastereoisomer, isotopically enriched analogs, solvates, prodrugs, or polymorphs thereof as claimed in claim 6, wherein

$R_{a1}$ represents hydroxy, and $X_2$ represents a bond; or
$R_{a1}$ represents:

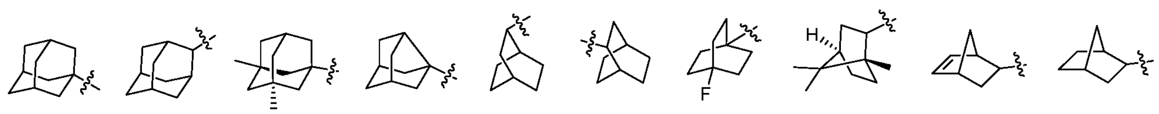

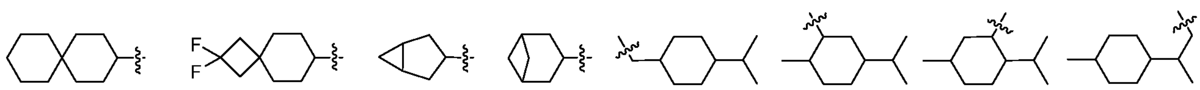

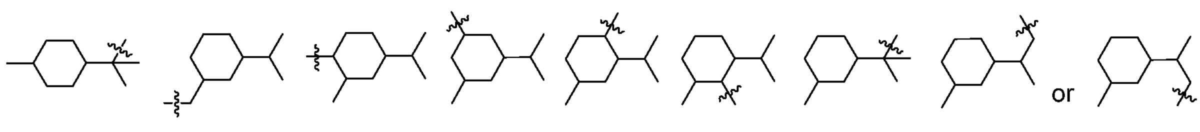

or

**21.** The compound of Formula (I) or salts, enantiomers, diastereoisomer, isotopically enriched analogs, solvates, prodrugs, or polymorphs thereof as claimed in claim 7 or 8, wherein $R_{a1}$ represents:

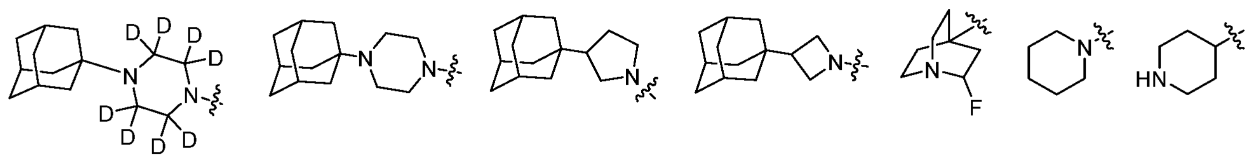

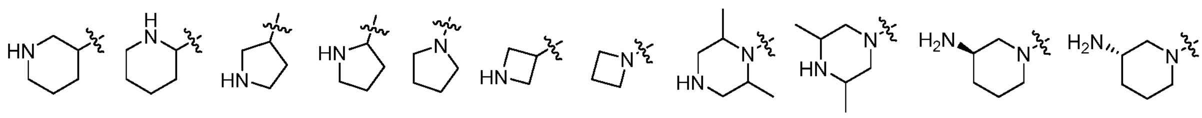

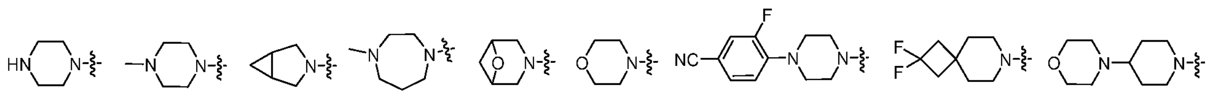

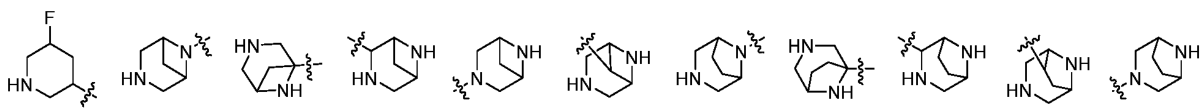

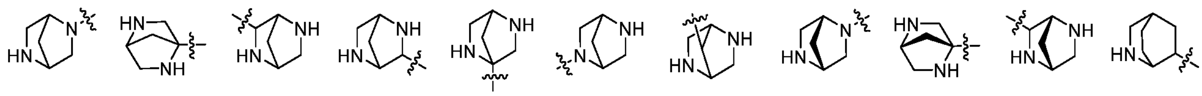

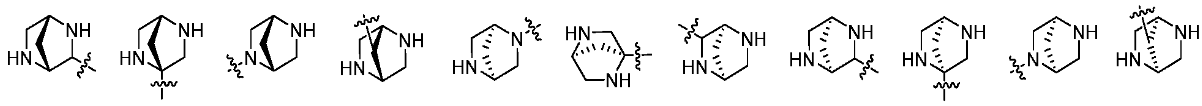

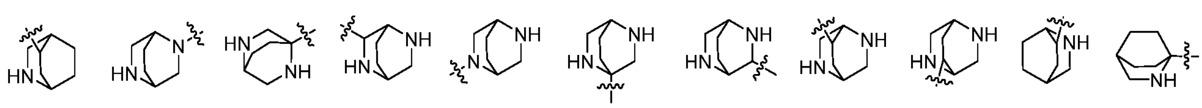

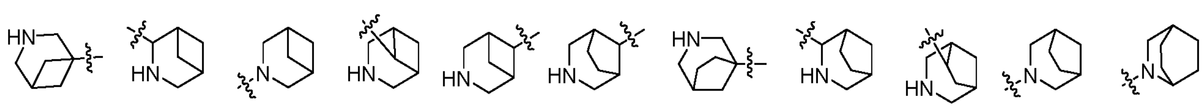

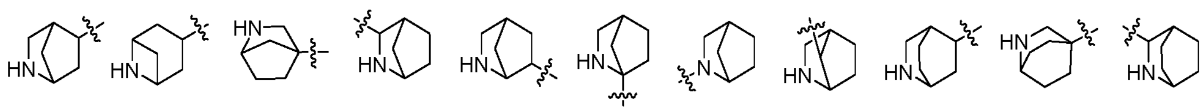

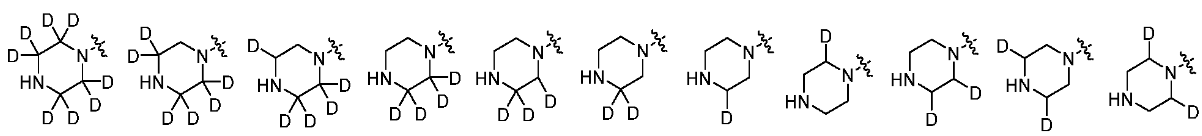

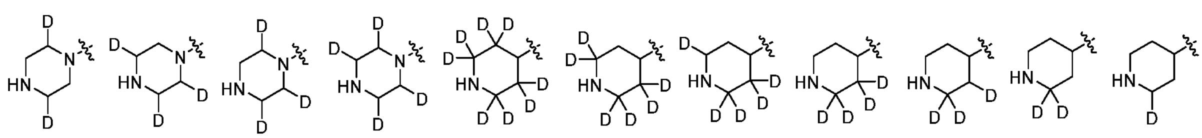

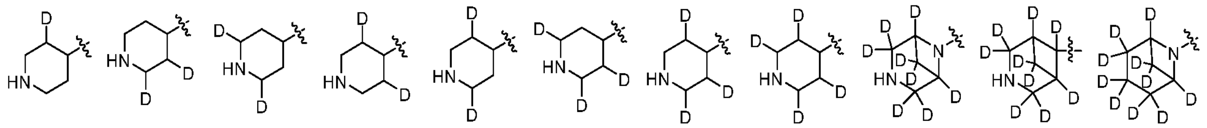

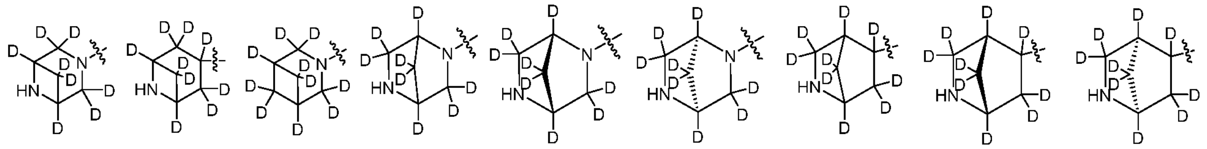

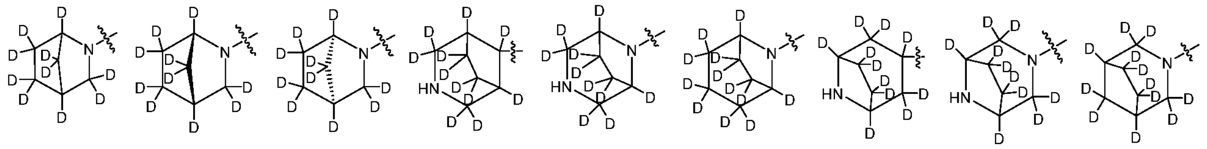

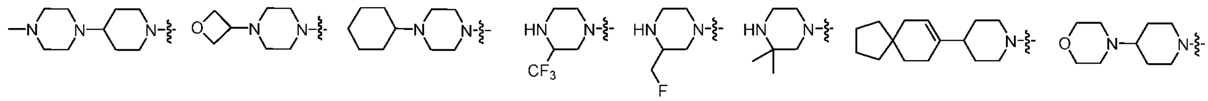

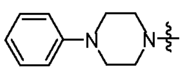 or 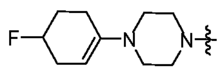

**22.** The compound of Formula (I) or salts, enantiomers, diastereoisomer, isotopically enriched analogs, solvates, prodrugs, or polymorphs thereof as claimed in claim 9, wherein
$R_{a1}$ represents:

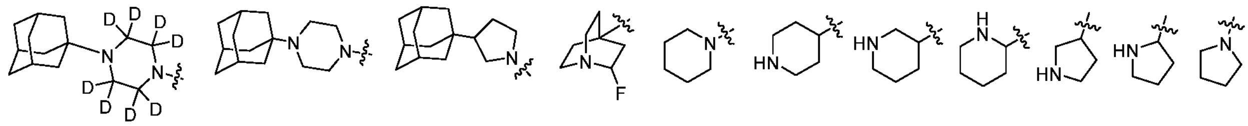

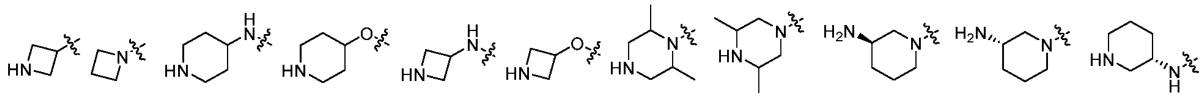

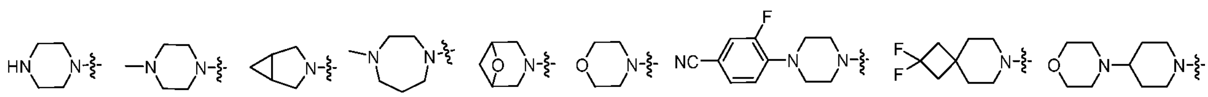

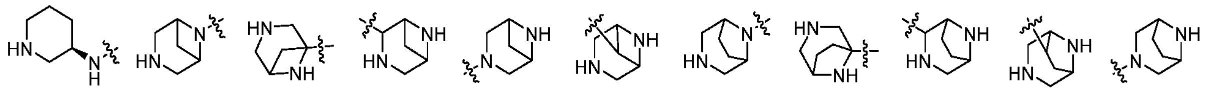

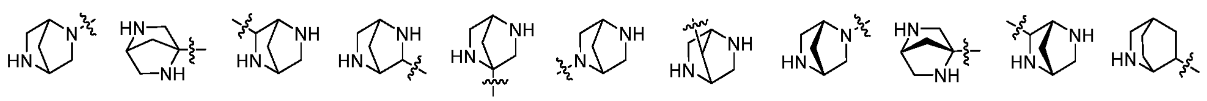

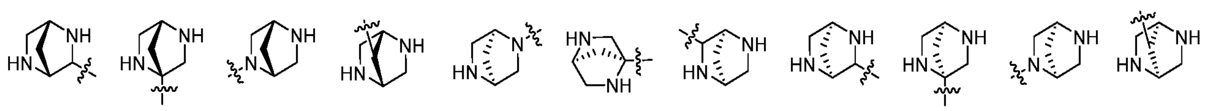

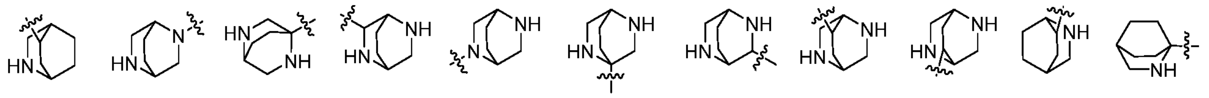

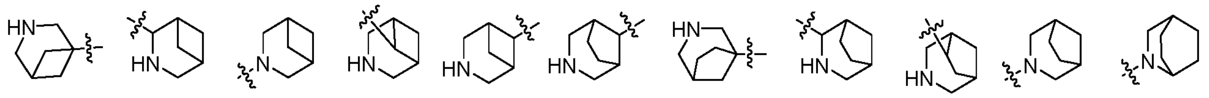

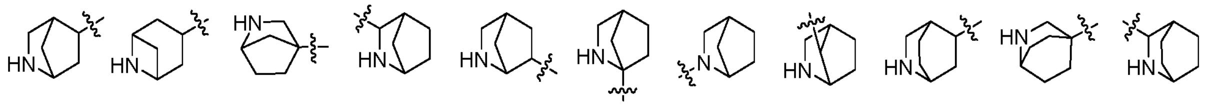

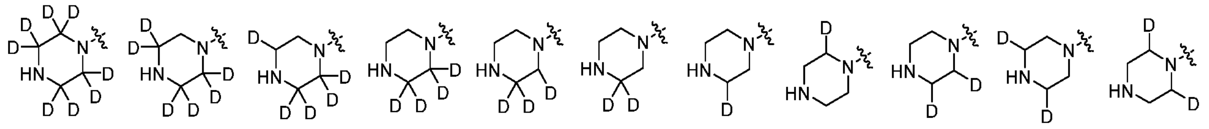

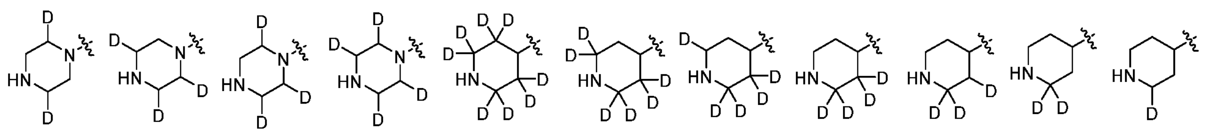

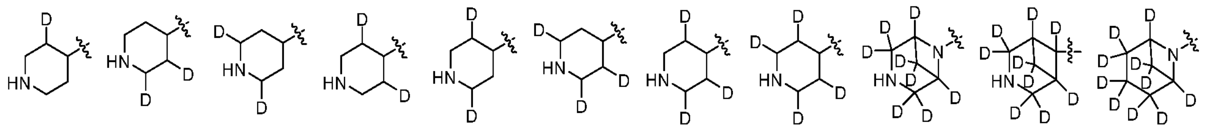

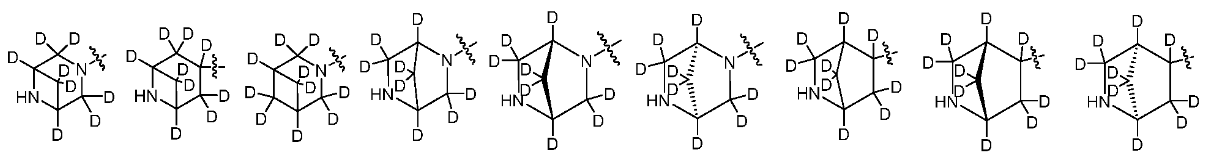

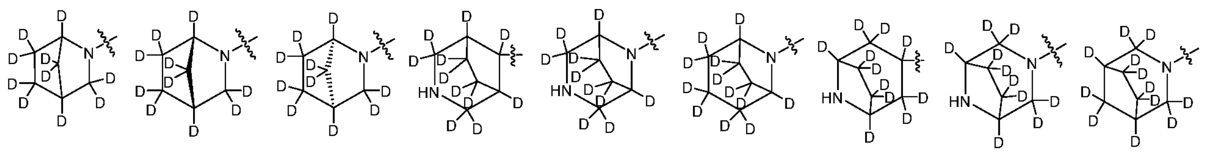

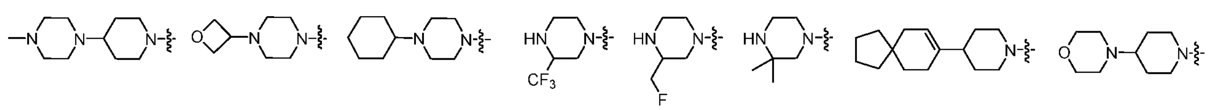

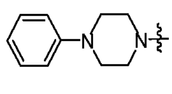 or 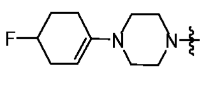

23. The compound of Formula (I) or salts, enantiomers, diastereoisomer, isotopically enriched analogs, solvates, prodrugs, or polymorphs thereof as claimed in claim 10, wherein $R_{a2}$-$X_3$- represents:

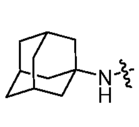 or 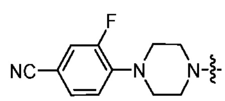

**24.** The compound of Formula (I) or salts, enantiomers, diastereoisomer, isotopically enriched analogs, solvates, prodrugs, or polymorphs thereof as claimed in claim 4, wherein $L_1$ represents the following formula:

$$\#\#\text{-}(C(R^{a1})(R^{a2}))_{m1}\text{-}(R_{d1}(C(R^{a3})(R^{a4}))_{m2})_{p1\text{-}};$$

$$\#\#\text{-}(C(R^{a1})(R^{a2}))_{m1}\text{-}(R_{d1}(C(R^{a3})(R^{a4}))_{m2})_{p1}\text{-}(R_{d1}(C(R^{a5})(R^{a6}))_{m3})_{p2}\text{-};$$

$$\#\#\text{-}(C(R^{a1})(R^{a2}))_{m1}\text{-}(R_{d1}(C(R^{a3})(R^{a4}))_{m2})_{p1}\text{-}(R_{d1}(C(R^{a5})(R^{a6}))_{m3})_{p2}\text{-}(R_{d1}(C(R^{a7})(R^{a8}))_{m4})_{p3}\text{-};$$

$$\#\#\text{-}(C(R^{a1})(R^{a2}))_{m1}\text{-}(R_{d2}(CXR^{a3})(R^{a4}))_{m2})_{p1}\text{-};$$

$$\#\#\text{-}(C(R^{a1})(R^{a2}))_{m1}\text{-}(R_{d2}(C(R^{a3})(R^{a4}))_{m2})_{p1}\text{-}(R_{d2}(C(R^{a5})(R^{a6}))_{m3})_{p2}\text{-};$$

$$\#\#\text{-}((C(R^{a1})(R^{a2}))_{m1}\text{-}(R_{d2}(C(R^{a3})(R^{a4}))_{m2})_{p1}\text{-}(R_{d2}(C(R^{a5})(R^{a6}))_{m3})_{p2}\text{-}(R_{d2}(C\ (R^{a7})(R^{a8}))_{m4})_{p3}\text{-};$$

$$\#\#\text{-}(C(R^{a1})(R^{a2}))_{m1}\text{-}(R_{d1}\text{-}R_{d2}\text{-}(C(R^{a3})(R^{a4}))_{m2})_{p1}\text{-};$$

$$\#\#\text{-}(C(R^{a1})(R^{a2}))_{m1}\text{-}(R_{d1}(C(R^{a3})(R^{a4}))_{m2})_{p1}\text{-}(R_{d2}(C(R^{a5})(R^{a6}))_{m3})_{p2}\text{-};$$

$$\#\#\text{-}(C(R^{a1})(R^{a2}))_{m1}\text{-}(R_{d2}\text{-}R_{d1}\text{-}(C(R^{a3})(R^{a4}))_{m2})_{p1}\text{-};\ \text{or}$$

$$\#\#\text{-}(C(R^{a1})(R^{a2}))_{m1}\text{-}(R_{d2}(C(R^{a3})(R^{a4}))_{m2})_{p1}\text{-}(R_{d1}(C(R^{a5})(R^{a6}))_{m3})_{p2}\text{-};$$

wherein each $R_{d1}$ is independently selected from the group consisting of O, S, $N(R_{d3})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_{d3}$ independently represents H or $C_{1-3}$ alkyl; and each group $R_{d2}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH- or any combination thereof;
$R^{a1}$, $R^{a2}$, $R^{a3}$, $R^{a4}$, $R^{a5}$, $R^{a6}$, $R^{a7}$ and $R^{a8}$ each independently represent H, deuterium, hydroxy, amino, mercapto, nitro, halogen, cyano, optionally deuterated $C_{1-6}$ alkyl, halogenated $C_{1-6}$ alkyl, optionally deuterated $C_{3-6}$cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof;
symbol ## indicates the point of attachment to $X_1$; and
m1, m2, m3, m4, p1, p2, p3 are each independently selected from the group consisting of an integer of 1, 2, 3, 4, 5, 6, 7, 8, 9, 10, 11, 12, 13, 14, or 15.

**25.** The compound of Formula (I) or salts, enantiomers, diastereoisomer, isotopically enriched analogs, solvates, prodrugs, or polymorphs thereof as claimed in claim 5, wherein Li represents the structure of the following formula:

$$\#\#\text{-}(C(R^{a1})(R^{a2}))_{m1}\text{-}(R_{e2}(C(R^{a3})(R^{a4}))_{m2})_{p1}\text{-};$$

$$\#\#\text{-}(C(R^{a1})(R^{a2}))_{m1}\text{-}(R_{e2}(C(R^{a3})(R^{a4}))_{m2})_{p1}\text{-}(R_{e2}(C(R^{a5})(R^{a6}))_{m3})_{p2}\text{-};$$

$$\#\#(C(R^{a1})(R^{a2}))_{m1}\text{-}(R_{e2}(C(R^{a3})(R^{a4})))_{m2})_{p1\text{-}}(R_{e2}(C(R^{a5}))m3)_{p2}\text{-}(R_{e2}(C(R^{a7})(R^{a8}))_{m4})_{p3}\text{-};$$

$$\#\#\text{-}(C(R^{a1})(R^{a2}))_{m1}\text{-}(R_{e3}(C(R^{a3})(R^{a4}))_{m2})_{p1}\text{-};$$

$$\#\#\text{-}(C(R^{a1})(R^{a2}))_{m1}\text{-}(R_{e3}(C(R^{a3})(R^{a4}))_{m2})_{p1}\text{-}(R_{e3}(C(R^{a5})(R^{a6}))_{m3})_{p2}\text{-};$$

$$\#\#\text{-}(C(R^{a1})(R^{a2}))_{m1}(R_{e3}(C(R^{a3})(R^{a4}))_{m2})_{p1}\text{-}(R_{e3}(C(R^{a5})(R^{a6}))_{m3})_{p2}(R_{e3}(C(R^{a7})(R^{a8}))_{m4})_{p3}\text{-};$$

$$\#\#\text{-}(C(R^{a1})(R^{a2}))_{m1}\text{-}(R_{e2}\text{-}Re_{3}\text{-}(C(R^{a3})(R^{a4}))_{m2})_{p1}\text{-};$$

$$\#\#\text{-}(C(R^{a1}\text{-}(R^{a2}))_{m1}\text{-}(R_{e2}(C(R^{a3})(R^{a4}))_{m2})_{p1}\text{-}(R_{e3}(C(R^{a5})(R^{a6}))_{m3})_{p2}\text{-};$$

$$\#\#\text{-}(C(R^{a1}\text{-}(R^{a2}))_{m1}\text{-}(R_{e2}(C(R^{a3})(R^{a4}))_{m2})_{p1}\text{-}; \text{ or}$$

$$\#\#\text{-}(C(R^{a1}\text{-}(R^{a2}))_{m1}\text{-}(R_{e3}(C(R^{a3})(R^{a4}))_{m2})_{p1}\text{-}(R_{e2}(C(R^{a5})(R^{a6}))_{m3})_{p2}\text{-};$$

wherein each $R_{e2}$ is independently selected from the group consisting of O, S, $N(R_{e4})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_{e4}$ independently represents H or $C_{1-3}$ alkyl; and each group $R_{e3}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-6}$ alkyl, optionally deuterated $C_{3-6}$cycloalkyl, hydroxy, amino, mercapto, halogen, cyano, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH- or any combination thereof;
$R^{a1}$, $R^{a2}$, $R^{a3}$, $R^{a4}$, $R^{a5}$, $R^{a6}$, $R^{a7}$ and $R^{a8}$ each independently represent H, deuterium, hydroxy, amino, mercapto, nitro, halogen, cyano, optionally deuterated $C_{1-6}$ alkyl, halogenated $C_{1-6}$ alkyl, optionally deuterated $C_{3-6}$cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof;
symbol ## indicates the point of attachment to $X_1$; and
m1, m2, m3, m4, p1, p2, p3 are each independently selected from the group consisting of an integer of 1, 2, 3, 4, 5, 6, 7, 8, 9, 10, 11, 12, 13, 14, or 15.

**26.** The compound of Formula (I) or salts, enantiomers, diastereoisomer, isotopically enriched analogs, solvates, prodrugs, or polymorphs thereof as claimed in claim 24 or 25, wherein Li represents the structure of the following formula:

$$\#\#\text{-}(C(R^{a1})(R^{a2}))_{m1}\text{-}(O(C(R^{a3})(R^{a4}))_{m2})_{p1}\text{-};$$

$$\#\#\text{-}(C(R^{a1})(R^{a2}))_{m1}\text{-}(O(C(R^{a3})(R^{a4}))_{m2})_{p1}\text{-}(O(C(R^{a5})(R^{a6}))_{m3})_{p2}\text{-};$$

$$\#\#\text{-}(C(R^{a1})(R^{a2}))_{m1}\text{-}(O(C(R^{a3})(R^{a4}))_{m2})_{p1}\text{-}(O(C(R^{a5})(R^{a6}))_{m3})_{p2}\text{-}(O(C(R^{a7})(R^{a8}))_{m4})_{p3}\text{-};$$

$$\#\#\text{-}(C(R^{a1})(R^{a2}))_{m1}\text{-}(N(R_{g9})(C(R^{a3})(R^{a4}))_{m2})_{p1}\text{-};$$

$$\#\#\text{-}(C(R^{a1})(R^{a2}))_{m1}\text{-}(N(R_{g9})(C(R^{a3})(R^{a4}))_{m2})_{p1}\text{-}(N(R_{g9})(C(R^{a5})(R^{a6}))_{m3})_{p2}\text{-};$$

$$\#\#\text{-}(C(R^{a1})(R^{a2}))_{m1}\text{-}(N(R_{g9})(C(R^{a3})(R^{a4}))_{m2})_{p1}\text{-}(N(R_{g9})(C(R^{a5})(R^{a6}))_{m3})_{p2}\text{-}(N(R_{g9})(C(R^{a7})(R^{a8}))_{m4})_{p3}\text{-};$$

$$\#\#\text{-}(C(R^{a1})(R^{a2}))_{m1}\text{-}(C(O)NH\text{-}(C(R^{a3})(R^{a4}))_{m2})_{p1}\text{-};$$

$$\#\#\text{-}(C(R^{a1})(R^{a2}))_{m1}\text{-}(C(O)NH\text{-}(C(R^{a3})(R^{a4}))_{m2})_{p1}\text{-}(C(O)NH\text{-}(C(R^{a5})(R^{a6}))_{m3})_{p2}\text{-};$$

$$\#\#\text{-}(C(R^{a1})(R^{a2}))_{m1}\text{-}(C(O)NH\text{-}(C(R^{a3})(R^{a4}))_{m2})_{p1}\text{-}(C(O)NH\text{-}(C(R^{a5})(R^{a6}))_{m3})_{p2}\text{-}(C(O)NH\text{-}(C(R^{a7})(R^{a8}))_{m4})_{p3}\text{-};$$

$$\#\#\text{-}(C(R^{a1})(R^{a2}))_{m1}\text{-}(C(O)NH\text{-}(C(R^{a3})(R^{a4}))_{m2})_{p1}\text{-}(O(C(R^{a5})(R^{a6}))_{m3})_{p2}\text{-};$$

$$\#\#\text{-}(C(R^{a1})(R^{a2}))_{m1}\text{-}(C(O)NH\text{-}(C(R^{a3})(R^{a4}))_{m2})_{p1}\text{-}(O(C(R^{a5})(R^{a6}))_{m3})_{p1}\text{-};$$

##-$(C(R^{a1})(R^{a2}))_{m1}$-$(NHC(O)(C(R^{a3})(R^{a4}))_{m2})_{p1}$-;

##-$(C(R^{a1})(R^{a2}))_{m1}$-$(NHC(O)(C(R^{a3})(R^{a4}))_{m2})_{p1}$-$(O(C(R^{a5})(R^{a6}))_{m3})_{p2}$-;

##-$(C(R^{a1})(R^{a2}))_{m1}$-$(NHC(O)(C(R^{a3})(R^{a4}))_{m2})_{p1}$-$(O(C(R^{a5})(R^{a6}))_{m3})_{p2}$-$(O(C(R^{a7})(R^{a8}))_{m4})_{p3}$-;

##-$(C(R^{a1})(R^{a2}))_{m1}$-$(NHC(O)(C(R^{a3})(R^{a4}))_{m2}$-$(O(C(R^{a5})(R^{a6}))_{m3})_{p1}$-;

##-$(C(R^{a1})(R^{a2}))_{m1}$-NHC(O)NH-$(C(R^{a3})(R^{a4}))_{m2}$-;

##$(C(R^{a1})(R^{a2}))_{m1}$-C(O)-$(C(R^{a3})(R^{a4}))_{m2}$-;

##$(C(R^{a1})(R^{a2}))_{m1}$-C(S)-$(C(R^{a3})(R^{a4}))_{m2}$-;

##-$(C(R^{a1})(R^{a2}))_{m1}$-S-$(C(R^{a3})(R^{a4}))_{m2}$-;

##-$(C(R^{a1})(R^{a2}))_{m1}$-($C_{6-10}$ arylene-$(C(R^{a3})(R^{a4}))_{m2})_{p1}$-;

##-$(C(R^{a1})(R^{a2}))_{m1}$-($C_{6-10}$ arylene-$(C(R^{a3})(R^{a4}))_{m2})_{p}$1-$C_{6-10}$ arylene-$(C(R^{a5})(R^{a6}))_{m3}$-;

##-$(C(R^{a1})(R^{a2}))_{m1}$-(4- to 15-membered heterocyclylene-$(C(R^{a3})(R^{aa}))_{m2})_{p1}$-;

##-$(C(R^{a1})(R^{a2}))_{m1}$-(4- to 15-membered heterocyclylene-$(C(R^{a3})(R^{aa}))_{m2})_{p1}$-(4- to 15-membered heterocyclylene-$(C(R^{a5})(R^{a6}))_{m3})_{p2}$-;

##-$(C(R^{a1})(R^{a2}))_{m1}$-(5- to 15-membered heteroarylene-$(C(R^{a3})(R^{a4}))_{m2})_{p1}$-;

##-$(C(R^{a1})(R^{a2}))_{m1}$-(5- to 15-membered heteroarylene-$(C(R^{a3})(R^{a4}))_{m2})_{p1}$-(5- to 15-membered heteroarylene-$(C(R^{a5})(R^{a6}))_{m3})_{p2}$-;

##-$(C(R^{a1})(R^{a2}))_{m1}$-($C_{3-15}$cycloalkylene-$(C(R^{a3})(R^{a4}))_{m2})_{p1}$-; or

##-$(C(R^{a1})(R^{a2}))_{m1}$-($C_{3-15}$cycloalkylene-$(C(R^{a3})(R^{a4}))_{m2})_{p1}$-($C_{3-15}$cycloalkylene-$(C(R^{a5})(R^{a6}))_{m3})_{p2}$-;

wherein each $R_{g9}$ independently represents H or $C_{1-3}$ alkyl;
the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-6}$ alkyl, optionally deuterated $C_{3-6}$cycloalkyl, hydroxy, amino, mercapto, halogen, optionally deuterated $C_{1-6}$ alkoxy, optionally deuterated $C_{1-6}$ alkyl-NH-, halogenated $C_{1-6}$ alkyl, $NH_2$-$C_{1-6}$ alkylene, optionally deuterated $C_{1-6}$ alkyl-NHC(O)-, optionally deuterated $C_{1-6}$ alkyl-C(O)NH-, cyano or any combination thereof;
$R^{a1}$, $R^{a2}$, $R^{a3}$, $R^{a4}$, $R^{a5}$, $R^{a6}$, $R^{a7}$ and $R^{a8}$each independently represent H, deuterium, hydroxy, amino, mercapto, nitro, halogen, cyano, optionally deuterated $C_{1-6}$ alkyl, halogenated $C_{1-6}$ alkyl, optionally deuterated $C_{3-6}$cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- or any combination thereof;
symbol ## indicates the point of attachment to $X_1$; and
m1, m2, m3, m4, p1, p2, p3 are each independently selected from the group consisting of an integer of 1, 2, 3, 4, 5, 6, 7, 8, 9, 10, 11, 12, 13, 14, or 15.

**27.** The compound of Formula (I) or salts, enantiomers, diastereoisomer, isotopically enriched analogs, solvates, prodrugs, or polymorphs thereof as claimed in claim 9, wherein $L_1$ represents the structure of the following formula:

##-$(C(R^{a1})(R^{a2}))_{m1}$-$(R_7(C(R^{a1})(R^{a4}))_{m2})_{p1}$-;

##-$(C(R^{a1})(R^{a2}))_{m1}$-$(R_7(C(R^{a3})(R^{a4}))_{m2})_{p1}$-$(R_7(C(R^{a5})(R^{a6}))_{m3})_{p2}$-;

$$\#\#\text{-}(C(R^{a1})(R^{a2}))_{m1}\text{-}(R_7(C(R^{a3})(R^{a4}))_{m2})_{p1}\text{-}(R_7(C(R^{a5})(R^{a6}))_{m3})_{p2}\text{-}(R_7(C(R^{a7})(R^{a8}))_{m4})_{p3}\text{-};$$

$$\#\#\text{-}(C(R^{a1})(R^{a2}))_{m1}\text{-}(R_8(C(R^{a3})(R^{a4}))_{m2})_{p1}\text{-};$$

$$\#\#\text{-}(C(R^{a1})(R^{a2}))_{m1}\text{-}(R_8(C(R^{a3})(R^{a4}))_{m2})_{p1}\text{-}(R_8(C(R^{a5})(R^{a6}))_{m3})_{p2}\text{-};$$

$$\#(C(R^{a1})(R^{a2}))_{m1}\text{-}(R_8(C(R^{a3})(R^{a4}))_{m2})_{p1}\text{-}(R_8(C(R^{a5})(R^{a6}))_{m3})_{p2}(R_8(C(R^{a7})(R^{a8}))_{m4})_{p3}\text{-};$$

$$\#\#\text{-}(C(R^{a1})(R^{a2}))_{m1}\text{-}(R_7\text{-}R_8\text{-}(C(R^{a3})(R^{a4}))_{m2})_{p1}\text{-};$$

$$\#\#(C(R^{a1})(R^{a2}))_{m1}\text{-}(R_7(C(R^{a3})(R^{a4}))_{m2})_{p1}\text{-}(R^8(C(R^{a5})(R^{a6}))_{m3})_{p2}\text{-};$$

$$\#\#(C(R^{a1})(R^{a2}))_{m1}\text{-}(R_8\text{-}R_7\text{-}(C(R^{a3})(R^{a4}))_{m2})_{p1}\text{-};\ \text{or}$$

$$\#\#(C(R^{a1})(R^{a2}))_{m1}\text{-}(R_8(C(R^{a3})(R^{a4}))_{m2})_{p1}\text{-}(R_7(C(R^{a5})(R^{a6}))_{m3})_{p2}\text{-};$$

wherein each group $R_7$ is independently selected from the group consisting of O, S, $N(R_9)$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_9$ independently represents H or $C_{1-3}$ alkyl; and each $R_8$ is independently selected from the group consisting of $C_{3-15}$cycloalkylene, 4- to 15-membered heterocyclylene, triazolylene or any combination thereof, wherein the $C_{3-15}$cycloalkylene, the 4- to 15-membered heterocyclylene and the triazolylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

$R^{a1}$, $R^{a2}$, $R^{a3}$, $R^{a4}$, $R^{a5}$, $R^{a6}$, $R^{a7}$ and $R^{a8}$ each independently represent H, deuterium, $C_{1-4}$ alkyl, halogen, $C_{3-6}$cycloalkyl or any combination thereof;

symbol ## indicates the point of attachment to $X_1$; and

m1, m2, m3, m4, p1, p2, p3 are each independently selected from the group consisting of an integer of 1, 2, 3, 4, 5, 6, 7, 8, 9, 10, 11, 12, 13, 14, or 15;

preferably, wherein $L_1$ represents the structure of the following formula:

$$\#\#\text{-}(C(R^{a1})(R^{a2}))_{m1}\text{-}(O(C(R^{a3})(R^{a4}))_{m2})_{p1}\text{-};$$

$$\#\#\text{-}(C(R^{a1})(R^{a2}))_{m1}\text{-}(O(C(Ra^3)(Ra^4))_{m2})_{p1}\text{-}(O(C(R^{a5})(R^{a6}))_{m3})_{p2}\text{-};$$

$$\#\#(C(R^{a1})(R^{a2}))_{m1}\text{-}(O(C(R^{a3})(R^{a4}))_{m2})_{p1}\text{-}(O(C(R^{a5})(R^{a6}))_{m3})_{p2}(O(C(R^{a7})(R^{a8}))_{m4})_{p3}\text{-};$$

$$\#\#\text{-}(C(R^{a1})(R^{a2}))_{m1}\text{-}(N(R_9)(C(R^{a3})(R^{a4}))_{m2})_{p1}\text{-};$$

$$\#\#\text{-}(C(R^{a1})(R^{a2}))_{m1}\text{-}(N(R_9)(C(R^{a3})(R^{a4}))_{m2})_{p1}\text{-}(N(R_9)(C(R^{a5})(R^{a6}))_{m3})_{p2}\text{-};$$

$$\#\#\text{-}(C(R^{a1})(R^{a2}))_{m1}\text{-}(N(R_9)(C(R^{a3})(R^{a4}))_{m2})_{p1}\text{-}(N(R_9)(C(R^{a5})(R^{a6}))_{m3})_{p2}\text{-}(N(R_9)(C(R^{a7})(R^{a8}))_{m4})_{p3}\text{-};$$

$$\#\#\text{-}(C(R^{a1})(R^{a2}))_{m1}\text{-}(C(O)NH\text{-}(C(R^{a3})(R^{a4}))_{m2})_{p1}\text{-};$$

$$\#\#(C(R^{a1})(R^{a2}))_{m1}\text{-}(C(O)NH\text{-}(C(R^{a3})(R^{a4}))_{m2})_{p1}\text{-}(C(O)NH\text{-}(C(R^{a5})(R^{a6}))_{m3})_{p2}\text{-};$$

$$\#\#(C(R^{a1})(R^{a2}))_{m1}\text{-}(C(O)NH\text{-}(C(R^{a3})(R^{a4}))_{m2})_{p1}\text{-}(C(O)NH\text{-}(C(R^{a5})(R^{a6}))_{m3})_{p2}\text{-}(C(O)NH\text{-};(C(R^{a7})(R^{a8}))_{m4})_{p3}\text{-};$$

$$\#\#(C(R^{a1})(R^{a2}))_{m1}\text{-}(C(O)NH\text{-}(C(R^{a3})(R^{a4}))_{m2})_{p1}\text{-}(O\text{-}(C(R^{a5})(R^{a6}))_{m3})_{p2}\text{-};$$

$\#\#(C(R^{a1})(R^{a2}))_{m1}$-(C(O)NH-$(C(R^{a3})(R^{a4}))_{m2})(O(C(R^{a5})(R^{a6}))_{m3})_{p1}$-;

$\#\#(C(R^{a1})(R^{a2}))_{m1}$-(NHC(O)-$(C(R^{a3})(R^{a4}))_{m2})_{p1}$-;

$\#\#(C(R^{a1})(R^{a2}))_{m1}$-(NHC(O)-$(C(R^{a3})(R^{a4}))_{m2})_{p1}$-$(O(C(R^{a5})(R^{a6}))_{m3})_{p2}$-;

$\#\#(C(R^{a1})(R^{a2}))_{m1}$-NHC(O)-$(C(R^{a3})(R^{a4}))_{m2})_{p1}$-(O-$(C(R^{a5})(R^{a6}))_{m3})_{p2}$-$(O(C(R^{a7})(R^{a8}))_{m4})_{p3}$-;

$\#\#(C(R^{a1})(R^{a2}))_{m1}$-NHC(O)-$(C(R^{a3})(R^{a4}))_{m2}$-$(O(C(R^{a5})(R^{a6}))_{m3})_{p1}$-;

##-$(C(R^{a1})(R^{a2}))_{m1}$-NHC(O)NH-$(C(R^{a3})(R^{a4}))_{m2}$-;

##-$(C(R^{a1})(R^{a2}))_{m1}$-C(O)-$(C(R^{a3})(R^{a4}))_{m2}$-;

$\#\#(C(R^{a1})(R^{a2}))_{m1}$-(C(S)-$(C(R^{a3})(R^{a4}))_{m2}$-;

##-$(C(R^{a1})(R^{a2}))_{m1}$-S-$(C(R^{a3})(R^{a4}))_{m2}$-;

##-$(C(R^{a1})(R^{a2}))_{m1}$-(4- to 15-membered heterocyclylene-$(C(R^{a3})(R^{aa}))_{m2})_{p1}$-;

##-$(C(R^{a1})(R^{a2}))_{m1}$-(4- to 15-membered heterocyclylene-$(C(R^{a3})(R^{a4}))_{m2})_{p1}$-(4- to 15-membered heterocyclylene-$(C(R^{a5})(R^{a6}))_{m3})_{p2}$-;

##-$(C(R^{a1})(R^{a2}))_{m1}$-( triazolylene -$(C(R^{a3})(R^{a4}))_{m2})_{p1}$-;

##-$(C(R^{a1})(R^{a2}))_{m1}$-( triazolylene -$(C(R^{a3})(R^{a4}))_{m2})_{p1}$-( triazolylene -$(C(R^{a5})(R^{a6}))_{m3})_{p2}$-;

##-$(C(R^{a1})(R^{a2}))_{m1}$-($C_{3-15}$cycloalkylene-$(C(R^{a3})(R^{a4}))_{m2})_{p1}$-; or

##-$(C(R^{a1})(R^{a2}))_{m1}$-($C_{3-15}$cycloalkylene-$(C(R^{a3})(R^{a4}))_{m2})_{p1}$-($C_{3-15}$cycloalkylene-$(C(R^{a5})(R^{a6}))_{m3})_{p2}$-;

wherein each $R_9$ independently represents H or $C_{1-3}$ alkyl;
the $C_{3-15}$cycloalkylene, the 4- to 15-membered heterocyclylene and the triazolylene are each independently optionally substituted with a substituent selected from the group consisting of D, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;
$R^{a1}$, $R^{a2}$, $R^{a3}$, $R^{a4}$, $R^{a5}$, $R^{a6}$, $R^{a7}$ and $R^{a8}$ each independently represent H, deuterium, $C_{1-4}$ alkyl, halogen, $C_{3-6}$cycloalkyl or any combination thereof;
symbol ## indicates the point of attachment to $X_1$; and
m1, m2, m3, m4, p1, p2, p3 are each independently selected from the group consisting of an integer of 1, 2, 3, 4, 5, 6, 7, 8, 9, 10, 11, 12, 13, 14, or 15.

**28.** The compound of Formula (I) or salts, enantiomers, diastereoisomer, isotopically enriched analogs, solvates, prodrugs, or polymorphs thereof as claimed in claim 26 or 27, wherein $L_1$ represents the following group: ##-$CH_2$-O-$CH_2$-, ##-$CH_2$-O-$(CH_2)_2$-, ##-$(CH_2)_1$-O-$(CH_2)_3$-, ##-$(CH_2)_1$-O-$(CH_2)_4$-, ##-$(CH_2)_1$-O-$(CH_2)_5$-, ##-$(CH_2)_1$-O-$(CH_2)_6$-, ##-$(CH_2)_1$-O-$(CH_2)_7$-, ##-$(CH_2)_1$-O-$(CH_2)_8$-, ##-$(CH_2)_1$-O-$(CH_2)_9$-, ##-$(CH_2)_1$-O-$(CH_2)_{10}$-, ##-$(CH_2)_2$-O-$(CH_2)_1$-, ##-$(CH_2)_2$-O-$(CH_2)_2$-, ##-$(CH_2)_2$-O-$(CH_2)_3$-, ##-$(CH_2)_2$-O-$(CH_2)_4$-, ##-$(CH_2)_2$-O-$(CH_2)_5$-, ##-$(CH_2)_2$-O-$(CH_2)_6$-, ##-$(CH_2)_2$-O-$(CH_2)_7$-, ##-$(CH_2)_2$-O-$(CH_2)_8$-, ##-$(CH_2)_2$-O-$(CH_2)_9$-, ##-$(CH_2)_2$-O-$(CH_2)_{10}$-, ##-$(CH_2)_2$-O-$(CH_2)_{11}$-, ##-$(CH_2)_2$-O-$(CH_2)_{12}$-, ##-$(CH_2)_3$-O-$(CH_2)_1$-, ##-$(CH_2)_3$-O-$(CH_2)_2$-, ##-$(CH_2)_3$-O-$(CH_2)_3$-, ##-$(CH_2)_3$-O-$(CH_2)_4$-, ##-$(CH_2)_3$-O-$(CH_2)_5$-, ##-$(CH_2)_3$-O-$(CH_2)_6$-, ##-$(CH_2)_3$-O-$(CH_2)_7$-, ##-$(CH_2)_4$-O-$(CH_2)_1$-, ##-$(CH_2)_4$-O-$(CH_2)_2$-, ##-$(CH_2)_4$-O-$(CH_2)_3$-, ##-$(CH_2)_4$-O-$(CH_2)_4$-, ##-$(CH_2)_4$-O-$(CH_2)_5$-, ##-$(CH_2)_4$-O-$(CH_2)_6$-, ##-$(CH_2)_5$-O-$(CH_2)_1$-, ##-$(CH_2)_5$-O-$(CH_2)_2$-, ##-$(CH_2)_5$-O-$(CH_2)_3$-, ##-$(CH_2)_5$-O-$(CH_2)_4$-, ##-$(CH_2)_5$-O-$(CH_2)_5$-, ##-$(CH_2)_6$-O-$(CH_2)_1$-, ##-$(CH_2)_6$-O-$(CH_2)_2$-, ##-$(CH_2)_6$-O-$(CH_2)_3$-, ##-$(CH_2)_6$-O-$(CH_2)_4$-, ##-$(CH_2)_7$-O-$(CH_2)_1$-, ##-$(CH_2)_7$-O-$(CH_2)_2$-, ##-$(CH_2)_7$-O-$(CH_2)_3$-, ##-$(CH_2)_8$-O-$(CH_2)_1$-, ##-$(CH_2)_8$-O-$(CH_2)_2$-, ##-$CH(CH_3)$-O-$(CH_2)_1$-, ##-$CH(CH_3)$-O-$(CH_2)_2$-, 99-$CH(CH_3)$-O-$(CH_2)_3$-, 99-$CH(CH_3)$-O-$(CH_2)_4$-, ##-$CH(CH_3)$-O-$(CH_2)_5$-,

##-$CH(CH_3)-O-(CH_2)_6-$, ##-$CH(CH_3)-O-(CH_2)_7-$, ##-$CH(CH_3)-O-(CH_2)_8-$, ##-$CH(CH_3)-O-(CH_2)_9-$, ##-$CH(CH_3)-O-(CH_2)_{10}-$, ##-$CH_2-(O(CH_2)_2)_2-$, ##-$CH_2-(O(CH_2)_2)_3-$, ##-$CH_2-(O(CH_2)_2)_4-$, ##-$CH_2-(O(CH_2)_2)_5-$, ##-$CH_2-(O(CH_2)_2)_6-$, ##-$CH_2-(O(CH_2)_2)_7-$, ##-$CH_2-(O(CH_2)_2)_8-$, ##-$CH_2-(O(CH_2)_2)_9-$, ##-$CH_2-(O(CH_2)_2)_{10}-$, ##-$CH_2-(O(CH_2)_2)_1-OCH_2-$, ##-$CH_2-(O(CH_2)_2)_2-OCH_2-$, ##-$CH_2-(O(CH_2)_2)_3-OCH_2-$, ##-$CH_2-(O(CH_2)_2)_4-OCH_2-$, ##-$CH_2-(O(CH_2)_2)_5-OCH_2-$, ##-$CH_2-(O(CH_2)_2)_6-OCH_2-$, ##-$CH_2-(O(CH_2)_2)_7-OCH_2-$, ##-$CH_2-(O(CH_2)_2)_8-OCH_2-$, ##-$CH_2-(O(CH_2)_2)_9-OCH_2-$, ##-$CH_2-(O(CH_2)_2)_{10}-OCH_2-$, ##-$(CH_2)_2-(O(CH_2)_2)_2-$, ##-$(CH_2)_2-(O(CH_2)_2)_3-$, ##-$(CH_2)_2-(O(CH_2)_2)_4-$, ##-$(CH_2)_2-(O(CH_2)_2)_5-$, ##-$(CH_2)_2-(O(CH_2)_2)_6-$, ##-$(CH_2)_2-(O(CH_2)_2)_7-$, ##-$(CH_2)_2-(O(CH_2)_2)_8-$, ##-$(CH_2)_2-(O(CH_2)_2)_9-$, ##-$(CH_2)_2-(O(CH_2)_2)_{10}-$, ##-$(CH_2)_3-(O(CH_2)_2)_2-$, ##-$(CH_2)_3-(O(CH_2)_2)_3-$, ##-$(CH_2)_3-(O(CH_2)_2)_4-$, ##-$(CH_2)_3-(O(CH_2)_2)_5-$, ##-$(CH_2)_3-(O(CH_2)_2)_6-$, ##-$(CH_2)_3-(O(CH_2)_2)_7-$, ##-$(CH_2)_3-(O(CH_2)_2)_8-$, ##-$(CH_2)_3-(O(CH_2)_2)_9-$, ##-$(CH_2)_3-(O(CH_2)_2)_{10}-$, ##-$(CH_2)_4-(O(CH_2)_2)_2-$, ##-$(CH_2)_4-(O(CH_2)_2)_3-$, ##-$(CH_2)_4-(O(CH_2)_2)_4-$, ##-$(CH_2)_4-(O(CH_2)_2)_5-$, ##-$(CH_2)_4-(O(CH_2)_2)_6-$, ##-$(CH_2)_4-(O(CH_2)_2)_7-$, ##-$(CH_2)_4-(O(CH_2)_2)_8-$, ##-$(CH_2)_4-(O(CH_2)_2)_9-$, ##-$(CH_2)_4-(O(CH_2)_2)_{10}-$, ##-$CH_2-(O(CH_2)_3)_2-$, ##-$CH_2-(O(CH_2)_3)_3-$, ##-$CH_2-(O(CH_2)_3)_4-$, ##-$CH_2-(O(CH_2)_3)_5-$, ##-$CH_2-(O(CH_2)_3)_6-$, ##-$CH_2-(O(CH_2)_3)_7-$, ##-$CH_2-(O(CH_2)_3)_8-$, ##-$CH_2-(O(CH_2)_3)_9-$, ##-$CH_2-(O(CH_2)_3)_{10}-$, ##-$(CH_2)_2-(O(CH_2)_3)_2-$, ##-$(CH_2)_2-(O(CH_2)_3)_3-$, ##-$(CH_2)_2-(O(CH_2)_3)_4-$, ##-$(CH_2)_2-(O(CH_2)_3)_5-$, ##-$(CH_2)_2-(O(CH_2)_3)_6-$, ##-$(CH_2)_2-(O(CH_2)_3)_7-$, ##-$(CH_2)_2-(O(CH_2)_3)_8-$, ##-$(CH_2)_2-(O(CH_2)_3)_9-$, ##-$(CH_2)_2-(O(CH_2)_3)_{10}-$, ##-$(CH_2)_3-(O(CH_2)_3)_2-$, ##-$(CH_2)_3-(O(CH_2)_3)_3-$, ##-$(CH_2)_3-(O(CH_2)_3)_4-$, ##-$(CH_2)_3-(O(CH_2)_3)_5-$, ##-$(CH_2)_3-(O(CH_2)_3)_6-$, ##-$(CH_2)_3-(O(CH_2)_3)_7-$, ##-$(CH_2)_3-(O(CH_2)_3)_8-$, ##-$(CH_2)_3-(O(CH_2)_3)_9-$, ##-$(CH_2)_3-(O(CH_2)_3)_{10}-$, ##-$CH_2-O-(CH_2)_2-O-(CH_2)_3-$, ##-$CH_2-(O(CH_2)_2)_2-(O(CH_2)_3)_2-$, ##-$CH_2-(O(CH_2)_2)_3-(O(CH_2)_3)_3-$, ##-$CH_2-(O(CH_2)_2)_4-(O(CH_2)_3)_4-$, ##-$CH_2-(O(CH_2)_2)_5-(O(CH_2)_3)_5-$, ##-$CH_2-(O(CH_2)_2)_6-(O(CH_2)_3)_6-$, ##-$(CH_2)_2-O-(CH_2)_2-O-(CH_2)_3-$, ##-$(CH_2)_2-(O(CH_2)_2)_2-(O(CH_2)_3)_2-$, ##-$(CH_2)_2-(O(CH_2)_2)_3-(O(CH_2)_3)_3-$, ##-$(CH_2)_2-(O(CH_2)_2)_4-(O(CH_2)_3)_4-$, ##-$(CH_2)_2-(O(CH_2)_2)_5-(O(CH_2)_3)_5-$, ##-$(CH_2)_2-(O(CH_2)_2)_6-(O(CH_2)_3)_6-$, ##-$(CH_2)_3-O-(CH_2)_2-O-(CH_2)_3-$, ##-$(CH_2)_3-(O(CH_2)_2)_2-(O(CH_2)_3)_2-$, ##-$(CH_2)_3-(O(CH_2)_2)_3-(O(CH_2)_3)_3-$, ##-$(CH_2)_3-(O(CH_2)_2)_4-(O(CH_2)_3)_4-$, ##-$(CH_2)_3-(O(CH_2)_2)_5-(O(CH_2)_3)_5-$, ##-$(CH_2)_3-(O(CH_2)_2)_6-(O(CH_2)_3)_6-$, ##-$CH_2-O-(CH_2)_3-O-(CH_2)_2-$, ##-$CH_2-(O(CH_2)_3)_2-(O(CH_2)_2)_2-$, ##-$CH_2-(O(CH_2)_3)_3-(O(CH_2)_2)_3-$, ##-$CH_2-(O(CH_2)_3)_4-(O(CH_2)_2)_4-$, ##-$CH_2-(O(CH_2)_3)_5-(O(CH_2)_2)_5-$, ##-$CH_2-(O(CH_2)_3)_6-(O(CH_2)_2)_6-$, ##-$(CH_2)_2-O-(CH_2)_3-O-(CH_2)_2-$, ##-$(CH_2)_2-(O(CH_2)_3)_2-(O(CH_2)_2)_2-$, ##-$(CH_2)_2-(O(CH_2)_3)_3-(O(CH_2)_2)_3-$, ##-$(CH_2)_2-(O(CH_2)_3)_4-(O(CH_2)_2)_4-$, ##-$(CH_2)_2-(O(CH_2)_3)_5-(O(CH_2)_2)_5-$, ##-$(CH_2)_2-(O(CH_2)_3)_6-(O(CH_2)_2)_6-$, ##-$(CH_2)_3-O-(CH_2)_3-O-(CH_2)_2-$, ##-$(CH_2)_3-(O(CH_2)_3)_2-(O(CH_2)_2)_2-$, ##-$(CH_2)_3-(O(CH_2)_3)_3-(O(CH_2)_2)_3-$, ##-$(CH_2)_3-(O(CH_2)_3)_4-(O(CH_2)_2)_4-$, ##-$(CH_2)_3-(O(CH_2)_3)_5-(O(CH_2)_2)_5-$, ##-$(CH_2)_3-(O(CH_2)_3)_6-(O(CH_2)_2)_6-$, ##-$CH_2-O-(CH_2)_2-O-CH_2-$, ##-$(CH_2)_2-O-(CH_2)_2-O-CH_2-$, ##-$(CH_2)_2-(O(CH_2)_2)_2-O-(CH_2)_3-$, ##-$(CH_2)_2-(O(CH_2)_2)_3-O-(CH_2)_3-$, ##-$(CH_2)_2-(O(CH_2)_2)_4-O-(CH_2)_3-$, ##-$(CH_2)_5-(O(CH_2)_2)_2-O-(CH_2)_5-$, ##-$(CH_2)_5-(O(CH_2)_2)_2-O-(CH_2)_6-$, ##-$CH_2-C(O)-CH_2-$, ##-$CH_2-C(O)-(CH_2)_2-$, ##-$(CH_2)_1-C(O)-(CH_2)_3-$, ##-$(CH_2)_1-C(O)-(CH_2)_4-$, ##-$(CH_2)_1-C(O)-(CH_2)_5-$, ##-$(CH_2)_1-C(O)-(CH_2)_6-$, ##-$(CH_2)_1-C(O)-(CH_2)_7-$, ##-$(CH_2)_1-C(O)-(CH_2)_8-$, ##-$(CH_2)_1-C(O)-(CH_2)_9-$, ##-$(CH_2)_1-C(O)-(CH_2)_{10}-$, ##-$(CH_2)_2-C(O)-(CH_2)_1-$, ##-$(CH_2)_2-C(O)-(CH_2)_2-$, ##-$(CH_2)_2-C(O)-(CH_2)_3-$, ##-$(CH_2)_2-C(O)-(CH_2)_4-$, ##-$(CH_2)_2-C(O)-(CH_2)_5-$, ##-$(CH_2)_2-C(O)-(CH_2)_6-$, ##-$(CH_2)_2-C(O)-(CH_2)_7-$, ##-$(CH_2)_2-C(O)-(CH_2)_8-$, ##-$(CH_2)_2-C(O)-(CH_2)_9-$, ##-$(CH_2)_2-C(O)-(CH_2)_{10}-$, ##-$(CH_2)_2-C(O)-(CH_2)_{11}-$, ##-$(CH_2)_2-C(O)-(CH_2)_{12}-$, ##-$(CH_2)_3-C(O)-(CH_2)_1-$, ##-$(CH_2)_3-C(O)-(CH_2)_2-$, ##-$(CH_2)_3-C(O)-(CH_2)_3-$, ##-$(CH_2)_3-C(O)-(CH_2)_4-$, ##-$(CH_2)_3-C(O)-(CH_2)_5-$, ##-$(CH_2)_3-C(O)-(CH_2)_6-$, ##-$(CH_2)_3-C(O)-(CH_2)_7-$, ##-$(CH_2)_4-C(O)-(CH_2)_1-$, ##-$(CH_2)_4-C(O)-(CH_2)_2-$, ##-$(CH_2)_4-C(O)-(CH_2)_3-$, ##-$(CH_2)_4-C(O)-(CH_2)_4-$, ##-$(CH_2)_4-C(O)-(CH_2)_5-$, ##-$(CH_2)_a-C(O)-(CH_2)_6-$, ##-$(CH_2)_5-C(O)-(CH_2)_1-$, ##-$(CH_2)_5-C(O)-(CH_2)_2-$, ##-$(CH_2)_5-C(O)-(CH_2)_3-$, ##-$(CH_2)_5-C(O)-(CH_2)_4-$, ##-$(CH_2)_5-C(O)-(CH_2)_5-$, ##-$(CH_2)_6-C(O)-(CH_2)_1-$, ##-$(CH_2)_6-C(O)-(CH_2)_2-$, ##-$(CH_2)_6-C(O)-(CH_2)_3-$, ##-$(CH_2)_6-C(O)-(CH_2)_4-$, ##-$(CH_2)_7-C(O)-(CH_2)_1-$, ##-$(CH_2)_7-C(O)-(CH_2)_2-$, ##-$(CH_2)_7-C(O)-(CH_2)_3-$, ##-$(CH_2)_8-C(O)-(CH_2)_1-$, ##-$(CH_2)_8-C(O)-(CH_2)_2-$, ##-$CH_2-S-CH_2-$, ##-$CH_2-S-(CH_2)_2-$, ##-$(CH_2)_1-S-(CH_2)_3-$, ##-$(CH_2)_1-S-(CH_2)_4-$, ##-$(CH_2)_1-S-(CH_2)_5-$, ##-$(CH_2)_1-S-(CH_2)_6-$, ##-$(CH_2)_1-S-(CH_2)_7-$, ##-$(CH_2)_1-S-(CH_2)_8-$, ##-$(CH_2)_1-S-(CH_2)_9-$, ##-$(CH_2)_1-S-(CH_2)_{10}-$, ##-$(CH_2)_2-S-(CH_2)_1-$, ##-$(CH_2)_2-S-(CH_2)_2-$, ##-$(CH_2)_2-S-(CH_2)_3-$, ##-$(CH_2)_2-S-(CH_2)_4-$, ##-$(CH_2)_2-S-(CH_2)_5-$, ##-$(CH_2)_2-S-(CH_2)_6-$, ##-$(CH_2)_2-S-(CH_2)_7-$, ##-$(CH_2)_2-S-(CH_2)_8-$, ##-$(CH_2)_2-S-(CH_2)_9-$, ##-$(CH_2)_2-S-(CH_2)_{10}-$, ##-$(CH_2)_2-S-(CH_2)_{11}-$, ##-$(CH_2)_2-S-(CH_2)_{12}-$, ##-$(CH_2)_3-S-(CH_2)_1-$, ##-$(CH_2)_3-S-(CH_2)_2-$, ##-$(CH_2)_3-S-(CH_2)_3-$, ##-$(CH_2)_3-S-(CH_2)_4-$, ##-$(CH_2)_3-S-(CH_2)_5-$, ##-$(CH_2)_3-S-(CH_2)_6-$, ##-$(CH_2)_3-S-(CH_2)_7-$, ##-$(CH_2)_4-S-(CH_2)_1-$, ##-$(CH_2)_4-S-(CH_2)_2-$, ##-$(CH_2)_4-S-(CH_2)_3-$, ##-$(CH_2)_4-S-(CH_2)_4-$, ##-$(CH_2)_4-S-(CH_2)_5-$, ##-$(CH_2)_4-S-(CH_2)_6-$, ##-$(CH_2)_5-S-(CH_2)_1-$, ##-$(CH_2)_5-S-(CH_2)_2-$, ##-$(CH_2)_5-S-(CH_2)_3-$, ##-$(CH_2)_5-S-(CH_2)_4-$, ##-$(CH_2)_5-S-(CH_2)_5-$, ##-$(CH_2)_6-S-(CH_2)_1-$, ##-$(CH_2)_6-S-(CH_2)_2-$, ##-$(CH_2)_6-S-(CH_2)_3-$, ##-$(CH_2)_6-S-(CH_2)_4-$, ##-$(CH_2)_7-S-(CH_2)_1-$, ##-$(CH_2)_7-S-(CH_2)_2-$, ##-$(CH_2)_7-S-(CH_2)_3-$, ##-$(CH_2)_8-S-(CH_2)_1-$, ##-$(CH_2)_8-S-(CH_2)_2-$, ##-$CH_2-C(S)-CH_2-$, ##-$CH_2-C(S)-(CH_2)_2-$, ##-$(CH_2)_1-C(S)-(CH_2)_3-$, ##-$(CH_2)_1-C(S)-(CH_2)_4-$, ##-$(CH_2)_1-C(S)-(CH_2)_5-$, ##-$(CH_2)_1-C(S)-(CH_2)_6-$, ##-$(CH_2)_1-C(S)-(CH_2)_7-$, ##-$(CH_2)_1-C(S)-(CH_2)_8-$, ##-$(CH_2)_1-C(S)-(CH_2)_9-$, ##-$(CH_2)_1-C(S)-(CH_2)_{10}-$, ##-$(CH_2)_2-C(S)-(CH_2)_1-$, ##-$(CH_2)_2-C(S)-(CH_2)_2-$, ##-$(CH_2)_2-C(S)-(CH_2)_3-$, ##-$(CH_2)_2-C(S)-(CH_2)_4-$, ##-$(CH_2)_2-C(S)-(CH_2)_5-$,

##-$(CH_2)_2$-C(S)-$(CH_2)_6$-, ##-$(CH_2)_2$-C(S)-$(CH_2)_7$-, ##-$(CH_2)_2$-C(S)-$(CH_2)_8$-, ##-$(CH_2)_2$-C(S)-$(CH_2)_9$-, ##-$(CH_2)_2$-C(S)-$(CH_2)_{10}$-, ##-$(CH_2)_2$-C(S)-$(CH_2)_{11}$-, ##-$(CH_2)_2$-C(S)-$(CH_2)_{12}$-, ##-$(CH_2)_3$-C(S)-$(CH_2)_1$-, ##-$(CH_2)_3$-C(S)-$(CH_2)_2$-, ##-$(CH_2)_3$-C(S)-$(CH_2)_3$-, ##-$(CH_2)_3$-C(S)-$(CH_2)_4$-, ##-$(CH_2)_3$-C(S)-$(CH_2)_5$-, ##-$(CH_2)_3$-C(S)-$(CH_2)_6$-, ##-$(CH_2)_3$-C(S)-$(CH_2)_7$-, ##-$(CH_2)_4$-C(S)-$(CH_2)_1$-, ##-$(CH_2)_4$-C(S)-$(CH_2)_2$-, ##-$(CH_2)_4$-C(S)-$(CH_2)_3$-, ##-$(CH_2)_4$-C(S)-$(CH_2)_4$-, ##-$(CH_2)_4$-C(S)-$(CH_2)_5$-, ##-$(CH_2)_4$-C(S)-$(CH_2)_6$-, ##-$(CH_2)_5$-C(S)-$(CH_2)_1$-, ##-$(CH_2)_5$-C(S)-$(CH_2)_2$-, ##-$(CH_2)_5$-C(S)-$(CH_2)_3$-, ##-$(CH_2)_5$-C(S)-$(CH_2)_4$-, ##-$(CH_2)_5$-C(S)-$(CH_2)_5$-, ##-$(CH_2)_6$-C(S)-$(CH_2)_1$-, ##-$(CH_2)_6$-C(S)-$(CH_2)_2$-, ##-$(CH_2)_6$-C(S)-$(CH_2)_3$-, ##-$(CH_2)_6$-C(S)-$(CH_2)_4$-, ##-$(CH_2)_7$-C(S)-$(CH_2)_l$-, ##-$(CH_2)_7$-C(S)-$(CH_2)_2$-, ##-$(CH_2)_7$-C(S)-$(CH_2)_3$-, ##-$(CH_2)_8$-C(S)-$(CH_2)_1$-, ##-$(CH_2)_8$-C(S)-$(CH_2)_2$-, ##-$CH_2$-C(O)O-$CH_2$-, ##-$CH_2$-C(O)O-$(CH_2)_2$-, ##-$(CH_2)_1$-C(O)O-$(CH_2)_3$-, ##-$(CH_2)_1$-C(O)O-$(CH_2)_4$-, ##-$(CH_2)_1$-C(O)O-$(CH_2)_5$-, ##-$(CH_2)_1$-C(O)O-$(CH_2)_6$-, ##-$(CH_2)_1$-C(O)O-$(CH_2)_7$-, ##-$(CH_2)_1$-C(O)O-$(CH_2)_8$-, ##-$(CH_2)_1$-C(O)O-$(CH_2)_9$-, ##-$(CH_2)_1$-C(O)O-$(CH_2)_{10}$-, ##-$(CH_2)_2$-C(O)O-$(CH_2)_1$-, ##-$(CH_2)_2$-C(O)O-$(CH_2)_2$-, ##-$(CH_2)_2$-C(O)O-$(CH_2)_3$-, ##-$(CH_2)_2$-C(O)O-$(CH_2)_4$-, ##-$(CH_2)_2$-C(O)O-$(CH_2)_5$-, ##-$(CH_2)_2$-C(O)O-$(CH_2)_6$-, ##-$(CH_2)_2$-C(O)O-$(CH_2)_7$-, ##-$(CH_2)_2$-C(O)O-$(CH_2)_8$-, ##-$(CH_2)_2$-C(O)O-$(CH_2)_9$-, ##-$(CH_2)_2$-C(O)O-$(CH_2)_{10}$-, ##-$(CH_2)_2$-C(O)O-$(CH_2)_{11}$-, ##-$(CH_2)_2$-C(O)O-$(CH_2)_{12}$-, ##-$(CH_2)_3$-C(O)O-$(CH_2)_1$-, ##-$(CH_2)_3$-C(O)O-$(CH_2)_2$-, ##-$(CH_2)_3$-C(O)O-$(CH_2)_3$-, ##-$(CH_2)_3$-C(O)O-$(CH_2)_4$-, ##-$(CH_2)_3$-C(O)O-$(CH_2)_5$-, ##-$(CH_2)_3$-C(O)O-$(CH_2)_6$-, ##-$(CH_2)_3$-C(O)O-$(CH_2)_7$-, ##-$(CH_2)_4$-C(O)O-$(CH_2)_1$-, ##-$(CH_2)_4$-C(O)O-$(CH_2)_2$-, ##-$(CH_2)_4$-C(O)O-$(CH_2)_3$-, ##-$(CH_2)_4$-C(O)O-$(CH_2)_4$-, ##-$(CH_2)_4$-C(O)O-$(CH_2)_5$-, ##-$(CH_2)_4$-C(O)O-$(CH_2)_6$-, ##-$(CH_2)_5$-C(O)O-$(CH_2)_1$-, ##-$(CH_2)_5$-C(O)O-$(CH_2)_2$-, ##-$(CH_2)_5$-C(O)O-$(CH_2)_3$-, ##-$(CH_2)_5$-C(O)O-$(CH_2)_4$-, ##-$(CH_2)_5$-C(O)O-$(CH_2)_5$-, ##-$(CH_2)_6$-C(O)O-$(CH_2)_1$-, ##-$(CH_2)_6$-C(O)O-$(CH_2)_2$-, ##-$(CH_2)_6$-C(O)O-$(CH_2)_3$-, ##-$(CH_2)_6$-C(O)O-$(CH_2)_4$-, ##-$(CH_2)_7$-C(O)O-$(CH_2)_1$-, ##-$(CH_2)_7$-C(O)O-$(CH_2)_2$-, ##-$(CH_2)_7$-C(O)O-$(CH_2)_3$-, ##-$(CH_2)_8$-C(O)O-$(CH_2)_1$-, ##-$(CH_2)_8$-C(O)O-$(CH_2)_2$-, ##-$CH_2$-OC(O)-$CH_2$-, ##-$CH_2$-OC(O)-$(CH_2)_2$-, ##-$(CH_2)_1$-OC(O)-$(CH_2)_3$-, ##-$(CH_2)_1$-OC(O)-$(CH_2)_4$-, ##-$(CH_2)_1$-OC(O)-$(CH_2)_5$-, ##-$(CH_2)_1$-OC(O)-$(CH_2)_6$-, ##-$(CH_2)_1$-OC(O)-$(CH_2)_7$-, ##-$(CH_2)_1$-OC(O)-$(CH_2)_8$-, ##-$(CH_2)_1$-OC(O)-$(CH_2)_9$-, ##-$(CH_2)_1$-OC(O)-$(CH_2)_{10}$-, ##-$(CH_2)_2$-OC(O)-$(CH_2)_1$-, ##-$(CH_2)_2$-OC(O)-$(CH_2)_2$-, ##-$(CH_2)_2$-OC(O)-$(CH_2)_3$-, ##-$(CH_2)_2$-OC(O)-$(CH_2)_4$-, ##-$(CH_2)_2$-OC(O)-$(CH_2)_5$-, ##-$(CH_2)_2$-OC(O)-$(CH_2)_6$-, ##-$(CH_2)_2$-OC(O)-$(CH_2)_7$-, ##-$(CH_2)_2$-OC(O)-$(CH_2)_8$-, ##-$(CH_2)_2$-OC(O)-$(CH_2)_9$-, ##-$(CH_2)_2$-OC(O)-$(CH_2)_{10}$-, ##-$(CH_2)_2$-OC(O)-$(CH_2)_{11}$-, ##-$(CH_2)_2$-OC(O)-$(CH_2)_{12}$-, ##-$(CH_2)_3$-OC(O)-$(CH_2)_1$-, ##-$(CH_2)_3$-OC(O)-$(CH_2)_2$-, ##-$(CH_2)_3$-OC(O)-$(CH_2)_3$-, ##-$(CH_2)_3$-OC(O)-$(CH_2)_4$-, ##-$(CH_2)_3$-OC(O)-$(CH_2)_5$-, ##-$(CH_2)_3$-OC(O)-$(CH_2)_6$-, ##-$(CH_2)_3$-OC(O)-$(CH_2)_7$-, ##-$(CH_2)_4$-OC(O)-$(CH_2)_1$-, ##-$(CH_2)_4$-OC(O)-$(CH_2)_2$-, ##-$(CH_2)_4$-OC(O)-$(CH_2)_3$-, ##-$(CH_2)_4$-OC(O)-$(CH_2)_4$-, ##-$(CH_2)_4$-OC(O)-$(CH_2)_5$-, ##-$(CH_2)_4$-OC(O)-$(CH_2)_6$-, ##-$(CH_2)_5$-OC(O)-$(CH_2)_1$-, ##-$(CH_2)_5$-OC(O)-$(CH_2)_2$-, ##-$(CH_2)_5$-OC(O)-$(CH_2)_3$-, ##-$(CH_2)_5$-OC(O)-$(CH_2)_4$-, ##-$(CH_2)_5$-OC(O)-$(CH_2)_5$-, ##-$(CH_2)_6$-OC(O)-$(CH_2)_1$-, ##-$(CH_2)_6$-OC(O)-$(CH_2)_2$-, ##-$(CH_2)_6$-OC(O)-$(CH_2)_3$-, ##-$(CH_2)_6$-OC(O)-$(CH_2)_4$-, ##-$(CH_2)_7$-OC(O)-$(CH_2)_1$-, ##-$(CH_2)_7$-OC(O)-$(CH_2)_2$-, ##-$(CH_2)_7$-OC(O)-$(CH_2)_3$-, ##-$(CH_2)_8$-OC(O)-$(CH_2)_1$-, ##-$(CH_2)_8$-OC(O)-$(CH_2)_2$-, ##-$(CH_2)_1$-N($R_{g10}$)-$(CH_2)_1$-, ##-$(CH_2)_1$-N($R_{g10}$)-$(CH_2)_2$-, ##-$(CH_2)_1$-N($R_{g10}$)-$(CH_2)_3$-, ##-$(CH_2)_1$-N($R_{g10}$)-$(CH_2)_4$-, ##-$(CH_2)_1$-N($R_{g10}$)-$(CH_2)_5$-, ##-$(CH_2)_1$-N($R_{g10}$)-$(CH_2)_6$-, ##-$(CH_2)_1$-N($R_{g10}$)-$(CH_2)_7$-, ##-$(CH_2)_1$-N($R_{g10}$)-$(CH_2)_8$-, ##-$(CH_2)_1$-N($R_{g10}$)-$(CH_2)_9$-, ##-$(CH_2)_1$-N($R_{g10}$)-$(CH_2)_{10}$-, ##-$(CH_2)_2$-N($R_{g10}$)-$(CH_2)_1$-, ##-$(CH_2)_2$-N($R_{g10}$)-$(CH_2)_2$-, ##-$(CH_2)_2$-N($R_{g10}$)-$(CH_2)_3$-, ##-$(CH_2)_2$-N($R_{g10}$)-$(CH_2)_4$-, ##-$(CH_2)_2$-N($R_{g10}$)-$(CH_2)_5$-, ##-$(CH_2)_2$-N($R_{g10}$)-$(CH_2)_6$-, ##-$(CH_2)_2$-N($R_{g10}$)-$(CH_2)_7$-, ##-$(CH_2)_2$-N($R_{g10}$)-$(CH_2)_8$-, ##-$(CH_2)_2$-N($R_{g10}$)-$(CH_2)_9$-, ##-$(CH_2)_2$-N($R_{g10}$)-$(CH_2)_{10}$-, ##-$(CH_2)_2$-N($R_{g10}$)-$(CH_2)_{11}$-, ##-$(CH_2)_2$-N($R_{g10}$)-$(CH_2)_{12}$-, ##-$(CH_2)_3$-N($R_{g10}$)-$(CH_2)_1$-, ##-$(CH_2)_3$-N($R_{g10}$)-$(CH_2)_2$-, ##-$(CH_2)_3$-N($R_{g10}$)-$(CH_2)_3$-, ##-$(CH_2)_4$-N($R_{g10}$)-$(CH_2)_1$-, ##-$(CH_2)_4$-N($R_{g10}$)-$(CH_2)_2$-, ##-$(CH_2)_4$-N($R_{g10}$)-$(CH_2)_3$-, ##-$(CH_2)_4$-N($R_{g10}$)-$(CH_2)_4$-, ##-$(CH_2)_5$-N($R_{g10}$)-$(CH_2)_1$-, ##-$(CH_2)_5$-N($R_{g10}$)-$(CH_2)_2$-, ##-$(CH_2)_5$-N($R_{g10}$)-$(CH_2)_3$-, ##-$(CH_2)_5$-N($R_{g10}$)-$(CH_2)_4$-, ##-$(CH_2)_5$-N($R_{g10}$)-$(CH_2)_5$-, ##-$(CH_2)_6$-N($R_{g10}$)-$(CH_2)_1$-, ##-$(CH_2)_6$-N($R_{g10}$)-$(CH_2)_2$-, ##-$(CH_2)_6$-N($R_{g10}$)-$(CH_2)_3$-, ##-$(CH_2)_7$-N($R_{g10}$)-$(CH_2)_1$-, ##-$(CH_2)_7$-N($R_{g10}$)-$(CH_2)_2$-, ##-$(CH_2)_7$-N($R_{g10}$)-$(CH_2)_3$-, ##-$(CH_2)_8$-N($R_{g10}$)-$(CH_2)_1$-, ##-$(CH_2)_8$-N $R_{g10}$)-$(CH_2)_2$-, ##-$(CH_2)_8$-N($R_{g10}$)-$(CH_2)_3$-, ##-CH($CH_3$)-N($R_{g10}$)-$(CH_2)_1$-, ##-CH($CH_3$)-N($R_{g10}$)-$(CH_2)_2$-, ##-CH($CH_3$)-N($R_{g10}$)-$(CH_2)_3$-, ##-CH($CH_3$)-N($R_{g10}$)-$(CH_2)_4$-, ##-CH($CH_3$)-N($R_{g10}$)-$(CH_2)_5$-, ##-CH($CH_3$)-N($R_{g10}$)-$(CH_2)_6$-, ##-CH($CH_3$)-N($R_{g10}$)-$(CH_2)_7$-, ##-CH($CH_3$)-N($R_{g10}$)-$(CH_2)_8$-, ##-CH($CH_3$)-N($R_{g10}$)-$(CH_2)_9$-, ##-CH($CH_3$)-N($R_{g10}$)-$(CH_2)_{10}$-, ##-$CH_2$C(O)NH$CH_2$-, ##-$(CH_2)_2$C(O)NH$(CH_2)_2$-, ##-$(CH_2)_2$C(O)NH$(CH_2)_3$-, ##-$(CH_2)_2$C(O)NH$(CH_2)_4$-, ##-$(CH_2)_2$C(O)NH$(CH_2)_5$-, ##-$(CH_2)_3$C(O)NH$(CH_2)_3$-, ##-$(CH_2)_3$C(O)NH$(CH_2)_4$-, ##-$(CH_2)_4$C(O)NH$(CH_2)_4$-, ##-$(CH_2)_5$C(O)NH$(CH_2)_5$-, ##-$(CH_2)_6$C(O)NH$(CH_2)_7$-, ##-$(CH_2)_6$C(O)NH$(CH_2)_6$-, ##-$(CH_2)_7$C(O)NH$(CH_2)_7$-, ##-$(CH_2)_8$C(O)NH$(CH_2)_8$, ##-$(CH_2)_9$C(O)NH$(CH_2)_9$-, ##-$(CH_2)_{10}$C(O)NH$(CH_2)_{10}$-, ##-$(CH_2)_2$C(O)NH$(CH_2)_2$-O-$(CH_2)_2$-, ##-$CH_2$NHC(O)$CH_2$-, ##-$(CH_2)_2$NHC(O)$(CH_2)_2$-, ##-$(CH_2)_2$NHC(O)$(CH_2)_3$-, ##-$(CH_2)_2$NHC(O)$(CH_2)_4$-, ##-$(CH_2)_2$NHC(O)$(CH_2)_5$-, ##-$(CH_2)_3$NHC(O)$(CH_2)_3$-, ##-$(CH_2)_3$NHC(O)$(CH_2)_4$-, ##-$(CH_2)_4$NHC(O)$(CH_2)_4$-, ##-$(CH_2)_5$NHC(O)$(CH_2)_5$-, ##-$(CH_2)_6$NHC(O)$(CH_2)_7$-, ##-$(CH_2)_6$NHC(O)$(CH_2)_6$-, ##-$(CH_2)_7$NHC(O)$(CH_2)_7$-, ##-$(CH_2)_8$NHC(O)$(CH_2)_8$, ##-$(CH_2)_9$NHC(O)$(CH_2)_9$-, ##-$(CH_2)_{10}$NHC(O)$(CH_2)_{10}$-, ##-$(CH_2)_4$NHC(O)$(CH_2)_8$-, ##-$(CH_2)_2$NHC(O)$(CH_2)_2$-O-$(CH_2)_2$-, ##-$(CH_2)_4$NHC(O)$CH_2$-, ##-$CH_2$-piperidinylene-$CH_2$-, ##-$CH_2$-piperidinylene-$(CH_2)_2$-, ##-$CH_2$-piperidinylene-$(CH_2)_3$-, ##-$CH_2$-piperidinylene-$(CH_2)_4$-, ##-$CH_2$-piperidinylene-$(CH_2)_5$-, ##-$CH_2$-piperidinylene-$(CH_2)_6$-, ##-$CH_2$-piperidinylene-$(CH_2)_7$-, ##-$CH_2$-piperidinylene-$(CH_2)_8$-, ##-$(CH_2)_2$-piperidinylene-$(CH_2)_1$-,

##-$(CH_2)_2$-piperidinylene-$(CH_2)_2$-, ##-$(CH_2)_2$-piperidinylene-$(CH_2)_3$-, ##-$(CH_2)_2$-piperidinylene-$(CH_2)_4$-, ##-$(CH_2)_2$-piperidinylene-$(CH_2)_5$-, ##-$(CH_2)_2$-piperidinylene-$(CH_2)_6$-, ##-$(CH_2)_2$-piperidinylene-$(CH_2)_7$-, ##-$(CH_2)_2$-piperidinylene-$(CH_2)_8$-, ##-$(CH_2)_3$-piperidinylene-$CH_2$-, ##-$(CH_2)_3$-piperidinylene-$(CH_2)_2$-, ##-$(CH_2)_3$-piperidinylene-$(CH_2)_3$-, ##-$(CH_2)_3$-piperidinylene-$(CH_2)_4$-, ##-$(CH_2)_3$-piperidinylene-$(CH_2)_5$-, ##-$(CH_2)_3$-piperidinylene-$(CH_2)_6$-, ##-$(CH_2)_3$-piperidinylene-$(CH_2)_7$-, ##-$(CH_2)_3$-piperidinylene-$(CH_2)_8$-, ##-$(CH_2)_4$-piperidinylene-$CH_2$-, ##-$(CH_2)_4$-piperidinylene-$(CH_2)_2$-, ##-$(CH_2)_4$-piperidinylene-$(CH_2)_3$-, ##-$(CH_2)_4$-piperidinylene-$(CH_2)_4$-, ##-$(CH_2)_4$-piperidinylene-$(CH_2)_5$-, ##-$(CH_2)_4$-piperidinylene-$(CH_2)_6$-, ##-$(CH_2)_4$-piperidinylene-$(CH_2)_7$-, ##-$(CH_2)_4$-piperidinylene-$(CH_2)_8$-, ##-$(CH_2)_5$-piperidinylene-$(CH_2)_1$-, ##-$(CH_2)_5$-piperidinylene-$(CH_2)_2$-, ##-$(CH_2)_5$-piperidinylene-$(CH_2)_3$-, ##-$(CH_2)_5$-piperidinylene-$(CH_2)_4$-, ##-$(CH_2)_5$-piperidinylene-$(CH_2)_5$-, ##-$(CH_2)_5$-piperidinylene-$(CH_2)_6$-, ##-$(CH_2)_5$-piperidinylene-$(CH_2)_7$-, ##-$(CH_2)_5$-piperidinylene-$(CH_2)_8$-, ##-$(CH_2)_6$-piperidinylene-$(CH_2)_1$-, ##-$(CH_2)_6$-piperidinylene-$(CH_2)_2$-, ##-$(CH_2)_6$-piperidinylene-$(CH_2)_3$-, ##-$(CH_2)_6$-piperidinylene-$(CH_2)_4$-, ##-$(CH_2)_6$-piperidinylene-$(CH_2)_5$-, ##-$(CH_2)_6$-piperidinylene-$(CH_2)_6$-, ##-$(CH_2)_6$-piperidinylene-$(CH_2)_7$-, ##-$(CH_2)_6$-piperidinylene-$(CH_2)_8$-, ##-$(CH_2)_7$-piperidinylene-$(CH_2)_1$-, ##-$(CH_2)_7$-piperidinylene-$(CH_2)_2$-, ##-$(CH_2)_7$-piperidinylene-$(CH_2)_3$-, ##-$(CH_2)_7$-piperidinylene-$(CH_2)_4$-, ##-$(CH_2)_7$-piperidinylene-$(CH_2)$ s-, ##-$(CH_2)_8$-piperidinylene-$CH_2$-, ##-$(CH_2)_8$-piperidinylene-$(CH_2)_2$-, ##-$(CH_2)_8$-piperidinylene-$(CH_2)_3$-, ##-$(CH_2)_8$-piperidinylene-$(CH_2)_4$-, ##-$(CH_2)_8$-piperidinylene-$(CH_2)_5$-, ##-$(CH_2)_8$-piperidinylene-$(CH_2)_6$-, ##-$(CH_2)_8$-piperidinylene-$(CH_2)_7$-, ##-$(CH_2)_8$-piperidinylene-$(CH_2)_8$-, ##-$CH_2$-piperazinylene-$CH_2$-, ##-$CH_2$-piperazinylene-$(CH_2)_2$-, ##-$CH_2$-piperazinylene-$(CH_2)_3$-, ##-$CH_2$-piperazinylene-$(CH_2)_4$-, ##-$CH_2$-piperazinylene-$(CH_2)_5$-, ##-$CH_2$-piperazinylene-$(CH_2)_6$-, ##-$CH_2$-piperazinylene-$(CH_2)_7$-, ##-$CH_2$-piperazinylene-$(CH_2)_8$-, ##-$(CH_2)_2$-piperazinylene-$(CH_2)_1$-, ##-$(CH_2)_2$-piperazinylene-$(CH_2)_2$-, ##-$(CH_2)_2$-piperazinylene-$(CH_2)_3$-, ##-$(CH_2)_2$-piperazinylene-$(CH_2)_4$-, ##-$(CH_2)_2$-piperazinylene-$(CH_2)_5$-, ##-$(CH_2)_2$-piperazinylene-$(CH_2)_6$-, ##-$(CH_2)_2$-piperazinylene-$(CH_2)_7$-, ##-$(CH_2)_2$-piperazinylene-$(CH_2)_8$-, ##-$(CH_2)_3$-piperazinylene-$CH_2$-, ##-$(CH_2)_3$-piperazinylene-$(CH_2)_2$-, ##-$(CH_2)_3$-piperazinylene-$(CH_2)_3$-, ##-$(CH_2)_3$-piperazinylene-$(CH_2)_4$-, ##-$(CH_2)_3$-piperazinylene-$(CH_2)_5$-, ##-$(CH_2)_3$-piperazinylene-$(CH_2)_6$-, ##-$(CH_2)_3$-piperazinylene-$(CH_2)_7$-, ##-$(CH_2)_3$-piperazinylene-$(CH_2)_8$-, ##-$(CH_2)_4$-piperazinylene-$CH_2$-, ##-$(CH_2)_4$-piperazinylene-$(CH_2)_2$-, ##-$(CH_2)_4$-piperazinylene-$(CH_2)_3$-, ##-$(CH_2)_4$-piperazinylene-$(CH_2)_4$-, ##-$(CH_2)_4$-piperazinylene-$(CH_2)_5$-, ##-$(CH_2)_4$-piperazinylene-$(CH_2)_6$-, ##-$(CH_2)_4$-piperazinylene-$(CH_2)_7$-, ##-$(CH_2)_4$-piperazinylene-$(CH_2)_8$-, ##-$(CH_2)_5$-piperazinylene-$(CH_2)_1$-, ##-$(CH_2)_5$-piperazinylene-$(CH_2)_2$-, ##-$(CH_2)_5$-piperazinylene-$(CH_2)_3$-, ##-$(CH_2)_5$-piperazinylene-$(CH_2)_4$-, ##-$(CH_2)_5$-piperazinylene-$(CH_2)_5$-, ##-$(CH_2)_5$-piperazinylene-$(CH_2)_6$-, ##-$(CH_2)_5$-piperazinylene-$(CH_2)_7$-, ##-$(CH_2)_5$-piperazinylene-$(CH_2)_8$-, ##-$(CH_2)_6$-piperazinylene-$(CH_2)_1$-, ##-$(CH_2)_6$-piperazinylene-$(CH_2)_2$-, ##-$(CH_2)_6$-piperazinylene-$(CH_2)_3$-, ##-$(CH_2)_6$-piperazinylene-$(CH_2)_4$-, ##-$(CH_2)_6$-piperazinylene-$(CH_2)_5$-, ##-$(CH_2)_6$-piperazinylene-$(CH_2)_6$-, ##-$(CH_2)_6$-piperazinylene-$(CH_2)_7$-, ##-$(CH_2)_6$-piperazinylene-$(CH_2)_8$-, ##-$(CH_2)_7$-piperazinylene-$(CH_2)_1$-, ##-$(CH_2)_7$-piperazinylene-$(CH_2)_2$-, ##-$(CH_2)_7$-piperazinylene-$(CH_2)_3$-, ##-$(CH_2)_7$-piperazinylene-$(CH_2)_4$-, ##-$(CH_2)_7$-piperazinylene-$(CH_2)_8$-, ##-$(CH_2)_8$-piperazinylene-$CH_2$-, ##-$(CH_2)_8$-piperazinylene-$(CH_2)_2$-, ##-$(CH_2)_8$-piperazinylene-$(CH_2)_3$-, ##-$(CH_2)_8$-piperazinylene-$(CH_2)_4$-, ##-$(CH_2)_8$-piperazinylene-$(CH_2)_5$-, ##-$(CH_2)_8$-piperazinylene-$(CH_2)_6$-, ##-$(CH_2)_8$-piperazinylene-$(CH_2)_7$-, ##-$(CH_2)_8$-piperazinylene-$(CH_2)_8$-, ##-$CH_2$-propylene-$CH_2$-, ##-$CH_2$-propylene-$(CH_2)_2$-, ##-$CH_2$-propylene-$(CH_2)_3$-, ##-$CH_2$-propylene-$(CH_2)_4$-, ##-$CH_2$-propylene-$(CH_2)_5$-, ##-$CH_2$-propylene-$(CH_2)_6$-, ##-$CH_2$-propylene-$(CH_2)_7$-, ##-$CH_2$-propylene-$(CH_2)_8$-, ##-$(CH_2)_2$-propylene-$(CH_2)_1$-, ##-$(CH_2)_2$-propylene-$(CH_2)_2$-, ##-$(CH_2)_2$-propylene-$(CH_2)_3$-, ##-$(CH_2)_2$-propylene-$(CH_2)_4$-, ##-$(CH_2)_2$-propylene-$(CH_2)_5$-, ##-$(CH_2)_2$-propylene-$(CH_2)_6$-, ##-$(CH_2)_2$-propylene-$(CH_2)_7$-, ##-$(CH_2)_2$-propylene-$(CH_2)_8$-, ##-$(CH_2)_3$-propylene-$CH_2$-, ##-$(CH_2)_3$-propylene-$(CH_2)_2$-, ##-$(CH_2)_3$-propylene-$(CH_2)_3$-, ##-$(CH_2)_3$-propylene-$(CH_2)_4$-, ##-$(CH_2)_3$-propylene-$(CH_2)_5$-, ##-$(CH_2)_3$-propylene-$(CH_2)_6$-, ##-$(CH_2)_3$-propylene-$(CH_2)_7$-, ##-$(CH_2)_3$-propylene-$(CH_2)_8$-, ##-$(CH_2)_4$-propylene-$CH_2$-, ##-$(CH_2)_4$-propylene-$(CH_2)_2$-, ##-$(CH_2)_4$-propylene-$(CH_2)_3$-, ##-$(CH_2)_4$-propylene-$(CH_2)_4$-, ##-$(CH_2)_4$-propylene-$(CH_2)_5$-, ##-$(CH_2)_4$-propylene-$(CH_2)_6$-, ##-$(CH_2)_4$-propylene-$(CH_2)_7$-, ##-$(CH_2)_4$-propylene-$(CH_2)_8$-, ##-$(CH_2)_5$-propylene-$(CH_2)_1$-, ##-$(CH_2)_5$-propylene-$(CH_2)_2$-, ##-$(CH_2)_5$-propylene-$(CH_2)_3$-, ##-$(CH_2)_5$-propylene-$(CH_2)_4$-, ##-$(CH_2)_5$-propylene-$(CH_2)_5$-, ##-$(CH_2)_5$-propylene-$(CH_2)_6$-, ##-$(CH_2)_5$-propylene-$(CH_2)_7$-, ##-$(CH_2)_5$-propylene-$(CH_2)_8$-, ##-$(CH_2)_6$-propylene-$(CH_2)_1$-, ##-$(CH_2)_6$-propylene-$(CH_2)_2$-, ##-$(CH_2)_6$-propylene-$(CH_2)_3$-, ##-$(CH_2)_6$-propylene-$(CH_2)_4$-, ##-$(CH_2)_6$-propylene-$(CH_2)_5$-, ##-$(CH_2)_6$-propylene-$(CH_2)_6$-, ##-$(CH_2)_6$-propylene-$(CH_2)_7$-, ##-$(CH_2)_6$-propylene-$(CH_2)_8$-, ##-$(CH_2)_7$-propylene-$(CH_2)_1$-, ##-$(CH_2)_7$-propylene-$(CH_2)_2$-, ##-$(CH_2)_7$-propylene-$(CH_2)_3$-, ##-$(CH_2)_7$-propylene-$(CH_2)_4$-, ##-$(CH_2)_7$-propylene-$(CH_2)$s-, ##-$(CH_2)_8$-propylene-$CH_2$-, ##-$(CH_2)_8$-propylene-$(CH_2)_2$-, ##-$(CH_2)_8$-propylene-$(CH_2)_3$-, ##-$(CH_2)_8$-propylene-$(CH_2)_4$-, ##-$(CH_2)_8$-propylene-$(CH_2)_5$-, ##-$(CH_2)_8$-propylene-$(CH_2)_6$-, ##-$(CH_2)_8$-propylene-$(CH_2)_7$-, ##-$(CH_2)_8$-propylene-$(CH_2)_8$-, ##-$CH_2$-diazacycloheptanylene-$CH_2$-, ##-$CH_2$-diazacycloheptanylene-$(CH_2)_2$-, ##-$CH_2$-diazacycloheptanylene-$(CH_2)_3$-, ##-$CH_2$-diazacycloheptanylene-$(CH_2)_4$-, ##-$CH_2$-diazacycloheptanylene-$(CH_2)_5$-, ##-$CH_2$-diazacycloheptanylene-$(CH_2)_6$-, ##-$CH_2$-diazacycloheptanylene-$(CH_2)_7$-, ##-$CH_2$-diazacycloheptanylene-$(CH_2)_8$-, ##-$(CH_2)_2$-diazacycloheptanylene-$(CH_2)_1$-, ##-$(CH_2)_2$-diazacycloheptanylene-$(CH_2)_2$-, ##-$(CH_2)_2$-diazacycloheptanylene-$(CH_2)_3$-, ##-$(CH_2)_2$-diazacycloheptanylene-$(CH_2)_4$-, ##-$(CH_2)_2$-diazacycloheptanylene-$(CH_2)_5$-, ##-$(CH_2)_2$-diazacyclohep-

tanylene-$(CH_2)_6$-, ##-$(CH_2)_2$-diazacycloheptanylene-$(CH_2)_7$-, ##-$(CH_2)_2$-diazacycloheptanylene-$(CH_2)_8$-, ##-$(CH_2)_3$-diazacycloheptanylene-$CH_2$-, ##-$(CH_2)_3$-diazacycloheptanylene-$(CH_2)_2$-, ##-$(CH_2)_3$-diazacycloheptanylene-$(CH_2)_3$-, ##-$(CH_2)_3$-diazacycloheptanylene-$(CH_2)_4$-, ##-$(CH_2)_3$-diazacycloheptanylene-$(CH_2)_5$-, ##-$(CH_2)_3$-diazacycloheptanylene-$(CH_2)_6$-, ##-$(CH_2)_3$-diazacycloheptanylene-$(CH_2)_7$-, ##-$(CH_2)_3$-diazacycloheptanylene-$(CH_2)_8$-, ##-$(CH_2)_4$-diazacycloheptanylene-$CH_2$-, ##-$(CH_2)_4$-diazacycloheptanylene-$(CH_2)_2$-, ##-$(CH_2)_4$-diazacycloheptanylene-$(CH_2)_3$-, ##-$(CH_2)_4$-diazacycloheptanylene-$(CH_2)_4$-, ##-$(CH_2)_4$-diazacycloheptanylene-$(CH_2)_5$-, ##-$(CH_2)_4$-diazacycloheptanylene-$(CH_2)_6$-, ##-$(CH_2)_4$-diazacycloheptanylene-$(CH_2)_7$-, ##-$(CH_2)_4$-diazacycloheptanylene-$(CH_2)_8$-, ##-$(CH_2)_5$-diazacycloheptanylene-$(CH_2)_1$-, ##-$(CH_2)_5$-diazacycloheptanylene-$(CH_2)_2$-, ##-$(CH_2)_5$-diazacycloheptanylene-$(CH_2)_3$-, ##-$(CH_2)_5$-diazacycloheptanylene-$(CH_2)_4$-, ##-$(CH_2)_5$-diazacycloheptanylene-$(CH_2)_5$-, ##-$(CH_2)_5$-diazacycloheptanylene-$(CH_2)_6$-, ##-$(CH_2)_5$-diazacycloheptanylene-$(CH_2)_7$-, ##-$(CH_2)_5$-diazacycloheptanylene-$(CH_2)_8$-, ##-$(CH_2)_6$-diazacycloheptanylene-$(CH_2)_1$-, ##-$(CH_2)$e-diazacycloheptanylene-$(CH_2)_2$-, ##-$(CH_2)_6$-diazacycloheptanylene-$(CH_2)_3$-, ##-$(CH_2)_6$-diazacycloheptanylene-$(CH_2)_4$-, ##-$(CH_2)_6$-diazacycloheptanylene-$(CH_2)_5$-, ##-$(CH_2)_6$-diazacycloheptanylene-$(CH_2)_6$-, ##-$(CH_2)_6$-diazacycloheptanylene-$(CH_2)_7$-, ##-$(CH_2)_6$-diazacycloheptanylene-$(CH_2)_8$-, ##-$(CH_2)_7$-diazacycloheptanylene-$(CH_2)_1$-, ##-$(CH_2)_7$-diazacycloheptanylene-$(CH_2)_2$-, ##-$(CH_2)_7$-diazacycloheptanylene-$(CH_2)_3$-, ##-$(CH_2)_7$-diazacycloheptanylene-$(CH_2)_4$-, ##-$(CH_2)_7$-diazacycloheptanylene-$(CH_2)_8$-, ##-$(CH_2)_8$-diazacycloheptanylene-$CH_2$-, ##-$(CH_2)_8$-diazacycloheptanylene-$(CH_2)_2$-, ##-$(CH_2)_8$-diazacycloheptanylene-$(CH_2)_3$-, ##-$(CH_2)_8$-diazacycloheptanylene-$(CH_2)_4$-, ##-$(CH_2)_8$-diazacycloheptanylene-$(CH_2)_5$-, ##-$(CH_2)_8$-diazacycloheptanylene-$(CH_2)_6$-, ##-$(CH_2)$s-diazacycloheptanylene-$(CH_2)_7$-, ##-$(CH_2)_8$-diazacycloheptanylene-$(CH_2)_8$-, ##-$CH_2$-diazabicyclo[2.2.1]heptanylene-$CH_2$-, ##-$CH_2$-diazabicyclo[2.2.1]heptanylene-$(CH_2)_2$-, ##-$CH_2$-diazabicyclo[2.2.1]heptanylene-$(CH_2)_3$-, ##-$CH_2$-diazabicyclo[2.2.1]heptanylene-$(CH_2)_4$-, ##-$CH_2$-diazabicyclo[2.2.1]heptanylene-$(CH_2)_5$-, ##-$CH_2$-diazabicyclo[2.2.1]heptanylene-$(CH_2)_6$-, ##-$CH_2$-diazabicyclo[2.2.1]heptanylene-$(CH_2)_7$-, ##-$CH_2$-diazabicyclo[2.2.1]heptanylene-$(CH_2)_8$-, ##-$(CH_2)_2$-diazabicyclo[2.2.1]heptanylene-$(CH_2)_1$-, ##-$(CH_2)_2$-diazabicyclo[2.2.1]heptanylene-$(CH_2)_2$-, ##-$(CH_2)_2$-diazabicyclo[2.2.1]heptanylene-$(CH_2)_3$-, ##-$(CH_2)_2$-diazabicyclo[2.2.1]heptanylene-$(CH_2)_4$-, ##-$(CH_2)_2$-diazabicyclo[2.2.1]heptanylene-$(CH_2)_5$-, ##-$(CH_2)_2$-diazabicyclo[2.2.1]heptanylene-$(CH_2)_6$-, ##-$(CH_2)_2$-diazabicyclo[2.2.1]heptanylene-$(CH_2)_7$-, ##-$(CH_2)_2$-diazabicyclo[2.2.1]heptanylene-$(CH_2)_8$-, ##-$(CH_2)_3$-diazabicyclo[2.2.1]heptanylene-$CH_2$-, ##-$(CH_2)_3$-diazabicyclo[2.2.l]heptanylene-$(CH_2)_2$-, ##-$(CH_2)_3$-diazabicyclo[2.2.1]heptanylene-$(CH_2)_3$-, ##-$(CH_2)_3$-diazabicyclo[2.2.1]heptanylene-$(CH_2)_4$-, ##-$(CH_2)_3$-diazabicyclo[2.2.1]heptanylene-$(CH_2)_5$-, ##-$(CH_2)_3$-diazabicyclo[2.2.1]heptanylene-$(CH_2)_6$-, ##-$(CH_2)_3$-diazabicyclo[2.2.1]heptanylene-$(CH_2)_7$-, ##-$(CH_2)_3$-diazabicyclo[2.2.1]heptanylene-$(CH_2)_8$-, ##-$(CH_2)_4$-diazabicyclo[2.2.1]heptanylene-$CH_2$-, ##-$(CH_2)_4$-diazabicyclo[2.2.1]heptanylene-$(CH_2)_2$-, ##-$(CH_2)_4$-diazabicyclo[2.2.1]heptanylene-$(CH_2)_3$-, ##-$(CH_2)_4$-diazabicyclo[2.2.1]heptanylene-$(CH_2)_4$-, ##-$(CH_2)_4$-diazabicyclo[2.2.1]heptanylene-$(CH_2)_5$-, ##-$(CH_2)_4$-diazabicyclo[2.2.1]heptanylene-$(CH_2)_6$-, ##-$(CH_2)_4$-diazabicyclo[2.2.1]heptanylene-$(CH_2)_7$-, ##-$(CH_2)_4$-diazabicyclo[2.2.1]heptanylene-$(CH_2)_8$-, ##-$(CH_2)_5$-diazabicyclo[2.2.1]heptanylene-$(CH_2)_1$-, ##-$(CH_2)_5$-diazabicyclo[2.2.1]heptanylene-$(CH_2)_2$-, ##-$(CH_2)_5$-diazabicyclo[2.2.1]heptanylene-$(CH_2)_3$-, ##-$(CH_2)_5$-diazabicyclo[2.2.1]heptanylene-$(CH_2)_4$-, ##-$(CH_2)_5$-diazabicyclo[2.2.1]heptanylene-$(CH_2)_5$-, ##-$(CH_2)_5$-diazabicyclo[2.2.1]heptanylene-$(CH_2)_6$-, ##-$(CH_2)_5$-diazabicyclo[2.2.1]heptanylene-$(CH_2)_7$-, ##-$(CH_2)_5$-diazabicyclo[2.2.1]heptanylene-$(CH_2)_8$-, ##-$(CH_2)_6$-diazabicyclo[2.2.1]heptanylene-$(CH_2)_1$-, ##-$(CH_2)_6$-diazabicyclo[2.2.1]heptanylene-$(CH_2)_2$-, ##-$(CH_2)_6$-diazabicyclo[2.2.1]heptanylene-$(CH_2)_3$-, ##-$(CH_2)_6$-diazabicyclo[2.2.1]heptanylene-$(CH_2)_4$-, ##-$(CH_2)_6$-diazabicyclo[2.2.1]heptanylene-$(CH_2)_5$-, ##-$(CH_2)_6$-diazabicyclo[2.2.1]heptanylene-$(CH_2)_6$-, ##-$(CH_2)_6$-diazabicyclo[2.2.1]heptanylene-$(CH_2)_7$-, ##-$(CH_2)_6$-diazabicyclo[2.2.1]heptanylene-$(CH_2)_8$-, ##-$(CH_2)_7$-diazabicyclo[2.2.1]heptanylene-$(CH_2)_1$-, ##-$(CH_2)_7$-diazabicyclo[2.2.1]heptanylene-$(CH_2)_2$-, ##-$(CH_2)_7$-diazabicyclo[2.2.1]heptanylene-$(CH_2)_3$-, ##-$(CH_2)_7$-diazabicyclo[2.2.1]heptanylene-$(CH_2)_4$-, ##-$(CH_2)_7$-diazabicyclo[2.2.1]heptanylene-$(CH_2)_8$-, ##-$(CH_2)_8$-diazabicyclo[2.2.1]heptanylene-$CH_2$-, ##-$(CH_2)_8$-diazabicyclo[2.2.1]heptanylene-$(CH_2)_2$-, ##-$(CH_2)_8$-diazabicyclo[2.2.1]heptanylene-$(CH_2)_3$-, ##-$(CH_2)_8$-diazabicyclo[2.2.1]heptanylene-$(CH_2)_4$-, ##-$(CH_2)_8$-diazabicyclo[2.2.1]heptanylene-$(CH_2)_5$-, ##-$(CH_2)_8$-diazabicyclo[2.2.1]heptanylene-$(CH_2)_6$-, ##-$(CH_2)_8$-diazabicyclo[2.2.1]heptanylene-$(CH_2)_7$-, ##-$(CH_2)_8$-diazabicyclo[2.2.1]heptanylene-$(CH_2)_8$-, ##-$CH_2$-diazabicyclo[3.1.1]heptanylene-$CH_2$-, ##-$CH_2$-diazabicyclo[3.1.1]heptanylene-$(CH_2)_2$-, ##-$CH_2$-diazabicyclo[3.1.1]heptanylene-$(CH_2)_3$-, ##-$CH_2$-diazabicyclo[3.1.1]heptanylene-$(CH_2)_4$-, ##-$CH_2$-diazabicyclo[3.1.1]heptanylene-$(CH_2)_5$-, ##-$CH_2$-diazabicyclo[3.1.1]heptanylene-$(CH_2)_6$-, ##-$CH_2$-diazabicyclo[3.1.1]heptanylene-$(CH_2)_7$-, ##-$CH_2$-diazabicyclo[3.1.1]heptanylene-$(CH_2)_8$-, ##-$(CH_2)_2$-diazabicyclo[3.1.1]heptanylene-$(CH_2)_1$-, ##-$(CH_2)_2$-diazabicyclo[3.1.1]heptanylene-$(CH_2)_2$-, ##-$(CH_2)_2$-diazabicyclo[3.1.1]heptanylene-$(CH_2)_3$-, ##-$(CH_2)_2$-diazabicyclo[3.1.1]heptanylene-$(CH_2)_4$-, ##-$(CH_2)_2$-diazabicyclo[3.1.1]heptanylene-$(CH_2)_5$-, ##-$(CH_2)_2$-diazabicyclo[3.1.1]heptanylene-$(CH_2)_6$-, ##-$(CH_2)_2$-diazabicyclo[3.1.1]heptanylene-$(CH_2)_7$-, ##-$(CH_2)_2$-diazabicyclo[3.1.1]heptanylene-$(CH_2)_8$-, ##-$(CH_2)_3$-diazabicyclo[3.1.1]heptanylene-$CH_2$-, ##-$(CH_2)_3$-diazabicyclo[3.1.1]heptanylene-$(CH_2)_2$-, ##-$(CH_2)_3$-diazabicyclo[3.1.1]heptanylene-$(CH_2)_3$-, ##-$(CH_2)_3$-diazabicyclo[3.1.1]heptanylene-$(CH_2)_4$-, ##-$(CH_2)_3$-diazabicyclo[3.1.1]heptanylene-$(CH_2)_5$-, ##-$(CH_2)_3$-diazabicyclo[3.1.1]heptanylene-$(CH_2)_6$-,

##-$(CH_2)_3$-diazabicyclo[3.1.1]heptanylene-$(CH_2)_7$-, ##-$(CH_2)_3$-diazabicyclo[3.1.1]heptanylene-$(CH_2)_8$-, ##-$(CH_2)_4$-diazabicyclo[3.1.1]heptanylene-$CH_2$-, ##-$(CH_2)_4$-diazabicyclo[3.1.1]heptanylene-$(CH_2)_2$-, ##-$(CH_2)_4$-diazabicyclo[3.1.1]heptanylene-$(CH_2)_3$-, ##-$(CH_2)_4$-diazabicyclo[3.1.1]heptanylene-$(CH_2)_4$-, ##-$(CH_2)_4$-diazabicyclo[3.1.1]heptanylene-$(CH_2)_5$-, ##-$(CH_2)_4$-diazabicyclo[3.1.1]heptanylene-$(CH_2)_6$-, ##-$(CH_2)_4$-diazabicyclo[3.1.1]heptanylene-$(CH_2)_7$-, ##-$(CH_2)_4$-diazabicyclo[3.1.1]heptanylene-$(CH_2)_8$-, ##-$(CH_2)_5$-diazabicyclo[3.1.1]heptanylene-$(CH_2)_1$-, ##-$(CH_2)_5$-diazabicyclo[3.1.1]heptanylene-$(CH_2)_2$-, ##-$(CH_2)_5$-diazabicyclo[3.1.1]heptanylene-$(CH_2)_3$-, ##-$(CH_2)_5$-diazabicyclo[3.1.1]heptanylene-$(CH_2)_4$-, ##-$(CH_2)_5$-diazabicyclo[3.1.1]heptanylene-$(CH_2)_5$-, ##-$(CH_2)_5$-diazabicyclo[3.1.1]heptanylene-$(CH_2)_6$-, ##-$(CH_2)_5$-diazabicyclo[3.1.1]heptanylene-$(CH_2)_7$-, ##-$(CH_2)_5$-diazabicyclo[3.1.1]heptanylene-$(CH_2)_8$-, ##-$(CH_2)_6$-diazabicyclo[3.1.1]heptanylene-$(CH_2)_1$-, ##-$(CH_2)_6$-diazabicyclo[3.1.1]heptanylene-$(CH_2)_2$-, ##-$(CH_2)_6$-diazabicyclo[3.1.1]heptanylene-$(CH_2)_3$-, ##-$(CH_2)_6$-diazabicyclo[3.1.1]heptanylene-$(CH_2)_4$-, ##-$(CH_2)_6$-diazabicyclo[3.1.1]heptanylene-$(CH_2)_5$-, ##-$(CH_2)_6$-diazabicyclo[3.1.1]heptanylene-$(CH_2)_6$-, ##-$(CH_2)_6$-diazabicyclo[3.1.1]heptanylene-$(CH_2)_7$-, ##-$(CH_2)_6$-diazabicyclo[3.1.1]heptanylene-$(CH_2)_8$-, ##-$(CH_2)_7$-diazabicyclo[3.1.1]heptanylene-$(CH_2)_1$-, ##-$(CH_2)_7$-diazabicyclo[3.1.1]heptanylene-$(CH_2)_2$-, ##-$(CH_2)_7$-diazabicyclo[3.1.1]heptanylene-$(CH_2)_3$-, ##-$(CH_2)_7$-diazabicyclo[3.1.1]heptanylene-$(CH_2)_4$-, ##-$(CH_2)_7$-diazabicyclo[3.1.1]heptanylene-$(CH_2)_8$-, ##-$(CH_2)_5$-diazabicyclo[3.1.1]heptanylene-$CH_2$-, ##-$(CH_2)_5$-diazabicyclo[3.1.1]heptanylene-$(CH_2)_2$-, ##-$(CH_2)_5$-diazabicyclo[3.1.1]heptanylene-$(CH_2)_3$-, ##-$(CH_2)_8$-diazabicyclo[3.1.1]heptanylene-$(CH_2)_4$-, ##-$(CH_2)_8$-diazabicyclo[3.1.1]heptanylene-$(CH_2)_5$-, ##-$(CH_2)_5$-diazabicyclo[3.1.1]heptanylene-$(CH_2)_6$-, ##-$(CH_2)_8$-diazabicyclo[3.1.1]heptanylene-$(CH_2)_7$-, ##-$(CH_2)_8$-diazabicyclo[3.1.1]heptanylene-$(CH_2)_8$-, ##-$CH_2$-diazabicyclo[3.2.1]octylene-$CH_2$-, ##-$CH_2$-diazabicyclo[3.2.1]octylene-$(CH_2)_2$-, ##-$CH_2$-diazabicyclo[3.2.1]octylene-$(CH_2)_3$-, ##-$CH_2$-diazabicyclo[3.2.1]octylene-$(CH_2)_4$-, ##-$CH_2$-diazabicyclo[3.2.1]octylene-$(CH_2)_5$-, ##-$CH_2$-diazabicyclo[3.2.1]octylene-$(CH_2)_6$-, ##-$CH_2$-diazabicyclo[3.2.1]octylene-$(CH_2)_7$-, ##-$CH_2$-diazabicyclo[3.2.1]octylene-$(CH_2)_5$-, ##-$(CH_2)_2$-diazabicyclo[3.2.1]octylene-$(CH_2)_1$-, ##-$(CH_2)_2$-diazabicyclo[3.2.1]octylene-$(CH_2)_2$-, ##-$(CH_2)_2$-diazabicyclo[3.2.1]octylene-$(CH_2)_3$-, ##-$(CH_2)_2$-diazabicyclo[3.2.1]octylene-$(CH_2)_4$-, ##-$(CH_2)_2$-diazabicyclo[3.2.1]octylene-$(CH_2)_5$-, ##-$(CH_2)_2$-diazabicyclo[3.2.1]octylene-$(CH_2)_6$-, ##-$(CH_2)_2$-diazabicyclo[3.2.1]octylene-$(CH_2)_7$-, ##-$(CH_2)_2$-diazabicyclo[3.2.1]octylene-$(CH_2)_8$-, ##-$(CH_2)_3$-diazabicyclo[3.2.1]octylene-$CH_2$-, ##-$(CH_2)_3$-diazabicyclo[3.2.1]octylene-$(CH_2)_2$-, ##-$(CH_2)_3$-diazabicyclo[3.2.1]octylene-$(CH_2)_3$-, ##-$(CH_2)_3$-diazabicyclo[3.2.1]octylene-$(CH_2)_4$-, ##-$(CH_2)_3$-diazabicyclo[3.2.1]octylene-$(CH_2)_5$-, ##-$(CH_2)_3$-diazabicyclo[3.2.1]octylene-$(CH_2)_6$-, ##-$(CH_2)_3$-diazabicyclo[3.2.1]octylene-$(CH_2)_7$-, ##-$(CH_2)_3$-diazabicyclo[3.2.1]octylene-$(CH_2)_8$-, ##-$(CH_2)_4$-diazabicyclo[3.2.1]octylene-$CH_2$-, ##-$(CH_2)_4$-diazabicyclo[3.2. 1]octylene-$(CH_2)_2$-, ##-$(CH_2)_4$-diazabicyclo[3.2.1]octylene-$(CH_2)_3$-, ##-$(CH_2)_4$-diazabicyclo[3.2.1]octylene-$(CH_2)_4$-, ##-$(CH_2)_4$-diazabicyclo[3.2.1]octylene-$(CH_2)_5$-, ##-$(CH_2)_4$-diazabicyclo[3.2.1]octylene-$(CH_2)_6$-, ##-$(CH_2)_4$-diazabicyclo[3.2.1]octylene-$(CH_2)_7$-, ##-$(CH_2)_4$-diazabicyclo[3.2.1]octylene-$(CH_2)_8$-, ##-$(CH_2)_5$-diazabicyclo[3.2.1]octylene-$(CH_2)_1$-, ##-$(CH_2)_5$-diazabicyclo[3.2.1]octylene-$(CH_2)_2$-, ##-$(CH_2)_5$-diazabicyclo[3.2.1]octylene-$(CH_2)_3$-, ##-$(CH_2)_5$-diazabicyclo[3.2.1]octylene-$(CH_2)_4$-, ##-$(CH_2)_5$-diazabicyclo[3.2.1]octylene-$(CH_2)_5$-, ##-$(CH_2)_5$-diazabicyclo[3.2.1]octylene-$(CH_2)_6$-, ##-$(CH_2)_5$-diazabicyclo[3.2.1]octylene-$(CH_2)_7$-, ##-$(CH_2)_5$-diazabicyclo[3.2.1]octylene-$(CH_2)_8$-, ##-$(CH_2)_6$-diazabicyclo[3.2. 1]octylene-$(CH_2)_1$-, ##-$(CH_2)_6$-diazabicyclo[3.2.1]octylene-$(CH_2)_2$-, ##-$(CH_2)_6$-diazabicyclo[3.2.1]octylene-$(CH_2)_3$-, ##-$(CH_2)_6$-diazabicyclo[3.2.1]octylene-$(CH_2)_4$-, ##-$(CH_2)_6$-diazabicyclo[3.2.1]octylene-$(CH_2)_5$-, ##-$(CH_2)_6$-diazabicyclo[3.2.1]octylene-$(CH_2)_6$-, ##-$(CH_2)_6$-diazabicyclo[3.2.1]octylene-$(CH_2)_7$-, ##-$(CH_2)_6$-diazabicyclo[3.2.1]octylene-$(CH_2)_8$-, ##-$(CH_2)_7$-diazabicyclo[3.2.1]octylene-$(CH_2)_1$-, ##-$(CH_2)_7$-diazabicyclo[3.2.1]octylene-$(CH_2)_2$-, ##-$(CH_2)_7$-diazabicyclo[3.2.1]octylene-$(CH_2)_3$-, ##-$(CH_2)_7$-diazabicyclo[3.2.1]octylene-$(CH_2)_4$-, ##-$(CH_2)_7$-diazabicyclo[3.2. 1]octylene-$(CH_2)_5$-, ##-$(CH_2)_8$-diazabicyclo[3.2.1]octylene-$CH_2$-, ##-$(CH_2)_8$-diazabicyclo[3.2.1]octylene-$(CH_2)_2$-, ##-$(CH_2)_8$-diazabicyclo[3.2.1]octylene-$(CH_2)_3$-, ##-$(CH_2)_8$-diazabicyclo[3.2.1]octylene-$(CH_2)_4$-, ##-$(CH_2)_8$-diazabicyclo[3.2.1]octylene-$(CH_2)_5$-, ##-$(CH_2)_8$-diazabicyclo[3.2.1]octylene-$(CH_2)_6$-, ##-$(CH_2)_8$-diazabicyclo[3.2.1]octylene-$(CH_2)_7$-, ##-$(CH_2)_8$-diazabicyclo[3.2.1]octylene-$(CH_2)_8$-, ##-$CH_2$-diazabicyclo[2.2.2]octylene-$CH_2$-, ##-$CH_2$-diazabicyclo[2.2.2]octylene-$(CH_2)_2$-, ##-$CH_2$-diazabicyclo[2.2.2]octylene-$(CH_2)_3$-, ##-$CH_2$-diazabicyclo[2.2.2]octylene-$(CH_2)_4$-, ##-$CH_2$-diazabicyclo[2.2.2]octylene-$(CH_2)_5$-, ##-$CH_2$-diazabicyclo[2.2.2]octylene-$(CH_2)_6$-, ##-$CH_2$-diazabicyclo[2.2.2]octylene-$(CH_2)_7$-, ##-$CH_2$-diazabicyclo[2.2.2]octylene-$(CH_2)_8$-, ##-$(CH_2)_2$-diazabicyclo[2.2.2]octylene-$(CH_2)_1$-, ##-$(CH_2)_2$-diazabicyclo[2.2.2]octylene-$(CH_2)_2$-, ##-$(CH_2)_2$-diazabicyclo[2.2.2]octylene-$(CH_2)_3$-, ##-$(CH_2)_2$-diazabicyclo[2.2.2]octylene-$(CH_2)_4$-, ##-$(CH_2)_2$-diazabicyclo[2.2.2]octylene-$(CH_2)_5$-, ##-$(CH_2)_2$-diazabicyclo[2.2.2]octylene-$(CH_2)_6$-, ##-$(CH_2)_2$-diazabicyclo[2.2.2]octylene-$(CH_2)_7$-, ##-$(CH_2)_2$-diazabicyclo[2.2.2]octylene-$(CH_2)_8$-, ##-$(CH_2)_3$-diazabicyclo[2.2.2]octylene-$CH_2$-, ##-$(CH_2)_3$-diazabicyclo[2.2.2]octylene-$(CH_2)_2$-, ##-$(CH_2)_3$-diazabicyclo[2.2.2]octylene-$(CH_2)_3$-, ##-$(CH_2)_3$-diazabicyclo[2.2.2]octylene-$(CH_2)_4$-, ##-$(CH_2)_3$-diazabicyclo[2.2.2]octylene-$(CH_2)_5$-, ##-$(CH_2)_3$-diazabicyclo[2.2.2]octylene-$(CH_2)_6$-, ##-$(CH_2)_3$-diazabicyclo[2.2.2]octylene-$(CH_2)_7$-, ##-$(CH_2)_3$-diazabicyclo[2.2.2]octylene-$(CH_2)_8$-, ##-$(CH_2)_4$-diazabicyclo[2.2.2]octylene-$CH_2$-, ##-$(CH_2)_4$-diazabicyclo[2.2.2]octylene-$(CH_2)_2$-, ##-$(CH_2)_4$-diazabicyclo[2.2.2]octylene-$(CH_2)_3$-, ##-$(CH_2)_4$-diazabicyclo[2.2.2]octylene-$(CH_2)_4$-, ##-$(CH_2)_4$-diazabicyclo[2.2.2]octylene-$(CH_2)_5$-, ##-$(CH_2)_4$-diazabicyclo[2.2.2]octylene-$(CH_2)_6$-, ##-$(CH_2)_4$-diazabicyclo[2.2.2]octylene-$(CH_2)_7$-, ##-$(CH_2)_4$-diazabi-

cyclo[2.2.2]octylene-$(CH_2)_8$-, ##-$(CH_2)_5$-diazabicyclo[2.2.2]octylene-$(CH_2)_1$-, ##-$(CH_2)_5$-diazabicyclo[2.2.2]octylene-$(CH_2)_2$-, ##-$(CH_2)_5$-diazabicyclo[2.2.2]octylene-$(CH_2)_3$-, ##-$(CH_2)_5$-diazabicyclo[2.2.2]octylene-$(CH_2)_4$-, ##-$(CH_2)_5$-diazabicyclo[2.2.2]octylene-$(CH_2)_5$-, ##-$(CH_2)_5$-diazabicyclo[2.2.2]octylene-$(CH_2)_6$-, ##-$(CH_2)_5$-diazabicyclo[2.2.2]octylene-$(CH_2)_7$-, ##-$(CH_2)_5$-diazabicyclo[2.2.2]octylene-$(CH_2)_8$-, ##-$(CH_2)_6$-diazabicyclo[2.2.2]octylene-$(CH_2)$i-, ##-$(CH_2)_6$-diazabicyclo[2.2.2]octylene-$(CH_2)_2$-, ##-$(CH_2)_6$-diazabicyclo[2.2.2]octylene-$(CH_2)_3$-, ##-$(CH_2)_6$-diazabicyclo[2.2.2]octylene-$(CH_2)_4$-, ##-$(CH_2)_6$-diazabicyclo[2.2.2]octylene-$(CH_2)_5$-, ##-$(CH_2)_6$-diazabicyclo[2.2.2]octylene-$(CH_2)_6$-, ##-$(CH_2)_6$-diazabicyclo[2.2.2]octylene-$(CH_2)_7$-, ##-$(CH_2)_6$-diazabicyclo[2.2.2]octylene-$(CH_2)_8$-, ##-$(CH_2)_7$-diazabicyclo[2.2.2]octylene-$(CH_2)_1$-, ##-$(CH_2)_7$-diazabicyclo[2.2.2]octylene-$(CH_2)_2$-, ##-$(CH_2)_7$-diazabicyclo[2.2.2]octylene-$(CH_2)_3$-, ##-$(CH_2)_7$-diazabicyclo[2.2.2]octylene-$(CH_2)_4$-, ##-$(CH_2)_7$-diazabicyclo[2.2.2]octylene-$(CH_2)_8$-, ##-$(CH_2)_8$-diazabicyclo[2.2.2]octylene-$CH_2$-, ##-$(CH_2)_8$-diazabicyclo[2.2.2]octylene-$(CH_2)_2$-, ##-$(CH_2)$s-diazabicyclo[2.2.2]octylene-$(CH_2)_3$-, ##-$(CH_2)_8$-diazabicyclo[2.2.2]octylene-$(CH_2)_4$-, ##-$(CH_2)_8$-diazabicyclo[2.2.2]octylene-$(CH_2)_5$-, ##-$(CH_2)_8$-diazabicyclo[2.2.2]octylene-$(CH_2)_6$-, ##-$(CH_2)_8$-diazabicyclo[2.2.2]octylene-$(CH_2)_7$-, or ##-$(CH_2)_8$-diazabicyclo[2.2.2]octylene-$(CH_2)_8$-;

wherein the propylene, the piperidinylene, the piperazinylene, the diazacycloheptanylene, the diazabicyclo [2.2.1]heptanylene, the diazabicyclo[3.1.1]heptanylene, the diazabicyclo[3.2.1]octylene, the diazabicyclo[2.2.2] octylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;
each $R_{g10}$ independently represents H or $C_{1-3}$ alkyl; and
symbol ## indicates the point of attachment to $X_1$;

preferably, wherein $L_1$ represents the following group:

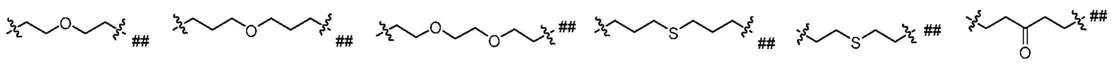

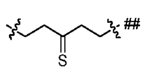 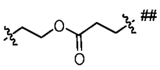 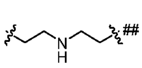 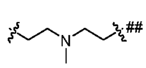 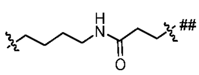 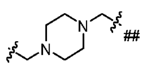 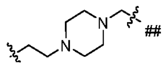

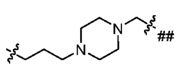 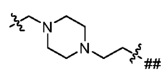 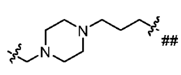 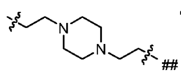 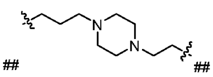 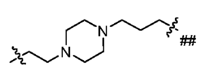

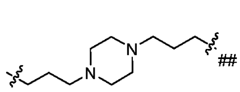 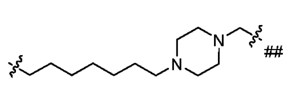 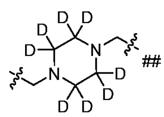 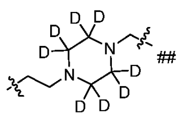 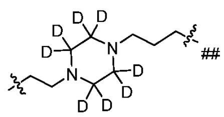

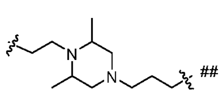 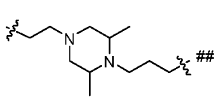 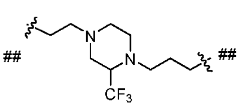 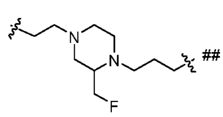 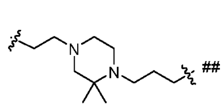

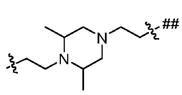 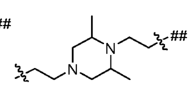 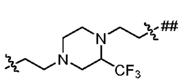 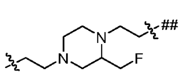 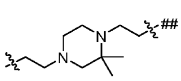 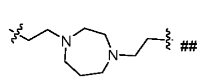

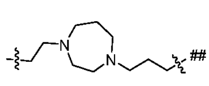 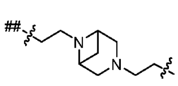 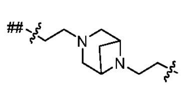 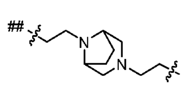 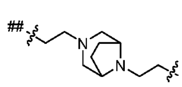 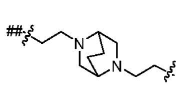

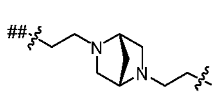 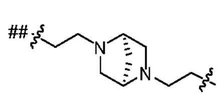 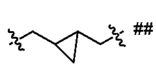 or 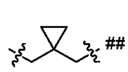 ;

wherein symbol ## indicates the point of attachment to $X_1$.

**29.** The compound of Formula (I) or salts, enantiomers, diastereoisomer, isotopically enriched analogs, solvates, prodrugs, or polymorphs thereof as claimed in any one of claims 4-6 and 8, wherein $L_1$ represents the structure of the following formula: ##-$C_{1-30}$ alkylene-, wherein a hydrogen atom of one or more $CH_2$ groups of the $C_{1-30}$ alkylene is optionally replaced with a substituent selected from the group consisting of: D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy or any combination thereof, and symbol ## indicates the point of attachment to $X_1$;

preferably, wherein $L_1$ represents the following group:
##-$CH_2$-, ##-$(CH_2)_2$-, ##-$(CH_2)_3$-, ##-$(CH_2)_4$-, ##-$(CH_2)_5$-, ##-$(CH_2)_2$-$CF_2$-$(CH_2)_2$-, ##-$(CH_2)_6$-, ##-$(CH_2)_7$-, ##-$(CH_2)_8$-, ##-$(CH_2)_9$-, ##-$(CH_2)_{10}$-, ##-$(CH_2)_{11}$-, ##-$(CH_2)_{12}$-, ##-$(CH_2)_{13}$-, ##-$(CH_2)_{14}$-, ##-$(CH_2)_{15}$-, ##-$(CH_2)_{16}$-, ##-$(CH_2)_{17}$-, ##-$(CH_2)_{18}$-, ##-$(CH_2)_{19}$-, ##-$(CH_2)_{20}$-, ##-$(CH_2)_{21}$-, ##-$(CH_2)_{22}$-, ##-$(CH_2)_{25}$-, or ##-$(CH_2)_{30}$-;
wherein the hydrogen atom of one or more $CH_2$ groups of the group is optionally replaced with a substituent selected from the group consisting of: D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy or any combination thereof, and symbol ## indicates the point of attachment to $X_1$.

**30.** The compound of Formula (I) or salts, enantiomers, diastereoisomer, isotopically enriched analogs, solvates, prodrugs, or polymorphs thereof as claimed in claim 7, wherein $L_1$ represents the structure of the following formula: ##-$C_{4-30}$ alkylene-, wherein a hydrogen atom of one or more $CH_2$ groups of the $C_{4-30}$ alkylene is optionally replaced with a substituent selected from the group consisting of: D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy or any combination thereof, and symbol ## indicates the point of attachment to $X_1$;

preferably, wherein $L_1$ represents the following group:
##-$(CH_2)_4$-, ##-$(CH_2)_5$-, ##-$(CH_2)_2$-$CF_2$-$(CH_2)_2$-, ##-$(CH_2)_6$-, ##-$(CH_2)_7$-, ##-$(CH_2)_8$-, ##-$(CH_2)_9$-, ##-$(CH_2)_{10}$-, ##-$(CH_2)_{11}$-, ##-$(CH_2)_{12}$-, ##-$(CH_2)_{13}$-, ##-$(CH_2)_{14}$-, ##-$(CH_2)_{15}$-, ##-$(CH_2)_{16}$-, ##-$(CH_2)_{17}$-, ##-$(CH_2)_{18}$-, ##-$(CH_2)_{19}$-, ##-$(CH_2)_{20}$-, ##-$(CH_2)_{21}$-, ##-$(CH_2)_{22}$-, ##-$(CH_2)_{25}$-, or ##-$(CH_2)_{30}$-;
wherein the hydrogen atom of one or more $CH_2$ groups of the group is optionally replaced with a substituent selected from the group consisting of: D, optionally deuterated $C_{1-4}$ alkyl, hydroxy, amino, mercapto, nitro, halogen, cyano, halogenated $C_{1-6}$ alkyl, optionally deuterated $C_{3-6}$cycloalkyl, $C_{1-6}$ alkoxy or any combination thereof, and symbol ## indicates the point of attachment to $X_1$.

**31.** The compound of Formula (I) or salts, enantiomers, diastereoisomer, isotopically enriched analogs, solvates, prodrugs, or polymorphs thereof as claimed in any one of claims 4-10, wherein $R_b$ represents the following group: $CH_3$, $CF_3$, $CD_3$, $CH_2CH_3$, -$(CH_2)_2$-$CH_3$, -$(CH_2)_3$-$CH_3$, -$(CH_2)_4$-$CH_3$, -$(CH_2)_5$-$CH_3$, -$(CH_2)_6$-$CH_3$,-$(CH_2)_7$-$CH_3$, -$(CH_2)_8$-$CH_3$, -$(CH_2)_9$-$CH_3$, -$(CH_Z)_{lo}$-$CH_3$, -$(CH_2)_{11}$-$CH_3$, -$(CH_2)_{12}$-$CH_3$, -$(CH_2)_{13}$-$CH_3$,-$(CH_2)_{14}$-$CH_3$, -$(CH_2)_{15}$-$CH_3$, -$(CH_2)_{16}$-$CH_3$, -$(CH_2)_{17}$-$CH_3$, -$(CH_Z)_{ls}$-$CH_3$, -$(CH_2)_{19}$-$CH_3$, -$(CH_2)_{20}$-$CH_3$, -$(CH_2)_{21}$-$CH_3$, -$(CH_2)_{22}$-$CH_3$, -$(CH_2)_{23}$-$CH_3$, -$(CH_2)_{24}$-$CH_3$, -$(CH_2)_{25}$-$CH_3$, -$(CH_2)_{26}$-$CH_3$, -$(CH_2)_{27}$-$CH_3$, -$(CH_2)_{28}$-$CH_3$, or -$(CH_2)_{29}$-$CH_3$.

**32.** The compound of Formula (I) or salts, enantiomers, diastereoisomer, isotopically enriched analogs, solvates, prodrugs, or polymorphs thereof as claimed in claim 1, wherein $L_2$ or $L_3$ each independently represent the structure of the following formula: -$C_{2-30}$ alkylene-, wherein a hydrogen atom of one or more $CH_2$ groups of the $C_{2-30}$ alkylene is optionally replaced with a substituent selected from the group consisting of: D, $C_{1-4}$ alkyl, halogen, $C_{3-6}$cycloalkyl or any combination thereof; or

preferably, wherein $L_2$ or $L_3$ each independently represent the following group:
-$CH_2$-, -$(CH_2)_2$-, -$(CH_2)_3$-, -$(CH_2)_4$-, -$(CH_2)_5$-, -$(CH_2)_2$-$CF_2$-$(CH_2)_2$-, -$(CH_2)_6$-, -$(CH_2)_7$-, -$(CH_2)_8$-,-$(CH_2)_9$-, -$(CH_2)_{10}$-, -$(CH_2)_{11}$-, -$(CH_2)_{12}$-, -$(CH_2)_{13}$-, -$(CH_2)_{14}$-, -$(CH_2)_{15}$-, -$(CH_2)_{16}$-, -$(CH_2)_{17}$-,-$(CH_2)_{18}$-, -$(CH_2)_{19}$-, -$(CH_2)_{20}$-, -$(CH_2)_{21}$-, -$(CH_2)_{22}$-, -$(CH_2)_{25}$-, or -$(CH_2)_{30}$-;
wherein the hydrogen atom of one or more $CH_2$ groups of the group is optionally replaced with a substituent selected from the group consisting of: D, $C_{1-4}$ alkyl, halogen, $C_{3-6}$cycloalkyl or any combination thereof.

**33.** The compound of Formula (I) or salts, enantiomers, diastereoisomer, isotopically enriched analogs, solvates, prodrugs, or polymorphs thereof as claimed in claim 1, wherein

(i) $L_2$ represents the structure of the following formula:

$-(C(R^{a9})(R^{a10}))_{m1}-(R_{12}(C(R^{a11})(R^{a12}))_{m2})_{p1}-$;

$-(C(R^{a9})(R^{a10}))_{m1}-(R_{12}(C(R^{a11})(R^{a12}))_{m2})_{p1}-R_{12}(R_{12}(C(R^{a13})(R^{a14}))_{m3})_{p2}-$;

$-(C(R^{a9})(R^{a10}))_{m1}-(R_{12}(C(R^{a11})(R^{a12}))_{m2})_{p1}-(R_{12}(C(R^{a13})(R^{a14}))_{m3})_{p2}-(R_{12}(C(R^{a15})(R^{a16}))_{m4})_{p3}-$;

$-(C(R^{a9})(R^{a10}))_{m1}-(R_{13}(C(R^{a11})(R^{a12}))_{m2})_{p1-}$;

$-(C(R^{a9})(R^{a10}))_{m1}-(R_{13}(C(R^{a11})(R^{a12}))_{m2})_{p1}-(R_{13}(C(R^{a13})(R^{a14}))_{m3})_{p2}-$;

$-(C(R^{a9})(R^{a10}))_{m1}-(R_{13}(C(Ra^{11})(R^{a12}))_{m2})_{p1}-(R_{13}(C(R^{a13})(R^{a14}))_{m3})_{p2}-(R_{13}(C(R^{a15})(R^{a16}))_{m4})_{p3}-$;

$-(C(R^{a9})(R^{a10}))_{m1}-(R_{12}-R_{13}-(C(R^{a11})(R^{a12}))_{m2})_{p1}-$;

$-(C(R^{a9})(R^{a10}))_{m1}-(R_{12}(C(R^{a11})(R^{a12}))_{m2})_{p1}-(R_{13}(C(R^{a13})(R^{a14}))_{m3})_{p2}-$;

$-(C(R^{a9})(R^{a10}))_{m1}-(R_{13}-R_{12}-(C(R^{a11})(R^{a12}))_{m2})_{p1-}$;

or

$-(C(R^{a9})(R^{a10}))_{m1}-(R_{13}(C(R^{a11})(R^{a12}))_{m2})_{p1}-(R_{12}(C(R^{a13})(R^{a14}))_{m3})_{p2}-$;

wherein each $R_{12}$ is independently selected from the group consisting of O, S, $N(R_{14})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_{14}$ independently represents H or $C_{1-3}$ alkyl; and each group $R_{13}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;

$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and

m1, m2, m3, m4, p1, p2, p3 are each independently selected from the group consisting of an integer of 1, 2, 3, 4, 5, 6, 7, 8, 9, 10, 11, 12, 13, 14, or 15; and/or

(ii) $L_3$ represents the structure of the following formula:

$-(C(R^{a9})(R^{a10}))_{m1}-(R_{16}(C(R^{a11})(R^{a12}))_{m2})_{p1}-$;

$-(C(R^{a9})(R^{a10}))_{m1}-(R_{16}(C(R^{a11})(R^{a12}))_{m2})_{p1}-(R_{16}(C(R^{a13})(R^{a14}))_{m3})_{p2}-$;

$-(C(R^{a9})(R^{a10}))_{m1}-(R_{16}(C(R^{a11})(R^{a12}))_{m2})_{p1}-(R_{16}(C(R^{a13})(R^{a14}))_{m3})_{p2}-(R_{16}(C(R^{a15})(R^{a16}))_{m4})_{p3}-$;

$-(C(R^{a9})(R^{a10}))_{m1}-(R_{17}(C(R^{a11})(R^{a12}))_{m2})_{p1}-$;

$-(C(R^{a9})(R^{a10}))_{m1}-(R_{17}(C(R^{a11})(R^{a12}))_{m2})_{p1}-(R_{17}(C(R^{a13})(R^{a14}))_{m3})_{p2}-$;

$-(C(R^{a9})(R^{a10}))_{m1}-(R_{17}(C(R^{a11})(R^{a12}))_{m2})_{p1}-(R_{17}(C(R^{a13})(R^{a14}))_{m3})_{p2}-(R_{17}(C(R^{a15})(R^{a16}))_{m4})_{p3}-$;

$-(C(R^{a9})(R^{a10}))_{m1}-(R_{16}-R_{17}-(C(R^{a11})(R^{a12}))_{m2})_{p1}-$;

$-(C(R^{a9})(R^{a10}))_{m1}-(R_{16}(C(R^{a11})(R^{a12}))_{m2})_{p1}-(R_{17}(C(R^{a13})(R^{a14}))_{m3})_{p2}-$;

$-(C(R^{a9})(R^{a10}))_{m1}-(R_{17}-R_{16}-(C(R^{a11})(R^{a12}))_{m2})_{p1}-$; or

$-(C(R^{a9})(R^{a10}))_{m1}-(R_{17}(C(R^{a11})(R^{a12}))_{m2})_{p1}-(R_{16}(C(R^{a13})(R^{a14}))_{m3})_{p2}-$;

wherein each group $R_{16}$ is independently selected from the group consisting of O, S, $N(R_{18})$, C(O), C(O)O, OC(O), C(O)NH, NHC(O), NHC(O)NH, C(S), S(O), $S(O)_2$, $S(O)_2O$, $OS(O)_2$, $S(O)_2NH$ or $NHS(O)_2$, wherein each $R_{18}$ independently represents H or $C_{1-3}$ alkyl; and each group $R_{17}$ is independently selected from the group consisting of optionally substituted $C_{3-15}$cycloalkylene, optionally substituted 4- to 15-membered heterocyclylene, optionally substituted $C_{6-10}$ arylene, optionally substituted 5- to 15-membered heteroarylene, $C_{2-6}$ alkenylene, $C_{2-6}$ alkynylene or any combination thereof, wherein the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;

$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and

m1, m2, m3, m4, p1, p2, p3 are each independently selected from the group consisting of an integer of 1, 2, 3, 4, 5, 6, 7, 8, 9, 10, 11, 12, 13, 14, or 15; or

preferably, wherein $L_2$ or $L_3$ each independently represent the structure of the following formula:

$-(C(R^{a9})(R^{a10})_{m1}-(O(C(R^{a11})(R^{a12}))_{m2})_{p1}-$;

$-(C(R^{a9})(R^{a10}))_{m1}-(O(C(R^{a11})(R^{a12}))_{m2})_{p1}-(O(C(R^{a13})(R^{a14}))_{m3})_{p2}-$;

$--(C(R^{a9})(R^{a10}))_{m1}-(O(C(R^{a11})(R^{a12}))_{m2})_{p1}-(O(C(R^{a13})(R^{a14}))_{m3})_{p2}-(O(C(R^{a15})(R^{a16}))_{m4})_{p3}-$;

$-(C(R^{a}9)(R^{a10}))_{m1}-(N(R_{g11})(C(R^{a11})(R^{a12}))_{m2})_{p1}-$;

$-(C(R^{a9})(R^{a10}))_{m1}-(N(R_{g11})(C(R^{a11})(R^{a12}))_{m2})_{p1}-(N(R_{g11})(C(R^{a13})(R^{a14}))_{m3})_{p2}-$;

$-(C(R^{a9})(R^{a10}))_{m1}-(N(R_{g11})(C(R^{a11})(R^{a12}))_{m2})_{p1}-(N(R_{g11})(C(R^{a13})(R^{a14}))_{m3})_{p2}-(N(R_{g11})(C(R^{a15})(R^{a16}))_{m4})_{p3}-$;

$-(C(R^{a9})(R^{a10}))_{m1}-(C(O)NH-(C(R^{a11})(R^{a12}))_{m2})_{p1}-$;

$-(C(R^{a9})(R^{a10}))_{m1}-(C(O)NH-(C(R^{a11})(R^{a12}))_{m2})_{p1}-(C(O)NH-(C(R^{a13})(R^{a14}))_{m3})_{p2}-$;

$-(C(R^{a9})(R^{a10}))_{m1}-(C(O)NH-(C(R^{a11})(R^{a12}))_{m2})_{p1}-(C(O)NH-(C(R^{a13})(R^{a14}))_{m3})_{p2}-(C(O)NH-(C(R^{a15})(R^{a16}))_{m4})_{p3}-$;

$-(C(R^{a9})(R^{a10}))_{m1}-(C(O)NH-(C(R^{a11})(R^{a12}))_{m2})_{p1}-(O-(C(R^{a13})(R^{a14}))_{m3})_{p2}-$;

$-(C(R^{a9})(R^{a10}))_{m1}-C(O)NH-(C(R^{a11})(R^{a12}))_{m2}-(O(C(R^{a13})(R^{a14}))_{m3})_{p1}-$;

$-(C(R^{a9})(R^{a10}))_{m1}-(NHC(O)-(C(R^{a11})(R^{a12}))_{m2})_{p1}-$;

$-(C(R^{a9})(R^{a10}))_{m1}-(NHC(O)-(C(R^{a11})(R^{a12}))_{m2})_{p1}-(O-(C(R^{a13})(R^{a14}))_{m3})_{p2}-$;

$-(C(R^{a9})(R^{a10}))_{m1}-(NHC(O)-(C(R^{a11})(R^{a12}))_{m2})_{p1}-(O-(C(R^{a13})(R^{a14}))_{m3})_{p2}-(O-C(R^{a15})(R^{a16}))_{m4})_{p3}-$;

$-(C(R^{a9})(R^{a10}))_{m1}$-NHC(O)-$(C(R^{a11})(R^{a12}))_{m2}$-$(O(C(R^{a13})(R^{a14}))_{m3})_{p1}$-;

$-(C(R^{a9})(R^{a10}))_{m1}$-NHC(O)NH-$(C(R^{a11})(R^{a12}))_{m2}$-;

$-(C(R^{a9})(R^{a10}))_{m1}$-(NHC(O)-$(C(R^{a11})(R^{a12}))_{m2}$-;

$-(C(R^{a9})(R^{a10}))_{m1}$-(NHC(O)-$(C(R^{a11})(R^{a12}))_{m2}$-;

$-(C(R^{a9})(R^{a10}))_{m1}$-S-$(C(R^{a11})(R^{a12}))_{m2}$-;

$-(C(R^{a9})(R^{a10}))_{m1}$-($C_{6-10}$ arylene-$(C(R^{a11})(R^{a12}))_{m2})_{p1}$-;

$-(C(R^{a9})(R^{a10}))_{m1}$-($C_{6-10}$ arylene-$(C(R^{a11})(R^{a12}))_{m2})_{p1}$-$C_{6-10}$ arylene-$(C(R^{a13})(R^{a14}))_{m3}$-;

$-(C(R^{a9})(R^{a10}))_{m1}$-(4- to 15-membered heterocyclylene-$(C(R^{a11})(R^{a12}))_{m2})_{p1}$-;

$-(C(R^{a9})(R^{a10}))_{m1}$-(4- to 15-membered heterocyclylene-$(C(R^{a11})(R^{a12}))_{m2})_{p1}$-(4- to 15-membered heterocyclylene-$(C(R^{a13})(R^{a14}))_{m3})_{p2}$-;

$-(C(R^{a9})(R^{a10}))_{m1}$-(5- to 15-membered heteroarylene-$(C(R^{a11})(R^{a12}))_{m2})_{p1}$-;

$-(C(R^{a9})(R^{a10}))_{m1}$-(5- to 15-membered heteroarylene-$(C(R^{a11})(R^{a12}))_{m2})_{p1}$-(5- to 15-membered heteroarylene-$(C(R^{a13})(R^{a14}))_{m3})_{p2}$-;

$-(C(R^{a9})(R^{a10}))_{m1}$-($C_{3-15}$cycloalkylene-$(C(R^{a11})(R^{a12}))_{m2})_{p1}$-; or

$-(C(R^{a9})(R^{a10}))_{m1}$-($C_{3-15}$cycloalkylene-$(C(R^{a11})(R^{a12}))_{m2})_{p1}$-($C_{3-15}$cycloalkylene-$(C(R^{a13})(R^{a14}))_{m3})_{p2}$-;

wherein each $R_{g11}$ independently represents H or $C_{1-3}$ alkyl;
the $C_{3-15}$cycloalkylene, the $C_{6-10}$ arylene, the 4- to 15-membered heterocyclylene and the 5- to 15-membered heteroarylene are each independently optionally substituted with a substituent selected from the group consisting of deuterium, optionally deuterated $C_{1-4}$ alkyl, optionally deuterated $C_{3-6}$cycloalkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof;
$R^{a9}$, $R^{a10}$, $R^{a11}$, $R^{a12}$, $R^{a13}$, $R^{a14}$, $R^{a15}$ and $R^{a16}$ each independently represent H, deuterium, halogen, $C_{1-4}$ alkyl, $C_{3-6}$cycloalkyl or any combination thereof; and
m1, m2, m3, m4, p1, p2, p3 are each independently selected from the group consisting of an integer of 1, 2, 3, 4, 5, 6, 7, 8, 9, 10, 11, 12, 13, 14, or 15; or
more preferably, wherein $L_2$ or $L_3$ each independently represent the structure of the following formula:
$-CH_2-O-CH_2-$, $-CH_2-O-(CH_2)_2-$, $-(CH_2)_1-O-(CH_2)_3-$, $-(CH_2)_1-O-(CH_2)_4-$, $-(CH_2)_1-O-(CH_2)_5-$, $-(CH_2)_1-O-(CH_2)_6-$, $-(CH_2)_1-O-(CH_2)_7-$, $-(CH_2)_1-O-(CH_2)_8-$, $-(CH_2)_1-O-(CH_2)_9-$, $-(CH_2)_1-O-(CH_2)_{10}-$, $-(CH_2)_2-O-(CH_2)_1-$, $-(CH_2)_2-O-(CH_2)_2-$, $-(CH_2)_2-O-(CH_2)_3-$, $-(CH_2)_2-O-(CH_2)_4-$, $-(CH_2)_2-O-(CH_2)_5-$, $-(CH_2)_2-O-(CH_2)_6-$, $-(CH_2)_2-O-(CH_2)_7-$, $-(CH_2)_2-O-(CH_2)_8-$, $-(CH_2)_2-O-(CH_2)_9-$, $-(CH_2)_2-O-(CH_2)_{10}-$, $-(CH_2)_2-O-(CH_2)_{11}-$, $-(CH_2)_2-O-(CH_2)_{12}-$, $-(CH_2)_3-O-(CH_2)_1-$, $-(CH_2)_3-O-(CH_2)_2-$, $-(CH_2)_3-O-(CH_2)_3-$, $-(CH_2)_3-O-(CH_2)_4-$, $-(CH_2)_3-O-(CH_2)_5-$, $-(CH_2)_3-O-(CH_2)_6-$, $-(CH_2)_3-O-(CH_2)_7-$, $-(CH_2)_4-O-(CH_2)_1-$, $-(CH_2)_4-O-(CH_2)_2-$, $-(CH_2)_4-O-(CH_2)_3-$, $-(CH_2)_4-O-(CH_2)_4-$, $-(CH_2)_4-O-(CH_2)_5-$, $-(CH_2)_4-O-(CH_2)_6-$, $-(CH_2)_5-O-(CH_2)_1-$, $-(CH_2)_5-O-(CH_2)_2-$, $-(CH_2)_5-O-(CH_2)_3-$, $-(CH_2)_5-O-(CH_2)_4-$, $-(CH_2)_5-O-(CH_2)_5-$, $-(CH_2)_6-O-(CH_2)_1-$, $-(CH_2)_6-O-(CH_2)_2-$, $-(CH_2)_6-O-(CH_2)_3-$, $-(CH_2)_6-O-(CH_2)_4-$, $-(CH_2)_7-O-(CH_2)_1-$, $-(CH_2)_7-O-(CH_2)_2-$, $-(CH_2)_7-O-(CH_2)_3-$, $-(CH_2)_8-O-(CH_2)_1-$, $-(CH_2)_8-O-(CH_2)_2-$, $-CH(CH_3)-O-(CH_2)_1-$,$-CH(CH_3)-O-(CH_2)_2-$, $-CH(CH_3)-O-(CH_2)_3-$, $-CH(CH_3)-O-(CH_2)_4-$, $-CH(CH_3)-O-(CH_2)_5-$, $-CH(CH_3)-O-(CH_2)_6-$, $-CH(CH_3)-O-(CH_2)_7-$, $-CH(CH_3)-O-(CH_2)_8-$, $-CH(CH_3)-O-(CH_2)_9-$, $-CH(CH_3)-O-(CH_2)_{10}-$, $-CH_2-(O(CH_2)_2)_2-$, $-CH_2-(O(CH_2)_2)_3-$, $-CH_2-(O(CH_2)_2)_4-$, $-CH_2-(O(CH_2)_2)_5-$, $-CH_2-(O(CH_2)_2)_6-$, $-CH_2-(O(CH_2)_2)_7-$, $-CH_2-(O(CH_2)_2)_8-$, $-CH_2-(O(CH_2)_2)_9-$, $-CH_2-(O(CH_2)_2)_{10}-$, $-CH_2-(O(CH_2)_2)_1-OCH_2-$, $-CH_2-(O(CH_2)_2)_2-OCH_2-$, $-CH_2-(O(CH_2)_2)_3-OCH_2-$, $-CH_2-(O(CH_2)_2)_4-OCH_2-$, $-CH_2-(O(CH_2)_2)_5-OCH_2-$, $-CH_2-(O(CH_2)_2)_6-OCH_2-$, $-CH_2-(O(CH_2)_2)_7-OCH_2-$, $-CH_2-(O(CH_2)_2)_8-OCH_2-$, $-CH_2-(O(CH_2)_2)_9-OCH_2-$, $-CH_2-(O(CH_2)_2)_{10}-OCH_2-$, $-(CH_2)_2-(O(CH_2)_2)_2-$, $-(CH_2)_2-(O(CH_2)_2)_3-$, $-(CH_2)_2-(O(CH_2)_2)_4-$, $-(CH_2)_2-(O(CH_2)_2)_5-$, $-(CH_2)_2-(O(CH_2)_2)_6-$, $-(CH_2)_2-(O(CH_2)_2)_7-$, $-(CH_2)_2-(O(CH_2)_2)_8-$, $-(CH_2)_2-(O(CH_2)_2)_9-$, $-(CH_2)_2-(O(CH_2)_2)_{10}-$, $-(CH_2)_3-(O(CH_2)_2)_2-$,

$-(CH_2)_3-(O(CH_2)_2)_3-$, $-(CH_2)_3-(O(CH_2)_2)_4-$, $-(CH_2)_3-(O(CH_2)_2)_5-$, $-(CH_2)_3-(O(CH_2)_2)_6-$, $-(CH_2)_3-(O(CH_2)_2)_7-$, $-(CH_2)_3-(O(CH_2)_2)_8-$, $-(CH_2)_3-(O(CH_2)_2)_9-$, $-(CH_2)_3-(O(CH_2)_2)_{10}-$, $-(CH_2)_4-(O(CH_2)_2)_2-$, $-(CH_2)_4-(O(CH_2)_2)_3-$, $-(CH_2)_4-(O(CH_2)_2)_4-$, $-(CH_2)_4-(O(CH_2)_2)_5-$, $-(CH_2)_4-(O(CH_2)_2)_6-$, $-(CH_2)_4-(O(CH_2)_2)_7-$, $-(CH_2)_4-(O(CH_2)_2)_8-$, $-(CH_2)_4-(O(CH_2)_2)_9-$, $-(CH_2)_4-(O(CH_2)_2)_{10}-$, $-CH_2-(O(CH_2)_3)_2-$, $-CH_2-(O(CH_2)_3)_3-$, $-CH_2-(O(CH_2)_3)_4-$, $-CH_2-(O(CH_2)_3)_5-$, $-CH_2-(O(CH_2)_3)_6-$, $-CH_2-(O(CH_2)_3)_7-$, $-CH_2-(O(CH_2)_3)_8-$, $-CH_2-(O(CH_2)_3)_9-$, $-CH_2-(O(CH_2)_3)_{10}-$, $-(CH_2)_2-(O(CH_2)_3)_2-$, $-(CH_2)_2-(O(CH_2)_3)_3-$, $-(CH_2)_2-(O(CH_2)_3)_4-$, $-(CH_2)_2-(O(CH_2)_3)_5-$,$-(CH_2)_2-(O(CH_2)_3)_6-$, $-(CH_2)_2-(O(CH_2)_3)_7-$, $-(CH_2)_2-(O(CH_2)_3)_8-$, $-(CH_2)_2-(O(CH_2)_3)_9-$, $-(CH_2)_2-(O(CH_2)_3)_{10}-$, $-(CH_2)_3-(O(CH_2)_3)_2-$, $-(CH_2)_3-(O(CH_2)_3)_3-$, $-(CH_2)_3-(O(CH_2)_3)_4-$, $-(CH_2)_3-(O(CH_2)_3)_5-$, $-(CH_2)_3-(O(CH_2)_3)_6-$, $-(CH_2)_3-(O(CH_2)_3)_7-$, $-(CH_2)_3-(O(CH_2)_3)_8-$, $-(CH_2)_3-(O(CH_2)_3)_9-$, $-(CH_2)_3-(O(CH_2)_3)_{10}-$, $-CH_2-O-(CH_2)_2-O-(CH_2)_3-$, $-CH_2-(O(CH_2)_2)_2-(O(CH_2)_3)_2-$, $-CH_2-(O(CH_2)_2)_3-(O(CH_2)_3)_3-$, $-CH_2-(O(CH_2)_2)_4-(O(CH_2)_3)_4-$, $-CH_2-(O(CH_2)_2)_5-(O(CH_2)_3)_5-$, $-CH_2-(O(CH_2)_2)_6-(O(CH_2)_3)_6-$, $-(CH_2)_2-O-(CH_2)_2-O-(CH_2)_3-$, $-(CH_2)_2-(O(CH_2)_2)_2-(O(CH_2)_3)_2-$, $-(CH_2)_2-(O(CH_2)_2)_3-(O(CH_2)_3)_3-$, $-(CH_2)_2-(O(CH_2)_2)_4-(O(CH_2)_3)_4-$, $-(CH_2)_2-(O(CH_2)_2)_5-(O(CH_2)_3)_5-$, $-(CH_2)_2-(O(CH_2)_2)_6-(O(CH_2)_3)_6-$, $-(CH_2)_3-O-(CH_2)_2-O-(CH_2)_3-$, $-(CH_2)_3-(O(CH_2)_2)_2-(O(CH_2)_3)_2-$, $-(CH_2)_3-(O(CH_2)_2)_3-(O(CH_2)_3)_3-$, $-(CH_2)_3-(O(CH_2)_2)_4-(O(CH_2)_3)_4-$, $-(CH_2)_3-(O(CH_2)_2)_5-(O(CH_2)_3)_5-$, $-(CH_2)_3-(O(CH_2)_2)_6-(O(CH_2)_3)_6-$, $-CH_2-O-(CH_2)_3-O-(CH_2)_2-$, $-CH_2-(O(CH_2)_3)_2-(O(CH_2)_2)_2-$, $-CH_2-(O(CH_2)_3)_3-(O(CH_2)_2)_3-$, $-CH_2-(O(CH_2)_3)_4-(O(CH_2)_2)_4-$, $-CH_2-(O(CH_2)_3)_5-(O(CH_2)_2)_5-$, $-CH_2-(O(CH_2)_3)_6-(O(CH_2)_2)_6-$, $-(CH_2)_2-O-(CH_2)_3-O-(CH_2)_2-$, $-(CH_2)_2-(O(CH_2)_3)_2-(O(CH_2)_2)_2-$, $-(CH_2)_2-(O(CH_2)_3)_3-(O(CH_2)_2)_3-$, $-(CH_2)_2-(O(CH_2)_3)_4-(O(CH_2)_2)_4-$, $-(CH_2)_2-(O(CH_2)_3)_5-(O(CH_2)_2)_5-$, $-(CH_2)_2-(O(CH_2)_3)_6-(O(CH_2)_2)_6-$, $-(CH_2)_3-O-(CH_2)_3-O-(CH_2)_2-$, $-(CH_2)_3-(O(CH_2)_3)_2-(O(CH_2)_2)_2-$, $-(CH_2)_3-(O(CH_2)_3)_3-(O(CH_2)_2)_3-$, $-(CH_2)_3-(O(CH_2)_3)_4-(O(CH_2)_2)_4-$, $-(CH_2)_3-(O(CH_2)_3)_5-(O(CH_2)_2)_5-$, $-(CH_2)_3-(O(CH_2)_3)_6-(O(CH_2)_2)_6-$, $-CH_2-O-(CH_2)_2-O-CH_2-$, $-(CH_2)_2-O-(CH_2)_2-O-CH_2-$, $-(CH_2)_2-(O(CH_2)_2)_2-O-(CH_2)_3-$, $-(CH_2)_2-(O(CH_2)_2)_3-O-(CH_2)_3-$, $-(CH_2)_2-(O(CH_2)_2)_4-O-(CH_2)_3-$, $-(CH_2)_5-(O(CH_2)_2)_2-O-(CH_2)_5-$, $-(CH_2)_5-(O(CH_2)_2)_2-O-(CH_2)_6-$, $-CH_2-C(O)-CH_2-$, $-CH_2-C(O)-(CH_2)_2-$, $-(CH_2)_1-C(O)-(CH_2)_3-$, $-(CH_2)_1-C(O)-(CH_2)_4-$, $-(CH_2)_1-C(O)-(CH_2)_5-$, $-(CH_2)_1-C(O)-(CH_2)_6-$, $-(CH_2)_1-C(O)-(CH_2)_7-$, $-(CH_2)_1-C(O)-(CH_2)_8-$, $-(CH_2)_1-C(O)-(CH_2)_9-$, $-(CH_2)_1-C(O)-(CH_2)_{10}-$, $-(CH_2)_2-C(O)-(CH_2)_1-$, $-(CH_2)_2-C(O)-(CH_2)_2-$, $-(CH_2)_2-C(O)-(CH_2)_3-$, $-(CH_2)_2-C(O)-(CH_2)_4-$, $-(CH_2)_2-C(O)-(CH_2)_5-$, $-(CH_2)_2-C(O)-(CH_2)_6-$, $-(CH_2)_2-C(O)-(CH_2)_7-$, $-(CH_2)_2-C(O)-(CH_2)_8-$, $-(CH_2)_2-C(O)-(CH_2)_9-$, $-(CH_2)_2-C(O)-(CH_2)_{10}-$, $-(CH_2)_2-C(O)-(CH_2)_{11}-$, $-(CH_2)_2-C(O)-(CH_2)_{12}-$, $-(CH_2)_3-C(O)-(CH_2)_1-$, $-(CH_2)_3-C(O)-(CH_2)_2-$, $-(CH_2)_3-C(O)-(CH_2)_3-$, $-(CH_2)_3-C(O)-(CH_2)_4-$, $-(CH_2)_3-C(O)-(CH_2)_5-$, $-(CH_2)_3-C(O)-(CH_2)_6-$, $-(CH_2)_3-C(O)-(CH_2)_7-$, $-(CH_2)_4-C(O)-(CH_2)_1-$, $-(CH_2)_4-C(O)-(CH_2)_2-$, $-(CH_2)_4-C(O)-(CH_2)_3-$, $-(CH_2)_4-C(O)-(CH_2)_4-$, $-(CH_2)_4-C(O)-(CH_2)_5-$, $-(CH_2)_4-C(O)-(CH_2)_6-$, $-(CH_2)_5-C(O)-(CH_2)_1-$, $-(CH_2)_5-C(O)-(CH_2)_2-$, $-(CH_2)_5-C(O)-(CH_2)_3-$, $-(CH_2)_5-C(O)-(CH_2)_4-$, $-(CH_2)_5-C(O)-(CH_2)_5-$, $-(CH_2)_6-C(O)-(CH_2)_1-$, $-(CH_2)_6-C(O)-(CH_2)_2-$, $-(CH_2)_6-C(O)-(CH_2)_3-$, $-(CH_2)_6-C(O)-(CH_2)_4-$, $-(CH_2)_7-C(O)-(CH_2)_1-$, $-(CH_2)_7-C(O)-(CH_2)_2-$, $-(CH_2)_7-C(O)-(CH_2)_3-$, $-(CH_2)_8-C(O)-(CH_2)_1-$, $-(CH_2)_8-C(O)-(CH_2)_2-$, $-CH_2-S-CH_2-$, $-CH_2-S-(CH_2)_2-$, $-(CH_2)_1-S-(CH_2)_3-$, $-(CH_2)_1-S-(CH_2)_4-$, $-(CH_2)_1-S-(CH_2)_5-$, $-(CH_2)_1-S-(CH_2)_6-$, $-(CH_2)_1-S-(CH_2)_7-$, $-(CH_2)_1-S-(CH_2)_8-$, $-(CH_2)_1-S-(CH_2)_9-$, $-(CH_2)_1-S-(CH_2)_{10}-$, $-(CH_2)_2-S-(CH_2)_1-$, $-(CH_2)_2-S-(CH_2)_2-$, $-(CH_2)_2-S-(CH_2)_3-$, $-(CH_2)_2-S-(CH_2)_4-$, $-(CH_2)_2-S-(CH_2)_5-$, $-(CH_2)_2-S-(CH_2)_6-$, $-(CH_2)_2-S-(CH_2)_7-$, $-(CH_2)_2-S-(CH_2)_8-$, $-(CH_2)_2-S-(CH_2)_9-$, $-(CH_2)_2-S-(CH_2)_{10}-$, $-(CH_2)_2-S-(CH_2)_{11}-$, $-(CH_2)_2-S-(CH_2)_{12}-$, $-(CH_2)_3-S-(CH_2)_1-$, $-(CH_2)_3-S-(CH_2)_2-$, $-(CH_2)_3-S-(CH_2)_3-$, $-(CH_2)_3-S-(CH_2)_4-$, $-(CH_2)_3-S-(CH_2)_5-$, $-(CH_2)_3-S-(CH_2)_6-$, $-(CH_2)_3-S-(CH_2)_7-$, $-(CH_2)_4-S-(CH_2)_1-$, $-(CH_2)_4-S-(CH_2)_2-$, $-(CH_2)_4-S-(CH_2)_3-$, $-(CH_2)_4-S-(CH_2)_4-$, $-(CH_2)_4-S-(CH_2)_5-$, $-(CH_2)_4-S-(CH_2)_6-$, $-(CH_2)_5-S-(CH_2)_1-$, $-(CH_2)_5-S-(CH_2)_2-$, $-(CH_2)_5-S-(CH_2)_3-$, $-(CH_2)_5-S-(CH_2)_4-$, $-(CH_2)_5-S-(CH_2)_5-$, $-(CH_2)_6-S-(CH_2)_1-$, $-(CH_2)_6-S-(CH_2)_2-$, $-(CH_2)_6-S-(CH_2)_3-$, $-(CH_2)_6-S-(CH_2)_4-$, $-(CH_2)_7-S-(CH_2)_1-$, $-(CH_2)_7-S-(CH_2)_2-$, $-(CH_2)_7-S-(CH_2)_3-$, $-(CH_2)_8-S-(CH_2)_1-$, $-(CH_2)_8-S-(CH_2)_2-$, $-CH_2-C(S)-CH_2-$, $-CH_2-C(S)-(CH_2)_2-$, $-(CH_2)_1-C(S)-(CH_2)_3-$, $-(CH_2)_1-C(S)-(CH_2)_4-$, $-(CH_2)_1-C(S)-(CH_2)_5-$, $-(CH_2)_1-C(S)-(CH_2)_6-$, $-(CH_2)_1-C(S)-(CH_2)_7-$, $-(CH_2)_1-C(S)-(CH_2)_8-$, $-(CH_2)_1-C(S)-(CH_2)_9-$, $-(CH_2)_1-C(S)-(CH_2)_{10}-$, $-(CH_2)_2-C(S)-(CH_2)_1-$, $-(CH_2)_2-C(S)-(CH_2)_2-$, $-(CH_2)_2-C(S)-(CH_2)_3-$, $-(CH_2)_2-C(S)-(CH_2)_4-$, $-(CH_2)_2-C(S)-(CH_2)_5-$, $-(CH_2)_2-C(S)-(CH_2)_6-$, $-(CH_2)_2-C(S)-(CH_2)_7-$, $-(CH_2)_2-C(S)-(CH_2)_8-$, $-(CH_2)_2-C(S)-(CH_2)_9-$, $-(CH_2)_2-C(S)-(CH_2)_{10}-$, $-(CH_2)_2-C(S)-(CH_2)_{11}-$, $-(CH_2)_2-C(S)-(CH_2)_{12}-$, $-(CH_2)_3-C(S)-(CH_2)_1-$, $-(CH_2)_3-C(S)-(CH_2)_2-$, $-(CH_2)_3-C(S)-(CH_2)_3-$, $-(CH_2)_3-C(S)-(CH_2)_4-$, $-(CH_2)_3-C(S)-(CH_2)_5-$, $-(CH_2)_3-C(S)-(CH_2)_6-$, $-(CH_2)_3-C(S)-(CH_2)_7-$, $-(CH_2)_4-C(S)-(CH_2)_1-$, $-(CH_2)_4-C(S)-(CH_2)_2-$,$-(CH_2)_4-C(S)-(CH_2)_3-$, $-(CH_2)_4-C(S)-(CH_2)_4-$, $-(CH_2)_4-C(S)-(CH_2)_5-$, $-(CH_2)_4-C(S)-(CH_2)_6-$, $-(CH_2)_5-C(S)-(CH_2)_1-$, $-(CH_2)_5-C(S)-(CH_2)_2-$, $-(CH_2)_5-C(S)-(CH_2)_3-$, $-(CH_2)_5-C(S)-(CH_2)_4-$, $-(CH_2)_5-C(S)-(CH_2)_5-$, $-(CH_2)_6-C(S)-(CH_2)_1-$, $-(CH_2)_6-C(S)-(CH_2)_2-$, $-(CH_2)_6-C(S)-(CH_2)_3-$, $-(CH_2)_6-C(S)-(CH_2)_4-$, $-(CH_2)_7-C(S)-(CH_2)_1-$, $-(CH_2)_7-C(S)-(CH_2)_2-$, $-(CH_2)_7-C(S)-(CH_2)_3-$, $-(CH_2)_8-C(S)-(CH_2)_1-$, $-(CH_2)_8-C(S)-(CH_2)_2-$, $-CH_2-C(O)O-CH_2-$, $-CH_2-C(O)O-(CH_2)_2-$, $-(CH_2)_1-C(O)O-(CH_2)_3-$, $-(CH_2)_1-C(O)$

$O-(CH_2)_4-$, $-(CH_2)_1-C(O)O-(CH_2)_5-$, $-(CH_2)_1-C(O)O-(CH_2)_6-$, $-(CH_2)_1-C(O)O-(CH_2)_7-$, $-(CH_2)_1-C(O)O-(CH_2)_8-$, $-(CH_2)_1-C(O)O-(CH_2)_9-$, $-(CH_2)_1-C(O)O-(CH_2)_{10}-$, $-(CH_2)_2-C(O)O-(CH_2)_1-$, $-(CH_2)_2-C(O)O-(CH_2)_2-$, $-(CH_2)_2-C(O)O-(CH_2)_3-$, $-(CH_2)_2-C(O)O-(CH_2)_4-$, $-(CH_2)_2-C(O)O-(CH_2)_5-$, $-(CH_2)_2-C(O)O-(CH_2)_6-$, $-(CH_2)_2-C(O)O-(CH_2)_7-$, $-(CH_2)_2-C(O)O-(CH_2)_8-$, $-(CH_2)_2-C(O)O-(CH_2)_9-$, $-(CH_2)_2-C(O)O-(CH_2)_{10}-$, $-(CH_2)_2-C(O)O-(CH_2)_{11}-$, $-(CH_2)_2-C(O)O-(CH_2)_{12}-$, $-(CH_2)_3-C(O)O-(CH_2)_1-$, $-(CH_2)_3-C(O)O-(CH_2)_2-$, $-(CH_2)_3-C(O)O-(CH_2)_3-$, $-(CH_2)_3-C(O)O-(CH_2)_4-$, $-(CH_2)_3-C(O)O-(CH_2)_5-$, $-(CH_2)_3-C(O)O-(CH_2)_6-$, $-(CH_2)_3-C(O)O-(CH_2)_7-$, $-(CH_2)_4-C(O)O-(CH_2)_1-$, $-(CH_2)_4-C(O)O-(CH_2)_2-$, $-(CH_2)_4-C(O)O-(CH_2)_3-$, $-(CH_2)_4-C(O)O-(CH_2)_4-$, $-(CH_2)_4-C(O)O-(CH_2)_5-$, $-(CH_2)_4-C(O)O-(CH_2)_6-$, $-(CH_2)_5-C(O)O-(CH_2)_1-$, $-(CH_2)_5-C(O)O-(CH_2)_2-$, $-(CH_2)_5-C(O)O-(CH_2)_3-$, $-(CH_2)_5-C(O)O-(CH_2)_4-$, $-(CH_2)_5-C(O)O-(CH_2)_5-$, $-(CH_2)_6-C(O)O-(CH_2)_1-$, $-(CH_2)_6-C(O)O-(CH_2)_2-$, $-(CH_2)_6-C(O)O-(CH_2)_3-$, $-(CH_2)_6-C(O)O-(CH_2)_4-$, $-(CH_2)_7-C(O)O-(CH_2)_1-$, $-(CH_2)_7-C(O)O-(CH_2)_2-$, $-(CH_2)_7-C(O)O-(CH_2)_3-$, $-(CH_2)_8-C(O)O-(CH_2)_1-$, $-(CH_2)_8-C(O)O-(CH_2)_2-$, $-CH_2-OC(O)-CH_2-$, $-CH_2-OC(O)-(CH_2)_2-$, $-(CH_2)_1-OC(O)-(CH_2)_3-$, $-(CH_2)_1-O-C(O)-(CH_2)_4-$, $-(CH_2)_1-OC(O)-(CH_2)_5-$, $-(CH_2)_1-OC(O)-(CH_2)_6-$, $-(CH_2)_1-OC(O)-(CH_2)_7-$, $-(CH_2)_1-O-C(O)-(CH_2)_8-$, $-(CH_2)_1-OC(O)-(CH_2)_9-$, $-(CH_2)_1-OC(O)-(CH_2)_{10}-$, $-(CH_2)_2-OC(O)-(CH_2)_1-$, $-(CH_2)_2-O-C(O)-(CH_2)_2-$, $-(CH_2)_2-OC(O)-(CH_2)_3-$, $-(CH_2)_2-OC(O)-(CH_2)_4-$, $-(CH_2)_2-OC(O)-(CH_2)_5-$, $-(CH_2)_2-O-C(O)-(CH_2)_6-$, $-(CH_2)_2-OC(O)-(CH_2)_7-$, $-(CH_2)_2-OC(O)-(CH_2)_8-$, $-(CH_2)_2-OC(O)-(CH_2)_9-$, $-(CH_2)_2-O-C(O)-(CH_2)_{10}-$, $-(CH_2)_2-OC(O)-(CH_2)_{11}-$, $-(CH_2)_2-OC(O)-(CH_2)_{12}-$, $-(CH_2)_3-OC(O)-(CH_2)_1-$, $-(CH_2)_3-O-C(O)-(CH_2)_2-$, $-(CH_2)_3-OC(O)-(CH_2)_3-$, $-(CH_2)_3-OC(O)-(CH_2)_4-$, $-(CH_2)_3-OC(O)-(CH_2)_5-$, $-(CH_2)_3-O-C(O)-(CH_2)_6-$, $-(CH_2)_3-OC(O)-(CH_2)_7-$, $-(CH_2)_4-OC(O)-(CH_2)_1-$, $-(CH_2)_4-OC(O)-(CH_2)_2-$, $-(CH_2)_4-O-C(O)-(CH_2)_3-$, $-(CH_2)_4-OC(O)-(CH_2)_4-$, $-(CH_2)_4-OC(O)-(CH_2)_5-$, $-(CH_2)_4-OC(O)-(CH_2)_6-$, $-(CH_2)_5-O-C(O)-(CH_2)_1-$, $-(CH_2)_5-OC(O)-(CH_2)_2-$, $-(CH_2)_5-OC(O)-(CH_2)_3-$, $-(CH_2)_5-OC(O)-(CH_2)_4-$, $-(CH_2)_5-OC(O)-(CH_2)_5-$, $-(CH_2)_6-OC(O)-(CH_2)_1-$, $-(CH_2)_6-OC(O)-(CH_2)_2-$, $-(CH_2)_6-OC(O)-(CH_2)_3-$, $-(CH_2)_6-O-C(O)-(CH_2)_4-$, $-(CH_2)_7-OC(O)-(CH_2)_1-$, $-(CH_2)_7-OC(O)-(CH_2)_2-$, $-(CH_2)_7-OC(O)-(CH_2)_3-$, $-(CH_2)_8-O-C(O)-(CH_2)_1-$, $-(CH_2)_8-OC(O)-(CH_2)_2-$, $-(CH_2)_1-N(R_{g11})-(CH_2)_1-$, $-(CH_2)_1-N(R_{g11})-(CH_2)_2-$, $-(CH_2)_1-N(R_{g11})-(CH_2)_3-$, $-(CH_2)_1-N(R_{g11})-(CH_2)_4-$, $-(CH_2)_1-N(R_{g11})-(CH_2)_5-$, $-(CH_2)_1-N(R_{g11})-(CH_2)_6-$, $-(CH_2)_1-N(R_{g11})-(CH_2)_7-$, $-(CH_2)_1-N(R_{g11})-(CH_2)_8-$, $-(CH_2)_1-N(R_{g11})-(CH_2)_9-$, $-(CH_2)_1-N(R_{g11})-(CH_2)_{10}-$, $-(CH_2)_2-N(R_{g11})-(CH_2)_1-$, $-(CH_2)_2-N(R_{g11})-(CH_2)_2-$, $-(CH_2)_2-N(R_{g11})-(CH_2)_3-$, $-(CH_2)_2-N(R_{g11})-(CH_2)_4-$, $-(CH_2)_2-N(R_{g11})-(CH_2)_5-$, $-(CH_2)_2-N(R_{g11})-(CH_2)_6-$, $-(CH_2)_2-N(R_{g11})-(CH_2)_7-$, $-(CH_2)_2-N(R_{g11})-(CH_2)_8-$, $-(CH_2)_2-N(R_{g11})-(CH_2)_9-$, $-(CH_2)_2-N(R_{g11})-(CH_2)_{10}-$, $-(CH_2)_2-N(R_{g11})-(CH_2)_{11}-$, $-(CH_2)_2-N(R_{g11})-(CH_2)_{12}-$, $-(CH_2)_3-N(R_{g11})-(CH_2)_1-$, $-(CH_2)_3-N(R_{g11})-(CH_2)_2-$, $-(CH_2)_3-N(R_{g11})-(CH_2)_3-$, $-(CH_2)_4-N(R_{g11})-(CH_2)_1-$, $-(CH_2)_4-N(R_{g11})-(CH_2)_2-$, $-(CH_2)_4-N(R_{g11})-(CH_2)_3-$, $-(CH_2)_4-N(R_{g11})-(CH_2)_4-$, $-(CH_2)_5-N(R_{g11})-(CH_2)_1-$, $-(CH_2)_5-N(R_{g11})-(CH_2)_2-$, $-(CH_2)_5-N(R_{g11})-(CH_2)_3-$, $-(CH_2)_5-N(R_{g11})-(CH_2)_4-$, $-(CH_2)_5-N(R_{g11})-(CH_2)_5-$, $-(CH_2)_6-N(R_{g11})-(CH_2)_1-$, $-(CH_2)_6-N(R_{g11})-(CH_2)_2-$, $-(CH_2)_6-N(R_{g11})-(CH_2)_3-$, $-(CH_2)_7-N(R_{g11})-(CH_2)_1-$, $-(CH_2)_7-N(R_{g11})-(CH_2)_2-$, $-(CH_2)_7-N(R_{g11})-(CH_2)_3-$, $-(CH_2)_8-N(R_{g11})-(CH_2)_1-$, $-(CH_2)_8-NR_{g11})-(CH_2)_2-$, $-(CH_2)_8-N(R_{g11})-(CH_2)_3-$, $-CH(CH_3)-N(R_{g11})-(CH_2)_1-$, $-CH(CH_3)-N(R_{g11})-(CH_2)_2-$, $-CH(CH_3)-N(R_{g11})-(CH_2)_3-$, $-CH(CH_3)-N(R_{g11})-(CH_2)_4-$, $-CH(CH_3)-N(R_{g11})-(CH_2)_5-$, $-CH(CH_3)-N(R_{g11})-(CH_2)_6-$, $-CH(CH_3)-N(R_{g11})-(CH_2)_7-$, $-CH(CH_3)-N(R_{g11})-(CH_2)_8-$, $-CH(CH_3)-N(R_{g11})-(CH_2)_9-$, $-CH(CH_3)-N(R_{g11})-(CH_2)_{10}-$, $-CH_2C(O)NHCH_2-$, $-(CH_2)_2C(O)NH(CH_2)_2-$, $-(CH_2)_2C(O)NH(CH_2)_3-$, $-(CH_2)_2C(O)NH(CH_2)_4-$, $-(CH_2)_2C(O)NH(CH_2)_5-$, $-(CH_2)_3C(O)NH(CH_2)_3-$, $-(CH_2)_3C(O)NH(CH_2)_4-$, $-(CH_2)_4C(O)NH(CH_2)_4-$, $-(CH_2)_5C(O)NH(CH_2)_5-$, $-(CH_2)_6C(O)NH(CH_2)_7-$, $-(CH_2)_6C(O)NH(CH_2)_6-$, $-(CH_2)_7C(O)NH(CH_2)_7-$, $-(CH_2)_8C(O)NH(CH_2)_8$, $-(CH_2)_9C(O)NH(CH_2)_9-$, $-(CH_2)_{10}C(O)NH(CH_2)_{10}-$, $-(CH_2)_2C(O)NH(CH_2)_2-O-(CH_2)_2-$, $-CH_2NHC(O)CH_2-$, $-(CH_2)_2NHC(O)(CH_2)_2-$, $-(CH_2)_2NHC(O)(CH_2)_3-$, $-(CH_2)_2NHC(O)(CH_2)_4-$, $-(CH_2)_2NHC(O)(CH_2)_5-$, $-(CH_2)_3NHC(O)(CH_2)_3-$, $-(CH_2)_3NHC(O)(CH_2)_4-$, $-(CH_2)_4NHC(O)(CH_2)_4-$, $-(CH_2)_5NHC(O)(CH_2)_5-$, $-(CH_2)_6NHC(O)(CH_2)_7-$, $-(CH_2)_6NHC(O)(CH_2)_6-$, $-(CH_2)_7NHC(O)(CH_2)_7-$, $-(CH_2)_8NHC(O)(CH_2)_8$, $-(CH_2)_9NHC(O)(CH_2)_9-$, $-(CH_2)_{10}NHC(O)(CH_2)_{10}-$, $-(CH_2)_4NHC(O)(CH_2)_8-$, $-(CH_2)_2NHC(O)(CH_2)_2-O-(CH_2)_2-$, $-(CH_2)_4NHC(O)CH_2-$, $-CH_2$-piperidinylene-$CH_2-$, $-CH_2$-piperidinylene-$(CH_2)_2-$, $-CH_2$-piperidinylene-$(CH_2)_3-$, $-CH_2$-piperidinylene-$(CH_2)_4-$, $-CH_2$-piperidinylene-$(CH_2)_5-$, $-CH_2$-piperidinylene-$(CH_2)_6-$, $-CH_2$-piperidinylene-$(CH_2)_7-$, $-CH_2$-piperidinylene-$(CH_2)_8-$, $-(CH_2)_2$-piperidinylene-$(CH_2)_1-$, $-(CH_2)_2$-piperidinylene-$(CH_2)_2-$, $-(CH_2)_2$-piperidinylene-$(CH_2)_3-$, $-(CH_2)_2$-piperidinylene-$(CH_2)_4-$, $-(CH_2)_2$-piperidinylene-$(CH_2)_5-$, $-(CH_2)_2$-piperidinylene-$(CH_2)_6-$, $-(CH_2)_2$-piperidinylene-$(CH_2)_7-$, $-(CH_2)_2$-piperidinylene-$(CH_2)_8-$, $-(CH_2)_3$-piperidinylene-$CH_2-$, $-(CH_2)_3$-piperidinylene-$(CH_2)_2-$, $-(CH_2)_3$-piperidinylene-$(CH_2)_3-$, $-(CH_2)_3$-piperidinylene-$(CH_2)_4-$, $-(CH_2)_3$-piperidinylene-$(CH_2)_5-$, $-(CH_2)_3$-piperidinylene-$(CH_2)_6-$, $-(CH_2)_3$-piperidinylene-$(CH_2)_7-$, $-(CH_2)_3$-piperidinylene-$(CH_2)_8-$, $-(CH_2)_4$-piperidinylene-$CH_2-$, $-(CH_2)_4$-piperidinylene-$(CH_2)_2-$, $-(CH_2)_4$-piperidinylene-$(CH_2)_3-$, $-(CH_2)_4$-piperidinylene-$(CH_2)_4-$, $-(CH_2)_4$-piperidinylene-$(CH_2)_5-$, $-(CH_2)_4$-piperidinylene-$(CH_2)_6-$, $-(CH_2)_4$-piperidinylene-$(CH_2)_7-$, $-(CH_2)_4$-piperidinylene-$(CH_2)_8-$, $-(CH_2)_5$-piperidinylene-$(CH_2)_1-$, $-(CH_2)_5$-piperidinylene-$(CH_2)_2-$, $-(CH_2)_5$-piperidinylene-$(CH_2)_3-$, $-(CH_2)_5$-piperidinylene-$(CH_2)_4-$, $-(CH_2)_5$-piperidinylene-$(CH_2)_5-$, $-(CH_2)_5$-piperidinylene-$(CH_2)_6-$, $-(CH_2)_5$-piperidinylene-$(CH_2)_7-$, $-(CH_2)_5$-piperidinylene-$(CH_2)_8-$, $-(CH_2)_6$-piperidinylene-$(CH_2)_1-$, $-(CH_2)_6$-piperidinylene-$(CH_2)_2-$,

-$(CH_2)_6$-piperidinylene-$(CH_2)_3$-, -$(CH_2)_6$-piperidinylene-$(CH_2)_4$-, -$(CH_2)_6$-piperidinylene-$(CH_2)_5$-, -$(CH_2)_6$-piperidinylene-$(CH_2)_6$-, -$(CH_2)_6$-piperidinylene-$(CH_2)_7$-, -$(CH_2)_6$-piperidinylene-$(CH_2)_8$-, -$(CH_2)_7$-piperidinylene-$(CH_2)_1$-, -$(CH_2)_7$-piperidinylene-$(CH_2)_2$-, -$(CH_2)_7$-piperidinylene-$(CH_2)_3$-, -$(CH_2)_7$-piperidinylene-$(CH_2)_4$-, -$(CH_2)_7$-piperidinylene-$(CH_2)_8$-, -$(CH_2)_8$-piperidinylene-$CH_2$-, -$(CH_2)_8$-piperidinylene-$(CH_2)_2$-, -$(CH_2)_8$-piperidinylene-$(CH_2)_3$-, -$(CH_2)_8$-piperidinylene-$(CH_2)_4$-, -$(CH_2)_8$-piperidinylene-$(CH_2)_5$-, -$(CH_2)_8$-piperidinylene-$(CH_2)_6$-, -$(CH_2)_8$-piperidinylene-$(CH_2)_7$-, -$(CH_2)_8$-piperidinylene-$(CH_2)_8$-, -$CH_2$-piperazinylene-$CH_2$-, -$CH_2$-piperazinylene-$(CH_2)_2$-, -$CH_2$-piperazinylene-$(CH_2)_3$-, -$CH_2$-piperazinylene-$(CH_2)_4$-, -$CH_2$-piperazinylene-$(CH_2)_5$-, -$CH_2$-piperazinylene-$(CH_2)_6$-, -$CH_2$-piperazinylene-$(CH_2)_7$-, -$CH_2$-piperazinylene-$(CH_2)_8$-, -$(CH_2)_2$-piperazinylene-$(CH_2)_1$-, -$(CH_2)_2$-piperazinylene-$(CH_2)_2$-, -$(CH_2)_2$-piperazinylene-$(CH_2)_3$-, -$(CH_2)_2$-piperazinylene-$(CH_2)_4$-, -$(CH_2)_2$-piperazinylene-$(CH_2)_5$-, -$(CH_2)_2$-piperazinylene-$(CH_2)_6$-, -$(CH_2)_2$-piperazinylene-$(CH_2)_7$-, -$(CH_2)_2$-piperazinylene-$(CH_2)_8$-, -$(CH_2)_3$-piperazinylene-$CH_2$-, -$(CH_2)_3$-piperazinylene-$(CH_2)_2$-, -$(CH_2)_3$-piperazinylene-$(CH_2)_3$-, -$(CH_2)_3$-piperazinylene-$(CH_2)_4$-, -$(CH_2)_3$-piperazinylene-$(CH_2)_5$-, -$(CH_2)_3$-piperazinylene-$(CH_2)_6$-, -$(CH_2)_3$-piperazinylene-$(CH_2)_7$-, -$(CH_2)_3$-piperazinylene-$(CH_2)_8$-, -$(CH_2)_4$-piperazinylene-$CH_2$-, -$(CH_2)_4$-piperazinylene-$(CH_2)_2$-, -$(CH_2)_4$-piperazinylene-$(CH_2)_3$-, -$(CH_2)_4$-piperazinylene-$(CH_2)_4$-, -$(CH_2)_4$-piperazinylene-$(CH_2)_5$-, -$(CH_2)_4$-piperazinylene-$(CH_2)_6$-, -$(CH_2)_4$-piperazinylene-$(CH_2)_7$-, -$(CH_2)_4$-piperazinylene-$(CH_2)_8$-, -$(CH_2)_5$-piperazinylene-$(CH_2)_1$-, -$(CH_2)_5$-piperazinylene-$(CH_2)_2$-, -$(CH_2)_5$-piperazinylene-$(CH_2)_3$-, -$(CH_2)_5$-piperazinylene-$(CH_2)_4$-, -$(CH_2)_5$-piperazinylene-$(CH_2)_5$-, -$(CH_2)_5$-piperazinylene-$(CH_2)_6$-, -$(CH_2)_5$-piperazinylene-$(CH_2)_7$-, -$(CH_2)_5$-piperazinylene-$(CH_2)_8$-, -$(CH_2)_6$-piperazinylene-$(CH_2)_1$-, -$(CH_2)_6$-piperazinylene-$(CH_2)_2$-, -$(CH_2)_6$-piperazinylene-$(CH_2)_3$-, -$(CH_2)_6$-piperazinylene-$(CH_2)_4$-, -$(CH_2)_6$-piperazinylene-$(CH_2)_5$-, -$(CH_2)_6$-piperazinylene-$(CH_2)_6$-, -$(CH_2)_6$-piperazinylene-$(CH_2)_7$-, -$(CH_2)_6$-piperazinylene-$(CH_2)_8$-, -$(CH_2)_7$-piperazinylene-$(CH_2)_1$-, -$(CH_2)_7$-piperazinylene-$(CH_2)_2$-, -$(CH_2)_7$-piperazinylene-$(CH_2)_3$-, -$(CH_2)_7$-piperazinylene-$(CH_2)_4$-, -$(CH_2)_7$-piperazinylene-$(CH_2)_8$-, -$(CH_2)_8$-piperazinylene-$CH_2$-, -$(CH_2)_8$-piperazinylene-$(CH_2)_2$-, -$(CH_2)_8$-piperazinylene-$(CH_2)_3$-, -$(CH_2)_8$-piperazinylene-$(CH_2)_4$-, -$(CH_2)_8$-piperazinylene-$(CH_2)_5$-, -$(CH_2)_8$-piperazinylene-$(CH_2)_6$-, -$(CH_2)_8$-piperazinylene-$(CH_2)_7$-, -$(CH_2)_8$-piperazinylene-$(CH_2)_8$-, -$CH_2$-propylene-$CH_2$-, -$CH_2$-propylene-$(CH_2)_2$-, -$CH_2$-propylene-$(CH_2)_3$-, -$CH_2$-propylene-$(CH_2)_4$-, -$CH_2$-propylene-$(CH_2)_5$-, -$CH_2$-propylene-$(CH_2)_6$-, -$CH_2$-propylene-$(CH_2)_7$-, -$CH_2$-propylene-$(CH_2)_8$-, -$(CH_2)_2$-propylene-$(CH_2)_1$-, -$(CH_2)_2$-propylene-$(CH_2)_2$-, -$(CH_2)_2$-propylene-$(CH_2)_3$-, -$(CH_2)_2$-propylene-$(CH_2)_4$-, -$(CH_2)_2$-propylene-$(CH_2)_5$-, -$(CH_2)_2$-propylene-$(CH_2)_6$-, -$(CH_2)_2$-propylene-$(CH_2)_7$-, -$(CH_2)_2$-propylene-$(CH_2)_8$-, -$(CH_2)_3$-propylene-$CH_2$-, -$(CH_2)_3$-propylene-$(CH_2)_2$-, -$(CH_2)_3$-propylene-$(CH_2)_3$-, -$(CH_2)_3$-propylene-$(CH_2)_4$-, - $(CH_2)_3$-propylene-$(CH_2)_5$-, -$(CH_2)_3$-propylene-$(CH_2)_6$-, -$(CH_2)_3$-propylene-$(CH_2)_7$-, -$(CH_2)_3$-propylene-$(CH_2)_8$-, -$(CH_2)_4$-propylene-$CH_2$-, -$(CH_2)_4$-propylene-$(CH_2)_2$-, -$(CH_2)_4$-propylene-$(CH_2)_3$-, -$(CH_2)_4$-propylene-$(CH_2)_4$-, -$(CH_2)_4$-propylene-$(CH_2)_5$-, -$(CH_2)_4$-propylene-$(CH_2)_6$-, -$(CH_2)_4$-propylene-$(CH_2)_7$-, -$(CH_2)_4$-propylene-$(CH_2)_8$-, -$(CH_2)_5$-propylene-$(CH_2)_1$-, -$(CH_2)_5$-propylene-$(CH_2)_2$-, -$(CH_2)_5$-propylene-$(CH_2)_3$-, -$(CH_2)_5$-propylene-$(CH_2)_4$-, -$(CH_2)_5$-propylene-$(CH_2)_5$-, -$(CH_2)_5$-propylene-$(CH_2)_6$-, -$(CH_2)_5$-propylene-$(CH_2)_7$-, -$(CH_2)_5$-propylene-$(CH_2)_8$-, -$(CH_2)_6$-propylene-$(CH_2)_1$-, -$(CH_2)_6$-propylene-$(CH_2)_2$-, -$(CH_2)_6$-propylene-$(CH_2)_3$-, -$(CH_2)_6$-propylene-$(CH_2)_4$-, -$(CH_2)_6$-propylene-$(CH_2)_5$-, -$(CH_2)_6$-propylene-$(CH_2)_6$-, -$(CH_2)_6$-propylene-$(CH_2)_7$-, -$(CH_2)_6$-propylene-$(CH_2)_8$-, -$(CH_2)_7$-propylene-$(CH_2)_1$-, -$(CH_2)_7$-propylene-$(CH_2)_2$-, -$(CH_2)_7$-propylene-$(CH_2)_3$-, -$(CH_2)_7$-propylene-$(CH_2)_4$-, -$(CH_2)_7$-propylene-$(CH_2)_8$-, -$(CH_2)_8$-propylene-$CH_2$-, -$(CH_2)_8$-propylene-$(CH_2)_2$-, -$(CH_2)_8$-propylene-$(CH_2)_3$-, -$(CH_2)_8$-propylene-$(CH_2)_4$-, -$(CH_2)_8$-propylene-$(CH_2)_5$-, -$(CH_2)_8$-propylene-$(CH_2)_6$-, -$(CH_2)_8$-propylene-$(CH_2)_7$-, -$(CH_2)_8$-propylene-$(CH_2)_8$-, -$CH_2$-diazacycloheptanylene-$CH_2$-, -$CH_2$-diazacycloheptanylene-$(CH_2)_2$-, -$CH_2$-diazacycloheptanylene-$(CH_2)_3$-, -$CH_2$-diazacycloheptanylene-$(CH_2)_4$-, -$CH_2$-diazacycloheptanylene-$(CH_2)_5$-, -$CH_2$-diazacycloheptanylene-$(CH_2)_6$-, -$CH_2$-diazacycloheptanylene-$(CH_2)_7$-, -$CH_2$-diazacycloheptanylene-$(CH_2)_8$-, -$(CH_2)_2$-diazacycloheptanylene-$(CH_2)_1$-, -$(CH_2)_2$-diazacycloheptanylene-$(CH_2)_2$-, -$(CH_2)_2$-diazacycloheptanylene-$(CH_2)_3$-, -$(CH_2)_2$-diazacycloheptanylene-$(CH_2)_4$-, -$(CH_2)_2$-diazacycloheptanylene-$(CH_2)_5$-, -$(CH_2)_2$-diazacycloheptanylene-$(CH_2)_6$-, -$(CH_2)_2$-diazacycloheptanylene-$(CH_2)_7$-, -$(CH_2)_2$-diazacycloheptanylene-$(CH_2)_8$-, -$(CH_2)_3$-diazacycloheptanylene-$CH_2$-, -$(CH_2)_3$-diazacycloheptanylene-$(CH_2)_2$-, -$(CH_2)_3$-diazacycloheptanylene-$(CH_2)_3$-, -$(CH_2)_3$-diazacycloheptanylene-$(CH_2)_4$-, -$(CH_2)_3$-diazacycloheptanylene-$(CH_2)_5$-, -$(CH_2)_3$-diazacycloheptanylene-$(CH_2)_6$-, -$(CH_2)_3$-diazacycloheptanylene-$(CH_2)_7$-, -$(CH_2)_3$-diazacycloheptanylene-$(CH_2)_8$-, -$(CH_2)_4$-diazacycloheptanylene-$CH_2$-, -$(CH_2)_4$-diazacycloheptanylene-$(CH_2)_2$-, -$(CH_2)_4$-diazacycloheptanylene-$(CH_2)_3$-, -$(CH_2)_4$-diazacycloheptanylene-$(CH_2)_4$-, -$(CH_2)_4$-diazacycloheptanylene-$(CH_2)_5$-, -$(CH_2)_4$-diazacycloheptanylene-$(CH_2)_6$-, -$(CH_2)_4$-diazacycloheptanylene-$(CH_2)_7$-, -$(CH_2)_4$-diazacycloheptanylene-$(CH_2)_8$-, -$(CH_2)_5$-diazacycloheptanylene-$(CH_2)_1$-, -$(CH_2)_5$-diazacycloheptanylene-$(CH_2)_2$-, -$(CH_2)_5$-diazacycloheptanylene-$(CH_2)_3$-, -$(CH_2)_5$-diazacycloheptanylene-$(CH_2)_4$-, -$(CH_2)_5$-diazacycloheptanylene-$(CH_2)_5$-, -$(CH_2)_5$-diazacycloheptanylene-$(CH_2)_6$-, -$(CH_2)_5$-diazacycloheptanylene-$(CH_2)_7$-, -$(CH_2)_5$-diazacycloheptanylene-$(CH_2)_8$-, -$(CH_2)_6$-diazacycloheptanylene-$(CH_2)_1$-, -$(CH_2)_6$-diazacycloheptanylene-$(CH_2)_2$-, -$(CH_2)_6$-diazacycloheptanylene-$(CH_2)_3$-, -$(CH_2)_6$-diazacycloheptanylene-$(CH_2)_4$-, -$(CH_2)_6$-diazacycloheptanylene-$(CH_2)_5$-, -$(CH_2)_6$-diazacycloheptanylene-$(CH_2)_6$-, -$(CH_2)_6$-diazacycloheptanylene-$(CH_2)_7$-, -$(CH_2)_6$-diazacycloheptanylene-$(CH_2)_8$-, -$(CH_2)_7$-diazacycloheptanylene-$(CH_2)_1$-, -$(CH_2)_7$-diaza-

cycloheptanylene-$(CH_2)_2$-, -$(CH_2)_7$-diazacycloheptanylene-$(CH_2)_3$-, -$(CH_2)_7$-diazacycloheptanylene-$(CH_2)_4$-, -$(CH_2)_7$-diazacycloheptanylene-$(CH_2)_8$-, -$(CH_2)_8$-diazacycloheptanylene-$CH_2$-, -$(CH_2)_8$-diazacycloheptanylene-$(CH_2)_2$-, -$(CH_2)_8$-diazacycloheptanylene-$(CH_2)_3$-, -$(CH_2)_8$-diazacycloheptanylene-$(CH_2)_4$-, -$(CH_2)_8$-diazacycloheptanylene-$(CH_2)_5$-, -$(CH_2)_8$-diazacycloheptanylene-$(CH_2)_6$-, -$(CH_2)_8$-diazacycloheptanylene-$(CH_2)_7$-, -$(CH_2)_8$-diazacycloheptanylene-$(CH_2)_8$-, -$CH_2$-diazabicyclo[2.2.1]heptanylene-$CH_2$-, -$CH_2$-diazabicyclo[2.2.1]heptanylene-$(CH_2)_2$-, -$CH_2$-diazabicyclo[2.2.1]heptanylene-$(CH_2)_3$-, -$CH_2$-diazabicyclo[2.2.1]heptanylene-$(CH_2)_4$-, -$CH_2$-diazabicyclo[2.2.1]heptanylene-$(CH_2)_5$-, -$CH_2$-diazabicyclo[2.2.1]heptanylene-$(CH_2)_6$-, -$CH_2$-diazabicyclo[2.2.1]heptanylene-$(CH_2)_7$-, -$CH_2$-diazabicyclo[2.2.1]heptanylene-$(CH_2)_8$-, -$(CH_2)_2$-diazabicyclo[2.2.1]heptanylene-$(CH_2)_1$-, -$(CH_2)_2$-diazabicyclo[2.2.1]heptanylene-$(CH_2)_2$-, -$(CH_2)_2$-diazabicyclo[2.2.1]heptanylene-$(CH_2)_3$-, -$(CH_2)_2$-diazabicyclo[2.2.1]heptanylene-$(CH_2)_4$-, -$(CH_2)_2$-diazabicyclo[2.2.1]heptanylene-$(CH_2)_5$-, -$(CH_2)_2$-diazabicyclo[2.2.1]heptanylene-$(CH_2)_6$-, -$(CH_2)_2$-diazabicyclo[2.2.1]heptanylene-$(CH_2)_7$-, -$(CH_2)_2$-diazabicyclo[2.2.1]heptanylene-$(CH_2)_8$-, -$(CH_2)_3$-diazabicyclo[2.2.1]heptanylene-$CH_2$-, -$(CH_2)_3$-diazabicyclo[2.2.1]heptanylene-$(CH_2)_2$-, -$(CH_2)_3$-diazabicyclo[2.2.1]heptanylene-$(CH_2)_3$-, -$(CH_2)_3$-diazabicyclo[2.2.1]heptanylene-$(CH_2)_4$-, -$(CH_2)_3$-diazabicyclo[2.2.1]heptanylene-$(CH_2)_5$-, -$(CH_2)_3$-diazabicyclo[2.2.1]heptanylene-$(CH_2)_6$-, -$(CH_2)_3$-diazabicyclo[2.2.1]heptanylene-$(CH_2)_7$-, -$(CH_2)_3$-diazabicyclo[2.2.1]heptanylene-$(CH_2)_8$-, -$(CH_2)_4$-diazabicyclo[2.2.1]heptanylene-$CH_2$-, -$(CH_2)_4$-diazabicyclo[2.2.1]heptanylene-$(CH_2)_2$-, -$(CH_2)_4$-diazabicyclo[2.2.1]heptanylene-$(CH_2)_3$-, -$(CH_2)_4$-diazabicyclo[2.2.1]heptanylene-$(CH_2)_4$-, -$(CH_2)_4$-diazabicyclo[2.2.1]heptanylene-$(CH_2)_5$-, -$(CH_2)_4$-diazabicyclo[2.2.1]heptanylene-$(CH_2)_6$-, -$(CH_2)_4$-diazabicyclo[2.2.1]heptanylene-$(CH_2)_7$-, -$(CH_2)_4$-diazabicyclo[2.2.1]heptanylene-$(CH_2)_8$-, -$(CH_2)_5$-diazabicyclo[2.2.1]heptanylene-$(CH_2)_1$-, -$(CH_2)_5$-diazabicyclo[2.2.1]heptanylene-$(CH_2)_2$-, -$(CH_2)_5$-diazabicyclo[2.2.1]heptanylene-$(CH_2)_3$-, -$(CH_2)_5$-diazabicyclo[2.2.1]heptanylene-$(CH_2)_4$-, -$(CH_2)_5$-diazabicyclo[2.2.1]heptanylene-$(CH_2)_5$-, -$(CH_2)_5$-diazabicyclo[2.2.1]heptanylene-$(CH_2)_6$-, -$(CH_2)_5$-diazabicyclo[2.2.1]heptanylene-$(CH_2)_7$-, -$(CH_2)_5$-diazabicyclo[2.2.1]heptanylene-$(CH_2)_8$-, -$(CH_2)_6$-diazabicyclo[2.2.1]heptanylene-$(CH_2)_1$-, -$(CH_2)_6$-diazabicyclo[2.2.1]heptanylene-$(CH_2)_2$-, -$(CH_2)_6$-diazabicyclo[2.2.1]heptanylene-$(CH_2)_3$-, -$(CH_2)_6$-diazabicyclo[2.2.1]heptanylene-$(CH_2)_4$-, -$(CH_2)_6$-diazabicyclo[2.2.1]heptanylene-$(CH_2)_5$-, -$(CH_2)_6$-diazabicyclo[2.2.1]heptanylene-$(CH_2)_6$-, -$(CH_2)_6$-diazabicyclo[2.2.1]heptanylene-$(CH_2)_7$-, -$(CH_2)_6$-diazabicyclo[2.2.1]heptanylene-$(CH_2)_8$-, -$(CH_2)_7$-diazabicyclo[2.2.1]heptanylene-$(CH_2)_1$-, -$(CH_2)_7$-diazabicyclo[2.2.1]heptanylene-$(CH_2)_2$-, -$(CH_2)_7$-diazabicyclo[2.2.1]heptanylene-$(CH_2)_3$-, -$(CH_2)_7$-diazabicyclo[2.2.1]heptanylene-$(CH_2)_4$-, -$(CH_2)_7$-diazabicyclo[2.2.1]heptanylene-$(CH_2)_8$-, -$(CH_2)_8$-diazabicyclo[2.2.1]heptanylene-$CH_2$-, -$(CH_2)_8$-diazabicyclo[2.2.1]heptanylene-$(CH_2)_2$-, -$(CH_2)_8$-diazabicyclo[2.2.1]heptanylene-$(CH_2)_3$-, -$(CH_2)_8$-diazabicyclo[2.2.1]heptanylene-$(CH_2)_4$-, -$(CH_2)_8$-diazabicyclo[2.2.1]heptanylene-$(CH_2)_5$-, -$(CH_2)_8$-diazabicyclo[2.2.1]heptanylene-$(CH_2)_6$-, -$(CH_2)_8$-diazabicyclo[2.2.1]heptanylene-$(CH_2)_7$-, -$(CH_2)_8$-diazabicyclo[2.2.1]heptanylene-$(CH_2)_8$-, -$CH_2$-diazabicyclo[3.1.1]heptanylene-$CH_2$-, -$CH_2$-diazabicyclo[3.1.1]heptanylene-$(CH_2)_2$-, -$CH_2$-diazabicyclo[3.1.1]heptanylene-$(CH_2)_3$-, -$CH_2$-diazabicyclo[3.1.1]heptanylene-$(CH_2)_4$-, -$CH_2$-diazabicyclo[3.1.1]heptanylene-$(CH_2)_5$-, -$CH_2$-diazabicyclo[3.1.1]heptanylene-$(CH_2)_6$-, -$CH_2$-diazabicyclo[3.1.1]heptanylene-$(CH_2)_7$-, -$CH_2$-diazabicyclo[3.1.1]heptanylene-$(CH_2)_8$-, -$(CH_2)_2$-diazabicyclo[3.1.1]heptanylene-$(CH_2)_1$-, -$(CH_2)_2$-diazabicyclo[3.1.1]heptanylene-$(CH_2)_2$-, -$(CH_2)_2$-diazabicyclo[3. 1. 1]heptanylene-$(CH_2)_3$-, -$(CH_2)_2$-diazabicyclo[3.1. 1]heptanylene-$(CH_2)_4$-, -$(CH_2)_2$-diazabicyclo[3. 1. 1]heptanylene-$(CH_2)_5$-, -$(CH_2)_2$-diazabicyclo[3.1. 1]heptanylene-$(CH_2)_6$-, -$(CH_2)_2$-diazabicyclo[3. 1. 1]heptanylene-$(CH_2)_7$-, -$(CH_2)_2$-diazabicyclo[3.1. 1]heptanylene-$(CH_2)_8$-, -$(CH_2)_3$-diazabicyclo[3.1.1]heptanylene-$CH_2$-, -$(CH_2)_3$-diazabicyclo[3.1.1]heptanylene-$(CH_2)_2$-, -$(CH_2)_3$-diazabicyclo[3.1.1]heptanylene-$(CH_2)_3$-, -$(CH_2)_3$-diazabicyclo[3.1.1]heptanylene-$(CH_2)_4$-, -$(CH_2)_3$-diazabicyclo[3.1.1]heptanylene-$(CH_2)_5$-, -$(CH_2)_3$-diazabicyclo[3.1.1]heptanylene-$(CH_2)_6$-, -$(CH_2)_3$-diazabicyclo[3.1.1]heptanylene-$(CH_2)_7$-, -$(CH_2)_3$-diazabicyclo[3.1.1]heptanylene-$(CH_2)_8$-, -$(CH_2)_4$-diazabicyclo[3.1.1]heptanylene-$CH_2$-, -$(CH_2)_4$-diazabicyclo[3.1.1]heptanylene-$(CH_2)_2$-, -$(CH_2)_4$-diazabicyclo[3.1.1]heptanylene-$(CH_2)_3$-, -$(CH_2)_4$-diazabicyclo[3.1.1]heptanylene-$(CH_2)_4$-, -$(CH_2)_4$-diazabicyclo[3. 1. 1]heptanylene-$(CH_2)_5$-, -$(CH_2)_4$-diazabicyclo[3.1. 1]heptanylene-$(CH_2)_6$-, -$(CH_2)_4$-diazabicyclo[3. 1. 1]heptanylene-$(CH_2)_7$-, -$(CH_2)_4$-diazabicyclo[3.1. 1]heptanylene-$(CH_2)_8$-, -$(CH_2)_5$-diazabicyclo[3.1.1]heptanylene-$(CH_2)_1$-, -$(CH_2)_5$-diazabicyclo[3.1.1]heptanylene-$(CH_2)_2$-, -$(CH_2)_5$-diazabicyclo[3.1.1]heptanylene-$(CH_2)_3$-, -$(CH_2)_5$-diazabicyclo[3.1.1]heptanylene-$(CH_2)_4$-, -$(CH_2)_5$-diazabicyclo[3.1.1]heptanylene-$(CH_2)_5$-, -$(CH_2)_5$-diazabicyclo[3.1.1]heptanylene-$(CH_2)_6$-, -$(CH_2)_5$-diazabicyclo[3.1.1]heptanylene-$(CH_2)_7$-, -$(CH_2)_5$-diazabicyclo[3.1.1]heptanylene-$(CH_2)_8$-, -$(CH_2)_6$-diazabicyclo[3.1.1]heptanylene-$(CH_2)_1$-, -$(CH_2)_6$-diazabicyclo[3.1.1]heptanylene-$(CH_2)_2$-, -$(CH_2)_6$-diazabicyclo[3.1.1]heptanylene-$(CH_2)_3$-, -$(CH_2)_6$-diazabicyclo[3.1.1]heptanylene-$(CH_2)_4$-, -$(CH_2)_6$-diazabicyclo[3. 1. 1]heptanylene-$(CH_2)_5$-, -$(CH_2)_6$-diazabicyclo[3.1. 1]heptanylene-$(CH_2)_6$-, -$(CH_2)_6$-diazabicyclo[3. 1. 1]heptanylene-$(CH_2)_7$-, -$(CH_2)_6$-diazabicyclo[3.1. 1]heptanylene-$(CH_2)_8$-, -$(CH_2)_7$-diazabicyclo[3.1.1]heptanylene-$(CH_2)_1$-, -$(CH_2)_7$-diazabicyclo[3.1.1]heptanylene-$(CH_2)_2$-, -$(CH_2)_7$-diazabicyclo[3.1.1]heptanylene-$(CH_2)_3$-, -$(CH_2)_7$-diazabicyclo[3.1.1]heptanylene-$(CH_2)_4$-, -$(CH_2)_7$-diazabicyclo[3.1.1]heptanylene-$(CH_2)_8$-, -$(CH_2)_8$-diazabicyclo[3.1.1]heptanylene-$CH_2$-, -$(CH_2)_8$-diazabicyclo[3.1.1]heptanylene-$(CH_2)_2$-, -$(CH_2)_8$-diazabicyclo[3.1.1]heptanylene-$(CH_2)_3$-, -$(CH_2)_8$-diazabicyclo[3.1.1]heptanylene-$(CH_2)_4$-, -$(CH_2)_8$-diazabicyclo[3.1.1]hep-

tanylene-$(CH_2)_5$-, -$(CH_2)_8$-diazabicyclo[3.1.1]heptanylene-$(CH_2)_6$-, -$(CH_2)_8$-diazabicyclo[3.1.1]heptanylene-$(CH_2)_7$-, -$(CH_2)_8$-diazabicyclo[3.1. 1]heptanylene-$(CH_2)_8$-, -$CH_2$-diazabicyclo[3.2.1]octylene-$CH_2$-, -$CH_2$-diazabicyclo[3.2. 1]octylene-$(CH_2)_2$-, -$CH_2$-diazabicyclo[3.2.1]octylene-$(CH_2)_3$-, -$CH_2$-diazabicyclo[3.2. 1]octylene-$(CH_2)_4$-, -$CH_2$-diazabicyclo[3.2.1]octylene-$(CH_2)_5$-, -$CH_2$-diazabicyclo[3.2. 1]octylene-$(CH_2)_6$-, -$CH_2$-diazabicyclo[3.2.1]octylene-$(CH_2)_7$-, -$CH_2$-diazabicyclo[3.2.1]octylene-$(CH_2)_8$-, -$(CH_2)_2$-diazabicyclo[3.2.1]octylene-$(CH_2)_1$-, -$(CH_2)_2$-diazabicyclo[3.2.1]octylene-$(CH_2)_2$-, -$(CH_2)_2$-diazabicyclo[3.2.1]octylene-$(CH_2)_3$-, -$(CH_2)_2$-diazabicyclo[3.2.1]octylene-$(CH_2)_4$-, -$(CH_2)_2$-diazabicyclo[3.2.1]octylene-$(CH_2)_5$-, -$(CH_2)_2$-diazabicyclo[3.2.1]octylene-$(CH_2)_6$-, -$(CH_2)_2$-diazabicyclo[3.2.1]octylene-$(CH_2)_7$-, -$(CH_2)_2$-diazabicyclo[3.2. 1]octylene-$(CH_2)_8$-, -$(CH_2)_3$-diazabicyclo[3.2.1]octylene-$CH_2$-, -$(CH_2)_3$-diazabicyclo[3.2. 1]octylene-$(CH_2)_2$-, -$(CH_2)_3$-diazabicyclo[3.2.1]octylene-$(CH_2)_3$-, -$(CH_2)_3$-diazabicyclo[3.2. 1]octylene-$(CH_2)_4$-, -$(CH_2)_3$-diazabicyclo[3.2.1]octylene-$(CH_2)_5$-, -$(CH_2)_3$-diazabicyclo[3.2.1]octylene-$(CH_2)_6$-, -$(CH_2)_3$-diazabicyclo[3.2.1]octylene-$(CH_2)_7$-, -$(CH_2)_3$-diazabicyclo[3.2.1]octylene-$(CH_2)_8$-, -$(CH_2)_4$-diazabicyclo[3.2.1]octylene-$CH_2$-, -$(CH_2)_4$-diazabicyclo[3.2.1]octylene-$(CH_2)_2$-, -$(CH_2)_4$-diazabicyclo[3.2.1]octylene-$(CH_2)_3$-, -$(CH_2)_4$-diazabicyclo[3.2.1]octylene-$(CH_2)_4$-, -$(CH_2)_4$-diazabicyclo[3.2.1]octylene-$(CH_2)_5$-, -$(CH_2)_4$-diazabicyclo[3.2.1]octylene-$(CH_2)_6$-, -$(CH_2)_4$-diazabicyclo[3.2.1]octylene-$(CH_2)_7$-, -$(CH_2)_4$-diazabicyclo[3.2.1]octylene-$(CH_2)_8$-, -$(CH_2)_5$-diazabicyclo[3.2.1]octylene-$(CH_2)_1$-, -$(CH_2)_5$-diazabicyclo[3.2.1]octylene-$(CH_2)_2$-, -$(CH_2)_5$-diazabicyclo[3.2.1]octylene-$(CH_2)_3$-, -$(CH_2)_5$-diazabicyclo[3.2.1]octylene-$(CH_2)_4$-, -$(CH_2)_5$-diazabicyclo[3.2.1]octylene-$(CH_2)_5$-, -$(CH_2)_5$-diazabicyclo[3.2.1]octylene-$(CH_2)_6$-, -$(CH_2)_5$-diazabicyclo[3.2.1]octylene-$(CH_2)_7$-, -$(CH_2)_5$-diazabicyclo[3.2.1]octylene-$(CH_2)_8$-, -$(CH_2)_6$-diazabicyclo[3.2.1]octylene-$(CH_2)_1$-, -$(CH_2)_6$-diazabicyclo[3.2.1]octylene-$(CH_2)_2$-, -$(CH_2)_6$-diazabicyclo[3.2.1]octylene-$(CH_2)_3$-, -$(CH_2)_6$-diazabicyclo[3.2.1]octylene-$(CH_2)_4$-, -$(CH_2)_6$-diazabicyclo[3.2.1]octylene-$(CH_2)_5$-, -$(CH_2)_6$-diazabicyclo[3.2.1]octylene-$(CH_2)_6$-, -$(CH_2)_6$-diazabicyclo[3.2.1]octylene-$(CH_2)_7$-, -$(CH_2)_6$-diazabicyclo[3.2.1]octylene-$(CH_2)_8$-, -$(CH_2)_7$-diazabicyclo[3.2.1]octylene-$(CH_2)_1$-, -$(CH_2)_7$-diazabicyclo[3.2.1]octylene-$(CH_2)_2$-, -$(CH_2)_7$-diazabicyclo[3.2.1]octylene-$(CH_2)_3$-, -$(CH_2)_7$-diazabicyclo[3.2.1]octylene-$(CH_2)_4$-, -$(CH_2)_7$-diazabicyclo[3.2.1]octylene-$(CH_2)_8$-, -$(CH_2)_8$-diazabicyclo[3.2.1]octylene-$CH_2$-, -$(CH_2)_8$-diazabicyclo[3.2.1]octylene-$(CH_2)_2$-, -$(CH_2)_8$-diazabicyclo[3.2.1]octylene-$(CH_2)_3$-, -$(CH_2)_8$-diazabicyclo[3.2.1]octylene-$(CH_2)_4$-, -$(CH_2)_8$-diazabicyclo[3.2.1]octylene-$(CH_2)_5$-, -$(CH_2)_8$-diazabicyclo[3.2.1]octylene-$(CH_2)_6$-, -$(CH_2)_8$-diazabicyclo[3.2.1]octylene-$(CH_2)_7$-, -$(CH_2)_8$-diazabicyclo[3.2.1]octylene-$(CH_2)_8$-, -$CH_2$-diazabicyclo[2.2.2]octylene-$CH_2$-, -$CH_2$-diazabicyclo[2.2.2]octylene-$(CH_2)_2$-, -$CH_2$-diazabicyclo[2.2.2]octylene-$(CH_2)_3$-, -$CH_2$-diazabicyclo[2.2.2]octylene-$(CH_2)_4$-, -$CH_2$-diazabicyclo[2.2.2]octylene-$(CH_2)_5$-, -$CH_2$-diazabicyclo[2.2.2]octylene-$(CH_2)_6$-, -$CH_2$-diazabicyclo[2.2.2]octylene-$(CH_2)_7$-, -$CH_2$-diazabicyclo[2.2.2]octylene-$(CH_2)_8$-, -$(CH_2)_2$-diazabicyclo[2.2.2]octylene-$(CH_2)_1$-, -$(CH_2)_2$-diazabicyclo[2.2.2]octylene-$(CH_2)_2$-, -$(CH_2)_2$-diazabicyclo[2.2.2]octylene-$(CH_2)_3$-, -$(CH_2)_2$-diazabicyclo[2.2.2]octylene-$(CH_2)_4$-, -$(CH_2)_2$-diazabicyclo[2.2.2]octylene-$(CH_2)_5$-, -$(CH_2)_2$-diazabicyclo[2.2.2]octylene-$(CH_2)_6$-, -$(CH_2)_2$-diazabicyclo[2.2.2]octylene-$(CH_2)_7$-, -$(CH_2)_2$-diazabicyclo[2.2.2]octylene-$(CH_2)_8$-, -$(CH_2)_3$-diazabicyclo[2.2.2]octylene-$CH_2$-, -$(CH_2)_3$-diazabicyclo[2.2.2]octylene-$(CH_2)_2$-, -$(CH_2)_3$-diazabicyclo[2.2.2]octylene-$(CH_2)_3$-, -$(CH_2)_3$-diazabicyclo[2.2.2]octylene-$(CH_2)_4$-, -$(CH_2)_3$-diazabicyclo[2.2.2]octylene-$(CH_2)_5$-, -$(CH_2)_3$-diazabicyclo[2.2.2]octylene-$(CH_2)_6$-, -$(CH_2)_3$-diazabicyclo[2.2.2]octylene-$(CH_2)_7$-, -$(CH_2)_3$-diazabicyclo[2.2.2]octylene-$(CH_2)_8$-, -$(CH_2)_4$-diazabicyclo[2.2.2]octylene-$CH_2$-, -$(CH_2)_4$-diazabicyclo[2.2.2]octylene-$(CH_2)_2$-, -$(CH_2)_4$-diazabicyclo[2.2.2]octylene-$(CH_2)_3$-, -$(CH_2)_4$-diazabicyclo[2.2.2]octylene-$(CH_2)_4$-, -$(CH_2)_4$-diazabicyclo[2.2.2]octylene-$(CH_2)_5$-, -$(CH_2)_4$-diazabicyclo[2.2.2]octylene-$(CH_2)_6$-, -$(CH_2)_4$-diazabicyclo[2.2.2]octylene-$(CH_2)_7$-, -$(CH_2)_4$-diazabicyclo[2.2.2]octylene-$(CH_2)_8$-, -$(CH_2)_5$-diazabicyclo[2.2.2]octylene-$(CH_2)_1$-, -$(CH_2)_5$-diazabicyclo[2.2.2]octylene-$(CH_2)_2$-, -$(CH_2)_5$-diazabicyclo[2.2.2]octylene-$(CH_2)_3$-, -$(CH_2)_5$-diazabicyclo[2.2.2]octylene-$(CH_2)_4$-, -$(CH_2)_5$-diazabicyclo[2.2.2]octylene-$(CH_2)_5$-, -$(CH_2)_5$-diazabicyclo[2.2.2]octylene-$(CH_2)_6$-, -$(CH_2)_5$-diazabicyclo[2.2.2]octylene-$(CH_2)_7$-, -$(CH_2)_5$-diazabicyclo[2.2.2]octylene-$(CH_2)_8$-, -$(CH_2)_6$-diazabicyclo[2.2.2]octylene-$(CH_2)_1$-, -$(CH_2)_6$-diazabicyclo[2.2.2]octylene-$(CH_2)_2$-, -$(CH_2)_6$-diazabicyclo[2.2.2]octylene-$(CH_2)_3$-, -$(CH_2)_6$-diazabicyclo[2.2.2]octylene-$(CH_2)_4$-, -$(CH_2)_6$-diazabicyclo[2.2.2]octylene-$(CH_2)_5$-, -$(CH_2)_6$-diazabicyclo[2.2.2]octylene-$(CH_2)_6$-, -$(CH_2)_6$-diazabicyclo[2.2.2]octylene-$(CH_2)_7$-, -$(CH_2)_6$-diazabicyclo[2.2.2]octylene-$(CH_2)_8$-, -$(CH_2)_7$-diazabicyclo[2.2.2]octylene-$(CH_2)_1$-, -$(CH_2)_7$-diazabicyclo[2.2.2]octylene-$(CH_2)_2$-, -$(CH_2)_7$-diazabicyclo[2.2.2]octylene-$(CH_2)_3$-, -$(CH_2)_7$-diazabicyclo[2.2.2]octylene-$(CH_2)_4$-, -$(CH_2)_7$-diazabicyclo[2.2.2]octylene-$(CH_2)_8$-, -$(CH_2)_8$-diazabicyclo[2.2.2]octylene-$CH_2$-, -$(CH_2)_8$-diazabicyclo[2.2.2]octylene-$(CH_2)_2$-, -$(CH_2)_8$-diazabicyclo[2.2.2]octylene-$(CH_2)_3$-, -$(CH_2)_8$-diazabicyclo[2.2.2]octylene-$(CH_2)_4$-, -$(CH_2)_8$-diazabicyclo[2.2.2]octylene-$(CH_2)_5$-, -$(CH_2)_8$-diazabicyclo[2.2.2]octylene-$(CH_2)_6$-, -$(CH_2)_8$-diazabicyclo[2.2.2]octylene-$(CH_2)_7$-, or -$(CH_2)_8$-diazabicyclo[2.2.2]octylene-$(CH_2)_8$-,

wherein the propylene, the piperidinylene, the piperazinylene, the diazacycloheptanylene, the diazabicyclo[2.2.1]heptanylene, the diazabicyclo[3.1.1]heptanylene, the diazabicyclo[3.2.1]octylene, the diazabicyclo[2.2.2]octylene are each independently optionally substituted with a substituent selected from the group consisting of deuterium, optionally deuterated $C_{1-4}$ alkyl, optionally deuterated $C_{3-6}$cycloalkyl, hydroxy,

amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH- or any combination thereof; and
each $R_{g11}$ independently represents H or $C_{1-3}$ alkyl; or
even more preferably, wherein $L_2$ or $L_3$ each independently represent the structure of the following formula:

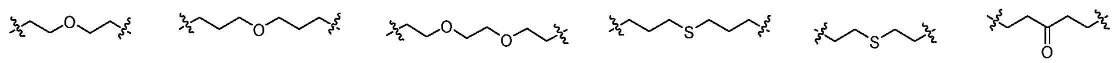

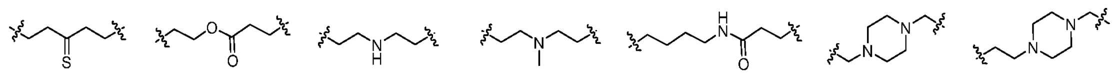

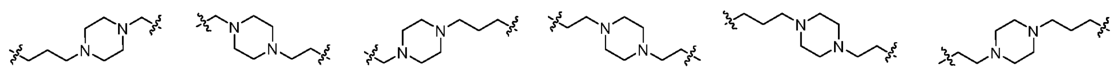

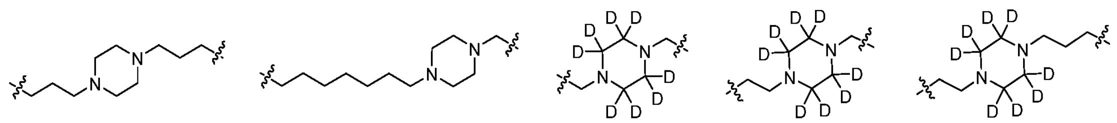

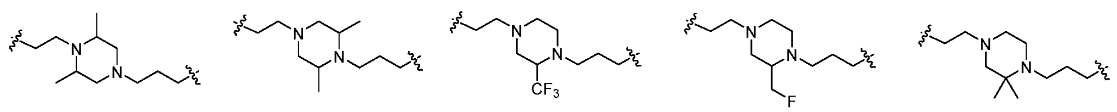

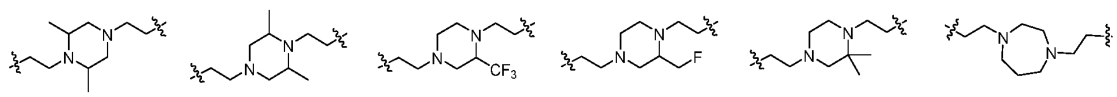

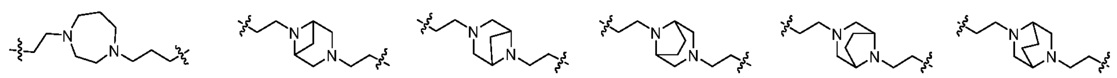

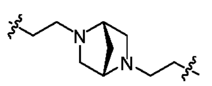 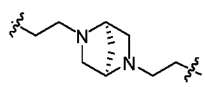 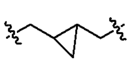 or 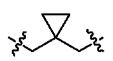

**34.** The compound of Formula (I) or salts, enantiomers, diastereoisomer, isotopically enriched analogs, solvates, prodrugs, or polymorphs thereof as claimed in claim 1, wherein $R_{b1}$ represents:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, 2-oxobicyclo[2.2.1]heptanyl or bicyclo[2.2.1]heptenyl, with each group being optionally substituted with one or more substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;
azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo [3.2.1]octyl, 3,8-diazabicyclo[3.2.1]octyl, 2,5-diazabicyclo[2.2.2]octyl, 3-azaspiro[5.5]undecyl or 7-azaspiro [3.5]nonyl, with each group being optionally substituted with one or more substituents selected from the group

consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

phenyl or naphthyl, wherein the phenyl or naphthyl is optionally substituted with one or more substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo [2,1-b]thiazolyl and imidazo[2,1-b]thiazolyl, with each group being optionally substituted with one or more substituents selected from the group consisting of D, optionally deuterated $C_{1-4}$ alkyl, optionally substituted $C_{3-15}$cycloalkyl, optionally substituted 4-to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

preferably, wherein $R_{b1}$ represents:

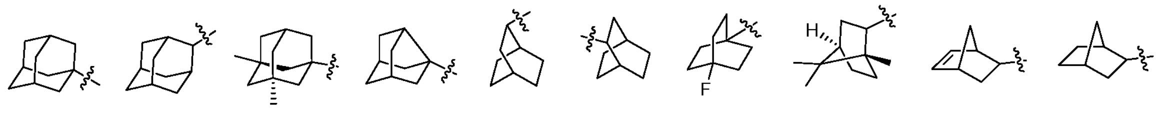

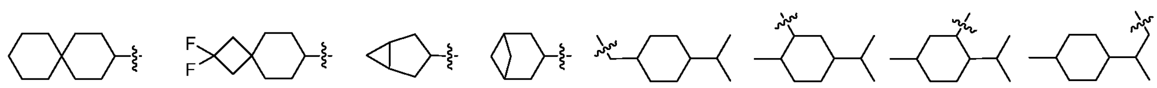

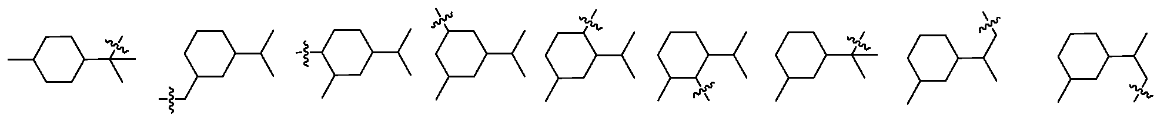

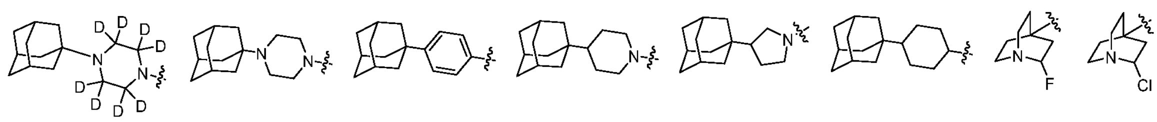

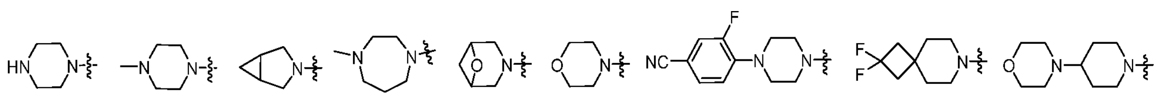

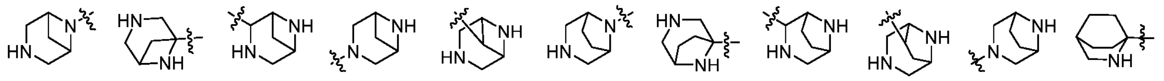

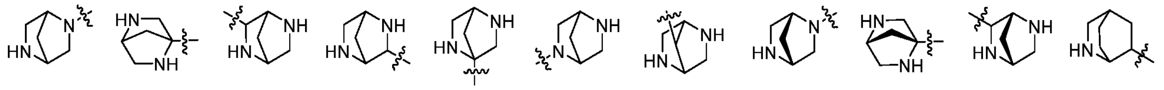

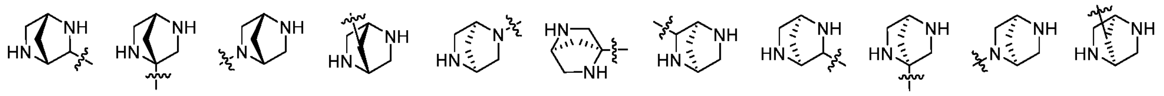

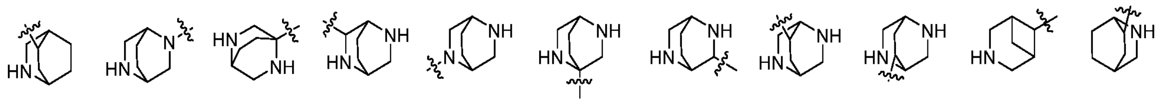

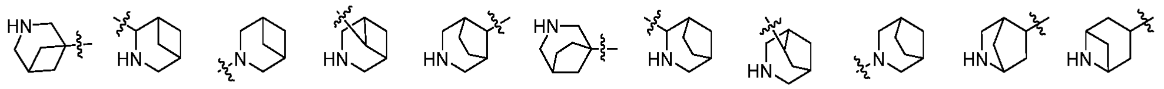

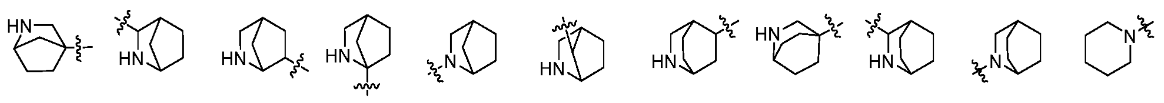

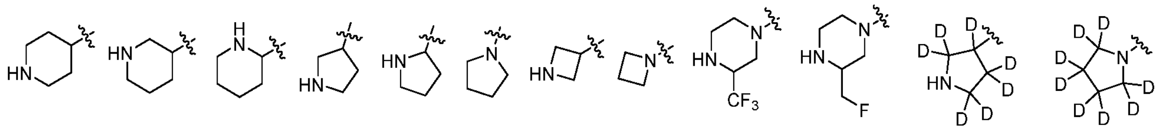

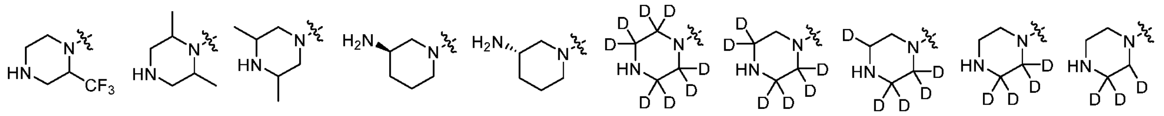

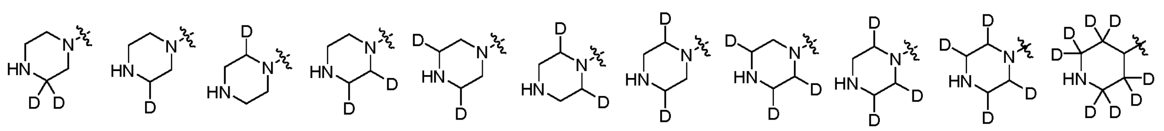

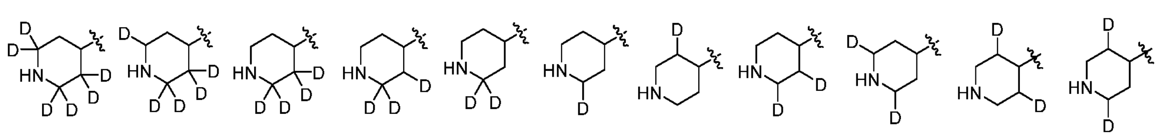

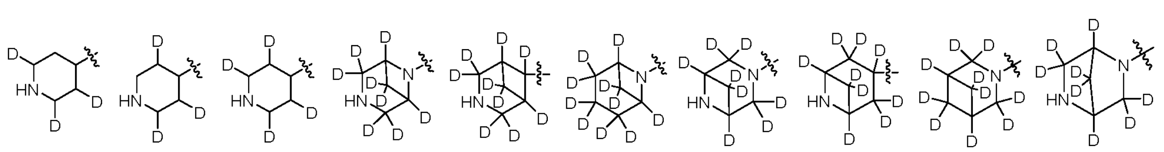

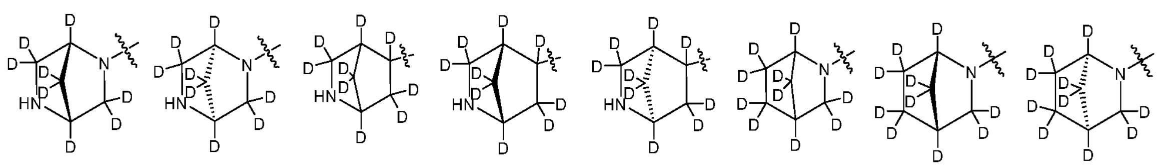

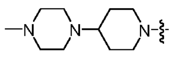 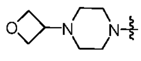 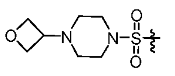 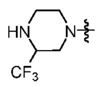 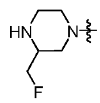 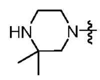 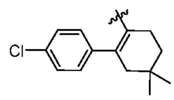 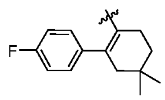

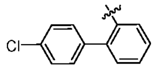 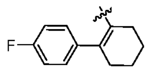 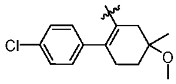 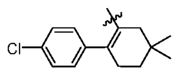 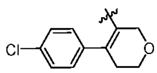 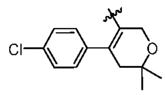 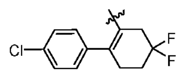

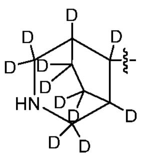 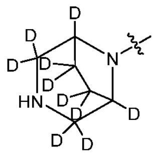 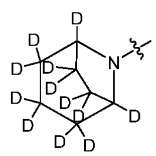 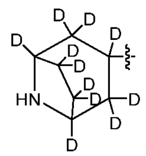 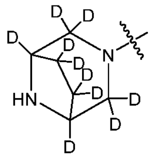 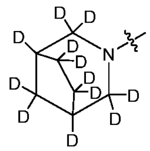 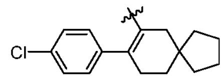

or

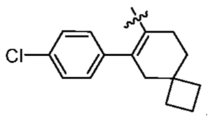

**35.** The compound of Formula (I) or salts, enantiomers, diastereoisomer, isotopically enriched analogs, solvates, prodrugs, or polymorphs thereof as claimed in claim 1, wherein $R_{b2}$ represents:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro[3.3]heptanyl, spiro[2.5]octyl, spiro[3.5]nonyl, spiro[4.4]nonyl, spiro[4.5]decyl, spiro[5.5]undecyl, p-menthanyl, m-menthanyl, quinuclidinyl, adamantanyl, noradamantanyl, bornyl, bicyclo[2.2.1]heptanyl, 2-oxobicyclo[2.2.1]heptanyl or bicyclo[2.2.1]heptenyl, with each group being optionally substituted with one or more substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl, diazacyclooctyl, 3-azabicyclo[3.1.0]hexyl, 6-azabicyclo[3.1.1]heptanyl, 2,5-diazabicyclo[2.2.1]heptanyl, 3,6-diazabicyclo[3.1.1]heptanyl, 3-azabicyclo [3.2.1]octyl, 3,8-diazabicyclo[3.2.1]octyl, 2,5-diazabicyclo[2.2.2]octyl, 3-azaspiro[5.5]undecyl or 7-azaspiro [3.5]nonyl, with each group being optionally substituted with one or more substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof;

phenyl or naphthyl, wherein the phenyl or naphthyl is optionally substituted with one or more substituents selected from the group consisting of D, optionally deuterated $C_{1-4}$ alkyl, optionally substituted $C_{3-15}$cycloalkyl, optionally substituted 4- to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are

each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl and imidazo[2,1-b]thiazolyl, with each group being optionally substituted with one or more substituents selected from the group consisting of D, optionally deuterated $C_{1-4}$ alkyl, optionally substituted $C_{3-15}$cycloalkyl, optionally substituted 4-to 15-membered heterocyclyl, optionally substituted $C_{6-15}$ aryl, optionally substituted 5- to 15-membered heteroaryl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof, and wherein the $C_{3-15}$cycloalkyl, the 4- to 15-membered heterocyclyl, the $C_{6-15}$ aryl, and the 5- to 15-membered heteroaryl are each independently optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene, $C_{1-4}$ alkyl-NHC(O)-, $C_{1-4}$ alkyl-C(O)NH-, or any combination thereof; or

$R_{b2}$ represents the following formula:

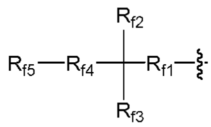

,

wherein $R_{f1}$, $R_{f2}$, $R_{f3}$, $R_{f4}$ and $R_{f5}$ are as defined in claim 13;
preferably, wherein $R_{b2}$ represents:

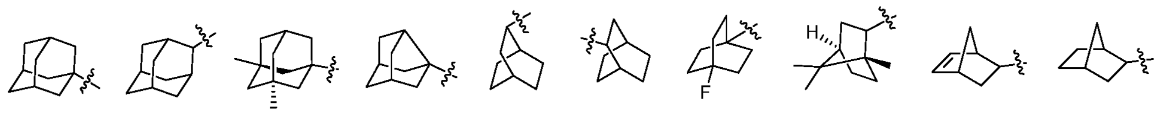

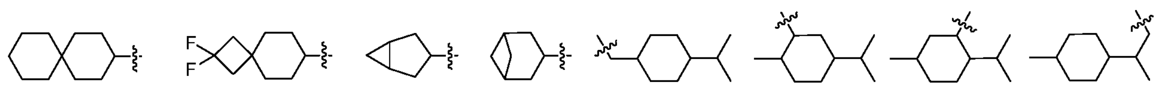

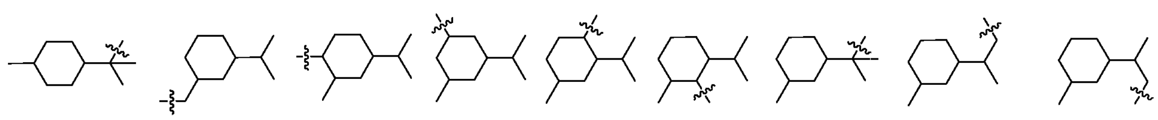

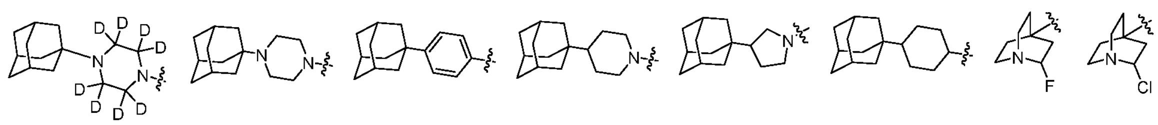

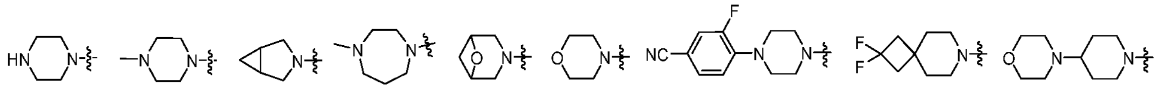

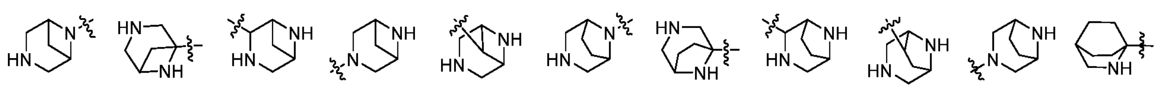

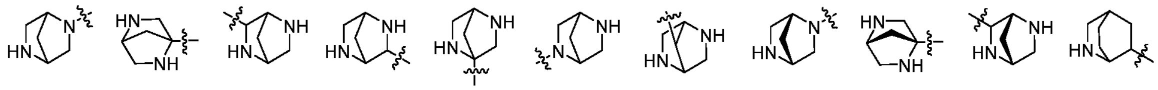

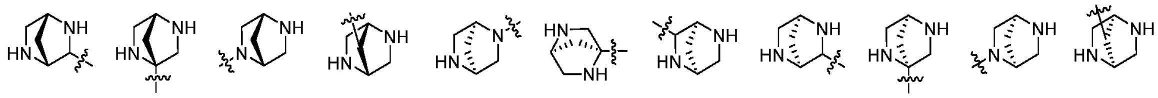

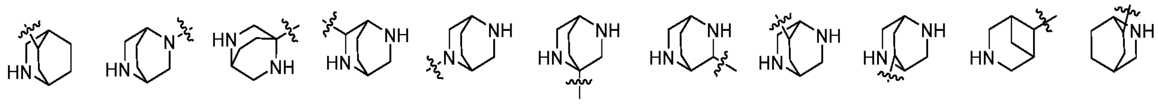

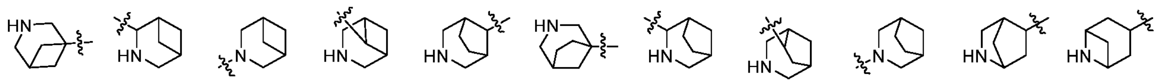

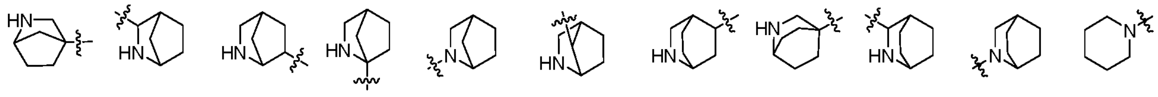

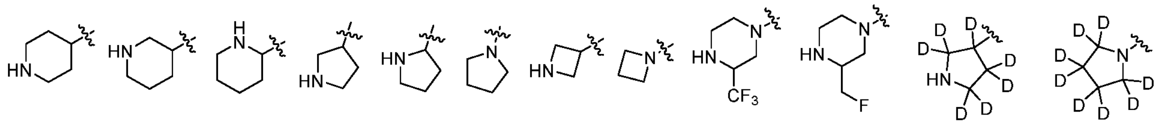

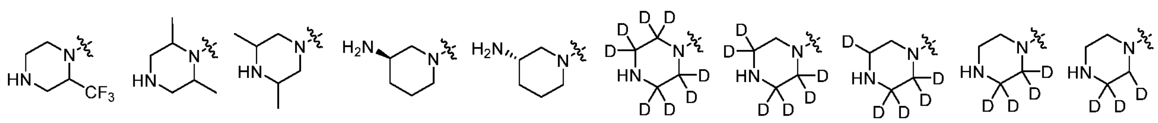

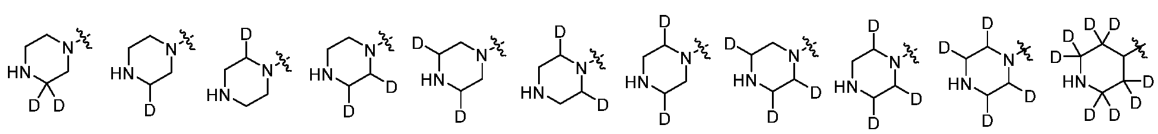

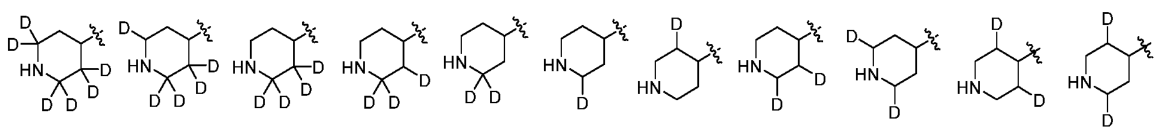

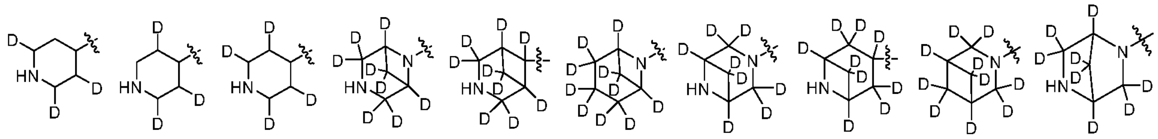

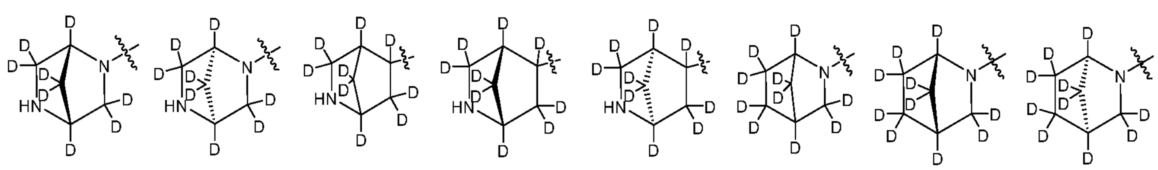

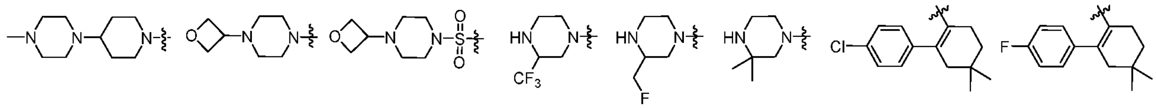

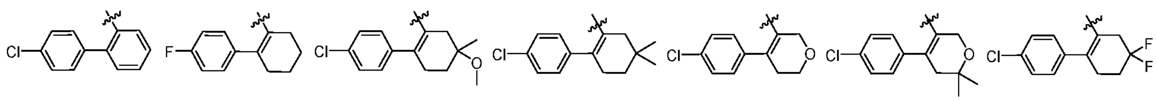

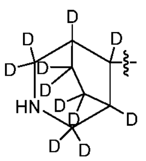 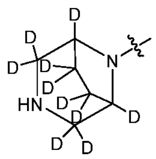 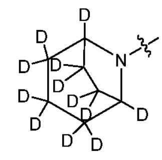 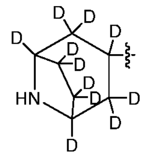 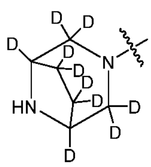 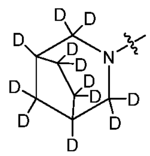 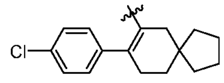

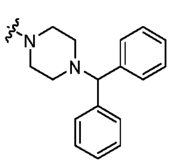 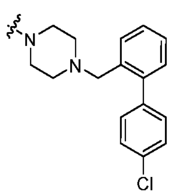 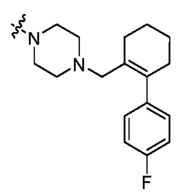 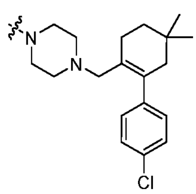 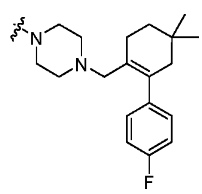 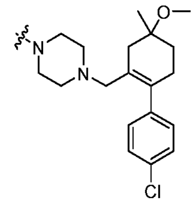

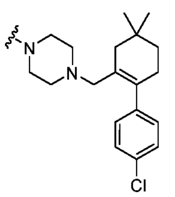 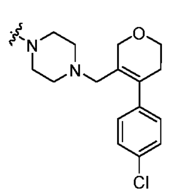 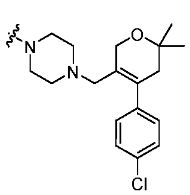 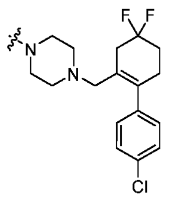 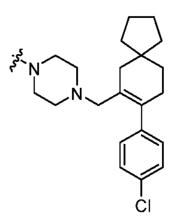 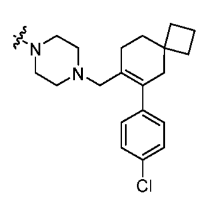

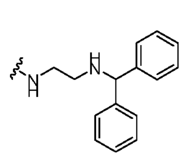 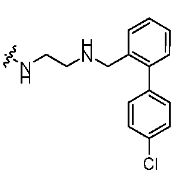 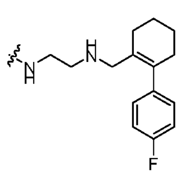 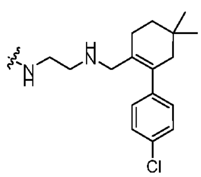 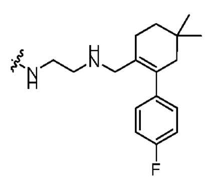 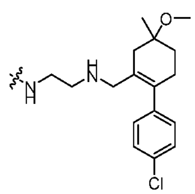

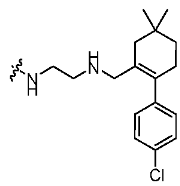 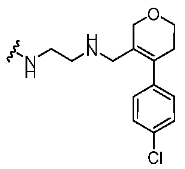 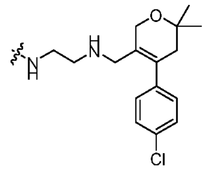 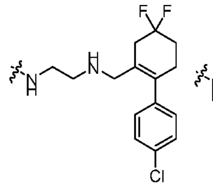 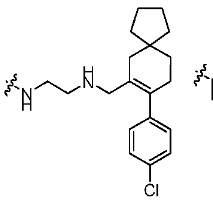 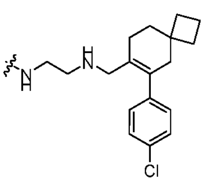

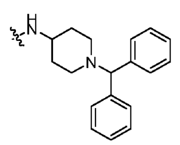 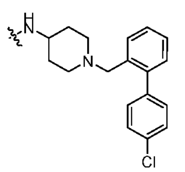 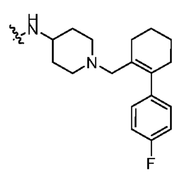 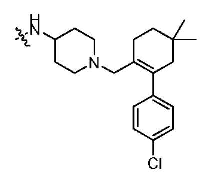 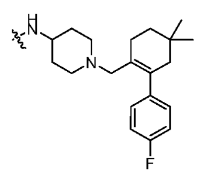 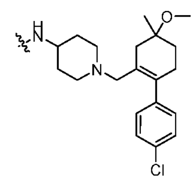

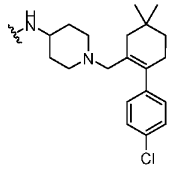 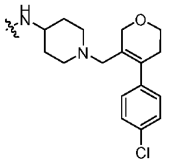 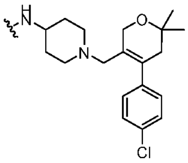 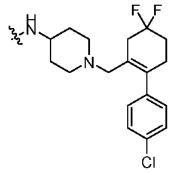 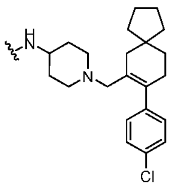 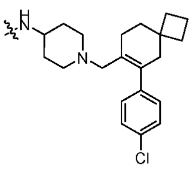

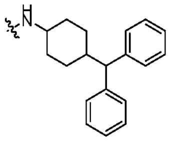 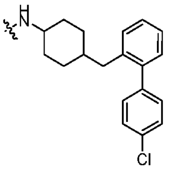 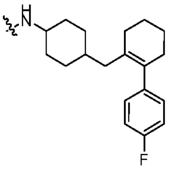 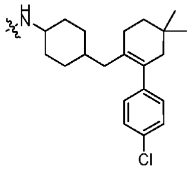 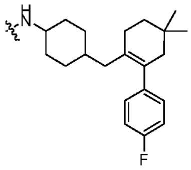 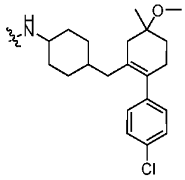

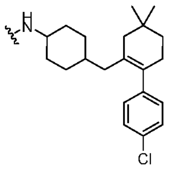 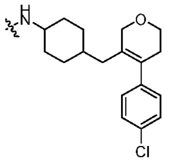 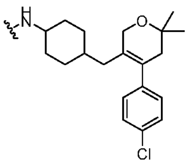 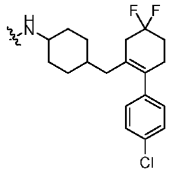 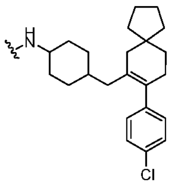 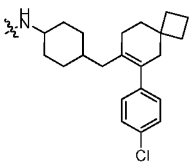

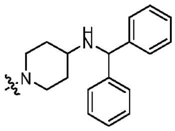 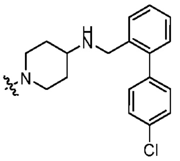 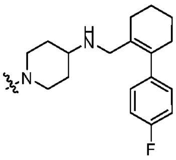 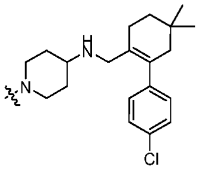 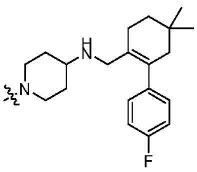 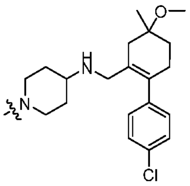

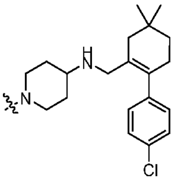 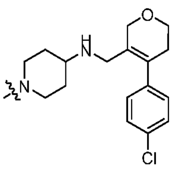 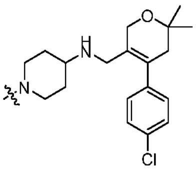 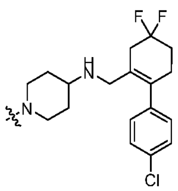 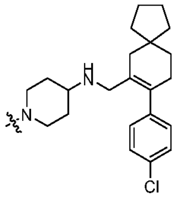 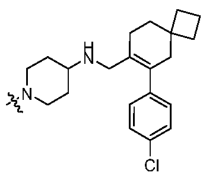

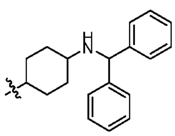 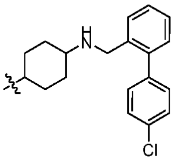 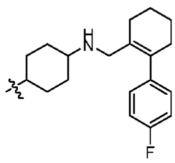 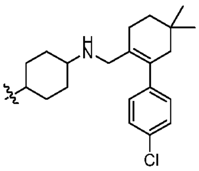 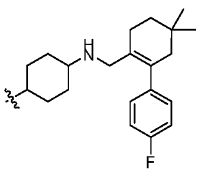 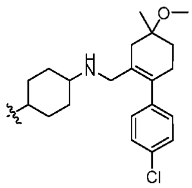

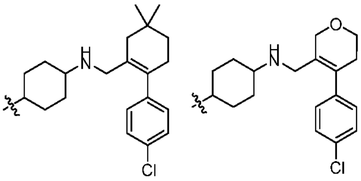 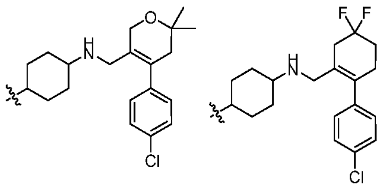 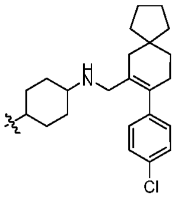 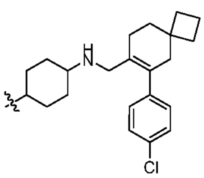

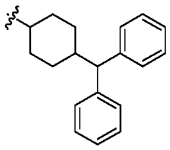 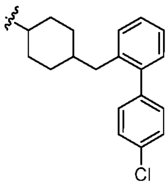 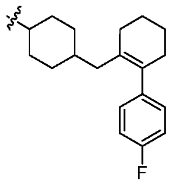 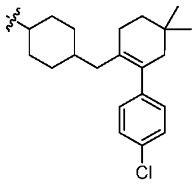 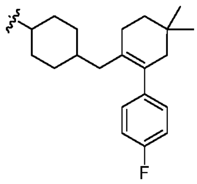 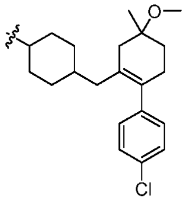

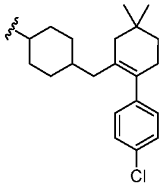 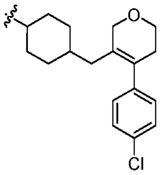 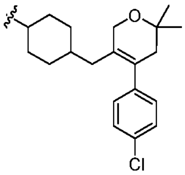 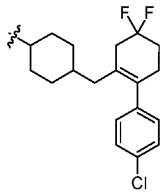 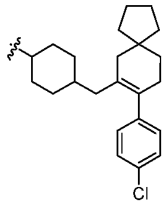 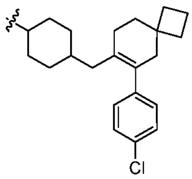

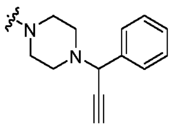 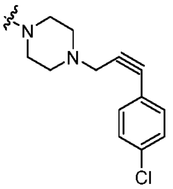 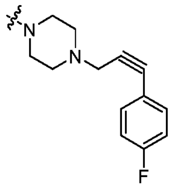 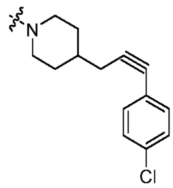 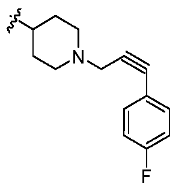 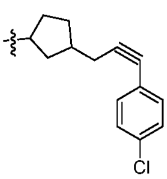

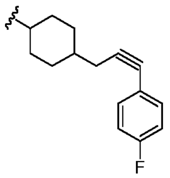 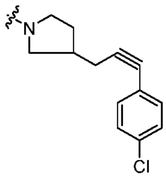 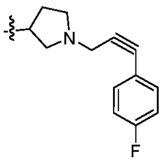 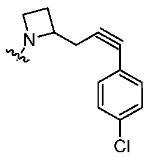 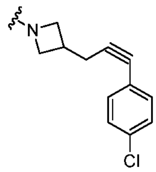 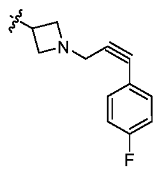

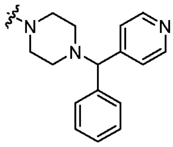 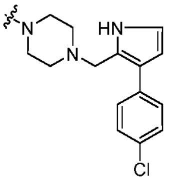 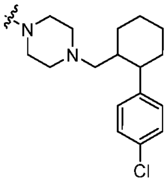 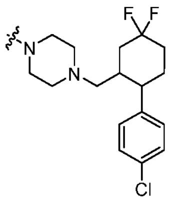 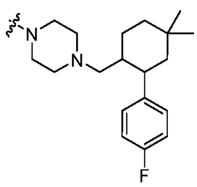 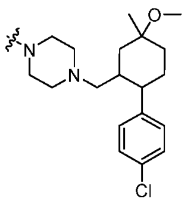

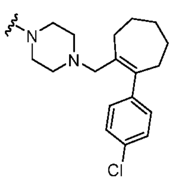 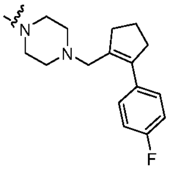 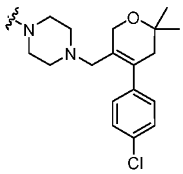 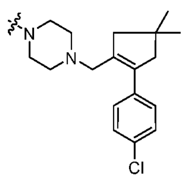 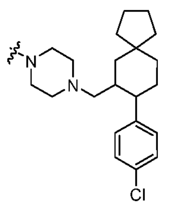 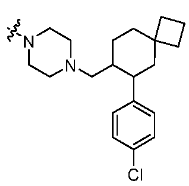

or

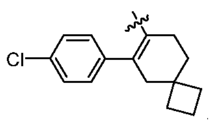

**36.** The compound of Formula (I) or salts, enantiomers, diastereoisomer, isotopically enriched analogs, solvates, prodrugs, or polymorphs thereof as claimed in claim 1, which is selected from the group consisting of:

3-(5-((4-(((3s,5s,7s)-adamantan-1-yl)amino)butyl)amino)-2-methyl-4-oxoquinazolin-3(4H)-yl)piperidine-2,6-dione;

3-(5-((5-(((1s,3s)-adamantan-1-yl)amino)pentyl)amino)-2-methyl-4-oxoquinazolin-3(4H)-yl)piperidine-2,6-dione;
3-(5-((6-(((3s,5s,7s)-adamantan-1-yl)amino)hexyl)amino)-2-methyl-4-oxoquinazolin-3(4H)-yl)piperidine-2,6-dione;
3-(5-((7-(((1s,3s)-adamantan-1-yl)amino)heptyl)amino)-2-methyl-4-oxoquinazolin-3(4H)-yl)piperidine-2,6-dione;
3-(5-((2-(2-(((1s,3s)-adamantan-1-yl)amino)ethoxy)ethyl)amino)-2-methyl-4-oxoquinazolin-3 (4H)-yl)piperidine-2,6-dione;
3-(5-((2-(2-(2-(((3s,5s,7s)-adamantan-1-yl)amino)ethoxy)ethoxy)ethyl)amino)-2-methyl-4-oxoquinazolin-3(4H)-yl)piperidine-2,6-dione;
3-(5-((2-(((1r,3r,5r,7r)-adamantan-2-yl)amino)ethyl)amino)-2-methyl-4-oxoquinazolin-3(4H)-yl)piperidine-2,6-dione;
3-(5-((3-(((1r,3r,5r,7r)-adamantan-2-yl)amino)propyl)amino)-2-methyl-4-oxoquinazolin-3(4H)-yl)piperidine-2,6-dione;
3-(5-((4-(((1r,3r,5r,7r)-adamantan-2-yl)amino)butyl)amino)-2-methyl-4-oxoquinazolin-3(4H)-yl)piperidine-2,6-dione;
3-(5-((5-(((1r,3r,5r,7r)-adamantan-2-yl)amino)pentyl)amino)-2-methyl-4-oxoquinazolin-3(4H)-yl)piperidine-2,6-dione;
3-(5-((5-(((3as,6as)-hexahydro-2,5-methanopentalen-3a(1H)-yl)amino)pentyl)amino)-2-methyl-4-oxoquinazolin-3(4H)-yl)piperidine-2,6-dione;
3-(5-((6-(((2R,3as,5S,6as)-hexahydro-2,5-methanopentalen-3a(1H)-yl)amino)hexyl)amino)-2-methyl-4-oxoquinazolin-3(4H)-yl)piperidine-2,6-dione;
3-(5-((7-(((3as,6as)-hexahydro-2,5-methanopentalen-3a(1H)-yl)amino)heptyl)amino)-2-methyl-4-oxoquinazolin-3(4H)-yl)piperidine-2,6-dione;
3-(5-((2-(2-(((3as,6as)-hexahydro-2,5-methanopentalen-3a(1H)-yl)amino)ethoxy)ethyl)amino)-2-methyl-4-oxoquinazolin-3(4H)-yl)piperidine-2,6-dione;
3-(5-((2-(2-(2-(((2R,3as,5S,6as)-hexahydro-2,5-methanopentalen-3a(1H)-yl)amino)ethoxy)ethoxy)ethyl)amino)-2-methyl-4-oxoquinazolin-3(4H)-yl)piperidine-2,6-dione;
3-(2-methyl-5-((5-(4-methyl-1,4-diazepan-1-yl)pentyl)amino)-4-oxoquinazolin-3(4H)-yl)piperidine-2,6-dione;
3-(2-methyl-5-((7-(4-methyl-1,4-diazepan-1-yl)heptyl)amino)-4-oxoquinazolin-3(4H)-yl)piperidine-2,6-dione;
3-(2-methyl-5-((2-(2-(4-methyl-1,4-diazepan-1-yl)ethoxy)ethyl)amino)-4-oxoquinazolin-3 (4H)-yl)piperidine-2,6-dione;
3-(5-((2-(2-hydroxyethoxy)ethyl)amino)-2-methyl-4-oxoquinazolin-3(4H)-yl)piperidine-2,6-dione;
3-(5-((2-(2-(6-oxa-3-azabicyclo[3.1.1]heptan-3-yl)ethoxy)ethyl)amino)-2-methyl-4-oxoquinazolin-3(4H)-yl)piperidine-2,6-dione;
3-(2-methyl-5-((2-(2-morpholinoethoxy)ethyl)amino)-4-oxoquinazolin-3(4H)-yl)piperidine-2,6-dione;
3-(2-methyl-5-((2-(2-(4-morpholinopiperidin-1-yl)ethoxy)ethyl)amino)-4-oxoquinazolin-3(4H)-yl)piperidine-2,6-dione;
3-(2-methyl-5-((2-(2-(4-(4-methylpiperazin-1-yl)piperidin-1-yl)ethoxy)ethyl)amino)-4-oxoquinazolin-3(4H)-yl) piperidine-2,6-dione;
3-(2-methyl-5-((2-(2-(4-(oxetan-3-yl)piperazin-1-yl)ethoxy)ethyl)amino)-4-oxoquinazolin-3 (4H)-yl)piperidine-2,6-dione;
3-(5-((7-hydroxyheptyl)amino)-2-methyl-4-oxoquinazolin-3(4H)-yl)piperidine-2,6-dione; 3-(5-((7-(6-oxa-3-azabicyclo[3.1.1]heptan-3-yl)heptyl)amino)-2-methyl-4-oxoquinazolin-3(4H)-yl)piperidine-2,6-dione;
3-(2-methyl-5-((7-(4-morpholinopiperidin-1-yl)heptyl)amino)-4-oxoquinazolin-3(4H)-yl)piperidine-2,6-dione;
3-(2-methyl-5-((7-(4-(oxetan-3-yl)piperazin-1-yl)heptyl)amino)-4-oxoquinazolin-3 (4H)-yl)piperidine-2,6-dione;
3-(5-((7-(((1 SR,3RS, 5 SR,7r)-3, 5-dimethyladamantan-1-yl)amino)heptyl)amino)-2-methyl-4-oxoquinazolin-3(4H)-yl)piperidine-2,6-dione;
3-(5-((7-((1-((3r,5r,7r)-adamantan-1-yl)ethyl)amino)heptyl)amino)-2-methyl-4-oxoquinazolin-3 (4H)-yl)piperidine-2,6-dione;
3 -(5 -((7-(3 -azabicyclo [3.1.0]hexan-3 -yl)heptyl)amino)-2-methyl-4-oxoquinazolin-3 (4H)-yl)piperidine-2,6-dione;
3-(5-((7-(2,2-difluoro-7-azaspiro[3.5]nonan-7-yl)heptyl)amino)-2-methyl-4-oxoquinazolin-3 (4H)-yl)piperidine-2,6-dione;
3-(5-((7-((4-fluorobicyclo[2.2.2]octan-1-yl)amino)heptyl)amino)-2-methyl-4-oxoquinazolin-3 (4H)-yl)piperidine-2,6-dione;
3-(2-methyl-4-oxo-5-((7-(((1R,2S,4R)-1,7,7-trimethylbicyclo[2.2.1]heptan-2-yl)amino)heptyl)amino)quinazolin-3 (4H)-yl)piperidine-2,6-dione;

3-(5-((7-((2-isopropyl-5-methylcyclohexyl)oxy)heptyl)amino)-2-methyl-4-oxoquinazolin-3(4H)-yl)piperidine-2,6-dione;
(3S)-3-(5-((7-((1-((3r,5r,7r)-adamantan-1-yl)ethyl)amino)heptyl)amino)-2-methyl-4-oxoquinazolin-3(4H)-yl)piperidine-2,6-dione;
(S)-3-(5-((7-(((1SR,3RS,SSR,7r)-3,5-dimethyladamantan-1-yl)amino)heptyl)amino)-2-methyl-4-oxoquinazolin-3(4H)-yl)piperidine-2,6-dione;
(3R)-3-(5-((7-(((3as,6as)-hexahydro-2,5-methanopentalen-3a(1H)-yl)amino)heptyl)amino)-2-methyl-4-oxoquinazolin-3(4H)-yl)piperidine-2,6-dione;
(3R)-3 -(5-((7-(((1s,3 s)-adamantan-1-yl)amino)heptyl)amino)-2-methyl-4-oxoquinazolin-3(4H)-yl)piperidine-2,6-dione;
(S)-3-(2-methyl-4-oxo-5-((7-(((1R,2S,4R)-1,7,7-trimethylbicyclo[2.2.1]heptan-2-yl)amino)heptyl)amino)quinazolin-3 (4H)-yl)piperidine-2,6-dione;
3-(5-((7-((1-((3r,5r,7r)-adamantan-1-yl)ethyl)amino)heptyl)amino)-4-oxo-2-(trifluoromethyl)quinazolin-3(4H)-yl)piperidine-2,6-dione;
3-(5-((7-(((1SR,3RS,5SR,7r)-3,5-dimethyladamantan-1-yl)amino)heptyl)amino)-4-oxo-2-(trifluoromethyl)quinazolin-3(4H)-yl)piperidine-2,6-dione;
3-(5-((7-(4-methyl-1,4-diazepan-1-yl)heptyl)amino)-4-oxo-2-(trifluoromethyl)quinazolin-3(4H)-yl)piperidine-2,6-dione;
3-(5-((7-(((3as,6as)-hexahydro-2,5-methanopentalen-3a(1H)-yl)amino)heptyl)amino)-4-oxo-2-(trifluoromethyl) quinazolin-3 (4H)-yl)piperidine-2,6-dione;
3-(5-((2-(2-(2-(((3s,5s,7s)-adamantan-1-yl)amino)ethoxy)ethoxy)ethyl)amino)-4-oxo-2-(trifluoromethyl)quinazolin-3(4H)-yl)piperidine-2,6-dione;
3-(5-((7-(((1s,3s)-adamantan-1-yl)amino)heptyl)amino)-4-oxo-2-(trifluoromethyl)quinazolin-3 (4H)-yl)piperidine-2,6-dione;
3-(5-((7-((4-fluorobicyclo[2.2.2]octan-1-yl)amino)heptyl)amino)-4-oxo-2-(trifluoromethyl)quinazolin-3 (4H)-yl) piperidine-2,6-dione;
3-(5-((7-(2,2-difluoro-7-azaspiro[3.5]nonan-7-yl)heptyl)amino)-4-oxo-2-(trifluoromethyl)quinazolin-3 (4H)-yl)piperidine-2,6-dione;
3-(5-((7-((2-isopropyl-5-methylcyclohexyl)oxy)heptyl)amino)-4-oxo-2-(trifluoromethyl)quinazolin-3 (4H)-yl)piperidine-2,6-dione;
3-(4-oxo-2-(trifluoromethyl)-5-((7-(((1R,2S,4R)-1,7,7-trimethylbicyclo[2.2.1]heptan-2-yl)amino)heptyl)amino) quinazolin-3 (4H)-yl)piperidine-2,6-dione;
(S)-3-(5-((7-(((1 SR,3RS,SSR,7r)-3,5-dimethyladamantan-1-yl)amino)heptyl)amino)-4-oxo-2-(trifluoromethyl) quinazolin-3 (4H)-yl)piperidine-2,6-dione;
(3 S)-3-(5-((7-((1-((3r,5r,7r)-adamantan-1-yl)ethyl)amino)heptyl)amino)-4-oxo-2-(trifluoromethyl)quinazolin-3 (4H)-yl)piperidine-2,6-dione;
(3R)-3-(5-((7-(((3as,6as)-hexahydro-2,5-methanopentalen-3a(1H)-yl)amino)heptyl)amino)-4-oxo-2-(trifluoromethyl)quinazolin-3(4H)-yl)piperidine-2,6-dione;
(3R)-3-(5-((7-(((1s,3 s)-adamantan-1-yl)amino)heptyl)amino)-4-oxo-2-(trifluoromethyl)quinazolin-3 (4H)-yl)piperidine-2,6-dione;
(S)-3-(5-((7-((4-fluorobicyclo[2.2.2]octan-1-yl)amino)heptyl)amino)-4-oxo-2-(trifluoromethyl)quinazolin-3(4H)-yl)piperidine-2,6-dione;
(3S)-3-(5-((7-((2-isopropyl-5-methylcyclohexyl)oxy)heptyl)amino)-4-oxo-2-(trifluoromethyl)quinazolin-3 (4H)-yl)piperidine-2,6-dione;
(S)-3-(4-oxo-2-(trifluoromethyl)-5-((7-(((1R,2S,4R)-1,7,7-trimethylbicyclo[2.2.1]heptan-2-yl)amino)heptyl)amino)quinazolin-3 (4H)-yl)piperidine-2,6-dione;
3-(5-((7-(((1s,3s)-adamantan-1-yl)amino)heptyl)amino)-2-butyl-4-oxoquinazolin-3(4H)-yl) piperidine-2,6-dione;
3-(5-((7-(((1s,3s)-adamantan-1-yl)amino)heptyl)amino)-2-(methyl-d3)-4-oxoquinazolin-3(4H)-yl)piperidine-2,6-dione;
3-(2-(7-(((3s,5s,7s)-adamantan-1-yl)amino)heptyl)-5-bromo-4-oxoquinazolin-3(4H)-yl)piperidine-2,6-dione;
3-(2-(7-(((3 s, 5 s,7s)-adamantan-1-yl)amino)heptyl)-5-fluoro-4-oxoquinazolin-3 (4H)-yl)piperidine-2,6-dione;
2-(7-(((3s,5s,7s)-adamantan-1-yl)amino)heptyl)-3-(2,6-dioxopiperidin-3-yl)-4-oxo-3,4-dihydroquinazoline-5-carbonitrile;
3-(2-(3-(3-(((3s,5s,7s)-adamantan-l-yl)(methyl)amino)propoxy)propyl)-5-bromo-4-oxoquinazolin-3(4H)-yl)piperidine-2,6-dione;
3-(5-(((1s,3s)-adamantan-1-yl)amino)-2-methyl-4-oxoquinazolin-3(4H)-yl)piperidine-2,6-dione;
3-(5-(4-(3-(((3s,5s,7s)-adamantan-1-yl)amino)propyl)piperazin-1-yl)-2-methyl-4-oxoquinazolin-3 (4H)-yl)piperidine-2,6-dione;
3-(6-(4-(3-(((3s,5s,7s)-adamantan-1-yl)amino)propyl)piperazin-1-yl)-2-methyl-4-oxoquinazolin-3 (4H)-yl)piperidine-2,6-dione;
4-(4-(3-(2,4-dioxocyclohexyl)-2-methyl-4-oxo-3,4-dihydroquinazolin-5-yl)piperazin-1-yl)-3-fluorobenzonitrile;
3-(7-(4-(3-(((3s,5s,7s)-adamantan-1-yl)aminoo)propyl)piperazin-1-yl)-2-methyl-4-oxoquinazolin-3 (4H)-yl)piperidine-2,6-dione;
3-(8-(4-(3-(((3s,5s,7s)-adamantan-1-yl)amino)propyl)piperazin-1-yl)-2-methyl-4-oxoquinazolin-3 (4H)-yl)piperidine-2,6-dione;
3-(5-(((2-((((3 s, 5 s,7s)-adamantan-1-yl)amino)methyl)cyclopropyl);
methyl)amino)-2-methyl-4-oxoquinazolin-3(4H)-yl)piperidine-2,6-dione;
3-(2-(8-(((3 s, 5 s,7s)-adamantan-1-yl)amino)octyl)-5-fluoro-4-oxoquinazolin-3 (4H)-yl)piperidine-2,6-dione;
3 -(5-((7-(((1s,3 s)-adamantan-1-yl)amino)heptyl)amino)-2-(8-(((3 s, 5 s,7s)-adamantan-1-yl)amino)octyl)-4-oxoquinazolin-3 (4H)-yl)piperidine-2,6-dione;
3-(5-((2-((2-(((1s,3s)-adamantan-1-yl)amino)ethyl)thio)ethyl)amino)-2-methyl-4-oxoquinazolin-3 (4H)-yl)piperidine-2,6-dione;
3 -(5-((5-(((1s,3 s)-adamantan-1-yl)amino)-3-oxopentyl)amino)-2-methyl-4-oxoquinazolin-3 (4H)-yl)piperidine-2,6-dione;
3-(5-((5-(((1s,3s)-adamantan-1-yl)amino)-3,3-difluoropentyl)amino)-2-methyl-4-oxoquinazolin-3 (4H)-yl)piperidine-2,6-dione;
3-(5-((5-(((1s,3s)-adamantan-1-yl)amino)-3-thioxopentyl)amino)-2-methyl-4-oxoquinazolin-3 (4H)-yl)piperidine-2,6-dione;
2-(((3s,5s,7s)-adamantan-1-yl)amino)ethyl 3-((3-(2,6-dioxopiperidin-3-yl)-2-methyl-4-oxo-3,4-dihydroquinazolin-5-yl)amino)propanoate;
N-(4-(((3s,5s,7s)-adamantan-1-yl)(methyl)amino)butyl)-3-((3-(2,6-dioxopiperidin-3-yl)-2-methyl-4-oxo-3,4-dihydroquinazolin-5-yl)amino)propanamide;
3-(5-((2-((2-(((1s,3 s)-adamantan-1-yl)amino)ethyl)amino)ethyl)amino)-2-methyl-4-oxoquinazolin-3(4H)-yl)piperidine-2,6-dione;
3-(5-((2-((2-(((1s,3 s)-adamantan-1-yl)amino)ethyl)(methyl)amino)ethyl)amino)-2-methyl-4-oxoquinazolin-3(4H)-yl)piperidine-2,6-dione;
3-(5-((7-hydroxyheptyl)oxy)-2-methyl-4-oxoquinazolin-3(4H)-yl)piperidine-2,6-dione; 3-(5-((7-(((1s,3s)-adamantan-1-yl)amino)heptyl)oxy)-2-methyl-4-oxoquinazolin-3(4H)-yl)piperidine-2,6-dione;
3-(2-methyl-5-((7-(4-(oxetan-3-yl)piperazin-1-yl)heptyl)oxy)-4-oxoquinazolin-3(4H)-yl)piperidine-2,6-dione;
3-(2-methyl-5-((7-(4-methylpiperazin-1-yl)heptyl)oxy)-4-oxoquinazolin-3(4H)-yl)piperidine-2,6-dione;
3-(5-((7-((1-((3r,5r,7r)-adamantan-l-yl)ethyl)amino)heptyl)oxy)-4-oxo-2-(trifluoromethyl)quinazolin-3 (4H)-yl)piperidine-2,6-dione;
3-(5-((7-(((3as,6as)-hexahydro-2,5-methanopentalen-3a(1H)-yl)amino)heptyl)oxy)-4-oxo-2-(trifluoromethyl) quinazolin-3(4H)-yl)piperidine-2,6-dione;
3-(5-((7-(((1s,3s)-adamantan-1-yl)amino)heptyl)oxy)-4-oxo-2-(trifluoromethyl)quinazolin-3 (4H)-yl)piperidine-2,6-dione;
3-(4-oxo-2-(trifluoromethyl)-5-((7-(((1R,2S,4R)-1,7,7-trimethylbicyclo[2.2.1]heptan-2-yl)amino)heptyl)oxy)quinazolin-3(4H)-yl)piperidine-2,6-dione;
(S)-3-(2-methyl-4-oxo-5-((7-(((1R,2S,4R)-1,7,7-trimethylbicyclo[2.2.1]heptan-2-yl)amino)heptyl)oxy)quinazolin-3(4H)-yl)piperidine-2,6-dione;
(S)-3-(5-((7-((4-fluorobicyclo[2.2.2]octan-1-yl)amino)heptyl)oxy)-2-methyl-4-oxoquinazolin-3 (4H)-yl)piperidine-2,6-dione;
(3R)-3-(5-((7-(((1s,3s)-adamantan-1-yl)amino)heptyl)oxy)-2-methyl-4-oxoquinazolin-3(4H)-yl)piperidine-2,6-dione;
(R)-3-(5-(2-(2-(2-(((3s,5s,7s)-adamantan-1-yl)amino)ethoxy)ethoxy)ethoxy)-2-methyl-4-oxoquinazolin-3(4H)-yl)piperidine-2,6-dione;
(S)-3-(5-((7-(((1 SR,3RS,SSR,7r)-3,5-dimethyladamantan-1-yl)amino)heptyl)oxy)-2-methyl-4-oxoquinazolin-3(4H)-yl)piperidine-2,6-dione;
(S)-3-(5-((7-(((1 SR,3RS,SSR,7r)-3,5-dimethyladamantan-1-yl)amino)heptyl)oxy)-4-oxo-2-(trifluoromethyl)quinazolin-3 (4H)-yl)piperidine-2,6-dione;
(3 S)-3-(5-((7-((1-((3r,5r,7r)-adamantan-1-yl)ethyl)amino)heptyl)oxy)-4-oxo-2-(trifluoromethyl)quinazolin-3 (4H)-yl)piperidine-2,6-dione;
(3R)-3-(5-((7-(((3as,6as)-hexahydro-2,5-methanopentalen-3a(1H)-yl)amino)heptyl)oxy)-4-oxo-2-(trifluoromethyl)quinazolin-3(4H)-yl)piperidine-2,6-dione;
(3R)-3-(5-((7-(((1s,3s)-adamantan-1-yl)amino)heptyl)oxy)-4-oxo-2-(trifluoromethyl)quinazolin-3 (4H)-yl)piperidine-2,6-dione;
(S)-3-(4-oxo-2-(trifluoromethyl)-5-((7-(((1R,2S,4R)-1,7,7-trimethylbicyclo[2.2.1]heptan-2-yl)amino)heptyl)oxy) quinazolin-3(4H)-yl)piperidine-2,6-dione;
3-(5-((7-(((1s,3s)-adamantan-1-yl)amino)heptyl)thio)-2-methyl-4-oxoquinazolin-3(4H)-yl)piperidine-2,6-dione;
3-(5-((7-(((1 SR,3RS, 5 SR,7r)-3, 5-dimethyladamantan-1-yl)amino)heptyl)thio)-4-oxo-2-(trifluoromethyl)quinazolin-3 (4H)-yl)piperidine-2,6-dione;
3-(5-((7-(((1s,3s)-adamantan-1-yl)amino)heptyl)thio)-4-oxo-2-(trifluoromethyl)quinazolin-3 (4H)-yl)piperidine-2,6-dione;
3-(4-oxo-2-(trifluoromethyl)-5-((7-(((1R,2S,4R)-1,7,7-trimethylbicyclo[2.2.1]heptan-2-yl)amino)heptyl)thio)quinazolin-3 (4H)-yl)piperidine-2,6-dione;
(S)-3-(5-((7-(((1 SR,3RS,SSR,7r)-3,5-dimethyladamantan-1-yl)amino)heptyl)thio)-2-methyl-4-oxoquinazolin-3(4H)-yl)piperidine-2,6-dione;
(3 S)-3-(5-((7-((1-((3r,5r,7r)-adamantan-1-yl)ethyl)amino)heptyl)thio)-2-methyl-4-oxoquinazolin-3 (4H)-yl)piperidine-2,6-dione;
(3R)-3-(5-((7-(((1s,3s)-adamantan-1-yl)amino)heptyl)thio)-2-methyl-4-oxoquinazolin-3(4H)-yl)piperidine-2,6-dione;
(S)-3-(2-methyl-4-oxo-5-((7-(((1R,2S,4R)-1,7,7-trimethylbicyclo[2.2.1]heptan-2-yl)amino)heptyl)thio)quinazolin-3 (4H)-yl)piperidine-2,6-dione;
(S)-3-(4-oxo-2-(trifluoromethyl)-5-((7-(((1R,2S,4R)-1,7,7-trimethylbicyclo[2.2.1]heptan-2-yl)amino)heptyl)thio) quinazolin-3 (4H)-yl)piperidine-2,6-dione;
(S)-3-(5-((7-((4-fluorobicyclo[2.2.2]octan-1-yl)amino)heptyl)thio)-4-oxo-2-(trifluoromethyl)quinazolin-3 (4H)-yl) piperidine-2,6-dione;
(3R)-3-(5-((7-(((1s,3s)-adamantan-1-yl)amino)heptyl)thio)-4-oxo-2-(trifluoromethyl)quinazolin-3 (4H)-yl)piperidine-2,6-dione;
(S)-3-(5-((7-(((1 SR,3RS,SSR,7r)-3,5-dimethyladamantan-1-yl)amino)heptyl)thio)-4-oxo-2-(trifluoromethyl) quinazolin-3 (4H)-yl)piperidine-2,6-dione;
3 -(5 -(3 -(((3 s, 5s,7s)-adamantan-1-yl)amino)prop-1-yn-1-yl)-2-methyl-4-oxoquinazolin-3 (4H)-yl)piperidine-2,6-dione;
3-(5-(5-hydroxypent-1-yn-1-yl)-2-methyl-4-oxoquinazolin-3(4H)-yl)piperidine-2,6-dione;
3 -(5 -(5 -(((3 s, 5 s,7s)-adamantan-1-yl)amino)pent-1-yn-1-yl)-2-methyl-4-oxoquinazolin-3 (4H)-yl)piperidine-2,6-dione;
3-(2-methyl-4-oxo-5-(5-(piperidin-1-yl)pent-1-yn-1-yl)quinazolin-3(4H)-yl)piperidine-2,6-dione;
3-(2-methyl-5-(5-(4-methylpiperazin-1-yl)pent-1-yn-1-yl)-4-oxoquinazolin-3(4H)-yl)piperidine-2,6-dione;
3 -(2-methyl-5 -(5 -(4-(oxetan-3-yl)piperazin-1-yl)pent-1-yn-1-yl)-4-oxoquinazolin-3 (4H)-yl)piperidine-2,6-dione;
3 -(2-methyl-5 -(5 -(4-methyl-1,4-diazepan-1-yl)pent-1-yn-1-yl)-4-oxoquinazolin-3 (4H)-yl)piperidine-2,6-dione;
3 -(5 -(5 -(6-oxa-3 -azabicyclo [3 .1.1]heptan-3 -yl)pent-1 -yn-1-yl)-2-methyl-4-oxoquinazolin-3 (4H)-yl)piperidine-2,6-dione;
3-(2-methyl-5-(5-(4-(4-methylpiperazin-1-yl)piperidin-1-yl)pent-1-yn-1-yl)-4-oxoquinazolin-3 (4H)-yl)piperidine-2,6-dione;
3 -(2-methyl-5 -(5 -(4-morpholinopiperidin-1 -yl)pent-1 -yn-1 -yl)-4-oxoquinazolin-3 (4H)-yl)piperidine-2,6-dione;
3-(5-(5-((1-((1s,3s)-adamantan-1-yl)ethyl)amino)pent-1-yn-1-yl)-2-methyl-4-oxoquinazolin-3 (4H)-yl)piperidine-2,6-dione;
3-(5-(8-hydroxyoct-1-yn-1-yl)-2-methyl-4-oxoquinazolin-3(4H)-yl)piperidine-2,6-dione;
3-(5-(8-(((1s,3 s)-adamantan-1-yl)amino)oct-1-yn-1-yl)-2-methyl-4-oxoquinazolin-3 (4H)-yl)piperidine-2,6-dione;
3 -(2-methyl-5 -(8-(4-(oxetan-3 -yl)piperazin-1 -yl)oct-1 -yn-1 -yl)-4-oxoquinazolin-3 (4H)-yl)piperidine-2,6-dione;
3-(2-methyl-5-(8-(4-methylpiperazin-1-yl)oct-1-yn-1-yl)-4-oxoquinazolin-3(4H)-yl)piperidine-2,6-dione;
3 -(2-methyl-5 -(8-(4-(4-methylpiperazin-1 -yl)piperidin-1 -yl)oct-1 -yn-1 -yl)-4-oxoquinazolin-3 (4H)-yl)piperidine-2,6-dione;
3 -(5 -(8-(6-oxa-3 -azabicyclo [3.1.1]heptan-3 -yl)oct-1-yn-1-yl)-2-methyl-4-oxoquinazolin-3 (4H)-yl)piperidine-2,6-dione;
3-(5-(5-hydroxypentyl)-2-methyl-4-oxoquinazolin-3(4H)-yl)piperidine-2,6-dione;
3-(5-(5-(((3s,5s,7s)-adamantan-1-yl)amino)pentyl)-2-methyl-4-oxoquinazolin-3(4H)-yl)piperidine-2,6-dione;
3-(2-methyl-5-(5-(4-(oxetan-3-yl)piperazin-1-yl)pentyl)-4-oxoquinazolin-3(4H)-yl)piperidine-2,6-dione;
3-(5-(5-(6-oxa-3-azabicyclo[3.1.1]heptan-3-yl)pentyl)-2-methyl-4-oxoquinazolin-3(4H)-yl)piperidine-2,6-dione;
3-(2-methyl-5-(5-(4-methylpiperazin-1-yl)pentyl)-4-oxoquinazolin-3(4H)-yl)piperidine-2,6-dione;

3-(2-methyl-5-(5-(4-(4-methylpiperazin-1-yl)piperidin-1-yl)pentyl)-4-oxoquinazolin-3(4H)-yl)piperidine-2,6-dione;
3-(5-(8-hydroxyoctyl)-2-methyl-4-oxoquinazolin-3(4H)-yl)piperidine-2,6-dione;
3-(5-(8-(((1s,3s)-adamantan-1-yl)amino)octyl)-2-methyl-4-oxoquinazolin-3(4H)-yl)piperidine-2,6-dione;
3-(2-methyl-5-(8-(4-(oxetan-3-yl)piperazin-1-yl)octyl)-4-oxoquinazolin-3(4H)-yl)piperidine-2,6-dione;
3-(2-methyl-5-(8-(4-(4-methylpiperazin-1-yl)piperidin-1-yl)octyl)-4-oxoquinazolin-3(4H)-yl)piperidine-2,6-dione;
3-(5-(8-(6-oxa-3-azabicyclo[3.1.1]heptan-3-yl)octyl)-2-methyl-4-oxoquinazolin-3(4H)-yl)piperidine-2,6-dione;
3-(2-methyl-5-(8-(4-methylpiperazin-1-yl)octyl)-4-oxoquinazolin-3(4H)-yl)piperidine-2,6-dione;
3-(4-oxo-2-(trifluoromethyl)-5-(8-(((1R,2S,4R)-1,7,7-trimethylbicyclo[2.2.1]heptan-2-yl)amino)octyl)quinazolin-3(4H)-yl)piperidine-2,6-dione;
3-(4-oxo-2-(trifluoromethyl)-5-(8-(((1R,2S,4R)-1,7,7-trimethylbicyclo[2.2.1]heptan-2-yl)amino)oct-1-yn-1-yl) quinazolin-3 (4H)-yl)piperidine-2,6-dione;
3-(5-(8-(((1s,3s)-adamantan-l-yl)amino)octyl)-4-oxo-2-(trifluoromethyl)quinazolin-3(4H)-yl)piperidine-2,6-dione;
3 -(5-(8-(((1s,3 s)-adamantan-1 -yl)amino)oct-1 -yn-1 -yl)-4-oxo-2-(trifluoromethyl)quinazolin-3 (4H)-yl)piperidine-2,6-dione;
3-(5-(8-((1-((3r,5r,7r)-adamantan-1-yl)ethyl)amino)octyl)-4-oxo-2-(trifluoromethyl)quinazolin-3 (4H)-yl)piperidine-2,6-dione;
3-(5-(8-((1-((3r,5r,7r)-adamantan-1-yl)ethyl)amino)oct-1-yn-1-yl)-4-oxo-2-(trifluoromethyl)quinazolin-3 (4H)-yl) piperidine-2,6-dione;
(S)-3-(2-methyl-4-oxo-5-(8-(((1R,2S,4R)-1,7,7-trimethylbicyclo[2.2.1]heptan-2-yl)amino)octyl)quinazolin-3(4H)-yl)piperidine-2,6-dione;
(S)-3-(2-methyl-4-oxo-5-(8-(((1R,2S,4R)-1,7,7-trimethylbicyclo[2.2.1]heptan-2-yl)amino)oct-1-yn-1-yl)quinazolin-3 (4H)-yl)piperidine-2,6-dione;
(3R)-3-(5-(8-(((1s,3s)-adamantan-1-yl)amino)octyl)-2-methyl-4-oxoquinazolin-3(4H)-yl)piperidine-2,6-dione;
(3R)-3-(5-(8-(((1s,3s)-adamantan-1-yl)amino)oct-1-yn-1-yl)-2-methyl-4-oxoquinazolin-3(4H)-yl)piperidine-2,6-dione;
(3R)-3-(5-(8-(((3as,6as)-hexahydro-2,5-methanopentalen-3a(1H)-yl)amino)octyl)-2-methyl-4-oxoquinazolin-3(4H)-yl)piperidine-2,6-dione;
(3R)-3-(5-(8-(((3 as,6as)-hexahydro-2, 5-methanopentalen-3 a(1H)-yl)amino)oct-1-yn-1-yl)-2-methyl-4-oxoquinazolin-3(4H)-yl)piperidine-2,6-dione;
(3S)-3-(5-(8-((1-((3r,5r,7r)-adamantan-1-yl)ethyl)amino)octyl)-2-methyl-4-oxoquinazolin-3(4H)-yl)piperidine-2,6-dione;
(3S)-3-(5-(8-((1-((3r,5r,7r)-adamantan-1-yl)ethyl)amino)oct-1-yn-1-yl)-2-methyl-4-oxoquinazolin-3(4H)-yl)piperidine-2,6-dione;
(S)-3-(5-(8-(((1 SR,3RS,SSR,7r)-3,5-dimethyladamantan-1-yl)amino)octyl)-4-oxo-2-(trifluoromethyl)quinazolin-3(4H)-yl)piperidine-2,6-dione;
(S)-3-(5-(8-(((1 SR,3RS,SSR,7r)-3,5-dimethyladamantan-1-yl)amino)oct-1-yn-1-yl)-4-oxo-2-(trifluoromethyl) quinazolin-3 (4H)-yl)piperidine-2,6-dione;
(3 S)-3-(5-(8-((1-((3r,5r,7r)-adamantan-1-yl)ethyl)amino)octyl)-4-oxo-2-(trifluoromethyl)quinazolin-3 (4H)-yl)piperidine-2,6-dione;
(3S)-3-(5-(8-((1-((3r,5r,7r)-adamantan-1-yl)ethyl)amino)oct-1-yn-1-yl)-4-oxo-2-(trifluoromethyl)quinazolin-3 (4H)-yl)piperidine-2,6-dione;
(3R)-3-(5-(8-(((1s,3s)-adamantan-1-yl)amino)octyl)-4-oxo-2-(trifluoromethyl)quinazolin-3(4H)-yl)piperidine-2,6-dione;
(3R)-3 -(5-(8-(((1s,3 s)-adamantan-1-yl)amino)oct-1-yn-1-yl)-4-oxo-2-(trifluoromethyl)quinazolin-3 (4H)-yl)piperidine-2,6-dione;
(3S)-3-(5-(8-((2-isopropyl-5-methylcyclohexyl)oxy)octyl)-4-oxo-2-(trifluoromethyl)quinazolin-3 (4H)-yl)piperidine-2,6-dione;
(3S)-3-(5-(8-((2-isopropyl-5-methylcyclohexyl)oxy)oct-1-yn-1-yl)-4-oxo-2-(trifluoromethyl)quinazolin-3 (4H)-yl) piperidine-2,6-dione;
(S)-3-(4-oxo-2-(trifluoromethyl)-5-(8-(((1R,2S,4R)-1,7,7-trimethylbicyclo[2.2.1]heptan-2-yl)amino)octyl)quinazolin-3(4H)-yl)piperidine-2,6-dione;
(S)-3-(4-oxo-2-(trifluoromethyl)-5-(8-(((1R,2S,4R)-1,7,7-trimethylbicyclo[2.2.1]heptan-2-yl)amino)oct-1-yn-1-yl)quinazolin-3 (4H)-yl)piperidine-2,6-dione;
3 -(6-(3-(((1s,3 s)-adamantan-1-yl)amino)prop-1-yn-1-yl)-2-methyl-4-oxoquinazolin-3 (4H)-yl)piperidine-2,6-dione;

3-(6-(3-(((1s,3s)-adamantan-1-yl)amino)propyl)-2-methyl-4-oxoquinazolin-3(4H)-yl)piperidine-2,6-dione;
3 -(6-(5-(((1s,3 s)-adamantan-1-yl)amino)pent-1-yn-1-yl)-2-methyl-4-oxoquinazolin-3 (4H)-yl)piperidine-2,6-dione;
3-(6-(5-(((1s,3s)-adamantan-1-yl)amino)pentyl)-2-methyl-4-oxoquinazolin-3(4H)-yl)piperidine-2,6-dione;
3 -(6-(8-(((3 s, 5s,7s)-adamantan-1-yl)amino)oct-1-yn-1-yl)-2-methyl-4-oxoquinazolin-3 (4H)-yl)piperidine-2,6-dione;
3-(6-(8-(((3s,5s,7s)-adamantan-1-yl)amino)octyl)-2-methyl-4-oxoquinazolin-3(4H)-yl)piperidine-2,6-dione;
3-(6-(8-((1-((1s,3s)-adamantan-1 -yl)ethyl)amino)oct-1 -yn-1 -yl)-2-methyl-4-oxoquinazolin-3 (4H)-yl)piperidine-2,6-dione;
3-(6-(8-((1 -((1s,3s)-adamantan-1-yl)ethyl)amino)octyl)-2-methyl-4-oxoquinazolin-3(4H)-yl)piperidine-2,6-dione;
3 -(7-(8-(((3 s, 5 s, 7s)-adamantan-1-yl) amino)oct-1-yn-1-yl)-2-methyl-4-oxo quinazolin-3 (4H)-yl)piperidine-2,6-dione;
3-(7-(8-(((3s,5s,7s)-adamantan-1-yl)amino)octyl)-2-methyl-4-oxoquinazolin-3(4H)-yl)piperidine-2,6-dione;
3-(7-(8-(((3s,5s,7s)-adamantan-1-yl)amino)octyl)-4-oxo-2-(trifluoromethyl)quinazolin-3(4H)-yl)piperidine-2,6-dione;
3 -(8-(8-(((3 s, 5 s, 7s)-adamantan-1-yl) amino)oct-1-yn-1-yl)-2-methyl-4-oxo quinazolin-3 (4H)-yl)piperidine-2,6-dione;
3-(8-(8-(((3s,5s,7s)-adamantan-1-yl)amino)octyl)-2-methyl-4-oxoquinazolin-3(4H)-yl)piperidine-2,6-dione;
3 -(8-(8-(((3 s, 5 s,7s)-adamantan-1-yl)amino)octyl)-4-oxo-2-(trifluoromethyl)quinazolin-3 (4H)-yl)piperidine-2,6-dione;
3-(5-(8-(((1s,3s)-adamantan-1-yl)amino)octyl)-2-(8-(((3s,5s,7s)-adamantan-1-yl)amino)octyl)-4-oxoquinazolin-3(4H)-yl)piperidine-2,6-dione;
(3r,5r,7r)-N-(5-((3-(2,6-dioxopiperidin-3-yl)-2-methyl-4-oxo-3,4-dihydroquinazolin-5-yl)amino)pentyl)adamantane-1-carboxamide;
N-((1s,3 s)-adamantan-1-yl)-7-((3-(2,6-dioxopiperidin-3-yl)-2-methyl-4-oxo-3,4-dihydroquinazolin-5-yl)amino)heptanamide;
3 -(5-((4-((((1s,3s)-adamantan-1-yl)amino)methyl)piperazin- 1 -yl)methyl)-2-methyl-4-oxoquinazolin-3(4H)-yl)piperidine-2,6-dione;
3-(5-((4-(2-(((3 s, 5 s,7s)-adamantan-1-yl)amino)ethyl)piperazin-1-yl)methyl)-2-methyl-4-oxoquinazolin-3(4H)-yl)piperidine-2,6-dione;
3-(5-((4-(3-(((1s,3s)-adamantan-1-yl)amino)propyl)piperazin-1-yl)methyl)-2-methyl-4-oxoquinazolin-3(4H)-yl)piperidine-2,6-dione;
3-(5-(2-(4-((((3s,5s,7s)-adamantan-1-yl)amino)methyl)piperazin-1-yl)ethyl)-2-methyl-4-oxoquinazolin-3(4H)-yl)piperidine-2,6-dione;
3-(5-(2-(4-(2-(((1s,3s)-adamantan-1-yl)amino)ethyl)piperazin-1-yl)ethyl)-2-methyl-4-oxoquinazolin-3(4H)-yl)piperidine-2,6-dione;
3-(5-(2-(4-(3-(((3s,5s,7s)-adamantan-1-yl)amino)propyl)piperazin-1-yl)ethyl)-2-methyl-4-oxoquinazolin-3(4H)-yl)piperidine-2,6-dione;
3-(5-(3-(4-((((1s,3s)-adamantan-1-yl)amino)methyl)piperazin-1-yl)propyl)-2-methyl-4-oxoquinazolin-3(4H)-yl)piperidine-2,6-dione;
3-(5-(3-(4-(2-(((3 s, 5 s,7s)-adamantan-1-yl)amino)ethyl)piperazin-1-yl)propyl)-2-methyl-4-oxoquinazolin-3(4H)-yl)piperidine-2,6-dione;
3-(5-(3-(4-(3-(((1s,3s)-adamantan-1-yl)amino)propyl)piperazin-1-yl)propyl)-2-methyl-4-oxoquinazolin-3(4H)-yl)piperidine-2,6-dione;
3-(5-((4-(((3r,5r,7r)-adamantan-1-yl)methyl)piperazin-1-yl)methyl)-2-methyl-4-oxoquinazolin-3 (4H)-yl)piperidine-2,6-dione;
3-(2-methyl-4-oxo-5-(piperazin-1-ylmethyl)quinazolin-3(4H)-yl)piperidine-2,6-dione;
3-(2-methyl-4-oxo-5-(3-(piperazin-1-yl)prop-1-yn-1-yl)quinazolin-3(4H)-yl)piperidine-2,6-dione;
3-(5-((4-(7-(((1s,3s)-adamantan-1-yl)amino)heptyl)piperazin-1-yl)methyl)-2-methyl-4-oxoquinazolin-3(4H)-yl)piperidine-2,6-dione;
3-(5-(3-(4-(2-(((3s,5s,7s)-adamantan-1-yl)amino)ethyl)piperazin-1-yl)prop-1-yn-1-yl)-2-methyl-4-oxoquinazolin-3(4H)-yl)piperidine-2,6-dione;
3-(5-(3-(4-(3-(((1s,3s)-adamantan-1-yl)amino)propyl)piperazin-1-yl)prop-1-yn-1-yl)-2-methyl-4-oxoquinazolin-3(4H)-yl)piperidine-2,6-dione;
3 -(5 -((2-(4-((1s,3s)-adamantan-1-yl)piperazin-1-yl)ethyl)thio)-2-methyl-4-oxoquinazolin-3 (4H)-yl)piperidine-2,6-dione;
3-(5-((3-(4-((3s,5s,7s)-adamantan-1-yl)piperazin-1-yl)propyl)thio)-2-methyl-4-oxoquinazolin-3 (4H)-yl)piperidine-2,6-dione;
3-(5-((2-(4-((1s,3s)-adamantan-1 -yl)piperazin-1 -yl)ethyl)amino)-2-methyl-4-oxoquinazolin-3 (4H)-yl)piperidine-2,6-dione;
3-(5-(2-(4-((1s,3s)-adamantan-1-yl)piperazin-1-yl)ethoxy)-2-methyl-4-oxoquinazolin-3 (4H)-yl)piperidine-2,6-dione;
3-(5-(3-(4-((1s,3s)-adamantan-1 -yl)piperazin-1 -yl)prop-1 -yn-1 -yl)-2-methyl-4-oxoquinazolin-3 (4H)-yl)piperidine-2,6-dione;
3 -(5 -(3 -(4-((1s,3s)-adamantan-1-yl)piperazin-1-yl)propyl)-2-methyl-4-oxoquinazolin-3 (4H)-yl)piperidine-2,6-dione;
3-(5-((2-(4-(((3r,5r,7r)-adamantan-1-yl)methyl)piperazin-1-yl)ethyl)thio)-2-methyl-4-oxoquinazolin-3(4H)-yl)piperidine-2,6-dione;
3 -(5 -((3-(4-(((1s,3 s)-adamantan-1 -yl)methyl)piperazin-1 -yl)propyl)thio)-2-methyl-4-oxoquinazolin-3(4H)-yl) piperidine-2,6-dione;
3-(5-((2-(4-(((3r,5r,7r)-adamantan-1-yl)methyl)piperazin-1-yl)ethyl)amino)-2-methyl-4-oxoquinazolin-3(4H)-yl) piperidine-2,6-dione;
3-(5-(2-(4-(((3r,5r,7r)-adamantan-1-yl)methyl)piperazin-1-yl)ethoxy)-2-methyl-4-oxoquinazolin-3 (4H)-yl)piperidine-2,6-dione;
3-(5-(3-(4-(((3r,5r,7r)-adamantan-1-yl)methyl)piperazin-1-yl)prop-1-yn-1-yl)-2-methyl-4-oxoquinazolin-3(4H)-yl)piperidine-2,6-dione;
3-(5-(3-(4-(((3r,5r,7r)-adamantan-1-yl)methyl)piperazin-1-yl)propyl)-2-methyl-4-oxoquinazolin-3 (4H)-yl)piperidine-2,6-dione;
3-(5-((2-(4-(2-((3r,5r,7r)-adamantan-1-yl)ethyl)piperazin-1-yl)ethyl)thio)-2-methyl-4-oxoquinazolin-3(4H)-yl)piperidine-2,6-dione;
3-(5-((2-(4-(2-((3r,5r,7r)-adamantan-1-yl)ethyl)piperazin-1-yl)ethyl)amino)-2-methyl-4-oxoquinazolin-3(4H)-yl) piperidine-2,6-dione;
3-(5-(2-(4-(2-((3r,5r,7r)-adamantan-l-yl)ethyl)piperazin-l-yl)ethoxy)-2-methyl-4-oxoquinazolin-3 (4H)-yl)piperidine-2,6-dione;
3-(5-(3-(4-(2-((3r,5r,7r)-adamantan-1-yl)ethyl)piperazin-1-yl)prop-1-yn-1-yl)-2-methyl-4-oxoquinazolin-3(4H)-yl)piperidine-2,6-dione;
3-(5-(3-(4-(2-((3r,5r,7r)-adamantan-1-yl)ethyl)piperazin-1-yl)propyl)-2-methyl-4-oxoquinazolin-3 (4H)-yl)piperidine-2,6-dione;
3-(5-((4-(4-((3r,5r,7r)-adamantan-1-yl)benzyl)piperazin-1-yl)methyl)-2-methyl-4-oxoquinazolin-3 (4H)-yl)piperidine-2,6-dione;
3-(5-((4-(2-((1s,3s)-adamantan-1-yl)ethyl)piperazin-1-yl)methyl)-2-methyl-4-oxoquinazolin-3 (4H)-yl)piperidine-2,6-dione;
3-(5-(3-(4-(4-((3r,5r,7r)-adamantan-1-yl)benzyl)piperazin-1-yl)prop-1-yn-1-yl)-2-methyl-4-oxoquinazolin-3(4H)-yl)piperidine-2,6-dione;
3-(5-(3-(4-(4-((3r,5r,7r)-adamantan-1-yl)benzyl)piperazin-1-yl)propyl)-2-methyl-4-oxoquinazolin-3(4H)-yl)piperidine-2,6-dione;
3-(5-((2-(4-((1s,3 s)-adamantan-1-yl)piperazin-1-yl-2,2,3,3, 5, 5,6,6-d8)ethyl)thio)-2-methyl-4-oxoquinazolin-3(4H)-yl)piperidine-2,6-dione;
3-(5-((2-(4-((1s,3s)-adamantan-1-yl)piperazin-1-yl-2,2,3,3,5,5,6,6-d8)ethyl)amino)-2-methyl-4-oxoquinazolin-3(4H)-yl)piperidine-2,6-dione;
3-(5-(2-(4-((1s,3s)-adamantan-1-yl)piperazin-1-yl-2,2,3,3,5,5,6,6-d8)ethoxy)-2-methyl-4-oxoquinazolin-3(4H)-yl)piperidine-2,6-dione;
3-(5-(3-(4-(((3r,5r,7r)-adamantan-1-yl)methyl)piperazin-1-yl-2,2,3,3,5,5,6,6-d8)prop-1-yn-1-yl)-2-methyl-4-oxoquinazolin-3 (4H)-yl)piperidine-2,6-dione;
3-(5-(3-(4-(2-((1s,3s)-adamantan-1-yl)ethyl)piperazin-1-yl-2,2,3,3,5,5,6,6-d8)prop-1-yn-1-yl)-2-methyl-4-oxoquinazolin-3(4H)-yl)piperidine-2,6-dione;
3-(5-(3-(4-(2-((1s,3s)-adamantan-1-yl)ethyl)piperazin-1-yl-2,2,3,3,5,5,6,6-d8)propyl)-2-methyl-4-oxoquinazolin-3(4H)-yl)piperidine-2,6-dione;
3-(5-(((1-((((1s,3s)-adamantan-1-yl)amino)methyl)cyclopropyl)methyl)amino)-2-methyl-4-oxoquinazolin-3(4H)-yl)piperidine-2,6-dione;
3-(5-((4-(2-(((2R,3as,5S,6as)-hexahydro-2,5-methanopentalen-3a(1H)-yl)amino)ethyl)piperazin-1-yl) methyl)-2-methyl-4-oxoquinazolin-3 (4H)-yl)piperidine-2,6-dione;
3-(5-((4-(3-(((3as,6as)-hexahydro-2,5-methanopentalen-3a(1H)-yl)amino)propyl)piperazin-1-yl)methyl)-2-methyl-4-oxoquinazolin-3(4H)-yl)piperidine-2,6-dione;
3-(5-((2-((2-(((1s,3s)-adamantan-1-yl)amino)ethyl)(methyl)amino)ethyl)amino)-2-methyl-4-oxoquinazolin-3(4H)-yl)piperidine-2,6-dione;
N-((3s,5s,7s)-adamantan-1-yl)-2-((3-(2,6-dioxopiperidin-3-yl)-2-methyl-4-oxo-3,4-dihydroquinazolin-5-yl)amino)ethane-1-sulfonamide;
N-((1s,3s)-adamantan-1-yl)-1-(1-(3-(2,6-dioxopiperidin-3-yl)-2-methyl-4-oxo-3,4-dihydroquinazolin-5-yl)piperidin-3-yl)methanesulfonamide;
N-((3s,5s,7s)-adamantan-1-yl)-2-((3-(2,6-dioxopiperidin-3-yl)-2-methyl-4-oxo-3,4-dihydroquinazolin-5-yl)thio)ethane-1-sulfonamide;
N-((3s,5s,7s)-adamantan-1-yl)-2-((3-(2,6-dioxopiperidin-3-yl)-2-methyl-4-oxo-3,4-dihydroquinazolin-5-yl)oxy)ethane-1-sulfonamide;
N-((3s,5s,7s)-adamantan-1-yl)-3-(3-(2,6-dioxopiperidin-3-yl)-2-methyl-4-oxo-3,4-dihydroquinazolin-5-yl)propane-1-sulfonamide;
N-((3s,5s,7s)-adamantan-1-yl)-3-((3-(2,6-dioxopiperidin-3-yl)-2-methyl-4-oxo-3,4-dihydroquinazolin-5-yl)amino)propane-1-sulfonamide;
3-(2-methyl-5-((4-((4-(oxetan-3-yl)piperazin-1-yl)sulfonyl)butyl)amino)-4-oxoquinazolin-3(4H)-yl)piperidine-2,6-dione;
3-(5-((3-(4-(((3s,5s,7s)-adamantan-1-yl)amino)-1H-1,2,3-triazol-1-yl)propyl)amino)-2-methyl-4-oxoquinazolin-3(4H)-yl)piperidine-2,6-dione;
3-(8-((2-(4-(bis(4-fluorophenyl)methyl)piperazin-1-yl)ethyl)amino)-3-methyl-1-oxoisoquinolin-2(1H)-yl)piperidine-2,6-dione;
3-(5-((3-(4-(2-((3r,5r,7r)-adamantan-1-yl)ethyl)piperazin-1-yl)propyl)thio)-2-methyl-4-oxoquinazolin-3(4H)-yl)piperidine-2,6-dione;
3-(5-((3-(4-benzhydrylpiperazin-1-yl)propyl)thio)-2-methyl-4-oxoquinazolin-3(4H)-yl)piperidine-2,6-dione;
3-(5-((3-(4-(bis(4-fluorophenyl)methyl)piperazin-1-yl)propyl)thio)-2-methyl-4-oxoquinazolin-3(4H)-yl)piperidine-2,6-dione;
3-(5-((3-(4-(bis(4-chlorophenyl)methyl)piperazin-1-yl)propyl)thio)-2-methyl-4-oxoquinazolin-3(4H)-yl)piperidine-2,6-dione;
3-(6-((3-(4-(2-((3r,5r,7r)-adamantan-1-yl)ethyl)piperazin-1-yl)propyl)thio)-2-methyl-4-oxoquinazolin-3(4H)-yl)piperidine-2,6-dione;
3-(7-((3-(4-(2-((3r,5r,7r)-adamantan-1-yl)ethyl)piperazin-1-yl)propyl)thio)-2-methyl-4-oxoquinazolin-3(4H)-yl)piperidine-2,6-dione;
3-(8-((3-(4-(2-((3r,5r,7r)-adamantan-1-yl)ethyl)piperazin-1-yl)propyl)thio)-2-methyl-4-oxoquinazolin-3(4H)-yl)piperidine-2,6-dione;
3-(6-((2-(4-(2-((3r,5r,7r)-adamantan-1-yl)ethyl)piperazin-1-yl)ethyl)amino)-2-methyl-4-oxoquinazolin-3(4H)-yl)piperidine-2,6-dione;
3-(7-((2-(4-(2-((3r,5r,7r)-adamantan-1-yl)ethyl)piperazin-1-yl)ethyl)amino)-2-methyl-4-oxoquinazolin-3(4H)-yl)piperidine-2,6-dione;
3-(8-((2-(4-(2-((3r,5r,7r)-adamantan-1-yl)ethyl)piperazin-1-yl)ethyl)amino)-2-methyl-4-oxoquinazolin-3(4H)-yl)piperidine-2,6-dione;
3-(5-(3-(2-((3r,5r,7r)-adamantan-1-yl)ethyl)-3,6-diazabicyclo[3.1.1]heptan-6-yl)-2-methyl-4-oxoquinazolin-3(4H)-yl)piperidine-2,6-dione;
3-(5-(6-(2-((3r,5r,7r)-adamantan-1-yl)ethyl)-3,6-diazabicyclo[3.1.1]heptan-3-yl)-2-methyl-4-oxoquinazolin-3(4H)-yl)piperidine-2,6-dione;
3-(5-(3-(2-((3r,5r,7r)-adamantan-1-yl)ethyl)-3,8-diazabicyclo[3.2.1]octan-8-yl)-2-methyl-4-oxoquinazolin-3(4H)-yl)piperidine-2,6-dione;
3-(5-(8-(2-((3r,5r,7r)-adamantan-1-yl)ethyl)-3,8-diazabicyclo[3.2.1]octan-3-yl)-2-methyl-4-oxoquinazolin-3(4H)-yl)piperidine-2,6-dione;
3-(5-(5-(2-((3r,5r,7r)-adamantan-1-yl)ethyl)-2,5-diazabicyclo[2.2.2]octan-2-yl)-2-methyl-4-oxoquinazolin-3(4H)-yl)piperidine-2,6-dione;
3-(5-((1S,4S)-5-(2-((3S,5S,7S)-adamantan-1-yl)ethyl)-2,5-diazabicyclo[2.2.1]heptan-2-yl)-2-methyl-4-oxoquinazolin-3(4H)-yl)piperidine-2,6-dione;
3-(5-((1R,4R)-5-(2-((3R,5R,7R)-adamantan-1-yl)ethyl)-2,5-diazabicyclo[2.2.1]heptan-2-yl)-2-methyl-4-oxoquinazolin-3(4H)-yl)piperidine-2,6-dione;
3-(5-(4-(2-((3r,5r,7r)-adamantan-1-yl)ethyl)-3,5-dimethylpiperazin-1-yl)-2-methyl-4-oxoquinazolin-3(4H)-yl)piperidine-2,6-dione;
3-(5-(4-(2-((3r,5r,7r)-adamantan-1-yl)ethyl)-2,6-dimethylpiperazin-1-yl)-2-methyl-4-oxoquinazolin-3(4H)-yl)piperidine-2,6-dione;
3-(5-(4-(2-((3r,5r,7r)-adamantan-1-yl)ethyl)-2-(trifluoromethyl)piperazin-1-yl)-2-methyl-4-oxoquinazolin-3(4H)-yl)piperidine-2,6-dione;

3-(5-(4-(2-((3r,5r,7r)-adamantan-1-yl)ethyl)-2-(fluoromethyl)piperazin-1-yl)-2-methyl-4-oxoquinazolin-3(4H)-yl)piperidine-2,6-dione;
3-(5-(4-(2-((3r,5r,7r)-adamantan-1-yl)ethyl)-2,2-dimethylpiperazin-1-yl)-2-methyl-4-oxoquinazolin-3(4H)-yl)piperidine-2,6-dione;
3-(5-(4-(2-((3r,5r,7r)-adamantan-1-yl)ethyl)-1,4-diazepan-1-yl)-2-methyl-4-oxoquinazolin-3 (4H)-yl)piperidine-2,6-dione;
3-(5-((3-(4-(2-((3r,5r,7r)-adamantan-1-yl)ethyl)-3,5-dimethylpiperazin-1-yl)propyl)thio)-2-methyl-4-oxoquinazolin-3(4H)-yl)piperidine-2,6-dione;
3-(5-((3-(4-(2-((3r,5r,7r)-adamantan-1-yl)ethyl)-2,6-dimethylpiperazin-1-yl)propyl)thio)-2-methyl-4-oxoquinazolin-3(4H)-yl)piperidine-2,6-dione;
3-(5-((3-(4-(2-((3r,5r,7r)-adamantan-1-yl)ethyl)-2-(trifluoromethyl)piperazin-1-yl)propyl)thio)-2-methyl-4-oxoquinazolin-3(4H)-yl)piperidine-2,6-dione;
3-(5-((3-(4-(2-((3r,5r,7r)-adamantan-1-yl)ethyl)-2-(fluoromethyl)piperazin-1-yl)propyl)thio)-2-methyl-4-oxoquinazolin-3(4H)-yl)piperidine-2,6-dione;
3-(5-((3-(4-(2-((3r,5r,7r)-adamantan-1-yl)ethyl)-2,2-dimethylpiperazin-1-yl)propyl)thio)-2-methyl-4-oxoquinazolin-3(4H)-yl)piperidine-2,6-dione;
3-(5-((3-(4-(2-((3r,5r,7r)-adamantan-1-yl)ethyl)-1,4-diazepan-1-yl)propyl)thio)-2-methyl-4-oxoquinazolin-3(4H)-yl)piperidine-2,6-dione;
3-(5-((2-(3-(2-((3r,5r,7r)-adamantan-1-yl)ethyl)-3,6-diazabicyclo[3.1.1]heptan-6-yl)ethyl)amino)-2-methyl-4-oxoquinazolin-3(4H)-yl)piperidine-2,6-dione;
3-(5-((2-(6-(2-((3r,5r,7r)-adamantan-1-yl)ethyl)-3,6-diazabicyclo[3.1.1]heptan-3-yl)ethyl)amino)-2-methyl-4-oxoquinazolin-3(4H)-yl)piperidine-2,6-dione;
3-(5-((2-(3-(2-((3r,5r,7r)-adamantan-1-yl)ethyl)-3,8-diazabicyclo[3.2.1]octan-8-yl)ethyl)amino)-2-methyl-4-oxoquinazolin-3 (4H)-yl)piperidine-2,6-dione;
3-(5-((2-(8-(2-((3r,5r,7r)-adamantan-1-yl)ethyl)-3,8-diazabicyclo[3.2.1]octan-3-yl)ethyl)amino)-2-methyl-4-oxoquinazolin-3 (4H)-yl)piperidine-2,6-dione;
3-(5-((2-(5-(2-((3r,5r,7r)-adamantan-1-yl)ethyl)-2,5-diazabicyclo[2.2.2]octan-2-yl)ethyl)amino)-2-methyl-4-oxoquinazolin-3 (4H)-yl)piperidine-2,6-dione;
3-(5-((2-((1S,4S)-5-(2-((3S,5S,7S)-adamantan-1-yl)ethyl)-2,5-diazabicyclo[2.2.1]heptan-2-yl)ethyl)amino)-2-methyl-4-oxoquinazolin-3(4H)-yl)piperidine-2,6-dione;
3-(5-((2-((1R,4R)-5-(2-((3R,5R,7R)-adamantan-1-yl)ethyl)-2,5-diazabicyclo[2.2.1]heptan-2-yl)ethyl)amino)-2-methyl-4-oxoquinazolin-3(4H)-yl)piperidine-2,6-dione;
3-(5-((2-(4-(2-((3r,5r,7r)-adamantan-1-yl)ethyl)-3,5-dimethylpiperazin-1-yl)ethyl)amino)-2-methyl-4-oxoquinazolin-3(4H)-yl)piperidine-2,6-dione;
3-(5-((2-(4-(2-((3r,5r,7r)-adamantan-1-yl)ethyl)-2,6-dimethylpiperazin-1-yl)ethyl)amino)-2-methyl-4-oxoquinazolin-3(4H)-yl)piperidine-2,6-dione;
3-(5-((2-(4-(2-((3r,5r,7r)-adamantan-1-yl)ethyl)-3-(trifluoromethyl)piperazin-1-yl)ethyl)amino)-2-methyl-4-oxoquinazolin-3 (4H)-yl)piperidine-2,6-dione;
3-(5-((2-(4-(2-((3r,5r,7r)-adamantan-1-yl)ethyl)-2-(fluoromethyl)piperazin-1-yl)ethyl)amino)-2-methyl-4-oxoquinazolin-3(4H)-yl)piperidine-2,6-dione;
3-(5-((2-(4-(2-((3r,5r,7r)-adamantan-1-yl)ethyl)-2,2-dimethylpiperazin-1-yl)ethyl)amino)-2-methyl-4-oxoquinazolin-3(4H)-yl)piperidine-2,6-dione;
3-(5-((2-(4-(2-((3r,5r,7r)-adamantan-1-yl)ethyl)-1,4-diazepan-1-yl)ethyl)amino)-2-methyl-4-oxoquinazolin-3(4H)-yl)piperidine-2,6-dione;
3-(5-((2-(4-benzhydrylpiperazin-1-yl)ethyl)amino)-2-methyl-4-oxoquinazolin-3(4H)-yl)piperidine-2,6-dione;
3-(5-((2-(4-((4'-chloro-5,5-dimethyl-3,4,5,6-tetrahydro-[1,1'-biphenyl]-2-yl)methyl)piperazin-1-yl)ethyl)amino)-2-methyl-4-oxoquinazolin-3(4H)-yl)piperidine-2,6-dione;
3-(5-((2-(4-((4'-chloro-[1,1'-biphenyl]-2-yl)methyl)piperazin-1-yl)ethyl)amino)-2-methyl-4-oxoquinazolin-3(4H)-yl)piperidine-2,6-dione;
3-(5-((2-(4-((4'-fluoro-5,5-dimethyl-3,4,5,6-tetrahydro-[1,1'-biphenyl]-2-yl)methyl)piperazin-1-yl)ethyl)amino)-2-methyl-4-oxoquinazolin-3(4H)-yl)piperidine-2,6-dione;
3-(5-((2-(4-((4'-fluoro-3,4,5,6-tetrahydro-[1,1'-biphenyl]-2-yl)methyl)piperazin-1-yl)ethyl)amino)-2-methyl-4-oxoquinazolin-3(4H)-yl)piperidine-2,6-dione;
3-(5-((2-(4-((4'-chloro-4-methoxy-4-methyl-3,4,5,6-tetrahydro-[1,1'-biphenyl]-2-yl)methyl)piperazin-1-yl)ethyl)amino)-2-methyl-4-oxoquinazolin-3(4H)-yl)piperidine-2,6-dione;
3-(5-((2-(4-((4'-chloro-4,4-dimethyl-3,4,5,6-tetrahydro-[1,1'-biphenyl]-2-yl)methyl)piperazin-1-yl)ethyl)amino)-2-methyl-4-oxoquinazolin-3(4H)-yl)piperidine-2,6-dione;
3-(5-((2-(4-((4-(4-chlorophenyl)-5,6-dihydro-2H-pyran-3-yl)methyl)piperazin-1-yl)ethyl)amino)-2-methyl-4-oxoquinazolin-3 (4H)-yl)piperidine-2,6-dione;
3-(5-((2-(4-((4-(4-chlorophenyl)-6,6-dimethyl-5,6-dihydro-2H-pyran-3-yl)methyl)piperazin-1-yl)ethyl)amino)-2-methyl-4-oxoquinazolin-3(4H)-yl)piperidine-2,6-dione;
3-(5-((2-(4-((4'-chloro-4,4-difluoro-3,4,5,6-tetrahydro-[1,1'-biphenyl]-2-yl)methyl)piperazin-1-yl)ethyl)amino)-2-methyl-4-oxoquinazolin-3(4H)-yl)piperidine-2,6-dione;
3-(5-((2-(4-((8-(4-chlorophenyl)spiro[4.5]dec-7-en-7-yl)methyl)piperazin-1-yl)ethyl)amino)-2-methyl-4-oxoquinazolin-3(4H)-yl)piperidine-2,6-dione;
3-(5-((2-(4-((6-(4-chlorophenyl)spiro[3.5]non-6-en-7-yl)methyl)piperazin-1-yl)ethyl)amino)-2-methyl-4-oxoquinazolin-3(4H)-yl)piperidine-2,6-dione;
3-(5-((3-(4-((4'-chloro-5,5-dimethyl-3,4,5,6-tetrahydro-[1,1'-biphenyl]-2-yl)methyl)piperazin-1-yl)propyl)thio)-2-methyl-4-oxoquinazolin-3(4H)-yl)piperidine-2,6-dione;
3-(5-((3-(4-((4'-chloro-[1,1'-biphenyl]-2-yl)methyl)piperazin-1-yl)propyl)thio)-2-methyl-4-oxoquinazolin-3(4H)-yl)piperidine-2,6-dione;
3-(5-((3-(4-((4'-fluoro-5,5-dimethyl-3,4,5,6-tetrahydro-[1,1'-biphenyl]-2-yl)methyl)piperazin-1-yl)propyl)thio)-2-methyl-4-oxoquinazolin-3(4H)-yl)piperidine-2,6-dione;
3-(5-((3-(4-((4'-fluoro-3,4,5,6-tetrahydro-[1,1'-biphenyl]-2-yl)methyl)piperazin-1-yl)propyl)thio)-2-methyl-4-oxoquinazolin-3(4H)-yl)piperidine-2,6-dione;
3-(5-((3-(4-((4'-chloro-4-methoxy-4-methyl-3,4,5,6-tetrahydro-[1,1'-biphenyl]-2-yl)methyl)piperazin-1-yl)propyl)thio)-2-methyl-4-oxoquinazolin-3(4H)-yl)piperidine-2,6-dione;
3-(5-((3-(4-((4'-chloro-4,4-dimethyl-3,4,5,6-tetrahydro-[1,1'-biphenyl]-2-yl)methyl)piperazin-1-yl)propyl)thio)-2-methyl-4-oxoquinazolin-3(4H)-yl)piperidine-2,6-dione;
3-(5-((3-(4-((4-(4-chlorophenyl)-5,6-dihydro-2H-pyran-3-yl)methyl)piperazin-1-yl)propyl)thio)-2-methyl-4-oxoquinazolin-3 (4H)-yl)piperidine-2,6-dione;
3-(5-((3-(4-((4-(4-chlorophenyl)-6,6-dimethyl-5,6-dihydro-2H-pyran-3-yl)methyl)piperazin-1-yl)propyl)thio)-2-methyl-4-oxoquinazolin-3(4H)-yl)piperidine-2,6-dione;
3-(5-((3-(4-((4'-chloro-4,4-difluoro-3,4,5,6-tetrahydro-[1,1'-biphenyl]-2-yl)methyl)piperazin-1-yl)propyl)thio)-2-methyl-4-oxoquinazolin-3(4H)-yl)piperidine-2,6-dione;
3-(5-((3-(4-((8-(4-chlorophenyl)spiro[4.5]dec-7-en-7-yl)methyl)piperazin-1-yl)propyl)thio)-2-methyl-4-oxoquinazolin-3(4H)-yl)piperidine-2,6-dione;
3-(5-((3-(4-((6-(4-chlorophenyl)spiro[3.5]non-6-en-7-yl)methyl)piperazin-1-yl)propyl)thio)-2-methyl-4-oxoquinazolin-3(4H)-yl)piperidine-2,6-dione;
4-(4-(2-((3-(2,6-dioxopiperidin-3-yl)-2-methyl-4-oxo-3,4-dihydroquinazolin-5-yl)thio)ethyl)piperazin-1-yl)-3-fluorobenzonitrile;
3-(5-((2-(4-(4-((1s,3s)-adamantan-1-yl)benzyl)piperazin-1-yl)ethyl)thio)-2-methyl-4-oxoquinazolin-3(4H)-yl)piperidine-2,6-dione;
3-(5-((2-(4-(((1s,3s)-adamantan-1-yl)methyl)piperazin-1-yl)ethyl)amino)-2-methyl-4-oxoquinazolin-3(4H)-yl)piperidine-2,6-dione;
3-(5-((2-(4-(4-((1s,3s)-adamantan-1-yl)benzyl)piperazin-1-yl)ethyl)amino)-2-methyl-4-oxoquinazolin-3(4H)-yl) piperidine-2,6-dione;
4-(4-(2-((3-(2,6-dioxopiperidin-3-yl)-2-methyl-4-oxo-3,4-dihydroquinazolin-5-yl)amino)ethyl)piperazin-1-yl)-3 -fluorobenzonitrile;
3-(5-(2-(4-((3s,5s,7s)-adamantan-1-yl)piperazin-1-yl)ethoxy)-2-methyl-4-oxoquinazolin-3(4H)-yl)piperidine-2,6-dione;
3-(5-(2-(4-(4-((1s,3s)-adamantan-1-yl)benzyl)piperazin-1-yl)ethoxy)-2-methyl-4-oxoquinazolin-3 (4H)-yl)piperidine-2,6-dione;
4-(4-(2-((3-(2,6-dioxopiperidin-3-yl)-2-methyl-4-oxo-3,4-dihydroquinazolin-5-yl)oxy)ethyl)piperazin-1 -yl)-3 -fluorobenzonitrile;
3-(5-((4-(2-((3r,5r,7r)-adamantan-1-yl)ethyl)piperazin-1-yl)methyl)-2-methyl-4-oxoquinazolin-3 (4H)-yl)piperidine-2,6-dione;
3-(5-((4-((1s,3 s)-adamantane-1-carbonyl)piperazin-1-yl)methyl)-2-methyl-4-oxoquinazolin-3(4H)-yl)piperidine-2,6-dione;
3-(5-(3-(4-(2-((1s,3s)-adamantan-1-yl)ethyl)piperazin-1-yl)prop-1-yn-1-yl)-2-methyl-4-oxoquinazolin-3(4H)-yl) piperidine-2,6-dione; or
3-(5-(3-(4-((3r,5r,7r)-adamantane-1-carbonyl)piperazin-1-yl)prop-1-yn-1-yl)-2-methyl-4-oxoquinazolin-3(4H)-yl)piperidine-2,6-dione.

**37.** The compound of Formula (I) or salts, enantiomers, diastereoisomer, isotopically enriched analogs, solvates, prodrugs, or polymorphs thereof as claimed in any one of claims 1-36, which is hydrochlorides, sulfates, citrates,

maleates, sulfonates, citrates, lactates, tartrates, fumarates, phosphates, dihydrophosphates, pyrophosphates, metaphosphates, oxalates, malonates, benzoates, mandelates, succinates, trifluoroacetates, hydroxyacetates, or p-toluenesulfonates of the compound of Formula (I).

**38.** A pharmaceutical composition comprising the compound of Formula (I) or pharmaceutically acceptable salts thereof as claimed in any one of claims 1-37, and at least one pharmaceutically acceptable carrier, and optionally further comprising at least one additional therapeutic agent, e.g., an anticancer agent.

**39.** Use of the compound of Formula (I) or pharmaceutically acceptable salts thereof as claimed in any one of claims 1-37, or the pharmaceutical composition as claimed in claim 38 for the manufacture of a medicament for the prevention and/or treatment of diseases or disorders associated with cereblon protein;

preferably, wherein the diseases or disorders associated with cereblon protein are selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes;

more preferably, wherein the diseases or disorders associated with cereblon protein are selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin’s lymphoma, Hodgkin’s lymphoma, anaplastic lymphoma, anaplastic large cell lymphoma, CD20 positive lymphoma, mantle cell lymphoma, primary lymphoma, B-cell lymphoma, recurrent B-cell non-Hodgkin’s lymphoma, recurrent diffuse large B-cell lymphoma, recurrent mediastinal (thymic) large B-cell lymphoma, primary mediastinal (thymic) large B-cell lymphoma, recurrent transformed non-Hodgkin’s lymphoma, refractory B-cell non-Hodgkin’s lymphoma, refractory diffuse large B-cell lymphoma, refractory primary mediastinal (thymic) large B-cell lymphoma, refractory transformed non-Hodgkin’s lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; neuroglioma; Peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing’s sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren’s syndrome, and atopic dermatitis; keratoconjunctivitis; inflammatory diseases, including Crohn’s disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

**40.** The compound of Formula (I) or pharmaceutically acceptable salts thereof as claimed in any one of claims 1-37, for use in the prevention and/or treatment of diseases or disorders associated with cereblon protein;

preferably, wherein the diseases or disorders associated with cereblon protein are selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes;

more preferably, wherein diseases or disorders associated with cereblon protein are selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer and patients with Cowden syndrome; pancreatic cancer; central nervous system tumor; neuroblastoma; neuroglioma; Peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

**41.** A method for treating or preventing diseases or disorders associated with cereblon protein in a subject, comprising administering to the subject a therapeutically effective amount of the compound of Formula (I) or pharmaceutically acceptable salts thereof as claimed in any one of claims 1-37, or the pharmaceutical composition as claimed in claim 38;

preferably, wherein the diseases or disorders associated with cereblon protein are selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes;
more preferably, wherein diseases or disorders associated with cereblon protein are selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML); lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma; inflammatory myofibroblastoma; colorectal cancer; intestinal cancer; brain glioma; astroblastoma; ovarian cancer; bronchial cancer; prostate cancer; breast cancer,

including triple negative breast cancer, sporadic breast cancer and patients with Cowden syndrome; pancreatic cancer; central nervous system tumor; neuroblastoma; neuroglioma; Peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

**INTERNATIONAL SEARCH REPORT**

International application No.
**PCT/CN2023/075345**

**A. CLASSIFICATION OF SUBJECT MATTER**

C07D401/04(2006.01)i；C07D401/14(2006.01)i；C07D405/04(2006.01)i；C07D405/14(2006.01)i；A61K31/517(2006.01)i；A61P35/00(2006.01)i；A61P37/00(2006.01)i

According to International Patent Classification (IPC) or to both national classification and IPC

**B. FIELDS SEARCHED**

Minimum documentation searched (classification system followed by classification symbols)

IPC：C07D401/-，C07D405/-，A61K31/-

Documentation searched other than minimum documentation to the extent that such documents are included in the fields searched

Electronic data base consulted during the international search (name of data base and, where practicable, search terms used)

CNKI, CNTXT, VEN, STN: 喹唑啉酮, 哌啶, 二酮, 度胺, cereblon, quinazo, piperidin, dionr, 基于式(I)中涵盖说明书表4-5化合物的部分进行了结构检索, structural search based on the part of formula (I) covering the compounds in table 4-5 of the description

**C. DOCUMENTS CONSIDERED TO BE RELEVANT**

| Category* | Citation of document, with indication, where appropriate, of the relevant passages | Relevant to claim No. |
|---|---|---|
| X | CN 101535291 A (CELGENE CORPORATION) 16 September 2009 (2009-09-16) claims 1-18 and embodiments | 1-40 |
| X | CN 103688176 A (CELGENE CORPORATION) 26 March 2014 (2014-03-26) claim 1 | 1-40 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 June 2023** | **30 June 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/075345**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **41**
because they relate to subject matter not required to be searched by this Authority, namely:
The subject matter of claim 41 relates to a method for treatment of the human or animal body (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/075345**

| Patent document cited in search report | Publication date (day/month/year) | Patent family member(s) | Publication date (day/month/year) |
|---|---|---|---|
| CN 101535291 A | 16 September 2009 | ES 2564935 T3 | 30 March 2016 |
| | | RS 56279 B1 | 29 December 2017 |
| | | CL 2007002752 A1 | 11 July 2008 |
| | | PT 2066656 E | 28 June 2012 |
| | | CY 1119248 T1 | 14 February 2018 |
| | | HUE 035389 T2 | 02 May 2018 |
| | | IL 197716 A0 | 24 December 2009 |
| | | IL 197716 A | 29 August 2013 |
| | | SI 2420497 T1 | 29 April 2016 |
| | | MY 150699 A | 28 February 2014 |
| | | HUE 026827 T2 | 28 July 2016 |
| | | PE 20080970 A1 | 19 July 2008 |
| | | LT 2428513 T | 25 October 2017 |
| | | DK 2420497 T3 | 07 March 2016 |
| | | PL 2420497 T3 | 30 June 2016 |
| | | PT 2428513 T | 28 August 2017 |
| | | WO 2008039489 A2 | 03 April 2008 |
| | | WO 2008039489 A3 | 28 August 2008 |
| | | EP 3239144 A1 | 01 November 2017 |
| | | US 2015080419 A1 | 19 March 2015 |
| | | US 9732064 B2 | 15 August 2017 |
| | | SI 2428513 T1 | 30 October 2017 |
| | | IL 250847 A0 | 30 April 2017 |
| | | UA 94964 C2 | 25 June 2011 |
| | | PL 2428513 T3 | 31 October 2017 |
| | | TW 201335147 A | 01 September 2013 |
| | | TWI 522351 B | 21 February 2016 |
| | | AR 105340 A2 | 27 September 2017 |
| | | DK 2428513 T3 | 21 August 2017 |
| | | ZA 200901950 B | 30 June 2010 |
| | | IL 228017 A0 | 30 September 2013 |
| | | IL 228017 A | 30 March 2017 |
| | | KR 20090061061 A | 15 June 2009 |
| | | KR 101414786 B1 | 03 July 2014 |
| | | US 2017369471 A1 | 28 December 2017 |
| | | EP 2066656 A2 | 10 June 2009 |
| | | EP 2066656 B1 | 25 April 2012 |
| | | US 2010093774 A1 | 15 April 2010 |
| | | US 8921385 B2 | 30 December 2014 |
| | | EP 2420498 A1 | 22 February 2012 |
| | | EP 2420498 B1 | 31 May 2017 |
| | | HRP 20120447 T1 | 30 June 2012 |
| | | AR 063015 A1 | 23 December 2008 |
| | | NI 200900040 A | 01 February 2010 |
| | | ES 2633818 T3 | 25 September 2017 |
| | | JP 2014028850 A | 13 February 2014 |
| | | JP 5901599 B2 | 13 April 2016 |
| | | JP 2016053066 A | 14 April 2016 |
| | | JP 6133383 B2 | 24 May 2017 |
| | | JP 2010504973 A | 18 February 2010 |

Form PCT/ISA/210 (patent family annex) (July 2022)

**INTERNATIONAL SEARCH REPORT**
**Information on patent family members**

International application No.
**PCT/CN2023/075345**

| Patent document cited in search report | Publication date (day/month/year) | Patent family member(s) | Publication date (day/month/year) |
|---|---|---|---|
| | | JP 5388854 B2 | 15 January 2014 |
| | | ME 02890 B | 20 April 2018 |
| | | DK 2066656 T3 | 21 May 2012 |
| | | SI 2066656 T1 | 29 June 2012 |
| | | HK 1167397 A1 | 30 November 2012 |
| | | PL 2066656 T3 | 28 September 2012 |
| | | JP 2017165744 A | 21 September 2017 |
| | | CR 10712 A | 13 May 2009 |
| | | HUE 035308 T2 | 02 May 2018 |
| | | RS 54607 B1 | 31 August 2016 |
| | | HRP 20171093 T1 | 06 October 2017 |
| | | ME 02420 B | 20 September 2016 |
| | | RU 2017113360 A3 | 18 October 2018 |
| | | RU 2012127334 A | 10 January 2014 |
| | | RU 2617989 C2 | 02 May 2017 |
| | | NZ 575830 A | 30 March 2012 |
| | | SI 2420498 T1 | 29 December 2017 |
| | | LT 2420498 T | 25 October 2017 |
| | | PL 2420498 T3 | 31 October 2017 |
| | | DK 2420498 T3 | 14 August 2017 |
| | | ES 2385680 T3 | 30 July 2012 |
| | | PT 2420498 T | 01 August 2017 |
| | | HRP 20171106 T1 | 06 October 2017 |
| | | AT 555104 T | 15 May 2012 |
| | | CO 6160323 A2 | 20 May 2010 |
| | | ME 01421 B | 20 December 2013 |
| | | CY 1119176 T1 | 14 February 2018 |
| | | CA 2663731 A1 | 03 April 2008 |
| | | CA 2663731 C | 19 May 2015 |
| | | ME 02829 B | 20 January 2018 |
| | | RU 2009115653 A | 10 November 2010 |
| | | RU 2463298 C2 | 10 October 2012 |
| | | RS 52349 B | 31 December 2012 |
| | | EP 2428513 A1 | 14 March 2012 |
| | | EP 2428513 B1 | 31 May 2017 |
| | | ES 2634915 T3 | 29 September 2017 |
| | | TW 200823192 A | 01 June 2008 |
| | | TWI 399372 B | 21 June 2013 |
| | | HRP 20160266 T1 | 08 April 2016 |
| | | MX 2009003038 A | 15 April 2009 |
| | | US 2008161328 A1 | 03 July 2008 |
| | | US 7635700 B2 | 22 December 2009 |
| | | CY 1112860 T1 | 13 April 2016 |
| | | EP 2420497 A1 | 22 February 2012 |
| | | EP 2420497 B1 | 16 December 2015 |
| CN 103688176 A | 26 March 2014 | AU 2012249491 A1 | 14 November 2013 |
| | | AU 2012249491 B2 | 15 December 2016 |
| | | CA 2834535 A1 | 01 November 2012 |
| | | US 2012322073 A1 | 20 December 2012 |
| | | US 9365640 B2 | 14 June 2016 |

Form PCT/ISA/210 (patent family annex) (July 2022)

**INTERNATIONAL SEARCH REPORT**
**Information on patent family members**

International application No.
**PCT/CN2023/075345**

| Patent document cited in search report | Publication date (day/month/year) | Patent family member(s) | | | Publication date (day/month/year) |
|---|---|---|---|---|---|
| | | NZ | 617142 | A | 27 November 2015 |
| | | ZA | 201308111 | B | 26 April 2017 |
| | | KR | 20140024914 | A | 03 March 2014 |
| | | AU | 2017201674 | A1 | 30 March 2017 |
| | | EP | 2702410 | A2 | 05 March 2014 |
| | | EP | 3096142 | A2 | 23 November 2016 |
| | | EP | 3096142 | A3 | 08 March 2017 |
| | | IL | 229114 | A0 | 31 December 2013 |
| | | IL | 229114 | B | 31 January 2018 |
| | | JP | 2014514579 | A | 19 June 2014 |
| | | JP | 6016892 | B2 | 26 October 2016 |
| | | MX | 2013012564 | A | 12 January 2015 |
| | | MX | 353482 | B | 16 January 2018 |
| | | JP | 2017037081 | A | 16 February 2017 |
| | | JP | 6389217 | B2 | 12 September 2018 |
| | | WO | 2012149299 | A2 | 01 November 2012 |
| | | WO | 2012149299 | A3 | 27 June 2013 |
| | | US | 2016319005 | A1 | 03 November 2016 |
| | | US | 10047151 | B2 | 14 August 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Clinical Pharmacology. People's Public Health Press, 2008
- CAS, 768-94-5